(19) Europäisches Patentamt
European Patent Office
Office européen des brevets

(11) EP 2 812 350 B1

(12) **EUROPEAN PATENT SPECIFICATION**

(45) Date of publication and mention of the grant of the patent:
**03.04.2019 Bulletin 2019/14**

(21) Application number: **13706133.9**

(22) Date of filing: **11.02.2013**

(51) Int Cl.:
***C07K 14/415*** *(2006.01)*

(86) International application number:
**PCT/US2013/025525**

(87) International publication number:
**WO 2013/120056 (15.08.2013 Gazette 2013/33)**

# (54) R-SPONDIN TRANSLOCATIONS AND METHODS USING THE SAME

R-SPONDIN-TRANSLOKATIONEN UND VERFAHREN DAMIT

TRANSLOCATIONS DE R-SPONDINE ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.02.2012 US 201261597746 P**
**23.07.2012 US 201261674763 P**

(43) Date of publication of application:
**17.12.2014 Bulletin 2014/51**

(60) Divisional application:
**19158106.5**

(73) Proprietor: **F. Hoffmann-La Roche AG**
**4070 Basel (CH)**

(72) Inventors:
- **MODRUSAN, Zora**
**South San Francisco, California 94080 (US)**
- **DE SAUVAGE, Frederic, J.**
**South San Francisco, California 94080 (US)**
- **DURINCK, Steffen**
**South San Francisco, California 94080 (US)**
- **SESHAGIRI, Somasekar**
**South San Francisco, California 94080 (US)**
- **STAWISKI, Eric William**
**South San Francisco, California 94080 (US)**

(74) Representative: **Brodbeck, Michel et al**
**F. Hoffmann-La Roche AG**
**Patent Department**
**Grenzacherstrasse 124**
**4070 Basel (CH)**

(56) References cited:
- **J. WAALER ET AL: "Novel Synthetic Antagonists of Canonical Wnt Signaling Inhibit Colorectal Cancer Cell Growth", CANCER RESEARCH, vol. 71, no. 1, 1 January 2011 (2011-01-01), pages 197-205, XP55060427, ISSN: 0008-5472, DOI: 10.1158/0008-5472.CAN-10-1282**
- **ROBERT CALLAHAN ET AL: "Common Integration Sites for MMTV in Viral Induced Mouse Mammary Tumors", JOURNAL OF MAMMARY GLAND BIOLOGY AND NEOPLASIA, KLUWER ACADEMIC PUBLISHERS-PLENUM PUBLISHERS, NE, vol. 133, no. 3, 15 August 2008 (2008-08-15), pages 309-321, XP019609089, ISSN: 1573-7039**
- **YOON JEONG KYO ET AL: "Cellular signaling and biological functions of R-spondins", CELLULAR SIGNALLING, ELSEVIER SCIENCE LTD, GB, vol. 24, no. 2, 1 February 2012 (2012-02-01), pages 369-377, XP009156223, ISSN: 0898-6568, DOI: 10.1016/J.CELLSIG.2011.09.023 [retrieved on 2011-10-01]**
- **, 27 March 2009 (2009-03-27), XP55060581, DOI: 10.1126/science.1084238) Retrieved from the Internet: URL:http://www.sciencemag.org/content/323/5922/1747.full.pdf?sid=666cea18-1bee-4e41-8a2b-c3c94dab06a3 [retrieved on 2013-04-22]**
- **SOMASEKAR SESHAGIRI ET AL: "Recurrent R-spondin fusions in colon cancer", NATURE, vol. 488, no. 7413, 30 August 2012 (2012-08-30), pages 660-664, XP55060432, ISSN: 0028-0836, DOI: 10.1038/nature11282**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 812 350 B1

SEQUENCE LISTING

<110> GENENTECH, INC. ET AL.

<120> R-SPONDIN TRANSLOCATIONS AND METHODS USING THE SAME

<130> P4853R1-WO

<140>
<141>

<150> 61/674,763
<151> 2012-07-23

<150> 61/597,746
<151> 2012-02-11

<160> 120

<170> PatentIn version 3.5

<210> 1
<211> 792
<212> DNA
<213> Homo sapiens

<400> 1

atgcggcttg ggctgtgtgt ggtggccctg gttctgagct ggacgcacct caccatcagc 60

agccggggga tcaaggggaa aaggcagagg cggatcagtg ccgaggggag ccaggcctgt 120

gccaaaggct gtgagctctg ctctgaagtc aacggctgcc tcaagtgctc acccaagctg 180

ttcatcctgc tggagaggaa cgacatccgc caggtgggcg tctgcttgcc gtcctgccca 240

cctggatact tcgacgcccg caacccccgac atgaacaagt gcatcaaatg caagatcgag 300

cactgtgagg cctgcttcag ccataacttc tgcaccaagt gtaaggaggg cttgtacctg 360

cacaagggcc gctgctatcc agcttgtccc gagggctcct cagctgccaa tggcaccatg 420

gagtgcagta gtcctgcgca atgtgaaatg agcgagtggt ctccgtgggg gccctgctcc 480

aagaagcagc agctctgtgg tttccggagg ggctccgagg agcggacacg cagggtgcta 540

catgcccctg tgggggacca tgctgcctgc tctgacacca aggagacccg gaggtgcaca 600

gtgaggagag tgccgtgtcc tgaggggcag aagaggagga agggaggcca gggccggcgg 660

gagaatgcca acaggaacct ggccaggaag gagagcaagg aggcgggtgc tggctctcga 720

agacgcaagg ggcagcaaca gcagcagcag caagggacag tggggccact cacatctgca 780

gggcctgcct ag 792

<210> 2
<211> 263
<212> PRT
<213> Homo sapiens

<400> 2

Met Arg Leu Gly Leu Cys Val Val Ala Leu Val Leu Ser Trp Thr His

```
1               5                   10                  15

Leu Thr Ile Ser Ser Arg Gly Ile Lys Gly Lys Arg Gln Arg Arg Ile
            20                  25                  30

Ser Ala Glu Gly Ser Gln Ala Cys Ala Lys Gly Cys Glu Leu Cys Ser
        35                  40                  45

Glu Val Asn Gly Cys Leu Lys Cys Ser Pro Lys Leu Phe Ile Leu Leu
    50                  55                  60

Glu Arg Asn Asp Ile Arg Gln Val Gly Val Cys Leu Pro Ser Cys Pro
65                  70                  75                  80

Pro Gly Tyr Phe Asp Ala Arg Asn Pro Asp Met Asn Lys Cys Ile Lys
                85                  90                  95

Cys Lys Ile Glu His Cys Glu Ala Cys Phe Ser His Asn Phe Cys Thr
            100                 105                 110

Lys Cys Lys Glu Gly Leu Tyr Leu His Lys Gly Arg Cys Tyr Pro Ala
        115                 120                 125

Cys Pro Glu Gly Ser Ser Ala Ala Asn Gly Thr Met Glu Cys Ser Ser
    130                 135                 140

Pro Ala Gln Cys Glu Met Ser Glu Trp Ser Pro Trp Gly Pro Cys Ser
145                 150                 155                 160

Lys Lys Gln Gln Leu Cys Gly Phe Arg Arg Gly Ser Glu Glu Arg Thr
                165                 170                 175

Arg Arg Val Leu His Ala Pro Val Gly Asp His Ala Ala Cys Ser Asp
            180                 185                 190

Thr Lys Glu Thr Arg Arg Cys Thr Val Arg Arg Val Pro Cys Pro Glu
        195                 200                 205

Gly Gln Lys Arg Arg Lys Gly Gly Gln Gly Arg Arg Glu Asn Ala Asn
    210                 215                 220

Arg Asn Leu Ala Arg Lys Glu Ser Lys Glu Ala Gly Ala Gly Ser Arg
225                 230                 235                 240

Arg Arg Lys Gly Gln Gln Gln Gln Gln Gln Gln Gly Thr Val Gly Pro
                245                 250                 255
```

Leu Thr Ser Ala Gly Pro Ala
260

<210> 3
<211> 732
<212> DNA
<213> Homo sapiens

<400> 3

```
atgcagtttc gccttttctc ctttgccctc atcattctga actgcatgga ttacagccac       60
tgccaaggca accgatggag acgcagtaag cgagctagtt atgtatcaaa tcccatttgc      120
aagggttgtt tgtcttgttc aaaggacaat gggtgtagcc gatgtcaaca gaagttgttc      180
ttcttccttc gaagagaagg gatgcgccag tatggagagt gcctgcattc ctgcccatcc      240
gggtactatg gacaccgagc cccagatatg aacagatgtg caagatgcag aatagaaaac      300
tgtgattctt gctttagcaa agacttttgt accaagtgca aagtaggctt ttatttgcat      360
agaggccgtt gctttgatga atgtccagat ggttttgcac cattagaaga aaccatggaa      420
tgtgtggaag gatgtgaagt tggtcattgg agcgaatggg gaacttgtag cagaaataat      480
cgcacatgtg gatttaaatg gggtctggaa accagaacac ggcaaattgt taaaaagcca      540
gtgaaagaca caatactgtg tccaaccatt gctgaatcca ggagatgcaa gatgacaatg      600
aggcattgtc caggagggaa gagaacacca aaggcgaagg agaagaggaa caagaaaaag      660
aaaaggaagc tgatagaaag ggcccaggag caacacagcg tcttcctagc tacagacaga      720
gctaaccaat aa                                                           732
```

<210> 4
<211> 243
<212> PRT
<213> Homo sapiens

<400> 4

```
Met Gln Phe Arg Leu Phe Ser Phe Ala Leu Ile Ile Leu Asn Cys Met
1               5                   10                  15

Asp Tyr Ser His Cys Gln Gly Asn Arg Trp Arg Arg Ser Lys Arg Ala
            20                  25                  30

Ser Tyr Val Ser Asn Pro Ile Cys Lys Gly Cys Leu Ser Cys Ser Lys
        35                  40                  45

Asp Asn Gly Cys Ser Arg Cys Gln Gln Lys Leu Phe Phe Phe Leu Arg
    50                  55                  60

Arg Glu Gly Met Arg Gln Tyr Gly Glu Cys Leu His Ser Cys Pro Ser
65                  70                  75                  80

Gly Tyr Tyr Gly His Arg Ala Pro Asp Met Asn Arg Cys Ala Arg Cys
                85                  90                  95

Arg Ile Glu Asn Cys Asp Ser Cys Phe Ser Lys Asp Phe Cys Thr Lys
            100                 105                 110

Cys Lys Val Gly Phe Tyr Leu His Arg Gly Arg Cys Phe Asp Glu Cys
        115                 120                 125

Pro Asp Gly Phe Ala Pro Leu Glu Glu Thr Met Glu Cys Val Glu Gly
    130                 135                 140

Cys Glu Val Gly His Trp Ser Glu Trp Gly Thr Cys Ser Arg Asn Asn
145                 150                 155                 160

Arg Thr Cys Gly Phe Lys Trp Gly Leu Glu Thr Arg Thr Arg Gln Ile
                165                 170                 175

Val Lys Lys Pro Val Lys Asp Thr Ile Leu Cys Pro Thr Ile Ala Glu
            180                 185                 190

Ser Arg Arg Cys Lys Met Thr Met Arg His Cys Pro Gly Gly Lys Arg
        195                 200                 205

Thr Pro Lys Ala Lys Glu Lys Arg Asn Lys Lys Lys Lys Arg Lys Leu
    210                 215                 220

Ile Glu Arg Ala Gln Glu Gln His Ser Val Phe Leu Ala Thr Asp Arg
225                 230                 235                 240

Ala Asn Gln
```

<210> 5
<211> 819
<212> DNA
<213> Homo sapiens

<400> 5

```
atgcacttgc gactgatttc ttggcttttt atcattttga actttatgga atacatcggc     60
agccaaaacg cctcccgggg aaggcgccag cgaagaatgc atcctaacgt tagtcaaggc    120
tgccaaggag gctgtgcaac atgctcagat tacaatggat gtttgtcatg taagcccaga    180
ctattttttg ctctggaaag aattggcatg aagcagattg gagtatgtct ctcttcatgt    240
ccaagtggat attatggaac tcgatatcca gatataaata agtgtacaaa atgcaaagct    300
gactgtgata cctgtttcaa caaaaatttc tgcacaaaat gtaaaagtgg attttactta    360
caccttggaa agtgccttga caattgccca gaagggttgg aagccaacaa ccatactatg    420
gagtgtgtca gtattgtgca ctgtgaggtc agtgaatgga atccttggag tccatgcacg    480
aagaagggaa aaacatgtgg cttcaaaaga gggactgaaa cacgggtccg agaaataata    540
cagcatcctt cagcaaaggg taacctgtgt cccccaacaa atgagacaag aaagtgtaca    600
gtgcaaagga agaagtgtca gaagggagaa cgaggaaaaa aaggaaggga gaggaaaaga    660
aaaaaaccta ataaaggaga aagtaaagaa gcaatacctg acagcaaaag tctggaatcc    720
agcaaagaaa tcccagagca acgagaaaac aaacagcagc agaagaagcg aaaagtccaa    780
gataaacaga aatcggtatc agtcagcact gtacactag                           819
```

<210> 6
<211> 219
<212> PRT
<213> Homo sapiens

<400> 6

```
Met His Leu Arg Leu Ile Ser Trp Leu Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys
        35                  40                  45

Ser Asp Tyr Asn Gly Cys Leu Ser Cys Lys Pro Arg Leu Phe Phe Ala
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Ala Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Asn
        115                 120                 125

Cys Pro Glu Gly Leu Glu Ala Asn Asn His Thr Met Glu Cys Val Ser
    130                 135                 140

Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val


                165                 170                 175

Arg Glu Ile Ile Gln His Pro Ser Ala Lys Gly Asn Leu Cys Pro Pro
            180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys
    210                 215
```

<210> 7
<211> 705
<212> DNA
<213> Homo sapiens

<400> 7

```
atgcgggcgc cactctgcct gctcctgctc gtcgcccacg ccgtggacat gctcgccctg       60
aaccgaagga agaagcaagt gggcactggc ctgggggggca actgcacagg ctgtatcatc     120
tgctcagagg agaacggctg ttccacctgc cagcagaggc tcttcctgtt catccgccgg      180
gaaggcatcc gccagtacgg caagtgcctg cacgactgtc cccctgggta cttcggcatc      240
cgcggccagg aggtcaacag gtgcaaaaaa tgtggggcca cttgtgagag ctgcttcagc      300
caggacttct gcatccggtg caagaggcag ttttacttgt acaaggggaa gtgtctgccc      360
acctgcccgc cgggcacttt ggcccaccag aacacacggg agtgccaggg ggagtgtgaa      420
ctgggtccct ggggcggctg gagcccctgc acacacaatg gaaagacctg cggctcggct      480
tggggcctgg agagccgggt acgagaggct ggccgggctg ggcatgagga ggcagccacc      540
tgccaggtgc tttctgagtc aaggaaatgt cccatccaga ggccctgccc aggagagagg      600
agccccggcc agaagaaggg caggaaggac cggcgcccac gcaaggacag gaagctggac      660
cgcaggctgg acgtgaggcc gcgccagccc ggcctgcagc cctga                      705
```

<210> 8
<211> 234
<212> PRT
<213> Homo sapiens

<400> 8

```
Met Arg Ala Pro Leu Cys Leu Leu Leu Leu Val Ala His Ala Val Asp
1               5                   10                  15

Met Leu Ala Leu Asn Arg Arg Lys Lys Gln Val Gly Thr Gly Leu Gly
            20                  25                  30

Gly Asn Cys Thr Gly Cys Ile Ile Cys Ser Glu Glu Asn Gly Cys Ser
        35                  40                  45
```

Thr Cys Gln Gln Arg Leu Phe Leu Phe Ile Arg Arg Glu Gly Ile Arg
50 55 60

Gln Tyr Gly Lys Cys Leu His Asp Cys Pro Pro Gly Tyr Phe Gly Ile
65 70 75 80

Arg Gly Gln Glu Val Asn Arg Cys Lys Lys Cys Gly Ala Thr Cys Glu
85 90 95

Ser Cys Phe Ser Gln Asp Phe Cys Ile Arg Cys Lys Arg Gln Phe Tyr
100 105 110

Leu Tyr Lys Gly Lys Cys Leu Pro Thr Cys Pro Pro Gly Thr Leu Ala
115 120 125

His Gln Asn Thr Arg Glu Cys Gln Gly Glu Cys Glu Leu Gly Pro Trp
130 135 140

Gly Gly Trp Ser Pro Cys Thr His Asn Gly Lys Thr Cys Gly Ser Ala
145 150 155 160

Trp Gly Leu Glu Ser Arg Val Arg Glu Ala Gly Arg Ala Gly His Glu
165 170 175

Glu Ala Ala Thr Cys Gln Val Leu Ser Glu Ser Arg Lys Cys Pro Ile
180 185 190

Gln Arg Pro Cys Pro Gly Glu Arg Ser Pro Gly Gln Lys Lys Gly Arg
195 200 205

Lys Asp Arg Arg Pro Arg Lys Asp Arg Lys Leu Asp Arg Arg Leu Asp
210 215 220

Val Arg Pro Arg Gln Pro Gly Leu Gln Pro
225 230

<210> 9
<211> 54
<212> PRT
<213> Artificial Sequence

<220>
<221> source
<223> /note="Description of Artificial Sequence: Synthetic polypeptide"

<400> 9

Met Phe Leu Ser Ala Val Phe Phe Ala Lys Ser Lys Ser Asn Glu Thr
1 5 10 15

```
Lys Ser Pro Leu Arg Gly Lys Glu Lys Asn Thr Leu Pro Leu Asn Gly
            20                  25                  30

Gly Leu Lys Met Thr Leu Ile Tyr Lys Glu Lys Thr Glu Gly Gly Asp
        35                  40                  45

Thr Asp Ser Glu Ile Leu
    50
```

<210> 10
<211> 74
<212> PRT
<213> Artificial Sequence

<220>
<221> source
<223> /note="Description of Artificial Sequence: Synthetic polypeptide"

<400> 10

```
Met Met Ala His Leu Asp Phe Phe Leu Thr Tyr Lys Trp Arg Ala Pro
1               5                   10                  15

Lys Ser Lys Ser Leu Asp Gln Leu Ser Pro Asn Phe Leu Leu Arg Gly
            20                  25                  30

Arg Ser Glu Thr Lys Ser Pro Leu Arg Gly Lys Glu Lys Asn Thr Leu
        35                  40                  45

Pro Leu Asn Gly Gly Leu Lys Met Thr Leu Ile Tyr Lys Glu Lys Thr
    50                  55                  60

Glu Gly Gly Asp Thr Asp Ser Glu Ile Leu
65                  70
```

<210> 11
<211> 19
<212> DNA
<213> Artificial Sequence

<220>
<221> source
<223> /note="Description of Artificial Sequence: Synthetic primer"

<400> 11
cttgcggaaa ggatgttgg 19

<210> 12
<211> 19
<212> DNA
<213> Artificial Sequence

<220>

<221> source
<223> /note="Description of Artificial Sequence: Synthetic primer"

<400> 12
actactcgca tcgcgcact 19

<210> 13
<211> 20
<212> DNA
<213> Artificial Sequence

<220>
<221> source
<223> /note="Description of Artificial Sequence: Synthetic primer"

<400> 13
aaactcggca tggatacgac 20

<210> 14
<211> 20
<212> DNA
<213> Artificial Sequence

<220>
<221> source
<223> /note="Description of Artificial Sequence: Synthetic primer"

<400> 14
tgcagtcaat gctccaactt 20

<210> 15
<211> 20
<212> DNA
<213> Artificial Sequence

<220>
<221> source
<223> /note="Description of Artificial Sequence: Synthetic primer"

<400> 15
aagcccatca acctctctca 20

<210> 16
<211> 20
<212> DNA
<213> Artificial Sequence

<220>
<221> source
<223> /note="Description of Artificial Sequence: Synthetic primer"

<400> 16
ctctacaccc ccaagtgcat 20

<210> 17
<211> 20
<212> DNA
<213> Artificial Sequence

<220>
<221> source
<223> /note="Description of Artificial Sequence: Synthetic primer"

<400> 17
aacaggagac ccgtacatgc 20

<210> 18
<211> 21
<212> DNA
<213> Artificial Sequence

<220>
<221> source
<223> /note="Description of Artificial Sequence: Synthetic primer"

<400> 18
ccagctgcta gctactgtgg a 21

<210> 19
<211> 21
<212> DNA
<213> Artificial Sequence

<220>
<221> source
<223> /note="Description of Artificial Sequence: Synthetic primer"

<400> 19
tgaaccgaag tttagcaatg g 21

<210> 20
<211> 20
<212> DNA
<213> Artificial Sequence

<220>
<221> source
<223> /note="Description of Artificial Sequence: Synthetic primer"

<400> 20
tgatgaactt tgcagccact 20

<210> 21
<211> 20
<212> DNA
<213> Artificial Sequence

<220>
<221> source
<223> /note="Description of Artificial Sequence: Synthetic primer"

<400> 21
agggccagat ttgagtgtgt 20

<210> 22
<211> 20
<212> DNA

<213> Artificial Sequence

<220>
<221> source
<223> /note="Description of Artificial Sequence: Synthetic primer"

<400> 22
gtgtatggcg tcgtgatgtc 20

<210> 23
<211> 20
<212> DNA
<213> Artificial Sequence

<220>
<221> source
<223> /note="Description of Artificial Sequence: Synthetic primer"

<400> 23
catgtcggag aacatctgga 20

<210> 24
<211> 20
<212> DNA
<213> Artificial Sequence

<220>
<221> source
<223> /note="Description of Artificial Sequence: Synthetic primer"

<400> 24
ccttactgcc ttgtgggaga 20

<210> 25
<211> 20
<212> DNA
<213> Artificial Sequence

<220>
<221> source
<223> /note="Description of Artificial Sequence: Synthetic primer"

<400> 25
cagagacccg tgctgagttt 20

<210> 26
<211> 20
<212> DNA
<213> Artificial Sequence

<220>
<221> source
<223> /note="Description of Artificial Sequence: Synthetic primer"

<400> 26
gactttggtg ccctcaacat 20

<210> 27

<211> 20
<212> DNA
<213> Artificial Sequence

<220>
<221> source
<223> /note="Description of Artificial Sequence: Synthetic primer"

<400> 27
aacgggaact cttagcagca 20

<210> 28
<211> 20
<212> DNA
<213> Artificial Sequence

<220>
<221> source
<223> /note="Description of Artificial Sequence: Synthetic primer"

<400> 28
gagacttcat gcgggagttc 20

<210> 29
<211> 21
<212> DNA
<213> Artificial Sequence

<220>
<221> source
<223> /note="Description of Artificial Sequence: Synthetic primer"

<400> 29
tggccttcgc taactacaag a 21

<210> 30
<211> 18
<212> DNA
<213> Artificial Sequence

<220>
<221> source
<223> /note="Description of Artificial Sequence: Synthetic primer"

<400> 30
gctctttggc gcggatta 18

<210> 31
<211> 20
<212> DNA
<213> Artificial Sequence

<220>
<221> source
<223> /note="Description of Artificial Sequence: Synthetic primer"

<400> 31
gttgcaaaag gcttgctgat 20

<210> 32
<211> 20
<212> DNA
<213> Artificial Sequence

<220>
<221> source
<223> /note="Description of Artificial Sequence: Synthetic primer"

<400> 32
tgattgatgc tgccaaacat 20

<210> 33
<211> 20
<212> DNA
<213> Artificial Sequence

<220>
<221> source
<223> /note="Description of Artificial Sequence: Synthetic primer"

<400> 33
atgaacctta tctcggccct 20

<210> 34
<211> 20
<212> DNA
<213> Artificial Sequence

<220>
<221> source
<223> /note="Description of Artificial Sequence: Synthetic primer"

<400> 34
atgtgtacgc agaagagcca 20

<210> 35
<211> 21
<212> DNA
<213> Artificial Sequence

<220>
<221> source
<223> /note="Description of Artificial Sequence: Synthetic primer"

<400> 35
ggaaaatcct catatttgcc a 21

<210> 36
<211> 20
<212> DNA
<213> Artificial Sequence

<220>
<221> source
<223> /note="Description of Artificial Sequence: Synthetic primer"

<400> 36

agacccagga ggagtgaggt 20

<210> 37
<211> 20
<212> DNA
<213> Artificial Sequence

<220>
<221> source
<223> /note="Description of Artificial Sequence: Synthetic primer"

<400> 37
agatgcccag atgcaaaagt 20

<210> 38
<211> 20
<212> DNA
<213> Artificial Sequence

<220>
<221> source
<223> /note="Description of Artificial Sequence: Synthetic primer"

<400> 38
ggctgagggt ggagtttgta 20

<210> 39
<211> 20
<212> DNA
<213> Artificial Sequence

<220>
<221> source
<223> /note="Description of Artificial Sequence: Synthetic primer"

<400> 39
ccccagttag aaggggaaga 20

<210> 40
<211> 20
<212> DNA
<213> Artificial Sequence

<220>
<221> source
<223> /note="Description of Artificial Sequence: Synthetic primer"

<400> 40
tggtgatcca gagaagaagc 20

<210> 41
<211> 20
<212> DNA
<213> Artificial Sequence

<220>
<221> source
<223> /note="Description of Artificial Sequence: Synthetic primer"

<400> 41
gggaggactc agagggagac 20

<210> 42
<211> 20
<212> DNA
<213> Artificial Sequence

<220>
<221> source
<223> /note="Description of Artificial Sequence: Synthetic primer"

<400> 42
tgcaggcact ctccatactg 20

<210> 43
<211> 20
<212> DNA
<213> Artificial Sequence

<220>
<221> source
<223> /note="Description of Artificial Sequence: Synthetic primer"

<400> 43
gcttcatgcc aattctttcc 20

<210> 44
<211> 20
<212> DNA
<213> Artificial Sequence

<220>
<221> source
<223> /note="Description of Artificial Sequence: Synthetic primer"

<400> 44
gccaattctt tccagagcaa 20

<210> 45
<211> 20
<212> DNA
<213> Artificial Sequence

<220>
<221> source
<223> /note="Description of Artificial Sequence: Synthetic primer"

<400> 45
gggctgaggt tgtagcactc 20

<210> 46
<211> 21
<212> DNA
<213> Artificial Sequence

<220>
<221> source

<223> /note="Description of Artificial Sequence: Synthetic primer"

<400> 46
tgacaccata atggattcct g 21

<210> 47
<211> 20
<212> DNA
<213> Artificial Sequence

<220>
<221> source
<223> /note="Description of Artificial Sequence: Synthetic primer"

<400> 47
aaagggcaca gattgccata 20

<210> 48
<211> 20
<212> DNA
<213> Artificial Sequence

<220>
<221> source
<223> /note="Description of Artificial Sequence: Synthetic primer"

<400> 48
actaggtggt ccagggtgtg 20

<210> 49
<211> 20
<212> DNA
<213> Artificial Sequence

<220>
<221> source
<223> /note="Description of Artificial Sequence: Synthetic primer"

<400> 49
tgctcaagca ggtaagatgc 20

<210> 50
<211> 20
<212> DNA
<213> Artificial Sequence

<220>
<221> source
<223> /note="Description of Artificial Sequence: Synthetic primer"

<400> 50
atggtctcca tcagctctcg 20

<210> 51
<211> 20
<212> DNA
<213> Artificial Sequence

<220>
<221> source
<223> /note="Description of Artificial Sequence: Synthetic primer"

<400> 51
aaactgaaaa tccccgctgt 20

<210> 52
<211> 20
<212> DNA
<213> Artificial Sequence

<220>
<221> source
<223> /note="Description of Artificial Sequence: Synthetic primer"

<400> 52
gctccagtca ccaaaaggag 20

<210> 53
<211> 20
<212> DNA
<213> Artificial Sequence

<220>
<221> source
<223> /note="Description of Artificial Sequence: Synthetic primer"

<400> 53
tgtggagtct cttgcgtgtc 20

<210> 54
<211> 20
<212> DNA
<213> Artificial Sequence

<220>
<221> source
<223> /note="Description of Artificial Sequence: Synthetic primer"

<400> 54
tggggatgag gtcgatgtat 20

<210> 55
<211> 20
<212> DNA
<213> Artificial Sequence

<220>
<221> source
<223> /note="Description of Artificial Sequence: Synthetic primer"

<400> 55
ccaaaaggtg tttcgtcctt 20

<210> 56
<211> 21
<212> DNA

<213> Artificial Sequence

<220>
<221> source
<223> /note="Description of Artificial Sequence: Synthetic primer"

<400> 56
caatttttcc actccaacac c 21

<210> 57
<211> 20
<212> DNA
<213> Artificial Sequence

<220>
<221> source
<223> /note="Description of Artificial Sequence: Synthetic primer"

<400> 57
catgtcaaac caccatccac 20

<210> 58
<211> 20
<212> DNA
<213> Artificial Sequence

<220>
<221> source
<223> /note="Description of Artificial Sequence: Synthetic primer"

<400> 58
atctggaagc aggggtcttt 20

<210> 59
<211> 20
<212> DNA
<213> Artificial Sequence

<220>
<221> source
<223> /note="Description of Artificial Sequence: Synthetic primer"

<400> 59
tccccatatt tctgcactcc 20

<210> 60
<211> 18
<212> DNA
<213> Artificial Sequence

<220>
<221> source
<223> /note="Description of Artificial Sequence: Synthetic primer"

<400> 60
ggagctacct gtggccct 18

<210> 61

<211> 20
<212> DNA
<213> Artificial Sequence

<220>
<221> source
<223> /note="Description of Artificial Sequence: Synthetic primer"

<400> 61
acgaaggctt cctcacagaa 20

<210> 62
<211> 20
<212> DNA
<213> Artificial Sequence

<220>
<221> source
<223> /note="Description of Artificial Sequence: Synthetic primer"

<400> 62
cacgcttttc atattcccgt 20

<210> 63
<211> 20
<212> DNA
<213> Artificial Sequence

<220>
<221> source
<223> /note="Description of Artificial Sequence: Synthetic primer"

<400> 63
tcccaaaggc ttcttcttga 20

<210> 64
<211> 19
<212> DNA
<213> Artificial Sequence

<220>
<221> source
<223> /note="Description of Artificial Sequence: Synthetic primer"

<400> 64
gtcgtgtacc ccagaggct 19

<210> 65
<211> 20
<212> DNA
<213> Artificial Sequence

<220>
<221> source
<223> /note="Description of Artificial Sequence: Synthetic primer"

<400> 65
gtgcaggaat tgggctatgt 20

<210> 66
<211> 20
<212> DNA
<213> Artificial Sequence

<220>
<221> source
<223> /note="Description of Artificial Sequence: Synthetic primer"

<400> 66
agcagggaag cctcctagtc 20

<210> 67
<211> 20
<212> DNA
<213> Artificial Sequence

<220>
<221> source
<223> /note="Description of Artificial Sequence: Synthetic primer"

<400> 67
ggtcagccag tgaggtcttc 20

<210> 68
<211> 20
<212> DNA
<213> Artificial Sequence

<220>
<221> source
<223> /note="Description of Artificial Sequence: Synthetic primer"

<400> 68
caaagcagac tttccaacgc 20

<210> 69
<211> 20
<212> DNA
<213> Artificial Sequence

<220>
<221> source
<223> /note="Description of Artificial Sequence: Synthetic primer"

<400> 69
cttctgatcg aagctttccg 20

<210> 70
<211> 20
<212> DNA
<213> Artificial Sequence

<220>
<221> source
<223> /note="Description of Artificial Sequence: Synthetic primer"

<400> 70

cactctcatc tctgggctcc 20

<210> 71
<211> 20
<212> DNA
<213> Artificial Sequence

<220>
<221> source
<223> /note="Description of Artificial Sequence: Synthetic oligonucleotide"

<400> 71
tgtaaaggag gttcgtggcg 20

<210> 72
<211> 20
<212> DNA
<213> Artificial Sequence

<220>
<221> source
<223> /note="Description of Artificial Sequence: Synthetic oligonucleotide"

<400> 72
ttctccgcag tgcatcctaa 20

<210> 73
<211> 20
<212> DNA
<213> Artificial Sequence

<220>
<221> source
<223> /note="Description of Artificial Sequence: Synthetic oligonucleotide"

<400> 73
aaatgtgcag tgcatcctaa 20

<210> 74
<211> 1019
<212> DNA
<213> Artificial Sequence

<220>
<221> source
<223> /note="Description of Artificial Sequence: Synthetic polynucleotide"

<400> 74

gagcacagac tcccttttct ttggcaagat ggcggagtac gacttgacta ctcgcatcgc 60

gcactttttg gatcggcatc tagtctttcc gcttcttgaa tttctctctg taaaggaggt 120

tcgtggcgga gagatgctga tcgcgctgaa ctgaccggtg cggcccgggg gtgagtggcg 180

agtctccctc tgagtcctcc ccagcagcgc ggccggcgcc ggctctttgg gcgaaccctc 240

```
cagttcctag actttgagag gcgtctctcc cccgcccgac cgcccagatg cagtttcgcc      300
ttttctcctt tgccctcatc attctgaact gcatggatta cagccactgc caaggcaacc      360
gatggagacg cagtaagcga gctagttatg tatcaaatcc catttgcaag ggttgtttgt      420
cttgttcaaa ggacaatggg tgtagccgat gtcaacagaa gttgttcttc ttccttcgaa      480
gagaagggat gcgccagtat ggagagtgcc tgcattcctg cccatccggg tactatggac      540
accgagcccc agatatgaac agatgtgcaa gatgcagaat agaaaactgt gattcttgct      600
ttagcaaaga cttttgtacc aagtgcaaag taggctttta tttgcataga ggccgttgct      660
ttgatgaatg tccagatggt tttgcaccat tagaagaaac catggaatgt gtggaaggat      720
gtgaagttgg tcattggagc gaatggggaa cttgtagcag aaataatcgc acatgtggat      780
ttaaatgggg tctggaaacc agaacacggc aaattgttaa aaagccagtg aaagacacaa      840
tactgtgtcc aaccattgct gaatccagga gatgcaagat gacaatgagg cattgtccag      900
gagggaagag aacaccaaag gcgaaggaga agaggaacaa gaaaaagaaa aggaagctga      960
tagaaagggc ccaggagcaa cacagcgtct tcctagctac agacagagct aaccaataa      1019
```

<210> 75
<211> 284
<212> PRT
<213> Artificial Sequence

<220>
<221> source
<223> /note="Description of Artificial Sequence: Synthetic polypeptide"

<400> 75

```
Met Ala Glu Tyr Asp Leu Thr Thr Arg Ile Ala His Phe Leu Asp Arg
1               5                   10                  15

His Leu Val Phe Pro Leu Leu Glu Phe Leu Ser Val Lys Glu Val Arg
            20                  25                  30

Gly Gly Glu Met Leu Ile Ala Leu Asn Met Gln Phe Arg Leu Phe Ser
        35                  40                  45

Phe Ala Leu Ile Ile Leu Asn Cys Met Asp Tyr Ser His Cys Gln Gly
    50                  55                  60

Asn Arg Trp Arg Arg Ser Lys Arg Ala Ser Tyr Val Ser Asn Pro Ile
65                  70                  75                  80

Cys Lys Gly Cys Leu Ser Cys Ser Lys Asp Asn Gly Cys Ser Arg Cys
                85                  90                  95

Gln Gln Lys Leu Phe Phe Phe Leu Arg Arg Glu Gly Met Arg Gln Tyr
```

```
            100                 105                 110

Gly Glu Cys Leu His Ser Cys Pro Ser Gly Tyr Tyr Gly His Arg Ala
        115                 120                 125

Pro Asp Met Asn Arg Cys Ala Arg Cys Arg Ile Glu Asn Cys Asp Ser
    130                 135                 140

Cys Phe Ser Lys Asp Phe Cys Thr Lys Cys Lys Val Gly Phe Tyr Leu
145                 150                 155                 160

His Arg Gly Arg Cys Phe Asp Glu Cys Pro Asp Gly Phe Ala Pro Leu
                165                 170                 175

Glu Glu Thr Met Glu Cys Val Glu Gly Cys Glu Val Gly His Trp Ser
            180                 185                 190

Glu Trp Gly Thr Cys Ser Arg Asn Asn Arg Thr Cys Gly Phe Lys Trp
        195                 200                 205

Gly Leu Glu Thr Arg Thr Arg Gln Ile Val Lys Lys Pro Val Lys Asp
    210                 215                 220

Thr Ile Leu Cys Pro Thr Ile Ala Glu Ser Arg Arg Cys Lys Met Thr
225                 230                 235                 240

Met Arg His Cys Pro Gly Gly Lys Arg Thr Pro Lys Ala Lys Glu Lys
                245                 250                 255

Arg Asn Lys Lys Lys Lys Arg Lys Leu Ile Glu Arg Ala Gln Glu Gln
            260                 265                 270

His Ser Val Phe Leu Ala Thr Asp Arg Ala Asn Gln
        275                 280
```

<210> 76
<211> 822
<212> DNA
<213> Artificial Sequence

<220>
<221> source
<223> /note="Description of Artificial Sequence: Synthetic polynucleotide"

<400> 76

atggatacga ctgcggcggc ggcgctgcct gcttttgtgg cgctcttgct cctctctcct 60

tggcctctcc tgggatcggc ccaaggccag ttctccgcag tgcatcctaa cgttagtcaa 120

ggctgccaag gaggctgtgc aacatgctca gattacaatg gatgtttgtc atgtaagccc 180

agactatttt ttgctctgga aagaattggc atgaagcaga ttggagtatg tctctcttca 240

tgtccaagtg gatattatgg aactcgatat ccagatataa ataagtgtac aaaatgcaaa 300

gctgactgtg atacctgttt caacaaaaat ttctgcacaa aatgtaaaag tggattttac 360

ttacaccttg gaaagtgcct tgacaattgc ccagaagggt tggaagccaa caaccatact 420

atggagtgtg tcagtattgt gcactgtgag gtcagtgaat ggaatccttg gagtccatgc 480

acgaagaagg gaaaaacatg tggcttcaaa agagggactg aaacacgggt ccgagaaata 540

atacagcatc cttcagcaaa gggtaacctg tgtcccccaa caaatgagac aagaaagtgt 600

acagtgcaaa ggaagaagtg tcagaaggga gaacgaggaa aaaaaggaag ggagaggaaa 660

agaaaaaaac ctaataaagg agaaagtaaa gaagcaatac ctgacagcaa aagtctggaa 720

tccagcaaag aaatcccaga gcaacgagaa aacaaacagc agcagaagaa gcgaaaagtc 780

caagataaac agaaatcggt atcagtcagc actgtacact ag 822

<210> 77
<211> 217
<212> PRT
<213> Artificial Sequence

<220>
<221> source
<223> /note="Description of Artificial Sequence: Synthetic polypeptide"

<400> 77

```
Met Asp Thr Thr Ala Ala Ala Ala Leu Pro Ala Phe Val Ala Leu Leu
1               5                   10                  15

Leu Leu Ser Pro Trp Pro Leu Leu Gly Ser Ala Gln Gly Gln Phe Ser
            20                  25                  30

Ala Val His Pro Asn Val Ser Gln Gly Cys Gln Gly Gly Cys Ala Thr
        35                  40                  45

Cys Ser Asp Tyr Asn Gly Cys Leu Ser Cys Lys Pro Arg Leu Phe Phe
    50                  55                  60

Ala Leu Glu Arg Ile Gly Met Lys Gln Ile Gly Val Cys Leu Ser Ser
65                  70                  75                  80

Cys Pro Ser Gly Tyr Tyr Gly Thr Arg Tyr Pro Asp Ile Asn Lys Cys
                85                  90                  95

Thr Lys Cys Lys Ala Asp Cys Asp Thr Cys Phe Asn Lys Asn Phe Cys
            100                 105                 110

Thr Lys Cys Lys Ser Gly Phe Tyr Leu His Leu Gly Lys Cys Leu Asp
        115                 120                 125

Asn Cys Pro Glu Gly Leu Glu Ala Asn Asn His Thr Met Glu Cys Val
    130                 135                 140

Ser Ile Val His Cys Glu Val Ser Glu Trp Asn Pro Trp Ser Pro Cys
145                 150                 155                 160

Thr Lys Lys Gly Lys Thr Cys Gly Phe Lys Arg Gly Thr Glu Thr Arg
                165                 170                 175

Val Arg Glu Ile Ile Gln His Pro Ser Ala Lys Gly Asn Leu Cys Pro
            180                 185                 190

Pro Thr Asn Glu Thr Arg Lys Cys Thr Val Gln Arg Lys Lys Cys Gln
        195                 200                 205

Lys Gly Glu Arg Gly Lys Lys Gly Arg
    210                 215
```

<210> 78
<211> 1884
<212> DNA
<213> Artificial Sequence

<220>

<221> source
<223> /note="Description of Artificial Sequence: Synthetic polynucleotide"

<400> 78

```
atggatacga ctgcggcggc ggcgctgcct gcttttgtgg cgctcttgct cctctctcct      60
tggcctctcc tgggatcggc ccaaggccag ttctccgcag gtggctgtac ttttgatgat     120
ggtccagggg cctgtgatta ccaccaggat ctgtatgatg actttgaatg ggtgcatgtt     180
agtgctcaag agcctcatta tctaccaccc gagatgcccc aaggttccta tatgatagtg     240
gactcttcag atcacgaccc tggagaaaaa gccagacttc agctgcctac aatgaaggag     300
aacgacactc actgcattga tttcagttac ctattatata gccagaaagg actgaatcct     360
ggcactttga acatattagt tagggtgaat aaaggacctc ttgccaatcc aatttggaat     420
gtgactggat tcacgggtag agattggctt cgggctgagc tagcagtgag caccttttgg     480
cccaatgaat atcaggtaat atttgaagct gaagtctcag gagggagaag tggttatatt     540
gccattgatg acatccaagt actgagttat ccttgtgata aatctcctca tttcctccgt     600
ctaggggatg tagaggtgaa tgcagggcaa aacgctacat ttcagtgcat tgccacaggg     660
agagatgctg tgcataacaa gttatggctc cagagacgaa atggagaaga tataccagta     720
```

gcccagacta agaacatcaa tcatagaagg tttgccgctt ccttcagatt gcaagaagtg 780
acaaaaactg accaggattt gtatcgctgt gtaactcagt cagaacgagg ttccggtgtg 840
tccaattttg ctcaacttat tgtgagagaa ccgccaagac ccattgctcc tcctcagctt 900
cttggtgttg ggcctacata tttgctgatc caactaaatg ccaactcgat cattggcgat 960
ggtcctatca tcctgaaaga agtagagtac cgaatgacat caggatcctg gacagaaacc 1020
catgcagtca atgctccaac ttacaaatta tggcatttag atccagatac cgaatatgag 1080
atccgagttc tacttacaag acctggtgaa ggtggaacgg ggctcccagg acctccacta 1140
atcaccagaa caaaatgtgc agtgcatcct aacgttagtc aaggctgcca aggaggctgt 1200
gcaacatgct cagattacaa tggatgtttg tcatgtaagc ccagactatt ttttgctctg 1260
gaaagaattg gcatgaagca gattggagta tgtctctctt catgtccaag tggatattat 1320
ggaactcgat atccagatat aaataagtgt acaaaatgca aagctgactg tgatacctgt 1380
ttcaacaaaa atttctgcac aaaatgtaaa agtggatttt acttacacct tggaaagtgc 1440
cttgacaatt gcccagaagg gttggaagcc aacaaccata ctatggagtg tgtcagtatt 1500
gtgcactgtg aggtcagtga atggaatcct tggagtccat gcacgaagaa gggaaaaaca 1560
tgtggcttca aaagagggac tgaaacacgg gtccgagaaa taatacagca tccttcagca 1620
aagggtaacc tgtgtccccc aacaaatgag acaagaaagt gtacagtgca aaggaagaag 1680
tgtcagaagg gagaacgagg aaaaaaagga agggagagga aaagaaaaaa acctaataaa 1740
ggagaaagta aagaagcaat acctgacagc aaaagtctgg aatccagcaa agaaatccca 1800
gagcaacgag aaaacaaaca gcagcagaag aagcgaaaag tccaagataa acagaaatcg 1860
gtatcagtca gcactgtaca ctag 1884

<210> 79
<211> 627
<212> PRT
<213> Artificial Sequence

<220>
<221> source
<223> /note="Description of Artificial Sequence: Synthetic polypeptide"

<400> 79

```
Met Asp Thr Thr Ala Ala Ala Ala Leu Pro Ala Phe Val Ala Leu Leu
1               5                   10                  15

Leu Leu Ser Pro Trp Pro Leu Leu Gly Ser Ala Gln Gly Gln Phe Ser
            20                  25                  30

Ala Gly Gly Cys Thr Phe Asp Asp Gly Pro Gly Ala Cys Asp Tyr His
        35                  40                  45
```

```
Gln Asp Leu Tyr Asp Asp Phe Glu Trp Val His Val Ser Ala Gln Glu
    50                  55                  60

Pro His Tyr Leu Pro Pro Glu Met Pro Gln Gly Ser Tyr Met Ile Val
65                  70                  75                  80

Asp Ser Ser Asp His Asp Pro Gly Glu Lys Ala Arg Leu Gln Leu Pro
                85                  90                  95

Thr Met Lys Glu Asn Asp Thr His Cys Ile Asp Phe Ser Tyr Leu Leu
            100                 105                 110

Tyr Ser Gln Lys Gly Leu Asn Pro Gly Thr Leu Asn Ile Leu Val Arg
        115                 120                 125

Val Asn Lys Gly Pro Leu Ala Asn Pro Ile Trp Asn Val Thr Gly Phe
    130                 135                 140

Thr Gly Arg Asp Trp Leu Arg Ala Glu Leu Ala Val Ser Thr Phe Trp
145                 150                 155                 160

Pro Asn Glu Tyr Gln Val Ile Phe Glu Ala Glu Val Ser Gly Gly Arg
                165                 170                 175

Ser Gly Tyr Ile Ala Ile Asp Asp Ile Gln Val Leu Ser Tyr Pro Cys
            180                 185                 190

Asp Lys Ser Pro His Phe Leu Arg Leu Gly Asp Val Glu Val Asn Ala
        195                 200                 205

Gly Gln Asn Ala Thr Phe Gln Cys Ile Ala Thr Gly Arg Asp Ala Val
    210                 215                 220

His Asn Lys Leu Trp Leu Gln Arg Arg Asn Gly Glu Asp Ile Pro Val
225                 230                 235                 240

Ala Gln Thr Lys Asn Ile Asn His Arg Arg Phe Ala Ala Ser Phe Arg
                245                 250                 255

Leu Gln Glu Val Thr Lys Thr Asp Gln Asp Leu Tyr Arg Cys Val Thr
            260                 265                 270

Gln Ser Glu Arg Gly Ser Gly Val Ser Asn Phe Ala Gln Leu Ile Val
        275                 280                 285

Arg Glu Pro Pro Arg Pro Ile Ala Pro Pro Gln Leu Leu Gly Val Gly
    290                 295                 300
```

```
Pro Thr Tyr Leu Leu Ile Gln Leu Asn Ala Asn Ser Ile Ile Gly Asp
305                 310                 315                 320

Gly Pro Ile Ile Leu Lys Glu Val Glu Tyr Arg Met Thr Ser Gly Ser
                325                 330                 335

Trp Thr Glu Thr His Ala Val Asn Ala Pro Thr Tyr Lys Leu Trp His
            340                 345                 350

Leu Asp Pro Asp Thr Glu Tyr Glu Ile Arg Val Leu Leu Thr Arg Pro
        355                 360                 365

Gly Glu Gly Gly Thr Gly Leu Pro Gly Pro Pro Leu Ile Thr Arg Thr
    370                 375                 380

Lys Cys Ala Val His Pro Asn Val Ser Gln Gly Cys Gln Gly Gly Cys
385                 390                 395                 400

Ala Thr Cys Ser Asp Tyr Asn Gly Cys Leu Ser Cys Lys Pro Arg Leu
                405                 410                 415

Phe Phe Ala Leu Glu Arg Ile Gly Met Lys Gln Ile Gly Val Cys Leu
            420                 425                 430

Ser Ser Cys Pro Ser Gly Tyr Tyr Gly Thr Arg Tyr Pro Asp Ile Asn
        435                 440                 445

Lys Cys Thr Lys Cys Lys Ala Asp Cys Asp Thr Cys Phe Asn Lys Asn
    450                 455                 460

Phe Cys Thr Lys Cys Lys Ser Gly Phe Tyr Leu His Leu Gly Lys Cys
465                 470                 475                 480

Leu Asp Asn Cys Pro Glu Gly Leu Glu Ala Asn Asn His Thr Met Glu
                485                 490                 495

Cys Val Ser Ile Val His Cys Glu Val Ser Glu Trp Asn Pro Trp Ser
            500                 505                 510

Pro Cys Thr Lys Lys Gly Lys Thr Cys Gly Phe Lys Arg Gly Thr Glu
        515                 520                 525

Thr Arg Val Arg Glu Ile Ile Gln His Pro Ser Ala Lys Gly Asn Leu
    530                 535                 540

Cys Pro Pro Thr Asn Glu Thr Arg Lys Cys Thr Val Gln Arg Lys Lys
```

545 550 555 560

Cys Gln Lys Gly Glu Arg Gly Lys Lys Gly Arg Glu Arg Lys Arg Lys
565 570 575

Lys Pro Asn Lys Gly Glu Ser Lys Glu Ala Ile Pro Asp Ser Lys Ser
580 585 590

Leu Glu Ser Ser Lys Glu Ile Pro Glu Gln Arg Glu Asn Lys Gln Gln
595 600 605

Gln Lys Lys Arg Lys Val Gln Asp Lys Gln Lys Ser Val Ser Val Ser
610 615 620

Thr Val His
625

<210> 80
<211> 6
<212> PRT
<213> Artificial Sequence

<220>
<221> source
<223> /note="Description of Artificial Sequence: Synthetic peptide"

<400> 80

Lys Trp Tyr Gly Trp Leu
1 5

<210> 81
<211> 11
<212> PRT
<213> Artificial Sequence

<220>
<221> source
<223> /note="Description of Artificial Sequence: Synthetic peptide"

<400> 81

Gly Glu Ile Val Leu Trp Ser Asp Ile Pro Gly
1 5 10

<210> 82
<211> 19
<212> DNA
<213> Artificial Sequence

<220>
<221> source
<223> /note="Description of Artificial Sequence: Synthetic oligonucleotide"

<400> 82
tcccatttgc aagggttgt 19

<210> 83
<211> 19
<212> DNA
<213> Artificial Sequence

<220>
<221> source
<223> /note="Description of Artificial Sequence: Synthetic oligonucleotide"

<400> 83
agctgactgt gatacctgt 19

<210> 84
<211> 10
<212> DNA
<213> Artificial Sequence

<220>
<221> source
<223> /note="Description of Artificial Sequence: Synthetic oligonucleotide"

<400> 84
ggacaacaca 10

<210> 85
<211> 75
<212> DNA
<213> Artificial Sequence

<220>
<221> source
<223> /note="Description of Artificial Sequence: Synthetic oligonucleotide"

<400> 85

**atttctctct gtaaaggagg ttcgtggcgg agagatgctg atcgcgctga actgaccggt 60**

**gcggcccggg ggtga 75**

<210> 86
<211> 75
<212> DNA
<213> Artificial Sequence

<220>
<221> source
<223> /note="Description of Artificial Sequence: Synthetic oligonucleotide"

<400> 86

**ttccgcttct tgaatttctc tctgtaaagg aggttcgtgg cggagagatg ctgatcgcgc 60**

**tgaactgacc ggtgc 75**

<210> 87
<211> 75
<212> DNA
<213> Artificial Sequence

<220>
<221> source
<223> /note="Description of Artificial Sequence: Synthetic oligonucleotide"

<400> 87

ccgcttcttg aatttctctc tgtaaaggag gttcgtggcg gagagatgct gatcgcgctg 60

aactgaccgg tgcgg 75

<210> 88
<211> 75
<212> DNA
<213> Artificial Sequence

<220>
<221> source
<223> /note="Description of Artificial Sequence: Synthetic oligonucleotide"

<400> 88

tcggcatcta gtctttccgc ttcttgaatt tctctctgta aaggaggttc gtggcggaga 60

gatgctgatc gcgct 75

<210> 89
<211> 75
<212> DNA
<213> Artificial Sequence

<220>
<221> source
<223> /note="Description of Artificial Sequence: Synthetic oligonucleotide"

<400> 89

ctttccgctt cttgaatttc tctctgtaaa ggaggttcgt ggcggagaga tgctgatcgc 60

gctgaactga ccggt 75

<210> 90
<211> 75
<212> DNA
<213> Artificial Sequence

<220>
<221> source
<223> /note="Description of Artificial Sequence: Synthetic oligonucleotide"

<400> 90

aatttctctc tgtaaaggag gttcgtggcg gagagatgct gatcgcgctg aactgaccgg 60

tgcggcccgg ggggg 75

<210> 91
<211> 75
<212> DNA
<213> Artificial Sequence

<220>
<221> source
<223> /note="Description of Artificial Sequence: Synthetic oligonucleotide"

<400> 91

cgcacttttt ggatcggcat ctagtctttc cgcttcttga atttctctct gtaaaggagg 60

ttcgtggcgg agaga 75

<210> 92
<211> 75
<212> DNA
<213> Artificial Sequence

<220>
<221> source
<223> /note="Description of Artificial Sequence: Synthetic oligonucleotide"

<400> 92

ctttccgctt cttgaatttc tctctgtaaa ggaggttcgt ggcggagaga tgctgatcgc 60

gctgaactgc ccggt 75

<210> 93
<211> 75
<212> DNA
<213> Artificial Sequence

<220>
<221> source
<223> /note="Description of Artificial Sequence: Synthetic oligonucleotide"

<400> 93

tctcctggga tcggcccaag gccagttctc cgcagtgcat cctaacgtta gtcaaggctg 60

ccaaggaggc tgtgc 75

<210> 94
<211> 75
<212> DNA
<213> Artificial Sequence

<220>
<221> source
<223> /note="Description of Artificial Sequence: Synthetic oligonucleotide"

<400> 94

ctcctgggat cggcccaagg ccagttctcc gcagtgcatc ctaacgttag tcaaggctgc 60

caaggaggct gtgca 75

<210> 95
<211> 75
<212> DNA
<213> Artificial Sequence

<220>
<221> source
<223> /note="Description of Artificial Sequence: Synthetic oligonucleotide"

<400> 95

tcctgggatc ggcccaaggc cagttctccg cagtgcatcc taacgttagt caaggctgcc 60

aaggaggctg tgcaa 75

<210> 96
<211> 75
<212> DNA
<213> Artificial Sequence

<220>
<221> source
<223> /note="Description of Artificial Sequence: Synthetic oligonucleotide"

<400> 96

cctgggatcg gcccaaggcc agttctccgc agtgcatcct aacgttagtc aaggctgcca 60

aggaggctgt gcaac 75

<210> 97
<211> 75
<212> DNA
<213> Artificial Sequence

<220>
<221> source
<223> /note="Description of Artificial Sequence: Synthetic oligonucleotide"

<400> 97

ctgggatcgg cccaaggaca gttctccgca gtgcatccta acgttagtca aggctgccaa 60

ggaggctgtg caaca 75

<210> 98
<211> 75
<212> DNA
<213> Artificial Sequence

<220>
<221> source
<223> /note="Description of Artificial Sequence: Synthetic oligonucleotide"

<400> 98

atcggcccaa ggccagttct ccgcagtgca tcctaacgtt agtcaaggct gccaaggagg 60

ctgtgcaaca tgctc 75

<210> 99
<211> 75
<212> DNA
<213> Artificial Sequence

<220>
<221> source
<223> /note="Description of Artificial Sequence: Synthetic oligonucleotide"

<400> 99

tcggcccaag gccagttctc cgcagtgcat cctaacgtta gtcaaggctg ccaaggaggc 60

tgtgcaacat gctca 75

<210> 100
<211> 75
<212> DNA
<213> Artificial Sequence

<220>
<221> source
<223> /note="Description of Artificial Sequence: Synthetic oligonucleotide"

<400> 100

ccaggacctc cactaatcac cagaacaaaa tgtgcagtgc atcctaacgt tagtcaaggc 60

tgccaaggag gctgt 75

<210> 101
<211> 75
<212> DNA
<213> Artificial Sequence

<220>
<221> source
<223> /note="Description of Artificial Sequence: Synthetic oligonucleotide"

<400> 101

caggacctcc actaatcacc agaacaaaat gtgcagtgca tcctaacgtt agtcaaggct 60

gccaaggagg ctgtg 75

<210> 102
<211> 75
<212> DNA
<213> Artificial Sequence

<220>
<221> source
<223> /note="Description of Artificial Sequence: Synthetic oligonucleotide"

<400> 102

aggacctcca ctaatcacca gaacaaaatg tgcagtgcat cctaacgtta gtcaaggctg 60

ccaaggaggc tgtgc 75

<210> 103
<211> 75
<212> DNA
<213> Artificial Sequence

<220>
<221> source
<223> /note="Description of Artificial Sequence: Synthetic oligonucleotide"

<400> 103

gccctccact aatcaccaga acaaaatgtg cagtgcatcc taacgttagt caaggctgcc 60

aaggaggctg tgcaa 75

<210> 104
<211> 75
<212> DNA
<213> Artificial Sequence

<220>
<221> source
<223> /note="Description of Artificial Sequence: Synthetic oligonucleotide"

<400> 104

ctaatcacca gaacaaaatg tgcagtgcat cctaacgtta gtcaaggctg ccaaggaggc 60

tgtgcaacat gccca 75

<210> 105
<211> 100
<212> DNA
<213> Artificial Sequence

<220>
<221> source
<223> /note="Description of Artificial Sequence: Synthetic polynucleotide"

<400> 105

ttggcataac ttgatatttc ttgttctcgc tttgtcacct aagctggagt gcagtggcac 60

aatcttagct cattacagcc ctgactttct ggttaaggtt 100

<210> 106
<211> 100
<212> DNA
<213> Artificial Sequence

<220>
<221> source
<223> /note="Description of Artificial Sequence: Synthetic polynucleotide"

<400> 106

tgttctcgct ttgtcaccta agctggagtg cagtggcaca atcttagctc attacagccc 60

tgactttctg gttaaggttt aaggatgtcg ccaggcgcag 100

<210> 107
<211> 100
<212> DNA
<213> Artificial Sequence

<220>
<221> source
<223> /note="Description of Artificial Sequence: Synthetic polynucleotide"

<400> 107

tcttgttctc gctttgtcac ctaagctgga gtgcagtggc acaatcttag ctcattacag 60

ccctgacttt ctggttaagg tttaaggatg tcgccaggcg 100

<210> 108
<211> 69
<212> DNA
<213> Artificial Sequence

<220>
<221> source
<223> /note="Description of Artificial Sequence: Synthetic oligonucleotide"

<400> 108

ttggcataac ttgatatttc ttgatcatct tgcaagatga aggtttaagg atgtcgccag 60

gcgcagtgg 69

<210> 109
<211> 56
<212> DNA
<213> Artificial Sequence

<220>
<221> source
<223> /note="Description of Artificial Sequence: Synthetic oligonucleotide"

<400> 109
caaacctgca tgttctgcac atgtatccca gaactaaaga ggaccactta acagtt 56

<210> 110
<211> 100
<212> DNA
<213> Artificial Sequence

<220>
<221> source
<223> /note="Description of Artificial Sequence: Synthetic polynucleotide"

<400> 110

tgcacatgta tcccagaact aaagaggacc acttaacagt tttgattaag taggtctggt 60

gtgtggccaa aaacctgcat ttctaacaag ctctcccagg 100

<210> 111
<211> 105
<212> DNA
<213> Artificial Sequence

<220>
<221> source
<223> /note="Description of Artificial Sequence: Synthetic polynucleotide"

<400> 111

aacaaacctg catgttctgc acatgtatcc cagaactaaa agtataaccc aaacttaaga 60

ggaccactta acagttttga ttaagtaggt ctggtgtgtg gccaa 105

<210> 112
<211> 11
<212> DNA
<213> Artificial Sequence

<220>
<221> source
<223> /note="Description of Artificial Sequence: Synthetic oligonucleotide"

<400> 112
caaagggaag a 11

<210> 113
<211> 100
<212> DNA
<213> Artificial Sequence

<220>
<221> source
<223> /note="Description of Artificial Sequence: Synthetic polynucleotide"

<400> 113

tgagccagta gctcctggag aaatcttgga agtctgggct ggcaaaggga agatttgtag 60

ctgagagtta agagaatggt tagatgtttt aaagaatggg 100

<210> 114
<211> 85
<212> DNA
<213> Artificial Sequence

<220>
<221> source
<223> /note="Description of Artificial Sequence: Synthetic oligonucleotide"

<400> 114

tgagccagta gctcctgtag aaatcttgga agtctgggct ggcaaaggga agatttgtag 60

ctgagagtta agagaatggt tagat 85

<210> 115
<211> 100
<212> DNA
<213> Artificial Sequence

<220>
<221> source
<223> /note="Description of Artificial Sequence: Synthetic polynucleotide"

<400> 115

gcctcccaag gtgctgagat tacaggcgtg agccatcgcg caattttagt gagaaaaatc 60

aacggcattt aatgttaact ccacgattac tgtttctttc 100

<210> 116
<211> 107
<212> DNA
<213> Artificial Sequence

<220>

<221> source
<223> /note="Description of Artificial Sequence: Synthetic polynucleotide"

<400> 116

ctaggcctcc caaggtgctg agattacagg cgtgagccat cgcgcatttt agtgagaaaa 60

atcaacggca tttaatgtta actccacgat tactgtttct ttcctca 107

<210> 117
<211> 100
<212> DNA
<213> Artificial Sequence

<220>
<221> source
<223> /note="Description of Artificial Sequence: Synthetic polynucleotide"

<400> 117

ttccgtttgt attgttttct tttggtagca tatatgccac acatatgatg tagtatatgg 60

gattatttta ccctatttga tggagcagat gaaagcaaaa 100

<210> 118
<211> 118
<212> DNA
<213> Artificial Sequence

<220>
<221> source
<223> /note="Description of Artificial Sequence: Synthetic polynucleotide"

<400> 118

ttttggtagc atatatgcca cacatatgat gtagtatatg ggattatttt accctattga 60

tggagcagat gaaagcaaaa tgcttctccc cattttaaga cattaaactt ctgtaaga 118

<210> 119
<211> 100
<212> DNA
<213> Artificial Sequence

<220>
<221> source
<223> /note="Description of Artificial Sequence: Synthetic polynucleotide"

<400> 119

tttttaaaat agtacctcag tagcaaaggg ccaactacac tgggacagca gttgtgtctc 60

cattaaatta ggtgtgcttt gattctccaa aataaagaat 100

<210> 120
<211> 114
<212> DNA
<213> Artificial Sequence

<220>
<221> source
<223> /note="Description of Artificial Sequence: Synthetic polynucleotide"

<400> 120

```
aaatcaatgt ttttttaaaa tagtacctca gtagcaaagg gccaactaca ctgggacagc        60
agttgtgtct ccattaaatt aggtgtgctt tgattctcca aaataaagaa tttt              114
```

## Description

Provided are therapies related to the treatment of pathological conditions, such as cancer.

Colorectal cancer (CRC) with over 100,000 new cases reported annually is the fourth most prevalent cancer and accounts for over 50,000 deaths per year in the United States (Siegel, R. et al., CA: A Cancer Journal for Clinicians 61:212-236 (2011)). Approximately 15% of CRCs exhibit microsatellite instability (MSI) arising from defects in DNA mismatch repair (MMR) system (Fearon, E. R., Annu. Rev. Pathol. 6:479-507 (2011)). The other ~85% of microsatellite stable (MSS) CRCs are the result of chromosomal instability (CIN) (Fearon, E. R., Annu. Rev. Pathol. 6:479-507 (2011)). Genomic studies have identified acquisition of mutations in genes like *APC*, *KRAS,* and *TP53* during CRC progression (Fearon, E. R., Annu. Rev. Pathol. 6:479-507 (2011)). Sequencing colon cancer protein-coding exons and whole genomes in a small number of samples have identified several additional mutations and chromosomal structural variants that likely contribute to oncogenesis (Wood, L. D. et al., Science 318:1108-1113 (2007); Timmermann, B. et al., PloS One 5:e15661 (2010)). However, recent insertional mutagenesis screens in mouse models of colon cancer suggested involvement of additional genes and pathways in CRC development (Starr, T. K. et al., Science 323:1747-1750 (2009); March, H. N. et al., Nat. Genet. 43:1202-1209 (2011)).

There remains a need to better understand the pathogenesis of cancers, in particular, human colon cancers and also to identify new therapeutic targets.

Provided herein are R-spondin-translocation antagonists and methods of using the same.

The invention relates to the embodiments as defined in the claims. One aspect of the invention relates to an antibody which binds to R-spondin 3 (RSPO3) for use in treating a cancer cell, wherein the cancer cell comprises an RSPO3 translocation. Also comprised by the present invention is an antibody which binds to RSPO3 for use in treating cancer, wherein the cancer comprising an RSPO3 translocation. The invention also relates to a method of identifying an individual with cancer who is more or less likely to exhibit benefit from treatment with an anti-cancer therapy comprising an antibody which binds to RSPO3, the method comprising: determining presence or absence of an RSPO3 translocation in a sample obtained from the individual, wherein presence of the RSPO3 translocation in the sample indicates that the individual is more likely to exhibit benefit from treatment with the anti-cancer therapy comprising the antibody which binds to RSPO3 or absence of the RSPO3 translocation indicates that the individual is less likely to exhibit benefit from treatment with the anti-cancer therapy comprising the antibody which binds to RSPO3.

Also provided herein are wnt pathway antagonists.

Furher provided herein are methods of inhibiting cell proliferation of a cancer cell comprising contacting the cancer cell with an effective amount of an R-spondin-translocation antagonist. Further provided herein are methods of treating cancer in an individual comprising administering to the individual an effective amount of an R-spondin-translocation antagonist. In some embodiments of any of the methods, the cancer or cancer cell comprises an R-spondin translocation.

Provided herein are methods of treating cancer in an individual comprising administering to the individual an effective amount of a wnt pathway antagonist, wherein treatment is based upon the individual having cancer comprising an R-spondin translocation. Provided herein are methods of treating a cancer cell, wherein the cancer cell comprises an R-spondin translocation, and wherein the method comprises providing an effective amount of a wnt pathway antagonist. Also provided herein are methods of treating cancer in an individual provided that the individual has been found to have cancer comprising an R-spondin translocation, the treatment comprising administering to the individual an effective amount of a wnt pathway antagonist.

Further, provided herein are methods for treating cancer in an individual, the method comprising: determining that a sample obtained from the individual comprises an R-spondin translocation, and administering an effective amount of an anti-cancer therapy comprising a wnt pathway antagonist to the individual, whereby the cancer is treated.

Provided herein are methods of treating cancer, comprising: (a) selecting an individual having cancer, wherein the cancer comprising an R-spondin translocation; and (b) administering to the individual thus selected an effective amount of a wnt pathway antagonist, whereby the cancer is treated.

Provided herein are also methods of identifying an individual with cancer who is more likely or less likely to exhibit benefit from treatment with an anti-cancer therapy comprising a wnt pathway antagonist, the method comprising: determining presence or absence of an R-spondin translocation in a sample obtained from the individual, wherein presence of the R-spondin translocation in the sample indicates that the individual is more likely to exhibit benefit from treatment with the anti-cancer therapy comprising the wnt pathway antagonist or absence of the R-spondin translocation indicates that the individual is less likely to exhibit benefit from treatment with the anti-cancer therapy comprising the wnt pathway antagonist. In some embodiments, the method further comprises administering an effective amount of the anti-cancer therapy comprising a wnt pathway antagonist.

Provided herein are methods for predicting whether an individual with cancer is more or less likely to respond effectively to treatment with an anti-cancer therapy comprising a wnt pathway antagonist, the method comprising determining an R-spondin translocation, whereby presence of the R-spondin translocation indicates that the individual is more likely to respond effectively to treatment with the wnt pathway antagonist and absence of the R-spondin translocation indicates that the individual is less likely to respond effectively to treatment with the wnt pathway antagonist. In some embodiments, the method further comprises administering an effective amount of the anti-cancer therapy comprising a wnt pathway antagonist.

Further provided herein are methods of predicting the response or lack of response of an individual with cancer to an anti-cancer therapy comprising a wnt pathway antagonist comprising detecting in a sample obtained from the individual presence or absence of an R-spondin translocation, wherein presence of the R-spondin translocation is predictive of response of the individual to the anti-cancer therapy comprising the wnt pathway antagonist and absence of the R-spondin translocation is predictive of lack of response of the individual to the anti-cancer therapy comprising the wnt pathway antagonist. In some embodiments, the method further comprises administering an effective amount of the anti-cancer therapy comprising a wnt pathway antagonist.

In some embodiments of any of the methods, the R-spondin translocation is a RSPO1 translocation, RSPO2 translocation, RSPO3 translocation and/or RSPO4 translocation. In some embodiments, the R-spondin translocation is a RSPO2 translocation. In some embodiments, the RSPO2 translocation comprises *EIF3E* and *RSPO2.* In some embodiments, the RSPO2 translocation comprises *EIF3E* exon 1 and *RSPO2* exon 2. In some embodiments, the RSPO2 translocation comprises *EIF3E* exon 1 and *RSPO2* exon 3. In some embodiments, the RSPO2 translocation comprises SEQ ID NO:71 In some embodiments, the R-spondin translocation is a RSPO3 translocation. In some embodiments, the RSPO3 translocation comprises *PTPRK* and *RSPO3.* In some embodiments, the RSPO3 translocation comprises *PTPRK* exon 1 and *RSPO3* exon 2. In some embodiments, the RSPO3 translocation comprises *PTPRK* exon 7 and *RSPO3* exon 2. In some embodiments, the RSPO3 translocation comprises SEQ ID NO:72 and/or SEQ ID NO:73. In some embodiments of any of the methods, the R-spondin translocation is detected at the chromosomal level (e.g., FISH), DNA level, RNA level (*e.g.*, RSPO1-translocation fusion transcript), and/or protein level (*e.g.*, RSPO1-translocation fusion polypeptide).

In some embodiments of any of the methods, the cancer is colorectal cancer. In some embodiments, the cancer is a colon cancer or rectal cancer.

1) In some embodiments of any of the methods, the wnt pathway antagonist is an antibody, binding polypeptide, small molecule, or polynucleotide. In some embodiments, the wnt pathway antagonist is an R-spondin antagonist. In some embodiments, the R-spondin antagonist is a RSPO1 antagonist, RSPO2 antagonist, RSPO3 antagonist, and/or RSPO4 antagonist. In some embodiments, the wnt pathway antagonist is an isolated monoclonal antibody which binds R-spondin. In some embodiments, the R-spondin is RSPO2 and/or RSPO3. In some embodiments, the R-spondin antagonist is an R-spondin-translocation antagonist. In some embodiments, the R-spondin-translocation antagonist binds a RSPO1-translocation fusion polypeptide and/or polynucleotide, RSPO2-translocation fusion polypeptide and/or polynucleotide, RSPO3-translocation fusion polypeptide and/or polynucleotide and/or RSPO4-translocation fusion polypeptide and/or polynucleotide. In some embodiments, the R-spondin-translocation antagonist binds a RSPO2-translocation fusion polypeptide and/or polynucleotide. In some embodiments, the RSPO2-translocation fusion polypeptide and/or polynucleotide comprises *EIF3E* and *RSPO2.* In some embodiments, the RSPO2-translocation fusion polypeptide and/or polynucleotide comprises *EIF3E* exon 1 and *RSPO2* exon 2. In some embodiments, the RSPO2-translocation fusion polypeptide and/or polynucleotide comprises *EIF3E* exon 1 and *RSPO2* exon 3. In some embodiments, the RSPO2-translocation fusion polypeptide and/or polynucleotide comprises SEQ ID NO:71. In some embodiments, the R-spondin-translocation fusion polypeptide and/or polynucleotide is a RSPO3-translocation fusion polypeptide and/or polynucleotide. In some embodiments, the RSPO3-translocation fusion polypeptide and/or polynucleotide comprises *PTPRK* and *RSPO3.* In some embodiments, the RSPO3-translocation fusion polypeptide and/or polynucleotide comprises *PTPRK* exon 1 and *RSPO3* exon 2. In some embodiments, the RSPO3-translocation fusion polypeptide and/or polynucleotide comprises *PTPRK* exon 7 and *RSPO3* exon 2. In some embodiments, the RSPO3-translocation fusion polypeptide and/or polynucleotide comprises SEQ ID NO:72 and/or SEQ ID NO:73. In some embodiments, the method further comprises an additional therapeutic agent.

Provided herein are isolated R-spondin-translocation antagonists, wherein the R-spondin-translocation antagonist is an antibody, binding polypeptide, small molecule, or polynucleotide. In some embodiments, the R-spondin-translocation antagonist binds a RSPO1-translocation fusion polypeptide and/or polynucleotide, RSPO2-translocation fusion polypeptide and/or polynucleotide, RSPO3-translocation fusion polypeptide and/or polynucleotide and/or RSPO4-translocation fusion polypeptide and/or polynucleotide. In some embodiments, the R-spondin-translocation antagonist binds a RSPO2-translocation fusion polypeptide and/or polynucleotide. In some embodiments, the RSPO2-translocation fusion polypeptide and/or polynucleotide comprises *EIF3E* and *RSPO2.* In some embodiments, the RSPO2-translocation fusion polypeptide and/or polynucleotide comprises *EIF3E* exon 1 and *RSPO2* exon 2. In some embodiments, the RSPO2-translocation fusion polypeptide and/or polynucleotide comprises *EIF3E* exon 1 and *RSPO2* exon 3. In some embodiments, the RSPO2-translocation fusion polypeptide and/or polynucleotide comprises SEQ ID NO:71. In some embodiments, the R-spondin-translocation fusion polypeptide and/or polynucleotide is a RSPO3-translocation fusion polypeptide and/or polynucleotide. In some embodiments, the RSPO3-translocation fusion polypeptide and/or polynucleotide comprises *PTPRK* and *RSPO3.* In some embodiments, the RSPO3-translocation fusion polypeptide and/or polynucleotide comprises *PTPRK* exon 1 and *RSPO3* exon 2. In some embodiments, the RSPO3-translocation fusion polypeptide and/or polynucleotide comprises *PTPRK* exon 7 and *RSPO3* exon 2. In some embodiments, the RSPO3-translocation fusion polypeptide and/or polynucleotide comprises SEQ ID NO:72 and/or SEQ ID NO:73.

## BRIEF DESCRIPTION OF THE FIGURES

Figure 1 | (**A**) Activation of an alternate novel 5' exon of *MRPL33* in a tumor specific manner alters the N-terminal end of MRPL33 and makes the protein longer. (**B**) The boxplot shows the read counts for the upstream exon normalized by total number of reads aligning to *MRPL33* for each sample. (C) Also shown is evidence of an alternate upstream *MRPL33* promoter region showing H3K27Ac marking by USCS genome browser as well as an EST mapping to the upstream exon. MRLP33 Amino Acid Sequence ***MFLSAVFF AKSKSN***ETKSPLRGKEKNTLPLNGGLKMTLIYKEKTEGG DTDSEIL (SEQ ID NO:9); MRLP33 alternative promoter amino acid sequence ***MMAHLDFFLTYKWRAPKSKSLDQLSPNFLLRGRS*** ETKSPLRGKEKNTLPLNGGLKMTLIYKEKTEGGDTDSEIL (SEQ ID NO: 10).

Figure 2 | Recurrent R-spondin translocations. (**A**) List of the type and frequency of R-spondin gene fusions in colon cancer. (**B**) Cartoon depicting the location, orientation and exon-intron architecture of *EIF3E-RSPO2* fusion on the genome. The read evidence for *EIF3E(e1)-RSPO2(e2)* fusion identified using RNA-seq data are shown. (**C**) Independent RT-PCR derived products confirming the *EIF3E-RSPO2* somatic fusion resolved on an agarose gel. RT-PCR products were Sanger sequenced to confirm the fusion junction and a relevant representative chromatogram is presented. (**D**) Schematic of the resulting EIF3E-RSPO2 fusion protein. (**E**) Tumors harboring R-spondin fusions show elevated expression of the corresponding *RSPO* gene shows on a heatmap. Figure 2 discloses SEQ ID NOS 85-92 and 71, respectively, in order of appearance.

Figure 3 | Recurrence of *PTPRK-RSPO3* gene fusion. (**A**) Cartoon depicting the location, orientation and exon-intron architecture of *PTPRK-RSPO3* gene fusion on the genome. The read evidence for *PTPRK(e1)-RSPO3(e2)* fusion identified using RNA-seq data are shown. (**B**) Independent RT-PCR derived products confirming the *PTPRK-RSPO3* somatic fusion resolved on an agarose gel. RT-PCR products were Sanger sequenced to confirm the fusion junction and a relevant representative chromatogram is presented. (**C**) Schematic of PTPRK, RSPO3 and the resulting PTPRK-RSPO3 fusion proteins. Figure 3 discloses SEQ ID NOS 93-99 and 72, respectively, in order of appearance.

Figure 4 | (**A**) *PTPRK(e7)-RSPO3*(e2) fusion. (**B**) Gel showing the validation of this fusion by RT-PCR. (**C**) Schematic diagram of the native and fusion proteins. Figure 4 discloses SEQ ID NOS 100-104 and 73, respectively, in order of appearance.

Figure 5 | RSPO fusion products activate Wnt signaling. (**A**) Secreted RSPO fusion proteins detected by Western blot in media from 293T cells transfected with expression constructs encoding the fusion proteins. The expected product is RSPO 1-387. (**B** and **C**) RSPO fusion proteins activate and potentiate Wnt signaling as measured using a luciferase reporter assay. Data shown are from condition media derived from cells transfected with the fusion constructs or directly transfected into the cell along with the reporter construct. Representative data from at least three experiments are shown. (**D**) Cartoon representing R-spondin mediated Wnt signaling pathway activation. (**E**) Plot depicting *RSPO* fusions and somatic mutations across a select set of Wnt signaling pathway genes.

Figure 6 | (**A**) *KRAS* mutations overlap with *RSPO* gene fusions. (**B**) RAS/RTK pathway alterations in colon cancer.

Figure 7 | Whole genome EIF3E-RSPO2 coordinates schematic and sequences. Figure 7 discloses SEQ ID NOS 105-108, respectively, in order of appearance.

Figure 8 | Whole genome EIF3E-RSPO2 coordinates schematic and sequences. Figure 8 discloses SEQ ID NOS 109-111, respectively, in order of appearance.

Figure 9 | Whole genome PTPRK-RSPO3 coordinates schematic and sequences. Figure 9 discloses SEQ ID NOS 112-116, respectively, in order of appearance.

Figure 10 | Whole genome PTPRK-RSPO3 coordinates schematic and sequences. Figure 10 discloses SEQ ID NOS 112 and 117-120, respectively, in order of appearance.

### *I. Definitions*

The terms "R-spondin" and "RSPO" refer herein to a native R-spondin from any vertebrate source, including mammals such as primates (*e.g.,* humans) and rodents (*e.g.,* mice and rats), unless otherwise indicated. The term encompasses "full-length," unprocessed R-spondin as well as any form of R-spondin that results from processing in the cell. The term also encompasses naturally occurring variants of R-spondin, *e.g.*, splice variants or allelic variants. R-spondin is a family of four proteins, R-spondin 1 (RSPO1), R-spondin 2 (RSPO2), R-spondin 3 (RSPO3), and R-spondin 4 (RSPO4). In some embodiments, the R-spondin is RSPO1. The sequence of an exemplary human RSPO1 nucleic acid sequence is SEQ ID NO:1 or an exemplary human RSPO1 is amino acid sequence of SEQ ID NO:2. In some embodiments, the R-spondin is RSPO2. The sequence of an exemplary human RSPO2 nucleic acid sequence is SEQ ID NO:3 or an exemplary human RSPO2 is amino acid sequence of SEQ ID NO:4. In some embodiments, the R-spondin is RSPO3. The sequence of an exemplary human RSPO3 nucleic acid sequence is SEQ ID NO:5 or an exemplary human RSPO3 is amino acid sequence of SEQ ID NO:6. In some embodiments, the R-spondin is RSPO4. The sequence of an exemplary human RSPO4 nucleic acid sequence is SEQ ID NO:7 or an exemplary human RSPO4 is amino acid sequence of SEQ ID NO:8.

"R-Spondin variant," "RSPO variant," or variations thereof, means an R-spondin polypeptide or polynucleotide, generally being or encoding an active R-Spondin polypeptide, as defined herein having at least about 80% amino acid sequence identity with any of the R-Spondin as disclosed herein. Such R-Spondin variants include, for instance, R-Spondin wherein one or more nucleic acid or amino acid residues are added or deleted. Ordinarily, an R-spondin variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to R-Spondin as disclosed herein. Ordinarily, R-Spondin variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, R-Spondin variant will have or encode a sequence having no more than one conservative amino acid substitution as compared to R-Spondin, alternatively no more than 2, 3, 4, 5, 6, 7, 8, 9, or 10 conservative amino acid substitution as compared to R-Spondin.

The terms "R-spondin translocation" and "RSPO translocation" refer herein to an R-spondin wherein a portion of a broken chromosome including, for example, R-spondin, variant, or fragment thereof or a second gene, variant, or fragment thereof, reattaches in a different chromosome location, for example, a chromosome location different from R-spondin native location or a chromosome location in and/or around the R-spondin native location which is different from the second gene's native location. The R-spondin translocation may be a RSPO1 translocation, RSPO2 translocation, RSPO3 translocation, and/or RSPO4 translocation.

The terms "R-spondin-translocation fusion polynucleotide" and "RSPO-translocation fusion polynucleotide" refer herein to the nucleic acid sequence of an R-spondin translocation gene product or fusion polynucleotide. The R-spondin-translocation fusion polynucleotide may be a RSPO1-translocation fusion polynucleotide, RSPO2-translocation fusion polynucleotide, RSPO3-translocation fusion polynucleotide, and/or RSPO4-translocation fusion polynucleotide. The terms "R-spondin-translocation fusion polypeptide" and "RSPO-translocation fusion polypeptide" refer herein to the amino acid sequence of an R-spondin translocation gene product or fusion polynucleotide. The R-spondin-translocation fusion polypeptide may be a RSPO1-translocation fusion polypeptide, RSPO2-translocation fusion polypeptide, RSPO3-translocation fusion polypeptide, and/or RSPO4-translocation fusion polypeptide.

The term "R-spondin-translocation antagonist" as defined herein is any molecule that partially or fully blocks, inhibits, or neutralizes a biological activity mediated by an R-spondin-translocation fusion polypeptide. In some embodiments such antagonist binds to R-spondin-translocation fusion polypeptide. According to one embodiment, the antagonist is a polypeptide. According to another embodiment, the antagonist is an anti-R-spondin-translocation antibody. According to another embodiment, the antagonist is a small molecule antagonist. According to another embodiment, the antagonist is a polynucleotide antagonist. The R-spondin translocation may be a RSPO1-translocation antagonist, RSPO2-translocation antagonist, RSPO3-translocation antagonist, and/or RSPO4-translocation antagonist.

The term "wnt pathway antagonist" as defined herein is any molecule that partially or fully blocks, inhibits, or neutralizes a biological activity mediated by the wnt pathway (*e.g.*, wnt pathway polypeptide). In some embodiments such antagonist binds to a wnt pathway polypeptide. According to one embodiment, the antagonist is a polypeptide. According to another embodiment, the antagonist is an antibody antagonist. According to another embodiment, the antagonist is a small molecule antagonist. According to another embodiment, the antagonist is a polynucleotide antagonist.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.*, methyl phosphonates, phosphotriesters, phosphoamidates, carbamates, etc.) and with charged linkages (*e.g.*, phosphorothioates, phosphorodithioates, etc.), those containing pendant moieties, such as, for example, proteins (*e.g.*, nucleases, toxins, antibodies, signal peptides, ply-L-lysine, etc.), those with intercalators (*e.g.*, acridine, psoralen, etc.), those containing chelators (*e.g.*, metals, radioactive metals, boron, oxidative metals, etc.), those containing alkylators, those with modified linkages (*e.g.,* alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), $(O)NR_2$ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to generally single-stranded, synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and following polymerization of a complementary nucleic acid, generally by providing a free 3'-OH group.

The term "small molecule" refers to any molecule with a molecular weight of about 2000 Daltons or less, preferably of about 500 Daltons or less.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "isolated" antibody is one which has been separated from a component of its natural environment. In some embodiments, an antibody is purified to greater than 95% or 99% purity as determined by, for example, electrophoretic (*e.g.*, SDS-PAGE, isoelectric focusing (IEF), capillary electrophoresis) or chromatographic (*e.g.*, ion exchange or reverse phase HPLC). For review of methods for assessment of antibody purity, *see, e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "antibody" herein is used in the broadest sense and encompasses various antibody structures, including but not limited to monoclonal antibodies, polyclonal antibodies, multispecific antibodies (*e.g.*, bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, $F(ab')_2$; diabodies; linear antibodies; single-chain antibody molecules (*e.g.*, scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "full length antibody," "intact antibody," and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, *i.e.*, the individual antibodies comprising the population are identical and/or bind the same epitope, except for possible variant antibodies, *e.g.*, containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 Daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "chimeric" antibody refers to an antibody in which a portion of the heavy and/or light chain is derived from a particular source or species, while the remainder of the heavy and/or light chain is derived from a different source or species.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (*e.g.*, CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g.*, a non-human antibody, refers to an antibody that has undergone humanization.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g.,* $IgG_1$, $IgG_2$, $IgG_3$, $IgG_4$, $IgA_1$, and $IgA_2$. The heavy chain constant domains that correspond to the different classes of immunoglobulins are called $\alpha$, $\delta$, $\varepsilon$, $\gamma$, and $\mu$, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g.,* B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat *et al*., *supra.* In one embodiment, for the VH, the subgroup is subgroup III as in Kabat *et al*., *supra.*

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See*, *e.g.*, Kindt et al., Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (*See* Almagro and Fransson, Front. Biosci. 13:1619-1633 (2008).) Unless otherwise indicated, HVR residues and other residues in the variable domain (*e.g*., FR residues) are numbered herein according to Kabat *et al*., *supra.*

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g*., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-R-spondin-translocation antibody" and "an antibody that binds to R-spondin-translocation fusion polypeptide" refer to an antibody that is capable of binding R-spondin-translocation fusion polypeptide with sufficient affinity such that the antibody is useful as a diagnostic and/or therapeutic agent in targeting R-spondin translocation. In one embodiment, the extent of binding of an anti-R-spondin translocation antibody to an unrelated, non-R-spondin-translocation fusion polypeptide, and/or nontranslocated-R-spondin polypeptide is less than about 10% of the binding of the antibody to R-spondin-translocation fusion polypeptides measured, *e.g*., by a radioimmunoassay (RIA). In certain embodiments, an antibody that binds to R-spondin-translocation fusion polypeptide has a dissociation constant (Kd) of $\leq$ 1 $\mu$M, $\leq$ 100 nM, $\leq$ 10 nM, $\leq$ 1 nM, $\leq$ 0.1 nM, $\leq$ 0.01 nM, or $\leq$ 0.001 nM (*e.g.,* $10^{-8}$ M or less, *e.g.,* from $10^{-8}$ M to $10^{-13}$ M, *e.g.,* from $10^{-9}$ M to $10^{-13}$ M). In certain embodiments, an anti- R-spondin translocation antibody binds to an epitope of R-spondin translocation that is unique among R-spondin translocations.

A "blocking" antibody or an "antagonist" antibody is one which inhibits or reduces biological activity of the antigen it binds. Preferred blocking antibodies or antagonist antibodies substantially or completely inhibit the biological activity of the antigen.

A "naked antibody" refers to an antibody that is not conjugated to a heterologous moiety (*e.g.,* a cytotoxic moiety) or radiolabel. The naked antibody may be present in a pharmaceutical formulation.

An "immunoconjugate" is an antibody conjugated to one or more heterologous molecule(s), including but not limited to a cytotoxic agent.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "detection" includes any means of detecting, including direct and indirect detection.

The term "biomarker" as used herein refers to an indicator, *e.g.*, predictive, diagnostic, and/or prognostic, which can be detected in a sample. The biomarker may serve as an indicator of a particular subtype of a disease or disorder (*e.g.*, cancer) characterized by certain, molecular, pathological, histological, and/or clinical features. In some embodiments, the biomarker is a gene. In some embodiments, the biomarker is a variation (*e.g.*, mutation and/or polymorphism) of a gene. In some embodiments, the biomarkers is a translocation. Biomarkers include, but are not limited to, polynucleotides (*e.g.*, DNA, and/or RNA), polypeptides, polypeptide and polynucleotide modifications (*e.g.*, posttranslational modifications), carbohydrates, and/or glycolipid-based molecular markers.

The "presence," "amount," or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information (*e.g.*, gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (*e.g.*, posttranslational modification of a polypeptide). Fragments of the transcribed polynucleotide, the translated polypeptide, or polynucleotide and/or polypeptide modifications (*e.g.*, post-translational modification of a polypeptide) shall also be regarded as expressed whether they originate from a transcript generated by alternative splicing or a degraded transcript, or from a post-translational processing of the polypeptide, *e.g.*, by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to an increased expression or increased levels of a biomarker in an individual relative to a control, such as an individual or individuals who are not suffering from the disease or disorder (*e.g.*, cancer) or an internal control (*e.g.*, housekeeping biomarker).

"Reduced expression," "reduced expression levels," or "reduced levels" refers to a decrease expression or decreased levels of a biomarker in an individual relative to a control, such as an individual or individuals who are not suffering from the disease or disorder (*e.g.*, cancer) or an internal control (*e.g.*, housekeeping biomarker).

The term "housekeeping biomarker" refers to a biomarker or group of biomarkers (*e.g.*, polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "multiplex-PCR" refers to a single PCR reaction carried out on nucleic acid obtained from a single source (*e.g.,* an individual) using more than one primer set for the purpose of amplifying two or more DNA sequences in a single reaction.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel et al., Current Protocols in Molecular Biology, Wiley Interscience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" can be identified as described by Sambrook et al., Molecular Cloning: A Laboratory Manual, New York: Cold Spring Harbor Press, 1989, and include the use of washing solution and hybridization conditions (*e.g.*, temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "diagnosis" is used herein to refer to the identification or classification of a molecular or pathological state, disease or condition (*e.g.*, cancer). For example, "diagnosis" may refer to identification of a particular type of cancer. "Diagnosis" may also refer to the classification of a particular subtype of cancer, *e.g.,* by histopathological criteria, or by molecular features (*e.g.,* a subtype characterized by expression of one or a combination of biomarkers (*e.g.*, particular genes or proteins encoded by said genes)).

The term "aiding diagnosis" is used herein to refer to methods that assist in making a clinical determination regarding the presence, or nature, of a particular type of symptom or condition of a disease or disorder (*e.g.*, cancer). For example, a method of aiding diagnosis of a disease or condition (*e.g.*, cancer) can comprise detecting certain biomarkers in a biological sample from an individual.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebrospinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (*e.g.*, tissue or cells) of the same subject or individual. For example, healthy and/or non-diseased cells or tissue adjacent to the diseased cells or tissue (*e.g.,* cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (*e.g.*, tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

For the purposes herein a "section" of a tissue sample is meant a single part or piece of a tissue sample, *e.g.,* a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of polynucleotide analysis or protocol, one may use the results of the polynucleotide expression analysis or protocol to determine whether a specific therapeutic regimen should be performed.

"Individual response" or "response" can be assessed using any endpoint indicating a benefit to the individual, including, without limitation, (1) inhibition, to some extent, of disease progression (*e.g.,* cancer progression), including slowing down and complete arrest; (2) a reduction in tumor size; (3) inhibition (*i.e.,* reduction, slowing down or complete stopping) of cancer cell infiltration into adjacent peripheral organs and/or tissues; (4) inhibition (*i.e.* reduction, slowing down or complete stopping) of metasisis; (5) relief, to some extent, of one or more symptoms associated with the disease or disorder (*e.g.*, cancer); (6) increase in the length of progression free survival; and/or (9) decreased mortality at a given point of time following treatment.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (*e.g.*, Kd values). The difference between said two values may be, for example, less than about 20%, less than about 10%, and/or less than about 5% as a function of the reference/comparator value. The phrase "substantially normal" refers to substantially similar to a reference (*e.g.*, normal reference).

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.*, Kd values). The difference between said two values may be, for example, greater than about 10%, greater than about 20%, greater than about 30%, greater than about 40%, and/or greater than about 50% as a function of the value for the reference/comparator molecule.

The word "label" when used herein refers to a detectable compound or composition. The label is typically conjugated or fused directly or indirectly to a reagent, such as a polynucleotide probe or an antibody, and facilitates detection of the reagent to which it is conjugated or fused. The label may itself be detectable (*e.g.*, radioisotope labels or fluorescent labels) or, in the case of an enzymatic label, may catalyze chemical alteration of a substrate compound or composition which results in a detectable product.

An "effective amount" of an agent refers to an amount effective, at dosages and for periods of time necessary, to achieve the desired therapeutic or prophylactic result.

A "therapeutically effective amount" of a substance/molecule of the disclosure, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies provided herein are used to delay development of a disease or to slow the progression of a disease.

The terms "cancer" and "cancerous" refer to or describe the physiological condition in mammals that is typically characterized by unregulated cell growth/proliferation. Examples of cancer include, but are not limited to, carcinoma, lymphoma (*e.g.*, Hodgkin's and non-Hodgkin's lymphoma), blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "anti-cancer therapy" refers to a therapy useful in treating cancer. Examples of anti-cancer therapeutic agents include, but are limited to, *e.g.*, chemotherapeutic agents, growth inhibitory agents, cytotoxic agents, agents used in radiation therapy, anti-angiogenesis agents, apoptotic agents, anti-tubulin agents, and other agents to treat cancer , anti-CD20 antibodies, platelet derived growth factor inhibitors (*e.g.,* Gleevec™ (Imatinib Mesylate)), a COX-2 inhibitor (*e.g.,* celecoxib), interferons, cytokines, antagonists (*e.g.*, neutralizing antibodies) that bind to one or more of the following targets PDGFR-beta, BlyS, APRIL, BCMA receptor(s), TRAIL/Apo2, and other bioactive and organic chemical agents, etc. Combinations thereof are also included in the disclosure.

The term "cytotoxic agent" as used herein refers to a substance that inhibits or prevents a cellular function and/or causes cell death or destruction. Cytotoxic agents include, but are not limited to, radioactive isotopes (*e.g.*, $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (*see, e.g.,* Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), pegylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, *e.g.*, paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (*e.g.,* LURTOTECAN®); rmRH (*e.g.,* ABARELIX®); BAY439006 (sorafenib; Bayer); SU-11248 (sunitinib, SUTENT®, Pfizer); perifosine, COX-2 inhibitor (*e.g.,* celecoxib or etoricoxib), proteosome inhibitor (*e.g.,* PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (*see* definition below); tyrosine kinase inhibitors (*see* definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this disclosure include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this disclosure include, but are not limited to, those chemotherapeutic agents described above.

A "growth inhibitory agent" when used herein refers to a compound or composition which inhibits growth of a cell (*e.g.,* a cell whose growth is dependent upon a wnt pathway gene and/or R-spondin translocation expression either *in vitro* or *in vivo*). Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al., (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.*, mice and rats). In certain embodiments, the individual or subject is a human.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce" or "inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (*e.g.*, a package or container) or kit comprising at least one reagent, *e.g.,* a medicament for treatment of a disease or disorder (*e.g.,* cancer), or a probe for specifically detecting a biomarker described herein. In certain embodiments, the manufacture or kit is promoted, distributed, or sold as a unit for performing the methods described herein.

A "target audience" is a group of people or an institution to whom or to which a particular medicament is being promoted or intended to be promoted, as by marketing or advertising, especially for particular uses, treatments, or indications, such as individuals, populations, readers of newspapers, medical literature, and magazines, television or internet viewers, radio or internet listeners, physicians, drug companies, etc.

As is understood by one skilled in the art, reference to "about" a value or parameter herein includes (and describes) embodiments that are directed to that value or parameter per se. For example, description referring to "about X" includes description of "X".

It is understood that aspect and embodiments of the disclosure described herein include "consisting" and/or "consisting essentially of" aspects and embodiments. As used herein, the singular form "a", "an", and "the" includes plural references unless indicated otherwise.

### *II. Methods and Uses*

Provided herein are methods utilizing a wnt pathway antagonist. In particular, provided herein are methods utilizing an R-spondin-translocation antagonist. For example, provided herein are methods of inhibiting cell proliferation of a cancer cell comprising contacting the cancer cell with an effective amount of an R-spondin-translocation antagonist. Also provided herein are methods of treating cancer in an individual comprising administering to the individual an effective amount of an R-spondin-translocation antagonist. In some embodiments, the cancer or cancer comprises an R-spondin translocation.

Also provided herein are methods of treating cancer in an individual comprising administering to the individual an effective amount of an anti-cancer therapy, wherein treatment is based upon the individual having cancer comprising one or more biomarkers. In some embodiments, the anti-cancer therapy comprises a wnt pathway antagonist. For example, provided are methods of treating cancer in an individual comprising administering to the individual an effective amount of a wnt pathway antagonist, wherein treatment is based upon the individual having cancer comprising an R-spondin translocation. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Further provided herein are methods of treating cancer in an individual provided that the individual has been found to have cancer comprising one or more biomarkers, the treatment comprising administering to the individual an effective amount of an anti-cancer therapy. In some embodiments, the anti-cancer therapy comprises a wnt pathway antagonist. For example, provided herein are methods of treating cancer in an individual provided that the individual has been found to have cancer comprising an R-spondin translocation, the treatment comprising administering to the individual an effective amount of a wnt pathway antagonist. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Provided herein are methods of treating a cancer cell, wherein the cancer cell comprises one or more biomarkers, the method comprising providing an effective amount of a wnt pathway antagonist. For example, provided herein are methods of treating a cancer cell, wherein the cancer cell comprises an R-spondin translocation, the method comprising providing an effective amount of a wnt pathway antagonist. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Provided herein are methods for treating cancer in an individual, the method comprising: determining that a sample obtained from the individual comprises one or more biomarkers, and administering an effective amount of an anti-cancer therapy comprising a wnt pathway antagonist to the individual, whereby the cancer is treated. For example, provided herein are methods for treating cancer in an individual, the method comprising: determining that a sample obtained from the individual comprises an R-spondin translocation, and administering an effective amount of an anti-cancer therapy comprising a wnt pathway antagonist to the individual, whereby the cancer is treated. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Provided herein are also methods of treating cancer, comprising: (a) selecting an individual having cancer, wherein the cancer comprises one or more biomarkers; and (b) administering to the individual thus selected an effective amount of a wnt pathway antagonist, whereby the cancer is treated. For example, provided herein are also methods of treating cancer, comprising: (a) selecting an individual having cancer, wherein the cancer comprises an R-spondin translocation; and (b) administering to the individual thus selected an effective amount of a wnt pathway antagonist, whereby the cancer is treated. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Further provided herein are methods of identifying an individual with cancer who is more or less likely to exhibit benefit from treatment with an anti-cancer therapy, the method comprising: determining presence or absence of one or more biomarkers in a sample obtained from the individual, wherein presence of the one or more biomarkers in the sample indicates that the individual is more likely to exhibit benefit from treatment with the anti-cancer therapy or absence of the one or more biomarkers indicates that the individual is less likely to exhibit benefit from treatment with the anti-cancer therapy. In some embodiments, the anti-cancer therapy comprises a wnt pathway antagonist. For example, provided herein are methods of identifying an individual with cancer who is more or less likely to exhibit benefit from treatment with an anti-cancer therapy comprising a wnt pathway antagonist, the method comprising: determining presence or absence of an R-spondin translocation in a sample obtained from the individual, wherein presence of the R-spondin translocation in the sample indicates that the individual is more likely to exhibit benefit from treatment with the anti-cancer therapy comprising the wnt pathway antagonist or absence of the R-spondin translocation indicates that the individual is less likely to exhibit benefit from treatment with the anti-cancer therapy comprising the wnt pathway antagonist. In some embodiments, the method further comprises administering an effective amount of a wnt pathway antagonist. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Provided herein are methods for predicting whether an individual with cancer is more or less likely to respond effectively to treatment with an anti-cancer therapy comprising a wnt pathway antagonist, the method comprising determining one or more biomarkers, whereby presence of the one or more biomarkers indicates that the individual is more likely to respond effectively to treatment with the wnt pathway antagonist and absence of the one or more biomarkers indicates that the individual is less likely to respond effectively to treatment with the wnt pathway antagonist. For example, provided herein are methods for predicting whether an individual with cancer is more or less likely to respond effectively to treatment with an anti-cancer therapy comprising a wnt pathway antagonist, the method comprising determining an R-spondin translocation, whereby presence of the R-spondin translocation indicates that the individual is more likely to respond effectively to treatment with the wnt pathway antagonist and absence of the R-spondin translocation indicates that the individual is less likely to respond effectively to treatment with the wnt pathway antagonist. In some embodiments, the method further comprises administering an effective amount of a wnt pathway antagonist. In some embodiments, the wnt pathway antagonist is an R-spondin antagonist (*e.g.,* RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

Provided herein are methods of predicting the response or lack of response of an individual with cancer to an anti-cancer therapy comprising a wnt pathway antagonist comprising detecting in a sample obtained from the individual presence or absence of one or more biomarkers, wherein presence of the one or more biomarkers is predictive of response of the individual to the anti-cancer therapy comprising the wnt pathway antagonist and absence of the one or more biomarkers is predictive of lack of response of the individual to the anti-cancer therapy comprising the wnt pathway antagonist. For example, provided herein are methods of predicting the response or lack of response of an individual with cancer to an anti-cancer therapy comprising a wnt pathway antagonist comprising detecting in a sample obtained from the individual presence or absence of an R-spondin translocation, wherein presence of the R-spondin translocation is predictive of response of the individual to the anti-cancer therapy comprising the wnt pathway antagonist and absence of the R-spondin translocation is predictive of lack of response of the individual to the anti-cancer therapy comprising the wnt pathway antagonist. In some embodiments, the method further comprises administering an effective amount of a wnt pathway antagonist. In some embodiments, the wnt pathway antagonist is an R-spondin antagonist (*e.g.*, RSPO1, RSPO2, RSPO3, and/or RSPO4 antagonist). In some embodiments, the wnt pathway antagonist is an R-spondin-translocation antagonist. In some embodiments, the R-spondin antagonist and/or R-spondin translocation antagonist is an isolated antibody that binds R-spondin (*e.g.*, RSPO1, RSPO2, RSPO3, and/or RSPO4).

In some embodiments of any of the methods, the one or more biomarkers comprise one or more genes listed in Table 2. In some embodiments, the presence of one or more biomarkers comprises the presence of a variation (*e.g.*, polymorphism or mutation) of one or more genes listed in Table 2 (*e.g.,* a variation (*e.g.,* polymorphism or mutation) in Table 2). In some embodiments of any of the methods, the one or more biomarkers comprise one or more genes listed in Table 3. In some embodiments, the presence of one or more biomarkers comprises the presence of a variation (*e.g.,* polymorphism or mutation) of one or more genes listed in Table 3 (*e.g.,* a variation (*e.g.,* polymorphism or mutation) in Table 3). In some embodiments of any of the methods, the one or more biomarkers comprise one or more genes listed in Table 4. In some embodiments, the presence of one or more biomarkers comprises the presence of a variation (*e.g.*, polymorphism or mutation) of one or more genes listed in Table 4 (*e.g.,* a variation (*e.g.*, polymorphism or mutation) in Table 4). In some embodiments of any of the methods, the one or more biomarkers comprise one or more genes listed in Table 5. In some embodiments, the presence of one or more biomarkers comprises the presence of a variation (*e.g.*, polymorphism or mutation) of one or more genes listed in Table 5 (*e.g.,* a variation (*e.g.,* polymorphism or mutation) in Table 5). In some embodiments, the variation (*e.g.,* polymorphism or mutation) is a somatic variation (*e.g.,* polymorphism or mutation).

In some embodiments of any of the methods, the one or more biomarkers comprise one or more genes selected from the group consisting of *KRAS, TP53, APC, PIK3CA, SMAD4, FBXW7, CSMD1*, *NRXN1*, *DNAH5, MRVI1*, *TRPS1*, *DMD, KIF2B, ATM, FAM5C, EVC2, OR2W3*, *SIN3A, SMARCA5, NCOR1*, *JARID2, TCF12, TCF7L2, PHF2, SOS2, RASGRF2, ARHGAP10*, *ARHGEF33, Rab40c*, *TET2, TET3, EP400*, *MLL, TMPRSS11A*, *ERBB3, EPHB4, EFNB3, EPHA1*, *TYRO3*, *TIE1*, *FLT, RIOK3*, *PRKCB, MUSK, MAP2K7, MAP4K5, PTPRN2, GPR4, GPR98, TOPORS,* and *SCN10A.* In some embodiments, the one or more biomarkers comprise one or more genes selected from the group consisting of *CSMD1*, *NRXN1*, *DNAH5, MRVI1, TRPS1*, *DMD, KIF2B, ATM, FAM5C, EVC2, OR2W3*, *TMPRSS11A*, and *SCN10A.* In some embodiments, the one or more biomarkers comprise *RAB40C, TCF12, C20orf132*, *GRIN3A*, and/or *SOS2.* In some embodiments, the one or more biomarkers comprise *ETV4, GRIND2D*, *FOXQ1*, and/or *CLDN1.* In some embodiments, the one or more biomarkers comprise *MRPL33.* In some embodiments In some embodiments, the one or more biomarkers comprise one or more transcriptional regulators (e.g., *TCF12, TCF7L2* and/or *PHF2)* In some embodiments, the one or more biomarkers comprise one or more Ras/Rho related regulators (*e.g., SOS1* (*e.g.*, R547W, T614M R854*, G1129V), *SOS2* (*e.g.,* R225*, R854C, and Q1296H) *RASGRF2*, *ARHGAP10*, *ARHGEF33* and/or *Rab40c* (*e.g.*, G251S)). In some embodiments, the one or more biomarkers comprise one or more chromatin modifying enzymes (*e.g.*, *TET1*, *TET2*, *TET3*, *EP400* and/or *MLL*). In some embodiments, the one or more chromatin modifying enzymes are *TET1* and/or *TET3.* In some embodiments, the one or more chromatin modifying enzymes are *TET1* (*e.g.*, R81H, E417A, K540T, K792T, S879L, S1012*, Q1322*, C1482Y, A1896V, and A2129V), *TET2* (*e.g.*, K108T, T118I, S289L, F373L, K1056N, Y1169*, A1497V, and V1857M), and/or *TET3* (*e.g.*, T165M, A874T, M977V, G1398R, and R1576Q/W). In some embodiments, the one or more biomarkers comprise one or more receptor tyrosine kinases (*e.g.*, *ERBB3*, *EPHB4*, *EFNB3*, *EPHA1*, *TYRO3*, *TIE1* and *FLT4*). In some embodiments, the one or more biomarkers comprise one or more kinases (*e.g.*, *RIOK3*, *PRKCB*, *MUSK, MAP2K7* and *MAP4K5*). In some embodiments, the one or more biomarkers comprise one or more protein phosphatase (*e.g., PTPRN2*). In some embodiments, the one or more biomarkers comprise one or more GPRCs (*e.g.*, *GPR4* and/or *GPR98*). In some embodiments, the one or more biomarkers comprise one or more E3-ligase (*e.g.*, *TOPORS*). In some embodiments, the presence of the one or more biomarkers comprise presence of a variation (*e.g.*, polymorphism or mutation) of the one or more biomarkers listed in Table 2, 3, 4, and/or 5 *(e.g.,* a variation (*e.g.*, polymorphism or mutation) in Table 2, 3, 4, and/or 5). In some embodiments, the variation (*e.g.*, polymorphism or mutation) comprise a somatic variation (*e.g.*, polymorphism or mutation).

In some embodiments of any of the methods, the one or more biomarkers comprise one or more RSPO (*e.g.,* RSPO1, RSPO2, RSPO3, and/or RSPO4). In some embodiments, presence of the one or more biomarkers is indicated by the presence of elevated expression levels (*e.g.*, compared to reference) of one or more RSPO (*e.g.,* RSPO1, RSPO2, RSPO3, and/or RSPO4). In some embodiments, the one or more biomarkers comprises RSPO1. In some embodiments, the one or more biomarkers comprises RSPO2. In some embodiments, the one or more biomarkers comprises RSPO3. In some embodiments, the one or more biomarkers comprises RSPO4.

In some embodiments of any of the methods, the one or more biomarkers comprise one or more genes listed in Table 6. In some embodiments, presence of the one or more biomarkers is indicated by the presence of elevated expression levels (*e.g.*, compared to reference) of one or more genes listed in Table 6. In some embodiments, the one or more biomarkers comprise *FOXA1*, *CLND1*, and/or *IGF2.* In some embodiments, presence of the one or more biomarkers is indicated by presence of elevated expression levels (*e.g.,* compared to reference) of *FOXA1, CLND1*, and/or *IGF2.* In some embodiments, the one or more biomarkers comprise a differentially expressed signaling pathway including, but not limited to, Calcium Signaling, cAMP-mediated signaling, Glutamate Receptor Signaling, Amyotrophic Lateral Sclerosis Signaling, Nitrogen Metabolism, Axonal Guidance Signaling, Role of IL-17A in Psoriasis, Serotonin Receptor Signaling, Airway Pathology in Chronic Obstructive Pulmonary Disease, Protein Kinase A Signaling, Bladder Cancer Signaling, HIF1α Signaling, Cardiac β-adrenergic Signaling, Synaptic Long Term Potentiation, Atherosclerosis Signaling, Circadian Rhythm Signaling, CREB Signaling in Neurons, G-Protein Coupled Receptor Signaling, Leukocyte Extravasation Signaling, Complement System, Eicosanoid Signaling, Tyrosine Metabolism, Cysteine Metabolism, Synaptic Long Term Depression, Role of IL-17A in Arthritis, Cellular Effects of Sildenafil (Viagra), Neuropathic Pain Signaling In Dorsal Horn Neurons, D-arginine and D-ornithine Metabolism, Role of IL-17F in Allergic Inflammatory Airway Diseases, Thyroid Cancer Signaling, Hepatic Fibrosis / Hepatic Stellate Cell Activation, Dopamine Receptor Signaling, Role of NANOG in Mammalian Embryonic Stem Cell Pluripotency, Chondroitin Sulfate Biosynthesis, Endothelin-1 Signaling, Keratan Sulfate Biosynthesis, Phototransduction Pathway, Wnt/β-catenin Signaling, Chemokine Signaling, Alanine and Aspartate Metabolism, Glycosphingolipid Biosynthesis - Neolactoseries, Bile Acid Biosynthesis, Role of Macrophages, Fibroblasts and Endothelial Cells in Rheumatoid Arthritis, α-Adrenergic Signaling, Taurine and Hypotaurine Metabolism, LPS/IL-1 Mediated Inhibition of RXR Function, Colorectal Cancer Metastasis Signaling, CCR3 Signaling in Eosinophils, and/or O-Glycan Biosynthesis.

In some embodiments of any of the methods, the one or more biomarkers comprise one or more genes listed in Table 7. In some embodiments, presence of the one or more biomarkers is indicated by the presence of elevated gene copy number (*e.g.*, compared to reference) of one or more genes listed in Table 7. In some embodiments, the one or more biomarkers comprise *IGF2, KRAS,* and/or *MYC.* In some embodiments, presence of the one or more biomarkers is indicated by the presence of elevated gene copy number (*e.g.,* compared to reference) of *IGF2, KRAS,* and/or *MYC.* In some embodiments, presence of the one or more biomarkers is indicated by the presence of reduced gene copy number (*e.g.*, compared to reference) of one or more genes listed in Table 7. In some embodiments, the one or more biomarkers comprise *FHIT, APC,* and/or *SMAD4.* In some embodiments, presence of the one or more biomarkers is indicated by the presence of reduced gene copy number (*e.g.,* compared to reference) of *FHIT, APC,* and/or *SMAD4.* In some embodiments, presence of the one or more biomarkers is indicated by the presence of elevated copy number (*e.g.*, compared to reference) of chromosome 20q. In some embodiments, presence of the one or more biomarkers is indicated by the presence of reduced copy number (*e.g.*, compared to reference) of chromosome 18q.

In some embodiments of any of the methods, the one or more biomarkers comprise one or more genes listed in Table 9. In some embodiments, presence of the one or more biomarkers is indicated by the presence of a variation (*e.g.*, polymorphism or mutation) of one or more genes listed in Table 9 (*e.g.,* a variation (*e.g.*, polymorphism or mutation) in Table 9) and/or alternative splicing (*e.g.*, compared to reference) of one or more genes listed in Table 9. In some embodiments, the one or more biomarkers comprise *TP53, NOTCH2, MRPL33,* and/or *EIF5B*. In some embodiments, the one or more biomarkers is *MRPL33.* In some embodiments, presence of the one or more biomarkers is indicated by the presence of a variation (*e.g.*, polymorphism or mutation) of *TP53*, *NOTCH2*, *MRPL33*, and/or *EIF5B* (*e.g.,* a variation (*e.g.,* polymorphism or mutation) in Table 9) and/or alternative splicing (*e.g.,* compared to reference) of *TP53*, *NOTCH2*, *MRPL33*, and/or *EIF5B.*

In some embodiments of any of the methods, the one or more biomarkers comprise a translocation (*e.g.*, rearrangement and/or fusion) of one or more genes listed in Table 10. In some embodiments, the presence of one or more biomarkers comprises the presence of a translocation (*e.g.,* rearrangement and/or fusion) of one or more genes listed in Table 10 (*e.g.,* a translocation (e.g., rearrangement and/or fusion) in Table 10). In some embodiments of any of the methods, the translocation (*e.g.,* rearrangement and/or fusion) is a PVT1 translocation (*e.g.,* rearrangement and/or fusion). In some embodiments, the PVT1 translocation (*e.g.*, rearrangement and/or fusion) comprises *PVT1* and *MYC.* In some embodiments, the RSPO2 translocation (*e.g.,* rearrangement and/or fusion) comprises *PVT1* and *IncDNA.* In some embodiments of any of the methods, the translocation (*e.g.,* rearrangement and/or fusion) is an R-spondin translocation (*e.g.*, rearrangement and/or fusion). In some embodiments, the R-spondin translocation (*e.g.*, rearrangement and/or fusion) is a RSPO1 translocation (*e.g.*, rearrangement and/or fusion). In some embodiments, the R-spondin translocation (*e.g.,* rearrangement and/or fusion) is a RSPO2 translocation (*e.g.*, rearrangement and/or fusion). In some embodiments, the RSPO2 translocation (*e.g.*, rearrangement and/or fusion) comprises *EIF3E* and *RSPO2.* In some embodiments, the RSPO2 translocation (*e.g.*, rearrangement and/or fusion) comprises *EIF3E* exon 1 and *RSPO2* exon 2. In some embodiments, the RSPO2 translocation (*e.g.,* rearrangement and/or fusion) comprises *EIF3E* exon 1 and *RSPO2* exon 3. In some embodiments, the RSPO2 translocation (*e.g.*, rearrangement and/or fusion) comprises SEQ ID NO:71. In some embodiments, the RSPO2 translocation (*e.g.*, rearrangement and/or fusion) is detectable by primers which include SEQ ID NO:12, 41, and/or 42. In some embodiments, the RSPO2 translocation (*e.g.,* rearrangement and/or fusion) is driven by the *EIF3E* promoter. In some embodiments, the RSPO2 translocation (*e.g.*, rearrangement and/or fusion) is driven by the *RSPO2* promoter. In some embodiments, the R-spondin translocation (*e.g.,* rearrangement and/or fusion) is a RSPO3 translocation (*e.g.*, rearrangement and/or fusion). In some embodiments, the RSPO3 translocation (*e.g.,* rearrangement and/or fusion) comprises *PTPRK* and *RSPO3.* In some embodiments, the RSPO3 translocation (*e.g.,* rearrangement and/or fusion) comprises *PTPRK* exon 1 and *RSPO3* exon 2. In some embodiments, the RSPO3 translocation (*e.g.,* rearrangement and/or fusion) comprises *PTPRK* exon 7 and *RSPO3* exon 2. In some embodiments, the RSPO3 translocation (*e.g.*, rearrangement and/or fusion) comprises SEQ ID NO:72 and/or SEQ ID NO:73. In some embodiments, the RSPO3 translocation (*e.g.*, rearrangement and/or fusion) is detectable by primers which include SEQ ID NO: 13, 14, 43, and/or 44. In some embodiments, the RSPO3 translocation (*e.g.*, rearrangement and/or fusion) is driven by the *PTPRK* promoter. In some embodiments, the RSPO3 translocation *(e.g.,* rearrangement and/or fusion) is driven by the *RSPO3* promoter. In some embodiments, the RSPO3 translocation (*e.g.*, rearrangement and/or fusion) comprises the PTPRK secretion signal sequence (and/or does not comprise the RSPO3 secretion signal sequence). In some embodiments, the R-spondin translocation (*e.g.*, rearrangement and/or fusion) is a RSPO4 translocation (*e.g.*, rearrangement and/or fusion). In some embodiments, the R-spondin translocation (*e.g.*, rearrangement and/or fusion) results in elevated expression levels of R-spondin (*e.g.*, compared to a reference without the R-spondin translocation). In some embodiments, the R-spondin translocation (*e.g.*, rearrangement and/or fusion) results in elevated activity and/or activation of R-spondin (*e.g.*, compared to a reference without the R-spondin translocation). In some embodiments, the presence of one or more biomarkers comprises an R-spondin translocation (*e.g.*, rearrangement and/or fusion), such as a translocation (*e.g.*, rearrangement and/or fusion) in Table 10, and *KRAS* and/or *BRAF.* In some embodiments, the presence of one or more biomarkers is presence of an R-spondin translocation (*e.g.*, rearrangement and/or fusion), such as a translocation (*e.g.,* rearrangement and/or fusion) in Table 10, and a variation (*e.g.,* polymorphism or mutation) *KRAS* and/or *BRAF.* In some embodiments, the presence of one or more biomarkers is presence of an R-spondin translocation (*e.g.,* rearrangement and/or fusion), such as a translocation (*e.g.,* rearrangement and/or fusion) in Table 10, and the absence of one or more biomarkers is absence of a variation (*e.g.,* polymorphism or mutation) *CTNNB1* and/or *APC.*

In some embodiments of any of the translocation (*e.g.*, rearrangement and/or fusion), the translocation (*e.g.,* rearrangement and/or fusion) is a somatic translocation (*e.g.,* rearrangement and/or fusion). In some embodiments, the translocation (*e.g.*, rearrangement and/or fusion) is an intra-chromosomal translocation (*e.g.*, rearrangement and/or fusion). In some embodiments, the translocation (*e.g.,* rearrangement and/or fusion) is an inter-chromosomal translocation (*e.g.,* rearrangement and/or fusion). In some embodiments, the translocation (*e.g.*, rearrangement and/or fusion) is an inversion. In some embodiments, the translocation (*e.g.*, rearrangement and/or fusion) is a deletion. In some embodiments, the translocation (*e.g.*, rearrangement and/or fusion) is a functional translocation fusion polynucleotide (*e.g.*, functional R-spondin-translocation fusion polynucleotide) and/or functional translocation fusion polypeptide (*e.g.*, functional R-spondin-translocation fusion polypeptide). In some embodiments, the functional translocation fusion polypeptide (*e.g.*, functional R-spondin-translocation fusion polypeptide) activates a pathway known to be modulated by one of the tranlocated genes (*e.g.*, wnt signaling pathway). In some embodiments, the pathway is canonical wnt signaling pathway. In some embodiments, the pathway is noncanonical wnt signaling pathway. In some embodiments, the Methods of determining pathway activation are known in the art and include luciferase reporter assays as described herein.

Examples of cancers and cancer cells include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer (*e.g.,* epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer. In some embodiments, the cancer is colorectal cancer. In some embodiments, the cancer is colon cancer. In some embodiments, the cancer is rectal cancer.

Presence and/or expression levels/amount of a biomarker (*e.g.*, R-spondin translocation) can be determined qualitatively and/or quantitatively based on any suitable criterion known in the art, including but not limited to DNA, mRNA, cDNA, proteins, protein fragments and/or gene copy number. In certain embodiments, presence and/or expression levels/amount of a biomarker in a first sample is increased as compared to presence/absence and/or expression levels/amount in a second sample. In certain embodiments, presence/absence and/or expression levels/amount of a biomarker in a first sample is decreased as compared to presence and/or expression levels/amount in a second sample. In certain embodiments, the second sample is a reference sample, reference cell, reference tissue, control sample, control cell, or control tissue. Additional disclosures for determining presence/absence and/or expression levels/amount of a gene are described herein.

In some embodiments of any of the methods, elevated expression refers to an overall increase of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, the elevated expression refers to the increase in expression level/amount of a biomarker in the sample wherein the increase is at least about any of 1.5X, 1.75X, 2X, 3X, 4X, 5X, 6X, 7X, 8X, 9X, 10X, 25X, 50X, 75X, or 100X the expression level/amount of the respective biomarker in a reference sample, reference cell, reference tissue, control sample, control cell, or control tissue. In some embodiments, elevated expression refers to an overall increase of greater than about 1.5 fold, about 1.75 fold, about 2 fold, about 2.25 fold, about 2.5 fold, about 2.75 fold, about 3.0 fold, or about 3.25 fold as compared to a reference sample, reference cell, reference tissue, control sample, control cell, control tissue, or internal control (*e.g.*, housekeeping gene).

In some embodiments of any of the methods, reduced expression refers to an overall reduction of about any of 10%, 20%, 30%, 40%, 50%, 60%, 70%, 80%, 90%, 95%, 96%, 97%, 98%, 99% or greater, in the level of biomarker (*e.g.,* protein or nucleic acid (*e.g.,* gene or mRNA)), detected by standard art known methods such as those described herein, as compared to a reference sample, reference cell, reference tissue, control sample, control cell, or control tissue. In certain embodiments, reduced expression refers to the decrease in expression level/amount of a biomarker in the sample wherein the decrease is at least about any of 0.9X, 0.8X, 0.7X, 0.6X, 0.5X, 0.4X, 0.3X, 0.2X, 0.1X, 0.05X, or 0.01X the expression level/amount of the respective biomarker in a reference sample, reference cell, reference tissue, control sample, control cell, or control tissue.

Presence and/or expression level/amount of various biomarkers in a sample can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In some embodiments, presence and/or expression level/amount of a biomarker is determined using a method comprising: (a) performing gene expression profiling, PCR (such as rtPCR), RNA-seq, microarray analysis, SAGE, MassARRAY technique, or FISH on a sample (such as a subject cancer sample); and b) determining presence and/or expression level/amount of a biomarker in the sample. In some embodiments, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qRT-PCR. In one embodiment, the PCR method is multiplex-PCR. In some embodiments, gene expression is measured by microarray. In some embodiments, gene expression is measured by qRT-PCR. In some embodiments, expression is measured by multiplex-PCR.

Methods for the evaluation of mRNAs in cells are well known and include, for example, hybridization assays using complementary DNA probes (such as in situ hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

Samples from mammals can be conveniently assayed for mRNAs using Northern, dot blot or PCR analysis. In addition, such methods can include one or more steps that allow one to determine the levels of target mRNA in a biological sample (*e.g.,* by simultaneously examining the levels a comparative control mRNA sequence of a "housekeeping" gene such as an actin family member). Optionally, the sequence of the amplified target cDNA can be determined.

Optional methods of the disclosure include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of anti-angiogenic therapy may be arrayed on a solid support. Hybridization of a labeled probe with a particular array member indicates that the sample from which the probe was derived expresses that gene.

According to some embodiments, presence and/or expression level/amount is measured by observing protein expression levels of an aforementioned gene. In certain embodiments, the method comprises contacting the biological sample with antibodies to a biomarker (*e.g.*, anti-R-spondin translocation antibodies) described herein under conditions permissive for binding of the biomarker, and detecting whether a complex is formed between the antibodies and biomarker. Such method may be an in vitro or in vivo method. In one embodiment, an antibody is used to select subjects eligible for therapy with wnt pathway antagonist, in particular R-spondin-translocation antagonist, *e.g.,* a biomarker for selection of individuals.

In certain embodiments, the presence and/or expression level/amount of biomarker proteins in a sample is examined using IHC and staining protocols. IHC staining of tissue sections has been shown to be a reliable method of determining or detecting presence of proteins in a sample. In one aspect, expression level of biomarker is determined using a method comprising: (a) performing IHC analysis of a sample (such as a subject cancer sample) with an antibody; and b) determining expression level of a biomarker in the sample. In some embodiments, IHC staining intensity is determined relative to a reference value.

IHC may be performed in combination with additional techniques such as morphological staining and/or fluorescence in-situ hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC typically will be labeled with a detectable moiety. Numerous labels are available which can be generally grouped into the following categories: (a) Radioisotopes, such as 35S, 14C, 125I, 3H, and 131I; (b) colloidal gold particles; (c) fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (*e.g.*, firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and β-D-galactosidase (β-D-Gal) with a chromogenic substrate (*e.g.,* p-nitrophenyl-β-D-galactosidase) or fluorogenic substrate (*e.g.,* 4-methylumbelliferyl-β-D-galactosidase). For a general review of these, *see* U.S. Patent Nos. 4,275,149 and 4,318,980.

Specimens thus prepared may be mounted and coverslipped. Slide evaluation is then determined, *e.g*., using a microscope, and staining intensity criteria, routinely used in the art, may be employed. In some embodiments, a staining pattern score of about 1+ or higher is diagnostic and/or prognostic. In certain embodiments, a staining pattern score of about 2+ or higher in an IHC assay is diagnostic and/or prognostic. In other embodiments, a staining pattern score of about 3 or higher is diagnostic and/or prognostic. In one embodiment, it is understood that when cells and/or tissue from a tumor or colon adenoma are examined using IHC, staining is generally determined or assessed in tumor cell and/or tissue (as opposed to stromal or surrounding tissue that may be present in the sample).

In alternative methods, the sample may be contacted with an antibody specific for said biomarker (*e.g*., anti-R-spondin translocation antibody) under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, *see, e.g.,* U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Presence and/or expression level/amount of a selected biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

In certain embodiments, the samples are normalized for both differences in the amount of the biomarker assayed and variability in the quality of the samples used, and variability between assay runs. Such normalization may be accomplished by detecting and incorporating the expression of certain normalizing biomarkers, including well known housekeeping genes, such as ACTB. Alternatively, normalization can be based on the mean or median signal of all of the assayed genes or a large subset thereof (global normalization approach). On a gene-by-gene basis, measured normalized amount of a subject tumor mRNA or protein is compared to the amount found in a reference set. Normalized expression levels for each mRNA or protein per tested tumor per subject can be expressed as a percentage of the expression level measured in the reference set. The presence and/or expression level/amount measured in a particular subject sample to be analyzed will fall at some percentile within this range, which can be determined by methods well known in the art.

In certain embodiments, relative expression level of a gene is determined as follows:

Relative expression gene1 sample1 = 2 exp (Ct housekeeping gene - Ct gene1) with Ct determined in a sample.
Relative expression gene1 reference RNA = 2 exp (Ct housekeeping gene - Ct gene1) with Ct determined in the reference sample.

$$\text{Normalized relative expression gene1 sample1} = (\text{relative expression gene1 sample1} / \text{relative expression gene1 reference RNA}) \times 100$$

Ct is the threshold cycle. The Ct is the cycle number at which the fluorescence generated within a reaction crosses the threshold line.

All experiments are normalized to a reference RNA, which is a comprehensive mix of RNA from various tissue sources (*e.g*., reference RNA #636538 from Clontech, Mountain View, CA). Identical reference RNA is included in each qRT-PCR run, allowing comparison of results between different experimental runs.

In one embodiment, the sample is a clinical sample. In another embodiment, the sample is used in a diagnostic assay. In some embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Genes or gene products can be detected from cancer or tumor tissue or from other body samples such as urine, sputum, serum or plasma. The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

In certain embodiments, a reference sample, reference cell, reference tissue, control sample, control cell, or control tissue is a single sample or combined multiple samples from the same subject or individual that are obtained at one or more different time points than when the test sample is obtained. For example, a reference sample, reference cell, reference tissue, control sample, control cell, or control tissue is obtained at an earlier time point from the same subject or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain embodiments, a reference sample, reference cell, reference tissue, control sample, control cell, or control tissue is a combined multiple samples from one or more healthy individuals who are not the subject or individual. In certain embodiments, a reference sample, reference cell, reference tissue, control sample, control cell, or control tissue is a combined multiple samples from one or more individuals with a disease or disorder (*e.g.*, cancer) who are not the subject or individual. In certain embodiments, a reference sample, reference cell, reference tissue, control sample, control cell, or control tissue is pooled RNA samples from normal tissues or pooled plasma or serum samples from one or more individuals who are not the subject or individual. In certain embodiments, a reference sample, reference cell, reference tissue, control sample, control cell, or control tissue is pooled RNA samples from tumor tissues or pooled plasma or serum samples from one or more individuals with a disease or disorder (*e.g.*, cancer) who are not the subject or individual.

In some embodiments of any of the methods, the wnt pathway antagonist is an R-spondin antagonist (*e.g.*, RSPO1, RSPO2, RSPO3, and/or RSPO4 antagonist). In some embodiments of any of the methods, the R-spondin antagonist in particular R-spondin-translocation antagonist is an antibody, binding polypeptide, binding small molecule, or polynucleotide. In some embodiments, the R-spondin antagonist in particular R-spondin-translocation antagonist is an antibody. In some embodiments, the antibody is a monoclonal antibody. In some embodiments, the antibody is a human, humanized, or chimeric antibody. In some embodiments, the antibody is an antibody fragment and the antibody fragment binds wnt pathway polypeptide in particular R-spondin antagonist and/or R-spondin-translocation fusion polypeptide.

In some embodiments of any of the methods, the individual according to any of the above embodiments may be a human.

In some embodiments of any of the methods, the method comprises administering to an individual having such cancer an effective amount of a wnt pathway antagonist in particular R-spondin-translocation antagonist. In one such embodiment, the method further comprises administering to the individual an effective amount of at least one additional therapeutic agent, as described below. In some embodiments, the individual may be a human.

The wnt pathway antagonist, in particular R-spondin-translocation antagonist, described herein can be used either alone or in combination with other agents in a therapy. For instance, a wnt pathway antagonist, in particular R-spondin-translocation antagonist, described herein may be co-administered with at least one additional therapeutic agent including another wnt pathway antagonist. In certain embodiments, an additional therapeutic agent is a chemotherapeutic agent.

Such combination therapies noted above encompass combined administration (where two or more therapeutic agents are included in the same or separate formulations), and separate administration, in which case, administration of the wnt pathway antagonist, in particular R-spondin-translocation antagonist, can occur prior to, simultaneously, and/or following, administration of the additional therapeutic agent and/or adjuvant. Wnt pathway antagonist, in particular R-spondin-translocation antagonist, can also be used in combination with radiation therapy.

A wnt pathway antagonist, in particular R-spondin-translocation antagonist (e.g., an antibody, binding polypeptide, and/or small molecule) described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, *e.g.,* by injections, such as intravenous or subcutaneous injections, depending in part on whether the administration is brief or chronic. Various dosing schedules including but not limited to single or multiple administrations over various time-points, bolus administration, and pulse infusion are contemplated herein.

Wnt pathway antagonist, in particular R-spondin antagonist (*e.g.*, an antibody, binding polypeptide, and/or small molecule) described herein may be formulated, dosed, and administered in a fashion consistent with good medical practice. Factors for consideration in this context include the particular disorder being treated, the particular mammal being treated, the clinical condition of the individual, the cause of the disorder, the site of delivery of the agent, the method of administration, the scheduling of administration, and other factors known to medical practitioners. The wnt pathway antagonist, in particular R-spondin antagonist, need not be, but is optionally formulated with one or more agents currently used to prevent or treat the disorder in question. The effective amount of such other agents depends on the amount of the wnt pathway antagonist, in particular R-spondin antagonist, present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a wnt pathway antagonist, in particular R-spondin antagonist, described herein (when used alone or in combination with one or more other additional therapeutic agents) will depend on the type of disease to be treated, the severity and course of the disease, whether the wnt pathway antagonist, in particular R-spondin antagonist, is administered for preventive or therapeutic purposes, previous therapy, the subject's clinical history and response to the wnt pathway antagonist, and the discretion of the attending physician. The wnt pathway antagonist, in particular R-spondin antagonist, is suitably administered to the individual at one time or over a series of treatments. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, *e.g.*, every week or every three weeks (*e.g.*, such that the individual receives from about two to about twenty, or *e.g.,* about six doses of the wnt pathway antagonist). An initial higher loading dose, followed by one or more lower doses may be administered. An exemplary dosing regimen comprises administering. However, other dosage regimens may be useful. The progress of this therapy is easily monitored by conventional techniques and assays.

It is understood that any of the above formulations or therapeutic methods may be carried out using an immunoconjugate of the disclosure in place of or in addition to the wnt pathway antagonist, in particular R-spondin antagonist.

### *III. Therapeutic Compositions*

Provided herein are wnt pathway antagonists useful in the methods described herein. In some embodiments, the wnt pathway antagonists are an antibody, binding polypeptide, binding small molecule, and/or polynucleotide. In some embodiments, the wnt pathway antagonists are canonical wnt pathway antagonists. In some embodiments, the wnt pathway antagonists are non-canonical wnt pathway antagonists.

In some embodiments, the wnt pathway antagonists are R-spondin antagonists. In some embodiments, the R-spondin antagonists are R-spondin-translocation antagonists. In some embodiments, the R-spondin antagonist inhibits LPR6 mediated wnt signaling. In some embodiments, the R-spondin antagonist inhibits and/or blocks the interaction of R-spondin and LRP6. In some embodiments, the R-spondin antagonist inhibits LGR5 mediated wnt signaling. In some embodiments, the R-spondin antagonist inhibits and/or blocks the interaction of R-spondin and LGR5. In some embodiments, the R-spondin antagonist inhibits KRM mediated wnt signaling. In some embodiments, the R-spondin antagonist inhibits and/or blocks the interaction of R-spondin and KRM. In some embodiments, the R-spondin antagonist inhibits syndecan (*e.g.*, syndecan 4) mediated wnt signaling. In some embodiments, the R-spondin antagonist inhibits and/or blocks the interaction of R-spondin and syndecan (*e.g.*, syndecan 4). Examples of R-spondin antagonists include, but are not limited to, those described in WO 2008/046649, WO 2008/020942, WO 2007/013666, WO 2005/040418, WO 2009/005809, US 8,088,374, US 7,541,431, WO 2011/076932, and/or US 2009/0074782.

A wnt signaling pathway component or wnt pathway polypeptide is a component that transduces a signal originating from an interaction between a Wnt protein and an Fz receptor. As the wnt signaling pathway is complex, and involves extensive feedback regulation. Example of wnt signaling pathway components include Wnt (*e.g.,* WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, WNT16), Frizzled (*e.g.*, Frz 1-10), RSPO (*e.g.*, RSPO1, RSPO2, RSPO3, and/or RSPO4), LGR (*e.g.*, LGR5), WTX, WISP (*e.g.*, WISP1, WISP2, and/or WISP3), βTrCp, STRA6, the membrane associated proteins LRP (*e.g.,* LRP5 and/or LRP6), Axin, and Dishevelled, the extracellular Wnt interactive proteins sFRP, WIF-1, the LRP inactivating proteins Dkk and Krn, the cytoplasmic protein β-catenin, members of the β-catenin "degradation complex" APC, GSK3β, CKIα and PP2A, the nuclear transport proteins APC, pygopus and bcl9/legless, and the transcription factors TCF/LEF, Groucho and various histone acetylases such as CBP/p300 and Brg-1.

#### *A. Antibodies*

In one aspect, provided herein isolated antibodies that bind to a wnt pathway polypeptide. In any of the above embodiments, an antibody is humanized. In a further aspect of the disclosure, an anti-wnt pathway antibody according to any of the above embodiments is a monoclonal antibody, including a chimeric, humanized or human antibody. In one embodiment, an anti-wnt pathway antibody is an antibody fragment, *e.g.*, an Fv, Fab, Fab', scFv, diabody, or $F(ab')_2$ fragment. In another embodiment, the antibody is a full length antibody, *e.g.*, an intact IgG1" antibody or other antibody class or isotype as defined herein.

In some embodiments of any of the antibodies, the anti-wnt pathway antibody is an anti-LRP6 antibody. Examples of anti-LRP6 antibodies include, but are not limited to, the anti-LRP6 antibodies described in U.S. Patent Application No. 2011/0256127. In some embodiments, the anti-LRP6 antibody inhibits signaling induced by a first Wnt isoform and potentiates signaling induced by a second Wnt isoform. In some embodiments, the first Wnt isoform is selected from the group consisting of Wnt3 and Wnt3a and the second Wnt isoform is selected from the group consisting of Wnt 1, 2, 2b, 4, 6, 7a, 7b, 8a, 9a, 9b, 10a, and 10b. In some embodiments, the first Wnt isoform is selected from the group consisting of Wnt 1, 2, 2b, 6, 8a, 9a, 9b, and 10b and the second Wnt isoform is selected from the group consisting of Wnt3 and Wnt3a.

In some embodiments of any of the antibodies, the anti-wnt pathway antibody is an anti-Frizzled antibody. Examples of anti-Frizzled antibodies include, but are not limited to, the anti-Frizzled antibodies described in U.S. Patent No. 7,947,277.

In some embodiments of any of the antibodies, the anti-wnt pathway antibody is an anti-STRA6 antibody. Examples of anti-STRA6 antibodies include, but are not limited to, the anti-STRA6 antibodies described in U.S. Patent No. 7,173,115, 7,741,439, and/or 7,855,278.

In some embodiments of any of the antibodies, the anti-wnt pathway antibody is an anti-SlOO-like cytokine polypeptide antibody. In some embodiments, the anti-S100-like cytokine polypeptide antibody is an anti-S100-A14 antibody. Examples of anti-S100-like cytokine polypeptide antibodies include, but are not limited to, the anti-S100-like cytokine polypeptide antibodies described in U.S. Patent No. 7,566,536 and/or 7,005,499.

In some embodiments of any of the antibodies, the anti-wnt pathway antibody is an anti-R-spondin antibody. In some embodiment, the R-spondin is RSPO1. In some embodiment, the R-spondin is RSPO2. In some embodiment, the R-spondin is RSPO3. In some embodiment, the R-spondin is RSPO4. In some embodiments, the R-spondin antagonist inhibits LPR6 mediated wnt signaling. In some embodiments, the R-spondin antagonist inhibits and/or blocks the interaction of R-spondin and LRP6. In some embodiments, the R-spondin antagonist inhibits LGR5 mediated wnt signaling. In some embodiments, the R-spondin antagonist inhibits and/or blocks the interaction of R-spondin and LGR5. In some embodiments, the R-spondin antagonist inhibits LGR4 mediated wnt signaling. In some embodiments, the R-spondin antagonist inhibits and/or blocks the interaction of R-spondin and LGR4. In some embodiments, the R-spondin antagonist inhibits ZNRF3 and/or RNF43 mediated wnt signaling. In some embodiments, the R-spondin antagonist inhibits and/or blocks the interaction of R-spondin and ZNRF3 and/or RNF43. In some embodiments, the R-spondin antagonist inhibits KRM mediated wnt signaling. In some embodiments, the R-spondin antagonist inhibits and/or blocks the interaction of R-spondin and KRM. In some embodiments, the R-spondin antagonist inhibits syndecan (*e.g.*, syndecan 4) mediated wnt signaling. In some embodiments, the R-spondin antagonist inhibits and/or blocks the interaction of R-spondin and syndecan (*e.g.*, syndecan 4). Examples of R-spondin antibodies include, but are not limited to, any antibody disclosed in US 2009/0074782, US 8088374, US 8,158,757, US8,1587,58 and/or US Biological R9417-50C.

In some embodiments, the anti-R-spondin antibody binds to an R-spondin-translocation fusion polypeptide. In some embodiments, the antibodies that bind to an R-spondin-translocation fusion polypeptide specifically bind an R-spondin-translocation fusion polypeptide, but do not substantially bind wild-type R-spondin and/or a second gene of the translocation. In some embodiments, the R-spondin-translocation fusion polypeptide is RSPO1-translocation fusion polypeptide. In some embodiments, the R-spondin-translocation fusion polypeptide is RSPO2-translocation fusion polypeptide. In some embodiments, the R-spondin-translocation fusion polypeptide is RSPO3-translocation fusion polypeptide. In some embodiments, the R-spondin-translocation fusion polypeptide is RSPO4-translocation fusion polypeptide. In some embodiments, the RSPO2-translocation fusion polypeptide comprises *EIF3E* and *RSPO2.* In some embodiments, the RSPO2 -translocation fusion polypeptide comprises *EIF3E* exon 1 and *RSPO2* exon 2. In some embodiments, the RSPO2-translocation fusion polypeptide comprises *EIF3E* exon 1 and *RSPO2* exon 3. In some embodiments, the RSPO2-translocation fusion polypeptide comprises SEQ ID NO:71. In some embodiments, the RSPO3-translocation fusion polypeptide comprises *PTPRK* and *RSPO3.* In some embodiments, the RSPO3-translocation fusion polypeptide comprises *PTPRK* exon 1 and *RSPO3* exon 2. In some embodiments, the RSPO3-translocation fusion polypeptide comprises *PTPRK* exon 7 and *RSPO3* exon 2. In some embodiments, the RSPO3-translocation fusion polypeptide comprises SEQ ID NO:72 and/or SEQ ID NO:73.

In a further aspect, an anti-wnt pathway antibody, in particular, an anti-R-spondin-translocation antibody, according to any of the above embodiments may incorporate any of the features, singly or in combination, as described in Sections below:

### *1. Antibody Affinity*

In certain embodiments, an antibody provided herein has a dissociation constant (Kd) of ≤ 1μM. In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (*see, e.g.,* Chen et al., J. Mol. Biol. 293:865-881(1999)). To establish conditions for the assay, MICROTITER® multi-well plates (Thermo Scientific) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (*e.g.*, consistent with assessment of the anti-VEGF antibody, Fab-12, in Presta et al., Cancer Res. 57:4593-4599 (1997)). The Fab of interest is then incubated overnight; however, the incubation may continue for a longer period (*e.g.,* about 65 hours) to ensure that equilibrium is reached. Thereafter, the mixtures are transferred to the capture plate for incubation at room temperature (*e.g.*, for one hour). The solution is then removed and the plate washed eight times with 0.1% polysorbate 20 (TWEEN-20®) in PBS. When the plates have dried, 150 μl/well of scintillant (MICRO-SCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE®-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with *N*-ethyl-*N'*-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 μg/ml (~0.2 μM) before injection at a flow rate of 5 μl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 μl/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. *See, e.g.,* Chen et al., J. Mol. Biol. 293:865-881 (1999). If the on-rate exceeds $10^6 M^{-1} s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### *2. Antibody Fragments*

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, *see* Hudson et al., Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see* also WO 93/16185; and U.S. Patent Nos. 5,571,894 and 5,587,458. For discussion of Fab and F(ab')$_2$ fragments comprising salvage receptor binding epitope residues and having increased *in vivo* half-life, *see* U.S. Patent No. 5,869,046.

Diabodies are antibody fragments with two antigen-binding sites that may be bivalent or bispecific. *See,* for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516 B1).

Antibody fragments can be made by various techniques, including but not limited to proteolytic digestion of an intact antibody as well as production by recombinant host cells (*e.g., E. coli* or phage), as described herein.

### *3. Chimeric and Humanized Antibodies*

In certain embodiments, an antibody provided herein is a chimeric antibody. Certain chimeric antibodies are described, *e.g.,* in U.S. Patent No. 4,816,567; and Morrison et al., Proc. Natl. Acad. Sci. USA, 81:6851-6855 (1984)). In one example, a chimeric antibody comprises a non-human variable region *(e.g.,* a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g*., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (*e.g.,* the antibody from which the HVR residues are derived), *e.g.,* to restore or improve antibody specificity or affinity.

Humanized antibodies and methods of making them are reviewed, *e.g*., in Almagro and Fransson, Front. Biosci. 13:1619-1633 (2008), and are further described, *e.g.,* in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al., J. Immunol. 151:2296 (1993)); framework regions derived from the consensus sequence of human antibodies of a particular subgroup of light or heavy chain variable regions (*see, e.g.,* Carter et al., Proc. Natl. Acad. Sci. USA, 89:4285 (1992); and Presta et al., J. Immunol., 151:2623 (1993)); human mature (somatically mutated) framework regions or human germline framework regions *(see, e.g.,* Almagro and Fransson, Front. Biosci. 13:1619-1633 (2008)); and framework regions derived from screening FR libraries (*see, e.g*., Baca et al., J. Biol. Chem. 272:10678-10684 (1997) and Rosok et al., J. Biol. Chem. 271:22611-22618 (1996)).

### *4. Human Antibodies*

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, *see* Lonberg, Nat. Biotech. 23:1117-1125 (2005). *See also*, *e.g.,* U.S. Patent Nos. 6,075,181 and 6,150,584 describing XENOMOUSE™ technology; U.S. Patent No. 5,770,429 describing HuMab® technology; U.S. Patent No. 7,041,870 describing K-M MOUSE® technology, and U.S. Patent Application Publication No. US 2007/0061900, describing VelociMouse® technology). Human variable regions from intact antibodies generated by such animals may be further modified, *e.g*., by combining with a different human constant region.

Human antibodies can also be made by hybridoma-based methods. Human myeloma and mouse-human heteromyeloma cell lines for the production of human monoclonal antibodies have been described. (*See, e.g.,* Kozbor J. Immunol., 133: 3001 (1984); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clin. Pharma., 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### *5. Library-Derived Antibodies*

Antibodies of the disclosure may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e.g.,* in Hoogenboom et al., in METHODS IN MOL. BIOL. 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in METHODS IN MOL. BIOL. 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (*e.g.,* from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### *6. Multispecific Antibodies*

In certain embodiments, an antibody provided herein is a multispecific antibody, *e.g.*, a bispecific antibody. Multispecific antibodies are monoclonal antibodies that have binding specificities for at least two different sites. In certain embodiments, one of the binding specificities is for wnt pathway polypeptide such as an R-spondin-translocation fusion polypeptide and the other is for any other antigen. In certain embodiments, bispecific antibodies may bind to two different epitopes of wnt pathway polypeptide such as an R-spondin-translocation fusion polypeptide. Bispecific antibodies may also be used to localize cytotoxic agents to cells which express wnt pathway polypeptide such as an R-spondin-translocation fusion polypeptide. Bispecific antibodies can be prepared as full length antibodies or antibody fragments.

Techniques for making multispecific antibodies include, but are not limited to, recombinant co-expression of two immunoglobulin heavy chain-light chain pairs having different specificities (*see* Milstein and Cuello, Nature 305: 537 (1983)), WO 93/08829, and Traunecker et al., EMBO J. 10: 3655 (1991)), and "knob-in-hole" engineering (*see, e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (*see, e.g.,* US Patent No. 4,676,980, and Brennan et al., Science, 229: 81 (1985)); using leucine zippers to produce bispecific antibodies (*see, e.g.,* Kostelny et al., J. Immunol., 148(5):1547-1553 (1992)); using "diabody" technology for making bispecific antibody fragments (*see, e.g.,* Hollinger et al., Proc. Natl. Acad. Sci. USA, 90:6444-6448 (1993)); and using single-chain Fv (sFv) dimers (*see, e.g.,* Gruber et al., J. Immunol., 152:5368 (1994)); and preparing trispecific antibodies as described, *e.g.,* in Tutt et al., J. Immunol. 147: 60 (1991).

Engineered antibodies with three or more functional antigen binding sites, including "Octopus antibodies," are also included herein (*see, e.g.,* US 2006/0025576).

The antibody or fragment herein also includes a "Dual Acting FAb" or "DAF" comprising an antigen binding site that binds to a wnt pathway polypeptide such as an R-spondin-translocation fusion polypeptide as well as another, different antigen (*see,* US 2008/0069820, for example).

### *7. Antibody Variants*

#### *a) Glycosylation variants*

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. *See, e.g.,* Wright et al., TIBTECH 15:26-32 (1997). The oligosaccharide may include various carbohydrates, *e.g.*, mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the disclosure may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (*e.g.*, complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e.*, between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al., J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al., Arch. Biochem. Biophys. 249:533-545 (1986); US 2003/0157108, Presta, L; and WO 2004/056312, Adams et al., especially at Example 11), and knockout cell lines, such as alpha-1,6-fucosyltransferase gene, FUT8, knockout CHO cells (*see, e.g.,* Yamane-Ohnuki et al., Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, *e.g.,* in which a biantennary oligosaccharide attached to the Fc region of the antibody is bisected by GlcNAc. Such antibody variants may have reduced fucosylation and/or improved ADCC function. Examples of such antibody variants are described, *e.g.,* in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g.,* in WO 1997/30087 (Patel et al.); WO 1998/58964 (Raju, S.); and WO 1999/22764 (Raju, S.).

***b) Fc region variants***

In certain embodiments, one or more amino acid modifications may be introduced into the Fc region of an antibody provided herein, thereby generating an Fc region variant. The Fc region variant may comprise a human Fc region sequence (*e.g.,* a human IgG1, IgG2, IgG3 or IgG4 Fc region) comprising an amino acid modification (*e.g.*, a substitution) at one or more amino acid positions.

In certain embodiments, the disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Nonlimiting examples of *in vitro* assays to assess ADCC activity of a molecule of interest is described in U.S. Patent No. 5,500,362 (*see, e.g.,* Hellstrom, I. et al., Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al., Proc. Natl. Acad. Sci. USA 95:652-656 (1998). C1q binding assays may also be carried out to confirm that the antibody is unable to bind C1q and hence lacks CDC activity. *See, e.g.,* C1q and C3c binding ELISA in WO 2006/029879 and WO 2005/100402. To assess complement activation, a CDC assay may be performed *(see,* for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art *(see, e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (*See, e.g.,* U.S. Patent No. 6,737,056; WO 2004/056312, and Shields et al., J. Biol. Chem. 9(2): 6591-6604 (2001).) In certain embodiments, an antibody variant comprises an Fc region with one or more amino acid substitutions which improve ADCC, *e.g.*, substitutions at positions 298, 333, and/or 334 of the Fc region (EU numbering of residues). In some embodiments, alterations are made in the Fc region that result in altered (*i.e.,* either improved or diminished) C1q binding and/or Complement Dependent Cytotoxicity (CDC), *e.g.,* as described in US Patent No. 6,194,551, WO 99/51642, and Idusogie et al., J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g.,* substitution of Fc region residue 434 (US Patent No. 7,371,826). *See also* Duncan & Winter, Nature 322:738-40 (1988); U.S. Patent No. 5,648,260; U.S. Patent No. 5,624,821; and WO 94/29351 concerning other examples of Fc region variants.

***c) Cysteine engineered antibody variants***

In certain embodiments, it may be desirable to create cysteine engineered antibodies, *e.g.*, "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, *e.g.,* in U.S. Patent No. 7,521,541.

***B. Immunoconjugates***

Further provided herein are immunoconjugates comprising an anti-wnt pathway antibody such as an R-spondin-translocation fusion polypeptide herein conjugated to one or more cytotoxic agents, such as chemotherapeutic agents or drugs, growth inhibitory agents, toxins (*e.g.*, protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (*see* Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $At^{211}$, $I^{131}$, $I^{125}$, $Y^{90}$, $Re^{186}$, $Re^{188}$, $Sm^{153}$ , $Bi^{212}$, $P^{32}$, $Pb^{212}$ and radioactive isotopes of Lu. When the radioconjugate is used for detection, it may comprise a radioactive atom for scintigraphic studies, for example $Tc^{99}$ or $I^{123}$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari el al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (*e.g.*, from Pierce Biotechnology, Inc., Rockford, IL., U.S.A).

### *C. Binding Polypeptides*

Provided herein are wnt pathway binding polypeptide antagonists for use as a wnt pathway antagonist in any of the methods described herein. Wnt pathway binding polypeptide antagonists are polypeptides that bind, preferably specifically, to a wnt pathway polypeptide.

In some embodiments of any of the wnt pathway binding polypeptide antagonists, the wnt pathway binding polypeptide antagonist is a chimeric polypeptide. In some embodiments, the wnt pathway binding polypeptide antagonist comprises (a) a Frizzled domain component, and (b) a Fc domain. For example, any wnt pathway antagonists described in U.S. Patent No. 7,947,277.

In some embodiments of any of the wnt pathway binding polypeptide antagonists, the wnt pathway binding polypeptide antagonist is a polypeptide that binds specifically to Dvl PDZ, wherein said polypeptide comprises a C-terminal region comprising a sequence with Gly at position -2, Trp or Tyr at position -1, Phe or Leu at position 0, and a hydrophobic or aromatic residue at position -3, wherein amino acid numbering is based on the C-terminal residue being in position 0. In some embodiments, position -6 is Trp. In some embodiments, position -1 is Trp. In some embodiments of any of the wnt pathway binding polypeptide antagonists, the wnt pathway binding polypeptide antagonist is a polypeptide that binds specifically to Dvl PDZ at a binding affinity of IC50=1.5 uM or better. In some embodiments, the polypeptide inhibits Dvl PDZ interaction with its endogenous binding partner. In some embodiments, the polypeptide inhibits endogenous Dvl-mediated Wnt signaling. In some embodiments, a polypeptide comprising a C-terminus consisting of KWYGWL (SEQ ID NO: 80). In some embodiments, the polypeptide comprises the amino acid sequence $X_1$-$X_2$-W-$X_3$-D-$X_4$-P, and wherein $X_1$ is L or V, $X_2$ is L, $X_3$ is S or T, and $X_4$ is I, F or L. In some embodiments, the polypeptide comprises the amino acid sequence GEIVLWSDIPG (SEQ ID NO:81). In some embodiments, the polypeptide is any polypeptide described in U.S. Patent No. 7,977,064 and/or 7,695,928.

In some embodiments of any of the wnt pathway binding polypeptide antagonists, the binding polypeptide binds WISP. In some embodiments, the WISP is WISP1, WISP2, and/or WISP3. In some embodiments, the polypeptide is any polypeptide described in U.S. Patent No. 6,387,657, 7,455,834, 7,732,567, 7,687,460, and/or 7,101,850 and/or U.S. Patent Application No. 2006/0292150.

In some embodiments of any of the wnt pathway binding polypeptide antagonists, the binding polypeptide binds a S100-like cytokine polypeptide. In some embodiments, the S100-like cytokine polypeptide is a S100-A14 polypeptide. In some embodiments, the polypeptide is any polypeptide described in U.S. Patent No. 7,566,536 and/or 7,005,499.

In some embodiments of any of the wnt pathway binding polypeptide antagonists, the wnt pathway binding polypeptide antagonist is a polypeptide that binds specifically to STRA6. In some embodiments, the polypeptide is any polypeptide described in U.S. Patent No. 7,173,115, 7,741,439, and/or 7,855,278.

In some embodiments of any of the wnt pathway binding polypeptide antagonists, the binding polypeptide binds R-spondin polypeptide. In some embodiment, the R-spondin polypeptide is RSPO1 polypeptide. In some embodiment, the R-spondin polypeptide is RSPO2 polypeptide. In some embodiment, the R-spondin polypeptide is RSPO3 polypeptide. In some embodiment, the R-spondin polypeptide is RSPO4 polypeptide.

In some embodiments of any of the binding polypeptides, the wnt pathway binding polypeptide antagonists bind to an R-spondin-translocation fusion polypeptide. In some embodiments, the binding polypeptide specifically bind an R-spondin-translocation fusion polypeptide, but do not substantially bind wild-type R-spondin and/or a second gene of the translocation. In some embodiments, the R-spondin-translocation fusion polypeptide is RSPO1-translocation fusion polypeptide. In some embodiments, the R-spondin-translocation fusion polypeptide is RSPO2-translocation fusion polypeptide. In some embodiments, the R-spondin-translocation fusion polypeptide is RSPO3-translocation fusion polypeptide. In some embodiments, the R-spondin-translocation fusion polypeptide is RSPO4-translocation fusion polypeptide. In some embodiments, the RSPO2-translocation fusion polypeptide comprises *EIF3E* and *RSPO2.* In some embodiments, the RSPO2 -translocation fusion polypeptide comprises *EIF3E* exon 1 and *RSPO2* exon 2. In some embodiments, the RSPO2-translocation fusion polypeptide comprises *EIF3E* exon 1 and *RSPO2* exon 3. In some embodiments, the RSPO2-translocation fusion polypeptide comprises SEQ ID NO:71. In some embodiments, the RSPO3-translocation fusion polypeptide comprises *PTPRK* and *RSPO3.* In some embodiments, the RSPO3-translocation fusion polypeptide comprises *PTPRK* exon 1 and *RSPO3* exon 2. In some embodiments, the RSPO3-translocation fusion polypeptide comprises *PTPRK* exon 7 and *RSPO3* exon 2. In some embodiments, the RSPO3-translocation fusion polypeptide comprises SEQ ID NO:72 and/or SEQ ID NO:73.

Binding polypeptides may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to a target, wnt pathway polypeptide, as described herein. Binding polypeptides may be identified without undue experimentation using well known techniques. In this regard, it is noted that techniques for screening polypeptide libraries for binding polypeptides that are capable of specifically binding to a polypeptide target are well known in the art (*see, e.g.,* U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al., Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al., (1990) Proc. Natl. Acad. Sci. USA, 87:6378; Lowman, H.B. et al., (1991) Biochemistry, 30:10832; Clackson, T. et al., (1991) Nature, 352: 624; Marks, J. D. et al., (1991), J. Mol. Biol., 222:581; Kang, A.S. et al., (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large polypeptide libraries to identify member(s) of those libraries which are capable of specifically binding to a target polypeptide, wnt pathway polypeptide. Phage display is a technique by which variant polypeptides are displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al., (1990) Proc. Natl. Acad. Sci. USA, 87:6378) or protein (Lowman, H.B. et al., (1991) Biochemistry, 30:10832; Clackson, T. et al., (1991) Nature, 352: 624; Marks, J. D. et al., (1991), J. Mol. Biol., 222:581; Kang, A.S. et al., (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Additional improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphylococcus aureus* protein A as an affinity tag has also been reported (Li et al., (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

### *D. Binding Small Molecules*

Provided herein are wnt pathway small molecule antagonists for use as a wnt pathway antagonist in any of the methods described herein. In some embodiments, the wnt pathway antagonist is a canonical wnt pathway antagonist. In some embodiments, the wnt pathway antagonist is a non-canonical wnt pathway antagonist.

In some embodiments of any of the small molecules, the wnt pathway small molecule antagonist is an R-spondin small molecule antagonist (*e.g.*, RSPO1, 2, 3, and/or 4 small molecule antagonist). In some embodiment, the R-spondin small molecule antagonist is RSPO1-translocation small molecule antagonist. In some embodiment, the R-spondin small molecule antagonist is RSPO2-translocation small molecule antagonist. In some embodiment, the R-spondin small molecule antagonist is RSPO3-translocation antagonist. In some embodiment, the R-spondin small molecule antagonist is RSPO4-translocation small molecule antagonist.

In some embodiments of any of the small molecules, the small molecule binds to an R-spondin-translocation fusion polypeptide. In some embodiments, small molecule specifically binds an R-spondin-translocation fusion polypeptide, but do not substantially bind wild-type R-spondin and/or a second gene of the translocation. In some embodiments, the R-spondin-translocation fusion polypeptide is RSPO1-translocation fusion polypeptide. In some embodiments, the R-spondin-translocation fusion polypeptide is RSPO2-translocation fusion polypeptide. In some embodiments, the R-spondin-translocation fusion polypeptide is RSPO3-translocation fusion polypeptide. In some embodiments, the R-spondin-translocation fusion polypeptide is RSPO4-translocation fusion polypeptide. In some embodiments, the RSPO2-translocation fusion polypeptide comprises *EIF3E* and *RSPO2.* In some embodiments, the RSPO2 -translocation fusion polypeptide comprises *EIF3E* exon 1 and *RSPO2* exon 2. In some embodiments, the RSPO2-translocation fusion polypeptide comprises *EIF3E* exon 1 and *RSPO2* exon 3. In some embodiments, the RSPO2-translocation fusion polypeptide comprises SEQ ID NO:71. In some embodiments, the RSPO3-translocation fusion polypeptide comprises *PTPRK* and *RSPO3.* In some embodiments, the RSPO3-translocation fusion polypeptide comprises *PTPRK* exon 1 and *RSPO3* exon 2. In some embodiments, the RSPO3-translocation fusion polypeptide comprises *PTPRK* exon 7 and *RSPO3* exon 2. In some embodiments, the RSPO3-translocation fusion polypeptide comprises SEQ ID NO:72 and/or SEQ ID NO:73.

Small molecules are preferably organic molecules other than binding polypeptides or antibodies as defined herein that bind, preferably specifically, to wnt pathway polypeptide as described herein. Organic small molecules may be identified and chemically synthesized using known methodology *(see, e.g.,* PCT Publication Nos. WO00/00823 and WO00/39585). Organic small molecules are usually less than about 2000 Daltons in size, alternatively less than about 1500, 750, 500, 250 or 200 Daltons in size, wherein such organic small molecules that are capable of binding, preferably specifically, to a polypeptide as described herein may be identified without undue experimentation using well known techniques. In this regard, it is noted that techniques for screening organic small molecule libraries for molecules that are capable of binding to a polypeptide target are well known in the art (*see, e.g.,* PCT Publication Nos. WO00/00823 and WO00/39585). Organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

### *E. Antagonist Polynucleotides*

Provided herein are wnt pathway polynucleotide antagonists for use as a wnt pathway antagonist in any of the methods described herein. The polynucleotide may be an antisense nucleic acid and/or a ribozyme. The antisense nucleic acids comprise a sequence complementary to at least a portion of an RNA transcript of a wnt pathway gene. However, absolute complementarity, although preferred, is not required. In some embodiments, the wnt pathway antagonist is a canonical wnt pathway antagonist. In some embodiments, the wnt pathway antagonist is a non-canonical wnt pathway antagonist. In some embodiments, wnt pathway polynucleotide is R-spondin. In some embodiments, the R-spondin is RSPO1. In some embodiments, the R-spondin is RSPO2. In some embodiments, the R-spondin is RSPO3. In some embodiments, the R-spondin is RSPO4. Examples of polynucleotide antagonists include those described in WO 2005/040418 such as TCCCATTTGCAAGGGTTGT (SEQ ID NO: 82) and/or AGCTGACTGTGATACCTGT(SEQ ID NO: 83).

In some embodiments of any of the polynucleotides, the polynucleotide binds to an R-spondin-translocation fusion polynucleotide. In some embodiments, polynucleotide specifically binds an R-spondin-translocation fusion polynucleotide, but do not substantially bind wild-type R-spondin and/or a second gene of the translocation. In some embodiments, the R-spondin-translocation fusion polynucleotide is RSPO1-translocation fusion polynucleotide. In some embodiments, the R-spondin-translocation fusion polynucleotide is RSPO2-translocation fusion polynucleotide. In some embodiments, the R-spondin-translocation fusion polynucleotide is RSPO3-translocation fusion polynucleotide. In some embodiments, the R-spondin-translocation fusion polynucleotide is RSPO4-translocation fusion polynucleotide. In some embodiments, the RSPO2-translocation fusion polynucleotide comprises *EIF3E* and *RSPO2.* In some embodiments, the RSPO2 -translocation fusion polynucleotide comprises *EIF3E* exon 1 and *RSPO2* exon 2. In some embodiments, the RSPO2-translocation fusion polynucleotide comprises *EIF3E* exon 1 and *RSPO2* exon 3. In some embodiments, the RSPO2-translocation fusion polynucleotide comprises SEQ ID NO:71. In some embodiments, the RSPO3-translocation fusion polynucleotide comprises *PTPRK* and *RSPO3.* In some embodiments, the RSPO3-translocation fusion polynucleotide comprises *PTPRK* exon 1 and *RSPO3* exon 2. In some embodiments, the RSPO3-translocation fusion polynucleotide comprises *PTPRK* exon 7 and *RSPO3* exon 2. In some embodiments, the RSPO3-translocation fusion polynucleotide comprises SEQ ID NO:72 and/or SEQ ID NO:73.

A sequence "complementary to at least a portion of an RNA," referred to herein, means a sequence having sufficient complementarity to be able to hybridize with the RNA, forming a stable duplex; in the case of double stranded wnt pathway antisense nucleic acids, a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an wnt pathway RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, *e.g.*, the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. *See generally,* Wagner, R., 1994, Nature 372:333-335. Thus, oligonucleotides complementary to either the 5'- or 3'-non-translated, noncoding regions of the wnt pathway gene, could be used in an antisense approach to inhibit translation of endogenous wnt pathway mRNA. Polynucleotides complementary to the 5' untranslated region of the mRNA should include the complement of the AUG start codon. Antisense polynucleotides complementary to mRNA coding regions are less efficient inhibitors of translation but could be used in accordance with the disclosure. Whether designed to hybridize to the 5'-, 3'- or coding region of wnt pathway mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

In one embodiment, the wnt pathway antisense nucleic acid of the disclosure is produced intracellularly by transcription from an exogenous sequence. For example, a vector or a portion thereof, is transcribed, producing an antisense nucleic acid (RNA) of the wnt pathway gene. Such a vector would contain a sequence encoding the wnt pathway antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others know in the art, used for replication and expression in vertebrate cells. Expression of the sequence encoding wnt pathway, or fragments thereof, can be by any promoter known in the art to act in vertebrate, preferably human cells. Such promoters can be inducible or constitutive. Such promoters include, but are not limited to, the SV40 early promoter region (Bernoist and Chambon, Nature 29:304-310 (1981), the promoter contained in the 3' long terminal repeat of Rous sarcoma virus (Yamamoto et al., Cell 22:787-797 (1980), the herpes thymidine promoter (Wagner et al., Proc. Natl. Acad. Sci. U.S.A. 78:1441-1445 (1981), the regulatory sequences of the metallothionein gene (Brinster et al., Nature 296:39-42 (1982)), etc.

### *F. Antibody and Binding Polypeptide Variants*

In certain embodiments, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into and/or substitutions of residues within the amino acid sequences of the antibody and/or binding polypeptide. Any combination of deletion, insertion, and substitution can be made to arrive at the final construct, provided that the final construct possesses the desired characteristics, *e.g.*, target-binding.

In certain embodiments, antibody variants and/or binding polypeptide variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody and/or binding polypeptide of interest and the products screened for a desired activity, *e.g.*, retained/improved antigen binding, decreased immunogenicity, or improved ADCC or CDC.

**TABLE 1**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.*, a humanized or human antibody). Generally, the resulting variant(s) selected for further study will have modifications (*e.g.*, improvements) in certain biological properties (*e.g.*, increased affinity, reduced immunogenicity) relative to the parent antibody and/or will have substantially retained certain biological properties of the parent antibody. An exemplary substitutional variant is an affinity matured antibody, which may be conveniently generated, *e.g.*, using phage display-based affinity maturation techniques such as those described herein. Briefly, one or more HVR residues are mutated and the variant antibodies displayed on phage and screened for a particular biological activity (*e.g.*, binding affinity).

Alterations (*e.g.,* substitutions) may be made in HVRs, *e.g.,* to improve antibody affinity. Such alterations may be made in HVR "hotspots," *i.e*., residues encoded by codons that undergo mutation at high frequency during the somatic maturation process (*see, e.g.,* Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, *e.g.,* in Hoogenboom et al., in METHODS IN MOL. BIOL. 178:1-37 (O'Brien et al., ed., Human Press, Totowa, NJ, (2001).) In some embodiments of affinity maturation, diversity is introduced into the variable genes chosen for maturation by any of a variety of methods (*e.g*., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.,* 4-6 residues at a time) are randomized. HVR residues involved in antigen binding may be specifically identified, *e.g*., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (*e.g*., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of the antibody and/or the binding polypeptide that may be targeted for mutagenesis is called "alanine scanning mutagenesis" as described by Cunningham and Wells (1989) Science, 244:1081-1085. In this method, a residue or group of target residues (*e.g*., charged residues such as arg, asp, his, lys, and glu) are identified and replaced by a neutral or negatively charged amino acid (*e.g*., alanine or poly alanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.,* for ADEPT) or a polypeptide which increases the serum half-life of the antibody.

### *G. Antibody and Binding Polypeptide Derivatives*

In certain embodiments, an antibody and/or binding polypeptide provided herein may be further modified to contain additional nonproteinaceous moieties that are known in the art and readily available. The moieties suitable for derivatization of the antibody and/or binding polypeptide include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer is attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions, etc.

In another embodiment, conjugates of an antibody and/or binding polypeptide to nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody and/or binding polypeptide-nonproteinaceous moiety are killed.

### *H. Recombinant Methods and Compositions*

Antibodies and/or binding polypeptides may be produced using recombinant methods and compositions, *e.g.*, as described in U.S. Patent No. 4,816,567. In one embodiment, isolated nucleic acid encoding an anti- wnt pathway antibody. Such nucleic acid may encode an amino acid sequence comprising the VL and/or an amino acid sequence comprising the VH of the antibody (*e.g.*, the light and/or heavy chains of the antibody). In a further embodiment, one or more vectors (*e.g.*, expression vectors) comprising such nucleic acid encoding the antibody and/or binding polypeptide are provided. In a further embodiment, a host cell comprising such nucleic acid is provided. In one such embodiment, a host cell comprises (*e.g.*, has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, *e.g.*, a Chinese Hamster Ovary (CHO) cell or lymphoid cell (*e.g.,* Y0, NS0, Sp20 cell). In one embodiment, a method of making an antibody such as an anti-wnt pathway antibody and/or binding polypeptide is provided, wherein the method comprises culturing a host cell comprising a nucleic acid encoding the antibody and/or binding polypeptide, as provided above, under conditions suitable for expression of the antibody and/or binding polypeptide, and optionally recovering the antibody and/or polypeptide from the host cell (or host cell culture medium).

For recombinant production of an antibody such as an anti-wnt pathway antibody and/or a binding polypeptide, nucleic acid encoding the antibody and/or the binding polypeptide, *e.g.,* as described above, is isolated and inserted into one or more vectors for further cloning and/or expression in a host cell. Such nucleic acid may be readily isolated and sequenced using conventional procedures (*e.g.*, by using oligonucleotide probes that are capable of binding specifically to genes encoding the heavy and light chains of the antibody).

Suitable host cells for cloning or expression of vectors include prokaryotic or eukaryotic cells described herein. For example, antibodies may be produced in bacteria, in particular when glycosylation and Fc effector function are not needed. For expression of antibody fragments and polypeptides in bacteria, *see, e.g.,* U.S. Patent Nos. 5,648,237, 5,789,199, and 5,840,523. (*See* also Charlton, METHODS IN MOL. BIOL., Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006).

Suitable host cells for the expression of glycosylated antibody and/or glycosylated binding polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See, e.g.,* US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR$^-$ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production and/or binding polypeptide production, *see, e.g.,* Yazaki and Wu, METHODS IN MOL. BIOL., Vol. 248 (B.K.C. Lo, ed., Humana Press, Totowa, NJ), pp. 255-268 (2003).

While the description relates primarily to production of antibodies and/or binding polypeptides by culturing cells transformed or transfected with a vector containing antibody- and binding polypeptide-encoding nucleic acid. It is, of course, contemplated that alternative methods, which are well known in the art, may be employed to prepare antibodies and/or binding polypeptides. For instance, the appropriate amino acid sequence, or portions thereof, may be produced by direct peptide synthesis using solid-phase techniques [*see, e.g.,* Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the antibody and/or binding polypeptide may be chemically synthesized separately and combined using chemical or enzymatic methods to produce the desired antibody and/or binding polypeptide.

### *IV. Methods of Screening and/or Identifying Wnt Pathway Antagonists With Desired Function*

Techniques for generating wnt pathway antagonists such as antibodies, binding polypeptides, and/or small molecules have been described above. Additional wnt pathway antagonists such as anti-wnt pathway antibodies, binding polypeptides, small molecules, and/or polynucleotides provided herein may be identified, screened for, or characterized for their physical/chemical properties and/or biological activities by various assays known in the art.

Provided herein are methods of screening for and/or identifying a wnt pathway antagonist which inhibits wnt pathway signaling, induces cancer cell cycle arrest, inhibits cancer cell proliferation, and/or promotes cancer cell death said method comprising: (a) contacting (i) a cancer cell, cancer tissue, and/or cancer sample, wherein the cancer cell, cancer tissue, and/or cancer comprises one or more biomarkers, and (ii) a reference cancer cell, reference cancer tissue, and/or reference cancer sample with a wnt pathway candidate antagonist, (b) determining the level of wnt pathway signaling, distribution of cell cycle stage, level of cell proliferation, and/or level of cancer cell death, whereby decreased level of wnt pathway signaling, a difference in distribution of cell cycle stage, decreased level of cell proliferation, and/or increased level of cancer cell death between the cancer cell, cancer tissue, and/or cancer sample, wherein the cancer cell, cancer tissue, and/or cancer comprises one or more biomarkers, and reference cancer cell, reference cancer tissue, and/or reference cancer sample identifies the wnt pathway candidate antagonist as an wnt pathway antagonist which inhibits wnt pathway signaling, induces cancer cell cycle arrest, inhibits cancer cell proliferation, and/or promotes cancer cell cancer death. In some embodiments, the wnt pathway antagonist is an R-spondin antagonist.

Further provided herein are methods of screening for and/or identifying a wnt pathway antagonist which inhibits wnt pathway signaling, induces cancer cell cycle arrest, inhibits cancer cell proliferation, and/or promotes cancer cell death said method comprising: (a) contacting a cancer cell, cancer tissue, and/or cancer sample, wherein the cancer cell, cancer tissue, and/or cancer comprises one or more biomarkers with a wnt pathway candidate antagonist, (b) determining the level of wnt pathway signaling, distribution of cell cycle stage, level of cell proliferation, and/or level of cancer cell death to the cancer cell, cancer tissue, and/or cancer sample in the absence of the wnt pathway candidate antagonist, whereby decreased level of wnt pathway signaling, a difference in distribution of cell cycle stage, decreased level of cell proliferation, and/or increased level of cancer cell death between the cancer cell, cancer tissue, and/or cancer sample in the presence of the wnt pathway candidate antagonist and the cancer cell, cancer tissue, and/or cancer sample in the absence of the wnt pathway candidate antagonist identifies the wnt pathway candidate antagonist as an wnt pathway antagonist which inhibits wnt pathway signaling, induces cancer cell cycle arrest, inhibits cancer cell proliferation, and/or promotes cancer cell cancer death. In some embodiments, the wnt pathway antagonist is an R-spondin antagonist.

In some embodiments of any of the methods, the one or more biomarkers is a translocation (*e.g.*, rearrangement and/or fusion) of one or more genes listed in Table 9. In some embodiments of any of the methods, the translocation (*e.g.*, rearrangement and/or fusion) is an R-spondin translocation (*e.g.*, rearrangement and/or fusion). In some embodiments, the R-spondin translocation (*e.g.*, rearrangement and/or fusion) is a RSPO1 translocation (*e.g.*, rearrangement and/or fusion). In some embodiments, the R-spondin translocation (*e.g.*, rearrangement and/or fusion) is a RSPO2 translocation (*e.g.,* rearrangement and/or fusion). In some embodiments, the RSPO2 translocation (*e.g.*, rearrangement and/or fusion) comprises *EIF3E* and *RSPO2.* In some embodiments, the RSPO2 translocation (*e.g.,* rearrangement and/or fusion) comprises *EIF3E* exon 1 and *RSPO2* exon 2. In some embodiments, the RSPO2 translocation (*e.g.,* rearrangement and/or fusion) comprises *EIF3E* exon 1 and *RSPO2* exon 3. In some embodiments, the RSPO2 translocation (*e.g.*, rearrangement and/or fusion) comprises SEQ ID NO:71 In some embodiments, the RSPO2 translocation (*e.g.*, rearrangement and/or fusion) is detectable by primers which include SEQ ID NO:12, 41, and/or 42. In some embodiments, the RSPO2 translocation (*e.g.,* rearrangement and/or fusion) is driven by the *EIF3E* promoter. In some embodiments, the RSPO2 translocation (*e.g.,* rearrangement and/or fusion) is driven by the *RSPO2* promoter. In some embodiments, the R-spondin translocation (*e.g.*, rearrangement and/or fusion) is a RSPO3 translocation (*e.g.,* rearrangement and/or fusion). In some embodiments, the RSPO3 translocation (*e.g.*, rearrangement and/or fusion) comprises *PTPRK* and *RSPO3.* In some embodiments, the RSPO3 translocation (*e.g.,* rearrangement and/or fusion) comprises *PTPRK* exon 1 and *RSPO3* exon 2. In some embodiments, the RSPO3 translocation (*e.g.*, rearrangement and/or fusion) comprises *PTPRK* exon 7 and *RSPO3* exon 2. In some embodiments, the RSPO3 translocation (*e.g.*, rearrangement and/or fusion) comprises SEQ ID NO:72 and/or SEQ ID NO:73. In some embodiments, the RSPO3 translocation (*e.g.*, rearrangement and/or fusion) is detectable by primers which include SEQ ID NO:13, 14, 43, and/or 44. In some embodiments, the RSPO3 translocation (*e.g.,* rearrangement and/or fusion) is driven by the *PTPRK* promoter. In some embodiments, the RSPO3 translocation (*e.g.*, rearrangement and/or fusion) is driven by the *RSPO3* promoter. In some embodiments, the RSPO3 translocation (*e.g.,* rearrangement and/or fusion) comprises the PTPRK secretion signal sequence (and/or does not comprise the RSPO3 secretion signal sequence). In some embodiments, the R-spondin translocation (*e.g.*, rearrangement and/or fusion) is a RSPO4 translocation (e.g., rearrangement and/or fusion). In some embodiments, the R-spondin translocation (*e.g.,* rearrangement and/or fusion) results in elevated expression levels of R-spondin (*e.g.,* compared to a reference without the R-spondin translocation. In some embodiments, the one or more biomarkers is an R-spondin translocation (*e.g.*, rearrangement and/or fusion) and *KRAS* and/or *BRAF.* In some embodiments, the presence of one or more biomarkers is presence of an R-spondin translocation (*e.g.*, rearrangement and/or fusion) and a variation (*e.g.*, polymorphism or mutation) *KRAS* and/or *BRAF*. In some embodiments, the presence of one or more biomarkers is presence of an R-spondin translocation (*e.g.*, rearrangement and/or fusion) and the absence of one or more biomarkers is absence of a variation (*e.g.,* polymorphism or mutation) *CTNNB1* and/or *APC.*

Methods of determining the level of wnt pathway signaling are known in the art and are described in the Examples herein. In some embodiments, the levels of wnt pathway signaling are determined using a luciferase reporter assay as described in the Examples. In some embodiments, the wnt pathway antagonist inhibits wnt pathway signaling by reducing the level of wnt pathway signaling by about any of 10, 20, 30, 40, 50, 60, 70, 80, 90, or 100%.

The growth inhibitory effects of a wnt pathway antagonist described herein may be assessed by methods known in the art, *e.g.,* using cells which express wnt pathway either endogenously or following transfection with the respective gene(s). For example, appropriate tumor cell lines, and wnt pathway polypeptide-transfected cells may be treated with a wnt pathway antagonist described herein at various concentrations for a few days (*e.g.,* 2-7) days and stained with crystal violet or MTT or analyzed by some other colorimetric assay. Another method of measuring proliferation would be by comparing $^{3}$H-thymidine uptake by the cells treated in the presence or absence an antibody, binding polypeptide, small molecule, and/or polynucleotides of the disclosure. After treatment, the cells are harvested and the amount of radioactivity incorporated into the DNA quantitated in a scintillation counter. Appropriate positive controls include treatment of a selected cell line with a growth inhibitory antibody known to inhibit growth of that cell line. Growth inhibition of tumor cells in vivo can be determined in various ways known in the art.

Methods of determining the distribution of cell cycle stage, level of cell proliferation, and/or level of cell death are known in the art. In some embodiments, cancer cell cycle arrest is arrest in G1.

In some embodiments, the wnt pathway antagonist will inhibit cancer cell proliferation of the cancer cell, cancer tissue, or cancer sample in vitro or in vivo by about 25-100% compared to the untreated cancer cell, cancer tissue, or cancer sample, more preferably, by about 30-100%, and even more preferably by about 50-100% or about 70-100%. For example, growth inhibition can be measured at a wnt pathway antagonist concentration of about 0.5 to about 30 μg/ml or about 0.5 nM to about 200 nM in cell culture, where the growth inhibition is determined 1-10 days after exposure of the tumor cells to the wnt pathway candidate antagonist. The wnt pathway antagonist is growth inhibitory in vivo if administration of the wnt pathway candidate antagonist at about 1 μg/kg to about 100 mg/kg body weight results in reduction in tumor size or reduction of tumor cell proliferation within about 5 days to 3 months from the first administration of the wnt pathway candidate antagonist, preferably within about 5 to 30 days.

To select for a wnt pathway antagonists which induces cancer cell death, loss of membrane integrity as indicated by, *e.g.*, propidium iodide (PI), trypan blue or 7AAD uptake may be assessed relative to a reference. A PI uptake assay can be performed in the absence of complement and immune effector cells. wnt pathway-expressing tumor cells are incubated with medium alone or medium containing the appropriate a wnt pathway antagonist. The cells are incubated for a 3-day time period. Following each treatment, cells are washed and aliquoted into 35 mm strainer-capped 12 x 75 tubes (1 ml per tube, 3 tubes per treatment group) for removal of cell clumps. Tubes then receive PI (10 μg/ml). Samples may be analyzed using a FACSCAN® flow cytometer and FACSCONVERT® CellQuest software (Becton Dickinson). Those wnt pathway antagonists that induce statistically significant levels of cell death as determined by PI uptake may be selected as cell death-inducing antibodies, binding polypeptides, small molecules, and/or polynucleotides.

To screen for wnt pathway antagonists which bind to an epitope on or interact with a polypeptide bound by an antibody of interest, a routine cross-blocking assay such as that described in Antibodies, A Laboratory Manual, Cold Spring Harbor Laboratory, Ed Harlow and David Lane (1988), can be performed. This assay can be used to determine if a candidate wnt pathway antagonist binds the same site or epitope as a known antibody. Alternatively, or additionally, epitope mapping can be performed by methods known in the art. For example, the antibody and/or binding polypeptide sequence can be mutagenized such as by alanine scanning, to identify contact residues. The mutant antibody is initially tested for binding with polyclonal antibody and/or binding polypeptide to ensure proper folding. In a different method, peptides corresponding to different regions of a polypeptide can be used in competition assays with the candidate antibodies and/or polypeptides or with a candidate antibody and/or binding polypeptide and an antibody with a characterized or known epitope.

In some embodiments of any of the methods of screening and/or identifying, the wnt pathway candidate antagonist is an antibody, binding polypeptide, small molecule, or polynucleotide. In some embodiments, the wnt pathway candidate antagonist is an antibody. In some embodiments, the wnt pathway antagonist (*e.g.*, R-spondin-translocation antagonist) antagonist is a small molecule.

In one aspect, a wnt pathway antagonist is tested for its antigen binding activity, *e.g.*, by known methods such as ELISA, Western blot, etc.

### *V. Pharmaceutical Formulations*

Pharmaceutical formulations of a wnt pathway antagonist as described herein are prepared by mixing such antibody having the desired degree of purity with one or more optional pharmaceutically acceptable carriers (REMINGTON'S PHARMA. SCI. 16th edition, Osol, A. Ed. (1980)), in the form of lyophilized formulations or aqueous solutions. In some embodiments, the wnt pathway antagonist is a small molecule, an antibody, binding polypeptide, and/or polynucleotide. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized formulations are described in US Patent No. 6,267,958. Aqueous antibody formulations include those described in US Patent No. 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in REMINGTON'S PHARMA. SCI. 16th edition, Osol, A. Ed. (1980).

Sustained-release preparations may be prepared. Suitable examples of sustained-release preparations include semipermeable matrices of solid hydrophobic polymers containing the wnt pathway antagonist, which matrices are in the form of shaped articles, *e.g.*, films, or microcapsules.

The formulations to be used for *in vivo* administration are generally sterile. Sterility may be readily accomplished, *e.g.*, by filtration through sterile filtration membranes.

### *VI. Articles of Manufacture*

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a wnt pathway antagonist (*e.g.,* R-spondin antagonist, *e.g.,* R-spondin-translocation antagonist) described herein. The label or package insert indicates that the composition is used for treating the condition of choice. Moreover, the article of manufacture may comprise (a) a first container with a composition contained therein, wherein the composition comprises a wnt pathway antagonist (*e.g.,* R-spondin antagonist, *e.g.,* R-spondin-translocation antagonist); and (b) a second container with a composition contained therein, wherein the composition comprises a further cytotoxic or otherwise therapeutic agent.

In some embodiments, the article of manufacture comprises a container, a label on said container, and a composition contained within said container; wherein the composition includes one or more reagents (*e.g.*, primary antibodies that bind to one or more biomarkers or probes and/or primers to one or more of the biomarkers described herein), the label on the container indicating that the composition can be used to evaluate the presence of one or more biomarkers in a sample, and instructions for using the reagents for evaluating the presence of one or more biomarkers in a sample. The article of manufacture can further comprise a set of instructions and materials for preparing the sample and utilizing the reagents. In some embodiments, the article of manufacture may include reagents such as both a primary and secondary antibody, wherein the secondary antibody is conjugated to a label, *e.g.,* an enzymatic label. In some embodiments, the article of manufacture one or more probes and/or primers to one or more of the biomarkers described herein.

In some embodiments of any of the articles of manufacture, the one or more biomarkers comprises a translocation (*e.g.*, rearrangement and/or fusion) of one or more genes listed in Table 9. In some embodiments of any of the articles of manufacture, the translocation (*e.g.*, rearrangement and/or fusion) is an R-spondin translocation (*e.g.*, rearrangement and/or fusion). In some embodiments, the R-spondin translocation (*e.g.*, rearrangement and/or fusion) is a RSPO1 translocation (*e.g.*, rearrangement and/or fusion). In some embodiments, the R-spondin translocation (*e.g.*, rearrangement and/or fusion) is a RSPO2 translocation (*e.g.*, rearrangement and/or fusion). In some embodiments, the RSPO2 translocation (*e.g.*, rearrangement and/or fusion) comprises *EIF3E* and *RSPO2.* In some embodiments, the RSPO2 translocation (*e.g.,* rearrangement and/or fusion) comprises *EIF3E* exon 1 and *RSPO2* exon 2. In some embodiments, the RSPO2 translocation (*e.g.,* rearrangement and/or fusion) comprises *EIF3E* exon 1 and *RSPO2* exon 3. In some embodiments, the RSPO2 translocation (*e.g.,* rearrangement and/or fusion) comprises SEQ ID NO:71. In some embodiments, the RSPO2 translocation (*e.g.*, rearrangement and/or fusion) is detectable by primers which include SEQ ID NO:12, 41, and/or 42. In some embodiments, the RSPO2 translocation (*e.g.,* rearrangement and/or fusion) is driven by the *EIF3E* promoter. In some embodiments, the RSPO2 translocation (*e.g.*, rearrangement and/or fusion) is driven by the *RSPO2* promoter. In some embodiments, the R-spondin translocation (*e.g.,* rearrangement and/or fusion) is a RSPO3 translocation (*e.g.*, rearrangement and/or fusion). In some embodiments, the RSPO3 translocation (*e.g.,* rearrangement and/or fusion) comprises *PTPRK* and *RSPO3.* In some embodiments, the RSPO3 translocation (*e.g.,* rearrangement and/or fusion) comprises *PTPRK* exon 1 and *RSPO3* exon 2. In some embodiments, the RSPO3 translocation (*e.g.,* rearrangement and/or fusion) comprises *PTPRK* exon 7 and *RSPO3* exon 2. In some embodiments, the RSPO3 translocation (*e.g.,* rearrangement and/or fusion) comprises SEQ ID NO:72 and/or SEQ ID NO:73. In some embodiments, the RSPO3 translocation (*e.g.*, rearrangement and/or fusion) is detectable by primers which include SEQ ID NO:13, 14, 43, and/or 44. In some embodiments, the RSPO3 translocation (*e.g.*, rearrangement and/or fusion) is driven by the *PTPRK* promoter. In some embodiments, the RSPO3 translocation (*e.g.*, rearrangement and/or fusion) is driven by the *RSPO3* promoter. In some embodiments, the RSPO3 translocation (*e.g.,* rearrangement and/or fusion) comprises the PTPRK secretion signal sequence (and/or does not comprise the RSPO3 secretion signal sequence). In some embodiments, the R-spondin translocation (*e.g.*, rearrangement and/or fusion) is a RSPO4 translocation (*e.g.*, rearrangement and/or fusion). In some embodiments, the R-spondin translocation (*e.g.*, rearrangement and/or fusion) results in elevated expression levels of R-spondin (*e.g.*, compared to a reference without the R-spondin translocation. In some embodiments, the one or more biomarkers is an R-spondin translocation (*e.g.*, rearrangement and/or fusion) and *KRAS* and/or *BRAF*. In some embodiments, the presence of one or more biomarkers is presence of an R-spondin translocation (*e.g.,* rearrangement and/or fusion) and a variation (*e.g.,* polymorphism or mutation) *KRAS* and/or *BRAF*. In some embodiments, the presence of one or more biomarkers is presence of an R-spondin translocation (*e.g.*, rearrangement and/or fusion) and the absence of one or more biomarkers is absence of a variation (*e.g.,* polymorphism or mutation) *CTNNB1* and/or *APC.*

In some embodiments of any of the articles of manufacture, the articles of manufacture comprise primers. In some embodiments, the primers are any of SEQ ID NO:12, 13, 14, 41, 42, 43, and/or 44.

In some embodiments of any of the article of manufacture, the wnt pathway antagonist (*e.g.*, R-spondin-translocation antagonist) is an antibody, binding polypeptide, small molecule, or polynucleotide. In some embodiments, the wnt pathway antagonist (*e.g.*, R-spondin-translocation antagonist) is a small molecule. In some embodiments, the wnt pathway antagonist (*e.g.*, R-spondin-translocation antagonist) is an antibody. In some embodiments, the antibody is a monoclonal antibody. In some embodiments, the antibody is a human, humanized, or chimeric antibody. In some embodiments, the antibody is an antibody fragment and the antibody fragment binds wnt pathway polypeptide (*e.g.*, R-spondin-translocation fusion polypeptide).

The article of manufacture in this embodiment of the disclosure may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Other optional components in the article of manufacture include one or more buffers (*e.g.*, block buffer, wash buffer, substrate buffer, etc), other reagents such as substrate (*e.g.*, chromogen) which is chemically altered by an enzymatic label, epitope retrieval solution, control samples (positive and/or negative controls), control slide(s) etc.

It is understood that any of the above articles of manufacture may include an immunoconjugate described herein in place of or in addition to a wnt pathway antagonist.

## EXAMPLES

The following are examples of methods and compositions provided herein. ***Materials and Methods for Examples***

*Samples, DNA and RNA preps and MSI testing*

*Patient-ma**tched fresh frozen primary colon tu*** mors and normal tissue samples were obtained from commercial sources subjected to genomic analysis described below. All tumor and normal tissue were subject to pathology review. From a set of 90 samples 74 tumor pairs were identified for further analysis. Tumor DNA and RNA were extracted using Qiagen AllPrep DNA/RNA kit (Qiagen, CA). Tumor samples were assessed for microsatellite instability using an MSI detection kit (Promega, WI).

*Exome capture and sequencing*

Seventy two tumor samples and matched normal tissues were analyzed by exome sequencing. Exome capture was performed using SeqCap EZ human exome library v2.0 (Nimblegen, WI) consisting of 2.1 million empirically optimized long oligonucleotides that target ~30,000 coding genes (~300,000 exons, total size 36.5 Mb). The library was capable of capturing a total of 44.1 Mb of the genome, including genes and exons represented in RefSeq (Jan 2010), CCDS (Sept 2009) and miRBase (v.14, Sept 2009). Exome capture libraries generated were sequenced on HiSeq 2000 (Illumina, CA). One lane of 2x75 bp paired-end data was collected for each sample.

*RNA-seq*

RNA from 68 colon tumor and matched normal sample pairs was used to generate RNA-seq libraries using TruSeq RNA Sample Preparation kit (Illumina, CA). RNA-seq libraries were multiplex (two per lane) and sequenced on HiSeq 2000 as per manufacturer's recommendation (Illumina, CA). ~30 million 2 x 75bp paired-end sequencing reads per sample were generated.

*Sequence data processing*

All short read data was evaluated for quality control using the Bioconductor ShortRead package. Morgan, M. et al., Bioinformatics 25, 2607-2608 (2009). To confirm that all samples were identified correctly, all exome and RNA-seq data variants that overlapped with the Illuman 2.5 M array data were compared and checked for consistency. An all by all germline variant comparison was also done between all samples to check that all pairs were correctly matched between the tumor and normal and correspondingly did not match with any other patient pair above a cutoff of 90%.

*Variant calling*

Sequencing reads were mapped to UCSC human genome (GRCh37/hg19) using BWA software set to default parameters. Li, H. & Durbin, R. Bioinformatics 25, 1754-1760 (2009). Local realignment, duplicate marking and raw variant calling were performed as described previously. DePristo, M. A. et al., Nat. Genet. 43, 491-498 (2011). Known germline variations represented in dbSNP Build 131 (Sherry, S. T. et al., Nucleic Acids Res 29, 308-311 (2001)), but not represented in COSMIC (Forbes, S. A. et al., Nucleic Acids Res. 38, D652-657 (2010)), were additionally filtered out. In addition variants that were present in both the tumor and normal samples were removed as germline variations. Remaining variations present in the tumor sample, but absent in the matched normal were predicted to be somatic. Predicted somatic variations were additionally filtered to include only positions with a minimum of 10x coverage in both the tumor and matched normal as well as an observed variant allele frequency of <3% in the matched normal and a significant difference in variant allele counts using Fisher's exact test. To evaluate the performance of this algorithm, 807 protein-altering variants were randomly selected and validated them using Sequenom (San Diego, CA) nucleic acid technology as described previously. Kan, Z. et al., Nature 466, 869-873 (2010). Of these, 93% (753) validated as cancer specific with the invalidated variants being equally split between not being seen in the tumor and also being seen in the adjacent normal (germline). Indels were called using the GATK Indel Genotyper Version 2 which reads both the tumor and normal BAM file for a given pair. DePristo, M. A. et al., Nat. Genet. 43, 491-498 (2011).

In order to identify variants grossly violating a binomial assumption, or variant calls affected by a specific mapper, Sequenom validated variants were additionally included using the following algorithm. Reads were mapped to UCSC human genome (GRCh37/hg19) using GSNAP. Wu, T. D. & Nacu, S. Bioinformatics 26, 873-881 (2010). Variants seen at least twice at a given position and greater than 10% allele frequency were selected. These variants were additionally filtered for significant biases in strand and position using Fisher's exact test. In addition variants that did not have adequate coverage in the adjacent normal as determined as at least a 1% chance of being missed using a beta-binomial distribution at a normal allele frequency of 12.5% were excluded. All novel protein-altering variants included in the second algorithm were validated by Sequenom, which resulted in a total of 515 additional variants. The effect of all non-synonymous somatic mutations on gene function was predicted using SIFT (Ng, P. C. & Henikoff, S. Genome Res 12, 436-446 (2002)) and PolyPhen (Ramensky, V., Bork, P. & Sunyaev, S. Nucleic Acids Res 30, 3894-3900 (2002)). All variants were annotated using Ensembl (release 59, www.ensembl.org).

*Validation of somatic mutations and indels*

Single base pair extension followed by nucleic acid mass spectrometry (Sequenom, CA) was used as described previously to validate the predicted somatic mutations. Tumor and matched normal DNA was whole genome amplified and using the REPLI-g Whole Genome Amplification Midi Kit (Qiagen, CA) and cleaned up as per manufacturer's recommendations and used. Variants found as expected in the tumor but absent in the normal were designated somatic. Those that were present in both tumor and normal were classified as germline. Variants that could not be validated in tumor or normal were designated as failed. For indel validation, primers for PCR were designed that will generate an amplicon of -300 bp that contained the indel region. The region was PCR amplified in both tumor and matched normal sample using Phusion (NEB, MA) as per manufacturer's instructions. The PCR fragments were then purified on a gel an isolated the relevant bands and Sanger sequenced them. The sequencing trace files were analyzed using Mutation Surveyor (SoftGenetics, PA). Indels that were present in the tumor and absent in the normal were designated somatic and are reported in Table 3.

*Mutational significance*

Mutational significance of genes was evaluated using a previously described method. Briefly this method can identify genes that have statistically significant more protein-altering mutations than what would be expected based on a calculated background mutation rate. The background mutation rate was calculated for six different nucleotide mutation categories (A,C,G,T,CG1,CG2) in which there was sufficient coverage (≥10x) in both the tumor and matched normal sample. A nonsynonymous to synonymous ratio, $r_i$, was calculated using a simulation of mutating all protein coding nucleotides and seeing if the resulting change would result in a synonymous or nonsynonymous change. The background mutation rate, $f_i$, was determined by multiplying the number of synonymous somatic variants by $r_i$ and normalizing by the total number of protein-coding nucleotides. The number of expected mutations for a given gene was determined as the number of protein-coding bases multiplied by $f_i$ and integrated across all mutation categories. A p-value was calculated using a Poisson probability function given the expected and observed number of mutations for each gene. P values were corrected for multiple testing using the Benjamini Hochberg method and the resulting q-values were converted to q-scores by taking the negative log10 of the q-values. Given that different mutation rates existed for the MSI and MSS samples, qscores were calculated separately for each with the two hypermutated samples being removed completely. In order to not underestimate the background mutation rates, the seven samples with less than 50% tumor content were excluded from the analysis. Pathway mutational significance was also calculated as previously described, with the exception that the BioCara Pathway database used used which was downloaded as part of MSigDB (Subramanian A. et al., Proc. of the Natl Acad. Of Sci. USA 102, 15545-15550 (2005)).

*Whole genome sequencing and analysis*

Paired-end DNA-Seq reads were aligned to GRCh37 using BWA. Further processing of the alignments to obtain mutation calls was similar to the exome sequencing analysis using the GATK pipeline. Copy-number was calculated by computing the number of reads in 10 kb non-overlapping bins and taking the ratio tumor/normal of these counts. Chromosomal breakpoints were predicted using breakdancer. Chen, K. et al., Nat. Methods 6, 677-681 (2009). Genome plots were created using Circos (Krzywinski, M. et al., Genome Res. 19, 1639-1459 (2009)).

*RNA-seq data analysis*

RNA-Seq reads were aligned to the human genome version GRCh37 using GSNAP (Wu, T.D. &Nacu, S. Bioinformatics 26, 873-881 (2010). Expression counts per gene were obtained by counting the number of reads aligning concordant and uniquely to each gene locus as defined by CCDS. The gene counts were then normalized for library size and subsequently variance stabilized using the DESeq Bioconductor software package. Anders, S. & Huber, W. Genome Biology 11, R106 (2010). Differential gene expression was computed by pairwise t-tests on the variance stabilized counts followed by correction for multiple testing using the Benjamini & Hochberg method.

*SNP array data generation and analysis*

Illumina HumanOmni2.5_4v1 arrays were used to assay 74 colon tumors and matched normals for genotype, DNA copy and LOH at -2.5 million SNP positions. These samples all passed our quality control metrics for sample identity and data quality (*see* below). A subset of 2295239 high-quality SNPs was selected for all analyses.

After making modifications to permit use with Illumina array data, the PICNIC (Greenman, C. D. et al., Biostatistics 11, 164-175 (2010)) algorithm was applied to estimate total copy number and allele-specific copy number / LOH. Modification included replacement of the segment initialization component with the CBS algorithm (Venkatraman, E. S. & Olshen, A. B. Bioinformatics 23, 657-663 (2007)), and adjustment of the prior distribution for background raw copy number signal (abjusted mean of 0.7393 and a standard deviation of 0.05). For the preprocessing required by PICNIC's hidden Markov model (HMM), a Bayesiaan model to estimate cluster centroids for each SNP. For SNP *k* and genotype *g*, observed data in normal sample were modeled as following a bivariate Gaussian distribution. Cluster centers for the three diploid genotypes were modeled jointly by a 6-dimensional Gaussian distribution with mean treated as a hyperparameter and set empirically based on a training set of 156 normal samples. Cluster center and within-genotype covariance matrices were modeled as inverse Wishart with scale matrix hyperparameters also set empirically and with degrees of freedom manually tuned to provide satisfactory results for a wide range of probe behavior and minor allele frequencies. Finally, signal for SNP *k* (for the A and B alleles separately) was transformed with a non-linear function: $y = {}_{k}x^{k} + {}_{k}$ with parameters selected based on the posterior distributions computed above.

Sample identity was verified using genotype concordance between all samples. Pairs of tumors from the same patient were expected to have > 90% concordance and all other pairs were expected to have < 80% concordance. Samples failing those criteria were excluded from all analyses. Following modified PICNIC, the quality of the overall HMM fit was assessed by measuring the root mean squared error (RMSE) between the raw and HMM-fitted value for each SNP. Samples with and RMSE >1.5 were excluded from all analyses. Finally to account for two commonly observed artifacts, fitted copy number values were set to "NA" for singletons with fitted copy number 0 or when the observed and fitted means differed by more tha 2 for regions of inferred copy gain.

*Recurrent DNA copy number gain and loss*

Genomic regions with recurrent DNA copy gain and loss were identified using GISTIC, version 2.0. Mermel, C. H. et al., Genome Biology 12, R41 (2011). Segmented integer total copy number values obtained from PICNIC, *c*, were converted to $\log_2$ ratio values, *y*, as $y = \log_2(c + 0.1) - 1$. Cutoffs of +/-0.2 were used to categorize $\log_2$ ratio values as gain or loss, respectively. A minimum segment length of 20 SNPs and a $\log_2$ ratio "cap" value of 3 were used.

*Fusion detection and validation*

Putative fusions were identified using a computational pipeline developed called GSTRUCT-fusions. The pipeline was based on a generate-and-test strategy that is fundamentally similar to methodology reported previously for finding readthrough fusions. Nacu, S. et al., BMC Med Genomics 4, 11 (2011). Paired-end reads were aligned using our alignment program GSNAP. Nacu, S. et al., BMC Med Genomics 4, 11 (2011). GSNAP has the ability to detect splices representing translocations, inversions, and other distant fusions within a single read end.

These distant splices provided one set of candidate fusions for the subsequent testing stage. The other set of candidate fusions derived from unpaired unique alignments, where each end of the paired-end read aligned uniquely to a different chromosome, and also from paired, but discordant unique alignments, where each end aligned uniquely to the same chromosome, but with an apparent genomic distance that exceeded 200,000 bp or with genomic orientations that suggested an inversion or scrambling event.

Candidate fusions were then filtered against known transcripts from RefSeq, aligned to the genome using GMAP. Wu, T. D. & Watanabe, C. K. Bioinformatics 21, 1859-1875 (2005). Both fragments flanking a distant splice, or both ends of an unpaired or discordant paired-end alignment, were required to map to known exon regions. This filtering step eliminated approximately 90% of the candidates. Candidate inversions and deletions were further eliminated that suggested rearrangements of the same gene, as well as apparent readthrough fusion events involving adjacent genes in the genome, which our previous research indicated were likely to have a transcriptional rather than genomic origin.

For the remaining candidate fusion events, artificial exon-exon junctions consisting of the exons distal to the supported donor exon and the exons proximal to the supported acceptor exon were constructed. The exons included in the proximal and distal computations were limited so that the cumulative length along each gene was within an estimated maximum insert length of 200 bp. As a control, all exon-exon junctions consisting of combinations of exons within the same gene were constructed for all genes contributing to a candidate fusion event.

In the testing stage of our pipeline, we constructed a genomic index from the artificial exon-exon junctions and controls using the GMAP_BUILD program included as part of the GMAP and GSNAP package. This genomic index and the GSNAP program with splice detection turned off were used to realign the original read ends that were not concordant to the genome. Reads were extracted that aligned to an intergenic junction corresponding to a candidate fusion, but not to a control intragenic junction.

The results of the re-alignment were filtered to require that each candidate fusion have at least one read with an overhang of 20 bp. Each candidate fusion was also required to have at least 10 supporting reads. For each remaining candidate fusion, the two component genes were aligned against each other using GMAP and eliminated the fusion if the alignment had any region containing 60 matches in a window of 75 bp. The exon-exon junction were also aligned against each of the component genes using GMAP and eliminated the fusion if the alignment had coverage greater than 90% of the junction and identity greater than 95%.

Validation of gene fusions was done using reverse transcription (RT)-PCR approach using both colon tumor and matched normal samples. 500 ng of total RNA was reverse transcribed to cDNA with a High Capacity cDNA Reverse Transcription kit (Life Technologies, CA) following manufacturer's instructions. 50 ng of cDNA was amplified in a 25 μl reaction containing 400 pM of each primer, 300 μM of each deoxynucleoside triphosphates and 2.5 units of LongAmp Taq DNA polymerase (New England Biolabs, MA). PCR was performed with an initial denaturation at 95°C for 3 minutes followed by 35 cycles of 95°C for 10 seconds, 56°C for 1 minute and 68°C for 30 seconds and a final extension step at 68°C for 10 minutes. 3 μl of PCR product was run on 1.2% agarose gel to identify samples containing gene fusion. Specific PCR products were purified with either a QIAquick PCR Purification kit or Gel Extraction kit (Qiagen, CA). The purified DNA was either sequenced directly with PCR primers specific to each fusion or cloned into TOPO cloning vector pCR2.1 (Life Technologies, CA) prior to Sanger sequencing. The clones were sequenced using Sanger sequencing on a ABI3730xl (Life Technologies, CA) as per manufacturer instructions. The Sanger sequencing trace files were analyzed using Sequencher (Gene Cordes Corp., MI).

*RSPO fusion activity testing*

Eukaryotic expression plasmid pRK5E driving the expression of c-terminal FLAG tag EIF3E, PTPKR (amino acids 1-387), RSPO2, RSPO3, EIF3E(e1)-RSPO2(e2), PTPRK(e1)-RSPO3(e2). PTPRK(e7)-RSPO3(e2) was generated using standard PCR and cloning strategies.

*Cells, conditioned media, immunoprecipitation and Western blot*

HEK 293T, human embryonic kidney cells, were maintained in DMEM supplemented with 10% FBS. For expression analysis and condition media generation 3 x $10^5$ HEK29T cells were plated in 6-well plates in 1.5 ml DMEM containing 10%FBS. Cells were transfected with 1 μg of DNA using Figure 6 (Roche) according to the manufacturer's instructions. Media was conditioned for 48 hours, collected, centrifuged, and used to stimulate the luciferase reporter assay (final concentration 0.1- 0.4X). For expression analysis, media was collected, centrifuged to remove debris and used for immunoprecipitation.

*Luciferase reporter assays*

HEK 293T cells were plated at a density of 50,000 cells/ml in 90 μl of media containing 2.5% FBS per well of a 96-well plate. After 24 hours, cells were transfected using Figure 6 according to manufacturer's instructions (Roche, CA) with the following DNA per well: 0.04 μg TOPbrite Firefly reporter (Nature Chem. Biol. 5, 217 - 219 (2009)), 0.02 μg pRL SV40-Renilla (Promega, WI) and 0.01 μg of the appropriate R-spondin or control constructs. Cells were stimulated with 25 μl of either fresh or conditioned media containing 10% FBS with or without rmWnt3a (20-100 ng/ml (final), R&D Systems, MN). Following 24 hours stimulation, 50 μl of media was removed and replaced with Dual-Glo luciferase detection reagents (Promega, WI) according to manufacturer's instructions. An Envision Luminometer (Perkin-Elmer, MA) was used to detect luminescence. To control for transfection efficiency, Firefly luciferase levels were normalized to Renilla luciferase levels to generate the measure of relative luciferase units (RLU). Experimental data was presented as mean ± SD from three independent wells.

*Immunoprecipitation and Western blot*

To confirm that the RSPO wild type and RSPO fusion proteins were secreted, FLAG tagged proteins were immunoprecipitated from the media using anti-FLAG-M2 antibody coupled beads (Sigma, MO), boiled in SDS-PAGE loading buffer, resolved on a 4-20% SDS-PAGE (Invitrogen, Carlsbad, CA) and transferred onto a nitrocellulose membrane. RSPO and other FLAG tagged proteins expressed in cells were detected from cell lysates using western blot as described before (Bijay p85 paper). Briefly, immunoprecipitated proteins and proteins from cell lysates were detected by Western blot using FLAG-HRP-conjugated antibody and chemiluminescences Super signal West Dura chemiluminescence detection substrate (Thermo Fisher Scientific, IL).

***Example 1-CRC Mutation Profile***

Identifying and understanding changes in cancer genomes is essential for the development of targeted therapeutics. In these examples, a systematically analysis of over 70 pairs of primary human colon cancers was undertaken by applying next generation sequencing to characterize their exomes, transcirptomes and copy number alterations. 36,303 protein altering somatic changes were identified that include several new recurrent mutations in Wnt pathway genes like *TCF12* and *TCF7L2*, chromatin remodeling proteins such as *TET2* and *TET3* and receptor tyrosine kinases including *ERBB3.* The analysis for significant cancer genes identified 18 candidates, including cell cycle checkpoint kinase ATM. The copy number and RNA-seq data analysis identified amplifications and corresponding overexpression of *IGF2* in a subset of colon tumors. Further, using RNA-seq data multiple fusion transcripts were identified including recurrent gene fusions of the R-spondin genes *RSPO2* and *RSPO3*, occurring in 10% of the samples. The RSPO fusion proteins were demonstrated to be biologically active and potentiate Wnt signaling. The *RSPO* fusions are mutually exclusive with *APC* mutations indicating that they likely play a role in activating Wnt signaling and tumorigenesis. The R-spondin gene fusions and several other gene mutations identified in these examples provide new opportunities for therapeutic intervention in colon cancer.

74 primary colon tumors and their matched adjacent normal samples were characterized. Whole-exome sequencing for 72 (15 MSI and 57 MSS) of the 74 colon tumor and adjacent normal sample pairs to assess the mutational spectra was performed. These 74 tumor/normal pairs were also analyzed on Illumina 2.5M array to assess chromosomal copy number changes. RNA-seq data for 68 tumor/normal pairs was also obtained. Finally, the genome of an MSI and MSS tumor/normal pair at 30x coverage from this set of samples was sequenced and analyzed.

Table 2 - Somatic mutations

| GeneName | Mutation | Ref | Position_cDNA | Var | GeneName | Mutation | Ref | Position_cDNA | Var | GeneName | Mutation | Ref | Position_cDNA | Var | GeneName | Mutation | Ref | Position_cDNA | Var |
|---|---|---|---|---|---|---|---|---|---|---|---|---|---|---|---|---|---|---|---|
| A1BG | A137T | C | 471 | T | ACSM1 | R133Q | C | 466 | T | AGL | R1098C | C | 3767 | T | ANKHD1 | R1224W | C | 3794 | T |
| A1BG | R345H | C | 1096 | T | ACSM2A | D431N | G | 1405 | A | AGMAT | L195P | A | 727 | G | ANKHD1 | S2352L | C | 7179 | T |
| A1BG | P487H | G | 1522 | T | ACSM2A | T249S | A | 859 | T | AGPAT2 | R121W | G | 427 | A | ANKHD1 | G62S | G | 308 | A |
| A1BG | E339D | C | 1079 | A | ACSM2A | T491M | C | 1586 | T | AGPAT2 | R90C | G | 334 | A | ANKHD1 | Y1156* | T | 3592 | G |
| A1BG | D187Y | C | 621 | A | ACSM2A | D396Y | G | 1300 | T | AGPAT6 | R223* | C | 1606 | T | ANKHD1 | S1996N | G | 6111 | A |
| A1CF | A391V | G | 1217 | A | ACSM2B | P416A | G | 1399 | C | AGPAT6 | I31T | T | 1031 | C | ANKHD1 | P1091Q | C | 3396 | A |
| A1CF | N287S | T | 905 | C | ACSM3 | V123I | G | 554 | A | AGPAT6 | A411V | C | 2171 | T | ANKHD1 | R1871H | G | 5736 | A |
| A2M | R945Q | C | 3142 | T | ACSM4 | R99Q | G | 313 | A | AGPHD1 | A267T | G | 912 | A | ANKHD1 | P2202L | C | 6729 | T |
| A2M | S1026N | C | 3385 | T | ACSM4 | D331Y | G | 1008 | T | AGPS | D180V | A | 685 | T | ANKHD1 | D1301G | A | 4026 | G |
| A2M | A1151P | C | 3759 | G | ACSM4 | E530D | G | 1607 | T | AGPS | K143T | A | 574 | C | ANKHD1 | - | T | 0 | A |
| A2M | G852R | C | 2862 | T | ACSM4 | K531T | A | 1609 | C | AGPS | A276V | C | 973 | T | ANKIB1 | E504K | G | 1886 | A |
| A2M | A88S | C | 570 | A | ACSM5 | D449V | A | 1493 | T | AGR2 | P126H | G | 419 | T | ANKIB1 | P291S | C | 1247 | T |
| A2M | T1209A | T | 3933 | C | ACSM5 | A226P | G | 823 | C | AGR3 | E89* | C | 336 | A | ANKIB1 | A714V | C | 2517 | T |
| A2M | R1297H | C | 4198 | T | ACSM5 | Q148R | A | 590 | G | AGRN | S330R | C | 1040 | A | ANKIB1 | A735V | C | 2580 | T |
| A2M | K664N | C | 2300 | A | ACSS1 | R537* | G | 1689 | A | AGRN | - | G | 0 | A | ANKK1 | R43C | C | 221 | T |
| A2M | H358Q | G | 1382 | T | ACSS1 | L497P | A | 1570 | G | AGRN | R1308C | C | 3972 | T | ANKK1 | L606M | C | 1910 | A |
| A2M | A90T | C | 576 | T | ACSS1 | A315P | C | 1023 | G | AGRN | A1506V | C | 4567 | T | ANKK1 | N200S | A | 693 | G |
| A2M | R598C | G | 2100 | A | ACSS3 | K606R | A | 1908 | G | AGRN | R339H | G | 1066 | A | ANKLE1 | R415W | C | 1519 | T |
| A2ML1 | S840Y | C | 2550 | A | ACSS3 | P26L | C | 168 | T | AGRN | C540R | T | 1668 | C | ANKLE2 | R659* | G | 2042 | A |
| A2ML1 | V924E | T | 2802 | A | ACSS3 | L294R | T | 972 | G | AGT | E423A | T | 1483 | G | ANKLE2 | I253S | A | 825 | C |
| A2ML1 | W737L | G | 2241 | T | ACTA1 | R314H | C | 1044 | T | AGT | D156H | C | 681 | G | ANKMY1 | V541M | C | 1760 | T |
| A2ML1 | T523A | A | 1598 | G | ACTA2 | R97H | C | 356 | T | AGTPBP1 | R314W | G | 940 | A | ANKMY1 | R20H | C | 198 | T |
| A2ML1 | L1202F | G | 3637 | T | ACTA2 | E109D | C | 393 | A | AGTPBP1 | R597I | C | 1790 | A | ANKMY1 | D514Y | C | 1679 | A |
| A2ML1 | F355V | T | 1094 | G | ACTB | V139A | A | 608 | G | AGTR1 | R137C | C | 800 | T | ANKMY2 | E440D | C | 1564 | A |
| A4GALT | P305L | G | 1404 | A | ACTB | V287M | C | 1051 | T | AGTRAP | H153R | A | 517 | G | ANKRD1 | N83H | T | 496 | G |
| A4GALT | R147W | G | 929 | A | ACTBL2 | V371I | C | 1213 | T | AGXT | I46F | A | 523 | T | ANKRD10 | T37A | T | 244 | C |
| A4GALT | A335D | G | 1494 | T | ACTBL2 | R40* | G | 220 | A | AGXT | A294V | C | 1268 | T | ANKRD10 | E332D | C | 1131 | A |
| A4GALT | F186C | A | 1047 | C | ACTL6A | L157F | G | 684 | T | AGXT2 | L342V | G | 1225 | C | ANKRD11 | A1840V | G | 5980 | A |
| AAAS | L18I | G | 215 | T | ACTL6B | H248Y | G | 849 | A | AGXT2 | T79M | G | 437 | A | ANKRD11 | R440* | G | 1779 | A |
| AAAS | R230* | G | 851 | A | ACTL6B | R338C | G | 1119 | A | AGXT2 | - | T | 0 | C | ANKRD11 | A1288T | C | 4323 | T |
| AACS | S84* | C | 457 | A | ACTL6B | K86N | C | 365 | A | AGXT2 | F108L | A | 525 | C | ANKRD11 | E2522K | C | 8025 | T |
| AACS | G592R | G | 1980 | A | ACTL7A | A349T | G | 1102 | A | AGXT2L1 | R468K | C | 1558 | T | ANKRD11 | S1698N | C | 5554 | T |
| AADAC | L173F | C | 643 | T | ACTL7B | A410G | G | 1294 | C | AGXT2L1 | V236A | A | 862 | G | ANKRD12 | R1308* | C | 4162 | T |

| | | | | | | | | | | | | | | | | | | | |
|---|---|---|---|---|---|---|---|---|---|---|---|---|---|---|---|---|---|---|---|
| AADAC | L344I | C | 1156 | A | ACTL7B | D247N | C | 804 | T | AGXT2L2 | R195H | C | 819 | T | ANKRD12 | R1791H | G | 5612 | A |
| AADACL2 | H111R | A | 452 | G | ACTL8 | A168T | G | 718 | A | AHCTF1 | N216T | T | 647 | G | ANKRD12 | A1210V | C | 3869 | T |
| AADACL2 | F15C | T | 164 | G | ACTL8 | V292A | T | 1091 | C | AHCTF1 | T1947A | T | 5839 | C | ANKRD12 | L306S | T | 1157 | C |
| AADACL2 | L302I | C | 1024 | A | ACTL9 | F350L | G | 1171 | T | AHCTF1 | R619C | G | 1855 | A | ANKRD13A | R174H | G | 780 | A |
| AADACL3 | D172Y | G | 719 | T | ACTL9 | S89L | G | 387 | A | AHCTF1 | L709I | G | 2125 | T | ANKRD13B | T57M | C | 324 | T |
| AADACL3 | R95H | G | 489 | A | ACTL9 | R190C | G | 689 | A | AHCYL2 | A488V | C | 1517 | T | ANKRD13B | G302S | G | 1058 | A |
| AADACL3 | R338S | A | 1219 | C | ACTL9 | R171H | C | 633 | T | AHDC1 | A709V | G | 2749 | A | ANKRD13B | R606W | C | 1970 | T |
| AADACL4 | V79M | G | 235 | A | ACTL9 | R400W | G | 1319 | A | AHDC1 | H1455R | T | 4987 | C | ANKRD13C | T452M | G | 1669 | A |
| AADAT | K282N | T | 963 | G | ACTN1 | N473S | T | 1601 | C | AHDC1 | Y1002C | T | 3628 | C | ANKRD13C | Q202L | T | 919 | A |
| AADAT | M306V | T | 1033 | C | ACTN1 | K198T | T | 776 | G | AHDC1 | R743L | C | 2851 | A | ANKRD13D | A223V | C | 1582 | T |
| AADAT | F52C | A | 272 | C | ACTN2 | E520A | A | 1725 | C | AHI1 | C952* | A | 3155 | T | ANKRD13D | D431N | G | 2205 | A |
| AAK1 | L665I | G | 2370 | T | ACTN2 | A882T | G | 2810 | A | AHNAK | G1840W | C | 5818 | A | ANKRD13D | A389T | G | 2079 | A |
| AAK1 | R141H | C | 799 | T | ACTN2 | L104W | T | 477 | G | AHNAK | L4394S | A | 13481 | G | ANKRD13D | R278H | G | 1747 | A |
| AAK1 | A592V | G | 2152 | A | ACTN2 | A257V | C | 936 | T | AHNAK | M1857V | T | 5869 | C | ANKRD13D | - | G | 0 | T |
| AAMP | S20R | T | 150 | G | ACTN2 | R506I | G | 1683 | T | AHNAK | G90W | C | 568 | A | ANKRD16 | P74H | G | 765 | T |
| AARS | I382N | A | 1252 | T | ACTN3 | Q450H | G | 1466 | C | AHNAK | L2241P | A | 7022 | G | ANKRD17 | L1237M | G | 3826 | T |
| AARS | R206W | G | 723 | A | ACTN4 | R339W | C | 1091 | T | AHNAK | E3455K | C | 10663 | T | ANKRD17 | K1505N | T | 4632 | G |
| AARS | A671S | C | 2118 | A | ACTR1B | R199H | C | 813 | T | AHNAK | K730N | T | 2490 | G | ANKRD17 | S792N | C | 2492 | T |
| AARS2 | R480W | G | 1441 | A | ACTR1B | F57L | G | 388 | T | AHNAK | K1652T | T | 5255 | G | ANKRD18A | A108V | G | 698 | A |
| AARS2 | A933S | C | 2800 | A | ACTR2 | Y330H | T | 1061 | C | AHNAK | P4127A | G | 12679 | C | ANKRD18A | A759V | G | 2651 | A |
| AARS2 | T530A | T | 1591 | C | ACTR3B | G356R | G | 1200 | A | AHNAK | F5768L | G | 17604 | T | ANKRD18A | T85A | T | 628 | C |
| AARS2 | - | C | 0 | A | ACTR3B | V377M | G | 1263 | A | AHNAK | K5022T | T | 15365 | G | ANKRD18A | N723D | T | 2542 | C |
| AARSD1 | N333K | A | 1217 | T | ACTR3C | E102K | C | 315 | T | AHNAK | F4073C | A | 12518 | C | ANKRD18B | R556H | G | 1763 | A |
| AASDHPP T | K151* | A | 604 | T | ACTR5 | R450Q | G | 1386 | A | AHNAK | V4051A | A | 12452 | G | ANKRD18B | E452* | G | 1450 | T |
| AASS | R267C | G | 895 | A | ACTR5 | R518W | C | 1589 | T | AHNAK | I3214S | A | 9941 | C | ANKRD2 | R127W | C | 646 | T |
| AASS | M689I | C | 2163 | A | ACTR5 | S389N | G | 1203 | A | AHNAK | A3200V | G | 9899 | A | ANKRD2 | - | A | 0 | G |
| AASS | E425* | C | 1369 | A | ACTR5 | R12H | G | 72 | A | AHNAK | K2141E | T | 6721 | C | ANKRD24 | H451Y | C | 1351 | T |
| AASS | G361R | C | 1177 | T | ACTR6 | R332L | G | 1760 | T | AHNAK | E1443* | C | 4627 | A | ANKRD24 | V1061M | G | 3181 | A |
| AASS | L133R | A | 494 | C | ACTR8 | A4G | G | 112 | C | AHNAK2 | G2891S | C | 8791 | T | ANKRD24 | S316G | A | 946 | G |
| AASS | G158R | C | 568 | T | ACTRT1 | G302R | C | 1096 | T | AHNAK2 | N5651S | T | 17072 | C | ANKRD24 | T723M | C | 2168 | T |
| AATF | G148D | G | 694 | A | ACTRT1 | V38I | C | 304 | T | AHNAK2 | V1186A | A | 3677 | G | ANKRD24 | - | G | 0 | T |
| AATK | H747R | T | 2265 | C | ACTRT2 | Y56C | A | 372 | G | AHNAK2 | V4034M | C | 12220 | T | ANKRD24 | L1222I | C | 3664 | A |
| AATK | Q362P | T | 1110 | G | ACVR1B | R444* | C | 1372 | T | AHNAK2 | M3891T | A | 11792 | G | ANKRD26 | R802* | G | 2576 | A |
| AATK | Y372F | T | 1140 | A | ACVR1C | R234C | G | 1061 | A | AHNAK2 | S2207T | A | 6739 | T | ANKRD26 | Q336R | T | 1179 | C |
| AATK | R485H | C | 1479 | T | ACVR2A | I488V | A | 2098 | G | AHNAK2 | A3792T | C | 11494 | T | ANKRD26 | M407V | T | 1391 | C |
| AATK | D464N | C | 1415 | T | ACVR2B | E50K | G | 620 | A | AHNAK2 | D4773G | T | 14438 | C | ANKRD26 | A908T | C | 2894 | T |
| ABAT | A226V | C | 865 | T | ACVR2B | K434N | G | 1774 | T | AHNAK2 | P3227S | G | 9799 | A | ANKRD26 | K910Q | T | 2900 | G |
| ABCA1 | R230H | C | 1084 | T | ACVR2B | V290A | T | 1341 | C | AHNAK2 | E5680G | T | 17159 | C | ANKRD26 | V532G | A | 1767 | C |
| ABCA1 | S824L | G | 2866 | A | ACVR2B | E286K | G | 1328 | A | AHNAK2 | G3250D | C | 9869 | T | ANKRD26 | F5C | A | 186 | C |

| | | | | | | | | | | | | | | | | | | | |
|---|---|---|---|---|---|---|---|---|---|---|---|---|---|---|---|---|---|---|---|
| ABCA1 | R1839H | C | 5911 | T | ACY3 | R159W | G | 646 | A | AHNAK2 | S4966Y | G | 15017 | T | ANKRD27 | C88R | A | 418 | G |
| ABCA1 | S689I | C | 2461 | A | ADAD1 | R508Q | G | 1708 | A | AHNAK2 | F4963C | A | 15008 | C | ANKRD27 | - | C | 0 | T |
| ABCA1 | Y1126C | T | 3772 | C | ADAD1 | T424A | A | 1455 | G | AHNAK2 | L3729V | A | 11305 | C | ANKRD28 | R401H | C | 1202 | T |
| ABCA1 | L932M | G | 3189 | T | ADAD1 | Y139C | A | 601 | G | AHNAK2 | G2494R | C | 7600 | T | ANKRD28 | R401C | G | 1201 | A |
| ABCA1 | A1010V | G | 3424 | A | ADAD1 | S11L | C | 217 | T | AHR | V660M | G | 2621 | A | ANKRD28 | R208C | G | 622 | A |
| ABCA1 | R2030Q | C | 6484 | T | ADAD1 | S346Y | C | 1222 | A | AHR | M348V | A | 1685 | G | ANKRD28 | I681S | A | 2042 | C |
| ABCA10 | E1168K | C | 4381 | T | ADAD1 | K365N | A | 1280 | C | AHRR | R72H | G | 259 | A | ANKRD28 | R208C | G | 622 | A |
| ABCA10 | E399* | C | 2074 | A | ADAD2 | A48V | C | 236 | T | AHSG | V128A | T | 459 | C | ANKRD28 | R171* | G | 511 | A |
| ABCA10 | E1443* | C | 5206 | A | ADAD2 | S62L | C | 278 | T | AHSP | R71W | C | 314 | T | ANKRD30A | T306M | C | 1016 | T |
| ABCA10 | T583I | G | 2627 | A | ADAD2 | G595W | G | 1876 | T | AHSP | D9Y | G | 128 | T | ANKRD30A | K468E | A | 1501 | G |
| ABCA11P | R341I | C | 1071 | A | ADAM10 | G500E | C | 1943 | T | AICDA | R128W | G | 461 | A | ANKRD30A | N454H | A | 1459 | C |
| ABCA11P | G607D | C | 1869 | T | ADAM10 | K714N | T | 2586 | G | AIFM2 | G231D | C | 710 | T | ANKRD30A | H1402N | C | 4303 | A |
| ABCA11P | I762L | T | 2333 | G | ADAM11 | R311W | C | 931 | T | AIFM3 | V337I | G | 1249 | A | ANKRD31 | N1060T | T | 3371 | G |
| ABCA11P | K621I | T | 1911 | A | ADAM11 | R284C | C | 850 | T | AIFM3 | T226M | C | 917 | T | ANKRD31 | E490D | C | 1662 | A |
| ABCA11P | E570D | C | 1759 | A | ADAM12 | Y310H | A | 1238 | G | AIM1 | R1527* | C | 5066 | T | ANKRD31 | G1211D | C | 3824 | T |
| ABCA12 | V1753A | A | 5478 | G | ADAM12 | H871R | T | 2922 | C | AIM1 | R1587* | C | 5246 | T | ANKRD32 | P443H | C | 1747 | A |
| ABCA12 | S119N | C | 576 | T | ADAM12 | R449C | G | 1655 | A | AIM1 | Y925C | A | 3261 | G | ANKRD32 | L335S | T | 1423 | C |
| ABCA12 | I736S | A | 2427 | C | ADAM12 | D399G | T | 1506 | C | AIM1 | A73S | G | 704 | T | ANKRD32 | E268* | G | 1221 | T |
| ABCA12 | V1305G | A | 4134 | C | ADAM17 | E406* | C | 1399 | A | AIM1L | D336G | T | 1006 | C | ANKRD32 | E319* | G | 1374 | T |
| ABCA12 | T1982I | G | 6165 | A | ADAM17 | R725H | C | 2357 | T | AIM1L | E524K | C | 1569 | T | ANKRD32 | T334A | A | 1419 | G |
| ABCA12 | R1299Q | C | 4116 | T | ADAM18 | K44N | G | 174 | T | AIM2 | E147* | C | 728 | A | ANKRD33B | A431V | C | 1377 | T |
| ABCA12 | A2568V | G | 7923 | A | ADAM18 | R543W | C | 1669 | T | AIMP1 | R73* | C | 258 | T | ANKRD33B | A168V | C | 588 | T |
| ABCA12 | K508N | T | 1744 | A | ADAM18 | K409T | A | 1268 | C | AIMP2 | T314M | C | 1060 | T | ANKRD33B | R425Q | G | 1359 | A |
| ABCA12 | A2405S | C | 7433 | A | ADAM19 | E179K | C | 599 | T | AIMP2 | R65C | C | 312 | T | ANKRD34A | T420M | C | 2552 | T |
| ABCA12 | A835V | G | 2724 | A | ADAM19 | S851P | A | 2615 | G | AIP | R323W | C | 1092 | T | ANKRD34A | T79M | C | 1529 | T |
| ABCA12 | E1679G | T | 5256 | C | ADAM19 | G136* | C | 470 | A | AIP | Y261H | T | 906 | C | ANKRD34B | R362I | C | 1758 | A |
| ABCA12 | R248I | C | 963 | A | ADAM2 | K441E | T | 1396 | C | AIPL1 | V269M | C | 866 | T | ANKRD34B | R24H | C | 744 | T |
| ABCA13 | R3162Q | G | 9509 | A | ADAM21P1 | A676T | G | 2026 | A | AJAP1 | A218T | G | 1346 | A | ANKRD34C | E45K | G | 133 | A |
| ABCA13 | R4217C | C | 12673 | T | ADAM21P1 | F448Y | T | 1343 | A | AJAP1 | N387T | A | 1854 | C | ANKRD34C | K367N | A | 1101 | C |
| ABCA13 | V5028A | T | 15107 | C | ADAM22 | M212V | A | 634 | G | AK1 | K147E | T | 535 | C | ANKRD35 | A512D | C | 1622 | A |
| ABCA13 | V3662I | G | 11008 | A | ADAM23 | G413* | G | 1565 | T | AK2 | D112N | C | 498 | T | ANKRD36 | C17* | C | 239 | A |
| ABCA13 | R3489W | C | 10489 | T | ADAM23 | I794M | A | 2710 | G | AK5 | G146D | G | 700 | A | ANKRD36 | K978N | A | 3122 | C |
| ABCA13 | D4133A | A | 12422 | C | ADAM23 | R292C | C | 1202 | T | AK5 | - | G | 0 | T | ANKRD36B | T689M | G | 2245 | A |
| ABCA13 | E117D | G | 375 | T | ADAM23 | W441* | G | 1651 | A | AK5 | R13M | G | 301 | T | ANKRD36B | E239* | C | 894 | A |
| ABCA13 | K1214T | A | 3665 | C | ADAM29 | E146* | G | 1025 | T | AK7 | R640C | C | 1962 | T | ANKRD40 | D99E | A | 567 | T |
| ABCA13 | K1671N | G | 5037 | T | ADAM29 | Q731H | G | 2782 | T | AK7 | R681I | G | 2086 | T | ANKRD42 | R558C | C | 1672 | T |

| | | | | | | | | | | | | | | | | | | | |
|---|---|---|---|---|---|---|---|---|---|---|---|---|---|---|---|---|---|---|---|
| ABCA13 | K3471N | G | 10437 | T | ADAM29 | C446Y | G | 1926 | A | AKAP1 | L53W | T | 158 | G | ANKRD42 | L617P | T | 1850 | C |
| ABCA13 | R3497Q | G | 10514 | A | ADAM29 | V69A | T | 795 | C | AKAP11 | S589P | T | 1940 | C | ANKRD43 | D377N | G | 1410 | A |
| ABCA13 | A4123T | G | 12391 | A | ADAM29 | K537N | A | 2200 | C | AKAP11 | R1608G | A | 4997 | G | ANKRD44 | P562S | G | 1684 | A |
| ABCA13 | K4706N | G | 14142 | T | ADAM30 | V481I | C | 1600 | T | AKAP11 | K629Q | A | 2060 | C | ANKRD44 | A837V | G | 2510 | A |
| ABCA13 | H5017R | A | 15074 | G | ADAM30 | F167V | A | 658 | C | AKAP11 | C837G | T | 2684 | G | ANKRD45 | V64I | C | 204 | T |
| ABCA13 | Q1918H | G | 5778 | T | ADAM32 | P452L | C | 1600 | T | AKAP11 | K1227T | A | 3855 | C | ANKRD46 | R126* | G | 555 | A |
| ABCA2 | R2103S | G | 6357 | T | ADAM32 | D332A | A | 1240 | C | AKAP12 | E147K | G | 679 | A | ANKRD49 | S43G | A | 266 | G |
| ABCA2 | P1895L | G | 5734 | A | ADAM32 | R511I | G | 1777 | T | AKAP12 | S850P | T | 2788 | C | ANKRD5 | R186C | C | 957 | T |
| ABCA2 | T1656M | G | 5017 | A | ADAM32 | W710C | G | 2375 | T | AKAP12 | L243P | T | 968 | C | ANKRD5 | E94* | G | 681 | T |
| ABCA3 | R1355Q | C | 4590 | T | ADAM9 | - | G | 0 | A | AKAP12 | E164K | G | 730 | A | ANKRD5 | S248L | C | 1144 | T |
| ABCA3 | R1236C | G | 4232 | A | ADAM9 | R474* | C | 1498 | T | AKAP12 | R814Q | G | 2681 | A | ANKRD50 | R324C | G | 2008 | A |
| ABCA3 | R1039H | C | 3642 | T | ADAM9 | T593M | C | 1856 | T | AKAP12 | K1734E | A | 5440 | G | ANKRD50 | C36Y | C | 1145 | T |
| ABCA3 | G887C | C | 3185 | A | ADAM9 | R725I | G | 2252 | T | AKAP12 | R671H | G | 2252 | A | ANKRD50 | R362* | G | 2122 | A |
| ABCA3 | R170C | G | 1034 | A | ADAMTS1 | R551I | C | 2107 | A | AKAP13 | M1278I | G | 4004 | A | ANKRD50 | K32E | T | 1132 | C |
| ABCA3 | S975* | G | 3450 | T | ADAMTS1 | R52C | G | 609 | A | AKAP13 | Y33N | T | 267 | A | ANKRD50 | Q1230R | T | 4727 | C |
| ABCA4 | V1828M | C | 5569 | T | ADAMTS1 | K771N | T | 2768 | G | AKAP13 | K2759N | A | 8447 | C | ANKRD50 | F224L | G | 1710 | T |
| ABCA4 | G1803S | C | 5494 | T | ADAMTS1 | S94G | T | 735 | C | AKAP13 | R2015H | G | 6214 | A | ANKRD52 | A297V | G | 980 | A |
| ABCA4 | R511H | C | 1619 | T | ADAMTS1 | R258H | C | 1228 | T | AKAP13 | S2198R | A | 6762 | C | ANKRD52 | R103Q | C | 398 | T |
| ABCA4 | E684D | C | 2139 | A | ADAMTS1 | L355I | G | 1518 | T | AKAP13 | E2431* | G | 7461 | T | ANKRD52 | A868T | C | 2692 | T |
| ABCA4 | R1307S | T | 4008 | G | ADAMTS1 | R190W | G | 1023 | A | AKAP3 | S356L | G | 1296 | A | ANKRD52 | R389C | G | 1255 | A |
| ABCA4 | R1055Q | C | 3251 | T | ADAMTS10 | P176S | G | 793 | A | AKAP3 | I598F | T | 2021 | A | ANKRD54 | R197* | G | 782 | A |
| ABCA4 | L33S | A | 185 | G | ADAMTS10 | C1002Y | C | 3272 | T | AKAP3 | S571Y | G | 1941 | T | ANKRD54 | H69R | T | 399 | C |
| ABCA5 | K1432N | C | 4442 | A | ADAMTS10 | A55T | C | 430 | T | AKAP3 | K433N | T | 1528 | G | ANKRD54 | P12Q | G | 228 | T |
| ABCA5 | D379Y | C | 1281 | A | ADAMTS10 | S570Y | G | 1976 | T | AKAP3 | F354L | A | 1289 | G | ANKRD55 | R48W | G | 294 | A |
| ABCA6 | G499D | C | 1671 | T | ADAMTS10 | R768H | C | 2570 | T | AKAP4 | G718V | C | 2277 | A | ANKRD55 | T144M | G | 583 | A |
| ABCA6 | A398S | C | 1367 | A | ADAMTS12 | V465I | C | 1729 | T | AKAP4 | L348I | G | 1166 | T | ANKRD55 | P591S | G | 1923 | A |
| ABCA6 | V560I | C | 1853 | T | ADAMTS12 | R604W | G | 2146 | A | AKAP4 | R176H | C | 651 | T | ANKRD56 | E789* | C | 2365 | A |
| ABCA6 | R1419I | C | 4431 | A | ADAMTS12 | V593I | C | 2113 | T | AKAP6 | G1260R | G | 3904 | C | ANKRD57 | G260C | G | 934 | T |
| ABCA6 | E799K | C | 2570 | T | ADAMTS12 | R1398Q | C | 4529 | T | AKAP6 | M2011I | G | 6159 | T | ANKRD6 | D293Y | G | 1218 | T |
| ABCA6 | K485E | T | 1628 | C | ADAMTS12 | H402Y | G | 1540 | A | AKAP6 | L1473I | C | 4543 | A | ANKRD6 | E330* | G | 1329 | T |
| ABCA6 | E476* | C | 1601 | A | ADAMTS12 | E566D | C | 2034 | A | AKAP6 | S955P | T | 2989 | C | ANKRD6 | - | T | 0 | C |
| ABCA7 | L1696V | T | 5317 | G | ADAMTS12 | R604W | G | 2146 | A | AKAP6 | R270Q | G | 935 | A | ANKRD7 | R66I | G | 365 | T |
| ABCA7 | A589V | C | 1997 | T | ADAMTS12 | Q325H | C | 1311 | A | AKAP6 | K436T | A | 1433 | C | ANKRD7 | N2T | A | 173 | C |
| ABCA7 | V1536I | G | 4837 | A | ADAMTS12 | E302K | C | 1240 | T | AKAP6 | V614I | G | 1966 | A | ANKRD7 | Q63H | A | 357 | C |
| ABCA7 | V541M | G | 1852 | A | ADAMTS12 | A23T | C | 403 | T | AKAP6 | D1731N | G | 5317 | A | ANKRD9 | R66H | C | 794 | T |
| ABCA7 | R770Q | G | 2540 | A | ADAMTS13 | G316D | G | 1391 | A | AKAP6 | R2105I | G | 6440 | T | ANKRD9 | Q268H | C | 1401 | A |

| | | | | | | | | | | | | | | | | | | | |
|---|---|---|---|---|---|---|---|---|---|---|---|---|---|---|---|---|---|---|---|
| ABCA7 | R2002W | C | 6235 | T | ADAMTS13 | D921G | A | 3206 | G | AKAP7 | A178T | G | 532 | A | ANKRD9 | E142K | C | 1021 | T |
| ABCA7 | R1754Q | G | 5492 | A | ADAMTS13 | Y957H | T | 3313 | C | AKAP7 | P319L | C | 956 | T | ANKS1A | Q1112K | C | 3472 | A |
| ABCA7 | S162* | C | 716 | A | ADAMTS13 | A1145T | G | 3877 | A | AKAP7 | N10H | A | 28 | C | ANKS1A | L1131H | T | 3530 | A |
| ABCA7 | Y750C | A | 2480 | G | ADAMTS13 | S1233Y | C | 4142 | A | AKAP7 | K15N | G | 45 | T | ANKS1A | - | G | 0 | A |
| ABCA7 | R1976H | G | 6158 | A | ADAMTS14 | V682I | G | 2044 | A | AKAP8 | R242Q | C | 786 | T | ANKS1A | A1073V | C | 3356 | T |
| ABCA7 | P723S | C | 2398 | T | ADAMTS14 | C877W | C | 2631 | G | AKAP8 | R447Q | C | 1401 | T | ANKS1A | R884H | G | 2789 | A |
| ABCA7 | G1786* | G | 5587 | T | ADAMTS14 | A956T | G | 2866 | A | AKAP8 | R206C | G | 677 | A | ANKS1B | D301N | C | 901 | T |
| ABCA7 | K1732N | G | 5427 | T | ADAMTS14 | G721R | G | 2161 | A | AKAP8 | R383H | C | 1209 | T | ANKS1B | G172R | C | 514 | T |
| ABCA8 | S348T | A | 1221 | T | ADAMTS14 | R879C | C | 2635 | T | AKAP8L | D89N | C | 364 | T | ANKS1B | P116Q | G | 347 | T |
| ABCA8 | - | C | 0 | T | ADAMTS14 | D297N | G | 889 | A | AKAP8L | D338N | C | 1111 | T | ANKS1B | K1151R | T | 3452 | C |
| ABCA8 | V334M | C | 1179 | T | ADAMTS14 | G765S | G | 2293 | A | AKAP9 | R3310W | C | 10161 | T | ANKS1B | Y745H | A | 2233 | G |
| ABCA8 | A1132T | C | 3573 | T | ADAMTS14 | G1206W | G | 3616 | T | AKAP9 | R3758C | C | 11505 | T | ANKS1B | K663R | T | 1988 | C |
| ABCA8 | V1034D | A | 3280 | T | ADAMTS14 | L174F | G | 522 | C | AKAP9 | I2828N | T | 8716 | A | ANKS1B | L263F | C | 789 | A |
| ABCA8 | R1088C | G | 3441 | A | ADAMTS14 | D297N | G | 889 | A | AKAP9 | A2986T | G | 9189 | A | ANKS4B | N75S | A | 267 | G |
| ABCA8 | P1208S | G | 3801 | A | ADAMTS15 | R507I | G | 1520 | T | AKAP9 | P1381L | C | 4375 | T | ANKS4B | A398D | C | 1236 | A |
| ABCA8 | E1118K | C | 3531 | T | ADAMTS15 | C555Y | G | 1664 | A | AKAP9 | T2701N | C | 8335 | A | ANKS6 | A872V | G | 2636 | A |
| ABCA8 | K620T | T | 2038 | G | ADAMTS15 | A765V | C | 2294 | T | AKAP9 | E136G | A | 640 | G | ANKS6 | G236V | C | 728 | A |
| ABCA9 | L416F | C | 1391 | G | ADAMTS15 | A870T | G | 2608 | A | AKAP9 | T211A | A | 864 | G | ANKS6 | F615C | A | 1865 | C |
| ABCA9 | A1567T | C | 4842 | T | ADAMTS15 | I54T | T | 161 | C | AKAP9 | E1921D | G | 5996 | T | ANKS6 | R541Q | C | 1643 | T |
| ABCA9 | R655Q | C | 2107 | T | ADAMTS15 | D189N | G | 565 | A | AKAP9 | G3627E | G | 11113 | A | ANKS6 | R412* | G | 1255 | A |
| ABCA9 | F563L | A | 1832 | T | ADAMTS15 | D372A | A | 1115 | C | AKAP9 | V1101A | T | 3535 | C | ANKZF1 | R146Q | G | 611 | A |
| ABCB1 | E256* | C | 1184 | A | ADAMTS16 | G993R | G | 3115 | A | AKAP9 | S2485Y | C | 7687 | A | ANKZF1 | R303Q | G | 1082 | A |
| ABCB1 | I1266V | T | 4214 | C | ADAMTS16 | V1011E | T | 3170 | A | AKD1 | R48H | C | 220 | T | ANKZF1 | R164Q | G | 665 | A |
| ABCB1 | F759L | A | 2693 | G | ADAMTS16 | K130N | G | 528 | T | AKD1 | N139I | T | 415 | A | ANKZF1 | R320Q | G | 1133 | A |
| ABCB1 | K1076N | C | 3646 | A | ADAMTS16 | L344I | C | 1168 | A | AKIRIN1 | R143C | C | 563 | T | ANLN | R16* | C | 267 | T |
| ABCB1 | E614* | C | 2258 | A | ADAMTS16 | R44H | G | 269 | A | AKIRIN2 | K86T | T | 782 | G | ANLN | R1040C | C | 3339 | T |
| ABCB1 | F335L | G | 1423 | T | ADAMTS16 | A947S | G | 2977 | T | AKNA | D278Y | C | 994 | A | ANO1 | R246W | C | 785 | T |
| ABCB1 | R286I | C | 1275 | A | ADAMTS17 | L748M | G | 2348 | T | AKNA | R1198W | G | 3754 | A | ANO10 | A572V | G | 1886 | A |
| ABCB1 | R262I | C | 1203 | A | ADAMTS17 | P466L | G | 1503 | A | AKNA | E611A | T | 1994 | G | ANO10 | M373V | T | 1288 | C |
| ABCB10 | R267H | C | 843 | T | ADAMTS17 | G895D | C | 2790 | T | AKNA | D552G | T | 1817 | C | ANO2 | T710I | G | 2201 | A |
| ABCB11 | R616C | G | 1971 | A | ADAMTS17 | R1075H | C | 3330 | T | AKNA | R1156* | G | 3628 | A | ANO2 | R704* | G | 2182 | A |
| ABCB11 | F1200C | A | 3724 | C | ADAMTS17 | T900M | G | 2805 | A | AKNA | L231M | G | 853 | T | ANO2 | S994Y | G | 3053 | T |
| ABCB4 | A578V | G | 1845 | A | ADAMTS18 | R902Q | C | 3124 | T | AKNA | S375I | C | 1286 | A | ANO2 | R570H | C | 1781 | T |
| ABCB4 | G687E | C | 2172 | T | ADAMTS18 | E678D | C | 2453 | A | AKNA | P1046T | G | 3298 | T | ANO2 | N247S | T | 812 | C |
| ABCB4 | L158V | G | 584 | C | ADAMTS18 | G891E | C | 3091 | T | AKNA | A1331V | G | 4154 | A | ANO2 | N161I | T | 554 | A |
| ABCB4 | M918L | T | 2864 | A | ADAMTS18 | G429D | C | 1705 | T | AKNAD1 | S780P | A | 2607 | G | ANO2 | D657N | C | 2041 | T |
| ABCB4 | K191N | C | 685 | A | ADAMTS18 | N905H | T | 3132 | G | AKNAD1 | E478D | C | 1703 | A | ANO2 | N402T | T | 1277 | G |
| ABCB4 | R159* | G | 587 | A | ADAMTS18 | V896I | C | 3105 | T | AKR1B1 | A35T | C | 183 | T | ANO2 | F198S | A | 665 | G |
| ABCB4 | G90E | C | 381 | T | ADAMTS18 | F750L | A | 2669 | C | AKR1B1 | F116S | A | 427 | G | ANO2 | G357V | C | 1142 | A |

| | | | | | | | | | | | | | | | | | | | |
|---|---|---|---|---|---|---|---|---|---|---|---|---|---|---|---|---|---|---|---|
| ABCB5 | R140Q | G | 856 | A | ADAMTS18 | R651W | G | 2370 | A | AKR1B1 | W21* | C | 143 | T | ANO3 | V2G | T | 857 | G |
| ABCB5 | R1027C | C | 3516 | T | ADAMTS18 | I540V | T | 2037 | C | AKR1B10 | G119V | G | 676 | T | ANO3 | R727Q | G | 3032 | A |
| ABCB5 | E1083* | G | 3684 | T | ADAMTS18 | F485S | A | 1873 | G | AKR1C3 | D300Y | G | 1550 | T | ANO4 | R408M | G | 1434 | T |
| ABCB5 | V472A | T | 1852 | C | ADAMTS18 | S473* | G | 1837 | T | AKR1C4 | V228A | T | 936 | C | ANO4 | S385G | A | 1364 | G |
| ABCB5 | H730R | A | 2626 | G | ADAMTS18 | S367R | A | 1520 | C | AKR1C4 | F89L | C | 520 | A | ANO4 | R798H | G | 2604 | A |
| ABCB5 | V1210I | G | 4065 | A | ADAMTS18 | E111* | C | 750 | A | AKR1C4 | V150I | G | 701 | A | ANO4 | S6P | T | 227 | C |
| ABCB6 | K569T | T | 2023 | G | ADAMTS19 | E624* | G | 2015 | T | AKR1C4 | S162P | T | 737 | C | ANO4 | R104I | G | 522 | T |
| ABCB8 | R547W | C | 1705 | T | ADAMTS19 | R969* | C | 3050 | T | AKR1D1 | A153V | C | 500 | T | ANO4 | F752C | T | 2466 | G |
| ABCB8 | R55C | C | 229 | T | ADAMTS19 | A1028S | G | 3227 | T | AKR1D1 | H93R | A | 320 | G | ANO4 | R884* | C | 2861 | T |
| ABCB9 | L30M | G | 666 | T | ADAMTS19 | T238A | A | 857 | G | AKR1E2 | - | G | 0 | T | ANO4 | F896C | T | 2898 | G |
| ABCB9 | V230A | A | 1267 | G | ADAMTS19 | M426I | G | 1423 | T | AKR7A2 | G95V | C | 306 | A | ANO4 | D912Y | G | 2945 | T |
| ABCB9 | Y412C | T | 1813 | C | ADAMTS19 | K438N | A | 1459 | C | AKT2 | - | C | 0 | T | ANO5 | F568V | T | 2019 | G |
| ABCC1 | D787G | A | 2535 | G | ADAMTS19 | - | G | 0 | A | AKT3 | - | C | 0 | T | ANO5 | G347D | G | 1357 | A |
| ABCC1 | R1158C | C | 3647 | T | ADAMTS19 | R656Q | G | 2112 | A | AKT3 | F207L | A | 822 | C | ANO5 | L113I | C | 654 | A |
| ABCC1 | G1519D | G | 4731 | A | ADAMTS2 | R941H | C | 2878 | T | ALAD | L336P | A | 1106 | G | ANO5 | R127I | G | 697 | T |
| ABCC10 | L187P | T | 775 | C | ADAMTS2 | D617N | C | 1905 | T | ALAS1 | D205A | A | 889 | C | ANO5 | N567H | A | 2016 | C |
| ABCC10 | T296I | C | 1102 | T | ADAMTS2 | A1005T | C | 3069 | T | ALAS2 | R368Q | C | 1241 | T | ANO5 | Q679P | A | 2353 | C |
| ABCC10 | T539M | C | 1831 | T | ADAMTS2 | R691H | C | 2128 | T | ALAS2 | R411C | G | 1369 | A | ANO6 | Q413R | A | 1573 | G |
| ABCC11 | A597T | C | 1888 | T | ADAMTS2 | A1020E | G | 3115 | T | ALB | S294L | C | 954 | T | ANO6 | R712C | C | 2469 | T |
| ABCC11 | R428Q | C | 1382 | T | ADAMTS2 | R368Q | C | 1159 | T | ALB | F173V | T | 590 | G | ANO6 | R253C | C | 1092 | T |
| ABCC11 | A556V | G | 1766 | A | ADAMTS2 | L849M | G | 2601 | T | ALB | P523S | C | 1640 | T | ANO7 | R149C | C | 548 | T |
| ABCC11 | K678N | C | 2133 | A | ADAMTS2 | E428K | C | 1338 | T | ALCAM | A69T | G | 705 | A | ANO7 | A130T | G | 491 | A |
| ABCC11 | T256A | T | 865 | C | ADAMTS20 | R63Q | C | 188 | T | ALCAM | H330N | C | 1488 | A | ANO7 | A183T | G | 650 | A |
| ABCC12 | D634N | C | 2246 | T | ADAMTS20 | R807I | C | 2420 | A | ALCAM | V184L | G | 1050 | T | ANO7 | R578H | G | 1836 | A |
| ABCC12 | - | C | 0 | T | ADAMTS20 | K1625N | C | 4875 | A | ALCAM | Y470C | A | 1909 | G | ANO7 | G763D | G | 2391 | A |
| ABCC12 | V867I | C | 2945 | T | ADAMTS20 | P1274L | G | 3821 | A | ALDH16A1 | E755K | G | 2379 | A | ANO7 | S65I | G | 297 | T |
| ABCC12 | R1273H | C | 4164 | T | ADAMTS20 | I1718S | A | 5153 | C | ALDH16A1 | A581V | C | 1858 | T | ANO7 | G269D | G | 909 | A |
| ABCC12 | D1301Y | C | 4247 | A | ADAMTS20 | N1542D | T | 4624 | C | ALDH16A1 | A581V | C | 1858 | T | ANO7 | A204V | C | 714 | T |
| ABCC12 | E1252* | C | 4100 | A | ADAMTS20 | S237* | G | 710 | T | ALDH16A1 | A782G | C | 2461 | G | ANO7 | A538T | G | 1715 | A |
| ABCC12 | - | C | 0 | A | ADAMTS20 | R1000C | G | 2998 | A | ALDH16A1 | R329W | C | 1101 | T | ANO7 | R451H | G | 1455 | A |
| ABCC12 | E928D | C | 3130 | A | ADAMTS20 | S1346P | A | 4036 | G | ALDH18A1 | R55H | C | 302 | T | ANO7 | A431T | G | 1394 | A |
| ABCC12 | A844T | C | 2876 | T | ADAMTS20 | N1781H | T | 5341 | G | ALDH18A1 | R456C | G | 1504 | A | ANO7 | D874G | A | 2724 | G |
| ABCC12 | E565* | C | 2039 | A | ADAMTS20 | E1617D | C | 4851 | A | ALDH18A1 | A80T | C | 376 | T | ANO7 | R336H | G | 1110 | A |
| ABCC12 | V249A | A | 1092 | G | ADAMTS20 | R598H | C | 1793 | T | ALDH18A1 | R64H | C | 329 | T | ANO8 | R1036Q | C | 3266 | T |
| ABCC2 | S978L | C | 3046 | T | ADAMTS20 | - | C | 0 | T | ALDH18A1 | R514Q | C | 1679 | T | ANO8 | L474P | A | 1580 | G |
| ABCC2 | D1396N | G | 4299 | A | ADAMTS3 | D223A | T | 705 | G | ALDH1A1 | Y90* | G | 325 | T | ANO8 | R140C | G | 577 | A |
| ABCC2 | A1450V | C | 4462 | T | ADAMTS3 | A531P | C | 1628 | G | ALDH1A1 | - | C | 0 | A | ANO8 | R711Q | C | 2291 | T |
| ABCC2 | R260W | C | 891 | T | ADAMTS3 | G226D | C | 714 | T | ALDH1A2 | V509M | C | 2293 | T | ANO9 | - | C | 0 | T |
| ABCC2 | F5L | C | 128 | A | ADAMTS3 | K756N | C | 2305 | A | ALDH1A2 | E416D | C | 2016 | A | ANO9 | R42C | G | 209 | A |

| | | | | | | | | | | | | | | | | | | | |
|---|---|---|---|---|---|---|---|---|---|---|---|---|---|---|---|---|---|---|---|
| ABCC3 | L351P | T | 1132 | C | ADAMTS3 | F180L | G | 577 | T | ALDH1A2 | I295T | A | 1652 | G | ANP32A | L228F | G | 799 | A |
| ABCC3 | P652L | C | 2035 | T | ADAMTS4 | V152M | C | 883 | T | ALDH1A3 | A194T | G | 1112 | A | ANP32C | L11P | A | 32 | G |
| ABCC4 | A1203V | G | 3723 | A | ADAMTS4 | R760C | G | 2707 | A | ALDH1B1 | R18C | C | 205 | T | ANPEP | R218Q | C | 967 | T |
| ABCC4 | T988M | G | 3078 | A | ADAMTS4 | A52T | C | 583 | T | ALDH1B1 | N333I | A | 1151 | T | ANPEP | V109I | C | 639 | T |
| ABCC4 | G34D | C | 216 | T | ADAMTS4 | E769D | C | 2736 | A | ALDH1B1 | T283I | C | 1001 | T | ANTXR1 | R551S | G | 1975 | T |
| ABCC5 | R1267Q | C | 4041 | T | ADAMTS5 | P919H | G | 2878 | T | ALDH1L1 | T451I | G | 1570 | A | ANTXR1 | A173V | C | 840 | T |
| ABCC5 | Q190H | C | 811 | A | ADAMTS5 | L667V | G | 2121 | C | ALDH1L1 | W546G | A | 1854 | C | ANTXR1 | A192V | C | 897 | T |
| ABCC5 | T1055M | G | 3405 | A | ADAMTS5 | R257W | G | 891 | A | ALDH1L1 | A514V | G | 1759 | A | ANTXR1 | Y176* | C | 850 | A |
| ABCC8 | R1394C | G | 4306 | A | ADAMTS5 | R170C | G | 630 | A | ALDH1L1 | A276V | G | 1045 | A | ANTXR2 | R484Q | C | 1977 | T |
| ABCC8 | A1303T | C | 4033 | T | ADAMTS5 | T746I | G | 2359 | A | ALDH1L2 | V720L | C | 2180 | A | ANTXR2 | K161T | T | 1245 | G |
| ABCC8 | A1185V | G | 3680 | A | ADAMTS5 | Y784C | T | 2473 | C | ALDH1L2 | R714Q | C | 2163 | T | ANUBL1 | E387* | C | 1411 | A |
| ABCC8 | - | A | 0 | G | ADAMTS5 | T607A | T | 1941 | C | ALDH1L2 | D491Y | C | 1493 | A | ANXA1 | R72Q | G | 397 | A |
| ABCC8 | E1400K | C | 4324 | T | ADAMTS5 | I547N | A | 1762 | T | ALDH2 | V57I | G | 610 | A | ANXA1 | - | A | 0 | G |
| ABCC8 | - | C | 0 | T | ADAMTS5 | P205L | G | 736 | A | ALDH2 | A120V | C | 800 | T | ANXA10 | R128* | C | 568 | T |
| ABCC9 | S562R | T | 1704 | G | ADAMTS6 | N838D | T | 3369 | C | ALDH3A1 | F388L | G | 1164 | T | ANXA10 | M83V | A | 433 | G |
| ABCC9 | V19A | A | 76 | G | ADAMTS6 | Q1039R | T | 3973 | C | ALDH3A2 | L49P | T | 367 | C | ANXA11 | - | A | 0 | G |
| ABCC9 | R1154W | G | 3480 | A | ADAMTS7 | G115S | C | 554 | T | ALDH3A2 | R311H | G | 1153 | A | ANXA13 | A133T | C | 470 | T |
| ABCC9 | F858L | G | 2594 | T | ADAMTS7 | A722T | C | 2375 | T | ALDH3A2 | G408R | G | 1443 | C | ANXA3 | K101T | A | 681 | C |
| ABCC9 | K506Q | T | 1536 | G | ADAMTS7 | A636V | G | 2118 | A | ALDH3B2 | E97D | C | 730 | A | ANXA4 | - | G | 0 | T |
| ABCD1 | R259W | C | 1174 | T | ADAMTS7 | C1671Y | C | 5223 | T | ALDH4A1 | S100N | C | 557 | T | ANXA6 | A98V | G | 445 | A |
| ABCD1 | R401W | C | 1600 | T | ADAMTS7 | R1669C | G | 5216 | A | ALDH4A1 | W361* | C | 1341 | T | ANXA6 | G88C | C | 414 | A |
| ABCD2 | T211M | G | 1058 | A | ADAMTS7 | D869A | T | 2817 | G | ALDH4A1 | F40S | A | 377 | G | ANXA7 | I223S | A | 725 | C |
| ABCD2 | N394H | T | 1606 | G | ADAMTS8 | R651H | C | 1952 | T | ALDH5A1 | V505I | G | 1541 | A | ANXA9 | A23T | G | 543 | A |
| ABCD2 | M216T | A | 1073 | G | ADAMTS9 | R177Q | C | 873 | T | ALDH6A1 | F185L | G | 654 | T | AOAH | W512* | C | 1837 | T |
| ABCD3 | M157V | A | 571 | G | ADAMTS9 | E186D | T | 901 | G | ALDH8A1 | E48K | C | 195 | T | AOC3 | G508R | G | 1682 | A |
| ABCD3 | R544* | C | 1732 | T | ADAMTS9 | E1931D | C | 6136 | A | ALDH8A1 | R69C | G | 258 | A | AOC3 | V539I | G | 1775 | A |
| ABCD3 | F166L | C | 600 | A | ADAMTS9 | S501Y | G | 1845 | T | ALDH9A1 | R357Q | C | 1375 | T | AOC3 | N375D | A | 1283 | G |
| ABCD4 | - | T | 0 | G | ADAMTS9 | R1816Q | C | 5790 | T | ALDH9A1 | T209A | T | 930 | C | AOX1 | D862N | G | 2825 | A |
| ABCF3 | R618* | C | 2037 | T | ADAMTS9 | M1264I | C | 4135 | T | ALDOB | L128F | G | 507 | A | AOX1 | R433* | C | 1538 | T |
| ABCF3 | T272I | C | 1000 | T | ADAMTS9 | P1601L | G | 5145 | A | ALDOB | V104M | C | 435 | T | AOX1 | K1237E | A | 3950 | G |
| ABCG1 | H634R | A | 2049 | G | ADAMTSL1 | G1000R | G | 3125 | A | ALDOB | A168T | C | 627 | T | AOX1 | R290K | G | 1110 | A |
| ABCG1 | R821W | C | 2609 | T | ADAMTSL2 | R88H | G | 695 | A | ALDOC | A297V | G | 1036 | A | AOX1 | A507V | C | 1761 | T |
| ABCG1 | S672L | C | 2163 | T | ADAMTSL3 | R231W | C | 915 | T | ALG1 | L193P | T | 609 | C | AOX1 | D555Y | G | 1904 | T |
| ABCG2 | S88L | G | 809 | A | ADAMTSL3 | V979M | G | 3159 | A | ALG1 | A10T | G | 59 | A | AOX1 | A664V | C | 2232 | T |
| ABCG2 | N288T | T | 1409 | G | ADAMTSL3 | Q23P | A | 292 | C | ALG12 | A179T | C | 809 | T | AP1AR | S174A | T | 875 | G |
| ABCG4 | A334T | G | 1336 | A | ADAMTSL3 | - | G | 0 | A | ALG13 | S52G | A | 166 | G | AP1B1 | T849I | G | 2730 | A |
| ABCG4 | P483L | C | 1784 | T | ADAMTSL3 | R632* | C | 2118 | T | ALG1L | R3Q | C | 172 | T | AP1B1 | N904S | T | 2895 | C |
| ABCG4 | - | T | 2275 | C | ADAMTSL3 | A1117V | C | 3574 | T | ALG2 | S207N | C | 682 | T | AP1B1 | S798G | T | 2576 | C |
| ABCG4 | C597Y | G | 2126 | A | ADAMTSL3 | H1400N | C | 4422 | A | ALG3 | R260C | G | 809 | A | AP1B1 | V326M | C | 1160 | T |

| | | | | | | | | | | | | | | | | | | | |
|---|---|---|---|---|---|---|---|---|---|---|---|---|---|---|---|---|---|---|---|
| ABCG4 | T513A | A | 1873 | G | ADAMTSL4 | R542W | C | 1860 | T | ALG3 | T135A | T | 434 | C | AP1B1 | G638C | C | 2096 | A |
| ABCG5 | P431L | G | 1432 | A | ADAMTSL4 | R478C | C | 1668 | T | ALG6 | R503I | G | 1813 | T | AP1B1 | R114C | G | 524 | A |
| ABCG5 | V233I | C | 837 | T | ADAMTSL5 | L250F | G | 748 | A | ALG8 | - | C | 0 | A | AP1G1 | I531S | A | 1592 | C |
| ABCG8 | I544M | T | 1722 | G | ADAP1 | G316D | C | 947 | T | ALG8 | V110I | C | 393 | T | AP1G1 | D530N | C | 1588 | T |
| ABHD1 | G11D | G | 146 | A | ADAP1 | A122D | G | 365 | T | ALG9 | A105V | G | 314 | A | AP1G2 | K109N | C | 1083 | A |
| ABHD10 | K195R | A | 611 | G | ADAR | F787L | G | 2361 | T | ALG9 | E692* | C | 2074 | A | AP1G2 | A522V | G | 2321 | A |
| ABHD10 | L290I | C | 895 | A | ADAR | N900I | T | 2699 | A | ALK | R401* | G | 2108 | A | AP1G2 | G286R | C | 1612 | T |
| ABHD12 | V289M | C | 1145 | T | ADAR | R1069W | G | 3205 | A | ALK | A585T | C | 2660 | T | AP1G2 | S78F | G | 989 | A |
| ABHD12B | L276P | T | 842 | C | ADARB1 | S583L | C | 1763 | T | ALK | D769G | T | 3213 | C | AP1G2 | D43N | C | 883 | T |
| ABHD12B | K92N | G | 291 | T | ADARB1 | S639G | A | 1930 | G | ALK | R551Q | C | 2559 | T | AP1M2 | R19C | G | 102 | A |
| ABHD12B | K280T | A | 854 | C | ADARB1 | R244H | G | 746 | A | ALK | R291H | C | 1779 | T | AP1M2 | V110I | C | 375 | T |
| ABHD12B | E302D | G | 921 | T | ADARB2 | T447M | G | 1666 | A | ALK | R188C | G | 1469 | A | AP2A1 | G335D | G | 1215 | A |
| ABHD13 | E317* | G | 1250 | T | ADARB2 | A332T | C | 1320 | T | ALK | R179H | C | 1443 | T | AP2A1 | K6N | G | 229 | T |
| ABHD14A | Q116R | A | 347 | G | ADARB2 | L595I | G | 2109 | T | ALK | R200S | T | 1507 | A | AP2A1 | K61E | A | 392 | G |
| ABHD15 | R315* | G | 1001 | A | ADARB2 | A303T | C | 1233 | T | ALK | F58L | G | 1081 | T | AP2A1 | N509D | A | 1736 | G |
| ABHD2 | R156W | C | 962 | T | ADAT1 | A473V | G | 1564 | A | ALK | R551* | G | 2558 | A | AP2A1 | V373I | G | 1328 | A |
| ABHD2 | R409C | C | 1721 | T | ADAT3 | D271N | G | 1039 | A | ALKBH2 | E25* | C | 466 | A | AP2A1 | A489T | G | 1676 | A |
| ABHD2 | D423N | G | 1763 | A | ADAT3 | A183V | C | 776 | T | ALKBH3 | P260S | C | 1221 | T | AP2A1 | F864L | C | 2803 | A |
| ABHD2 | R254W | C | 1256 | T | ADAT3 | R261C | C | 1009 | T | ALKBH3 | N168T | A | 946 | C | AP2A1 | R959C | C | 3086 | T |
| ABHD3 | G374D | C | 1261 | T | ADC | A380V | C | 1457 | T | ALKBH4 | A248T | C | 782 | T | AP2A2 | T189A | A | 746 | G |
| ABHD3 | L69I | G | 345 | T | ADCK1 | D403Y | G | 1306 | T | ALKBH5 | R327C | C | 1670 | T | AP2A2 | A800V | C | 2580 | T |
| ABHD4 | R305W | C | 983 | T | ADCK2 | P34S | C | 278 | T | ALKBH7 | P161L | C | 870 | T | AP2A2 | R633Q | G | 2079 | A |
| ABHD4 | R278* | C | 902 | T | ADCK4 | A256V | G | 1069 | A | ALKBH8 | Q305* | G | 1048 | A | AP2S1 | - | C | 0 | A |
| ABHD5 | D243N | G | 850 | A | ADCK4 | A86T | C | 558 | T | ALKBH8 | S317R | T | 1084 | G | AP3B1 | R104Q | C | 436 | T |
| ABHD5 | E128D | G | 507 | T | ADCK4 | R518H | C | 1855 | T | ALKBH8 | E144* | C | 565 | A | AP3B1 | A884T | C | 2775 | T |
| ABHD5 | I322M | T | 1089 | G | ADCK5 | L389R | T | 1220 | G | ALLC | R194* | C | 687 | T | AP3B1 | E275K | C | 948 | T |
| ABHD8 | F197L | G | 831 | T | ADCK5 | P474Q | C | 1475 | A | ALLC | M156I | G | 575 | T | AP3B1 | T835I | G | 2629 | A |
| ABI1 | R138W | G | 579 | A | ADCK5 | R449C | C | 1399 | T | ALMS1 | - | T | 0 | C | AP3B2 | G710S | C | 2305 | T |
| ABI2 | F500L | T | 1796 | G | ADCY1 | R345C | C | 1055 | T | ALMS1 | L313S | T | 1049 | C | AP3B2 | A579V | G | 1913 | A |
| ABI2 | R237I | G | 1006 | T | ADCY1 | N1004S | A | 3033 | G | ALMS1 | D3857G | A | 11681 | G | AP3B2 | E924K | C | 2947 | T |
| ABI3 | G144D | G | 929 | A | ADCY1 | S711L | C | 2154 | T | ALMS1 | A3390V | C | 10280 | T | AP3B2 | G271S | C | 988 | T |
| ABI3BP | Y438C | T | 1423 | C | ADCY1 | A991T | G | 2993 | A | ALMS1 | S290R | A | 979 | C | AP3B2 | L809F | G | 2602 | A |
| ABI3BP | P556L | G | 1777 | A | ADCY1 | G359S | G | 1097 | A | ALMS1 | N359H | A | 1186 | C | AP3D1 | S471R | T | 1634 | G |
| ABI3BP | T1319P | T | 4065 | G | ADCY1 | D997N | G | 3011 | A | ALMS1 | S513P | T | 1648 | C | AP3D1 | E863A | T | 2811 | G |
| ABI3BP | - | C | 0 | A | ADCY1 | R346C | C | 1058 | T | ALMS1 | K1121N | A | 3474 | C | AP3D1 | E209K | C | 848 | T |
| ABI3BP | E1712K | C | 5244 | T | ADCY10 | F336L | G | 1193 | T | ALMS1 | L1423V | T | 4378 | G | AP3M1 | R263H | C | 797 | T |
| ABI3BP | K1700T | T | 5209 | G | ADCY10 | H1515Y | G | 4728 | A | ALMS1 | E2022D | G | 6177 | T | AP3M2 | S287F | C | 1092 | T |
| ABI3BP | P1349S | G | 4155 | A | ADCY10 | P438L | G | 1498 | A | ALOX12 | T405A | A | 1266 | G | AP3S2 | V124A | A | 764 | G |
| ABL1 | A941V | C | 3196 | T | ADCY10 | E739* | C | 2400 | A | ALOX12 | R599C | C | 1848 | T | AP4B1 | A76D | G | 432 | T |

| | | | | | | | | | | | | | | | | | | | |
|---|---|---|---|---|---|---|---|---|---|---|---|---|---|---|---|---|---|---|---|
| ABL1 | G409W | G | 1599 | T | ADCY10 | S607Y | G | 2005 | T | ALOX12B | K241T | T | 984 | G | AP4B1 | A660V | G | 2184 | A |
| ABL1 | F91V | T | 645 | G | ADCY2 | M848T | T | 2632 | C | ALOX15 | R500* | G | 1510 | A | AP4B1 | T733A | T | 2402 | C |
| ABL2 | R580Q | C | 1943 | T | ADCY2 | K937R | A | 2899 | G | ALOX15 | R466W | G | 1408 | A | AP4E1 | L90P | T | 299 | C |
| ABL2 | V468I | C | 1606 | T | ADCY2 | S100T | T | 387 | A | ALOX15 | V100M | C | 310 | T | AP4E1 | E164K | G | 520 | A |
| ABL2 | G24D | C | 275 | T | ADCY2 | R480H | G | 1528 | A | ALOX15 | E398D | T | 1206 | G | AP4E1 | S987R | A | 2989 | C |
| ABLIM1 | R754* | G | 2260 | A | ADCY2 | M767I | G | 2390 | T | ALOX15 | P299S | G | 907 | A | AP4M1 | N139S | A | 574 | G |
| ABLIM2 | A110V | G | 349 | A | ADCY2 | P894L | C | 2770 | T | ALOX15B | A239T | G | 828 | A | AP4M1 | G39R | G | 273 | A |
| ABLIM3 | I260V | A | 1017 | G | ADCY2 | V118F | G | 441 | T | ALOX15B | Y149C | A | 559 | G | AP4M1 | K191N | G | 731 | T |
| ABP1 | A9T | G | 85 | A | ADCY2 | - | G | 0 | T | ALOX15B | Y256C | A | 880 | G | AP4S1 | E75D | G | 433 | T |
| ABP1 | N660S | A | 2039 | G | ADCY2 | F612V | T | 1923 | G | ALOX15B | A236T | G | 819 | A | AP4S1 | L138I | C | 794 | A |
| ABP1 | C181Y | G | 602 | A | ADCY2 | L665P | T | 2083 | C | ALOX15B | L289V | T | 978 | G | APAF1 | E925* | G | 3350 | T |
| ABR | E405K | C | 1323 | T | ADCY2 | T687A | A | 2148 | G | ALOX5 | V51M | G | 204 | A | APAF1 | D825A | A | 3051 | C |
| ABT1 | A178T | G | 563 | A | ADCY3 | Q150H | C | 1302 | A | ALOX5 | L267M | C | 852 | A | APAF1 | L140F | C | 995 | T |
| ABT1 | R170H | G | 540 | A | ADCY3 | P131L | G | 1244 | A | ALOX5 | E135* | G | 456 | T | APAF1 | E777* | G | 2906 | T |
| ABT1 | V121M | G | 392 | A | ADCY3 | A160T | C | 1330 | T | ALOX5 | E274D | G | 875 | T | APBA1 | A427V | G | 1503 | A |
| ABT1 | R244H | G | 762 | A | ADCY3 | E947V | T | 3692 | A | ALOX5 | R652H | G | 2008 | A | APBA1 | R492M | C | 1698 | A |
| ABT1 | R164C | C | 521 | T | ADCY4 | Q853R | T | 2672 | C | ALOX5AP | F92C | T | 373 | G | APBA1 | E373K | C | 1340 | T |
| ABT1 | R150H | G | 480 | A | ADCY4 | A68V | G | 317 | A | ALOXE3 | E804* | C | 2441 | A | APBA1 | K498I | T | 1716 | A |
| ABT1 | R204H | G | 642 | A | ADCY4 | P28L | G | 197 | A | ALOXE3 | G713S | C | 2168 | T | APBA1 | D474G | T | 1644 | C |
| ABTB1 | R465W | C | 1479 | T | ADCY4 | A42T | C | 238 | T | ALOXE3 | S237A | A | 740 | C | APBA1 | A296T | C | 1109 | T |
| ABTB1 | V117M | G | 435 | A | ADCY4 | T802I | G | 2519 | A | ALOXE3 | R367* | G | 1130 | A | APBA1 | E624K | C | 2093 | T |
| ABTB1 | G383V | G | 1234 | T | ADCY5 | R346C | G | 2249 | A | ALOXE3 | R212H | C | 666 | T | APBA2 | V174D | T | 715 | A |
| ABTB2 | R829H | C | 2716 | T | ADCY5 | Q422* | G | 2477 | A | ALPI | T293M | C | 955 | T | APBA2 | R102H | G | 499 | A |
| ABTB2 | S429P | A | 1515 | G | ADCY5 | A363V | G | 2301 | A | ALPI | A460V | C | 1456 | T | APBA3 | T178M | G | 710 | A |
| ABTB2 | L582M | G | 1974 | T | ADCY5 | R1117W | G | 4562 | A | ALPK1 | V296M | G | 1165 | A | APBA3 | R358W | G | 1249 | A |
| ABTB2 | R313G | T | 1167 | C | ADCY5 | P222L | G | 1878 | A | ALPK1 | A86T | G | 535 | A | APBB1 | E567K | C | 1799 | T |
| ABTB2 | K691T | T | 2302 | G | ADCY5 | A1054T | C | 4373 | T | ALPK1 | I1124M | A | 3651 | G | APBB1 | P64S | G | 290 | A |
| ACAA1 | S124L | G | 544 | A | ADCY5 | A482V | G | 2658 | A | ALPK1 | K1125E | A | 3652 | G | APBB1 | G136D | C | 507 | T |
| ACAA1 | M1I | C | 176 | T | ADCY6 | R739W | G | 2875 | A | ALPK1 | A144S | G | 709 | T | APBB1IP | H183R | A | 1003 | G |
| ACACA | R1017Q | C | 3532 | T | ADCY7 | V927M | G | 3068 | A | ALPK2 | A1737V | G | 5424 | A | APBB1IP | A487V | C | 1915 | T |
| ACACA | I1909N | A | 6208 | T | ADCY7 | R388H | G | 1452 | A | ALPK2 | S821Y | G | 2676 | T | APBB1IP | P270S | C | 1263 | T |
| ACACA | M1270V | T | 4290 | C | ADCY7 | G435R | G | 1592 | A | ALPK2 | A1294E | G | 4095 | T | APBB2 | S424Y | G | 1815 | T |
| ACACA | R1768H | C | 5785 | T | ADCY7 | R495Q | G | 1773 | A | ALPK2 | A702V | G | 2319 | A | APBB2 | R740* | G | 2762 | A |
| ACACA | V1114A | A | 3823 | G | ADCY8 | R179C | G | 791 | A | ALPK2 | E1312* | C | 4148 | A | APBB3 | T73M | G | 233 | A |
| ACACA | G263V | C | 1270 | A | ADCY8 | L216M | G | 902 | T | ALPK2 | A994D | G | 3195 | T | APBB3 | I76L | T | 241 | G |
| ACACB | R1511H | G | 4532 | A | ADCY8 | T555A | T | 1919 | C | ALPK2 | N545K | A | 1849 | C | APC | R876* | C | 3006 | T |
| ACACB | S712L | C | 2135 | T | ADCY8 | Q303E | G | 1163 | C | ALPK3 | S698N | G | 2260 | A | APC | H1009R | A | 3406 | G |
| ACACB | R724* | C | 2170 | T | ADCY8 | P474L | G | 1677 | A | ALPK3 | A1436V | C | 4474 | T | APC | R1450* | C | 4728 | T |
| ACACB | G2005D | G | 6014 | A | ADCY8 | A314V | G | 1197 | A | ALPK3 | R642Q | G | 2092 | A | APC | R1114* | C | 3720 | T |

| | | | | | | | | | | | | | | | | | | | |
|---|---|---|---|---|---|---|---|---|---|---|---|---|---|---|---|---|---|---|---|
| ACACB | R2384C | C | 7150 | T | ADCY8 | A992V | G | 3231 | A | ALPK3 | R1254C | C | 3927 | T | APC | Q1529* | C | 4965 | T |
| ACACB | A600T | G | 1798 | A | ADCY8 | N854K | G | 2818 | T | ALPK3 | E1461D | G | 4550 | T | APC | S1861Y | C | 5962 | A |
| ACACB | R1389C | C | 4165 | T | ADCY8 | S705Y | G | 2370 | T | ALPL | R30* | C | 338 | T | APC | R653M | G | 2338 | T |
| ACACB | V241M | G | 721 | A | ADCY8 | G422A | C | 1521 | G | ALPP | A198T | G | 861 | A | APC | R876Q | G | 3007 | A |
| ACACB | P580H | C | 1739 | A | ADCY8 | T1218A | T | 3908 | C | ALPPL2 | S99T | T | 347 | A | APC | R2816Q | G | 8827 | A |
| ACACB | G1816D | G | 5447 | A | ADCY9 | Y1184H | A | 4089 | G | ALPPL2 | A97S | G | 341 | T | APC | R653K | G | 2338 | A |
| ACAD10 | D299Y | G | 1072 | T | ADCY9 | L833M | G | 3036 | T | ALS2 | S492F | G | 1848 | A | APC | Q1378* | C | 4512 | T |
| ACAD10 | P755L | C | 2441 | T | ADCY9 | V558M | C | 2211 | T | ALS2 | R350W | G | 1421 | A | APC | R232* | C | 1074 | T |
| ACAD10 | G632V | G | 2072 | T | ADCY9 | - | A | 0 | G | ALS2 | P258Q | G | 1146 | T | APC | Q238* | C | 1092 | T |
| ACAD10 | H1088R | A | 3440 | G | ADCY9 | V1215I | C | 4182 | T | ALS2 | F792V | A | 2747 | C | APC | R564* | C | 2070 | T |
| ACAD11 | K207Q | T | 1591 | G | ADCYAP1 | E45K | G | 252 | A | ALS2 | E347* | C | 1412 | A | APC | N1290K | T | 4250 | A |
| ACAD11 | E215* | C | 1615 | A | ADCYAP1 | R172Q | G | 634 | A | ALS2CL | A257T | C | 853 | T | APC | R876* | C | 3006 | T |
| ACAD8 | R389W | C | 1253 | T | ADCYAP1R 1 | G341R | G | 1047 | A | ALS2CL | R134W | G | 484 | A | APC | E1521* | G | 4941 | T |
| ACAD8 | G275R | G | 911 | A | ADCYAP1R 1 | E97* | G | 315 | T | ALS2CL | R607W | G | 1903 | A | APC | E1408* | G | 4602 | T |
| ACAD9 | F70L | C | 291 | A | ADCYAP1R 1 | R288I | G | 889 | T | ALS2CL | F406L | G | 1302 | T | APC | Q215* | C | 1023 | T |
| ACADL | E260* | C | 1006 | A | ADCYAP1R 1 | H182L | A | 571 | T | ALS2CR11 | R135C | G | 448 | A | APC | R1386* | A | 4536 | T |
| ACADL | R50* | G | 376 | A | ADD2 | R432G | G | 1739 | C | ALS2CR11 | K951I | T | 2897 | A | APC | E1306* | G | 4296 | T |
| ACADL | S210G | T | 856 | C | ADD2 | T675M | G | 2469 | A | ALS2CR11 | K951* | T | 2896 | A | APC | G1312* | G | 4314 | T |
| ACADL | R51I | C | 380 | A | ADD3 | R434H | G | 1668 | A | ALS2CR11 | S1427A | A | 4324 | C | APC | R232* | C | 1074 | T |
| ACADL | D211N | C | 859 | T | ADD3 | F689V | T | 2432 | G | ALS2CR11 | S1210Y | G | 3674 | T | APC | R283* | C | 1227 | T |
| ACADM | K289E | A | 944 | G | ADD3 | E30A | A | 456 | C | ALS2CR11 | S659Y | G | 2021 | T | APC | R876* | C | 3006 | T |
| ACADM | I283N | T | 927 | A | ADH1A | K19N | C | 171 | A | ALS2CR12 | R30C | G | 135 | A | APC | Q1367* | C | 4479 | T |
| ACADS | R247C | C | 857 | T | ADH1C | A157V | G | 822 | A | ALS2CR12 | E387* | C | 1206 | A | APC | E1379* | G | 4515 | T |
| ACADS | S252R | C | 874 | A | ADH1C | A222V | G | 1017 | A | ALS2CR12 | N373K | A | 1166 | C | APC | S117* | C | 730 | A |
| ACADSB | A340T | G | 1032 | A | ADH4 | F343I | A | 1193 | T | ALS2CR12 | K259N | T | 824 | G | APC | S1201* | C | 3982 | A |
| ACADSB | E266K | G | 810 | A | ADH4 | L291I | G | 1037 | T | ALS2CR8 | P256L | C | 1088 | T | APC | S1355Y | C | 4444 | A |
| ACADSB | E318D | A | 968 | C | ADH4 | V278G | A | 999 | C | ALX1 | P276S | C | 832 | T | APC | S1400L | C | 4579 | T |
| ACADVL | N203D | A | 642 | G | ADHFE1 | S117* | C | 381 | A | ALX1 | A95S | G | 289 | T | APC | T1705A | A | 5493 | G |
| ACAN | R2232H | G | 7069 | A | ADHFE1 | R307M | G | 951 | T | ALX1 | R312* | C | 940 | T | APC | R1920* | C | 6138 | T |
| ACAN | G2365 W | G | 7467 | T | ADHFE1 | A2T | G | 35 | A | ALX1 | R312Q | G | 941 | A | APC | M2047V | A | 6519 | G |
| ACAN | A248T | G | 1116 | A | ADHFE1 | S213L | C | 669 | T | ALX4 | M364T | A | 1098 | G | APC | S2307L | C | 7300 | T |
| ACAN | D1964G | A | 6265 | G | ADIPOQ | E89K | G | 333 | A | ALX4 | R244Q | C | 738 | T | APC | R1450* | C | 4728 | T |
| ACAN | R95W | C | 657 | T | ADIPOQ | D59Y | G | 243 | T | ALX4 | A64T | C | 197 | T | APC | R302* | C | 1284 | T |
| ACAN | E438D | G | 1688 | T | ADK | G108V | G | 373 | T | AMAC1L1 | R90C | C | 389 | T | APC | Q1338* | C | 4392 | T |
| ACAN | F361L | C | 1457 | A | ADM | - | G | 0 | A | AMAC1L2 | T184I | C | 724 | T | APC | R564* | C | 2070 | T |
| ACAN | L758R | T | 2647 | G | ADM | M9L | A | 180 | C | AMAC1L3 | H214R | A | 762 | G | APC | Q1378* | C | 4512 | T |

| | | | | | | | | | | | | | | | | | | | |
|---|---|---|---|---|---|---|---|---|---|---|---|---|---|---|---|---|---|---|---|
| ACAP1 | E505D | G | 1721 | T | ADNP | K266N | T | 1118 | G | AMBN | L21M | C | 164 | A | APC | R499* | C | 1875 | T |
| ACAP1 | R535H | G | 1810 | A | ADNP | A529T | C | 1905 | T | AMBN | S73Y | C | 321 | A | APC2 | Y1795C | A | 5592 | G |
| ACAP1 | K185T | A | 760 | C | ADNP | P993L | G | 3298 | A | AMBN | E264* | G | 893 | T | APC2 | L96M | C | 494 | A |
| ACAP2 | R349H | C | 1277 | T | ADNP | I247V | T | 1059 | C | AMBP | W114L | C | 604 | A | APC2 | V1684M | G | 5258 | A |
| ACAP2 | N111D | T | 562 | C | ADNP2 | H233D | C | 1152 | G | AMBP | R349C | G | 1308 | A | APCDD1 | R564H | G | 1691 | A |
| ACAP2 | N92H | T | 505 | G | ADORA1 | S50L | C | 560 | T | AMBP | K88T | T | 526 | G | APCDD1 | R354H | G | 1061 | A |
| ACAP3 | T744M | G | 2434 | A | ADORA1 | P73S | C | 628 | T | AMBRA1 | R755H | C | 2624 | T | APCDD1 | V417L | G | 1249 | T |
| ACAP3 | A683V | G | 2251 | A | ADORA2A | M211T | T | 1082 | C | AMBRA1 | W1236* | C | 4068 | T | APCDD1 | R176W | C | 526 | T |
| ACAP3 | H451Q | G | 1556 | T | ADPGK | L176I | G | 620 | T | AMDHD1 | R141Q | G | 528 | A | APCDD1L | A3S | C | 238 | A |
| ACAP3 | V103M | C | 510 | T | ADPRHL2 | A17S | G | 79 | T | AMDHD1 | V172M | G | 620 | A | APCDD1L | A49V | G | 377 | A |
| ACAP3 | R677C | G | 2232 | A | ADRA1A | K372N | C | 1552 | A | AMDHD1 | V58F | G | 278 | T | APCDD1L | R180W | G | 769 | A |
| ACAP3 | G422S | C | 1467 | T | ADRA1B | L283R | T | 971 | G | AMDHD1 | A107T | G | 425 | A | APCDD1L | V145I | C | 664 | T |
| ACAT1 | R258C | C | 848 | T | ADRA1D | A324T | C | 1087 | T | AMDHD2 | A218V | C | 686 | T | APCDD1L | T340M | G | 1250 | A |
| ACAT1 | G341R | G | 1097 | A | ADRA2B | A140T | C | 418 | T | AMFR | R596H | C | 1996 | T | APCDD1L | F462L | G | 1617 | T |
| ACAT2 | I106M | A | 2078 | G | ADRA2B | V142I | C | 424 | T | AMFR | R352Q | C | 1264 | T | APCS | E155G | A | 561 | G |
| ACBD3 | R223* | G | 722 | A | ADRA2B | E86K | C | 256 | T | AMHR2 | R463H | G | 1466 | A | APEH | L650H | T | 1984 | A |
| ACBD3 | E233Q | C | 752 | G | ADRA2C | L109V | T | 363 | G | AMHR2 | A366T | G | 1174 | A | APEH | F356L | C | 1103 | A |
| ACBD4 | - | A | 0 | G | ADRA2C | A144V | C | 469 | T | AMIGO3 | D477N | C | 1659 | T | APEX1 | A273T | G | 1083 | A |
| ACBD4 | E83D | G | 718 | T | ADRB1 | R165H | G | 580 | A | AMIGO3 | R15H | C | 274 | T | APH1B | K69T | A | 276 | C |
| ACBD5 | E320K | C | 1023 | T | ADRB2 | S396G | A | 1425 | G | AMIGO3 | V454I | C | 1590 | T | API5 | A120V | C | 532 | T |
| ACCN1 | R30H | C | 89 | T | ADRB2 | N398S | A | 1432 | G | AMIGO3 | Q316H | C | 1178 | A | APIP | A121D | G | 362 | T |
| ACCN1 | R198H | C | 1466 | T | ADRB3 | R155W | G | 660 | A | AMIGO3 | L50V | G | 378 | C | APITD1 | S43N | G | 544 | A |
| ACCN1 | F460V | A | 2251 | C | ADRB3 | A107T | C | 516 | T | AMMECR1 | R173C | G | 685 | A | APLF | R41I | G | 293 | T |
| ACCN2 | R190C | C | 797 | T | ADRBK1 | - | G | 0 | T | AMMECR1L | R229H | C | 947 | T | APLF | S164Y | C | 662 | A |
| ACCN2 | T316M | C | 1176 | T | ADRBK1 | F452L | T | 1620 | C | AMMECR1L | - | C | 0 | A | APLF | D334N | G | 1171 | A |
| ACCN2 | C367W | C | 1330 | G | ADRBK1 | R173W | C | 783 | T | AMN1 | M19V | T | 221 | C | APLP1 | V380I | G | 1276 | A |
| ACCN3 | T201A | A | 601 | G | ADRBK2 | R578H | G | 1925 | A | AMOT | R471C | G | 1411 | A | APLP1 | M329V | A | 1123 | G |
| ACCN3 | L359P | T | 1076 | C | ADRBK2 | D500E | T | 1692 | G | AMOT | S175G | T | 523 | C | APLP1 | C181* | C | 681 | A |
| ACCN3 | L299I | C | 895 | A | ADRM1 | A256V | C | 843 | T | AMOT | R446Q | C | 1337 | T | APLP2 | A253V | C | 915 | T |
| ACCN4 | M555R | T | 1678 | G | ADSL | T368M | C | 1132 | T | AMOT | Q180* | G | 538 | A | APLP2 | R494Q | G | 1638 | A |
| ACCN4 | R325H | G | 988 | A | ADSL | P24L | C | 100 | T | AMOT | R99* | G | 295 | A | APOA1 | V10M | C | 394 | T |
| ACCN4 | V643G | T | 1942 | G | ADSS | R430* | G | 1605 | A | AMOT | Y599S | T | 1796 | G | APOA2 | A5T | C | 71 | T |
| ACCN4 | G40R | G | 132 | C | ADSSL1 | E437D | G | 1313 | T | AMOTL1 | P418Q | C | 1423 | A | APOA4 | Q152* | G | 569 | A |
| ACCN5 | P294H | G | 928 | T | AEBP1 | P349T | C | 1350 | A | AMOTL1 | P376S | C | 1296 | T | APOA4 | A183T | C | 662 | T |
| ACCN5 | G247V | C | 787 | A | AEBP1 | A923T | G | 3072 | A | AMOTL1 | - | T | 0 | C | APOA4 | E344K | C | 1145 | T |
| ACCS | R370H | G | 1253 | A | AEBP1 | P322L | C | 1270 | T | AMOTL1 | R569Q | G | 1876 | A | APOA5 | R204H | C | 646 | T |
| ACCS | V293M | G | 1021 | A | AEBP1 | K140N | G | 725 | T | AMOTL2 | G256A | C | 946 | G | APOA5 | A195T | C | 618 | T |
| ACD | L52I | G | 419 | T | AEBP1 | D559Y | G | 1980 | T | AMPD1 | D732N | C | 2242 | T | APOB | S3036Y | G | 9235 | T |

| | | | | | | | | | | | | | | | | | | | |
|---|---|---|---|---|---|---|---|---|---|---|---|---|---|---|---|---|---|---|---|
| ACE | A1252V | C | 3779 | T | AEBP1 | K753N | G | 2564 | T | AMPD1 | V159A | A | 524 | G | APOB | K859N | C | 2705 | A |
| ACE | E788G | A | 2387 | G | AEBP1 | T866I | C | 2902 | T | AMPD1 | D322Y | C | 1012 | A | APOB | R50W | G | 276 | A |
| ACE2 | P284S | G | 953 | A | AES | L25R | A | 113 | C | AMPD2 | V222A | T | 1025 | C | APOB | R1513Q | C | 4666 | T |
| ACE2 | S47C | G | 243 | C | AFAP1 | S124P | A | 643 | G | AMPD2 | R457W | C | 1729 | T | APOB | P4200S | G | 1272 6 | A |
| ACER1 | R258Q | C | 851 | T | AFAP1 | K163N | C | 762 | A | AMPD2 | R546H | G | 1997 | A | APOB | V2997M | C | 9117 | T |
| ACER1 | I173S | A | 596 | C | AFAP1 | P572T | G | 1987 | T | AMPD2 | E550G | A | 2009 | G | APOB | A913V | G | 2866 | A |
| ACHE | P81L | G | 381 | A | AFAP1 | A158T | C | 745 | T | AMPD3 | R171Q | G | 857 | A | APOB | R1513Q | C | 4666 | T |
| ACIN1 | R1034C | G | 3428 | A | AFAP1 | R361H | C | 1355 | T | AMPD3 | R91W | C | 616 | T | APOB | N2035S | T | 6232 | C |
| ACIN1 | L111* | A | 660 | T | AFAP1L1 | R285H | G | 952 | A | AMPD3 | L724M | C | 2515 | A | APOB | - | C | 0 | A |
| ACLY | V785I | C | 2358 | T | AFAP1L1 | V711M | G | 2229 | A | AMPH | F183L | A | 763 | G | APOB | G724C | C | 2298 | A |
| ACLY | A657V | G | 1975 | A | AFAP1L1 | R170S | G | 608 | T | AMPH | L255F | G | 979 | A | APOB | S2980F | G | 9067 | A |
| ACMSD | G24R | G | 206 | A | AFAP1L1 | R436H | G | 1405 | A | AMY2B | T463A | A | 2003 | G | APOB | K1661R | T | 5110 | C |
| ACMSD | A162V | C | 621 | T | AFAP1L2 | L199P | A | 596 | G | ANAPC1 | - | A | 0 | G | APOB | G1181C | C | 3669 | A |
| ACMSD | A162V | C | 621 | T | AFAP1L2 | P669H | G | 2006 | T | ANAPC1 | M347T | A | 1813 | G | APOB | W397* | C | 1319 | T |
| ACN9 | K28T | A | 1217 | C | AFAP1L2 | D176G | T | 527 | C | ANAPC1 | K543T | T | 2401 | G | APOB | G3716C | C | 1127 4 | A |
| ACO1 | V768I | G | 2440 | A | AFAP1L2 | A37V | G | 110 | A | ANAPC2 | R323C | G | 972 | A | APOB | Q1789P | T | 5494 | G |
| ACO1 | W782R | T | 2482 | A | AFF1 | A696T | G | 2352 | A | ANAPC5 | N91H | T | 393 | G | APOB | - | T | 0 | C |
| ACO2 | A106V | C | 334 | T | AFF1 | T898I | C | 2959 | T | ANAPC7 | R424* | G | 1271 | A | APOB | A4481V | G | 1357 0 | A |
| ACO2 | G240S | G | 735 | A | AFF2 | R927C | C | 3258 | T | ANAPC7 | L274M | A | 821 | T | APOB | L4299I | G | 1302 3 | T |
| ACO2 | V668I | G | 2019 | A | AFF2 | A248V | C | 1222 | T | ANGEL1 | A669T | C | 2118 | T | APOB | S3036Y | G | 9235 | T |
| ACO2 | E314K | G | 957 | A | AFF2 | Q14R | A | 520 | G | ANGEL1 | R585H | C | 1867 | T | APOB | H2067N | G | 6327 | T |
| ACOT1 | T202M | C | 913 | T | AFF2 | P239L | C | 1195 | T | ANGEL1 | E639D | C | 2030 | A | APOBEC1 | D75N | C | 244 | T |
| ACOT11 | R314H | G | 1023 | A | AFF2 | S715* | C | 2623 | A | ANGEL2 | V306I | C | 1071 | T | APOBEC2 | D26N | G | 120 | A |
| ACOT11 | A319V | C | 1038 | T | AFF2 | P252S | C | 1233 | T | ANGEL2 | H448R | T | 1498 | C | APOBEC3B | L404I | C | 1265 | A |
| ACOT11 | R184H | G | 633 | A | AFF2 | R926H | G | 3256 | A | ANGPT1 | R494Q | C | 1978 | T | APOBEC3B | I118T | T | 408 | C |
| ACOT11 | G202C | G | 686 | T | AFF3 | R59I | C | 320 | A | ANGPT2 | E212D | C | 1111 | A | APOBEC3C | R67C | C | 239 | T |
| ACOT12 | E119* | C | 384 | A | AFF4 | R831Q | C | 2872 | T | ANGPT2 | S40F | G | 594 | A | APOBEC3C | R66H | G | 237 | A |
| ACOT12 | K114T | T | 370 | G | AFF4 | R17Q | C | 430 | T | ANGPT4 | Q190* | G | 671 | A | APOBEC3C | A131V | C | 432 | T |
| ACOT7 | A277T | C | 976 | T | AFF4 | K747T | T | 2620 | G | ANGPT4 | N158S | T | 576 | C | APOBEC3D | F267S | T | 1207 | C |
| ACOT8 | R86W | G | 347 | A | AFF4 | R560K | C | 2059 | T | ANGPT4 | P50S | G | 251 | A | APOBEC3F | R121H | G | 719 | A |
| ACOX1 | E311V | T | 1222 | A | AFG3L2 | R711I | C | 2302 | A | ANGPTL1 | A256T | C | 1214 | T | APOBEC3G | Y340H | T | 1375 | C |
| ACOX3 | D507N | C | 1597 | T | AFG3L2 | R783Q | C | 2518 | T | ANGPTL1 | N127S | T | 828 | C | APOBEC3H | E152D | G | 532 | T |
| ACOX3 | E283K | C | 925 | T | AFG3L2 | E734* | C | 2370 | A | ANGPTL1 | M427R | A | 1728 | C | APOBEC4 | S74Y | G | 493 | T |
| ACOX3 | V567L | C | 1777 | A | AFG3L2 | R283Q | C | 1018 | T | ANGPTL1 | K377T | T | 1578 | G | APOBEC4 | R36* | G | 378 | A |
| ACOX3 | R334H | C | 1079 | T | AFM | T281M | C | 935 | T | ANGPTL1 | K47N | C | 589 | A | APOE | R56C | C | 332 | T |
| ACOX3 | R328H | C | 1061 | T | AFMID | E250K | G | 757 | A | ANGPTL2 | R124H | C | 989 | T | APOE | S147N | G | 606 | A |

| | | | | | | | | | | | | | | | | | | | |
|---|---|---|---|---|---|---|---|---|---|---|---|---|---|---|---|---|---|---|---|
| ACOXL | E107K | G | 543 | A | AFP | R337I | G | 1026 | T | ANGPTL2 | E37* | C | 727 | A | APOE | R258H | G | 939 | A |
| ACOXL | D474N | G | 1644 | A | AFP | Y60D | T | 194 | G | ANGPTL4 | R136Q | G | 602 | A | APOF | P319T | G | 1033 | T |
| ACOXL | V160I | G | 702 | A | AFP | L179I | C | 551 | A | ANGPTL4 | R336H | G | 1202 | A | APOH | S3Y | G | 67 | T |
| ACOXL | Y86S | A | 481 | C | AFP | T210A | A | 644 | G | ANGPTL4 | R136Q | G | 602 | A | APOL1 | E108G | A | 590 | G |
| ACP1 | Q106* | C | 412 | T | AFP | A317V | C | 966 | T | ANGPTL5 | K95R | T | 882 | C | APOL1 | R319H | G | 1223 | A |
| ACP2 | R161C | G | 598 | A | AFTPH | K333T | A | 1312 | C | ANGPTL6 | R376L | C | 1366 | A | APOL4 | A142T | C | 649 | T |
| ACP2 | R86C | G | 373 | A | AFTPH | R54H | G | 475 | A | ANGPTL6 | V334M | C | 1239 | T | APOL4 | L326P | A | 1202 | G |
| ACP2 | F323L | G | 1086 | T | AFTPH | R736Q | G | 2521 | A | ANGPTL6 | E225D | C | 914 | A | APOL4 | E101* | C | 526 | A |
| ACP6 | R162H | C | 933 | T | AGAP1 | A404T | G | 1706 | A | ANGPTL7 | A262T | G | 1023 | A | APOL5 | M256V | A | 766 | G |
| ACPL2 | G343R | G | 1164 | A | AGAP1 | M697T | T | 2586 | C | ANK1 | S1653Y | G | 5042 | T | APOL5 | G216R | G | 646 | A |
| ACPP | P349T | C | 1095 | A | AGAP1 | A460V | C | 1875 | T | ANK1 | E68* | C | 286 | A | APOO | R113I | C | 570 | A |
| ACPP | K91Q | A | 321 | C | AGAP1 | G754S | G | 2756 | A | ANK1 | R1001H | C | 3086 | T | APOO | - | T | 0 | C |
| ACPP | R105Q | G | 364 | A | AGAP1 | D753N | G | 2753 | A | ANK1 | Q43* | G | 211 | A | APP | G68D | C | 237 | T |
| ACPT | V230I | G | 688 | A | AGAP2 | - | C | 0 | A | ANK1 | V575M | C | 1807 | T | APP | R359Q | C | 1110 | T |
| ACRBP | H108Y | G | 370 | A | AGAP2 | K443N | C | 1329 | A | ANK1 | S1921C | G | 5846 | C | APP | R469H | C | 1440 | T |
| ACRC | R664C | C | 2104 | T | AGAP3 | R513C | C | 1537 | T | ANK1 | D184N | C | 634 | T | APPBP2 | E569D | T | 1995 | G |
| ACRC | K308N | G | 1038 | T | AGAP3 | G271R | G | 811 | A | ANK1 | - | C | 0 | T | APPL1 | S496Y | C | 1634 | A |
| ACRC | S186L | C | 671 | T | AGAP3 | S305L | C | 914 | T | ANK1 | F1802C | A | 5489 | C | APPL1 | R505Q | G | 1661 | A |
| ACRV1 | R257Q | C | 1037 | T | AGAP3 | R481H | G | 1442 | A | ANK1 | E1232* | C | 3778 | A | APPL1 | P264H | C | 938 | A |
| ACRV1 | S260Y | G | 1046 | T | AGAP3 | P343T | C | 1027 | A | ANK1 | R281L | C | 926 | A | APPL2 | - | A | 0 | G |
| ACRV1 | R257Q | C | 1037 | T | AGAP3 | V311M | G | 931 | A | ANK1 | R39* | G | 397 | A | APPL2 | I83T | A | 466 | G |
| ACSBG1 | Y138N | A | 474 | T | AGAP3 | A661T | G | 1981 | A | ANK2 | R506H | G | 1570 | A | APPL2 | E614* | C | 2058 | A |
| ACSBG1 | R565H | C | 1756 | T | AGAP3 | N491H | A | 1471 | C | ANK2 | D3948N | G | 11895 | A | APPL2 | K613T | T | 2056 | G |
| ACSBG1 | K447N | C | 1403 | A | AGAP6 | H132R | A | 793 | G | ANK2 | R2052* | C | 6207 | T | APPL2 | T561N | G | 1900 | T |
| ACSBG1 | R437C | G | 1371 | A | AGAP6 | R137C | C | 807 | T | ANK2 | G1783C | G | 5400 | T | APRT | D35G | T | 139 | C |
| ACSBG1 | L141R | A | 484 | C | AGBL2 | G635R | C | 2003 | T | ANK2 | S3428T | T | 10335 | A | APTX | R56G | G | 166 | C |
| ACSBG2 | E79K | G | 477 | A | AGBL2 | K810T | T | 2529 | G | ANK2 | T675A | A | 2076 | G | APTX | E348K | C | 1042 | T |
| ACSBG2 | L386F | C | 1398 | T | AGBL3 | D139N | G | 668 | A | ANK2 | A1809V | C | 5479 | T | AQP10 | G138D | G | 453 | A |
| ACSBG2 | I283M | C | 1091 | G | AGBL3 | R441Q | G | 1575 | A | ANK2 | F3103S | T | 9361 | C | AQP10 | P101T | C | 341 | A |
| ACSBG2 | A225T | G | 915 | A | AGBL3 | R906M | G | 2970 | T | ANK2 | T3222A | A | 9717 | G | AQP10 | P150T | C | 488 | A |
| ACSF2 | L27P | T | 123 | C | AGBL3 | T972A | A | 3167 | G | ANK2 | F3258C | T | 9826 | G | AQP12B | A38T | C | 175 | T |
| ACSF2 | Q166* | C | 539 | T | AGBL3 | F29C | T | 339 | G | ANK2 | T1863M | C | 5641 | T | AQP2 | H177N | C | 619 | A |
| ACSF2 | A357T | G | 1112 | A | AGBL3 | R319W | C | 1208 | T | ANK3 | S1894Y | G | 5873 | T | AQP3 | F220V | A | 739 | C |
| ACSF2 | R631* | C | 1934 | T | AGBL3 | D458E | T | 1627 | G | ANK3 | R805C | G | 2605 | A | AQP4 | A192T | C | 670 | T |
| ACSF3 | A131V | C | 769 | T | AGBL4 | S282F | G | 962 | A | ANK3 | E864K | C | 2782 | T | AQP5 | G191D | G | 1094 | A |
| ACSF3 | R388H | G | 1540 | A | AGBL4 | A353T | C | 1174 | T | ANK3 | A2382V | G | 7337 | A | AQP6 | F39L | C | 454 | A |
| ACSL1 | R237* | G | 1035 | A | AGBL4 | I160S | A | 596 | C | ANK3 | I16N | A | 239 | T | AQP8 | A143T | G | 552 | A |
| ACSL1 | E131G | T | 718 | C | AGBL4 | N84T | T | 368 | G | ANK3 | E4317D | C | 13143 | A | AQP8 | R213H | G | 763 | A |
| ACSL1 | - | T | 0 | C | AGBL5 | A240T | G | 877 | A | ANK3 | K4305T | T | 13106 | G | AQP8 | T254A | A | 885 | G |

| | | | | | | | | | | | | | | | | | | | |
|---|---|---|---|---|---|---|---|---|---|---|---|---|---|---|---|---|---|---|---|
| ACSL1 | F617C | A | 2176 | C | AGBL5 | R699Q | G | 2255 | A | ANK3 | R2596C | G | 7978 | A | AQP9 | E287D | G | 1124 | T |
| ACSL3 | K720R | A | 2690 | G | AGBL5 | M764L | A | 2449 | C | ANK3 | S1806L | G | 5609 | A | AQP9 | T26P | A | 339 | C |
| ACSL3 | S226Y | C | 1208 | A | AGBL5 | S425N | G | 1433 | A | ANKAR | L958I | C | 2872 | A | AQR | V15A | A | 186 | G |
| ACSL4 | R133H | C | 795 | T | AGBL5 | V695I | G | 2242 | A | ANKDD1A | E16D | G | 77 | T | AQR | R1288H | C | 4005 | T |
| ACSL4 | Y394C | T | 1578 | C | AGGF1 | R688Q | G | 2445 | A | ANKDD1A | L310I | C | 957 | A | AQR | R905I | C | 2856 | A |
| ACSL4 | R45I | C | 531 | A | AGGF1 | K583N | G | 2131 | T | ANKFN1 | R260H | G | 814 | A | AQR | A668V | G | 2145 | A |
| ACSL5 | - | G | 0 | T | AGGF1 | E616* | G | 2228 | T | ANKFN1 | S649Y | C | 1981 | A | AR | S244L | C | 1255 | T |
| ACSL6 | V505I | C | 1622 | T | AGGF1 | K646N | A | 2320 | C | ANKFY1 | R747H | C | 2278 | T | AR | V478A | T | 1957 | C |
| ACSL6 | I611L | T | 1940 | G | AGK | R9Q | G | 286 | A | ANKFY1 | A259T | C | 813 | T | AR | Q75L | A | 748 | T |
| ACSL6 | N181K | A | 652 | T | AGK | R351* | C | 1311 | T | ANKFY1 | V162L | C | 522 | A | ARAF | V525L | G | 1705 | T |
| ACSL6 | D400N | C | 1307 | T | AGL | L1060I | C | 3653 | A | ANKH | G429S | C | 1616 | T | ARAP1 | R76H | C | 430 | T |
| ACSM1 | D435Y | C | 1371 | A | AGL | V211I | G | 1106 | A | ANKH | S123L | G | 699 | A | ARAP1 | L608M | G | 2025 | T |
| ARAP2 | K1496N | C | 4978 | A | ATAD2 | L938I | G | 2920 | T | BAIAP2L2 | V93M | C | 422 | T | BTBD17 | D440N | C | 1318 | T |
| ARAP2 | Y139H | A | 905 | G | ATAD2 | E909* | C | 2833 | A | BAIAP3 | A1185V | C | 3564 | T | BTBD18 | G624E | C | 2160 | T |
| ARAP3 | D1420V | T | 4325 | A | ATAD2 | R792Q | C | 2483 | T | BAMBI | G108W | G | 878 | T | BTBD18 | C478* | A | 1723 | T |
| ARAP3 | P4H | G | 77 | T | ATAD2B | M497T | A | 1834 | G | BANK1 | R730M | G | 2463 | T | BTBD2 | A252T | C | 754 | T |
| ARAP3 | E1312* | C | 4000 | A | ATAD3A | K313N | G | 1033 | T | BANK1 | R515* | C | 1817 | T | BTBD3 | L218H | T | 653 | A |
| ARAP3 | R308H | C | 989 | T | ATAD3A | D378N | G | 1226 | A | BANK1 | E46K | G | 410 | A | BTBD3 | R473Q | G | 1418 | A |
| ARAP3 | R37Q | C | 176 | T | ATAD3B | R342W | C | 1140 | T | BANP | V229A | T | 838 | C | BTBD7 | T667M | G | 2326 | A |
| ARC | Q243H | C | 930 | A | ATAD3B | - | G | 0 | T | BAP1 | G128* | C | 854 | A | BTBD7 | T667M | G | 2326 | A |
| ARC | V68I | C | 403 | T | ATAD5 | - | T | 0 | C | BARD1 | - | C | 0 | T | BTBD7 | F973C | A | 3244 | C |
| ARCN1 | R476Q | G | 1592 | A | ATAD5 | K1453Q | A | 4735 | C | BARD1 | H324N | G | 1105 | T | BTBD8 | R216C | C | 882 | T |
| ARF1 | R19C | C | 198 | T | ATAD5 | R1448H | G | 4721 | A | BARHL1 | D169G | A | 698 | G | BTBD9 | R257* | G | 921 | A |
| ARF4 | W78G | A | 500 | C | ATAD5 | K425T | A | 1652 | C | BARHL1 | D10N | G | 220 | A | BTBD9 | C162R | A | 636 | G |
| ARFGAP1 | A158T | G | 593 | A | ATCAY | A148T | G | 872 | A | BARHL2 | A238T | C | 754 | T | BTBD9 | D201Y | C | 753 | A |
| ARFGAP1 | P153Q | C | 579 | A | ATCAY | V13M | G | 467 | A | BARHL2 | E187K | C | 601 | T | BTBD9 | D37N | C | 261 | T |
| ARFGAP3 | G308* | C | 1142 | A | ATE1 | R56Q | C | 254 | T | BARX1 | R124H | C | 545 | T | BTD | P135L | C | 492 | T |
| ARFGEF1 | S1593P | A | 5167 | G | ATF1 | D70Y | G | 486 | T | BASP1 | E34G | A | 361 | G | BTD | A103T | G | 395 | A |
| ARFGEF1 | M725I | C | 2565 | T | ATF3 | R156Q | G | 600 | A | BASP1 | E50* | G | 408 | T | BTD | R211C | C | 719 | T |
| ARFGEF1 | S495L | G | 1874 | A | ATF5 | R70Q | G | 776 | A | BAT2L1 | A441V | C | 1377 | T | BTD | F18Y | T | 141 | A |
| ARFGEF1 | Q1490R | T | 4859 | C | ATF6 | H488Y | C | 1529 | T | BAT2L1 | D1005N | G | 3068 | A | BTG3 | R17* | G | 203 | A |
| ARFGEF1 | R1253* | G | 4147 | A | ATF6 | E346G | A | 1104 | G | BAT2L1 | V1673I | G | 5072 | A | BTG3 | K90N | C | 424 | A |
| ARFGEF1 | N584H | T | 2140 | G | ATF6 | G18E | G | 120 | A | BAT2L1 | R1067W | C | 3254 | T | BTG3 | F65C | A | 348 | C |
| ARFGEF2 | R1348Q | G | 4043 | A | ATF6 | R317* | C | 1016 | T | BAT2L2 | F2032S | T | 6361 | C | BTK | R288Q | C | 1027 | T |
| ARFGEF2 | E73K | G | 217 | A | ATF6B | A623V | G | 1901 | A | BAT2L2 | R2825Q | G | 8740 | A | BTN1A1 | A302T | G | 970 | A |
| ARFGEF2 | R1109C | C | 3325 | T | ATF6B | P170L | G | 542 | A | BAT2L2 | S880P | T | 2904 | C | BTN1A1 | A103T | G | 373 | A |
| ARFGEF2 | S1213T | T | 3637 | A | ATF6B | S8N | C | 56 | T | BAT2L2 | E364K | G | 1356 | A | BTN1A1 | A395V | C | 1250 | T |
| ARFGEF2 | P27H | C | 80 | A | ATF7 | E377* | C | 1287 | A | BATF | S24F | C | 313 | T | BTN2A1 | A136T | G | 618 | A |
| ARFGEF2 | T1597M | C | 4790 | T | ATF7 | P410L | G | 1387 | A | BATF2 | R33* | G | 228 | A | BTN2A1 | L427P | T | 1492 | C |

| | | | | | | | | | | | | | | | | | | | |
|---|---|---|---|---|---|---|---|---|---|---|---|---|---|---|---|---|---|---|---|
| ARFGEF2 | S1266P | T | 3796 | C | ATF7IP | R696K | G | 2240 | A | BAX | L113P | T | 338 | C | BTN2A1 | Q295K | C | 1095 | A |
| ARFGEF2 | K1727R | A | 5180 | G | ATF7IP | P159A | C | 628 | G | BAX | L125M | C | 373 | A | BTN2A2 | R143C | C | 538 | T |
| ARFGEF2 | S218F | C | 653 | T | ATF7IP | P159A | C | 628 | G | BAZ1A | S1119Y | G | 3684 | T | BTN2A2 | K294N | G | 993 | T |
| ARFIP2 | F182L | A | 683 | C | ATF7IP | G46D | G | 290 | A | BAZ1A | W530* | C | 1918 | T | BTN3A3 | E366G | A | 1340 | G |
| ARG1 | C53Y | G | 216 | A | ATF7IP | R1098* | C | 3445 | T | BAZ1A | R1511H | C | 4860 | T | BTNL3 | A283S | G | 1031 | T |
| ARG1 | R79M | G | 294 | T | ATF7IP2 | A361S | G | 1302 | T | BAZ1A | K575N | T | 2053 | G | BTNL9 | A112S | G | 565 | T |
| ARGFX | S84Y | C | 261 | A | ATF7IP2 | G510C | G | 1749 | T | BAZ1A | K940T | T | 3147 | G | BTNL9 | S470P | T | 1639 | C |
| ARGFX | N106T | A | 327 | C | ATG10 | R139* | C | 709 | T | BAZ1B | R199Q | C | 935 | T | BTRC | A47T | G | 257 | A |
| ARHGAP10 | V348I | G | 1281 | A | ATG10 | E5* | G | 307 | T | BAZ1B | V1167M | C | 3838 | T | BTRC | R560* | C | 1796 | T |
| ARHGAP10 | V406I | G | 1455 | A | ATG12 | L166V | G | 613 | C | BAZ1B | R1452M | C | 4694 | A | BUB1 | T461A | T | 1500 | C |
| ARHGAP10 | F538L | T | 1851 | C | ATG16L1 | K41I | A | 236 | T | BAZ1B | R1238H | C | 4052 | T | BUB1 | D937G | T | 2929 | C |
| ARHGAP10 | P677T | C | 2268 | A | ATG16L1 | R303C | C | 1021 | T | BAZ2A | Q719* | G | 2349 | A | BUB1 | K566N | C | 1817 | A |
| ARHGAP10 | P281L | C | 1081 | T | ATG16L1 | A599V | C | 1910 | T | BAZ2A | R1492M | C | 4669 | A | BUB1 | R244C | G | 849 | A |
| ARHGAP10 | V348I | G | 1281 | A | ATG16L1 | G547V | G | 1754 | T | BAZ2A | P122T | G | 558 | T | BUB1B | L165R | T | 706 | G |
| ARHGAP10 | F558L | T | 1913 | G | ATG16L1 | R439Q | G | 1430 | A | BAZ2A | R1793W | G | 5571 | A | BUB1B | E469* | G | 1617 | T |
| ARHGAP10 | E180K | G | 777 | A | ATG16L1 | T462A | A | 1498 | G | BAZ2B | R1938* | G | 6308 | A | BUB3 | L30M | C | 297 | A |
| ARHGAP10 | R400I | G | 1438 | T | ATG16L1 | K507N | G | 1635 | T | BAZ2B | S604Y | G | 2307 | T | BUB3 | C62G | T | 393 | G |
| ARHGAP11 A | R839H | G | 3238 | A | ATG2A | R574C | G | 1835 | A | BAZ2B | R210* | G | 1124 | A | BUD13 | H157R | T | 494 | C |
| ARHGAP11 A | N978I | A | 3655 | T | ATG2A | P656S | G | 2081 | A | BAZ2B | A293S | C | 1373 | A | BUD13 | P327L | G | 1004 | A |
| ARHGAP11 A | M614I | G | 2564 | A | ATG2A | P1552L | G | 4770 | A | BAZ2B | G764W | C | 2786 | A | BUD13 | E608* | C | 1846 | A |
| ARHGAP11 A | K152N | G | 1178 | T | ATG2A | R400H | C | 1314 | T | BAZ2B | R1384C | G | 4646 | A | BVES | L261I | G | 938 | T |
| ARHGAP12 | E792* | C | 2609 | A | ATG2A | E1349* | C | 4160 | A | BAZ2B | T577A | T | 2225 | C | BZRAP1 | E1269K | C | 4676 | T |
| ARHGAP12 | S516P | A | 1781 | G | ATG2B | A1715V | G | 6038 | A | BAZ2B | S247R | A | 1237 | C | BZRAP1 | W1555R | A | 5534 | G |
| ARHGAP12 | G279V | C | 1071 | A | ATG2B | R1651* | G | 5845 | A | BBOX1 | A100V | C | 667 | T | BZRAP1 | R561Q | C | 2553 | T |
| ARHGAP12 | K484N | C | 1687 | A | ATG2B | K1374T | T | 5015 | G | BBS1 | R490W | C | 1530 | T | BZRAP1 | - | T | 0 | C |
| ARHGAP12 | R208I | C | 858 | A | ATG4B | D275N | G | 823 | A | BBS10 | N543S | T | 1697 | C | BZW2 | - | G | 0 | T |
| ARHGAP15 | V408F | G | 1389 | T | ATG4B | R492* | C | 1474 | T | BBS12 | R368C | C | 1295 | T | C10orf10 | R4W | G | 228 | A |
| ARHGAP15 | R27I | G | 247 | T | ATG4C | R192Q | G | 782 | A | BBS12 | F544L | T | 1825 | G | C10orf103 | G22E | G | 65 | A |
| ARHGAP15 | D48N | G | 309 | A | ATG4D | R102H | G | 479 | A | BBS12 | D687G | A | 2253 | G | C10orf105 | P95L | G | 284 | A |
| ARHGAP15 | R200I | G | 766 | T | ATG4D | R339Q | G | 1190 | A | BBS2 | R315W | G | 1364 | A | C10orf107 | E142* | G | 729 | T |
| ARHGAP15 | E335* | G | 1170 | T | ATG5 | E179* | C | 859 | A | BBS2 | G375D | C | 1545 | T | C10orf107 | F11V | T | 336 | G |
| ARHGAP17 | D471V | T | 1505 | A | ATG7 | K667N | G | 2026 | T | BBS4 | A504V | C | 1552 | T | C10orf113 | A5T | C | 33 | T |
| ARHGAP19 | K259E | T | 804 | C | ATG9A | E541* | C | 1833 | A | BBS4 | N375H | A | 1164 | C | C10orf114 | D122G | T | 813 | C |
| ARHGAP20 | A1077V | G | 3515 | A | ATG9A | R758H | C | 2485 | T | BBS5 | N112H | A | 408 | C | C10orf118 | E882* | C | 2911 | A |
| ARHGAP20 | Y665C | T | 2279 | C | ATG9A | V87M | C | 471 | T | BBS5 | N379S | A | 1210 | G | C10orf118 | E88D | T | 531 | G |
| ARHGAP20 | - | C | 0 | A | ATIC | R172H | G | 841 | A | BBS7 | I649V | T | 2129 | C | C10orf118 | R685C | G | 2320 | A |

| | | | | | | | | | | | | | | | | | | | |
|---|---|---|---|---|---|---|---|---|---|---|---|---|---|---|---|---|---|---|---|
| ARHGAP20 | - | T | 0 | C | ATIC | G439R | G | 1641 | A | BBS7 | Y558* | G | 1858 | T | C10orf118 | Y202* | A | 873 | C |
| ARHGAP20 | K816R | T | 2732 | C | ATIC | C241Y | G | 1048 | A | BBS9 | I451L | A | 1872 | T | C10orf119 | M285I | C | 1025 | A |
| ARHGAP20 | G1069* | C | 3490 | A | ATIC | L131V | T | 717 | G | BBS9 | R173I | G | 1039 | T | C10orf119 | F229V | A | 855 | C |
| ARHGAP20 | D987Y | C | 3244 | A | ATIC | T182M | C | 871 | T | BBX | E879K | G | 2922 | A | C10orf119 | A210V | G | 799 | A |
| ARHGAP20 | Y806H | A | 2701 | G | ATIC | G62R | G | 510 | A | BBX | F409S | T | 1513 | C | C10orf12 | R207C | C | 726 | T |
| ARHGAP21 | E1812K | C | 5921 | T | ATIC | V153M | G | 783 | A | BBX | G590* | G | 2055 | T | C10orf12 | N971I | A | 3019 | T |
| ARHGAP21 | V1815I | C | 5930 | T | ATIC | E336* | G | 1332 | T | BCAN | T430M | C | 1625 | T | C10orf12 | - | A | 3851 | T |
| ARHGAP21 | L311F | T | 1420 | A | ATIC | S475L | C | 1750 | T | BCAN | G51S | G | 487 | A | C10orf12 | V1039I | G | 3222 | A |
| ARHGAP21 | R1768W | G | 5789 | A | ATL1 | R55Q | G | 405 | A | BCAN | R599W | C | 2131 | T | C10orf12 | D199E | C | 704 | G |
| ARHGAP21 | V1147I | C | 3926 | T | ATL1 | G122* | G | 605 | T | BCAN | T714A | A | 2476 | G | C10orf12 | E487K | G | 1566 | A |
| ARHGAP21 | K641T | T | 2409 | G | ATL2 | K417Q | T | 1251 | G | BCAP29 | K96* | A | 337 | T | C10orf122 | R76W | G | 381 | A |
| ARHGAP21 | S637* | G | 2397 | T | ATL3 | K357T | T | 1070 | G | BCAP29 | L64I | C | 241 | A | C10orf122 | R160I | C | 634 | A |
| ARHGAP22 | R610H | C | 1985 | T | ATM | R13H | G | 423 | A | BCAP29 | R119C | C | 406 | T | C10orf128 | Q145H | T | 435 | G |
| ARHGAP22 | R449Q | C | 1502 | T | ATM | F1445C | T | 4719 | G | BCAR1 | R612W | G | 1834 | A | C10orf129 | S217R | A | 774 | C |
| ARHGAP22 | A367T | C | 1255 | T | ATM | Y2124C | A | 6756 | G | BCAR1 | V557I | C | 1669 | T | C10orf137 | A871T | G | 2843 | A |
| ARHGAP23 | A1010T | G | 3028 | A | ATM | Y2404D | T | 7595 | G | BCAR1 | G322C | C | 964 | A | C10orf137 | - | G | 0 | T |
| ARHGAP24 | S369N | G | 1572 | A | ATM | T915A | A | 3128 | G | BCAR1 | H349R | T | 1046 | C | C10orf137 | V143M | G | 659 | A |
| ARHGAP25 | A265V | C | 1159 | T | ATM | R2763* | C | 8672 | T | BCAR3 | R80W | G | 481 | A | C10orf137 | E29K | G | 317 | A |
| ARHGAP25 | R378* | C | 1497 | T | ATM | R248L | G | 1128 | T | BCAR3 | R800I | C | 2642 | A | C10orf137 | E46* | G | 368 | T |
| ARHGAP25 | M139T | T | 781 | C | ATM | C977Y | G | 3315 | A | BCAS1 | T153A | T | 795 | C | C10orf137 | R306W | C | 1148 | T |
| ARHGAP26 | A383T | G | 1525 | A | ATM | R1466Q | G | 4782 | A | BCAS1 | A73V | G | 556 | A | C10orf140 | E444G | T | 3584 | C |
| ARHGAP26 | L145S | T | 812 | C | ATM | K24T | A | 456 | C | BCAS1 | F565L | G | 2033 | T | C10orf140 | G429E | C | 3539 | T |
| ARHGAP26 | R501W | C | 1879 | T | ATM | - | T | 0 | C | BCAS3 | D794E | T | 2382 | A | C10orf140 | F173V | A | 2770 | C |
| ARHGAP26 | A512P | G | 1912 | C | ATM | Q2297* | C | 7274 | T | BCAS3 | P521L | C | 1562 | T | C10orf18 | G1024D | G | 3696 | A |
| ARHGAP27 | A646V | G | 1937 | A | ATM | N2875S | A | 9009 | G | BCAS4 | L136F | C | 506 | T | C10orf18 | R1579I | G | 5361 | T |
| ARHGAP27 | M864V | T | 2590 | C | ATM | V734A | T | 2586 | C | BCAT1 | G14R | C | 482 | T | C10orf18 | R2278C | C | 7457 | T |
| ARHGAP28 | A502V | C | 1505 | T | ATM | G449V | G | 1731 | T | BCAT1 | I141T | A | 864 | G | C10orf27 | A8D | G | 413 | T |
| ARHGAP28 | L385R | T | 1154 | G | ATM | I352N | T | 1440 | A | BCAT1 | K107T | T | 762 | G | C10orf28 | G120E | G | 484 | A |
| ARHGAP28 | R143* | C | 427 | T | ATM | R805* | C | 2798 | T | BCCIP | V162A | T | 497 | C | C10orf28 | S54R | A | 285 | C |
| ARHGAP29 | A1150T | C | 3631 | T | ATM | R1730* | C | 5573 | T | BCCIP | R151C | C | 463 | T | C10orf28 | D154N | G | 585 | A |
| ARHGAP29 | A959V | G | 3059 | A | ATM | L1826V | T | 5861 | G | BCDIN3D | S77P | A | 234 | G | C10orf28 | E654* | G | 2085 | T |
| ARHGAP29 | V1202M | C | 3787 | T | ATM | G2083* | G | 6632 | T | BCHE | - | A | 0 | G | C10orf35 | R59Q | G | 176 | A |
| ARHGAP29 | R13C | G | 220 | A | ATM | Q1537L | A | 4995 | T | BCHE | K208T | T | 790 | G | C10orf46 | F252C | A | 1239 | C |
| ARHGAP29 | L598I | G | 1975 | T | ATMIN | Q385R | A | 1172 | G | BCHE | S100I | C | 466 | A | C10orf53 | G137C | G | 421 | T |
| ARHGAP29 | E476* | C | 1609 | A | ATMIN | N750H | A | 2266 | C | BCKDHA | R178H | G | 533 | A | C10orf54 | H126R | T | 436 | C |
| ARHGAP29 | E387* | C | 1342 | A | ATN1 | D104N | G | 547 | A | BCKDHA | A224T | G | 670 | A | C10orf55 | S38L | G | 454 | A |
| ARHGAP30 | R304H | C | 1257 | T | ATN1 | R884H | G | 2888 | A | BCKDHA | R102Q | G | 305 | A | C10orf62 | A149V | C | 617 | T |
| ARHGAP30 | R445H | C | 1680 | T | ATN1 | Q498H | G | 1731 | T | BCKDHA | Y348C | A | 1043 | G | C10orf62 | R139W | C | 586 | T |
| ARHGAP31 | S688L | C | 2595 | T | ATOH7 | E146D | C | 859 | A | BCKDHB | R111* | C | 378 | T | C10orf67 | I45K | A | 134 | T |

| | | | | | | | | | | | | | | | | | | | |
|---|---|---|---|---|---|---|---|---|---|---|---|---|---|---|---|---|---|---|---|
| ARHGAP31 | S17N | G | 582 | A | ATOH8 | P137H | C | 706 | A | BCKDHB | A5T | G | 60 | A | C10orf68 | L61I | C | 654 | A |
| ARHGAP31 | P673S | C | 2549 | T | ATP10A | D975N | C | 3029 | T | BCKDK | A358T | G | 1695 | A | C10orf68 | K12T | A | 508 | C |
| ARHGAP31 | S526Y | C | 2109 | A | ATP10A | E221K | C | 767 | T | BCL11A | R826* | G | 2704 | A | C10orf71 | P1394L | C | 4469 | T |
| ARHGAP31 | F861V | T | 3113 | G | ATP10A | A804V | G | 2517 | A | BCL11A | A532V | G | 1823 | A | C10orf71 | A426V | C | 1565 | T |
| ARHGAP31 | R1231M | G | 4224 | T | ATP10A | F572L | G | 1822 | T | BCL11A | P353S | G | 1285 | A | C10orf72 | R208W | G | 646 | A |
| ARHGAP32 | A1375T | C | 4123 | T | ATP10A | Y390C | T | 1275 | C | BCL11A | F17L | G | 279 | T | C10orf72 | R100C | G | 322 | A |
| ARHGAP32 | A412V | G | 1235 | A | ATP10A | A1042V | G | 3231 | A | BCL11B | R472H | C | 1425 | T | C10orf76 | G577D | C | 1850 | T |
| ARHGAP32 | F370L | A | 1108 | G | ATP10A | A1284T | C | 3956 | T | BCL11B | D857N | C | 2579 | T | C10orf76 | G245R | C | 853 | T |
| ARHGAP32 | V563I | C | 1687 | T | ATP10A | A1401V | G | 4308 | A | BCL11B | E535D | C | 1615 | G | C10orf78 | S280G | A | 838 | G |
| ARHGAP32 | Q141H | C | 423 | A | ATP10A | - | C | 0 | G | BCL11B | G83S | C | 257 | T | C10orf79 | R1314* | G | 4056 | A |
| ARHGAP32 | P1351T | G | 4051 | T | ATP10A | A1211T | C | 3737 | T | BCL11B | S468N | C | 1413 | T | C10orf79 | Q790* | G | 2484 | A |
| ARHGAP32 | P1348S | G | 4042 | A | ATP10A | F1300S | A | 4005 | G | BCL11B | S358A | A | 1082 | C | C10orf79 | S296L | G | 1003 | A |
| ARHGAP33 | R933C | C | 2882 | T | ATP10A | E696D | C | 2194 | A | BCL11B | P268T | G | 812 | T | C10orf79 | S1002F | G | 3121 | A |
| ARHGAP36 | R170W | C | 853 | T | ATP10A | R968H | C | 3009 | T | BCL11B | G415D | C | 1254 | T | C10orf79 | G107C | C | 435 | A |
| ARHGAP36 | P21H | C | 407 | A | ATP10A | D1271E | G | 3919 | T | BCL2A1 | A123V | G | 695 | A | C10orf79 | F341V | A | 1137 | C |
| ARHGAP39 | G774C | C | 2405 | A | ATP10A | - | C | 0 | A | BCL2A1 | E47* | C | 466 | A | C10orf81 | P271H | C | 1374 | A |
| ARHGAP39 | R737W | G | 2294 | A | ATP10A | R977K | C | 3036 | T | BCL2L10 | W163C | C | 538 | A | C10orf81 | R213Q | G | 1200 | A |
| ARHGAP39 | D326N | C | 1061 | T | ATP10A | F954L | G | 2968 | T | BCL2L10 | L188M | G | 611 | T | C10orf90 | R619C | G | 1976 | A |
| ARHGAP39 | R94W | G | 365 | A | ATP10A | R850C | G | 2654 | A | BCL2L11 | P21L | C | 335 | T | C10orf90 | A84T | C | 371 | T |
| ARHGAP39 | V59I | C | 260 | T | ATP10A | R793W | G | 2483 | A | BCL2L14 | G261R | G | 987 | A | C10orf90 | A84T | C | 371 | T |
| ARHGAP39 | R933C | G | 2882 | A | ATP10A | R590Q | C | 1875 | T | BCL3 | C115Y | G | 588 | A | C10orf92 | T2432I | G | 7634 | A |
| ARHGAP39 | P1055S | G | 3248 | A | ATP10A | V230M | C | 794 | T | BCL3 | R264C | C | 1034 | T | C10orf92 | A201T | C | 940 | T |
| ARHGAP39 | G446R | C | 1421 | T | ATP10B | R1363* | G | 4934 | A | BCL6 | R594Q | C | 2148 | T | C10orf92 | F2479L | G | 7776 | T |
| ARHGAP4 | E209A | T | 684 | G | ATP10B | Q1146H | C | 4285 | A | BCL6 | A410T | C | 1595 | T | C10orf92 | A2243T | C | 7066 | T |
| ARHGAP40 | V219M | G | 655 | A | ATP10B | P1388H | G | 5010 | T | BCL6 | D338G | T | 1380 | C | C10orf92 | A1936V | G | 6146 | A |
| ARHGAP40 | E456K | G | 1366 | A | ATP10B | M371T | A | 1959 | G | BCL6 | R550H | C | 2016 | T | C10orf92 | K2386N | T | 7497 | G |
| ARHGAP40 | R608C | C | 1822 | T | ATP10B | R835H | C | 3351 | T | BCL6 | R594Q | C | 2148 | T | C10orf92 | F2192L | G | 6915 | T |
| ARHGAP40 | K481T | A | 1442 | C | ATP10B | Q894H | C | 3529 | A | BCL6 | C354Y | C | 1428 | T | C10orf96 | R35H | G | 355 | A |
| ARHGAP42 | Q702R | A | 2108 | G | ATP10B | R977H | C | 3777 | T | BCL6B | A369S | G | 1167 | T | C11orf1 | R67H | G | 220 | A |
| ARHGAP5 | A808S | G | 2737 | T | ATP10D | R897C | C | 2958 | T | BCL7A | T52M | C | 292 | T | C11orf16 | K22N | C | 190 | A |
| ARHGAP5 | K220N | G | 975 | T | ATP11A | N340D | A | 1106 | G | BCL7A | R44Q | G | 268 | A | C11orf16 | S16R | G | 172 | T |
| ARHGAP5 | K254N | A | 1077 | C | ATP11A | G491S | G | 1559 | A | BCL7B | D179Y | C | 959 | A | C11orf16 | V17M | C | 173 | T |
| ARHGAP5 | F276C | T | 1142 | G | ATP11A | A602T | G | 1892 | A | BCL9 | E802* | G | 3144 | T | C11orf2 | L325M | C | 1013 | A |
| ARHGAP6 | R58W | G | 1045 | A | ATP11A | T269M | C | 894 | T | BCL9 | P1201T | C | 4341 | A | C11orf2 | L674I | C | 2060 | A |
| ARHGAP9 | R631Q | C | 2085 | T | ATP11A | Y233D | T | 785 | G | BCL9L | D261N | C | 1746 | T | C11orf21 | P124H | G | 423 | T |
| ARHGAP9 | R516G | T | 1739 | C | ATP11B | E659D | G | 2237 | T | BCL9L | A385T | C | 2118 | T | C11orf24 | A182T | C | 986 | T |
| ARHGAP9 | S371L | G | 1305 | A | ATP11B | L462S | T | 1645 | C | BCLAF1 | R39H | C | 369 | T | C11orf30 | E793G | A | 2521 | G |
| ARHGAP9 | Q714P | T | 2334 | G | ATP11B | D341Y | G | 1281 | T | BCLAF1 | R429W | G | 1538 | A | C11orf30 | S1213L | C | 3781 | T |
| ARHGDIB | K49N | C | 251 | A | ATP11C | K52E | T | 253 | C | BCLAF1 | K483N | T | 1702 | G | C11orf35 | R241Q | C | 785 | T |

| | | | | | | | | | | | | | | | | | | | |
|---|---|---|---|---|---|---|---|---|---|---|---|---|---|---|---|---|---|---|---|
| ARHGEF1 | P843L | C | 2528 | T | ATP11C | R284H | C | 950 | T | BCLAF1 | R94I | C | 534 | A | C11orf40 | S117F | G | 350 | A |
| ARHGEF1 | E764* | G | 2290 | T | ATP12A | G567S | G | 1886 | A | BCLAF1 | R69Q | C | 459 | T | C11orf41 | A1356T | G | 4190 | A |
| ARHGEF1 | R546W | C | 1636 | T | ATP12A | K466T | A | 1584 | C | BCLAF1 | S422R | T | 1517 | G | C11orf41 | P1486L | C | 4581 | T |
| ARHGEF1 | F434V | T | 1300 | G | ATP13A1 | - | C | 0 | T | BCMO1 | A303T | G | 1368 | A | C11orf41 | A1804V | C | 5535 | T |
| ARHGEF1 | V522M | G | 1564 | A | ATP13A1 | E924K | C | 2797 | T | BCMO1 | N374H | A | 1581 | C | C11orf41 | M1223T | T | 3792 | C |
| ARHGEF1 | R69W | C | 205 | T | ATP13A1 | F209C | A | 653 | C | BCO2 | R139Q | G | 537 | A | C11orf41 | T1136A | A | 3530 | G |
| ARHGEF10 | R969C | C | 2905 | T | ATP13A2 | P79S | G | 269 | A | BCO2 | R332W | C | 1115 | T | C11orf41 | R1507C | C | 4643 | T |
| ARHGEF10 | T716M | C | 2147 | T | ATP13A3 | A894T | C | 3472 | T | BCO2 | R487Q | G | 1581 | A | C11orf41 | L548V | T | 1766 | G |
| ARHGEF10 | E214D | G | 642 | T | ATP13A3 | F911L | A | 3523 | G | BCOR | N784S | T | 2580 | C | C11orf41 | R1415H | G | 4368 | A |
| ARHGEF10 | F894C | T | 2681 | G | ATP13A3 | T169M | G | 1298 | A | BCOR | R118I | C | 582 | A | C11orf41 | L1814M | C | 5564 | A |
| ARHGEF10 L | L526I | C | 1735 | A | ATP13A3 | I838M | T | 3306 | C | BCOR | E946D | C | 3067 | A | C11orf42 | R279* | C | 885 | T |
| ARHGEF10 L | P1155S | C | 3622 | T | ATP13A3 | R138C | G | 1204 | A | BCOR | T329I | G | 1215 | A | C11orf42 | G285E | G | 904 | A |
| ARHGEF11 | M897V | T | 3729 | C | ATP13A4 | L909M | G | 3048 | T | BCOR | K1360R | T | 4308 | C | C11orf42 | A94T | G | 330 | A |
| ARHGEF11 | A787T | C | 3399 | T | ATP13A5 | E752* | C | 2372 | A | BCOR | V806I | C | 2645 | T | C11orf42 | E99G | A | 346 | G |
| ARHGEF11 | R1314L | C | 4981 | A | ATP13A5 | H513L | T | 1656 | A | BCORL1 | T345M | C | 1148 | T | C11orf44 | A32T | G | 117 | A |
| ARHGEF11 | G1505C | C | 5553 | A | ATP13A5 | V256M | C | 884 | T | BCORL1 | A1560V | C | 4793 | T | C11orf44 | P24S | C | 93 | T |
| ARHGEF11 | R1140W | G | 4458 | A | ATP13A5 | P389H | G | 1284 | T | BCORL1 | A1434T | G | 4414 | A | C11orf45 | R112Q | C | 529 | T |
| ARHGEF11 | - | C | 0 | A | ATP13A5 | V758M | C | 2390 | T | BCR | S429L | C | 2037 | T | C11orf45 | T3M | G | 202 | A |
| ARHGEF12 | P1423L | C | 4275 | T | ATP1A1 | R74* | C | 472 | T | BCR | R364Q | G | 1842 | A | C11orf46 | R239H | G | 873 | A |
| ARHGEF12 | S745N | G | 2241 | A | ATP1A1 | S40A | T | 370 | G | BCS1L | N267T | A | 937 | C | C11orf46 | V187L | G | 716 | T |
| ARHGEF12 | D862N | G | 2591 | A | ATP1A1 | R607Q | G | 2072 | A | BCS1L | V205I | G | 750 | A | C11orf46 | N89H | A | 422 | C |
| ARHGEF15 | P72H | C | 367 | A | ATP1A2 | R443Q | G | 1417 | A | BDH1 | A257T | C | 1080 | T | C11orf61 | Q292K | G | 897 | T |
| ARHGEF15 | A74V | C | 373 | T | ATP1A2 | D744N | G | 2319 | A | BDH1 | E118K | C | 663 | T | C11orf63 | T671M | C | 2309 | T |
| ARHGEF15 | R48Q | G | 295 | A | ATP1A2 | D750A | A | 2338 | C | BDH2 | T208M | G | 744 | A | C11orf65 | N260K | G | 850 | T |
| ARHGEF16 | N466S | A | 1802 | G | ATP1A3 | R877W | G | 2767 | A | BDKRB1 | F86V | T | 476 | G | C11orf65 | K14N | C | 112 | A |
| ARHGEF16 | A157T | G | 874 | A | ATP1A3 | I61F | T | 319 | A | BDKRB1 | R232W | C | 914 | T | C11orf66 | R129W | C | 510 | T |
| ARHGEF16 | E344K | G | 1435 | A | ATP1A4 | G343W | G | 1498 | T | BDKRB2 | T128M | C | 579 | T | C11orf66 | I199V | A | 720 | G |
| ARHGEF16 | A94D | C | 686 | A | ATP1A4 | R212W | C | 1105 | T | BDKRB2 | T128M | C | 579 | T | C11orf66 | S18L | C | 178 | T |
| ARHGEF16 | A79T | G | 640 | A | ATP1A4 | G891C | G | 3142 | T | BDNF | R311* | G | 1072 | A | C11orf74 | E127* | G | 475 | T |
| ARHGEF16 | Q442H | G | 1731 | T | ATP1A4 | R358H | G | 1544 | A | BDNF | T173M | G | 659 | A | C11orf75 | R54H | C | 419 | T |
| ARHGEF17 | I1360T | T | 4100 | C | ATP1A4 | F872L | C | 3087 | A | BDNF | T231M | G | 833 | A | C11orf80 | R540H | G | 1626 | A |
| ARHGEF17 | A1240V | C | 3740 | T | ATP1B4 | T146M | C | 494 | T | BDNF | E146K | C | 577 | T | C11orf82 | W296C | G | 938 | T |
| ARHGEF17 | S546* | C | 1658 | A | ATP2A1 | V726M | G | 2365 | A | BDP1 | V9G | T | 289 | G | C11orf82 | G333* | G | 1047 | T |
| ARHGEF18 | S768R | A | 2555 | C | ATP2A2 | V687I | G | 2622 | A | BDP1 | - | T | 0 | G | C11orf82 | R345* | C | 1083 | T |
| ARHGEF18 | P51L | C | 405 | T | ATP2A3 | R328H | C | 1134 | T | BDP1 | R1830M | G | 5752 | T | C11orf83 | Q58* | C | 264 | T |
| ARHGEF18 | S798L | C | 2646 | T | ATP2A3 | T247M | G | 891 | A | BDP1 | G526D | G | 1840 | A | C11orf85 | M145T | A | 707 | G |
| ARHGEF19 | R685W | G | 2190 | A | ATP2A3 | D456N | C | 1517 | T | BDP1 | S33T | T | 360 | A | C11orf85 | R53C | G | 430 | A |
| ARHGEF19 | E259D | C | 914 | A | ATP2A3 | L49P | A | 297 | G | BDP1 | T1816A | A | 5709 | G | C11orf85 | E193K | C | 850 | T |

| | | | | | | | | | | | | | | | | | | | |
|---|---|---|---|---|---|---|---|---|---|---|---|---|---|---|---|---|---|---|---|
| ARHGEF2 | A890T | C | 2690 | T | ATP2B1 | M640I | C | 2101 | A | BDP1 | E82D | A | 509 | C | C11orf87 | V66L | G | 519 | C |
| ARHGEF2 | S523L | G | 1590 | A | ATP2B1 | T30M | G | 270 | A | BDP1 | R2556* | C | 7929 | T | C11orf9 | M876I | G | 2724 | A |
| ARHGEF2 | G947D | C | 2862 | T | ATP2B1 | R744Q | C | 2412 | T | BEGAIN | V178I | C | 603 | T | C11orf9 | D18N | G | 148 | A |
| ARHGEF2 | M29I | C | 109 | T | ATP2B2 | T1220M | G | 3729 | A | BEND2 | R453H | C | 1491 | T | C11orf9 | P302L | C | 1001 | T |
| ARHGEF3 | R377W | G | 1239 | A | ATP2B2 | A742T | C | 2294 | T | BEND2 | P341L | G | 1155 | A | C11orf9 | Y407D | T | 1315 | G |
| ARHGEF3 | N40T | T | 229 | G | ATP2B3 | V1164M | G | 3816 | A | BEND3 | I701T | A | 2293 | G | C11orf91 | - | T | 582 | C |
| ARHGEF33 | E32K | G | 359 | A | ATP2B3 | A128V | C | 709 | T | BEND3 | A161T | C | 672 | T | C11orf94 | S54F | G | 198 | A |
| ARHGEF33 | Q48K | C | 407 | A | ATP2B3 | R1099M | G | 3622 | T | BEND3 | Y788* | G | 2555 | T | C11orf95 | E189D | C | 586 | A |
| ARHGEF33 | E232* | G | 959 | T | ATP2B3 | A32V | C | 421 | T | BEND3 | G183R | C | 738 | T | C12orf10 | R188Q | G | 615 | A |
| ARHGEF33 | E167K | G | 764 | A | ATP2B3 | L535I | C | 1929 | A | BEND3 | R644Q | C | 2122 | T | C12orf11 | R366* | G | 1633 | A |
| ARHGEF33 | T512I | C | 1800 | T | ATP2B4 | A854V | C | 3684 | T | BEND4 | S406Y | G | 1797 | T | C12orf11 | R336W | G | 1543 | A |
| ARHGEF33 | R452W | C | 1619 | T | ATP2B4 | P202S | C | 1727 | T | BEND5 | P266L | G | 884 | A | C12orf12 | T168M | G | 937 | A |
| ARHGEF33 | Q48K | C | 407 | A | ATP2B4 | I933V | A | 3920 | G | BEND5 | R269L | C | 893 | A | C12orf23 | K6T | A | 391 | C |
| ARHGEF33 | S297P | T | 1154 | C | ATP2C1 | T683I | C | 2048 | T | BEND5 | R29Q | C | 173 | T | C12orf26 | S313Y | C | 959 | A |
| ARHGEF33 | K827N | G | 2746 | T | ATP2C1 | A318G | C | 953 | G | BEND5 | L97R | A | 377 | C | C12orf29 | R272H | G | 815 | A |
| ARHGEF37 | Y302C | A | 1068 | G | ATP2C2 | G271R | G | 900 | A | BEND7 | K224T | T | 671 | G | C12orf34 | A129T | G | 551 | A |
| ARHGEF37 | R547H | G | 1803 | A | ATP2C2 | Q929P | A | 2875 | C | BEND7 | A210V | G | 629 | A | C12orf34 | S351N | G | 1218 | A |
| ARHGEF38 | R95W | C | 427 | T | ATP4A | A969T | C | 2934 | T | BEST1 | D168G | A | 1263 | G | C12orf34 | A174S | G | 686 | T |
| ARHGEF4 | Q936* | C | 4004 | T | ATP4A | R668H | C | 2032 | T | BEST3 | R388Q | C | 1390 | T | C12orf35 | N235K | C | 1119 | A |
| ARHGEF4 | G985R | G | 4151 | A | ATP4A | G379D | C | 1165 | T | BEST3 | R388W | G | 1389 | A | C12orf35 | F983C | T | 3362 | G |
| ARHGEF4 | R391W | C | 1690 | T | ATP4A | D21G | T | 91 | C | BET3L | F103L | A | 405 | C | C12orf35 | K1390N | G | 4584 | T |
| ARHGEF6 | A720S | C | 3364 | A | ATP4A | R703C | G | 2136 | A | BEX1 | R56T | C | 407 | G | C12orf35 | A1276T | G | 4240 | A |
| ARHGEF6 | E259* | C | 1981 | A | ATP4A | K487R | T | 1489 | C | BFAR | Q416R | A | 1524 | G | C12orf35 | V1382I | G | 4558 | A |
| ARHGEF7 | R555Q | G | 1914 | A | ATP4A | A76V | G | 256 | A | BFSP1 | A220S | C | 698 | A | C12orf35 | N235H | A | 1117 | C |
| ARHGEF9 | - | C | 0 | G | ATP4A | W901R | A | 2730 | G | BFSP1 | D433N | C | 1337 | T | C12orf35 | V551A | T | 2066 | C |
| ARID1A | R1109W | C | 3696 | T | ATP4A | F317L | A | 980 | T | BFSP1 | R469Q | C | 1446 | T | C12orf35 | S563Y | C | 2102 | A |
| ARID1A | R2143H | G | 6799 | A | ATP4A | A120T | C | 387 | T | BFSP1 | A477V | G | 1470 | A | C12orf35 | S1095A | T | 3697 | G |
| ARID1A | S2079I | G | 6607 | T | ATP4B | R19H | C | 98 | T | BFSP1 | E409* | C | 1265 | A | C12orf35 | L1267V | T | 4213 | G |
| ARID1A | R1223H | G | 4039 | A | ATP5A1 | S106Y | G | 462 | T | BFSP2 | P263L | C | 877 | T | C12orf4 | R335Q | C | 1092 | T |
| ARID1A | R1276Q | G | 4198 | A | ATP5A1 | R322H | C | 1110 | T | BFSP2 | D270N | G | 897 | A | C12orf4 | T216I | G | 735 | A |
| ARID1A | S1000Y | C | 3370 | A | ATP5C1 | R23* | C | 146 | T | BFSP2 | R154Q | G | 550 | A | C12orf4 | R335Q | C | 1092 | T |
| ARID1B | R2252H | G | 6755 | A | ATP5L2 | T85M | G | 581 | A | BFSP2 | R199* | C | 684 | T | C12orf40 | N526T | A | 1731 | C |
| ARID1B | S1175R | A | 3523 | C | ATP5SL | - | C | 0 | T | BHLHA15 | Y178C | A | 589 | G | C12orf40 | S627Y | C | 2034 | A |
| ARID1B | L1764F | G | 5292 | T | ATP6AP1 | A185V | C | 615 | T | BHLHA15 | R70L | G | 265 | T | C12orf40 | Q179H | G | 691 | T |
| ARID1B | G568R | G | 1702 | A | ATP6AP1L | - | G | 0 | A | BHLHB9 | K47N | G | 712 | T | C12orf41 | H49L | T | 221 | A |
| ARID1B | P1999L | C | 5996 | T | ATP6AP1L | F45L | C | 1460 | A | BHLHE22 | G224S | G | 1204 | A | C12orf41 | E198* | C | 667 | A |
| ARID1B | G324A | G | 971 | C | ATP6AP1L | P78Q | C | 1558 | A | BHLHE22 | A297V | C | 1424 | T | C12orf41 | R94M | C | 356 | A |
| ARID1B | G326A | G | 977 | C | ATP6V0A1 | M362I | G | 1253 | A | BHMT2 | G67* | G | 258 | T | C12orf42 | I143V | T | 653 | C |
| ARID1B | P605L | C | 1814 | T | ATP6V0A4 | R449C | G | 1628 | A | BHMT2 | M222I | G | 725 | T | C12orf43 | S245N | C | 757 | T |

| | | | | | | | | | | | | | | | | | | | |
|---|---|---|---|---|---|---|---|---|---|---|---|---|---|---|---|---|---|---|---|
| ARID1B | P1475L | C | 4424 | T | ATP6V0A4 | L345P | A | 1317 | G | BICC1 | A640V | C | 1923 | T | C12orf43 | A176G | G | 550 | C |
| ARID2 | S668Y | C | 2131 | A | ATP6V0A4 | N74H | T | 503 | G | BICC1 | G151D | G | 456 | A | C12orf45 | A92T | G | 307 | A |
| ARID2 | S832* | C | 2623 | A | ATP6V0D1 | F294L | G | 1033 | T | BICC1 | K107T | A | 324 | C | C12orf45 | T17A | A | 82 | G |
| ARID2 | L1399* | T | 4324 | G | ATP6V1B1 | R124Q | G | 444 | A | BICC1 | R632* | C | 1898 | T | C12orf48 | D109V | A | 438 | T |
| ARID2 | R1504W | C | 4638 | T | ATP6V1B1 | V493M | G | 1550 | A | BICC1 | K744N | G | 2236 | T | C12orf50 | K325T | T | 1142 | G |
| ARID2 | Q958* | C | 3000 | T | ATP6V1B1 | A276V | C | 900 | T | BICD1 | M480V | A | 1519 | G | C12orf50 | R33Q | C | 266 | T |
| ARID2 | D444Y | G | 1458 | T | ATP6V1B1 | S169Y | C | 579 | A | BICD1 | P931L | C | 2873 | T | C12orf51 | A1789V | G | 5384 | A |
| ARID2 | Q488R | A | 1591 | G | ATP6V1B2 | R494I | G | 1521 | T | BICD1 | - | G | 3009 | T | C12orf51 | P985S | G | 2971 | A |
| ARID2 | R294I | G | 1009 | T | ATP6V1C1 | Y367C | A | 1345 | G | BICD1 | A72V | C | 296 | T | C12orf51 | H3988R | T | 11981 | C |
| ARID3A | R476C | C | 1716 | T | ATP6V1C1 | V183I | G | 792 | A | BICD2 | E220K | C | 726 | T | C12orf51 | R3638C | G | 10930 | A |
| ARID3A | E287D | G | 1151 | T | ATP6V1C1 | N324S | A | 1216 | G | BICD2 | L257M | G | 837 | T | C12orf51 | D2234V | T | 6719 | A |
| ARID3B | M242V | A | 926 | G | ATP6V1C2 | R230Q | G | 798 | A | BICD2 | A814T | C | 2508 | T | C12orf51 | A3821T | C | 11479 | T |
| ARID3B | G472S | G | 1616 | A | ATP6V1D | K208N | C | 1175 | A | BID | A137T | C | 736 | T | C12orf51 | S3050C | T | 9166 | A |
| ARID3C | L107R | A | 413 | C | ATP6V1E2 | R135C | G | 1517 | A | BID | K203N | C | 936 | A | C12orf51 | T2389M | G | 7184 | A |
| ARID4A | G1106* | G | 3689 | T | ATP6V1F | N46T | A | 138 | C | BIN1 | Q573H | C | 2131 | A | C12orf51 | G2335D | C | 7022 | T |
| ARID4A | K528T | A | 1956 | C | ATP6V1H | E185* | C | 705 | A | BIN1 | Q54* | G | 572 | A | C12orf51 | L3124R | A | 9389 | C |
| ARID4A | W630* | G | 2262 | A | ATP6V1H | E25D | T | 227 | G | BIN1 | D537Y | C | 2021 | A | C12orf51 | E2572K | C | 7732 | T |
| ARID4A | K482N | G | 1819 | T | ATP7A | G618E | G | 1853 | A | BIN1 | V525L | C | 1985 | A | C12orf51 | A953V | G | 2876 | A |
| ARID4B | R1059* | G | 3552 | A | ATP7A | I603T | T | 1808 | C | BIN2 | D522N | C | 1626 | T | C12orf51 | A563V | G | 1706 | A |
| ARID4B | I436N | A | 1684 | T | ATP7B | I945V | T | 2990 | C | BIN2 | F220L | G | 722 | T | C12orf51 | E81* | C | 259 | A |
| ARID4B | S1152R | T | 3831 | G | ATP7B | Q383H | T | 1306 | A | BIRC2 | D79Y | G | 1634 | T | C12orf52 | S153L | C | 938 | T |
| ARID4B | S702L | G | 2482 | A | ATP7B | K164N | C | 649 | A | BIRC2 | E422D | G | 2665 | T | C12orf52 | R245H | G | 1214 | A |
| ARID5A | A17T | G | 149 | A | ATP7B | R1151C | G | 3608 | A | BIRC2 | E578D | A | 3133 | C | C12orf52 | K28N | G | 564 | T |
| ARID5B | E845K | G | 2943 | A | ATP7B | A753V | G | 2415 | A | BIRC5 | D70Y | G | 329 | T | C12orf53 | V107M | C | 549 | T |
| ARID5B | R999Q | G | 3406 | A | ATP8A1 | R735W | G | 2435 | A | BIRC6 | E1604* | G | 4944 | T | C12orf54 | T39N | C | 173 | A |
| ARID5B | A1009G | C | 3436 | G | ATP8A1 | R581Q | C | 1974 | T | BIRC6 | I667N | T | 2134 | A | C12orf56 | S353Y | G | 1058 | T |
| ARID5B | - | G | 0 | A | ATP8A1 | A1016T | C | 3278 | T | BIRC6 | R1030H | G | 3223 | A | C12orf56 | A303T | C | 907 | T |
| ARID5B | - | T | 0 | C | ATP8A1 | P1033L | G | 3330 | A | BIRC6 | Y1928F | A | 5917 | T | C12orf60 | D193Y | G | 781 | T |
| ARID5B | R34I | G | 511 | T | ATP8A1 | Q468H | T | 1636 | G | BIRC6 | - | A | 0 | C | C12orf60 | K201N | A | 807 | C |
| ARID5B | N1136H | A | 3816 | C | ATP8A1 | R14H | C | 273 | T | BIRC6 | T374R | C | 1255 | G | C12orf63 | K626M | A | 1955 | T |
| ARIH1 | R132I | G | 709 | T | ATP8A2 | I126V | A | 518 | G | BIRC6 | K1272N | A | 3950 | C | C12orf63 | C492Y | G | 1553 | A |
| ARIH2 | R342C | C | 1363 | T | ATP8A2 | - | A | 0 | C | BIRC6 | N2010Y | A | 6162 | T | C12orf64 | E664* | G | 1989 | T |
| ARL10 | V82G | T | 341 | G | ATP8A2 | A855T | G | 2705 | A | BIRC6 | R3284H | G | 9985 | A | C12orf64 | K140T | A | 418 | C |
| ARL11 | S177N | G | 865 | A | ATP8A2 | F961L | T | 3023 | C | BIRC6 | T3864A | A | 11724 | G | C12orf64 | L6I | C | 15 | A |
| ARL13B | K310T | A | 1204 | C | ATP8A2 | R588W | C | 1904 | T | BIRC7 | A71T | G | 425 | A | C12orf65 | N58T | A | 817 | C |
| ARL13B | R79W | C | 510 | T | ATP8A2 | V506M | G | 1658 | A | BIRC8 | S100* | G | 1547 | C | C12orf65 | - | A | 1145 | G |
| ARL14 | T61A | A | 368 | G | ATP8A2 | L264I | C | 932 | A | BLK | L138F | C | 993 | T | C12orf66 | T225M | G | 728 | A |

| | | | | | | | | | | | | | | | | | | | |
|---|---|---|---|---|---|---|---|---|---|---|---|---|---|---|---|---|---|---|---|
| ARL16 | R7W | G | 118 | A | ATP8A2 | R216C | C | 788 | T | BLK | S341L | C | 1603 | T | C12orf69 | S118L | G | 426 | A |
| ARL4C | V124A | A | 385 | G | ATP8A2 | K151E | A | 593 | G | BLNK | N185H | T | 553 | G | C12orf69 | E104V | T | 384 | A |
| ARL4D | R92C | C | 455 | T | ATP8A2 | K345N | G | 1177 | T | BLOC1S2 | D82Y | C | 267 | A | C12orf70 | E86G | A | 433 | G |
| ARL5A | N59S | T | 488 | C | ATP8A2 | E698K | G | 2234 | A | BLOC1S2 | T38N | G | 136 | T | C12orf72 | H234R | A | 910 | G |
| ARL6IP1 | P172H | G | 644 | T | ATP8A2 | I1003S | T | 3150 | G | BLVRA | V141A | T | 498 | C | C13orf1 | A58T | C | 277 | T |
| ARL6IP1 | K200N | C | 729 | A | ATP8B1 | R628W | G | 1882 | A | BLZF1 | R159C | C | 898 | T | C13orf1 | A58T | C | 277 | T |
| ARL6IP4 | S339L | C | 1016 | T | ATP8B1 | Y924D | A | 2770 | C | BLZF1 | - | A | 0 | G | C13orf18 | P419Q | G | 1861 | T |
| ARL6IP6 | E66* | G | 907 | T | ATP8B1 | R882M | C | 2645 | A | BMF | E8D | C | 259 | A | C13orf18 | E640K | C | 2523 | T |
| ARL8A | V53M | C | 326 | T | ATP8B1 | V1202M | C | 3604 | T | BMI1 | S274N | G | 1326 | A | C13orf18 | V197A | A | 1195 | G |
| ARL9 | A175T | G | 779 | A | ATP8B1 | F673C | A | 2018 | C | BMI1 | Q140K | C | 923 | A | C13orf23 | G711D | C | 2966 | T |
| ARL9 | R77W | C | 485 | T | ATP8B1 | D379N | C | 1135 | T | BMI1 | T63A | A | 692 | G | C13orf26 | D179G | A | 549 | G |
| ARMC2 | S444L | C | 1499 | T | ATP8B1 | F124V | A | 370 | C | BMP1 | K814T | A | 2929 | C | C13orf26 | Y29H | T | 98 | C |
| ARMC2 | V620M | G | 2026 | A | ATP8B2 | R338H | G | 1013 | A | BMP1 | R181Q | G | 1030 | A | C13orf27 | Q140* | G | 608 | A |
| ARMC2 | F310C | T | 1097 | G | ATP8B2 | R576W | C | 1726 | T | BMP10 | V255M | C | 922 | T | C13orf28 | D127N | G | 446 | A |
| ARMC2 | K435N | G | 1473 | T | ATP8B2 | A212V | C | 635 | T | BMP10 | E225* | C | 832 | A | C13orf28 | L54M | C | 227 | A |
| ARMC2 | R854I | G | 2729 | T | ATP8B2 | S1194P | T | 3580 | C | BMP10 | K91T | T | 431 | G | C13orf33 | A66V | C | 522 | T |
| ARMC3 | A389V | C | 1250 | T | ATP8B2 | L460F | G | 1380 | T | BMP15 | R8I | G | 23 | T | C13orf34 | I428V | A | 1282 | G |
| ARMC3 | Y820H | T | 2542 | C | ATP8B2 | R1193H | G | 3578 | A | BMP2K | R1092C | C | 3440 | T | C13orf36 | S28F | C | 529 | T |
| ARMC4 | G798D | C | 2486 | T | ATP8B3 | R562W | G | 1918 | A | BMP2KL | P251S | C | 751 | T | C14orf1 | T75M | G | 297 | A |
| ARMC4 | R629H | C | 1979 | T | ATP8B3 | V1080I | C | 3472 | T | BMP3 | S120L | C | 679 | T | C14orf1 | R126W | G | 449 | A |
| ARMC4 | E279K | C | 928 | T | ATP8B3 | H410P | T | 1463 | G | BMP4 | R280W | G | 1232 | A | C14orf101 | V113I | G | 459 | A |
| ARMC4 | E786A | T | 2450 | G | ATP8B3 | - | C | 0 | T | BMP5 | R181Q | C | 1241 | T | C14orf101 | K274T | A | 943 | C |
| ARMC4 | R6M | C | 110 | A | ATP8B3 | L334H | A | 1235 | T | BMP5 | F68S | A | 902 | G | C14orf102 | A582G | G | 1782 | C |
| ARMC4 | C784F | C | 2444 | A | ATP8B3 | L185P | A | 788 | G | BMP5 | R49W | G | 844 | A | C14orf102 | W328* | C | 1020 | T |
| ARMC4 | K937T | T | 2903 | G | ATP8B4 | R549* | G | 1787 | A | BMP5 | L424V | A | 1969 | C | C14orf102 | A881T | C | 2678 | T |
| ARMC4 | Q811H | T | 2526 | G | ATP8B4 | A484T | C | 1592 | T | BMP5 | K284N | T | 1551 | G | C14orf104 | E683V | T | 2129 | A |
| ARMC5 | R429C | C | 1409 | T | ATP8B4 | L1173V | A | 3659 | C | BMP5 | E224* | C | 1369 | A | C14orf104 | R339Q | C | 1097 | T |
| ARMC5 | C240Y | G | 843 | A | ATP9A | R201Q | C | 867 | T | BMP6 | A451T | G | 1510 | A | C14orf104 | E806* | C | 2497 | A |
| ARMC5 | P654Q | C | 2085 | A | ATP9A | S244N | C | 996 | T | BMP6 | S85L | C | 413 | T | C14orf106 | Q386H | C | 1617 | A |
| ARMC5 | R688Q | G | 2187 | A | ATP9A | A592T | C | 2039 | T | BMP7 | R69H | C | 712 | T | C14orf106 | T1094P | T | 3739 | G |
| ARMC5 | R936H | G | 2931 | A | ATP9A | K1031E | T | 3356 | C | BMP8A | C334R | T | 1346 | C | C14orf115 | V133M | G | 588 | A |
| ARMC5 | A878T | G | 2756 | A | ATP9A | R455H | C | 1629 | T | BMP8B | P280L | G | 1215 | A | C14orf115 | R276H | G | 1018 | A |
| ARMC6 | Q431* | C | 1291 | T | ATP9A | E489K | C | 1730 | T | BMPER | E427K | G | 1653 | A | C14orf115 | L137I | C | 600 | A |
| ARMC6 | P258S | C | 772 | T | ATP9A | G394D | C | 1446 | T | BMPER | A578T | G | 2106 | A | C14orf135 | A962T | G | 3438 | A |
| ARMC7 | E75* | G | 525 | T | ATP9B | I1015V | A | 3052 | G | BMPER | T385M | C | 1528 | T | C14orf135 | R559* | C | 2229 | T |
| ARMC8 | R22H | G | 336 | A | ATP9B | G678R | G | 2041 | A | BMPER | A451V | C | 1726 | T | C14orf142 | - | T | 380 | G |
| ARMC8 | S76N | G | 498 | A | ATP9B | Y216H | T | 655 | C | BMPER | R439H | G | 1690 | A | C14orf145 | E800D | C | 2571 | A |
| ARMC8 | R401W | C | 1472 | T | ATPIF1 | E51D | G | 204 | T | BMPER | S184N | G | 925 | A | C14orf145 | R146I | C | 608 | A |
| ARMC9 | R388Q | G | 1163 | A | ATR | G481D | C | 1564 | T | BMPER | L252M | C | 1128 | A | C14orf145 | W493C | C | 1650 | A |

| | | | | | | | | | | | | | | | | | | | |
|---|---|---|---|---|---|---|---|---|---|---|---|---|---|---|---|---|---|---|---|
| ARMC9 | T383M | C | 1148 | T | ATR | E293* | C | 999 | A | BMPR1A | G434D | G | 1849 | A | C14orf145 | K983Q | T | 3118 | G |
| ARMC9 | P155T | C | 463 | A | ATR | E2128D | C | 6506 | A | BMPR1A | K102N | A | 854 | C | C14orf145 | E766K | C | 2467 | T |
| ARMCX1 | R244H | G | 1102 | A | ATR | Q1334E | G | 4122 | C | BMPR1A | K333Q | A | 1545 | C | C14orf145 | R568H | C | 1874 | T |
| ARMCX1 | G17R | G | 420 | A | ATR | L1361V | A | 4203 | C | BMPR1B | E184D | A | 552 | C | C14orf145 | - | C | 0 | A |
| ARMCX1 | K77Q | A | 600 | C | ATRIP | R709M | G | 2239 | T | BMPR1B | E452* | G | 1354 | T | C14orf145 | Q175H | T | 696 | G |
| ARMCX2 | A39T | C | 569 | T | ATRIP | E573K | G | 1830 | A | BMPR2 | G311R | G | 1470 | A | C14orf153 | S397L | C | 1190 | T |
| ARMCX3 | E351* | G | 1531 | T | ATRN | P29S | C | 153 | T | BMPR2 | A35V | C | 643 | T | C14orf166 | I230V | A | 853 | G |
| ARMCX5 | E515D | A | 2426 | C | ATRN | F174C | T | 589 | G | BMPR2 | H331Y | C | 1530 | T | C14orf166 | R145C | C | 598 | T |
| ARNT | P785S | G | 2554 | A | ATRN | I194M | T | 650 | G | BMS1 | T248A | A | 805 | G | C14orf166B | G315S | G | 1067 | A |
| ARNT | R101* | G | 502 | A | ATRN | R551Q | G | 1720 | A | BMS1 | R887C | C | 2722 | T | C14orf166B | N104S | A | 435 | G |
| ARNT | - | C | 0 | A | ATRNL1 | S1063N | G | 3314 | A | BMS1 | R75Q | G | 287 | A | C14orf166B | L483V | T | 1571 | G |
| ARNT | L548V | A | 1843 | C | ATRNL1 | - | G | 0 | T | BMX | R99Q | G | 484 | A | C14orf174 | R615C | C | 2129 | T |
| ARNT | K419N | T | 1458 | G | ATRX | I2026L | T | 6291 | G | BMX | R414K | G | 1429 | A | C14orf177 | S44F | C | 550 | T |
| ARNT2 | R652Q | G | 2121 | A | ATRX | K2359R | T | 7291 | C | BNC1 | L779F | C | 2423 | A | C14orf179 | L55S | T | 198 | C |
| ARNT2 | L141I | C | 587 | A | ATRX | K1867N | C | 5816 | A | BNC1 | R451M | C | 1438 | A | C14orf182 | R4Q | C | 1732 | T |
| ARNT2 | P579S | C | 1901 | T | ATRX | Q2330R | T | 7204 | C | BNC1 | E612* | C | 1920 | A | C14orf21 | P595S | C | 1876 | T |
| ARNTL | S278I | G | 1188 | T | ATRX | K46T | T | 352 | G | BNC2 | A445V | G | 1392 | A | C14orf21 | R542H | G | 1718 | A |
| ARNTL | K405T | A | 1569 | C | ATXN1 | K307R | T | 1858 | C | BNC2 | S575R | T | 1781 | G | C14orf28 | Y118H | T | 618 | C |
| ARPC1A | I141S | T | 542 | G | ATXN1 | A505V | G | 2452 | A | BNC2 | S575R | T | 1781 | G | C14orf37 | T612N | G | 1976 | T |
| ARPC1A | M352T | T | 1179 | C | ATXN1 | E357K | C | 2007 | T | BNC2 | R291H | C | 930 | T | C14orf37 | M360T | A | 1220 | G |
| ARRB1 | V20I | C | 280 | T | ATXN10 | R426* | C | 1464 | T | BNC2 | F778C | A | 2391 | C | C14orf37 | N111H | T | 472 | G |
| ARRB2 | S352Y | C | 1117 | A | ATXN10 | L310P | T | 1117 | C | BNC2 | F442L | G | 1384 | T | C14orf37 | E74D | T | 363 | G |
| ARRDC1 | A81T | G | 305 | A | ATXN1L | T560M | C | 1967 | T | BNC2 | E313K | C | 995 | T | C14orf38 | A124V | G | 371 | A |
| ARRDC2 | V30M | G | 231 | A | ATXN1L | G433D | G | 1586 | A | BNIPL | A266V | C | 953 | T | C14orf38 | V717M | C | 2149 | T |
| ARRDC2 | T134I | C | 544 | T | ATXN2 | A1199V | G | 3758 | A | BOC | R821* | C | 2800 | T | C14orf38 | E574D | C | 1722 | A |
| ARRDC2 | V275I | G | 966 | A | ATXN2L | P1020S | C | 3225 | T | BOC | V625M | G | 2212 | A | C14orf38 | N551S | T | 1652 | C |
| ARRDC3 | E344D | T | 1299 | G | ATXN2L | D240N | G | 885 | A | BOC | A472T | G | 1753 | A | C14orf39 | Q282H | C | 1006 | A |
| ARRDC5 | T124I | G | 371 | A | ATXN2L | R301Q | G | 1069 | A | BOC | V403I | G | 1546 | A | C14orf4 | E720K | C | 3057 | T |
| ARSA | G475S | C | 1670 | T | ATXN2L | R365* | C | 1260 | T | BOC | R630C | C | 2227 | T | C14orf4 | P258L | G | 1672 | A |
| ARSB | L289I | G | 1404 | T | ATXN2L | P1071L | C | 3379 | T | BOD1L | R3024C | G | 9206 | A | C14orf4 | Y423C | T | 2167 | C |
| ARSE | A463T | C | 1454 | T | ATXN3 | R285* | G | 922 | A | BOD1L | M1120I | C | 3496 | T | C14orf4 | E762D | C | 3185 | A |
| ARSF | R179C | C | 756 | T | ATXN7 | S803F | C | 2961 | T | BOD1L | E1087* | C | 3395 | A | C14orf43 | A164T | C | 894 | T |
| ARSH | R125W | C | 373 | T | ATXN7 | P942Q | C | 3378 | A | BOD1L | T2111A | T | 6467 | C | C14orf43 | R447Q | C | 1744 | T |
| ARSI | R324W | G | 1550 | A | ATXN7 | G778A | G | 2886 | C | BOD1L | K1650R | T | 5085 | C | C14orf43 | R598W | G | 2196 | A |
| ARSI | R544H | C | 2211 | T | ATXN7 | A214T | G | 1193 | A | BOD1L | R1141H | C | 3558 | T | C14orf43 | R455* | G | 1767 | A |
| ARSI | T348I | G | 1623 | A | ATXN7 | N881S | A | 3195 | G | BOD1L | M2584V | T | 7886 | C | C14orf43 | E954* | C | 3264 | A |
| ARSI | R454Q | C | 1941 | T | ATXN7L1 | K779R | T | 2363 | C | BOD1L | P1537S | G | 4745 | A | C14orf45 | K274N | G | 945 | T |
| ARSK | Q53* | C | 362 | T | ATXN7L2 | P219S | C | 670 | T | BOD1L | T1647A | T | 5075 | C | C14orf45 | E75D | G | 348 | T |
| ART3 | A178T | G | 651 | A | ATXN7L2 | R623Q | G | 1883 | A | BOD1L | A1619D | G | 4992 | T | C14orf49 | E191K | C | 586 | T |

| | | | | | | | | | | | | | | | | | | | |
|---|---|---|---|---|---|---|---|---|---|---|---|---|---|---|---|---|---|---|---|
| ART5 | R267W | G | 1218 | A | ATXN7L2 | A693V | C | 2093 | T | BOD1L | T2245I | G | 6870 | A | C14orf50 | N400S | A | 1295 | G |
| ART5 | R145Q | C | 853 | T | ATXN7L2 | A707T | G | 2134 | A | BOD1L | A2087T | C | 6395 | T | C14orf50 | R268C | C | 898 | T |
| ARV1 | R186W | C | 555 | T | AUH | F238L | G | 738 | T | BOD1L | R519* | G | 1691 | A | C14orf68 | V279A | T | 909 | C |
| ARVCF | A732T | C | 2486 | T | AUH | S201F | G | 626 | A | BOD1L | R2970C | G | 9044 | A | C14orf68 | R254H | G | 834 | A |
| ARVCF | R368W | G | 1394 | A | AUP1 | H48R | T | 361 | C | BOD1L | S182F | G | 681 | A | C14orf73 | V114I | G | 416 | A |
| ARVCF | G693S | C | 2369 | T | AURKAIP1 | R154W | G | 836 | A | BOD1L | A2425V | G | 7410 | A | C14orf73 | R178C | C | 608 | T |
| ARVCF | R576W | G | 2018 | A | AURKB | H133Y | G | 511 | A | BOD1L | S2260L | G | 6915 | A | C14orf73 | R256H | G | 843 | A |
| ARVCF | Q138R | T | 705 | C | AURKC | R37H | G | 299 | A | BOD1L | S2167R | T | 6635 | G | C14orf73 | A220T | G | 734 | A |
| ARVCF | R638Q | C | 2205 | T | AURKC | L74I | C | 409 | A | BOLA1 | I36T | T | 812 | C | C14orf73 | T540M | C | 1695 | T |
| ARX | R329K | C | 1197 | T | AUTS2 | V769I | G | 2626 | A | BOLA1 | A92V | C | 980 | T | C14orf73 | T509M | C | 1602 | T |
| ARX | A469T | C | 1616 | T | AUTS2 | E894G | A | 3002 | G | BPGM | P153S | C | 648 | T | C14orf73 | Q675* | C | 2099 | T |
| ARX | M363V | T | 1298 | C | AUTS2 | P417S | C | 1570 | T | BPGM | G180S | G | 729 | A | C14orf80 | V465M | G | 1523 | A |
| AS3MT | A55V | C | 241 | T | AVEN | L145I | G | 563 | T | BPI | E3D | G | 98 | T | C15orf2 | A80T | G | 712 | A |
| AS3MT | E94A | A | 358 | C | AVEN | S132I | C | 525 | A | BPIL1 | R103C | C | 502 | T | C15orf2 | P337L | C | 1484 | T |
| ASAH1 | I146V | T | 603 | C | AVIL | E815* | C | 2472 | A | BPIL1 | F242C | T | 920 | G | C15orf2 | P645H | C | 2408 | A |
| ASAP1 | A608V | G | 1851 | A | AVIL | C621Y | C | 1891 | T | BPIL1 | A255T | G | 958 | A | C15orf2 | A67T | G | 673 | A |
| ASAP1 | P365S | G | 1121 | A | AVIL | K340N | C | 1049 | A | BPIL2 | T3A | T | 118 | C | C15orf2 | S994C | A | 3454 | T |
| ASAP1 | R211M | C | 660 | A | AVL9 | M596V | A | 2007 | G | BPIL2 | F488V | A | 1573 | C | C15orf2 | Q657L | A | 2444 | T |
| ASAP1 | G833R | C | 2525 | T | AVP | L13R | A | 88 | C | BPIL3 | Y183C | A | 548 | G | C15orf2 | T580A | A | 2212 | G |
| ASAP1 | S13L | G | 66 | A | AVPI1 | A129T | C | 385 | T | BPIL3 | F102L | C | 306 | A | C15orf2 | D428G | A | 1757 | G |
| ASAP1 | Q1031* | G | 3119 | A | AVPR1A | Q311R | T | 2906 | C | BPIL3 | N151T | A | 452 | C | C15orf2 | E610V | A | 2303 | T |
| ASAP1 | A117V | G | 378 | A | AVPR1A | A63T | C | 2161 | T | BPIL3 | Y366H | T | 1096 | C | C15orf2 | P749S | C | 2719 | T |
| ASAP2 | A168T | G | 842 | A | AVPR1B | R351H | G | 1517 | A | BPIL3 | N435S | A | 1304 | G | C15orf2 | K162N | G | 960 | T |
| ASAP2 | A292T | G | 1214 | A | AVPR1B | A109V | C | 791 | T | BPTF | G543S | G | 1688 | A | C15orf2 | R177M | G | 1004 | T |
| ASAP2 | A588T | G | 2102 | A | AVPR2 | A63T | G | 332 | A | BPTF | S1115* | C | 3405 | G | C15orf2 | P240R | C | 1193 | G |
| ASAP2 | R817W | C | 2789 | T | AWAT1 | R200H | G | 640 | A | BPTF | K2761N | G | 8344 | T | C15orf2 | S785Y | C | 2828 | A |
| ASAP3 | C325R | A | 1018 | G | AXIN1 | G233D | C | 1070 | T | BPTF | R2184C | C | 6611 | T | C15orf2 | E1035* | G | 3577 | T |
| ASB10 | G3D | C | 134 | T | AXIN1 | R395C | G | 1555 | A | BPTF | K1491N | G | 4534 | T | C15orf23 | E264K | G | 905 | A |
| ASB10 | R317C | G | 1075 | A | AXIN1 | K161T | T | 854 | G | BRAF | A718V | G | 2214 | A | C15orf27 | E309K | G | 1201 | A |
| ASB10 | M1V | T | 127 | C | AXIN2 | M120I | C | 674 | A | BRAF | V600E | A | 1860 | T | C15orf33 | Y373C | T | 1412 | C |
| ASB10 | A242V | G | 851 | A | AXIN2 | R714W | G | 2454 | A | BRAF | V600E | A | 1860 | T | C15orf33 | I394M | A | 1476 | C |
| ASB10 | A242V | G | 851 | A | AXIN2 | S296N | C | 1201 | T | BRAF | A305V | G | 975 | A | C15orf39 | P1033S | C | 3417 | T |
| ASB11 | G109A | C | 377 | G | AXIN2 | D434E | G | 1616 | T | BRAF | V600E | A | 1860 | T | C15orf39 | D202Y | G | 924 | T |
| ASB12 | R215L | C | 840 | A | AXL | G46V | G | 327 | T | BRAF | M53T | A | 219 | G | C15orf41 | K78T | A | 483 | C |
| ASB13 | P88L | G | 290 | A | AZGP1 | A46T | C | 273 | T | BRAF | V600E | A | 1860 | T | C15orf42 | R1574* | C | 4825 | T |
| ASB13 | R29W | G | 112 | A | AZGP1 | D55Y | C | 300 | A | BRAP | - | C | 0 | T | C15orf42 | R623I | G | 1973 | T |
| ASB14 | E189K | C | 686 | T | AZI1 | L401P | A | 1450 | G | BRAP | - | A | 0 | G | C15orf42 | S503G | A | 1612 | G |
| ASB14 | V176A | A | 648 | G | AZI1 | V161M | C | 729 | T | BRAP | A242D | G | 919 | T | C15orf42 | A17T | G | 154 | A |
| ASB16 | R420H | G | 1343 | A | AZI1 | R305Q | C | 1162 | T | BRCA1 | G1384W | C | 4382 | A | C15orf42 | V942A | T | 2930 | C |

| | | | | | | | | | | | | | | | | | | | |
|---|---|---|---|---|---|---|---|---|---|---|---|---|---|---|---|---|---|---|---|
| ASB16 | R128Q | G | 467 | A | AZI1 | A513V | G | 1786 | A | BRCA1 | K1254T | T | 3993 | G | C15orf43 | K126Q | A | 393 | C |
| ASB17 | L282I | G | 984 | T | AZI1 | R925H | C | 3022 | T | BRCA2 | R2896C | C | 8919 | T | C15orf44 | G406S | C | 1216 | T |
| ASB2 | K606N | C | 1818 | A | AZI1 | R374L | C | 1369 | A | BRCA2 | T1887M | C | 5893 | T | C15orf52 | P263L | G | 804 | A |
| ASB2 | S536G | T | 1606 | C | AZI1 | T501I | G | 1750 | A | BRCA2 | G1840V | G | 5752 | T | C15orf54 | G115R | G | 711 | A |
| ASB2 | R450H | C | 1349 | T | AZI1 | R32H | C | 343 | T | BRCA2 | L1457S | T | 4603 | C | C15orf55 | L429M | C | 1285 | A |
| ASB2 | P198L | G | 593 | A | AZU1 | V62M | G | 200 | A | BRCA2 | F701C | T | 2335 | G | C15orf55 | R221H | G | 662 | A |
| ASB3 | N278S | T | 1074 | C | B2M | I66N | T | 267 | A | BRCA2 | L951I | C | 3084 | A | C15orf55 | Q121H | A | 363 | C |
| ASB3 | N306S | T | 1158 | C | B2M | F76C | T | 297 | G | BRCA2 | K956T | A | 3100 | C | C15orf55 | K105N | G | 315 | T |
| ASB3 | V435I | C | 1544 | T | B2M | E89* | G | 335 | T | BRCA2 | S3332Y | C | 10228 | A | C15orf55 | Q721H | G | 2163 | T |
| ASB4 | S333Y | C | 1069 | A | B3GALNT1 | S106Y | G | 666 | T | BRD1 | E520D | C | 1590 | A | C15orf59 | L217F | C | 996 | A |
| ASB4 | S139R | A | 486 | C | B3GALNT1 | A130T | C | 737 | T | BRD1 | E41* | C | 151 | A | C15orf59 | R26* | G | 421 | A |
| ASB4 | L154I | C | 531 | A | B3GALNT2 | D398E | A | 1423 | C | BRD1 | R668H | C | 2033 | T | C15orf60 | R25W | C | 101 | T |
| ASB4 | N262S | A | 856 | G | B3GALT2 | A408T | C | 1978 | T | BRD1 | A689V | G | 2096 | A | C15orf60 | D102Y | G | 332 | T |
| ASB5 | S23L | G | 182 | A | B3GALT2 | R284Q | C | 1607 | T | BRD1 | R933Q | C | 2828 | T | C15orf60 | T109M | C | 354 | T |
| ASB5 | H173Q | A | 633 | T | B3GALT5 | F160L | C | 1072 | A | BRD1 | A215T | C | 673 | T | C15orf61 | Q73H | G | 284 | T |
| ASB5 | L251V | A | 865 | C | B3GALTL | G57* | G | 318 | T | BRD1 | R737Q | C | 2240 | T | C15orf61 | L120V | T | 423 | G |
| ASB6 | R324H | C | 1137 | T | B3GAT1 | P247T | G | 1132 | T | BRD1 | R259H | C | 806 | T | C15orf63 | E84* | G | 4361 | T |
| ASB6 | A320E | G | 1125 | T | B3GAT1 | V89L | C | 658 | A | BRD3 | G649R | C | 2131 | T | C16orf11 | R359Q | G | 1355 | A |
| ASB6 | K268N | C | 970 | A | B3GAT1 | V11I | C | 424 | T | BRD4 | R669C | G | 2227 | A | C16orf11 | P30H | C | 368 | A |
| ASB6 | R278H | C | 999 | T | B3GAT3 | R45L | C | 163 | A | BRD4 | N560K | A | 1902 | T | C16orf11 | P192Q | C | 854 | A |
| ASB7 | F315C | T | 1729 | G | B3GAT3 | V8A | A | 52 | G | BRD4 | R1060C | G | 3400 | A | C16orf11 | G104R | G | 589 | A |
| ASB7 | A304S | G | 1695 | T | B3GNT4 | A56T | G | 522 | A | BRD4 | P56H | G | 389 | T | C16orf11 | A463T | G | 1666 | A |
| ASB8 | R259C | G | 944 | A | B3GNT4 | L242P | T | 1081 | C | BRD4 | L1361I | G | 4303 | T | C16orf11 | P102T | C | 583 | A |
| ASB8 | P275S | G | 992 | A | B3GNT4 | N227D | A | 1035 | G | BRD7 | Q133R | T | 403 | C | C16orf3 | A7V | G | 579 | A |
| ASCC2 | S315Y | G | 1058 | T | B3GNT6 | G282S | G | 932 | A | BRD7 | R613* | G | 1842 | A | C16orf42 | R276H | C | 934 | T |
| ASCC3 | K1957T | T | 6199 | G | B3GNT6 | P346S | C | 1124 | T | BRD7 | R96W | G | 291 | A | C16orf45 | T187M | C | 746 | T |
| ASCC3 | V1198A | A | 3922 | G | B3GNT6 | R350C | C | 1136 | T | BRD8 | D834Y | C | 2872 | A | C16orf46 | R351Q | C | 1117 | T |
| ASCC3 | R518C | G | 1881 | A | B3GNT9 | P159H | G | 476 | T | BRD8 | Q61H | T | 555 | G | C16orf48 | D310N | C | 1250 | T |
| ASCC3 | R675C | G | 2352 | A | B3GNT9 | V135M | C | 403 | T | BRD8 | R831* | G | 2863 | A | C16orf48 | Q293H | C | 1201 | G |
| ASCC3 | G1009C | C | 3354 | A | B3GNTL1 | S114L | G | 355 | A | BRD8 | R54H | C | 533 | T | C16orf54 | A88T | C | 358 | T |
| ASCC3 | - | C | 0 | T | B3GNTL1 | P90T | G | 282 | T | BRD8 | V40I | C | 490 | T | C16orf57 | R252H | G | 838 | A |
| ASCC3 | R1808C | G | 5751 | A | B4GALNT1 | A498T | C | 1924 | T | BRD8 | S916R | G | 3120 | T | C16orf59 | A121T | G | 420 | A |
| ASCL1 | H177Y | C | 1100 | T | B4GALNT1 | A30T | C | 520 | T | BRD8 | E219A | T | 1028 | G | C16orf62 | R318Q | G | 965 | A |
| ASCL3 | Y144H | A | 490 | G | B4GALNT1 | R5C | G | 445 | A | BRD8 | R98W | G | 664 | A | C16orf62 | N304K | C | 924 | G |
| ASF1A | K129E | A | 579 | G | B4GALNT1 | S314I | C | 1373 | A | BRD9 | R60* | G | 345 | A | C16orf62 | V259I | G | 787 | A |
| ASF1A | Q75R | A | 418 | G | B4GALNT1 | R228Q | C | 1115 | T | BRD9 | D109G | T | 493 | C | C16orf68 | R188W | C | 820 | T |
| ASF1A | E105K | G | 507 | A | B4GALNT2 | R303C | C | 966 | T | BRDT | E474* | G | 1733 | T | C16orf68 | V373M | G | 1375 | A |
| ASH1L | R1306Q | C | 4557 | T | B4GALNT2 | G383D | G | 1207 | A | BRDT | R853W | C | 2870 | T | C16orf68 | E286* | G | 1114 | T |
| ASH1L | R2426Q | C | 7917 | T | B4GALNT2 | R494Q | G | 1540 | A | BRDT | Q164H | G | 805 | T | C16orf7 | G605R | C | 1938 | T |

| | | | | | | | | | | | | | | | | | | | |
|---|---|---|---|---|---|---|---|---|---|---|---|---|---|---|---|---|---|---|---|
| ASH1L | R1516H | C | 5187 | T | B4GALNT3 | R121H | G | 375 | A | BRDT | K298N | G | 1207 | T | C16orf7 | R221M | C | 787 | A |
| ASH1L | R2968* | G | 9542 | A | B4GALNT3 | G553D | G | 1671 | A | BRDT | D878N | G | 2945 | A | C16orf7 | S589L | G | 1891 | A |
| ASH2L | A126V | C | 435 | T | B4GALNT3 | M906V | A | 2729 | G | BRE | I312V | A | 1072 | G | C16orf7 | P484H | G | 1576 | T |
| ASNA1 | G171S | G | 525 | A | B4GALNT4 | R114W | C | 340 | T | BRE | R23Q | G | 206 | A | C16orf70 | S403L | C | 1372 | T |
| ASNSD1 | V589M | G | 2178 | A | B4GALNT4 | R881W | C | 2641 | T | BRE | F207V | T | 757 | G | C16orf71 | P15L | C | 522 | T |
| ASPA | L147M | C | 530 | A | B4GALNT4 | A238T | G | 712 | A | BRF1 | I321L | T | 1610 | G | C16orf71 | G76S | G | 704 | A |
| ASPDH | A27V | G | 142 | A | B4GALNT4 | R815C | C | 2443 | T | BRF2 | D30N | C | 198 | T | C16orf72 | L196I | C | 1013 | A |
| ASPDH | G126W | C | 438 | A | B4GALNT4 | R878H | G | 2633 | A | BRI3BP | A123T | G | 453 | A | C16orf73 | L465V | A | 1403 | C |
| ASPDH | R89C | G | 327 | A | B4GALNT4 | R850C | C | 2548 | T | BRI3BP | - | A | 0 | C | C16orf73 | R342* | G | 1034 | A |
| ASPDH | V121M | C | 423 | T | B4GALNT4 | R756H | G | 2267 | A | BRI3BP | G14V | G | 127 | T | C16orf74 | - | A | 0 | G |
| ASPG | G386V | G | 1157 | T | B4GALT3 | R3Q | C | 231 | T | BRIP1 | G290S | C | 1009 | T | C16orf75 | V107A | T | 361 | C |
| ASPG | R270C | C | 808 | T | B4GALT3 | S49Y | G | 369 | T | BRIP1 | A745T | C | 2374 | T | C16orf78 | M28I | G | 201 | A |
| ASPG | A223T | G | 667 | A | B4GALT4 | G255* | C | 1405 | A | BRIP1 | A745T | C | 2374 | T | C16orf78 | L208V | T | 739 | G |
| ASPG | P58H | C | 173 | A | B4GALT4 | G218D | C | 1295 | T | BRIP1 | L358P | A | 1214 | G | C16orf78 | D241V | A | 839 | T |
| ASPG | F204L | C | 612 | A | B4GALT4 | D154N | C | 1102 | T | BRIP1 | K479T | T | 1577 | G | C16orf78 | R95T | G | 401 | C |
| ASPG | R210W | C | 628 | T | B4GALT5 | H176Y | G | 714 | A | BRIX1 | I186L | A | 919 | C | C16orf80 | R100C | G | 604 | A |
| ASPG | R270C | C | 808 | T | B4GALT6 | E125K | C | 670 | T | BRMS1 | I121V | T | 508 | C | C16orf88 | R392C | G | 1179 | A |
| ASPG | L448R | T | 1343 | G | B4GALT7 | A273T | G | 928 | A | BRMS1L | K98N | A | 420 | C | C16orf88 | K442E | T | 1329 | C |
| ASPH | G40* | C | 387 | A | B9D2 | R75G | G | 402 | C | BRP44 | L75V | A | 422 | C | C16orf90 | A39T | C | 113 | T |
| ASPH | E129G | T | 655 | C | BAAT | R297H | C | 999 | T | BRP44L | S108Y | G | 445 | T | C16orf91 | A95T | C | 283 | T |
| ASPH | A189V | G | 835 | A | BAAT | L406I | G | 1325 | T | BRPF1 | V1065M | G | 3597 | A | C16orf91 | S165I | C | 494 | A |
| ASPH | H493Y | G | 1746 | A | BAAT | E54* | C | 269 | A | BRPF1 | S460T | T | 1782 | A | C16orf91 | I176M | T | 528 | C |
| ASPHD1 | R222W | C | 778 | T | BACE1 | G395V | C | 1645 | A | BRPF1 | T441S | A | 1725 | T | C16orf92 | F117L | T | 372 | G |
| ASPHD1 | - | G | 0 | T | BACE1 | R412* | G | 1695 | A | BRPF1 | R946C | C | 3240 | T | C16orf93 | Y214* | G | 642 | T |
| ASPHD2 | R290H | G | 1307 | A | BACE1 | T164I | G | 952 | A | BRPF3 | R6W | C | 240 | T | C17orf101 | P292L | G | 966 | A |
| ASPHD2 | G121A | G | 800 | C | BACE2 | A300V | C | 1362 | T | BRPF3 | A763T | G | 2511 | A | C17orf101 | S96G | T | 377 | C |
| ASPHD2 | R258W | C | 1210 | T | BACH1 | L87F | A | 404 | C | BRPF3 | V1109I | G | 3549 | A | C17orf101 | G256R | C | 857 | T |
| ASPM | Q1495P | T | 4741 | G | BACH1 | N529S | A | 1729 | G | BRPF3 | R970H | G | 3133 | A | C17orf102 | R15* | G | 132 | A |
| ASPM | L250R | A | 1006 | C | BACH1 | R142I | G | 568 | T | BRS3 | A160V | C | 707 | T | C17orf102 | S25N | C | 163 | T |
| ASPM | A2663T | C | 8244 | T | BACH2 | F253C | A | 1466 | C | BRSK1 | V448I | G | 1342 | A | C17orf104 | F152V | T | 596 | G |
| ASPM | A1913V | G | 5995 | A | BAD | P101S | G | 572 | A | BRSK1 | - | G | 0 | T | C17orf104 | H503N | C | 1649 | A |
| ASPM | R2996W | G | 9243 | A | BAG1 | A26V | G | 511 | A | BRSK1 | V626M | G | 1876 | A | C17orf105 | I39V | A | 118 | G |
| ASPM | K2683R | T | 8305 | C | BAG3 | Y205C | A | 920 | G | BRSK1 | R776* | C | 2326 | T | C17orf105 | R138H | G | 416 | A |
| ASPM | V2362I | C | 7341 | T | BAG3 | K534T | A | 1907 | C | BRSK2 | A174V | C | 774 | T | C17orf106 | R92C | C | 572 | T |
| ASPM | A2461V | G | 7639 | A | BAG4 | P315L | C | 1020 | T | BRSK2 | R410H | G | 1482 | A | C17orf28 | L280M | G | 987 | T |
| ASPM | E3458D | T | 1063 1 | G | BAG4 | Y321H | T | 1037 | C | BRSK2 | S501G | A | 1754 | G | C17orf28 | V58M | C | 321 | T |
| ASPM | K3080N | T | 9497 | A | BAGE3 | K4315N | C | 1316 4 | A | BRWD1 | S696N | C | 2415 | T | C17orf39 | E178* | G | 575 | T |
| ASPM | R1164H | C | 3748 | T | BAGE3 | G4749S | C | 1446 | T | BRWD1 | E1647G | T | 5268 | C | C17orf42 | R278Q | C | 903 | T |

| | | | | | | | | | | | | | | | | | | | |
|---|---|---|---|---|---|---|---|---|---|---|---|---|---|---|---|---|---|---|---|
| | | | | | | | | 4 | | | | | | | | | | | |
| ASPM | E918K | C | 3009 | T | BAGE3 | R4196Q | C | 12806 | T | BRWD1 | R1074C | G | 3548 | A | C17orf46 | Q270H | C | 906 | A |
| ASPM | V1610D | A | 5086 | T | BAGE3 | V4395A | A | 13403 | G | BRWD1 | R1011Q | C | 3360 | T | C17orf47 | R51* | G | 287 | A |
| ASPN | R370K | C | 1353 | T | BAGE3 | E1152* | C | 3673 | A | BRWD1 | K1749T | T | 5574 | G | C17orf47 | L35I | G | 239 | T |
| ASPN | R84Q | C | 495 | T | BAGE3 | P3967S | G | 12118 | A | BRWD3 | C112R | A | 551 | G | C17orf50 | H157R | A | 515 | G |
| ASPN | S348R | T | 1286 | G | BAGE3 | G2479R | C | 7654 | T | BRWD3 | S1643C | T | 5144 | A | C17orf51 | S144T | A | 686 | T |
| ASPN | K276T | T | 1071 | G | BAGE3 | Y4941C | T | 15041 | C | BRWD3 | T362M | G | 1302 | A | C17orf51 | P61S | G | 437 | A |
| ASPN | K252N | T | 1000 | G | BAGE3 | E1436D | T | 4527 | G | BSDC1 | L179F | G | 547 | A | C17orf53 | R244I | G | 916 | T |
| ASPN | A25T | C | 317 | T | BAGE3 | S1308P | A | 4141 | G | BSG | R380S | C | 1236 | A | C17orf53 | R558Q | G | 1858 | A |
| ASPRV1 | G162W | C | 1061 | A | BAGE3 | S4304* | G | 13130 | T | BSG | R317C | C | 1047 | T | C17orf55 | W210C | C | 1062 | A |
| ASPRV1 | G203V | C | 1185 | A | BAGE3 | R2884* | G | 8869 | A | BSN | D1685N | G | 5167 | A | C17orf56 | T453M | G | 1380 | A |
| ASPRV1 | E240D | C | 1297 | A | BAGE3 | K1396N | T | 4407 | G | BSN | K650E | A | 2062 | G | C17orf56 | N94D | T | 302 | C |
| ASPSCR1 | R299W | C | 897 | T | BAGE3 | R1292Q | C | 4094 | T | BSN | R3664W | C | 11104 | T | C17orf56 | S216P | A | 668 | G |
| ASTE1 | V316I | C | 1153 | T | BAHCC1 | V1911I | G | 5731 | A | BSN | R1408C | C | 4336 | T | C17orf56 | R60H | C | 201 | T |
| ASTE1 | R570C | G | 1915 | A | BAHCC1 | A2542V | C | 7625 | T | BSN | K3342R | A | 10139 | G | C17orf56 | L20I | G | 80 | T |
| ASTE1 | Q307H | C | 1128 | A | BAHCC1 | R1684H | G | 5051 | A | BSN | P1243S | C | 3841 | T | C17orf57 | S931L | C | 3203 | T |
| ASTL | P105H | G | 314 | T | BAHCC1 | R319C | C | 955 | T | BSN | R2942Q | G | 8939 | A | C17orf57 | S960Y | C | 3290 | A |
| ASTN1 | D597N | C | 2001 | T | BAHCC1 | A1400T | G | 4198 | A | BSN | R1023C | C | 3181 | T | C17orf57 | K635N | G | 2316 | T |
| ASTN1 | R850C | G | 2760 | A | BAHCC1 | R2430W | C | 7288 | T | BSN | R3676C | C | 11140 | T | C17orf57 | N700H | A | 2509 | C |
| ASTN1 | Y203C | T | 820 | C | BAHCC1 | K1729N | G | 5187 | T | BSN | L3785I | C | 11467 | A | C17orf57 | L517V | C | 1960 | G |
| ASTN1 | S790L | G | 2581 | A | BAHCC1 | P1963L | C | 5888 | T | BSN | D805N | G | 2527 | A | C17orf58 | R82Q | C | 560 | T |
| ASTN1 | T1131M | G | 3604 | A | BAHCC1 | G528D | G | 1583 | A | BSN | P253L | C | 872 | T | C17orf59 | N219H | T | 761 | G |
| ASTN1 | R1253H | C | 3970 | T | BAHD1 | A702T | G | 2175 | A | BSND | F11L | C | 287 | A | C17orf63 | A488V | G | 1676 | A |
| ASTN1 | N1204H | T | 3822 | G | BAHD1 | R172W | C | 585 | T | BSND | A192T | G | 828 | A | C17orf63 | A43V | G | 341 | A |
| ASTN1 | R1012Q | C | 3247 | T | BAHD1 | R533H | G | 1669 | A | BSPRY | E182A | A | 584 | C | C17orf66 | - | T | 0 | C |
| ASTN2 | G1038W | C | 3213 | A | BAI1 | T1124M | C | 3554 | T | BSX | R133C | G | 446 | A | C17orf68 | A825T | C | 2532 | T |
| ASTN2 | G326* | C | 1077 | A | BAI1 | P75L | C | 407 | T | BTAF1 | S1319P | T | 3967 | C | C17orf70 | R522* | G | 2283 | A |
| ASTN2 | F732V | A | 2295 | C | BAI1 | D1173N | G | 3700 | A | BTAF1 | K396* | A | 1198 | T | C17orf70 | R188W | G | 1281 | A |
| ASTN2 | R653C | G | 2058 | A | BAI1 | A596V | C | 1970 | T | BTAF1 | R1025* | A | 3085 | T | C17orf71 | R127Q | G | 422 | A |
| ASXL1 | L857P | T | 2996 | C | BAI1 | N607K | C | 2004 | A | BTAF1 | G1294V | G | 3893 | T | C17orf71 | A830V | C | 2531 | T |
| ASXL1 | R302C | C | 1330 | T | BAI1 | L108P | T | 506 | C | BTAF1 | D1625N | G | 4885 | A | C17orf71 | G950V | G | 2891 | T |
| ASXL1 | S25L | C | 500 | T | BAI1 | T950M | C | 3032 | T | BTAF1 | K1239N | G | 3729 | T | C17orf71 | C197R | T | 631 | C |
| ASXL1 | A1248V | C | 4169 | T | BAI1 | V405M | G | 1396 | A | BTBD1 | G365D | C | 1297 | T | C17orf74 | Q146H | G | 511 | T |
| ASXL1 | D756N | G | 2692 | A | BAI1 | R490H | G | 1652 | A | BTBD11 | A833T | G | 3025 | A | C17orf75 | N181T | T | 573 | G |
| ASXL1 | S1341N | G | 4448 | A | BAI1 | E671D | G | 2196 | T | BTBD11 | V613G | T | 2366 | G | C17orf76 | D83A | T | 276 | G |

| | | | | | | | | | | | | | | | | | | | |
|---|---|---|---|---|---|---|---|---|---|---|---|---|---|---|---|---|---|---|---|
| ASXL1 | A158V | C | 899 | T | BAI1 | F553L | C | 1842 | A | BTBD11 | R434H | G | 1829 | A | C17orf78 | P154H | C | 511 | A |
| ASXL2 | S250* | G | 749 | T | BAI2 | R965C | G | 3247 | A | BTBD11 | A185T | G | 1081 | A | C17orf80 | L25V | T | 267 | G |
| ASXL3 | V868I | G | 2657 | A | BAI2 | P1556S | G | 5020 | A | BTBD11 | A561V | C | 2210 | T | C17orf80 | K113N | G | 533 | T |
| ASXL3 | R1052M | G | 3210 | T | BAI2 | G1055A | C | 3518 | G | BTBD11 | V635A | T | 2432 | C | C17orf85 | R491C | G | 1494 | A |
| ASXL3 | A52V | C | 210 | T | BAI3 | R1452W | C | 5175 | T | BTBD11 | R710C | C | 2656 | T | C17orf85 | R497I | C | 1513 | A |
| ASXL3 | D106Y | G | 371 | T | BAI3 | F73I | T | 1038 | A | BTBD11 | F1063L | C | 3717 | G | C17orf87 | A24D | G | 71 | T |
| ASXL3 | I299S | T | 951 | G | BAI3 | T428I | C | 2104 | T | BTBD11 | D571G | A | 2240 | G | C17orf97 | G7A | G | 36 | C |
| ASXL3 | R933W | C | 2852 | T | BAI3 | S408L | C | 2044 | T | BTBD12 | R1366H | C | 4738 | T | C17orf98 | A2V | G | 27 | A |
| ASXL3 | Y2125* | T | 6430 | G | BAI3 | - | G | 0 | T | BTBD12 | V1284M | C | 4491 | T | C17orf99 | T297M | C | 890 | T |
| ASZ1 | K208T | T | 686 | G | BAI3 | F611L | C | 2654 | A | BTBD12 | W800R | A | 3039 | G | C18orf1 | R280I | G | 1507 | T |
| ATAD1 | P186S | G | 935 | A | BAI3 | K1046R | A | 3958 | G | BTBD12 | T697M | G | 2731 | A | C18orf1 | P2L | C | 673 | T |
| ATAD1 | H256N | G | 1145 | T | BAI3 | R1197Q | G | 4411 | A | BTBD12 | R1060Q | C | 3820 | T | C18orf22 | K277N | G | 969 | T |
| ATAD2 | T1311A | T | 4039 | C | BAIAP2 | L393I | C | 1270 | A | BTBD12 | E971* | C | 3552 | A | C18orf25 | I237L | A | 1088 | C |
| ATAD2 | R156Q | C | 575 | T | BAIAP2L1 | T458M | G | 1589 | A | BTBD12 | I854M | A | 3203 | C | C18orf26 | A55S | G | 209 | T |
| ATAD2 | R1367C | G | 4207 | A | BAIAP2L1 | N152H | T | 670 | G | BTBD16 | S395L | C | 1435 | T | C18orf26 | K30N | G | 136 | T |
| ATAD2 | L185V | G | 661 | C | BAIAP2L2 | R228H | C | 828 | T | BTBD16 | E217K | G | 900 | A | C18orf32 | E43* | C | 339 | A |
| ATAD2 | Q1377R | T | 4238 | C | BAIAP2L2 | V169M | C | 650 | T | BTBD17 | F231L | A | 691 | G | C18orf34 | G867D | C | 2742 | T |
| C18orf34 | I611V | T | 1973 | C | C4orf43 | A113D | C | 336 | A | CAMTA1 | F681V | T | 2248 | G | CCDC88C | A1289V | G | 3965 | A |
| C18orf54 | P144S | C | 546 | T | C4orf46 | E83* | C | 492 | A | CAMTA1 | A802V | C | 2612 | T | CCDC88C | P2009L | G | 6125 | A |
| C18orf54 | R517C | C | 1665 | T | C4orf49 | T197A | T | 769 | C | CAMTA2 | R1180* | G | 3662 | A | CCDC88C | E1819D | C | 5556 | A |
| C18orf8 | E162K | G | 605 | A | C4orf49 | R31C | G | 271 | A | CAND1 | M323I | G | 1406 | T | CCDC88C | A485V | G | 1553 | A |
| C18orf8 | - | A | 0 | C | C4orf50 | E59* | C | 1473 | A | CAND1 | A354V | C | 1498 | T | CCDC88C | A302V | G | 1004 | A |
| C18orf8 | K556* | A | 1787 | T | C4orf6 | R34H | G | 276 | A | CAND1 | K661T | A | 2419 | C | CCDC88C | S787G | T | 2458 | C |
| C19orf12 | E95K | C | 410 | T | C5 | V1364A | A | 4121 | G | CAND2 | E935K | G | 2844 | A | CCDC89 | V321M | C | 1108 | T |
| C19orf12 | K37N | C | 238 | A | C5 | R1206C | G | 3646 | A | CAND2 | S1096* | C | 3328 | A | CCDC9 | R230Q | G | 896 | A |
| C19orf18 | S78F | G | 337 | A | C5 | L1129V | A | 3415 | C | CANT1 | L5Q | A | 448 | T | CCDC9 | G518D | G | 1760 | A |
| C19orf2 | I48F | A | 439 | T | C5 | Y256D | A | 796 | C | CANX | R479* | C | 1435 | T | CCDC91 | E190* | G | 584 | T |
| C19orf2 | P333S | C | 1294 | T | C5 | T136I | G | 437 | A | CAP1 | E156* | G | 1027 | T | CCDC91 | E208K | G | 638 | A |
| C19orf2 | E276K | G | 1123 | A | C5AR1 | T62M | C | 234 | T | CAP1 | Q260H | G | 1341 | T | CCDC93 | V488D | A | 1601 | T |
| C19orf2 | R413Q | G | 1535 | A | C5orf13 | C45R | A | 325 | G | CAP2 | V46M | G | 668 | A | CCDC93 | K352T | T | 1193 | G |
| C19orf21 | E283V | A | 944 | T | C5orf22 | D172N | G | 641 | A | CAP2 | G228V | G | 1215 | T | CCDC93 | K308Q | T | 1060 | G |
| C19orf21 | R406C | C | 1312 | T | C5orf25 | R40C | C | 525 | T | CAPN1 | G411R | G | 1374 | A | CCDC93 | E292K | C | 1012 | T |
| C19orf22 | A162T | C | 552 | T | C5orf25 | R828C | C | 2889 | T | CAPN1 | L370P | T | 1252 | C | CCDC94 | R82C | C | 280 | T |
| C19orf25 | P115H | G | 383 | T | C5orf30 | A87T | G | 567 | A | CAPN10 | G526S | G | 1772 | A | CCDC94 | L300P | T | 935 | C |
| C19orf26 | A714T | C | 2415 | T | C5orf30 | R45Q | G | 442 | A | CAPN12 | A440T | C | 1627 | T | CCDC96 | K498R | T | 1556 | C |
| C19orf26 | - | T | 0 | C | C5orf30 | K166T | A | 805 | C | CAPN12 | F367L | G | 1410 | T | CCDC96 | N496I | T | 1550 | A |
| C19orf28 | T390M | G | 1339 | A | C5orf32 | G50S | G | 812 | A | CAPN12 | L443P | A | 1637 | G | CCDC96 | S555Y | G | 1727 | T |
| C19orf29 | Y398C | T | 1450 | C | C5orf33 | R378Q | C | 1133 | T | CAPN13 | S247C | T | 912 | A | CCDC97 | R146Q | G | 559 | A |
| C19orf29 | R571Q | C | 1969 | T | C5orf35 | V194L | G | 966 | C | CAPN13 | R262Q | C | 958 | T | CCDC97 | F111L | T | 453 | C |

| | | | | | | | | | | | | | | | | | | | |
|---|---|---|---|---|---|---|---|---|---|---|---|---|---|---|---|---|---|---|---|
| C19orf29 | A452V | G | 1612 | A | C5orf36 | G1029R | C | 3086 | T | CAPN13 | R56C | G | 339 | A | CCDC99 | R203H | G | 887 | A |
| C19orf29 | R685H | C | 2311 | T | C5orf36 | R1087H | C | 3261 | T | CAPN14 | R277Q | C | 972 | T | CCDC99 | S252N | G | 1034 | A |
| C19orf29 | E550D | C | 1907 | A | C5orf36 | S1161Y | G | 3483 | T | CAPN14 | N503I | T | 1650 | A | CCDC99 | R264Q | G | 1070 | A |
| C19orf29 | T269A | T | 1062 | C | C5orf36 | E1157* | C | 3470 | A | CAPN14 | - | A | 0 | G | CCIN | R43H | G | 239 | A |
| C19orf29 | R114W | G | 597 | A | C5orf36 | L659I | G | 1976 | T | CAPN14 | P5S | G | 155 | A | CCIN | R174C | C | 631 | T |
| C19orf29OS | A98T | G | 292 | A | C5orf36 | F274L | G | 823 | T | CAPN14 | R444I | C | 1473 | A | CCIN | P177S | C | 640 | T |
| C19orf36 | G45R | G | 557 | A | C5orf38 | R30Q | G | 206 | A | CAPN2 | V485M | G | 1762 | A | CCIN | R327C | C | 1090 | T |
| C19orf43 | S53L | G | 216 | A | C5orf38 | K113N | G | 456 | T | CAPN2 | R418Q | G | 1562 | A | CCK | A18T | C | 958 | T |
| C19orf44 | V571A | T | 1785 | C | C5orf41 | Q465H | G | 1395 | C | CAPN2 | A179T | G | 844 | A | CCK | R106C | G | 1222 | A |
| C19orf45 | S246L | C | 878 | T | C5orf41 | R17Q | G | 50 | A | CAPN2 | R318W | C | 1261 | T | CCKAR | P146S | G | 631 | A |
| C19orf46 | A211T | C | 743 | T | C5orf41 | F613C | T | 1838 | G | CAPN2 | R285Q | G | 1163 | A | CCKAR | R73Q | C | 413 | T |
| C19orf46 | R267Q | C | 912 | T | C5orf42 | R785* | G | 2447 | A | CAPN5 | E433K | G | 1297 | A | CCKAR | S233Y | G | 893 | T |
| C19orf46 | G222V | C | 777 | A | C5orf42 | L1379V | A | 4229 | C | CAPN6 | R263C | G | 955 | A | CCKBR | R264* | C | 983 | T |
| C19orf46 | A345T | C | 1145 | T | C5orf42 | K3092Q | T | 9368 | G | CAPN6 | A86E | G | 425 | T | CCKBR | G268R | G | 995 | A |
| C19orf46 | A266S | C | 908 | A | C5orf42 | E2906* | C | 8810 | A | CAPN7 | K723N | G | 2422 | T | CCKBR | A62T | G | 377 | A |
| C19orf47 | R244H | C | 731 | T | C5orf42 | - | C | 0 | A | CAPN7 | F543C | T | 1881 | G | CCKBR | R243H | G | 921 | A |
| C19orf51 | R288S | G | 862 | T | C5orf42 | R1424I | C | 4365 | A | CAPN8 | P385H | G | 1154 | T | CCL14 | C51Y | C | 231 | T |
| C19orf54 | G323D | C | 1088 | T | C5orf42 | R978Q | C | 3027 | T | CAPN8 | - | C | 0 | T | CCL17 | M6I | G | 147 | A |
| C19orf54 | P346S | G | 1156 | A | C5orf44 | R336M | G | 1034 | T | CAPN8 | Q608H | C | 1824 | A | CCL2 | A6T | G | 72 | A |
| C19orf55 | A389T | G | 1165 | A | C5orf46 | G13* | C | 38 | A | CAPN8 | E175* | C | 523 | A | CCL2 | V3I | G | 63 | A |
| C19orf55 | F109L | T | 325 | C | C5orf46 | L65I | G | 194 | T | CAPN8 | F46C | A | 137 | C | CCL25 | C31Y | G | 198 | A |
| C19orf55 | D223N | G | 667 | A | C5orf47 | A59V | C | 281 | T | CAPN9 | A667V | C | 2113 | T | CCL25 | - | G | 0 | A |
| C19orf60 | A176V | C | 527 | T | C5orf48 | L23I | C | 80 | A | CAPN9 | R275Q | G | 937 | A | CCL25 | R93Q | G | 384 | A |
| C19orf60 | V201A | T | 602 | C | C5orf49 | Y66H | A | 665 | G | CAPNS1 | K9Q | A | 179 | C | CCL28 | R50H | C | 224 | T |
| C19orf60 | A181T | G | 541 | A | C5orf49 | Y66H | A | 665 | G | CAPRIN1 | P266S | C | 985 | T | CCL7 | R71W | C | 251 | T |
| C19orf61 | Q62* | G | 527 | A | C5orf49 | R105C | G | 782 | A | CAPRIN1 | W316* | G | 1137 | A | CCL7 | R47I | G | 180 | T |
| C19orf62 | K323T | A | 1066 | C | C5orf51 | H207N | C | 638 | A | CAPRIN1 | P409T | C | 1414 | A | CCM2 | N12H | A | 353 | C |
| C19orf63 | R109W | C | 391 | T | C5orf54 | F480L | A | 1904 | C | CAPRIN1 | K125N | A | 564 | C | CCNA1 | Q80* | C | 502 | T |
| C19orf66 | R14C | C | 338 | T | C5orf58 | L8I | C | 22 | A | CAPRIN1 | H554R | A | 1850 | G | CCNA1 | Q128H | A | 648 | C |
| C19orf70 | D82G | T | 659 | C | C5orf60 | L84I | G | 318 | T | CAPRIN2 | Q417H | C | 2002 | A | CCNA1 | A262S | G | 1048 | T |
| C19orf71 | R93H | G | 299 | A | C6 | S273R | T | 1082 | G | CAPRIN2 | - | C | 0 | A | CCNA1 | V341I | G | 1285 | A |
| C19orf77 | R27C | G | 158 | A | C6orf103 | A553E | C | 1699 | A | CAPRIN2 | K178N | C | 1285 | A | CCNA1 | L192I | C | 838 | A |
| C1orf100 | R122Q | G | 478 | A | C6orf103 | W632G | T | 1935 | G | CAPRIN2 | R149H | C | 1197 | T | CCNB1 | A44V | C | 384 | T |
| C1orf100 | R13H | G | 151 | A | C6orf103 | F1004C | T | 3052 | G | CAPS | R275C | C | 3381 | T | CCNB2 | R6C | C | 207 | T |
| C1orf100 | R77* | C | 342 | T | C6orf114 | G59V | C | 699 | A | CAPSL | R6H | C | 144 | T | CCNB3 | Q1153E | C | 3755 | G |
| C1orf101 | D435Y | G | 1357 | T | C6orf118 | H199Y | G | 616 | A | CAPZA1 | E200D | A | 1272 | C | CCNB3 | K492N | G | 1774 | T |
| C1orf103 | F574C | A | 1977 | C | C6orf118 | L447I | A | 1360 | T | CAPZA2 | E282D | G | 985 | T | CCND1 | R260C | C | 987 | T |
| C1orf103 | F300L | A | 1154 | G | C6orf118 | A348V | G | 1064 | A | CAPZA2 | K91N | G | 412 | T | CCND1 | L233F | C | 906 | T |
| C1orf106 | A448T | G | 1542 | A | C6orf118 | A212T | C | 655 | T | CAPZA2 | V32G | T | 234 | G | CCND1 | A187T | G | 768 | A |

| | | | | | | | | | | | | | | | | | | | |
|---|---|---|---|---|---|---|---|---|---|---|---|---|---|---|---|---|---|---|---|
| C1orf107 | V511I | G | 1588 | A | C6orf118 | A212V | G | 656 | A | CAPZA2 | K147N | A | 580 | C | CCNE1 | P407L | C | 1403 | T |
| C1orf107 | S182Y | C | 602 | A | C6orf120 | A19V | C | 82 | T | CAPZA3 | A226E | C | 835 | A | CCNE1 | A214V | C | 824 | T |
| C1orf107 | K389T | A | 1223 | C | C6orf125 | R62* | G | 225 | A | CAPZB | R330I | C | 989 | A | CCNE2 | T47A | T | 245 | C |
| C1orf107 | R437Q | G | 1367 | A | C6orf132 | R931C | G | 2791 | A | CARD10 | R749W | G | 2462 | A | CCNF | K503T | A | 1571 | C |
| C1orf109 | L159I | G | 665 | T | C6orf138 | L690F | G | 2102 | A | CARD10 | R404Q | C | 1428 | T | CCNF | V705I | G | 2176 | A |
| C1orf110 | S162Y | G | 503 | T | C6orf145 | E169K | C | 1000 | T | CARD10 | A541T | C | 1838 | T | CCNG2 | E225K | G | 1029 | A |
| C1orf110 | F70L | G | 228 | T | C6orf146 | N182S | T | 545 | C | CARD11 | T353A | T | 1461 | C | CCNG2 | - | G | 1391 | T |
| C1orf112 | L612V | T | 2181 | G | C6orf150 | E385D | T | 1250 | G | CARD11 | R891Q | C | 3076 | T | CCNG2 | C117R | T | 705 | C |
| C1orf112 | S566I | G | 2044 | T | C6orf150 | F379S | A | 1231 | G | CARD11 | R912W | G | 3138 | A | CCNH | Q136* | G | 631 | A |
| C1orf113 | E306D | G | 1082 | T | C6orf150 | M32I | C | 191 | T | CARD11 | R1027* | G | 3483 | A | CCNI | E64* | C | 767 | A |
| C1orf113 | V96M | G | 450 | A | C6orf162 | R29C | C | 174 | T | CARD11 | A1046T | C | 3540 | T | CCNI2 | V283I | G | 898 | A |
| C1orf113 | P551L | C | 1816 | T | C6orf165 | R601H | G | 1914 | A | CARD11 | R920C | G | 3162 | A | CCNJ | R61C | C | 540 | T |
| C1orf114 | T7S | G | 173 | C | C6orf165 | K604T | A | 1923 | C | CARD11 | N444K | G | 1736 | T | CCNJ | S316L | C | 1306 | T |
| C1orf116 | S177R | T | 779 | G | C6orf165 | M67I | G | 313 | C | CARD11 | M12I | C | 440 | T | CCNJ | V283A | T | 1207 | C |
| C1orf124 | P333H | C | 1334 | A | C6orf167 | F532L | A | 1862 | T | CARD11 | R424W | G | 1674 | A | CCNK | G354S | G | 1060 | A |
| C1orf125 | E994Q | G | 3367 | C | C6orf167 | A785S | C | 2619 | A | CARD11 | S562* | G | 2089 | T | CCNK | E52V | A | 155 | T |
| C1orf125 | A365V | C | 1481 | T | C6orf167 | V1008M | C | 3288 | T | CARD14 | A282V | C | 1046 | T | CCNK | A336V | C | 1007 | T |
| C1orf125 | L466P | T | 1784 | C | C6orf167 | Q148H | C | 710 | A | CARD14 | A10T | G | 229 | A | CCNL1 | F334L | A | 1121 | C |
| C1orf125 | L957I | C | 3256 | A | C6orf168 | S24P | A | 353 | G | CARD6 | D490N | G | 1484 | A | CCNL1 | H465Q | A | 1514 | T |
| C1orf125 | H164R | A | 878 | G | C6orf168 | E350K | C | 1331 | T | CARD6 | F596V | T | 1802 | G | CCNL1 | A309T | C | 1044 | T |
| C1orf125 | R574* | C | 2107 | T | C6orf170 | L1129I | G | 3453 | T | CARD8 | D349G | T | 1260 | C | CCNL1 | R239* | G | 834 | A |
| C1orf125 | K109T | A | 713 | C | C6orf170 | K338N | C | 1082 | A | CARD8 | D329Y | C | 1199 | A | CCNL2 | L291I | G | 877 | T |
| C1orf125 | A670V | C | 2396 | T | C6orf174 | G368C | C | 1947 | A | CARD9 | R305H | C | 1080 | T | CCNL2 | H512R | T | 1541 | C |
| C1orf127 | P4L | G | 16 | A | C6orf174 | G368C | C | 1947 | A | CARHSP1 | D87N | C | 619 | T | CCNL2 | A179V | G | 542 | A |
| C1orf127 | A151V | G | 457 | A | C6orf174 | R454K | C | 2206 | T | CARHSP1 | P44L | G | 491 | A | CCNL2 | L184I | G | 556 | T |
| C1orf127 | Q87* | G | 264 | A | C6orf174 | F277C | A | 1675 | C | CARM1 | T480M | C | 1565 | T | CCNO | A87V | G | 417 | A |
| C1orf128 | R131W | C | 502 | T | C6orf182 | R256* | C | 1343 | T | CARNS1 | R789C | C | 2455 | T | CCNT1 | I244S | A | 1054 | C |
| C1orf128 | H208R | A | 734 | G | C6orf203 | L148P | T | 1133 | C | CARNS1 | L902I | C | 2794 | A | CCNT1 | K596T | T | 2110 | G |
| C1orf129 | V358M | G | 1171 | A | C6orf203 | G24C | G | 760 | T | CARNS1 | R659Q | G | 2066 | A | CCNT2 | Q45R | A | 164 | G |
| C1orf129 | E90* | G | 367 | T | C6orf203 | Q140P | A | 1109 | C | CARS | A631T | C | 1961 | T | CCNYL1 | F348V | T | 1253 | G |
| C1orf129 | L139R | T | 1575 | G | C6orf204 | T766A | T | 2918 | C | CARS | K131E | T | 461 | C | CCPG1 | R397S | C | 1356 | A |
| C1orf130 | S29Y | C | 152 | A | C6orf204 | P742S | G | 2846 | A | CARS | A215T | C | 713 | T | CCPG1 | E92K | C | 439 | T |
| C1orf131 | T142M | G | 462 | A | C6orf204 | F357L | A | 1691 | G | CARS2 | E167K | C | 544 | T | CCR2 | A306V | C | 1402 | T |
| C1orf135 | R187Q | C | 615 | T | C6orf204 | E759D | C | 2899 | A | CARS2 | P251L | G | 797 | A | CCR4 | T348M | C | 1211 | T |
| C1orf14 | V253F | C | 757 | A | C6orf204 | K660N | C | 2602 | A | CASC1 | N577H | T | 1795 | G | CCR4 | C110R | T | 496 | C |
| C1orf14 | R681M | C | 2042 | A | C6orf204 | K220T | T | 1281 | G | CASC1 | R470S | T | 1476 | G | CCR5 | F263L | C | 911 | A |
| C1orf14 | A597T | C | 1789 | T | C6orf211 | D291G | A | 1131 | G | CASC1 | E278* | C | 898 | A | CCR7 | P278S | G | 895 | A |
| C1orf14 | M565I | C | 1695 | A | C6orf221 | R158C | C | 525 | T | CASC1 | R54Q | C | 227 | T | CCR9 | L87P | T | 440 | C |
| C1orf141 | Q319H | C | 1068 | A | C6orf222 | A442V | G | 1503 | A | CASC1 | E45* | C | 199 | A | CCR9 | N12D | A | 214 | G |

| | | | | | | | | | | | | | | | | | | | |
|---|---|---|---|---|---|---|---|---|---|---|---|---|---|---|---|---|---|---|---|
| C1orf145 | P120T | G | 362 | T | C6orf222 | A12V | G | 213 | A | CASC3 | T689I | C | 2361 | T | CCRL1 | Q26* | C | 172 | T |
| C1orf147 | P99H | G | 307 | T | C6orf222 | - | C | 0 | T | CASC3 | D171E | T | 808 | G | CCRL1 | Q26* | C | 172 | T |
| C1orf159 | - | T | 0 | C | C6orf222 | D494Y | C | 1658 | A | CASC5 | S682Y | C | 2206 | A | CCRL2 | R330C | C | 1101 | T |
| C1orf159 | G219V | C | 867 | A | C6orf223 | A208V | C | 643 | T | CASC5 | Q1255H | A | 3926 | C | CCRL2 | T102I | C | 418 | T |
| C1orf168 | E650* | C | 2029 | A | C6orf223 | A232S | G | 714 | T | CASC5 | A1599T | G | 4956 | A | CCRN4L | P220L | C | 852 | T |
| C1orf170 | P215S | G | 643 | A | C6orf57 | R104C | C | 334 | T | CASC5 | F1667V | T | 5160 | G | CCT2 | V104I | G | 404 | A |
| C1orf173 | S856P | A | 2785 | G | C6orf64 | F116S | A | 447 | G | CASD1 | K541I | A | 1909 | T | CCT2 | R516H | G | 1641 | A |
| C1orf173 | N1041D | T | 3340 | C | C6orf64 | W83L | C | 348 | A | CASD1 | K80N | G | 527 | T | CCT3 | P75Q | G | 444 | T |
| C1orf173 | S893Y | G | 2897 | T | C6orf70 | - | T | 0 | C | CASD1 | R496C | C | 1773 | T | CCT3 | R518Q | C | 1773 | T |
| C1orf173 | Q518R | T | 1772 | C | C6orf70 | L466* | T | 1430 | G | CASD1 | S579Y | C | 2023 | A | CCT4 | R441* | G | 1618 | A |
| C1orf173 | A1375T | C | 4342 | T | C6orf81 | E183K | G | 574 | A | CASD1 | W657G | T | 2256 | G | CCT4 | H129R | T | 683 | C |
| C1orf173 | F1163C | A | 3707 | C | C6orf81 | A291T | G | 898 | A | CASD1 | I762T | T | 2572 | C | CCT6A | R314H | G | 1160 | A |
| C1orf173 | G1162E | C | 3704 | T | C6orf94 | S207Y | C | 724 | A | CASK | G206S | C | 1091 | T | CCT6A | D284N | G | 1069 | A |
| C1orf173 | T1036N | G | 3326 | T | C6orf94 | A132T | G | 498 | A | CASK | R537* | G | 2084 | A | CCT6B | A417T | C | 1343 | T |
| C1orf173 | K982T | T | 3164 | G | C6orf97 | Q519H | G | 1598 | T | CASKIN1 | V1284I | C | 3882 | T | CCT6B | D480Y | C | 1532 | A |
| C1orf173 | K765I | T | 2513 | A | C6orf97 | R39W | C | 156 | T | CASKIN1 | R952H | C | 2887 | T | CCT6B | K146T | T | 531 | G |
| C1orf173 | E641* | C | 2140 | A | C6orf97 | E271D | A | 854 | C | CASKIN1 | R271K | C | 844 | T | CCT7 | Y358H | T | 1213 | C |
| C1orf174 | R54* | G | 259 | A | C6orf97 | R46Q | G | 178 | A | CASKIN1 | T689N | G | 2098 | T | CCT8 | A70S | C | 415 | A |
| C1orf175 | V441A | T | 1322 | C | C7 | C791Y | G | 2731 | A | CASKIN1 | E656K | C | 1998 | T | CCT8 | C430R | A | 1495 | G |
| C1orf175 | A636V | C | 1907 | T | C7 | V536I | G | 1965 | A | CASKIN2 | E242D | C | 1276 | A | CCT8L2 | D300N | C | 1158 | T |
| C1orf175 | M1340T | T | 4019 | C | C7 | C797Y | G | 2749 | A | CASKIN2 | E227K | C | 1229 | T | CCT8L2 | R133Q | C | 658 | T |
| C1orf183 | W169C | C | 665 | G | C7 | E720A | A | 2518 | C | CASKIN2 | G773S | C | 2867 | T | CCT8L2 | L228P | A | 943 | G |
| C1orf183 | A117T | C | 507 | T | C7orf10 | G357D | G | 1094 | A | CASKIN2 | E671K | C | 2561 | T | CCT8L2 | N291D | T | 1131 | C |
| C1orf185 | T137I | C | 410 | T | C7orf10 | V301I | G | 925 | A | CASKIN2 | A1120V | G | 3909 | A | CD101 | R814C | C | 2498 | T |
| C1orf185 | A2T | G | 4 | A | C7orf11 | - | C | 0 | A | CASKIN2 | P1069S | G | 3755 | A | CD101 | Q57P | A | 228 | C |
| C1orf186 | A15T | C | 682 | T | C7orf16 | R135G | A | 1031 | G | CASP1 | K203E | T | 643 | C | CD101 | P717S | C | 2207 | T |
| C1orf186 | E123K | C | 1006 | T | C7orf27 | A652T | C | 2211 | T | CASP10 | M1I | G | 438 | T | CD101 | K993T | A | 3036 | C |
| C1orf189 | K15N | C | 71 | A | C7orf29 | G83A | G | 341 | C | CASP2 | R361C | C | 1322 | T | CD109 | K429N | G | 1399 | T |
| C1orf190 | T108N | C | 416 | A | C7orf30 | L42F | C | 277 | T | CASP4 | A290V | G | 1780 | A | CD109 | S119I | G | 468 | T |
| C1orf194 | R46W | G | 212 | A | C7orf31 | I311M | T | 1242 | C | CASP4 | G211D | C | 1543 | T | CD109 | V531A | T | 1704 | C |
| C1orf200 | I165V | T | 643 | C | C7orf34 | K55N | G | 163 | T | CASP5 | R414W | G | 1272 | A | CD109 | L1321F | A | 4075 | C |
| C1orf201 | N172D | T | 649 | C | C7orf42 | V180M | G | 793 | A | CASP5 | A125P | C | 405 | G | CD109 | S1322I | G | 4077 | T |
| C1orf228 | P294S | C | 1187 | T | C7orf43 | S300A | A | 1084 | C | CASP5 | E354D | C | 1094 | A | CD109 | E541D | A | 1735 | C |
| C1orf26 | - | T | 0 | C | C7orf44 | K90R | T | 588 | C | CASP8 | M163I | G | 685 | A | CD109 | P91S | C | 383 | T |
| C1orf26 | G90S | G | 362 | A | C7orf45 | A58V | C | 224 | T | CASP8 | Q388* | C | 1358 | T | CD160 | L123R | A | 591 | C |
| C1orf27 | A289S | G | 1021 | T | C7orf45 | A129V | C | 437 | T | CASP8 | I501T | T | 1698 | C | CD163 | - | A | 0 | C |
| C1orf27 | A24V | C | 227 | T | C7orf45 | H212Y | C | 685 | T | CASP8 | P291H | C | 1068 | A | CD163 | N454S | T | 1361 | C |
| C1orf49 | L84R | T | 363 | G | C7orf47 | Q168* | G | 693 | A | CASP8AP2 | P866L | C | 2832 | T | CD163 | S448Y | G | 1343 | T |
| C1orf49 | K101R | A | 414 | G | C7orf47 | P53H | G | 349 | T | CASP8AP2 | K1631* | A | 5126 | T | CD163L1 | L270I | G | 834 | T |

| | | | | | | | | | | | | | | | | | | | |
|---|---|---|---|---|---|---|---|---|---|---|---|---|---|---|---|---|---|---|---|
| C1orf51 | S55* | C | 300 | A | C7orf51 | E381D | G | 1302 | T | CASP8AP2 | E1294K | G | 4115 | A | CD163L1 | E1428* | C | 4308 | A |
| C1orf51 | S218N | G | 789 | A | C7orf52 | P40S | G | 357 | A | CASP8AP2 | R317C | C | 1184 | T | CD163L1 | G704D | C | 2137 | T |
| C1orf54 | M1K | T | 772 | A | C7orf53 | T100P | A | 433 | C | CASP8AP2 | K999E | A | 3230 | G | CD163L1 | G1211C | C | 3657 | A |
| C1orf55 | E328* | C | 1001 | A | C7orf57 | R297* | C | 890 | T | CASQ1 | A212T | G | 830 | A | CD163L1 | L1332I | G | 4020 | T |
| C1orf58 | F329C | T | 1392 | G | C7orf57 | D194N | G | 581 | A | CASQ2 | R121C | G | 601 | A | CD163L1 | N847S | T | 2566 | C |
| C1orf61 | K177N | C | 530 | A | C7orf58 | F945C | T | 3301 | G | CASQ2 | P353Q | G | 1298 | T | CD164 | S49F | G | 212 | A |
| C1orf64 | A58E | C | 241 | A | C7orf60 | V359L | C | 1241 | G | CASQ2 | L79R | A | 476 | C | CD164L2 | G5* | C | 154 | A |
| C1orf65 | R380W | C | 1241 | T | C7orf61 | Y241C | T | 722 | C | CASR | R331W | C | 1429 | T | CD177 | A8T | G | 22 | A |
| C1orf65 | T28M | C | 186 | T | C7orf62 | V110A | A | 515 | G | CASR | V1073A | T | 3656 | C | CD177P1 | P135L | G | 404 | A |
| C1orf65 | E403G | A | 1311 | G | C7orf62 | L89P | A | 452 | G | CASR | L663F | C | 2425 | T | CD180 | M77V | T | 387 | C |
| C1orf65 | R307W | C | 1022 | T | C7orf62 | P205S | G | 799 | A | CASR | A860V | C | 3017 | T | CD19 | R163H | G | 532 | A |
| C1orf65 | R345Q | G | 1137 | A | C7orf63 | K125N | G | 626 | T | CASR | G447D | G | 1778 | A | CD1A | A102T | G | 837 | A |
| C1orf66 | R194C | C | 1210 | T | C7orf63 | R473* | C | 1668 | T | CASS4 | R469Q | G | 1631 | A | CD1A | Y261H | T | 1314 | C |
| C1orf68 | G230* | G | 739 | T | C7orf63 | V775I | G | 2574 | A | CASS4 | P167S | C | 724 | T | CD1A | G246D | G | 1270 | A |
| C1orf68 | K80Q | A | 289 | C | C7orf63 | K125N | G | 626 | T | CAST | T259M | C | 939 | T | CD1B | L316W | A | 1055 | C |
| C1orf69 | P209S | C | 627 | T | C7orf64 | E283G | A | 889 | G | CAST | K527N | G | 1744 | T | CD1B | Y230C | T | 797 | C |
| C1orf70 | E149K | C | 456 | T | C7orf64 | R245W | C | 774 | T | CASZ1 | E1695D | C | 5403 | G | CD1C | L221P | T | 954 | C |
| C1orf74 | R122H | C | 622 | T | C7orf64 | Q98H | A | 335 | C | CASZ1 | A829V | G | 2804 | A | CD1C | I299T | T | 1188 | C |
| C1orf74 | T144I | G | 688 | A | C7orf64 | R105S | A | 356 | C | CASZ1 | G1465D | C | 4712 | T | CD1C | E80* | G | 530 | T |
| C1orf77 | G179D | G | 848 | A | C7orf65 | P16L | C | 82 | T | CASZ1 | A1604T | C | 5128 | T | CD1C | A159T | G | 767 | A |
| C1orf83 | R203W | C | 806 | T | C7orf66 | F109L | A | 382 | C | CASZ1 | R66H | C | 515 | T | CD1E | K330I | A | 1228 | T |
| C1orf83 | K47N | G | 340 | T | C7orf69 | K122T | A | 410 | C | CASZ1 | A1040V | G | 3437 | A | CD1E | G218D | G | 892 | A |
| C1orf84 | V30G | T | 176 | G | C7orf70 | K228N | C | 1152 | A | CASZ1 | R244H | C | 1049 | T | CD1E | P21H | C | 301 | A |
| C1orf84 | R31C | C | 178 | T | C7orf70 | R157H | C | 938 | T | CASZ1 | S530N | C | 1907 | T | CD1E | P346H | C | 1276 | A |
| C1orf85 | P299H | G | 923 | T | C7orf71 | G99S | G | 1011 | A | CASZ1 | A456T | C | 1684 | T | CD1E | S36P | T | 345 | C |
| C1orf85 | T87I | G | 287 | A | C7orf72 | V406I | G | 1266 | A | CASZ1 | V1110M | C | 3646 | T | CD1E | K358N | G | 1313 | T |
| C1orf87 | R220C | G | 766 | A | C8A | R484H | G | 1547 | A | CASZ1 | R487H | C | 1778 | T | CD2 | T235A | A | 811 | G |
| C1orf87 | G245V | C | 842 | A | C8A | R111C | C | 427 | T | CASZ1 | K1401Q | T | 4519 | G | CD2 | E76D | G | 336 | T |
| C1orf87 | H423R | T | 1376 | C | C8A | R484C | C | 1546 | T | CASZ1 | K646N | C | 2256 | A | CD2 | K85T | A | 362 | C |
| C1orf88 | P90H | C | 634 | A | C8A | C121Y | G | 458 | A | CAT | - | A | 0 | G | CD200 | F176L | C | 672 | A |
| C1orf9 | K260N | A | 904 | C | C8A | R559I | G | 1772 | T | CAT | L39R | T | 190 | G | CD200R1L | G24E | C | 297 | T |
| C1orf9 | D987N | G | 3083 | A | C8B | G577D | C | 1797 | T | CAT | A97T | G | 363 | A | CD200R1L | L180I | G | 764 | T |
| C1orf9 | - | G | 0 | T | C8orf33 | H58R | A | 227 | G | CATSPER 1 | R332W | G | 1132 | A | CD209 | - | C | 0 | A |
| C1orf9 | K1097Q | A | 3413 | C | C8orf33 | K72N | G | 270 | T | CATSPER 1 | T676M | G | 2165 | A | CD209 | L48I | G | 209 | T |
| C1orf92 | G375D | G | 1278 | A | C8orf33 | F241V | T | 775 | G | CATSPER 1 | A466V | G | 1535 | A | CD209 | Q98H | C | 361 | A |
| C1orf93 | E102A | A | 393 | C | C8orf38 | T57A | A | 184 | G | CATSPER 2 | E435K | C | 1317 | T | CD209 | Y31H | A | 158 | G |

| | | | | | | | | | | | | | | | | | | | |
|---|---|---|---|---|---|---|---|---|---|---|---|---|---|---|---|---|---|---|---|
| C1orf94 | A187T | G | 1098 | A | C8orf41 | R355H | C | 1589 | T | CATSPER 2 | K60N | C | 194 | A | CD22 | P419H | C | 1333 | A |
| C1orf95 | W113C | G | 444 | T | C8orf42 | G20V | C | 633 | A | CATSPER 3 | D350N | G | 1134 | A | CD244 | F121V | A | 444 | C |
| C1QA | A15T | G | 43 | A | C8orf42 | P131S | G | 965 | A | CATSPER 3 | Y129H | T | 471 | C | CD244 | E311K | C | 1014 | T |
| C1QA | N137S | A | 410 | G | C8orf45 | R570C | C | 1879 | T | CATSPER 4 | A110T | G | 328 | A | CD247 | R92Q | C | 348 | T |
| C1QB | P53H | C | 498 | A | C8orf47 | R313Q | G | 1086 | A | CATSPER 4 | A196V | C | 587 | T | CD247 | P163L | G | 561 | A |
| C1QBP | S150L | G | 527 | A | C8orf47 | E366* | G | 1244 | T | CATSPER 4 | R90* | C | 268 | T | CD248 | A303T | C | 924 | T |
| C1QC | K86E | A | 354 | G | C8orf48 | D116Y | G | 495 | T | CATSPER B | L1004I | G | 3150 | T | CD248 | G220C | C | 675 | A |
| C1QC | G103D | G | 406 | A | C8orf59 | P79H | G | 348 | T | CATSPER B | G521E | C | 1702 | T | CD248 | L561I | G | 1698 | T |
| C1QL2 | N186T | T | 1177 | G | C8orf74 | R99W | C | 324 | T | CATSPER B | S1106G | T | 3456 | C | CD27 | R78W | C | 443 | T |
| C1QL2 | A245T | C | 1353 | T | C8orf76 | F268C | A | 834 | C | CATSPER B | I572M | T | 1856 | C | CD274 | I38M | T | 200 | G |
| C1QL2 | V181A | A | 1162 | G | C8orf80 | M42T | A | 268 | G | CAV1 | A120T | G | 636 | A | CD276 | D45N | G | 435 | A |
| C1QL3 | L55M | G | 1103 | T | C8orf80 | L532P | A | 1738 | G | CBARA1 | G71A | C | 307 | G | CD28 | T209A | A | 625 | G |
| C1QL4 | S16P | A | 752 | G | C8orf82 | A149T | C | 603 | T | CBARA1 | - | C | 0 | G | CD28 | K55R | A | 164 | G |
| C1QTNF2 | Q324H | C | 976 | A | C8orf82 | G99D | C | 454 | T | CBFA2T2 | A262V | C | 824 | T | CD300A | P284H | C | 1139 | A |
| C1QTNF2 | G91C | C | 275 | A | C8orf85 | P54L | C | 180 | T | CBFA2T2 | P318S | C | 991 | T | CD300A | D112N | G | 622 | A |
| C1QTNF2 | R73W | G | 221 | A | C8orf85 | R73H | G | 237 | A | CBFA2T2 | E11* | G | 443 | T | CD300E | S206A | A | 616 | C |
| C1QTNF2 | E291K | C | 875 | T | C8orf85 | A83E | C | 267 | A | CBFA2T2 | T297A | A | 928 | G | CD300LF | T140M | G | 522 | A |
| C1QTNF2 | K143N | T | 433 | G | C8orf86 | S17I | C | 75 | A | CBFA2T3 | A572V | G | 1873 | A | CD300LG | G21C | G | 102 | T |
| C1QTNF3 | G51S | C | 858 | T | C8orf86 | M74V | T | 245 | C | CBL | A602T | G | 1942 | A | CD34 | I253M | A | 1081 | C |
| C1QTNF4 | A103V | G | 575 | A | C9 | T155K | G | 560 | T | CBLB | A215T | C | 965 | T | CD36 | R96C | C | 970 | T |
| C1QTNF5 | D79Y | C | 467 | A | C9 | T24M | G | 167 | A | CBLB | E135D | T | 727 | G | CD37 | S27N | G | 201 | A |
| C1QTNF6 | A221T | C | 734 | T | C9 | K435Q | T | 1399 | G | CBLC | G141R | G | 484 | A | CD3EAP | V405M | G | 1213 | A |
| C1QTNF6 | Y206S | T | 690 | G | C9 | E334* | C | 1096 | A | CBLC | S381L | C | 1205 | T | CD4 | G180D | G | 784 | A |
| C1QTNF8 | R60Q | C | 453 | T | C9orf100 | S303T | A | 1025 | T | CBLN2 | E107K | C | 1093 | T | CD40LG | R11Q | G | 88 | A |
| C1QTNF8 | S130P | A | 662 | G | C9orf100 | K334N | T | 1120 | G | CBLN3 | W31* | C | 564 | T | CD47 | F291L | A | 977 | G |
| C1R | E42D | C | 126 | A | C9orf102 | R433W | A | 1602 | T | CBLN3 | P6S | G | 487 | A | CD47 | N142T | T | 531 | G |
| C1R | E319K | C | 955 | T | C9orf102 | D521Y | G | 1561 | T | CBLN4 | A151V | G | 1753 | A | CD48 | G38C | C | 200 | A |
| C1R | P471S | G | 1411 | A | C9orf11 | E42* | C | 208 | A | CBR1 | V144I | G | 605 | A | CD48 | T233M | G | 786 | A |
| C1RL | T426M | G | 1294 | A | C9orf116 | P12L | G | 56 | A | CBR1 | H184Y | C | 725 | T | CD5 | G46S | G | 239 | A |
| C1RL | L29I | G | 102 | T | C9orf117 | A365V | C | 1134 | T | CBR4 | F180S | A | 708 | G | CD5 | P2S | C | 107 | T |
| C1S | L377F | G | 1759 | C | C9orf125 | V118I | C | 674 | T | CBS | H507R | T | 1689 | C | CD5 | D450N | G | 1451 | A |
| C20orf103 | A25T | G | 565 | A | C9orf125 | H48R | T | 465 | C | CBS | G432E | C | 1464 | T | CD53 | R165* | C | 605 | T |

| | | | | | | | | | | | | | | | | | | | |
|---|---|---|---|---|---|---|---|---|---|---|---|---|---|---|---|---|---|---|---|
| C20orf107 | T97N | C | 386 | A | C9orf125 | R229W | G | 1007 | A | CBX3 | E65D | A | 623 | C | CD53 | D120N | G | 470 | A |
| C20orf107 | E118K | G | 448 | A | C9orf129 | G167D | C | 864 | T | CBX4 | G232R | C | 872 | T | CD55 | N148T | A | 522 | C |
| C20orf108 | S148N | G | 534 | A | C9orf129 | R190C | G | 932 | A | CBX4 | M274I | C | 1000 | T | CD55 | E424* | G | 1564 | T |
| C20orf108 | T149A | A | 536 | G | C9orf131 | S1013L | C | 3065 | T | CBX6 | R199C | G | 719 | A | CD58 | R152* | G | 521 | A |
| C20orf111 | R251Q | C | 889 | T | C9orf131 | T1050I | C | 3176 | T | CBX6 | R162H | C | 609 | T | CD58 | P220R | G | 726 | C |
| C20orf112 | R195H | C | 727 | T | C9orf139 | P15Q | C | 1558 | A | CBX6 | R199H | C | 720 | T | CD59 | G11R | C | 141 | T |
| C20orf114 | R373H | G | 1279 | A | C9orf139 | S61G | A | 1695 | G | CBX7 | R245* | G | 939 | A | CD5L | R150H | C | 546 | T |
| C20orf114 | R229H | G | 847 | A | C9orf139 | D99G | A | 1810 | G | CBX8 | R322Q | C | 1083 | T | CD5L | R32H | C | 192 | T |
| C20orf117 | T973M | G | 3260 | A | C9orf140 | P317L | G | 1078 | A | CBY1 | F3L | C | 287 | A | CD5L | E109* | C | 422 | A |
| C20orf117 | R514Q | C | 1883 | T | C9orf150 | D111N | G | 1026 | A | CBY3 | A17T | C | 105 | T | CD6 | H367Y | C | 1322 | T |
| C20orf117 | S223L | G | 1010 | A | C9orf156 | E133D | T | 476 | G | CC2D1A | P793T | C | 2676 | A | CD68 | P147L | C | 514 | T |
| C20orf12 | V644I | C | 2139 | T | C9orf167 | A145V | C | 630 | T | CC2D1B | R543Q | C | 1767 | T | CD7 | K206N | C | 727 | A |
| C20orf12 | R148H | C | 652 | T | C9orf167 | A409V | C | 1422 | T | CC2D1B | R534Q | C | 1740 | T | CD70 | R11W | G | 181 | A |
| C20orf12 | R125S | C | 584 | A | C9orf167 | A145V | C | 630 | T | CC2D1B | E92V | T | 414 | A | CD70 | R138C | G | 562 | A |
| C20orf123 | R253Q | C | 772 | T | C9orf169 | P89S | C | 752 | T | CC2D1B | L492M | G | 1613 | T | CD72 | E273* | C | 982 | A |
| C20orf123 | R426W | G | 1290 | A | C9orf170 | R41Q | G | 209 | A | CC2D1B | R299L | C | 1035 | A | CD79A | P83L | C | 433 | T |
| C20orf123 | L201F | G | 615 | A | C9orf171 | G282D | G | 893 | A | CC2D2A | F404S | T | 1450 | C | CD80 | E181* | C | 904 | A |
| C20orf123 | R383H | C | 1162 | T | C9orf172 | A668V | C | 2003 | T | CC2D2A | T1032A | A | 3333 | G | CD80 | R107H | C | 683 | T |
| C20orf132 | E119* | C | 354 | A | C9orf172 | R720H | G | 2159 | A | CC2D2A | A375V | C | 1363 | T | CD80 | E44* | C | 493 | A |
| C20orf132 | Q57E | G | 168 | C | C9orf173 | L217P | T | 676 | C | CC2D2A | R1049* | C | 3384 | T | CD82 | A77T | G | 473 | A |
| C20orf132 | L568P | A | 1702 | G | C9orf24 | R6H | C | 216 | T | CC2D2A | R1224W | C | 3909 | T | CD82 | L13I | C | 281 | A |
| C20orf132 | E944* | C | 2829 | A | C9orf25 | R149W | G | 752 | A | CC2D2A | L1616R | T | 5086 | G | CD83 | A84T | G | 421 | A |
| C20orf132 | S915A | A | 2742 | C | C9orf3 | K693Q | A | 2077 | C | CCAR1 | R300Q | G | 1018 | A | CD86 | V199I | G | 711 | A |
| C20orf132 | Q57K | G | 168 | T | C9orf4 | R238I | C | 713 | A | CCAR1 | R552H | G | 1774 | A | CD8A | E203D | C | 665 | A |
| C20orf135 | G208C | G | 665 | T | C9orf41 | V388M | C | 1315 | T | CCAR1 | S456G | A | 1485 | G | CD93 | I527T | A | 1728 | G |
| C20orf135 | R161H | G | 525 | A | C9orf43 | K258N | G | 1172 | T | CCAR1 | L388V | T | 1281 | G | CD93 | D337Y | C | 1157 | A |
| C20orf135 | H47R | A | 183 | G | C9orf5 | E401D | C | 1235 | A | CCBE1 | D293G | T | 878 | C | CD97 | F760S | T | 2402 | C |
| C20orf141 | R34C | C | 183 | T | C9orf50 | G412R | C | 1436 | G | CCBE1 | R162Q | C | 485 | T | CD99L2 | A201S | C | 719 | A |
| C20orf141 | E13D | G | 122 | T | C9orf57 | S157Y | G | 566 | T | CCBE1 | S328Y | G | 983 | T | CDA | K26N | G | 260 | T |
| C20orf141 | R236* | C | 789 | T | C9orf7 | T52I | C | 235 | T | CCBL1 | V31M | C | 254 | T | CDADC1 | R510C | C | 1641 | T |
| C20orf151 | R86L | C | 414 | A | C9orf7 | A59T | G | 255 | A | CCBL1 | E117K | C | 512 | T | CDAN1 | E526D | C | 1695 | A |
| C20orf151 | R179W | G | 692 | A | C9orf72 | S108N | C | 387 | T | CCBL2 | R355H | C | 1402 | T | CDAN1 | S311T | A | 1048 | T |
| C20orf151 | Q503R | T | 1665 | C | C9orf72 | F367C | A | 1164 | C | CCBL2 | S341Y | G | 1360 | T | CDC14A | G100D | G | 763 | A |
| C20orf152 | R498P | G | 1666 | C | C9orf78 | L31P | A | 146 | G | CCBL2 | K82Q | T | 582 | G | CDC14A | N140S | A | 883 | G |
| C20orf160 | R26C | C | 89 | T | C9orf79 | V165M | G | 559 | A | CCBP2 | L303I | C | 1005 | A | CDC14C | R55C | G | 615 | A |
| C20orf160 | R131* | C | 404 | T | C9orf79 | V86I | G | 322 | A | CCDC101 | A72V | C | 402 | T | CDC16 | Q87H | G | 459 | T |
| C20orf160 | S41L | C | 135 | T | C9orf82 | S256R | T | 855 | G | CCDC101 | A66T | G | 383 | A | CDC16 | R510* | C | 1726 | T |
| C20orf166 | L62I | C | 963 | A | C9orf84 | F159L | A | 736 | G | CCDC102B | E326* | G | 1210 | T | CDC16 | P399L | C | 1394 | T |
| C20orf173 | P41Q | G | 267 | T | C9orf84 | F1308C | A | 4184 | C | CCDC104 | D166N | G | 694 | A | CDC20 | R380Q | G | 1240 | A |

| | | | | | | | | | | | | | | | | | | | |
|---|---|---|---|---|---|---|---|---|---|---|---|---|---|---|---|---|---|---|---|
| C20orf173 | - | C | 0 | A | C9orf89 | R37C | C | 237 | T | CCDC104 | E96* | G | 484 | T | CDC20 | D464Y | G | 1491 | T |
| C20orf177 | T277A | A | 1244 | G | C9orf9 | G136S | G | 853 | A | CCDC105 | G17V | G | 149 | T | CDC20 | T443A | A | 1428 | G |
| C20orf177 | T136A | A | 821 | G | C9orf91 | A142V | C | 862 | T | CCDC105 | R399C | C | 1294 | T | CDC20B | P365R | G | 1240 | C |
| C20orf177 | P172L | C | 930 | T | C9orf91 | G13D | G | 475 | A | CCDC105 | - | G | 0 | T | CDC20B | A403T | C | 1353 | T |
| C20orf186 | C295Y | G | 884 | A | C9orf91 | P308T | C | 1359 | A | CCDC105 | R15H | G | 143 | A | CDC20B | E189K | C | 711 | T |
| C20orf186 | A579T | G | 1735 | A | C9orf93 | R81Q | G | 570 | A | CCDC106 | A148T | G | 1177 | A | CDC20B | E123* | C | 513 | A |
| C20orf186 | L81R | T | 242 | G | C9orf93 | E272* | G | 1142 | T | CCDC106 | R207H | G | 1355 | A | CDC20B | Q34P | T | 247 | G |
| C20orf194 | R7C | G | 87 | A | C9orf93 | - | G | 0 | T | CCDC106 | D256N | G | 1501 | A | CDC23 | Q488K | G | 1493 | T |
| C20orf194 | R822G | T | 2532 | C | C9orf93 | R345* | C | 1361 | T | CCDC108 | V313I | C | 1021 | T | CDC23 | A538V | G | 1644 | A |
| C20orf194 | D652Y | C | 2022 | A | C9orf93 | L458S | T | 1701 | C | CCDC108 | A148T | C | 526 | T | CDC23 | S345Y | G | 1065 | T |
| C20orf194 | R156* | G | 534 | A | C9orf93 | K593T | A | 2106 | C | CCDC108 | R580H | C | 1823 | T | CDC25A | N70H | T | 617 | G |
| C20orf20 | V106I | G | 387 | A | C9orf93 | Q663H | A | 2317 | C | CCDC108 | K1346N | T | 4122 | G | CDC25B | G187R | G | 633 | A |
| C20orf200 | P70H | G | 1007 | T | C9orf98 | R159K | C | 998 | T | CCDC108 | D504N | C | 1594 | T | CDC25B | K473N | G | 1493 | T |
| C20orf201 | R106H | C | 457 | T | C9orf98 | P142H | G | 947 | T | CCDC108 | Y180C | T | 623 | C | CDC25B | Q116R | A | 421 | G |
| C20orf202 | G96C | G | 349 | T | C9orf98 | F96C | A | 809 | C | CCDC108 | R35K | C | 188 | T | CDC25C | R448Q | C | 1622 | T |
| C20orf24 | R111C | C | 461 | T | CA1 | E107D | T | 436 | G | CCDC109A | A277T | G | 850 | A | CDC26 | R23Q | C | 427 | T |
| C20orf26 | R583H | G | 1824 | A | CA1 | V257L | C | 884 | G | CCDC109A | E218D | A | 675 | C | CDC27 | Y641N | A | 2048 | T |
| C20orf27 | T172M | G | 667 | A | CA10 | L11I | G | 708 | T | CCDC109B | S292* | C | 1008 | A | CDC27 | L698I | G | 2219 | T |
| C20orf29 | R95H | G | 467 | A | CA10 | R274H | C | 821 | T | CCDC11 | L107F | T | 412 | A | CDC37 | N178S | T | 649 | C |
| C20orf3 | M58I | C | 465 | A | CA12 | V202I | C | 1045 | T | CCDC110 | E541D | T | 1684 | G | CDC42 | R110* | C | 544 | T |
| C20orf4 | R276W | C | 919 | T | CA13 | R228C | C | 1024 | T | CCDC110 | K195N | C | 646 | A | CDC42BPA | G1722R | C | 6107 | T |
| C20orf43 | A62T | G | 227 | A | CA14 | L79P | T | 591 | C | CCDC110 | E122* | C | 425 | A | CDC42BPA | R450C | G | 2291 | A |
| C20orf43 | G204D | G | 654 | A | CA14 | F230L | T | 1043 | C | CCDC111 | Y326* | T | 1411 | G | CDC42BPG | K704N | C | 2112 | A |
| C20orf46 | R161Q | C | 1154 | T | CA14 | E25A | A | 429 | C | CCDC112 | R51C | G | 439 | A | CDC42BPG | G207R | C | 619 | T |
| C20orf54 | R358M | C | 1315 | A | CA14 | D49N | G | 500 | A | CCDC113 | Q245K | C | 789 | A | CDC42BPG | V1301M | C | 3901 | T |
| C20orf57 | G265W | C | 870 | A | CA14 | S237* | C | 1065 | A | CCDC114 | R554C | G | 2343 | A | CDC42BPG | A231T | C | 691 | T |
| C20orf7 | A282T | G | 963 | A | CA3 | S29L | C | 169 | T | CCDC116 | R586H | G | 1918 | A | CDC42BPG | E521* | C | 1561 | A |
| C20orf71 | F148S | T | 653 | C | CA3 | F146C | T | 520 | G | CCDC117 | M277I | G | 1007 | A | CDC42BPG | R1539Q | C | 4616 | T |
| C20orf71 | L189I | C | 775 | A | CA5A | A208V | G | 689 | A | CCDC117 | M277I | G | 1007 | A | CDC42EP1 | R188C | C | 982 | T |
| C20orf71 | L200I | C | 808 | A | CA5A | R26C | G | 142 | A | CCDC120 | G407E | G | 1220 | A | CDC42EP1 | D191G | A | 992 | G |
| C20orf85 | A2V | C | 66 | T | CA5A | A231T | C | 757 | T | CCDC121 | P51L | G | 267 | A | CDC42EP1 | L15M | C | 463 | A |
| C20orf94 | A2T | G | 184 | A | CA6 | - | G | 0 | A | CCDC123 | G681S | C | 2130 | T | CDC42EP2 | V185M | G | 1003 | A |
| C20orf96 | K183N | C | 688 | A | CA7 | R215Q | G | 753 | A | CCDC123 | R9H | C | 115 | T | CDC42EP3 | E212K | C | 1635 | T |
| C21orf2 | G268V | C | 1002 | A | CA8 | M1T | A | 250 | G | CCDC123 | - | A | 0 | C | CDC42EP3 | P240S | G | 1719 | A |
| C21orf29 | R514* | G | 1606 | A | CA9 | H140N | C | 522 | A | CCDC125 | R459C | G | 1483 | A | CDC42EP5 | M99V | T | 637 | C |
| C21orf29 | E628K | C | 1948 | T | CAB39 | T288M | C | 1292 | T | CCDC125 | D247Y | C | 847 | A | CDC42SE2 | N51T | A | 595 | C |
| C21orf29 | W644C | C | 1998 | A | CAB39 | S229P | T | 1114 | C | CCDC127 | S181N | C | 675 | T | CDC45 | A179G | C | 612 | G |
| C21orf29 | E256K | C | 832 | T | CAB39L | K296Q | T | 1384 | G | CCDC129 | D607Y | G | 1857 | T | CDC45 | R309C | C | 1001 | T |
| C21orf63 | K58R | A | 646 | G | CABC1 | D396N | G | 3957 | A | CCDC129 | D209E | C | 665 | A | CDC45 | F10L | C | 106 | A |

| | | | | | | | | | | | | | | | | | | | |
|---|---|---|---|---|---|---|---|---|---|---|---|---|---|---|---|---|---|---|---|
| C21orf67 | P33A | G | 343 | C | CABIN1 | E1056G | A | 3294 | G | CCDC129 | R190Q | G | 607 | A | CDC6 | G483W | G | 1918 | T |
| C21orf67 | P167S | G | 745 | A | CABIN1 | R1754W | C | 5387 | T | CCDC129 | E364D | G | 1130 | T | CDC73 | R263H | G | 972 | A |
| C21orf7 | F207V | T | 748 | G | CABIN1 | P1595L | C | 4911 | T | CCDC13 | R425Q | C | 1358 | T | CDC73 | R513Q | G | 1722 | A |
| C21orf70 | E153K | G | 505 | A | CABIN1 | Q1869* | C | 5732 | T | CCDC13 | K711N | C | 2217 | A | CDC73 | Y54H | T | 344 | C |
| C21orf88 | S95A | A | 359 | C | CABLES1 | L403M | C | 1207 | A | CCDC13 | D650N | C | 2032 | T | CDC73 | A102V | C | 489 | T |
| C21orf91 | R265H | C | 885 | T | CABP4 | P246H | C | 814 | A | CCDC130 | V110M | G | 578 | A | CDC73 | C145R | T | 617 | C |
| C22orf23 | Q70H | C | 419 | A | CABP5 | R173C | G | 642 | A | CCDC132 | Y648N | T | 2070 | A | CDC73 | E302* | G | 1088 | T |
| C22orf30 | D1330N | C | 4178 | T | CABP5 | G82V | C | 370 | A | CCDC132 | R493H | G | 1606 | A | CDC73 | A106S | G | 500 | T |
| C22orf30 | L1261R | A | 3972 | C | CABP7 | R135W | C | 744 | T | CCDC132 | K243N | A | 857 | C | CDCA2 | R611* | C | 1831 | T |
| C22orf30 | R427C | G | 1469 | A | CABYR | E100D | A | 452 | C | CCDC132 | K246N | G | 866 | T | CDCA2 | S253P | T | 757 | C |
| C22orf30 | L24V | A | 260 | C | CACHD1 | T115M | C | 344 | T | CCDC134 | R124G | A | 474 | G | CDCA2 | S1000P | T | 2998 | C |
| C22orf31 | S102L | G | 357 | A | CACHD1 | R1184C | C | 3550 | T | CCDC134 | R217Q | G | 754 | A | CDCA2 | N379H | A | 1135 | C |
| C22orf33 | G122S | C | 577 | T | CACNA1A | - | C | 0 | T | CCDC135 | R615H | G | 1933 | A | CDCA5 | P198H | G | 662 | T |
| C22orf33 | L26F | C | 291 | A | CACNA1A | G28R | C | 318 | T | CCDC135 | D332N | G | 1083 | A | CDCA7L | R387C | G | 1439 | A |
| C22orf33 | K263N | T | 1002 | G | CACNA1A | A110V | G | 565 | A | CCDC135 | V608M | G | 1911 | A | CDCP2 | R176W | G | 1374 | A |
| C22orf36 | R115* | G | 367 | A | CACNA1A | A110V | G | 565 | A | CCDC136 | E638* | G | 2279 | T | CDCP2 | P18T | G | 900 | T |
| C22orf42 | S175Y | G | 597 | T | CACNA1A | E894D | C | 2918 | A | CCDC136 | D1102V | A | 3672 | T | CDH1 | L214R | T | 832 | G |
| C22orf43 | - | C | 0 | T | CACNA1A | Y1244* | G | 3968 | T | CCDC136 | E838D | G | 2881 | T | CDH1 | S191N | G | 763 | A |
| C22orf43 | E198D | T | 892 | A | CACNA1A | R201W | G | 837 | A | CCDC136 | R497W | C | 1856 | T | CDH1 | R732Q | G | 2386 | A |
| C22orf43 | R75H | C | 522 | T | CACNA1A | L105V | A | 549 | C | CCDC136 | N237H | A | 1076 | C | CDH10 | R54C | G | 668 | A |
| C2CD2 | R105Q | C | 556 | T | CACNA1A | A875V | G | 2860 | A | CCDC136 | K529R | A | 1953 | G | CDH10 | A490D | G | 1977 | T |
| C2CD2 | S209F | G | 868 | A | CACNA1B | A57T | G | 169 | A | CCDC137 | R170W | C | 544 | T | CDH10 | G439* | C | 1823 | A |
| C2CD2 | A529T | C | 1827 | T | CACNA1B | A407V | C | 1220 | T | CCDC137 | V13L | G | 73 | T | CDH10 | T701A | T | 2609 | C |
| C2CD2 | L627I | G | 2121 | T | CACNA1B | R2231S | C | 6691 | A | CCDC14 | S267G | T | 890 | C | CDH10 | S747F | G | 2748 | A |
| C2CD2L | I123L | A | 726 | C | CACNA1B | F346S | T | 1037 | C | CCDC141 | E8* | C | 79 | A | CDH10 | - | C | 0 | T |
| C2CD2L | R532H | G | 1954 | A | CACNA1B | R1907Q | G | 5720 | A | CCDC141 | R226C | G | 733 | A | CDH10 | L64I | G | 698 | T |
| C2CD3 | S307Y | G | 1147 | T | CACNA1B | V1376M | G | 4126 | A | CCDC141 | P648H | G | 2000 | T | CDH11 | T115M | G | 960 | A |
| C2CD3 | V1659L | C | 5202 | G | CACNA1B | T1802M | C | 5405 | T | CCDC142 | R597Q | C | 2187 | T | CDH11 | R693H | C | 2694 | T |
| C2CD3 | - | C | 0 | T | CACNA1B | R579W | C | 1735 | T | CCDC144A | V521A | T | 1638 | C | CDH11 | V374G | A | 1737 | C |
| C2CD3 | R62Q | C | 412 | T | CACNA1B | A653T | G | 1957 | A | CCDC144B | A68T | G | 345 | A | CDH11 | W453* | C | 1975 | T |
| C2CD3 | K1455N | C | 4592 | A | CACNA1B | E675K | G | 2023 | A | CCDC144B | - | T | 0 | C | CDH11 | P580L | G | 2355 | A |
| C2CD3 | V1235A | A | 3931 | G | CACNA1B | E1555* | G | 4663 | T | CCDC144B | N705K | C | 2258 | A | CDH11 | P647Q | G | 2556 | T |
| C2CD4A | N66I | A | 338 | T | CACNA1B | E1555D | G | 4665 | T | CCDC146 | R637Q | G | 2037 | A | CDH11 | W57L | C | 786 | A |
| C2CD4C | V24M | C | 275 | T | CACNA1C | Y1264C | A | 3791 | G | CCDC146 | A479V | C | 1563 | T | CDH11 | D520Y | C | 2174 | A |
| C2CD4C | T73M | G | 423 | A | CACNA1C | A1287V | C | 3860 | T | CCDC147 | V106A | T | 451 | C | CDH11 | Q213R | T | 1254 | C |
| C2orf16 | Q216K | C | 697 | A | CACNA1C | S1330G | A | 3988 | G | CCDC147 | K272N | G | 950 | T | CDH12 | V464I | C | 2099 | T |
| C2orf16 | T1458K | C | 4424 | A | CACNA1C | G2116R | G | 6346 | A | CCDC147 | K516Q | A | 1680 | C | CDH12 | P35S | G | 812 | A |
| C2orf16 | V343I | G | 1078 | A | CACNA1C | T598M | C | 1793 | T | CCDC147 | R587Q | G | 1894 | A | CDH12 | G87C | C | 968 | A |
| C2orf16 | D1481V | A | 4493 | T | CACNA1C | A2059V | C | 6176 | T | CCDC147 | - | A | 0 | C | CDH12 | Q151K | G | 1160 | T |

| | | | | | | | | | | | | | | | | | | | |
|---|---|---|---|---|---|---|---|---|---|---|---|---|---|---|---|---|---|---|---|
| C2orf16 | S62Y | C | 236 | A | CACNA1C | T1801A | A | 5401 | G | CCDC148 | E567K | C | 1850 | T | CDH12 | Q60E | G | 887 | C |
| C2orf16 | Q72H | A | 267 | C | CACNA1C | R2122Q | G | 6365 | A | CCDC148 | Q267* | G | 950 | A | CDH13 | V96I | G | 286 | A |
| C2orf16 | F206C | T | 668 | G | CACNA1D | R864C | C | 2708 | T | CCDC148 | L446R | A | 1488 | C | CDH15 | P783L | C | 2413 | T |
| C2orf16 | S460Y | C | 1430 | A | CACNA1D | A671T | G | 2129 | A | CCDC15 | R779* | C | 2594 | T | CDH15 | R112H | G | 400 | A |
| C2orf16 | D819N | G | 2506 | A | CACNA1D | R2132H | G | 6513 | A | CCDC15 | Q333H | G | 1258 | T | CDH15 | R35H | G | 169 | A |
| C2orf16 | K887Q | A | 2710 | C | CACNA1D | R1545G | A | 4751 | G | CCDC15 | V688M | G | 2321 | A | CDH15 | G757S | G | 2334 | A |
| C2orf16 | L1322V | T | 4015 | G | CACNA1D | A1278T | G | 3950 | A | CCDC15 | E68* | G | 461 | T | CDH16 | V56M | C | 333 | T |
| C2orf16 | R1695H | G | 5135 | A | CACNA1D | P43S | C | 245 | T | CCDC150 | K148N | G | 579 | T | CDH16 | R776C | G | 2493 | A |
| C2orf18 | V201I | G | 663 | A | CACNA1D | A1278T | G | 3950 | A | CCDC150 | A907V | C | 2855 | T | CDH16 | A536S | C | 1773 | A |
| C2orf29 | S251Y | C | 915 | A | CACNA1D | A1481V | C | 4560 | T | CCDC151 | A365T | C | 1236 | T | CDH16 | S236N | C | 874 | T |
| C2orf29 | N412H | A | 1397 | C | CACNA1D | K1280Q | A | 3956 | C | CCDC151 | D332V | T | 1138 | A | CDH17 | R193Q | C | 703 | T |
| C2orf3 | R188I | C | 697 | A | CACNA1D | L1551* | T | 4770 | G | CCDC151 | N494D | T | 1623 | C | CDH17 | - | C | 0 | T |
| C2orf3 | R287C | G | 993 | A | CACNA1D | N1895D | A | 5801 | G | CCDC151 | A275V | G | 967 | A | CDH17 | Y306* | A | 1043 | T |
| C2orf39 | W244* | G | 805 | A | CACNA1D | R1357W | C | 4187 | T | CCDC154 | E302D | C | 1073 | A | CDH18 | R638C | G | 2432 | A |
| C2orf39 | A683S | G | 2121 | T | CACNA1E | G2136C | G | 6829 | T | CCDC154 | T97M | G | 457 | A | CDH18 | R558Q | C | 2193 | T |
| C2orf39 | A433E | C | 1372 | A | CACNA1E | E398* | G | 1615 | T | CCDC154 | R126Q | C | 544 | T | CDH18 | E391D | T | 1693 | G |
| C2orf39 | L23I | C | 141 | A | CACNA1E | E398* | G | 1615 | T | CCDC154 | T573M | G | 1885 | A | CDH19 | N772H | T | 2607 | G |
| C2orf39 | D60N | G | 252 | A | CACNA1E | A176T | G | 949 | A | CCDC157 | R469W | C | 2114 | T | CDH19 | K29N | C | 380 | A |
| C2orf39 | Q456H | G | 1442 | T | CACNA1E | R378H | G | 1556 | A | CCDC157 | R469Q | G | 2115 | A | CDH19 | A676T | C | 2319 | T |
| C2orf40 | A17V | C | 159 | T | CACNA1E | P744L | C | 2654 | T | CCDC157 | A407V | C | 1929 | T | CDH19 | D691N | C | 2364 | T |
| C2orf43 | R42M | C | 205 | A | CACNA1E | V1471M | G | 4834 | A | CCDC157 | L84P | T | 960 | C | CDH19 | A269T | C | 1098 | T |
| C2orf43 | T255P | T | 843 | G | CACNA1E | V1475A | T | 4847 | C | CCDC157 | P220L | C | 1368 | T | CDH19 | E724* | C | 2463 | A |
| C2orf43 | F265L | A | 875 | C | CACNA1E | A2V | C | 428 | T | CCDC157 | R274H | G | 1530 | A | CDH19 | S407Y | G | 1513 | T |
| C2orf47 | A90V | C | 591 | T | CACNA1E | L1365I | C | 4516 | A | CCDC157 | A225T | G | 1382 | A | CDH19 | M1I | C | 296 | T |
| C2orf53 | V15A | A | 570 | G | CACNA1E | T1194M | C | 4004 | T | CCDC158 | R583Q | C | 1901 | T | CDH2 | N298D | T | 1316 | C |
| C2orf55 | P673L | G | 2350 | A | CACNA1E | S745L | C | 2657 | T | CCDC158 | E1098* | C | 3445 | A | CDH2 | V90M | C | 692 | T |
| C2orf55 | R236Q | C | 1039 | T | CACNA1E | A1126S | G | 3799 | T | CCDC158 | I273L | T | 970 | G | CDH2 | R823* | G | 2891 | A |
| C2orf55 | R162M | C | 817 | A | CACNA1E | S1180L | C | 3962 | T | CCDC158 | S63Y | G | 341 | T | CDH2 | N492H | T | 1898 | G |
| C2orf56 | I282T | T | 885 | C | CACNA1E | F1703V | T | 5530 | G | CCDC158 | E58* | C | 325 | A | CDH2 | R697H | C | 2514 | T |
| C2orf57 | A375V | C | 1175 | T | CACNA1E | A36V | C | 530 | T | CCDC159 | R148Q | G | 942 | A | CDH2 | F267L | G | 1225 | T |
| C2orf57 | S102G | A | 355 | G | CACNA1E | T564M | C | 2114 | T | CCDC160 | E229* | G | 1006 | T | CDH2 | V254A | A | 1185 | G |
| C2orf60 | R149* | G | 711 | A | CACNA1F | A1090V | G | 3331 | A | CCDC17 | R56W | G | 316 | A | CDH20 | A694T | G | 2092 | A |
| C2orf60 | A119V | G | 622 | A | CACNA1F | S177G | T | 591 | C | CCDC17 | G178D | C | 683 | T | CDH20 | G198S | G | 604 | A |
| C2orf63 | L498R | A | 1730 | C | CACNA1F | V1019A | A | 3118 | G | CCDC18 | R1081Q | G | 3764 | A | CDH20 | A691V | C | 2084 | T |
| C2orf65 | E320* | C | 1075 | A | CACNA1F | S39R | T | 177 | G | CCDC19 | G529D | C | 1651 | T | CDH22 | S322G | T | 1365 | C |
| C2orf65 | T144A | T | 547 | C | CACNA1G | E776* | G | 2326 | T | CCDC19 | Q323H | C | 1034 | A | CDH22 | V210M | C | 1029 | T |
| C2orf65 | - | C | 0 | A | CACNA1G | R1387H | G | 4160 | A | CCDC19 | R254I | C | 826 | A | CDH22 | R533H | C | 1999 | T |
| C2orf65 | F384V | A | 1267 | C | CACNA1G | E1896K | G | 5686 | A | CCDC21 | A59V | C | 307 | T | CDH22 | T417M | G | 1651 | A |
| C2orf65 | R378I | C | 1250 | A | CACNA1G | G229V | G | 686 | T | CCDC21 | A433V | C | 1429 | T | CDH22 | F217L | G | 1052 | T |

| | | | | | | | | | | | | | | | | | | | |
|---|---|---|---|---|---|---|---|---|---|---|---|---|---|---|---|---|---|---|---|
| C2orf67 | C418Y | C | 1517 | T | CACNA1G | W356R | T | 1066 | A | CCDC21 | V691L | G | 2202 | T | CDH23 | - | G | 0 | T |
| C2orf67 | P463L | G | 1652 | A | CACNA1G | C1441Y | G | 4322 | A | CCDC21 | K727T | A | 2311 | C | CDH23 | I664V | A | 2377 | G |
| C2orf67 | K422Q | T | 1528 | G | CACNA1G | M386V | A | 1156 | G | CCDC22 | R433H | G | 1468 | A | CDH23 | I2172T | T | 6902 | C |
| C2orf67 | K159T | T | 740 | G | CACNA1G | G1026R | G | 3076 | A | CCDC22 | R607Q | G | 1990 | A | CDH23 | R839H | G | 2903 | A |
| C2orf67 | Y114H | A | 604 | G | CACNA1G | L512I | C | 1534 | A | CCDC22 | R388H | G | 1333 | A | CDH23 | D1418G | A | 4640 | G |
| C2orf7 | P16T | G | 114 | T | CACNA1G | G1413D | G | 4238 | A | CCDC24 | L192V | T | 745 | G | CDH23 | E1314K | G | 4327 | A |
| C2orf7 | A21T | C | 129 | T | CACNA1G | F1520S | T | 4559 | C | CCDC27 | R509Q | G | 1610 | A | CDH23 | Y1626* | C | 5265 | A |
| C2orf7 | R110W | G | 396 | A | CACNA1H | P277L | C | 1078 | T | CCDC27 | P132H | C | 479 | A | CDH23 | A3058V | C | 9560 | T |
| C2orf70 | R122Q | G | 396 | A | CACNA1H | R1412W | C | 4482 | T | CCDC28A | E67V | A | 366 | T | CDH23 | I456V | A | 1753 | G |
| C2orf70 | V18L | G | 83 | T | CACNA1H | G1106D | G | 3565 | A | CCDC30 | Q381R | A | 1232 | G | CDH23 | L2602I | C | 8191 | A |
| C2orf70 | G130D | G | 420 | A | CACNA1H | V1850M | G | 5796 | A | CCDC30 | Q295H | G | 975 | T | CDH23 | E2554V | A | 8048 | T |
| C2orf71 | P1188S | G | 3562 | A | CACNA1H | L1547F | C | 4887 | T | CCDC33 | A406S | G | 1216 | T | CDH23 | L173F | C | 904 | T |
| C2orf71 | A717S | C | 2149 | A | CACNA1H | V1921M | G | 6009 | A | CCDC33 | A290T | G | 868 | A | CDH23 | F182L | C | 933 | A |
| C2orf71 | T438I | G | 1313 | A | CACNA1H | R1202Q | G | 3853 | A | CCDC33 | A641V | C | 1922 | T | CDH23 | D2267G | A | 7187 | G |
| C2orf71 | P1051L | G | 3152 | A | CACNA1I | R1467H | G | 4400 | A | CCDC36 | A481V | C | 1829 | T | CDH23 | R2683C | C | 8434 | T |
| C2orf71 | S784Y | G | 2351 | T | CACNA1I | R601W | C | 1801 | T | CCDC36 | N7T | A | 407 | C | CDH24 | R74W | G | 480 | A |
| C2orf71 | L469I | G | 1405 | T | CACNA1I | P946Q | C | 2837 | A | CCDC36 | K21N | G | 450 | T | CDH24 | I535V | T | 1863 | C |
| C2orf74 | E134* | G | 400 | T | CACNA1I | L1739M | C | 5215 | A | CCDC36 | Q165P | A | 881 | C | CDH24 | R362C | G | 1344 | A |
| C2orf77 | E419D | C | 1351 | A | CACNA1I | A453T | G | 1357 | A | CCDC36 | S170Y | C | 896 | A | CDH26 | E107D | G | 621 | T |
| C2orf78 | Q519* | C | 1676 | T | CACNA1I | K908T | A | 2723 | C | CCDC37 | R118H | G | 452 | A | CDH26 | P749L | C | 2546 | T |
| C2orf78 | L463F | C | 1508 | T | CACNA1I | D1741N | G | 5221 | A | CCDC37 | T238N | C | 812 | A | CDH26 | P493L | C | 1778 | T |
| C2orf78 | F650L | C | 2071 | A | CACNA1I | R601Q | G | 1802 | A | CCDC37 | E291K | G | 970 | A | CDH26 | A374V | C | 1421 | T |
| C2orf81 | S320A | A | 1267 | C | CACNA1S | A1028V | G | 3310 | A | CCDC37 | N299H | A | 994 | C | CDH3 | A312V | C | 1479 | T |
| C2orf82 | R8H | G | 321 | A | CACNA1S | K1549N | C | 4874 | A | CCDC38 | S423L | G | 1502 | A | CDH3 | L168M | T | 1046 | A |
| C2orf84 | L48I | T | 142 | A | CACNA1S | R252C | G | 981 | A | CCDC38 | K304N | C | 1146 | A | CDH4 | G733S | G | 2208 | A |
| C2orf84 | R169I | G | 506 | T | CACNA1S | A818V | G | 2680 | A | CCDC39 | I363K | A | 1208 | T | CDH4 | A636V | C | 1918 | T |
| C2orf85 | V118I | G | 380 | A | CACNA2D1 | R273Q | C | 1157 | T | CCDC39 | K373R | T | 1238 | C | CDH4 | V753L | G | 2268 | C |
| C2orf85 | G50W | G | 176 | T | CACNA2D1 | - | C | 0 | T | CCDC39 | R78C | G | 352 | A | CDH5 | A187T | G | 707 | A |
| C2orf86 | L592F | G | 2302 | A | CACNA2D1 | I915M | T | 3084 | C | CCDC39 | N331D | T | 1111 | C | CDH5 | R622W | C | 2012 | T |
| C2orf86 | R227Q | C | 1208 | T | CACNA2D1 | P525S | G | 1912 | A | CCDC39 | D99Y | C | 415 | A | CDH6 | R702Q | G | 2370 | A |
| C2orf86 | R227Q | C | 1208 | T | CACNA2D2 | T752A | T | 2254 | C | CCDC39 | L583I | G | 1867 | T | CDH6 | F60L | C | 445 | A |
| C2orf86 | M34T | A | 629 | G | CACNA2D3 | E96K | G | 334 | A | CCDC39 | - | A | 0 | T | CDH7 | A446T | G | 1661 | A |
| C2orf86 | T715I | G | 2672 | A | CACNA2D3 | A1070V | C | 3257 | T | CCDC40 | S598N | G | 1813 | A | CDH8 | S408T | A | 2177 | T |
| C2orf86 | R483Q | C | 1976 | T | CACNA2D3 | E544K | G | 1678 | A | CCDC40 | R979H | G | 2956 | A | CDH8 | S59P | A | 1130 | G |
| C2orf86 | F432V | A | 1822 | C | CACNA2D3 | M935V | A | 2851 | G | CCDC40 | R557W | C | 1689 | T | CDH8 | P214S | G | 1595 | A |
| C2orf88 | K5N | A | 394 | C | CACNA2D4 | M926I | C | 3009 | A | CCDC40 | E435D | G | 1325 | T | CDH8 | L291H | A | 1827 | T |
| C2orf89 | T398M | G | 1236 | A | CACNA2D4 | E1026D | C | 3309 | A | CCDC40 | T585N | C | 1774 | A | CDH8 | E798* | C | 3347 | A |
| C2orf89 | R370L | C | 1152 | A | CACNA2D4 | R628* | G | 2113 | A | CCDC41 | R630* | G | 2421 | A | CDH8 | L81F | G | 1196 | A |
| C2orf89 | R441H | C | 1365 | T | CACNA2D4 | S602Y | G | 2036 | T | CCDC41 | K571Q | T | 2244 | G | CDH8 | L291H | A | 1827 | T |

| | | | | | | | | | | | | | | | | | | | |
|---|---|---|---|---|---|---|---|---|---|---|---|---|---|---|---|---|---|---|---|
| C3 | K1381N | C | 4236 | A | CACNB1 | E307K | C | 1073 | T | CCDC41 | R511Q | C | 2065 | T | CDH9 | - | C | 0 | A |
| C3 | T1333I | G | 4091 | A | CACNB1 | F385L | A | 1307 | G | CCDC42 | K49R | T | 373 | C | CDH9 | D240Y | C | 891 | A |
| C3 | R1512H | C | 4628 | T | CACNB2 | V177A | T | 590 | C | CCDC43 | I63N | A | 232 | T | CDH9 | S143L | G | 601 | A |
| C3 | R881C | G | 2734 | A | CACNB2 | K637R | A | 1970 | G | CCDC45 | S243Y | C | 830 | A | CDH9 | R678S | C | 2207 | A |
| C3 | V602M | C | 1897 | T | CACNB2 | E443D | G | 1389 | T | CCDC46 | Q819R | T | 2675 | C | CDH9 | Y732N | A | 2367 | T |
| C3 | C728* | G | 2277 | T | CACNB3 | - | G | 0 | A | CCDC47 | A428T | C | 1618 | T | CDH9 | Q762H | T | 2459 | G |
| C3 | A1089P | C | 3358 | G | CACNB3 | R397W | C | 1390 | T | CCDC48 | G11S | G | 31 | A | CDH9 | L706M | G | 2289 | T |
| C3 | T1144M | G | 3524 | A | CACNB4 | L342V | A | 1092 | C | CCDC48 | E116G | A | 347 | G | CDH9 | F698Y | A | 2266 | T |
| C3 | M1563V | T | 4780 | C | CACNB4 | I279R | A | 904 | C | CCDC50 | A453V | C | 1948 | T | CDH9 | R267Q | C | 973 | T |
| C3 | - | A | 0 | C | CACNG2 | A197V | G | 1572 | A | CCDC50 | R263Q | G | 1378 | A | CDH9 | R267* | G | 972 | A |
| C3AR1 | G37* | C | 201 | A | CACNG3 | V76M | G | 1428 | A | CCDC51 | R194H | C | 667 | T | CDHR1 | A617T | G | 1952 | A |
| C3orf1 | G187D | G | 762 | A | CACNG3 | R234Q | G | 1903 | A | CCDC51 | V298I | C | 978 | T | CDHR1 | R636C | C | 2009 | T |
| C3orf1 | A127V | C | 582 | T | CACNG3 | R65M | G | 1396 | T | CCDC51 | R257H | C | 856 | T | CDHR1 | R749H | G | 2349 | A |
| C3orf15 | R292C | C | 951 | T | CACNG4 | R105L | G | 329 | T | CCDC51 | L320I | A | 1044 | T | CDHR1 | F546L | C | 1741 | A |
| C3orf15 | R610* | C | 1905 | T | CACNG5 | T74A | A | 280 | G | CCDC52 | S764P | A | 2550 | G | CDHR2 | F866L | T | 2870 | C |
| C3orf15 | W278L | G | 910 | T | CACNG6 | P105S | C | 903 | T | CCDC52 | Q602R | T | 2065 | C | CDHR2 | G752V | G | 2529 | T |
| C3orf15 | K537R | A | 1687 | G | CACNG7 | D158N | G | 684 | A | CCDC52 | E596D | C | 2048 | A | CDHR3 | P321S | C | 1041 | T |
| C3orf15 | L724I | C | 2247 | A | CACNG8 | R150C | C | 551 | T | CCDC53 | T37A | T | 268 | C | CDHR3 | S35Y | C | 184 | A |
| C3orf16 | R45W | G | 590 | A | CAD | T920M | C | 2921 | T | CCDC54 | D206E | C | 865 | G | CDHR3 | I105M | T | 395 | G |
| C3orf16 | G251D | C | 1209 | T | CAD | R516Q | G | 1709 | A | CCDC54 | K7Q | A | 266 | C | CDHR4 | K202R | T | 614 | C |
| C3orf17 | S503T | C | 1700 | G | CAD | V1929I | G | 5947 | A | CCDC54 | H39Q | C | 364 | A | CDHR4 | Y47H | A | 148 | G |
| C3orf17 | F392S | A | 1367 | G | CAD | R2122H | G | 6527 | A | CCDC55 | R526W | C | 1639 | T | CDHR4 | V465M | C | 1402 | T |
| C3orf20 | D277N | G | 1281 | A | CAD | R1948H | G | 6005 | A | CCDC55 | E487D | G | 1524 | T | CDHR5 | R360C | G | 1183 | A |
| C3orf20 | R312* | C | 1386 | T | CAD | R2024Q | G | 6233 | A | CCDC56 | I71V | T | 231 | C | CDHR5 | S770R | G | 2415 | C |
| C3orf22 | S119P | A | 734 | G | CAD | R191* | C | 733 | T | CCDC57 | T315A | T | 980 | C | CDHR5 | R782Q | C | 2450 | T |
| C3orf23 | V135I | G | 648 | A | CAD | I113T | T | 500 | C | CCDC57 | Q819P | T | 2493 | G | CDHR5 | E123K | C | 472 | T |
| C3orf25 | A566V | G | 1860 | A | CAD | S1052A | T | 3316 | G | CCDC58 | R135Q | C | 411 | T | CDIPT | R173W | G | 1394 | A |
| C3orf30 | A434V | C | 1341 | T | CAD | F1266L | C | 3960 | A | CCDC6 | E139* | C | 1051 | A | CDK10 | F302V | T | 929 | G |
| C3orf30 | D179N | G | 575 | A | CADM1 | L147M | G | 439 | T | CCDC6 | V400M | C | 1834 | T | CDK10 | C227Y | G | 705 | A |
| C3orf32 | M1I | C | 272 | T | CADM2 | S188N | G | 563 | A | CCDC6 | E334K | C | 1636 | T | CDK12 | R708C | C | 2433 | T |
| C3orf34 | Q96R | T | 710 | C | CADM2 | S230P | T | 688 | C | CCDC6 | E285D | C | 1491 | A | CDK12 | S316N | G | 1258 | A |
| C3orf38 | - | G | 0 | A | CADM3 | G107E | G | 477 | A | CCDC6 | E139* | C | 1051 | A | CDK12 | V1187A | T | 3871 | C |
| C3orf39 | R24W | G | 401 | A | CADM3 | A239V | C | 873 | T | CCDC60 | R470C | C | 1873 | T | CDK12 | S192Y | C | 886 | A |
| C3orf45 | R70* | C | 295 | T | CADM3 | P124S | C | 527 | T | CCDC60 | N368D | A | 1567 | G | CDK13 | R1426I | G | 4882 | T |
| C3orf55 | S120P | T | 499 | C | CADM4 | D378N | C | 1181 | T | CCDC61 | R160H | G | 479 | A | CDK13 | V1330M | G | 4593 | A |
| C3orf56 | R226H | G | 917 | A | CADPS | E1260* | C | 4138 | A | CCDC62 | C180Y | G | 684 | A | CDK13 | G13R | G | 642 | A |
| C3orf56 | P166H | C | 737 | A | CADPS | R1095W | G | 3643 | A | CCDC62 | N300D | A | 1043 | G | CDK14 | G268V | G | 934 | T |
| C3orf57 | R58H | C | 1744 | T | CADPS | E1086* | C | 3616 | A | CCDC63 | Y245* | C | 930 | A | CDK14 | T125S | A | 504 | T |
| C3orf57 | I42V | T | 1695 | C | CADPS | E334* | C | 1360 | A | CCDC63 | A425T | G | 1468 | A | CDK15 | E83D | G | 335 | T |

| | | | | | | | | | | | | | | | | | | | |
|---|---|---|---|---|---|---|---|---|---|---|---|---|---|---|---|---|---|---|---|
| C3orf57 | R31* | G | 1662 | A | CADPS2 | R212I | C | 655 | A | CCDC63 | R186Q | G | 752 | A | CDK15 | P326L | C | 1063 | T |
| C3orf58 | C337G | T | 1544 | G | CADPS2 | T519P | T | 1575 | G | CCDC63 | R273H | G | 1013 | A | CDK16 | A281S | G | 1365 | T |
| C3orf59 | E34A | T | 422 | G | CADPS2 | L659M | G | 1995 | T | CCDC63 | D456N | G | 1561 | A | CDK18 | V398M | G | 1411 | A |
| C3orf59 | K215T | T | 965 | G | CADPS2 | F296C | A | 907 | C | CCDC64 | R315C | C | 968 | T | CDK18 | K325E | A | 1192 | G |
| C3orf59 | Q41P | T | 443 | G | CADPS2 | A160T | C | 498 | T | CCDC64B | Q98* | G | 337 | A | CDK19 | N156H | T | 646 | G |
| C3orf59 | G217R | C | 970 | T | CAGE1 | T175A | T | 1088 | C | CCDC64B | Q203* | G | 652 | A | CDK2 | E2D | G | 243 | T |
| C3orf59 | D113G | T | 659 | C | CAGE1 | S277N | C | 1395 | T | CCDC65 | K345R | A | 1261 | G | CDK2 | K89T | A | 503 | C |
| C3orf59 | N255Y | T | 1084 | A | CAGE1 | E539K | C | 2180 | T | CCDC65 | Q86H | A | 485 | C | CDK20 | A240V | G | 1025 | A |
| C3orf59 | P466L | G | 1718 | A | CALB1 | F103C | A | 490 | C | CCDC65 | K166N | A | 725 | C | CDK20 | P228S | G | 988 | A |
| C3orf59 | Q381P | T | 1463 | G | CALB1 | E86D | C | 440 | A | CCDC66 | V125I | G | 443 | A | CDK2AP1 | E71G | T | 734 | C |
| C3orf62 | E141K | C | 1458 | T | CALCOCO 2 | K154T | A | 512 | C | CCDC66 | K196N | G | 658 | T | CDK2AP2 | T126M | G | 926 | A |
| C3orf62 | E139D | C | 1454 | A | CALCR | S351F | G | 1228 | A | CCDC66 | I307S | T | 990 | G | CDK4 | D129Y | C | 611 | A |
| C3orf63 | K1347T | T | 4139 | G | CALCR | M253V | T | 933 | C | CCDC67 | E15D | G | 145 | T | CDK5 | R214G | T | 1322 | C |
| C3orf63 | E122* | C | 463 | A | CALCR | F94L | G | 458 | T | CCDC67 | E53* | G | 257 | T | CDK5 | N135S | T | 1086 | C |
| C3orf63 | R172C | G | 613 | A | CALCRL | R417C | G | 1963 | A | CCDC67 | K229Q | A | 785 | C | CDK5R1 | E18V | A | 742 | T |
| C3orf63 | N1634H | T | 4999 | G | CALCRL | D90A | T | 983 | G | CCDC67 | N426S | A | 1377 | G | CDK5R1 | P198L | C | 1282 | T |
| C3orf63 | N1316H | T | 4045 | G | CALCRL | E457V | T | 2084 | A | CCDC68 | R258C | G | 1047 | A | CDK5RAP1 | R169Q | C | 660 | T |
| C3orf63 | R755Q | C | 2363 | T | CALD1 | Q668K | C | 2231 | A | CCDC69 | L210M | G | 803 | T | CDK5RAP1 | H376N | G | 1280 | T |
| C3orf64 | E232D | T | 1439 | G | CALD1 | V657M | G | 2198 | A | CCDC69 | S139* | G | 591 | T | CDK5RAP1 | D441N | C | 1475 | T |
| C3orf70 | S187Y | G | 751 | T | CALD1 | T82I | C | 474 | T | CCDC7 | E205* | G | 922 | T | CDK5RAP2 | E442* | C | 1504 | A |
| C3orf72 | A143T | G | 558 | A | CALD1 | R619H | G | 2085 | A | CCDC7 | R339Q | G | 1325 | A | CDK5RAP2 | E1216D | C | 3828 | A |
| C3orf75 | A90V | G | 440 | A | CALD1 | R593Q | G | 2007 | A | CCDC71 | P220L | G | 766 | A | CDK5RAP2 | L216M | G | 826 | T |
| C3orf75 | A75V | G | 395 | A | CALD1 | S743Y | C | 2457 | A | CCDC71 | V450M | C | 1455 | T | CDK5RAP2 | E1511* | C | 4711 | A |
| C3orf77 | S1265L | C | 3962 | T | CALD1 | K594N | A | 2011 | T | CCDC71 | R66C | G | 303 | A | CDK5RAP2 | K1028Q | T | 3262 | G |
| C3orf77 | E1570* | G | 4876 | T | CALHM2 | R173W | G | 1022 | A | CCDC73 | E1009* | C | 3069 | A | CDK5RAP3 | D293N | G | 961 | A |
| C3orf77 | S90T | G | 437 | C | CALHM2 | Y54H | A | 665 | G | CCDC73 | K573N | T | 1763 | G | CDK5RAP3 | I45T | T | 218 | C |
| C3orf77 | A1560D | C | 4847 | A | CALHM3 | R177W | G | 737 | A | CCDC73 | - | C | 0 | A | CDK6 | R220C | G | 1070 | A |
| C3orf77 | H1654Y | C | 5128 | T | CALR | E240D | G | 800 | T | CCDC75 | - | G | 0 | T | CDK8 | R237* | C | 1212 | T |
| C3orf77 | S448L | C | 1511 | T | CALR | N188D | A | 642 | G | CCDC75 | K83N | G | 382 | T | CDK8 | R200* | C | 1101 | T |
| C3orf77 | S800Y | C | 2567 | A | CALR | K64T | A | 271 | C | CCDC78 | L51I | G | 243 | T | CDK8 | D240G | A | 1222 | G |
| C3orf77 | S1173Y | C | 3686 | A | CALR3 | E351K | C | 1114 | T | CCDC78 | A172V | G | 607 | A | CDK8 | R71* | C | 714 | T |
| C3orf77 | L1401R | T | 4370 | G | CALU | K251N | G | 1584 | T | CCDC78 | L53F | G | 249 | A | CDKAL1 | P295L | C | 1058 | T |
| C3orf78 | R19Q | G | 207 | A | CAMK1 | T114M | G | 519 | A | CCDC8 | S237* | G | 1484 | T | CDKAL1 | Y148H | T | 616 | C |
| C4BPA | N528S | A | 1777 | G | CAMK1G | R112W | C | 579 | T | CCDC8 | N270I | T | 1583 | A | CDKAL1 | A557V | C | 1844 | T |
| C4BPA | D341Y | G | 1215 | T | CAMK1G | S129L | C | 631 | T | CCDC8 | R50H | C | 923 | T | CDKAL1 | - | G | 0 | T |
| C4orf14 | P286S | G | 2094 | A | CAMK2A | G397A | C | 1194 | G | CCDC80 | R442K | C | 1325 | T | CDKL1 | P895H | G | 2684 | T |
| C4orf17 | E224K | G | 1032 | A | CAMK2B | A365T | C | 1303 | T | CCDC80 | R822H | C | 2465 | T | CDKL1 | Q898P | T | 2693 | G |
| C4orf21 | Q987* | G | 3085 | A | CAMK2N1 | R55W | G | 1004 | A | CCDC80 | S548Y | G | 1643 | T | CDKL1 | F907L | G | 2721 | T |
| C4orf21 | S1507P | A | 4645 | G | CAMKK1 | R506Q | C | 1665 | T | CCDC81 | A184T | G | 978 | A | CDKL2 | D262Y | C | 1298 | A |

| | | | | | | | | | | | | | | | | | | | |
|---|---|---|---|---|---|---|---|---|---|---|---|---|---|---|---|---|---|---|---|
| C4orf21 | S2102Y | G | 6431 | T | CAMKK1 | V123A | A | 516 | G | CCDC81 | Y620C | A | 2287 | G | CDKL2 | F145V | A | 947 | C |
| C4orf21 | Y1149* | A | 3573 | C | CAMKK1 | E358K | C | 1220 | T | CCDC82 | K523Q | T | 1582 | G | CDKL2 | F82L | A | 760 | C |
| C4orf22 | T96M | C | 287 | T | CAMKK2 | V179I | C | 1364 | T | CCDC84 | R167Q | G | 556 | A | CDKL3 | R464H | C | 1510 | T |
| C4orf23 | R597H | G | 1808 | A | CAMKV | P420T | G | 1737 | T | CCDC84 | A240V | C | 775 | T | CDKL3 | K375N | C | 1244 | A |
| C4orf23 | K389T | A | 1184 | C | CAMLG | M38I | G | 234 | A | CCDC85A | S410T | G | 1731 | C | CDKL3 | S177Y | G | 649 | T |
| C4orf29 | E144G | A | 775 | G | CAMSAP1 | D133N | C | 466 | T | CCDC85A | R270H | G | 1311 | A | CDKL4 | R53C | G | 157 | A |
| C4orf31 | R25Q | C | 601 | T | CAMSAP1 | A1536V | G | 4676 | A | CCDC85A | I540L | A | 2120 | C | CDKL5 | R840H | G | 2794 | A |
| C4orf31 | L145V | A | 960 | C | CAMSAP1 | R1108W | G | 3391 | A | CCDC85B | R26L | G | 457 | T | CDKN2A | R124H | C | 583 | T |
| C4orf35 | E142V | A | 484 | T | CAMSAP1 | A1312T | C | 4003 | T | CCDC87 | R370H | C | 1177 | T | CDKN2A | R80* | G | 450 | A |
| C4orf35 | E241K | G | 780 | A | CAMSAP1 | S455L | G | 1433 | A | CCDC87 | Y818* | A | 2522 | C | CDKN2A | A76T | C | 438 | T |
| C4orf35 | P21Q | C | 121 | A | CAMSAP1L 1 | S375R | A | 1393 | C | CCDC88A | A305T | C | 1755 | T | CDKN2A | R107H | C | 532 | T |
| C4orf37 | V21M | C | 151 | T | CAMSAP1L 1 | S282Y | C | 1115 | A | CCDC88A | R1412W | G | 5076 | A | CDKN2AIP | S151N | G | 659 | A |
| C4orf37 | E151K | C | 541 | T | CAMSAP1L 1 | S1029* | C | 3356 | A | CCDC88A | T15A | T | 885 | C | CDKN2B | R60H | C | 507 | T |
| C4orf37 | T253P | T | 847 | G | CAMSAP1L 1 | E1230* | G | 3958 | T | CCDC88A | R982* | G | 3786 | A | CDKN2B | R105W | G | 641 | A |
| C4orf38 | A32T | C | 706 | T | CAMTA1 | E310D | G | 1137 | T | CCDC88B | D512N | G | 1578 | A | CDNF | E163* | C | 613 | A |
| C4orf39 | L33M | C | 172 | A | CAMTA1 | A1117V | C | 3557 | T | CCDC88B | R979W | C | 2979 | T | CDNF | Q149P | T | 572 | G |
| C4orf41 | E869* | G | 2807 | T | CAMTA1 | R163W | C | 694 | T | CCDC88B | L123M | T | 411 | A | CDNF | E66* | C | 322 | A |
| C4orf41 | W105C | G | 517 | T | CAMTA1 | W967* | G | 3107 | A | CCDC88B | R472W | C | 1458 | T | CDO1 | T148M | G | 1000 | A |
| C4orf41 | K305N | G | 1117 | T | CAMTA1 | N231T | A | 899 | C | CCDC88C | S689F | G | 2165 | A | CDO1 | L95M | G | 840 | T |
| C4orf41 | R360W | C | 1280 | T | CAMTA1 | A849T | G | 2752 | A | CCDC88C | S317Y | G | 1049 | T | CDON | R1112Q | C | 3463 | T |
| CDON | S885* | G | 2782 | T | CHST1 | R237C | G | 1380 | A | COCH | R148* | C | 846 | T | CREBBP | R2104C | G | 7115 | A |
| CDON | E501K | C | 1629 | T | CHST1 | S201R | G | 1274 | T | COCH | P505S | C | 1917 | T | CREBBP | R1964H | C | 6696 | T |
| CDON | P690L | G | 2197 | A | CHST10 | R208Q | C | 1009 | T | COCH | S300Y | C | 1303 | A | CREBBP | Q2199L | T | 7401 | A |
| CDON | T1162N | G | 3613 | T | CHST10 | R229Q | C | 1072 | T | COG1 | P851T | C | 2587 | A | CREBBP | A1907V | G | 6525 | A |
| CDON | R58C | G | 300 | A | CHST10 | G220D | C | 1045 | T | COG1 | K130T | A | 425 | C | CREBBP | A1782T | C | 6149 | T |
| CDON | S945Y | G | 2962 | T | CHST11 | H174R | A | 814 | G | COG1 | G305R | G | 949 | A | CREBBP | R1664C | G | 5795 | A |
| CDON | F31C | A | 220 | C | CHST11 | R175H | G | 817 | A | COG1 | R867C | C | 2635 | T | CREBBP | R2052W | G | 6959 | A |
| CDR2 | A247V | G | 1048 | A | CHST12 | R276W | C | 961 | T | COG2 | R442H | G | 1440 | A | CREBBP | A619T | C | 2660 | T |
| CDR2 | Q368R | T | 1411 | C | CHST12 | E409D | A | 1362 | C | COG2 | E62K | G | 299 | A | CREBBP | P2346L | G | 7842 | A |
| CDR2L | T184M | C | 551 | T | CHST13 | G133S | G | 447 | A | COG4 | A122V | G | 378 | A | CRELD1 | S86G | A | 855 | G |
| CDR2L | R214Q | G | 641 | A | CHST13 | A137T | G | 459 | A | COG4 | R266Q | C | 810 | T | CRELD1 | S178C | A | 1131 | T |
| CDRT15 | G122E | C | 381 | T | CHST14 | H318R | A | 1153 | G | COG5 | R804H | C | 2936 | T | CRELD2 | E373K | G | 1252 | A |
| CDRT15 | K144N | T | 448 | G | CHST15 | S408L | G | 1866 | A | COG5 | L627V | A | 2404 | C | CRHBP | P303L | C | 1330 | T |
| CDS1 | R399I | G | 1619 | T | CHST15 | R519H | C | 2199 | T | COG5 | R418C | G | 1777 | A | CRHBP | D139Y | G | 837 | T |
| CDS2 | Y333C | A | 1305 | G | CHST15 | L431F | G | 1934 | A | COG5 | Y342D | A | 1549 | C | CRHBP | E245K | G | 1155 | A |
| CDS2 | R248W | C | 1049 | T | CHST15 | A385V | G | 1797 | A | COG6 | S334Y | C | 1002 | A | CRIM1 | R350* | C | 1415 | T |

| | | | | | | | | | | | | | | | | | | | |
|---|---|---|---|---|---|---|---|---|---|---|---|---|---|---|---|---|---|---|---|
| CDT1 | P89T | C | 319 | A | CHST15 | M89I | C | 910 | T | COG6 | E139K | G | 416 | A | CRIP1 | E45G | A | 287 | G |
| CDT1 | R427* | C | 1333 | T | CHST15 | R79Q | C | 879 | T | COG6 | D567G | A | 1701 | G | CRIPAK | A59D | C | 324 | A |
| CDYL | R329C | C | 1116 | T | CHST3 | A461T | G | 1818 | A | COG7 | R756C | G | 2452 | A | CRIPT | C83Y | G | 380 | A |
| CDYL | E160K | G | 609 | A | CHST4 | R335H | G | 1202 | A | COG7 | A511V | G | 1718 | A | CRISP1 | T134M | G | 503 | A |
| CDYL | A249T | G | 876 | A | CHST4 | R52H | G | 353 | A | COIL | A230V | G | 720 | A | CRISP2 | R88C | G | 518 | A |
| CDYL | I316F | A | 1077 | T | CHST4 | R270H | G | 1007 | A | COIL | A25T | C | 104 | T | CRISP2 | E46* | C | 392 | A |
| CDYL2 | R285Q | C | 959 | T | CHST4 | F57C | T | 368 | G | COL10A1 | G86R | C | 352 | T | CRISP2 | R88H | C | 519 | T |
| CDYL2 | R79C | G | 340 | A | CHST5 | A214V | G | 2036 | A | COL10A1 | M343L | T | 1123 | A | CRISPLD1 | G53V | G | 533 | T |
| CDYL2 | V404I | C | 1315 | T | CHST6 | A13V | G | 218 | A | COL10A1 | S659A | A | 2071 | C | CRISPLD1 | N211D | A | 1006 | G |
| CEACAM1 | R58Q | C | 406 | T | CHST6 | V56M | C | 346 | T | COL11A1 | I124V | T | 688 | C | CRISPLD1 | M423V | A | 1642 | G |
| CEACAM1 6 | T147M | C | 440 | T | CHST6 | R315C | G | 1123 | A | COL11A1 | T1404P | T | 4528 | G | CRKL | K146R | A | 946 | G |
| CEACAM1 9 | P298L | C | 1103 | T | CHST6 | G357D | C | 1250 | T | COL11A1 | Q1188* | G | 3880 | A | CRLF3 | R180C | G | 647 | A |
| CEACAM2 1 | R127I | G | 406 | T | CHST6 | Q346* | G | 1216 | A | COL11A1 | A1770V | G | 5627 | A | CRLS1 | L176I | C | 683 | A |
| CEACAM3 | P3A | C | 248 | G | CHST6 | R177C | G | 709 | A | COL11A1 | G379V | C | 1454 | A | CRLS1 | R155Q | G | 621 | A |
| CEACAM4 | A117T | C | 460 | T | CHST8 | D221N | G | 1166 | A | COL12A1 | S981N | C | 3252 | T | CRMP1 | R352Q | C | 1144 | T |
| CEACAM5 | N292S | A | 1022 | G | CHST8 | A388T | G | 1667 | A | COL12A1 | K831T | T | 2802 | G | CRMP1 | T332M | G | 1084 | A |
| CEACAM5 | T298M | C | 1040 | T | CHST9 | H201N | G | 887 | T | COL12A1 | A279S | C | 1145 | A | CRMP1 | E549K | C | 1734 | T |
| CEACAM5 | - | A | 0 | G | CHST9 | R280C | G | 1124 | A | COL12A1 | S1244N | C | 4041 | T | CRMP1 | D529N | C | 1674 | T |
| CEACAM6 | P3S | C | 225 | T | CHST9 | K167T | T | 786 | G | COL12A1 | E898K | C | 3002 | T | CRMP1 | A496T | C | 1575 | T |
| CEACAM6 | P220T | C | 876 | A | CHST9 | E48* | C | 428 | A | COL12A1 | T1353A | T | 4367 | C | CRMP1 | T215N | G | 733 | T |
| CEACAM6 | P59L | C | 394 | T | CHSY1 | Q729H | C | 2663 | A | COL12A1 | Q909H | T | 3037 | G | CRMP1 | R62C | G | 273 | A |
| CEACAM6 | A105T | G | 531 | A | CHSY1 | S788N | C | 2839 | T | COL12A1 | C2666* | G | 8308 | T | CRMP1 | A199V | G | 685 | A |
| CEACAM6 | S321R | A | 1179 | C | CHSY3 | F205L | C | 973 | A | COL12A1 | I2148V | T | 6752 | C | CRMP1 | E239K | C | 804 | T |
| CEACAM7 | A206V | G | 819 | A | CHSY3 | Q356R | A | 1425 | G | COL12A1 | E2979K | C | 9245 | T | CRMP1 | D202Y | C | 693 | A |
| CEACAM8 | K126Q | T | 478 | G | CHSY3 | M644L | A | 2288 | T | COL12A1 | E2531* | C | 7901 | A | CRMP1 | - | C | 0 | A |
| CEACAM8 | V229L | C | 787 | G | CHSY3 | L275I | C | 1181 | A | COL12A1 | E1342K | C | 4334 | T | CRNKL1 | H414Y | G | 1272 | A |
| CEBPZ | R1019C | G | 3201 | A | CHSY3 | R879Q | G | 2994 | A | COL12A1 | Q1313H | T | 4249 | G | CRNKL1 | D470G | T | 1441 | C |
| CEBPZ | E825D | C | 2621 | A | CHTF18 | M198I | G | 594 | A | COL12A1 | V596L | C | 2096 | A | CRNKL1 | N7S | T | 52 | C |
| CECR1 | F235L | G | 976 | T | CHTF18 | R434W | C | 1300 | T | COL12A1 | D440N | C | 1628 | T | CRNN | T21M | G | 125 | A |
| CECR2 | L300I | C | 898 | A | CHUK | E194D | C | 669 | A | COL12A1 | G173R | C | 827 | T | CROCC | L758F | C | 2341 | T |
| CECR2 | R523W | C | 1567 | T | CIAPIN1 | A254V | G | 1002 | A | COL12A1 | G2872A | C | 8925 | G | CROCC | R1866C | C | 5665 | T |
| CECR2 | R1332Q | G | 3995 | A | CIB2 | S49G | T | 474 | C | COL13A1 | E706K | G | 2652 | A | CROCC | R491Q | G | 1541 | A |
| CECR2 | R338G | A | 1012 | G | CIB2 | S32* | G | 424 | T | COL14A1 | R566Q | G | 1967 | A | CROCC | A1740G | C | 5288 | G |
| CECR2 | T1024M | C | 3071 | T | CIB3 | E64K | C | 240 | T | COL14A1 | P1772H | C | 5585 | A | CROCC | R1280W | C | 3907 | T |
| CECR2 | N110K | T | 330 | G | CIB3 | E155D | C | 515 | A | COL14A1 | F1048S | T | 3413 | C | CROCC | R1154C | C | 3529 | T |
| CECR5 | V365A | A | 1120 | G | CIB3 | E26D | C | 128 | A | COL14A1 | R1604* | C | 5080 | T | CROCC | Q1683R | A | 5117 | G |
| CECR5 | Q349* | G | 1071 | A | CIC | R351W | C | 1091 | T | COL14A1 | G138W | G | 682 | T | CROT | R215H | G | 843 | A |

| | | | | | | | | | | | | | | | | | | | |
|---|---|---|---|---|---|---|---|---|---|---|---|---|---|---|---|---|---|---|---|
| CECR6 | V477M | C | 1555 | T | CIC | - | T | 0 | C | COL14A1 | A822T | G | 2734 | A | CROT | Y141C | A | 621 | G |
| CEL | G567E | G | 1716 | A | CIC | R353* | C | 1097 | T | COL14A1 | F1048C | T | 3413 | G | CROT | E30* | G | 287 | T |
| CEL | V245M | G | 749 | A | CIC | T303M | C | 948 | T | COL15A1 | G987* | G | 3382 | T | CRP | N176H | T | 630 | G |
| CEL | A203S | G | 623 | T | CIC | P1336L | C | 4047 | T | COL15A1 | A165T | G | 916 | A | CRP | K140N | C | 524 | A |
| CEL | R156H | G | 483 | A | CIC | A669T | G | 2045 | A | COL15A1 | G39C | G | 538 | T | CRTAC1 | D273N | C | 1173 | T |
| CEL | P61L | C | 198 | T | CIC | S68T | T | 242 | A | COL15A1 | G1179* | G | 3958 | T | CRTAM | E24* | G | 119 | T |
| CELA1 | Q37H | C | 152 | G | CIDEA | T131M | C | 507 | T | COL15A1 | R552I | G | 2078 | T | CRTAP | A295T | G | 982 | A |
| CELA1 | A140T | C | 459 | T | CIDEB | T155A | T | 1651 | C | COL15A1 | E904* | G | 3133 | T | CRTAP | P289L | C | 965 | T |
| CELA2B | A23T | G | 92 | A | CIDEC | L138V | G | 437 | C | COL16A1 | A1233V | G | 4215 | A | CRTC1 | P428L | C | 1371 | T |
| CELA3B | S116L | C | 366 | T | CIITA | W822* | G | 2615 | A | COL16A1 | D152N | C | 971 | T | CRTC1 | H627Y | C | 1967 | T |
| CELF2 | Q405P | A | 1374 | C | CILP | R175H | C | 691 | T | COL16A1 | G1291R | C | 4388 | T | CRTC1 | E246D | A | 826 | C |
| CELF2 | A182V | C | 705 | T | CILP | R145C | G | 600 | A | COL16A1 | P536H | G | 2124 | T | CRTC2 | L343M | G | 1155 | T |
| CELF3 | F140L | G | 419 | T | CILP | R1034H | C | 3268 | T | COL16A1 | - | C | 0 | A | CRX | R69H | G | 410 | A |
| CELF3 | E72K | C | 213 | T | CILP | R484Q | C | 1618 | T | COL16A1 | F886L | G | 3175 | T | CRY1 | H411Y | G | 2090 | A |
| CELF4 | A391D | G | 1568 | T | CILP2 | G967S | G | 2984 | A | COL16A1 | G656R | C | 2483 | T | CRY2 | R50H | G | 171 | A |
| CELF4 | Q60* | G | 574 | A | CINP | S113Y | G | 403 | T | COL17A1 | R1280H | C | 4130 | T | CRYAA | Y109H | T | 417 | C |
| CELF4 | A373T | C | 1513 | T | CIRH1A | G119V | G | 533 | T | COL17A1 | Q602* | G | 2095 | A | CRYAB | E88G | T | 309 | C |
| CELF4 | P406L | G | 1613 | A | CIT | R1102W | G | 3357 | A | COL17A1 | S1492I | C | 4766 | A | CRYBA4 | R103H | G | 343 | A |
| CELF4 | - | C | 0 | T | CIT | G1729D | C | 5239 | T | COL17A1 | A635T | C | 2194 | T | CRYBA4 | - | G | 0 | A |
| CELF4 | A354V | G | 1457 | A | CIT | C1448Y | C | 4396 | T | COL17A1 | R143* | G | 718 | A | CRYBB1 | R92H | C | 406 | T |
| CELF5 | V270M | G | 845 | A | CIT | P1871H | G | 5665 | T | COL18A1 | L652M | C | 1975 | A | CRYBB3 | S138G | A | 492 | G |
| CELF5 | R222H | G | 702 | A | CIT | V376I | C | 1179 | T | COL18A1 | D854N | G | 2581 | A | CRYBG3 | P162H | C | 549 | A |
| CELF6 | A280V | G | 1094 | A | CIT | R1083W | G | 3300 | A | COL18A1 | G1383S | G | 4168 | A | CRYBG3 | G347W | G | 1103 | T |
| CELF6 | S105I | C | 569 | A | CIT | E98K | C | 345 | T | COL18A1 | G302S | G | 925 | A | CRYBG3 | G347V | G | 1104 | T |
| CELSR1 | V2577M | C | 7729 | T | CITED1 | P200L | G | 797 | A | COL18A1 | G1102R | G | 3325 | A | CRYBG3 | S145N | G | 498 | A |
| CELSR1 | V702M | C | 2104 | T | CIZ1 | F807L | G | 2624 | T | COL18A1 | D1028G | A | 3104 | G | CRYBG3 | E537D | A | 1675 | C |
| CELSR1 | R647C | G | 1939 | A | CIZ1 | E751D | C | 2456 | A | COL18A1 | D1187N | G | 3580 | A | CRYBG3 | R572C | C | 1778 | T |
| CELSR1 | D2286N | C | 6856 | T | CIZ1 | R329W | G | 1188 | A | COL18A1 | S1377N | G | 4151 | A | CRYBG3 | L754I | C | 2324 | A |
| CELSR1 | A905V | G | 2714 | A | CIZ1 | R310Q | C | 1132 | T | COL19A1 | - | G | 0 | T | CRYGB | T107I | G | 368 | A |
| CELSR1 | R2031H | C | 6092 | T | CKAP2 | K452T | A | 1445 | C | COL19A1 | G95D | G | 386 | A | CRYGB | L147P | A | 488 | G |
| CELSR1 | G1662C | C | 4984 | A | CKAP2L | K243Q | T | 806 | G | COL1A1 | R1260H | C | 3898 | T | CRYGC | S87F | G | 298 | A |
| CELSR1 | A504D | G | 1511 | T | CKAP2L | E174K | C | 599 | T | COL1A1 | L1384V | G | 4269 | C | CRYL1 | R197H | C | 653 | T |
| CELSR1 | S2140N | C | 6419 | T | CKAP4 | R148K | C | 527 | T | COL1A1 | R1217W | G | 3768 | A | CRYL1 | A45T | C | 196 | T |
| CELSR1 | N1482D | T | 4444 | C | CKAP5 | G1813W | C | 5547 | A | COL1A1 | R487C | G | 1578 | A | CRYL1 | E100A | T | 362 | G |
| CELSR1 | T337A | T | 1009 | C | CKAP5 | E239K | C | 825 | T | COL1A2 | S404Y | C | 1682 | A | CRYL1 | V95A | A | 347 | G |
| CELSR1 | L2454F | G | 7360 | A | CKAP5 | E1888K | C | 5772 | T | COL1A2 | G87R | G | 730 | A | CRYZL1 | L316V | A | 946 | C |
| CELSR1 | L1265Q | A | 3794 | T | CKB | G117D | C | 430 | T | COL1A2 | G793V | G | 2849 | T | CRYZL1 | W282* | C | 845 | T |
| CELSR1 | R1044W | G | 3130 | A | CKM | D221N | C | 735 | T | COL1A2 | P1011S | C | 3502 | T | CS | L21R | A | 252 | C |
| CELSR2 | L17P | T | 111 | C | CKM | G356D | C | 1141 | T | COL1A2 | G1081C | G | 3712 | T | CSAD | - | T | 0 | C |

| | | | | | | | | | | | | | | | | | | | |
|---|---|---|---|---|---|---|---|---|---|---|---|---|---|---|---|---|---|---|---|
| CELSR2 | L783M | C | 2408 | A | CKM | W228L | C | 757 | A | COL1A2 | A462T | G | 1855 | A | CSAD | R102W | G | 538 | A |
| CELSR2 | R345C | C | 1094 | T | CKM | R252H | C | 829 | T | COL20A1 | V1015I | G | 3143 | A | CSDC2 | - | G | 0 | A |
| CELSR2 | R444Q | G | 1392 | A | CKM | D190Y | C | 642 | A | COL20A1 | W404* | G | 1312 | A | CSDC2 | A59T | G | 719 | A |
| CELSR2 | - | G | 0 | A | CKMT1A | L379I | C | 1302 | A | COL20A1 | V417I | G | 1349 | A | CSDE1 | Y102H | A | 826 | G |
| CELSR2 | R989W | C | 3026 | T | CKMT2 | V126I | G | 489 | A | COL20A1 | L98F | C | 392 | T | CSDE1 | K110N | T | 852 | G |
| CELSR2 | R842H | G | 2586 | A | CKMT2 | R220W | C | 771 | T | COL20A1 | A673V | C | 2118 | T | CSE1L | N795D | A | 2506 | G |
| CELSR2 | G1904S | G | 5771 | A | CKMT2 | A365T | G | 1206 | A | COL20A1 | P770S | C | 2408 | T | CSE1L | N338S | A | 1136 | G |
| CELSR2 | G1948C | G | 5903 | T | CLASP1 | A157V | G | 860 | A | COL20A1 | K222T | A | 765 | C | CSE1L | G534W | G | 1723 | T |
| CELSR3 | R398H | C | 1474 | T | CLASP1 | R742C | G | 2614 | A | COL20A1 | D326N | G | 1076 | A | CSF1R | F737L | G | 2503 | T |
| CELSR3 | L2237V | G | 6990 | C | CLASP1 | A635T | C | 2293 | T | COL21A1 | E358K | C | 1470 | T | CSF1R | R142C | G | 716 | A |
| CELSR3 | R2835C | G | 8784 | A | CLASP1 | P94S | G | 670 | A | COL21A1 | G931D | C | 3190 | T | CSF1R | A960T | C | 3170 | T |
| CELSR3 | R2069W | G | 6486 | A | CLASP1 | R717* | G | 2539 | A | COL21A1 | F40L | A | 516 | G | CSF2RB | R177W | C | 746 | T |
| CELSR3 | A2478V | G | 7714 | A | CLASP1 | S266Y | G | 1187 | T | COL21A1 | - | C | 0 | A | CSF3R | R367W | G | 1647 | A |
| CELSR3 | R386H | C | 1438 | T | CLASP2 | S1224* | G | 4025 | T | COL22A1 | A2V | G | 452 | A | CSF3R | Y196H | A | 1134 | G |
| CELSR3 | R359C | G | 1356 | A | CLASP2 | - | C | 0 | A | COL22A1 | A141T | C | 868 | T | CSF3R | L285F | G | 1401 | A |
| CELSR3 | R301C | G | 1182 | A | CLASP2 | R603Q | C | 2162 | T | COL22A1 | R214W | G | 1087 | A | CSF3R | A859V | G | 3124 | A |
| CELSR3 | G206E | C | 898 | T | CLASP2 | V24A | A | 425 | G | COL22A1 | G982R | C | 3391 | T | CSGALNAC T1 | K380N | C | 1794 | A |
| CELSR3 | G2104C | C | 6591 | A | CLC | G86D | C | 333 | T | COL22A1 | P978L | G | 3380 | A | CSGALNAC T2 | G457V | G | 1705 | T |
| CELSR3 | E1454K | C | 4641 | T | CLCA2 | D771G | A | 2474 | G | COL22A1 | R1465H | C | 4841 | T | CSH2 | G187A | C | 702 | G |
| CEND1 | C119Y | C | 532 | T | CLCA2 | F285V | T | 1015 | G | COL22A1 | G1162* | C | 3931 | A | CSHL1 | S193Y | G | 578 | T |
| CENPA | R43W | C | 327 | T | CLCA4 | S242R | A | 768 | C | COL22A1 | E769* | C | 2752 | A | CSMD1 | S449Y | G | 1346 | T |
| CENPA | R80C | C | 438 | T | CLCC1 | S55N | C | 272 | T | COL24A1 | C1545W | G | 5002 | C | CSMD1 | F272I | A | 814 | T |
| CENPB | E460D | C | 1587 | A | CLCN1 | P155S | C | 550 | T | COL24A1 | G1285D | C | 4221 | T | CSMD1 | A1950P | C | 5848 | G |
| CENPB | S468L | G | 1610 | A | CLCN1 | F385L | T | 1240 | C | COL24A1 | R875C | G | 2990 | A | CSMD1 | R2193* | G | 6577 | A |
| CENPE | S810L | G | 2519 | A | CLCN1 | R199C | C | 682 | T | COL24A1 | S464G | T | 1757 | C | CSMD1 | V2609I | C | 7825 | T |
| CENPE | D2123G | T | 6458 | C | CLCN1 | E291K | G | 958 | A | COL24A1 | D477Y | C | 1796 | A | CSMD1 | R2133K | C | 6398 | T |
| CENPE | I1242V | T | 3814 | C | CLCN1 | R626Q | G | 1964 | A | COL24A1 | F772L | A | 2681 | G | CSMD1 | V1513M | C | 4537 | T |
| CENPE | A74V | G | 311 | A | CLCN2 | R629H | C | 2058 | T | COL25A1 | E437K | C | 1840 | T | CSMD1 | K520Q | T | 1558 | G |
| CENPE | N2576K | A | 7818 | C | CLCN2 | R350W | G | 1220 | A | COL25A1 | P602L | G | 2336 | A | CSMD1 | T1436M | G | 4307 | A |
| CENPE | K1942T | T | 5915 | G | CLCN2 | M349T | A | 1218 | G | COL25A1 | A7T | C | 550 | T | CSMD1 | G962C | C | 2884 | A |
| CENPF | K1944N | G | 6000 | T | CLCN2 | R471C | G | 1583 | A | COL25A1 | E136* | C | 937 | A | CSMD1 | R437H | C | 1310 | T |
| CENPF | L477P | T | 1598 | C | CLCN3 | N168S | A | 1057 | G | COL25A1 | E67K | C | 730 | T | CSMD1 | G900V | C | 2699 | A |
| CENPF | R402H | G | 1373 | A | CLCN3 | R373C | C | 1671 | T | COL27A1 | R707Q | G | 2120 | A | CSMD1 | A978T | C | 2932 | T |
| CENPF | K113N | A | 507 | T | CLCN3 | F399C | T | 1750 | G | COL27A1 | R412H | G | 1235 | A | CSMD1 | C483* | A | 1449 | T |
| CENPF | Y2864H | T | 8758 | C | CLCN3 | V597A | T | 2344 | C | COL27A1 | P645S | C | 1933 | T | CSMD1 | G90V | C | 269 | A |
| CENPF | R2309C | C | 7093 | T | CLCN4 | R501S | G | 1894 | T | COL27A1 | P1581H | C | 4742 | A | CSMD1 | G1425E | C | 4274 | T |
| CENPF | D265Y | G | 961 | T | CLCN5 | A652S | G | 2336 | T | COL27A1 | R1474I | G | 4421 | T | CSMD1 | F3081L | A | 9241 | G |
| CENPF | K429N | G | 1455 | T | CLCN6 | R583Q | G | 1751 | A | COL28A1 | G441R | C | 1462 | T | CSMD1 | L2670F | G | 8008 | A |

| | | | | | | | | | | | | | | | | | | | |
|---|---|---|---|---|---|---|---|---|---|---|---|---|---|---|---|---|---|---|---|
| CENPF | E949K | G | 3013 | A | CLCN6 | E595K | G | 1786 | A | COL28A1 | T17M | G | 191 | A | CSMD1 | P521Q | G | 1562 | T |
| CENPF | E1445K | G | 4501 | A | CLCN7 | A655T | C | 2000 | T | COL28A1 | R741W | G | 2362 | A | CSMD1 | P8S | G | 22 | A |
| CENPF | N1730K | T | 5358 | A | CLCNKA | S350Y | C | 1068 | A | COL28A1 | P698S | G | 2233 | A | CSMD1 | S216Y | G | 647 | T |
| CENPF | E2140D | G | 6588 | T | CLCNKA | R438C | C | 1331 | T | COL2A1 | R560W | G | 1843 | A | CSMD1 | G2196S | C | 6586 | T |
| CENPF | K2448Q | A | 7510 | C | CLCNKB | A329T | G | 1096 | A | COL2A1 | A94T | C | 445 | T | CSMD1 | A2406S | C | 7216 | A |
| CENPF | E2859A | A | 8744 | C | CLCNKB | G456W | G | 1477 | T | COL2A1 | L1412F | G | 4399 | A | CSMD1 | C2266R | A | 6796 | G |
| CENPF | K3029T | A | 9254 | C | CLCNKB | L536M | C | 1717 | A | COL2A1 | G819D | C | 2621 | T | CSMD1 | G1803R | C | 5407 | T |
| CENPH | E190D | A | 657 | C | CLDN1 | V100I | C | 567 | T | COL2A1 | D1309G | T | 4091 | C | CSMD1 | V2968F | C | 8902 | A |
| CENPI | E430K | G | 1457 | A | CLDN1 | K106E | T | 585 | C | COL2A1 | I54S | A | 326 | C | CSMD1 | G3171* | C | 9511 | A |
| CENPJ | R284C | G | 1036 | A | CLDN10 | A4V | C | 241 | T | COL3A1 | G420S | G | 1428 | A | CSMD1 | V2610M | C | 7828 | T |
| CENPJ | F1192L | G | 3762 | T | CLDN10 | I122L | A | 393 | C | COL3A1 | R1363Q | G | 4258 | A | CSMD2 | A610T | C | 1857 | T |
| CENPJ | F133L | G | 585 | T | CLDN14 | W30R | A | 955 | G | COL3A1 | E71* | G | 381 | T | CSMD2 | L3367F | G | 1012 8 | A |
| CENPM | V157M | C | 557 | T | CLDN14 | Y62* | G | 1053 | C | COL4A1 | P198S | G | 714 | A | CSMD2 | D1503N | C | 4536 | T |
| CENPN | S62R | T | 260 | G | CLDN16 | K112Q | A | 582 | C | COL4A1 | - | C | 0 | T | CSMD2 | A1269T | C | 3834 | T |
| CENPQ | S178R | C | 623 | A | CLDN17 | F66L | G | 337 | T | COL4A1 | R585C | G | 1875 | A | CSMD2 | Q1660H | C | 5009 | G |
| CENPT | E339* | C | 1052 | A | CLDN19 | G49W | C | 336 | A | COL4A1 | V1659I | C | 5097 | T | CSMD2 | A3525T | C | 1060 2 | T |
| CEP110 | E2014D | G | 6302 | C | CLDN2 | R189I | G | 892 | T | COL4A1 | T1464S | G | 4513 | C | CSMD2 | L1186F | G | 3585 | A |
| CEP110 | K819Q | A | 2715 | C | CLDN25 | H108Y | C | 322 | T | COL4A1 | K640Q | T | 2040 | G | CSMD2 | G2456S | C | 7395 | T |
| CEP110 | - | G | 0 | T | CLDN25 | E42K | G | 124 | A | COL4A2 | R334W | C | 1306 | T | CSMD2 | R3261C | G | 9810 | A |
| CEP120 | E467D | C | 1706 | A | CLDN5 | G70S | C | 1269 | T | COL4A2 | E1345* | G | 4339 | T | CSMD2 | T3301M | G | 9931 | A |
| CEP120 | E695* | C | 2388 | A | CLDN5 | A203V | G | 1669 | A | COL4A2 | R740Q | G | 2525 | A | CSMD2 | L1863P | A | 5617 | G |
| CEP120 | K292T | T | 1180 | G | CLDN5 | K150N | T | 1511 | G | COL4A2 | - | G | 0 | T | CSMD2 | L171M | G | 540 | T |
| CEP135 | V227M | G | 803 | A | CLDN6 | I123L | T | 796 | G | COL4A2 | K544N | A | 1938 | C | CSMD2 | F2038L | A | 6141 | G |
| CEP135 | R162I | G | 609 | T | CLDN6 | V220I | C | 1087 | T | COL4A2 | K556N | A | 1974 | C | CSMD3 | A1586S | C | 5001 | A |
| CEP152 | I1261M | A | 3903 | C | CLDN8 | L165P | A | 642 | G | COL4A3 | L475M | C | 1585 | A | CSMD3 | - | C | 0 | T |
| CEP152 | K515T | T | 1664 | G | CLDN8 | T121M | G | 510 | A | COL4A3 | P1338S | C | 4174 | T | CSMD3 | R2688H | C | 8308 | T |
| CEP152 | H1420Y | G | 4378 | A | CLDN8 | E163* | C | 635 | A | COL4A3 | A932V | C | 2957 | T | CSMD3 | A3693V | G | 1132 3 | A |
| CEP152 | K379N | C | 1257 | A | CLDN9 | A102V | C | 878 | T | COL4A3 | T1172M | C | 3677 | T | CSMD3 | P2348H | G | 7288 | T |
| CEP152 | K368N | C | 1224 | A | CLDND1 | Q249* | G | 956 | A | COL4A3 | A1439V | C | 4478 | T | CSMD3 | R1574Q | C | 4966 | T |
| CEP152 | K280N | C | 960 | A | CLDND1 | R27C | G | 290 | A | COL4A3 | Y1521C | A | 4724 | G | CSMD3 | A1527V | G | 4825 | A |
| CEP164 | D404N | G | 1357 | A | CLDND1 | K128T | T | 594 | G | COL4A3BP | R641Q | C | 2216 | T | CSMD3 | R2180W | G | 6783 | A |
| CEP164 | F408L | C | 1371 | A | CLEC12A | R87I | G | 448 | T | COL4A3BP | R728* | G | 2476 | A | CSMD3 | P565L | G | 1939 | A |
| CEP164 | R1361C | C | 4228 | T | CLEC12A | N176T | A | 655 | C | COL4A3BP | K498N | C | 1788 | A | CSMD3 | N3574D | T | 1096 5 | C |
| CEP164 | K794T | A | 2528 | C | CLEC12A | S90Y | C | 457 | A | COL4A4 | G843V | C | 2736 | A | CSMD3 | G1785R | C | 5598 | T |
| CEP192 | S116N | G | 427 | A | CLEC12A | L117R | T | 478 | G | COL4A4 | E964G | T | 3099 | C | CSMD3 | P971T | G | 3156 | T |
| CEP192 | I2517S | T | 7630 | G | CLEC14A | R434M | C | 1648 | A | COL4A4 | A1567V | G | 4908 | A | CSMD3 | I2520V | T | 7803 | C |

| | | | | | | | | | | | | | | | | | | | |
|---|---|---|---|---|---|---|---|---|---|---|---|---|---|---|---|---|---|---|---|
| CEP192 | H194N | C | 660 | A | CLEC16A | R817H | G | 2680 | A | COL4A5 | - | A | 0 | G | CSMD3 | F2502L | G | 7751 | T |
| CEP192 | D53N | G | 237 | A | CLEC16A | A1042V | C | 3355 | T | COL4A5 | T1511M | C | 4776 | T | CSMD3 | I2186L | T | 6801 | G |
| CEP192 | P397L | C | 1270 | T | CLEC16A | T998A | A | 3222 | G | COL4A6 | G1262R | C | 3885 | T | CSMD3 | T2176I | G | 6772 | A |
| CEP192 | Q2108H | G | 6404 | T | CLEC16A | A791T | G | 2601 | A | COL4A6 | T1671M | G | 5113 | A | CSMD3 | S1952P | A | 6099 | G |
| CEP192 | R2118H | G | 6433 | A | CLEC16A | K75N | G | 455 | T | COL4A6 | A891V | G | 2773 | A | CSMD3 | F1418V | A | 4497 | C |
| CEP192 | S645A | T | 2013 | G | CLEC16A | F751C | T | 2482 | G | COL4A6 | P761L | G | 2383 | A | CSMD3 | Q287K | G | 1104 | T |
| CEP192 | T1655M | C | 5044 | T | CLEC17A | E48D | G | 182 | T | COL4A6 | S394* | G | 1282 | T | CSMD3 | K148T | T | 688 | G |
| CEP192 | I2055S | T | 6244 | G | CLEC17A | R213Q | G | 676 | A | COL4A6 | A355V | G | 1165 | A | CSMD3 | E113* | C | 582 | A |
| CEP250 | R399C | C | 1915 | T | CLEC1A | D280A | T | 902 | G | COL5A1 | G631V | G | 2306 | T | CSMD3 | T1173S | T | 3762 | A |
| CEP250 | R2032* | C | 6814 | T | CLEC1B | R15W | G | 243 | A | COL5A1 | D418N | G | 1666 | A | CSN3 | T151M | C | 538 | T |
| CEP250 | A800T | G | 3118 | A | CLEC2A | E100* | C | 350 | A | COL5A1 | P463L | C | 1802 | T | CSN3 | V155A | T | 550 | C |
| CEP250 | R121* | C | 1081 | T | CLEC2B | Q43H | T | 786 | G | COL5A1 | P290H | C | 1283 | A | CSNK1A1 | Y320C | T | 959 | C |
| CEP250 | S2331Y | C | 7712 | A | CLEC2B | N19D | T | 712 | C | COL5A1 | G1066V | G | 3611 | T | CSNK1D | K294N | T | 1198 | G |
| CEP290 | L1331V | A | 3991 | C | CLEC2D | R84I | G | 273 | T | COL5A1 | G1495S | G | 4897 | A | CSNK1E | T334M | G | 1112 | A |
| CEP290 | T835I | G | 2504 | A | CLEC2D | R37C | C | 131 | T | COL5A1 | R449L | G | 1760 | T | CSNK1G2 | R309W | C | 1447 | T |
| CEP290 | K918N | C | 2754 | A | CLEC2D | N19T | A | 78 | C | COL5A2 | R821* | G | 2736 | A | CSNK1G2 | R263W | C | 1309 | T |
| CEP290 | S722Y | G | 2165 | T | CLEC2L | E145A | A | 434 | C | COL5A2 | A394V | G | 1456 | A | CSNK1G2 | R263W | C | 1309 | T |
| CEP290 | K170N | C | 510 | A | CLEC2L | I87F | A | 259 | T | COL5A2 | P686T | G | 2331 | T | CSNK1G2 | L411P | T | 1754 | C |
| CEP290 | R151Q | C | 452 | T | CLEC2L | A80T | G | 238 | A | COL5A2 | E1066* | C | 3471 | A | CSNK1G3 | A189V | C | 1285 | T |
| CEP290 | E9* | C | 25 | A | CLEC2L | E145K | G | 433 | A | COL5A2 | G1023D | C | 3343 | T | CSNK1G3 | K263N | A | 1508 | C |
| CEP290 | V758A | A | 2273 | G | CLEC3A | D127Y | G | 491 | T | COL5A2 | A331T | C | 1266 | T | CSNK2A2 | M209I | C | 769 | T |
| CEP350 | R427Q | G | 1698 | A | CLEC3A | R156C | C | 578 | T | COL5A3 | K266N | C | 884 | A | CSNK2A2 | R44Q | C | 273 | T |
| CEP350 | P986S | C | 3374 | T | CLEC3A | R156C | C | 578 | T | COL5A3 | V1201M | C | 3687 | T | CSPG4 | R672* | G | 2107 | A |
| CEP350 | Y1522C | A | 4983 | G | CLEC4C | A181V | G | 734 | A | COL5A3 | P1294L | G | 3967 | A | CSPG4 | G624S | C | 1963 | T |
| CEP350 | R1728Q | G | 5601 | A | CLEC4C | F46V | A | 328 | C | COL5A3 | A1096T | C | 3372 | T | CSPG4 | L1674I | G | 5113 | T |
| CEP350 | R1881C | C | 6059 | T | CLEC4D | S8N | G | 216 | A | COL5A3 | G1187S | C | 3645 | T | CSPG4 | T2184M | G | 6644 | A |
| CEP350 | A202S | G | 1022 | T | CLEC4F | A315V | G | 1021 | A | COL5A3 | G701V | C | 2188 | A | CSPG4 | A2288G | G | 6956 | C |
| CEP350 | S1157* | C | 3888 | A | CLEC4F | R258S | T | 851 | G | COL5A3 | D741V | T | 2308 | A | CSPG4 | W1668R | A | 5095 | G |
| CEP350 | R1204C | C | 4028 | T | CLEC4F | - | C | 0 | T | COL5A3 | E1708K | C | 5208 | T | CSPG5 | A338T | C | 3111 | T |
| CEP350 | F2548V | T | 8060 | G | CLEC4F | T392S | G | 1252 | C | COL5A3 | - | C | 0 | A | CSPG5 | G394V | C | 3280 | A |
| CEP350 | R1482W | C | 4862 | T | CLEC4F | N144S | T | 508 | C | COL6A1 | R731H | G | 2306 | A | CSPG5 | S428L | G | 3382 | A |
| CEP350 | R3003S | A | 9427 | C | CLEC4G | A227T | C | 748 | T | COL6A1 | F762L | C | 2400 | A | CSPP1 | R413* | C | 1268 | T |
| CEP55 | R385* | C | 1335 | T | CLEC5A | A162V | G | 682 | A | COL6A1 | A132T | G | 508 | A | CSPP1 | I276V | A | 857 | G |
| CEP55 | K29Q | A | 267 | C | CLEC6A | M2I | G | 123 | A | COL6A1 | K121E | A | 475 | G | CSPP1 | V1117I | G | 3380 | A |
| CEP55 | R64* | C | 372 | T | CLEC7A | E242D | C | 913 | A | COL6A1 | G404R | G | 1324 | A | CSPP1 | N147H | A | 470 | C |
| CEP57 | R81* | C | 462 | T | CLEC9A | S110N | G | 942 | A | COL6A1 | R211H | G | 746 | A | CSPP1 | K207E | A | 650 | G |
| CEP57 | K345T | A | 1255 | C | CLEC9A | E139D | G | 1030 | T | COL6A1 | A224T | G | 784 | A | CSRNP1 | R122H | C | 543 | T |
| CEP63 | E691K | G | 2244 | A | CLGN | V248I | C | 1183 | T | COL6A1 | G556R | G | 1780 | A | CSRNP1 | S432T | C | 1473 | G |
| CEP68 | Q618* | C | 2055 | T | CLGN | E266* | C | 1237 | A | COL6A1 | - | G | 0 | A | CSRNP1 | K8T | T | 201 | G |

| | | | | | | | | | | | | | | | | | | | |
|---|---|---|---|---|---|---|---|---|---|---|---|---|---|---|---|---|---|---|---|
| CEP68 | P103S | C | 510 | T | CLGN | F408S | A | 1664 | G | COL6A1 | R79C | C | 349 | T | CSRNP2 | G388D | C | 1461 | T |
| CEP68 | R441W | C | 1524 | T | CLIC2 | L79F | C | 456 | A | COL6A1 | C810G | T | 2542 | G | CSRNP2 | R184W | G | 848 | A |
| CEP68 | D525G | A | 1777 | G | CLIC4 | R206H | G | 814 | A | COL6A2 | D330N | G | 1092 | A | CSRNP2 | T5M | G | 312 | A |
| CEP70 | - | A | 0 | G | CLIC4 | A64V | C | 388 | T | COL6A2 | A137T | G | 513 | A | CSRNP2 | F49L | G | 445 | T |
| CEP72 | L633R | T | 1988 | G | CLIC5 | R196C | G | 739 | A | COL6A2 | R734C | C | 2304 | T | CSRNP3 | S593Y | C | 2028 | A |
| CEP76 | R148H | C | 669 | T | CLIC6 | K594N | G | 1782 | T | COL6A2 | R784C | C | 2454 | T | CSRP2 | A188T | C | 645 | T |
| CEP78 | G52V | G | 431 | T | CLINT1 | K131N | C | 583 | A | COL6A3 | R1727W | G | 5632 | A | CSRP3 | G135R | C | 520 | T |
| CEP97 | N229D | A | 719 | G | CLINT1 | A400V | G | 1389 | A | COL6A3 | V1924I | C | 6223 | T | CST1 | R46H | C | 208 | T |
| CER1 | E174K | C | 565 | T | CLINT1 | D185V | T | 744 | A | COL6A3 | R1738H | C | 5666 | T | CST1 | Y64C | T | 262 | C |
| CER1 | H208R | T | 668 | C | CLINT1 | A267V | G | 990 | A | COL6A3 | P744L | G | 2684 | A | CST11 | L109P | A | 360 | G |
| CERCAM | S380L | C | 1537 | T | CLIP1 | S47L | G | 295 | A | COL6A3 | G139S | C | 868 | T | CST2 | V78M | C | 303 | T |
| CERCAM | E454G | A | 1759 | G | CLIP1 | R511H | C | 1687 | T | COL6A3 | G615C | C | 2296 | A | CST2 | R66C | G | 267 | A |
| CERCAM | G567S | G | 2097 | A | CLIP1 | K35N | T | 260 | G | COL6A3 | V2793I | C | 8830 | T | CST2 | F84L | G | 323 | T |
| CERK | D186N | C | 669 | T | CLIP2 | A583V | C | 2075 | T | COL6A3 | A1370T | C | 4561 | T | CST6 | A119V | C | 410 | T |
| CERK | A375T | C | 1236 | T | CLIP2 | C249Y | G | 1073 | A | COL6A3 | R2342Q | C | 7478 | T | CST6 | D109N | G | 379 | A |
| CERK | E202K | C | 717 | T | CLIP2 | E933G | A | 3125 | G | COL6A3 | K2916E | T | 9199 | C | CST7 | R20Q | G | 269 | A |
| CERKL | E91* | C | 271 | A | CLIP2 | A869V | C | 2933 | T | COL6A3 | G2705E | C | 8567 | T | CST8 | K25N | A | 432 | C |
| CES1 | W176* | C | 635 | T | CLIP2 | R515C | C | 1870 | T | COL6A3 | - | C | 0 | A | CST9L | F87L | G | 560 | T |
| CES1 | D420E | G | 1368 | T | CLIP2 | Q775H | G | 2652 | T | COL6A3 | - | C | 0 | T | CST9L | K140N | C | 719 | A |
| CES1 | D91Y | C | 379 | A | CLIP3 | T276M | G | 1055 | A | COL6A3 | G2190V | C | 7022 | A | CSTF1 | R426Q | G | 1477 | A |
| CES1 | A81V | G | 350 | A | CLIP3 | A271T | C | 1039 | T | COL6A3 | D1958Y | C | 6325 | A | CSTF2 | A482V | C | 1461 | T |
| CES2 | R136Q | G | 1391 | A | CLIP3 | R86C | G | 484 | A | COL6A3 | R1464Q | C | 4844 | T | CSTF2T | Q524H | T | 1618 | G |
| CES3 | L527M | C | 1650 | A | CLIP4 | D50Y | G | 387 | T | COL6A3 | K1156N | C | 3921 | A | CSTF3 | A42V | G | 291 | A |
| CES7 | R497Q | C | 1610 | T | CLIP4 | M277R | T | 1069 | G | COL6A3 | R874Q | C | 3074 | T | CT45A5 | D75Y | C | 468 | A |
| CES7 | L566F | G | 1816 | A | CLIP4 | V31A | T | 331 | C | COL6A3 | D598N | C | 2245 | T | CTAGE1 | E98* | C | 396 | A |
| CES7 | R486Q | C | 1577 | T | CLIP4 | P458H | C | 1612 | A | COL6A3 | R1504W | G | 4963 | A | CTAGE1 | R173W | G | 621 | A |
| CES7 | R530I | C | 1709 | A | CLIP4 | K394N | A | 1421 | C | COL6A5 | R2471Q | G | 7906 | A | CTAGE1 | A651T | C | 2055 | T |
| CES7 | - | C | 0 | A | CLIP4 | E410K | G | 1467 | A | COL6A5 | V1864M | G | 6084 | A | CTAGE5 | D148V | A | 779 | T |
| CES8 | F175L | C | 525 | A | CLIP4 | R497K | G | 1729 | A | COL6A5 | D76N | G | 720 | A | CTAGE5 | T291M | C | 1208 | T |
| CES8 | A500T | G | 1498 | A | CLK1 | R458* | G | 1707 | A | COL6A5 | D218N | G | 1146 | A | CTAGE5 | E504* | G | 1846 | T |
| CETN1 | L137I | C | 451 | A | CLK1 | W272R | A | 1149 | G | COL6A5 | R252M | G | 1249 | T | CTAGE9 | D33N | C | 97 | T |
| CETN2 | E99D | C | 364 | A | CLK2 | Q475R | T | 1740 | C | COL6A5 | S386Y | C | 1651 | A | CTBP1 | R173H | C | 700 | T |
| CETP | L313P | T | 1068 | C | CLK2 | S131T | A | 707 | T | COL6A5 | R884C | C | 3144 | T | CTBP1 | R36Q | C | 289 | T |
| CETP | V239I | G | 845 | A | CLK2 | N295I | T | 1200 | A | COL6A5 | P1632S | C | 5388 | T | CTBP2 | R697Q | C | 2221 | T |
| CETP | R54* | C | 290 | T | CLK2 | P401T | G | 1517 | T | COL6A5 | A1891V | C | 6166 | T | CTBP2 | R513Q | C | 1669 | T |
| CETP | L245P | T | 864 | C | CLK2 | R268H | C | 1119 | T | COL6A5 | S1971Y | C | 6406 | A | CTBP2 | C349S | A | 1176 | T |
| CETP | A212V | C | 765 | T | CLK2 | A446T | C | 1652 | T | COL6A5 | A2312T | G | 7428 | A | CTBP2 | A446T | C | 1467 | T |
| CFD | A15V | C | 69 | T | CLK2 | F272C | A | 1131 | C | COL6A6 | R1173C | C | 3548 | T | CTBP2 | Y76H | A | 357 | G |
| CFD | D141N | G | 446 | A | CLK4 | R127H | C | 516 | T | COL6A6 | A1818T | G | 5483 | A | CTBP2 | T42M | G | 256 | A |

| | | | | | | | | | | | | | | | | | | | |
|---|---|---|---|---|---|---|---|---|---|---|---|---|---|---|---|---|---|---|---|
| CFDP1 | A93V | G | 411 | A | CLK4 | R24H | C | 207 | T | COL6A6 | H1746R | A | 5268 | G | CTBS | H326R | T | 1026 | C |
| CFH | R257H | G | 1010 | A | CLK4 | V191I | C | 707 | T | COL6A6 | T424M | C | 1302 | T | CTBS | M92T | A | 324 | G |
| CFH | G35D | G | 344 | A | CLK4 | E369D | T | 1243 | G | COL6A6 | K869N | A | 2638 | C | CTCF | K405E | A | 1657 | G |
| CFH | G894V | G | 2921 | T | CLLU1OS | M101I | C | 305 | A | COL6A6 | S460L | C | 1410 | T | CTCF | D693N | G | 2521 | A |
| CFH | R175Q | G | 764 | A | CLMN | A814T | C | 2556 | T | COL6A6 | R786C | C | 2387 | T | CTCF | R377C | C | 1573 | T |
| CFH | S933Y | C | 3038 | A | CLMN | N849T | T | 2662 | G | COL6A6 | F320L | T | 989 | C | CTCFL | A233T | C | 890 | T |
| CFH | I8S | T | 263 | G | CLMN | D766A | T | 2413 | G | COL6A6 | R1739* | C | 5246 | T | CTDP1 | Q736H | G | 2355 | T |
| CFH | L12V | T | 274 | G | CLN3 | L113M | A | 610 | T | COL6A6 | Q1072* | C | 3245 | T | CTDP1 | - | G | 0 | T |
| CFH | G45D | G | 374 | A | CLN5 | M1I | G | 1295 | A | COL6A6 | R1745M | G | 5265 | T | CTDSP2 | R152H | C | 984 | T |
| CFH | S58Y | C | 413 | A | CLN6 | R110C | G | 486 | A | COL6A6 | D451Y | G | 1382 | T | CTDSP2 | A241T | C | 1250 | T |
| CFH | I685T | T | 2294 | C | CLN8 | A84V | C | 490 | T | COL7A1 | R1772Q | C | 5423 | T | CTH | R265* | C | 937 | T |
| CFHR1 | R302W | C | 992 | T | CLNK | E281K | C | 1081 | T | COL7A1 | A1232S | C | 3802 | A | CTH | A357V | C | 1214 | T |
| CFHR1 | R309H | G | 1014 | A | CLNK | D16N | C | 286 | T | COL7A1 | P367S | G | 1207 | A | CTHRC1 | C126R | T | 518 | C |
| CFHR1 | E37D | A | 199 | C | CLOCK | F480V | A | 1689 | C | COL7A1 | G1353R | C | 4165 | T | CTHRC1 | P69H | C | 348 | A |
| CFHR1 | R69C | C | 293 | T | CLOCK | K712T | T | 2386 | G | COL7A1 | A18V | G | 161 | A | CTLA4 | I102T | T | 462 | C |
| CFHR2 | R132W | C | 471 | T | CLP1 | R170Q | G | 648 | A | COL7A1 | R2745Q | C | 8342 | T | CTNNA1 | R379H | G | 1136 | A |
| CFHR2 | P41S | C | 198 | T | CLPB | R520C | G | 1608 | A | COL7A1 | P1753T | G | 5365 | T | CTNNA1 | M726V | A | 2176 | G |
| CFHR4 | N310K | T | 999 | A | CLPB | P644S | G | 1980 | A | COL7A1 | R2795C | G | 8491 | A | CTNNA1 | T654M | C | 1961 | T |
| CFHR4 | A309V | C | 995 | T | CLPP | R83C | C | 370 | T | COL7A1 | G1374A | C | 4229 | G | CTNNA2 | A252T | G | 759 | A |
| CFHR4 | V313A | T | 1007 | C | CLPTM1 | R90H | G | 274 | A | COL7A1 | K2562N | C | 7794 | A | CTNNA2 | A586T | G | 1761 | A |
| CFHR5 | Y63* | T | 226 | G | CLPTM1 | R74H | G | 226 | A | COL7A1 | G1988V | C | 6071 | A | CTNNA2 | F609L | C | 1832 | A |
| CFI | S553R | T | 1737 | G | CLPTM1L | R221C | G | 919 | A | COL8A1 | P296H | C | 1132 | A | CTNNA2 | R328C | C | 987 | T |
| CFI | D211Y | C | 711 | A | CLPTM1L | T490M | G | 1727 | A | COL8A1 | V110L | G | 573 | T | CTNNA3 | F718S | A | 2328 | G |
| CFL1 | R21H | C | 296 | T | CLPTM1L | R533W | G | 1855 | A | COL8A1 | G607C | G | 2064 | T | CTNNA3 | Q376* | G | 1301 | A |
| CFP | R359* | G | 1196 | A | CLPTM1L | A353V | G | 1316 | A | COL8A2 | G618S | C | 1859 | T | CTNNB1 | S45P | T | 289 | C |
| CFP | R438Q | C | 1434 | T | CLRN1 | Y171* | A | 804 | C | COL8A2 | Y599C | T | 1803 | C | CTNNB1 | R225C | C | 829 | T |
| CFTR | G1185D | G | 3686 | A | CLRN2 | A13V | C | 38 | T | COL9A1 | P271L | G | 958 | A | CTNNB1 | T41A | A | 277 | G |
| CFTR | I331T | T | 1124 | C | CLRN2 | A207V | C | 620 | T | COL9A2 | G241V | C | 819 | A | CTNNB1 | G730S | G | 2344 | A |
| CFTR | R1097C | C | 3421 | T | CLRN3 | - | C | 0 | T | COL9A3 | P562H | C | 1688 | A | CTNNB1 | Q302H | A | 1062 | C |
| CFTR | K698E | A | 2224 | G | CLRN3 | V129M | C | 548 | T | COL9A3 | R402Q | G | 1208 | A | CTNNB1 | E571* | G | 1867 | T |
| CFTR | K166M | A | 629 | T | CLSPN | K287Q | T | 939 | G | COL9A3 | G346D | G | 1040 | A | CTNNBL1 | R485C | C | 1570 | T |
| CFTR | R555S | A | 1797 | C | CLSPN | V1250A | A | 3829 | G | COL9A3 | R423Q | G | 1271 | A | CTNNBL1 | - | T | 0 | C |
| CFTR | D1152A | A | 3587 | C | CLSPN | - | A | 0 | G | COLEC10 | D132N | G | 469 | A | CTNNBL1 | - | G | 0 | T |
| CGA | V92A | A | 376 | G | CLSPN | A1215T | C | 3723 | T | COLEC11 | V257M | G | 769 | A | CTNNBL1 | E223A | A | 785 | C |
| CGGBP1 | P85S | G | 715 | A | CLSPN | R815* | G | 2523 | A | COLEC11 | A213T | G | 637 | A | CTNNBL1 | R38C | C | 229 | T |
| CGN | R267H | G | 933 | A | CLSPN | R795C | G | 2463 | A | COLEC11 | A12V | C | 35 | T | CTNND1 | R344* | C | 1601 | T |
| CGN | R991W | C | 3104 | T | CLSPN | D1323Y | C | 4047 | A | COLEC12 | A270V | G | 1024 | A | CTNND1 | M298V | A | 1463 | G |
| CGN | E527K | G | 1712 | A | CLSTN1 | M819T | A | 3249 | G | COLEC12 | G542C | C | 1839 | A | CTNND1 | R215H | G | 1215 | A |
| CGNL1 | R45Q | G | 212 | A | CLSTN1 | S436F | G | 2100 | A | COLEC12 | A233T | C | 912 | T | CTNND1 | H273Y | C | 1388 | T |

| | | | | | | | | | | | | | | | | | | | |
|---|---|---|---|---|---|---|---|---|---|---|---|---|---|---|---|---|---|---|---|
| CGREF1 | G393E | C | 1183 | T | CLSTN2 | S780R | A | 2528 | C | COLEC12 | Q172H | C | 731 | A | CTNND1 | A683D | C | 2619 | A |
| CGREF1 | G376E | C | 1132 | T | CLSTN2 | R777C | C | 2519 | T | COLQ | G243V | C | 854 | A | CTNND1 | K399N | G | 1768 | T |
| CGREF1 | L135P | A | 409 | G | CLSTN2 | A880V | C | 2829 | T | COLQ | P140S | G | 544 | A | CTNND1 | A373T | G | 1688 | A |
| CHAC1 | A241T | G | 1030 | A | CLSTN2 | V828L | G | 2672 | T | COMMD1 | - | T | 603 | C | CTNND2 | V660G | A | 2169 | C |
| CHAC2 | L148R | T | 538 | G | CLSTN2 | E505D | A | 1705 | C | COMMD2 | T112M | G | 390 | A | CTNND2 | R82* | G | 434 | A |
| CHAD | A166T | C | 649 | T | CLSTN2 | R428W | C | 1472 | T | COMMD3 | V45A | T | 578 | C | CTNND2 | R889Q | C | 2856 | T |
| CHAD | G97D | C | 443 | T | CLSTN3 | R947H | G | 3118 | A | COMMD6 | F86V | A | 394 | C | CTNND2 | A615S | C | 2033 | A |
| CHAD | D261E | A | 936 | C | CLSTN3 | E39D | G | 395 | T | COMMD7 | A82D | G | 851 | T | CTNS | R114C | C | 799 | T |
| CHADL | R518H | C | 1606 | T | CLSTN3 | E950K | G | 3126 | A | COMMD7 | Q115H | C | 951 | A | CTNS | A212V | C | 1094 | T |
| CHADL | R107H | C | 373 | T | CLTC | M1538I | G | 4888 | A | COMP | V487L | C | 1495 | A | CTNS | V163M | G | 946 | A |
| CHADL | R582* | G | 1797 | A | CLTC | Q1083R | A | 3522 | G | COMP | L9F | G | 61 | A | CTPS | R389W | C | 1673 | T |
| CHADL | A175T | C | 576 | T | CLTC | F368C | T | 1377 | G | COPA | D399G | T | 1274 | C | CTPS | V34A | T | 609 | C |
| CHAF1A | R319H | G | 1056 | A | CLTC | N1469T | A | 4680 | C | COPB2 | Q687H | C | 2243 | A | CTPS2 | K193N | T | 1323 | G |
| CHAF1A | E388K | G | 1262 | A | CLU | R279H | C | 909 | T | COPB2 | I295N | A | 1066 | T | CTR9 | K982Q | A | 3101 | C |
| CHAF1A | V917M | G | 2849 | A | CLU | N479T | T | 1509 | G | COPB2 | R17* | G | 231 | A | CTR9 | A48V | C | 300 | T |
| CHAF1A | R528W | C | 1682 | T | CLU | F482C | A | 1518 | C | COPG | - | C | 0 | A | CTRC | G87* | G | 285 | T |
| CHAF1B | T432M | C | 1446 | T | CLUAP1 | S367P | T | 1204 | C | COPG | V579M | G | 1839 | A | CTSA | R344C | C | 1312 | T |
| CHAF1B | C191G | T | 722 | G | CLUL1 | I436T | T | 1452 | C | COPG | I383T | T | 1252 | C | CTSA | G76S | G | 508 | A |
| CHAF1B | Y225D | T | 824 | G | CLVS2 | - | G | 0 | T | COPG | F498L | T | 1596 | C | CTSA | I465L | A | 1675 | C |
| CHAT | R186W | C | 709 | T | CLYBL | D73N | G | 244 | A | COPG | R247C | C | 843 | T | CTSB | I184V | T | 804 | C |
| CHCHD1 | Q81H | G | 256 | T | CLYBL | I29V | A | 112 | G | COPG2 | R238H | C | 713 | T | CTSB | S257L | G | 1024 | A |
| CHCHD1 | P101S | C | 314 | T | CLYBL | D68E | T | 231 | G | COPG2 | L223V | G | 667 | C | CTSC | K227N | C | 782 | A |
| CHCHD2 | A67V | G | 363 | A | CMAS | N373H | A | 1196 | C | COPS2 | P300Q | G | 978 | T | CTSC | L381H | A | 1243 | T |
| CHCHD6 | R162C | C | 556 | T | CMAS | E426K | G | 1355 | A | COPS2 | A234V | G | 780 | A | CTSC | V348A | A | 1144 | G |
| CHCHD6 | E169Q | G | 577 | C | CMKLR1 | R151C | G | 815 | A | COPS3 | T311A | T | 1038 | C | CTSC | R250Q | C | 850 | T |
| CHCHD7 | M108T | T | 494 | C | CMPK1 | Y61* | C | 213 | A | COPS3 | V164M | C | 597 | T | CTSE | F246L | C | 856 | A |
| CHD1 | R1189Q | C | 3716 | T | CMPK2 | Q191R | T | 572 | C | COPS6 | R315* | C | 980 | T | CTSG | R120Q | C | 396 | T |
| CHD1 | R900Q | C | 2849 | T | CMTM1 | K80T | A | 306 | C | COPS6 | M206I | G | 655 | T | CTSG | R122* | G | 401 | A |
| CHD1 | D1388N | C | 4312 | T | CMTM3 | Y59F | A | 702 | T | COPS7A | R151W | C | 628 | T | CTSL1 | V126M | G | 1266 | A |
| CHD1 | R697* | G | 2239 | A | CMTM4 | R191H | C | 754 | T | COPS8 | P185S | C | 1206 | T | CTSL1 | R153W | C | 1347 | T |
| CHD1 | P450H | G | 1499 | T | CMTM5 | R8Q | G | 239 | A | COQ10A | S129P | T | 385 | C | CTSL2 | V282A | A | 1095 | G |
| CHD1 | F1414Y | A | 4391 | T | CMYA5 | V3613A | T | 10910 | C | COQ10B | A29T | G | 223 | A | CTSL2 | K54N | C | 412 | A |
| CHD1 | R1574I | C | 4871 | A | CMYA5 | A3703D | C | 11180 | A | COQ2 | A267V | G | 800 | A | CTSO | I244M | T | 802 | C |
| CHD1L | A668T | G | 2022 | A | CMYA5 | T2928A | A | 8854 | G | COQ3 | C239R | A | 740 | G | CTSS | A164T | C | 751 | T |
| CHD1L | R319Q | G | 976 | A | CMYA5 | E409D | G | 1299 | T | COQ6 | P373L | C | 1198 | T | CTSW | V301I | G | 943 | A |
| CHD1L | R457C | C | 1389 | T | CMYA5 | S589Y | C | 1838 | A | COQ6 | D146Y | G | 516 | T | CTSW | R62H | G | 227 | A |
| CHD1L | K519N | A | 1577 | T | CMYA5 | S771Y | C | 2384 | A | COQ9 | P214L | C | 657 | T | CTTN | A321T | G | 1167 | A |
| CHD1L | R806H | G | 2437 | A | CMYA5 | S1667Y | C | 5072 | A | CORIN | P1017H | G | 3050 | T | CTTN | T364M | C | 1297 | T |

| | | | | | | | | | | | | | | | | | | | |
|---|---|---|---|---|---|---|---|---|---|---|---|---|---|---|---|---|---|---|---|
| CHD2 | R1546* | C | 5343 | T | CMYA5 | I2441M | T | 7395 | G | CORIN | K780N | T | 2340 | G | CTTNBP2 | A1069T | C | 3297 | T |
| CHD2 | S1221F | C | 4369 | T | CMYA5 | E3590* | G | 10840 | T | CORO1B | R30H | C | 193 | T | CTTNBP2 | T1481A | T | 4533 | C |
| CHD2 | I1223F | A | 4374 | T | CMYA5 | S3692L | C | 11147 | T | CORO1B | - | A | 0 | G | CTTNBP2 | N79I | T | 328 | A |
| CHD2 | R403Q | G | 1915 | A | CMYA5 | E3796* | G | 11458 | T | CORO1B | R70H | C | 313 | T | CTTNBP2 | I259M | T | 869 | C |
| CHD2 | - | T | 0 | C | CMYA5 | L3901I | C | 11773 | A | CORO1C | A424V | G | 1271 | A | CTTNBP2 | V1361I | C | 4173 | T |
| CHD3 | G157R | G | 470 | A | CNBD1 | I263S | T | 788 | G | CORO2B | R113Q | G | 632 | A | CTTNBP2 | R1271W | G | 3903 | A |
| CHD3 | R1960C | C | 5879 | T | CNDP1 | S282C | A | 1072 | T | CORO6 | V280M | C | 839 | T | CTTNBP2 | K244N | C | 824 | A |
| CHD3 | R1744* | C | 5231 | T | CNDP1 | R401I | G | 1430 | T | CORO7 | A146T | C | 538 | T | CTTNBP2 | A86T | C | 348 | T |
| CHD3 | F847S | T | 2541 | C | CNDP2 | P136L | C | 723 | T | CORO7 | - | A | 0 | G | CTTNBP2 | A869V | G | 2698 | A |
| CHD3 | P2013H | C | 6039 | A | CNDP2 | A146V | C | 753 | T | CORT | R80* | C | 593 | T | CTTNBP2 | R1219C | G | 3747 | A |
| CHD3 | E511D | G | 1534 | T | CNDP2 | E40K | G | 434 | A | COTL1 | S115R | T | 506 | G | CTTNBP2NL | L18I | C | 277 | A |
| CHD3 | L1200M | C | 3599 | A | CNGA1 | R547H | C | 1781 | T | COX10 | R142Q | G | 502 | A | CTTNBP2NL | E245K | G | 958 | A |
| CHD3 | F1063V | T | 3188 | G | CNGA1 | P364L | G | 1232 | A | COX10 | A199S | G | 672 | T | CTU1 | R72H | C | 280 | T |
| CHD4 | R1338I | C | 4177 | A | CNGA1 | R712Q | C | 2276 | T | COX15 | P310T | G | 979 | T | CTU1 | T114M | G | 406 | A |
| CHD4 | G776D | C | 2491 | T | CNGA2 | S530T | G | 1622 | C | COX15 | V49A | A | 197 | G | CTU2 | V38L | G | 160 | C |
| CHD4 | - | C | 0 | A | CNGA2 | E427K | G | 1312 | A | COX16 | R85M | C | 398 | A | CUBN | L3216I | G | 9712 | T |
| CHD4 | W421L | C | 1426 | A | CNGA2 | A80S | G | 271 | T | COX18 | G84D | C | 343 | T | CUBN | H1089Y | G | 3331 | A |
| CHD4 | K1718T | T | 5317 | G | CNGA2 | T5N | C | 47 | A | COX19 | A4D | G | 101 | T | CUBN | D1055V | T | 3230 | A |
| CHD4 | K1051R | T | 3316 | C | CNGA3 | A162V | C | 1055 | T | COX19 | K53E | T | 247 | C | CUBN | R1921H | C | 5828 | T |
| CHD4 | S781N | C | 2506 | T | CNGA3 | G548C | G | 2212 | T | COX7A2 | E72D | T | 527 | G | CUBN | N1280I | T | 3905 | A |
| CHD5 | D81Y | C | 341 | A | CNGA3 | R21K | G | 632 | A | COX7A2L | E33* | C | 927 | A | CUBN | S3173A | A | 9583 | C |
| CHD5 | V1875M | C | 5723 | T | CNGA4 | R496W | C | 1593 | T | COX8A | A16T | G | 120 | A | CUBN | N1646H | T | 5002 | G |
| CHD5 | E1664K | C | 5090 | T | CNGA4 | R143C | C | 534 | T | CP | Q711* | G | 2394 | A | CUBN | P1529L | G | 4652 | A |
| CHD5 | K98N | C | 394 | A | CNGB1 | A78V | G | 290 | A | CP | K712N | T | 2399 | G | CUBN | E938K | C | 2878 | T |
| CHD6 | L2434I | G | 7478 | T | CNGB1 | A1141V | G | 3479 | A | CP | D640Y | C | 2181 | A | CUBN | G3471E | C | 10478 | T |
| CHD6 | A345T | C | 1211 | T | CNGB1 | R729Q | C | 2243 | T | CP | R436Q | C | 1570 | T | CUBN | G818R | C | 2518 | T |
| CHD6 | R155Q | C | 642 | T | CNGB1 | P115L | G | 401 | A | CPA1 | R386C | C | 1306 | T | CUEDC1 | G92S | C | 688 | T |
| CHD6 | V1274A | A | 3999 | G | CNGB3 | R456C | G | 1412 | A | CPA1 | K194N | G | 732 | T | CUEDC2 | R264L | C | 937 | A |
| CHD6 | R1595C | G | 4961 | A | CNGB3 | P309Q | G | 972 | T | CPA2 | - | G | 0 | T | CUL2 | A516V | G | 1782 | A |
| CHD6 | R810C | G | 2606 | A | CNGB3 | - | C | 0 | G | CPA2 | A193T | G | 596 | A | CUL2 | S637L | G | 2145 | A |
| CHD6 | T2204A | T | 6788 | C | CNIH | V52A | A | 259 | G | CPA3 | K124N | G | 424 | T | CUL2 | K629N | C | 2122 | A |
| CHD6 | S2629Y | G | 8064 | T | CNIH | F130L | A | 494 | C | CPA3 | A177T | G | 581 | A | CUL2 | V184A | A | 786 | G |
| CHD6 | G1915* | C | 5921 | A | CNIH3 | S159N | G | 1358 | A | CPA3 | I39T | T | 168 | C | CUL2 | M566I | C | 1933 | A |
| CHD6 | F1499L | A | 4675 | C | CNIH3 | E71K | G | 1093 | A | CPA4 | T405M | C | 1242 | T | CUL3 | R305C | G | 1252 | A |

| | | | | | | | | | | | | | | | | | | | |
|---|---|---|---|---|---|---|---|---|---|---|---|---|---|---|---|---|---|---|---|
| CHD6 | I361M | A | 1261 | C | CNKSR1 | R147* | C | 478 | T | CPA4 | K20T | A | 87 | C | CUL3 | I145L | T | 772 | G |
| CHD7 | R1330W | C | 4465 | T | CNKSR2 | E83D | A | 285 | C | CPA4 | - | G | 0 | T | CUL4A | A335V | C | 1088 | T |
| CHD7 | A2391V | C | 7649 | T | CNKSR2 | R806Q | G | 2453 | A | CPA4 | S284L | C | 879 | T | CUL4A | F665C | T | 2078 | G |
| CHD7 | A2726S | G | 8653 | T | CNKSR2 | Q319H | G | 993 | T | CPA5 | R129C | C | 1005 | T | CUL4B | V183A | A | 950 | G |
| CHD7 | D1356G | A | 4544 | G | CNKSR2 | A556E | C | 1703 | A | CPA6 | K434N | C | 1558 | A | CUL4B | R365I | C | 1496 | A |
| CHD7 | P1987H | C | 6437 | A | CNN1 | S254L | C | 925 | T | CPA6 | V107A | A | 576 | G | CUL7 | R1019H | C | 3142 | T |
| CHD7 | R1608G | A | 5299 | G | CNNM1 | R714W | C | 2429 | T | CPAMD8 | E1155K | C | 3462 | T | CUL7 | T1449A | T | 4431 | C |
| CHD7 | R858Q | G | 3050 | A | CNNM1 | P841L | C | 2811 | T | CPAMD8 | R340H | C | 1018 | T | CUL7 | R960C | G | 2964 | A |
| CHD7 | F1482L | C | 4923 | A | CNNM1 | R586W | C | 2045 | T | CPAMD8 | Y499F | T | 1495 | A | CUL9 | R1139Q | G | 3491 | A |
| CHD8 | I1740V | T | 5218 | C | CNNM1 | R584W | C | 2039 | T | CPAMD8 | R616H | C | 1846 | T | CUL9 | R2066Q | G | 6272 | A |
| CHD8 | P2146S | G | 6436 | A | CNNM1 | N474S | A | 1710 | G | CPAMD8 | R1146Q | C | 3436 | T | CUL9 | G1560D | G | 4754 | A |
| CHD8 | D1735N | C | 5203 | T | CNNM1 | R210W | C | 917 | T | CPAMD8 | P490S | G | 1467 | A | CUL9 | R2168C | C | 6577 | T |
| CHD8 | D1058A | T | 3173 | G | CNNM2 | R488C | C | 1629 | T | CPAMD8 | T648M | G | 1942 | A | CUL9 | M1633V | A | 4972 | G |
| CHD8 | R1396C | G | 4186 | A | CNNM2 | D532G | A | 1762 | G | CPAMD8 | A272V | G | 814 | A | CUL9 | R1878C | C | 5707 | T |
| CHD8 | I805M | A | 2415 | C | CNNM2 | S489P | T | 1632 | C | CPAMD8 | S250N | C | 748 | T | CUL9 | M781L | A | 2416 | T |
| CHD9 | R1357Q | G | 4279 | A | CNNM2 | G461D | G | 1549 | A | CPB1 | R237H | G | 1044 | A | CUL9 | G386V | G | 1232 | T |
| CHD9 | R1533Q | G | 4807 | A | CNNM2 | V567I | G | 1866 | A | CPB1 | V335M | G | 1337 | A | CUL9 | L2420V | C | 7333 | G |
| CHD9 | R1794H | G | 5590 | A | CNNM3 | Y557H | T | 1697 | C | CPB2 | G196C | C | 603 | A | CUL9 | Q2208R | A | 6698 | G |
| CHD9 | K2205Q | A | 6822 | C | CNNM3 | T619M | C | 1884 | T | CPB2 | F201L | G | 620 | T | CUX1 | P1264L | C | 3811 | T |
| CHD9 | R2567I | G | 7909 | T | CNNM3 | Y646C | A | 1965 | G | CPD | G4D | G | 68 | A | CUX1 | D806G | A | 2437 | G |
| CHD9 | E1376K | G | 4335 | A | CNNM3 | R506Q | G | 1545 | A | CPE | A72T | G | 494 | A | CUX1 | S903R | A | 2727 | C |
| CHD9 | G372D | G | 1324 | A | CNNM4 | L150P | T | 547 | C | CPE | G375V | G | 1404 | T | CUX1 | A487V | C | 1483 | T |
| CHD9 | A1072T | G | 3423 | A | CNNM4 | A473T | G | 1515 | A | CPEB1 | K312N | C | 936 | A | CUX1 | A409V | C | 1246 | T |
| CHD9 | G2701V | G | 8311 | T | CNNM4 | G404S | G | 1308 | A | CPEB1 | E84V | T | 251 | A | CUX1 | A65V | C | 214 | T |
| CHD9 | G2857* | G | 8778 | T | CNOT1 | R1992Q | C | 6266 | T | CPEB2 | - | G | 0 | T | CUX2 | E220D | G | 813 | T |
| CHD9 | H314R | A | 1150 | G | CNOT1 | E843K | C | 2818 | T | CPEB3 | Y629C | T | 2059 | C | CUX2 | A757V | C | 2423 | T |
| CHD9 | E715D | A | 2354 | C | CNOT1 | K596T | T | 2078 | G | CPEB4 | R589* | C | 2313 | T | CUX2 | P1433S | C | 4450 | T |
| CHD9 | R2387* | C | 7368 | T | CNOT1 | R1189H | C | 3857 | T | CPLX4 | R74I | C | 408 | A | CUZD1 | Y601C | T | 2752 | C |
| CHDH | F481L | A | 1879 | G | CNOT1 | T209M | G | 917 | A | CPM | R337I | C | 1057 | A | CUZD1 | Y170C | T | 1459 | C |
| CHEK2 | M374T | A | 1197 | G | CNOT1 | L1946P | A | 6128 | G | CPM | F31Y | A | 139 | T | CWC15 | F209V | A | 748 | C |
| CHEK2 | P225S | G | 749 | A | CNOT1 | M673T | A | 2309 | G | CPN1 | R322W | G | 1216 | A | CWC22 | R128H | C | 683 | T |
| CHEK2 | V25I | C | 149 | T | CNOT1 | G919D | C | 3047 | T | CPN1 | D312N | C | 1186 | T | CWC22 | E404D | T | 1512 | G |
| CHEK2 | Q36* | G | 182 | A | CNOT1 | R1870H | C | 5900 | T | CPN1 | A241T | C | 973 | T | CWC25 | R34W | G | 253 | A |
| CHEK2 | E364* | C | 1166 | A | CNOT1 | Y1548H | A | 4933 | G | CPN2 | R278W | G | 922 | A | CWF19L1 | R399Q | C | 1283 | T |
| CHEK2 | E348G | T | 1119 | C | CNOT1 | Q1271L | T | 4103 | A | CPN2 | R316H | C | 1037 | T | CWF19L2 | P341H | G | 1045 | T |
| CHERP | H500Y | G | 1572 | A | CNOT1 | D362N | C | 1375 | T | CPN2 | V212A | A | 725 | G | CWF19L2 | K524T | T | 1594 | G |
| CHERP | P584S | G | 1824 | A | CNOT1 | - | C | 0 | T | CPNE1 | R129W | G | 520 | A | CX3CR1 | A383V | G | 1148 | A |
| CHERP | K256E | T | 840 | C | CNOT1 | Y594H | A | 2071 | G | CPNE1 | T505I | G | 1649 | A | CX3CR1 | T249M | G | 746 | A |
| CHERP | R523W | G | 1641 | A | CNOT1 | R1941* | G | 6112 | A | CPNE1 | N208S | T | 758 | C | CXADR | - | G | 0 | A |

| | | | | | | | | | | | | | | | | | | | |
|---|---|---|---|---|---|---|---|---|---|---|---|---|---|---|---|---|---|---|---|
| CHERP | S830L | G | 2563 | A | CNOT1 | R1640Q | C | 5210 | T | CPNE1 | K261N | C | 918 | A | CXCL12 | I79V | T | 279 | C |
| CHFR | V627L | C | 1943 | A | CNOT1 | N415H | T | 1534 | G | CPNE3 | L150V | C | 506 | G | CXCL14 | - | T | 517 | C |
| CHFR | F242L | A | 790 | C | CNOT1 | A224T | C | 961 | T | CPNE3 | N138T | A | 471 | C | CXCL16 | R169H | C | 1038 | T |
| CHGA | R456W | C | 1626 | T | CNOT10 | S342A | T | 1340 | G | CPNE5 | T65A | T | 818 | C | CXCL17 | G31C | C | 307 | A |
| CHGA | A180V | C | 799 | T | CNOT2 | F317S | T | 1529 | C | CPNE5 | F193L | G | 1204 | T | CXCL17 | P71S | G | 427 | A |
| CHGA | V411I | G | 1491 | A | CNOT2 | K435Q | A | 1882 | C | CPNE5 | R78C | G | 857 | A | CXCL17 | T66I | G | 413 | A |
| CHGB | A512D | C | 1739 | A | CNOT3 | S657L | C | 2286 | T | CPNE6 | R102Q | G | 551 | A | CXCL3 | N102K | G | 384 | T |
| CHGB | R178* | C | 736 | T | CNOT3 | S667L | C | 2316 | T | CPNE7 | G378D | G | 1263 | A | CXCL3 | I100M | T | 378 | C |
| CHGB | R202I | G | 809 | T | CNOT3 | N592D | A | 2090 | G | CPNE7 | R367M | G | 1230 | T | CXCL9 | R120H | C | 398 | T |
| CHGB | E450* | G | 1552 | T | CNOT3 | G304S | G | 1226 | A | CPNE7 | R27L | G | 210 | T | CXCR1 | P257H | G | 891 | T |
| CHI3L1 | R304C | G | 1036 | A | CNOT4 | R323Q | C | 1275 | T | CPNE7 | G8E | G | 153 | A | CXCR1 | D11N | C | 152 | T |
| CHI3L2 | Y336D | T | 1077 | G | CNOT4 | A220V | G | 966 | A | CPNE8 | R157S | C | 568 | A | CXCR1 | G324D | C | 1092 | T |
| CHIC1 | D47E | T | 218 | A | CNOT4 | R44Q | C | 438 | T | CPNE8 | G543* | C | 1724 | A | CXCR1 | N311H | T | 1052 | G |
| CHIC1 | P32S | C | 171 | T | CNOT6 | M192V | A | 923 | G | CPNE8 | K405N | T | 1312 | G | CXCR1 | R144H | C | 552 | T |
| CHIC2 | Y149C | T | 530 | C | CNOT6 | Y482C | A | 1794 | G | CPNE8 | E293D | T | 976 | G | CXCR2 | T237M | C | 1137 | T |
| CHID1 | Q100L | T | 299 | A | CNOT6 | R87C | C | 608 | T | CPNE8 | P412S | G | 1331 | A | CXCR2 | D94N | G | 707 | A |
| CHIT1 | A170S | C | 543 | A | CNOT6L | A313V | G | 938 | A | CPNE9 | F76L | C | 418 | A | CXCR2 | T116A | A | 773 | G |
| CHIT1 | Y346C | T | 1072 | C | CNOT6L | V363M | C | 1087 | T | CPO | W297L | G | 936 | T | CXCR2 | I169S | T | 933 | G |
| CHL1 | L1043R | T | 3770 | G | CNP | S82L | C | 389 | T | CPOX | R332W | G | 1213 | A | CXCR5 | V108M | G | 432 | A |
| CHL1 | V663F | G | 2629 | T | CNP | E266K | G | 940 | A | CPOX | G308C | C | 1141 | A | CXCR5 | A188T | G | 672 | A |
| CHL1 | A24V | C | 713 | T | CNPY3 | R121Q | G | 733 | A | CPOX | G189C | C | 784 | A | CXCR6 | A277T | G | 1215 | A |
| CHL1 | A725T | G | 2815 | A | CNPY3 | G67S | G | 570 | A | CPOX | L418S | A | 1472 | G | CXCR7 | W19* | G | 367 | A |
| CHL1 | R438H | G | 1955 | A | CNPY4 | F163S | T | 620 | C | CPPED1 | T105M | G | 425 | A | CXCR7 | R320S | C | 1268 | A |
| CHL1 | R82W | C | 886 | T | CNR1 | I105T | A | 522 | G | CPPED1 | A285T | C | 964 | T | CXorf1 | Y12* | C | 304 | A |
| CHL1 | E447* | G | 1981 | T | CNRIP1 | P5S | G | 619 | A | CPPED1 | N170S | T | 620 | C | CXorf22 | R29L | G | 152 | T |
| CHL1 | L592F | G | 2418 | T | CNRIP1 | Y25D | A | 679 | C | CPS1 | E838* | G | 2591 | T | CXorf27 | A98T | G | 315 | A |
| CHL1 | Q862H | G | 3228 | T | CNST | L623S | T | 2137 | C | CPS1 | P739Q | C | 2295 | A | CXorf30 | K916N | G | 2748 | T |
| CHL1 | N864H | A | 3232 | C | CNTD2 | D83N | C | 296 | T | CPS1 | A1186T | G | 3635 | A | CXorf36 | G195D | C | 659 | T |
| CHM | D143G | T | 458 | C | CNTD2 | A109T | C | 374 | T | CPS1 | R832C | C | 2573 | T | CXorf56 | A65V | G | 240 | A |
| CHM | L527F | C | 1611 | G | CNTF | F98C | T | 373 | G | CPS1 | S1209L | C | 3705 | T | CXorf57 | N597K | T | 1900 | G |
| CHM | R293* | G | 907 | A | CNTFR | A22T | C | 358 | T | CPS1 | I10S | T | 108 | G | CXorf64 | Q123H | G | 449 | T |
| CHML | R90C | G | 432 | A | CNTFR | N190D | T | 862 | C | CPSF1 | E451K | C | 1426 | T | CXorf64 | P181S | C | 621 | T |
| CHMP1A | V192M | C | 707 | T | CNTLN | E737G | A | 2294 | G | CPSF1 | G1292R | C | 3949 | T | CXorf66 | Y350C | T | 1073 | C |
| CHMP2A | Q121* | G | 517 | A | CNTN1 | E54D | G | 241 | T | CPSF2 | D110Y | G | 565 | T | CXXC1 | R415H | C | 1977 | T |
| CHMP4A | T141A | T | 722 | C | CNTN1 | Y864C | A | 2670 | G | CPSF2 | K350T | A | 1286 | C | CXXC1 | A388T | C | 1895 | T |
| CHMP4B | K178N | G | 699 | T | CNTN1 | V61F | G | 260 | T | CPSF3 | L526P | T | 1783 | C | CXXC5 | S18T | G | 767 | C |
| CHMP4B | L180V | T | 703 | G | CNTN1 | R186W | C | 635 | T | CPSF3 | E339* | G | 1221 | T | CYB561 | H160Y | G | 608 | A |
| CHMP6 | A46T | G | 214 | A | CNTN2 | - | T | 0 | C | CPSF3L | R215C | G | 726 | A | CYB561 | V138M | C | 542 | T |
| CHMP7 | - | T | 0 | C | CNTN2 | W731* | G | 2387 | A | CPSF3L | T93S | G | 361 | C | CYB5B | L139M | C | 574 | A |

| | | | | | | | | | | | | | | | | | | | |
|---|---|---|---|---|---|---|---|---|---|---|---|---|---|---|---|---|---|---|---|
| CHMP7 | V212F | G | 1282 | T | CNTN2 | L928I | C | 2976 | A | CPSF3L | P2H | G | 88 | T | CYB5B | E76A | A | 386 | C |
| CHN1 | D286G | T | 1171 | C | CNTN3 | K444T | T | 1359 | G | CPSF4L | R73Q | C | 280 | T | CYB5B | E89* | G | 424 | T |
| CHN1 | E49* | C | 459 | A | CNTN3 | V1026G | A | 3105 | C | CPSF7 | D406E | A | 1299 | C | CYB5D1 | K53N | G | 548 | T |
| CHN2 | D336N | G | 1536 | A | CNTN3 | S457Y | G | 1398 | T | CPT1A | N733H | T | 2367 | G | CYB5D2 | L114P | T | 929 | C |
| CHODL | R130Q | G | 780 | A | CNTN4 | - | G | 0 | A | CPT1B | V419M | C | 1360 | T | CYB5RL | R168Q | C | 826 | T |
| CHORDC1 | E301A | T | 1312 | G | CNTN4 | R584M | G | 2030 | T | CPT1B | G506C | C | 1621 | A | CYBA | A161T | C | 517 | T |
| CHPF | A591T | C | 2020 | T | CNTN4 | Q383R | A | 1427 | G | CPT1B | H445L | T | 1439 | A | CYC1 | G100S | G | 341 | A |
| CHPF | A122V | G | 614 | A | CNTN4 | S487G | A | 1738 | G | CPT1C | V458I | G | 1577 | A | CYC1 | V136M | G | 449 | A |
| CHPF | R435L | C | 1553 | A | CNTN4 | I864S | T | 2870 | G | CPT1C | R315W | C | 1148 | T | CYFIP1 | A511T | G | 1531 | A |
| CHPF2 | V358I | G | 2585 | A | CNTN4 | S882F | C | 2924 | T | CPT1C | V232G | T | 900 | G | CYFIP1 | L646P | T | 1937 | C |
| CHPF2 | R532* | C | 3107 | T | CNTN5 | V919I | G | 2760 | A | CPT1C | S676* | C | 2232 | A | CYFIP1 | R562W | C | 1684 | T |
| CHPF2 | E689G | A | 3579 | G | CNTN5 | T667I | C | 2005 | T | CPT2 | H584P | A | 2266 | C | CYFIP1 | A351T | G | 1051 | A |
| CHPF2 | I180V | A | 2051 | G | CNTN5 | I738F | A | 2217 | T | CPVL | T372A | T | 1233 | C | CYFIP1 | Y691C | A | 2072 | G |
| CHPF2 | V118M | G | 1865 | A | CNTN5 | R229Q | G | 691 | A | CPVL | R464Q | C | 1510 | T | CYFIP2 | R1123H | G | 3506 | A |
| CHPF2 | - | A | 0 | G | CNTN5 | D151Y | G | 456 | T | CPVL | - | A | 0 | G | CYFIP2 | T975A | A | 3061 | G |
| CHPF2 | G47R | G | 1652 | A | CNTN5 | V756A | T | 2272 | C | CPXCR1 | Y259C | A | 1035 | G | CYFIP2 | T54A | A | 298 | G |
| CHRD | G69W | G | 451 | T | CNTN5 | S975Y | C | 2929 | A | CPXM1 | R414W | G | 1305 | A | CYFIP2 | I290M | T | 1008 | G |
| CHRD | A472T | G | 1660 | A | CNTN5 | S1042Y | C | 3130 | A | CPXM1 | R518H | C | 1618 | T | CYFIP2 | F299V | T | 1033 | G |
| CHRD | A719V | C | 2402 | T | CNTN6 | S745L | C | 2861 | T | CPXM1 | A673V | G | 2083 | A | CYLC1 | K585N | G | 1792 | T |
| CHRD | R734Q | G | 2447 | A | CNTN6 | G596C | G | 2413 | T | CPXM1 | E710K | C | 2193 | T | CYLC1 | E590* | G | 1805 | T |
| CHRD | R927* | C | 3025 | T | CNTN6 | - | G | 0 | T | CPXM1 | A53V | G | 223 | A | CYLC2 | E291* | G | 941 | T |
| CHRDL2 | S182L | G | 819 | A | CNTN6 | P285Q | C | 1481 | A | CPXM2 | P85S | G | 408 | A | CYLC2 | K190T | A | 639 | C |
| CHRDL2 | P385H | G | 1428 | T | CNTNAP1 | - | A | 0 | G | CPXM2 | R325H | C | 1129 | T | CYLC2 | D216Y | G | 716 | T |
| CHRDL2 | A268V | G | 1077 | A | CNTNAP1 | R382S | C | 1360 | A | CPXM2 | - | A | 0 | G | CYLC2 | K320N | G | 1030 | T |
| CHRDL2 | P29S | G | 359 | A | CNTNAP1 | E129K | G | 601 | A | CPZ | H120Y | C | 532 | T | CYLD | K578T | A | 2148 | C |
| CHRM2 | Y131C | A | 1009 | G | CNTNAP2 | A674T | G | 2536 | A | CR1 | R1544H | G | 4771 | A | CYorf15A | R90I | G | 269 | T |
| CHRM2 | N284H | A | 1467 | C | CNTNAP2 | P621H | C | 2378 | A | CR1 | R101C | C | 441 | T | CYP11A1 | G86D | C | 412 | T |
| CHRM2 | K214R | A | 1258 | G | CNTNAP2 | K34E | A | 616 | G | CR1 | F1711L | C | 5273 | A | CYP11A1 | - | C | 0 | T |
| CHRM4 | P207H | G | 671 | T | CNTNAP2 | K331N | G | 1509 | T | CR1 | F2058L | C | 6314 | A | CYP11A1 | R185H | C | 709 | T |
| CHRM5 | K428E | A | 1952 | G | CNTNAP2 | Y658D | T | 2488 | G | CR1 | L2318I | C | 7092 | A | CYP11A1 | V213I | C | 792 | T |
| CHRM5 | Y171H | T | 1181 | C | CNTNAP3 | D496N | C | 1560 | T | CR2 | - | G | 0 | T | CYP11A1 | G86D | C | 412 | T |
| CHRM5 | T224A | A | 1340 | G | CNTNAP3 | S265G | T | 867 | C | CRABP2 | T58I | G | 328 | A | CYP11B1 | Q70H | C | 217 | A |
| CHRNA1 | V263I | C | 854 | T | CNTNAP3 | L432I | G | 1368 | T | CRADD | R13H | G | 142 | A | CYP11B1 | R101C | G | 308 | A |
| CHRNA1 | V53M | C | 224 | T | CNTNAP4 | R402Q | G | 1344 | A | CRAMP1L | R842* | C | 2611 | T | CYP11B1 | R209H | C | 633 | T |
| CHRNA1 | C187Y | C | 627 | T | CNTNAP4 | F985L | C | 3094 | A | CRAMP1L | - | T | 0 | C | CYP11B1 | A418T | C | 1259 | T |
| CHRNA1 | Y322C | T | 1032 | C | CNTNAP4 | T1214I | C | 3780 | T | CRAMP1L | R218H | G | 740 | A | CYP11B1 | R403W | G | 1214 | A |
| CHRNA1 | V330I | C | 1055 | T | CNTNAP4 | L415R | T | 1383 | G | CRAMP1L | S240* | C | 806 | A | CYP11B1 | R525C | G | 1580 | A |
| CHRNA10 | R151H | C | 524 | T | CNTNAP4 | R1266H | G | 3936 | A | CRAT | E156K | C | 761 | T | CYP11B2 | A414V | G | 1244 | A |
| CHRNA10 | R143H | C | 500 | T | CNTNAP4 | R282H | G | 984 | A | CRB1 | G850C | G | 2683 | T | CYP11B2 | R454H | C | 1364 | T |

| | | | | | | | | | | | | | | | | | | | |
|---|---|---|---|---|---|---|---|---|---|---|---|---|---|---|---|---|---|---|---|
| CHRNA10 | A130T | C | 460 | T | CNTNAP4 | K901T | A | 2841 | C | CRB1 | G936R | G | 2941 | A | CYP11B2 | R141Q | C | 425 | T |
| CHRNA2 | Y240C | T | 1328 | C | CNTNAP4 | S212Y | C | 774 | A | CRB1 | G454* | G | 1495 | T | CYP17A1 | A457T | C | 1541 | T |
| CHRNA2 | G169D | C | 1115 | T | CNTNAP4 | Q219R | A | 795 | G | CRB1 | D249N | G | 880 | A | CYP17A1 | R21W | T | 233 | A |
| CHRNA2 | A177T | C | 1138 | T | CNTNAP4 | G717V | G | 2289 | T | CRB1 | N287T | A | 995 | C | CYP17A1 | R496C | G | 1658 | A |
| CHRNA2 | S406N | C | 1826 | T | CNTNAP4 | T5A | A | 398 | G | CRB1 | M774I | G | 2457 | A | CYP19A1 | R499M | C | 1650 | A |
| CHRNA4 | C161R | A | 703 | G | CNTNAP5 | Q864H | G | 2956 | C | CRB1 | F786Y | T | 2492 | A | CYP19A1 | S314F | G | 1095 | A |
| CHRNA4 | R369Q | C | 1328 | T | CNTNAP5 | D519Y | G | 1919 | T | CRB1 | T1264K | C | 3926 | A | CYP1A1 | - | A | 0 | G |
| CHRNA4 | P559S | G | 1897 | A | CNTNAP5 | K164N | G | 856 | T | CRB2 | G795S | G | 2384 | A | CYP1B1 | R80C | G | 640 | A |
| CHRNA5 | R248H | G | 943 | A | CNTNAP5 | G1256D | G | 4131 | A | CRB2 | E792K | G | 2375 | A | CYP1B1 | A338T | C | 1414 | T |
| CHRNA5 | V441I | G | 1521 | A | CNTNAP5 | I518M | T | 1918 | G | CRB2 | F1207L | C | 3622 | A | CYP20A1 | F364L | T | 1171 | C |
| CHRNA6 | A32V | G | 141 | A | CNTNAP5 | R643W | C | 2291 | T | CRB3 | V62M | G | 380 | A | CYP24A1 | I447S | A | 1734 | C |
| CHRNA6 | K114T | T | 387 | G | CNTNAP5 | R839Q | G | 2880 | A | CRBN | S272Y | G | 838 | T | CYP24A1 | R310W | G | 1322 | A |
| CHRNA6 | F471V | A | 1457 | C | CNTNAP5 | R1121* | C | 3725 | T | CRBN | W223R | A | 690 | G | CYP26A1 | E413* | G | 1282 | T |
| CHRNA9 | S127F | C | 519 | T | CNTROB | A530V | C | 2514 | T | CREB3 | R159* | C | 913 | T | CYP26B1 | R473C | G | 1621 | A |
| CHRNA9 | R138Q | G | 552 | A | CNTROB | K90N | G | 1195 | T | CREB3 | A304P | G | 1348 | C | CYP26B1 | T504M | G | 1715 | A |
| CHRNB1 | V56I | G | 233 | A | CNTROB | K710N | G | 3055 | T | CREB3L1 | T416M | C | 1682 | T | CYP26B1 | V479I | C | 1639 | T |
| CHRNB1 | V132M | G | 461 | A | COASY | R231C | C | 893 | T | CREB3L1 | E265D | G | 1230 | T | CYP26B1 | R363H | C | 1292 | T |
| CHRNB2 | V454I | G | 1624 | A | COASY | R528H | G | 1785 | A | CREB3L1 | R508M | G | 1958 | T | CYP26C1 | T486A | A | 1456 | G |
| CHRNB2 | R358H | G | 1337 | A | COBL | D520G | T | 1744 | C | CREB3L1 | P62H | C | 620 | A | CYP26C1 | A320T | G | 958 | A |
| CHRNB3 | G246E | G | 865 | A | COBL | R358Q | C | 1258 | T | CREB3L1 | A235V | C | 1139 | T | CYP26C1 | L188I | C | 562 | A |
| CHRNB4 | R410S | C | 1342 | A | COBL | - | C | 0 | A | CREB3L2 | M381T | A | 1494 | G | CYP26C1 | D370Y | G | 1108 | T |
| CHRNB4 | L345I | G | 1145 | T | COBL | L207P | A | 805 | G | CREB3L2 | T118A | T | 704 | C | CYP27A1 | R323Q | G | 1395 | A |
| CHRND | E94* | G | 312 | T | COBLL1 | L586S | A | 1762 | G | CREB3L2 | S103N | C | 660 | T | CYP27A1 | R225C | C | 1100 | T |
| CHRND | R363H | G | 1120 | A | COBLL1 | E654K | C | 1965 | T | CREB3L4 | R278H | G | 1099 | A | CYP27A1 | F185I | T | 980 | A |
| CHRND | - | A | 0 | G | COBLL1 | R284H | C | 856 | T | CREB3L4 | G59D | G | 442 | A | CYP27B1 | S44T | A | 282 | T |
| CHRND | S154Y | C | 493 | A | COBLL1 | I269L | T | 810 | G | CREB5 | Q268R | A | 1193 | G | CYP27C1 | I132L | T | 525 | G |
| CHRNG | A259T | G | 796 | A | COBLL1 | L122F | C | 371 | A | CREBBP | R742C | G | 3029 | A | CYP27C1 | S130N | C | 520 | T |
| CHST1 | R408H | C | 1894 | T | COCH | P465S | C | 1797 | T | CREBBP | R413Q | C | 2043 | T | CYP2A13 | R129H | G | 395 | A |
| CYP2A13 | H254Y | C | 769 | T | DERL3 | - | C | 0 | T | DNAH5 | D2761Y | C | 8386 | A | DST | T2904A | T | 8710 | C |
| CYP2A7 | T216M | G | 1189 | A | DES | R227C | C | 765 | T | DNAH5 | E2250K | C | 6853 | T | DST | E1340K | C | 4018 | T |
| CYP2B6 | R336H | G | 1014 | A | DES | R315H | G | 1030 | A | DNAH5 | R761I | C | 2387 | A | DST | E650* | C | 1948 | A |
| CYP2C18 | I472V | A | 1613 | G | DES | R160Q | G | 565 | A | DNAH5 | - | C | 0 | A | DSTYK | S514N | C | 1572 | T |
| CYP2C18 | R186Q | G | 756 | A | DES | S460N | G | 1465 | A | DNAH5 | R2943H | C | 8933 | T | DTD1 | V67A | T | 380 | C |
| CYP2C18 | L205R | T | 813 | G | DES | T445M | C | 1420 | T | DNAH5 | V1022D | A | 3170 | T | DTHD1 | K516N | A | 1693 | C |
| CYP2C18 | D165Y | G | 692 | T | DET1 | R123Q | C | 368 | T | DNAH5 | R3398H | C | 10298 | T | DTHD1 | R711I | G | 2277 | T |
| CYP2C19 | E318K | G | 1034 | A | DET1 | R35H | C | 104 | T | DNAH5 | M2928V | T | 8887 | C | DTL | R77L | G | 544 | T |
| CYP2C19 | R335Q | G | 1086 | A | DET1 | R265W | G | 793 | A | DNAH5 | P615H | G | 1949 | T | DTL | S468F | C | 1717 | T |
| CYP2C8 | S460F | G | 1474 | A | DEXI | L2F | G | 459 | A | DNAH5 | K4200R | T | 12704 | C | DTNA | P706A | C | 2416 | G |
| CYP2C9 | R433Q | G | 1310 | A | DFFB | R172Q | G | 838 | A | DNAH5 | R3767H | C | 11405 | T | DTWD2 | A102S | C | 338 | A |

| | | | | | | | | | | | | | | | | | | | |
|---|---|---|---|---|---|---|---|---|---|---|---|---|---|---|---|---|---|---|---|
| CYP2C9 | L11I | C | 43 | A | DFFB | E321D | G | 1286 | T | DNAH5 | A3294T | C | 9985 | T | DTX1 | A117T | G | 685 | A |
| CYP2D6 | G113R | C | 452 | T | DFNA5 | R351W | G | 1477 | A | DNAH5 | R3553Q | C | 10763 | T | DTX1 | P19L | C | 392 | T |
| CYP2D6 | L160I | G | 593 | T | DFNA5 | P142L | G | 851 | A | DNAH5 | N3429K | G | 10392 | C | DTX2 | Q25P | A | 584 | C |
| CYP2E1 | E328* | G | 1006 | T | DFNA5 | A394T | C | 1606 | T | DNAH5 | R2503C | G | 7612 | A | DTX3 | R268W | C | 1111 | T |
| CYP2E1 | D470Y | G | 1432 | T | DFNA5 | S252F | G | 1181 | A | DNAH5 | S4425R | G | 13380 | T | DTX3L | P222L | C | 854 | T |
| CYP2E1 | K160N | G | 504 | T | DFNB31 | R818S | G | 2621 | T | DNAH5 | R2501W | G | 7606 | A | DTX3L | L453I | C | 1546 | A |
| CYP2E1 | E328* | G | 1006 | T | DFNB31 | A834T | C | 2669 | T | DNAH5 | Q451E | G | 1456 | C | DTX3L | R660Q | G | 2168 | A |
| CYP2J2 | A133T | C | 441 | T | DFNB59 | D219Y | G | 1011 | T | DNAH5 | T3876P | T | 11731 | G | DTX4 | R402W | C | 1461 | T |
| CYP2J2 | C227R | A | 723 | G | DGAT1 | P238L | G | 981 | A | DNAH5 | M2461T | A | 7487 | G | DULLARD | L20P | A | 439 | G |
| CYP2J2 | T456A | T | 1410 | C | DGAT1 | V192A | A | 843 | G | DNAH5 | V2044I | C | 6235 | T | DUOX1 | V1162I | G | 3830 | A |
| CYP2J2 | R200H | C | 643 | T | DGAT2 | G296V | G | 1146 | T | DNAH5 | R956C | G | 2971 | A | DUOX1 | R1214C | C | 3986 | T |
| CYP2R1 | I301T | A | 902 | G | DGAT2 | D356N | G | 1325 | A | DNAH5 | R4243H | C | 12833 | T | DUOX1 | R1294W | C | 4226 | T |
| CYP2R1 | V64D | A | 191 | T | DGAT2L6 | F16L | C | 148 | A | DNAH5 | E4026K | C | 12181 | T | DUOX1 | R1024H | G | 3417 | A |
| CYP2R1 | G486D | C | 1457 | T | DGCR14 | R191G | T | 615 | C | DNAH5 | R3755K | C | 11369 | T | DUOX1 | I1045N | T | 3480 | A |
| CYP2S1 | P77H | C | 285 | A | DGCR14 | R242Q | C | 769 | T | DNAH5 | R3096Q | C | 9392 | T | DUOX1 | R1473W | C | 4763 | T |
| CYP2U1 | R466Q | G | 1672 | A | DGCR2 | R38C | G | 360 | A | DNAH5 | E3093D | C | 9384 | A | DUOX2 | R1211H | C | 4013 | T |
| CYP2W1 | G375S | G | 1136 | A | DGCR2 | D62N | C | 432 | T | DNAH5 | S2458N | C | 7478 | T | DUOX2 | T1321A | T | 4342 | C |
| CYP3A43 | T374M | C | 1121 | T | DGCR2 | R85W | G | 501 | A | DNAH5 | D1341Y | C | 4126 | A | DUOX2 | D193G | T | 959 | C |
| CYP3A43 | A337T | G | 1009 | A | DGCR2 | F35L | A | 351 | G | DNAH5 | N375H | T | 1228 | G | DUOX2 | F140L | A | 799 | G |
| CYP3A5 | E283* | C | 947 | A | DGCR6 | Q153H | G | 611 | T | DNAH5 | E54K | C | 265 | T | DUOX2 | D534Y | C | 1981 | A |
| CYP3A5 | A121D | G | 462 | T | DGCR8 | K457T | A | 1799 | C | DNAH5 | K3920T | T | 11864 | G | DUOX2 | R317S | G | 1330 | T |
| CYP3A7 | R478H | C | 1538 | T | DGCR8 | V526I | G | 2005 | A | DNAH5 | T2176M | G | 6632 | A | DUPD1 | A160V | G | 479 | A |
| CYP3A7 | A150D | G | 554 | T | DGCR8 | F532L | C | 2025 | A | DNAH5 | R2771C | G | 8416 | A | DUPD1 | Q203P | T | 608 | G |
| CYP46A1 | Q175K | C | 624 | A | DGKA | P718S | C | 2416 | T | DNAH6 | I2463M | A | 7526 | G | DUS1L | S186L | G | 759 | A |
| CYP46A1 | S99P | T | 396 | C | DGKA | - | A | 0 | G | DNAH6 | C4109F | G | 12463 | T | DUS1L | Y98D | A | 494 | C |
| CYP4A11 | V3I | C | 58 | T | DGKA | T637A | A | 2173 | G | DNAH6 | L342M | C | 1161 | A | DUS2L | S121F | C | 532 | T |
| CYP4A11 | K115N | T | 396 | G | DGKA | K643T | A | 2192 | C | DNAH6 | K1349E | A | 4182 | G | DUS2L | L207I | C | 789 | A |
| CYP4A11 | R96C | G | 337 | A | DGKB | R466H | C | 1562 | T | DNAH6 | Y1415F | A | 4381 | T | DUS3L | Q487K | G | 1501 | T |
| CYP4B1 | R439H | G | 1352 | A | DGKB | L781M | G | 2506 | T | DNAH6 | V2100A | T | 6436 | C | DUS3L | W539R | A | 1657 | T |
| CYP4B1 | P421S | C | 1297 | T | DGKB | K408T | T | 1388 | G | DNAH6 | F3165C | T | 9631 | G | DUS3L | R551W | G | 1693 | A |
| CYP4B1 | R398W | C | 1228 | T | DGKD | - | T | 0 | C | DNAH6 | E3236K | G | 9843 | A | DUS3L | R584Q | C | 1793 | T |
| CYP4F12 | E71* | G | 272 | T | DGKG | H289R | T | 1406 | C | DNAH7 | L252M | G | 855 | T | DUS3L | - | T | 0 | C |
| CYP4F2 | E72G | T | 266 | C | DGKG | Q16H | T | 588 | G | DNAH7 | R688W | G | 2163 | A | DUS3L | P397L | G | 1232 | A |
| CYP4F2 | R46H | C | 188 | T | DGKG | G385S | C | 1693 | T | DNAH7 | N2282T | T | 6946 | G | DUS3L | Q230R | T | 731 | C |
| CYP4F22 | R515C | C | 1547 | T | DGKG | F583L | G | 2289 | T | DNAH7 | D1809N | C | 5526 | T | DUS4L | R101H | G | 664 | A |
| CYP4F22 | T410M | C | 1233 | T | DGKG | G249W | C | 1285 | A | DNAH7 | F3304C | A | 10012 | C | DUS4L | A107V | C | 682 | T |
| CYP4F22 | S395N | G | 1188 | A | DGKH | R273C | C | 838 | T | DNAH7 | R1839* | G | 5616 | A | DUSP1 | A327T | C | 1222 | T |
| CYP4F22 | F132L | C | 400 | A | DGKH | A751D | C | 2273 | A | DNAH7 | D3765N | C | 11394 | T | DUSP10 | T110I | G | 568 | A |
| CYP4F22 | L303S | T | 912 | C | DGKH | Q275R | A | 845 | G | DNAH8 | S4350P | T | 13048 | C | DUSP10 | I210L | T | 867 | G |

| | | | | | | | | | | | | | | | | | | | |
|---|---|---|---|---|---|---|---|---|---|---|---|---|---|---|---|---|---|---|---|
| CYP4F8 | H263R | A | 788 | G | DGKI | R258Q | C | 1003 | T | DNAH8 | A3779V | C | 11336 | T | DUSP12 | R230* | C | 720 | T |
| CYP4F8 | N420D | A | 1258 | G | DGKI | A1041V | G | 3352 | A | DNAH8 | A1878T | G | 5632 | A | DUSP13 | A15T | C | 586 | T |
| CYP4F8 | S109P | T | 325 | C | DGKI | A689S | C | 2295 | A | DNAH8 | Q2318E | C | 6952 | G | DUSP13 | L241I | G | 1264 | T |
| CYP4F8 | F244V | T | 730 | G | DGKI | V1018M | C | 3282 | T | DNAH8 | Q911K | C | 2731 | A | DUSP13 | G200E | C | 1142 | T |
| CYP4V2 | R296C | C | 1190 | T | DGKI | A283D | G | 1078 | T | DNAH8 | Q1340* | C | 4018 | T | DUSP16 | S92P | A | 906 | G |
| CYP4Z1 | K75T | A | 227 | C | DGKI | R794C | G | 2610 | A | DNAH8 | N3960T | A | 11879 | C | DUSP16 | I185F | T | 1185 | A |
| CYP51A1 | D152N | C | 620 | T | DGKI | I946M | T | 3068 | C | DNAH8 | V2003A | T | 6008 | C | DUSP22 | A41V | C | 609 | T |
| CYP51A1 | R506* | G | 1682 | A | DGKI | G590E | C | 1999 | T | DNAH8 | I259S | T | 776 | G | DUSP22 | R119C | C | 842 | T |
| CYP51A1 | R258H | C | 939 | T | DGKI | R237* | G | 939 | A | DNAH8 | F648L | T | 1942 | C | DUSP23 | R37Q | G | 181 | A |
| CYP7A1 | T142M | G | 488 | A | DGKQ | S371L | G | 1186 | A | DNAH8 | S705Y | C | 2114 | A | DUSP27 | R409W | C | 1391 | T |
| CYP7A1 | R483W | G | 1510 | A | DGKQ | G629E | C | 1960 | T | DNAH8 | D807N | G | 2419 | A | DUSP27 | E145D | G | 601 | T |
| CYP7A1 | S327R | T | 1042 | G | DGKZ | C793Y | G | 2503 | A | DNAH8 | E1033* | G | 3097 | T | DUSP27 | D319N | G | 1121 | A |
| CYP7A1 | R154K | C | 524 | T | DGKZ | P264L | C | 916 | T | DNAH8 | E1742D | G | 5226 | T | DUSP27 | W938G | T | 2978 | G |
| CYP7B1 | R361Q | C | 1286 | T | DGKZ | E1010* | G | 3153 | T | DNAH8 | R3012Q | G | 9035 | A | DUSP4 | F259L | G | 1167 | T |
| CYP8B1 | R28C | G | 407 | A | DGKZ | R530G | C | 1713 | G | DNAH8 | L3396M | C | 10186 | A | DUSP5 | K294Q | A | 1164 | C |
| CYP8B1 | R496H | C | 1812 | T | DGKZ | I474T | T | 1546 | C | DNAH8 | F3963S | T | 11888 | C | DUSP6 | V239I | C | 1195 | T |
| CYR61 | E137* | G | 633 | T | DGKZ | A176T | G | 651 | A | DNAH8 | E4027D | A | 12081 | C | DUSP8 | I30T | A | 526 | G |
| CYSLTR1 | R22H | C | 358 | T | DGKZ | I537V | A | 1734 | G | DNAH8 | K4363E | A | 13087 | G | DUSP8 | R602C | G | 2241 | A |
| CYSLTR2 | F5C | T | 17 | G | DGKZ | R468H | G | 1528 | A | DNAH9 | R2965C | C | 8961 | T | DUSP8 | G23R | C | 504 | T |
| CYTH1 | A386T | C | 1227 | T | DHCR7 | G213C | C | 910 | A | DNAH9 | A2803V | C | 8476 | T | DUXA | R152* | G | 499 | A |
| CYTH1 | V142I | C | 495 | T | DHCR7 | I370V | T | 1381 | C | DNAH9 | L570R | T | 1777 | G | DUXA | R105H | C | 359 | T |
| CYTH2 | P76H | C | 527 | A | DHCR7 | A417G | G | 1523 | C | DNAH9 | R965L | G | 2962 | T | DUXA | R162K | C | 530 | T |
| CYTH2 | R318Q | G | 1253 | A | DHCR7 | V340I | C | 1291 | T | DNAH9 | R4271C | C | 12879 | T | DVL1L1 | R227C | G | 964 | A |
| CYTH2 | I391S | T | 1472 | G | DHDH | A111V | C | 372 | T | DNAH9 | Q4411R | A | 13300 | G | DVL1L1 | A385T | C | 1438 | T |
| CYTH3 | E268K | C | 939 | T | DHDH | F268S | T | 843 | C | DNAH9 | R2072C | C | 6282 | T | DVL1L1 | R227H | C | 965 | T |
| CYTH4 | E64A | A | 378 | C | DHDH | R127H | G | 420 | A | DNAH9 | R3286H | G | 9925 | A | DYDC1 | R59H | C | 341 | T |
| CYTH4 | N221D | A | 848 | G | DHDPSL | G199S | G | 956 | A | DNAH9 | R733W | C | 2265 | T | DYM | D562N | C | 2049 | T |
| CYTL1 | R134C | G | 427 | A | DHFRL1 | K64N | C | 529 | A | DNAH9 | R803I | G | 2476 | T | DYM | R602Q | C | 2170 | T |
| CYTSA | A1016T | G | 3249 | A | DHFRP1 | M112I | C | 830 | A | DNAH9 | E1050D | G | 3218 | T | DYM | E125V | T | 739 | A |
| CYTSA | R399W | C | 1398 | T | DHH | P119H | G | 663 | T | DNAH9 | G1310E | G | 3997 | A | DYM | H570N | G | 2073 | T |
| CYTSA | R740W | C | 2421 | T | DHH | A302V | G | 1212 | A | DNAH9 | R1640* | C | 4986 | T | DYM | R533* | G | 1962 | A |
| CYTSB | R831H | G | 2543 | A | DHODH | R231C | C | 712 | T | DNAH9 | Y2756C | A | 8335 | G | DYNC1H1 | T4552A | A | 1381 8 | G |
| CYTSB | I824V | A | 2521 | G | DHODH | T31M | C | 113 | T | DNAH9 | Y3699H | T | 11163 | C | DYNC1H1 | G1955D | G | 6028 | A |
| CYTSB | R310W | C | 979 | T | DHODH | V104M | G | 331 | A | DNAH9 | H3978R | A | 12001 | G | DYNC1H1 | T2428M | C | 7447 | T |
| CYTSB | E190* | G | 619 | T | DHPS | P185H | G | 656 | T | DNAH9 | L4484I | C | 13518 | A | DYNC1H1 | A2772V | C | 8479 | T |
| CYTSB | E390* | G | 1219 | T | DHRS1 | L282I | G | 1068 | T | DNAI1 | V466M | G | 1567 | A | DYNC1H1 | N799K | C | 2561 | G |
| CYYR1 | P111H | G | 654 | T | DHRS1 | A174V | G | 745 | A | DNAI2 | D146G | A | 536 | G | DYNC1H1 | R914H | G | 2905 | A |
| D2HGDH | L505M | C | 1722 | A | DHRS12 | P275L | G | 838 | A | DNAI2 | S398L | C | 1292 | T | DYNC1H1 | N2003T | A | 6172 | C |
| D2HGDH | D403N | G | 1416 | A | DHRS13 | A3S | C | 134 | A | DNAJA1 | T288A | A | 1045 | G | DYNC1H1 | N518S | A | 1717 | G |

| | | | | | | | | | | | | | | | | | | | |
|---|---|---|---|---|---|---|---|---|---|---|---|---|---|---|---|---|---|---|---|
| D2HGDH | G98S | G | 501 | A | DHRS2 | R276W | C | 1264 | T | DNAJA3 | F328C | T | 1060 | G | DYNC1H1 | R2453H | G | 7522 | A |
| DAAM1 | F972L | C | 3041 | A | DHRS7C | V302M | C | 917 | T | DNAJA4 | P181L | C | 770 | T | DYNC1H1 | R3219C | C | 9819 | T |
| DAAM1 | R31Q | G | 217 | A | DHRS7C | A136V | G | 420 | A | DNAJA4 | P359H | C | 1304 | A | DYNC1H1 | A1303V | C | 4072 | T |
| DAAM2 | R209C | C | 781 | T | DHRS9 | D188Y | G | 2065 | T | DNAJB11 | R206* | C | 1545 | T | DYNC1I1 | K327N | G | 1174 | T |
| DAAM2 | T146P | A | 592 | C | DHTKD1 | A574T | G | 1782 | A | DNAJB12 | Q404H | C | 1362 | A | DYNC1I1 | K25Q | A | 266 | C |
| DAAM2 | R597H | G | 1946 | A | DHTKD1 | G566R | G | 1758 | A | DNAJB13 | G10R | G | 779 | A | DYNC1I2 | E29* | G | 252 | T |
| DAAM2 | T482M | C | 1601 | T | DHTKD1 | V806M | G | 2478 | A | DNAJB13 | I159N | T | 1227 | A | DYNC1LI1 | I87M | A | 365 | C |
| DAAM2 | P954S | C | 3016 | T | DHTKD1 | S634N | G | 1963 | A | DNAJB13 | K66T | A | 948 | C | DYNC2H1 | E670* | G | 2152 | T |
| DAAM2 | R414W | C | 1396 | T | DHX15 | S2P | A | 161 | G | DNAJB13 | E149D | G | 1198 | T | DYNC2H1 | D2059Y | G | 6319 | T |
| DAAM2 | E735V | A | 2360 | T | DHX15 | R706Q | C | 2274 | T | DNAJB2 | A50T | G | 436 | A | DYNC2H1 | E3167* | G | 9643 | T |
| DAAM2 | K85N | G | 411 | T | DHX15 | D621Y | C | 2018 | A | DNAJB2 | R286W | C | 1144 | T | DYNC2H1 | R4030G | A | 12232 | G |
| DAAM2 | F957L | C | 3027 | A | DHX29 | A902T | C | 2853 | T | DNAJB5 | S66R | A | 557 | C | DYNC2H1 | R2426H | G | 7421 | A |
| DAB1 | T215M | G | 782 | A | DHX29 | N841D | T | 2670 | C | DNAJB5 | R236C | C | 1067 | T | DYNC2H1 | V1975A | T | 6068 | C |
| DAB1 | Q452K | G | 1492 | T | DHX29 | R984Q | C | 3100 | T | DNAJB6 | P273H | C | 1023 | A | DYNC2H1 | R2620* | C | 8002 | T |
| DAB1 | G48R | C | 280 | T | DHX29 | W161* | C | 632 | T | DNAJB7 | R297C | G | 1021 | A | DYNC2H1 | - | G | 0 | A |
| DAB2 | Q319P | T | 1424 | G | DHX29 | S657P | A | 2118 | G | DNAJB9 | D121Y | G | 907 | T | DYNC2H1 | S3292P | T | 10018 | C |
| DAB2 | - | T | 2781 | G | DHX29 | R832H | C | 2644 | T | DNAJC10 | R180Q | G | 954 | A | DYNC2H1 | E38* | G | 256 | T |
| DAB2 | D244N | C | 1198 | T | DHX30 | R324H | G | 983 | A | DNAJC10 | P639Q | C | 2331 | A | DYNC2H1 | K2225T | A | 6818 | C |
| DAB2IP | R333H | G | 1180 | A | DHX30 | V1192I | G | 3586 | A | DNAJC10 | R366Q | G | 1512 | A | DYNC2H1 | R2325Q | G | 7118 | A |
| DAB2IP | R132H | G | 577 | A | DHX30 | R323C | C | 979 | T | DNAJC10 | R180* | C | 953 | T | DYNC2H1 | E2632K | G | 8038 | A |
| DAB2IP | A836V | C | 2689 | T | DHX30 | R1208* | C | 3634 | T | DNAJC11 | R104* | G | 434 | A | DYNC2H1 | Q3166H | A | 9642 | C |
| DAB2IP | A437T | G | 1491 | A | DHX34 | V517I | G | 1898 | A | DNAJC11 | E105K | C | 437 | T | DYNC2H1 | E3992A | A | 12119 | C |
| DAB2IP | P94S | C | 462 | T | DHX34 | L175P | T | 873 | C | DNAJC13 | R1745H | G | 5482 | A | DYNC2H1 | E4139G | A | 12560 | G |
| DAB2IP | R333C | C | 1179 | T | DHX34 | S750T | G | 2598 | C | DNAJC13 | V670I | G | 2256 | A | DYNC2H1 | E4217* | G | 12793 | T |
| DAB2IP | T76M | C | 409 | T | DHX34 | P575H | C | 2073 | A | DNAJC13 | R1209C | C | 3873 | T | DYNLL1P1 | A25T | C | 73 | T |
| DACH1 | S149I | C | 446 | A | DHX35 | V522L | G | 1597 | T | DNAJC13 | R2084* | C | 6498 | T | DYNLRB1 | R122* | C | 627 | T |
| DACH1 | V518M | C | 1552 | T | DHX35 | R371M | G | 1145 | T | DNAJC16 | L410P | T | 1393 | C | DYNLRB2 | R58C | C | 292 | T |
| DACH1 | P185H | G | 554 | T | DHX35 | R533* | C | 1630 | T | DNAJC17 | N43H | T | 154 | G | DYRK1A | R559C | C | 1750 | T |
| DACH2 | T148N | C | 443 | A | DHX36 | T903A | T | 2788 | C | DNAJC2 | R303W | G | 1158 | A | DYRK1A | E160K | G | 553 | A |
| DACH2 | Q390H | G | 1170 | T | DHX36 | G51D | C | 233 | T | DNAJC2 | A348T | C | 1293 | T | DYRK1B | W297R | A | 1169 | G |
| DACH2 | E496* | G | 1486 | T | DHX36 | Q580R | T | 1820 | C | DNAJC2 | L116V | A | 597 | C | DYRK1B | T441M | G | 1602 | A |
| DACT1 | T795M | C | 2408 | T | DHX36 | R165* | G | 574 | A | DNAJC21 | A247T | G | 966 | A | DYRK1B | A399T | C | 1475 | T |
| DACT1 | G661R | G | 2005 | A | DHX36 | K798T | T | 2474 | G | DNAJC21 | R192W | C | 801 | T | DYRK1B | M596L | T | 2066 | G |
| DACT1 | R587H | G | 1784 | A | DHX36 | E436* | C | 1387 | A | DNAJC21 | K541T | A | 1849 | C | DYRK4 | F179L | C | 679 | A |
| DACT1 | A707T | G | 2143 | A | DHX37 | R1101C | G | 3400 | A | DNAJC22 | R80C | C | 1442 | T | DYRK4 | A314T | G | 1082 | A |
| DACT1 | F241V | T | 745 | G | DHX37 | R307L | C | 1019 | A | DNAJC24 | K33Q | A | 213 | C | DYSF | E2035G | A | 6245 | G |

| | | | | | | | | | | | | | | | | | | | |
|---|---|---|---|---|---|---|---|---|---|---|---|---|---|---|---|---|---|---|---|
| DACT2 | I461T | A | 1471 | G | DHX37 | R940S | C | 2919 | A | DNAJC24 | A18T | G | 168 | A | DYSF | D2061N | G | 6322 | A |
| DACT2 | R197G | T | 678 | C | DHX37 | N123S | T | 467 | C | DNAJC24 | E61K | G | 297 | A | DYSF | R1845W | C | 5674 | T |
| DACT3 | S101R | T | 375 | G | DHX37 | A736T | C | 2305 | T | DNAJC25 | E203* | G | 663 | T | DYSF | V677M | G | 2170 | A |
| DACT3 | A518V | G | 1627 | A | DHX38 | R1164W | C | 3845 | T | DNAJC4 | S33N | G | 560 | A | DYSF | A1080G | C | 3380 | G |
| DAG1 | S239P | T | 1133 | C | DHX38 | R639Q | G | 2271 | A | DNAJC5 | R36W | C | 259 | T | DYSF | L1099P | T | 3437 | C |
| DAG1 | T436M | C | 1725 | T | DHX38 | A772T | G | 2669 | A | DNAJC5 | T146M | C | 590 | T | DYSF | D1248N | G | 3883 | A |
| DAG1 | P398S | C | 1610 | T | DHX38 | R324W | C | 1325 | T | DNAJC5 | N62S | A | 338 | G | DYSF | R1625* | C | 5014 | T |
| DAG1 | R640Q | G | 2337 | A | DHX38 | R203* | C | 962 | T | DNAJC5 | A70T | G | 361 | A | DYSF | E1887K | G | 5800 | A |
| DAGLA | G465S | G | 1509 | A | DHX38 | S979L | C | 3291 | T | DNAJC5 | A63V | C | 341 | T | DYSF | E1353K | G | 4198 | A |
| DAGLB | A286V | G | 1027 | A | DHX38 | W215* | G | 1000 | A | DNAJC5B | R77I | G | 521 | T | DYSF | R409* | C | 1366 | T |
| DAGLB | A145V | G | 604 | A | DHX38 | R201L | G | 957 | T | DNAJC5B | K50N | G | 441 | T | DYSF | K111T | A | 473 | C |
| DAGLB | R568C | G | 1872 | A | DHX40 | R712* | C | 2281 | T | DNAJC5B | E34* | G | 391 | T | DYSF | - | T | 0 | C |
| DAGLB | F536L | A | 1776 | G | DHX57 | D160N | C | 605 | T | DNAJC5B | R193Q | G | 869 | A | DYSF | I517L | A | 1690 | C |
| DAK | R397Q | G | 1447 | A | DHX57 | R526Q | C | 1704 | T | DNAJC6 | R806* | C | 2617 | T | DYSF | H711R | A | 2273 | G |
| DALRD3 | L19M | G | 62 | T | DHX57 | H980Q | A | 3067 | C | DNAJC6 | D104Y | G | 511 | T | DYSF | R1113H | G | 3479 | A |
| DALRD3 | Y497C | T | 1497 | C | DHX57 | S462Y | G | 1512 | T | DNAJC6 | R174Q | G | 722 | A | DYSF | G1667V | G | 5141 | T |
| DAO | G315R | G | 1096 | A | DHX57 | F400C | A | 1326 | C | DNAJC6 | S709I | G | 2327 | T | DYTN | R146C | G | 553 | A |
| DAO | R286C | C | 1009 | T | DHX57 | S341Y | G | 1149 | T | DNAJC7 | R68* | G | 439 | A | DYTN | S442C | T | 1441 | A |
| DAO | A36T | G | 259 | A | DHX8 | R365W | C | 1165 | T | DNAJC9 | E181* | C | 2214 | A | DYTN | T223I | G | 785 | A |
| DAOA | C132* | C | 442 | A | DHX8 | G17D | G | 122 | A | DNASE1 | A157V | C | 654 | T | DYTN | S177T | C | 647 | G |
| DAP3 | E285D | G | 979 | T | DHX8 | R494W | C | 1552 | T | DNASE1L2 | A41D | C | 255 | A | DYTN | P317S | G | 1066 | A |
| DAP3 | S252Y | C | 879 | A | DHX8 | S500Y | C | 1571 | A | DNASE1L2 | P290A | C | 1001 | G | DYX1C1 | L48V | A | 510 | C |
| DAPK1 | P1369H | C | 4289 | A | DHX9 | A450V | C | 1459 | T | DNASE1L2 | Q162H | G | 619 | T | DYX1C1 | K143N | T | 797 | G |
| DAPK1 | V309I | G | 1108 | A | DHX9 | R1052* | C | 3264 | T | DNASE1L2 | S225G | A | 806 | G | DYX1C1 | D125Y | C | 741 | A |
| DAPK1 | E113V | A | 521 | T | DHX9 | R1154W | C | 3570 | T | DNASE1L3 | R206H | C | 1102 | T | DZIP1 | R595* | G | 2216 | A |
| DAPK1 | M764V | A | 2473 | G | DHX9 | R141* | C | 531 | T | DNASE1L3 | E242* | C | 1209 | A | DZIP1 | K805N | T | 2848 | G |
| DAPK1 | G1418S | G | 4435 | A | DIAPH1 | K1089T | T | 3407 | G | DNASE2 | C347Y | C | 1186 | T | DZIP1 | K605T | T | 2247 | G |
| DAPK1 | A496S | G | 1669 | T | DIAPH1 | T472A | T | 1555 | C | DNASE2 | - | C | 0 | T | DZIP1 | E505* | C | 1946 | A |
| DAPK3 | R454C | G | 1453 | A | DIAPH1 | E203* | C | 748 | A | DNASE2B | K271Q | A | 844 | C | DZIP1 | E348K | C | 1475 | T |
| DAPK3 | V257I | C | 862 | T | DIAPH1 | K38T | T | 254 | G | DND1 | R225C | G | 717 | A | DZIP1 | A817V | G | 2883 | A |
| DAPK3 | G20D | C | 152 | T | DIAPH2 | P266H | C | 1193 | A | DNER | R483C | G | 1582 | A | DZIP1L | R147H | C | 803 | T |
| DAPP1 | S137F | C | 478 | T | DIAPH2 | Y231H | T | 1087 | C | DNHD1 | G883R | G | 3211 | A | DZIP1L | E750D | C | 2613 | A |
| DARC | R91H | G | 447 | A | DIAPH3 | R1191* | G | 3663 | A | DNHD1 | R1084C | C | 3814 | T | DZIP1L | L584V | G | 2113 | C |
| DARC | V106E | T | 492 | A | DIAPH3 | T917M | G | 2842 | A | DNHD1 | E3573D | G | 11283 | T | DZIP3 | T405A | A | 1443 | G |
| DARS2 | R542* | C | 2351 | T | DIAPH3 | A750V | G | 2341 | A | DNHD1 | A4570V | C | 14273 | T | DZIP3 | M783I | G | 2579 | C |
| DARS2 | E167* | G | 1226 | T | DIAPH3 | S302R | T | 996 | G | DNHD1 | R2191* | C | 7135 | T | E2F2 | R168H | C | 930 | T |
| DARS2 | L486F | C | 2183 | T | DICER1 | R1060H | C | 3361 | T | DNHD1 | A3578V | C | 11297 | T | E2F8 | R154C | G | 460 | A |
| DAXX | R306Q | C | 1122 | T | DICER1 | E1813Q | C | 5619 | G | DNHD1 | R2423C | C | 7831 | T | E2F8 | R183* | G | 547 | A |
| DAZAP1 | R130M | G | 578 | T | DICER1 | E1813Q | C | 5619 | G | DNHD1 | R3551C | C | 11215 | T | E4F1 | A134T | G | 448 | A |

| | | | | | | | | | | | | | | | | | | | |
|---|---|---|---|---|---|---|---|---|---|---|---|---|---|---|---|---|---|---|---|
| DAZL | T274M | G | 1109 | A | DICER1 | E866K | C | 2778 | T | DNHD1 | S877L | C | 3194 | T | E4F1 | R62H | G | 233 | A |
| DAZL | T177M | G | 818 | A | DICER1 | K704T | T | 2293 | G | DNHD1 | R4411C | C | 13795 | T | E4F1 | P86H | C | 305 | A |
| DAZL | - | C | 0 | T | DIDO1 | R2171H | C | 6838 | T | DNHD1 | Q1435R | A | 4868 | G | EAF1 | A5T | G | 438 | A |
| DBC1 | L90M | G | 734 | T | DIDO1 | R2207W | G | 6945 | A | DNHD1 | D148N | G | 1006 | A | EAF1 | K156T | A | 892 | C |
| DBC1 | R92H | C | 741 | T | DIDO1 | R1845C | G | 5859 | A | DNHD1 | R2222H | G | 7229 | A | EBAG9 | F19L | C | 281 | A |
| DBC1 | S530I | C | 2055 | A | DIDO1 | R37Q | C | 436 | T | DNHD1 | A3534V | C | 11165 | T | EBF1 | A405T | C | 1496 | T |
| DBC1 | M1I | C | 469 | A | DIDO1 | R62W | G | 510 | A | DNHD1 | G3538D | G | 11177 | A | EBF1 | R209W | G | 908 | A |
| DBF4B | R511C | C | 1744 | T | DIDO1 | Y1391C | T | 4498 | C | DNHD1 | L4038I | C | 12676 | A | EBF1 | R51W | G | 434 | A |
| DBH | V434M | G | 1312 | A | DIMT1L | M154V | T | 621 | C | DNHD1 | G1081R | G | 3805 | C | EBF1 | F287L | G | 1144 | T |
| DBN1 | E630K | C | 2060 | T | DIMT1L | T237I | G | 871 | A | DNHD1 | R4442H | G | 13889 | A | EBF2 | R363I | C | 1105 | A |
| DBN1 | S391A | A | 1343 | C | DIO1 | D84G | A | 257 | G | DNHD1 | L3188I | C | 10126 | A | EBF3 | M528V | T | 1655 | C |
| DBN1 | I283L | T | 1019 | G | DIO3 | V184I | G | 696 | A | DNHD1 | - | G | 0 | T | EBF3 | G582R | C | 1817 | T |
| DBNDD1 | P230L | G | 689 | A | DIP2A | I1541N | T | 4777 | A | DNHD1 | R2233C | C | 7261 | T | EBF3 | P316L | G | 1020 | A |
| DBNDD1 | I132F | T | 394 | A | DIP2A | R863W | C | 2742 | T | DNHD1 | A2283V | C | 7412 | T | EBF3 | A383V | G | 1221 | A |
| DBNL | A98V | C | 391 | T | DIP2A | Q172L | A | 670 | T | DNLZ | - | C | 0 | A | EBF3 | N287D | T | 932 | C |
| DBR1 | V441A | A | 1476 | G | DIP2A | P1070S | C | 3363 | T | DNM1 | W525* | G | 1667 | A | EBF3 | G119R | C | 428 | T |
| DBT | D127N | C | 393 | T | DIP2A | R1469W | C | 4560 | T | DNM1 | V174M | G | 612 | A | EBF3 | E399D | C | 1270 | A |
| DBX1 | P209S | G | 625 | A | DIP2A | A677T | G | 2184 | A | DNM1 | A251T | G | 843 | A | EBF4 | V274I | G | 820 | A |
| DBX2 | P118S | G | 524 | A | DIP2A | A415E | C | 1399 | A | DNM1L | R736H | G | 2209 | A | EBI3 | E213* | G | 690 | T |
| DBX2 | E41D | C | 295 | A | DIP2B | E294D | A | 1038 | T | DNM1L | D281G | A | 844 | G | EBNA1BP2 | A115T | C | 485 | T |
| DCAF10 | S333Y | C | 1363 | A | DIP2B | I235L | A | 859 | C | DNM1L | R472W | C | 1416 | T | ECE1 | R590H | C | 1769 | T |
| DCAF11 | E36D | G | 835 | T | DIP2B | R708W | C | 2278 | T | DNM1L | S411L | C | 1234 | T | ECE1 | A446V | G | 1337 | A |
| DCAF12 | D30A | T | 431 | G | DIP2B | K663M | A | 2144 | T | DNM2 | A269V | C | 970 | T | ECE1 | M645V | T | 1933 | C |
| DCAF12 | S309T | A | 1267 | T | DIP2B | V1534I | G | 4756 | A | DNM2 | D106N | G | 480 | A | ECE2 | R108H | G | 323 | A |
| DCAF12 | - | A | 0 | G | DIP2B | R1310W | C | 4084 | T | DNM2 | R672H | G | 2179 | A | ECE2 | S642Y | C | 1925 | A |
| DCAF12 | G450E | C | 1691 | T | DIP2B | K771N | A | 2469 | C | DNM3 | R818H | G | 2629 | A | ECE2 | R438C | C | 1312 | T |
| DCAF12L1 | A66T | C | 439 | T | DIP2C | W316* | C | 1035 | T | DNM3 | K315Q | A | 1119 | C | ECEL1 | A243T | C | 938 | T |
| DCAF12L1 | V125M | C | 616 | T | DIP2C | L901I | G | 2789 | T | DNMBP | S726* | G | 2269 | T | ECEL1 | A114T | C | 551 | T |
| DCAF12L1 | R76W | G | 469 | A | DIP2C | E202* | C | 692 | A | DNMBP | P1433L | G | 4390 | A | ECEL1 | V249I | C | 956 | T |
| DCAF12L1 | S173R | T | 760 | G | DIP2C | S1462P | A | 4472 | G | DNMBP | P610S | G | 1920 | A | ECH1 | R59H | C | 261 | T |
| DCAF12L2 | D414Y | C | 1267 | A | DIP2C | P479L | G | 1524 | A | DNMBP | V1590I | C | 4860 | T | ECHDC1 | T141P | T | 457 | G |
| DCAF12L2 | T131P | T | 418 | G | DIP2C | S1346L | G | 4125 | A | DNMBP | G591S | C | 1863 | T | ECHDC2 | R78C | G | 262 | A |
| DCAF12L2 | L404P | A | 1238 | G | DIRAS3 | R103H | C | 945 | T | DNMBP | - | T | 0 | C | ECHS1 | S186L | G | 913 | A |
| DCAF12L2 | R307* | G | 946 | A | DIRAS3 | E210* | C | 1265 | A | DNMBP | R1284Q | C | 3943 | T | ECM1 | R232H | G | 829 | A |
| DCAF12L2 | R137W | G | 436 | A | DIRC2 | F85L | C | 653 | A | DNMBP | R1125W | G | 3465 | A | ECM1 | R198Q | G | 727 | A |
| DCAF12L2 | R132C | G | 421 | A | DIS3 | P381H | G | 1243 | T | DNMT1 | R262Q | C | 965 | T | ECM1 | F345V | T | 1167 | G |
| DCAF13 | R459Q | G | 1376 | A | DIS3 | P381T | G | 1242 | T | DNMT1 | R974H | C | 3101 | T | ECSIT | V152I | C | 588 | T |
| DCAF13 | S55R | C | 165 | G | DIS3 | K9Q | T | 126 | G | DNMT1 | A1329T | C | 4165 | T | ECT2 | K801N | G | 2560 | T |
| DCAF15 | Q241R | A | 743 | G | DIS3L | G306* | G | 931 | T | DNMT1 | D718V | T | 2333 | A | ECT2L | R233* | C | 858 | T |

| | | | | | | | | | | | | | | | | | | | |
|---|---|---|---|---|---|---|---|---|---|---|---|---|---|---|---|---|---|---|---|
| DCAF17 | E334* | G | 1327 | T | DIS3L | V393A | T | 1193 | C | DNMT1 | I1444N | A | 4511 | T | ECT2L | S324I | G | 1132 | T |
| DCAF4 | R377Q | G | 1350 | A | DIS3L | R873C | C | 2632 | T | DNMT1 | R1008Q | C | 3203 | T | ECT2L | F663L | C | 2150 | A |
| DCAF4L1 | A189V | C | 663 | T | DIS3L | A90S | G | 283 | T | DNMT3A | A353V | G | 1396 | A | ECT2L | D97N | G | 450 | A |
| DCAF4L1 | A79V | C | 333 | T | DIS3L | Q673* | C | 2032 | T | DNMT3A | A380T | C | 1476 | T | ECT2L | R685Q | G | 2215 | A |
| DCAF4L1 | D362N | G | 1181 | A | DIS3L2 | R11Q | G | 308 | A | DNMT3B | R537W | C | 1930 | T | EDA | T338M | C | 1255 | T |
| DCAF4L2 | D72N | C | 311 | T | DISC1 | R418H | G | 1306 | A | DNMT3B | R683H | G | 2369 | A | EDA2R | Y8C | T | 23 | C |
| DCAF4L2 | G374V | C | 1218 | A | DISC1 | S24N | G | 124 | A | DNMT3B | F549L | C | 1968 | A | EDAR | A174T | C | 964 | T |
| DCAF4L2 | L349F | G | 1142 | A | DISC1 | R84K | G | 304 | A | DNMT3B | G15S | G | 364 | A | EDARADD | T5M | C | 181 | T |
| DCAF4L2 | A358T | C | 1169 | T | DISP1 | S538Y | C | 1777 | A | DNTT | E492* | G | 1576 | T | EDC4 | A445T | G | 1523 | A |
| DCAF4L2 | S129Y | G | 483 | T | DISP1 | S1043L | C | 3292 | T | DNTT | C188G | T | 664 | G | EDC4 | V203M | G | 797 | A |
| DCAF5 | S350Y | G | 1268 | T | DISP1 | T197A | A | 753 | G | DNTT | R411H | G | 1334 | A | EDEM1 | A455T | G | 1496 | A |
| DCAF5 | P758S | G | 2491 | A | DISP1 | A616V | C | 2011 | T | DNTTIP2 | T87A | T | 297 | C | EDEM2 | K266R | T | 903 | C |
| DCAF6 | R661K | G | 2076 | A | DISP1 | M464T | T | 1555 | C | DOC2A | D83G | T | 439 | C | EDEM2 | S522L | G | 1671 | A |
| DCAF6 | R243H | G | 822 | A | DISP1 | S957L | C | 3034 | T | DOC2B | P255L | G | 764 | A | EDEM3 | P746H | G | 2504 | T |
| DCAF6 | V637I | G | 2003 | A | DISP2 | R261Q | G | 869 | A | DOCK1 | E843K | G | 2591 | A | EDEM3 | S266I | C | 1064 | A |
| DCAF6 | R191Q | G | 666 | A | DISP2 | R97Q | G | 377 | A | DOCK1 | N1515T | A | 4608 | C | EDEM3 | R265Q | C | 1061 | T |
| DCAF6 | R40I | G | 213 | T | DISP2 | L79P | T | 323 | C | DOCK1 | A1665T | G | 5057 | A | EDF1 | R133Q | C | 426 | T |
| DCAF6 | P715S | C | 2237 | T | DIXDC1 | Q367H | G | 1101 | T | DOCK1 | R1327W | C | 4043 | T | EDF1 | D136N | C | 434 | T |
| DCAF8 | F376L | G | 1563 | T | DIXDC1 | T555M | C | 1664 | T | DOCK1 | R1694L | G | 5145 | T | EDF1 | L130F | G | 416 | A |
| DCAF8 | V440I | C | 1753 | T | DIXDC1 | R564Q | G | 1691 | A | DOCK1 | D600G | A | 1863 | G | EDIL3 | L137R | A | 829 | C |
| DCAF8 | G205S | C | 1048 | T | DIXDC1 | R364K | G | 1091 | A | DOCK1 | T596I | C | 1851 | T | EDIL3 | G35D | C | 523 | T |
| DCAF8 | N58S | T | 608 | C | DKK3 | E114* | C | 565 | A | DOCK1 | R1419W | C | 4319 | T | EDIL3 | Q361H | T | 1502 | G |
| DCAF8 | F347S | A | 1475 | G | DKK3 | L359P | A | 1301 | G | DOCK1 | P83L | C | 312 | T | EDN1 | R162S | A | 753 | C |
| DCBLD1 | T458M | C | 1498 | T | DLC1 | R1182H | C | 3955 | T | DOCK10 | V248M | C | 810 | T | EDN3 | R137W | C | 795 | T |
| DCC | L1198M | C | 3592 | A | DLC1 | G834S | C | 2910 | T | DOCK10 | T1953M | G | 5926 | A | EDN3 | R230H | G | 1075 | A |
| DCC | S1202N | G | 3605 | A | DLC1 | S586R | G | 2168 | T | DOCK10 | A140V | G | 487 | A | EDN3 | K188Q | A | 948 | C |
| DCC | V764I | G | 2290 | A | DLC1 | E1335* | C | 4413 | A | DOCK10 | M1216K | A | 3715 | T | EDNRA | S382L | C | 1660 | T |
| DCC | S690R | A | 2068 | C | DLC1 | L476R | A | 1837 | C | DOCK10 | A177V | G | 598 | A | EDNRA | C270Y | G | 1324 | A |
| DCDC1 | E204D | T | 814 | G | DLC1 | Q328H | C | 1394 | A | DOCK10 | L1179Q | A | 3604 | T | EDNRA | E335K | G | 1518 | A |
| DCDC1 | K315T | T | 1146 | G | DLD | F190C | T | 850 | G | DOCK10 | L1820I | G | 5526 | T | EDNRB | R100H | C | 452 | T |
| DCDC2 | V35I | C | 405 | T | DLEC1 | V243I | G | 748 | A | DOCK10 | H1430N | G | 4356 | T | EDNRB | T140N | G | 572 | T |
| DCDC2 | E442* | C | 1626 | A | DLEC1 | S844L | C | 2552 | T | DOCK10 | R352* | G | 1122 | A | EDNRB | I312M | A | 1089 | C |
| DCDC2B | - | A | 0 | G | DLEU7 | R92* | G | 324 | A | DOCK10 | F206L | G | 686 | T | EDNRB | R291* | G | 1024 | A |
| DCDC5 | P290L | G | 869 | A | DLEU7 | A158V | G | 766 | A | DOCK10 | R135* | G | 471 | A | EEA1 | A672S | C | 2279 | A |
| DCDC5 | R501W | G | 1501 | A | DLEU7 | A17V | G | 100 | A | DOCK11 | F1990C | T | 6043 | G | EEA1 | K333N | C | 1264 | A |
| DCDC5 | H318Y | G | 952 | A | DLG1 | I612V | T | 2024 | C | DOCK11 | V760A | T | 2353 | C | EEF1A1P9 | E45A | T | 1126 | G |
| DCDC5 | Q798H | T | 2392 | G | DLG1 | F660S | A | 2169 | G | DOCK11 | Q1276H | G | 3902 | T | EEF1A2 | R247C | G | 810 | A |
| DCDC5 | P740S | G | 2216 | A | DLG2 | A111D | G | 644 | T | DOCK2 | E1616V | A | 4899 | T | EEF1A2 | G446A | C | 1408 | G |
| DCDC5 | K129N | C | 387 | A | DLG2 | V296I | C | 1198 | T | DOCK2 | - | A | 0 | C | EEF1A2 | A46E | G | 208 | T |

| | | | | | | | | | | | | | | | | | | | |
|---|---|---|---|---|---|---|---|---|---|---|---|---|---|---|---|---|---|---|---|
| DCDC5 | E116* | C | 346 | A | DLG2 | T235M | G | 1016 | A | DOCK2 | M1333T | T | 4050 | C | EEF1D | A537V | G | 2069 | A |
| DCHS1 | R2064H | C | 6603 | T | DLG2 | A271V | G | 1124 | A | DOCK2 | V1175M | G | 3575 | A | EEF1D | Q182R | T | 1004 | C |
| DCHS1 | R850H | C | 2961 | T | DLG2 | G342R | C | 1336 | T | DOCK2 | P732S | C | 2246 | T | EEF2 | C41Y | C | 205 | T |
| DCHS1 | V1684E | A | 5463 | T | DLG2 | Q39H | T | 429 | G | DOCK3 | K1215N | G | 3668 | T | EEF2 | G699S | C | 2178 | T |
| DCHS1 | A1433V | G | 4710 | A | DLG4 | R614W | G | 2693 | A | DOCK3 | Y1373* | C | 4142 | A | EEF2K | A617T | G | 2310 | A |
| DCHS1 | D473Y | C | 1829 | A | DLG4 | R356Q | C | 1920 | T | DOCK3 | R1498S | G | 4517 | T | EEF2K | V388M | G | 1623 | A |
| DCHS1 | Q1310H | C | 4342 | A | DLG4 | R155S | C | 1318 | A | DOCK3 | R1857W | C | 5592 | T | EEF2K | V168M | G | 963 | A |
| DCHS2 | E2645* | C | 7933 | A | DLG5 | R249Q | C | 752 | T | DOCK3 | T555K | C | 1687 | A | EFCAB1 | D210G | T | 789 | C |
| DCHS2 | E2575D | C | 7725 | A | DLG5 | V1374I | C | 4126 | T | DOCK3 | R621W | C | 1884 | T | EFCAB2 | K211N | A | 774 | C |
| DCHS2 | D2419Y | C | 7255 | A | DLG5 | E1290K | C | 3874 | T | DOCK3 | R844C | C | 2553 | T | EFCAB2 | E145D | G | 576 | T |
| DCHS2 | Q1585H | C | 4755 | A | DLG5 | A1708T | C | 5128 | T | DOCK3 | R499Q | G | 1519 | A | EFCAB4A | Q9H | G | 748 | T |
| DCHS2 | N1424T | T | 4271 | G | DLG5 | E257Q | C | 775 | G | DOCK3 | R1774C | C | 5343 | T | EFCAB4A | R100H | G | 1020 | A |
| DCHS2 | E568K | C | 2063 | T | DLG5 | R1730Q | C | 5195 | T | DOCK3 | F236L | C | 731 | A | EFCAB4B | F705L | G | 2687 | T |
| DCHS2 | P2272H | G | 6815 | T | DLG5 | G731C | C | 2197 | A | DOCK3 | S791Y | C | 2395 | A | EFCAB5 | F1140L | C | 3612 | A |
| DCHS2 | L1214I | G | 3640 | T | DLG5 | E1472K | C | 4420 | T | DOCK3 | I1053S | T | 3181 | G | EFCAB6 | A955V | G | 3118 | A |
| DCHS2 | E127* | C | 379 | A | DLG5 | T1422A | T | 4270 | C | DOCK3 | D1457G | A | 4393 | G | EFCAB6 | R246I | C | 991 | A |
| DCHS2 | E2606K | C | 7816 | T | DLG5 | Y1390H | A | 4174 | G | DOCK4 | H1373Y | G | 4116 | A | EFCAB6 | G525D | C | 1828 | T |
| DCHS2 | C2603Y | C | 7808 | T | DLG5 | N390I | T | 1175 | A | DOCK4 | R132H | C | 394 | T | EFCAB6 | R1207C | G | 3873 | A |
| DCHS2 | E2895* | C | 8683 | A | DLG5 | D610Y | C | 1834 | A | DOCK4 | A757V | G | 2269 | A | EFCAB6 | S1239N | C | 3970 | T |
| DCHS2 | S1630Y | G | 4889 | T | DLGAP1 | A602T | C | 1871 | T | DOCK4 | L592* | A | 1774 | C | EFCAB6 | V170G | A | 763 | C |
| DCHS2 | D1176Y | C | 3526 | A | DLGAP1 | D105Y | C | 380 | A | DOCK4 | R1927H | C | 5779 | T | EFCAB6 | R429* | G | 1539 | A |
| DCHS2 | S781Y | G | 2342 | T | DLGAP1 | T126M | G | 444 | A | DOCK4 | - | A | 0 | G | EFCAB6 | F233L | A | 953 | T |
| DCK | R104Q | G | 509 | A | DLGAP1 | L945P | A | 2901 | G | DOCK4 | L1058V | A | 3171 | C | EFCAB6 | K172N | T | 770 | G |
| DCK | L216F | C | 844 | T | DLGAP1 | D506N | C | 1583 | T | DOCK4 | L1818F | G | 5451 | A | EFCAB6 | G137V | C | 664 | A |
| DCLK1 | A18V | G | 265 | A | DLGAP2 | E189D | G | 635 | T | DOCK4 | Q1247H | C | 3740 | A | EFCAB7 | V553M | G | 1903 | A |
| DCLK1 | S289* | G | 1078 | T | DLGAP2 | R912Q | G | 2803 | A | DOCK4 | N1227T | T | 3679 | G | EFCAB7 | D8N | G | 268 | A |
| DCLK2 | R780H | G | 2339 | A | DLGAP2 | R105W | C | 381 | T | DOCK4 | V758M | C | 2271 | T | EFCAB8 | T1222M | C | 3759 | T |
| DCLK2 | S392R | A | 1174 | C | DLGAP2 | Y380C | A | 1207 | G | DOCK4 | R177W | G | 528 | A | EFCAB8 | G527S | G | 1673 | A |
| DCLK3 | S10I | C | 520 | A | DLGAP2 | R912W | C | 2802 | T | DOCK5 | K1473E | A | 4461 | G | EFCAB8 | T1189M | C | 3660 | T |
| DCLK3 | A119T | C | 846 | T | DLGAP2 | R637Q | G | 1978 | A | DOCK5 | L703P | T | 2152 | C | EFCAB8 | N211T | A | 726 | C |
| DCLK3 | E523K | C | 2058 | T | DLGAP2 | F709V | T | 2193 | G | DOCK5 | A809T | G | 2469 | A | EFCAB8 | Q303H | G | 1003 | T |
| DCLRE1A | K948R | T | 3538 | C | DLGAP2 | G72R | G | 282 | A | DOCK5 | R1653H | G | 5002 | A | EFCAB8 | M794V | A | 2474 | G |
| DCLRE1A | G941C | C | 3516 | A | DLGAP3 | R593H | C | 2047 | T | DOCK5 | D331V | A | 1036 | T | EFCAB8 | N437H | A | 1403 | C |
| DCLRE1A | L381R | A | 1837 | C | DLGAP3 | G757C | C | 2538 | A | DOCK5 | A1346T | G | 4080 | A | EFEMP1 | D31N | C | 527 | T |
| DCLRE1B | T426M | C | 1723 | T | DLGAP3 | A820T | C | 2727 | T | DOCK5 | E1458* | G | 4416 | T | EFEMP1 | R362Q | C | 1521 | T |
| DCLRE1C | F393C | A | 1216 | C | DLGAP3 | P720T | G | 2427 | T | DOCK6 | L667Q | A | 2041 | T | EFEMP2 | L276M | G | 1020 | T |
| DCLRE1C | L639F | G | 1953 | A | DLGAP4 | S90R | C | 469 | A | DOCK6 | T1793M | G | 5419 | A | EFEMP2 | Y155H | A | 657 | G |
| DCLRE1C | P396T | G | 1224 | T | DLGAP4 | R617* | C | 2048 | T | DOCK6 | V1756M | C | 5307 | T | EFEMP2 | C247R | A | 933 | G |
| DCN | S214N | C | 895 | T | DLGAP4 | A774V | C | 2520 | T | DOCK6 | R323C | G | 1008 | A | EFHA2 | T477N | C | 1472 | A |

| | | | | | | | | | | | | | | | | | | | |
|---|---|---|---|---|---|---|---|---|---|---|---|---|---|---|---|---|---|---|---|
| DCN | R156C | G | 720 | A | DLGAP4 | A272T | G | 1013 | A | DOCK6 | A922V | G | 2806 | A | EFHA2 | A54V | C | 203 | T |
| DCP1A | A279V | G | 929 | A | DLGAP4 | A39V | C | 315 | T | DOCK6 | P69S | G | 246 | A | EFHA2 | A103T | G | 349 | A |
| DCP1A | R132* | G | 487 | A | DLGAP4 | S320T | T | 1157 | A | DOCK7 | V1289I | C | 3899 | T | EFHA2 | R210* | C | 670 | T |
| DCP1A | - | C | 0 | T | DLGAP4 | R515C | C | 1742 | T | DOCK7 | R854C | G | 2594 | A | EFHA2 | L395I | C | 1225 | A |
| DCP2 | T213P | A | 835 | C | DLGAP5 | T759I | G | 2493 | A | DOCK7 | R1701* | G | 5135 | A | EFHA2 | V462I | G | 1426 | A |
| DCP2 | K224T | A | 869 | C | DLGAP5 | L293V | A | 1094 | C | DOCK7 | S1177T | A | 3563 | T | EFHB | T384M | G | 1313 | A |
| DCP2 | N337T | A | 1208 | C | DLK1 | V184I | G | 792 | A | DOCK7 | S963Y | G | 2922 | T | EFHB | P106S | G | 478 | A |
| DCPS | P239Q | C | 1045 | A | DLK2 | P182H | G | 796 | T | DOCK7 | R1326Q | C | 4011 | T | EFHB | R733Q | C | 2360 | T |
| DCST1 | D418N | G | 1348 | A | DLK2 | R219H | C | 907 | T | DOCK7 | K830N | T | 2524 | G | EFHB | K494T | T | 1643 | G |
| DCST1 | D597G | A | 1886 | G | DLL1 | V644M | C | 2264 | T | DOCK8 | V759M | G | 2387 | A | EFHB | Y427* | A | 1443 | C |
| DCST2 | R365C | G | 1152 | A | DLL1 | Q645H | C | 2269 | A | DOCK8 | R934Q | G | 2913 | A | EFHB | T343A | T | 1189 | C |
| DCST2 | R299Q | C | 955 | T | DLL3 | G344S | G | 1088 | A | DOCK8 | V726A | T | 2289 | C | EFHB | K293Q | T | 1039 | G |
| DCST2 | D214N | C | 699 | T | DLL3 | - | T | 0 | C | DOCK8 | R539H | G | 1728 | A | EFHC1 | I122T | T | 468 | C |
| DCST2 | R90M | C | 328 | A | DLL3 | G325C | G | 1031 | T | DOCK8 | R1388Q | G | 4275 | A | EFHC1 | E274D | A | 925 | C |
| DCST2 | S172P | A | 573 | G | DLX2 | A120T | C | 720 | T | DOCK8 | Q81H | G | 355 | T | EFHC1 | Q584P | A | 1854 | C |
| DCT | V496A | A | 1914 | G | DLX3 | Y50H | A | 374 | G | DOCK8 | M1287T | T | 3972 | C | EFHC1 | R244Q | G | 834 | A |
| DCT | F209V | A | 1052 | C | DLX3 | R130Q | C | 615 | T | DOCK9 | - | T | 0 | C | EFHC2 | S431Y | G | 1292 | T |
| DCT | R27Q | C | 507 | T | DMAP1 | P313Q | C | 1016 | A | DOCK9 | M1429V | T | 4340 | C | EFHC2 | M508V | T | 1522 | C |
| DCTN1 | R773C | G | 2628 | A | DMAP1 | R252C | C | 832 | T | DOCK9 | R1763W | G | 5342 | A | EFHC2 | C57W | G | 171 | C |
| DCTN1 | R1219H | C | 3967 | T | DMBT1 | V626M | G | 1982 | A | DOCK9 | M2049I | C | 6202 | A | EFHC2 | R228W | G | 682 | A |
| DCTN1 | R953Q | C | 3169 | T | DMBT1 | G577D | G | 1836 | A | DOCK9 | I1493V | T | 4532 | C | EFHD1 | A57T | G | 646 | A |
| DCTN2 | L178P | A | 679 | G | DMBT1 | W1077* | G | 3337 | A | DOHH | P151L | G | 615 | A | EFHD2 | I112V | A | 411 | G |
| DCTN4 | I359S | A | 1178 | C | DMBT1 | R2027C | C | 6185 | T | DOK1 | K334Q | A | 1669 | C | EFNA1 | A16T | G | 564 | A |
| DCTN5 | P158L | C | 624 | T | DMBT1 | R2537Q | G | 7716 | A | DOK1 | A410D | C | 1898 | A | EFNA2 | V90M | G | 268 | A |
| DCTN6 | N105T | A | 401 | C | DMBX1 | R72C | C | 220 | T | DOK2 | G4R | C | 93 | T | EFNB2 | G309S | C | 1075 | T |
| DCTPP1 | E66G | T | 292 | C | DMBX1 | Y321C | A | 968 | G | DOK2 | R91H | C | 355 | T | EFNB3 | R106H | G | 714 | A |
| DCUN1D1 | K166N | C | 652 | A | DMD | I2353M | A | 7266 | C | DOK3 | V96M | C | 291 | T | EFNB3 | R106C | C | 713 | T |
| DCUN1D3 | R37H | C | 396 | T | DMD | I2236T | A | 6914 | G | DOK5 | R295Q | G | 1234 | A | EFNB3 | R262H | G | 1182 | A |
| DCUN1D5 | N140S | T | 687 | C | DMD | A3492T | C | 1068 1 | T | DOK5 | R283H | G | 1198 | A | EFR3A | A449V | C | 1571 | T |
| DCX | S63N | C | 360 | T | DMD | A2986V | G | 9164 | A | DOK6 | S262Y | C | 975 | A | EFR3A | A575V | C | 1949 | T |
| DDA1 | A91V | C | 399 | T | DMD | F911V | A | 2938 | C | DOLK | K321N | C | 1393 | A | EFR3A | V453A | T | 1583 | C |
| DDA1 | - | T | 434 | C | DMD | L460V | A | 1585 | C | DOPEY1 | E869G | A | 2866 | G | EFR3A | F592V | T | 1999 | G |
| DDAH1 | E137D | T | 506 | A | DMD | Y3092H | A | 9481 | G | DOPEY1 | D481G | A | 1702 | G | EFR3A | R628Q | G | 2108 | A |
| DDB1 | R1138Q | C | 3639 | T | DMD | R3391Q | C | 1037 9 | T | DOPEY1 | M1062V | A | 3444 | G | EFR3B | R237W | C | 892 | T |
| DDB1 | T985A | T | 3179 | C | DMD | A203T | C | 814 | T | DOPEY1 | G2269W | G | 7065 | T | EFR3B | Q578R | A | 1916 | G |
| DDB1 | I258T | A | 999 | G | DMD | S1511I | C | 4739 | A | DOPEY2 | R1915Q | G | 5829 | A | EFR3B | F473I | T | 1600 | A |
| DDC | K432N | T | 1365 | G | DMD | G754R | C | 2467 | G | DOPEY2 | R746H | G | 2322 | A | EFR3B | N538S | A | 1796 | G |
| DDHD1 | L580I | G | 1738 | T | DMD | R3381* | G | 1034 | A | DOPEY2 | S1139N | G | 3501 | A | EFTUD1 | C379Y | C | 1305 | T |

| | | | | | | | | 8 | | | | | | | | | | | |
|---|---|---|---|---|---|---|---|---|---|---|---|---|---|---|---|---|---|---|---|
| DDHD2 | S25Y | C | 353 | A | DMD | D1916G | T | 5954 | C | DOPEY2 | R2032H | G | 6180 | A | EFTUD1 | R1095* | G | 3452 | A |
| DDHD2 | R476K | G | 1706 | A | DMD | R1728H | C | 5390 | T | DOPEY2 | Y297C | A | 975 | G | EFTUD1 | V495A | A | 1653 | G |
| DDHD2 | R570Q | G | 1988 | A | DMD | A798V | G | 2600 | A | DOPEY2 | T778M | C | 2418 | T | EFTUD2 | R495W | G | 1744 | A |
| DDHD2 | D707Y | G | 2398 | T | DMD | L553I | G | 1864 | T | DOPEY2 | R1626Q | G | 4962 | A | EFTUD2 | L617I | G | 2110 | T |
| DDI1 | R83Q | G | 491 | A | DMD | D2505G | T | 7721 | C | DOPEY2 | E2286* | G | 6941 | T | EFTUD2 | A445T | C | 1594 | T |
| DDI1 | V293A | T | 1121 | C | DMKN | A415V | G | 1421 | A | DOT1L | T431A | A | 1327 | G | EFTUD2 | Q892H | C | 2937 | A |
| DDI1 | Q211R | A | 875 | G | DMP1 | S435C | C | 1403 | G | DOT1L | R411H | G | 1268 | A | EGF | C380Y | G | 1584 | A |
| DDI1 | D343N | G | 1270 | A | DMPK | G577D | C | 2274 | T | DOT1L | A195T | G | 619 | A | EGF | Q510* | C | 1973 | T |
| DDI2 | R267M | G | 971 | T | DMPK | T286M | G | 1401 | A | DOT1L | A1432T | G | 4330 | A | EGF | R394* | C | 1625 | T |
| DDIT3 | A154V | G | 641 | A | DMRT1 | M1L | A | 150 | C | DOT1L | P1260L | C | 3815 | T | EGF | S226Y | C | 1122 | A |
| DDIT3 | Q26* | G | 256 | A | DMRT1 | S347I | G | 1189 | T | DOT1L | R413H | G | 1274 | A | EGF | S1064L | C | 3636 | T |
| DDOST | R139* | G | 521 | A | DMRT1 | A42V | C | 274 | T | DOT1L | R344H | G | 1067 | A | EGFL7 | A156T | G | 1377 | A |
| DDR2 | F424L | T | 1708 | C | DMRT1 | E110D | G | 479 | T | DOT1L | K550Q | A | 1684 | C | EGFLAM | K288N | G | 1210 | T |
| DDR2 | K616N | G | 2286 | T | DMRT2 | R212H | G | 835 | A | DOT1L | A370T | G | 1144 | A | EGFLAM | R802M | G | 2751 | T |
| DDX1 | I36L | A | 495 | C | DMRT2 | P470L | C | 1609 | T | DPAGT1 | F284L | A | 1073 | G | EGFLAM | R924W | C | 3116 | T |
| DDX10 | V638A | T | 1990 | C | DMRT2 | N301I | A | 1102 | T | DPCD | R11W | C | 80 | T | EGFLAM | A354D | C | 1407 | A |
| DDX10 | N59T | A | 253 | C | DMRT3 | S463L | C | 1426 | T | DPEP1 | L153P | T | 661 | C | EGFLAM | R368Q | G | 1449 | A |
| DDX11 | G689S | G | 2316 | A | DMRTA1 | N384H | A | 1375 | C | DPEP1 | R110W | C | 531 | T | EGFR | R427C | C | 1456 | T |
| DDX11 | S96Y | C | 538 | A | DMRTA1 | T94M | C | 506 | T | DPEP1 | R198C | C | 795 | T | EGFR | R671C | C | 2188 | T |
| DDX11L8 | N788S | A | 2363 | G | DMRTA2 | R205H | C | 1007 | T | DPEP2 | R110H | C | 381 | T | EGFR | R165Q | G | 671 | A |
| DDX12 | A375V | G | 1375 | A | DMRTB1 | A52V | C | 210 | T | DPEP2 | M54V | T | 212 | C | EGFR | R671C | C | 2188 | T |
| DDX17 | E485K | C | 1553 | T | DMRTB1 | Y288H | T | 917 | C | DPEP3 | R234H | C | 1056 | T | EGLN1 | E375V | T | 4280 | A |
| DDX17 | T667A | T | 2099 | C | DMRTC2 | Q268R | A | 886 | G | DPEP3 | N135T | T | 759 | G | EGLN1 | K291R | T | 4028 | C |
| DDX18 | D176E | T | 656 | A | DMTF1 | S593G | A | 2103 | G | DPF1 | R110I | C | 329 | A | EGLN2 | R233C | C | 1051 | T |
| DDX18 | S559Y | C | 1804 | A | DMTF1 | R265* | C | 1119 | T | DPF1 | R343Q | C | 1028 | T | EGLN2 | P403Q | C | 1562 | A |
| DDX18 | R539H | G | 1744 | A | DMTF1 | T315A | A | 1269 | G | DPF1 | K269R | T | 806 | C | EGLN2 | R355W | C | 1417 | T |
| DDX19A | T21P | A | 224 | C | DMTF1 | P603T | C | 2133 | A | DPF3 | R79H | C | 236 | T | EGR2 | P369S | G | 1431 | A |
| DDX19B | - | T | 0 | C | DMWD | R100C | G | 344 | A | DPH1 | L174I | C | 520 | A | EGR2 | S429I | C | 1612 | A |
| DDX20 | E141A | A | 750 | C | DMWD | R586C | G | 1802 | A | DPH1 | P55H | C | 164 | A | EGR2 | R353H | C | 1384 | T |
| DDX21 | R440H | G | 1417 | A | DMWD | S354P | A | 1106 | G | DPH1 | A379V | C | 1136 | T | EGR3 | A139T | C | 773 | T |
| DDX21 | R440C | C | 1416 | T | DMWD | K271N | C | 859 | A | DPH2 | I104V | A | 482 | G | EGR3 | P269L | G | 1164 | A |
| DDX21 | I245L | A | 831 | C | DMXL1 | R1340W | C | 4098 | T | DPH3 | L79* | A | 332 | C | EGR3 | T124M | G | 729 | A |
| DDX24 | K96N | C | 387 | A | DMXL1 | H2570Y | C | 7788 | T | DPP10 | R91Q | G | 627 | A | EGR4 | A39V | G | 504 | A |
| DDX24 | P217L | G | 749 | A | DMXL1 | R1678C | C | 5112 | T | DPP10 | K362E | A | 1439 | G | EHBP1 | F584L | C | 2234 | A |
| DDX24 | Y457H | A | 1468 | G | DMXL1 | H1865P | A | 5674 | C | DPP10 | L144I | C | 785 | A | EHBP1 | K398N | A | 1676 | C |
| DDX24 | R793C | G | 2476 | A | DMXL1 | - | G | 0 | T | DPP10 | R602I | G | 2160 | T | EHBP1L1 | V1162M | G | 3749 | A |
| DDX25 | A2V | C | 146 | T | DMXL1 | V2518I | G | 7632 | A | DPP4 | D725N | C | 2734 | T | EHBP1L1 | E800K | G | 2663 | A |
| DDX25 | A472T | G | 1555 | A | DMXL1 | D2808G | A | 8503 | G | DPP4 | E117* | C | 910 | A | EHD1 | K13Q | T | 292 | G |

| | | | | | | | | | | | | | | | | | | | |
|---|---|---|---|---|---|---|---|---|---|---|---|---|---|---|---|---|---|---|---|
| DDX25 | G442S | G | 1465 | A | DMXL1 | K92N | G | 356 | T | DPP6 | V16M | G | 633 | A | EHD2 | T246M | C | 987 | T |
| DDX26B | A825T | G | 2807 | A | DMXL1 | N201S | A | 682 | G | DPP6 | Y825C | A | 2615 | G | EHD2 | P319S | C | 1205 | T |
| DDX26B | R345Q | G | 1368 | A | DMXL2 | R1362Q | C | 4292 | T | DPP6 | Y493C | A | 1619 | G | EHD2 | V174M | G | 770 | A |
| DDX26B | F738C | T | 2547 | G | DMXL2 | G1188S | C | 3769 | T | DPP7 | S168R | T | 507 | G | EHD3 | R167W | C | 784 | T |
| DDX27 | R713Q | G | 2147 | A | DMXL2 | - | A | 0 | G | DPP7 | D237G | T | 715 | C | EHD3 | K461E | A | 1666 | G |
| DDX28 | A433V | G | 2152 | A | DMXL2 | M2662I | C | 8193 | T | DPP7 | L429P | A | 1291 | G | EHD3 | R287Q | G | 1145 | A |
| DDX28 | H359Q | G | 1931 | T | DMXL2 | R2794Q | C | 8588 | T | DPP7 | F63L | G | 194 | T | EHD3 | E91D | G | 558 | T |
| DDX28 | P506L | G | 2371 | A | DMXL2 | P937T | G | 3016 | T | DPP8 | A269T | C | 2386 | T | EHD3 | E513D | G | 1824 | T |
| DDX28 | Q428* | G | 2136 | A | DMXL2 | T873P | T | 2824 | G | DPP8 | M21T | A | 1643 | G | EHD4 | V444M | C | 1414 | T |
| DDX31 | A620S | C | 2010 | A | DMXL2 | L2294I | G | 7087 | T | DPP9 | R231W | G | 732 | A | EHD4 | F203L | G | 693 | T |
| DDX31 | R590W | G | 1920 | A | DMXL2 | R829Q | C | 2693 | T | DPP9 | A860T | C | 2619 | T | EHF | L3P | T | 119 | C |
| DDX31 | I732V | T | 2346 | C | DNA2 | E164* | C | 490 | A | DPP9 | T408M | G | 1264 | A | EHF | R278C | C | 943 | T |
| DDX31 | Q602* | G | 1956 | A | DNAH1 | K2332N | G | 6996 | T | DPPA4 | V193L | C | 632 | A | EHF | P13H | C | 149 | A |
| DDX31 | K775T | T | 2476 | G | DNAH1 | K1062E | A | 3184 | G | DPPA5 | P113T | G | 407 | T | EHF | F288C | T | 974 | G |
| DDX31 | D657N | C | 2121 | T | DNAH1 | A3042T | G | 9124 | A | DPY19L1 | A243V | G | 873 | A | EHHADH | G286R | C | 932 | T |
| DDX39 | V140I | C | 418 | T | DNAH1 | S295N | G | 884 | A | DPY19L1 | A114V | G | 486 | A | EHMT1 | A63T | G | 214 | A |
| DDX39 | R223Q | C | 668 | T | DNAH1 | R1827H | G | 5480 | A | DPY19L3 | E552* | G | 1869 | T | EHMT1 | Y952H | T | 2881 | C |
| DDX3X | T498A | A | 2347 | G | DNAH1 | A162T | G | 484 | A | DPY19L4 | T242A | A | 848 | G | EHMT1 | V651M | G | 1978 | A |
| DDX4 | G180D | G | 631 | A | DNAH1 | R1132H | G | 3395 | A | DPY19L4 | R14I | G | 165 | T | EHMT1 | D1176Y | G | 3553 | T |
| DDX4 | L671F | G | 2105 | T | DNAH1 | A1993V | C | 5978 | T | DPY19L4 | R613I | G | 1962 | T | EI24 | L286R | T | 857 | G |
| DDX41 | S544L | G | 1631 | A | DNAH1 | R597H | G | 1790 | A | DPYD | R561Q | C | 1783 | T | EI24 | C92Y | G | 275 | A |
| DDX41 | G331S | C | 991 | T | DNAH1 | A825V | C | 2474 | T | DPYD | D11N | C | 132 | T | EI24 | E55* | G | 163 | T |
| DDX41 | E274D | C | 822 | A | DNAH1 | S2487I | G | 7460 | T | DPYD | A112T | C | 435 | T | EIF1AY | R13L | G | 185 | T |
| DDX42 | - | G | 0 | A | DNAH1 | R3672W | C | 11014 | T | DPYD | R561Q | C | 1783 | T | EIF2A | K388N | A | 1273 | C |
| DDX42 | R827Q | G | 2715 | A | DNAH1 | R3776L | G | 11327 | T | DPYD | A340S | C | 1119 | A | EIF2AK1 | K421Q | T | 1408 | G |
| DDX43 | S340* | C | 1177 | A | DNAH1 | R374H | G | 1121 | A | DPYS | A469G | G | 1535 | C | EIF2AK3 | R541H | C | 1924 | T |
| DDX43 | I114M | T | 500 | G | DNAH1 | R3696C | C | 11086 | T | DPYS | K368N | T | 1233 | G | EIF2AK3 | R1065* | G | 3495 | A |
| DDX46 | R354Q | G | 1219 | A | DNAH1 | K689R | A | 2066 | G | DPYSL2 | A193T | G | 938 | A | EIF2AK3 | A704T | C | 2412 | T |
| DDX49 | T350M | C | 1106 | T | DNAH1 | I802V | A | 2404 | G | DPYSL2 | I24L | A | 431 | C | EIF2AK3 | T537K | G | 1912 | T |
| DDX5 | E81D | T | 413 | G | DNAH1 | L1554P | T | 4661 | C | DPYSL3 | D185N | C | 751 | T | EIF2AK3 | K125Q | T | 675 | G |
| DDX50 | T188A | A | 669 | G | DNAH1 | P3179L | C | 9536 | T | DPYSL4 | R75M | G | 388 | T | EIF2AK4 | R483H | G | 1498 | A |
| DDX51 | I202T | A | 644 | G | DNAH10 | R72I | G | 240 | T | DPYSL4 | V51I | G | 315 | A | EIF2AK4 | L1588V | T | 4812 | G |
| DDX53 | S189F | C | 654 | T | DNAH10 | S4252Y | C | 12780 | A | DPYSL4 | A260V | C | 943 | T | EIF2B3 | K148E | T | 569 | C |
| DDX53 | T561A | A | 1769 | G | DNAH10 | R3394W | C | 10205 | T | DPYSL4 | - | G | 0 | T | EIF2C1 | R712H | G | 2348 | A |
| DDX54 | V303M | C | 935 | T | DNAH10 | R346C | C | 1061 | T | DPYSL4 | H36R | A | 271 | G | EIF2C2 | R663H | C | 2028 | T |
| DDX54 | E636K | C | 1934 | T | DNAH10 | V728A | T | 2208 | C | DPYSL5 | Y472C | A | 1573 | G | EIF2C2 | R837* | G | 2549 | A |

| | | | | | | | | | | | | | | | | | | | |
|---|---|---|---|---|---|---|---|---|---|---|---|---|---|---|---|---|---|---|---|
| DDX55 | I40T | T | 143 | C | DNAH10 | A3666T | G | 11021 | A | DPYSL5 | E223D | G | 827 | C | EIF2C3 | R689* | C | 2414 | T |
| DDX55 | P13H | C | 62 | A | DNAH10 | H3875Q | T | 11650 | A | DQX1 | P72S | G | 434 | A | EIF2C4 | R58H | G | 418 | A |
| DDX55 | R584I | G | 1775 | T | DNAH10 | S1615P | T | 4868 | C | DQX1 | H223D | G | 887 | C | EIF2C4 | R56W | C | 411 | T |
| DDX56 | E167K | C | 606 | T | DNAH10 | T4453R | C | 13383 | G | DQX1 | L601F | G | 2021 | A | EIF2S2 | R329H | C | 1208 | T |
| DDX58 | K508R | T | 1681 | C | DNAH10 | R1184* | C | 3575 | T | DQX1 | R572Q | C | 1935 | T | EIF2S2 | T111P | T | 553 | G |
| DDX59 | R318H | C | 1167 | T | DNAH10 | R3699H | G | 11121 | A | DRAM1 | G106R | G | 779 | A | EIF2S2 | L54V | A | 382 | C |
| DDX59 | V342A | A | 1239 | G | DNAH10 | R4071W | C | 12236 | T | DRAM2 | F25L | A | 693 | C | EIF3A | R1087W | G | 3387 | A |
| DDX59 | E170* | C | 722 | A | DNAH10 | A1616T | G | 4871 | A | DRAP1 | R126W | C | 621 | T | EIF3A | E875D | C | 2753 | G |
| DDX59 | R113C | G | 551 | A | DNAH10 | E3804* | G | 11435 | T | DRD1 | R23C | G | 1012 | A | EIF3A | E774K | C | 2448 | T |
| DDX59 | S617Y | G | 2064 | T | DNAH10 | E1432K | G | 4319 | A | DRD2 | R145C | G | 668 | A | EIF3B | K604E | A | 1810 | G |
| DDX59 | R264Q | C | 1005 | T | DNAH10 | R1562I | G | 4710 | T | DRD2 | R296L | C | 1122 | A | EIF3B | R664C | C | 1990 | T |
| DDX59 | E183D | T | 763 | G | DNAH10 | S2371Y | C | 7137 | A | DRD3 | A393V | G | 1468 | A | EIF3D | K35N | T | 176 | G |
| DDX6 | - | C | 0 | A | DNAH10 | G2577C | G | 7754 | T | DRD3 | S307L | G | 1210 | A | EIF3D | R95Q | C | 355 | T |
| DDX60 | R261C | G | 1073 | A | DNAH10 | D3705G | A | 11139 | G | DRD3 | A161V | G | 772 | A | EIF3E | Q247H | C | 769 | A |
| DDX60L | H251Y | G | 972 | A | DNAH10 | K4036T | A | 12132 | C | DRD4 | R412C | C | 1246 | T | EIF3E | R271W | G | 839 | A |
| DDX60L | S1562N | C | 4906 | T | DNAH11 | I1088S | T | 3294 | G | DRGX | R35H | C | 104 | T | EIF3E | L26I | G | 104 | T |
| DDX60L | F1520V | A | 4779 | C | DNAH11 | R2075C | C | 6254 | T | DRGX | R207C | G | 619 | A | EIF3G | V312I | C | 998 | T |
| DDX60L | E669D | T | 2228 | G | DNAH11 | S2452L | C | 7386 | T | DRGX | K134T | T | 401 | G | EIF3H | R294C | G | 880 | A |
| DDX60L | S428A | A | 1503 | C | DNAH11 | R2852Q | G | 8586 | A | DRGX | C187* | G | 561 | T | EIF3H | E193* | C | 577 | A |
| DEAF1 | T472M | G | 2108 | A | DNAH11 | A3214V | C | 9672 | T | DRP2 | D716N | G | 2414 | A | EIF3J | F179L | T | 675 | G |
| DECR2 | T274S | A | 958 | T | DNAH11 | V2080A | T | 6270 | C | DRP2 | A406D | C | 1485 | A | EIF3J | V251A | T | 890 | C |
| DECR2 | T55M | C | 302 | T | DNAH11 | R2879C | C | 8666 | T | DRP2 | R840C | C | 2786 | T | EIF3J | A2V | C | 143 | T |
| DEDD | R43C | G | 362 | A | DNAH11 | G3149S | G | 9476 | A | DSC1 | Q164H | C | 754 | A | EIF3K | Y21C | A | 249 | G |
| DEDD2 | R276W | G | 894 | A | DNAH11 | S692R | A | 2105 | C | DSC1 | K538T | T | 1875 | G | EIF3L | I571V | A | 1711 | G |
| DEDD2 | R172W | G | 582 | A | DNAH11 | S3156N | G | 9498 | A | DSC3 | G487R | C | 1614 | T | EIF3L | I127V | A | 379 | G |
| DEDD2 | S254P | A | 828 | G | DNAH11 | T3790P | A | 11399 | C | DSCAM | T1728M | G | 5661 | A | EIF3L | T419M | C | 1256 | T |
| DEF6 | K583M | A | 1753 | T | DNAH11 | A1281V | C | 3873 | T | DSCAM | R1644Q | C | 5409 | T | EIF4A1P4 | S7F | C | 156 | T |
| DEF6 | L229P | T | 691 | C | DNAH11 | A1281V | C | 3873 | T | DSCAM | I877K | A | 3108 | T | EIF4A1P4 | S2Y | C | 141 | A |
| DEF8 | R72W | C | 303 | T | DNAH11 | Q1014* | C | 3071 | T | DSCAM | T651M | G | 2430 | A | EIF4A3 | R172C | G | 692 | A |
| DEF8 | D406N | G | 1305 | A | DNAH11 | Y2336H | T | 7037 | C | DSCAM | D775E | G | 2803 | T | EIF4B | R246C | C | 942 | T |
| DEFB110 | G27V | C | 126 | A | DNAH11 | V718A | T | 2184 | C | DSCAM | A676T | C | 2504 | T | EIF4B | R167* | C | 705 | T |
| DEFB112 | S53* | G | 158 | T | DNAH11 | - | G | 0 | T | DSCAM | L544F | G | 2108 | A | EIF4E1B | P30H | C | 673 | A |
| DEFB114 | R32C | G | 94 | A | DNAH11 | E259* | G | 806 | T | DSCAM | A1167S | C | 3977 | A | EIF4E1B | R172G | A | 1098 | G |

| | | | | | | | | | | | | | | | | | | | |
|---|---|---|---|---|---|---|---|---|---|---|---|---|---|---|---|---|---|---|---|
| DEFB115 | A30V | C | 89 | T | DNAH11 | N762K | T | 2317 | G | DSCAM | S870A | A | 3086 | C | EIF4E1B | N37H | A | 693 | C |
| DEFB121 | A75E | G | 338 | T | DNAH11 | R1562Q | G | 4716 | A | DSCAM | L751F | C | 2731 | G | EIF4E2 | P224S | C | 1343 | T |
| DEFB123 | P66S | C | 376 | T | DNAH11 | E1624* | G | 4901 | T | DSCAM | R498Q | C | 1971 | T | EIF4E3 | E160* | C | 486 | A |
| DEFB129 | E151* | G | 482 | T | DNAH11 | F2107V | T | 6350 | G | DSCAML1 | D224Y | C | 670 | A | EIF4E3 | V206I | C | 624 | T |
| DEFB133 | R39* | G | 115 | A | DNAH11 | K2359E | A | 7106 | G | DSCAML1 | P1014L | G | 3041 | A | EIF4E3 | - | C | 0 | A |
| DEFB133 | F51L | G | 153 | T | DNAH11 | T3013M | C | 9069 | T | DSCAML1 | A151T | C | 451 | T | EIF4ENIF1 | R255* | G | 987 | A |
| DEFB134 | D15G | T | 105 | C | DNAH11 | E3700* | G | 11129 | T | DSCAML1 | A1604S | C | 4810 | A | EIF4ENIF1 | A73D | G | 442 | T |
| DEFB135 | A2T | G | 4 | A | DNAH11 | R790G | A | 2399 | G | DSCAML1 | V1762I | C | 5284 | T | EIF4ENIF1 | A258V | G | 997 | A |
| DEFB136 | N63D | T | 187 | C | DNAH11 | D1494A | A | 4512 | C | DSCAML1 | R1368Q | C | 4103 | T | EIF4ENIF1 | D88H | C | 486 | G |
| DEGS1 | F236L | C | 874 | A | DNAH12 | R2241I | C | 6903 | A | DSCAML1 | V678G | A | 2033 | C | EIF4ENIF1 | S679Y | G | 2260 | T |
| DENND1A | P472L | G | 1617 | A | DNAH12 | R622H | C | 2046 | T | DSCAML1 | D629Y | C | 1885 | A | EIF4ENIF1 | E234K | C | 924 | T |
| DENND1A | R597H | C | 1992 | T | DNAH12 | Y1559H | A | 4856 | G | DSCC1 | E258K | C | 907 | T | EIF4G1 | P671T | C | 2210 | A |
| DENND1A | P767S | G | 2501 | A | DNAH12 | K2360T | T | 7260 | G | DSCR3 | A61T | C | 419 | T | EIF4G2 | - | A | 0 | G |
| DENND1C | R420* | G | 1350 | A | DNAH12 | T1550M | G | 4830 | A | DSCR3 | N275T | T | 1062 | G | EIF4G2 | P428L | G | 1283 | A |
| DENND1C | A760T | C | 2370 | T | DNAH12 | E1048K | C | 3323 | T | DSCR4 | S61R | T | 265 | G | EIF4G2 | F111L | A | 331 | G |
| DENND1C | R515H | C | 1636 | T | DNAH12 | K751R | T | 2433 | C | DSCR6 | P137L | C | 620 | T | EIF4G2 | R787H | C | 2360 | T |
| DENND2A | S572Y | G | 2133 | T | DNAH12 | D2196V | T | 6768 | A | DSE | R6W | C | 210 | T | EIF4G3 | P132L | G | 590 | A |
| DENND2A | Q29* | G | 503 | A | DNAH14 | E471* | G | 1626 | T | DSE | F480S | T | 1633 | C | EIF4G3 | D852E | A | 2751 | C |
| DENND2A | S572Y | G | 2133 | T | DNAH14 | F4379L | C | 13352 | A | DSEL | K56R | T | 1641 | C | EIF4G3 | R517Q | C | 1745 | T |
| DENND2C | R862H | C | 3211 | T | DNAH14 | R2804C | C | 8625 | T | DSEL | Y1177C | T | 5004 | C | EIF5 | R15H | G | 566 | A |
| DENND2C | R658* | G | 2598 | A | DNAH14 | L1938I | T | 6027 | A | DSEL | K56R | T | 1641 | C | EIF5 | E384* | G | 1672 | T |
| DENND2C | K546N | C | 2264 | A | DNAH14 | G1594* | G | 4995 | T | DSEL | P1158H | G | 4947 | T | EIF5 | N170H | A | 1030 | C |
| DENND2C | R798Q | C | 3019 | T | DNAH14 | L136P | T | 622 | C | DSEL | C838R | A | 3986 | G | EIF5 | E262* | G | 1306 | T |
| DENND2C | E760K | C | 2904 | T | DNAH14 | E206* | G | 831 | T | DSEL | L247F | C | 2215 | A | EIF5B | E602G | A | 2007 | G |
| DENND2C | F540C | A | 2245 | C | DNAH14 | F769V | T | 2520 | G | DSEL | L243F | G | 2201 | A | EIF5B | - | G | 0 | A |
| DENND2D | Q78* | G | 462 | A | DNAH14 | I784M | T | 2567 | G | DSEL | A573V | G | 3192 | A | EIF5B | S990F | C | 3171 | T |
| DENND3 | V1004M | G | 3288 | A | DNAH14 | L928M | C | 2997 | A | DSEL | R90H | C | 1743 | T | ELAC1 | A139V | C | 523 | T |
| DENND3 | A25V | C | 352 | T | DNAH14 | S3858Y | C | 11788 | A | DSEL | Q482H | C | 2920 | A | ELAC2 | Q810* | G | 2502 | A |
| DENND3 | D1073Y | G | 3495 | T | DNAH14 | L4223* | T | 12883 | G | DSEL | F123S | A | 1842 | G | ELAC2 | V790M | C | 2442 | T |
| DENND4A | H174Q | G | 907 | C | DNAH17 | T3693M | G | 11078 | A | DSG1 | A184T | G | 762 | A | ELAC2 | R477I | C | 1504 | A |
| DENND4A | L1732I | A | 5579 | T | DNAH17 | A2801V | G | 8402 | A | DSG1 | F747L | C | 2453 | A | ELAVL1 | V166M | C | 496 | T |
| DENND4A | R1544* | G | 5015 | A | DNAH17 | A4222V | G | 12665 | A | DSG2 | P538L | C | 1801 | T | ELAVL2 | P326H | G | 1003 | T |
| DENND4A | R365Q | C | 1479 | T | DNAH17 | T305M | G | 914 | A | DSG2 | A641T | G | 2109 | A | ELAVL2 | R337H | C | 1036 | T |
| DENND4A | R865H | C | 2979 | T | DNAH17 | S3407G | T | 10219 | C | DSG2 | F310V | T | 1116 | G | ELAVL2 | Q80R | T | 265 | C |

| | | | | | | | | | | | | | | | | | | | |
|---|---|---|---|---|---|---|---|---|---|---|---|---|---|---|---|---|---|---|---|
| DENND4A | A129T | C | 770 | T | DNAH17 | G2869V | C | 8606 | A | DSG2 | D509N | G | 1713 | A | ELAVL4 | N138S | A | 728 | G |
| DENND4B | R347C | G | 1440 | A | DNAH17 | S3949N | C | 11846 | T | DSG3 | R429H | G | 1369 | A | ELF1 | L468V | A | 1717 | C |
| DENND4B | P228L | G | 1084 | A | DNAH17 | N3281S | T | 9842 | C | DSG3 | R67I | G | 283 | T | ELF1 | E158* | C | 787 | A |
| DENND4B | R1348Q | C | 4444 | T | DNAH17 | R3741W | G | 11221 | A | DSG3 | V538I | G | 1695 | A | ELF2 | Q471R | T | 1618 | C |
| DENND4B | P328R | G | 1384 | C | DNAH17 | G1501S | C | 4501 | T | DSG3 | A195T | G | 666 | A | ELF2 | K458N | T | 1580 | G |
| DENND4B | S1278L | G | 4234 | A | DNAH17 | R488H | C | 1463 | T | DSG3 | Q341K | C | 1104 | A | ELF3 | A32V | C | 3287 | T |
| DENND4B | R97H | C | 691 | T | DNAH17 | E3617K | C | 10849 | T | DSG3 | V260G | T | 862 | G | ELF3 | G118W | G | 3544 | T |
| DENND4C | H1335Y | C | 4036 | T | DNAH17 | E3573K | C | 10717 | T | DSG3 | I405S | T | 1297 | G | ELFN2 | A353V | G | 1063 | A |
| DENND4C | E1155D | A | 3498 | T | DNAH17 | P3144L | G | 9431 | A | DSG3 | E498K | G | 1575 | A | ELFN2 | R598Q | C | 1798 | T |
| DENND4C | S828L | C | 2516 | T | DNAH17 | A2834V | G | 8501 | A | DSG4 | R128Q | G | 518 | A | ELFN2 | R423H | C | 1273 | T |
| DENND5A | P992H | G | 3230 | T | DNAH17 | F2702L | G | 8106 | T | DSG4 | G634D | G | 2036 | A | ELK1 | R335W | G | 1103 | A |
| DENND5A | P619H | G | 2111 | T | DNAH17 | E1290D | C | 3870 | A | DSG4 | E270D | G | 945 | T | ELK1 | K315N | C | 1045 | A |
| DENND5A | S1157L | G | 3725 | A | DNAH17 | A522T | C | 1564 | T | DSG4 | L310I | C | 1063 | A | ELK3 | I76T | T | 506 | C |
| DENND5B | R707C | G | 2119 | A | DNAH2 | E240* | G | 732 | T | DSG4 | T927N | C | 2915 | A | ELK3 | D310N | G | 1207 | A |
| DENND5B | G1125V | C | 3374 | A | DNAH2 | F1587L | C | 4775 | A | DSP | R1738* | C | 5553 | T | ELK3 | A42T | G | 403 | A |
| DENND5B | M506I | C | 1518 | A | DNAH2 | R1991H | G | 5986 | A | DSP | N1197T | A | 3931 | C | ELK3 | K192T | A | 854 | C |
| DENND5B | A309V | G | 926 | A | DNAH2 | D2109N | G | 6339 | A | DSP | R2586* | C | 8097 | T | ELL | A254T | C | 813 | T |
| DENND5B | H359Y | G | 1075 | A | DNAH2 | R1978H | G | 5947 | A | DSP | R2586Q | G | 8098 | A | ELL | R522C | G | 1617 | A |
| DENND5B | R723C | G | 2167 | A | DNAH2 | R2255C | C | 6777 | T | DSP | R1308W | C | 4263 | T | ELL2 | R209M | C | 976 | A |
| DENND5B | R934* | G | 2800 | A | DNAH2 | M3250L | A | 9762 | C | DSPP | E129* | G | 505 | T | ELL2 | P384S | G | 1500 | A |
| DENND5B | D607E | A | 1821 | C | DNAH2 | V3443M | G | 10341 | A | DSPP | N157H | A | 589 | C | ELMO1 | R76* | G | 874 | A |
| DENND5B | L268I | G | 802 | T | DNAH2 | S4269N | G | 12820 | A | DSPP | N440S | A | 1439 | G | ELMO2 | R574* | G | 1930 | A |
| DENND5B | D518E | A | 1554 | C | DNAH3 | D2941Y | C | 8821 | A | DST | R7470* | G | 22408 | A | ELMO2 | A112V | G | 545 | A |
| DEPDC1 | L700F | T | 2217 | A | DNAH3 | R2359Q | C | 7076 | T | DST | A1055T | C | 3163 | T | ELMO2 | A241T | C | 931 | T |
| DEPDC1 | D133H | C | 514 | G | DNAH3 | D2355N | C | 7063 | T | DST | R488H | C | 1463 | T | ELMO2 | A385V | G | 1364 | A |
| DEPDC1B | R34H | C | 169 | T | DNAH3 | K3148N | T | 9444 | A | DST | S2221F | G | 6662 | A | ELMO2 | Y48H | A | 352 | G |
| DEPDC1B | A251T | C | 819 | T | DNAH3 | Y3772C | T | 11315 | C | DST | S7537N | C | 22610 | T | ELMO3 | G417S | G | 1306 | A |
| DEPDC4 | K143N | T | 432 | G | DNAH3 | R513H | C | 1538 | T | DST | R7152M | C | 21455 | A | ELMO3 | A431V | C | 1349 | T |
| DEPDC5 | R986C | C | 3026 | T | DNAH3 | E1388* | C | 4162 | A | DST | D6301G | T | 18902 | C | ELMO3 | R390H | G | 1226 | A |
| DEPDC5 | A602T | G | 1874 | A | DNAH3 | G2436W | C | 7306 | A | DST | R7301W | G | 21901 | A | ELMO3 | Y306C | A | 974 | G |
| DEPDC5 | S692A | T | 2144 | G | DNAH3 | R308Q | C | 923 | T | DST | R1373Q | C | 4118 | T | ELMOD2 | K96T | A | 419 | C |
| DEPDC6 | R54C | C | 347 | T | DNAH3 | S706N | C | 2117 | T | DST | I5996V | T | 17986 | C | ELMOD3 | R148C | C | 1058 | T |
| DEPDC6 | A364V | C | 1278 | T | DNAH3 | N3982H | T | 11944 | G | DST | K670N | T | 2010 | A | ELMOD3 | E106D | G | 934 | T |
| DEPDC6 | R163C | C | 674 | T | DNAH3 | A3679T | C | 1103 | T | DST | E5770D | T | 17310 | G | ELOF1 | A80V | G | 303 | A |

| | | | | | | | | | | | | | | | | | | |
|---|---|---|---|---|---|---|---|---|---|---|---|---|---|---|---|---|---|---|
| | | | | | | | | 5 | | | | | | | | | | |
| DEPDC7 | A305V | C | 1006 | T | DNAH3 | K3593N | C | 10779 | A | DST | A4552V | G | 13655 | A | ELOVL1 | R48H | C | 261 | T |
| DEPDC7 | K31T | A | 184 | C | DNAH3 | E981K | C | 2941 | T | DST | K4047T | T | 12140 | G | ELOVL1 | I49T | A | 264 | G |
| DERA | V38M | G | 244 | A | DNAH3 | E811K | C | 2431 | T | DST | M3962I | C | 11886 | A | ELOVL2 | F39L | A | 199 | G |
| DERA | R287Q | G | 992 | A | DNAH3 | N681T | T | 2042 | G | DST | E3807K | C | 11419 | T | ELOVL3 | F107C | T | 541 | G |
| DERL1 | R229* | G | 971 | A | DNAH5 | E4463K | C | 13492 | T | DST | F3300L | A | 9900 | C | ELOVL3 | F158L | T | 695 | G |
| DERL3 | R18Q | C | 72 | T | DNAH5 | R3743I | C | 11333 | A | DST | E3005* | C | 9013 | A | ELOVL4 | E96G | T | 588 | C |
| ELOVL4 | S294* | G | 1182 | T | FAM46C | R268C | C | 1049 | T | FREM3 | R1698M | C | 5093 | A | GPM6A | G181* | C | 587 | A |
| ELOVL5 | R324W | G | 1311 | A | FAM46D | R318K | G | 1192 | A | FREM3 | F344V | A | 1030 | C | GPN1 | F122C | T | 386 | G |
| ELOVL6 | R192L | C | 739 | A | FAM47A | Q585R | T | 1787 | C | FREM3 | V1593M | C | 4777 | T | GPN3 | Q279* | G | 891 | A |
| ELSPBP1 | F114L | C | 520 | A | FAM47A | R255C | G | 796 | A | FREM3 | P815S | G | 2443 | A | GPR1 | H146R | T | 800 | C |
| ELTD1 | G104A | C | 375 | G | FAM47B | A612D | C | 1853 | A | FRMD1 | A176T | C | 591 | T | GPR101 | S331R | T | 991 | G |
| ELTD1 | A542V | G | 1689 | A | FAM47B | E75K | G | 241 | A | FRMD3 | E393* | C | 1384 | A | GPR101 | R380C | G | 1138 | A |
| ELTD1 | D423E | A | 1333 | C | FAM47B | A101V | C | 320 | T | FRMD3 | - | A | 0 | G | GPR107 | A45T | G | 640 | A |
| ELTD1 | F201V | A | 665 | C | FAM47C | K1025I | A | 3088 | T | FRMD4A | R900Q | C | 3068 | T | GPR107 | D563Y | G | 2194 | T |
| EMCN | K260N | C | 959 | A | FAM47C | S928Y | C | 2797 | A | FRMD4A | Y96* | G | 657 | T | GPR107 | S148N | G | 950 | A |
| EMD | F241S | T | 1010 | C | FAM47C | Y1022D | T | 3078 | G | FRMD4A | A919V | G | 3125 | A | GPR109B | I64V | T | 250 | C |
| EME1 | D447N | G | 1412 | A | FAM48A | Q807R | T | 2604 | C | FRMD4A | R851C | G | 2920 | A | GPR110 | - | A | 0 | C |
| EME2 | A306T | G | 916 | A | FAM48A | R506Q | C | 1701 | T | FRMD4A | N803S | T | 2777 | C | GPR111 | R376I | G | 1127 | T |
| EMID1 | A411V | C | 1359 | T | FAM48A | K399T | T | 1380 | G | FRMD4A | R480H | C | 1808 | T | GPR111 | G455D | G | 1364 | A |
| EMID1 | H336R | A | 1134 | G | FAM48A | Y409C | T | 1410 | C | FRMD4A | R851H | C | 2921 | T | GPR112 | Y2775* | T | 8616 | A |
| EMID1 | G305S | G | 1040 | A | FAM48A | S320T | A | 1142 | T | FRMD4A | I145M | A | 804 | C | GPR112 | K352N | G | 1347 | T |
| EMID1 | A391V | C | 1299 | T | FAM49A | R164C | G | 711 | A | FRMD4B | G267* | C | 883 | A | GPR112 | K453N | G | 1650 | T |
| EMID2 | T74M | C | 434 | T | FAM49A | R123G | T | 588 | C | FRMD4B | D757G | T | 2354 | C | GPR112 | E807D | G | 2712 | T |
| EMID2 | P174L | C | 734 | T | FAM49A | R140* | G | 639 | A | FRMD5 | V364I | C | 1267 | T | GPR112 | E908* | G | 3013 | T |
| EMID2 | A231G | C | 905 | G | FAM49A | D64Y | C | 411 | A | FRMD6 | R557C | C | 1865 | T | GPR112 | I1667T | T | 5291 | C |
| EMILIN1 | A140V | C | 918 | T | FAM50A | N44I | A | 241 | T | FRMD6 | R404W | C | 1406 | T | GPR112 | M1978T | T | 6224 | C |
| EMILIN1 | V63M | G | 686 | A | FAM53A | R221H | C | 860 | T | FRMD8 | R208Q | G | 815 | A | GPR112 | S806Y | C | 2708 | A |
| EMILIN1 | R596W | C | 2285 | T | FAM53A | G233D | C | 896 | T | FRMPD1 | - | A | 0 | G | GPR113 | A481V | G | 1442 | A |
| EMILIN1 | R748H | G | 2742 | A | FAM53A | R387C | G | 1357 | A | FRMPD1 | Y330C | A | 1088 | G | GPR113 | A771V | G | 2312 | A |
| EMILIN1 | F873C | T | 3117 | G | FAM53B | R154H | C | 673 | T | FRMPD1 | N1143T | A | 3527 | C | GPR113 | Q260K | G | 778 | T |
| EMILIN1 | A136V | C | 906 | T | FAM54B | T116M | C | 481 | T | FRMPD1 | E1279* | G | 3934 | T | GPR113 | L297H | A | 890 | T |
| EMILIN2 | R673M | G | 2177 | T | FAM55A | R452H | C | 1355 | T | FRMPD1 | E1484K | G | 4549 | A | GPR113 | A392V | G | 1175 | A |
| EMILIN2 | R377I | G | 1289 | T | FAM55B | K62T | A | 283 | C | FRMPD2 | L449M | G | 1648 | T | GPR113 | V299I | C | 895 | T |
| EMILIN3 | R692W | G | 2267 | A | FAM55C | R339C | C | 1607 | T | FRMPD2 | C141R | A | 724 | G | GPR114 | E26K | G | 158 | A |
| EMILIN3 | L402M | G | 1397 | T | FAM55C | L358V | T | 1664 | G | FRMPD2 | S647P | A | 2242 | G | GPR114 | A121T | G | 443 | A |
| EML1 | D365G | A | 1163 | G | FAM55D | Q380* | G | 1319 | A | FRMPD2 | F535L | G | 1908 | T | GPR114 | - | G | 0 | A |

| | | | | | | | | | | | | | | | | | | | |
|---|---|---|---|---|---|---|---|---|---|---|---|---|---|---|---|---|---|---|---|
| EML1 | F416L | T | 1317 | G | FAM57B | A49T | C | 491 | T | FRMPD4 | A945V | C | 2834 | T | GPR115 | V501M | G | 1501 | A |
| EML1 | R302W | C | 973 | T | FAM58A | L69P | A | 309 | G | FRMPD4 | E1117K | G | 3349 | A | GPR115 | E289D | G | 867 | T |
| EML2 | E569G | T | 1741 | C | FAM58A | P15R | G | 147 | C | FRMPD4 | A522V | C | 1565 | T | GPR116 | F1095L | G | 3514 | T |
| EML3 | A273T | C | 815 | T | FAM58A | S118N | C | 456 | T | FRRS1 | R278Q | C | 1435 | T | GPR116 | A723V | G | 2397 | A |
| EML3 | R854P | C | 2559 | G | FAM59A | V343F | C | 1031 | A | FRS2 | V473M | G | 1927 | A | GPR116 | S1218R | G | 3883 | T |
| EML3 | A107T | C | 317 | T | FAM59A | K656N | C | 1972 | A | FRS2 | E128D | A | 894 | C | GPR116 | V682L | C | 2273 | G |
| EML3 | P238Q | G | 711 | T | FAM59A | G410R | C | 1232 | T | FRY | S539N | G | 2112 | A | GPR116 | K491Q | T | 1700 | G |
| EML3 | R185H | C | 552 | T | FAM59B | S767L | C | 2300 | T | FRY | T2194M | C | 7077 | T | GPR116 | N411T | T | 1461 | G |
| EML3 | K572N | C | 1714 | A | FAM59B | K986T | A | 2957 | C | FRY | R757C | C | 2765 | T | GPR12 | A87T | C | 481 | T |
| EML4 | D765G | A | 2556 | G | FAM5B | R67H | G | 512 | A | FRY | T2194M | C | 7077 | T | GPR120 | I349L | A | 1101 | C |
| EML4 | N90S | A | 531 | G | FAM5B | P771S | C | 2623 | T | FRY | P53L | C | 654 | T | GPR123 | R355W | C | 1063 | T |
| EML4 | N183T | A | 810 | C | FAM5B | L518P | T | 1865 | C | FRY | H1121Y | C | 3857 | T | GPR123 | T706M | C | 2117 | T |
| EML5 | R464I | C | 1577 | A | FAM5C | A298V | G | 1125 | A | FRY | R2484C | C | 7946 | T | GPR123 | R163C | C | 487 | T |
| EML5 | T1454I | G | 4547 | A | FAM5C | G162* | C | 716 | A | FRY | S1822T | G | 5961 | C | GPR123 | A834T | G | 2500 | A |
| EML5 | R292Q | C | 1061 | T | FAM5C | R530H | C | 1821 | T | FRY | G1176D | G | 4023 | A | GPR123 | R427Q | G | 1280 | A |
| EML5 | N364H | T | 1276 | G | FAM5C | R507* | G | 1751 | A | FRY | V2377I | G | 7625 | A | GPR123 | Q174* | C | 520 | T |
| EML5 | S1808P | A | 5608 | G | FAM5C | L97I | G | 521 | T | FRY | I279S | T | 1332 | G | GPR123 | A878T | G | 2632 | A |
| EML5 | A1606V | G | 5003 | A | FAM5C | R681W | G | 2273 | A | FRY | L604I | C | 2306 | A | GPR123 | D1002Y | G | 3004 | T |
| EML5 | H1080R | T | 3425 | C | FAM5C | R353H | C | 1290 | T | FRY | F799C | T | 2892 | G | GPR124 | A1151G | C | 3465 | G |
| EML5 | K1763T | T | 5474 | G | FAM5C | C441S | A | 1553 | T | FRY | G1378E | G | 4629 | A | GPR124 | P708H | C | 2136 | A |
| EML5 | R475I | C | 1610 | A | FAM63A | F363L | G | 1089 | T | FRY | K2017T | A | 6546 | C | GPR124 | E707K | G | 2132 | A |
| EML5 | K405N | T | 1401 | G | FAM65A | L810M | C | 2428 | A | FRY | S2350Y | C | 7545 | A | GPR125 | R1170H | C | 3779 | T |
| EML6 | R230H | G | 1209 | A | FAM65A | S196N | G | 587 | A | FRY | S2579Y | C | 8232 | A | GPR125 | A906T | C | 2986 | T |
| EML6 | K841T | A | 3042 | C | FAM65B | S559L | G | 1852 | A | FRY | R2830K | G | 8985 | A | GPR125 | R692Q | C | 2345 | T |
| EML6 | V1036A | T | 3627 | C | FAM65C | Q937H | C | 3223 | A | FRYL | A194T | C | 580 | T | GPR126 | M1032T | T | 3506 | C |
| EML6 | R1508H | G | 5043 | A | FAM65C | R102C | G | 716 | A | FRYL | L1407V | A | 4219 | C | GPR126 | N345T | A | 1445 | C |
| EML6 | D136V | A | 927 | T | FAM65C | R810W | G | 2840 | A | FRYL | Q1797* | G | 5389 | A | GPR126 | I95M | T | 696 | G |
| EML6 | K804N | A | 2932 | C | FAM69A | L156I | G | 537 | T | FRYL | A1185V | G | 3554 | A | GPR126 | I774S | T | 2732 | G |
| EML6 | K1082Q | A | 3764 | C | FAM69A | L204I | G | 681 | T | FRYL | R1341* | G | 4021 | A | GPR128 | D392N | G | 1442 | A |
| EML6 | R1377Q | G | 4650 | A | FAM69B | A238T | G | 808 | A | FRYL | R985* | G | 2953 | A | GPR128 | A2S | G | 272 | T |
| EMP1 | D59Y | G | 422 | T | FAM69B | R344C | C | 1126 | T | FRYL | S856N | C | 2567 | T | GPR128 | V679A | T | 2304 | C |
| EMP2 | A78T | C | 442 | T | FAM69C | R181W | G | 550 | A | FRYL | T2155A | T | 6463 | C | GPR132 | P365T | G | 1680 | T |
| EMP3 | V21M | G | 315 | A | FAM69C | F91L | G | 282 | T | FRYL | N1669S | T | 5006 | C | GPR132 | R316C | G | 1533 | A |
| EMR1 | T432M | C | 1333 | T | FAM69C | L187M | G | 568 | T | FRYL | G901V | C | 2702 | A | GPR132 | C280F | C | 1426 | A |
| EMR1 | E539V | A | 1654 | T | FAM69C | A152D | G | 464 | T | FSCB | T239S | T | 1024 | A | GPR132 | A246T | C | 1323 | T |
| EMR1 | A887T | G | 2697 | A | FAM69C | E81K | C | 250 | T | FSCB | V714I | C | 2449 | T | GPR133 | D215N | G | 1202 | A |
| EMR1 | G249D | G | 784 | A | FAM69C | I353L | T | 1066 | G | FSCN1 | F187L | T | 655 | C | GPR133 | S701R | A | 2660 | C |
| EMR1 | T794M | C | 2419 | T | FAM69C | E319D | T | 966 | G | FSCN2 | G53R | G | 298 | A | GPR137C | F348C | T | 1043 | G |
| EMR1 | A887T | G | 2697 | A | FAM71A | A391V | C | 1576 | T | FSCN2 | K32N | G | 237 | T | GPR139 | R291W | G | 1172 | A |

| | | | | | | | | | | | | | | | | | | | |
|---|---|---|---|---|---|---|---|---|---|---|---|---|---|---|---|---|---|---|---|
| EMR2 | R725Q | C | 2630 | T | FAM71A | R440H | G | 1723 | A | FSCN2 | L65M | C | 334 | A | GPR141 | Y127C | A | 669 | G |
| EMR2 | A415S | C | 1699 | A | FAM71A | R30Q | G | 493 | A | FSCN3 | A214S | G | 859 | T | GPR141 | K121E | A | 650 | G |
| EMR2 | K479N | C | 1893 | A | FAM71A | E36G | A | 511 | G | FSCN3 | R285C | C | 1072 | T | GPR141 | S37A | T | 398 | G |
| EMR3 | R222H | C | 813 | T | FAM71A | R67H | G | 604 | A | FSCN3 | A452V | C | 1574 | T | GPR143 | A211T | C | 719 | T |
| EMR3 | - | C | 0 | A | FAM71A | E265* | G | 1197 | T | FSCN3 | T240M | C | 938 | T | GPR144 | A145V | C | 434 | T |
| EMR3 | Y306C | T | 1065 | C | FAM71B | K416E | T | 1342 | C | FSD1 | S24N | G | 147 | A | GPR144 | P772S | C | 2314 | T |
| EMR3 | V617A | A | 1998 | G | FAM71B | A364V | G | 1187 | A | FSD1 | R271H | G | 888 | A | GPR144 | G743V | G | 2228 | T |
| EMR3 | F595L | G | 1933 | T | FAM71B | A247V | G | 836 | A | FSD1 | V395I | G | 1259 | A | GPR144 | R176C | C | 526 | T |
| EMX1 | S230I | G | 1067 | T | FAM71B | A375V | G | 1220 | A | FSD1L | N515S | A | 1663 | G | GPR144 | R514H | G | 1541 | A |
| EMX2 | R26C | C | 899 | T | FAM71B | T252M | G | 851 | A | FSD1L | N275H | A | 942 | C | GPR144 | A404V | C | 1211 | T |
| EMX2 | E195K | G | 1406 | A | FAM71B | M18T | A | 149 | G | FSHR | K608N | C | 1944 | A | GPR144 | K715N | G | 2145 | T |
| EMX2 | A160T | G | 1301 | A | FAM71B | G256W | C | 862 | A | FSIP1 | K367Q | T | 1317 | G | GPR148 | A131T | G | 393 | A |
| EN1 | G363D | C | 2104 | T | FAM71B | I318N | A | 1049 | T | FSIP1 | R376H | C | 1345 | T | GPR148 | T262A | A | 786 | G |
| EN2 | A325T | G | 1222 | A | FAM71C | M124V | A | 792 | G | FSIP2 | R5471I | G | 16412 | T | GPR149 | A491V | G | 1572 | A |
| ENAM | L27I | C | 360 | A | FAM71C | R75C | C | 645 | T | FSIP2 | D5789Y | G | 17365 | T | GPR149 | R310H | C | 1029 | T |
| ENAM | G1071R | G | 3492 | A | FAM71E1 | R98C | G | 650 | A | FSIP2 | D6423N | G | 19267 | A | GPR149 | R542C | G | 1724 | A |
| ENAM | E55D | G | 446 | T | FAM71E1 | V119I | C | 713 | T | FSIP2 | - | G | 0 | A | GPR152 | L290P | A | 874 | G |
| ENAM | A863S | G | 2868 | T | FAM71E2 | I812L | T | 2620 | G | FST | G59D | G | 559 | A | GPR152 | W111* | C | 337 | T |
| ENAM | R778* | A | 2613 | T | FAM73A | - | G | 0 | A | FST | R35L | G | 487 | T | GPR153 | A222T | C | 924 | T |
| ENC1 | Y245H | A | 1864 | G | FAM73A | Q396H | G | 1220 | T | FSTL3 | A198V | C | 628 | T | GPR153 | A50T | C | 408 | T |
| ENC1 | E128G | T | 1514 | C | FAM73B | T214I | C | 641 | T | FSTL4 | I794T | A | 2631 | G | GPR153 | R73H | C | 478 | T |
| ENDOG | A130T | G | 599 | A | FAM73B | R398Q | G | 1193 | A | FSTL4 | G14V | C | 291 | A | GPR155 | - | C | 0 | A |
| ENDOU | - | A | 0 | G | FAM73B | E429* | G | 1285 | T | FSTL4 | T768M | G | 2553 | A | GPR155 | F244L | A | 971 | T |
| ENG | S167I | C | 901 | A | FAM73B | G370* | G | 1108 | T | FSTL5 | R404C | G | 1647 | A | GPR156 | S95N | C | 730 | T |
| ENGASE | R43P | G | 136 | C | FAM75A6 | T645A | T | 1962 | C | FSTL5 | D252Y | C | 1191 | A | GPR156 | A412V | G | 1681 | A |
| ENGASE | P639S | C | 1923 | T | FAM75A6 | K9Q | T | 54 | G | FSTL5 | D252Y | C | 1191 | A | GPR156 | D478Y | C | 1878 | A |
| ENO3 | A234T | G | 794 | A | FAM78B | N81S | T | 709 | C | FSTL5 | A537V | G | 2047 | A | GPR158 | K856Q | A | 2925 | C |
| ENO3 | K239N | G | 811 | T | FAM78B | E16D | C | 515 | A | FSTL5 | G317D | C | 1387 | T | GPR158 | L687P | T | 2419 | C |
| ENOPH1 | A138V | C | 681 | T | FAM81B | M388I | G | 1210 | A | FSTL5 | R404C | G | 1647 | A | GPR158 | R236H | G | 1066 | A |
| ENOSF1 | D274N | C | 820 | T | FAM81B | K345R | A | 1080 | G | FSTL5 | E48D | T | 581 | G | GPR158 | V346I | G | 1395 | A |
| ENOSF1 | P361H | G | 1082 | T | FAM82A1 | R130C | C | 504 | T | FTCD | V498M | C | 1536 | T | GPR158 | T920A | A | 3117 | G |
| ENOSF1 | T54M | G | 161 | A | FAM82A1 | P180S | C | 654 | T | FTCD | D74N | C | 264 | T | GPR161 | E334* | C | 1314 | A |
| ENOSF1 | A97T | C | 289 | T | FAM82A2 | R209K | C | 1694 | T | FTCD | C523Y | C | 1612 | T | GPR162 | R115H | G | 879 | A |
| ENOSF1 | E190K | C | 568 | T | FAM82B | N157K | A | 631 | C | FTCD | R381W | G | 1185 | A | GPR162 | G5V | G | 549 | T |
| ENOX1 | V580I | C | 2316 | T | FAM83A | R347Q | G | 1385 | A | FTCD | R382C | G | 1188 | A | GPR162 | R217W | C | 1184 | T |
| ENOX1 | P103L | G | 886 | A | FAM83A | R318C | C | 1297 | T | FTCD | R320W | G | 1002 | A | GPR17 | S146N | G | 511 | A |
| ENOX1 | R298C | G | 1470 | A | FAM83C | R578W | G | 1850 | A | FTCD | Y225H | A | 717 | G | GPR17 | K331Q | A | 1065 | C |
| ENOX1 | R315C | G | 1521 | A | FAM83C | L273F | G | 935 | A | FTHL17 | V111I | C | 431 | T | GPR17 | R301C | C | 975 | T |
| ENOX1 | R298H | C | 1471 | T | FAM83D | S263L | C | 829 | T | FTMT | D104E | T | 321 | G | GPR171 | A38V | G | 344 | A |

| | | | | | | | | | | | | | | | | | | | |
|---|---|---|---|---|---|---|---|---|---|---|---|---|---|---|---|---|---|---|---|
| ENOX1 | E397K | C | 1767 | T | FAM83E | E245K | C | 798 | T | FTMT | R69H | G | 215 | A | GPR171 | F104L | G | 543 | T |
| ENOX2 | A222V | G | 687 | A | FAM83E | R440H | C | 1384 | T | FTSJ1 | C312Y | G | 1258 | A | GPR172A | V160M | G | 807 | A |
| ENPEP | D221N | G | 1003 | A | FAM83F | R349W | C | 1139 | T | FTSJ2 | R53W | G | 186 | A | GPR172A | P184Q | C | 880 | A |
| ENPEP | A262V | C | 1127 | T | FAM83H | D624N | C | 2335 | T | FTSJ2 | F117C | A | 379 | C | GPR172A | P403L | C | 1537 | T |
| ENPEP | I403S | T | 1550 | G | FAM83H | Q799R | T | 2861 | C | FTSJ3 | M420T | A | 1905 | G | GPR172A | S137R | C | 740 | A |
| ENPEP | D849Y | G | 2887 | T | FAM83H | A366V | G | 1562 | A | FTSJ3 | A377V | G | 1776 | A | GPR172B | R310C | G | 1601 | A |
| ENPP1 | R821C | C | 2481 | T | FAM84A | T200I | C | 599 | T | FTSJD1 | K592T | T | 2112 | G | GPR173 | L105F | C | 571 | T |
| ENPP1 | F708L | T | 2142 | C | FAM86A | R299W | G | 964 | A | FTSJD1 | Y543H | A | 1964 | G | GPR176 | E360* | C | 1944 | A |
| ENPP1 | R476Q | G | 1447 | A | FAM86A | Y131C | T | 461 | C | FTSJD2 | F401L | C | 1367 | A | GPR179 | A379V | G | 1157 | A |
| ENPP2 | S302L | G | 991 | A | FAM86A | Q235* | G | 772 | A | FUBP1 | R451C | G | 1440 | A | GPR179 | E2231* | C | 6712 | A |
| ENPP3 | Y201H | T | 929 | C | FAM86B2 | V84A | A | 251 | G | FUBP1 | R365* | G | 1182 | A | GPR179 | R478Q | C | 1454 | T |
| ENPP3 | F270L | T | 1136 | C | FAM86C | C154G | T | 460 | G | FUBP1 | P517L | G | 1639 | A | GPR179 | Q1533* | G | 4618 | A |
| ENPP5 | K361N | C | 1344 | A | FAM8A1 | F317L | C | 1006 | A | FUBP1 | Q179P | T | 625 | G | GPR179 | K1685R | T | 5075 | C |
| ENPP5 | P274Q | G | 1082 | T | FAM90A1 | P71L | G | 212 | A | FUBP1 | R451C | G | 1440 | A | GPR179 | R752H | C | 2276 | T |
| ENPP5 | S3L | G | 269 | A | FAM90A1 | E172K | C | 514 | T | FUK | R243Q | G | 786 | A | GPR179 | E2266* | C | 6817 | A |
| ENPP6 | E52D | C | 298 | A | FAM90A1 | T350K | G | 1049 | T | FUK | Y18H | T | 110 | C | GPR179 | S841L | G | 2543 | A |
| ENSA | M77I | C | 327 | A | FAM90A1 | P71S | G | 211 | A | FUNDC2 | V130G | T | 643 | G | GPR18 | T279M | G | 1337 | A |
| ENSA | Q3H | C | 105 | A | FAM90A1 | E127D | C | 381 | A | FUNDC2 | K68T | A | 457 | C | GPR19 | V141L | C | 776 | A |
| ENTHD1 | K87N | C | 512 | A | FAM90A20 | P228L | C | 683 | T | FURIN | A369T | G | 1384 | A | GPR19 | D387Y | C | 1514 | A |
| ENTPD1 | S381P | T | 1319 | C | FAM91A1 | A379T | G | 1381 | A | FURIN | A489V | C | 1745 | T | GPR19 | D191Y | C | 926 | A |
| ENTPD1 | E90* | G | 446 | T | FAM91A1 | V514I | G | 1786 | A | FURIN | G586D | G | 2036 | A | GPR20 | R238W | G | 801 | A |
| ENTPD1 | F260C | T | 957 | G | FAM92A1 | E191K | G | 674 | A | FURIN | Y571H | T | 1990 | C | GPR20 | P2S | G | 93 | A |
| ENTPD1 | I284L | A | 1028 | C | FAM92A2 | K185N | G | 555 | T | FURIN | T262P | A | 1063 | C | GPR22 | S106* | C | 1660 | A |
| ENTPD3 | L408R | T | 1341 | G | FAM92A2 | F291V | T | 871 | G | FUS | P431L | C | 1383 | T | GPR26 | A288T | G | 915 | A |
| ENTPD3 | V358G | T | 1191 | G | FAM92B | T39M | G | 272 | A | FUT1 | H117Y | G | 1324 | A | GPR26 | E165K | G | 546 | A |
| ENTPD3 | K119N | A | 475 | C | FAM98A | P69L | G | 260 | A | FUT1 | R212H | C | 1610 | T | GPR27 | L296I | C | 886 | A |
| ENTPD3 | K270N | G | 928 | T | FAM98A | S284N | C | 905 | T | FUT1 | W313C | C | 1914 | A | GPR27 | R268H | G | 803 | A |
| ENTPD3 | E451* | G | 1469 | T | FAM9A | A24V | G | 182 | A | FUT11 | A111S | G | 374 | T | GPR3 | A5V | C | 113 | T |
| ENTPD8 | T39I | G | 300 | A | FAM9A | R286K | C | 968 | T | FUT11 | D287G | A | 903 | G | GPR31 | T267M | G | 1298 | A |
| ENTPD8 | A351T | C | 1235 | T | FAM9B | R13H | C | 227 | T | FUT2 | T299M | C | 1017 | T | GPR31 | A71T | C | 709 | T |
| ENTPD8 | V488I | C | 1646 | T | FAM9C | S118Y | G | 563 | T | FUT2 | V4I | G | 131 | A | GPR32 | S330A | T | 1125 | G |
| ENTPD8 | A203V | G | 792 | A | FANCA | R1186M | C | 3599 | A | FUT6 | P6L | G | 17 | A | GPR35 | A317V | C | 950 | T |
| EOMES | P472S | G | 1432 | A | FANCA | L908P | A | 2765 | G | FUT7 | P152H | G | 1474 | T | GPR35 | C168Y | G | 503 | A |
| EOMES | G596S | C | 1804 | T | FANCA | P808S | G | 2464 | A | FUT7 | R323H | C | 1987 | T | GPR35 | P263S | C | 787 | T |
| EP300 | Q974R | A | 4140 | G | FANCC | R548* | G | 1897 | A | FUT7 | V199M | C | 1614 | T | GPR37 | A31D | G | 743 | T |
| EP300 | P551S | C | 2870 | T | FANCC | - | C | 0 | A | FUT8 | R559* | C | 3402 | T | GPR37 | T458A | T | 2023 | C |
| EP300 | W1466L | G | 5616 | T | FANCD2 | K864N | G | 2685 | T | FUT9 | S296G | A | 1212 | G | GPR37 | E371K | C | 1762 | T |
| EP300 | Q2215H | A | 7864 | C | FANCD2 | E1011* | G | 3124 | T | FUT9 | F53L | C | 485 | A | GPR37L1 | I242T | T | 831 | C |
| EP300 | P1986A | C | 7175 | G | FANCD2 | F421L | C | 1356 | A | FUZ | I79V | T | 399 | C | GPR39 | R398C | C | 1661 | T |

| | | | | | | | | | | | | | | | | | | | |
|---|---|---|---|---|---|---|---|---|---|---|---|---|---|---|---|---|---|---|---|
| EP300 | Q1862P | A | 6804 | C | FANCD2 | I1125V | A | 3466 | G | FXR1 | R505W | C | 1897 | T | GPR39 | V73M | G | 686 | A |
| EP300 | A1936V | C | 7026 | T | FANCD2 | R1273* | C | 3910 | T | FXR1 | R315* | C | 1327 | T | GPR4 | R14H | C | 986 | T |
| EP300 | R397Q | G | 2409 | A | FANCG | L355I | G | 1555 | T | FXR1 | A515V | C | 1928 | T | GPR4 | R14C | G | 985 | A |
| EP300 | R604Q | G | 3030 | A | FANCG | A360T | C | 1570 | T | FXR1 | G582D | G | 2129 | A | GPR4 | G94W | C | 1225 | A |
| EP300 | C1183Y | G | 4767 | A | FANCI | M363I | G | 1179 | T | FXR1 | E410D | A | 1614 | C | GPR44 | R239Q | C | 828 | T |
| EP300 | Q2298* | C | 8111 | T | FANCI | A9T | G | 115 | A | FXYD2 | Q47* | G | 205 | A | GPR52 | W192* | G | 614 | A |
| EP400 | A2523T | G | 7676 | A | FANCI | V509A | T | 1616 | C | FXYD3 | G109C | G | 472 | T | GPR52 | V123A | T | 406 | C |
| EP400 | A2523T | G | 7676 | A | FANCI | L421F | C | 1351 | T | FYB | K125N | C | 498 | A | GPR52 | K182N | A | 584 | C |
| EP400 | T576M | C | 1836 | T | FANCI | S1211Y | C | 3722 | A | FYB | K45N | C | 258 | A | GPR55 | R296H | C | 1080 | T |
| EP400 | V1193I | G | 3686 | A | FANCI | C650S | T | 2038 | A | FYB | K741T | T | 2345 | G | GPR56 | L426I | C | 1607 | A |
| EP400 | R1786C | C | 5465 | T | FANCL | - | T | 0 | C | FYB | E475K | C | 1546 | T | GPR56 | V369I | G | 1436 | A |
| EP400 | T556M | C | 1776 | T | FANCM | R1570C | C | 4807 | T | FYCO1 | A967V | G | 3106 | A | GPR6 | P116L | C | 347 | T |
| EP400 | R2320H | G | 7068 | A | FANCM | S1829C | C | 5585 | G | FYCO1 | K1154T | T | 3667 | G | GPR6 | V315M | G | 943 | A |
| EP400 | R1786C | C | 5465 | T | FANCM | K636N | A | 2007 | C | FYCO1 | R442W | G | 1530 | A | GPR6 | A106V | C | 317 | T |
| EP400 | P2076L | C | 6336 | T | FANCM | K932T | A | 2894 | C | FYCO1 | E411K | C | 1437 | T | GPR6 | E350D | G | 1050 | T |
| EP400 | P2855L | C | 8673 | T | FANCM | S1874G | A | 5719 | G | FYN | R77C | G | 836 | A | GPR61 | S41P | T | 804 | C |
| EP400 | A2655V | C | 8073 | T | FANCM | Q564K | C | 1789 | A | FYN | F109L | A | 934 | C | GPR63 | R405C | G | 1691 | A |
| EP400 | D951N | G | 2960 | A | FANK1 | M1L | A | 105 | T | FYTTD1 | R42Q | G | 347 | A | GPR63 | M385T | A | 1632 | G |
| EP400 | S1431C | C | 4401 | G | FANK1 | A227T | G | 679 | A | FYTTD1 | I297V | A | 1111 | G | GPR64 | N194D | T | 744 | C |
| EPAS1 | A506T | G | 2026 | A | FAP | Y729H | A | 2393 | G | FZD1 | Q117H | G | 764 | T | GPR68 | Q344H | C | 1297 | G |
| EPAS1 | D258N | G | 1282 | A | FAR1 | R160H | G | 634 | A | FZD1 | R391C | C | 1584 | T | GPR68 | G100S | C | 563 | T |
| EPAS1 | A698T | G | 2602 | A | FAR1 | K221Q | A | 816 | C | FZD1 | M520T | T | 1972 | C | GPR75 | N310I | T | 1200 | A |
| EPAS1 | G633D | G | 2408 | A | FAR2 | R459C | C | 1643 | T | FZD1 | T503M | C | 1921 | T | GPR75 | P256S | G | 1037 | A |
| EPB41 | - | G | 0 | A | FAR2 | - | G | 0 | A | FZD1 | A430V | C | 1702 | T | GPR75 | T297A | T | 1160 | C |
| EPB41L1 | - | G | 0 | T | FAR2 | R386I | G | 1425 | T | FZD10 | A92T | G | 730 | A | GPR78 | V43I | G | 544 | A |
| EPB41L1 | R892C | C | 4123 | T | FAR2 | E407D | A | 1489 | C | FZD10 | R424Q | G | 1727 | A | GPR78 | R227C | C | 1096 | T |
| EPB41L1 | A921T | G | 4210 | A | FARP1 | A541V | C | 1887 | T | FZD10 | A205T | G | 1069 | A | GPR78 | R168H | G | 920 | A |
| EPB41L2 | R663H | C | 2060 | T | FARP1 | R711G | A | 2396 | G | FZD10 | R261H | G | 1238 | A | GPR78 | R71W | C | 628 | T |
| EPB41L2 | L202R | A | 677 | C | FARP1 | S383L | C | 1413 | T | FZD10 | C309R | T | 1381 | C | GPR78 | R342C | C | 1441 | T |
| EPB41L2 | G681W | C | 2113 | A | FARP2 | R872C | C | 2784 | T | FZD2 | V379M | G | 1267 | A | GPR78 | R236H | G | 1124 | A |
| EPB41L2 | E708* | C | 2194 | A | FARP2 | F756L | C | 2438 | A | FZD2 | C100R | T | 430 | C | GPR78 | P341L | C | 1439 | T |
| EPB41L2 | E360G | T | 1151 | C | FARS2 | A225V | C | 1005 | T | FZD2 | V464I | G | 1522 | A | GPR81 | V237M | C | 1213 | T |
| EPB41L3 | V1002M | C | 3345 | T | FARSA | R392W | G | 1222 | A | FZD2 | R442H | G | 1457 | A | GPR83 | V378F | C | 1304 | A |
| EPB41L3 | E777K | C | 2670 | T | FARSA | R298W | G | 940 | A | FZD3 | K502N | G | 1984 | T | GPR85 | A192V | G | 1179 | A |
| EPB41L3 | R546K | C | 1978 | T | FARSA | G470D | C | 1457 | T | FZD3 | R420Q | G | 1737 | A | GPR87 | V47M | C | 604 | T |
| EPB41L3 | T278A | T | 1173 | C | FARSB | K483Q | T | 1711 | G | FZD6 | R416Q | G | 1523 | A | GPR87 | K290* | T | 1333 | A |
| EPB41L3 | - | C | 0 | T | FASN | R1662H | C | 5102 | T | FZD7 | A390T | G | 1229 | A | GPR88 | R31H | G | 531 | A |
| EPB41L3 | D64A | T | 532 | G | FASN | A2323V | G | 7085 | A | FZD7 | S95Y | C | 345 | A | GPR88 | R60* | C | 617 | T |
| EPB41L4A | R595Q | C | 2061 | T | FASN | W1511C | C | 4650 | A | FZD7 | S378L | C | 1194 | T | GPR97 | - | T | 0 | C |

| | | | | | | | | | | | | | | | | | | |
|---|---|---|---|---|---|---|---|---|---|---|---|---|---|---|---|---|---|---|
| EPB41L4A | R430H | C | 1566 | T | FASN | V1973M | C | 6034 | T | FZD8 | A455V | G | 1369 | A | GPR97 | A337S | G | 1112 | T |
| EPB41L4A | R246Q | C | 1014 | T | FASTK | H222N | G | 742 | T | FZD9 | A285V | C | 1083 | T | GPR97 | R139Q | G | 519 | A |
| EPB41L4A | T68M | G | 480 | A | FASTK | R543C | G | 1705 | A | FZD9 | A212T | G | 863 | A | GPR97 | - | G | 0 | A |
| EPB41L4A | R316C | G | 1223 | A | FASTKD2 | A238T | G | 1060 | A | FZD9 | E127D | G | 610 | T | GPR98 | S2200Y | C | 6695 | A |
| EPB41L4A | R442Q | C | 1602 | T | FASTKD2 | S36* | C | 455 | A | FZD9 | W537R | T | 1838 | C | GPR98 | A5484T | G | 16546 | A |
| EPB41L4A | N401H | T | 1478 | G | FASTKD3 | R406C | G | 1353 | A | FZD9 | T393P | A | 1406 | C | GPR98 | V1312M | G | 4030 | A |
| EPB41L4A | R479H | C | 1713 | T | FAT1 | E4457K | C | 13557 | T | FZR1 | R313Q | G | 972 | A | GPR98 | R5246* | C | 15832 | T |
| EPB41L4B | E329K | C | 1503 | T | FAT1 | E2924K | C | 8958 | T | FZR1 | R477H | G | 1464 | A | GPR98 | K4079N | G | 12333 | T |
| EPB41L5 | T158A | A | 686 | G | FAT1 | E1420* | C | 4446 | A | G2E3 | S701F | C | 2221 | T | GPR98 | G4416A | G | 13343 | C |
| EPB41L5 | R378* | C | 1346 | T | FAT1 | P509L | G | 1714 | A | G2E3 | R161Q | G | 601 | A | GPR98 | A6224V | C | 18767 | T |
| EPB41L5 | C154Y | G | 675 | A | FAT1 | A173T | C | 705 | T | G2E3 | R382* | C | 1263 | T | GPR98 | E664* | G | 2086 | T |
| EPB41L5 | F257V | T | 983 | G | FAT1 | P1351L | G | 4240 | A | G2E3 | S438Y | C | 1432 | A | GPR98 | - | T | 0 | C |
| EPB42 | T564M | G | 2149 | A | FAT1 | L3065S | A | 9382 | G | G2E3 | F599V | T | 1914 | G | GPR98 | A1047E | C | 3236 | A |
| EPB49 | R23H | G | 523 | A | FAT1 | P4455L | G | 13552 | A | G2E3 | L633F | C | 2016 | T | GPR98 | P2313L | C | 7034 | T |
| EPC1 | R199* | G | 865 | A | FAT1 | - | C | 0 | A | G3BP1 | D338V | A | 1158 | T | GPR98 | G2898V | G | 8789 | T |
| EPC1 | C185G | A | 823 | C | FAT1 | V2957I | C | 9057 | T | G3BP1 | V126I | G | 521 | A | GPR98 | R1740W | A | 5314 | T |
| EPC1 | F94C | A | 551 | C | FAT1 | R2600* | G | 7986 | A | G3BP1 | S388F | C | 1308 | T | GPR98 | L1742F | G | 5322 | T |
| EPC2 | H732R | A | 2229 | G | FAT1 | S2313F | G | 7126 | A | G3BP2 | E315K | C | 1729 | T | GPR98 | D2039H | G | 6211 | C |
| EPC2 | R332C | C | 1028 | T | FAT1 | E833A | T | 2686 | G | G3BP2 | E187D | C | 1347 | G | GPR98 | I639M | T | 2013 | G |
| EPHA1 | V103M | C | 394 | T | FAT1 | S458N | C | 1561 | T | G3BP2 | E152D | T | 1242 | G | GPR98 | I977V | A | 3025 | G |
| EPHA1 | R929C | G | 2872 | A | FAT2 | L3809I | G | 11438 | T | G6PC | K60T | A | 258 | C | GPR98 | F1292L | C | 3972 | A |
| EPHA1 | R844W | G | 2617 | A | FAT2 | R3465Q | C | 10407 | T | G6PC | K207R | A | 699 | G | GPR98 | A1602V | C | 4901 | T |
| EPHA1 | C944R | A | 2917 | G | FAT2 | T1361M | G | 4095 | A | G6PC | K354N | G | 1141 | T | GPR98 | S1796Y | C | 5483 | A |
| EPHA1 | Y493C | T | 1565 | C | FAT2 | H3853Y | G | 11570 | A | G6PC2 | F28C | T | 175 | G | GPR98 | S2200Y | C | 6695 | A |
| EPHA1 | R844Q | C | 2618 | T | FAT2 | R214* | G | 653 | A | G6PC3 | A156V | C | 683 | T | GPR98 | L2492R | T | 7571 | G |
| EPHA1 | R351H | C | 1139 | T | FAT2 | R4304Q | C | 12924 | T | G6PD | V387I | C | 1212 | T | GPR98 | L3032I | C | 9190 | A |
| EPHA10 | V317M | C | 1035 | T | FAT2 | Y492D | A | 1487 | C | G6PD | Q289H | C | 920 | A | GPR98 | A3782V | C | 11441 | T |
| EPHA10 | G238E | C | 799 | T | FAT2 | V3004I | C | 9023 | T | GAA | E762K | G | 2651 | A | GPR98 | L4323I | C | 13063 | A |
| EPHA10 | A225V | G | 760 | A | FAT3 | V301A | T | 919 | C | GAA | E748D | G | 2611 | T | GPR98 | S5821Y | C | 17558 | A |
| EPHA10 | R11H | C | 118 | T | FAT3 | S3505L | C | 10531 | T | GAA | A452V | C | 1722 | T | GPR98 | F6181C | T | 18638 | G |

| | | | | | | | | | | | | | | | | | | |
|---|---|---|---|---|---|---|---|---|---|---|---|---|---|---|---|---|---|---|
| EPHA2 | V383M | C | 1302 | T | FAT3 | T2527I | C | 7597 | T | GAA | G461S | G | 1748 | A | GPR98 | I3519R | T | 10652 | G |
| EPHA2 | P63L | G | 343 | A | FAT3 | R4213C | C | 12654 | T | GAB1 | P127S | C | 806 | T | GPRASP1 | A1012T | G | 3744 | A |
| EPHA3 | S91P | T | 496 | C | FAT3 | S3200L | C | 9616 | T | GAB1 | - | G | 0 | A | GPRASP1 | R1222C | C | 4374 | T |
| EPHA3 | A427V | C | 1505 | T | FAT3 | E3493D | G | 10496 | T | GAB1 | T157N | C | 897 | A | GPRASP2 | V738I | G | 2832 | A |
| EPHA3 | T102S | A | 529 | T | FAT3 | V1661I | G | 4998 | A | GAB1 | D86Y | G | 683 | T | GPRC5A | R238C | C | 1362 | T |
| EPHA3 | A748T | G | 2467 | A | FAT3 | R4233H | G | 12715 | A | GAB2 | R100C | G | 323 | A | GPRC5A | S110F | C | 979 | T |
| EPHA4 | A193T | C | 619 | T | FAT3 | V62I | G | 201 | A | GAB3 | R322C | G | 1012 | A | GPRC5B | A31T | C | 283 | T |
| EPHA4 | A458T | C | 1414 | T | FAT3 | L621F | C | 1878 | T | GAB3 | L552F | G | 1702 | A | GPRC5B | P82L | G | 437 | A |
| EPHA4 | P694A | G | 2122 | C | FAT3 | E1522K | G | 4581 | A | GAB4 | N444D | T | 1438 | C | GPRC5B | P367S | G | 1291 | A |
| EPHA4 | R311Q | C | 974 | T | FAT3 | E1137* | G | 3426 | T | GAB4 | E483A | T | 1556 | G | GPRC5B | G280S | C | 1030 | T |
| EPHA4 | P112L | G | 377 | A | FAT3 | A3964V | C | 11908 | T | GAB4 | V382F | C | 1252 | A | GPRC5B | A194T | C | 772 | T |
| EPHA4 | C73F | C | 260 | A | FAT3 | T4190M | C | 12586 | T | GABBR2 | A46T | C | 596 | T | GPRC5C | G43* | G | 638 | T |
| EPHA5 | I808L | T | 3023 | G | FAT3 | A991V | C | 2989 | T | GABBR2 | F760L | G | 2740 | T | GPRC5D | V252G | A | 755 | C |
| EPHA5 | R541* | G | 2222 | A | FAT3 | S1404N | G | 4228 | A | GABBR2 | R304Q | C | 1371 | T | GPRC6A | A687T | C | 2059 | T |
| EPHA6 | H686N | C | 2094 | A | FAT3 | G3374R | G | 10137 | A | GABBR2 | V138I | C | 872 | T | GPRC6A | T881I | G | 2642 | A |
| EPHA6 | R182H | G | 583 | A | FAT3 | D928Y | G | 2799 | T | GABBR2 | R174W | G | 980 | A | GPRC6A | Y494C | T | 1481 | C |
| EPHA6 | A614V | C | 1879 | T | FAT3 | S1461Y | C | 4399 | A | GABPB2 | A84V | C | 582 | T | GPRIN1 | R386C | G | 1334 | A |
| EPHA6 | A153V | C | 496 | T | FAT3 | K2320R | A | 6976 | G | GABRA1 | F253C | T | 1226 | G | GPRIN1 | R995C | G | 3161 | A |
| EPHA6 | V845M | G | 2571 | A | FAT3 | A3704V | C | 11128 | T | GABRA1 | E396* | G | 1654 | T | GPRIN2 | A359V | C | 1349 | T |
| EPHA6 | A9V | C | 64 | T | FAT3 | T51S | A | 168 | T | GABRA2 | Y237C | T | 717 | C | GPRIN2 | S122P | T | 637 | C |
| EPHA7 | M572T | A | 1900 | G | FAT3 | G427C | G | 1296 | T | GABRA2 | D441E | A | 1330 | C | GPRIN3 | N45D | T | 652 | C |
| EPHA7 | Q987H | T | 3146 | G | FAT3 | P1649L | C | 4963 | T | GABRA4 | E283* | C | 1830 | A | GPS1 | S16Y | C | 77 | A |
| EPHA7 | E460K | C | 1563 | T | FAT4 | R511C | C | 1544 | T | GABRA4 | N47H | T | 1122 | G | GPS2 | S214L | G | 1156 | A |
| EPHA7 | L195S | A | 769 | G | FAT4 | A2421T | G | 7274 | A | GABRA5 | K190N | A | 910 | C | GPSM1 | F551L | C | 1873 | A |
| EPHA7 | T122A | T | 549 | C | FAT4 | K945N | G | 2848 | T | GABRA5 | N43S | A | 468 | G | GPSM2 | Y65H | T | 966 | C |
| EPHA8 | A665S | G | 2065 | T | FAT4 | R2329C | C | 6998 | T | GABRA5 | Q224H | G | 1012 | T | GPSM2 | R400H | G | 1972 | A |
| EPHA8 | G156C | G | 538 | T | FAT4 | V1430I | G | 4301 | A | GABRA6 | E320K | G | 1233 | A | GPT | R252H | G | 978 | A |
| EPHA8 | R522H | G | 1637 | A | FAT4 | D1883N | G | 5660 | A | GABRA6 | V32I | G | 369 | A | GPT2 | R206Q | G | 729 | A |
| EPHA8 | S770R | A | 2380 | C | FAT4 | Q47R | A | 153 | G | GABRB1 | A363T | G | 1379 | A | GPX2 | G28C | C | 84 | A |
| EPHA8 | R946Q | G | 2909 | A | FAT4 | A3113V | C | 9351 | T | GABRB1 | R405H | G | 1506 | A | GPX4 | R39C | C | 192 | T |
| EPHA8 | R992W | C | 3046 | T | FAT4 | R1806H | G | 5430 | A | GABRB1 | P54S | C | 452 | T | GPX4 | A140T | G | 495 | A |
| EPHA8 | A306V | C | 989 | T | FAT4 | V134I | G | 413 | A | GABRB1 | R416C | C | 1538 | T | GPX8 | L34I | C | 175 | A |
| EPHA8 | V418M | G | 1324 | A | FAT4 | R3615W | C | 10856 | T | GABRB1 | T258A | A | 1064 | G | GRAMD1A | R686W | C | 2056 | T |

| | | | | | | | | | | | | | | | | | | | |
|---|---|---|---|---|---|---|---|---|---|---|---|---|---|---|---|---|---|---|---|
| EPHB1 | D706N | G | 2486 | A | FAT4 | S4685R | C | 14068 | G | GABRB2 | T106P | T | 316 | G | GRAMD1A | R139H | G | 416 | A |
| EPHB1 | E873D | G | 2989 | T | FAT4 | A132T | G | 407 | A | GABRB2 | R387W | G | 1159 | A | GRAMD1A | S301R | C | 903 | A |
| EPHB1 | Y471F | A | 1782 | T | FAT4 | L540M | C | 1631 | A | GABRB3 | A5V | G | 94 | A | GRAMD1A | A640T | G | 1918 | A |
| EPHB1 | G33D | G | 468 | A | FAT4 | G947D | G | 2853 | A | GABRB3 | S356N | C | 1147 | T | GRAMD1A | G368S | G | 1102 | A |
| EPHB1 | Q839H | G | 2887 | T | FAT4 | L2423S | T | 7281 | C | GABRB3 | R353H | C | 1138 | T | GRAMD1B | R612W | C | 2163 | T |
| EPHB1 | K643N | G | 2299 | C | FAT4 | F2513I | T | 7550 | A | GABRB3 | K127N | T | 461 | G | GRAMD1B | G70S | G | 537 | A |
| EPHB2 | R164Q | G | 509 | A | FAT4 | T3352N | C | 10068 | A | GABRD | - | T | 0 | C | GRAMD1B | P57L | C | 499 | T |
| EPHB2 | R306H | G | 935 | A | FAT4 | A4031V | C | 12105 | T | GABRD | A263V | C | 871 | T | GRAMD1B | P18S | C | 381 | T |
| EPHB2 | G501S | G | 1519 | A | FAT4 | A132T | G | 407 | A | GABRD | V127M | G | 462 | A | GRAMD1B | M202I | G | 935 | T |
| EPHB2 | R610W | C | 1846 | T | FAT4 | W906* | G | 2731 | A | GABRE | L6F | G | 70 | A | GRAMD2 | M325V | T | 997 | C |
| EPHB2 | R1033W | C | 3115 | T | FAT4 | G2170V | G | 6522 | T | GABRE | I234T | A | 755 | G | GRAMD2 | E63* | C | 211 | A |
| EPHB2 | S218L | C | 671 | T | FAT4 | L419P | T | 1269 | C | GABRG1 | S16I | C | 215 | A | GRAMD3 | K177N | G | 731 | T |
| EPHB3 | Q997R | A | 3442 | G | FAT4 | E4032K | G | 12107 | A | GABRG2 | D461G | A | 1740 | G | GRAMD3 | K141E | A | 621 | G |
| EPHB3 | A683T | G | 2499 | A | FAT4 | V4258I | G | 12785 | A | GABRG3 | R309C | C | 925 | T | GRAMD4 | P545S | C | 1846 | T |
| EPHB3 | E85G | A | 706 | G | FAT4 | E1642* | G | 4937 | T | GABRG3 | S360P | T | 1078 | C | GRAMD4 | R476Q | G | 1640 | A |
| EPHB3 | T94M | C | 733 | T | FAT4 | R1685Q | G | 5067 | A | GABRG3 | A330V | C | 989 | T | GRAP | S115L | G | 544 | A |
| EPHB3 | M752I | G | 2708 | A | FAT4 | R2203W | C | 6620 | T | GABRG3 | L55P | T | 164 | C | GRB10 | R315Q | C | 975 | T |
| EPHB4 | R866H | C | 3066 | T | FAT4 | D2664N | G | 8003 | A | GABRG3 | C419F | G | 1256 | T | GRB10 | A166T | C | 527 | T |
| EPHB4 | R535W | G | 2072 | A | FAT4 | D3012Y | G | 9047 | T | GABRG3 | E26* | G | 76 | T | GRB10 | Y354H | A | 1091 | G |
| EPHB4 | R165C | G | 962 | A | FAT4 | L3123V | T | 9380 | G | GABRP | D415G | A | 1442 | G | GRB10 | F261V | A | 812 | C |
| EPHB4 | R535W | G | 2072 | A | FAT4 | E3293* | G | 9890 | T | GABRQ | G569V | G | 1726 | T | GRB14 | R260K | C | 1318 | T |
| EPHB4 | R866H | C | 3066 | T | FAT4 | A1693T | G | 5090 | A | GABRR2 | M401V | T | 1260 | C | GRB7 | R339Q | G | 1016 | A |
| EPHB4 | S726L | G | 2646 | A | FATE1 | - | G | 0 | T | GABRR3 | S183L | G | 1232 | A | GRB7 | A160V | C | 479 | T |
| EPHB6 | L814F | C | 3227 | T | FAU | R115H | C | 451 | T | GABRR3 | F41L | G | 807 | T | GRB7 | R461H | G | 1382 | A |
| EPHB6 | V491I | G | 2258 | A | FBF1 | H1028Y | G | 3356 | A | GABRR3 | F33V | A | 781 | C | GREB1 | R1758W | C | 5572 | T |
| EPHB6 | R133C | C | 1184 | T | FBF1 | P257L | G | 1044 | A | GAD1 | A277V | C | 1380 | T | GREB1 | R1254C | C | 4060 | T |
| EPHB6 | Q86H | G | 1045 | T | FBF1 | A833T | C | 2771 | T | GAD1 | G506D | G | 2067 | A | GREB1 | A135T | G | 703 | A |
| EPHB6 | R106* | C | 1103 | T | FBF1 | A1012V | G | 3309 | A | GAD2 | R27* | C | 582 | T | GREB1 | S1427L | C | 4580 | T |
| EPHX1 | A205T | G | 693 | A | FBF1 | R997C | G | 3263 | A | GADD45A | G39R | G | 549 | C | GREB1 | V1211I | G | 3931 | A |
| EPHX1 | G225W | G | 753 | T | FBF1 | A313T | C | 1211 | T | GADD45A | Q79R | A | 670 | G | GREB1 | A1808V | C | 5723 | T |
| EPHX3 | R52Q | C | 364 | T | FBF1 | K1123N | C | 3643 | A | GADD45B | D128N | G | 604 | A | GREB1 | P347S | C | 1339 | T |
| EPHX4 | A308T | G | 1020 | A | FBL | A228T | C | 796 | T | GADD45G | P153T | C | 566 | A | GREB1 | P1417T | C | 4549 | A |
| EPHX4 | Y120* | T | 458 | G | FBLN1 | R615W | C | 1946 | T | GADL1 | R7C | G | 170 | A | GREB1 | R1697W | C | 5389 | T |
| EPM2AIP1 | R56C | G | 393 | A | FBLN1 | G287R | G | 962 | A | GAK | A404T | C | 1321 | T | GREB1 | Y568C | A | 2003 | G |
| EPM2AIP1 | E393* | C | 1404 | A | FBLN2 | R1097H | G | 3409 | A | GAK | A476S | C | 1537 | A | GREB1 | R1049Q | G | 3446 | A |
| EPN1 | R174Q | G | 1068 | A | FBLN2 | G234D | G | 820 | A | GAK | T761A | T | 2392 | C | GREB1 | - | G | 0 | T |

| | | | | | | | | | | | | | | | | | | | |
|---|---|---|---|---|---|---|---|---|---|---|---|---|---|---|---|---|---|---|---|
| EPN1 | T580M | C | 2286 | T | FBLN2 | S551P | T | 1770 | C | GAL | F96C | T | 505 | G | GREB1L | S257L | C | 1041 | T |
| EPN2 | A428T | G | 1711 | A | FBLN5 | E488* | C | 1767 | A | GAL | N84H | A | 468 | C | GREB1L | G444S | G | 1601 | A |
| EPN2 | A102T | G | 733 | A | FBLN7 | T407M | C | 1491 | T | GAL3ST1 | Q366* | G | 1414 | A | GREB1L | P1383T | C | 4418 | A |
| EPN3 | P412H | C | 1423 | A | FBLN7 | V2A | T | 276 | C | GAL3ST1 | R317C | G | 1267 | A | GREB1L | R1889C | C | 5936 | T |
| EPN3 | L188M | C | 750 | A | FBLN7 | E102K | G | 575 | A | GAL3ST2 | V103M | G | 438 | A | GREB1L | L1182M | C | 3815 | A |
| EPOR | E197D | C | 727 | A | FBN1 | K2839N | C | 8845 | A | GAL3ST2 | G326S | G | 1107 | A | GREB1L | D1619Y | G | 5126 | T |
| EPRS | G1108D | C | 3593 | T | FBN1 | R464C | G | 1718 | A | GAL3ST2 | A189T | G | 696 | A | GRHL1 | R434* | C | 1441 | T |
| EPRS | Y690H | A | 2338 | G | FBN1 | A1152V | G | 3783 | A | GAL3ST2 | A164T | G | 621 | A | GRHL1 | Y356H | T | 1207 | C |
| EPRS | - | C | 0 | A | FBN1 | T791A | T | 2699 | C | GAL3ST2 | G326S | G | 1107 | A | GRHL1 | A47V | C | 281 | T |
| EPRS | G465W | C | 1663 | A | FBN1 | A252D | G | 1083 | T | GAL3ST2 | R211C | C | 762 | T | GRHL1 | R188W | C | 703 | T |
| EPS15L1 | E809D | C | 2452 | A | FBN1 | E2253* | C | 7085 | A | GAL3ST2 | L140P | T | 550 | C | GRHL1 | R301* | C | 1042 | T |
| EPS15L1 | A596V | G | 1812 | A | FBN1 | R974H | C | 3249 | T | GAL3ST3 | A186V | G | 839 | A | GRHL1 | R434Q | G | 1442 | A |
| EPS8 | R571Q | C | 2149 | T | FBN1 | D2411N | C | 7559 | T | GAL3ST3 | R252W | G | 1036 | A | GRHL1 | R536* | C | 1747 | T |
| EPS8 | R571* | G | 2148 | A | FBN1 | R2051K | C | 6480 | T | GAL3ST3 | V116M | C | 628 | T | GRHL2 | - | T | 0 | C |
| EPS8 | L149I | G | 882 | T | FBN1 | R1840C | G | 5846 | A | GAL3ST3 | A295S | C | 1165 | A | GRHL2 | E137K | G | 747 | A |
| EPS8L1 | P410L | C | 1333 | T | FBN1 | Q1751K | G | 5579 | T | GAL3ST3 | E410K | C | 1510 | T | GRHL2 | R537Q | G | 1948 | A |
| EPS8L1 | G405R | G | 1317 | A | FBN1 | E2193K | C | 6905 | T | GAL3ST3 | S16I | C | 329 | A | GRHL3 | A529V | C | 1713 | T |
| EPS8L2 | P244S | C | 977 | T | FBN1 | D1197G | T | 3918 | C | GAL3ST4 | K76T | T | 416 | G | GRHL3 | V261I | G | 908 | A |
| EPS8L3 | R229W | G | 915 | A | FBN1 | F13L | A | 367 | C | GALC | D49E | G | 362 | T | GRHL3 | F550L | T | 1775 | C |
| EPS8L3 | P187L | G | 790 | A | FBN2 | P830Q | G | 2928 | T | GALC | R127Q | C | 595 | T | GRHPR | - | A | 0 | G |
| EPS8L3 | A547V | G | 1870 | A | FBN2 | R1068W | G | 3641 | A | GALK1 | R117W | G | 349 | A | GRHPR | K233T | A | 783 | C |
| EPS8L3 | A494V | G | 1711 | A | FBN2 | C1621F | C | 5301 | A | GALK2 | P89L | C | 364 | T | GRHPR | V239G | T | 801 | G |
| EPSTI1 | E244* | C | 795 | A | FBN2 | A246V | G | 1176 | A | GALK2 | E298D | G | 992 | T | GRIA1 | R455H | G | 1507 | A |
| EPSTI1 | E92D | C | 341 | A | FBN2 | C980W | G | 3379 | C | GALNT1 | R134Q | G | 504 | A | GRIA1 | - | A | 2864 | C |
| EPYC | E258G | T | 866 | C | FBN2 | R2094W | G | 6719 | A | GALNT10 | A421T | G | 1398 | A | GRIA1 | R126L | G | 520 | T |
| ERAL1 | K320N | G | 993 | T | FBN2 | E908G | T | 3162 | C | GALNT10 | V559A | T | 1813 | C | GRIA1 | G871C | G | 2754 | T |
| ERAP1 | Y145H | A | 691 | G | FBN2 | N1218S | T | 4092 | C | GALNT11 | E412V | A | 1465 | T | GRIA1 | S645F | C | 2077 | T |
| ERAP2 | R205C | C | 1324 | T | FBN2 | Q2564* | G | 8129 | A | GALNT11 | R126H | G | 607 | A | GRIA2 | G520R | G | 2017 | A |
| ERAP2 | D641V | A | 2633 | T | FBN2 | G2442* | C | 7763 | A | GALNT14 | Q40* | G | 179 | A | GRIA2 | L269F | G | 1266 | T |
| ERAS | S181L | C | 793 | T | FBN2 | N732D | T | 2633 | C | GALNT14 | D114G | T | 402 | C | GRIA2 | S613L | C | 2297 | T |
| ERBB2 | G1056S | G | 3404 | A | FBN2 | G666* | C | 2435 | A | GALNT14 | Q433H | C | 1360 | A | GRIA3 | A14V | C | 333 | T |
| ERBB2 | L755M | T | 2501 | A | FBN3 | L37S | A | 396 | G | GALNT2 | R194* | C | 652 | T | GRIA3 | D416Y | G | 1538 | T |
| ERBB2IP | E303K | G | 998 | A | FBN3 | T2628M | G | 8169 | A | GALNT2 | F316L | C | 1020 | A | GRIA3 | R692* | A | 2366 | T |
| ERBB2IP | N156I | A | 558 | T | FBN3 | W2332C | C | 7282 | A | GALNT2 | S560L | C | 1751 | T | GRIA4 | T875M | C | 3070 | T |
| ERBB2IP | K356T | A | 1158 | C | FBN3 | P2662L | G | 8271 | A | GALNT3 | Y497C | T | 2266 | C | GRIA4 | V780I | G | 2784 | A |
| ERBB2IP | D400E | T | 1291 | G | FBN3 | G2434D | C | 7587 | T | GALNT3 | M408I | C | 2000 | T | GRIA4 | S598N | G | 2239 | A |
| ERBB3 | P262S | C | 977 | T | FBN3 | A623V | G | 2154 | A | GALNT3 | G428D | C | 2059 | T | GRIA4 | E488* | G | 1908 | T |
| ERBB3 | S846I | G | 2730 | T | FBN3 | M862T | A | 2871 | G | GALNT5 | R747W | C | 2724 | T | GRIA4 | A854T | G | 3006 | A |
| ERBB3 | G284R | G | 1043 | A | FBN3 | V1559D | A | 4962 | T | GALNT5 | R561H | G | 2167 | A | GRID1 | A453V | G | 1358 | A |

| | | | | | | | | | | | | | | | | | | | |
|---|---|---|---|---|---|---|---|---|---|---|---|---|---|---|---|---|---|---|---|
| ERBB3 | G325R | G | 1166 | A | FBN3 | Q199R | T | 882 | C | GALNT5 | I174T | T | 1006 | C | GRID1 | R148H | C | 443 | T |
| ERBB3 | M60K | T | 372 | A | FBN3 | V2101A | A | 6588 | G | GALNT5 | E264* | G | 1275 | T | GRID1 | R977Q | C | 2930 | T |
| ERBB3 | V104M | G | 503 | A | FBN3 | G1735D | C | 5490 | T | GALNT5 | R784Q | G | 2836 | A | GRID1 | R535W | G | 1603 | A |
| ERBB3 | V295A | T | 1077 | C | FBN3 | E1435K | C | 4589 | T | GALNT6 | T346P | T | 1357 | G | GRID2 | P991A | C | 3229 | G |
| ERBB4 | T461A | T | 1692 | C | FBN3 | G41D | C | 408 | T | GALNT6 | R430H | C | 1610 | T | GRID2 | K167T | A | 758 | C |
| ERBB4 | R81* | G | 552 | A | FBN3 | P2078Q | G | 6519 | T | GALNT7 | W649S | G | 2029 | C | GRID2 | T537M | C | 1868 | T |
| ERBB4 | R106C | G | 627 | A | FBN3 | R2108C | G | 6608 | A | GALNT7 | Y112C | A | 418 | G | GRID2 | A744V | C | 2489 | T |
| ERBB4 | R1273W | G | 4128 | A | FBP2 | T40M | G | 186 | A | GALNT7 | F556V | T | 1749 | G | GRID2 | R128M | G | 641 | T |
| ERBB4 | R168W | G | 813 | A | FBP2 | K231N | C | 760 | A | GALNT7 | E256* | G | 849 | T | GRID2 | R550* | C | 1906 | T |
| ERBB4 | R488W | G | 1773 | A | FBRSL1 | A293T | G | 877 | A | GALNT7 | E516* | G | 1629 | T | GRID2IP | R834W | G | 2500 | A |
| ERBB4 | Y1301C | T | 4213 | C | FBRSL1 | R424W | C | 1270 | T | GALNT8 | A271T | G | 903 | A | GRID2IP | R772H | C | 2315 | T |
| ERBB4 | E874* | C | 2931 | A | FBRSL1 | R436W | C | 1306 | T | GALNT8 | R294W | C | 972 | T | GRID2IP | A38V | G | 113 | A |
| ERBB4 | A1039E | G | 3427 | T | FBXL13 | N192I | T | 1002 | A | GALNT8 | L213F | G | 731 | T | GRID2IP | A1190V | G | 3569 | A |
| ERBB4 | V20I | C | 369 | T | FBXL16 | F133L | A | 729 | G | GALNT9 | E315K | C | 1059 | T | GRID2IP | R1144W | G | 3430 | A |
| ERC1 | T511A | A | 1937 | G | FBXL17 | R216* | G | 848 | A | GALNT9 | R533H | C | 1714 | T | GRIK1 | K464R | T | 1423 | C |
| ERC1 | N303H | A | 1313 | C | FBXL17 | R216* | G | 848 | A | GALNT9 | V214M | C | 756 | T | GRIK1 | V832E | A | 2527 | T |
| ERC1 | E591D | A | 2179 | C | FBXL17 | T277A | T | 1618 | C | GALNTL2 | E395* | G | 1686 | T | GRIK1 | F225L | G | 707 | T |
| ERC1 | R1020I | G | 3465 | T | FBXL18 | R446C | G | 1413 | A | GALNTL2 | A266V | C | 1300 | T | GRIK2 | E233* | G | 1187 | T |
| ERC2 | R415C | G | 1499 | A | FBXL18 | G93D | C | 355 | T | GALNTL2 | E395K | G | 1686 | A | GRIK2 | R15C | C | 533 | T |
| ERC2 | R619Q | C | 2112 | T | FBXL19 | C39R | T | 273 | C | GALNTL4 | E207K | C | 1040 | T | GRIK2 | E723* | G | 2657 | T |
| ERC2 | A688S | C | 2318 | A | FBXL19 | R626C | C | 2034 | T | GALNTL4 | R272Q | C | 1236 | T | GRIK2 | E743K | G | 2717 | A |
| ERC2 | D760Y | C | 2534 | A | FBXL19 | P205L | C | 772 | T | GALNTL5 | T158A | A | 693 | G | GRIK2 | E469* | G | 1895 | T |
| ERC2 | S666Y | G | 2253 | T | FBXL19 | R312Q | G | 1093 | A | GALNTL5 | - | G | 0 | T | GRIK2 | E665K | G | 2483 | A |
| ERC2 | R619Q | C | 2112 | T | FBXL2 | R235Q | G | 795 | A | GALNTL5 | K267N | G | 1022 | T | GRIK2 | R873C | C | 3107 | T |
| ERC2 | E350D | C | 1306 | A | FBXL20 | E83D | C | 492 | A | GALNTL5 | E321* | G | 1182 | T | GRIK3 | R450W | G | 1365 | A |
| ERC2 | S321R | T | 1217 | G | FBXL3 | P96Q | G | 612 | T | GALNTL6 | R126C | C | 1033 | T | GRIK3 | V106M | C | 333 | T |
| ERC2 | E257K | C | 1025 | T | FBXL3 | V419M | C | 1580 | T | GALNTL6 | N238I | A | 1370 | T | GRIK3 | D155N | C | 480 | T |
| ERCC2 | A144V | G | 509 | A | FBXL3 | D423N | C | 1592 | T | GALNTL6 | Y399* | C | 1854 | A | GRIK4 | E762K | G | 2341 | A |
| ERCC2 | Q187K | G | 637 | T | FBXL4 | S596L | G | 2216 | A | GALNTL6 | A454V | C | 2018 | T | GRIK4 | R760Q | G | 2336 | A |
| ERCC2 | F403C | A | 1286 | C | FBXL4 | R98Q | C | 722 | T | GALR1 | S140L | C | 419 | T | GRIK4 | G60D | G | 236 | A |
| ERCC2 | R75I | C | 302 | A | FBXL5 | V324G | A | 1096 | C | GALR1 | N233S | A | 698 | G | GRIK4 | E233G | A | 755 | G |
| ERCC3 | D258E | A | 869 | T | FBXL5 | M18T | A | 178 | G | GALR1 | A80T | G | 238 | A | GRIK5 | R682W | G | 2079 | A |
| ERCC3 | K528N | C | 1679 | A | FBXL6 | G419D | C | 1281 | T | GALR2 | A222T | G | 745 | A | GRIK5 | R41H | C | 157 | T |
| ERCC4 | L49I | C | 154 | A | FBXL7 | G393S | G | 1658 | A | GALR2 | V291A | T | 953 | C | GRIK5 | L254P | A | 796 | G |
| ERCC4 | A257V | C | 779 | T | FBXL7 | R49H | G | 627 | A | GALR3 | V94I | G | 305 | A | GRIK5 | A401V | G | 1237 | A |
| ERCC4 | S561I | G | 1691 | T | FBXL7 | V191I | G | 1052 | A | GALR3 | R180H | G | 564 | A | GRIK5 | A101T | C | 336 | T |
| ERCC4 | S613L | C | 1847 | T | FBXL7 | R317C | C | 1430 | T | GALR3 | L59P | T | 201 | C | GRIK5 | Y686H | A | 2091 | G |
| ERCC4 | A315V | C | 953 | T | FBXL7 | T160M | C | 960 | T | GALT | P145T | C | 475 | A | GRIN1 | P473S | C | 1423 | T |
| ERCC5 | P414L | C | 2664 | T | FBXL7 | R353W | C | 1538 | T | GALT | T350P | A | 1090 | C | GRIN1 | C329R | T | 991 | C |

| | | | | | | | | | | | | | | | | | | | |
|---|---|---|---|---|---|---|---|---|---|---|---|---|---|---|---|---|---|---|---|
| ERCC5 | Q900H | A | 4123 | C | FBXL7 | K411T | A | 1713 | C | GALT | R223H | G | 710 | A | GRIN1 | E246K | G | 742 | A |
| ERCC6 | D454Y | C | 1439 | A | FBXO10 | T902M | G | 2754 | A | GALT | I32V | A | 136 | G | GRIN1 | K343N | G | 1035 | T |
| ERCC6 | R670W | G | 2087 | A | FBXO11 | R800W | G | 2471 | A | GAMT | N170D | T | 585 | C | GRIN2A | A136P | C | 716 | G |
| ERCC6 | V780I | C | 2417 | T | FBXO11 | R208C | G | 695 | A | GAMT | R192H | C | 652 | T | GRIN2A | K1124N | C | 3682 | A |
| ERCC6 | K663E | T | 2066 | C | FBXO15 | C70R | A | 288 | G | GAN | E3D | G | 153 | T | GRIN2A | N491S | T | 1782 | C |
| ERCC6 | S146F | G | 516 | A | FBXO16 | W219R | A | 805 | G | GAN | R477Q | G | 1574 | A | GRIN2A | R504W | G | 1820 | A |
| ERCC6L | D507N | C | 1655 | T | FBXO17 | T261M | G | 942 | A | GAN | S183Y | C | 692 | A | GRIN2B | A271V | G | 1022 | A |
| ERCC6L | W268* | C | 940 | T | FBXO17 | H72Y | G | 374 | A | GANAB | Y460H | A | 1411 | G | GRIN2B | I766N | A | 2507 | T |
| ERCC8 | K232E | T | 737 | C | FBXO17 | P361H | G | 1242 | T | GANAB | D717Y | C | 2182 | A | GRIN2B | A109T | C | 535 | T |
| ERCC8 | A203T | C | 650 | T | FBXO18 | D914N | G | 2844 | A | GANAB | G238E | C | 746 | T | GRIN2B | - | T | 0 | C |
| ERF | P518L | G | 1711 | A | FBXO18 | V1093I | G | 3381 | A | GANAB | R839Q | C | 2549 | T | GRIN2C | A834T | C | 2647 | T |
| ERF | E41K | C | 279 | T | FBXO18 | I512S | T | 1639 | G | GANAB | Q436P | T | 1340 | G | GRIN2C | L78I | G | 379 | T |
| ERF | E434K | C | 1458 | T | FBXO18 | I989S | T | 3070 | G | GANAB | D496Y | C | 1519 | A | GRIN2C | A608V | G | 1970 | A |
| ERG | R57H | C | 356 | T | FBXO18 | F536L | T | 1710 | C | GANC | I74M | T | 462 | G | GRIN2D | T429M | C | 1374 | T |
| ERG | D182N | C | 730 | T | FBXO2 | R177C | G | 871 | A | GAP43 | T233M | C | 1166 | T | GRIN3A | H103Y | G | 908 | A |
| ERG | L165* | A | 680 | T | FBXO21 | Y172C | T | 554 | C | GAP43 | K191N | G | 1041 | T | GRIN3A | R366M | C | 1698 | A |
| ERG | R147W | G | 625 | A | FBXO21 | I347T | A | 1079 | G | GAPDH | A329T | G | 1651 | A | GRIN3A | R225C | G | 1274 | A |
| ERGIC1 | G172R | G | 653 | A | FBXO21 | L179* | A | 575 | C | GAPDH | V68A | T | 869 | C | GRIN3A | I954T | A | 3462 | G |
| ERGIC2 | N375S | T | 1124 | C | FBXO22OS | V327I | G | 1084 | A | GAPT | L19I | C | 517 | A | GRIN3A | R708* | G | 2723 | A |
| ERI3 | D293N | C | 1059 | T | FBXO24 | P540H | C | 1631 | A | GAPVD1 | R43H | G | 288 | A | GRIN3A | R1090C | G | 3869 | A |
| ERI3 | E67K | C | 381 | T | FBXO24 | R527C | C | 1591 | T | GAPVD1 | D810N | G | 2588 | A | GRIN3A | E871A | T | 3213 | G |
| ERICH1 | V35I | C | 181 | T | FBXO24 | D112N | G | 346 | A | GARNL3 | V46M | G | 488 | A | GRIN3A | R225C | G | 1274 | A |
| ERICH1 | E409K | C | 1303 | T | FBXO24 | L187I | C | 571 | A | GARNL3 | R396H | G | 1539 | A | GRIN3A | A127V | G | 981 | A |
| ERICH1 | D400N | C | 1276 | T | FBXO25 | V143M | G | 546 | A | GARNL3 | L236I | C | 1058 | A | GRIN3B | G866W | G | 2596 | T |
| ERLEC1 | G5D | G | 145 | A | FBXO25 | K146N | G | 557 | T | GARS | T127A | A | 620 | G | GRINA | R304Q | G | 1189 | A |
| ERLIN1 | A254T | C | 3468 | T | FBXO27 | V222G | A | 785 | C | GART | A529S | C | 1849 | A | GRIP1 | E899Q | C | 2936 | G |
| ERLIN2 | A225D | C | 789 | A | FBXO28 | R325* | C | 992 | T | GAS1 | C203Y | C | 1018 | T | GRIP1 | R985I | C | 3195 | A |
| ERMN | H12Y | G | 268 | A | FBXO3 | R329H | C | 1014 | T | GAS2 | S180Y | C | 845 | A | GRIP1 | N421S | T | 1503 | C |
| ERMN | S245N | C | 968 | T | FBXO30 | A428T | C | 1449 | T | GAS2L2 | P739S | G | 2243 | A | GRIP1 | T215M | G | 885 | A |
| ERMN | R75I | C | 458 | A | FBXO31 | R288H | C | 907 | T | GAS2L2 | P778H | G | 2361 | T | GRIP1 | N160D | T | 719 | C |
| ERMP1 | V231I | C | 781 | T | FBXO32 | K143N | C | 621 | A | GAS2L2 | K502N | C | 1534 | A | GRIPAP1 | V776I | C | 2361 | T |
| ERMP1 | Y446C | T | 1427 | C | FBXO32 | K131R | T | 584 | C | GAS2L2 | R439I | C | 1344 | A | GRIPAP1 | R471H | C | 1447 | T |
| ERN1 | R946Q | C | 2950 | T | FBXO33 | N512T | T | 1873 | G | GAS2L2 | D431N | C | 1319 | T | GRK1 | V399L | G | 1427 | C |
| ERN1 | D167N | C | 612 | T | FBXO34 | E100K | G | 543 | A | GAS2L3 | Y158H | T | 603 | C | GRK4 | G562D | G | 2028 | A |
| ERN1 | H43R | T | 241 | C | FBXO34 | R278S | C | 1077 | A | GAS2L3 | R610H | G | 1960 | A | GRK5 | R216C | C | 975 | T |
| ERN2 | A736V | G | 2376 | A | FBXO34 | R670W | C | 2253 | T | GAS2L3 | P17L | C | 181 | T | GRK5 | N306S | A | 1246 | G |
| ERN2 | T731M | G | 2361 | A | FBXO38 | A1053S | G | 3325 | T | GAS6 | R557C | G | 1822 | A | GRK5 | K389N | G | 1496 | T |
| ERO1L | E311* | C | 1155 | A | FBXO38 | E557K | G | 1837 | A | GAS6 | P102L | G | 458 | A | GRK5 | V466I | G | 1725 | A |
| ERO1L | V140A | A | 643 | G | FBXO38 | R1158C | C | 3640 | T | GAS7 | A4T | C | 171 | T | GRK5 | C520* | C | 1889 | A |

| | | | | | | | | | | | | | | | | | | | |
|---|---|---|---|---|---|---|---|---|---|---|---|---|---|---|---|---|---|---|---|
| ERO1L | R55K | C | 388 | T | FBXO38 | I185N | T | 722 | A | GAS7 | R92Q | C | 436 | T | GRK6 | Y309H | T | 1085 | C |
| ERO1LB | V440F | C | 1520 | A | FBXO39 | M112I | G | 457 | A | GAS7 | T466K | G | 1558 | T | GRK7 | R68H | G | 340 | A |
| ERO1LB | R286H | C | 1059 | T | FBXO39 | D238N | G | 833 | A | GAS8 | A460T | G | 1500 | A | GRK7 | R35H | G | 241 | A |
| ERO1LB | Y177H | A | 731 | G | FBXO39 | A40V | C | 240 | T | GAST | R57* | C | 236 | T | GRK7 | R32Q | G | 232 | A |
| ERO1LB | N264H | T | 992 | G | FBXO40 | H313Q | C | 1353 | A | GATA1 | V278M | G | 943 | A | GRK7 | L264I | C | 927 | A |
| ERP29 | L19M | C | 173 | A | FBXO40 | G337D | G | 1424 | A | GATA2 | R330* | G | 1320 | A | GRLF1 | R783* | C | 2347 | T |
| ERP29 | R75H | G | 342 | A | FBXO40 | L562I | C | 2098 | A | GATA2 | A109V | G | 658 | A | GRLF1 | R1187Q | G | 3560 | A |
| ERP29 | A163T | G | 605 | A | FBXO41 | A377V | G | 1130 | A | GATA3 | V68F | G | 770 | T | GRLF1 | A435V | C | 1304 | T |
| ERP44 | R148W | G | 642 | A | FBXO42 | P420H | G | 1476 | T | GATA3 | S317F | C | 1518 | T | GRLF1 | R1187Q | G | 3560 | A |
| ERV3 | E325K | C | 1474 | T | FBXO42 | V74D | A | 438 | T | GATA3 | K377N | A | 1699 | C | GRLF1 | K221Q | A | 661 | C |
| ERV3 | S172P | A | 1015 | G | FBXO43 | W532* | C | 2309 | T | GATA3 | A396T | G | 1754 | A | GRM1 | M442V | A | 1794 | G |
| ERVWE1 | R26C | G | 1119 | A | FBXO43 | V625I | C | 2586 | T | GATA4 | A388V | C | 1721 | T | GRM1 | R305H | G | 1384 | A |
| ESAM | D132G | T | 525 | C | FBXO43 | V324M | C | 1683 | T | GATA4 | C274F | G | 1379 | T | GRM1 | P898A | C | 3162 | G |
| ESCO1 | R206H | C | 1703 | T | FBXO43 | R132I | C | 1108 | A | GATA4 | P307S | C | 1477 | T | GRM1 | A265D | C | 1264 | A |
| ESCO1 | K490Q | T | 2554 | G | FBXO44 | R219W | C | 757 | T | GATA6 | R456H | G | 1644 | A | GRM1 | - | A | 0 | G |
| ESCO2 | P592H | C | 1845 | A | FBXO44 | R174H | G | 623 | A | GATA6 | L454V | T | 1637 | G | GRM2 | T791I | C | 2606 | T |
| ESM1 | G105S | C | 459 | T | FBXO45 | S54P | T | 457 | C | GATAD2A | V592M | G | 2135 | A | GRM2 | A740T | G | 2452 | A |
| ESPL1 | L101I | C | 392 | A | FBXO45 | R229I | G | 983 | T | GATAD2A | T336A | A | 1367 | G | GRM2 | Q848R | A | 2777 | G |
| ESPL1 | L344R | T | 1122 | G | FBXO46 | G283R | C | 970 | T | GATAD2A | R185W | C | 914 | T | GRM2 | A16V | C | 281 | T |
| ESPN | F114L | T | 508 | C | FBXO48 | A23T | C | 475 | T | GATAD2B | T328S | T | 1226 | A | GRM2 | F428C | T | 1517 | G |
| ESPN | Y380C | A | 1307 | G | FBXO48 | I46M | A | 546 | C | GATS | E79K | C | 491 | T | GRM3 | I530S | T | 2688 | G |
| ESPN | R806W | C | 2584 | T | FBXO6 | R45Q | G | 269 | A | GATS | Q114H | C | 598 | A | GRM3 | V271I | G | 1910 | A |
| ESPN | D237G | A | 878 | G | FBXO7 | P83T | C | 574 | A | GATSL3 | S43N | C | 258 | T | GRM4 | G417D | C | 1420 | T |
| ESPNL | V436L | G | 1569 | T | FBXO8 | E252G | T | 1618 | C | GBA | R398* | G | 1314 | A | GRM4 | M839T | A | 2686 | G |
| ESPNL | A84T | G | 513 | A | FBXO8 | E216* | C | 1509 | A | GBA2 | Y72C | T | 739 | C | GRM4 | K901T | T | 2872 | G |
| ESPNL | S752L | C | 2518 | T | FBXW10 | L652I | C | 2173 | A | GBA2 | W645* | C | 2459 | T | GRM4 | G24D | C | 241 | T |
| ESR1 | A58T | G | 542 | A | FBXW11 | D373N | C | 1488 | T | GBA3 | I103V | A | 409 | G | GRM5 | R727* | G | 2570 | A |
| ESR1 | A491T | G | 1841 | A | FBXW12 | R411H | G | 1418 | A | GBAS | T32A | A | 123 | G | GRM5 | A1070T | C | 3599 | T |
| ESR1 | R211I | G | 1002 | T | FBXW12 | G136D | G | 593 | A | GBAS | K91T | A | 301 | C | GRM5 | R869M | C | 2997 | A |
| ESR1 | G215* | G | 1013 | T | FBXW12 | A287T | G | 1045 | A | GBE1 | F543C | A | 2272 | C | GRM5 | S440P | A | 1709 | G |
| ESR2 | R207Q | C | 1038 | T | FBXW12 | V144A | T | 617 | C | GBF1 | R678Q | G | 2293 | A | GRM5 | R292H | C | 1266 | T |
| ESR2 | P118L | G | 771 | A | FBXW12 | S225Y | C | 860 | A | GBF1 | S1239G | A | 3975 | G | GRM5 | R1028H | C | 3474 | T |
| ESRP1 | R140Q | G | 602 | A | FBXW2 | K302T | T | 1093 | G | GBF1 | A1739T | G | 5475 | A | GRM5 | R762K | C | 2676 | T |
| ESRP1 | V520F | G | 1741 | T | FBXW4P1 | V406I | C | 1835 | T | GBF1 | H1440R | A | 4579 | G | GRM5 | V217M | C | 1040 | T |
| ESRP1 | I188S | T | 746 | G | FBXW4P1 | L343M | G | 1646 | T | GBF1 | A1243V | C | 3988 | T | GRM5 | E1122K | C | 3755 | T |
| ESRP1 | L281P | T | 1025 | C | FBXW7 | S582L | G | 1894 | A | GBF1 | R530C | C | 1848 | T | GRM5 | A855T | C | 2954 | T |
| ESRP1 | G534R | G | 1783 | A | FBXW7 | E369* | C | 1254 | A | GBF1 | A1243V | C | 3988 | T | GRM5 | D333N | C | 1388 | T |
| ESRP1 | R539Q | G | 1799 | A | FBXW7 | K185N | C | 704 | A | GBP1 | S428L | G | 1503 | A | GRM6 | R486* | G | 1456 | A |
| ESRP1 | R582Q | G | 1928 | A | FBXW7 | H470R | T | 1558 | C | GBP1 | R48H | C | 363 | T | GRM6 | R777Q | C | 2330 | T |

| | | | | | | | | | | | | | | | | | | | |
|---|---|---|---|---|---|---|---|---|---|---|---|---|---|---|---|---|---|---|---|
| ESRP2 | A397T | C | 1728 | T | FBXW7 | R465H | C | 1543 | T | GBP1 | S502L | G | 1725 | A | GRM6 | A447V | G | 1340 | A |
| ESRRA | R352Q | G | 1226 | A | FBXW7 | R222* | G | 813 | A | GBP2 | A313T | C | 1206 | T | GRM6 | A390T | C | 1168 | T |
| ESRRB | G492D | G | 1776 | A | FBXW7 | I257N | A | 919 | T | GBP2 | A240T | C | 987 | T | GRM7 | R659Q | G | 2250 | A |
| ESRRB | R184C | C | 851 | T | FBXW7 | R505C | G | 1662 | A | GBP2 | Q517R | T | 1819 | C | GRM7 | Y762H | T | 2558 | C |
| ESRRB | A307V | C | 1221 | T | FBXW7 | R465C | G | 1542 | A | GBP3 | C223R | A | 872 | G | GRM7 | T634M | C | 2175 | T |
| ESRRB | E151K | G | 752 | A | FBXW7 | N542I | T | 1774 | A | GBP4 | A354D | G | 1159 | T | GRM7 | K389E | A | 1439 | G |
| ESRRB | I206M | T | 919 | G | FBXW7 | S582L | G | 1894 | A | GBP4 | S50Y | G | 247 | T | GRM7 | A812T | G | 2708 | A |
| ESRRG | K402E | T | 1471 | C | FBXW7 | D560G | T | 1828 | C | GBP5 | D140N | C | 955 | T | GRM7 | S352P | T | 1328 | C |
| ESRRG | V58I | C | 439 | T | FBXW7 | R465C | G | 1542 | A | GBP5 | P37H | G | 647 | T | GRM7 | A251V | C | 1026 | T |
| ESRRG | T46M | G | 404 | A | FBXW7 | R367* | G | 1248 | A | GBP5 | T438A | T | 1849 | C | GRM7 | F808L | T | 2698 | G |
| ESX1 | P113L | G | 422 | A | FBXW7 | E369* | C | 1254 | A | GBP6 | R523H | G | 1661 | A | GRM8 | R30I | C | 898 | A |
| ESX1 | P121Q | G | 446 | T | FBXW7 | R224Q | C | 820 | T | GBP6 | N323K | C | 1062 | G | GRM8 | Q503* | G | 2316 | A |
| ESX1 | P284H | G | 935 | T | FBXW7 | D600Y | C | 1947 | A | GBP6 | V180I | G | 631 | A | GRM8 | W305* | C | 1724 | T |
| ESYT1 | R967C | C | 2949 | T | FBXW7 | R505C | G | 1662 | A | GBP6 | A9V | C | 119 | T | GRM8 | S219L | G | 1465 | A |
| ESYT1 | A859V | C | 2626 | T | FBXW7 | R465H | C | 1543 | T | GBP6 | S270Y | C | 902 | A | GRM8 | K382N | C | 1955 | A |
| ESYT1 | L142F | C | 474 | T | FBXW8 | A567T | G | 1781 | A | GBP7 | R577* | G | 1868 | A | GRM8 | R355I | C | 1873 | A |
| ESYT1 | R332* | C | 1044 | T | FBXW8 | D285N | G | 935 | A | GBX1 | P54L | G | 161 | A | GRM8 | - | A | 0 | G |
| ESYT2 | R826H | C | 2477 | T | FBXW8 | V431I | G | 1373 | A | GBX1 | N85D | T | 253 | C | GRM8 | R30I | C | 898 | A |
| ESYT2 | S236G | T | 706 | C | FBXW8 | S526N | G | 1659 | A | GBX1 | K283N | C | 849 | A | GRN | T409K | C | 1445 | A |
| ESYT2 | S534A | A | 1600 | C | FBXW8 | D588N | G | 1844 | A | GBX2 | A141V | G | 460 | A | GRPEL2 | R96G | A | 396 | G |
| ESYT2 | N559H | T | 1675 | G | FBXW9 | R7L | C | 57 | A | GC | F178C | A | 628 | C | GRPR | A274T | G | 1218 | A |
| ESYT3 | R417W | C | 1435 | T | FCAMR | G57D | C | 645 | T | GCAT | A98V | C | 356 | T | GRTP1 | V244M | C | 784 | T |
| ESYT3 | V404M | G | 1396 | A | FCAR | R110W | C | 338 | T | GCC1 | A98T | C | 717 | T | GRTP1 | A143S | C | 481 | A |
| ESYT3 | P48S | C | 328 | T | FCAR | P167S | C | 509 | T | GCC1 | R662H | C | 2410 | T | GRWD1 | V186M | G | 757 | A |
| ESYT3 | N180I | A | 725 | T | FCAR | R110W | C | 338 | T | GCC1 | R569W | G | 2130 | A | GRWD1 | R182Q | G | 746 | A |
| ETAA1 | S569T | G | 1836 | C | FCAR | A51S | G | 161 | T | GCC2 | Q1060H | G | 3227 | T | GRXCR1 | R150W | C | 448 | T |
| ETAA1 | R139H | G | 546 | A | FCER1A | N256T | A | 857 | C | GCC2 | E535K | G | 1650 | A | GRXCR1 | D137N | G | 409 | A |
| ETAA1 | V546D | T | 1767 | A | FCER1G | R45* | C | 176 | T | GCDH | R243W | C | 804 | T | GRXCR1 | Q234H | G | 702 | T |
| ETAA1 | P53S | C | 287 | T | FCF1 | S89P | T | 316 | C | GCDH | R94W | C | 357 | T | GSC | S223* | G | 815 | T |
| ETFA | I269L | T | 886 | G | FCGBP | V849I | C | 2553 | T | GCDH | R82H | G | 322 | A | GSC | V114D | A | 488 | T |
| ETFDH | E412D | A | 1568 | T | FCGBP | V525A | A | 1582 | G | GCET2 | R13Q | C | 172 | T | GSDMA | R167* | C | 617 | T |
| ETNK1 | G323D | G | 990 | A | FCGBP | Q4923H | C | 14777 | A | GCFC1 | D615Y | C | 2033 | A | GSDMA | R427H | G | 1398 | A |
| ETNK2 | V291M | C | 913 | T | FCGBP | R534H | C | 1609 | T | GCFC1 | E18D | C | 244 | A | GSDMB | R273I | C | 949 | A |
| ETS1 | V137I | C | 494 | T | FCGBP | V4787I | C | 14367 | T | GCFC1 | G348S | C | 1232 | T | GSDMB | G304V | C | 1042 | A |
| ETS1 | S228F | G | 768 | A | FCGBP | P4736S | G | 14214 | A | GCFC1 | M1I | C | 193 | T | GSDMC | C115Y | C | 1226 | T |
| ETS2 | E287K | G | 1319 | A | FCGR2A | K243T | A | 766 | C | GCFC1 | R252H | C | 945 | T | GSDMC | T22P | T | 946 | G |
| ETS2 | L446I | C | 1796 | A | FCGR3A | D118E | G | 538 | T | GCFC1 | G802C | C | 2594 | A | GSDMC | E15D | C | 927 | G |

| | | | | | | | | | | | | | | | | | | | |
|---|---|---|---|---|---|---|---|---|---|---|---|---|---|---|---|---|---|---|---|
| ETV1 | K47Q | T | 246 | G | FCGR3A | F255C | A | 948 | C | GCFC1 | R482H | C | 1635 | T | GSDMC | D131Y | C | 1273 | A |
| ETV2 | A72V | C | 654 | T | FCGR3B | V265M | C | 797 | T | GCG | S71F | G | 212 | A | GSDMC | L12W | A | 917 | C |
| ETV3 | V209M | C | 690 | T | FCGRT | A242T | G | 1126 | A | GCG | Y65* | G | 195 | C | GSG1L | R300* | G | 983 | A |
| ETV3 | R475Q | C | 1489 | T | FCHO1 | L70F | C | 433 | T | GCG | Y65* | G | 195 | C | GSG1L | L10P | A | 114 | G |
| ETV5 | T484A | T | 1697 | C | FCHO1 | D313N | G | 1162 | A | GCG | Y5D | A | 13 | C | GSG1L | R322Q | C | 1050 | T |
| ETV6 | P214L | C | 915 | T | FCHO2 | A521T | G | 1677 | A | GCGR | V326I | G | 1253 | A | GSG1L | V139A | A | 501 | G |
| ETV6 | R369W | C | 1379 | T | FCHSD1 | A665T | C | 2044 | T | GCK | A450T | C | 1510 | T | GSG2 | G117W | G | 382 | T |
| ETV6 | R259Q | G | 1050 | A | FCHSD1 | A580T | C | 1789 | T | GCK | H142R | T | 587 | C | GSK3A | V133M | C | 525 | T |
| ETV6 | R399H | G | 1470 | A | FCHSD1 | R117W | G | 400 | A | GCKR | A614V | C | 1904 | T | GSK3A | R243C | G | 855 | A |
| ETV7 | A99V | G | 538 | A | FCHSD2 | S698R | T | 2261 | G | GCKR | F299C | T | 959 | G | GSK3A | A82T | C | 372 | T |
| ETV7 | P18H | G | 295 | T | FCN2 | G308* | G | 936 | T | GCLC | P206Q | G | 1101 | T | GSK3B | R111* | G | 563 | A |
| EVC | A368T | G | 1286 | A | FCRL2 | S59Y | G | 223 | T | GCM1 | H423Y | G | 1479 | A | GSK3B | R367* | G | 1331 | A |
| EVC | R643H | G | 2112 | A | FCRL3 | P284H | G | 1024 | T | GCM2 | R265I | C | 942 | A | GSK3B | A231T | C | 923 | T |
| EVC | R678C | C | 2216 | T | FCRL3 | R293Q | C | 1051 | T | GCM2 | K449N | T | 1495 | G | GSK3B | Y140C | T | 651 | C |
| EVC | K533T | A | 1782 | C | FCRL3 | L299M | G | 1068 | T | GCM2 | R265I | C | 942 | A | GSN | T733M | C | 2267 | T |
| EVC2 | T290M | G | 923 | A | FCRL3 | V283A | A | 1021 | G | GCM2 | R177I | C | 678 | A | GSN | A424T | G | 1339 | A |
| EVC2 | L188F | G | 616 | A | FCRL4 | R413W | G | 1373 | A | GCN1L1 | V750M | C | 2261 | T | GSN | F392L | C | 1245 | A |
| EVC2 | A1177V | G | 3584 | A | FCRL5 | A363T | C | 1245 | T | GCN1L1 | P1094L | G | 3294 | A | GSN | E722G | A | 2234 | G |
| EVC2 | P1256S | G | 3820 | A | FCRL5 | T962A | T | 3042 | C | GCN1L1 | P993S | G | 2990 | A | GSPT1 | K359T | T | 1324 | G |
| EVC2 | L1212M | G | 3688 | T | FCRL5 | T213A | T | 795 | C | GCN1L1 | V1658M | C | 4985 | T | GSR | R233C | G | 697 | A |
| EVC2 | T647I | G | 1994 | A | FCRL5 | L75I | G | 381 | T | GCN1L1 | V732I | C | 2207 | T | GSR | R233H | C | 698 | T |
| EVC2 | R335W | G | 1057 | A | FCRL6 | D366Y | G | 1097 | T | GCN1L1 | A247V | G | 753 | A | GSTA2 | F222L | A | 822 | C |
| EVC2 | A703T | C | 2161 | T | FCRLA | R192G | A | 816 | G | GCN1L1 | A72T | C | 227 | T | GSTA2 | F197L | A | 747 | C |
| EVC2 | L253P | A | 812 | G | FCRLB | A187V | C | 775 | T | GCNT2 | F34L | T | 637 | G | GSTA4 | E33* | C | 245 | A |
| EVC2 | A897D | G | 2744 | T | FDPS | S9F | C | 141 | T | GCNT3 | K281N | G | 1291 | C | GSTA5 | I219M | A | 816 | C |
| EVC2 | D1202Y | C | 3658 | A | FDPS | K323N | A | 1084 | C | GCNT3 | R264H | G | 1239 | A | GSTA5 | I188L | T | 721 | G |
| EVC2 | E473* | C | 1471 | A | FDX1 | M137I | G | 662 | A | GCNT3 | D304Y | G | 1358 | T | GSTA5 | Y95H | A | 442 | G |
| EVI5 | E278* | C | 842 | A | FDX1 | E95* | G | 534 | T | GCNT3 | K311N | G | 1381 | T | GSTCD | R600* | C | 2018 | T |
| EVI5L | R765H | G | 2366 | A | FDXR | L57P | A | 203 | G | GCNT4 | S453* | G | 2220 | T | GSTCD | R257K | G | 990 | A |
| EVL | S360L | C | 1166 | T | FECH | W307* | C | 955 | T | GCNT4 | V103A | A | 1170 | G | GSTCD | R480C | C | 1658 | T |
| EVPL | V200M | C | 826 | T | FECH | Y282H | A | 878 | G | GCNT7 | G244R | C | 1802 | T | GSTM5 | R101H | G | 345 | A |
| EVPL | A173V | G | 746 | A | FECH | V45I | C | 167 | T | GCNT7 | E238K | C | 1784 | T | GSTO2 | L93P | T | 907 | C |
| EVPL | A1664T | C | 5218 | T | FEM1B | R246Q | G | 1352 | A | GCNT7 | E128K | C | 1454 | T | GSTO2 | E85* | G | 882 | T |
| EVPL | W424L | C | 1499 | A | FEM1B | L36I | C | 721 | A | GDAP1 | L28M | C | 161 | A | GSTP1 | L63P | T | 437 | C |
| EVPL | R1757C | G | 5497 | A | FEM1C | E163D | C | 1051 | A | GDAP2 | R174H | C | 771 | T | GSTZ1 | Q207* | C | 901 | T |
| EVPLL | R60W | C | 433 | T | FEM1C | F554S | A | 2223 | G | GDAP2 | R187C | G | 809 | A | GSX2 | A170T | G | 822 | A |
| EVX2 | A304V | G | 1048 | A | FEM1C | L87V | A | 821 | C | GDE1 | L23V | G | 231 | C | GTDC1 | D297E | A | 1044 | T |
| EVX2 | G103D | C | 445 | T | FER | K29N | G | 471 | T | GDF1 | A305V | G | 2308 | A | GTF2A1 | Q78H | C | 666 | A |
| EWSR1 | R298W | C | 913 | T | FER | E315* | G | 1327 | T | GDF10 | A190V | G | 835 | A | GTF2A1 | A100V | G | 731 | A |

| | | | | | | | | | | | | | | | | | | | |
|---|---|---|---|---|---|---|---|---|---|---|---|---|---|---|---|---|---|---|---|
| EWSR1 | G613D | G | 1859 | A | FER | D479G | A | 1820 | G | GDF10 | A47T | C | 405 | T | GTF2E1 | R432H | G | 1388 | A |
| EXD1 | Q336H | C | 1199 | A | FER | K8N | G | 408 | T | GDF10 | R277H | C | 1096 | T | GTF2E1 | K272R | A | 908 | G |
| EXD1 | A90V | G | 460 | A | FER | E390D | A | 1554 | C | GDF10 | V148G | A | 709 | C | GTF2E1 | H234R | A | 794 | G |
| EXD1 | K410T | T | 1420 | G | FER1L6 | V1382L | G | 4158 | T | GDF11 | T149M | C | 483 | T | GTF2E1 | D5E | T | 108 | G |
| EXD2 | H442R | A | 1453 | G | FER1L6 | E1478K | G | 4446 | A | GDF15 | L110H | T | 361 | A | GTF2F1 | R460H | C | 1664 | T |
| EXD2 | T549I | C | 1774 | T | FER1L6 | V1065M | G | 3207 | A | GDF2 | V193M | C | 737 | T | GTF2F1 | E326K | C | 1261 | T |
| EXD2 | - | G | 0 | T | FER1L6 | N598S | A | 1807 | G | GDF3 | R84C | G | 315 | A | GTF2F1 | A357T | C | 1354 | T |
| EXD3 | P436S | G | 1502 | A | FER1L6 | E677* | G | 2043 | T | GDF3 | R266H | C | 862 | T | GTF2F1 | P332L | G | 1280 | A |
| EXO1 | R557H | G | 2263 | A | FER1L6 | G810D | G | 2443 | A | GDF3 | R250G | T | 813 | C | GTF2H2D | P124S | C | 565 | T |
| EXO1 | K451T | A | 1945 | C | FER1L6 | R10I | G | 43 | T | GDF3 | R84C | G | 315 | A | GTF2H4 | C16Y | G | 270 | A |
| EXOC1 | R580H | G | 2087 | A | FER1L6 | D1435Y | G | 4317 | T | GDF5OS | A166S | G | 938 | T | GTF2IRD1 | A53T | G | 370 | A |
| EXOC1 | R562Q | G | 2033 | A | FER1L6 | F1707L | C | 5135 | A | GDF6 | P21S | G | 161 | A | GTF2IRD1 | R208* | C | 835 | T |
| EXOC1 | A692V | C | 2423 | T | FERD3L | E75* | C | 282 | A | GDF6 | Y381* | A | 1243 | C | GTF2IRD1 | K303N | G | 1122 | T |
| EXOC1 | A328T | G | 1330 | A | FERMT1 | E666A | T | 2786 | G | GDF6 | P90L | G | 369 | A | GTF2IRD1 | - | G | 0 | A |
| EXOC2 | E214K | C | 776 | T | FERMT1 | A345V | G | 1823 | A | GDF7 | R221H | G | 1238 | A | GTF2IRD1 | P630L | C | 2102 | T |
| EXOC2 | I139F | T | 551 | A | FERMT1 | R98C | G | 1081 | A | GDF7 | R351H | G | 1628 | A | GTF2IRD2 | V599G | A | 1986 | C |
| EXOC2 | R518H | C | 1689 | T | FERMT2 | R666C | G | 2182 | A | GDF7 | A396D | C | 1763 | A | GTF3C1 | R799C | G | 2435 | A |
| EXOC2 | L277F | G | 965 | A | FERMT2 | R525H | C | 1760 | T | GDF9 | W278* | C | 1701 | T | GTF3C1 | A1922V | G | 5805 | A |
| EXOC3 | R406Q | G | 1244 | A | FERMT2 | G415D | C | 1430 | T | GDF9 | R122L | C | 1232 | A | GTF3C1 | N1665D | T | 5033 | C |
| EXOC3 | E124G | A | 398 | G | FERMT3 | A144V | C | 580 | T | GDF9 | L430V | A | 2155 | C | GTF3C1 | G733W | C | 2237 | A |
| EXOC3L | - | C | 0 | T | FERMT3 | R629Q | G | 2035 | A | GDF9 | T95A | T | 1150 | C | GTF3C2 | A490T | C | 1898 | T |
| EXOC3L | G39V | C | 357 | A | FES | L285F | C | 995 | T | GDNF | G127C | C | 548 | A | GTF3C3 | A168V | G | 593 | A |
| EXOC4 | Q628R | A | 1912 | G | FES | R154C | C | 602 | T | GDNF | Y173H | A | 686 | G | GTF3C4 | S611L | C | 2396 | T |
| EXOC4 | Y789D | T | 2394 | G | FES | A22V | C | 207 | T | GDPD1 | Y286C | A | 994 | G | GTF3C4 | D804N | G | 2974 | A |
| EXOC5 | F222C | A | 916 | C | FETUB | N125S | A | 635 | G | GDPD2 | E331G | A | 1243 | G | GTF3C4 | W169* | G | 1071 | A |
| EXOC5 | E16* | C | 297 | A | FEZ1 | R16* | G | 238 | A | GDPD2 | R395W | C | 1434 | T | GTF3C4 | R326* | C | 1540 | T |
| EXOC6 | L674I | C | 2034 | A | FEZ1 | E142D | T | 618 | G | GDPD3 | D309N | C | 1303 | T | GTF3C4 | L557V | T | 2233 | G |
| EXOC6 | E15K | G | 57 | A | FEZ2 | M358L | T | 1119 | A | GDPD4 | T231A | T | 942 | C | GTF3C5 | E441G | A | 1330 | G |
| EXOC6 | L136I | C | 420 | A | FEZ2 | E123D | T | 416 | A | GDPD4 | G202E | C | 856 | T | GTF3C6 | K94N | A | 492 | C |
| EXOC6 | S284P | T | 864 | C | FEZF1 | H474R | T | 1489 | C | GDPD5 | M499V | T | 1984 | C | GTPBP1 | R649* | C | 2178 | T |
| EXOC6 | N638D | A | 1926 | G | FEZF2 | A44V | G | 350 | A | GDPD5 | T55M | G | 653 | A | GTPBP10 | - | G | 0 | T |
| EXOC6 | N769D | A | 2319 | G | FEZF2 | E82G | T | 464 | C | GDPD5 | P485H | G | 1943 | T | GTPBP3 | D255N | G | 828 | A |
| EXOC6B | R785Q | C | 2485 | T | FEZF2 | L79I | G | 454 | T | GEMIN4 | L689H | A | 2185 | T | GTPBP4 | P365T | C | 1175 | A |
| EXOC6B | P456H | G | 1498 | T | FEZF2 | R315C | G | 1162 | A | GEMIN4 | T392M | G | 1294 | A | GTPBP4 | K490Q | A | 1550 | C |
| EXOC6B | R785W | G | 2484 | A | FEZF2 | E248D | T | 963 | G | GEMIN4 | R917K | C | 2869 | T | GTPBP5 | P233H | C | 716 | A |
| EXOC7 | V218I | C | 706 | T | FFAR1 | R258Q | G | 773 | A | GEMIN4 | V840M | C | 2637 | T | GTPBP5 | A252T | G | 772 | A |
| EXOC7 | Q686H | C | 2112 | A | FFAR1 | A229P | G | 685 | C | GEMIN4 | E11* | C | 150 | A | GTPBP5 | C206Y | G | 635 | A |
| EXOC7 | G497S | C | 1543 | T | FFAR2 | H247P | A | 740 | C | GEMIN4 | E800K | C | 2517 | T | GTSE1 | R130Q | G | 601 | A |
| EXOC7 | S705N | C | 2168 | T | FGA | R181* | G | 620 | A | GEMIN5 | G276C | C | 902 | A | GUCA1B | R63* | G | 283 | A |

| | | | | | | | | | | | | | | | | | | | |
|---|---|---|---|---|---|---|---|---|---|---|---|---|---|---|---|---|---|---|---|
| EXOC7 | P640L | G | 1973 | A | FGA | E702K | C | 2183 | T | GEMIN5 | I201V | T | 677 | C | GUCA1C | G33S | C | 230 | T |
| EXOC8 | T425M | G | 1361 | A | FGA | G235R | C | 782 | T | GEMIN5 | E1450K | C | 4424 | T | GUCA1C | L46M | G | 269 | T |
| EXOC8 | K242E | T | 811 | C | FGA | A403T | C | 1286 | T | GEMIN5 | R719W | G | 2231 | A | GUCA2A | P75S | G | 229 | A |
| EXOC8 | R388* | G | 1249 | A | FGB | R196C | C | 649 | T | GEMIN5 | D195G | T | 660 | C | GUCA2A | A108V | G | 329 | A |
| EXOSC1 | R100Q | C | 331 | T | FGB | S6Y | C | 80 | A | GEMIN5 | F387L | G | 1237 | T | GUCY1A2 | V653M | C | 2347 | T |
| EXOSC10 | E302K | C | 954 | T | FGB | Y368C | A | 1166 | G | GEMIN7 | A61T | G | 332 | A | GUCY1A2 | P707L | G | 2510 | A |
| EXOSC10 | K806N | C | 2468 | A | FGD2 | E498K | G | 1663 | A | GEN1 | L81P | T | 315 | C | GUCY1A3 | R95I | G | 725 | T |
| EXOSC4 | G30C | G | 198 | T | FGD2 | S139N | G | 587 | A | GEN1 | I9M | T | 100 | G | GUCY1A3 | R574* | C | 2161 | T |
| EXOSC5 | S217L | G | 673 | A | FGD2 | R478H | G | 1604 | A | GET4 | R186H | G | 651 | A | GUCY1A3 | G183S | G | 988 | A |
| EXOSC5 | E77K | C | 252 | T | FGD3 | H670Y | C | 2504 | T | GFAP | R258C | G | 832 | A | GUCY1A3 | K53N | G | 600 | T |
| EXOSC6 | R265P | C | 824 | G | FGD3 | E169K | G | 1001 | A | GFAP | R30H | C | 149 | T | GUCY1B2 | M72V | T | 647 | C |
| EXOSC7 | P69L | C | 254 | T | FGD3 | A324V | C | 1467 | T | GFAP | P229S | G | 745 | A | GUCY1B2 | E160* | C | 911 | A |
| EXOSC9 | A295T | G | 988 | A | FGD4 | R181M | G | 966 | T | GFI1 | G290D | C | 1188 | T | GUCY1B3 | I543M | A | 1749 | G |
| EXOSC9 | - | G | 0 | A | FGD4 | I116V | A | 770 | G | GFI1 | T417M | G | 1569 | A | GUCY1B3 | R574* | C | 1840 | T |
| EXOSC9 | S113L | C | 443 | T | FGD4 | K5R | A | 438 | G | GFI1 | V305M | C | 1232 | T | GUCY1B3 | E588A | A | 1883 | C |
| EXOSC9 | D245Y | G | 838 | T | FGD4 | S390Y | C | 1593 | A | GFI1B | A39V | C | 941 | T | GUCY2C | A1031S | C | 3228 | A |
| EXPH5 | P198Q | G | 704 | T | FGD5 | K12N | G | 146 | T | GFI1B | F252L | C | 1581 | A | GUCY2C | D517V | T | 1687 | A |
| EXPH5 | A91T | C | 382 | T | FGD5 | G1252S | G | 3864 | A | GFM1 | A265G | C | 832 | G | GUCY2C | N1018S | T | 3190 | C |
| EXPH5 | - | A | 0 | G | FGD5 | A505V | C | 1624 | T | GFM1 | S279L | C | 874 | T | GUCY2C | R319* | G | 1092 | A |
| EXPH5 | R1303Q | C | 4019 | T | FGD5 | A1266T | G | 3906 | A | GFM1 | A264T | G | 828 | A | GUCY2D | R964C | C | 2964 | T |
| EXPH5 | K498T | T | 1604 | G | FGD5 | V1025I | G | 3183 | A | GFM1 | R303Q | G | 946 | A | GUCY2D | G180S | G | 612 | A |
| EXT1 | R595H | C | 2437 | T | FGD5 | N817Y | A | 2559 | T | GFOD1 | S254L | G | 1426 | A | GUCY2D | D903G | A | 2782 | G |
| EXT1 | A331V | G | 1645 | A | FGD6 | L79M | G | 459 | T | GFOD1 | V176M | C | 1191 | T | GUCY2D | R424W | C | 1344 | T |
| EXT1 | - | C | 0 | A | FGD6 | F956S | A | 3091 | G | GFOD2 | S138L | G | 759 | A | GUCY2D | R574C | C | 1794 | T |
| EXT2 | - | T | 0 | C | FGD6 | E1279* | C | 4059 | A | GFPT1 | R111H | C | 511 | T | GUCY2F | F576L | A | 2018 | G |
| EXT2 | S19L | C | 497 | T | FGD6 | I52T | A | 379 | G | GFPT1 | A316T | C | 1125 | T | GUCY2F | S420C | T | 1550 | A |
| EXT2 | R197* | C | 1030 | T | FGD6 | K33T | T | 322 | G | GFPT1 | R633Q | C | 2077 | T | GUF1 | T336A | A | 1200 | G |
| EXTL1 | R191W | C | 1438 | T | FGF1 | G135S | C | 483 | T | GFRA1 | G55V | C | 532 | A | GUF1 | A323V | C | 1162 | T |
| EXTL1 | F86V | T | 1123 | G | FGF10 | M204V | T | 725 | C | GFRA2 | R178H | C | 1208 | T | GUF1 | K442T | A | 1519 | C |
| EXTL1 | F397L | T | 2058 | A | FGF10 | S128N | C | 498 | T | GFRA2 | R218H | C | 1328 | T | GUF1 | E451* | G | 1545 | T |
| EXTL2 | V98A | A | 1730 | G | FGF11 | E151D | G | 704 | T | GFRA3 | A236T | C | 953 | T | GUF1 | R585W | C | 1947 | T |
| EXTL2 | L300R | A | 2336 | C | FGF11 | K189T | A | 817 | C | GFRA3 | R3H | C | 255 | T | GUF1 | E653Q | G | 2151 | C |
| EXTL3 | L260F | G | 1395 | C | FGF12 | Y134H | A | 1226 | G | GFRA4 | A214V | G | 641 | A | GUSB | R261H | C | 913 | T |
| EXTL3 | S201T | G | 1217 | C | FGF12 | A2D | G | 831 | T | GGA2 | A63V | G | 271 | A | GUSB | V75M | C | 354 | T |
| EXTL3 | G888S | G | 3277 | A | FGF12 | R58H | C | 999 | T | GGA2 | R416G | T | 1329 | C | GUSB | R625K | C | 2005 | T |
| EYA1 | Q384R | T | 1791 | C | FGF13 | G124D | C | 371 | T | GGA3 | G480D | C | 1448 | T | GUSB | G512R | C | 1665 | T |
| EYA1 | S83T | A | 887 | T | FGF13 | Q39H | T | 117 | A | GGA3 | R22H | C | 74 | T | GXYLT1 | S198L | G | 818 | A |
| EYA1 | F82V | A | 884 | C | FGF13 | D110Y | C | 328 | A | GGCT | S136F | G | 542 | A | GXYLT2 | V330G | T | 1150 | G |
| EYA1 | L374* | A | 1761 | C | FGF14 | R27M | C | 176 | A | GGCX | A22T | C | 145 | T | GYG2 | S85L | C | 536 | T |

| | | | | | | | | | | | | | | | | | | | |
|---|---|---|---|---|---|---|---|---|---|---|---|---|---|---|---|---|---|---|---|
| EYA1 | S169R | T | 1145 | G | FGF16 | A9T | G | 25 | A | GGCX | - | A | 0 | G | GYG2 | D46Y | G | 418 | T |
| EYA2 | T292M | C | 1272 | T | FGF16 | Y53H | T | 157 | C | GGCX | T631A | T | 1972 | C | GYG2 | E428K | G | 1564 | A |
| EYA2 | R248L | G | 1140 | T | FGF17 | R96Q | G | 790 | A | GGH | G12D | C | 318 | T | GYLTL1B | R267W | C | 898 | T |
| EYA2 | R255Q | G | 1161 | A | FGF18 | V37M | G | 646 | A | GGH | M160T | A | 762 | G | GYLTL1B | R267W | C | 898 | T |
| EYA2 | G173S | G | 914 | A | FGF19 | R99W | G | 757 | A | GGN | S327L | G | 1115 | A | GYLTL1B | S109N | G | 425 | A |
| EYA3 | - | T | 0 | C | FGF19 | R43H | C | 590 | T | GGN | P626S | G | 2011 | A | GYLTL1B | R699H | G | 2195 | A |
| EYA3 | S86L | G | 498 | A | FGF2 | R249Q | G | 814 | A | GGN | R334Q | C | 1136 | T | GYPA | S139Y | G | 532 | T |
| EYA3 | R418C | G | 1493 | A | FGF2 | R262Q | G | 853 | A | GGPS1 | R117C | C | 521 | T | GYS1 | R498C | G | 1689 | A |
| EYA3 | K285N | C | 1096 | A | FGF23 | R179Q | C | 682 | T | GGT1 | A64T | G | 677 | A | GYS2 | N228S | T | 938 | C |
| EYA4 | A635T | G | 2361 | A | FGF23 | R160Q | C | 625 | T | GGT5 | R362C | G | 1418 | A | GYS2 | A252T | C | 1009 | T |
| EYA4 | T295I | C | 1342 | T | FGF3 | R44W | G | 621 | A | GGT5 | R419Q | C | 1590 | T | GYS2 | K702N | C | 2361 | A |
| EYA4 | K400T | A | 1657 | C | FGF3 | R181C | G | 1032 | A | GGT6 | S168Y | G | 563 | T | GYS2 | F312L | G | 1191 | T |
| EYS | S1628F | G | 5410 | A | FGF4 | A168T | C | 821 | T | GGT7 | R593H | C | 1823 | T | GYS2 | E61D | C | 438 | A |
| EYS | R794Q | C | 2908 | T | FGF4 | R134Q | C | 720 | T | GGT7 | W222* | C | 711 | T | GZF1 | S265N | G | 871 | A |
| EYS | T3002A | T | 9531 | C | FGF5 | S127T | T | 605 | A | GGTLC1 | F80L | A | 371 | G | GZF1 | D170N | G | 585 | A |
| EYS | I454S | A | 1888 | C | FGF5 | K236N | G | 934 | T | GH2 | A184V | G | 613 | A | GZMA | R202* | C | 639 | T |
| EYS | T695A | T | 2610 | C | FGF7 | R78M | G | 762 | T | GH2 | T93M | G | 340 | A | GZMB | P160S | G | 483 | A |
| EYS | V2252A | A | 7282 | G | FGF7 | L14F | C | 569 | T | GH2 | Q22K | G | 126 | T | GZMH | L8F | C | 69 | A |
| EYS | R1463G | T | 4914 | C | FGF7 | E132* | G | 923 | T | GHDC | A189T | C | 583 | T | GZMK | A125V | C | 444 | T |
| EYS | N1906S | T | 6244 | C | FGF8 | S57F | G | 229 | A | GHDC | R85G | T | 271 | C | GZMK | Q95* | C | 353 | T |
| EYS | D1286G | T | 4384 | C | FGF8 | R206W | G | 675 | A | GHRHR | - | G | 0 | T | GZMK | D196G | A | 657 | G |
| EYS | R1242I | C | 4252 | A | FGF9 | V7A | T | 550 | C | GHSR | P41L | G | 165 | A | GZMM | V5G | T | 59 | G |
| EYS | N1163H | T | 4014 | G | FGFR1 | R675Q | C | 2753 | T | GIGYF1 | R202W | G | 1814 | A | GZMM | - | G | 0 | T |
| EYS | N968S | T | 3430 | C | FGFR1OP2 | E52* | G | 496 | T | GIGYF1 | S212G | T | 1844 | C | H1F0 | S66L | C | 635 | T |
| EYS | F182C | A | 1072 | C | FGFR1OP2 | K199N | G | 939 | T | GIGYF1 | R128C | G | 1592 | A | H1FNT | R155Q | G | 776 | A |
| EZH2 | C300R | A | 1020 | G | FGFR1OP2 | K13T | A | 380 | C | GIGYF2 | D55Y | G | 360 | T | H1FNT | A192T | G | 886 | A |
| EZH2 | R358C | G | 1194 | A | FGFR3 | R850* | C | 2548 | T | GIGYF2 | Q684R | A | 2248 | G | H1FOO | K323N | G | 974 | T |
| EZH2 | E725K | C | 2295 | T | FGFR4 | A590V | C | 1769 | T | GIGYF2 | E1138V | A | 3610 | T | H1FX | S31P | A | 463 | G |
| EZH2 | N428T | T | 1405 | G | FGFR4 | G214R | G | 640 | C | GIGYF2 | M768I | G | 2501 | A | H2AFX | A71T | C | 266 | T |
| EZH2 | E169D | T | 629 | G | FGFR4 | R892L | G | 2675 | T | GIMAP1 | A234E | C | 841 | A | H2AFX | V125A | A | 429 | G |
| EZR | P480L | G | 1573 | A | FGFR4 | Q74* | C | 220 | T | GIMAP1 | A218T | G | 792 | A | H2AFY | A58V | G | 767 | A |
| F10 | E304K | G | 948 | A | FGFRL1 | V492A | T | 2055 | C | GIMAP2 | L206F | G | 712 | C | H2AFY2 | P107S | C | 583 | T |
| F10 | E54K | G | 198 | A | FGFRL1 | S224L | C | 1251 | T | GIMAP2 | S50L | C | 243 | T | H2AFY2 | A68T | G | 466 | A |
| F10 | Q144H | G | 470 | T | FGFRL1 | S486T | T | 2036 | A | GIMAP4 | L65I | C | 276 | A | H2BFM | P229L | C | 686 | T |
| F11 | F210C | T | 981 | G | FGFRL1 | G300S | G | 1478 | A | GIMAP4 | A243E | C | 811 | A | H2BFM | V96I | G | 286 | A |
| F11R | R9S | C | 132 | A | FGG | Y314C | T | 967 | C | GIMAP6 | R152Q | C | 879 | T | H2BFWT | R3H | C | 35 | T |
| F12 | G65D | C | 243 | T | FGG | R8W | G | 48 | A | GIMAP6 | K347N | C | 1465 | A | H2BFWT | E49* | C | 172 | A |
| F13A1 | D575N | C | 1989 | T | FGG | Y382H | A | 1170 | G | GIMAP7 | P90T | C | 425 | A | H3F3B | S87I | C | 393 | A |
| F13A1 | R261H | C | 1048 | T | FGGY | R109Q | G | 510 | A | GIMAP8 | A609D | C | 2400 | A | H6PD | R455Q | G | 1637 | A |

| | | | | | | | | | | | | | | | | | | | |
|---|---|---|---|---|---|---|---|---|---|---|---|---|---|---|---|---|---|---|---|
| F13A1 | G313D | C | 1204 | T | FGGY | R118* | C | 536 | T | GIMAP8 | S299A | T | 1469 | G | HABP2 | R480C | C | 1534 | T |
| F13A1 | E659* | C | 2241 | A | FGL2 | F356L | A | 1099 | G | GIN1 | Q226H | T | 773 | G | HACE1 | T624P | T | 2147 | G |
| F13A1 | P656H | G | 2233 | T | FGL2 | F437V | A | 1342 | C | GINS1 | E158* | G | 566 | T | HACE1 | R332Q | C | 1272 | T |
| F13A1 | G449D | C | 1612 | T | FGL2 | E170* | C | 541 | A | GINS1 | R83H | G | 342 | A | HACL1 | S157N | C | 838 | T |
| F13A1 | F214C | A | 907 | C | FGR | R267H | C | 1089 | T | GINS2 | E112K | C | 435 | T | HACL1 | P248S | G | 1110 | A |
| F13A1 | D197N | C | 855 | T | FGR | T297I | G | 1179 | A | GINS3 | F155L | C | 673 | A | HADH | S78L | C | 233 | T |
| F13B | K207N | T | 665 | G | FH | D179N | C | 574 | T | GINS4 | L65F | C | 403 | T | HADHA | C4Y | C | 141 | T |
| F13B | E509K | C | 1569 | T | FHAD1 | E214G | A | 779 | G | GIPC1 | A251T | C | 783 | T | HADHA | D442N | C | 1454 | T |
| F13B | E337D | C | 1055 | A | FHAD1 | - | A | 0 | C | GIPC1 | S68N | C | 235 | T | HADHA | F327C | A | 1110 | C |
| F2 | T510M | C | 1585 | T | FHAD1 | F641C | T | 2060 | G | GIPC2 | E115K | G | 536 | A | HADHB | L121I | C | 465 | A |
| F2 | V515A | T | 1600 | C | FHDC1 | D817N | G | 2637 | A | GIPC3 | R225C | C | 700 | T | HADHB | G427* | G | 1383 | T |
| F2R | L423M | C | 1532 | A | FHDC1 | L754V | T | 2448 | G | GIPR | L431M | C | 1390 | A | HADHB | L261I | C | 885 | A |
| F2RL2 | F338V | A | 1216 | C | FHDC1 | R1098C | C | 3480 | T | GIPR | L134S | T | 500 | C | HAGH | A87V | G | 634 | A |
| F2RL3 | R219W | C | 830 | T | FHOD1 | V321I | C | 1209 | T | GIT1 | E404* | C | 1381 | A | HAL | T5M | G | 311 | A |
| F2RL3 | A236T | G | 881 | A | FHOD1 | F156C | A | 715 | C | GIT1 | G200V | C | 770 | A | HAL | E421K | C | 1558 | T |
| F2RL3 | R352W | C | 1229 | T | FHOD3 | F134V | T | 497 | G | GIT1 | D643E | G | 2100 | C | HAL | F496L | G | 1785 | T |
| F2RL3 | A272V | C | 990 | T | FHOD3 | R1353C | C | 4154 | T | GIT1 | R564W | G | 1861 | A | HAL | R356Q | C | 1364 | T |
| F5 | V1623A | A | 5070 | G | FHOD3 | A68T | G | 299 | A | GIT2 | L307P | A | 1085 | G | HAL | M273R | A | 1115 | C |
| F5 | R652H | C | 2157 | T | FHOD3 | S366L | C | 1194 | T | GIT2 | G720V | C | 2324 | A | HAMP | R59* | C | 246 | T |
| F5 | R1731W | G | 5393 | A | FHOD3 | R1353C | C | 4154 | T | GIT2 | R613G | T | 2002 | C | HAND1 | G90S | C | 524 | T |
| F5 | D497N | C | 1691 | T | FHOD3 | R489G | A | 1562 | G | GIT2 | T405A | T | 1378 | C | HAND1 | A139T | C | 671 | T |
| F5 | G1232S | C | 3896 | T | FHOD3 | E1128K | G | 3479 | A | GIT2 | Q196R | T | 752 | C | HAND2 | T204A | T | 1550 | C |
| F5 | K2093N | C | 6481 | A | FIBIN | Y43D | T | 573 | G | GJA1 | M125I | G | 532 | A | HAO1 | R84H | C | 303 | T |
| F5 | R1626P | C | 5079 | G | FIBP | R360H | C | 1200 | T | GJA1 | E227* | G | 836 | T | HAO1 | D331Y | C | 1043 | A |
| F5 | L1122F | G | 3566 | A | FIBP | Y173C | T | 639 | C | GJA10 | Y526D | T | 1576 | G | HAO1 | R84H | C | 303 | T |
| F5 | A355D | G | 1266 | T | FIG4 | Y510H | T | 1652 | C | GJA3 | A276T | C | 1003 | T | HAO1 | Y50H | A | 200 | G |
| F7 | P189H | C | 601 | A | FIG4 | R244H | G | 855 | A | GJA3 | P126S | G | 553 | A | HAO2 | R48W | C | 425 | T |
| F7 | T143M | C | 463 | T | FIG4 | G281D | G | 966 | A | GJA4 | L127M | C | 467 | A | HAO2 | R59H | G | 459 | A |
| F8 | R2166* | G | 6697 | A | FIG4 | F101S | T | 426 | C | GJA5 | T56M | G | 329 | A | HAP1 | P164L | G | 491 | A |
| F8 | H1938N | G | 6013 | T | FIG4 | R384I | G | 1275 | T | GJA5 | F84L | G | 414 | T | HAP1 | Q40* | G | 118 | A |
| F8 | A1622T | C | 5065 | T | FIGF | R351Q | C | 1482 | T | GJA8 | E249D | G | 810 | T | HAP1 | - | C | 0 | A |
| F8 | R2166* | G | 6697 | A | FIGN | S89R | T | 580 | G | GJA9 | D416N | C | 1527 | T | HAP1 | V192I | C | 574 | T |
| F8 | A1311T | C | 4132 | T | FIGN | A557T | C | 1984 | T | GJA9 | M368V | T | 1383 | C | HAPLN2 | C290G | T | 1275 | G |
| F8 | V1984I | C | 6151 | T | FIGN | L206F | G | 931 | A | GJA9 | E361* | C | 1362 | A | HAPLN3 | R110C | G | 543 | A |
| F8 | W2089L | C | 6467 | A | FIGN | R684C | G | 2365 | A | GJB1 | W44C | G | 226 | T | HAPLN3 | R248S | G | 957 | T |
| F8 | T2141A | T | 6622 | C | FIGN | P305L | G | 1229 | A | GJB3 | G125R | G | 988 | A | HAPLN4 | G231S | C | 755 | T |
| F8 | A1551V | G | 4853 | A | FIGN | R641W | G | 2236 | A | GJB3 | A114T | G | 955 | A | HAPLN4 | E260* | C | 842 | A |
| F8 | S921R | T | 2962 | G | FIGN | S619L | G | 2171 | A | GJB4 | A96T | G | 656 | A | HAPLN4 | A308T | C | 986 | T |
| F9 | P324S | C | 999 | T | FIGN | P270L | G | 1124 | A | GJB4 | F68L | C | 574 | A | HAPLN4 | R60C | G | 242 | A |

| | | | | | | | | | | | | | | | | | | | |
|---|---|---|---|---|---|---|---|---|---|---|---|---|---|---|---|---|---|---|---|
| F9 | V331I | G | 1020 | A | FIGNL1 | S259Y | G | 1106 | T | GJB5 | W129* | G | 559 | A | HARS | R375H | C | 1844 | T |
| F9 | - | G | 0 | T | FIGNL1 | V651M | C | 2281 | T | GJB5 | R124W | C | 543 | T | HARS | R86C | G | 976 | A |
| FAAH2 | R232Q | G | 815 | A | FIGNL1 | R181P | C | 872 | G | GJB7 | T177A | T | 768 | C | HARS2 | R215Q | G | 867 | A |
| FAAH2 | V70I | G | 328 | A | FILIP1 | T1136M | G | 3738 | A | GJC1 | K228N | C | 958 | A | HARS2 | R138H | G | 636 | A |
| FABP1 | E62K | C | 283 | T | FILIP1 | L589S | A | 2097 | G | GJC1 | F197V | A | 863 | C | HAS1 | A209V | G | 661 | A |
| FABP12 | S56R | T | 229 | G | FILIP1 | E436D | C | 1639 | A | GJC1 | P272L | G | 1089 | A | HAS1 | A542V | G | 1660 | A |
| FABP3 | Q96P | T | 349 | G | FILIP1 | V241A | A | 1053 | G | GJC2 | R101C | C | 476 | T | HAS1 | R427H | C | 1315 | T |
| FABP4 | F17V | A | 120 | C | FILIP1 | R224H | C | 1002 | T | GJC2 | R240* | C | 893 | T | HAS1 | A510V | G | 1564 | A |
| FABP7 | E102* | G | 340 | T | FILIP1L | L894Q | A | 3152 | T | GJC3 | V81A | A | 242 | G | HAS2 | R320W | G | 1496 | A |
| FADS1 | K139T | T | 478 | G | FILIP1L | L722F | T | 2637 | G | GJD2 | R246W | G | 736 | A | HAS2 | T110A | T | 866 | C |
| FADS2 | K251N | G | 903 | T | FILIP1L | E137D | C | 882 | A | GJD2 | R278C | G | 832 | A | HAS2 | K536T | T | 2145 | G |
| FADS2 | R216H | G | 797 | A | FIP1L1 | E256* | G | 766 | T | GJD4 | A40V | C | 277 | T | HAS2 | I534N | A | 2139 | T |
| FADS2 | F85L | C | 405 | A | FIP1L1 | D831N | G | 2491 | A | GJD4 | A340T | G | 1176 | A | HAS2 | E379K | C | 1673 | T |
| FADS6 | H291R | T | 879 | C | FIS1 | H86Y | G | 481 | A | GK | L174V | C | 699 | G | HAS2 | K149N | C | 985 | A |
| FADS6 | L120I | G | 365 | T | FITM1 | G84C | G | 539 | T | GK | G296V | G | 1066 | T | HAS3 | R152C | C | 610 | T |
| FAF1 | - | A | 0 | G | FKBP10 | E47K | G | 243 | A | GK | R505C | C | 1692 | T | HAS3 | W350C | G | 1206 | T |
| FAF1 | R554W | G | 2112 | A | FKBP11 | K184R | T | 670 | C | GK2 | R24H | C | 164 | T | HAS3 | P82S | C | 400 | T |
| FAF1 | G293* | C | 1329 | A | FKBP14 | I81T | A | 418 | G | GK2 | L61I | G | 274 | T | HAUS1 | L185I | C | 633 | A |
| FAF2 | F390L | C | 1223 | A | FKBP15 | R1185H | C | 3554 | T | GK5 | I138L | T | 564 | G | HAUS3 | G354R | C | 1290 | T |
| FAF2 | T425M | C | 1327 | T | FKBP15 | P1140L | G | 3419 | A | GK5 | C390R | A | 1320 | G | HAUS3 | K85N | T | 485 | G |
| FAF2 | D389N | G | 1218 | A | FKBP15 | C287Y | C | 860 | T | GK5 | S364R | T | 1242 | G | HAUS6 | K359N | T | 1545 | G |
| FAH | A147V | C | 595 | T | FKBP1C | R19C | C | 166 | T | GK5 | L53I | G | 309 | T | HAUS7 | E60K | C | 222 | T |
| FAHD1 | L148F | C | 705 | T | FKBP1C | R19C | C | 166 | T | GKN1 | R157S | C | 532 | A | HAVCR1 | T198P | T | 1125 | G |
| FAHD2A | K294N | G | 1164 | T | FKBP4 | D63N | G | 374 | A | GKN1 | K14R | A | 104 | G | HAVCR2 | L192F | T | 781 | A |
| FAHD2B | K294N | C | 1153 | A | FKBP4 | T143M | C | 615 | T | GKN1 | K118N | G | 417 | T | HAX1 | S66G | A | 274 | G |
| FAIM2 | R281H | C | 987 | T | FKBP4 | R373W | C | 1304 | T | GKN2 | T41I | G | 231 | A | HBE1 | V127A | A | 725 | G |
| FAM102A | - | C | 0 | T | FKBP4 | V273A | T | 1005 | C | GKN2 | S125Y | G | 483 | T | HBEGF | G190* | C | 871 | A |
| FAM102A | V354M | C | 1436 | T | FKBP6 | L200F | C | 667 | T | GLA | D25G | T | 96 | C | HBEGF | - | T | 0 | C |
| FAM102B | T114S | A | 680 | T | FKBP8 | S282L | G | 958 | A | GLB1 | - | C | 0 | A | HBG2 | A75V | G | 307 | A |
| FAM102B | - | G | 0 | T | FKBP9 | P367H | C | 1269 | A | GLB1 | W277R | A | 974 | T | HBM | A133V | C | 418 | T |
| FAM105B | P254Q | C | 839 | A | FKBP9 | S275R | A | 992 | C | GLB1 | D441N | C | 1466 | T | HBP1 | A115T | G | 425 | A |
| FAM107A | P57L | G | 786 | A | FKBP9 | E507* | G | 1688 | T | GLB1L | T326N | G | 1291 | T | HBQ1 | G60D | G | 213 | A |
| FAM107B | D185Y | C | 787 | A | FKRP | R154H | G | 810 | A | GLB1L | R440C | G | 1632 | A | HBS1L | P234L | G | 894 | A |
| FAM108B1 | R156I | C | 768 | A | FKRP | R390C | C | 1517 | T | GLB1L | S52I | C | 469 | A | HBS1L | V612I | C | 2027 | T |
| FAM108C1 | I279T | T | 955 | C | FKRP | C191Y | G | 921 | A | GLB1L | - | C | 0 | T | HBS1L | R615Q | C | 2037 | T |
| FAM108C1 | S27R | C | 200 | A | FKTN | L78V | T | 356 | G | GLB1L3 | T57A | A | 1340 | G | HCCS | P110L | C | 531 | T |
| FAM108C1 | R158C | C | 591 | T | FLAD1 | R422M | G | 1587 | T | GLB1L3 | R214H | G | 1812 | A | HCFC1 | T295M | G | 1228 | A |
| FAM109A | A70T | C | 657 | T | FLAD1 | R140H | G | 741 | A | GLB1L3 | - | G | 0 | T | HCFC1 | A907T | C | 3063 | T |
| FAM109A | L61P | A | 631 | G | FLAD1 | R530C | C | 1910 | T | GLCCI1 | R256C | C | 1323 | T | HCFC1 | R374C | G | 1464 | A |

| | | | | | | | | | | | | | | | | | | | |
|---|---|---|---|---|---|---|---|---|---|---|---|---|---|---|---|---|---|---|---|
| FAM109A | A40V | G | 568 | A | FLCN | R17H | C | 505 | T | GLDC | D712N | C | 2285 | T | HCFC1 | R630C | G | 2232 | A |
| FAM109B | Y11C | A | 219 | G | FLCN | E211D | C | 1088 | A | GLDC | G69D | C | 357 | T | HCFC2 | E191* | G | 674 | T |
| FAM110A | R82H | G | 579 | A | FLG | S3864N | C | 11627 | T | GLDC | R884I | C | 2802 | A | HCFC2 | I667V | A | 2102 | G |
| FAM110B | R337H | G | 1890 | A | FLG | A3925T | C | 11809 | T | GLDN | D225N | G | 678 | A | HCFC2 | L166S | T | 600 | C |
| FAM110B | A160V | C | 1359 | T | FLG | Q1177K | G | 3565 | T | GLDN | F341S | T | 1027 | C | HCFC2 | E191* | G | 674 | T |
| FAM110B | T168M | C | 1383 | T | FLG | R587C | G | 1795 | A | GLDN | R414Q | G | 1246 | A | HCFC2 | G239V | G | 819 | T |
| FAM110B | V111M | G | 1211 | A | FLG | H684R | T | 2087 | C | GLE1 | R227H | G | 786 | A | HCK | V389F | G | 1336 | T |
| FAM110B | S189P | T | 1445 | C | FLG | Q499* | G | 1531 | A | GLE1 | E358D | G | 1180 | T | HCLS1 | R467W | G | 1491 | A |
| FAM110B | A160T | G | 1358 | A | FLG | G441R | C | 1357 | T | GLE1 | G508R | G | 1628 | A | HCLS1 | D452N | C | 1446 | T |
| FAM110B | L116M | C | 1226 | A | FLG | G1034R | C | 3136 | T | GLG1 | R1101Q | C | 3352 | T | HCN1 | R458I | C | 1431 | A |
| FAM111A | K10N | G | 257 | T | FLG | T3579A | T | 10771 | C | GLG1 | R933H | C | 2848 | T | HCN1 | P734Q | G | 2259 | T |
| FAM111A | R209C | C | 852 | T | FLG | S1276N | C | 3863 | T | GLG1 | Y497C | T | 1540 | C | HCN1 | Y699H | A | 2153 | G |
| FAM111B | K135N | G | 596 | T | FLG | S673Y | G | 2054 | T | GLG1 | - | C | 0 | T | HCN1 | L111P | A | 390 | G |
| FAM113A | R426G | T | 1779 | C | FLG | S622R | G | 1902 | T | GLG1 | R501* | G | 1551 | A | HCN2 | P230Q | C | 742 | A |
| FAM113A | V239A | A | 1219 | G | FLG | D309E | G | 963 | T | GLI1 | T320M | C | 1037 | T | HCN2 | S251L | C | 805 | T |
| FAM113B | R300H | G | 1630 | A | FLG | R42W | G | 160 | A | GLI1 | V107I | G | 397 | A | HCN3 | L602M | C | 1812 | A |
| FAM114A1 | V86M | G | 432 | A | FLG | A10V | G | 65 | A | GLI1 | E572D | G | 1794 | T | HCN3 | D354E | C | 1070 | G |
| FAM114A1 | V260G | T | 955 | G | FLG2 | G307* | C | 992 | A | GLI2 | R806H | G | 2477 | A | HCN3 | S293L | C | 886 | T |
| FAM114A2 | E76D | T | 228 | G | FLG2 | G1421R | C | 4334 | G | GLI2 | R516L | G | 1607 | T | HCN4 | R1154W | G | 4454 | A |
| FAM116A | R438H | C | 1384 | T | FLG2 | H1406N | G | 4289 | T | GLI2 | A151T | G | 511 | A | HCN4 | R417H | C | 2244 | T |
| FAM116B | V330A | A | 1061 | G | FLG2 | S941T | C | 2895 | G | GLI2 | A188V | C | 623 | T | HCN4 | P224H | G | 1665 | T |
| FAM116B | P195L | G | 656 | A | FLG2 | T2383I | G | 7221 | A | GLI2 | S662L | C | 2045 | T | HCN4 | A412G | G | 2229 | C |
| FAM116B | R253G | T | 829 | C | FLI1 | V225A | T | 1015 | C | GLI2 | S1570P | T | 4768 | C | HCN4 | R332W | G | 1988 | A |
| FAM116B | T49I | G | 218 | A | FLII | R1241W | G | 3773 | A | GLI3 | S1461Y | G | 4468 | T | HCRTR1 | A282T | G | 1229 | A |
| FAM117A | R437H | C | 1390 | T | FLII | G653W | C | 2009 | A | GLI3 | G1168R | C | 3588 | T | HCRTR1 | R260H | G | 1164 | A |
| FAM117B | A575P | G | 1723 | C | FLII | K958N | C | 2926 | A | GLI3 | S1154R | G | 3548 | T | HCRTR1 | G167C | G | 884 | T |
| FAM117B | P308Q | C | 923 | A | FLII | D1188G | T | 3615 | C | GLI3 | D1088N | C | 3348 | T | HDAC10 | A92T | C | 627 | T |
| FAM118A | E64K | G | 1024 | A | FLNA | R1959H | C | 5914 | T | GLI3 | R1542Q | C | 4711 | T | HDAC2 | S81R | G | 243 | C |
| FAM118A | N222S | A | 1499 | G | FLNA | G1023D | C | 3106 | T | GLI3 | V1073I | C | 3303 | T | HDAC2 | F245L | G | 735 | T |
| FAM118A | V210I | G | 1462 | A | FLNA | R496W | G | 1524 | A | GLI3 | G264V | C | 877 | A | HDAC2 | R499G | T | 1495 | C |
| FAM118B | A56V | C | 350 | T | FLNA | A818T | C | 2490 | T | GLI3 | A57T | C | 255 | T | HDAC3 | M24T | A | 151 | G |
| FAM119A | R192Q | C | 751 | T | FLNA | V1609M | C | 4863 | T | GLI3 | P653L | G | 2044 | A | HDAC4 | G623C | C | 2659 | A |
| FAM120A | L245M | C | 758 | A | FLNA | V456M | C | 1404 | T | GLI3 | S191N | C | 658 | T | HDAC4 | A235T | C | 1495 | T |
| FAM120A | P556T | C | 1691 | A | FLNA | S860N | C | 2617 | T | GLI3 | A1337T | C | 4095 | T | HDAC4 | T367M | G | 1892 | A |
| FAM120A | A619T | G | 1880 | A | FLNA | E1351K | C | 4089 | T | GLI3 | N1530K | G | 4676 | T | HDAC4 | A552T | C | 2446 | T |
| FAM120AOS | G132C | C | 1277 | A | FLNB | G756C | G | 2431 | T | GLI4 | P155L | C | 549 | T | HDAC4 | Q731* | G | 2983 | A |

| | | | | | | | | | | | | | | | | | | | | | | | |
|---|---|---|---|---|---|---|---|---|---|---|---|---|---|---|---|---|---|---|---|---|---|---|---|
| FAM120B | S516P | T | 1654 | C | FLNB | T116M | C | 512 | T | GLIPR1 | R39* | C | 263 | T | HDAC4 | P578L | G | 2525 | A |
| FAM120B | H864R | A | 2699 | G | FLNB | V2096M | G | 6451 | A | GLIPR1 | P106S | C | 464 | T | HDAC4 | K259R | T | 1568 | C |
| FAM120B | F314L | T | 1048 | C | FLNB | L1476F | G | 4593 | T | GLIPR1 | L264I | C | 938 | A | HDAC4 | - | C | 0 | A |
| FAM122A | S262I | G | 853 | T | FLNC | E2268K | G | 7063 | A | GLIPR1L1 | E43K | G | 173 | A | HDAC5 | R477H | C | 1762 | T |
| FAM122A | N168S | A | 571 | G | FLNC | D866N | G | 2857 | A | GLIPR1L2 | L84V | T | 287 | G | HDAC5 | T980A | T | 3270 | C |
| FAM122B | R56H | C | 587 | T | FLNC | D2479N | G | 7696 | A | GLIPR2 | E147* | G | 439 | T | HDAC5 | - | T | 0 | C |
| FAM123A | I384V | T | 1818 | C | FLNC | - | G | 0 | T | GLIS1 | R238C | G | 1279 | A | HDAC5 | F984V | A | 3282 | C |
| FAM123B | R177C | G | 802 | A | FLNC | R81C | C | 502 | T | GLIS1 | D529N | C | 2152 | T | HDAC6 | Q519P | A | 1556 | C |
| FAM123B | E384* | C | 1423 | A | FLNC | A1378V | C | 4394 | T | GLIS1 | L399F | G | 1762 | A | HDAC6 | A231T | G | 691 | A |
| FAM123B | G105D | C | 587 | T | FLNC | G973C | G | 3178 | T | GLIS2 | A437T | G | 2130 | A | HDAC7 | A146V | G | 437 | A |
| FAM123B | E244* | C | 1003 | A | FLNC | V215M | G | 904 | A | GLIS2 | L328F | C | 1803 | T | HDAC7 | E398K | C | 1192 | T |
| FAM123C | R463Q | G | 1647 | A | FLNC | R157H | G | 731 | A | GLIS3 | A208V | G | 623 | A | HDAC7 | G868V | C | 2603 | A |
| FAM123C | P438Q | C | 1572 | A | FLNC | D965Y | G | 3154 | T | GLIS3 | R637C | G | 1909 | A | HDAC7 | E119G | T | 356 | C |
| FAM123C | R90* | C | 527 | T | FLNC | D1950Y | G | 6109 | T | GLIS3 | R394H | C | 1181 | T | HDAC9 | H786Q | C | 2399 | G |
| FAM124A | L223I | C | 798 | A | FLRT1 | A402V | C | 1546 | T | GLMN | Y401* | A | 1285 | C | HDAC9 | D601H | G | 1842 | C |
| FAM124A | P364L | C | 1222 | T | FLRT1 | R226H | G | 1018 | A | GLO1 | - | C | 0 | T | HDAC9 | R628H | G | 1924 | A |
| FAM124A | H489Q | C | 1598 | G | FLRT2 | E151D | A | 1220 | C | GLP1R | R48C | C | 185 | T | HDAC9 | P23H | C | 109 | A |
| FAM124A | R116W | C | 477 | T | FLRT2 | A449V | C | 2113 | T | GLP2R | T287M | C | 1373 | T | HDAC9 | V10M | G | 69 | A |
| FAM124B | G366A | C | 1363 | G | FLRT2 | R583Q | G | 2515 | A | GLP2R | A487T | G | 1972 | A | HDAC9 | K61N | G | 224 | T |
| FAM124B | G21D | C | 328 | T | FLRT2 | R52Q | G | 922 | A | GLP2R | K241T | A | 1235 | C | HDAC9 | K97N | A | 332 | C |
| FAM124B | F171S | A | 778 | G | FLRT2 | E118* | G | 1119 | T | GLRA2 | Y195H | T | 915 | C | HDAC9 | E145D | A | 476 | C |
| FAM125A | L112M | C | 423 | A | FLRT3 | R47H | C | 397 | T | GLRA3 | Y261C | T | 1285 | C | HDAC9 | R409Q | G | 1267 | A |
| FAM125A | S58Y | C | 262 | A | FLRT3 | M343V | T | 1284 | C | GLRA3 | F443V | A | 1830 | C | HDC | G201V | C | 1005 | A |
| FAM125B | A303V | C | 989 | T | FLRT3 | Q69P | T | 463 | G | GLRA3 | R351* | G | 1554 | A | HDC | - | A | 0 | G |
| FAM125B | K283T | A | 929 | C | FLT1 | L757V | G | 2521 | C | GLRA4 | E227* | C | 1100 | A | HDGF | D150N | C | 533 | T |
| FAM126A | P167H | G | 733 | T | FLT1 | R1257H | C | 4022 | T | GLRA4 | G375V | C | 1545 | A | HDGF | G168V | C | 588 | A |
| FAM126A | D262Y | C | 1017 | A | FLT1 | N539T | T | 1868 | G | GLRB | D429Y | G | 1555 | T | HDGF | P185S | G | 638 | A |
| FAM129A | R221Q | C | 856 | T | FLT1 | R183C | G | 799 | A | GLRX3 | P157A | C | 491 | G | HDGFL1 | P153L | C | 585 | T |
| FAM129A | R268I | C | 997 | A | FLT3 | S976R | G | 3010 | T | GLRX3 | K29N | A | 109 | C | HDGFL1 | A149V | C | 573 | T |
| FAM129A | E75G | T | 418 | C | FLT3 | R973* | G | 2999 | A | GLS2 | L132I | G | 673 | T | HDGFL1 | G67S | G | 326 | A |
| FAM129B | R618C | G | 2066 | A | FLT3 | Q946H | C | 2920 | A | GLS2 | R109Q | C | 605 | T | HDGFL1 | R73C | C | 344 | T |
| FAM129B | P460H | G | 1593 | T | FLT3 | F804C | A | 2493 | C | GLS2 | F92L | G | 555 | T | HDHD1A | T103M | G | 308 | A |
| FAM129B | V631A | A | 2106 | G | FLT4 | D1067E | G | 3280 | T | GLT1D1 | E341D | A | 1171 | C | HDLBP | E564G | T | 1919 | C |
| FAM129B | E379K | C | 1349 | T | FLT4 | R476W | G | 1505 | A | GLT25D1 | E183D | G | 612 | T | HDLBP | E1107K | C | 3547 | T |
| FAM129C | G289A | G | 1004 | C | FLT4 | R1031* | G | 3170 | A | GLT25D1 | W261* | G | 846 | A | HDLBP | R529Q | C | 1814 | T |
| FAM129C | L669S | T | 2144 | C | FLT4 | A761T | C | 2360 | T | GLT25D2 | Y568H | A | 2074 | G | HDLBP | R1114G | T | 3568 | C |
| FAM131B | Q26H | T | 222 | G | FLT4 | R1031* | G | 3170 | A | GLT25D2 | V219I | C | 1027 | T | HDLBP | E936D | C | 3036 | A |
| FAM131B | S228L | G | 827 | A | FLT4 | D105N | C | 392 | T | GLT6D1 | S160R | A | 734 | C | HDLBP | R163C | G | 715 | A |
| FAM131B | R73Q | C | 362 | T | FLVCR2 | L351P | T | 1428 | C | GLT8D1 | Y221H | A | 1015 | G | HDX | D455G | T | 1476 | C |

| | | | | | | | | | | | | | | | | | | | |
|---|---|---|---|---|---|---|---|---|---|---|---|---|---|---|---|---|---|---|---|
| FAM134A | A143V | C | 564 | T | FLVCR2 | C474R | T | 1796 | C | GLT8D2 | K151E | T | 857 | C | HDX | E535D | C | 1717 | A |
| FAM134C | R245H | C | 794 | T | FLVCR2 | E499D | A | 1873 | C | GLT8D2 | R5Q | C | 420 | T | HEATR1 | I707S | A | 2235 | C |
| FAM134C | F154S | A | 521 | G | FLYWCH1 | E272K | G | 1177 | A | GLTPD2 | R181H | G | 595 | A | HEATR1 | R1336C | G | 4121 | A |
| FAM135A | N1474T | A | 5035 | C | FLYWCH1 | E5K | G | 376 | A | GLTSCR1 | H917N | C | 2943 | A | HEATR2 | A233T | G | 717 | A |
| FAM135A | S1048R | T | 3758 | G | FLYWCH1 | R439W | C | 1678 | T | GLTSCR1 | P446H | C | 1531 | A | HEATR2 | A683V | C | 2068 | T |
| FAM135B | A823D | G | 2641 | T | FLYWCH1 | A308S | G | 1285 | T | GLTSCR1 | T1075M | C | 3418 | T | HEATR3 | R665Q | G | 2136 | A |
| FAM135B | P563H | G | 1861 | T | FLYWCH1 | M335V | A | 1366 | G | GLTSCR1 | Y1201H | T | 3795 | C | HEATR4 | G76S | C | 636 | T |
| FAM135B | C495Y | C | 1657 | T | FMN1 | E1102G | T | 3305 | C | GLTSCR1 | R1474W | C | 4614 | T | HEATR4 | I509M | T | 1937 | C |
| FAM135B | K905N | C | 2888 | A | FMN1 | L951F | G | 2851 | A | GLTSCR1 | V1095I | G | 3477 | A | HEATR5A | R352C | G | 1054 | A |
| FAM135B | R701Q | C | 2275 | T | FMN2 | E1835K | G | 5503 | A | GLTSCR1 | K1066N | A | 3392 | C | HEATR5A | Y834* | G | 2502 | C |
| FAM135B | R1211Q | C | 3805 | T | FMN2 | A664T | G | 1990 | A | GLTSCR2 | R391W | C | 1180 | T | HEATR5A | S1385R | T | 4153 | G |
| FAM135B | E1130D | T | 3563 | G | FMN2 | P1076L | C | 3227 | T | GLTSCR2 | V89M | G | 274 | A | HEATR5A | F1857C | A | 5570 | C |
| FAM135B | F1057L | G | 3344 | T | FMN2 | P1244L | C | 3731 | T | GLTSCR2 | E123D | G | 378 | T | HEATR5B | S688Y | G | 2159 | T |
| FAM136A | A61V | G | 216 | A | FMNL1 | - | G | 0 | A | GLTSCR2 | R454Q | G | 1370 | A | HEATR5B | K1744T | T | 5327 | G |
| FAM13A | D155Y | C | 671 | A | FMNL1 | Y59H | T | 375 | C | GLUD1 | M69I | C | 304 | A | HEATR5B | L939V | A | 2911 | C |
| FAM13A | G108S | C | 530 | T | FMNL1 | E679K | G | 2235 | A | GLUD2 | R34C | C | 177 | T | HEATR6 | M1155V | T | 3480 | C |
| FAM13A | R989H | C | 3174 | T | FMNL1 | A1018T | G | 3252 | A | GLUD2 | A28V | C | 160 | T | HEATR6 | S493Y | G | 1495 | T |
| FAM13A | S817N | C | 2658 | T | FMNL2 | A792T | G | 2741 | A | GLUD2 | S166Y | C | 574 | A | HEATR6 | L847P | A | 2557 | G |
| FAM13A | Y629* | G | 2095 | T | FMNL2 | A41T | G | 488 | A | GLUL | R324H | C | 1512 | T | HEATR6 | V1008M | C | 3039 | T |
| FAM13A | E450K | C | 1556 | T | FMNL2 | A521T | G | 1928 | A | GLYAT | P83H | G | 403 | T | HEATR7A | T402A | A | 1261 | G |
| FAM13B | G396S | C | 1638 | T | FMNL2 | R738Q | G | 2580 | A | GLYATL1 | T166M | C | 497 | T | HEATR7A | V367M | G | 1156 | A |
| FAM13B | F40L | G | 572 | T | FMNL2 | G909* | G | 3092 | T | GLYATL1 | A161S | G | 481 | T | HEATR7A | T180M | C | 596 | T |
| FAM13C | T56A | T | 185 | C | FMNL3 | N877D | T | 2863 | C | GLYATL3 | D51V | A | 265 | T | HEATR7A | A153T | G | 514 | A |
| FAM13C | A94V | G | 300 | A | FMNL3 | G208V | C | 857 | A | GLYATL3 | F267L | C | 914 | A | HEATR7A | Q233* | C | 754 | T |
| FAM13C | E601* | C | 1820 | A | FMNL3 | A753V | G | 2492 | A | GLYCTK | F347L | C | 1101 | A | HEATR7B2 | Q597* | G | 2279 | A |
| FAM13C | M23V | T | 86 | C | FMNL3 | R302H | C | 1139 | T | GLYCTK | Q472H | G | 1476 | T | HEATR7B2 | G1153D | C | 3948 | T |
| FAM149A | R655W | C | 1963 | T | FMO1 | V463I | G | 1509 | A | GLYR1 | A433T | C | 1374 | T | HEATR7B2 | L337V | A | 1499 | C |
| FAM149B1 | D497N | G | 1663 | A | FMO1 | R291H | G | 994 | A | GLYR1 | F46I | A | 213 | T | HEATR7B2 | A914V | G | 3231 | A |
| FAM149B1 | A504V | C | 1685 | T | FMO3 | D76E | T | 339 | A | GM2A | L84F | A | 577 | C | HECA | R289H | G | 1151 | A |
| FAM149B1 | M1R | T | 176 | G | FMO4 | C440Y | G | 1649 | A | GMCL1L | Y428C | A | 1534 | G | HECA | R152C | C | 739 | T |
| FAM149B1 | R367I | G | 1274 | T | FMO4 | H513Y | C | 1867 | T | GMCL1L | R450M | G | 1600 | T | HECA | R150C | C | 733 | T |
| FAM149B2 | E283* | G | 847 | T | FMO4 | N320S | A | 1289 | G | GMEB2 | R529W | G | 2064 | A | HECA | K456R | A | 1652 | G |
| FAM149B2 | R262C | C | 784 | T | FMO4 | W479G | T | 1765 | G | GMEB2 | G112S | C | 813 | T | HECA | D112N | G | 619 | A |
| FAM149B2 | L276P | T | 827 | C | FMO5 | V343I | C | 1108 | T | GMIP | R391M | C | 1289 | A | HECTD1 | R371Q | C | 1601 | T |
| FAM151A | R318W | G | 1113 | A | FMO5 | D341Y | C | 1102 | A | GMIP | - | C | 0 | A | HECTD1 | E2536A | T | 8096 | G |
| FAM151B | T200M | C | 754 | T | FMO6P | A16S | G | 46 | T | GML | A82T | G | 334 | A | HECTD1 | N2413H | T | 7726 | G |
| FAM153A | H210N | C | 685 | A | FMR1 | R611C | C | 1959 | T | GMPPA | Y341C | A | 1093 | G | HECTD1 | R2373H | C | 7607 | T |
| FAM154A | R323Q | C | 1248 | T | FMR1NB | G10R | G | 102 | A | GMPR | R146H | G | 555 | A | HECTD1 | S2283R | T | 7336 | G |
| FAM154A | D203N | C | 887 | T | FMR1NB | D150Y | G | 522 | T | GMPR | R39Q | G | 234 | A | HECTD1 | N1368S | T | 4592 | C |

| | | | | | | | | | | | | | | | | | | | |
|---|---|---|---|---|---|---|---|---|---|---|---|---|---|---|---|---|---|---|---|
| FAM154B | R52Q | G | 236 | A | FMR1NB | - | T | 0 | C | GMPR2 | K20R | A | 128 | G | HECTD1 | K550Q | T | 2137 | G |
| FAM154B | S56L | C | 248 | T | FMR1NB | D215N | G | 717 | A | GMPR2 | S312F | C | 1004 | T | HECTD2 | T452A | A | 1454 | G |
| FAM154B | D78G | A | 314 | G | FN1 | R1725H | C | 5544 | T | GNA11 | V164G | T | 733 | G | HECTD2 | K761N | G | 2383 | T |
| FAM155A | R197Q | C | 729 | T | FN1 | R1493* | G | 4847 | A | GNA11 | I56V | A | 408 | G | HECTD2 | T158M | C | 573 | T |
| FAM155A | C376S | A | 1265 | T | FN1 | A810V | G | 2799 | A | GNA13 | I366V | T | 1305 | C | HECTD3 | R395C | G | 1255 | A |
| FAM155A | A125T | C | 512 | T | FN1 | R830C | G | 2858 | A | GNA13 | S90I | C | 478 | A | HECTD3 | I601V | T | 1873 | C |
| FAM155A | D54G | T | 300 | C | FN1 | - | A | 0 | G | GNA13 | R227K | C | 889 | T | HECW1 | A644V | C | 2536 | T |
| FAM155A | Q82R | T | 384 | C | FN1 | R2149G | T | 6815 | C | GNA14 | G3D | C | 522 | T | HECW1 | S1271L | C | 4417 | T |
| FAM155A | R39Q | C | 255 | T | FN1 | V944I | C | 3200 | T | GNA15 | R150C | C | 706 | T | HECW1 | V1452M | G | 4959 | A |
| FAM158A | A24T | C | 233 | T | FN1 | G1616R | C | 5216 | T | GNA15 | K36N | G | 366 | T | HECW1 | G18R | G | 657 | C |
| FAM160A1 | A962T | G | 3459 | A | FN1 | F1585L | G | 5125 | T | GNA15 | V199M | G | 853 | A | HECW1 | R1050Q | G | 3754 | A |
| FAM160A2 | E223* | C | 978 | A | FN1 | E888* | C | 3032 | A | GNA15 | D158N | G | 730 | A | HECW1 | E898K | G | 3297 | A |
| FAM160B1 | V576A | T | 2062 | C | FN1 | L256F | G | 1136 | A | GNAI1 | E65G | A | 567 | G | HECW1 | I90V | A | 873 | G |
| FAM160B1 | R603* | C | 2142 | T | FN1 | E855D | T | 2935 | G | GNAL | R296I | G | 1185 | T | HECW1 | R296C | C | 1491 | T |
| FAM160B2 | H641N | C | 1950 | A | FN3KRP | S242L | C | 775 | T | GNAO1 | A12V | C | 881 | T | HECW1 | S302L | C | 1510 | T |
| FAM161A | K710Q | T | 2140 | G | FNBP1 | F539S | A | 1803 | G | GNAS | R16C | C | 49 | T | HECW1 | A243T | G | 1332 | A |
| FAM161A | E253* | C | 769 | A | FNBP1 | H17R | T | 237 | C | GNAS | R844H | G | 2534 | A | HECW1 | I1250S | T | 4354 | G |
| FAM161A | I472N | A | 1427 | T | FNBP1L | R516H | G | 1547 | A | GNAS | R844C | C | 2533 | T | HECW2 | R45W | G | 316 | A |
| FAM161A | N482T | T | 1457 | G | FNBP1L | R156I | G | 467 | T | GNAS | R844C | C | 2533 | T | HECW2 | R739Q | C | 2399 | T |
| FAM161B | C415R | A | 1631 | G | FNBP4 | R803M | C | 2561 | A | GNAS | G230* | G | 691 | T | HECW2 | S1022N | C | 3248 | T |
| FAM161B | P433H | G | 1686 | T | FNDC1 | D261V | A | 982 | T | GNAT2 | K21N | C | 250 | A | HECW2 | R1312M | C | 4118 | A |
| FAM164A | K75N | G | 279 | T | FNDC1 | R1299C | C | 4095 | T | GNAT3 | F223L | A | 763 | C | HECW2 | H470Y | G | 1591 | A |
| FAM164A | R189* | C | 619 | T | FNDC1 | L505F | C | 1713 | T | GNAZ | D201G | A | 1268 | G | HEG1 | S646L | G | 2005 | A |
| FAM164C | E209V | A | 799 | T | FNDC1 | P666H | C | 2197 | A | GNAZ | D94N | G | 946 | A | HEG1 | S782I | C | 2413 | A |
| FAM164C | F125C | T | 547 | G | FNDC1 | R1140* | C | 3618 | T | GNB1 | G216D | C | 979 | T | HEG1 | F766L | G | 2366 | T |
| FAM166A | E83K | C | 302 | T | FNDC1 | T1057M | C | 3370 | T | GNB1 | T47M | G | 472 | A | HELLS | N828H | A | 2587 | C |
| FAM166A | I120T | A | 414 | G | FNDC1 | R564Q | G | 1891 | A | GNB2 | R48W | C | 624 | T | HELLS | I116T | T | 452 | C |
| FAM167B | R57M | G | 359 | T | FNDC1 | A1273T | G | 4017 | A | GNB2 | A203T | G | 1089 | A | HELQ | R710C | G | 2291 | A |
| FAM168A | - | C | 0 | A | FNDC1 | A1229T | G | 3885 | A | GNB2 | R52H | G | 637 | A | HELQ | N101D | T | 464 | C |
| FAM168A | Y237H | A | 988 | G | FNDC1 | P1853H | C | 5758 | A | GNB2 | D228N | G | 1164 | A | HELT | N314D | A | 940 | G |
| FAM169A | M129I | C | 478 | A | FNDC1 | R252Q | G | 955 | A | GNB2L1 | T94I | G | 374 | A | HELZ | H1790N | G | 5555 | T |
| FAM170B | F231L | G | 783 | T | FNDC1 | E364D | A | 1292 | C | GNB3 | S74A | T | 625 | G | HELZ | A1670V | G | 5196 | A |
| FAM171A1 | Y241C | T | 729 | C | FNDC1 | R1346I | G | 4237 | T | GNB3 | I171S | T | 917 | G | HELZ | F1431L | A | 4480 | C |
| FAM171A1 | R706W | G | 2123 | A | FNDC1 | P1446S | C | 4536 | T | GNB4 | - | C | 0 | A | HELZ | K970N | C | 3097 | A |
| FAM171A1 | L306I | G | 923 | T | FNDC1 | D1521N | G | 4761 | A | GNE | D84Y | C | 350 | A | HELZ | I381L | T | 1328 | G |
| FAM171A2 | R352H | C | 1216 | T | FNDC3A | A648V | C | 2248 | T | GNE | R39Q | C | 216 | T | HELZ | K56N | T | 355 | G |
| FAM171A2 | R608H | C | 1984 | T | FNDC3A | R113H | G | 643 | A | GNE | D530E | G | 1690 | T | HEMGN | R43I | C | 370 | A |
| FAM171A2 | V63A | A | 349 | G | FNDC3A | D788G | A | 2668 | G | GNG13 | K61R | T | 275 | C | HEMGN | E38* | C | 354 | A |
| FAM173A | A101D | C | 419 | A | FNDC3A | R1122C | C | 3669 | T | GNGT2 | A32V | G | 185 | A | HEMK1 | A149T | G | 741 | A |

| | | | | | | | | | | | | | | | | | | | |
|---|---|---|---|---|---|---|---|---|---|---|---|---|---|---|---|---|---|---|---|
| FAM173B | R101H | C | 315 | T | FNDC3B | P595H | C | 1956 | A | GNL2 | D228A | T | 782 | G | HEMK1 | A60T | G | 474 | A |
| FAM174B | K157T | T | 612 | G | FNDC3B | R744S | G | 2404 | T | GNL2 | Q452R | T | 1454 | C | HEMK1 | V71A | T | 508 | C |
| FAM175B | Y391H | T | 1216 | C | FNDC3B | V221I | G | 833 | A | GNL3L | F199L | C | 736 | G | HEPACAM | R288H | C | 1269 | T |
| FAM175B | K64T | A | 236 | C | FNDC5 | R59Q | C | 224 | T | GNL3L | R522C | C | 1703 | T | HEPACAM | M193I | C | 985 | A |
| FAM175B | E317* | G | 994 | T | FNDC7 | S220Y | C | 659 | A | GNL3L | P410H | C | 1368 | A | HEPACAM | R92Q | C | 681 | T |
| FAM176A | A121T | C | 786 | T | FNIP1 | R977* | G | 2991 | A | GNL3L | R495C | C | 1622 | T | HEPACAM2 | W205* | C | 638 | T |
| FAM176A | M136I | C | 833 | T | FNIP2 | G890S | G | 2668 | A | GNL3L | N8H | A | 161 | C | HEPH | V889M | G | 2997 | A |
| FAM178A | T818I | C | 2585 | T | FNTA | A70T | G | 256 | A | GNLY | G125R | G | 501 | A | HEPH | V113A | T | 670 | C |
| FAM178A | S841G | A | 2653 | G | FNTA | L295R | T | 932 | G | GNLY | R94W | C | 408 | T | HEPH | R729H | G | 2518 | A |
| FAM178A | R388C | C | 1294 | T | FNTB | F23V | T | 67 | G | GNPDA1 | M215V | T | 696 | C | HEPH | R1144H | G | 3763 | A |
| FAM178B | R793H | C | 2378 | T | FNTB | G403S | G | 1207 | A | GNPDA2 | E127* | C | 536 | A | HEPH | R319H | G | 1288 | A |
| FAM178B | R224H | C | 671 | T | FNTB | L416S | T | 1247 | C | GNPTAB | R1169M | C | 3685 | A | HEPHL1 | A452V | C | 1512 | T |
| FAM178B | R604H | C | 1811 | T | FOLH1 | E102* | C | 565 | A | GNPTAB | P1096S | G | 3465 | A | HEPHL1 | L390F | C | 1325 | T |
| FAM178B | P537H | G | 1610 | T | FOLH1B | E313K | G | 1463 | A | GNPTAB | L303* | A | 1087 | C | HEPHL1 | E797D | G | 2548 | T |
| FAM179A | A574T | G | 2071 | A | FOLR2 | F208V | T | 797 | G | GNPTG | A114T | G | 383 | A | HEPHL1 | F119C | T | 513 | G |
| FAM179A | S959N | G | 3227 | A | FOLR2 | R119H | G | 531 | A | GNS | P266H | G | 967 | T | HEPHL1 | F601C | T | 1959 | G |
| FAM179A | R955Q | G | 3215 | A | FOLR4 | F90S | T | 269 | C | GOLGA1 | D48N | C | 476 | T | HEPHL1 | F687L | C | 2218 | A |
| FAM179A | A865T | G | 2944 | A | FOLR4 | S191R | C | 573 | A | GOLGA1 | R411C | G | 1565 | A | HERC1 | A3805V | G | 1156 2 | A |
| FAM179A | E9K | G | 376 | A | FOSL1 | - | T | 1003 | A | GOLGA2 | R537C | G | 1622 | A | HERC1 | R883I | C | 2796 | A |
| FAM179A | D716Y | G | 2497 | T | FOXA2 | Y168C | T | 688 | C | GOLGA2B | R62Q | C | 513 | T | HERC1 | R3453H | C | 1050 6 | T |
| FAM179B | G413V | G | 1421 | T | FOXA2 | D447E | A | 1526 | T | GOLGA2B | D19N | C | 383 | T | HERC1 | - | T | 0 | C |
| FAM179B | R524C | C | 1753 | T | FOXB1 | A275V | C | 1303 | T | GOLGA3 | A1049V | G | 3705 | A | HERC1 | R106* | G | 464 | A |
| FAM181A | P238S | C | 1019 | T | FOXB1 | Y128C | A | 862 | G | GOLGA3 | R903C | G | 3266 | A | HERC1 | Q3182R | T | 9693 | C |
| FAM182A | - | G | 0 | T | FOXB2 | A271V | C | 812 | T | GOLGA3 | F1347C | A | 4599 | C | HERC1 | L662P | A | 2133 | G |
| FAM182B | G136V | C | 786 | A | FOXB2 | L298P | T | 893 | C | GOLGA4 | Y1617D | T | 5148 | G | HERC1 | A4388V | G | 1331 1 | A |
| FAM183A | V39M | G | 144 | A | FOXB2 | H142P | A | 425 | C | GOLGA4 | M2202V | A | 6903 | G | HERC1 | R258* | G | 920 | A |
| FAM183B | D57A | T | 1105 | G | FOXC1 | T153M | C | 458 | T | GOLGA4 | - | T | 0 | C | HERC1 | R3248Q | C | 9891 | T |
| FAM184A | R932I | C | 2890 | A | FOXC1 | D178N | G | 532 | A | GOLGA4 | K354N | G | 1361 | T | HERC1 | D4859G | T | 1472 4 | C |
| FAM184A | T723M | G | 2263 | A | FOXC2 | A291G | C | 957 | G | GOLGA4 | K374N | G | 1421 | T | HERC1 | N3902D | T | 1185 2 | C |
| FAM184A | T730M | G | 2284 | A | FOXC2 | D137N | G | 494 | A | GOLGA4 | E1132* | G | 3693 | T | HERC1 | P1074S | G | 3368 | A |
| FAM184A | - | C | 0 | A | FOXD2 | G214D | G | 2760 | A | GOLGA4 | E1358A | A | 4372 | C | HERC1 | D2381Y | C | 7289 | A |
| FAM184A | L266F | C | 893 | A | FOXD2 | P445H | C | 3453 | A | GOLGA5 | S33G | A | 279 | G | HERC1 | R455K | C | 1512 | T |
| FAM184A | R238I | C | 808 | A | FOXD2 | P233S | C | 2816 | T | GOLGA5 | E565K | G | 1875 | A | HERC1 | R1448W | G | 4490 | A |
| FAM184B | A654T | C | 2173 | T | FOXD4L1 | A68V | C | 376 | T | GOLGA5 | E623K | G | 2049 | A | HERC2 | A4806V | G | 1452 6 | A |
| FAM184B | H439Y | G | 1528 | A | FOXE1 | A164V | C | 1151 | T | GOLGA5 | F126C | T | 559 | G | HERC2 | R577C | G | 1838 | A |

| | | | | | | | | | | | | | | | | | | | |
|---|---|---|---|---|---|---|---|---|---|---|---|---|---|---|---|---|---|---|---|
| FAM184B | V36M | C | 319 | T | FOXF1 | A24V | C | 103 | T | GOLGA7 | R118* | C | 430 | T | HERC2 | G581S | C | 1850 | T |
| FAM184B | M868I | C | 2817 | A | FOXF1 | D373Y | G | 1149 | T | GOLGA7 | I127M | T | 459 | G | HERC2 | E4709K | C | 14234 | T |
| FAM184B | R820W | G | 2671 | A | FOXF2 | F216L | T | 760 | C | GOLGB1 | E2021* | C | 6172 | A | HERC2 | R746H | C | 2346 | T |
| FAM186A | G2201R | C | 6739 | T | FOXG1 | G361S | G | 1450 | A | GOLGB1 | E1852* | C | 5665 | A | HERC2 | A4634V | G | 14010 | A |
| FAM186A | I792M | T | 2514 | C | FOXG1 | R281W | C | 1210 | T | GOLGB1 | E889D | C | 2778 | A | HERC2 | V3665I | C | 11102 | T |
| FAM186A | E2010* | C | 6166 | A | FOXG1 | A188T | G | 931 | A | GOLGB1 | E750* | C | 2359 | A | HERC2 | A1939T | C | 5924 | T |
| FAM186A | K1258T | T | 3911 | G | FOXG1 | A436T | G | 1675 | A | GOLGB1 | R2480C | G | 7549 | A | HERC2 | V395D | A | 1293 | T |
| FAM186A | K931N | T | 2931 | G | FOXG1 | A375T | G | 1492 | A | GOLGB1 | L1723I | G | 5278 | T | HERC2 | A3263D | G | 9897 | T |
| FAM186A | S203Y | G | 746 | T | FOXG1 | A411V | C | 1601 | T | GOLGB1 | A100T | C | 409 | T | HERC2 | A4734T | C | 14309 | T |
| FAM186B | E495D | C | 1647 | A | FOXH1 | Q19* | G | 634 | A | GOLGB1 | E1929V | T | 5897 | A | HERC2 | - | T | 0 | C |
| FAM186B | V230I | C | 850 | T | FOXH1 | Y285C | T | 1433 | C | GOLGB1 | S2980L | G | 9050 | A | HERC2 | I2702F | T | 8213 | A |
| FAM186B | A64V | G | 353 | A | FOXI1 | P68S | C | 250 | T | GOLGB1 | N3027H | T | 9190 | G | HERC2 | Q4330H | C | 13099 | A |
| FAM186B | E55* | C | 325 | A | FOXI2 | G292D | G | 914 | A | GOLGB1 | D1763N | C | 5398 | T | HERC2 | R2784H | C | 8460 | T |
| FAM187B | E131K | C | 440 | T | FOXI2 | R141C | C | 460 | T | GOLGB1 | E1064D | T | 3303 | G | HERC2 | G4433R | C | 13406 | T |
| FAM188A | C131F | C | 613 | A | FOXJ2 | R256H | G | 1852 | A | GOLGB1 | K951Q | T | 2962 | G | HERC2 | T3098P | T | 9401 | G |
| FAM188B | L497M | C | 1566 | A | FOXJ3 | P224T | G | 939 | T | GOLGB1 | E902K | C | 2815 | T | HERC2 | A2208V | G | 6732 | A |
| FAM188B | R48Q | G | 220 | A | FOXJ3 | K134T | T | 670 | G | GOLGB1 | K882Q | T | 2755 | G | HERC2 | P278S | G | 941 | A |
| FAM188B | G630A | G | 1966 | C | FOXK1 | I331N | T | 992 | A | GOLIM4 | R284Q | C | 1541 | T | HERC2 | H4355Y | G | 13172 | A |
| FAM189A2 | L406R | T | 1321 | G | FOXK2 | P478Q | C | 1607 | A | GOLIM4 | R478Q | C | 2123 | T | HERC2 | A4634V | G | 14010 | A |
| FAM189A2 | R402I | G | 1309 | T | FOXK2 | R631Q | G | 2066 | A | GOLM1 | E341V | T | 1165 | A | HERC2 | W627* | C | 1990 | T |
| FAM189A2 | R164H | G | 595 | A | FOXK2 | R351W | C | 1225 | T | GOLM1 | E341* | C | 1164 | A | HERC2 | R3906C | G | 11825 | A |
| FAM189B | R370C | G | 1714 | A | FOXK2 | R544W | C | 1804 | T | GOLPH3 | R269W | G | 1121 | A | HERC2 | L3384I | G | 10259 | T |
| FAM189B | M1V | T | 607 | C | FOXN1 | D263N | G | 816 | A | GOLPH3 | R231H | C | 1008 | T | HERC2 | I1071N | A | 3321 | T |
| FAM18A | L14P | A | 148 | G | FOXN1 | A143T | G | 456 | A | GON4L | R2112I | C | 6384 | A | HERC2 | R698H | C | 2202 | T |
| FAM18B2 | S204Y | G | 707 | T | FOXN1 | P219S | C | 684 | T | GON4L | V304M | C | 959 | T | HERC3 | A532T | G | 1833 | A |
| FAM190A | R19I | G | 344 | T | FOXN2 | D360E | T | 1341 | A | GON4L | V26I | C | 125 | T | HERC3 | K575R | A | 1963 | G |
| FAM190A | V817I | G | 2737 | A | FOXN2 | S83R | T | 510 | G | GORAB | E333Q | G | 1017 | C | HERC3 | Q326H | A | 1217 | C |
| FAM190A | L662P | T | 2273 | C | FOXN2 | R386Q | G | 1418 | A | GORAB | R251M | G | 772 | T | HERC4 | V140A | A | 667 | G |
| FAM190A | L29V | C | 373 | G | FOXN3 | S187L | G | 576 | A | GORASP1 | V67M | C | 1021 | T | HERC4 | R315Q | C | 1192 | T |
| FAM190A | L581* | T | 2030 | A | FOXN3 | D364N | C | 1106 | T | GORASP2 | R217H | G | 650 | A | HERC4 | S294N | C | 1129 | T |
| FAM190A | R766C | C | 2584 | T | FOXN4 | A450T | C | 1453 | T | GOT2 | R287C | G | 911 | A | HERC5 | S113* | C | 491 | A |
| FAM190A | K79T | A | 524 | C | FOXN4 | P23H | G | 173 | T | GP1BA | T91M | C | 272 | T | HERC5 | S640L | C | 2072 | T |

| | | | | | | | | | | | | | | | | | | | |
|---|---|---|---|---|---|---|---|---|---|---|---|---|---|---|---|---|---|---|---|
| FAM190A | R123Q | G | 656 | A | FOXO1 | R316Q | C | 1332 | T | GP1BA | Y201H | T | 601 | C | HERC5 | S664L | C | 2144 | T |
| FAM190B | R773Q | G | 2512 | A | FOXO1 | A574D | G | 2106 | T | GP2 | P487S | G | 1609 | A | HERC6 | - | T | 0 | G |
| FAM190B | R805H | G | 2608 | A | FOXO4 | R92Q | G | 607 | A | GP2 | G32V | C | 245 | A | HERPUD1 | A194G | C | 701 | G |
| FAM192A | G191R | C | 830 | T | FOXP1 | Q196H | C | 742 | A | GP2 | V252F | C | 904 | A | HES6 | A122T | C | 583 | T |
| FAM193A | A1006S | G | 3367 | T | FOXP1 | S450L | G | 1503 | A | GP5 | A483T | C | 1519 | T | HESX1 | R159W | G | 802 | A |
| FAM193A | Q627H | G | 2232 | T | FOXP1 | R527* | G | 1733 | A | GP5 | T512A | T | 1606 | C | HEXA | Y82C | T | 452 | C |
| FAM193B | E690K | C | 2068 | T | FOXP2 | E379K | G | 1509 | A | GP5 | R372C | G | 1186 | A | HEXB | R533H | G | 1715 | A |
| FAM194A | V48E | A | 196 | T | FOXP2 | D727N | G | 2553 | A | GP5 | G212R | C | 706 | T | HEXB | V39M | G | 232 | A |
| FAM194A | E54V | T | 214 | A | FOXP2 | H435Y | C | 1677 | T | GP5 | R15H | C | 116 | T | HEXDC | P557T | C | 2085 | A |
| FAM194A | - | A | 0 | G | FOXP2 | K458N | G | 1748 | T | GP6 | Q470H | T | 1438 | G | HEXDC | A425T | G | 1689 | A |
| FAM194A | A344V | G | 1084 | A | FOXP2 | D738N | G | 2586 | A | GP6 | E41D | C | 151 | A | HEXIM2 | V160M | G | 710 | A |
| FAM194A | F140V | A | 471 | C | FOXP4 | R337Q | G | 1468 | A | GP9 | R112H | G | 557 | A | HEY2 | A116T | G | 543 | A |
| FAM196B | R454Q | C | 2743 | T | FOXR1 | R228* | C | 907 | T | GPA33 | C261R | A | 1125 | G | HEY2 | D120E | C | 557 | G |
| FAM196B | N460T | T | 2761 | G | FOXRED1 | Y413H | T | 1298 | C | GPAM | - | A | 0 | G | HEYL | R148H | C | 763 | T |
| FAM196B | T468M | G | 2785 | A | FOXRED1 | F119L | C | 418 | A | GPAM | G541E | C | 1820 | T | HFE | V343I | G | 1149 | A |
| FAM196B | F23L | A | 1451 | T | FPGS | R531* | C | 1641 | T | GPAT2 | R693W | G | 2246 | A | HFE2 | S206F | C | 855 | T |
| FAM198A | A27V | C | 456 | T | FPGS | G87W | G | 309 | T | GPAT2 | P782S | G | 2513 | A | HFM1 | K1308T | T | 4022 | G |
| FAM198B | A43V | G | 535 | A | FPGS | S518I | G | 1603 | T | GPATCH1 | S251L | C | 1066 | T | HFM1 | F1274V | A | 3919 | C |
| FAM198B | A65V | G | 601 | A | FPR1 | V59L | C | 270 | G | GPATCH1 | S760G | A | 2592 | G | HFM1 | A907V | G | 2819 | A |
| FAM198B | R115C | G | 750 | A | FPR1 | M55I | C | 260 | T | GPATCH1 | K230Q | A | 1002 | C | HFM1 | S31P | A | 190 | G |
| FAM198B | L38I | G | 519 | T | FPR1 | P79S | G | 330 | A | GPATCH1 | F623L | T | 2183 | G | HFM1 | T48A | T | 241 | C |
| FAM19A1 | S8P | T | 512 | C | FPR1 | L252I | G | 849 | T | GPATCH1 | P478S | C | 1746 | T | HFM1 | S1174Y | G | 3620 | T |
| FAM19A2 | K55T | T | 170 | G | FPR1 | S181L | G | 637 | A | GPATCH3 | E148D | C | 468 | A | HFM1 | K677N | C | 2130 | A |
| FAM19A5 | A84T | G | 251 | A | FPR1 | H90Q | A | 365 | C | GPATCH3 | R214Q | C | 665 | T | HFM1 | - | C | 0 | A |
| FAM200B | S32I | G | 933 | T | FPR2 | G185C | G | 868 | T | GPATCH8 | Q1192P | T | 3639 | G | HGD | S106F | G | 777 | A |
| FAM200B | F355C | T | 1902 | G | FPR2 | R238H | G | 1028 | A | GPATCH8 | R859H | C | 2640 | T | HGD | S47L | G | 600 | A |
| FAM20B | P327T | C | 1315 | A | FPR3 | A68T | G | 381 | A | GPATCH8 | A126T | C | 440 | T | HGF | R234C | G | 927 | A |
| FAM20C | H204Y | C | 841 | T | FPR3 | E270* | G | 987 | T | GPATCH8 | A305G | G | 978 | C | HGF | R234H | C | 928 | T |
| FAM20C | R409C | C | 1456 | T | FPR3 | L97I | C | 468 | A | GPATCH8 | S1417Y | G | 4314 | T | HGF | I39S | A | 343 | C |
| FAM20C | Y370C | A | 1340 | G | FRA10AC1 | S251A | A | 949 | C | GPATCH8 | G58V | C | 237 | A | HGFAC | A327V | C | 1007 | T |
| FAM21A | A553V | C | 1703 | T | FRAS1 | A2739T | G | 8655 | A | GPBP1 | D368N | G | 2376 | A | HGFAC | V480M | G | 1465 | A |
| FAM21A | D85A | A | 299 | C | FRAS1 | R926* | C | 3216 | T | GPBP1 | R87Q | G | 1534 | A | HGFAC | G592S | G | 1801 | A |
| FAM21C | Q52H | G | 238 | T | FRAS1 | R3654H | G | 11401 | A | GPBP1L1 | K135R | T | 1626 | C | HGS | R328Q | G | 1060 | A |
| FAM25C | I54T | A | 159 | G | FRAS1 | G3220C | G | 10098 | T | GPC1 | R400H | G | 1197 | A | HGS | R518W | C | 1629 | T |
| FAM26D | T194N | C | 625 | A | FRAS1 | A3904V | C | 12151 | T | GPC1 | A328T | G | 980 | A | HGS | A562T | G | 1761 | A |
| FAM26E | L210P | T | 680 | C | FRAS1 | R270H | G | 1249 | A | GPC1 | D272Y | G | 812 | T | HGS | V607M | G | 1896 | A |
| FAM32A | P60S | C | 198 | T | FRAS1 | G1499* | G | 4935 | T | GPC2 | A317T | C | 1117 | T | HGSNAT | E149* | G | 445 | T |

| | | | | | | | | | | | | | | | | | | | |
|---|---|---|---|---|---|---|---|---|---|---|---|---|---|---|---|---|---|---|---|
| FAM35B | I122K | T | 2238 | A | FRAS1 | E2900K | G | 9138 | A | GPC3 | C265R | A | 1239 | G | HGSNAT | A643T | G | 1927 | A |
| FAM38A | A1836V | G | 5507 | A | FRAS1 | F2172V | T | 6954 | G | GPC4 | R174C | G | 1045 | A | HGSNAT | T258M | C | 773 | T |
| FAM38A | G1843R | C | 5527 | T | FRAS1 | D3093E | T | 9719 | G | GPC5 | L13I | C | 409 | A | HHATL | A150T | C | 598 | T |
| FAM38A | D1878G | T | 5633 | C | FRAS1 | N3879S | A | 12076 | G | GPC5 | G570R | G | 2080 | A | HHATL | D262N | C | 934 | T |
| FAM38A | R1766C | G | 5296 | A | FREM1 | V498M | C | 2276 | T | GPC5 | A104V | C | 683 | T | HHATL | F395V | A | 1333 | C |
| FAM38A | R936W | G | 2806 | A | FREM1 | V586A | A | 2541 | G | GPC5 | A81T | G | 613 | A | HHIP | R350I | G | 1704 | T |
| FAM38A | Y1137C | T | 3410 | C | FREM1 | F474S | A | 2205 | G | GPC5 | E161* | G | 853 | T | HHIP | G555R | G | 2318 | C |
| FAM38A | P58H | G | 173 | T | FREM1 | D1506G | T | 5301 | C | GPC6 | C254F | G | 1376 | T | HHIP | G538S | G | 2267 | A |
| FAM38A | P1279S | G | 3835 | A | FREM1 | D447G | T | 2124 | C | GPC6 | R126W | C | 991 | T | HHIP | A288T | G | 1517 | A |
| FAM38B | A153V | G | 598 | A | FREM1 | V1525M | C | 5357 | T | GPC6 | A477T | G | 2044 | A | HHIP | D513N | G | 2192 | A |
| FAM38B | I430T | A | 1429 | G | FREM1 | N1944H | T | 6614 | G | GPCPD1 | I607N | A | 2033 | T | HHIPL1 | R714H | G | 2206 | A |
| FAM38B | K114N | T | 482 | G | FREM1 | K1313N | C | 4723 | A | GPCPD1 | K528Q | T | 1795 | G | HHIPL1 | E98K | G | 357 | A |
| FAM38B | L623I | G | 2007 | T | FREM1 | E1077K | C | 4013 | T | GPCPD1 | - | C | 0 | A | HHIPL2 | K303N | C | 968 | A |
| FAM38B | I480M | A | 1580 | C | FREM2 | D1562Y | G | 4900 | T | GPD1L | E333K | G | 1198 | A | HHIPL2 | N480K | A | 1499 | C |
| FAM38B | - | C | 0 | A | FREM2 | Y902C | A | 2921 | G | GPD2 | P571H | C | 2084 | A | HHIPL2 | R155H | C | 523 | T |
| FAM3B | A151D | C | 518 | A | FREM2 | Q1486* | C | 4672 | T | GPD2 | R557H | G | 2042 | A | HHIPL2 | K303N | C | 968 | A |
| FAM3D | P37S | G | 420 | A | FREM2 | Y2706C | A | 8333 | G | GPD2 | R227L | G | 1052 | T | HHLA2 | S197N | G | 1004 | A |
| FAM3D | R24* | G | 381 | A | FREM2 | S3039G | A | 9331 | G | GPD2 | E666* | G | 2368 | T | HIAT1 | G178E | G | 669 | A |
| FAM40A | R785W | C | 2375 | T | FREM2 | F259L | C | 993 | G | GPER | V270I | G | 808 | A | HIAT1 | R218W | C | 788 | T |
| FAM40A | A80T | G | 260 | A | FREM2 | V484A | T | 1667 | C | GPHA2 | D122G | T | 420 | C | HIAT1 | G407E | G | 1356 | A |
| FAM43A | A247V | C | 1674 | T | FREM2 | K1540T | A | 4835 | C | GPHB5 | E72* | C | 214 | A | HIBCH | L195I | G | 878 | T |
| FAM43A | R321C | C | 1895 | T | FREM2 | E792Q | G | 2590 | C | GPHN | A454V | C | 2482 | T | HIC1 | R604H | G | 1811 | A |
| FAM43A | M167I | G | 1435 | A | FREM2 | A159V | C | 692 | T | GPHN | S604L | C | 2932 | T | HIC1 | P441L | C | 1322 | T |
| FAM43B | D273E | C | 1354 | G | FREM2 | W629L | G | 2102 | T | GPI | R457Q | G | 1370 | A | HIC1 | G597S | G | 1789 | A |
| FAM43B | D273E | C | 1354 | G | FREM2 | E2699* | G | 8311 | T | GPI | L287P | T | 860 | C | HIF1A | S15R | A | 308 | C |
| FAM43B | R51L | G | 687 | T | FREM2 | - | T | 0 | C | GPI | R428Q | G | 1283 | A | HIF1A | E682D | A | 2311 | C |
| FAM46B | T365M | G | 1260 | A | FREM2 | M434I | G | 1518 | T | GPKOW | R314Q | C | 1020 | T | HIF1A | I772L | A | 2579 | C |
| FAM46B | R105W | G | 479 | A | FREM2 | V2270G | T | 7025 | G | GPLD1 | - | T | 0 | A | HIF1AN | R338I | G | 1013 | T |
| FAM46B | R264H | C | 957 | T | FREM2 | R2278C | C | 7048 | T | GPLD1 | D712Y | C | 2245 | A | HIF1AN | A128D | C | 383 | A |
| FAM46B | R262Q | C | 951 | T | FREM3 | R209W | G | 625 | A | GPM6A | K111T | T | 378 | G | HIF3A | Q298H | G | 925 | T |
| FAM46B | R299C | G | 1061 | A | FREM3 | V971A | A | 2912 | G | GPM6A | I226L | T | 722 | G | HIF3A | L314M | C | 971 | A |
| HIF3A | E74V | A | 252 | T | INPP4B | Y401* | G | 1548 | T | KIAA0232 | R538* | C | 1991 | T | LAMA2 | R2710H | G | 8234 | A |
| HIF3A | E451* | G | 1382 | T | INPP4B | G548D | C | 1988 | T | KIAA0232 | F761C | T | 2661 | G | LAMA2 | S2263W | C | 6893 | G |
| HIF3A | E332D | G | 1027 | T | INPP5A | K115Q | A | 620 | C | KIAA0240 | D56V | A | 494 | T | LAMA3 | Y869* | C | 2693 | A |
| HIGD1A | P45S | G | 384 | A | INPP5A | V311A | T | 1209 | C | KIAA0240 | R811H | G | 2759 | A | LAMA3 | S1469N | G | 4492 | A |
| HIGD1A | V86A | A | 508 | G | INPP5B | G452* | C | 1448 | A | KIAA0284 | R1541H | G | 4850 | A | LAMA3 | E1903* | G | 5793 | T |
| HIGD1C | D52Y | G | 154 | T | INPP5B | M812I | C | 2530 | A | KIAA0284 | G1290D | G | 4097 | A | LAMA3 | A587S | G | 1845 | T |
| HINFP | E63* | G | 259 | T | INPP5B | L189S | A | 660 | G | KIAA0284 | R348H | G | 1271 | A | LAMA3 | A2099V | C | 6382 | T |
| HINT2 | L76R | A | 269 | C | INPP5D | A416T | G | 1246 | A | KIAA0284 | M588L | A | 1990 | T | LAMA3 | A3173V | C | 9604 | T |

| | | | | | | | | | | | | | | | | | | | |
|---|---|---|---|---|---|---|---|---|---|---|---|---|---|---|---|---|---|---|---|
| HIP1 | L779P | A | 2363 | G | INPP5D | V50I | G | 148 | A | KIAA0284 | T180M | C | 767 | T | LAMA3 | R1296W | C | 3972 | T |
| HIP1 | T50M | G | 176 | A | INPP5D | P1134L | C | 3401 | T | KIAA0284 | L134P | T | 629 | C | LAMA3 | R3189C | C | 9651 | T |
| HIP1R | A469V | C | 1465 | T | INPP5D | R552* | C | 1654 | T | KIAA0317 | R380Q | C | 1644 | T | LAMA3 | I1704T | T | 5197 | C |
| HIP1R | T839M | C | 2575 | T | INPP5E | A463V | G | 1791 | A | KIAA0317 | E285K | C | 1358 | T | LAMA3 | E1177K | G | 3615 | A |
| HIP1R | T983I | C | 3007 | T | INPP5F | E250K | G | 914 | A | KIAA0319 | R887Q | C | 3185 | T | LAMA3 | R2149W | C | 6531 | T |
| HIPK1 | V816I | G | 2446 | A | INPP5F | E353D | A | 1225 | C | KIAA0319L | V660M | C | 2213 | T | LAMA3 | F464L | C | 1478 | A |
| HIPK1 | S1079N | G | 3236 | A | INPP5F | K774N | G | 2488 | T | KIAA0319L | G243S | C | 962 | T | LAMA3 | S1148L | C | 3529 | T |
| HIPK1 | N220T | A | 659 | C | INPP5J | T529A | A | 1634 | G | KIAA0355 | S654L | C | 2820 | T | LAMA3 | C2462Y | G | 7471 | A |
| HIPK2 | V686I | C | 2151 | T | INPP5J | R424Q | G | 1320 | A | KIAA0355 | R849H | G | 3405 | A | LAMA4 | D1739Y | C | 5506 | A |
| HIPK3 | R38I | G | 418 | T | INPP5J | R346* | C | 1085 | T | KIAA0368 | T1440M | G | 4319 | A | LAMA4 | L1215P | A | 3935 | G |
| HIPK3 | A140T | G | 723 | A | INPP5J | R781C | C | 2390 | T | KIAA0368 | P1876L | G | 5627 | A | LAMA4 | A1058V | G | 3464 | A |
| HIPK3 | E1009A | A | 3331 | C | INPP5J | S622N | G | 1914 | A | KIAA0368 | A1320V | G | 3959 | A | LAMA4 | S967L | G | 3191 | A |
| HIPK4 | R335C | G | 1288 | A | INPP5J | D420N | G | 1307 | A | KIAA0368 | G358S | C | 1072 | T | LAMA4 | T801M | G | 2693 | A |
| HIPK4 | R603Q | C | 2093 | T | INPP5J | S501L | C | 1551 | T | KIAA0368 | W1092C | C | 3276 | A | LAMA5 | R1644H | C | 4998 | T |
| HIRA | Q405P | T | 1471 | G | INPP5K | - | C | 0 | A | KIAA0368 | S1998N | C | 5993 | T | LAMA5 | A2459T | C | 7442 | T |
| HIRA | A564T | C | 1947 | T | INPPL1 | A973V | C | 3122 | T | KIAA0368 | F1257C | A | 3770 | C | LAMA5 | G3089D | C | 9333 | T |
| HIRA | F28L | A | 339 | G | INPPL1 | E1132K | G | 3598 | A | KIAA0368 | L1166I | G | 3496 | T | LAMA5 | P792L | G | 2442 | A |
| HIRA | Q1001R | T | 3259 | C | INPPL1 | R347Q | G | 1244 | A | KIAA0368 | T752A | T | 2254 | C | LAMA5 | I1232T | A | 3762 | G |
| HIRA | R407M | C | 1477 | A | INPPL1 | K978N | G | 3138 | T | KIAA0406 | E810K | C | 2667 | T | LAMA5 | D104G | T | 378 | C |
| HIRIP3 | R266W | G | 1257 | A | INSC | A531T | G | 1637 | A | KIAA0415 | R1503H | G | 4508 | A | LAMA5 | M1650I | C | 5017 | T |
| HIRIP3 | R385H | C | 1615 | T | INSC | E13K | G | 83 | A | KIAA0415 | T913M | C | 2738 | T | LAMA5 | T703M | G | 2175 | A |
| HIST1H1B | A24T | C | 122 | T | INSM1 | A392T | G | 1321 | A | KIAA0415 | Q757* | C | 2269 | T | LAMA5 | G3344E | C | 10098 | T |
| HIST1H1B | K154R | T | 513 | C | INSM1 | C440Y | G | 1466 | A | KIAA0415 | S1029F | C | 3086 | T | LAMA5 | Y2877F | T | 8697 | A |
| HIST1H1C | K159N | C | 520 | A | INSM1 | T206A | A | 763 | G | KIAA0415 | T1439I | C | 4316 | T | LAMA5 | T657S | T | 2036 | A |
| HIST1H1C | K212N | C | 679 | G | INSM2 | M222T | T | 876 | C | KIAA0415 | P921L | C | 2762 | T | LAMA5 | L528F | G | 1649 | A |
| HIST1H1D | I45T | A | 189 | G | INSM2 | R358Q | G | 1284 | A | KIAA0430 | I1674S | A | 5228 | C | LAMA5 | G1474R | C | 4487 | T |
| HIST1H1E | - | T | 718 | C | INSR | G582D | C | 1854 | T | KIAA0430 | R534C | G | 1807 | A | LAMA5 | V1173M | C | 3584 | T |
| HIST1H1E | G91C | G | 331 | T | INSR | A1204S | C | 3719 | A | KIAA0430 | R434H | C | 1508 | T | LAMA5 | P2938L | G | 8880 | A |
| HIST1H1T | G167W | C | 542 | A | INSR | D893N | C | 2786 | T | KIAA0430 | S450* | G | 1556 | T | LAMA5 | V2046M | C | 6203 | T |
| HIST1H1T | G167R | C | 542 | T | INSR | E390* | C | 1277 | A | KIAA0430 | D82H | C | 451 | G | LAMA5 | R3565W | G | 10760 | A |
| HIST1H2AD | I79S | A | 236 | C | INSRR | V179M | C | 932 | T | KIAA0430 | G122C | C | 571 | A | LAMA5 | T315M | G | 1011 | A |
| HIST1H2AE | T60A | A | 216 | G | INSRR | T460I | G | 1776 | A | KIAA0430 | F495L | G | 1692 | T | LAMA5 | R2314Q | C | 7008 | T |
| HIST1H2AE | L98I | C | 330 | A | INSRR | R1255C | G | 4160 | A | KIAA0430 | G6E | C | 224 | T | LAMA5 | D1983E | G | 6016 | T |
| HIST1H2AL | H124Y | C | 469 | T | INSRR | Q1230* | G | 4085 | A | KIAA0467 | R823H | G | 2930 | A | LAMA5 | G558D | C | 1740 | T |
| HIST1H2BL | Y84C | T | 276 | C | INSRR | M966T | A | 3294 | G | KIAA0467 | R1980C | C | 6400 | T | LAMB1 | L454F | T | 1547 | G |
| HIST1H2BL | Y43C | T | 153 | C | INSRR | Q1051R | T | 3549 | C | KIAA0467 | K1463R | A | 4850 | G | LAMB1 | N130D | T | 573 | C |

| | | | | | | | | | | | | | | | | | | | |
|---|---|---|---|---|---|---|---|---|---|---|---|---|---|---|---|---|---|---|---|
| HIST1H2BL | R93T | C | 303 | G | INSRR | L833R | A | 2895 | C | KIAA0467 | R2042H | G | 6587 | A | LAMB2 | - | C | 0 | T |
| HIST1H2BN | K12N | G | 938 | T | INTS1 | - | C | 0 | T | KIAA0467 | - | G | 0 | T | LAMB2 | V526M | C | 1741 | T |
| HIST1H2BO | M63I | G | 227 | T | INTS1 | L1692M | G | 5074 | T | KIAA0467 | R2451C | C | 7813 | T | LAMB2 | R246W | G | 901 | A |
| HIST1H3B | F105L | A | 313 | G | INTS1 | R1594C | G | 4780 | A | KIAA0467 | R1169H | G | 3968 | A | LAMB2 | Q945R | T | 2999 | C |
| HIST1H3B | T33A | T | 97 | C | INTS1 | A1583T | C | 4747 | T | KIAA0467 | T1435A | A | 4765 | G | LAMB2 | R107H | C | 485 | T |
| HIST1H3D | P44H | G | 528 | T | INTS1 | P152L | G | 455 | A | KIAA0467 | R1579H | G | 5198 | A | LAMB2 | K87N | C | 426 | A |
| HIST1H3E | P67Q | C | 200 | A | INTS1 | R205C | G | 613 | A | KIAA0467 | P771L | C | 2774 | T | LAMB2 | R853Q | C | 2723 | T |
| HIST1H3G | E106K | C | 747 | T | INTS1 | M1453I | C | 4359 | T | KIAA0494 | R4H | C | 988 | T | LAMB3 | R430C | G | 1678 | A |
| HIST1H3J | F68L | A | 202 | G | INTS10 | K539T | A | 1843 | C | KIAA0513 | V103M | G | 527 | A | LAMB3 | R1135W | G | 3793 | A |
| HIST1H3J | A32V | G | 95 | A | INTS2 | R741Q | C | 2222 | T | KIAA0528 | E264* | C | 909 | A | LAMB3 | R439W | G | 1705 | A |
| HIST1H4D | G10D | C | 29 | T | INTS2 | L74I | G | 220 | T | KIAA0528 | S606P | A | 1935 | G | LAMB3 | T1148S | T | 3832 | A |
| HIST1H4G | I67S | A | 200 | C | INTS2 | R140C | G | 418 | A | KIAA0556 | G761S | G | 2305 | A | LAMB3 | A260T | C | 1168 | T |
| HIST1H4I | A16V | C | 59 | T | INTS3 | A951V | C | 3420 | T | KIAA0556 | L629P | T | 1910 | C | LAMB3 | Y362C | T | 1475 | C |
| HIST1H4I | K45Q | A | 145 | C | INTS3 | M131T | T | 960 | C | KIAA0556 | G1238D | G | 3737 | A | LAMB3 | R367H | C | 1490 | T |
| HIST1H4L | Q28R | T | 84 | C | INTS3 | R440H | G | 1887 | A | KIAA0556 | D1576N | G | 4750 | A | LAMB3 | R367C | G | 1489 | A |
| HIST2H2AB | V115I | C | 343 | T | INTS4 | F348V | A | 1070 | C | KIAA0556 | D1122Y | G | 3388 | T | LAMB3 | E858D | T | 2964 | G |
| HIST2H2BE | V42A | A | 167 | G | INTS4 | R445* | G | 1361 | A | KIAA0562 | V10I | C | 353 | T | LAMB3 | R2S | T | 396 | A |
| HIST2H2BF | S15P | A | 53 | G | INTS5 | A884T | C | 2703 | T | KIAA0562 | R409Q | C | 1551 | T | LAMB4 | R1624Q | C | 4951 | T |
| HIST3H3 | G35S | C | 103 | T | INTS5 | L301M | G | 954 | T | KIAA0562 | R222Q | C | 990 | T | LAMB4 | Q1194H | T | 3662 | A |
| HIST3H3 | T46M | G | 137 | A | INTS5 | L502F | G | 1557 | A | KIAA0562 | S20G | T | 383 | C | LAMB4 | L1685P | A | 5134 | G |
| HIVEP1 | R3Q | G | 340 | A | INTS6 | E603* | C | 2280 | A | KIAA0564 | Y1824* | G | 5541 | T | LAMB4 | R275H | C | 904 | T |
| HIVEP1 | P435L | C | 1636 | T | INTS7 | Q258* | G | 877 | A | KIAA0564 | R116* | G | 415 | A | LAMB4 | L1489V | A | 4545 | C |
| HIVEP1 | V2705M | G | 8445 | A | INTS7 | D933G | T | 2903 | C | KIAA0564 | D1073N | C | 3286 | T | LAMB4 | T41M | G | 202 | A |
| HIVEP1 | P769L | C | 2638 | T | INTS7 | V787G | A | 2465 | C | KIAA0564 | - | C | 0 | T | LAMC1 | V911M | G | 2986 | A |
| HIVEP1 | R615M | G | 2176 | T | INTS8 | L741S | T | 2348 | C | KIAA0564 | R1714H | C | 5210 | T | LAMC1 | G1261S | G | 4036 | A |
| HIVEP1 | N1436S | A | 4639 | G | INTS8 | - | A | 0 | G | KIAA0564 | K1628N | C | 4953 | A | LAMC1 | P327S | C | 1234 | T |
| HIVEP1 | S1785L | C | 5686 | T | INTS8 | A991T | G | 3097 | A | KIAA0564 | F1825C | A | 5543 | C | LAMC1 | R1011C | C | 3286 | T |
| HIVEP1 | S1919Y | C | 6088 | A | INTS8 | K779Q | A | 2461 | C | KIAA0564 | L1391V | A | 4240 | C | LAMC1 | A125T | G | 628 | A |
| HIVEP2 | S2052Y | G | 6795 | T | INTS8 | K984T | A | 3077 | C | KIAA0564 | F915L | G | 2814 | T | LAMC1 | Q611R | A | 2087 | G |
| HIVEP2 | R1028* | G | 3722 | A | INTS9 | Q437* | G | 1608 | A | KIAA0564 | R664Q | C | 2060 | T | LAMC2 | S848R | T | 2609 | A |
| HIVEP2 | R1028* | G | 3722 | A | INTS9 | A109V | G | 625 | A | KIAA0564 | Q618H | T | 1923 | G | LAMC2 | L1030M | T | 3153 | A |
| HIVEP2 | R1028* | G | 3722 | A | INTS9 | V617I | C | 2148 | T | KIAA0586 | L803I | C | 2634 | A | LAMC2 | R717W | C | 2214 | T |
| HIVEP2 | V267A | A | 1440 | G | INTU | G880C | G | 2741 | T | KIAA0649 | E1164D | G | 4041 | T | LAMC3 | A1244T | G | 3828 | A |
| HIVEP2 | S1253L | G | 4398 | A | INVS | V722I | G | 2349 | A | KIAA0649 | A1190T | G | 4117 | A | LAMC3 | C866F | G | 2695 | T |
| HIVEP2 | S1354Y | G | 4701 | T | INVS | W155L | G | 649 | T | KIAA0664 | R1264W | G | 3790 | A | LAMC3 | D1493G | A | 4576 | G |
| HIVEP3 | P153S | G | 1343 | A | IP6K1 | G262D | C | 1087 | T | KIAA0664 | D910N | C | 2728 | T | LAMC3 | P382S | C | 1242 | T |

| | | | | | | | | | | | | | | | | | | | |
|---|---|---|---|---|---|---|---|---|---|---|---|---|---|---|---|---|---|---|---|
| HIVEP3 | S576N | C | 2613 | T | IP6K1 | R316W | G | 1248 | A | KIAA0664 | Q213R | T | 638 | C | LAMC3 | E509G | A | 1624 | G |
| HIVEP3 | R990G | T | 3854 | C | IP6K2 | R156H | C | 714 | T | KIAA0664 | R276H | C | 827 | T | LAMC3 | R1073W | C | 3315 | T |
| HIVEP3 | H180P | T | 1425 | G | IP6K2 | - | T | 0 | G | KIAA0748 | R184Q | C | 684 | T | LAMC3 | H1131Y | C | 3489 | T |
| HIVEP3 | V461L | C | 2267 | A | IP6K3 | A240V | G | 1255 | A | KIAA0748 | R474C | G | 1553 | A | LAMC3 | A318T | G | 1050 | A |
| HIVEP3 | R18W | G | 938 | A | IP6K3 | R168M | C | 1039 | A | KIAA0753 | R50Q | C | 508 | T | LAMP1 | V285I | G | 1047 | A |
| HIVEP3 | R1762S | G | 6170 | T | IPCEF1 | A139P | C | 571 | G | KIAA0776 | R535C | C | 1669 | T | LAMP2 | - | T | 0 | G |
| HIVEP3 | D376N | C | 2012 | T | IPCEF1 | N436H | T | 1462 | G | KIAA0776 | F368C | T | 1169 | G | LAMP2 | R144I | C | 611 | A |
| HJURP | A533T | C | 1663 | T | IPCEF1 | K51R | T | 308 | C | KIAA0776 | E774* | G | 2386 | T | LAMP2 | R293Q | C | 1058 | T |
| HJURP | I411M | A | 1299 | C | IPCEF1 | Q409H | C | 1383 | A | KIAA0802 | R1573W | C | 4717 | T | LAMP2 | K161N | C | 663 | A |
| HJURP | G333W | C | 1063 | A | IPMK | R411Q | C | 1555 | T | KIAA0802 | A1203V | C | 3608 | T | LANCL2 | G372V | G | 1693 | T |
| HK1 | G449R | G | 1345 | A | IPO11 | R533* | C | 1727 | T | KIAA0802 | A848T | G | 2542 | A | LANCL2 | C408Y | G | 1801 | A |
| HK1 | G483C | G | 1447 | T | IPO11 | V570A | T | 1839 | C | KIAA0802 | V1518M | G | 4552 | A | LANCL3 | P314L | C | 1160 | T |
| HK2 | R42Q | G | 725 | A | IPO11 | F548L | C | 1774 | A | KIAA0802 | L505V | T | 1513 | G | LANCL3 | K312N | G | 1155 | T |
| HK2 | S155L | C | 1064 | T | IPO11 | E741D | A | 2353 | C | KIAA0802 | L1380I | C | 4138 | A | LANCL3 | Y316S | A | 1166 | C |
| HK2 | K146N | G | 1038 | T | IPO13 | A18S | G | 714 | T | KIAA0831 | G420R | C | 1294 | T | LAPTM4B | A174V | C | 521 | T |
| HK3 | V439I | C | 1407 | T | IPO13 | Y275H | T | 1485 | C | KIAA0831 | N169T | T | 542 | G | LARGE | Q691H | C | 2645 | A |
| HK3 | R730C | G | 2280 | A | IPO13 | T508P | A | 2184 | C | KIAA0895 | A282T | C | 895 | T | LARGE | R308W | G | 1494 | A |
| HK3 | P170H | G | 601 | T | IPO13 | R863H | G | 3250 | A | KIAA0895 | D380Y | C | 1189 | A | LARGE | V341I | C | 1593 | T |
| HK3 | R475C | G | 1515 | A | IPO13 | R321Q | G | 1624 | A | KIAA0895 | R285Q | C | 905 | T | LARGE | M223K | A | 1240 | T |
| HK3 | E307D | C | 1013 | A | IPO4 | R953* | G | 2867 | A | KIAA0895 | A282T | C | 895 | T | LARP1 | V135A | T | 404 | C |
| HKDC1 | P520L | C | 1692 | T | IPO4 | R434M | C | 1311 | A | KIAA0895 | R339* | G | 1066 | A | LARP1 | R366* | C | 1096 | T |
| HKDC1 | A582T | G | 1877 | A | IPO4 | A856V | G | 2577 | A | KIAA0895 | F56L | G | 219 | T | LARP1 | R285H | G | 854 | A |
| HKDC1 | G151D | G | 585 | A | IPO4 | A262V | G | 795 | A | KIAA0913 | A1321T | G | 4178 | A | LARP1 | R956* | C | 2866 | T |
| HKDC1 | I565T | T | 1827 | C | IPO5 | A1107T | G | 3499 | A | KIAA0913 | A1369T | G | 4322 | A | LARP1 | F974Y | T | 2921 | A |
| HKDC1 | L484R | T | 1584 | G | IPO5 | R294H | G | 1061 | A | KIAA0913 | Y1241C | A | 3939 | G | LARP1B | R70H | G | 420 | A |
| HKDC1 | T216A | A | 779 | G | IPO5 | V441M | G | 1501 | A | KIAA0922 | A504V | C | 1560 | T | LARP1B | R119Q | G | 567 | A |
| HKDC1 | D471V | A | 1545 | T | IPO5 | F456C | T | 1547 | G | KIAA0922 | N791S | A | 2421 | G | LARP1B | E410* | G | 1439 | T |
| HKR1 | R504* | C | 1779 | T | IPO5 | E577* | G | 1909 | T | KIAA0922 | E272* | G | 863 | T | LARP1B | K693M | A | 2289 | T |
| HKR1 | S106L | C | 586 | T | IPO5 | S790Y | C | 2549 | A | KIAA0947 | T1836A | A | 5728 | G | LARP4 | - | T | 0 | C |
| HKR1 | K523T | A | 1837 | C | IPO7 | D15G | A | 186 | G | KIAA0947 | V1947I | G | 6061 | A | LARP4 | N657H | A | 2113 | C |
| HKR1 | R461M | G | 1651 | T | IPO7 | H816N | C | 2588 | A | KIAA0947 | A975V | C | 3146 | T | LARP4B | F276L | A | 869 | T |
| HLA-DRB5 | G231A | C | 755 | G | IPO8 | R771* | G | 2481 | A | KIAA0947 | T550A | A | 1870 | G | LARP4B | - | A | 0 | G |
| HLCS | G57S | C | 640 | T | IPO8 | E891A | T | 2842 | G | KIAA0947 | D2021Y | G | 6283 | T | LARP4B | I558T | A | 1714 | G |
| HLCS | D571N | C | 2182 | T | IPO8 | K411Q | T | 1401 | G | KIAA0947 | A976V | C | 3149 | T | LARP6 | E79D | C | 308 | A |
| HLF | L8H | T | 548 | A | IPO8 | V257L | C | 939 | G | KIAA1009 | R1251C | G | 3866 | A | LARP7 | I322N | T | 1268 | A |
| HLTF | E396* | C | 1380 | A | IPO8 | E650D | T | 2120 | G | KIAA1009 | S544L | G | 1746 | A | LARS2 | E29K | G | 347 | A |
| HLTF | R27C | G | 273 | A | IPO9 | T759I | C | 2345 | T | KIAA1009 | E179D | T | 652 | G | LARS2 | A496T | G | 1748 | A |
| HLTF | A427V | G | 1474 | A | IPO9 | R787C | C | 2428 | T | KIAA1012 | G864* | C | 2926 | A | LARS2 | K73T | A | 480 | C |
| HLTF | T346I | G | 1231 | A | IPO9 | R935C | C | 2872 | T | KIAA1012 | S973P | A | 3253 | G | LASS2 | R53Q | C | 544 | T |

| | | | | | | | | | | | | | | | | | | | |
|---|---|---|---|---|---|---|---|---|---|---|---|---|---|---|---|---|---|---|---|
| HLX | G431S | G | 1748 | A | IPP | R333Q | C | 1101 | T | KIAA1012 | F1198L | G | 3930 | T | LASS2 | A152V | G | 841 | A |
| HLX | G459A | G | 1833 | C | IPP | R227* | G | 782 | A | KIAA1012 | R172Q | C | 851 | T | LASS3 | E95D | C | 943 | A |
| HLX | L237P | T | 1167 | C | IPPK | L237P | A | 987 | G | KIAA1024 | R741C | C | 2296 | T | LASS3 | R132Q | C | 1053 | T |
| HMCN1 | R383Q | G | 1377 | A | IQCA1 | L399I | G | 1195 | T | KIAA1024 | V73A | T | 293 | C | LASS3 | E95D | C | 943 | A |
| HMCN1 | E3704* | G | 11339 | T | IQCA1 | R436* | G | 1306 | A | KIAA1024 | R816W | C | 2521 | T | LASS6 | R121C | C | 561 | T |
| HMCN1 | H4643N | C | 14156 | A | IQCA1 | A171V | G | 512 | A | KIAA1024 | S124L | C | 446 | T | LAT | C65Y | G | 230 | A |
| HMCN1 | V720I | G | 2387 | A | IQCA1 | K479E | T | 1435 | C | KIAA1024 | R617H | G | 1925 | A | LAT | P112S | C | 370 | T |
| HMCN1 | G5344* | G | 16259 | T | IQCA1 | L397I | G | 1189 | T | KIAA1024 | V73A | T | 293 | C | LAT | L169P | T | 542 | C |
| HMCN1 | A3972V | C | 12144 | T | IQCE | Q302H | G | 1090 | T | KIAA1024 | K578N | G | 1809 | T | LAT | - | T | 0 | C |
| HMCN1 | T1647M | C | 5169 | T | IQCF2 | Q51P | A | 181 | C | KIAA1024 | K458N | G | 1449 | C | LAT2 | G105E | G | 1036 | A |
| HMCN1 | V3616M | G | 11075 | A | IQCF3 | V49A | T | 1311 | C | KIAA1024 | S464I | G | 1466 | T | LATS2 | R415W | G | 1649 | A |
| HMCN1 | T2549I | C | 7875 | T | IQCF5 | E148* | C | 495 | A | KIAA1024 | K722N | A | 2241 | C | LATS2 | P208L | G | 1029 | A |
| HMCN1 | P1950S | C | 6077 | T | IQCF5 | R84H | C | 304 | T | KIAA1024 | N728D | A | 2257 | G | LATS2 | R391H | C | 1578 | T |
| HMCN1 | P4846A | C | 14765 | G | IQCF5 | E11* | C | 84 | A | KIAA1024 | S855P | T | 2638 | C | LBP | E199K | G | 756 | A |
| HMCN1 | E2697G | A | 8319 | G | IQCG | I168M | A | 929 | C | KIAA1033 | R828Q | G | 2570 | A | LBP | F62S | T | 346 | C |
| HMCN1 | L2715I | C | 8372 | A | IQCH | L15F | A | 111 | C | KIAA1033 | R982Q | G | 3032 | A | LBP | R322* | C | 1125 | T |
| HMCN1 | K3517T | A | 10779 | C | IQCH | C865Y | G | 2660 | A | KIAA1033 | A126T | G | 463 | A | LBX1 | A92T | C | 419 | T |
| HMCN1 | E798* | G | 2621 | T | IQGAP1 | A1511T | G | 4655 | A | KIAA1033 | - | G | 0 | T | LBXCOR1 | S55F | C | 164 | T |
| HMCN1 | - | G | 0 | T | IQGAP1 | P1157L | C | 3594 | T | KIAA1033 | V582G | T | 1832 | G | LBXCOR1 | R876H | G | 2627 | A |
| HMCN1 | Y2664C | A | 8220 | G | IQGAP1 | P1049H | C | 3270 | A | KIAA1045 | I34V | A | 182 | G | LBXCOR1 | S294L | C | 881 | T |
| HMCN1 | K3141N | A | 9652 | C | IQGAP1 | R803H | G | 2532 | A | KIAA1045 | R161C | C | 563 | T | LBXCOR1 | - | T | 0 | C |
| HMCN1 | V3247G | T | 9969 | G | IQGAP2 | E347K | G | 1336 | A | KIAA1107 | F1016V | T | 3046 | G | LBXCOR1 | F638C | T | 1913 | G |
| HMCN1 | E4391* | G | 13400 | T | IQGAP2 | A398T | G | 1489 | A | KIAA1107 | N775H | A | 2323 | C | LBXCOR1 | H810R | A | 2429 | G |
| HMCN1 | F4892V | T | 14903 | G | IQGAP3 | P1533H | G | 4609 | T | KIAA1107 | K928T | A | 2783 | C | LCA5 | - | C | 0 | T |
| HMCN1 | T1647M | C | 5169 | T | IQGAP3 | T1289M | G | 3877 | A | KIAA1107 | K1195Q | A | 3583 | C | LCA5 | E389* | C | 1600 | A |
| HMG20A | S322I | G | 1323 | T | IQSEC1 | A644T | C | 2033 | T | KIAA1109 | L3340M | C | 10391 | A | LCA5L | F15L | G | 410 | T |
| HMG20A | K21N | G | 421 | T | IQSEC1 | R870L | C | 2712 | A | KIAA1109 | R994H | G | 3354 | A | LCA5L | S597R | A | 2156 | T |
| HMG20A | R98* | C | 650 | T | IQSEC1 | A130V | G | 492 | A | KIAA1109 | Q1340R | A | 4392 | G | LCA5L | T268R | G | 1168 | C |
| HMG20A | R339* | C | 1373 | T | IQSEC1 | R1055C | G | 3266 | A | KIAA1109 | R4889* | C | 15038 | T | LCAT | T37M | G | 520 | A |
| HMGCL | T292M | G | 919 | A | IQSEC2 | E569D | C | 1908 | A | KIAA1109 | S4544R | A | 14003 | C | LCE1B | S78G | A | 318 | G |
| HMGCR | R641H | G | 2234 | A | IQSEC2 | R85C | G | 454 | A | KIAA1109 | R147C | C | 812 | T | LCE1C | M1V | T | 52 | C |
| HMGCR | R496Q | G | 1799 | A | IQSEC3 | L974P | T | 2921 | C | KIAA1109 | A2522V | C | 7938 | T | LCE1C | Q4H | C | 63 | A |
| HMGCS2 | R506C | G | 1566 | A | IQSEC3 | A288S | G | 862 | T | KIAA1109 | T3355A | A | 10436 | G | LCE1F | G116C | G | 346 | T |

| | | | | | | | | | | | | | | | | | | | |
|---|---|---|---|---|---|---|---|---|---|---|---|---|---|---|---|---|---|---|---|
| HMGN5 | E240* | C | 1047 | A | IQSEC3 | P138S | C | 412 | T | KIAA1109 | S3828F | C | 11856 | T | LCE1F | R89* | A | 265 | T |
| HMGXB3 | G1317S | G | 3949 | A | IQSEC3 | R316W | C | 946 | T | KIAA1109 | - | T | 0 | G | LCE1F | G53V | G | 158 | T |
| HMGXB4 | - | A | 0 | G | IQUB | E46* | C | 713 | A | KIAA1109 | H4659R | A | 14349 | G | LCE3A | A86V | G | 257 | A |
| HMHA1 | R165W | C | 493 | T | IQUB | R735H | C | 2781 | T | KIAA1109 | R211H | G | 1005 | A | LCE3D | R67M | C | 257 | A |
| HMHA1 | A521V | C | 1562 | T | IQUB | L418R | A | 1830 | C | KIAA1109 | W1067C | G | 3574 | C | LCE4A | S27P | T | 335 | C |
| HMHA1 | A205S | G | 613 | T | IQUB | R563I | C | 2265 | A | KIAA1109 | A3784P | G | 11723 | C | LCK | R219Q | G | 777 | A |
| HMHA1 | N830K | C | 2490 | A | IQUB | D513Y | C | 2114 | A | KIAA1109 | E314K | G | 1313 | A | LCK | S451P | T | 1472 | C |
| HMHA1 | R155C | C | 463 | T | IRAK1 | T625A | T | 2041 | C | KIAA1109 | K1417N | A | 4624 | C | LCK | - | G | 0 | T |
| HMOX1 | R85C | C | 592 | T | IRAK1 | T114A | T | 508 | C | KIAA1109 | S2657* | C | 8343 | A | LCK | R417C | C | 1370 | T |
| HMOX1 | R262S | C | 1123 | A | IRAK2 | V446M | G | 1426 | A | KIAA1109 | H2683N | C | 8420 | A | LCLAT1 | A253T | G | 966 | A |
| HMX1 | R196Q | C | 587 | T | IRAK2 | P95L | C | 374 | T | KIAA1147 | A149T | C | 445 | T | LCLAT1 | A110V | C | 538 | T |
| HMX2 | A42T | G | 381 | A | IRAK2 | K122T | A | 455 | C | KIAA1147 | N451S | T | 1352 | C | LCMT1 | G289E | G | 866 | A |
| HMX2 | W40* | G | 377 | A | IRAK2 | F284L | C | 942 | A | KIAA1161 | P526S | G | 1802 | A | LCMT2 | S336L | G | 1211 | A |
| HMX2 | A183T | G | 804 | A | IRAK3 | A277T | G | 931 | A | KIAA1161 | R264C | G | 1016 | A | LCN15 | M75V | T | 248 | C |
| HMX3 | Y109C | A | 326 | G | IRAK3 | S574R | C | 1824 | A | KIAA1161 | A460V | G | 1605 | A | LCN8 | P322L | G | 1618 | A |
| HN1 | - | A | 0 | G | IRAK3 | - | G | 0 | T | KIAA1161 | N364D | T | 1316 | C | LCN9 | A143T | G | 427 | A |
| HNF1A | P153H | C | 684 | A | IRAK4 | R183* | C | 629 | T | KIAA1161 | A645T | C | 2159 | T | LCN9 | G97V | G | 290 | T |
| HNF1B | R235W | G | 1065 | A | IRAK4 | R12C | C | 116 | T | KIAA1161 | R601C | G | 2027 | A | LCOR | A287V | C | 1381 | T |
| HNF1B | M402I | C | 1568 | A | IRAK4 | F168C | T | 585 | G | KIAA1161 | R528W | G | 1808 | A | LCOR | P34L | C | 622 | T |
| HNF4A | L84M | C | 339 | A | IREB2 | T91I | C | 421 | T | KIAA1161 | R374C | G | 1346 | A | LCOR | K341N | G | 1544 | T |
| HNRNPA2 B1 | S102A | A | 473 | C | IRF2 | R9C | G | 233 | A | KIAA1199 | P1045L | C | 3422 | T | LCORL | S272T | C | 826 | G |
| HNRNPA3 | N195S | A | 821 | G | IRF3 | D102V | T | 687 | A | KIAA1199 | T1107M | C | 3608 | T | LCORL | T360I | G | 1090 | A |
| HNRNPAB | - | T | 1254 | G | IRF4 | A222D | C | 791 | A | KIAA1199 | R218C | C | 940 | T | LCORL | T568A | T | 1713 | C |
| HNRNPCL 1 | V55I | C | 389 | T | IRF4 | A403T | G | 1333 | A | KIAA1199 | V1174I | G | 3808 | A | LCORL | R141Q | C | 433 | T |
| HNRNPF | R116* | G | 695 | A | IRF6 | A77G | G | 403 | C | KIAA1199 | P884T | C | 2938 | A | LCP1 | Q497* | G | 1727 | A |
| HNRNPF | T35M | G | 453 | A | IRF6 | Q359P | T | 1249 | G | KIAA1199 | R242Q | G | 1013 | A | LCP1 | T399A | T | 1433 | C |
| HNRNPF | R81W | G | 590 | A | IRF7 | P184H | G | 961 | T | KIAA1199 | K342N | A | 1314 | C | LCP1 | D260A | T | 1017 | G |
| HNRNPH1 | G8R | C | 57 | T | IRF8 | R11* | C | 453 | T | KIAA1199 | N680S | A | 2327 | G | LCP1 | V615M | C | 2081 | T |
| HNRNPH1 | R192I | C | 610 | A | IRF8 | L10I | C | 450 | A | KIAA1210 | D1607N | C | 4819 | T | LCP2 | - | C | 0 | T |
| HNRNPH1 | K35N | T | 140 | G | IRF8 | L14P | T | 463 | C | KIAA1210 | R1141W | G | 3421 | A | LCP2 | I513T | A | 1745 | G |
| HNRNPK | R86C | G | 465 | A | IRGC | L326M | C | 1175 | A | KIAA1210 | S498R | T | 1492 | G | LCT | G754R | C | 2271 | T |
| HNRNPK | R69C | G | 414 | A | IRGC | R151H | G | 651 | A | KIAA1210 | R209W | G | 625 | A | LCT | V397M | C | 1200 | T |
| HNRNPL | G462D | C | 1396 | T | IRGC | G375D | G | 1323 | A | KIAA1211 | R461Q | G | 1773 | A | LCT | G1561D | C | 4693 | T |
| HNRNPM | V724I | G | 2402 | A | IRGC | R28H | G | 282 | A | KIAA1211 | E272D | G | 1207 | T | LCT | D616G | T | 1858 | C |
| HNRNPM | E582K | G | 1976 | A | IRGQ | W502R | A | 1660 | T | KIAA1211 | L1067R | T | 3591 | G | LCT | A694T | C | 2091 | T |
| HNRNPM | R485C | C | 1685 | T | IRGQ | R219H | C | 812 | T | KIAA1211 | E330* | G | 1379 | T | LCT | P342S | G | 1035 | A |
| HNRNPM | L320M | C | 1190 | A | IRGQ | S489I | C | 1622 | A | KIAA1211 | A157T | G | 860 | A | LCT | K970R | T | 2920 | C |
| HNRNPM | R60H | G | 411 | A | IRGQ | E371D | C | 1269 | A | KIAA1211 | A1035V | C | 3495 | T | LCT | A694S | C | 2091 | A |

| | | | | | | | | | | | | | | | | | | | |
|---|---|---|---|---|---|---|---|---|---|---|---|---|---|---|---|---|---|---|---|
| HNRNPR | A604T | C | 1969 | T | IRS1 | G669S | C | 3026 | T | KIAA1211 | A568T | G | 2093 | A | LCT | R1916Q | C | 5758 | T |
| HNRNPR | G203S | C | 766 | T | IRS1 | S383L | G | 2169 | A | KIAA1211 | L75M | C | 614 | A | LCT | S991Y | G | 2983 | T |
| HNRNPU | S420L | G | 1423 | A | IRS1 | P929S | G | 3806 | A | KIAA1211 | V88I | G | 653 | A | LCT | S990R | G | 2981 | T |
| HNRNPU | R727H | C | 2344 | T | IRS1 | A1228V | G | 4704 | A | KIAA1211 | S888N | G | 3054 | A | LCT | I316S | A | 958 | C |
| HNRNPU | G245D | C | 898 | T | IRS1 | K177N | C | 1552 | A | KIAA1211 | L549I | C | 2036 | A | LCT | Y206D | A | 627 | C |
| HNRNPU | R572Q | C | 1879 | T | IRS2 | S770P | A | 2823 | G | KIAA1211 | A501V | C | 1893 | T | LCTL | A175T | C | 655 | T |
| HNRNPU | Q545H | T | 1799 | G | IRS2 | A563V | G | 2203 | A | KIAA1217 | R885H | G | 2684 | A | LCTL | T82M | G | 377 | A |
| HNRNPU | N294T | T | 1045 | G | IRS2 | A293V | G | 1393 | A | KIAA1217 | R1188Q | G | 3593 | A | LDB2 | R38I | C | 437 | A |
| HNRNPUL 1 | R138H | G | 702 | A | IRS4 | P33L | G | 175 | A | KIAA1217 | A233T | G | 727 | A | LDB3 | I265T | T | 817 | C |
| HNRNPUL 2 | R700Q | C | 2292 | T | IRS4 | E271G | T | 889 | C | KIAA1217 | G402R | G | 1234 | A | LDB3 | R546* | C | 1659 | T |
| HNRPA1L3 | R146K | G | 541 | A | IRS4 | T620A | T | 1935 | C | KIAA1217 | R818W | C | 2482 | T | LDB3 | S550N | G | 1672 | A |
| HNRPA1L3 | G110D | G | 433 | A | IRX1 | A110S | G | 380 | T | KIAA1217 | P1173S | C | 3547 | T | LDB3 | R148L | G | 466 | T |
| HNRPDL | H60N | G | 354 | T | IRX2 | A356E | G | 1316 | T | KIAA1217 | I1445T | T | 4364 | C | LDB3 | L43F | C | 150 | T |
| HOMER1 | T341A | T | 2464 | C | IRX4 | R61H | C | 301 | T | KIAA1217 | R759H | G | 2306 | A | LDB3 | P187L | C | 583 | T |
| HOMER2 | T117M | G | 536 | A | IRX4 | V289I | C | 984 | T | KIAA1217 | S229L | C | 716 | T | LDB3 | T350M | C | 1072 | T |
| HOOK2 | Y177H | A | 700 | G | IRX4 | N195S | T | 703 | C | KIAA1217 | S1554L | C | 4691 | T | LDB3 | L568M | C | 1725 | A |
| HOOK2 | S665I | C | 2165 | A | IRX5 | G107* | G | 731 | T | KIAA1239 | L706V | T | 2964 | G | LDB3 | A683V | C | 2071 | T |
| HOOK2 | R714C | G | 2311 | A | IRX6 | T154A | A | 1792 | G | KIAA1244 | R923Q | G | 2934 | A | LDHA | R106H | G | 590 | A |
| HOOK2 | A666V | G | 2168 | A | IRX6 | S117N | G | 1682 | A | KIAA1244 | L163P | T | 654 | C | LDHAL6A | G60D | G | 440 | A |
| HOOK2 | L358P | A | 1244 | G | ISCA1 | F36Y | A | 107 | T | KIAA1257 | P155L | G | 632 | A | LDHB | A35V | G | 202 | A |
| HOOK2 | R254G | T | 931 | C | ISG15 | G37S | G | 260 | A | KIAA1257 | T331P | T | 1159 | G | LDHB | G98* | C | 390 | A |
| HOOK2 | D188G | T | 734 | C | ISL1 | A267V | C | 1348 | T | KIAA1257 | E287D | C | 1029 | A | LDHB | G30E | C | 187 | T |
| HOOK2 | E126D | C | 549 | A | ISL1 | I72F | A | 762 | T | KIAA1267 | S1070I | C | 3294 | A | LDHB | E62* | C | 282 | A |
| HOOK3 | T36M | C | 349 | T | ISL1 | R112H | G | 883 | A | KIAA1267 | E908K | C | 2807 | T | LDHD | R198Q | C | 640 | T |
| HOOK3 | A263V | C | 1030 | T | ISL2 | V234A | T | 861 | C | KIAA1267 | S342N | C | 1110 | T | LDLR | S110T | T | 496 | A |
| HOOK3 | Q133H | A | 641 | C | ISLR | D140N | G | 775 | A | KIAA1267 | Q841* | G | 2606 | A | LDLR | N169S | A | 674 | G |
| HOOK3 | K387N | G | 1403 | T | ISLR | S282T | G | 1202 | C | KIAA1267 | A3V | G | 93 | A | LDLR | R26I | G | 245 | T |
| HORMAD1 | N71D | T | 317 | C | ISLR | L134M | C | 757 | A | KIAA1267 | R64Q | C | 276 | T | LDLRAD2 | R47C | C | 326 | T |
| HORMAD2 | R276I | G | 1182 | T | ISLR | D35N | G | 460 | A | KIAA1274 | D502Y | G | 1785 | T | LDLRAD2 | L148M | C | 629 | A |
| HOXA1 | E305K | C | 975 | T | ISLR2 | P317L | C | 1735 | T | KIAA1274 | E384K | G | 1431 | A | LDLRAD2 | L148M | C | 629 | A |
| HOXA13 | R323G | T | 996 | C | ISLR2 | V352I | G | 1839 | A | KIAA1274 | V365A | T | 1375 | C | LDLRAD3 | A250V | C | 770 | T |
| HOXA3 | R73C | G | 417 | A | ISLR2 | A314V | C | 1726 | T | KIAA1324 | E490K | G | 1604 | A | LDLRAD3 | D289N | G | 886 | A |
| HOXA3 | A98T | C | 492 | T | ISLR2 | R466W | C | 2181 | T | KIAA1324 | R992M | G | 3111 | T | LECT1 | I220T | A | 738 | G |
| HOXA5 | R222H | C | 902 | T | ISM1 | R258H | G | 779 | A | KIAA1324 | R883Q | G | 2784 | A | LECT1 | V75A | A | 303 | G |
| HOXA5 | P14S | G | 277 | A | ISM1 | D389N | G | 1171 | A | KIAA1324 | T919A | A | 2891 | G | LEF1 | H90P | T | 924 | G |
| HOXA5 | N245H | T | 970 | G | ISM1 | R366Q | G | 1103 | A | KIAA1324L | E180* | C | 724 | A | LEFTY1 | C264R | A | 870 | G |
| HOXA7 | C29* | G | 219 | T | ISM2 | T302I | G | 962 | A | KIAA1324L | E114A | T | 527 | G | LEFTY2 | Q298R | T | 1242 | C |
| HOXA9 | C157Y | C | 500 | T | ISM2 | G508S | C | 1579 | T | KIAA1328 | M292I | G | 898 | A | LEKR1 | Q248R | A | 2078 | G |

| | | | | | | | | | | | | | | | | | | | |
|---|---|---|---|---|---|---|---|---|---|---|---|---|---|---|---|---|---|---|---|
| HOXC10 | A120V | C | 473 | T | ISM2 | P334H | G | 1058 | T | KIAA1328 | A525V | C | 1596 | T | LENG1 | P135L | G | 591 | A |
| HOXC13 | H68Q | C | 346 | G | ISOC1 | E250K | G | 764 | A | KIAA1370 | N621I | T | 2015 | A | LENG8 | A254T | G | 815 | A |
| HOXC4 | R158C | C | 1079 | T | ISX | P70H | C | 1161 | A | KIAA1370 | R31Q | C | 245 | T | LENG8 | T620M | C | 1914 | T |
| HOXC4 | R208C | C | 1229 | T | ISX | R86H | G | 1209 | A | KIAA1370 | I363M | T | 1242 | C | LENG8 | A477V | C | 1485 | T |
| HOXC5 | Y4C | A | 281 | G | ITCH | E818* | G | 2588 | T | KIAA1377 | A26T | G | 346 | A | LENG9 | G171R | C | 630 | G |
| HOXC9 | P45S | C | 231 | T | ITCH | R639H | G | 2052 | A | KIAA1377 | R68* | C | 472 | T | LENG9 | G154D | C | 580 | T |
| HOXD10 | S125L | C | 629 | T | ITCH | P442L | C | 1461 | T | KIAA1377 | I757L | A | 2539 | C | LEO1 | R532H | C | 1650 | T |
| HOXD11 | P249L | C | 816 | T | ITFG1 | R521H | C | 1779 | T | KIAA1383 | Y620H | T | 1985 | C | LEP | S130N | G | 440 | A |
| HOXD12 | T115A | A | 415 | G | ITFG1 | K252N | T | 973 | G | KIAA1383 | D927Y | G | 2906 | T | LEPR | M1I | G | 188 | T |
| HOXD12 | A135D | C | 476 | A | ITFG1 | W314C | C | 1159 | A | KIAA1407 | K751E | T | 2398 | C | LEPRE1 | N732S | T | 2236 | C |
| HOXD3 | R198H | G | 2014 | A | ITFG2 | D136N | G | 545 | A | KIAA1407 | E526K | C | 1723 | T | LEPRE1 | - | T | 0 | C |
| HOXD3 | C90* | C | 1691 | A | ITFG2 | A206V | C | 756 | T | KIAA1409 | P1661T | C | 4981 | A | LEPRE1 | R210* | G | 669 | A |
| HOXD3 | P332L | C | 2416 | T | ITFG2 | R141C | C | 560 | T | KIAA1409 | A2603T | G | 7807 | A | LEPREL1 | R136H | C | 605 | T |
| HOXD4 | R158Q | G | 885 | A | ITFG2 | R116H | G | 486 | A | KIAA1409 | A1350E | C | 4049 | A | LEPREL1 | Q406* | G | 1414 | A |
| HOXD4 | G63R | G | 599 | A | ITFG2 | R422C | C | 1403 | T | KIAA1409 | R568C | C | 1702 | T | LEPREL2 | G489V | G | 1466 | T |
| HOXD8 | Y139H | T | 1042 | C | ITGA1 | A823T | G | 2925 | A | KIAA1409 | Y1340C | A | 4019 | G | LEPREL2 | R320W | C | 958 | T |
| HP | R277C | C | 873 | T | ITGA1 | A236V | C | 1165 | T | KIAA1409 | R1517C | C | 4549 | T | LEPROT | P46S | C | 274 | T |
| HP1BP3 | D4E | A | 152 | C | ITGA1 | F222S | T | 1123 | C | KIAA1409 | T1980M | C | 5939 | T | LEPROTL1 | I8S | T | 130 | G |
| HPCAL1 | A61T | G | 562 | A | ITGA1 | Q539K | C | 2073 | A | KIAA1409 | M2420I | G | 7260 | A | LETM1 | P219L | G | 953 | A |
| HPCAL1 | S6R | A | 397 | C | ITGA10 | S476C | A | 1601 | T | KIAA1409 | V1336I | G | 4006 | A | LETM2 | W141C | G | 524 | T |
| HPCAL4 | R139C | G | 807 | A | ITGA10 | G92D | G | 450 | A | KIAA1409 | I2056V | A | 6166 | G | LETMD1 | K350N | A | 1080 | C |
| HPCAL4 | R115H | C | 736 | T | ITGA10 | C736R | T | 2381 | C | KIAA1409 | G2423E | G | 7268 | A | LEUTX | A176V | C | 527 | T |
| HPD | V131A | A | 432 | G | ITGA11 | R550* | G | 1735 | A | KIAA1409 | N1049H | A | 3145 | C | LGALS1 | S8I | G | 118 | T |
| HPD | A244V | G | 771 | A | ITGA11 | G712R | C | 2221 | T | KIAA1409 | K1081I | A | 3242 | T | LGALS12 | R117C | C | 690 | T |
| HPDL | E245* | G | 1009 | T | ITGA11 | R463Q | C | 1475 | T | KIAA1409 | K1353T | A | 4058 | C | LGALS12 | V68G | T | 544 | G |
| HPGDS | W148R | A | 533 | G | ITGA11 | A1173V | G | 3605 | A | KIAA1409 | E1488* | G | 4462 | T | LGALS14 | R27S | C | 520 | A |
| HPN | G24R | G | 315 | A | ITGA11 | L633M | G | 1984 | T | KIAA1429 | R1622I | C | 4878 | A | LGALS4 | G231W | C | 905 | A |
| HPN | R144H | G | 676 | A | ITGA11 | T599I | G | 1883 | A | KIAA1429 | R1571C | G | 4724 | A | LGALS8 | V245I | G | 933 | A |
| HPRT1 | K73T | A | 385 | C | ITGA11 | F329L | G | 1074 | T | KIAA1429 | S1301N | C | 3915 | T | LGALS8 | D25N | G | 273 | A |
| HPS1 | G685D | C | 2288 | T | ITGA11 | S126Y | G | 464 | T | KIAA1429 | L391V | A | 1184 | C | LGI1 | N167H | A | 759 | C |
| HPS1 | R690C | G | 2302 | A | ITGA2 | R553Q | G | 1801 | A | KIAA1430 | P245H | G | 854 | T | LGI2 | R79Q | C | 422 | T |
| HPS1 | S464F | G | 1625 | A | ITGA2 | K192N | G | 719 | T | KIAA1430 | V319D | A | 1076 | T | LGI3 | G363D | C | 1378 | T |
| HPS3 | L981I | C | 3081 | A | ITGA2 | N894D | A | 2823 | G | KIAA1432 | N922T | A | 2765 | C | LGI4 | P376S | G | 1646 | A |
| HPS3 | E134D | G | 542 | T | ITGA2B | A647V | G | 1972 | A | KIAA1432 | R980Q | G | 2939 | A | LGI4 | R124C | G | 890 | A |
| HPS3 | R714Q | G | 2281 | A | ITGA2B | S427R | T | 1311 | G | KIAA1462 | K1039N | C | 3219 | A | LGI4 | E108K | C | 842 | T |
| HPS3 | T591M | C | 1912 | T | ITGA2B | P605L | G | 1846 | A | KIAA1462 | P1333L | G | 4100 | A | LGR4 | E85* | C | 697 | A |
| HPS3 | F989L | T | 3107 | G | ITGA3 | L736V | C | 2670 | G | KIAA1462 | V414I | C | 1342 | T | LGR4 | E862K | C | 3028 | T |
| HPS4 | A396V | G | 1187 | A | ITGA3 | P357H | C | 1534 | A | KIAA1462 | R73H | C | 320 | T | LGR4 | G654V | C | 2405 | A |
| HPS5 | T38M | G | 391 | A | ITGA4 | D385N | G | 1583 | A | KIAA1462 | Q182P | T | 647 | G | LGR5 | H414N | C | 1288 | A |

| | | | | | | | | | | | | | | | | | | | |
|---|---|---|---|---|---|---|---|---|---|---|---|---|---|---|---|---|---|---|---|
| HPS5 | R124* | G | 648 | A | ITGA4 | A87V | C | 690 | T | KIAA1462 | E1190G | T | 3671 | C | LGR5 | R520C | C | 1606 | T |
| HPS5 | K598N | T | 2072 | G | ITGA4 | R565W | C | 2123 | T | KIAA1462 | S694R | T | 2182 | G | LGR5 | L113I | C | 385 | A |
| HPS6 | L396F | G | 1273 | T | ITGA4 | S426L | C | 1707 | T | KIAA1462 | A473V | G | 1520 | A | LGR5 | F110C | T | 377 | G |
| HPS6 | R667* | C | 2084 | T | ITGA4 | V587A | T | 2190 | C | KIAA1467 | W554C | G | 1685 | T | LGR6 | V734M | G | 2289 | A |
| HPSE | P10L | G | 166 | A | ITGA5 | L187M | G | 627 | T | KIAA1467 | I336T | T | 1030 | C | LGSN | E134* | C | 434 | A |
| HPSE | F386C | A | 1294 | C | ITGA6 | T106M | C | 520 | T | KIAA1467 | P393L | C | 1201 | T | LGSN | A81T | C | 275 | T |
| HPSE2 | Y253H | A | 817 | G | ITGA7 | G249R | C | 965 | T | KIAA1468 | L745F | C | 2448 | T | LGTN | C489Y | C | 1675 | T |
| HPSE2 | A249T | C | 805 | T | ITGA7 | E596* | C | 2006 | A | KIAA1468 | R789W | C | 2580 | T | LHCGR | R26C | G | 98 | A |
| HPSE2 | A565V | G | 1754 | A | ITGA8 | P194T | G | 934 | T | KIAA1468 | K877N | G | 2846 | T | LHCGR | H684N | G | 2072 | T |
| HPSE2 | R267Q | C | 860 | T | ITGA8 | E702K | C | 2458 | T | KIAA1468 | A99V | C | 511 | T | LHCGR | A449T | C | 1367 | T |
| HPSE2 | R564W | G | 1750 | A | ITGA8 | R895* | G | 3037 | A | KIAA1468 | I661S | T | 2197 | G | LHCGR | A28V | G | 105 | A |
| HPSE2 | Q541* | G | 1681 | A | ITGA8 | I376M | G | 1482 | C | KIAA1486 | P195L | C | 997 | T | LHCGR | W367S | C | 1122 | G |
| HPSE2 | R494C | G | 1540 | A | ITGA9 | D154N | G | 716 | A | KIAA1486 | S350F | C | 1462 | T | LHFPL1 | R48Q | C | 383 | T |
| HPX | R256C | G | 867 | A | ITGA9 | E623D | G | 2125 | T | KIAA1486 | T98P | A | 705 | C | LHFPL2 | - | C | 0 | A |
| HR | A766V | G | 3778 | A | ITGA9 | G438E | G | 1569 | A | KIAA1486 | V387I | G | 1572 | A | LHFPL5 | P57L | C | 548 | T |
| HRASLS | A149V | C | 855 | T | ITGA9 | E752G | A | 2511 | G | KIAA1486 | S648L | C | 2356 | T | LHFPL5 | G82D | G | 623 | A |
| HRASLS5 | K118T | T | 513 | G | ITGAD | V669I | G | 2006 | A | KIAA1522 | R615H | G | 1888 | A | LHPP | R234H | G | 729 | A |
| HRASLS5 | K118T | T | 513 | G | ITGAD | T807P | A | 2420 | C | KIAA1522 | G261S | G | 825 | A | LHPP | A82T | G | 272 | A |
| HRC | H339R | T | 1203 | C | ITGAD | V669I | G | 2006 | A | KIAA1522 | V254I | G | 804 | A | LHX1 | E371K | G | 1834 | A |
| HRH1 | R175H | G | 866 | A | ITGAD | E958G | A | 2874 | G | KIAA1529 | - | A | 0 | G | LHX1 | S383L | C | 1871 | T |
| HRH1 | L86I | C | 598 | A | ITGAD | G338D | G | 1014 | A | KIAA1529 | R32Q | G | 1447 | A | LHX1 | R259W | C | 1498 | T |
| HRH2 | V23M | G | 718 | A | ITGAD | S676R | A | 2027 | C | KIAA1530 | A232T | G | 1040 | A | LHX1 | E211K | G | 1354 | A |
| HRH2 | F380L | T | 1789 | C | ITGAE | G696S | C | 2192 | T | KIAA1530 | D2Y | G | 350 | T | LHX1 | R184H | G | 1274 | A |
| HRH3 | A32T | C | 391 | T | ITGAE | - | C | 0 | T | KIAA1539 | A150T | C | 448 | T | LHX2 | A185T | G | 551 | A |
| HRH3 | R138* | G | 709 | A | ITGAE | V416A | A | 1353 | G | KIAA1539 | G378R | C | 1132 | T | LHX2 | L156R | T | 465 | G |
| HRH4 | S11P | T | 131 | C | ITGAE | L378M | G | 1238 | T | KIAA1539 | - | A | 0 | T | LHX3 | S179R | G | 656 | T |
| HRH4 | R49* | C | 245 | T | ITGAE | A358T | C | 1178 | T | KIAA1543 | R742Q | G | 2326 | A | LHX3 | V124I | C | 489 | T |
| HRH4 | R236I | G | 807 | T | ITGAL | R520Q | G | 1735 | A | KIAA1543 | R1000Q | G | 3100 | A | LHX5 | R78C | G | 806 | A |
| HRH4 | R341I | G | 1122 | T | ITGAL | I961T | T | 3058 | C | KIAA1543 | V52M | G | 255 | A | LHX5 | A6T | C | 590 | T |
| HRNR | G766C | C | 2372 | A | ITGAL | T417I | C | 1426 | T | KIAA1543 | P209H | C | 727 | A | LHX6 | R174W | G | 580 | A |
| HRNR | G989D | C | 3042 | T | ITGAM | A304T | G | 1008 | A | KIAA1543 | K1179Q | A | 3636 | C | LHX8 | A245D | C | 1398 | A |
| HRNR | R896C | G | 2762 | A | ITGAM | P543T | C | 1725 | A | KIAA1549 | P711H | G | 2181 | T | LHX8 | E44D | G | 796 | T |
| HRNR | R429C | G | 1361 | A | ITGAM | - | G | 0 | T | KIAA1549 | P1446L | G | 4386 | A | LHX8 | E200* | G | 1262 | T |
| HRNR | R571C | G | 1787 | A | ITGAM | R732H | G | 2293 | A | KIAA1549 | I1151N | A | 3501 | T | LHX8 | S58L | C | 837 | T |
| HRNR | A834D | G | 2577 | T | ITGAM | D1009N | G | 3123 | A | KIAA1549 | R1901W | G | 5750 | A | LHX9 | R269C | C | 1230 | T |
| HRNR | S225A | A | 749 | C | ITGAV | R540Q | G | 1893 | A | KIAA1549 | F481S | A | 1491 | G | LHX9 | R256Q | G | 1192 | A |
| HRNR | I88S | A | 339 | C | ITGAV | W209* | G | 901 | A | KIAA1549 | A469T | C | 1454 | T | LHX9 | M34I | G | 527 | A |
| HRSP12 | R120Q | C | 453 | T | ITGAX | R571W | C | 1778 | T | KIAA1549 | R1501H | C | 4551 | T | LHX9 | R150Q | G | 874 | A |
| HS1BP3 | S225L | G | 700 | A | ITGAX | E410K | G | 1295 | A | KIAA1549 | P531S | G | 1640 | A | LIF | P90L | G | 425 | A |

| | | | | | | | | | | | | | | | | | | | |
|---|---|---|---|---|---|---|---|---|---|---|---|---|---|---|---|---|---|---|---|
| HS1BP3 | S36F | G | 133 | A | ITGAX | V236M | G | 773 | A | KIAA1586 | I476M | T | 1635 | G | LIFR | R508W | G | 1685 | A |
| HS1BP3 | P181L | G | 568 | A | ITGAX | Q933H | A | 2866 | C | KIAA1614 | P54H | C | 216 | A | LIFR | R1066Q | C | 3360 | T |
| HS1BP3 | A286T | C | 882 | T | ITGAX | E1162D | G | 3553 | T | KIAA1614 | D484N | G | 1505 | A | LIFR | R178C | G | 695 | A |
| HS3ST1 | R205H | C | 1789 | T | ITGB1 | R495H | C | 1705 | T | KIAA1614 | G894R | G | 2735 | A | LIFR | K537N | C | 1774 | A |
| HS3ST2 | A2T | G | 438 | A | ITGB1BP2 | C218R | T | 725 | C | KIAA1614 | A1105T | G | 3368 | A | LIFR | I444N | A | 1494 | T |
| HS3ST3A1 | P245L | G | 1532 | A | ITGB1BP2 | T47A | A | 212 | G | KIAA1632 | V995I | C | 3018 | T | LIFR | R597* | G | 1952 | A |
| HS3ST4 | A200V | C | 991 | T | ITGB2 | V600I | C | 1986 | T | KIAA1632 | R2320H | C | 6994 | T | LIFR | N59K | A | 340 | C |
| HS3ST4 | R218H | G | 1045 | A | ITGB2 | V439M | C | 1503 | T | KIAA1632 | M2188V | T | 6597 | C | LIFR | - | C | 0 | T |
| HS3ST4 | E452D | A | 1748 | C | ITGB3 | D551N | G | 1671 | A | KIAA1632 | L1086I | G | 3291 | T | LIG1 | E785K | C | 2473 | T |
| HS3ST4 | S42P | T | 516 | C | ITGB3BP | K61N | T | 244 | G | KIAA1632 | E2241D | T | 6758 | G | LIG1 | S163N | C | 608 | T |
| HS3ST5 | A7V | G | 20 | A | ITGB4 | P896L | C | 2874 | T | KIAA1632 | E1742Q | C | 5259 | G | LIG1 | A374T | C | 1240 | T |
| HS3ST6 | T313M | G | 938 | A | ITGB4 | E1311K | G | 4118 | A | KIAA1632 | I1720V | T | 5193 | C | LIG1 | A60V | G | 299 | A |
| HS3ST6 | W274* | C | 822 | T | ITGB4 | N205S | A | 801 | G | KIAA1632 | R955* | G | 2898 | A | LIG1 | P395Q | G | 1304 | T |
| HS6ST1 | R214H | C | 655 | T | ITGB4 | S1543Y | C | 4815 | A | KIAA1644 | P173Q | G | 651 | T | LIG3 | A685V | C | 2158 | T |
| HS6ST2 | S188Y | G | 563 | T | ITGB6 | K404N | C | 1450 | A | KIAA1644 | S25P | A | 206 | G | LIG3 | L381R | T | 1246 | G |
| HS6ST2 | R478Q | C | 1433 | T | ITGB6 | C511Y | C | 1770 | T | KIAA1671 | V1599M | G | 5502 | A | LIG4 | L328I | G | 1112 | T |
| HS6ST3 | R163Q | G | 512 | A | ITGB6 | E644* | C | 2168 | A | KIAA1683 | K516N | C | 1764 | A | LIG4 | S160Y | G | 609 | T |
| HS6ST3 | F351S | T | 1076 | C | ITGB7 | H214R | T | 645 | C | KIAA1683 | R956W | G | 3082 | A | LIG4 | S822L | G | 2595 | A |
| HS6ST3 | R418C | C | 1276 | T | ITGB7 | H487N | G | 1463 | T | KIAA1683 | R1176H | C | 3743 | T | LILRA1 | - | A | 0 | G |
| HS6ST3 | L14F | C | 64 | T | ITGB7 | A115D | G | 348 | T | KIAA1683 | R19C | G | 271 | A | LILRA2 | P218L | C | 786 | T |
| HS6ST3 | W195* | G | 609 | A | ITGB7 | R146C | G | 440 | A | KIAA1683 | S510L | G | 1745 | A | LILRA3 | D434N | C | 1483 | T |
| HSBP1 | A53T | G | 246 | A | ITGB8 | N497D | A | 2173 | G | KIAA1683 | S1241C | T | 3937 | A | LILRA3 | D162N | C | 667 | T |
| HSCB | R99H | G | 361 | A | ITGBL1 | C96Y | G | 506 | A | KIAA1683 | A32V | G | 311 | A | LILRA5 | R268H | C | 923 | T |
| HSD11B1 | M110I | G | 499 | T | ITGBL1 | C185Y | G | 773 | A | KIAA1683 | R1219H | C | 3872 | T | LILRB1 | S486L | C | 1790 | T |
| HSD11B1L | A64V | C | 441 | T | ITGBL1 | Q314H | G | 1161 | T | KIAA1704 | D87E | T | 364 | G | LILRB1 | V372E | T | 1448 | A |
| HSD11B1L | E220D | G | 910 | C | ITGBL1 | D214Y | G | 859 | T | KIAA1712 | S253L | C | 1063 | T | LILRB1 | V81A | T | 575 | C |
| HSD11B1L | R169H | G | 756 | A | ITIH1 | S30L | C | 113 | T | KIAA1715 | E30K | C | 336 | T | LILRB2 | R500Q | C | 1659 | T |
| HSD11B1L | H234R | A | 951 | G | ITIH1 | G668D | G | 2027 | A | KIAA1715 | S421T | C | 1510 | G | LILRB2 | E390* | C | 1328 | A |
| HSD17B1 | R137H | G | 4130 | A | ITIH2 | F8L | C | 190 | A | KIAA1731 | A280S | G | 989 | T | LILRB2 | S220Y | G | 819 | T |
| HSD17B11 | N69H | T | 521 | G | ITIH3 | D829G | A | 2492 | G | KIAA1731 | E1219* | G | 3806 | T | LILRB3 | D536G | T | 1609 | C |
| HSD17B12 | R302Q | G | 1140 | A | ITIH3 | M372V | A | 1120 | G | KIAA1731 | S1654L | C | 5112 | T | LILRB3 | R542S | C | 1628 | G |
| HSD17B13 | V243I | C | 792 | T | ITIH3 | E437G | A | 1316 | G | KIAA1737 | V323A | T | 1147 | C | LILRB3 | A104T | C | 312 | T |
| HSD17B14 | K70R | T | 475 | C | ITIH3 | E576D | G | 1734 | T | KIAA1737 | R341H | G | 1201 | A | LILRB4 | Q50* | C | 162 | T |
| HSD17B14 | D171N | C | 777 | T | ITIH3 | T806M | C | 2423 | T | KIAA1751 | V500I | C | 1654 | T | LILRB4 | A362V | C | 1099 | T |
| HSD17B2 | T140M | C | 583 | T | ITIH3 | F217L | C | 657 | A | KIAA1755 | V1017M | C | 3321 | T | LILRB5 | R155M | C | 542 | A |
| HSD17B2 | L354W | T | 1225 | G | ITIH4 | Y157C | T | 501 | C | KIAA1755 | R997H | C | 3262 | T | LIM2 | A114V | G | 372 | A |
| HSD17B3 | S184Y | G | 599 | T | ITIH4 | R900H | C | 2730 | T | KIAA1755 | R963C | G | 3159 | A | LIM2 | Y203H | A | 638 | G |
| HSD17B3 | S184Y | G | 599 | T | ITIH4 | R439H | C | 1347 | T | KIAA1755 | L666I | G | 2268 | T | LIMCH1 | A754V | C | 2695 | T |
| HSD17B3 | K119N | T | 405 | A | ITIH4 | G434D | C | 1332 | T | KIAA1755 | Q1020R | T | 3331 | C | LIMCH1 | G985R | G | 3387 | A |

| | | | | | | | | | | | | | | | | | | | |
|---|---|---|---|---|---|---|---|---|---|---|---|---|---|---|---|---|---|---|---|
| HSD17B4 | V376G | T | 1311 | G | ITIH4 | R47Q | C | 171 | T | KIAA1755 | D750N | C | 2520 | T | LIMCH1 | R1206H | G | 4051 | A |
| HSD17B4 | K285N | G | 1039 | T | ITIH4 | R915C | G | 2774 | A | KIAA1797 | R732K | G | 2559 | A | LIMD2 | R26H | C | 255 | T |
| HSD17B6 | A302S | G | 1014 | T | ITIH5 | L205P | A | 693 | G | KIAA1797 | S1048F | C | 3507 | T | LIMD2 | - | T | 0 | G |
| HSD17B7 | P245L | C | 789 | T | ITIH5 | A195T | C | 662 | T | KIAA1797 | S656I | G | 2331 | T | LIME1 | L99M | C | 382 | A |
| HSD3B7 | R179C | C | 825 | T | ITIH5 | R459W | G | 1454 | A | KIAA1797 | R1547I | G | 5004 | T | LIME1 | E251D | A | 840 | C |
| HSD3B7 | F205L | C | 905 | A | ITIH5 | P682H | G | 2124 | T | KIAA1841 | R447Q | G | 1581 | A | LIMK1 | R575W | C | 1825 | T |
| HSDL1 | Y11H | A | 210 | G | ITIH5 | K117T | T | 429 | G | KIAA1841 | V386A | T | 1398 | C | LIMK1 | E405K | G | 1315 | A |
| HSDL1 | R137* | G | 588 | A | ITIH5L | N878T | T | 2663 | G | KIAA1841 | L168I | C | 743 | A | LIMK1 | - | T | 0 | G |
| HSF1 | H354Y | C | 1230 | T | ITIH5L | E618* | C | 1882 | A | KIAA1919 | K180T | A | 892 | C | LIMK2 | E252D | G | 986 | T |
| HSF1 | P430S | C | 1458 | T | ITIH5L | L391I | G | 1201 | T | KIAA1919 | - | T | 0 | C | LIMS2 | P254L | G | 919 | A |
| HSF2BP | R312H | C | 1267 | T | ITLN2 | M102T | A | 363 | G | KIAA1919 | G330V | G | 1342 | T | LIMS2 | F79V | A | 393 | C |
| HSF2BP | R84H | C | 583 | T | ITM2A | T8A | T | 166 | C | KIAA1919 | I320L | A | 1311 | C | LIN28A | D33G | A | 212 | G |
| HSF2BP | A7T | C | 351 | T | ITPA | V24I | G | 262 | A | KIAA1919 | T326A | A | 1329 | G | LIN28A | H147N | C | 553 | A |
| HSP90AA2 | P38T | G | 457 | T | ITPKA | R281W | C | 908 | T | KIAA1958 | T571M | C | 2008 | T | LIN28A | R192* | C | 688 | T |
| HSP90AA2 | F434L | G | 1647 | T | ITPKA | R440C | C | 1385 | T | KIAA1958 | E209K | G | 753 | A | LIN54 | A104T | C | 688 | T |
| HSP90AB1 | E232K | G | 803 | A | ITPKA | N165H | A | 560 | C | KIAA1967 | G813E | G | 2575 | A | LIN9 | Q590H | C | 1770 | A |
| HSP90B1 | E487* | G | 1593 | T | ITPKB | G455D | C | 1364 | T | KIAA1967 | P427L | C | 1417 | T | LINGO1 | A92T | C | 326 | T |
| HSP90B1 | E582K | G | 1878 | A | ITPKC | I591V | A | 1804 | G | KIAA1967 | G28D | G | 220 | A | LINGO1 | R122Q | C | 417 | T |
| HSP90B2P | D374G | A | 1121 | G | ITPR1 | F1500L | C | 4860 | A | KIAA1984 | R236C | C | 741 | T | LINGO1 | Q488R | T | 1515 | C |
| HSPA12A | A392V | G | 1280 | A | ITPR1 | F696L | T | 2448 | G | KIAA2013 | L604I | G | 2001 | T | LINGO2 | Q181K | G | 995 | T |
| HSPA12A | P107H | G | 425 | T | ITPR1 | Q2168P | A | 6863 | C | KIAA2018 | R2094Q | C | 6692 | T | LINGO2 | A346V | G | 1491 | A |
| HSPA12A | V519I | C | 1660 | T | ITPR1 | G2411V | G | 7592 | T | KIAA2018 | Q1753H | T | 5670 | A | LINGO2 | S398R | G | 1648 | T |
| HSPA12A | E228D | C | 789 | A | ITPR1 | R2442C | C | 7684 | T | KIAA2018 | A767V | G | 2711 | A | LINGO3 | R386C | G | 1284 | A |
| HSPA12A | S190L | G | 674 | A | ITPR1 | A1243V | C | 4088 | T | KIAA2018 | R1776* | G | 5737 | A | LINGO3 | P262S | G | 912 | A |
| HSPA12B | I374V | A | 1122 | G | ITPR1 | A1597V | C | 5150 | T | KIAA2018 | S1339Y | G | 4427 | T | LINGO3 | H559Y | G | 1803 | A |
| HSPA14 | A265T | G | 1032 | A | ITPR1 | A1707V | C | 5480 | T | KIAA2022 | S1454F | G | 5013 | A | LINGO4 | S256N | C | 1705 | T |
| HSPA2 | A180V | C | 615 | T | ITPR1 | A1216V | C | 4007 | T | KIAA2022 | K585E | T | 2405 | C | LINGO4 | T98I | G | 1231 | A |
| HSPA2 | E447K | G | 1415 | A | ITPR1 | K1956R | A | 6227 | G | KIAA2022 | G353W | C | 1709 | A | LINS1 | S651F | G | 2175 | A |
| HSPA2 | D161G | A | 558 | G | ITPR1 | K2674E | A | 8380 | G | KIAA2026 | R506W | G | 1732 | A | LINS1 | E686* | C | 2279 | A |
| HSPA2 | E579D | G | 1813 | T | ITPR1 | S207F | C | 980 | T | KIAA2026 | A306V | G | 1133 | A | LINS1 | L308I | G | 1145 | T |
| HSPA2 | A179S | G | 611 | T | ITPR1 | R289W | C | 1225 | T | KIAA2026 | G1864R | C | 5806 | T | LINS1 | E10* | C | 251 | A |
| HSPA4 | M301I | G | 1192 | T | ITPR1 | F1860L | C | 5940 | A | KIAA2026 | I1777M | A | 5547 | C | LINS1 | T753N | G | 2481 | T |
| HSPA4 | Y626F | A | 2166 | T | ITPR1 | A1925T | G | 6133 | A | KIAA2026 | R574C | G | 1936 | A | LIPC | E381D | G | 1754 | T |
| HSPA4L | A832V | C | 2658 | T | ITPR1 | K221T | A | 1022 | C | KIAA2026 | S347Y | G | 1256 | T | LIPE | G336S | C | 1283 | T |
| HSPA5 | E358* | C | 1276 | A | ITPR2 | L2266I | G | 7213 | T | KIDINS220 | E1655K | C | 5145 | T | LIPE | V603I | C | 2084 | T |
| HSPA6 | A181V | C | 955 | T | ITPR2 | E1362D | C | 4503 | A | KIDINS220 | A697T | C | 2271 | T | LIPE | P247L | G | 1017 | A |
| HSPA8 | K458R | T | 1451 | C | ITPR2 | E616* | C | 2263 | A | KIDINS220 | I1458T | A | 4555 | G | LIPF | F156L | C | 514 | A |
| HSPA8 | I216N | A | 725 | T | ITPR2 | F1704C | A | 5528 | C | KIDINS220 | R100H | C | 481 | T | LIPF | R274C | C | 866 | T |
| HSPA8 | L391V | A | 1249 | C | ITPR2 | R505H | C | 1931 | T | KIDINS220 | D1540Y | C | 4800 | A | LIPH | D247Y | C | 881 | A |

| | | | | | | | | | | | | | | | | | | | |
|---|---|---|---|---|---|---|---|---|---|---|---|---|---|---|---|---|---|---|---|
| HSPA9 | E649K | C | 2071 | T | ITPR2 | Q2160R | T | 6896 | C | KIDINS220 | R951Q | C | 3034 | T | LIPI | S317L | G | 976 | A |
| HSPB1 | R127Q | G | 549 | A | ITPR2 | A445D | G | 1751 | T | KIF11 | R384C | C | 1240 | T | LIPK | K191N | G | 573 | T |
| HSPB8 | G132C | G | 917 | T | ITPR2 | P2512S | G | 7951 | A | KIF12 | T221I | G | 677 | A | LIPK | E352D | A | 1056 | C |
| HSPBAP1 | Y258C | T | 917 | C | ITPR3 | V432M | G | 2152 | A | KIF13A | R426Q | C | 1383 | T | LIPK | L354V | T | 1060 | G |
| HSPBAP1 | A240V | G | 863 | A | ITPR3 | S752F | C | 3113 | T | KIF13A | A73T | C | 323 | T | LIPM | R139* | C | 415 | T |
| HSPBAP1 | E31D | C | 237 | A | ITPR3 | A1972T | G | 6772 | A | KIF13A | - | T | 0 | C | LIPN | T36A | A | 106 | G |
| HSPBP1 | A128P | C | 716 | G | ITPR3 | T1803M | C | 6266 | T | KIF13A | V1269A | A | 3912 | G | LIPN | M271I | G | 813 | T |
| HSPD1 | K493E | T | 1745 | C | ITPR3 | R2258H | G | 7631 | A | KIF13A | S852G | T | 2660 | C | LIPT2 | L105M | G | 320 | T |
| HSPD1 | K82Q | T | 512 | G | ITPR3 | R1857C | C | 6427 | T | KIF13A | Q584* | G | 1856 | A | LITAF | G46W | C | 188 | A |
| HSPG2 | A3343V | G | 10108 | A | ITPR3 | R527C | C | 2437 | T | KIF13A | V683I | C | 2153 | T | LLGL1 | G203R | G | 703 | A |
| HSPG2 | T251M | G | 832 | A | ITPR3 | R1455H | G | 5222 | A | KIF13B | V1716M | C | 5205 | T | LLGL1 | A39T | G | 211 | A |
| HSPG2 | R363C | G | 1167 | A | ITPR3 | K1699E | A | 5953 | G | KIF13B | V914I | C | 2799 | T | LLGL1 | H888N | C | 2758 | A |
| HSPG2 | V529M | C | 1665 | T | ITPR3 | K2547T | A | 8498 | C | KIF13B | P606L | G | 1876 | A | LLGL2 | R891C | C | 2825 | T |
| HSPG2 | T1115A | T | 3423 | C | ITPR3 | R1849H | G | 6404 | A | KIF13B | M504I | C | 1571 | T | LLGL2 | S464N | G | 1545 | A |
| HSPG2 | R824T | C | 2551 | G | ITPRIP | A43V | G | 300 | A | KIF13B | R1117H | C | 3409 | T | LLGL2 | A407T | G | 1373 | A |
| HSPG2 | L3754M | G | 11340 | T | ITPRIPL1 | Q563R | A | 1687 | G | KIF13B | - | C | 0 | A | LLGL2 | S427R | A | 1433 | C |
| HSPG2 | G1823D | C | 5548 | T | ITPRIPL1 | S472F | C | 1414 | T | KIF13B | G745* | C | 2292 | A | LLPH | R109* | G | 381 | A |
| HSPG2 | D227V | T | 760 | A | ITPRIPL2 | A395T | G | 1685 | A | KIF14 | Q1255H | C | 4204 | A | LMAN1 | S153Y | G | 485 | T |
| HSPG2 | R893H | C | 2758 | T | ITPRIPL2 | A420G | C | 1761 | G | KIF14 | S1102N | C | 3744 | T | LMAN1L | V93A | T | 417 | C |
| HSPG2 | A946S | C | 2916 | A | ITPRIPL2 | R400C | C | 1700 | T | KIF14 | R731I | C | 2631 | A | LMAN1L | S253I | G | 897 | T |
| HSPH1 | N409T | T | 1226 | G | ITPRIPL2 | T265I | C | 1296 | T | KIF14 | N627T | T | 2319 | G | LMAN1L | R288M | G | 1002 | T |
| HSPH1 | Y742D | A | 2224 | C | ITSN1 | Q416R | A | 1535 | G | KIF14 | S272R | T | 1253 | G | LMAN2 | R314H | C | 1146 | T |
| HTATIP2 | A113T | G | 406 | A | ITSN1 | F1669I | T | 5293 | A | KIF14 | E159K | C | 914 | T | LMAN2L | N274S | T | 845 | C |
| HTR1A | K418T | T | 1667 | G | ITSN1 | R312Q | G | 1223 | A | KIF15 | M1132I | G | 3545 | T | LMBR1 | R496* | G | 1640 | A |
| HTR1A | A50T | C | 562 | T | ITSN1 | T349M | C | 1334 | T | KIF15 | S233L | C | 847 | T | LMBR1 | V433A | A | 1452 | G |
| HTR1A | A50V | G | 563 | A | ITSN1 | F47Y | T | 428 | A | KIF16B | R1129H | C | 3544 | T | LMBR1 | L155R | A | 618 | C |
| HTR1A | L74R | A | 635 | C | ITSN1 | R434Q | G | 1589 | A | KIF16B | E853* | C | 2715 | A | LMBR1 | R44I | C | 285 | A |
| HTR1A | R176H | C | 941 | T | ITSN1 | R1123H | G | 3656 | A | KIF16B | E698* | C | 2250 | A | LMBR1L | A75V | G | 567 | A |
| HTR1B | Y211H | A | 692 | G | ITSN1 | T6A | A | 304 | G | KIF17 | R232W | G | 1005 | A | LMBRD1 | T250M | G | 979 | A |
| HTR1D | R374Q | C | 1164 | T | ITSN1 | H1403D | C | 4495 | G | KIF17 | S124G | T | 681 | C | LMBRD1 | K138E | T | 642 | C |
| HTR1E | A208V | C | 1326 | T | ITSN1 | Y1479H | T | 4723 | C | KIF17 | S210L | G | 940 | A | LMBRD2 | Q130* | G | 851 | A |
| HTR1E | S337Y | C | 1713 | A | ITSN1 | R1104Q | G | 3599 | A | KIF18A | K808N | C | 2606 | G | LMBRD2 | K342T | T | 1488 | G |
| HTR1F | L4V | T | 64 | G | ITSN1 | K1265Q | A | 4081 | C | KIF18A | L140R | A | 601 | C | LMBRD2 | R32I | C | 558 | A |
| HTR2A | V324L | C | 1102 | A | ITSN2 | K584Q | T | 2194 | G | KIF18B | G753D | C | 2274 | T | LMCD1 | R92Q | G | 507 | A |
| HTR2A | E454D | T | 1494 | G | ITSN2 | T1184M | G | 3995 | A | KIF18B | R663C | G | 2003 | A | LMF1 | P546L | G | 1641 | A |
| HTR2A | P144L | G | 563 | A | ITSN2 | R961W | G | 3325 | A | KIF19 | R382M | G | 1145 | T | LMF1 | R461H | C | 1386 | T |
| HTR2A | S219L | G | 788 | A | ITSN2 | Y182H | A | 988 | G | KIF1A | A1609T | C | 4853 | T | LMF1 | D229G | T | 690 | C |
| HTR2B | R434* | G | 1813 | A | ITSN2 | F109L | A | 771 | C | KIF1A | R602L | C | 1833 | A | LMF2 | W572R | A | 1712 | G |

| | | | | | | | | | | | | | | | | | | | |
|---|---|---|---|---|---|---|---|---|---|---|---|---|---|---|---|---|---|---|---|
| HTR2C | I225T | T | 1493 | C | ITSN2 | A143S | C | 871 | A | KIF1A | A423T | C | 1295 | T | LMF2 | Y341C | T | 1020 | C |
| HTR3A | E433D | G | 1321 | T | IVL | L540P | T | 1683 | C | KIF1A | R1710H | C | 5157 | T | LMF2 | A320T | C | 956 | T |
| HTR3A | A39D | C | 138 | A | IVL | E247Q | G | 803 | C | KIF1A | P305L | G | 942 | A | LMLN | - | T | 2077 | C |
| HTR3A | Q371K | C | 1133 | A | IVNS1ABP | P544H | G | 2254 | T | KIF1A | R791C | G | 2399 | A | LMLN | T4A | A | 10 | G |
| HTR3A | L127I | C | 401 | A | IVNS1ABP | - | A | 0 | G | KIF1A | R1302H | C | 3933 | T | LMNA | R343W | C | 1239 | T |
| HTR3C | G148C | G | 476 | T | IVNS1ABP | H394R | T | 1804 | C | KIF1A | K802N | C | 2434 | A | LMNA | Q251R | A | 964 | G |
| HTR3D | V423L | G | 1267 | T | IVNS1ABP | V292I | C | 1497 | T | KIF1B | L1782I | C | 5596 | A | LMNB1 | R336C | C | 1367 | T |
| HTR3D | V273I | G | 817 | A | IVNS1ABP | E632* | C | 2517 | A | KIF1B | D1095G | A | 3536 | G | LMNB1 | D155G | A | 825 | G |
| HTR3D | A295V | C | 884 | T | IVNS1ABP | M545I | C | 2258 | A | KIF1C | D623N | G | 2224 | A | LMNB1 | K156N | A | 829 | C |
| HTR3D | P370L | C | 1109 | T | IVNS1ABP | I157N | A | 1093 | T | KIF20A | R218* | C | 878 | T | LMNB2 | A505T | C | 1636 | T |
| HTR3E | A459V | C | 1376 | T | IWS1 | T651N | G | 2212 | T | KIF20A | T832A | A | 2720 | G | LMNB2 | R244Q | C | 854 | T |
| HTR3E | A292T | G | 874 | A | IWS1 | S621L | G | 2122 | A | KIF20A | R772C | C | 2540 | T | LMNB2 | R138W | G | 535 | A |
| HTR3E | N46T | A | 137 | C | IWS1 | D334N | C | 1260 | T | KIF20B | A919T | G | 2827 | A | LMNB2 | R84W | G | 373 | A |
| HTR4 | W46C | C | 302 | A | JAG1 | A959V | G | 3306 | A | KIF20B | K955N | G | 2937 | T | LMO2 | L156F | G | 1296 | A |
| HTR4 | K174N | C | 686 | A | JAG1 | K428T | T | 1713 | G | KIF20B | E1834D | G | 5574 | C | LMO7 | R1250C | C | 4019 | T |
| HTR7 | A200V | G | 626 | A | JAG1 | E228K | C | 1112 | T | KIF21A | G911C | C | 3151 | A | LMO7 | K85Q | A | 1513 | C |
| HTR7 | A160T | C | 505 | T | JAG2 | S1092G | T | 3678 | C | KIF21A | G911C | C | 3151 | A | LMO7 | R627Q | G | 2151 | A |
| HTR7 | N376H | T | 1153 | G | JAGN1 | A113T | G | 506 | A | KIF21A | N649I | T | 2366 | A | LMOD2 | T184A | A | 707 | G |
| HTR7 | D142N | C | 451 | T | JAGN1 | L170I | C | 677 | A | KIF21A | R825H | C | 2894 | T | LMOD3 | E12* | C | 214 | A |
| HTRA2 | A85V | C | 884 | T | JAK1 | K736E | T | 2455 | C | KIF21A | V1533A | A | 5018 | G | LMOD3 | K285N | T | 1035 | G |
| HTRA2 | G261E | G | 1412 | A | JAK1 | R532C | G | 1843 | A | KIF21A | G908* | C | 3142 | A | LMTK2 | S600F | C | 2092 | T |
| HTRA4 | A172V | C | 630 | T | JAK1 | L531M | G | 1840 | T | KIF21A | K675Q | T | 2443 | G | LMTK2 | A267V | C | 1093 | T |
| HTRA4 | V345A | T | 1149 | C | JAK3 | P181L | G | 642 | A | KIF21B | S1193L | G | 3895 | A | LMTK2 | S439P | T | 1608 | C |
| HTT | A2694V | C | 8226 | T | JAK3 | R944H | C | 2931 | T | KIF21B | A956T | C | 3183 | T | LMTK2 | L342F | C | 1317 | T |
| HTT | A2285T | G | 6998 | A | JAK3 | L1091R | A | 3372 | C | KIF21B | Q808* | G | 2739 | A | LMTK2 | D276G | A | 1120 | G |
| HTT | Q2478H | G | 7579 | T | JAK3 | Q630H | C | 1990 | A | KIF21B | R1619H | C | 5173 | T | LMTK2 | F636L | T | 2201 | G |
| HTT | A653V | C | 2103 | T | JAK3 | A343V | G | 1128 | A | KIF21B | T1214M | G | 3958 | A | LMTK3 | R997M | C | 2990 | A |
| HTT | M633T | T | 2043 | C | JAKMIP1 | C630F | C | 2339 | A | KIF21B | R958W | G | 3189 | A | LMTK3 | A558T | C | 1672 | T |
| HTT | L2753P | T | 8403 | C | JAKMIP1 | R321C | G | 1411 | A | KIF21B | K1134Q | T | 3717 | G | LMTK3 | G1216R | C | 3646 | T |
| HTT | R1508H | G | 4668 | A | JAKMIP1 | R774C | G | 2770 | A | KIF22 | V307L | G | 959 | T | LMX1A | A102T | C | 687 | T |
| HTT | N1661D | A | 5126 | G | JAKMIP1 | T440P | T | 1768 | G | KIF24 | E580D | C | 1929 | A | LMX1B | F141L | T | 428 | C |
| HTT | Q2529* | C | 7730 | T | JAKMIP1 | Q191R | T | 1022 | C | KIF24 | D50N | C | 337 | T | LNP1 | F89V | T | 1127 | G |
| HTT | R924L | G | 2916 | T | JAKMIP1 | E13K | C | 487 | T | KIF24 | R231C | G | 880 | A | LNP1 | F124C | T | 1233 | G |
| HUS1B | V231A | A | 711 | G | JAKMIP2 | E320K | C | 1426 | T | KIF24 | R240H | C | 908 | T | LNX1 | P347H | G | 1355 | T |
| HUWE1 | E50K | C | 550 | T | JAKMIP2 | T749M | G | 2714 | A | KIF24 | - | T | 0 | C | LNX1 | L65I | G | 508 | T |
| HUWE1 | V2613G | A | 8240 | C | JAKMIP2 | A18V | G | 521 | A | KIF24 | R76C | G | 415 | A | LNX1 | R682I | C | 2360 | A |
| HUWE1 | A3007V | G | 9422 | A | JAKMIP2 | E730* | C | 2656 | A | KIF24 | F124L | G | 561 | T | LONP1 | A817T | C | 2482 | T |
| HUWE1 | R2603W | G | 8209 | A | JAKMIP2 | R587Q | C | 2228 | T | KIF25 | S221G | A | 1052 | G | LONP1 | A94T | C | 313 | T |
| HUWE1 | R3782C | G | 1174 6 | A | JAKMIP3 | R343C | C | 1165 | T | KIF25 | S52Y | C | 546 | A | LONP2 | P247H | C | 833 | A |

| | | | | | | | | | | | | | | | | | | | |
|---|---|---|---|---|---|---|---|---|---|---|---|---|---|---|---|---|---|---|---|
| HUWE1 | P4237L | G | 1311 2 | A | JAKMIP3 | R58W | C | 310 | T | KIF26A | R627H | G | 1880 | A | LONP2 | L476I | C | 1519 | A |
| HUWE1 | K414E | T | 1642 | C | JAKMIP3 | F364C | T | 1229 | G | KIF26A | A1331V | C | 3992 | T | LONRF2 | V556I | C | 2306 | T |
| HUWE1 | L4229V | A | 1308 7 | C | JARID2 | T508M | C | 1767 | T | KIF26A | A1702T | G | 5104 | A | LONRF2 | S300Y | G | 1539 | T |
| HUWE1 | G913V | C | 3140 | A | JARID2 | R753H | G | 2502 | A | KIF26A | V259I | G | 775 | A | LONRF2 | - | C | 0 | A |
| HUWE1 | R4239C | G | 1311 7 | A | JARID2 | R387H | G | 1404 | A | KIF26A | R1227H | G | 3680 | A | LONRF3 | E537G | A | 1641 | G |
| HUWE1 | A3787V | G | 1176 2 | A | JARID2 | R733C | C | 2441 | T | KIF26B | A776V | C | 2327 | T | LOX | R255S | T | 1065 | G |
| HUWE1 | R2521C | G | 7963 | A | JARID2 | G471D | G | 1656 | A | KIF26B | V1901M | G | 5701 | A | LOX | R337Q | C | 1310 | T |
| HUWE1 | H4100Y | G | 1270 0 | A | JARID2 | A49V | C | 390 | T | KIF26B | E1899K | G | 5695 | A | LOX | R281Q | C | 1142 | T |
| HVCN1 | W45L | C | 156 | A | JARID2 | S166Y | C | 741 | A | KIF26B | R456H | G | 1367 | A | LOXHD1 | L1481I | G | 4588 | T |
| HYAL1 | R240C | G | 897 | A | JAZF1 | R203C | G | 773 | A | KIF26B | G1579V | G | 4736 | T | LOXHD1 | R1380H | C | 4286 | T |
| HYAL2 | T334A | T | 3293 | C | JDP2 | G4D | G | 204 | A | KIF26B | R404Q | G | 1211 | A | LOXHD1 | Q925H | C | 2922 | A |
| HYAL2 | R116C | G | 2639 | A | JHDM1D | S848N | C | 2641 | T | KIF26B | R2024H | G | 6071 | A | LOXHD1 | R722W | G | 2311 | A |
| HYAL2 | G321S | C | 3254 | T | JKAMP | E254A | A | 761 | C | KIF27 | E774K | C | 2464 | T | LOXHD1 | W1677* | C | 5178 | T |
| HYAL3 | A109V | G | 599 | A | JKAMP | F78C | T | 233 | G | KIF27 | R623Q | C | 2012 | T | LOXHD1 | M1603I | C | 4956 | A |
| HYAL3 | R291I | C | 1145 | A | JKAMP | F312C | T | 935 | G | KIF27 | R804C | G | 2554 | A | LOXHD1 | A758V | G | 2420 | A |
| HYAL3 | H255R | T | 1037 | C | JMJD1C | A2208V | G | 6842 | A | KIF27 | E1136* | C | 3550 | A | LOXHD1 | L297R | A | 1037 | C |
| HYAL4 | K279N | A | 1475 | T | JMJD1C | N1545H | T | 4852 | G | KIF27 | L1055I | G | 3307 | T | LOXHD1 | G193D | C | 725 | T |
| HYDIN2 | R163* | G | 615 | A | JMJD1C | F1473C | A | 4637 | C | KIF27 | R623Q | C | 2012 | T | LOXHD1 | A171D | G | 510 | T |
| HYDIN2 | V2565I | C | 7821 | T | JMJD1C | S1714P | A | 5359 | G | KIF2A | E34* | G | 121 | T | LOXHD1 | C844W | G | 2679 | C |
| HYI | R118Q | C | 353 | T | JMJD1C | R2309C | G | 7144 | A | KIF2A | D242Y | G | 745 | T | LOXL2 | A133V | G | 737 | A |
| HYI | R118Q | C | 353 | T | JMJD1C | E1884* | C | 5869 | A | KIF2B | Y586* | T | 1852 | A | LOXL2 | P773L | G | 2657 | A |
| HYI | K98N | T | 294 | G | JMJD6 | R374M | C | 1445 | A | KIF2B | A112T | G | 428 | A | LOXL2 | V229M | C | 1024 | T |
| HYOU1 | R304C | G | 979 | A | JMJD6 | G138D | C | 737 | T | KIF2B | T320M | C | 1053 | T | LOXL2 | R189* | G | 904 | A |
| HYOU1 | A690T | C | 2137 | T | JMJD7-PLA2G4B | L635F | C | 1912 | T | KIF2B | R293H | G | 972 | A | LOXL3 | A305V | G | 986 | A |
| HYOU1 | E654D | T | 2031 | G | JOSD1 | - | T | 1289 | A | KIF2B | L499P | T | 1590 | C | LOXL3 | V343M | C | 1099 | T |
| IAH1 | V121M | G | 398 | A | JPH1 | A653V | G | 1998 | A | KIF2B | L662* | T | 2079 | G | LOXL3 | R579* | G | 1807 | A |
| IAPP | N47I | A | 292 | T | JPH1 | A559T | C | 1715 | T | KIF2C | N186D | A | 669 | G | LOXL4 | R593Q | C | 1929 | T |
| IARS | K1063N | C | 3263 | A | JPH1 | R338H | C | 1053 | T | KIF3A | - | C | 0 | T | LPA | A1934T | C | 5845 | T |
| IARS | P543L | G | 1702 | A | JPH2 | L695M | G | 2956 | T | KIF3B | S122* | C | 532 | A | LPA | - | C | 0 | T |
| IARS | W163* | C | 563 | T | JPH2 | A277T | C | 1702 | T | KIF3C | R785C | G | 2933 | A | LPA | T1691M | G | 5117 | A |
| IARS2 | P72H | C | 330 | A | JPH2 | E315K | C | 1816 | T | KIF3C | R324L | C | 1551 | A | LPA | T1183I | G | 3593 | A |
| IARS2 | P262H | C | 900 | A | JPH2 | R232H | C | 1568 | T | KIF3C | H208Y | G | 1202 | A | LPA | F2029C | A | 6131 | C |
| IARS2 | E772K | G | 2429 | A | JPH3 | I737T | T | 2452 | C | KIF3C | R758Q | C | 2853 | T | LPA | T1699A | T | 5140 | C |
| IBSP | R24Q | G | 138 | A | JPH3 | D180N | G | 780 | A | KIF4A | R806C | C | 2498 | T | LPAR1 | S363F | G | 1088 | A |
| IBTK | M797V | T | 2939 | C | JPH3 | Y61N | T | 423 | A | KIF4A | R265Q | G | 876 | A | LPAR2 | R320C | G | 1097 | A |

| | | | | | | | | | | | | | | | | | | | |
|---|---|---|---|---|---|---|---|---|---|---|---|---|---|---|---|---|---|---|---|
| IBTK | I746N | A | 2787 | T | JPH3 | G72D | G | 457 | A | KIF4B | Q906H | A | 2718 | C | LPAR3 | R322C | G | 1003 | A |
| IBTK | L632V | A | 2444 | C | JPH3 | A374T | G | 1362 | A | KIF4B | R762H | G | 2285 | A | LPAR3 | P222L | G | 704 | A |
| IBTK | S486P | A | 2006 | G | JPH3 | G513R | G | 1779 | A | KIF4B | G1100V | G | 3299 | T | LPAR5 | R119H | C | 356 | T |
| ICA1 | Q28H | C | 271 | A | JPH3 | P149L | C | 688 | T | KIF4B | K74Q | A | 220 | C | LPAR5 | S252N | C | 755 | T |
| ICA1L | S454P | A | 1518 | G | JPH3 | R629H | G | 2128 | A | KIF4B | A368T | G | 1102 | A | LPAR5 | A122T | C | 364 | T |
| ICA1L | R156H | C | 625 | T | JPH3 | G290D | G | 1111 | A | KIF4B | E567* | G | 1699 | T | LPAR5 | A206V | G | 617 | A |
| ICA1L | D256Y | C | 924 | A | JPH3 | A491V | C | 1714 | T | KIF5A | T767I | C | 2508 | T | LPAR5 | R52C | G | 154 | A |
| ICAM3 | A492V | G | 1530 | A | JPH4 | A600T | C | 1798 | T | KIF5A | E758K | G | 2480 | A | LPAR6 | V290A | A | 1668 | G |
| ICAM3 | R93Q | C | 333 | T | JPH4 | R305H | C | 914 | T | KIF5A | G568R | G | 1910 | A | LPAR6 | T157A | T | 1268 | C |
| ICAM4 | R218L | G | 692 | T | JRKL | R510Q | G | 1776 | A | KIF5A | E137D | A | 619 | C | LPAR6 | S253Y | G | 1557 | T |
| ICAM5 | A230V | C | 754 | T | JRKL | E459G | A | 1623 | G | KIF5A | E486K | G | 1664 | A | LPAR6 | F238C | A | 1512 | C |
| ICK | R151Q | C | 799 | T | JRKL | F162C | T | 732 | G | KIF5B | R868* | G | 3060 | A | LPCAT1 | R104H | C | 444 | T |
| ICK | I250V | T | 1095 | C | JRKL | I269M | T | 1054 | G | KIF5B | D401G | T | 1660 | C | LPCAT1 | - | C | 0 | A |
| ICK | S174F | G | 868 | A | JRKL | E469* | G | 1652 | T | KIF5C | Q474P | A | 1789 | C | LPCAT2 | A110D | C | 513 | A |
| ICOS | R14H | G | 108 | A | JSRP1 | D23G | T | 101 | C | KIF5C | N912I | A | 3103 | T | LPCAT2 | L178M | C | 716 | A |
| ICOS | D64N | G | 257 | A | JSRP1 | E248D | C | 777 | A | KIF5C | E311* | G | 1299 | T | LPCAT4 | L405R | A | 1308 | C |
| ICT1 | A112V | C | 341 | T | JUB | Q397R | T | 1566 | C | KIF5C | E764* | G | 2658 | T | LPGAT1 | L119M | G | 582 | T |
| ID2 | P51S | C | 1011 | T | JUN | A306V | G | 1960 | A | KIF6 | R649K | C | 2041 | T | LPHN1 | R1150W | G | 3719 | A |
| IDE | R824H | C | 2528 | T | JUN | L121P | A | 1405 | G | KIF6 | T260I | G | 874 | A | LPHN1 | R231H | C | 963 | T |
| IDE | Y609S | T | 1883 | G | JUNB | R250L | G | 1025 | T | KIF7 | D332N | C | 1071 | T | LPHN1 | R987H | C | 3231 | T |
| IDE | R839* | G | 2572 | A | JUP | R444H | C | 1450 | T | KIF7 | R859Q | C | 2653 | T | LPHN1 | K146N | C | 709 | A |
| IDE | E751D | T | 2310 | G | JUP | A620T | C | 1977 | T | KIF7 | R841W | G | 2598 | A | LPHN1 | R36C | G | 377 | A |
| IDH2 | M248T | A | 857 | G | JUP | N309D | T | 1044 | C | KIF7 | R841W | G | 2598 | A | LPHN2 | R1150C | C | 4093 | T |
| IDH3A | R326I | G | 1060 | T | KAL1 | R651W | G | 2101 | A | KIF7 | T354M | G | 1138 | A | LPHN2 | R179C | C | 1180 | T |
| IDH3B | V352A | A | 1086 | G | KAL1 | R679I | C | 2186 | A | KIFAP3 | L407I | G | 1447 | T | LPHN2 | D571G | A | 2357 | G |
| IDH3B | K193R | T | 609 | C | KAL1 | R262* | G | 934 | A | KIFAP3 | D604Y | C | 2038 | A | LPHN2 | V219I | G | 1300 | A |
| IDH3G | E173K | C | 714 | T | KALRN | V2585M | G | 7853 | A | KIFC3 | A308V | G | 1116 | A | LPHN2 | M1154I | G | 4107 | A |
| IDH3G | A195V | G | 781 | A | KALRN | R1125W | C | 3473 | T | KIFC3 | R218* | G | 845 | A | LPHN2 | T301M | C | 1547 | T |
| IDI2 | R207W | G | 684 | A | KALRN | T352M | C | 1155 | T | KIFC3 | R776S | C | 2521 | A | LPHN2 | T424P | A | 1915 | C |
| IDO1 | R77C | C | 343 | T | KALRN | E1427K | G | 4379 | A | KIFC3 | R656Q | C | 2160 | T | LPHN2 | Y1421* | C | 4908 | G |
| IDUA | R163C | C | 575 | T | KALRN | A1390V | C | 4269 | T | KIN | G295R | C | 931 | T | LPHN3 | P1558H | C | 4673 | A |
| IDUA | D435N | G | 1391 | A | KALRN | R2962H | G | 8985 | A | KIN | R82K | C | 293 | T | LPHN3 | M663L | A | 1987 | T |
| IER2 | Q161* | C | 809 | T | KALRN | K1294N | G | 3982 | T | KIN | N74H | T | 268 | G | LPHN3 | W1090S | G | 3269 | C |
| IER3IP1 | L79V | A | 332 | C | KALRN | N2552D | A | 7754 | G | KIR2DL4 | R54C | C | 160 | T | LPHN3 | K132N | A | 396 | C |
| IER5L | Q308R | T | 1132 | C | KALRN | R2764Q | G | 8391 | A | KIR2DS4 | H173Y | C | 517 | T | LPHN3 | E1391* | G | 4171 | T |
| IFFO1 | R83W | G | 281 | A | KALRN | L1304F | A | 4012 | C | KIR2DS4 | N84S | A | 251 | G | LPIN1 | V451A | T | 1714 | C |
| IFFO1 | R408H | C | 1257 | T | KALRN | R302H | G | 1005 | A | KIR3DL1 | L451I | C | 1351 | A | LPIN1 | K342N | A | 1388 | C |
| IFFO1 | W197* | C | 625 | T | KALRN | Q2541P | A | 7722 | C | KIR3DX1 | T64P | A | 228 | C | LPIN1 | Y837H | T | 2871 | C |
| IFFO1 | R250Q | C | 783 | T | KALRN | - | T | 0 | C | KIRREL | R506* | C | 1920 | T | LPIN2 | P373S | G | 1356 | A |

| | | | | | | | | | | | | | | | | | | | |
|---|---|---|---|---|---|---|---|---|---|---|---|---|---|---|---|---|---|---|---|
| IFFO2 | D341N | C | 1021 | T | KANK1 | E406K | G | 1868 | A | KIRREL | V6I | G | 420 | A | LPIN2 | K553N | C | 1898 | A |
| IFI16 | P405L | C | 1474 | T | KANK1 | A849E | C | 3198 | A | KIRREL | K315T | A | 1348 | C | LPIN3 | K799Q | A | 2486 | C |
| IFI27 | A115V | C | 467 | T | KANK2 | A644T | C | 2245 | T | KIRREL | P335T | C | 1407 | A | LPIN3 | Y687H | T | 2150 | C |
| IFI27L1 | M51L | A | 359 | T | KANK2 | R133C | G | 712 | A | KIRREL | N463T | A | 1792 | C | LPIN3 | G647D | G | 2031 | A |
| IFI27L1 | A30V | C | 297 | T | KANK4 | R471H | C | 1412 | T | KIRREL2 | G621* | G | 2073 | T | LPIN3 | G186C | G | 647 | T |
| IFI30 | A190T | G | 641 | A | KANK4 | A115T | C | 343 | T | KIRREL2 | P702L | C | 2317 | T | LPIN3 | R807W | C | 2510 | T |
| IFI35 | R110W | C | 524 | T | KANK4 | D821N | C | 2461 | T | KIRREL2 | R361H | G | 1294 | A | LPIN3 | S161Y | C | 573 | A |
| IFI35 | R135Q | G | 600 | A | KANK4 | R385* | G | 1153 | A | KIRREL3 | P124S | G | 443 | A | LPO | R80Q | G | 494 | A |
| IFI44 | R412* | C | 1319 | T | KARS | V99M | C | 417 | T | KIRREL3 | V737I | C | 2282 | T | LPP | R568Q | G | 1949 | A |
| IFI44 | A416T | G | 1331 | A | KAT2A | R251W | G | 805 | A | KIRREL3 | - | T | 0 | C | LPP | V423I | G | 1513 | A |
| IFI44L | R427W | C | 1458 | T | KAT2A | F199V | A | 649 | C | KIRREL3 | F267L | G | 874 | T | LPP | Q454* | C | 1606 | T |
| IFI44L | K389N | A | 1346 | C | KAT2B | R490H | G | 1924 | A | KIRREL3 | S174F | G | 594 | A | LPP | P167L | C | 746 | T |
| IFI44L | A447V | C | 1519 | T | KATNA1 | L33F | G | 142 | A | KIT | A616V | C | 1944 | T | LPXN | R330Q | C | 989 | T |
| IFI6 | A62T | C | 272 | T | KATNA1 | E482D | C | 1491 | A | KIT | R49C | C | 242 | T | LPXN | R215H | C | 644 | T |
| IFIH1 | E642* | C | 2320 | A | KATNA1 | D190H | C | 613 | G | KIT | D52G | A | 252 | G | LRAT | T88M | C | 325 | T |
| IFIH1 | R806H | C | 2813 | T | KATNA1 | R298* | G | 937 | A | KIT | T847M | C | 2637 | T | LRBA | D1657V | T | 5214 | A |
| IFIT1 | E140* | G | 585 | T | KATNAL1 | F298I | A | 1060 | T | KL | R920H | G | 2767 | A | LRBA | T1588M | G | 5007 | A |
| IFIT1B | R208* | C | 702 | T | KATNAL1 | N168T | T | 671 | G | KL | A984V | C | 2959 | T | LRBA | R2144C | G | 6674 | A |
| IFIT2 | R184H | G | 720 | A | KATNAL1 | M33V | T | 265 | C | KL | Q586R | A | 1765 | G | LRBA | A901T | C | 2945 | T |
| IFIT2 | R251H | G | 921 | A | KATNAL1 | L389R | A | 1334 | C | KL | S996Y | C | 2995 | A | LRBA | T2307A | T | 7163 | C |
| IFIT3 | F172L | C | 696 | A | KATNAL1 | G251D | C | 920 | T | KLB | F369L | C | 1204 | A | LRBA | E2103K | C | 6551 | T |
| IFIT3 | A155T | G | 643 | A | KATNAL2 | E398* | G | 1192 | T | KLB | I769V | A | 2402 | G | LRBA | E757D | T | 2515 | G |
| IFIT3 | A179V | C | 716 | T | KATNAL2 | T115A | A | 343 | G | KLB | A619T | G | 1952 | A | LRCH1 | R152Q | G | 592 | A |
| IFIT3 | R245C | C | 913 | T | KATNAL2 | A469V | C | 1406 | T | KLB | F53L | C | 256 | A | LRCH2 | C307F | C | 951 | A |
| IFLTD1 | R388L | C | 1408 | A | KATNAL2 | G121V | G | 362 | T | KLB | K346N | G | 1135 | T | LRCH2 | P68S | G | 233 | A |
| IFLTD1 | S89Y | G | 511 | T | KATNAL2 | I72L | A | 214 | C | KLC2 | A266V | C | 1040 | T | LRCH2 | A87T | C | 290 | T |
| IFLTD1 | Q384H | C | 1397 | A | KATNAL2 | S125L | C | 374 | T | KLC3 | V472M | G | 1516 | A | LRCH3 | P42Q | C | 169 | A |
| IFLTD1 | E342G | T | 1270 | C | KATNAL2 | L267I | C | 799 | A | KLC4 | - | T | 2265 | C | LRCH3 | D200Y | G | 642 | T |
| IFLTD1 | R302C | G | 1149 | A | KATNB1 | Q14* | C | 432 | T | KLF1 | W318G | A | 1015 | C | LRCH3 | R349* | C | 1089 | T |
| IFLTD1 | S39Y | G | 361 | T | KATNB1 | V150M | G | 840 | A | KLF10 | S329* | G | 1140 | T | LRCH3 | R91Q | G | 316 | A |
| IFNA13 | E190K | C | 634 | T | KATNB1 | A379T | G | 1527 | A | KLF10 | A430V | G | 1443 | A | LRCH4 | P23H | G | 121 | T |
| IFNA2 | P27S | G | 147 | A | KBTBD10 | R59C | C | 252 | T | KLF10 | L178I | G | 686 | T | LRDD | R718W | G | 2294 | A |
| IFNA5 | L80I | G | 294 | T | KBTBD10 | L63V | T | 264 | G | KLF10 | P278S | G | 986 | A | LRDD | S523N | C | 1710 | T |
| IFNAR1 | K211N | A | 785 | C | KBTBD12 | K395E | A | 1650 | G | KLF10 | A12V | G | 189 | A | LRDD | L89M | G | 407 | T |
| IFNE | F60V | A | 797 | C | KBTBD12 | H561N | C | 2148 | A | KLF11 | M508T | T | 1685 | C | LRDD | K879* | T | 2777 | A |
| IFNG | K103E | T | 435 | C | KBTBD12 | G321* | G | 1428 | T | KLF12 | A136V | G | 434 | A | LRDD | R637Q | C | 2052 | T |
| IFNG | - | A | 0 | G | KBTBD12 | E26* | G | 543 | T | KLF12 | N59S | T | 203 | C | LRFN1 | A742V | G | 2225 | A |
| IFNG | K110N | C | 458 | A | KBTBD13 | R188H | G | 563 | A | KLF12 | R396H | C | 1214 | T | LRFN1 | A712V | G | 2135 | A |
| IFNGR1 | Y172C | T | 637 | C | KBTBD13 | L35F | C | 103 | T | KLF13 | R219C | C | 1013 | T | LRFN1 | A589T | C | 1765 | T |

| | | | | | | | | | | | | | | | | | | | |
|---|---|---|---|---|---|---|---|---|---|---|---|---|---|---|---|---|---|---|---|
| IFNGR1 | L247I | G | 861 | T | KBTBD2 | E131D | C | 1093 | A | KLF14 | K208E | T | 622 | C | LRFN1 | V92A | A | 275 | G |
| IFNGR1 | E110* | C | 450 | A | KBTBD3 | F190V | A | 1208 | C | KLF14 | G221D | C | 662 | T | LRFN1 | S731P | A | 2191 | G |
| IFNGR1 | E38* | C | 234 | A | KBTBD3 | R356Q | C | 1707 | T | KLF15 | R343H | C | 1259 | T | LRFN1 | D498N | C | 1492 | T |
| IFNK | K176N | G | 551 | T | KBTBD3 | R356* | G | 1706 | A | KLF15 | G302S | C | 1135 | T | LRFN2 | S236C | G | 1250 | C |
| IFRD1 | K357T | A | 1540 | C | KBTBD3 | Q309K | G | 1565 | T | KLF15 | R110G | T | 559 | C | LRFN2 | D725N | C | 2716 | T |
| IFRD2 | Q301H | T | 903 | G | KBTBD5 | G205S | G | 713 | A | KLF17 | P102H | C | 363 | A | LRFN2 | R755C | G | 2806 | A |
| IFRD2 | V114M | C | 340 | T | KBTBD5 | R402H | G | 1305 | A | KLF17 | T125M | C | 432 | T | LRFN3 | A523V | C | 2292 | T |
| IFT122 | A848T | G | 2734 | A | KBTBD5 | V220I | G | 758 | A | KLF17 | L132P | T | 453 | C | LRFN3 | P12S | C | 758 | T |
| IFT122 | A701V | C | 2294 | T | KBTBD6 | R670H | C | 2244 | T | KLF2 | G308S | G | 1020 | A | LRFN3 | V470I | G | 2132 | A |
| IFT122 | D899Y | G | 2887 | T | KBTBD6 | R483* | G | 1682 | A | KLF3 | P139L | C | 721 | T | LRFN3 | T595M | C | 2508 | T |
| IFT140 | L980P | A | 3164 | G | KBTBD6 | S500G | T | 1733 | C | KLF3 | H314R | A | 1246 | G | LRFN4 | R81H | G | 582 | A |
| IFT140 | R63L | C | 413 | A | KBTBD6 | R670C | G | 2243 | A | KLF3 | K105N | A | 620 | C | LRFN5 | K420T | A | 2457 | C |
| IFT140 | A284T | C | 1075 | T | KBTBD6 | K579T | T | 1971 | G | KLF4 | D441N | C | 1795 | T | LRFN5 | I536F | A | 2804 | T |
| IFT140 | T72M | G | 440 | A | KBTBD6 | I25M | A | 310 | C | KLF5 | D418G | A | 1789 | G | LRFN5 | R681H | G | 3240 | A |
| IFT140 | H57Y | G | 394 | A | KBTBD7 | A175T | C | 832 | T | KLF5 | T398A | A | 1728 | G | LRFN5 | L77V | T | 1427 | G |
| IFT140 | R1201C | G | 3826 | A | KBTBD7 | E624K | C | 2179 | T | KLF6 | G167R | C | 760 | T | LRFN5 | T332A | A | 2192 | G |
| IFT140 | D44G | T | 356 | C | KBTBD7 | Y497H | A | 1798 | G | KLF8 | P141T | C | 709 | A | LRFN5 | P378H | C | 2331 | A |
| IFT140 | D1324N | C | 4195 | T | KBTBD7 | V265A | A | 1103 | G | KLF9 | A222S | C | 1925 | A | LRFN5 | R445H | G | 2532 | A |
| IFT172 | S249N | C | 850 | T | KBTBD8 | R92Q | G | 324 | A | KLHDC1 | A382V | C | 1235 | T | LRFN5 | K646N | G | 3136 | T |
| IFT172 | T147I | G | 544 | A | KBTBD8 | Q560H | A | 1729 | T | KLHDC10 | D8Y | G | 156 | T | LRGUK | S633L | C | 1967 | T |
| IFT172 | Y91H | A | 375 | G | KCMF1 | R246W | C | 736 | T | KLHDC3 | I281T | T | 1011 | C | LRGUK | P413H | C | 1307 | A |
| IFT172 | G675S | C | 2127 | T | KCMF1 | - | T | 1147 | G | KLHDC4 | R432H | C | 1402 | T | LRGUK | N182S | A | 614 | G |
| IFT172 | R1667C | G | 5103 | A | KCNA1 | V486I | G | 2561 | A | KLHDC7A | R681H | G | 2094 | A | LRGUK | R443I | G | 1397 | T |
| IFT172 | D605A | T | 1918 | G | KCNA1 | V388M | G | 2267 | A | KLHDC7A | S163T | G | 540 | C | LRGUK | V498A | T | 1562 | C |
| IFT172 | V211M | C | 735 | T | KCNA10 | S54G | T | 548 | C | KLHDC7A | C573Y | G | 1770 | A | LRGUK | E710* | G | 2197 | T |
| IFT52 | R274Q | G | 951 | A | KCNA10 | R356C | G | 1454 | A | KLHDC7A | A346V | C | 1089 | T | LRIG1 | V650A | A | 2474 | G |
| IFT57 | K394N | C | 1182 | A | KCNA10 | T61M | G | 570 | A | KLHDC7B | E444K | G | 1464 | A | LRIG1 | P452A | G | 1879 | C |
| IFT57 | E224* | C | 670 | A | KCNA2 | P105S | G | 809 | A | KLHDC8B | A74V | C | 430 | T | LRIG1 | A377T | C | 1654 | T |
| IFT74 | Y97C | A | 417 | G | KCNA2 | F241L | G | 1219 | T | KLHDC9 | R318H | G | 1095 | A | LRIG1 | G770D | C | 2834 | T |
| IFT74 | A558V | C | 1800 | T | KCNA2 | R73* | G | 713 | A | KLHL1 | A319D | G | 1716 | T | LRIG1 | N292T | T | 1400 | G |
| IFT80 | K131Q | T | 824 | G | KCNA3 | R516* | G | 1770 | A | KLHL10 | R287H | G | 1013 | A | LRIG2 | D81E | T | 441 | G |
| IFT81 | K114N | A | 890 | C | KCNA3 | R105H | C | 538 | T | KLHL10 | K77T | A | 383 | C | LRIG3 | R203* | G | 853 | A |
| IFT81 | E322* | G | 1512 | T | KCNA3 | R367H | C | 1324 | T | KLHL12 | E491G | T | 1691 | C | LRIG3 | R7H | C | 266 | T |
| IFT81 | L546I | T | 2184 | A | KCNA3 | R105H | C | 538 | T | KLHL13 | E364D | T | 2002 | G | LRIG3 | E982K | C | 3190 | T |
| IFT81 | K528N | G | 2132 | T | KCNA3 | Q271* | G | 1035 | A | KLHL14 | R560* | G | 2066 | A | LRIG3 | E266D | T | 1044 | G |
| IFT81 | L642V | T | 2472 | G | KCNA4 | E2* | C | 1156 | A | KLHL14 | F393C | A | 1566 | C | LRIT1 | R84H | C | 273 | T |
| IFT88 | I731T | T | 2519 | C | KCNA4 | S603Y | G | 2960 | T | KLHL14 | D570G | T | 2097 | C | LRIT1 | R429* | G | 1307 | A |
| IFT88 | S612C | C | 2162 | G | KCNA5 | P91L | C | 501 | T | KLHL15 | G287E | C | 1116 | T | LRIT1 | S38G | T | 134 | C |
| IFT88 | E39K | G | 442 | A | KCNA5 | R354Q | G | 1290 | A | KLHL17 | R324M | G | 1078 | T | LRIT2 | A447D | G | 1346 | T |

| | | | | | | | | | | | | | | | | | | | |
|---|---|---|---|---|---|---|---|---|---|---|---|---|---|---|---|---|---|---|---|
| IFT88 | K749T | A | 2573 | C | KCNA5 | R597Q | G | 2019 | A | KLHL18 | R53Q | G | 178 | A | LRIT2 | Q254H | C | 768 | A |
| IGBP1 | K253R | A | 1257 | G | KCNA6 | R524I | G | 2437 | T | KLHL2 | R372C | C | 1364 | T | LRIT3 | A278T | G | 1025 | A |
| IGBP1 | R330M | G | 1488 | T | KCNA7 | A183V | G | 904 | A | KLHL2 | G589R | G | 2015 | A | LRIT3 | A469V | C | 1599 | T |
| IGDCC3 | A493T | C | 1729 | T | KCNA7 | R293Q | C | 1234 | T | KLHL21 | A179V | G | 588 | A | LRIT3 | V529D | T | 1779 | A |
| IGDCC3 | R132C | G | 646 | A | KCNAB1 | I188S | T | 627 | G | KLHL21 | V359M | C | 1127 | T | LRMP | R400G | A | 2064 | G |
| IGDCC3 | R132C | G | 646 | A | KCNAB1 | L271I | C | 875 | A | KLHL22 | V351I | C | 1208 | T | LRMP | R216I | G | 1513 | T |
| IGDCC3 | - | A | 0 | G | KCNAB3 | A377T | C | 1129 | T | KLHL23 | I100L | A | 636 | C | LRMP | V71M | G | 1077 | A |
| IGDCC4 | P271S | G | 1021 | A | KCNB1 | A238T | C | 879 | T | KLHL23 | N332T | A | 1333 | C | LRP10 | R561H | G | 2269 | A |
| IGDCC4 | S553G | T | 1867 | C | KCNB1 | T376A | T | 1293 | C | KLHL24 | F77C | T | 580 | G | LRP11 | A239T | C | 721 | T |
| IGDCC4 | D473V | T | 1628 | A | KCNB1 | D358N | C | 1239 | T | KLHL24 | E108D | G | 674 | C | LRP11 | D337A | T | 1016 | G |
| IGF1 | F19L | G | 239 | T | KCNB1 | L155R | A | 631 | C | KLHL24 | K188N | A | 914 | C | LRP12 | P129L | G | 481 | A |
| IGF1R | T500M | C | 2110 | T | KCNB1 | A702V | G | 2272 | A | KLHL25 | G450R | C | 1623 | T | LRP12 | V229F | C | 780 | A |
| IGF1R | L181M | C | 1152 | A | KCNB1 | K807N | C | 2588 | A | KLHL26 | R305C | C | 940 | T | LRP12 | E235K | C | 798 | T |
| IGF1R | D918Y | G | 3363 | T | KCNB1 | K253N | C | 926 | A | KLHL26 | A454T | G | 1387 | A | LRP1B | L2283P | A | 7820 | G |
| IGF1R | A943T | G | 3438 | A | KCNB2 | N526D | A | 1576 | G | KLHL28 | R577H | C | 1934 | T | LRP1B | L4509I | G | 1449 7 | T |
| IGF1R | R513Q | G | 2149 | A | KCNB2 | T214M | C | 641 | T | KLHL29 | G145V | G | 1151 | T | LRP1B | D1657Y | C | 5941 | A |
| IGF1R | N1049D | A | 3756 | G | KCNB2 | Y248C | A | 743 | G | KLHL3 | V507I | C | 1963 | T | LRP1B | R1815W | G | 6415 | A |
| IGF2 | P158L | G | 858 | A | KCNB2 | F261L | C | 783 | A | KLHL3 | M273I | C | 1263 | A | LRP1B | I1460T | A | 5351 | G |
| IGF2 | Q33H | C | 484 | A | KCNC1 | A421V | C | 1317 | T | KLHL3 | Q381* | G | 1585 | A | LRP1B | R3069C | G | 1017 7 | A |
| IGF2 | V29I | C | 470 | T | KCNC1 | V356M | G | 1121 | A | KLHL3 | R240Q | C | 1163 | T | LRP1B | S3238G | T | 1068 4 | C |
| IGF2AS | R143* | C | 427 | T | KCNC2 | A227D | G | 1224 | T | KLHL30 | Q24* | C | 177 | T | LRP1B | N210Y | T | 1600 | A |
| IGF2BP1 | E62* | G | 518 | T | KCNC2 | I243S | A | 1272 | C | KLHL30 | R202C | C | 711 | T | LRP1B | S3497N | C | 1146 2 | T |
| IGF2BP1 | E352D | G | 1390 | T | KCNC2 | R33H | C | 642 | T | KLHL30 | V561I | G | 1788 | A | LRP1B | N1298K | A | 4866 | T |
| IGF2BP1 | R199W | C | 929 | T | KCNC3 | A326T | C | 1140 | T | KLHL31 | R67W | G | 340 | A | LRP1B | D539N | C | 2587 | T |
| IGF2BP2 | R256H | C | 863 | T | KCNC3 | R435H | C | 1468 | T | KLHL31 | R196W | G | 727 | A | LRP1B | G2038A | C | 7085 | G |
| IGF2BP3 | G472* | C | 1771 | A | KCNC4 | S335P | T | 1030 | C | KLHL32 | Y371H | T | 1474 | C | LRP1B | G35D | C | 1076 | T |
| IGF2BP3 | A14T | C | 397 | T | KCND1 | G159D | C | 1774 | T | KLHL32 | R477H | G | 1793 | A | LRP1B | R2075C | G | 7195 | A |
| IGF2BP3 | T306I | G | 1274 | A | KCND1 | R49W | G | 1443 | A | KLHL33 | R246Q | C | 737 | T | LRP1B | N4223H | T | 1363 9 | G |
| IGF2BP3 | E477* | C | 1786 | A | KCND1 | E492* | C | 2772 | A | KLHL33 | R263W | G | 787 | A | LRP1B | E3741* | C | 1219 3 | A |
| IGF2BP3 | E235* | C | 1060 | A | KCND2 | R247H | G | 1705 | A | KLHL34 | R190W | G | 1110 | A | LRP1B | R3547I | C | 1161 2 | A |
| IGF2R | P79S | C | 383 | T | KCND2 | E117K | G | 1314 | A | KLHL34 | R415Q | C | 1786 | T | LRP1B | K2557E | T | 8641 | C |
| IGF2R | R1260W | C | 3926 | T | KCND2 | D462G | A | 2350 | G | KLHL34 | D148G | T | 985 | C | LRP1B | K2113N | C | 7311 | A |
| IGF2R | A1074T | G | 3368 | A | KCND2 | R247C | C | 1704 | T | KLHL34 | A166T | C | 1038 | T | LRP1B | S1772* | G | 6287 | T |
| IGF2R | R256C | C | 914 | T | KCND2 | R539C | C | 2580 | T | KLHL34 | R234W | G | 1242 | A | LRP1B | T1714I | G | 6113 | A |

| | | | | | | | | | | | | | | | | | | | |
|---|---|---|---|---|---|---|---|---|---|---|---|---|---|---|---|---|---|---|---|
| IGF2R | G1769C | G | 5453 | T | KCND2 | R311H | G | 1897 | A | KLHL34 | T406M | G | 1759 | A | LRP1B | R1072C | G | 4186 | A |
| IGF2R | - | G | 0 | A | KCND3 | R308Q | C | 1403 | T | KLHL35 | P333L | G | 1002 | A | LRP1B | K517R | T | 2522 | C |
| IGF2R | A1763V | C | 5436 | T | KCND3 | R550H | C | 2129 | T | KLHL36 | R614W | C | 2005 | T | LRP1B | E145* | C | 1405 | A |
| IGF2R | R1484M | G | 4599 | T | KCND3 | R293H | C | 1358 | T | KLHL36 | Y519C | A | 1721 | G | LRP1B | Q30H | C | 1062 | A |
| IGF2R | F1909L | C | 5875 | A | KCND3 | T57M | G | 650 | A | KLHL36 | R334W | C | 1165 | T | LRP1B | W3985* | C | 12926 | T |
| IGFBP1 | Y158C | A | 769 | G | KCND3 | P400L | G | 1679 | A | KLHL36 | R528H | G | 1748 | A | LRP1B | R2597C | G | 8761 | A |
| IGFBP3 | R150C | G | 559 | A | KCND3 | S438L | G | 1793 | A | KLHL38 | I537T | A | 1634 | G | LRP2 | G2701D | C | 8388 | T |
| IGFBP3 | K253E | T | 868 | C | KCNE4 | S23P | T | 551 | C | KLHL38 | A427T | C | 1303 | T | LRP2 | T620I | G | 2145 | A |
| IGFBP3 | S146L | G | 548 | A | KCNE4 | R24H | G | 555 | A | KLHL38 | R321W | G | 985 | A | LRP2 | L2004M | G | 6296 | T |
| IGFL1 | C81* | C | 266 | A | KCNF1 | R417C | C | 1739 | T | KLHL38 | S555F | G | 1688 | A | LRP2 | R2095* | G | 6569 | A |
| IGFL2 | W43* | G | 420 | A | KCNF1 | S307T | T | 1409 | A | KLHL38 | K468N | C | 1428 | A | LRP2 | R2095* | G | 6569 | A |
| IGFL4 | A33V | G | 98 | A | KCNF1 | E426K | G | 1766 | A | KLHL4 | M587I | G | 1841 | A | LRP2 | D2054E | A | 6448 | C |
| IGFN1 | R49Q | G | 276 | A | KCNG1 | P48L | G | 429 | A | KLHL4 | R288Q | G | 943 | A | LRP2 | R3726H | C | 11463 | T |
| IGFN1 | V540I | G | 1748 | A | KCNG1 | E376K | C | 1412 | T | KLHL4 | S369Y | C | 1186 | A | LRP2 | R2505H | C | 7800 | T |
| IGHA2 | A50V | G | 149 | A | KCNG1 | R64W | G | 476 | A | KLHL4 | G483* | G | 1527 | T | LRP2 | R3726C | G | 11462 | A |
| IGHA2 | A280V | G | 839 | A | KCNG2 | A337V | C | 1010 | T | KLHL4 | R694I | G | 2161 | T | LRP2 | W2322* | C | 7252 | T |
| IGHD3-3 | E6Q | C | 16 | G | KCNG2 | A283V | C | 848 | T | KLHL5 | A226T | G | 751 | A | LRP2 | N4312S | T | 13221 | C |
| IGHE | S119L | G | 356 | A | KCNG2 | W108C | G | 324 | T | KLHL5 | - | A | 0 | G | LRP2 | F4014I | A | 12326 | T |
| IGHE | R289Q | C | 866 | T | KCNG3 | V77A | A | 826 | G | KLHL5 | S171R | T | 588 | G | LRP2 | R3765* | G | 11579 | A |
| IGHG2 | R180H | C | 539 | T | KCNH1 | S879L | G | 2664 | A | KLHL5 | R644G | A | 2005 | G | LRP2 | L2954I | G | 9146 | T |
| IGHG3 | R170W | G | 507 | A | KCNH1 | T698M | G | 2121 | A | KLHL5 | S50Y | C | 224 | A | LRP2 | K2380N | C | 7426 | A |
| IGHG4 | K126N | T | 378 | G | KCNH1 | S854Y | G | 2589 | T | KLHL5 | E365K | G | 1168 | A | LRP2 | S1816Y | G | 5733 | T |
| IGHM | T160M | G | 1127 | A | KCNH1 | A816V | G | 2475 | A | KLHL5 | D582G | A | 1820 | G | LRP2 | F1154C | A | 3747 | C |
| IGHMBP2 | A247T | G | 841 | A | KCNH1 | R791H | C | 2400 | T | KLHL6 | D37Y | C | 145 | A | LRP2 | K1141N | C | 3709 | A |
| IGHMBP2 | E7* | G | 121 | T | KCNH1 | F648L | A | 1970 | G | KLHL6 | G565R | C | 1729 | T | LRP2 | H763R | T | 2574 | C |
| IGHV1-18 | L83F | G | 306 | A | KCNH1 | R520W | G | 1586 | A | KLHL6 | A500V | G | 1535 | A | LRP2 | G514* | C | 1826 | A |
| IGHV1-24 | E29D | C | 147 | A | KCNH2 | A561V | G | 2084 | A | KLHL6 | L266M | G | 832 | T | LRP2 | V285F | C | 1139 | A |
| IGHV1-45 | A98T | C | 350 | T | KCNH2 | A228T | C | 1084 | T | KLHL8 | R404* | G | 1552 | A | LRP2 | R2056Q | C | 6453 | T |
| IGHV1-45 | R6I | C | 75 | A | KCNH2 | L1136I | G | 3808 | T | KLHL8 | D120E | G | 702 | T | LRP2 | G3085E | C | 9540 | T |
| IGHV1-58 | R63C | G | 248 | A | KCNH3 | G104V | G | 571 | T | KLHL8 | D112V | T | 677 | A | LRP2 | R3835C | G | 11789 | A |
| IGHV2-70 | Q35H | C | 181 | A | KCNH3 | A368T | G | 1362 | A | KLHL8 | A538T | C | 1954 | T | LRP3 | L512V | C | 1627 | G |
| IGHV2-70 | R59H | C | 252 | T | KCNH3 | D680N | G | 2298 | A | KLHL8 | I415V | T | 1585 | C | LRP3 | T527M | C | 1673 | T |
| IGHV3-13 | G107R | C | 399 | T | KCNH3 | Y524C | A | 1831 | G | KLHL8 | E62V | T | 527 | A | LRP3 | R223C | C | 760 | T |
| IGHV3-21 | V56G | A | 246 | C | KCNH3 | A547E | C | 1900 | A | KLHL9 | R221C | G | 1232 | A | LRP4 | T467I | G | 1643 | A |

| | | | | | | | | | | | | | | | | | | | |
|---|---|---|---|---|---|---|---|---|---|---|---|---|---|---|---|---|---|---|---|
| IGHV3-38 | V48I | C | 220 | T | KCNH3 | K117N | G | 611 | T | KLHL9 | T442R | G | 1896 | C | LRP4 | R247C | G | 982 | A |
| IGHV3-7 | L64M | G | 269 | T | KCNH3 | F280V | T | 1098 | G | KLHL9 | L349I | G | 1616 | T | LRP4 | R524H | C | 1814 | T |
| IGHV3-74 | T97M | G | 526 | A | KCNH3 | V1033A | T | 3358 | C | KLK11 | R26H | C | 189 | T | LRP4 | L1019P | A | 3299 | G |
| IGHV3-74 | S78R | G | 470 | T | KCNH3 | R344H | G | 1291 | A | KLK11 | W88* | C | 376 | T | LRP5 | V580I | G | 1813 | A |
| IGHV3OR1 6-8 | A80T | C | 316 | T | KCNH4 | S251L | G | 1085 | A | KLK12 | C186R | A | 673 | G | LRP5 | G1276R | G | 3901 | A |
| IGHV4-28 | V108M | C | 394 | T | KCNH4 | A451V | G | 1685 | A | KLK13 | R244Q | C | 774 | T | LRP5 | V1410M | G | 4303 | A |
| IGHV4-34 | S26T | A | 107 | T | KCNH5 | A894D | G | 2950 | T | KLK14 | R86H | C | 476 | T | LRP5 | R1237Q | G | 3785 | A |
| IGHV4-39 | P68S | G | 252 | A | KCNH5 | L595M | A | 2052 | T | KLK15 | E251D | T | 784 | G | LRP5 | R752Q | G | 2330 | A |
| IGHV4-39 | T114P | T | 390 | G | KCNH5 | L709F | G | 2394 | A | KLK2 | V60M | G | 219 | A | LRP5L | R237H | C | 1234 | T |
| IGHV7-81 | D92G | T | 329 | C | KCNH6 | R447C | C | 1419 | T | KLK2 | A17V | C | 91 | T | LRP6 | V700A | A | 2241 | G |
| IGKC | A37T | C | 108 | T | KCNH6 | L239M | C | 795 | A | KLK2 | - | G | 0 | T | LRP6 | S1020G | T | 3200 | C |
| IGKV1-16 | Q77K | G | 256 | T | KCNH6 | A40V | C | 199 | T | KLK3 | S11A | T | 72 | G | LRP6 | F1502C | A | 4647 | C |
| IGKV1-16 | K83N | C | 276 | G | KCNH7 | R1082I | C | 3345 | A | KLK6 | S212A | A | 877 | C | LRP8 | G150R | C | 550 | T |
| IGKV1D-17 | Q60* | C | 359 | T | KCNH7 | I150T | A | 549 | G | KLK7 | T214A | T | 742 | C | LRP8 | R463C | G | 1489 | A |
| IGKV3D-11 | E101D | A | 400 | C | KCNH7 | D603N | C | 1907 | T | KLK7 | Q26H | C | 180 | A | LRP8 | R102W | G | 406 | A |
| IGKV3D-20 | T25M | C | 171 | T | KCNH7 | G901* | C | 2801 | A | KLK8 | L28M | G | 261 | T | LRP8 | A893V | G | 2780 | A |
| IGKV4-1 | Q47H | G | 316 | C | KCNH7 | L785V | A | 2453 | C | KLK9 | R122C | G | 451 | A | LRP8 | N655D | T | 2065 | C |
| IGLL1 | E210K | C | 728 | T | KCNH7 | S379A | A | 1235 | C | KLK9 | - | T | 0 | C | LRPAP1 | V216I | C | 793 | T |
| IGLL5 | A176V | C | 801 | T | KCNH7 | K346T | T | 1137 | G | KLRAQ1 | G160S | G | 635 | A | LRRC10 | R43H | C | 452 | T |
| IGLL5 | A176V | C | 801 | T | KCNH8 | T300I | C | 1165 | T | KLRAQ1 | R548C | C | 1799 | T | LRRC10 | V217A | A | 974 | G |
| IGLON5 | N76T | A | 227 | C | KCNH8 | A34T | G | 366 | A | KLRC2 | E10* | C | 35 | A | LRRC14 | G121D | G | 508 | A |
| IGLV1-50 | C14Y | G | 92 | A | KCNH8 | E89D | G | 533 | T | KLRC3 | N189H | T | 610 | G | LRRC14B | R185W | C | 581 | T |
| IGLV2-18 | R53C | C | 170 | T | KCNIP2 | G209S | C | 625 | T | KLRC4 | R151Q | C | 634 | T | LRRC15 | R209Q | C | 626 | T |
| IGLV2-23 | L99I | C | 457 | A | KCNIP2 | D193V | T | 578 | A | KLRC4 | K70N | C | 392 | A | LRRC15 | A392T | C | 1174 | T |
| IGLV2-23 | G78R | G | 394 | A | KCNIP3 | K27R | A | 215 | G | KLRC4 | E37K | C | 291 | T | LRRC16A | A712T | G | 2502 | A |
| IGLV2-33 | G14D | G | 99 | A | KCNJ1 | M1T | A | 53 | G | KLRD1 | S166F | C | 757 | T | LRRC16A | K1061T | A | 3550 | C |
| IGLV2-33 | K59R | A | 234 | G | KCNJ1 | K336E | T | 1057 | C | KLRF1 | E4K | G | 74 | A | LRRC16B | N936D | A | 2963 | G |
| IGLV2-8 | K87R | A | 422 | G | KCNJ1 | E52* | C | 205 | A | KLRK1 | S151* | G | 616 | T | LRRC16B | V741M | G | 2378 | A |
| IGLV3-12 | G99E | G | 305 | A | KCNJ11 | T341R | G | 1590 | C | KNDC1 | A1440V | C | 4320 | T | LRRC16B | R1008C | C | 3179 | T |
| IGLV3-16 | A37V | C | 148 | T | KCNJ11 | R192H | C | 1143 | T | KNDC1 | G1453S | G | 4358 | A | LRRC16B | K58R | A | 330 | G |
| IGLV3-27 | R79* | C | 273 | T | KCNJ12 | R229C | C | 1390 | T | KNDC1 | Q442H | G | 1327 | T | LRRC17 | F331V | T | 1286 | G |
| IGLV4-3 | G87D | G | 292 | A | KCNJ12 | V163M | G | 1192 | A | KNDC1 | S1024* | C | 3072 | A | LRRC18 | R124H | C | 448 | T |
| IGLV4-3 | D105N | G | 345 | A | KCNJ12 | V168G | T | 1208 | G | KNDC1 | K1569N | A | 4708 | T | LRRC18 | L34V | A | 177 | C |
| IGLV4-3 | E53K | G | 189 | A | KCNJ14 | A120T | G | 763 | A | KNDC1 | P1645S | C | 4934 | T | LRRC20 | A30V | G | 248 | A |
| IGLV4-69 | A54T | G | 220 | A | KCNJ14 | G46C | G | 541 | T | KNDC1 | P339L | C | 1017 | T | LRRC23 | R187W | C | 779 | T |
| IGLV5-37 | S85G | A | 258 | G | KCNJ14 | G173S | G | 922 | A | KNG1 | E209D | G | 839 | T | LRRC23 | S166N | G | 717 | A |
| IGLV5-37 | W118* | G | 359 | A | KCNJ14 | V180I | G | 943 | A | KNG1 | H439Y | C | 1527 | T | LRRC23 | R312W | C | 1154 | T |
| IGLV5-45 | R44H | G | 159 | A | KCNJ15 | R334W | C | 1303 | T | KNG1 | Y140C | A | 631 | G | LRRC23 | G239D | G | 936 | A |
| IGLV5-45 | S90C | C | 297 | G | KCNJ16 | R176* | C | 1012 | T | KNTC1 | R935Q | G | 2967 | A | LRRC23 | E339K | G | 1235 | A |

| | | | | | | | | | | | | | | | | | | | |
|---|---|---|---|---|---|---|---|---|---|---|---|---|---|---|---|---|---|---|---|
| IGLV6-57 | F82L | C | 332 | A | KCNJ16 | E277D | G | 1317 | T | KNTC1 | E1393* | G | 4340 | T | LRRC24 | R147Q | C | 564 | T |
| IGLV7-43 | T30A | A | 122 | G | KCNJ2 | C76S | G | 610 | C | KNTC1 | N1930T | A | 5952 | C | LRRC26 | P310Q | G | 1037 | T |
| IGSF1 | T375P | T | 1203 | G | KCNJ3 | T456I | C | 1844 | T | KNTC1 | - | G | 0 | T | LRRC27 | R25M | G | 179 | T |
| IGSF1 | A785V | G | 2434 | A | KCNJ3 | E335Q | G | 1480 | C | KNTC1 | T659I | C | 2139 | T | LRRC27 | V136I | G | 511 | A |
| IGSF1 | T1141A | T | 3501 | C | KCNJ4 | S194L | G | 840 | A | KNTC1 | E251D | G | 916 | T | LRRC27 | L240P | T | 824 | C |
| IGSF1 | V488I | C | 1542 | T | KCNJ4 | T345A | T | 1292 | C | KNTC1 | R570* | C | 1871 | T | LRRC3 | A99T | G | 612 | A |
| IGSF1 | A649S | C | 2025 | A | KCNJ4 | V22I | C | 323 | T | KPNA1 | S159Y | G | 653 | T | LRRC30 | D65N | G | 193 | A |
| IGSF1 | E732* | C | 2274 | A | KCNJ4 | H2Y | G | 263 | A | KPNA1 | R395W | G | 1360 | A | LRRC30 | A261T | G | 781 | A |
| IGSF1 | G235W | C | 783 | A | KCNJ5 | E213K | G | 951 | A | KPNA2 | S461N | G | 1514 | A | LRRC31 | M202I | C | 673 | A |
| IGSF10 | K471T | T | 1412 | G | KCNJ6 | P276L | G | 1417 | A | KPNA2 | R68H | G | 335 | A | LRRC32 | T483M | G | 1586 | A |
| IGSF10 | L227F | T | 681 | G | KCNJ6 | R270H | C | 1399 | T | KPNA4 | E469G | T | 1712 | C | LRRC32 | G139D | C | 554 | T |
| IGSF10 | V646M | C | 1936 | T | KCNJ6 | R118W | G | 942 | A | KPNA4 | K18N | T | 360 | G | LRRC32 | R251W | G | 889 | A |
| IGSF10 | I2080F | T | 6238 | A | KCNJ6 | T258M | G | 1363 | A | KPNA5 | L423S | T | 1416 | C | LRRC33 | R280H | G | 1042 | A |
| IGSF10 | A1078T | C | 3232 | T | KCNJ6 | E378D | C | 1724 | G | KPNA5 | V303A | T | 1056 | C | LRRC33 | V578A | T | 1936 | C |
| IGSF10 | M320T | A | 959 | G | KCNJ8 | R383H | C | 1486 | T | KPNA6 | L482I | C | 1537 | A | LRRC33 | M521T | T | 1765 | C |
| IGSF10 | D2599N | C | 7795 | T | KCNJ9 | R303C | C | 1149 | T | KPNA7 | A142T | C | 464 | T | LRRC33 | R645Q | G | 2137 | A |
| IGSF10 | R2378* | G | 7132 | A | KCNK10 | I255V | T | 1214 | C | KPNA7 | D461N | C | 1421 | T | LRRC34 | G338D | C | 1129 | T |
| IGSF10 | L1781I | G | 5341 | T | KCNK10 | F252V | A | 1205 | C | KPNA7 | L180I | G | 578 | T | LRRC36 | T233A | A | 716 | G |
| IGSF10 | R1032Q | C | 3095 | T | KCNK10 | A111T | C | 782 | T | KPNA7 | S146L | G | 477 | A | LRRC36 | S389N | G | 1185 | A |
| IGSF11 | R413Q | C | 1544 | T | KCNK10 | A41T | C | 572 | T | KPNB1 | E308D | G | 1260 | T | LRRC37A3 | N1291T | T | 4275 | G |
| IGSF11 | A20S | C | 364 | A | KCNK10 | - | C | 0 | T | KPNB1 | D599N | G | 2131 | A | LRRC37B | S625P | T | 1915 | C |
| IGSF11 | I354M | T | 1368 | C | KCNK10 | K490N | C | 1921 | A | KPNB1 | Q596R | A | 2123 | G | LRRC39 | Y309C | T | 1125 | C |
| IGSF11 | Y321* | G | 1269 | T | KCNK10 | N141T | T | 873 | G | KPNB1 | G317E | G | 1286 | A | LRRC4 | D406N | C | 1474 | T |
| IGSF21 | P144L | C | 814 | T | KCNK13 | S236G | A | 1147 | G | KPNB1 | L499V | T | 1831 | G | LRRC4 | R169W | G | 763 | A |
| IGSF21 | R302C | C | 1287 | T | KCNK13 | R308* | C | 1363 | T | KPRP | V18D | T | 111 | A | LRRC40 | L231I | G | 771 | T |
| IGSF21 | E405K | G | 1596 | A | KCNK13 | P118L | C | 794 | T | KPRP | A136V | C | 465 | T | LRRC40 | R347I | C | 1120 | A |
| IGSF22 | R450C | G | 1488 | A | KCNK18 | A195T | G | 583 | A | KPRP | R312H | G | 993 | A | LRRC41 | W751* | C | 2273 | T |
| IGSF22 | R1017H | C | 3190 | T | KCNK2 | H87R | A | 410 | G | KPRP | G546E | G | 1695 | A | LRRC42 | N234H | A | 1221 | C |
| IGSF22 | K1320N | C | 4100 | A | KCNK2 | F355L | C | 1215 | A | KPRP | R352H | G | 1113 | A | LRRC42 | P395L | C | 1705 | T |
| IGSF22 | T383M | G | 1288 | A | KCNK2 | T153N | C | 608 | A | KPRP | E116D | G | 406 | T | LRRC43 | K606N | G | 1843 | T |
| IGSF3 | R999H | C | 3701 | T | KCNK2 | T93S | C | 428 | G | KPTN | P268S | G | 906 | A | LRRC43 | L108R | T | 348 | G |
| IGSF3 | D472G | T | 2120 | C | KCNK2 | V289M | G | 1015 | A | KPTN | R409* | G | 1329 | A | LRRC43 | R132W | C | 419 | T |
| IGSF3 | D1046N | C | 3841 | T | KCNK3 | E37V | A | 235 | T | KRAS | G13D | C | 219 | T | LRRC43 | R160Q | G | 504 | A |
| IGSF3 | R922C | G | 3469 | A | KCNK3 | S343N | G | 1153 | A | KRAS | G12D | C | 216 | T | LRRC43 | G257E | G | 795 | A |
| IGSF3 | L751M | G | 2956 | T | KCNK5 | E484K | C | 1789 | T | KRAS | D57N | C | 350 | T | LRRC43 | S510N | G | 1554 | A |
| IGSF3 | S847L | G | 3245 | A | KCNK5 | V368I | C | 1441 | T | KRAS | G12D | C | 216 | T | LRRC46 | R318I | G | 1231 | T |
| IGSF5 | R367I | G | 1203 | T | KCNK7 | S225G | T | 897 | C | KRAS | G12V | C | 216 | A | LRRC47 | G544R | C | 1658 | T |
| IGSF6 | R201C | G | 663 | A | KCNK9 | R137H | C | 474 | T | KRAS | G12A | C | 216 | G | LRRC47 | R414W | G | 1268 | A |
| IGSF6 | - | C | 788 | A | KCNK9 | V374I | C | 1184 | T | KRAS | G13D | C | 219 | T | LRRC48 | L46M | C | 426 | A |

| | | | | | | | | | | | | | | | | | | | |
|---|---|---|---|---|---|---|---|---|---|---|---|---|---|---|---|---|---|---|---|
| IGSF8 | M381T | A | 1359 | G | KCNMA1 | A1033S | C | 3097 | A | KRAS | G13D | C | 219 | T | LRRC48 | R57C | C | 459 | T |
| IGSF8 | G123D | C | 585 | T | KCNMA1 | A664T | C | 1990 | T | KRAS | G12V | C | 216 | A | LRRC48 | S333L | C | 1288 | T |
| IGSF9 | P563H | G | 1886 | T | KCNMA1 | R989H | C | 2966 | T | KRAS | G12D | C | 216 | T | LRRC48 | E512K | G | 1824 | A |
| IGSF9 | P351Q | G | 1250 | T | KCNMA1 | R858Q | C | 2573 | T | KRAS | G12D | C | 216 | T | LRRC49 | S520N | G | 1806 | A |
| IGSF9 | F531L | A | 1791 | C | KCNMA1 | S966F | G | 2897 | A | KRAS | G12V | C | 216 | A | LRRC4B | E171K | C | 648 | T |
| IGSF9B | L773M | G | 2548 | T | KCNMA1 | - | T | 0 | C | KRAS | Y64H | A | 371 | G | LRRC4B | R600C | G | 1935 | A |
| IGSF9B | R251W | G | 982 | A | KCNMA1 | R640* | G | 1918 | A | KRAS | G12V | C | 216 | A | LRRC4B | A129T | C | 522 | T |
| IGSF9B | E1018D | C | 3285 | A | KCNMA1 | E669* | C | 2005 | A | KRAS | G12C | C | 215 | A | LRRC4B | T439A | T | 1452 | C |
| IGSF9B | G1133 W | C | 3628 | A | KCNMA1 | E604A | T | 1811 | G | KRAS | G12D | C | 216 | T | LRRC4B | F359S | A | 1213 | G |
| IGSF9B | V630A | A | 2120 | G | KCNMA1 | D475A | T | 1424 | G | KRAS | G12S | C | 215 | T | LRRC4B | A227S | C | 816 | A |
| IGSF9B | R364C | G | 1321 | A | KCNMA1 | F252L | G | 756 | T | KRAS | G12V | C | 216 | A | LRRC4B | T226M | G | 814 | A |
| IGSF9B | Q1086H | C | 3489 | A | KCNMA1 | T362N | G | 1085 | T | KRAS | G12D | C | 216 | T | LRRC4B | F587L | G | 1898 | T |
| IGSF9B | P513L | G | 1769 | A | KCNMB1 | F159V | A | 886 | C | KRAS | G12R | C | 215 | G | LRRC4B | R161W | G | 618 | A |
| IGSF9B | A317V | G | 1181 | A | KCNMB1 | K4T | T | 422 | G | KRAS | G12D | C | 216 | T | LRRC4C | R68C | G | 2166 | A |
| IGSF9B | H502Q | G | 1737 | T | KCNMB2 | K107* | A | 970 | T | KRAS | G12V | C | 216 | A | LRRC4C | V257M | C | 2733 | T |
| IHH | L20V | G | 58 | C | KCNMB2 | V77M | G | 880 | A | KRAS | G12D | C | 216 | T | LRRC4C | Y399* | G | 3161 | T |
| IK | - | G | 0 | A | KCNMB2 | D27V | A | 731 | T | KRAS | G12S | C | 215 | T | LRRC4C | T486I | G | 3421 | A |
| IK | Y445D | T | 1472 | G | KCNMB2 | S122Y | C | 1016 | A | KRAS | G13D | C | 219 | T | LRRC50 | A684V | C | 2227 | T |
| IK | R85H | G | 393 | A | KCNMB3 | R18H | C | 345 | T | KRAS | G12A | C | 216 | G | LRRC50 | Y277H | T | 1005 | C |
| IK | R356* | C | 1205 | T | KCNN1 | R438Q | G | 1572 | A | KRAS | G12V | C | 216 | A | LRRC50 | R288I | G | 1039 | T |
| IK | K398N | A | 1333 | C | KCNN1 | H489Y | C | 1724 | T | KRAS | G12V | C | 216 | A | LRRC50 | E458D | G | 1550 | T |
| IKBIP | D299G | T | 1006 | C | KCNN2 | G183S | G | 1004 | A | KRAS | G12V | C | 216 | A | LRRC52 | R90* | C | 558 | T |
| IKBIP | R226* | G | 786 | A | KCNN2 | D136G | A | 864 | G | KRAS | G13D | C | 219 | T | LRRC52 | L68V | T | 492 | G |
| IKBKAP | G342V | C | 1333 | A | KCNN2 | H25R | A | 531 | G | KRAS | L19F | C | 238 | A | LRRC55 | V307M | G | 1066 | A |
| IKBKAP | R478I | C | 1741 | A | KCNN2 | A270V | C | 1266 | T | KRAS | G12D | C | 216 | T | LRRC55 | R157H | G | 617 | A |
| IKBKB | R446W | C | 1513 | T | KCNN2 | L241P | T | 1179 | C | KRAS | G12V | C | 216 | A | LRRC55 | E178* | G | 679 | T |
| IKBKB | R144Q | G | 608 | A | KCNQ1 | R591H | G | 1880 | A | KRAS | G12V | C | 216 | A | LRRC55 | W53C | G | 306 | T |
| IKBKB | I598S | T | 1970 | G | KCNQ1 | R192H | G | 683 | A | KRAS | A146T | C | 617 | T | LRRC57 | K235N | C | 1073 | A |
| IKBKB | E707* | G | 2296 | T | KCNQ1 | G589C | G | 1873 | T | KRAS | G12V | C | 216 | A | LRRC58 | R215S | G | 739 | T |
| IKBKE | R27H | G | 453 | A | KCNQ1 | A329T | G | 1093 | A | KRAS | G12V | C | 216 | A | LRRC59 | P217H | G | 886 | T |
| IKBKE | R456Q | G | 1740 | A | KCNQ2 | A715T | C | 2320 | T | KRAS | G12V | C | 216 | A | LRRC59 | K249M | T | 982 | A |
| IKBKE | L316P | T | 1320 | C | KCNQ2 | V718L | C | 2329 | G | KRBA1 | P797S | C | 2389 | T | LRRC6 | K282N | C | 920 | A |
| IKBKE | I222V | A | 1037 | G | KCNQ2 | R749H | C | 2423 | T | KRBA1 | P626S | C | 1876 | T | LRRC61 | G106C | G | 758 | T |
| IKZF1 | R213* | C | 792 | T | KCNQ2 | R612L | C | 2012 | A | KRBA1 | T241M | C | 722 | T | LRRC66 | R518C | G | 1559 | A |
| IKZF1 | T333M | C | 1153 | T | KCNQ2 | K707N | C | 2298 | A | KRBA2 | R211H | C | 638 | T | LRRC66 | W188* | C | 571 | T |
| IKZF2 | D380N | C | 1289 | T | KCNQ3 | Q628H | C | 2305 | A | KRCC1 | R223Q | C | 1062 | T | LRRC66 | F458L | G | 1381 | T |
| IKZF3 | T387M | G | 1222 | A | KCNQ3 | - | C | 0 | T | KRCC1 | R217Q | C | 1044 | T | LRRC66 | V279A | A | 843 | G |
| IKZF3 | S339R | G | 1079 | T | KCNQ4 | - | T | 0 | C | KREMEN1 | R309H | G | 939 | A | LRRC69 | E245D | G | 778 | T |
| IKZF3 | A8V | G | 85 | A | KCNQ4 | I279V | A | 917 | G | KREMEN2 | P98L | C | 655 | T | LRRC69 | Y276C | A | 870 | G |

| | | | | | | | | | | | | | | | | | | | |
|---|---|---|---|---|---|---|---|---|---|---|---|---|---|---|---|---|---|---|---|
| IKZF3 | F495L | G | 1547 | T | KCNQ4 | P341L | C | 1104 | T | KRI1 | A607V | G | 1840 | A | LRRC7 | S720Y | C | 2189 | A |
| IKZF4 | R255W | C | 1352 | T | KCNQ4 | R433W | C | 1379 | T | KRI1 | P618T | G | 1872 | T | LRRC7 | P1222H | C | 3695 | A |
| IKZF4 | V126M | G | 965 | A | KCNQ4 | L345I | C | 1115 | A | KRIT1 | V718M | C | 2936 | T | LRRC7 | M180I | G | 570 | T |
| IKZF4 | R204C | C | 1199 | T | KCNQ5 | R178* | C | 930 | T | KRIT1 | D259G | T | 1560 | C | LRRC7 | E21D | A | 93 | C |
| IKZF4 | R255Q | G | 1353 | A | KCNQ5 | T712M | C | 2533 | T | KRIT1 | R623C | G | 2651 | A | LRRC7 | P1329A | C | 4015 | G |
| IL10 | A107V | G | 379 | A | KCNQ5 | R821H | G | 2860 | A | KRT1 | R65Q | C | 253 | T | LRRC7 | Y320* | C | 990 | A |
| IL10 | V139M | C | 474 | T | KCNQ5 | R457* | C | 1767 | T | KRT1 | R239Q | C | 775 | T | LRRC7 | Q1017K | C | 3079 | A |
| IL10RA | I536T | T | 1684 | C | KCNRG | P199H | C | 836 | A | KRT10 | N353S | T | 1068 | C | LRRC7 | G818C | G | 2482 | T |
| IL11RA | R257H | G | 819 | A | KCNRG | - | G | 0 | A | KRT10 | L211I | G | 641 | T | LRRC7 | R325W | C | 1003 | T |
| IL12B | - | C | 0 | A | KCNS1 | A88V | G | 660 | A | KRT12 | F101L | A | 325 | G | LRRC7 | S799Y | C | 2426 | A |
| IL12B | S71P | A | 253 | G | KCNS1 | A252V | G | 1152 | A | KRT12 | R414Q | C | 1265 | T | LRRC70 | E28* | G | 321 | T |
| IL12RB1 | D487G | T | 1524 | C | KCNS2 | R419C | C | 1605 | T | KRT12 | A403V | G | 1232 | A | LRRC70 | K218Q | A | 891 | C |
| IL12RB1 | R484H | C | 1515 | T | KCNS2 | V65I | G | 543 | A | KRT13 | A124T | C | 417 | T | LRRC8A | T95P | A | 537 | C |
| IL12RB2 | R256Q | G | 1407 | A | KCNS2 | G398D | G | 1543 | A | KRT13 | S153I | C | 505 | A | LRRC8A | T716M | C | 2401 | T |
| IL12RB2 | G82D | G | 885 | A | KCNS3 | D348Y | G | 1493 | T | KRT14 | R201H | C | 689 | T | LRRC8A | R719Q | G | 2410 | A |
| IL12RB2 | - | G | 0 | T | KCNT1 | V92I | G | 341 | A | KRT15 | R379* | G | 4721 | A | LRRC8B | Y759D | T | 2635 | G |
| IL13 | Q144* | C | 444 | T | KCNT1 | V332G | T | 1062 | G | KRT15 | E347D | C | 4627 | A | LRRC8B | Q630* | C | 2248 | T |
| IL13RA1 | N91S | A | 339 | G | KCNT1 | R464S | C | 1457 | A | KRT17 | R103C | G | 375 | A | LRRC8B | T787I | C | 2720 | T |
| IL13RA1 | D229V | A | 753 | T | KCNT1 | R780Q | G | 2406 | A | KRT17 | R386H | C | 1225 | T | LRRC8B | T48M | C | 503 | T |
| IL13RA1 | E56K | G | 233 | A | KCNT1 | R365C | C | 1160 | T | KRT17 | R385H | C | 1222 | T | LRRC8C | R344H | G | 1286 | A |
| IL15 | G103R | G | 678 | A | KCNT2 | I549T | A | 1706 | G | KRT19 | T323K | G | 1029 | T | LRRC8D | H356Y | C | 1460 | T |
| IL15RA | H41R | T | 220 | C | KCNT2 | A378T | C | 1192 | T | KRT20 | V404M | C | 1252 | T | LRRC8D | R585* | C | 2147 | T |
| IL16 | V919M | G | 3131 | A | KCNT2 | K746N | C | 2298 | A | KRT20 | L12M | G | 76 | T | LRRC8D | R588W | C | 2156 | T |
| IL16 | V61A | T | 558 | C | KCNT2 | I534M | A | 1662 | C | KRT20 | T317A | T | 991 | C | LRRC8D | T209M | C | 1020 | T |
| IL16 | V299M | G | 1271 | A | KCNU1 | S1080* | C | 3326 | A | KRT222 | S234Y | G | 743 | T | LRRC8D | R588Q | G | 2157 | A |
| IL16 | Q948* | C | 3218 | T | KCNU1 | T564M | C | 1778 | T | KRT222 | V209I | C | 667 | T | LRRC8D | L567F | A | 2095 | C |
| IL16 | E149* | G | 821 | T | KCNU1 | - | G | 0 | A | KRT222 | N239S | T | 758 | C | LRRC8E | A416T | G | 1347 | A |
| IL16 | D755Y | G | 2639 | T | KCNU1 | I267M | T | 888 | G | KRT23 | L412I | G | 1659 | T | LRRFIP2 | A36T | C | 529 | T |
| IL17B | K26T | T | 128 | G | KCNU1 | F1050L | C | 3237 | A | KRT24 | A319V | G | 1013 | A | LRRFIP2 | R49H | C | 569 | T |
| IL17F | L22I | G | 172 | T | KCNV1 | R448C | G | 1684 | A | KRT24 | S51I | C | 209 | A | LRRFIP2 | R257* | G | 1192 | A |
| IL17RA | R218H | G | 786 | A | KCNV1 | S378R | T | 1474 | G | KRT25 | A65V | G | 255 | A | LRRFIP2 | - | C | 0 | A |
| IL17RA | G497C | G | 1622 | T | KCNV2 | R27H | G | 318 | A | KRT25 | T382A | T | 1205 | C | LRRIQ1 | S1128A | T | 3493 | G |
| IL17RA | A690T | G | 2201 | A | KCNV2 | R9Q | G | 264 | A | KRT26 | G31W | C | 140 | A | LRRIQ1 | R1212* | C | 3745 | T |
| IL17RC | W282R | T | 1062 | C | KCNV2 | G34C | G | 338 | T | KRT27 | - | C | 0 | A | LRRIQ1 | E1420D | G | 4371 | T |
| IL17RC | E83K | G | 465 | A | KCNV2 | S42N | G | 363 | A | KRT28 | D295N | C | 949 | T | LRRIQ1 | R827C | C | 2590 | T |
| IL17RC | R501H | G | 1720 | A | KCTD1 | A259T | C | 775 | T | KRT28 | E78K | C | 298 | T | LRRIQ1 | Q872K | C | 2725 | A |
| IL17RD | - | A | 0 | G | KCTD1 | R257H | C | 770 | T | KRT28 | K148N | C | 510 | A | LRRIQ1 | E245D | G | 846 | T |
| IL17RD | F384L | G | 1241 | T | KCTD1 | G466A | C | 1397 | G | KRT3 | R76Q | C | 293 | T | LRRIQ1 | E273G | A | 929 | G |
| IL17RD | A78T | C | 321 | T | KCTD1 | R398C | G | 1192 | A | KRT3 | G580S | C | 1804 | T | LRRIQ1 | K368N | G | 1215 | T |

| | | | | | | | | | | | | | | | | | | | |
|---|---|---|---|---|---|---|---|---|---|---|---|---|---|---|---|---|---|---|---|
| IL17RD | V478I | C | 1521 | T | KCTD10 | N169I | T | 594 | A | KRT31 | L192F | G | 621 | A | LRRIQ1 | D1428N | G | 4393 | A |
| IL17RD | R442* | G | 1413 | A | KCTD11 | M5I | G | 1069 | A | KRT31 | A236T | C | 753 | T | LRRIQ3 | E514D | C | 1542 | A |
| IL17RD | S264Y | G | 880 | T | KCTD12 | F230L | A | 930 | G | KRT34 | P410T | G | 1259 | T | LRRK1 | R631Q | G | 2211 | A |
| IL17RE | L393I | C | 1195 | A | KCTD14 | V199A | A | 641 | G | KRT34 | T343M | G | 1059 | A | LRRK1 | E551* | G | 1970 | T |
| IL18 | L56I | G | 386 | T | KCTD14 | I247S | A | 785 | C | KRT34 | R335L | C | 1035 | A | LRRK1 | G792C | G | 2693 | T |
| IL18R1 | A301G | C | 1025 | G | KCTD16 | R344H | G | 1701 | A | KRT35 | R204H | C | 654 | T | LRRK1 | R51W | C | 470 | T |
| IL18R1 | E176K | G | 649 | A | KCTD16 | K296N | A | 1558 | C | KRT35 | K233N | C | 742 | A | LRRK2 | L242F | C | 782 | T |
| IL18R1 | S21A | T | 184 | G | KCTD16 | R361W | C | 1751 | T | KRT35 | N296D | T | 929 | C | LRRK2 | E768* | G | 2360 | T |
| IL18R1 | P215T | C | 766 | A | KCTD17 | G74R | G | 221 | A | KRT36 | C37G | A | 109 | C | LRRK2 | E1790* | G | 5426 | T |
| IL18RAP | R61Q | G | 771 | A | KCTD18 | R247H | C | 1251 | T | KRT36 | E93K | C | 277 | T | LRRK2 | R1693Q | G | 5136 | A |
| IL19 | D90G | A | 294 | G | KCTD18 | R256* | G | 1277 | A | KRT37 | A94T | C | 280 | T | LRRK2 | R1941C | C | 5879 | T |
| IL1A | V182M | C | 700 | T | KCTD18 | V57A | A | 681 | G | KRT38 | R200H | C | 599 | T | LRRK2 | - | C | 0 | T |
| IL1A | - | A | 0 | G | KCTD19 | L868F | C | 2655 | A | KRT38 | C343F | C | 1028 | A | LRRK2 | E2082V | A | 6303 | T |
| IL1B | F262L | G | 997 | T | KCTD19 | E497D | C | 1542 | A | KRT39 | A363V | G | 1124 | A | LRRK2 | N928H | A | 2840 | C |
| IL1B | E221* | C | 872 | A | KCTD20 | R371* | C | 1502 | T | KRT39 | R331* | G | 1027 | A | LRRK2 | - | G | 0 | T |
| IL1B | K204N | T | 823 | G | KCTD20 | S289I | G | 1257 | T | KRT39 | I461F | T | 1417 | A | LRRK2 | R1538H | G | 4671 | A |
| IL1B | K181N | C | 754 | A | KCTD3 | R117H | G | 644 | A | KRT4 | L422P | A | 1536 | G | LRRK2 | R1711Q | G | 5190 | A |
| IL1F8 | R115* | T | 451 | A | KCTD3 | E746* | G | 2530 | T | KRT4 | E391D | C | 1444 | A | LRRK2 | N2506H | A | 7574 | C |
| IL1F8 | I128L | T | 490 | G | KCTD6 | E187D | A | 660 | C | KRT4 | D227N | C | 950 | T | LRRN1 | V642I | G | 2685 | A |
| IL1R1 | K315N | G | 1263 | T | KCTD6 | R90W | C | 367 | T | KRT4 | L19F | G | 326 | A | LRRN1 | K477R | A | 2191 | G |
| IL1R1 | R42H | G | 443 | A | KCTD7 | A196V | C | 587 | T | KRT40 | I150N | A | 481 | T | LRRN1 | P372S | C | 1875 | T |
| IL1R1 | E214* | G | 958 | T | KCTD8 | P397H | G | 1474 | T | KRT40 | S328Y | G | 1015 | T | LRRN2 | R405C | G | 1601 | A |
| IL1R2 | C28Y | G | 312 | A | KCTD8 | S163N | C | 772 | T | KRT5 | P217L | G | 813 | A | LRRN2 | R378C | G | 1520 | A |
| IL1R2 | V149L | G | 674 | T | KDELC1 | G449D | C | 1683 | T | KRT5 | G108S | C | 485 | T | LRRN2 | R131W | G | 779 | A |
| IL1R2 | L175I | C | 752 | A | KDELC1 | Y101C | T | 639 | C | KRT6B | K424N | C | 1320 | A | LRRN2 | S106L | G | 705 | A |
| IL1R2 | T316A | A | 1175 | G | KDELC2 | F217L | G | 717 | T | KRT7 | A61V | C | 309 | T | LRRN2 | P7T | G | 407 | T |
| IL1RAP | F483V | T | 1653 | G | KDELC2 | P302S | G | 970 | A | KRT71 | R410W | G | 1298 | A | LRRN3 | I147N | T | 1379 | A |
| IL1RAP | K578T | A | 1939 | C | KDELC2 | Y384H | A | 1216 | G | KRT71 | R46Q | C | 207 | T | LRRN3 | P68S | C | 1141 | T |
| IL1RAP | - | A | 2270 | C | KDELC2 | L423V | A | 1333 | C | KRT71 | S281R | T | 911 | G | LRRN3 | R387* | C | 2098 | T |
| IL1RAP | A595S | G | 1989 | T | KDELR1 | D112G | T | 530 | C | KRT72 | S447N | C | 1396 | T | LRRN4 | A340T | C | 1243 | T |
| IL1RAPL1 | L607I | C | 2492 | A | KDELR2 | R47C | G | 324 | A | KRT73 | - | T | 0 | G | LRRN4 | V651M | C | 2176 | T |
| IL1RAPL1 | S102F | C | 978 | T | KDELR3 | Q187H | A | 717 | C | KRT73 | R75Q | C | 259 | T | LRRN4 | R68C | G | 427 | A |
| IL1RAPL2 | R339Q | G | 1772 | A | KDM1B | A720T | G | 2399 | A | KRT73 | K240N | C | 755 | A | LRRN4 | K553R | T | 1883 | C |
| IL1RL1 | C468Y | G | 1674 | A | KDM1B | A30E | C | 330 | A | KRT74 | R506* | G | 1564 | A | LRRN4 | E464K | C | 1615 | T |
| IL1RL2 | P332L | C | 1121 | T | KDM2B | A917T | C | 2822 | T | KRT75 | R506H | C | 1576 | T | LRRN4 | D259N | C | 1000 | T |
| IL1RL2 | K391N | G | 1299 | T | KDM2B | C624Y | C | 1944 | T | KRT75 | R200W | G | 657 | A | LRRN4CL | G201E | C | 909 | T |
| IL1RN | L95P | T | 406 | C | KDM2B | R996C | G | 3059 | A | KRT76 | T452A | T | 1384 | C | LRRN4CL | A213T | C | 944 | T |
| IL20 | V53A | T | 521 | C | KDM2B | R733C | G | 2270 | A | KRT77 | G501R | C | 1530 | T | LRRTM1 | L139M | G | 1072 | T |
| IL21R | R323Q | G | 1238 | A | KDM2B | A86T | C | 329 | T | KRT77 | S242I | C | 754 | A | LRRTM1 | A54V | G | 818 | A |

| | | | | | | | | | | | | | | | | | | | |
|---|---|---|---|---|---|---|---|---|---|---|---|---|---|---|---|---|---|---|---|
| IL21R | C499Y | G | 1766 | A | KDM3A | L18I | C | 417 | A | KRT78 | Y201H | A | 665 | G | LRRTM1 | A343G | G | 1685 | C |
| IL22RA1 | P396L | G | 1226 | A | KDM3A | L477V | T | 1794 | G | KRT78 | Y304H | A | 974 | G | LRRTM2 | R318Q | C | 1410 | T |
| IL22RA1 | S483N | C | 1487 | T | KDM3A | K490Q | A | 1833 | C | KRT79 | E334K | C | 1035 | T | LRRTM3 | D526G | A | 2155 | G |
| IL23R | Q211K | C | 802 | A | KDM3A | K901N | G | 3068 | T | KRT79 | T104M | G | 346 | A | LRRTM3 | L224P | T | 1249 | C |
| IL23R | D118Y | G | 523 | T | KDM3B | R954C | C | 3060 | T | KRT79 | K359N | C | 1112 | A | LRRTM3 | G149* | G | 1023 | T |
| IL23R | S121Y | C | 533 | A | KDM3B | I1313M | A | 4139 | G | KRT80 | R114H | C | 439 | T | LRRTM3 | S60F | C | 757 | T |
| IL23R | A201T | G | 772 | A | KDM3B | R1028W | C | 3282 | T | KRT84 | R346M | C | 1085 | A | LRRTM3 | E296D | G | 1466 | T |
| IL24 | A14S | G | 315 | T | KDM3B | D1649G | A | 5146 | G | KRT84 | E215K | C | 691 | T | LRRTM3 | V324A | T | 1549 | C |
| IL25 | D97Y | G | 547 | T | KDM3B | F1274V | T | 4020 | G | KRT85 | V87M | C | 379 | T | LRRTM3 | F330C | T | 1567 | G |
| IL26 | K104N | T | 347 | G | KDM4A | T625A | A | 2007 | G | KRTAP1-1 | C177W | A | 595 | C | LRRTM4 | K470Q | T | 1632 | G |
| IL26 | V93I | C | 312 | T | KDM4A | R839I | G | 2650 | T | KRTAP1-5 | R165H | C | 541 | T | LRRTM4 | R466G | T | 1620 | C |
| IL27RA | W115C | G | 768 | C | KDM4B | V508A | T | 1749 | C | KRTAP10-1 | C262Y | C | 830 | T | LRRTM4 | L69F | T | 431 | G |
| IL28A | T105M | C | 366 | T | KDM4B | A349V | C | 1272 | T | KRTAP10-11 | V207F | G | 645 | T | LRSAM1 | S433L | C | 1902 | T |
| IL28RA | R265Q | C | 807 | T | KDM4B | K334T | A | 1227 | C | KRTAP10-5 | S157N | C | 496 | T | LRSAM1 | V693I | G | 2681 | A |
| IL28RA | A496V | G | 1500 | A | KDM4B | Y693H | T | 2303 | C | KRTAP10-8 | P63H | C | 210 | A | LRSAM1 | R273C | C | 1421 | T |
| IL2RB | A239T | C | 914 | T | KDM4B | V853M | G | 2783 | A | KRTAP11-1 | R161I | C | 513 | A | LRSAM1 | L88I | C | 866 | A |
| IL2RB | P510H | G | 1728 | T | KDM4B | A293T | G | 1103 | A | KRTAP11-1 | S154P | A | 491 | G | LRTM1 | A307T | C | 1082 | T |
| IL2RG | R289Q | C | 958 | T | KDM4B | A685T | G | 2279 | A | KRTAP11-1 | D54N | C | 191 | T | LRTM1 | Q107H | C | 484 | A |
| IL3 | A140V | C | 597 | T | KDM4C | R220* | C | 1223 | T | KRTAP12-2 | C85G | A | 294 | C | LRTOMT | Y263C | A | 1659 | G |
| IL31RA | R234Q | G | 766 | A | KDM4C | T884M | C | 3216 | T | KRTAP12-3 | V28M | G | 130 | A | LRWD1 | R416W | C | 1398 | T |
| IL31RA | T11M | C | 97 | T | KDM4C | V723I | G | 2732 | A | KRTAP13-1 | N33S | A | 111 | G | LRWD1 | V511M | G | 1683 | A |
| IL31RA | V466M | G | 1461 | A | KDM4C | E268* | G | 1367 | T | KRTAP13-1 | S37N | G | 123 | A | LRWD1 | V313M | G | 1089 | A |
| IL31RA | A92T | G | 339 | A | KDM4D | H205Y | C | 1445 | T | KRTAP13-2 | S114L | G | 385 | A | LRWD1 | A452T | G | 1506 | A |
| IL31RA | I559L | A | 1740 | C | KDM4D | R129H | G | 1218 | A | KRTAP13-3 | R54M | C | 217 | A | LSAMP | V38I | C | 112 | T |
| IL31RA | G723V | G | 2233 | T | KDM4DL | D192N | G | 594 | A | KRTAP19-2 | G6V | C | 105 | A | LSAMP | E77D | T | 231 | G |
| IL31RA | R211H | G | 697 | A | KDM4DL | F268L | C | 824 | A | KRTAP19-3 | F77L | G | 259 | T | LSG1 | R517* | G | 1864 | A |
| IL33 | A24V | C | 126 | T | KDM5A | R696W | G | 2449 | A | KRTAP19-3 | S2N | C | 33 | T | LSG1 | R502Q | C | 1820 | T |
| IL4 | F136C | T | 472 | G | KDM5A | R1508W | G | 4885 | A | KRTAP19- | R33H | C | 98 | T | LSG1 | A627T | C | 2194 | T |

| | | | | | | | | | | | | | | | | | | | |
|---|---|---|---|---|---|---|---|---|---|---|---|---|---|---|---|---|---|---|---|
| | | | | | | | | | | 4 | | | | | | | | | |
| IL4I1 | T27I | G | 643 | A | KDM5A | P469Q | G | 1769 | T | KRTAP19-6 | G52D | C | 155 | T | LSM11 | R224T | G | 675 | C |
| IL4I1 | K197T | T | 1153 | G | KDM5A | N372T | T | 1478 | G | KRTAP19-8 | R33H | C | 131 | T | LSM12 | V139A | A | 564 | G |
| IL4I1 | R245Q | C | 1297 | T | KDM5A | V181M | C | 904 | T | KRTAP21-1 | G15S | C | 93 | T | LSM14A | R272C | C | 1010 | T |
| IL4I1 | R119C | G | 918 | A | KDM5A | K1586N | T | 5121 | G | KRTAP22-1 | G30V | G | 115 | T | LSM14A | R438H | G | 1509 | A |
| IL4I1 | P373S | G | 1680 | A | KDM5B | L1181I | G | 3654 | T | KRTAP23-1 | Q45K | G | 136 | T | LSM14A | R272C | C | 1010 | T |
| IL4I1 | M225I | C | 1238 | A | KDM5B | C1217Y | C | 3763 | T | KRTAP24-1 | Y187H | A | 585 | G | LSM3 | R28Q | G | 586 | A |
| IL4I1 | S200L | G | 1162 | A | KDM5B | P986L | G | 3070 | A | KRTAP24-1 | N207K | A | 647 | C | LSM4 | R55Q | C | 335 | T |
| IL4I1 | E173K | C | 1080 | T | KDM5B | R1570H | C | 4822 | T | KRTAP25-1 | S5Y | G | 38 | T | LSM4 | R88C | G | 433 | A |
| IL4I1 | K57E | T | 732 | C | KDM5B | P30H | G | 202 | T | KRTAP26-1 | G121D | C | 616 | T | LSM6 | R4Q | G | 148 | A |
| IL4R | V568I | G | 1943 | A | KDM5B | R1277H | C | 3943 | T | KRTAP27-1 | T153I | G | 484 | A | LSP1 | I337T | T | 1118 | C |
| IL4R | T73M | C | 459 | T | KDM5B | L1406V | A | 4329 | C | KRTAP27-1 | V85F | C | 279 | A | LSR | A310V | C | 1152 | T |
| IL4R | P755T | C | 2504 | A | KDM5B | R1281C | G | 3954 | A | KRTAP3-1 | G76R | C | 266 | T | LSS | A354T | C | 1137 | T |
| IL5RA | F202C | A | 1180 | C | KDM5B | K1152N | C | 3569 | A | KRTAP4-2 | R219H | C | 656 | T | LSS | G122R | C | 441 | T |
| IL5RA | D305G | T | 1489 | C | KDM5B | E1116D | C | 3461 | A | KRTAP4-3 | C150F | C | 449 | A | LSS | R438H | C | 1390 | T |
| IL6 | R106Q | G | 317 | A | KDM5C | E158K | C | 1005 | T | KRTAP5-10 | P52S | C | 199 | T | LSS | A245V | G | 811 | A |
| IL6R | Y408* | C | 1661 | A | KDM5C | G591E | C | 2305 | T | KRTAP5-10 | G189E | G | 611 | A | LSS | R347H | C | 1117 | T |
| IL6ST | - | C | 0 | T | KDM5C | R1453W | G | 4890 | A | KRTAP5-11 | S21C | T | 61 | A | LSS | K65T | T | 271 | G |
| IL6ST | S754L | G | 2525 | A | KDM5C | G604S | C | 2343 | T | KRTAP5-11 | G20S | C | 58 | T | LTA4H | V586I | C | 1824 | T |
| IL7R | E446* | G | 1465 | T | KDM5C | C618Y | C | 2386 | T | KRTAP5-6 | C53F | G | 209 | T | LTA4H | E349K | C | 1113 | T |
| ILDR1 | G151E | C | 579 | T | KDM5C | C1188S | C | 4096 | G | KRTAP9-1 | C249Y | G | 746 | A | LTB4R | A232T | G | 1015 | A |
| ILDR1 | R442C | G | 1451 | A | KDM5C | Q813* | G | 2970 | A | KRTAP9-1 | T152A | A | 454 | G | LTB4R | G319D | G | 1277 | A |
| ILDR1 | D363V | T | 1215 | A | KDM5C | R378L | C | 1666 | A | KRTCAP2 | G67D | C | 231 | T | LTB4R2 | G11R | G | 1648 | A |
| ILDR1 | E134* | C | 527 | A | KDM5D | R159H | C | 748 | T | KSR1 | S758N | G | 2538 | A | LTBP1 | A248T | G | 742 | A |
| ILDR2 | L11F | G | 87 | A | KDM5D | L1393F | G | 4449 | A | KSR1 | N583S | A | 2013 | G | LTBP1 | T736M | C | 2207 | T |
| ILDR2 | - | C | 0 | T | KDM5D | P1412H | G | 4507 | T | KSR1 | R687Q | G | 2325 | A | LTBP1 | C186R | T | 556 | C |
| ILDR2 | S410P | A | 1284 | G | KDM6A | K1018* | A | 3093 | T | KSR2 | G775V | C | 2324 | A | LTBP1 | M408I | G | 1224 | A |
| ILF2 | Q71H | C | 339 | A | KDM6A | R1213Q | G | 3679 | A | KSR2 | Y489* | A | 1467 | T | LTBP1 | G1651V | G | 4952 | T |
| ILF3 | E636K | G | 2223 | A | KDM6B | R586W | C | 2145 | T | KSR2 | L443V | A | 1327 | C | LTBP1 | F1408V | T | 4222 | G |

| | | | | | | | | | | | | | | | | | | | | |
|---|---|---|---|---|---|---|---|---|---|---|---|---|---|---|---|---|---|---|---|---|
| ILF3 | M670T | T | 2326 | C | KDM6B | E687K | G | 2448 | A | KTELC1 | C54Y | G | 245 | A | LTBP1 | L1715V | T | 5143 | G |
| ILF3 | A153D | C | 775 | A | KDM6B | S119P | T | 744 | C | KTELC1 | - | G | 0 | T | LTBP2 | P302L | G | 1292 | A |
| ILF3 | D804N | G | 2727 | A | KDM6B | F1396V | T | 4575 | G | KTN1 | R368S | A | 1172 | T | LTBP2 | A1421V | G | 4649 | A |
| ILK | R323C | C | 1151 | T | KDR | E407D | C | 1517 | A | KTN1 | R452H | G | 1423 | A | LTBP2 | R1565C | G | 5080 | A |
| ILK | F342L | C | 1210 | A | KDR | G893D | C | 2974 | T | KY | G288S | C | 924 | T | LTBP2 | T1796A | T | 5773 | C |
| ILK | R349H | G | 1230 | A | KDR | R1032* | G | 3390 | A | KY | K600E | T | 1860 | C | LTBP2 | A1690T | C | 5455 | T |
| ILKAP | K174T | T | 697 | G | KDR | V542A | A | 1921 | G | KY | R509W | G | 1587 | A | LTBP2 | R409H | C | 1613 | T |
| ILKAP | R114W | G | 516 | A | KDR | R819* | G | 2751 | A | KYNU | G112E | G | 593 | A | LTBP2 | P118H | G | 740 | T |
| ILVBL | L168P | A | 624 | G | KDR | Q645H | T | 2231 | G | KYNU | M189V | A | 823 | G | LTBP2 | G1738D | C | 5600 | T |
| ILVBL | V535I | C | 1724 | T | KDR | S1037L | G | 3406 | A | KYNU | R399Q | G | 1454 | A | LTBP3 | G646S | C | 2205 | T |
| ILVBL | V46M | C | 257 | T | KDSR | E227D | T | 1073 | G | KYNU | R28K | G | 341 | A | LTBP3 | R269W | G | 1074 | A |
| ILVBL | R591W | G | 1892 | A | KEAP1 | A191D | G | 728 | T | KYNU | P56T | C | 424 | A | LTBP4 | R1290W | C | 3868 | T |
| IMMP2L | I100V | T | 741 | C | KEAP1 | T142M | G | 581 | A | L1CAM | A1253T | C | 3947 | T | LTBP4 | A176T | G | 526 | A |
| IMMP2L | R73H | C | 661 | T | KERA | K333T | T | 1617 | G | L1CAM | Y784H | A | 2540 | G | LTF | A481T | C | 1737 | T |
| IMMP2L | S43L | G | 571 | A | KERA | R279Q | C | 1455 | T | L1CAM | R575H | C | 1914 | T | LTF | S327L | G | 1276 | A |
| IMMT | R623H | C | 2256 | T | KERA | N101H | T | 920 | G | L1CAM | R1109H | C | 3516 | T | LTF | A169V | G | 802 | A |
| IMMT | R525C | G | 1961 | A | KHDC1L | P12L | G | 79 | A | L1CAM | R407C | G | 1409 | A | LTK | P492L | G | 1626 | A |
| IMMT | K270N | T | 1198 | A | KHDC1L | E18K | C | 96 | T | L1CAM | V1217A | A | 3840 | G | LTK | R810I | C | 2580 | A |
| IMPA2 | A50T | G | 390 | A | KHDRBS1 | E381K | G | 1308 | A | L1CAM | - | C | 0 | T | LTV1 | S244L | C | 865 | T |
| IMPACT | G98S | G | 433 | A | KHDRBS1 | A247V | C | 907 | T | L1CAM | R473C | G | 1607 | A | LTV1 | R377* | C | 1263 | T |
| IMPACT | F37L | T | 252 | G | KHDRBS2 | Q92H | C | 555 | A | L1TD1 | R745* | C | 2528 | T | LTV1 | R401I | G | 1336 | T |
| IMPACT | E168* | G | 643 | T | KHDRBS3 | P7S | C | 429 | T | L1TD1 | T677A | A | 2324 | G | LUC7L | R267C | G | 910 | A |
| IMPACT | K292N | G | 1017 | T | KHDRBS3 | F67V | T | 609 | G | L1TD1 | E131D | A | 688 | C | LUC7L3 | R350* | C | 1182 | T |
| IMPAD1 | A303D | G | 1191 | T | KHK | P162L | C | 998 | T | L1TD1 | G448D | G | 1638 | A | LUC7L3 | D134G | A | 535 | G |
| IMPDH2 | L227P | A | 720 | G | KHK | D82G | A | 758 | G | L1TD1 | R789I | G | 2661 | T | LUM | R315H | C | 1333 | T |
| IMPDH2 | R203H | C | 648 | T | KHNYN | R438H | G | 1515 | A | L2HGDH | K246N | C | 817 | A | LUZP1 | S1000P | A | 3316 | G |
| IMPDH2 | Q478H | T | 1474 | G | KHSRP | P537A | G | 1719 | C | L2HGDH | V330I | C | 1067 | T | LUZP1 | L45M | G | 451 | T |
| IMPG1 | T465I | G | 1584 | A | KHSRP | G312R | C | 1044 | T | L3MBTL | R478Q | G | 1565 | A | LUZP1 | P479L | G | 1754 | A |
| IMPG1 | E232D | C | 886 | A | KHSRP | G697S | C | 2199 | T | L3MBTL | R356C | C | 1198 | T | LUZP1 | G490V | C | 1787 | A |
| IMPG2 | L1003F | G | 3210 | A | KHSRP | Q166H | T | 608 | G | L3MBTL | R374H | G | 1253 | A | LUZP2 | M203V | A | 607 | G |
| IMPG2 | M965I | C | 3098 | A | KIAA0020 | E640* | C | 2125 | A | L3MBTL2 | K416N | G | 1406 | T | LUZP2 | M87I | G | 261 | T |
| IMPG2 | E272* | C | 1017 | A | KIAA0090 | R724H | C | 2214 | T | L3MBTL3 | V663I | G | 2157 | A | LUZP4 | R73W | C | 224 | T |
| IMPG2 | R80W | G | 441 | A | KIAA0090 | R513L | C | 1581 | A | L3MBTL3 | A552T | G | 1824 | A | LY6H | R103Q | C | 437 | T |
| IMPG2 | R672I | C | 2218 | A | KIAA0090 | R385Q | C | 1197 | T | L3MBTL4 | I474V | T | 1580 | C | LY6K | F168L | C | 745 | A |
| IMPG2 | D189V | T | 769 | A | KIAA0090 | L934V | A | 2843 | C | L3MBTL4 | K596N | C | 1948 | A | LY6K | K192N | A | 817 | C |
| IMPG2 | K344T | T | 1234 | G | KIAA0100 | E1875* | C | 5722 | A | L3MBTL4 | K159T | T | 636 | G | LY6K | P71L | C | 453 | T |
| IMPG2 | D23Y | C | 270 | A | KIAA0100 | T300I | G | 998 | A | L3MBTL4 | E30K | C | 248 | T | LY75 | F1205S | A | 3642 | G |
| IMPG2 | K666T | T | 2200 | G | KIAA0100 | R347H | C | 1139 | T | LACRT | A15V | G | 98 | A | LY75 | A387V | G | 1188 | A |
| INADL | E869* | G | 2719 | T | KIAA0100 | D149E | A | 546 | T | LAD1 | R374Q | C | 1180 | T | LY75 | W1336R | A | 4034 | G |

| | | | | | | | | | | | | | | | | | | | |
|---|---|---|---|---|---|---|---|---|---|---|---|---|---|---|---|---|---|---|---|
| INCENP | R577C | C | 1931 | T | KIAA0100 | N804T | T | 2510 | G | LAD1 | V79M | C | 294 | T | LY75 | Y906C | T | 2745 | C |
| INCENP | R451H | G | 1554 | A | KIAA0100 | Q2175H | C | 6624 | A | LAG3 | D322N | G | 1313 | A | LY75 | Y1222C | T | 3693 | C |
| INE2 | R263W | C | 923 | T | KIAA0100 | L644I | G | 2029 | T | LAG3 | R57* | C | 518 | T | LY75 | S605Y | G | 1842 | T |
| INF2 | V159M | G | 618 | A | KIAA0100 | R2092C | G | 6373 | A | LAGE3 | R143H | C | 770 | T | LY75 | R487I | C | 1488 | A |
| INF2 | R660W | C | 2121 | T | KIAA0141 | R274C | C | 954 | T | LAMA1 | R1133* | G | 3491 | A | LY9 | A496V | C | 1517 | T |
| ING1 | G368D | G | 1565 | A | KIAA0141 | R172Q | G | 649 | A | LAMA1 | S1916L | G | 5841 | A | LY9 | V339A | T | 1046 | C |
| ING5 | A102V | C | 331 | T | KIAA0141 | M1I | G | 137 | A | LAMA1 | G1503C | C | 4601 | A | LYG2 | A99T | C | 316 | T |
| ING5 | R98* | C | 318 | T | KIAA0146 | R585C | C | 1762 | T | LAMA1 | A208T | C | 716 | T | LYPD3 | A55T | C | 271 | T |
| INHA | R247C | C | 919 | T | KIAA0146 | R355C | C | 1072 | T | LAMA1 | V2403D | A | 7302 | T | LYPD4 | H43N | G | 1339 | T |
| INHBA | K235Q | T | 950 | G | KIAA0146 | - | A | 0 | C | LAMA1 | I738T | A | 2307 | G | LYPD5 | A17V | G | 131 | A |
| INHBA | A41V | G | 369 | A | KIAA0146 | G350C | G | 1057 | T | LAMA1 | G2159S | C | 6569 | T | LYPD6 | E100D | A | 589 | C |
| INHBC | C316* | C | 1075 | A | KIAA0182 | R345C | C | 1209 | T | LAMA1 | L2779I | G | 8429 | T | LYPD6B | Q112R | A | 736 | G |
| INHBE | W127* | G | 605 | A | KIAA0182 | P542L | C | 1801 | T | LAMA1 | V1529I | C | 4679 | T | LYPLA1 | A66V | G | 361 | A |
| INHBE | R250Q | G | 973 | A | KIAA0182 | S710L | C | 2305 | T | LAMA1 | E3061K | C | 9275 | T | LYPLA1 | T195M | G | 748 | A |
| INMT | E222K | G | 680 | A | KIAA0182 | - | A | 0 | G | LAMA1 | T1171A | T | 3605 | C | LYPLA2 | K93N | G | 586 | T |
| INMT | R132W | C | 410 | T | KIAA0182 | P410S | C | 1404 | T | LAMA1 | C279* | G | 931 | T | LYSMD1 | R215Q | C | 1305 | T |
| INO80 | D1312G | T | 4252 | C | KIAA0182 | G994S | G | 3156 | A | LAMA1 | G2210A | C | 6723 | G | LYSMD1 | P205H | G | 1275 | T |
| INO80 | Q655R | T | 2281 | C | KIAA0195 | E534G | A | 1804 | G | LAMA2 | L2272I | C | 6919 | A | LYSMD2 | D140G | T | 934 | C |
| INO80B | K37N | G | 205 | T | KIAA0195 | M893I | G | 2882 | A | LAMA2 | L2477P | T | 7535 | C | LYST | A3655V | G | 1113 9 | A |
| INO80D | R553C | G | 2062 | A | KIAA0195 | V1259M | G | 3978 | A | LAMA2 | V2911I | G | 8836 | A | LYST | L566S | A | 1872 | G |
| INPP1 | R283C | C | 1547 | T | KIAA0195 | E530K | G | 1791 | A | LAMA2 | W2686* | G | 8162 | A | LYST | G3587S | C | 1093 4 | T |
| INPP4A | R829W | C | 2878 | T | KIAA0195 | S674L | C | 2224 | T | LAMA2 | A2877G | C | 8735 | G | LYST | D15N | C | 218 | T |
| INPP4A | E524K | G | 1963 | A | KIAA0196 | R864H | C | 2853 | T | LAMA2 | H326Q | T | 1083 | A | LYST | R1352I | C | 4230 | A |
| INPP4A | R901H | G | 3095 | A | KIAA0196 | E547* | C | 1901 | A | LAMA2 | E1764G | A | 5396 | G | LYST | I3404T | A | 1038 6 | G |
| INPP4A | D124Y | G | 763 | T | KIAA0226 | D596Y | C | 1786 | A | LAMA2 | R2787C | C | 8464 | T | LYVE1 | F206V | A | 902 | C |
| INPP4A | D675Y | G | 2416 | T | KIAA0226 | R256Q | C | 767 | T | LAMA2 | F1329L | C | 4092 | A | LYVE1 | S98Y | G | 579 | T |
| INPP4A | N831H | A | 2884 | C | KIAA0226 | L541I | G | 1621 | T | LAMA2 | Q2035* | C | 6208 | T | LYZ | L12I | C | 89 | A |
| INPP4B | A693T | C | 2422 | T | KIAA0226 | P45H | G | 134 | T | LAMA2 | I2271S | T | 6917 | G | LYZL1 | A127V | C | 437 | T |
| INPP4B | H352Y | G | 1399 | A | KIAA0226 | F702V | A | 2104 | C | LAMA2 | S2454Y | C | 7466 | A | LYZL1 | A4T | G | 67 | A |
| LYZL4 | R79H | C | 512 | T | MMP25 | K264T | A | 1028 | C | MYOF | - | A | 0 | G | NPAT | S422N | C | 1367 | T |
| LYZL6 | G38V | C | 305 | A | MMP27 | T403S | T | 1230 | A | MYOM1 | Q219* | G | 989 | A | NPAT | S1422L | G | 4367 | A |
| LYZL6 | V53L | C | 349 | A | MMP27 | V137A | A | 433 | G | MYOM1 | V791M | C | 2705 | T | NPAT | E1282* | C | 3946 | A |
| LZTFL1 | R272* | G | 989 | A | MMP28 | V77M | C | 488 | T | MYOM1 | R246* | G | 1070 | A | NPAT | K269Q | T | 907 | G |
| LZTR1 | G675R | G | 2382 | A | MMP3 | S252P | A | 797 | G | MYOM2 | A314T | G | 1081 | A | NPAT | R112Q | C | 437 | T |
| LZTR1 | R267H | G | 1159 | A | MMP3 | D47Y | C | 182 | A | MYOM2 | - | A | 0 | T | NPBWR1 | A37V | C | 1585 | T |
| LZTS1 | R300W | G | 1256 | A | MMP7 | F65L | G | 222 | T | MYOM2 | R1314H | G | 4082 | A | NPBWR1 | A95T | G | 1758 | A |
| LZTS2 | R479W | C | 1640 | T | MMP9 | P658H | C | 1992 | A | MYOM2 | S268P | T | 943 | C | NPBWR2 | R240Q | C | 1059 | T |

| | | | | | | | | | | | | | | | | | | | |
|---|---|---|---|---|---|---|---|---|---|---|---|---|---|---|---|---|---|---|---|
| LZTS2 | T180M | C | 744 | T | MMP9 | A333D | C | 1017 | A | MYOM2 | E215D | G | 786 | T | NPC1 | E229K | C | 824 | T |
| LZTS2 | R367Q | G | 1305 | A | MMRN1 | L577I | C | 2048 | A | MYOM3 | R284C | G | 1013 | A | NPC1 | P48S | G | 281 | A |
| LZTS2 | R577Q | G | 1935 | A | MMRN1 | G883S | G | 2966 | A | MYOM3 | R810Q | C | 2592 | T | NPC1 | F842S | A | 2664 | G |
| M6PR | I118V | T | 522 | C | MMRN1 | V998A | T | 3312 | C | MYOT | G287V | G | 1234 | T | NPC1 | A286T | C | 995 | T |
| MAB21L1 | W291* | C | 1430 | T | MMRN1 | S155Y | C | 783 | A | MYOT | D147G | A | 814 | G | NPC1L1 | R770Q | C | 2365 | T |
| MAB21L2 | Q48R | A | 1248 | G | MMRN1 | K631T | A | 2211 | C | MYOT | G126D | G | 751 | A | NPC1L1 | G1159D | C | 3532 | T |
| MAB21L2 | S39L | C | 1221 | T | MMRN1 | A1102S | G | 3623 | T | MYOZ2 | P109H | C | 539 | A | NPC1L1 | A19T | C | 111 | T |
| MAB21L2 | D91N | G | 1376 | A | MMRN2 | T441M | G | 1396 | A | MYOZ3 | A123T | G | 954 | A | NPC1L1 | I641T | A | 1978 | G |
| MACC1 | P50S | G | 457 | A | MMS19 | L264P | A | 791 | G | MYOZ3 | P198L | C | 1180 | T | NPC1L1 | S1033N | C | 3154 | T |
| MACC1 | S436Y | G | 1616 | T | MN1 | A562T | C | 2639 | T | MYPN | S1029G | A | 3573 | G | NPC1L1 | R968H | C | 2959 | T |
| MACF1 | A1092V | C | 3327 | T | MN1 | S609N | C | 2781 | T | MYPN | K655R | A | 2452 | G | NPC1L1 | A296T | C | 942 | T |
| MACF1 | T907I | C | 2851 | T | MN1 | A1091T | C | 4226 | T | MYPN | G511E | G | 2020 | A | NPEPL1 | A92T | G | 322 | A |
| MACF1 | E1713D | G | 5270 | T | MN1 | S814N | C | 3396 | T | MYPN | S831G | A | 2979 | G | NPEPPS | L557V | C | 1892 | G |
| MACF1 | R5277* | C | 15960 | T | MN1 | R458H | C | 2328 | T | MYPN | G970E | G | 3397 | A | NPFFR1 | R363Q | C | 1088 | T |
| MACF1 | V4125E | T | 12505 | A | MN1 | P448H | G | 2298 | T | MYPN | - | G | 0 | T | NPFFR2 | R293* | C | 975 | T |
| MACF1 | R415Q | G | 1296 | A | MN1 | G74S | C | 1175 | T | MYPN | K1170N | A | 3998 | C | NPFFR2 | K503N | A | 1607 | C |
| MACF1 | L3955I | C | 11994 | A | MNAT1 | R295K | G | 985 | A | MYPOP | R86C | G | 343 | A | NPHP1 | R458G | T | 1446 | C |
| MACF1 | K4542R | A | 13756 | G | MND1 | Y71C | A | 301 | G | MYPOP | R248Q | C | 830 | T | NPHP1 | S630L | G | 1963 | A |
| MACF1 | K1210N | G | 3682 | T | MND1 | - | G | 0 | A | MYPOP | A126V | G | 464 | A | NPHP1 | - | C | 0 | T |
| MACF1 | K3857N | A | 11702 | C | MNDA | M72I | G | 477 | A | MYPOP | A42E | G | 212 | T | NPHP1 | R347* | G | 1113 | A |
| MACF1 | T4343A | A | 13158 | G | MNDA | E301K | G | 1162 | A | MYPOP | R161Q | C | 569 | T | NPHP3 | S404G | T | 1314 | C |
| MACROD1 | D107G | T | 439 | C | MNDA | E258D | G | 1035 | T | MYSM1 | D224Y | C | 710 | A | NPHP3 | R93Q | C | 382 | T |
| MACROD2 | E226K | G | 676 | A | MNDA | N399S | A | 1457 | G | MYSM1 | E519* | C | 1595 | A | NPHP3 | L1127I | G | 3483 | T |
| MACROD2 | K340Q | A | 1018 | C | MNS1 | R311Q | C | 1097 | T | MYSM1 | D92Y | C | 314 | A | NPHP4 | R229Q | C | 952 | T |
| MAD1L1 | L463M | G | 1397 | T | MNT | P416L | G | 1653 | A | MYSM1 | L280P | A | 879 | G | NPHP4 | A1110T | C | 3594 | T |
| MAD1L1 | R412W | G | 1244 | A | MNT | Q556P | T | 2073 | G | MYSM1 | N565D | T | 1733 | C | NPHP4 | P165L | G | 760 | A |
| MAD1L1 | R102H | C | 315 | T | MNT | A177V | G | 936 | A | MYST1 | A7D | C | 36 | A | NPHP4 | P776L | G | 2593 | A |
| MAD1L1 | V355I | C | 1073 | T | MNX1 | D366G | T | 1292 | C | MYST1 | V406A | T | 1233 | C | NPHP4 | A1059T | C | 3441 | T |
| MAD1L1 | R576H | C | 1737 | T | MOBKL2A | V90I | C | 621 | T | MYST1 | R136H | G | 423 | A | NPHP4 | F805V | A | 2679 | C |
| MAD1L1 | R528W | G | 1592 | A | MOBKL2C | P140L | G | 476 | A | MYST2 | G101D | G | 582 | A | NPHP4 | R682Q | C | 2311 | T |
| MAD1L1 | W317* | C | 961 | T | MOBKL2C | G105R | C | 370 | T | MYST2 | A133V | C | 678 | T | NPHS1 | G153R | C | 457 | T |
| MAD2L2 | T206M | G | 694 | A | MOBP | T68A | A | 351 | G | MYST2 | R127Q | G | 660 | A | NPHS1 | R207W | G | 619 | A |
| MADCAM1 | S77G | A | 239 | G | MOCOS | V155I | G | 484 | A | MYST2 | E262D | A | 1066 | T | NPHS1 | C101* | A | 303 | T |
| MADCAM1 | R190Q | G | 579 | A | MOCOS | M617V | A | 1870 | G | MYST3 | I699T | A | 2508 | G | NPHS1 | R864H | C | 2591 | T |
| MADCAM1 | L15F | C | 53 | T | MOCOS | R441M | G | 1343 | T | MYST3 | R1087H | C | 3672 | T | NPHS1 | T798M | G | 2393 | A |

| | | | | | | | | | | | | | | | | | | | |
|---|---|---|---|---|---|---|---|---|---|---|---|---|---|---|---|---|---|---|---|
| MADCAM1 | L13M | C | 47 | A | MOCOS | D256N | G | 787 | A | MYST3 | P1298L | G | 4305 | A | NPHS2 | R13H | C | 107 | T |
| MADD | R162W | C | 649 | T | MOCS1 | A508V | G | 1527 | A | MYST4 | I32V | A | 583 | G | NPLOC4 | E89D | C | 267 | G |
| MADD | E774K | G | 2485 | A | MOCS1 | V599M | C | 1799 | T | MYST4 | P862S | C | 3073 | T | NPM2 | R120C | C | 1373 | T |
| MADD | R699C | C | 2260 | T | MOCS1 | R413S | T | 1243 | G | MYST4 | P1445S | C | 4822 | T | NPNT | R80* | C | 238 | T |
| MADD | A1035T | G | 3268 | A | MOCS2 | F82S | A | 919 | G | MYST4 | T1787M | C | 5849 | T | NPNT | C151Y | G | 452 | A |
| MADD | R967C | C | 3064 | T | MOCS3 | I60L | A | 195 | C | MYST4 | E1373G | A | 4607 | G | NPPA | T6S | T | 115 | A |
| MADD | M1526V | A | 4741 | G | MOGAT1 | P288Q | C | 911 | A | MYST4 | T575A | A | 2212 | G | NPPB | D2N | C | 106 | T |
| MADD | R764H | G | 2456 | A | MOGAT1 | E191* | G | 619 | T | MYST4 | A930V | C | 3278 | T | NPPB | P88L | G | 365 | A |
| MAEA | R357H | G | 1097 | A | MOGAT2 | L273* | T | 864 | G | MYST4 | S797G | A | 2878 | G | NPR1 | L520I | C | 2030 | A |
| MAEL | A303V | C | 1152 | T | MOGAT3 | H116R | T | 405 | C | MYST4 | G343E | G | 1517 | A | NPR1 | G1036V | G | 3579 | T |
| MAF | P78L | G | 1045 | A | MOGS | R535Q | C | 1767 | T | MYST4 | R1137C | C | 3898 | T | NPR1 | R1035Q | G | 3576 | A |
| MAFB | S74R | G | 612 | C | MON1A | R431C | G | 1550 | A | MYT1 | R965Q | G | 3258 | A | NPR2 | R945C | C | 3088 | T |
| MAFK | R57H | G | 399 | A | MON1A | T462M | G | 1644 | A | MYT1 | T503M | C | 1872 | T | NPR2 | L565M | C | 1948 | A |
| MAFK | T2M | C | 234 | T | MON1A | G303C | C | 1166 | A | MYT1 | E662D | G | 2350 | C | NPR2 | Y136H | T | 661 | C |
| MAG | R576H | G | 1886 | A | MON1B | R445Q | G | 1681 | A | MYT1 | A386T | G | 1520 | A | NPR2 | V102M | G | 559 | A |
| MAG | P269L | C | 965 | T | MON1B | R219H | G | 1003 | A | MYT1 | A567T | G | 2063 | A | NPR2 | S434L | C | 1556 | T |
| MAG | R51W | C | 310 | T | MON2 | R808Q | G | 2814 | A | MYT1 | E665K | G | 2357 | A | NPR3 | R199C | C | 938 | T |
| MAG | R624Q | G | 2030 | A | MON2 | A687V | C | 2451 | T | MYT1L | S776R | G | 3076 | T | NPR3 | W302R | T | 1247 | C |
| MAG | A422T | G | 1423 | A | MON2 | H96Q | T | 679 | G | MYT1L | S971P | A | 3659 | G | NPRL2 | E353K | C | 1496 | T |
| MAG | Y60H | T | 337 | C | MON2 | Y233H | T | 1088 | C | MYT1L | K76E | T | 974 | C | NPRL3 | T441M | G | 1608 | A |
| MAG | E574K | G | 1879 | A | MON2 | R1081* | C | 3632 | T | MYT1L | T1035M | G | 3852 | A | NPRL3 | E493K | C | 1763 | T |
| MAG | V338I | G | 1171 | A | MON2 | L1365V | T | 4484 | G | MYT1L | D1013N | C | 3785 | T | NPRL3 | S369F | G | 1392 | A |
| MAGEA11 | N255K | T | 867 | A | MORC1 | V397M | C | 1272 | T | MYT1L | K753T | T | 3006 | G | NPRL3 | E249A | T | 1032 | G |
| MAGEA4 | D243N | G | 921 | A | MORC1 | E844* | C | 2613 | A | MYT1L | L200P | A | 1347 | G | NPS | E54* | G | 180 | T |
| MAGEA6 | F201V | T | 826 | G | MORC1 | F709C | A | 2209 | C | MYT1L | T63A | T | 935 | C | NPSR1 | F85L | C | 259 | A |
| MAGEB1 | E167D | A | 822 | C | MORC1 | A676V | G | 2110 | A | MYT1L | G44D | C | 879 | T | NPSR1 | R125H | G | 378 | A |
| MAGEB1 | R275K | G | 1145 | A | MORC1 | D113Y | C | 420 | A | MZF1 | G94S | C | 523 | T | NPTN | L305M | G | 1111 | T |
| MAGEB10 | Q272K | C | 1059 | A | MORC2 | R110C | G | 737 | A | MZF1 | P126L | G | 620 | A | NPTN | A230T | C | 886 | T |
| MAGEB18 | K233T | A | 885 | C | MORC2 | G869R | C | 3014 | T | MZF1 | R46H | C | 380 | T | NPTX1 | G190D | C | 727 | T |
| MAGEB18 | D247Y | G | 926 | T | MORC3 | D592Y | G | 1850 | T | MZF1 | G57W | C | 412 | A | NPTX1 | E339D | C | 1175 | A |
| MAGEB2 | A310V | C | 1030 | T | MORC3 | T869M | C | 2682 | T | MZF1 | S424G | T | 1513 | C | NPTX2 | S261A | T | 946 | G |
| MAGEB3 | - | A | 1776 | G | MORC3 | R110H | G | 405 | A | MZF1 | R432I | C | 1538 | A | NPTX2 | L429R | T | 1451 | G |
| MAGEB4 | R12H | G | 119 | A | MORC4 | L550R | A | 1924 | C | N4BP1 | S562Y | G | 1922 | T | NPY | V20M | G | 171 | A |
| MAGEB6 | K34E | A | 249 | G | MORN2 | D55V | A | 471 | T | N4BP1 | F10L | G | 267 | T | NPY1R | R72I | C | 747 | A |
| MAGEC1 | G105W | G | 599 | T | MORN4 | G133R | C | 397 | T | N4BP1 | E306G | T | 1154 | C | NPY2R | R187W | C | 1048 | T |
| MAGEC1 | S1138R | C | 3700 | A | MORN4 | R181* | G | 541 | A | N4BP2 | N1316Y | A | 4362 | T | NPY2R | D347N | G | 1528 | A |
| MAGEC1 | S658Y | C | 2259 | A | MORN5 | A56T | G | 228 | A | N4BP2 | V1556M | G | 5082 | A | NPY5R | A136V | C | 589 | T |
| MAGEC1 | K1046N | A | 3424 | C | MORN5 | G5V | G | 76 | T | N4BP2 | F476V | T | 1842 | G | NPY5R | R364* | C | 1272 | T |
| MAGEC2 | L141I | G | 769 | T | MOS | R319H | C | 956 | T | N4BP2 | F476L | T | 1844 | G | NQO2 | A143V | C | 1140 | T |

| | | | | | | | | | | | | | | | | | | | |
|---|---|---|---|---|---|---|---|---|---|---|---|---|---|---|---|---|---|---|---|
| MAGEC2 | G64C | C | 538 | A | MOS | C24* | G | 72 | T | N4BP2 | K749N | A | 2663 | C | NR0B1 | D424N | C | 1505 | T |
| MAGEC3 | G110E | G | 329 | A | MOSC2 | K229N | G | 885 | T | N6AMT2 | K38T | T | 161 | G | NR0B1 | R124W | G | 605 | A |
| MAGEC3 | A158V | C | 473 | T | MOSPD1 | M1T | A | 189 | G | NAA11 | A104T | C | 483 | T | NR0B2 | P228L | G | 709 | A |
| MAGEC3 | E332K | G | 994 | A | MOSPD3 | R97C | C | 491 | T | NAA16 | E780D | G | 2664 | T | NR1D1 | F439L | G | 1948 | T |
| MAGEC3 | L467R | T | 1400 | G | MOV10 | R730L | G | 2579 | T | NAA25 | S572F | G | 1735 | A | NR1D1 | R215W | G | 1274 | A |
| MAGEC3 | E234* | G | 700 | T | MOV10 | P997L | C | 3380 | T | NAA25 | Y466C | T | 1417 | C | NR1D2 | E273* | G | 1136 | T |
| MAGEC3 | P108L | C | 323 | T | MOV10 | Y944* | T | 3222 | G | NAA25 | I972S | A | 2935 | C | NR1D2 | E502D | G | 1825 | T |
| MAGEC3 | P412T | C | 1234 | A | MOV10L1 | N673H | A | 2100 | C | NAA25 | R728C | G | 2202 | A | NR1D2 | L305P | T | 1233 | C |
| MAGED1 | R505C | C | 1666 | T | MOXD1 | V212L | C | 753 | A | NAA30 | P17H | C | 204 | A | NR1D2 | D73E | T | 538 | A |
| MAGED1 | T63M | C | 341 | T | MOXD1 | A71V | G | 331 | A | NAA40 | T79M | C | 363 | T | NR1D2 | K230E | A | 1007 | G |
| MAGED2 | E319K | G | 1188 | A | MOXD1 | L427I | G | 1398 | T | NAAA | A168T | C | 604 | T | NR1H2 | P187L | C | 836 | T |
| MAGEE1 | A300T | G | 1105 | A | MPDU1 | A245T | G | 733 | A | NAALAD2 | S40Y | C | 228 | A | NR1H2 | R357W | C | 1345 | T |
| MAGEE1 | R9C | C | 232 | T | MPDU1 | P254L | C | 761 | T | NAALAD2 | E693* | G | 2186 | T | NR1H2 | A23T | G | 343 | A |
| MAGEE1 | P490H | C | 1676 | A | MPDZ | H863R | T | 2602 | C | NAALADL2 | Q27K | C | 207 | A | NR1H2 | A301T | G | 1177 | A |
| MAGEE1 | L55I | C | 370 | A | MPDZ | P1449H | G | 4360 | T | NAALADL2 | E607K | G | 1947 | A | NR1H3 | Q157* | C | 469 | T |
| MAGEE2 | R446I | C | 1522 | A | MPDZ | F1545C | A | 4648 | C | NAALADL2 | F604L | T | 1940 | G | NR1H4 | L212S | T | 635 | C |
| MAGI1 | H1239R | T | 3716 | C | MPDZ | L1511* | A | 4546 | C | NAB1 | R121Q | G | 672 | A | NR1H4 | S248L | C | 743 | T |
| MAGI1 | V971M | C | 2911 | T | MPDZ | N1367S | T | 4114 | C | NAB1 | V56M | G | 476 | A | NR1I2 | R420Q | G | 1307 | A |
| MAGI1 | E353K | C | 1057 | T | MPDZ | S1016Y | G | 3061 | T | NAB2 | V122I | G | 742 | A | NR1I3 | E29D | C | 290 | A |
| MAGI1 | R1372W | G | 4114 | A | MPEG1 | L121F | G | 447 | A | NAB2 | A226T | G | 1054 | A | NR2C1 | S193L | G | 834 | A |
| MAGI1 | G1219S | C | 3655 | T | MPEG1 | V448M | C | 1428 | T | NAB2 | R254W | C | 1138 | T | NR2C2 | Q475H | G | 1642 | T |
| MAGI1 | E1271K | C | 3811 | T | MPEG1 | E64K | C | 276 | T | NAB2 | A489T | G | 1843 | A | NR2E3 | T333M | C | 1188 | T |
| MAGI1 | S255F | G | 764 | A | MPG | A37T | G | 340 | A | NACA2 | T853M | G | 2840 | A | NR2E3 | D52Y | G | 344 | T |
| MAGI1 | I837M | A | 2511 | C | MPHOSPH 10 | T570A | A | 2060 | G | NACA2 | A1769V | G | 5588 | A | NR2F1 | R373* | C | 2804 | T |
| MAGI2 | D91G | T | 526 | C | MPHOSPH 9 | L312R | A | 1042 | C | NACAD | G145R | C | 433 | T | NR2F1 | M296V | A | 2573 | G |
| MAGI2 | A963V | G | 3142 | A | MPHOSPH 9 | S657Y | G | 2077 | T | NACAD | A1480T | C | 4438 | T | NR2F1 | R392C | C | 2861 | T |
| MAGI2 | I79T | A | 490 | G | MPHOSPH 9 | E546* | C | 1743 | A | NACAD | G1187V | C | 3560 | A | NR3C1 | E285K | C | 1136 | T |
| MAGI2 | R215W | G | 897 | A | MPI | A141T | G | 473 | A | NACAD | M602I | C | 1806 | A | NR3C1 | Y393H | A | 1460 | G |
| MAGI2 | R130C | G | 642 | A | MPL | P85S | C | 295 | T | NACC1 | A71V | C | 380 | T | NR3C1 | R470* | G | 1691 | A |
| MAGI2 | E124G | T | 625 | C | MPND | V303I | G | 942 | A | NACC1 | V319A | T | 1124 | C | NR3C2 | K442T | T | 1659 | G |
| MAGI2 | G102* | C | 558 | A | MPND | A365T | G | 1128 | A | NADSYN1 | R411C | C | 1357 | T | NR3C2 | R537* | G | 1943 | A |
| MAGI2 | V82M | C | 498 | T | MPND | R372W | C | 1149 | T | NADSYN1 | V162A | T | 611 | C | NR4A1 | P193L | C | 578 | T |
| MAGI3 | R856L | G | 2642 | T | MPO | R531C | G | 1768 | A | NADSYN1 | R96C | C | 412 | T | NR4A2 | D499N | C | 1858 | T |
| MAGI3 | R808W | C | 2497 | T | MPO | C638Y | C | 2090 | T | NADSYN1 | E154D | A | 588 | C | NR4A2 | D434N | C | 1663 | T |
| MAGI3 | A845T | G | 2608 | A | MPO | Q642* | G | 2101 | A | NAE1 | A40T | C | 195 | T | NR4A2 | R344C | G | 1393 | A |
| MAGI3 | R766Q | G | 2372 | A | MPO | G627S | C | 2056 | T | NAIF1 | G116S | C | 566 | T | NR4A3 | E562* | G | 1728 | T |

| | | | | | | | | | | | | | | | | | | | |
|---|---|---|---|---|---|---|---|---|---|---|---|---|---|---|---|---|---|---|---|
| MAGIX | H94Q | C | 329 | A | MPO | P17H | G | 227 | T | NAIF1 | V202M | C | 824 | T | NR4A3 | V87M | G | 303 | A |
| MAGIX | L97I | T | 336 | A | MPP1 | K299N | C | 1045 | A | NAIF1 | A95V | G | 504 | A | NR5A1 | R114W | G | 537 | A |
| MAGOH | R47I | C | 302 | A | MPP2 | R452Q | C | 1401 | T | NAIF1 | V284I | C | 1070 | T | NR5A1 | A340V | G | 1216 | A |
| MAGOHB | R122Q | C | 417 | T | MPP2 | E502K | C | 1550 | T | NALCN | P573S | G | 1799 | A | NRAP | R1510Q | C | 4682 | T |
| MAGOHB | S108Y | G | 375 | T | MPP2 | R432C | G | 1340 | A | NALCN | R1495W | G | 4565 | A | NRAP | L577F | G | 1882 | A |
| MAK | Y165H | A | 876 | G | MPP3 | R257H | C | 1031 | T | NALCN | E128A | T | 465 | G | NRAP | R809C | G | 2578 | A |
| MAK | R272* | G | 1197 | A | MPP3 | A215T | C | 904 | T | NALCN | N1274H | T | 3902 | G | NRAP | E1684G | T | 5204 | C |
| MAK | N245H | T | 1116 | G | MPP3 | R257H | C | 1031 | T | NALCN | R1127H | C | 3462 | T | NRAP | K1598N | C | 4947 | A |
| MAK | K36N | C | 491 | A | MPP4 | R387H | C | 1368 | T | NALCN | H569Q | A | 1789 | T | NRAP | E1007* | C | 3172 | A |
| MAK16 | E284G | A | 891 | G | MPP4 | A560V | G | 1887 | A | NALCN | R297S | G | 971 | T | NRAP | D88G | T | 416 | C |
| MALT1 | L246V | T | 994 | G | MPP4 | D524G | T | 1779 | C | NALCN | R164M | C | 573 | A | NRAP | P80T | G | 391 | T |
| MAMDC2 | R568H | G | 2320 | A | MPP4 | R422H | C | 1473 | T | NALCN | Y102H | A | 386 | G | NRAS | Q61L | T | 436 | A |
| MAMDC2 | G380V | G | 1756 | T | MPP4 | R290Q | C | 1077 | T | NALCN | A1157V | G | 3552 | A | NRAS | G12A | C | 289 | G |
| MAMDC4 | R914C | C | 2790 | T | MPP4 | I217T | A | 858 | G | NAMPT | E258* | C | 1080 | A | NRCAM | P1007H | G | 3491 | T |
| MAMDC4 | W503* | G | 1558 | A | MPP4 | M113L | T | 545 | G | NAMPTL | G198A | C | 593 | G | NRCAM | V633I | C | 2368 | T |
| MAMDC4 | R288Q | G | 913 | A | MPP5 | D313N | G | 1403 | A | NAMPTL | G42E | C | 125 | T | NRCAM | I1183S | A | 4019 | C |
| MAMDC4 | A1197V | C | 3640 | T | MPP5 | E260* | G | 1244 | T | NAMPTL | S256Y | G | 767 | T | NRCAM | R1135* | G | 3874 | A |
| MAMDC4 | R389H | G | 1216 | A | MPP5 | T641M | C | 2388 | T | NAMPTL | E70* | C | 208 | A | NRD1 | A98V | G | 483 | A |
| MAMDC4 | G797S | G | 2439 | A | MPP6 | A197V | C | 675 | T | NANOGP8 | N156T | A | 683 | C | NRD1 | R1120H | C | 3549 | T |
| MAMDC4 | R664W | C | 2040 | T | MPP6 | Q2R | A | 90 | G | NANOGP8 | S65N | G | 410 | A | NRD1 | K975T | T | 3114 | G |
| MAMDC4 | G174W | G | 570 | T | MPP7 | D260E | A | 1040 | C | NANOGP8 | E33* | G | 313 | T | NRD1 | L903V | A | 2897 | C |
| MAMDC4 | V196A | T | 637 | C | MPPE1 | G201R | C | 930 | T | NANOS3 | Y10H | T | 141 | C | NRD1 | E717K | C | 2339 | T |
| MAMDC4 | Q393K | C | 1227 | A | MPPE1 | E270G | T | 1138 | C | NANP | T119A | T | 582 | C | NRD1 | C637Y | C | 2100 | T |
| MAML1 | L49M | C | 408 | A | MPPED1 | T102M | C | 749 | T | NANP | T131M | G | 619 | A | NRF1 | T324M | C | 1088 | T |
| MAML1 | A914T | G | 3003 | A | MPPED1 | N269H | A | 1249 | C | NANS | A187T | G | 629 | A | NRF1 | V518A | T | 1670 | C |
| MAML1 | H106R | A | 580 | G | MPPED2 | K268N | T | 1267 | G | NAP1L2 | R353Q | C | 1414 | T | NRF1 | T340M | C | 1136 | T |
| MAML3 | R669H | C | 2863 | T | MPRIP | T383M | C | 1204 | T | NAP1L2 | N436T | T | 1663 | G | NRG1 | Y63C | A | 188 | G |
| MAML3 | N548S | T | 2500 | C | MPRIP | K278N | G | 914 | T | NAP1L3 | P353H | G | 1363 | T | NRG1 | A336T | G | 1498 | A |
| MAML3 | R193* | G | 1434 | A | MPRIP | Y1192C | A | 3573 | G | NAP1L3 | E426* | C | 1581 | A | NRG1 | T535M | C | 2096 | T |
| MAMLD1 | D163Y | G | 553 | T | MPST | A303T | G | 1083 | A | NAP1L5 | A10V | G | 147 | A | NRG1 | E418* | G | 1744 | T |
| MAMSTR | A256V | G | 831 | A | MPV17L | G173D | G | 662 | A | NAP1L5 | A62V | G | 303 | A | NRG2 | D122N | C | 589 | T |
| MAMSTR | P162T | G | 548 | T | MPV17L2 | P227T | C | 923 | A | NAP1L5 | K85E | T | 371 | C | NRG3 | S617F | C | 1850 | T |
| MAMSTR | P46H | G | 201 | T | MPZ | R108C | G | 335 | A | NAPA | V263A | A | 1088 | G | NRG3 | T681A | A | 2041 | G |
| MAN1A1 | A440V | G | 1761 | A | MPZ | G249R | C | 758 | T | NAPRT1 | E471D | C | 1434 | A | NRG3 | V457A | T | 1370 | C |
| MAN1B1 | G239R | G | 1062 | A | MPZ | R46G | T | 149 | C | NAPRT1 | Q343H | C | 1050 | A | NRG3 | R157C | C | 469 | T |
| MAN1C1 | E278D | G | 1164 | T | MPZL2 | A106T | C | 457 | T | NAPRT1 | - | C | 0 | A | NRIP3 | R77Q | C | 344 | T |
| MAN2A1 | A119V | C | 1408 | T | MR1 | Q115L | A | 349 | T | NARF | T335M | C | 1144 | T | NRIP3 | A135V | G | 518 | A |
| MAN2A1 | E160* | G | 1530 | T | MRAP | R126* | C | 563 | T | NARF | R371I | G | 1252 | T | NRIP3 | R202Q | C | 719 | T |
| MAN2A1 | F1035L | C | 4157 | A | MRAP2 | A129V | C | 521 | T | NARG2 | M335V | T | 1238 | C | NRN1 | G112S | C | 552 | T |

| | | | | | | | | | | | | | | | | | | | | | | | |
|---|---|---|---|---|---|---|---|---|---|---|---|---|---|---|---|---|---|---|---|---|---|---|---|
| MAN2A2 | - | T | 0 | C | MRC1L1 | Q242R | A | 828 | G | NARG2 | S519Y | G | 1791 | T | NRP1 | E784K | C | 2722 | T |
| MAN2A2 | A246V | C | 755 | T | MRC2 | G1445R | G | 4735 | A | NARG2 | V109L | C | 560 | G | NRP1 | R463H | C | 1760 | T |
| MAN2A2 | D355G | A | 1082 | G | MRC2 | R82Q | G | 647 | A | NARS | T175M | G | 752 | A | NRP1 | F652C | A | 2327 | C |
| MAN2B1 | W1006R | A | 3057 | G | MRC2 | R114W | C | 742 | T | NAT1 | L239R | T | 889 | G | NRP1 | R235* | G | 1075 | A |
| MAN2B1 | A559V | G | 1717 | A | MRC2 | A1446T | G | 4738 | A | NAT10 | R847H | G | 2784 | A | NRP1 | H223R | T | 1040 | C |
| MAN2B2 | R374* | C | 1156 | T | MRC2 | Y681H | T | 2443 | C | NAT10 | R810W | C | 2672 | T | NRP1 | L119I | G | 727 | T |
| MAN2B2 | R740W | C | 2254 | T | MRC2 | N1333K | C | 4401 | G | NAT10 | A436T | G | 1550 | A | NRP1 | A537V | G | 1982 | A |
| MAN2B2 | P219L | C | 692 | T | MRFAP1 | P30S | C | 741 | T | NAT10 | V510A | T | 1773 | C | NRP2 | R466C | C | 2187 | T |
| MAN2B2 | Y746C | A | 2273 | G | MRGPRD | A69V | G | 206 | A | NAT14 | D14Y | G | 120 | T | NRP2 | P575L | C | 2515 | T |
| MAN2B2 | D433G | A | 1334 | G | MRGPRD | F167L | G | 501 | T | NAT14 | D29V | A | 166 | T | NRP2 | E612Q | G | 2625 | C |
| MAN2B2 | H965R | A | 2930 | G | MRGPRE | R283W | G | 1154 | A | NAT15 | T149A | A | 530 | G | NRP2 | E576D | G | 2519 | T |
| MAN2B2 | R70H | G | 245 | A | MRGPRX2 | R141H | C | 510 | T | NAT15 | A103T | G | 392 | A | NRXN1 | A219V | G | 1996 | A |
| MAN2C1 | R183Q | C | 565 | T | MRGPRX3 | F273L | C | 1180 | A | NAT2 | E136* | G | 513 | T | NRXN1 | S173L | G | 1858 | A |
| MAN2C1 | R208H | C | 640 | T | MRGPRX4 | R95C | C | 703 | T | NAT8B | R166W | G | 646 | A | NRXN1 | R61C | G | 1521 | A |
| MANBA | A461V | G | 1482 | A | MRGPRX4 | A140V | C | 839 | T | NAT8B | T89R | G | 416 | C | NRXN1 | G1330D | C | 5329 | T |
| MANBA | R26C | G | 176 | A | MRGPRX4 | T31M | C | 512 | T | NAT8L | R140H | G | 419 | A | NRXN1 | C931R | A | 4131 | G |
| MANEA | R393H | G | 1312 | A | MRI1 | A65V | C | 260 | T | NAV1 | R1614W | C | 5260 | T | NRXN1 | R635H | C | 3244 | T |
| MANEA | F20L | C | 194 | A | MRM1 | L211V | T | 870 | G | NAV1 | - | G | 0 | A | NRXN1 | R896Q | C | 4027 | T |
| MANEAL | E397K | G | 1502 | A | MRO | R203I | C | 757 | A | NAV1 | A210T | G | 1048 | A | NRXN1 | R1207Q | C | 4960 | T |
| MAOA | R291I | G | 995 | T | MRPL1 | R223Q | G | 997 | A | NAV1 | F1442L | C | 4746 | A | NRXN1 | R856W | G | 3906 | A |
| MAOA | R297Q | G | 1013 | A | MRPL1 | A151V | C | 781 | T | NAV1 | R1756Q | G | 5687 | A | NRXN1 | - | C | 0 | T |
| MAP1A | G517C | G | 1580 | T | MRPL10 | R93H | C | 731 | T | NAV2 | A883T | G | 2746 | A | NRXN1 | A165V | G | 1834 | A |
| MAP1A | T1035I | C | 3135 | T | MRPL12 | T194A | A | 705 | G | NAV2 | L850M | C | 2647 | A | NRXN1 | T550I | G | 2989 | A |
| MAP1A | P2097S | C | 6320 | T | MRPL15 | Q139H | G | 480 | T | NAV2 | R1062* | C | 3283 | T | NRXN1 | L665I | G | 3333 | T |
| MAP1A | E211* | G | 662 | T | MRPL15 | K169N | A | 570 | C | NAV2 | R2384H | G | 7250 | A | NRXN1 | K539N | C | 2957 | A |
| MAP1A | K646N | G | 1969 | T | MRPL17 | F15V | A | 148 | C | NAV2 | F1644C | T | 5030 | G | NRXN1 | - | C | 0 | T |
| MAP1A | S1088F | C | 3294 | T | MRPL18 | R108H | G | 445 | A | NAV2 | R1967Q | G | 5999 | A | NRXN1 | I892L | T | 4014 | G |
| MAP1A | G1358R | G | 4103 | A | MRPL18 | R108H | G | 445 | A | NAV3 | M2257I | G | 6944 | A | NRXN2 | S333L | G | 1210 | A |
| MAP1A | R3003H | G | 9039 | A | MRPL19 | K214N | A | 667 | C | NAV3 | V1481A | T | 4615 | C | NRXN2 | R1085H | C | 3466 | T |
| MAP1B | K1217T | A | 3948 | C | MRPL2 | R130H | C | 814 | T | NAV3 | G1294S | G | 4053 | A | NRXN2 | A1322V | G | 4177 | A |
| MAP1B | V2205M | G | 6911 | A | MRPL21 | G150* | C | 466 | A | NAV3 | D843N | G | 2700 | A | NRXN2 | A275T | C | 1035 | T |
| MAP1B | K781N | A | 2641 | C | MRPL21 | T164A | T | 508 | C | NAV3 | E714D | G | 2315 | T | NRXN2 | R651W | G | 2163 | A |
| MAP1B | E1001K | G | 3299 | A | MRPL21 | A245T | C | 751 | T | NAV3 | I1096T | T | 3460 | C | NRXN2 | A1146V | G | 3649 | A |
| MAP1B | E1009D | G | 3325 | T | MRPL22 | R63G | A | 228 | G | NAV3 | A1225S | G | 3846 | T | NRXN2 | G1618V | C | 5065 | A |
| MAP1B | D1370Y | G | 4406 | T | MRPL24 | S28F | G | 220 | A | NAV3 | E552D | G | 1829 | T | NRXN2 | A804T | C | 2622 | T |
| MAP1LC3A | A100T | G | 434 | A | MRPL27 | P119S | G | 355 | A | NAV3 | E679A | A | 2209 | C | NRXN2 | R868W | G | 2814 | A |
| MAP1LC3B | K65E | A | 821 | G | MRPL28 | R163* | G | 564 | A | NAV3 | P947T | C | 3012 | A | NRXN3 | E549K | G | 1645 | A |
| MAP1LC3C | R46G | T | 201 | C | MRPL28 | D160G | T | 556 | C | NAV3 | E1583* | G | 4920 | T | NRXN3 | D6N | G | 16 | A |
| MAP1S | A623V | C | 1889 | T | MRPL3 | F347L | A | 1201 | G | NAV3 | K1695N | G | 5258 | T | NRXN3 | E981K | G | 2941 | A |

| | | | | | | | | | | | | | | | | | | | |
|---|---|---|---|---|---|---|---|---|---|---|---|---|---|---|---|---|---|---|---|
| MAP2 | R905* | C | 3219 | T | MRPL30 | R33C | C | 295 | T | NAV3 | T349S | A | 1218 | T | NRXN3 | V328A | T | 983 | C |
| MAP2 | R1583C | C | 5253 | T | MRPL35 | R100I | G | 333 | T | NBAS | R304S | C | 938 | A | NRXN3 | T752S | A | 2254 | T |
| MAP2 | I1287V | A | 4365 | G | MRPL37 | R27K | G | 157 | A | NBAS | A995S | C | 3009 | A | NRXN3 | T965M | C | 2894 | T |
| MAP2 | V1390A | T | 4675 | C | MRPL37 | R326W | C | 1053 | T | NBAS | D2060A | T | 6205 | G | NRXN3 | A53V | C | 158 | T |
| MAP2 | R905* | C | 3219 | T | MRPL37 | A311V | C | 1009 | T | NBAS | Q990R | T | 2995 | C | NRXN3 | E224K | G | 670 | A |
| MAP2 | S886G | A | 3162 | G | MRPL38 | P131L | G | 930 | A | NBAS | Q2322R | T | 6991 | C | NRXN3 | R574H | G | 1721 | A |
| MAP2K1 | F53L | T | 690 | G | MRPL4 | A267T | G | 953 | A | NBAS | E778K | C | 2358 | T | NSA2 | R11H | G | 401 | A |
| MAP2K1 | A132V | C | 926 | T | MRPL40 | R73Q | G | 871 | A | NBAS | R456L | C | 1393 | A | NSD1 | R1171H | G | 3557 | A |
| MAP2K1 | V258I | G | 1303 | A | MRPL40 | R164Q | G | 1144 | A | NBAS | K379T | T | 1162 | G | NSD1 | G1929W | G | 5830 | T |
| MAP2K1 | K35N | G | 636 | T | MRPL44 | F195L | T | 652 | C | NBAS | E225D | T | 701 | G | NSD1 | R1587H | G | 4805 | A |
| MAP2K1 | - | T | 1711 | C | MRPL44 | F271L | T | 882 | G | NBEA | F1903V | T | 5913 | G | NSD1 | R2633W | C | 7942 | T |
| MAP2K2 | P291H | G | 1126 | T | MRPL45 | R96H | G | 447 | A | NBEA | R2219H | G | 6862 | A | NSD1 | E1569D | G | 4752 | T |
| MAP2K3 | R46W | C | 136 | T | MRPL47 | K229N | C | 717 | A | NBEA | R203* | C | 813 | T | NSD1 | K2067E | A | 6244 | G |
| MAP2K3 | K247T | A | 740 | C | MRPL49 | P53L | C | 249 | T | NBEA | - | T | 0 | C | NSD1 | C342Y | G | 1070 | A |
| MAP2K4 | - | G | 0 | C | MRPL52 | G39D | G | 146 | A | NBEA | V1379A | T | 4342 | C | NSD1 | E1248K | G | 3787 | A |
| MAP2K4 | E152* | G | 517 | T | MRPL54 | A55T | G | 197 | A | NBEA | R203* | C | 813 | T | NSFL1C | A3V | G | 102 | A |
| MAP2K5 | R20H | G | 686 | A | MRPS11 | R118W | C | 617 | T | NBEA | R2756W | C | 8472 | T | NSL1 | K259N | C | 796 | A |
| MAP2K7 | - | A | 0 | G | MRPS12 | R44W | C | 440 | T | NBEA | E2002G | A | 6211 | G | NSMAF | E639* | C | 1976 | A |
| MAP2K7 | E258K | G | 772 | A | MRPS14 | R112H | C | 352 | T | NBEA | V2229A | T | 6892 | C | NSMAF | N220H | T | 719 | G |
| MAP2K7 | R195L | G | 584 | T | MRPS18A | R247H | C | 752 | T | NBEA | R696Q | G | 2293 | A | NSMCE1 | C194G | A | 661 | C |
| MAP2K7 | R195L | G | 584 | T | MRPS18A | - | A | 0 | G | NBEA | R46G | A | 342 | G | NSUN2 | V216M | C | 958 | T |
| MAP3K1 | V1274A | T | 3821 | C | MRPS2 | - | T | 0 | C | NBEA | G1309R | G | 4131 | A | NSUN2 | R687* | G | 2371 | A |
| MAP3K10 | P627H | C | 2168 | A | MRPS21 | R43Q | G | 190 | A | NBEA | G2881W | G | 8847 | T | NSUN2 | P474Q | G | 1733 | T |
| MAP3K10 | - | G | 0 | T | MRPS22 | C231Y | G | 1124 | A | NBEA | F2297L | C | 7097 | A | NSUN2 | A722T | C | 2476 | T |
| MAP3K11 | R208Q | C | 1116 | T | MRPS22 | K203N | A | 1041 | C | NBEA | E103* | G | 513 | T | NSUN3 | R99Q | G | 507 | A |
| MAP3K11 | R147C | G | 932 | A | MRPS23 | D62Y | C | 221 | A | NBEA | S304* | C | 1117 | A | NSUN3 | Y29C | A | 297 | G |
| MAP3K11 | R757H | C | 2763 | T | MRPS25 | T71M | G | 353 | A | NBEA | S760R | A | 2484 | C | NSUN3 | R255H | G | 975 | A |
| MAP3K11 | Y322C | T | 1458 | C | MRPS26 | A69T | G | 329 | A | NBEA | R1001* | C | 3207 | T | NSUN5 | R85* | G | 279 | A |
| MAP3K11 | R203Q | C | 1101 | T | MRPS27 | S164Y | G | 501 | T | NBEA | V1895F | G | 5889 | T | NSUN7 | N446H | A | 1831 | C |
| MAP3K11 | P675L | G | 2517 | A | MRPS27 | Q275* | G | 833 | A | NBEA | S2621R | C | 8069 | A | NSUN7 | R279Q | G | 1331 | A |
| MAP3K12 | R572Q | C | 1715 | T | MRPS28 | F80V | A | 261 | C | NBEA | - | G | 0 | T | NT5C2 | R456H | C | 1455 | T |
| MAP3K12 | S847N | C | 2540 | T | MRPS35 | D203Y | G | 655 | T | NBEA | E2298K | G | 7098 | A | NT5C3L | R47* | G | 452 | A |
| MAP3K12 | G167W | C | 499 | A | MRPS35 | R164W | C | 538 | T | NBEAL1 | A152T | G | 787 | A | NT5DC1 | - | G | 0 | A |
| MAP3K12 | R744* | G | 2230 | A | MRPS5 | A385V | G | 1363 | A | NBEAL1 | V429A | T | 1619 | C | NT5DC2 | D552G | T | 1695 | C |
| MAP3K12 | S678G | T | 2032 | C | MRPS5 | A233V | G | 907 | A | NBEAL1 | N2622S | A | 8198 | G | NT5DC3 | R156W | G | 507 | A |
| MAP3K12 | Q773R | T | 2318 | C | MRPS6 | A114T | G | 518 | A | NBEAL1 | A1195S | G | 3916 | T | NT5E | E357* | G | 1118 | T |
| MAP3K12 | R656Q | C | 1967 | T | MRPS7 | R180H | G | 772 | A | NBEAL1 | N1367S | A | 4433 | G | NT5M | A119T | G | 571 | A |
| MAP3K12 | V212A | A | 635 | G | MRPS7 | R178H | G | 766 | A | NBEAL1 | E2153* | G | 6790 | T | NT5M | K99N | G | 513 | T |
| MAP3K13 | R592* | C | 2029 | T | MRPS9 | H318R | A | 1063 | G | NBEAL2 | V2715A | T | 8163 | C | NTAN1 | R204C | G | 703 | A |

| | | | | | | | | | | | | | | | | | | | |
|---|---|---|---|---|---|---|---|---|---|---|---|---|---|---|---|---|---|---|---|
| MAP3K13 | G262* | G | 1039 | T | MRS2 | R314C | C | 1062 | T | NBEAL2 | E1616K | G | 4865 | A | NTF6B | W213C | C | 639 | A |
| MAP3K15 | A327V | G | 980 | A | MRS2 | V181I | G | 663 | A | NBEAL2 | A1832T | G | 5513 | A | NTHL1 | A11S | C | 50 | A |
| MAP3K15 | R1302G | T | 3904 | C | MRS2 | E372D | G | 1238 | T | NBEAL2 | A2199V | C | 6615 | T | NTN1 | A457V | C | 1477 | T |
| MAP3K2 | Y452C | T | 1638 | C | MRS2 | S228Y | C | 805 | A | NBEAL2 | R2584H | G | 7770 | A | NTN1 | Y77C | A | 337 | G |
| MAP3K3 | G532E | G | 1914 | A | MRS2 | S368A | T | 1224 | G | NBEAL2 | R1759H | G | 5295 | A | NTN3 | R332H | G | 1198 | A |
| MAP3K3 | T106I | C | 636 | T | MRVI1 | E291K | C | 950 | T | NBEAL2 | P2453L | C | 7377 | T | NTN3 | D440N | G | 1521 | A |
| MAP3K3 | L138S | T | 732 | C | MRVI1 | R553H | C | 1737 | T | NBEAL2 | A2716T | G | 8165 | A | NTN3 | N405K | C | 1418 | A |
| MAP3K4 | Y668H | T | 2144 | C | MRVI1 | P517H | G | 1629 | T | NBEAL2 | R424Q | G | 1290 | A | NTN3 | D360Y | G | 1281 | T |
| MAP3K4 | L766S | T | 2439 | C | MRVI1 | G389S | C | 1244 | T | NBEAL2 | V680M | G | 2057 | A | NTN3 | G401D | G | 1405 | A |
| MAP3K4 | T1365A | A | 4235 | G | MRVI1 | E282* | C | 923 | A | NBEAL2 | P2679L | C | 8055 | T | NTN4 | M1T | A | 451 | G |
| MAP3K4 | S87N | G | 402 | A | MRVI1 | K281N | C | 922 | A | NBEAL2 | V1931L | G | 5810 | T | NTN4 | A407V | G | 1669 | A |
| MAP3K4 | M577V | A | 1871 | G | MRVI1 | P517H | G | 1629 | T | NBEAL2 | G702D | G | 2124 | A | NTN4 | R45Q | C | 583 | T |
| MAP3K4 | G1475V | G | 4566 | T | MRVI1 | Q844K | G | 2609 | T | NBEAL2 | R265H | G | 813 | A | NTN4 | A179T | C | 984 | T |
| MAP3K4 | G411D | G | 1374 | A | MRVI1 | A71T | C | 290 | T | NBEAL2 | E1959K | G | 5894 | A | NTN4 | K32R | T | 544 | C |
| MAP3K5 | R1272Q | C | 4176 | T | MS4A1 | T41M | C | 275 | T | NBN | L302I | G | 1014 | T | NTN5 | R386W | G | 1252 | A |
| MAP3K5 | R1370Q | C | 4470 | T | MS4A1 | K50N | G | 303 | T | NBPF15 | C443F | G | 1817 | T | NTN5 | A379V | G | 1232 | A |
| MAP3K5 | G81S | C | 602 | T | MS4A10 | E48K | G | 238 | A | NBPF3 | Q144K | C | 780 | A | NTNG2 | A6V | C | 793 | T |
| MAP3K5 | L246I | G | 1097 | T | MS4A12 | N232K | T | 753 | A | NBR1 | R347* | C | 1179 | T | NTRK1 | M671T | T | 2068 | C |
| MAP3K6 | R428C | G | 1522 | A | MS4A12 | - | G | 0 | A | NBR1 | Q558H | G | 1814 | T | NTRK1 | R347C | C | 1095 | T |
| MAP3K6 | R923Q | C | 3008 | T | MS4A14 | E20K | G | 623 | A | NBR1 | T800M | C | 2539 | T | NTRK2 | D385G | A | 2092 | G |
| MAP3K6 | P604H | G | 2051 | T | MS4A14 | K258N | G | 1339 | T | NBR1 | R937W | C | 2949 | T | NTRK3 | K602R | T | 1967 | C |
| MAP3K6 | A199S | C | 835 | A | MS4A2 | F173C | T | 620 | G | NBR1 | N569S | A | 1846 | G | NTRK3 | K9N | C | 189 | A |
| MAP3K6 | Q165H | C | 735 | A | MS4A4A | V99M | G | 385 | A | NBR2 | L82I | C | 521 | A | NTRK3 | D75G | T | 386 | C |
| MAP3K6 | P228H | G | 923 | T | MS4A6E | I48S | T | 208 | G | NCAM1 | D266G | A | 797 | G | NTRK3 | A830S | C | 2650 | A |
| MAP3K6 | R651W | G | 2191 | A | MS4A7 | R237W | C | 854 | T | NCAM1 | T316A | A | 946 | G | NTRK3 | E287D | C | 1023 | A |
| MAP3K6 | F1081L | A | 3481 | G | MS4A7 | S85P | T | 398 | C | NCAM1 | G725C | G | 2173 | T | NTS | D25Y | G | 182 | T |
| MAP3K7 | A7S | C | 181 | A | MS4A7 | E152* | G | 599 | T | NCAM1 | H800N | C | 2398 | A | NTSR2 | R109H | C | 361 | T |
| MAP3K7 | A61S | C | 343 | A | MS4A8B | S55L | C | 367 | T | NCAM1 | F37L | C | 111 | A | NTSR2 | A60V | G | 214 | A |
| MAP3K7 | Y585C | T | 1916 | C | MSC | D43G | T | 446 | C | NCAM1 | H696N | C | 2086 | A | NTSR2 | A65V | G | 229 | A |
| MAP3K7 | Q537* | G | 1771 | A | MSC | A118V | G | 671 | A | NCAM2 | L174I | C | 769 | A | NUAK1 | R410H | C | 2609 | T |
| MAP3K8 | T316M | C | 1636 | T | MSGN1 | G85S | G | 276 | A | NCAM2 | R66W | A | 445 | T | NUAK1 | I656V | T | 3346 | C |
| MAP3K9 | E38A | T | 113 | G | MSGN1 | A141T | G | 444 | A | NCAM2 | K450N | G | 1599 | T | NUAK1 | E233A | T | 2078 | G |
| MAP3K9 | G933V | C | 2798 | A | MSH2 | Q337* | C | 1232 | T | NCAM2 | E767* | G | 2548 | T | NUAK1 | D187N | C | 1939 | T |
| MAP3K9 | A39E | G | 116 | T | MSH2 | E530* | G | 1811 | T | NCAN | L602M | C | 1903 | A | NUAK2 | R385C | G | 1153 | A |
| MAP4 | P157S | G | 557 | A | MSH2 | E580* | G | 1961 | T | NCAN | T871M | C | 2711 | T | NUAK2 | R15S | G | 43 | T |
| MAP4 | C552W | A | 1744 | C | MSH3 | K860Q | A | 2658 | C | NCAN | R71Q | G | 311 | A | NUB1 | G330E | G | 1049 | A |
| MAP4 | K521N | C | 1651 | A | MSH4 | T927N | C | 2884 | A | NCAN | R1282* | C | 3943 | T | NUBP1 | T293M | C | 921 | T |
| MAP4 | - | A | 0 | G | MSH4 | V930D | T | 2893 | A | NCAN | R637C | C | 2008 | T | NUBP1 | G280S | G | 881 | A |
| MAP4 | A2258V | G | 6861 | A | MSH4 | R352H | G | 1159 | A | NCAN | R1194C | C | 3679 | T | NUBP2 | T133M | C | 518 | T |

| | | | | | | | | | | | | | | | | | | | |
|---|---|---|---|---|---|---|---|---|---|---|---|---|---|---|---|---|---|---|---|
| MAP4K1 | R589C | G | 1873 | A | MSH4 | I183V | A | 651 | G | NCAN | P728L | C | 2282 | T | NUBP2 | V121M | G | 481 | A |
| MAP4K1 | S797P | A | 2497 | G | MSH4 | L204V | T | 714 | G | NCAPD2 | R126H | G | 1176 | A | NUBP2 | A268T | G | 922 | A |
| MAP4K2 | T443M | G | 1420 | A | MSH4 | R822I | G | 2569 | T | NCAPD2 | R519H | G | 2355 | A | NUBP2 | G186R | G | 676 | A |
| MAP4K2 | P299H | G | 988 | T | MSH4 | E881D | G | 2747 | T | NCAPD2 | L170I | C | 1307 | A | NUCB1 | V312M | G | 1268 | A |
| MAP4K3 | A179V | G | 861 | A | MSH5 | M839T | T | 2595 | C | NCAPD2 | K456Q | A | 2165 | C | NUCB1 | R377Q | G | 1464 | A |
| MAP4K3 | S893I | C | 3003 | A | MSH6 | R1076H | G | 3379 | A | NCAPD3 | R1235* | G | 4309 | A | NUCB1 | R348M | G | 1377 | T |
| MAP4K3 | E416G | T | 1572 | C | MSH6 | R577H | G | 1882 | A | NCAPG | E800* | G | 2574 | T | NUCB1 | R381C | C | 1475 | T |
| MAP4K4 | T47M | C | 214 | T | MSH6 | R961I | G | 3034 | T | NCAPG | I793L | A | 2553 | C | NUCB2 | K281T | A | 1087 | C |
| MAP4K4 | R425C | C | 1347 | T | MSI2 | V52G | T | 328 | G | NCAPG2 | L457S | A | 1484 | G | NUDC | E126D | G | 501 | T |
| MAP4K4 | A196V | C | 661 | T | MSL1 | E380* | G | 1138 | T | NCAPG2 | A701T | C | 2215 | T | NUDCD1 | R521H | C | 1673 | T |
| MAP4K4 | - | G | 0 | A | MSL2 | I534T | A | 2334 | G | NCAPH | E147D | A | 484 | C | NUDCD1 | R521H | C | 1673 | T |
| MAP4K4 | G729V | G | 2260 | T | MSL2 | R537C | G | 2342 | A | NCAPH | G321R | G | 1004 | A | NUDCD1 | L566V | A | 1807 | C |
| MAP4K4 | T684A | A | 2124 | G | MSL2 | R466C | G | 2129 | A | NCAPH | D612H | G | 1877 | C | NUDCD1 | E451* | C | 1462 | A |
| MAP4K4 | K74N | G | 296 | T | MSL2 | P146S | G | 1169 | A | NCAPH | R696M | G | 2130 | T | NUDT11 | V72A | A | 367 | G |
| MAP4K4 | R1114I | G | 3415 | T | MSLN | G439V | G | 1360 | T | NCAPH2 | E46K | G | 214 | A | NUDT12 | K453E | T | 1454 | C |
| MAP4K4 | F1139C | T | 3490 | G | MSLN | A247T | G | 783 | A | NCAPH2 | T565I | C | 1772 | T | NUDT13 | R11K | G | 150 | A |
| MAP4K5 | R172* | G | 834 | A | MSLN | A503T | G | 1551 | A | NCAPH2 | A264T | G | 868 | A | NUDT14 | V45I | C | 133 | T |
| MAP4K5 | E705Q | C | 2433 | G | MSLN | S20N | G | 103 | A | NCBP1 | K707N | G | 2377 | T | NUDT16L1 | F62V | T | 193 | G |
| MAP4K5 | R172* | G | 834 | A | MSLN | D431Y | G | 1335 | T | NCBP1 | F152C | T | 711 | G | NUDT17 | P119S | G | 367 | A |
| MAP4K5 | S670I | C | 2329 | A | MSLNL | R736H | C | 2207 | T | NCDN | V316M | G | 1073 | A | NUDT18 | T104R | G | 430 | C |
| MAP4K5 | K571T | T | 2032 | G | MSLNL | T437M | G | 1310 | A | NCDN | R610Q | G | 1956 | A | NUDT18 | A122T | C | 483 | T |
| MAP4K5 | L493M | G | 1797 | T | MSLNL | R415C | G | 1243 | A | NCEH1 | H404R | T | 1349 | C | NUDT21 | R66C | G | 369 | A |
| MAP7 | L371W | A | 1230 | C | MSLNL | T78I | G | 233 | A | NCF2 | A219T | C | 846 | T | NUDT22 | S185R | A | 603 | C |
| MAP7D1 | S733I | G | 2418 | T | MSLNL | R90H | C | 269 | T | NCF4 | D234G | A | 885 | G | NUDT22 | S65A | T | 243 | G |
| MAP7D1 | Q690H | G | 2290 | T | MSLNL | T656R | G | 1967 | C | NCF4 | R105Q | G | 498 | A | NUDT6 | R269M | C | 840 | A |
| MAP7D1 | P743S | C | 2447 | T | MSMB | E64K | G | 277 | A | NCK2 | P228S | C | 1124 | T | NUDT8 | R42C | G | 134 | A |
| MAP7D2 | E546* | C | 1655 | A | MSMP | S26F | G | 217 | A | NCKAP1 | R393H | C | 1937 | T | NUDT9 | K222T | A | 989 | C |
| MAP7D2 | K465N | C | 1414 | A | MSN | R81H | G | 414 | A | NCKAP1 | H595Q | A | 2544 | T | NUF2 | A149V | C | 598 | T |
| MAP7D2 | R41W | G | 140 | A | MSN | E185* | G | 725 | T | NCKAP1 | G972R | C | 3673 | T | NUF2 | R19C | C | 207 | T |
| MAP7D2 | C595Y | C | 1803 | T | MSN | D166G | A | 669 | G | NCKAP1 | - | T | 4164 | G | NUFIP1 | R423Q | C | 1315 | T |
| MAP7D2 | E546* | C | 1655 | A | MSN | I97T | T | 462 | C | NCKAP1L | R223S | C | 746 | A | NUFIP1 | G267* | C | 846 | A |
| MAP7D3 | R607C | G | 2040 | A | MSR1 | R44C | G | 130 | A | NCKAP1L | L487M | C | 1538 | A | NUMA1 | E526* | C | 1730 | A |
| MAP9 | S392L | G | 1439 | A | MSR1 | T454A | T | 1360 | C | NCKAP1L | M812I | G | 2515 | T | NUMA1 | A2097T | C | 6443 | T |
| MAP9 | R21I | C | 326 | A | MSR1 | E235K | C | 703 | T | NCKAP1L | Q446P | A | 1416 | C | NUMA1 | M259T | A | 930 | G |
| MAPK1 | L244I | G | 919 | T | MSR1 | S18R | T | 52 | G | NCKAP1L | A746T | G | 2315 | A | NUMB | N405D | T | 1231 | C |
| MAPK10 | S50C | T | 675 | A | MSRA | G154D | G | 710 | A | NCKAP5 | F651V | A | 2325 | C | NUMB | R33H | C | 116 | T |
| MAPK12 | G184W | C | 866 | A | MSRB2 | G182C | G | 544 | T | NCKAP5 | P1037L | G | 3484 | A | NUMBL | R292W | G | 1042 | A |
| MAPK14 | R73Q | G | 665 | A | MSRB2 | R158C | C | 472 | T | NCKAP5 | R1513Q | C | 4912 | T | NUMBL | R62C | G | 352 | A |
| MAPK15 | E187D | G | 680 | T | MSRB3 | S161L | C | 543 | T | NCKAP5 | R877S | C | 3005 | A | NUMBL | K204R | T | 779 | C |

| | | | | | | | | | | | | | | | | | | | |
|---|---|---|---|---|---|---|---|---|---|---|---|---|---|---|---|---|---|---|---|
| MAPK4 | E554K | G | 2660 | A | MST1 | R374H | C | 1483 | T | NCKAP5 | H1433R | T | 4672 | C | NUMBL | P28L | G | 251 | A |
| MAPK4 | A422V | C | 2265 | T | MST1 | A367T | C | 1461 | T | NCKAP5 | R762M | C | 2659 | A | NUMBL | K315N | C | 1113 | A |
| MAPK4 | D538N | G | 2612 | A | MST1 | M448V | T | 1704 | C | NCKAP5 | Q1805R | T | 5788 | C | NUP107 | E792* | G | 2489 | T |
| MAPK6 | D179E | T | 1325 | A | MST1 | P153R | G | 820 | C | NCKAP5 | S20R | G | 434 | T | NUP133 | A587T | C | 1851 | T |
| MAPK7 | R543W | C | 2013 | T | MST1R | R1248H | C | 3771 | T | NCKAP5 | E1905A | T | 6088 | G | NUP133 | R775* | G | 2415 | A |
| MAPK7 | R369C | C | 1491 | T | MST1R | R1118H | C | 3381 | T | NCKAP5 | S1702Y | G | 5479 | T | NUP153 | R133W | G | 597 | A |
| MAPK7 | A579T | G | 2121 | A | MST1R | R308H | C | 951 | T | NCKAP5 | E150K | C | 822 | T | NUP153 | E850D | C | 2750 | A |
| MAPK7 | R326H | G | 1363 | A | MST1R | P863L | G | 2616 | A | NCKAP5 | D27N | C | 453 | T | NUP153 | P554S | G | 1860 | A |
| MAPK8 | R189G | C | 746 | G | MST1R | I1186F | T | 3584 | A | NCKAP5L | R1171C | G | 3713 | A | NUP155 | T272M | G | 1019 | A |
| MAPK8IP1 | R259W | C | 945 | T | MSTN | - | C | 0 | T | NCKAP5L | G723V | C | 2370 | A | NUP155 | S399Y | G | 1400 | T |
| MAPK8IP1 | A495T | G | 1653 | A | MSTN | R52I | C | 288 | A | NCKIPSD | R191C | G | 665 | A | NUP155 | D485G | T | 1658 | C |
| MAPK8IP2 | R674W | C | 2020 | T | MSTO1 | A334T | G | 1024 | A | NCLN | - | G | 0 | A | NUP155 | R1389W | G | 4369 | A |
| MAPK8IP2 | D77N | G | 229 | A | MSX2 | G262D | G | 912 | A | NCLN | P463L | C | 1542 | T | NUP155 | I1254M | A | 3966 | C |
| MAPK8IP2 | M83I | G | 249 | T | MSX2 | P225T | C | 800 | A | NCOA1 | K1015N | A | 3697 | C | NUP155 | F823V | A | 2671 | C |
| MAPK8IP3 | D818N | G | 2599 | A | MSX2 | T257M | C | 897 | T | NCOA1 | S137A | T | 1061 | G | NUP160 | R1136H | C | 3492 | T |
| MAPK8IP3 | A768T | G | 2449 | A | MSX2 | Y264C | A | 918 | G | NCOA1 | F300V | T | 1550 | G | NUP160 | R1133* | G | 3482 | A |
| MAPK8IP3 | S282L | C | 992 | T | MT1G | R55W | G | 233 | A | NCOA2 | T281M | G | 1004 | A | NUP160 | D384N | C | 1235 | T |
| MAPK8IP3 | F413L | C | 1386 | A | MT1G | F82V | A | 314 | C | NCOA2 | R338C | G | 1174 | A | NUP160 | A17T | C | 134 | T |
| MAPKAP1 | Q55H | C | 234 | A | MT1G | D81N | C | 311 | T | NCOA2 | G637W | C | 2071 | A | NUP188 | E1241K | G | 3742 | A |
| MAPKAP1 | F289L | G | 936 | T | MT1M | C48Y | G | 253 | A | NCOA2 | W954R | A | 3022 | G | NUP188 | - | G | 0 | T |
| MAPKAPK 2 | I54L | A | 353 | C | MT1X | P61S | C | 196 | T | NCOA2 | E96* | C | 448 | A | NUP188 | R1385Q | G | 4175 | A |
| MAPKAPK 5 | A197T | G | 848 | A | MTA1 | A536V | C | 1821 | T | NCOA2 | A1046V | G | 3299 | A | NUP188 | E216* | G | 667 | T |
| MAPKAPK 5 | N305S | A | 1173 | G | MTA1 | R633H | G | 2112 | A | NCOA2 | H449P | T | 1508 | G | NUP188 | L1013V | T | 3058 | G |
| MAPKBP1 | W371C | G | 1349 | T | MTA1 | A71V | C | 426 | T | NCOA3 | R36Q | G | 298 | A | NUP188 | M1667V | A | 5020 | G |
| MAPKBP1 | A846T | G | 2772 | A | MTA1 | E135K | G | 617 | A | NCOA3 | T194P | A | 771 | C | NUP205 | V190A | T | 595 | C |
| MAPKBP1 | R303C | C | 1143 | T | MTA1 | D715Y | G | 2357 | T | NCOA4 | - | T | 0 | C | NUP205 | R980H | G | 2965 | A |
| MAPKBP1 | G754S | G | 2496 | A | MTA3 | R490W | C | 1468 | T | NCOA4 | V641I | G | 2173 | A | NUP205 | D102Y | G | 330 | T |
| MAPRE2 | V130A | T | 559 | C | MTAP | I31L | A | 91 | C | NCOA4 | L10I | C | 280 | A | NUP205 | F1826L | C | 5504 | A |
| MAPT | V368I | G | 1422 | A | MTBP | T386A | A | 1201 | G | NCOA4 | - | G | 0 | T | NUP205 | R154W | C | 486 | T |
| MAPT | E176G | A | 847 | G | MTBP | K528T | A | 1628 | C | NCOA5 | R61W | G | 346 | A | NUP205 | R1808L | G | 5449 | T |
| MARCH1 | T34P | T | 588 | G | MTBP | T689S | C | 2111 | G | NCOA6 | S1490L | G | 7040 | A | NUP205 | V1645A | T | 4960 | C |
| MARCH1 | R20* | G | 546 | A | MTCH2 | G254R | C | 949 | T | NCOA6 | P1326S | G | 6547 | A | NUP205 | F531S | T | 1618 | C |
| MARCH10 | R660C | G | 2263 | A | MTCP1 | A54V | G | 875 | A | NCOA6 | R143Q | C | 2999 | T | NUP205 | A1207V | C | 3646 | T |
| MARCH10 | E638D | C | 2199 | A | MTERF | E347K | C | 1133 | T | NCOA6 | S1709Y | G | 7697 | T | NUP210 | I1042S | A | 3208 | C |
| MARCH10 | S450Y | G | 1634 | T | MTERFD1 | L195I | G | 682 | T | NCOA6 | Q709H | C | 4698 | A | NUP210 | R1321C | G | 4044 | A |
| MARCH4 | G244D | C | 2498 | T | MTF1 | R260W | G | 919 | A | NCOR1 | E354* | C | 1300 | A | NUP210 | P670H | G | 2092 | T |
| MARCH4 | R33H | C | 1865 | T | MTF1 | D63G | T | 329 | C | NCOR1 | R66Q | C | 437 | T | NUP210 | K265N | C | 878 | A |

| | | | | | | | | | | | | | | | | | | | |
|---|---|---|---|---|---|---|---|---|---|---|---|---|---|---|---|---|---|---|---|
| MARCH5 | A244V | C | 1063 | T | MTF1 | G68R | C | 343 | T | NCOR1 | G972S | C | 3154 | T | NUP210 | S94G | T | 363 | C |
| MARCH6 | R592Q | G | 1907 | A | MTF2 | Y525* | T | 1864 | A | NCOR1 | V1474A | A | 4661 | G | NUP210L | T67A | T | 271 | C |
| MARCH6 | A596T | G | 1918 | A | MTF2 | R347Q | G | 1329 | A | NCOR1 | R310H | C | 1169 | T | NUP210L | S145L | G | 506 | A |
| MARCH6 | R48Q | G | 275 | A | MTFMT | K284T | T | 877 | G | NCOR2 | T2315M | G | 7100 | A | NUP210L | Q1236* | G | 3778 | A |
| MARCH7 | C99G | T | 555 | G | MTFMT | S125* | G | 400 | T | NCOR2 | P1112L | G | 3491 | A | NUP210L | Y1697* | A | 5163 | C |
| MARCH8 | C248* | G | 742 | T | MTFR1 | P193H | C | 689 | A | NCOR2 | P796S | G | 2542 | A | NUP210L | S1359Y | G | 4148 | T |
| MARCH8 | E251G | T | 750 | C | MTG1 | L105Q | T | 364 | A | NCOR2 | R2474Q | C | 7577 | T | NUP210L | S1308Y | G | 3995 | T |
| MARCH8 | R286G | G | 854 | C | MTHFD1L | A442T | G | 1598 | A | NCOR2 | A1976T | C | 6082 | T | NUP210L | Q729H | T | 2259 | G |
| MARCO | S230G | A | 823 | G | MTHFD1L | K909N | G | 3001 | T | NCOR2 | D776N | C | 2482 | T | NUP210L | N129H | T | 457 | G |
| MARCO | P34T | C | 235 | A | MTHFD2 | L116V | T | 426 | G | NCOR2 | E459K | C | 1531 | T | NUP214 | A255T | G | 874 | A |
| MARK1 | P697S | C | 2355 | T | MTHFR | L270P | A | 809 | G | NCR1 | P86L | C | 295 | T | NUP214 | E344D | G | 1143 | T |
| MARK1 | R722Q | G | 2431 | A | MTHFR | R92Q | C | 275 | T | NCR2 | W54* | G | 250 | A | NUP214 | S1752G | A | 5365 | G |
| MARK1 | R309* | C | 1191 | T | MTHFS | I179F | T | 596 | A | NCS1 | E84K | G | 336 | A | NUP214 | K716E | A | 2257 | G |
| MARK1 | S699Y | C | 2362 | A | MTHFSD | R335Q | C | 1055 | T | NCSTN | P421H | C | 1387 | A | NUP35 | R52Q | G | 258 | A |
| MARK2 | P569T | C | 1917 | A | MTHFSD | L26V | A | 127 | C | NDE1 | R275Q | G | 1650 | A | NUP35 | G278E | G | 936 | A |
| MARK2 | R328W | C | 1194 | T | MTIF2 | T194M | G | 730 | A | NDE1 | Q167K | C | 1325 | A | NUP37 | L218F | T | 719 | A |
| MARK2 | M34V | A | 312 | G | MTIF2 | E402K | C | 1353 | T | NDEL1 | E101* | G | 301 | T | NUP43 | D58G | T | 230 | C |
| MARK2 | L361M | C | 1293 | A | MTIF2 | R168M | C | 652 | A | NDN | R265Q | C | 880 | T | NUP43 | I4M | A | 69 | C |
| MARK3 | R682C | C | 2710 | T | MTIF2 | Q250R | T | 898 | C | NDNL2 | W278C | C | 958 | A | NUP85 | E263K | G | 1047 | A |
| MARK4 | R420H | G | 1264 | A | MTIF2 | I519T | A | 1705 | G | NDNL2 | A287V | G | 984 | A | NUP88 | S87G | T | 289 | C |
| MARK4 | G368S | G | 1107 | A | MTL5 | K401N | T | 1343 | G | NDOR1 | S569* | C | 1789 | A | NUP88 | R467* | G | 1429 | A |
| MARK4 | R418H | G | 1258 | A | MTM1 | E305K | G | 967 | A | NDOR1 | F523L | C | 1652 | A | NUP93 | R376H | G | 1228 | A |
| MARS | R727W | C | 2202 | T | MTM1 | R207H | G | 674 | A | NDP | C95Y | C | 692 | T | NUP93 | R77* | C | 330 | T |
| MARS | Q843H | A | 2552 | C | MTM1 | N428S | A | 1337 | G | NDRG1 | L160I | G | 667 | T | NUP93 | R44C | C | 231 | T |
| MARVELD 2 | - | G | 0 | A | MTM1 | T138M | C | 467 | T | NDRG1 | A108T | C | 511 | T | NUP93 | R77* | C | 330 | T |
| MAS1 | R185* | C | 567 | T | MTM1 | R520Q | G | 1613 | A | NDRG2 | R62H | C | 268 | T | NUP98 | R995C | G | 3400 | A |
| MASP1 | V643I | C | 2153 | T | MTM1 | L119P | T | 410 | C | NDRG3 | S264L | G | 848 | A | NUP98 | Y493H | A | 1894 | G |
| MASP1 | E636G | T | 2297 | C | MTM1 | R174K | G | 575 | A | NDRG3 | R23I | C | 125 | A | NUP98 | E1585D | C | 5172 | A |
| MASP1 | D513E | G | 1929 | T | MTMR1 | Q344P | A | 1188 | C | NDRG4 | R75W | C | 336 | T | NUP98 | R204C | G | 1027 | A |
| MASP2 | A360T | C | 1094 | T | MTMR1 | N345K | C | 1192 | G | NDRG4 | A129V | C | 499 | T | NUP98 | R817S | G | 2866 | T |
| MASP2 | G634R | C | 1916 | T | MTMR1 | G52* | G | 311 | T | NDST2 | S624G | T | 2427 | C | NUPL1 | P332L | C | 1245 | T |
| MASP2 | R555I | C | 1680 | A | MTMR10 | T516M | G | 1645 | A | NDST3 | P118T | C | 755 | A | NUPL1 | S102P | T | 554 | C |
| MASP2 | C142R | A | 440 | G | MTMR10 | G722S | C | 2262 | T | NDST3 | D415Y | G | 1646 | T | NVL | R798* | G | 2652 | A |
| MAST1 | E486* | G | 1495 | T | MTMR10 | R575Q | C | 1822 | T | NDST3 | K250E | A | 1151 | G | NVL | L776V | A | 2586 | C |
| MAST1 | R540* | C | 1657 | T | MTMR11 | A397V | G | 1410 | A | NDST4 | F720L | G | 2839 | T | NVL | E527D | T | 1841 | G |
| MAST1 | P368L | C | 1142 | T | MTMR11 | A171T | C | 731 | T | NDST4 | L598I | G | 2471 | T | NVL | E79D | T | 497 | G |
| MAST1 | R561C | C | 1720 | T | MTMR12 | T473I | G | 1589 | A | NDST4 | S382N | C | 1824 | T | NWD1 | A1219T | G | 3663 | A |
| MAST2 | R530W | C | 1871 | T | MTMR12 | N600H | T | 1969 | G | NDST4 | Q332H | T | 1675 | G | NWD1 | S1397N | G | 4198 | A |
| MAST2 | E941G | A | 3105 | G | MTMR14 | T560M | C | 1801 | T | NDST4 | L184F | G | 1229 | A | NWD1 | S526I | G | 1585 | T |

| | | | | | | | | | | | | | | | | | | | |
|---|---|---|---|---|---|---|---|---|---|---|---|---|---|---|---|---|---|---|---|
| MAST3 | F367L | T | 1099 | C | MTMR14 | R600L | G | 1921 | T | NDUFA10 | V251I | C | 774 | T | NXF1 | I397M | A | 1327 | C |
| MAST3 | T1004A | A | 3010 | G | MTMR15 | K482N | G | 1706 | T | NDUFA10 | F275L | A | 846 | G | NXNL1 | H184Q | G | 599 | T |
| MAST3 | I953V | A | 2857 | G | MTMR15 | A332T | G | 1254 | A | NDUFA12 | F108C | A | 378 | C | NXNL1 | A192V | G | 622 | A |
| MAST3 | P929L | C | 2786 | T | MTMR15 | C954R | T | 3120 | C | NDUFA4 | A26V | G | 276 | A | NXPH1 | K116N | G | 1259 | T |
| MAST4 | R199H | G | 893 | A | MTMR15 | R824C | C | 2730 | T | NDUFA6 | R131W | G | 453 | A | NXPH2 | R188H | C | 670 | T |
| MAST4 | R1503W | C | 4804 | T | MTMR15 | I205M | T | 875 | G | NDUFA9 | A364V | C | 1101 | T | NXPH2 | C187Y | C | 667 | T |
| MAST4 | R1675* | C | 5320 | T | MTMR15 | R697I | G | 2350 | T | NDUFA9 | R222W | C | 674 | T | NXPH3 | P215H | C | 742 | A |
| MAST4 | A2051V | C | 6449 | T | MTMR2 | A629S | C | 2226 | A | NDUFA9 | P268L | C | 813 | T | NXPH3 | A208T | G | 720 | A |
| MAST4 | R874W | C | 2917 | T | MTMR2 | K610Q | T | 2169 | G | NDUFA9 | A372T | G | 1124 | A | NXPH4 | R244C | C | 905 | T |
| MAST4 | R917* | C | 3046 | T | MTMR2 | M180I | C | 881 | A | NDUFAF1 | A161T | C | 879 | T | NYNRIN | P8H | C | 341 | A |
| MASTL | S684L | C | 2108 | T | MTMR3 | P309L | C | 1268 | T | NDUFAF1 | R179Q | C | 934 | T | NYNRIN | P1867S | C | 5917 | T |
| MASTL | P371L | C | 1169 | T | MTMR3 | R356W | C | 1408 | T | NDUFAF1 | P275L | G | 1222 | A | NYNRIN | R1855Q | G | 5882 | A |
| MASTL | R650I | G | 2006 | T | MTMR3 | R797* | C | 2731 | T | NDUFAF4 | E89K | C | 345 | T | NYNRIN | P547S | C | 1957 | T |
| MAT2A | A157T | G | 592 | A | MTMR4 | R156C | G | 576 | A | NDUFB10 | R16C | C | 153 | T | NYNRIN | R827Q | G | 2798 | A |
| MAT2A | R84H | G | 374 | A | MTMR4 | Q794H | C | 2492 | A | NDUFB10 | Y62C | A | 292 | G | NYNRIN | E991* | G | 3289 | T |
| MAT2A | I266T | T | 920 | C | MTMR6 | C72Y | C | 977 | T | NDUFB4 | R12H | G | 86 | A | NYX | E157K | G | 925 | A |
| MAT2B | E190D | A | 686 | C | MTMR6 | R594C | G | 2542 | A | NDUFB5 | V108G | T | 322 | G | OAS1 | S376Y | C | 1233 | A |
| MAT2B | R312* | C | 1050 | T | MTMR7 | I545F | T | 1668 | A | NDUFB5 | I138S | T | 412 | G | OAS2 | K197N | G | 731 | T |
| MATK | S435L | G | 1304 | A | MTMR7 | R73H | C | 253 | T | NDUFB7 | R56Q | C | 241 | T | OAS3 | K207T | A | 799 | C |
| MATK | A393V | G | 1178 | A | MTMR9 | T454M | C | 1775 | T | NDUFC2 | R59C | G | 650 | A | OAS3 | P224T | C | 849 | A |
| MATN1 | G403S | C | 1243 | T | MTMR9 | K8N | G | 438 | T | NDUFS1 | R552Q | C | 1655 | T | OAS3 | T673M | C | 2197 | T |
| MATN1 | A340V | G | 1055 | A | MTMR9 | R77* | C | 643 | T | NDUFS1 | R422H | C | 1265 | T | OASL | R352Q | C | 1326 | T |
| MATN1 | E289D | C | 903 | A | MTNR1A | R225H | C | 876 | T | NDUFS1 | R36* | G | 106 | A | OASL | D333G | T | 1269 | C |
| MATN2 | G412D | G | 1466 | A | MTNR1B | A28V | C | 186 | T | NDUFS5 | R16Q | G | 134 | A | OAT | A404T | C | 1303 | T |
| MATN2 | D175N | G | 754 | A | MTNR1B | T157I | C | 573 | T | NDUFS8 | Y104C | A | 418 | G | OAZ2 | V176G | A | 527 | C |
| MATN2 | Y884F | A | 2882 | T | MTNR1B | F102L | C | 409 | A | NDUFV1 | R449Q | G | 1499 | A | OBFC1 | H317Y | G | 1117 | A |
| MATN3 | K477N | T | 1494 | G | MTNR1B | R316H | G | 1050 | A | NDUFV2 | N62H | A | 301 | C | OBFC1 | - | A | 0 | G |
| MATN4 | V178M | C | 541 | T | MTO1 | - | G | 0 | C | NDUFV3 | M407L | A | 1288 | T | OBSCN | A983V | C | 2992 | T |
| MATN4 | L358I | G | 1081 | T | MTO1 | Q643H | G | 2053 | T | NDUFV3 | K62N | G | 255 | T | OBSCN | R2408W | C | 7266 | T |
| MATN4 | A619V | G | 1865 | A | MTOR | R403Q | C | 1285 | T | NEB | S6547N | C | 19843 | T | OBSCN | A5481V | C | 16486 | T |
| MAX | R60Q | C | 310 | T | MTOR | A1792V | G | 5452 | A | NEB | E5904* | C | 17913 | A | OBSCN | E7801K | G | 23445 | A |
| MBD2 | Y196H | A | 815 | G | MTOR | R491Q | C | 1549 | T | NEB | K3562N | C | 10889 | A | OBSCN | W7577L | G | 22774 | T |
| MBD2 | G198* | C | 821 | A | MTOR | N1899D | T | 5772 | C | NEB | - | C | 0 | A | OBSCN | G813V | G | 2482 | T |
| MBD3 | R65C | G | 193 | A | MTOR | T1844A | T | 5607 | C | NEB | E2594D | C | 7985 | A | OBSCN | S4833N | G | 14542 | A |
| MBD4 | - | C | 0 | A | MTOR | G716S | C | 2223 | T | NEB | S6213A | A | 18840 | C | OBSCN | R6749H | G | 20290 | A |
| MBD4 | R251M | C | 928 | A | MTOR | V2417M | C | 7326 | T | NEB | V1155M | C | 3666 | T | OBSCN | G7107E | G | 2136 | A |

| | | | | | | | | | | | | | | | | | | | |
|---|---|---|---|---|---|---|---|---|---|---|---|---|---|---|---|---|---|---|---|
| | | | | | | | | | | | | | | | | | | 4 | |
| MBD5 | T720A | A | 2287 | G | MTOR | R628H | C | 1960 | T | NEB | R6013Q | C | 18241 | T | OBSCN | A5808T | G | 17466 | A |
| MBD5 | A897T | G | 2818 | A | MTOR | I2457M | A | 7448 | C | NEB | - | C | 0 | A | OBSCN | Y6412C | A | 19279 | G |
| MBD5 | I521N | T | 1691 | A | MTOR | L783R | A | 2425 | C | NEB | D4825N | C | 14676 | T | OBSCN | N6638I | A | 19957 | T |
| MBD5 | A1320V | C | 4088 | T | MTPAP | L422I | G | 1264 | T | NEB | V3031I | C | 9294 | T | OBSCN | E1261A | A | 3826 | C |
| MBD5 | H330Q | C | 1119 | A | MTPAP | - | C | 0 | A | NEB | Q2660R | T | 8182 | C | OBSCN | R231H | G | 736 | A |
| MBD5 | H113N | C | 466 | A | MTR | L1192I | C | 3968 | A | NEB | G2554D | C | 7864 | T | OBSCN | L3795I | C | 11427 | A |
| MBD5 | S550Y | C | 1778 | A | MTR | W1150* | G | 3844 | A | NEB | S4529R | T | 13788 | G | OBSCN | A255T | G | 807 | A |
| MBD6 | R374Q | G | 1345 | A | MTRF1 | F55C | A | 164 | C | NEB | - | A | 0 | G | OBSCN | R245C | C | 777 | T |
| MBD6 | I37T | T | 334 | C | MTRR | Q359* | C | 1105 | T | NEB | V2883I | C | 8850 | T | OBSCN | G5240D | G | 15763 | A |
| MBL2 | V234I | C | 765 | T | MTRR | L451F | G | 1383 | T | NEB | R3538W | G | 10815 | A | OBSCN | R1397* | C | 4233 | T |
| MBL2 | G51E | C | 217 | T | MTRR | R621S | A | 1893 | T | NEB | R2799H | C | 8599 | T | OBSCN | E3049D | G | 9191 | T |
| MBLAC2 | R242Q | C | 1201 | T | MTRR | L370F | G | 1140 | T | NEB | E1957G | T | 6073 | C | OBSCN | H3860N | C | 11622 | A |
| MBNL1 | R29Q | G | 1928 | A | MTSS1 | A127T | C | 697 | T | NEB | R6172G | T | 18717 | C | OBSCN | S4542Y | C | 13669 | A |
| MBNL1 | Q44R | A | 1973 | G | MTSS1L | A189V | G | 826 | A | NEB | A5408T | C | 16425 | T | OBSCN | K5773N | G | 17363 | T |
| MBNL3 | E227D | C | 760 | A | MTSS1L | G560S | C | 1938 | T | NEB | E4640* | C | 14121 | A | OBSCN | E6132K | G | 18438 | A |
| MBOAT7 | A346T | C | 1517 | T | MTSS1L | A562T | C | 1944 | T | NEB | E4152* | C | 12657 | A | OBSCN | D6221N | G | 18705 | A |
| MBOAT7 | G385W | C | 1634 | A | MTTP | M456I | G | 1381 | T | NEB | E3454A | T | 10564 | G | OBSCN | R465C | C | 1437 | T |
| MBP | E129D | C | 651 | A | MTTP | M499I | G | 1510 | A | NEB | K3302N | C | 10109 | A | OBSCN | Y5141C | A | 15466 | G |
| MBP | A135V | G | 668 | A | MTTP | L624M | C | 1883 | A | NEB | K823Q | T | 2670 | G | OBSL1 | V1820I | C | 5515 | T |
| MBP | K28N | T | 348 | G | MTUS1 | L641V | G | 2395 | C | NEB | D733V | T | 2401 | A | OBSL1 | A1619T | C | 4912 | T |
| MBTPS1 | T242M | G | 1258 | A | MTUS1 | A587D | G | 2234 | T | NEB | L4010V | G | 12231 | C | OBSL1 | R415W | G | 1300 | A |
| MBTPS1 | L562M | G | 2217 | T | MTUS2 | E754* | G | 2318 | T | NEBL | L406I | G | 1613 | T | OBSL1 | A1480G | G | 4496 | C |
| MC1R | G43R | G | 127 | A | MTUS2 | G1130S | G | 3446 | A | NEBL | G91D | C | 669 | T | OBSL1 | D1644N | C | 4987 | T |
| MC1R | S485L | C | 1454 | T | MTUS2 | R252C | C | 812 | T | NEBL | F594C | A | 2178 | C | OBSL1 | T1436M | G | 4364 | A |
| MC2R | L55I | G | 340 | T | MTUS2 | A920V | C | 2817 | T | NECAB1 | C285* | C | 1132 | A | OBSL1 | F816L | A | 2503 | G |
| MC3R | V330I | G | 988 | A | MTUS2 | P758H | C | 2331 | A | NECAB1 | S289Y | C | 1143 | A | OC90 | - | T | 0 | C |
| MC3R | H190Y | C | 568 | T | MTUS2 | P608H | C | 1881 | A | NECAB2 | R385Q | G | 1177 | A | OC90 | V74M | C | 320 | T |
| MC4R | C138F | C | 832 | A | MTUS2 | E390D | G | 1228 | T | NECAB3 | R369C | G | 1161 | A | OC90 | E422D | T | 1366 | G |
| MC4R | R18C | G | 471 | A | MTUS2 | A1238E | C | 3771 | A | NECAB3 | S361T | A | 1137 | T | OC90 | N268T | T | 903 | G |
| MC4R | K73T | T | 637 | G | MTUS2 | L1271I | C | 3869 | A | NECAB3 | E93K | C | 333 | T | OC90 | P44H | G | 131 | T |
| MC5R | V202G | T | 827 | G | MTX2 | V186I | G | 767 | A | NECAP1 | D87A | A | 338 | C | OC90 | R371* | G | 1211 | A |

| | | | | | | | | | | | | | | | | | | | |
|---|---|---|---|---|---|---|---|---|---|---|---|---|---|---|---|---|---|---|---|
| MC5R | R303Q | G | 1130 | A | MTX3 | F72L | A | 235 | G | NEDD1 | F14S | T | 187 | C | OC90 | I122M | A | 366 | C |
| MC5R | V75M | G | 445 | A | MUC1 | L108M | G | 388 | T | NEDD1 | I493M | A | 1625 | G | OC90 | N142S | T | 425 | C |
| MC5R | G165S | G | 715 | A | MUC12 | A493V | C | 1478 | T | NEDD1 | E539D | G | 1763 | T | OCA2 | R566C | G | 1852 | A |
| MCAM | R127H | C | 409 | T | MUC12 | S4960Y | C | 14879 | A | NEDD4 | V549I | C | 1945 | T | OCA2 | D372N | C | 1270 | T |
| MCAM | D476Y | C | 1455 | A | MUC13 | F227L | A | 718 | G | NEDD4 | V761A | A | 2582 | G | OCA2 | K613N | T | 1995 | G |
| MCART6 | A35T | C | 284 | T | MUC13 | E456D | C | 1407 | A | NEDD4 | A822V | G | 2765 | A | OCA2 | A339T | C | 1171 | T |
| MCART6 | S194Y | G | 762 | T | MUC13 | T450M | G | 1388 | A | NEDD4 | E632* | C | 2194 | A | OCA2 | - | T | 0 | C |
| MCC | R65H | C | 610 | T | MUC16 | I5936M | T | 17808 | C | NEDD4L | G247D | G | 740 | A | OCEL1 | L126P | T | 379 | C |
| MCC | D237G | T | 1126 | C | MUC16 | S3128Y | G | 9383 | T | NEDD4L | L230P | T | 689 | C | OCEL1 | K140R | A | 421 | G |
| MCC | S22G | T | 480 | C | MUC16 | V7888I | C | 23662 | T | NEDD9 | P109Q | G | 493 | T | OCIAD1 | D219N | G | 845 | A |
| MCC | E619K | C | 2271 | T | MUC16 | P4164H | G | 12491 | T | NEDD9 | T764A | T | 2457 | C | OCIAD2 | D148N | C | 675 | T |
| MCC | S24G | T | 486 | C | MUC16 | A3135V | G | 9404 | A | NEFH | R323H | G | 1001 | A | OCLN | R162I | G | 921 | T |
| MCCC1 | A195T | C | 730 | T | MUC16 | A273V | G | 818 | A | NEFH | A70T | G | 241 | A | OCLN | R268Q | G | 1239 | A |
| MCCC1 | R281* | G | 988 | A | MUC16 | T2565A | T | 7693 | C | NEFH | A68T | G | 235 | A | OCM2 | K29T | T | 178 | G |
| MCF2 | T911I | G | 2763 | A | MUC16 | T6502I | G | 19505 | A | NEFH | S801Y | C | 2435 | A | OCRL | E899K | G | 2860 | A |
| MCF2 | R139W | G | 446 | A | MUC16 | A6156T | C | 18466 | T | NEFL | G32D | C | 690 | T | OCRL | S78F | C | 398 | T |
| MCF2 | - | C | 0 | T | MUC16 | L1474I | G | 4420 | T | NEFL | K499T | T | 2091 | G | ODC1 | R183W | G | 1058 | A |
| MCF2 | Q340R | T | 1050 | C | MUC16 | E6809* | C | 20425 | A | NEFL | - | C | 0 | T | ODC1 | E7D | T | 532 | G |
| MCF2 | R176H | C | 558 | T | MUC16 | A3749T | C | 11245 | T | NEFM | E123* | G | 1149 | T | ODF1 | R34Q | G | 209 | A |
| MCF2L | A154T | G | 482 | A | MUC16 | P7257Q | G | 21770 | T | NEFM | A213V | C | 1420 | T | ODF1 | R63C | C | 295 | T |
| MCF2L2 | T219M | G | 954 | A | MUC16 | T8509A | T | 25525 | C | NEFM | T889N | C | 3448 | A | ODF1 | E30K | G | 196 | A |
| MCF2L2 | P619T | G | 2153 | T | MUC16 | A5410T | C | 16228 | T | NEFM | R196C | C | 1368 | T | ODF1 | R242Q | G | 833 | A |
| MCF2L2 | R406G | T | 1514 | C | MUC16 | T6622I | G | 19865 | A | NEFM | R137Q | G | 1192 | A | ODF2 | L458P | T | 1684 | C |
| MCF2L2 | R926Q | C | 3075 | T | MUC16 | S2768P | A | 8302 | G | NEFM | P673Q | C | 2800 | A | ODF2 | - | G | 0 | A |
| MCHR1 | G264S | G | 1486 | A | MUC16 | G667W | C | 1999 | A | NEFM | K793N | G | 3161 | T | ODF2 | E577D | G | 2042 | T |
| MCHR2 | W9C | C | 118 | A | MUC16 | L8512W | A | 25535 | C | NEFM | F452V | T | 2136 | G | ODF2 | R271Q | G | 1123 | A |
| MCHR2 | F208S | A | 714 | G | MUC16 | K8082N | T | 24246 | G | NEGR1 | I345T | A | 1274 | G | ODF2L | T31A | T | 438 | C |
| MCHR2 | A265D | G | 885 | T | MUC16 | T6901I | G | 20702 | A | NEGR1 | G324E | C | 1211 | T | ODF2L | L488R | A | 1810 | C |
| MCHR2 | F207L | A | 712 | C | MUC16 | S6881F | G | 2064 | A | NEIL1 | A111T | G | 837 | A | ODF2L | K387T | T | 1507 | G |

| | | | | | | | | | | | | | | | | | | | |
|---|---|---|---|---|---|---|---|---|---|---|---|---|---|---|---|---|---|---|---|
| | | | | | | | | 2 | | | | | | | | | | | |
| MCL1 | G148R | C | 502 | T | MUC16 | S6863I | C | 20588 | A | NEIL3 | F589V | T | 1883 | G | ODF2L | E141A | T | 769 | G |
| MCM10 | R259Q | G | 880 | A | MUC16 | S6623F | G | 19868 | A | NEK1 | R742C | G | 2804 | A | ODF3 | R14H | G | 358 | A |
| MCM10 | N772S | A | 2419 | G | MUC16 | T7023A | T | 21067 | C | NEK1 | D128Y | C | 962 | A | ODF3L1 | S210P | T | 851 | C |
| MCM2 | D221N | G | 905 | A | MUC16 | N6782S | T | 20345 | C | NEK1 | R630C | G | 2468 | A | ODF3L1 | T52M | C | 378 | T |
| MCM2 | T158M | C | 717 | T | MUC16 | M6087I | C | 18261 | A | NEK10 | L308I | G | 1196 | T | ODF3L2 | Q173H | C | 755 | A |
| MCM2 | A452V | C | 1599 | T | MUC16 | S6021Y | G | 18062 | T | NEK10 | C639Y | C | 2190 | T | ODF3L2 | R98H | C | 529 | T |
| MCM2 | T666S | C | 2241 | G | MUC16 | S5348Y | G | 16043 | T | NEK10 | S781L | G | 2616 | A | ODF4 | N165T | A | 682 | C |
| MCM2 | R33W | C | 341 | T | MUC16 | V5214F | C | 15640 | A | NEK10 | S618F | G | 2127 | A | ODZ1 | F2276L | G | 6889 | T |
| MCM3 | L344P | A | 1031 | G | MUC16 | V4753A | A | 14258 | G | NEK10 | E382D | T | 1420 | G | ODZ1 | A231T | C | 752 | T |
| MCM3 | G809D | C | 2426 | T | MUC16 | A4548S | C | 13642 | A | NEK10 | F347C | A | 1314 | C | ODZ1 | F161L | G | 544 | C |
| MCM3AP | E659K | C | 2230 | T | MUC16 | I4528M | T | 13584 | C | NEK10 | N96K | A | 562 | C | ODZ1 | V94I | C | 341 | T |
| MCM3AP | S680L | G | 2294 | A | MUC16 | F4459L | G | 13377 | T | NEK10 | K17T | T | 324 | G | ODZ1 | Y1573C | T | 4779 | C |
| MCM3AP | A17V | G | 305 | A | MUC16 | S3993Y | G | 11978 | T | NEK11 | L397F | A | 1484 | C | ODZ1 | I2363V | T | 7148 | C |
| MCM3AP | L1518M | G | 4807 | T | MUC16 | S3711Y | G | 11132 | T | NEK11 | Q33H | A | 392 | C | ODZ1 | - | C | 0 | T |
| MCM3AP | E421K | C | 1516 | T | MUC16 | T3159K | G | 9476 | T | NEK11 | K258Q | A | 1065 | C | ODZ1 | F2366C | A | 7158 | C |
| MCM3AP | A867T | C | 2854 | T | MUC16 | E2683* | C | 8047 | A | NEK11 | E377* | G | 1422 | T | ODZ1 | Q2216K | G | 6707 | T |
| MCM4 | Q594* | C | 1875 | T | MUC16 | E2346A | T | 7037 | G | NEK5 | R96W | G | 426 | A | ODZ1 | S1272Y | G | 3876 | T |
| MCM4 | H420N | C | 1353 | A | MUC16 | I1488M | A | 4464 | C | NEK5 | R443I | C | 1468 | A | ODZ1 | S1137Y | G | 3471 | T |
| MCM4 | D655G | A | 2059 | G | MUC16 | T837N | G | 2510 | T | NEK5 | G140R | C | 558 | T | ODZ1 | T455A | T | 1424 | C |
| MCM5 | G203E | G | 762 | A | MUC16 | D713E | G | 2139 | T | NEK5 | K49N | C | 287 | A | ODZ1 | I450S | A | 1410 | C |
| MCM6 | V376I | C | 1187 | T | MUC16 | A282V | G | 845 | A | NEK7 | R282Q | G | 1159 | A | ODZ1 | E621K | C | 1922 | T |
| MCM6 | R722Q | C | 2226 | T | MUC17 | A1263T | G | 3851 | A | NEK7 | T199M | C | 910 | T | ODZ2 | R59K | G | 176 | A |
| MCM7 | R611H | C | 2478 | T | MUC17 | R4318W | C | 13016 | T | NEK7 | L222R | T | 979 | G | ODZ2 | T1701A | A | 5101 | G |
| MCM7 | R590Q | C | 2415 | T | MUC17 | I1445T | T | 4398 | C | NEK8 | Q53H | G | 159 | T | ODZ2 | S1780N | G | 5339 | A |
| MCM7 | R396* | G | 1832 | A | MUC17 | G4228R | G | 12746 | A | NEK8 | P689L | C | 2066 | T | ODZ2 | R2164C | C | 6490 | T |
| MCM7 | L356F | G | 1712 | A | MUC17 | P1407L | C | 4284 | T | NEK9 | K199N | C | 751 | A | ODZ2 | R358S | G | 1074 | T |
| MCM8 | T205A | A | 990 | G | MUC17 | R4422W | C | 1332 | T | NEK9 | V240I | C | 872 | T | ODZ2 | R2141W | C | 6421 | T |

| | | | | | | | | | | | | | | | | | | | |
|---|---|---|---|---|---|---|---|---|---|---|---|---|---|---|---|---|---|---|---|
| | | | | | | | | 8 | | | | | | | | | | | |
| MCOLN1 | R486C | C | 1579 | T | MUC17 | G3151C | G | 9515 | T | NEK9 | V468M | C | 1556 | T | ODZ2 | M2218V | A | 6652 | G |
| MCOLN2 | R217W | G | 890 | A | MUC17 | V3529M | G | 1064 9 | A | NEK9 | T250M | G | 903 | A | ODZ2 | P182T | C | 544 | A |
| MCOLN3 | S374G | T | 1171 | C | MUC17 | L1280* | T | 3903 | G | NELF | F279I | A | 1067 | T | ODZ2 | A876V | C | 2627 | T |
| MCPH1 | P367L | C | 1168 | T | MUC17 | V2186A | T | 6621 | C | NELL1 | W149* | G | 600 | A | ODZ2 | R2086W | C | 6256 | T |
| MCPH1 | S834P | T | 2568 | C | MUC2 | C574Y | G | 1748 | A | NELL1 | - | A | 0 | T | ODZ2 | E1559K | G | 4675 | A |
| MCRS1 | R439H | C | 1316 | T | MUC2 | A216T | G | 673 | A | NELL1 | D206A | A | 770 | C | ODZ3 | V690I | G | 2191 | A |
| MCTP1 | L482P | A | 1445 | G | MUC2 | R1385H | G | 4181 | A | NELL2 | V766F | C | 2421 | A | ODZ3 | P2449S | C | 7468 | T |
| MCTP1 | K604R | T | 1811 | C | MUC2 | N770D | A | 2335 | G | NELL2 | S513Y | G | 1663 | T | ODZ3 | D72Y | G | 337 | T |
| MCTP1 | N960K | G | 2880 | C | MUC2 | R2308H | G | 6950 | A | NELL2 | H682R | T | 2170 | C | ODZ3 | V2596M | G | 7909 | A |
| MCTP1 | L813F | G | 2437 | A | MUC2 | P2549S | C | 7672 | T | NELL2 | R244I | C | 856 | A | ODZ3 | T209A | A | 748 | G |
| MCTP1 | R699Q | C | 2096 | T | MUC2 | R486H | G | 1484 | A | NELL2 | K220T | T | 784 | G | ODZ3 | N1136T | A | 3530 | C |
| MCTP1 | F523C | A | 1568 | C | MUC2 | A249T | G | 772 | A | NELL2 | L28I | G | 207 | T | ODZ3 | S1338R | C | 4137 | G |
| MCTP2 | K827T | A | 2829 | C | MUC2 | K454N | G | 1389 | T | NENF | T92M | C | 332 | T | ODZ3 | A2490T | G | 7591 | A |
| MCTS1 | G36D | G | 640 | A | MUC2 | G1362E | G | 4112 | A | NES | A962V | G | 3018 | A | ODZ3 | A1177V | C | 3653 | T |
| MDGA1 | E248K | C | 742 | T | MUC4 | A1870T | C | 5680 | T | NETO1 | R261H | C | 1440 | T | ODZ3 | Y1187H | T | 3682 | C |
| MDGA1 | R327* | G | 979 | A | MUC4 | A4685T | C | 1412 5 | T | NETO1 | R5C | G | 671 | A | ODZ3 | S127P | T | 502 | C |
| MDGA1 | T862M | G | 2585 | A | MUC4 | A760T | C | 2350 | T | NETO1 | A75T | C | 881 | T | ODZ3 | F328V | T | 1105 | G |
| MDGA1 | S745Y | G | 2234 | T | MUC4 | R2432H | C | 7367 | T | NETO1 | K249T | T | 1404 | G | ODZ3 | L1296* | T | 4010 | G |
| MDGA2 | R30C | G | 249 | A | MUC4 | G870S | C | 2680 | T | NETO1 | Y215* | G | 1303 | T | ODZ3 | K1794T | A | 5504 | C |
| MDGA2 | I502S | A | 1666 | C | MUC4 | - | C | 0 | T | NETO1 | D105Y | C | 971 | A | ODZ3 | S2071Y | C | 6335 | A |
| MDH1B | F416L | A | 1524 | C | MUC4 | S4133N | C | 1247 0 | T | NETO2 | R273* | G | 1202 | A | ODZ4 | D2218N | C | 7115 | T |
| MDM1 | K660Q | T | 2114 | G | MUC4 | S4133G | T | 1246 9 | C | NETO2 | G356W | C | 1451 | A | ODZ4 | S1902Y | G | 6168 | T |
| MDM1 | E338* | C | 1148 | A | MUC5B | S3751N | G | 1137 8 | A | NETO2 | T430A | T | 1673 | C | ODZ4 | R2238W | G | 7175 | A |
| MDM1 | F238V | A | 848 | C | MUC5B | T3267M | C | 9926 | T | NETO2 | V205I | C | 998 | T | ODZ4 | A1165T | C | 3956 | T |
| MDM1 | R236I | C | 843 | A | MUC5B | V5398I | G | 1631 8 | A | NEU2 | R283H | G | 848 | A | ODZ4 | A1962V | G | 6348 | A |
| MDM2 | V234A | T | 1003 | C | MUC5B | A799V | C | 2522 | T | NEU3 | R278W | C | 1759 | T | ODZ4 | L2162F | G | 6947 | A |
| MDM2 | R189C | C | 867 | T | MUC5B | R3985W | C | 1207 9 | T | NEU3 | P122S | C | 1291 | T | ODZ4 | V1023I | C | 3530 | T |
| MDN1 | Q3186H | C | 9674 | A | MUC5B | P3737L | C | 1133 6 | T | NEU4 | A212T | G | 1063 | A | ODZ4 | L1412V | A | 4697 | C |
| MDN1 | V3293I | C | 9993 | T | MUC5B | T897M | C | 2816 | T | NEU4 | R399C | C | 1624 | T | ODZ4 | V122A | A | 828 | G |
| MDN1 | R3240C | G | 9834 | A | MUC5B | G1558V | G | 4799 | T | NEU4 | R143S | C | 856 | A | ODZ4 | R1816H | C | 5910 | T |
| MDN1 | R892I | C | 2791 | A | MUC5B | A966T | G | 3022 | A | NEU4 | F213S | T | 1067 | C | ODZ4 | A346T | C | 1499 | T |
| MDN1 | G4048S | C | 1225 8 | T | MUC5B | V5240M | G | 1584 4 | A | NEURL | R132C | C | 803 | T | ODZ4 | C1057Y | C | 3633 | T |

| | | | | | | | | | | | | | | | | | | | |
|---|---|---|---|---|---|---|---|---|---|---|---|---|---|---|---|---|---|---|---|
| MDN1 | G2262R | C | 6900 | T | MUC5B | L840P | T | 2645 | C | NEURL | S501L | C | 1911 | T | ODZ4 | A2496V | G | 7950 | A |
| MDN1 | R3240C | G | 9834 | A | MUC5B | V5359I | G | 16201 | A | NEURL | R548H | G | 2052 | A | ODZ4 | R973W | G | 3380 | A |
| MDN1 | S3878P | A | 11748 | G | MUC5B | R5466H | G | 16523 | A | NEURL1B | V69I | G | 339 | A | ODZ4 | T513A | T | 2000 | C |
| MDN1 | M703T | A | 2224 | G | MUC5B | P2303L | C | 7034 | T | NEURL1B | A496V | C | 1621 | T | OFCC1 | V111A | A | 381 | G |
| MDN1 | V628L | C | 1998 | A | MUC5B | F1309L | T | 4051 | C | NEURL2 | S136G | T | 702 | C | OFD1 | E490K | G | 1795 | A |
| MDN1 | R66H | C | 313 | T | MUC5B | E2157K | G | 6595 | A | NEURL4 | R1016C | G | 3053 | A | OFD1 | R344Q | G | 1358 | A |
| MDN1 | R984H | C | 3067 | T | MUC5B | F5274L | C | 15948 | A | NEURL4 | R366H | C | 1104 | T | OGDH | T205A | A | 666 | G |
| MDN1 | T4798A | T | 14508 | C | MUC6 | T2342M | G | 7076 | A | NEURL4 | K198T | T | 600 | G | OGDH | R275Q | G | 877 | A |
| MDN1 | V3254I | C | 9876 | T | MUC6 | T1245I | G | 3785 | A | NEUROD1 | R113H | C | 796 | T | OGDH | R994C | C | 3033 | T |
| MDN1 | A1911V | G | 5848 | A | MUC6 | E1144K | C | 3481 | T | NEUROD1 | R144H | C | 889 | T | OGDHL | V428I | C | 1368 | T |
| MDN1 | V35A | A | 220 | G | MUC6 | G839E | C | 2567 | T | NEUROD2 | P301T | G | 1122 | T | OGDHL | R758W | G | 2358 | A |
| MDN1 | C666Y | C | 2113 | T | MUCL1 | P84L | C | 319 | T | NEUROD6 | C123* | A | 692 | T | OGDHL | D810G | T | 2515 | C |
| MDN1 | - | T | 16906 | A | MUCL1 | V80A | T | 307 | C | NEUROD6 | E66* | C | 519 | A | OGDHL | R629C | G | 1971 | A |
| MDN1 | D4864G | T | 14707 | C | MUDENG | C325* | C | 1402 | A | NEUROG1 | A111S | C | 590 | A | OGDHL | R958H | C | 2959 | T |
| MDN1 | D4565E | A | 13811 | C | MUDENG | L149I | C | 872 | A | NEUROG1 | P120S | G | 617 | A | OGFOD1 | H451R | A | 1470 | G |
| MDN1 | S4427R | T | 13395 | G | MUL1 | H118N | G | 489 | T | NEUROG2 | S232F | G | 1022 | A | OGFOD2 | K82R | A | 245 | G |
| MDN1 | K3972T | T | 12031 | G | MUL1 | A342V | G | 1162 | A | NEXN | R127H | G | 564 | A | OGFR | P105S | C | 338 | T |
| MDN1 | S3763R | A | 11405 | C | MUL1 | R73W | G | 354 | A | NEXN | S243Y | C | 912 | A | OGFRL1 | R122H | G | 499 | A |
| MDN1 | I3588S | A | 10879 | C | MUL1 | C62S | C | 322 | G | NEXN | K454Q | A | 1544 | C | OGFRL1 | R218Q | G | 787 | A |
| MDN1 | R2012C | G | 6150 | A | MUM1 | V433I | G | 1396 | A | NEXN | I613L | A | 2021 | C | OGG1 | R97C | C | 632 | T |
| ME1 | E227K | C | 796 | T | MUM1 | L39V | C | 214 | G | NF1 | R1695W | C | 5466 | T | OGN | I142M | T | 619 | C |
| ME2 | R225* | C | 945 | T | MUM1 | K452E | A | 1453 | G | NF1 | A1858T | G | 5955 | A | OGT | R557H | G | 1887 | A |
| ME2 | P370S | C | 1380 | T | MUM1 | I517L | A | 1648 | C | NF1 | L104S | T | 694 | C | OGT | Q106R | A | 534 | G |
| ME3 | A556V | G | 1769 | A | MUM1L1 | P251S | C | 1400 | T | NF1 | R416* | C | 1629 | T | OIT3 | R506H | G | 1735 | A |
| ME3 | L238I | G | 814 | T | MUM1L1 | G435D | G | 1953 | A | NF2 | Q459H | G | 1818 | T | OIT3 | R506H | G | 1735 | A |
| ME3 | R547K | C | 1742 | T | MUM1L1 | S200L | C | 1248 | T | NFASC | V662I | G | 2271 | A | OLA1 | F407V | A | 1850 | C |
| MEAF6 | S136I | C | 424 | A | MURC | V147L | G | 504 | C | NFASC | A1224V | C | 3958 | T | OLAH | F106C | T | 504 | G |
| MEAF6 | G181D | C | 559 | T | MURC | S146Y | C | 502 | A | NFASC | V663I | G | 2274 | A | OLAH | F150C | T | 636 | G |
| MECOM | E729* | C | 2283 | A | MUS81 | W182C | G | 899 | T | NFASC | A1074T | G | 3507 | A | OLFM1 | - | T | 1707 | C |
| MECOM | G498S | C | 1590 | T | MUS81 | V305I | G | 1266 | A | NFASC | A1046V | C | 3424 | T | OLFM3 | R129W | G | 385 | A |
| MECP2 | R97H | C | 345 | T | MUSK | L571F | G | 1847 | T | NFASC | G456V | G | 1654 | T | OLFM3 | A358V | G | 1073 | A |
| MECR | F250L | G | 776 | T | MUSK | R842H | G | 2659 | A | NFASC | A253V | C | 1045 | T | OLFM3 | R267C | G | 799 | A |

| | | | | | | | | | | | | | | | | | | | |
|---|---|---|---|---|---|---|---|---|---|---|---|---|---|---|---|---|---|---|---|
| MECR | R139W | G | 441 | A | MUSK | E831D | G | 2627 | T | NFASC | F1218L | C | 3941 | A | OLFM3 | T107N | G | 320 | T |
| MECR | G121R | C | 387 | T | MUSK | R842H | G | 2659 | A | NFAT5 | K634Q | A | 3108 | C | OLFM4 | E452D | G | 1434 | T |
| MECR | T163I | G | 514 | A | MUT | E564* | C | 1818 | A | NFATC1 | V318I | G | 1018 | A | OLFM4 | G297R | G | 967 | A |
| MED1 | T998I | G | 3199 | A | MUT | R467Q | C | 1528 | T | NFATC1 | S297L | C | 956 | T | OLFM4 | L109I | C | 403 | A |
| MED1 | V452A | A | 1561 | G | MUT | Q383R | T | 1276 | C | NFATC1 | Q610* | C | 1894 | T | OLFML1 | I148K | T | 837 | A |
| MED1 | K1501N | T | 4709 | G | MUTYH | S346L | G | 1253 | A | NFATC1 | T926A | A | 2842 | G | OLFML2A | L501P | T | 1502 | C |
| MED1 | S1290Y | G | 4075 | T | MUTYH | N279T | T | 1052 | G | NFATC1 | R670I | G | 2075 | T | OLFML2A | R611H | G | 1832 | A |
| MED1 | S1124R | T | 3576 | G | MUTYH | Q527H | T | 1797 | G | NFATC1 | R920Q | G | 2825 | A | OLFML2A | L550P | T | 1649 | C |
| MED12 | R815W | C | 2642 | T | MUTYH | S6A | A | 232 | C | NFATC2 | R754Q | C | 2481 | T | OLFML2B | E311D | C | 1357 | A |
| MED12 | R540C | C | 1817 | T | MVD | S251N | C | 782 | T | NFATC2 | V750I | C | 2468 | T | OLFML2B | V679I | C | 2459 | T |
| MED12 | L272M | T | 1013 | A | MVK | S135L | C | 597 | T | NFATC2 | T533M | G | 1818 | A | OLFML2B | V679I | C | 2459 | T |
| MED12 | V1119G | T | 3555 | G | MVP | D615N | G | 1981 | A | NFATC2 | R572Q | C | 1935 | T | OLFML2B | K179N | T | 961 | A |
| MED12L | S718Y | C | 2191 | A | MVP | E412K | G | 1372 | A | NFATC3 | V192M | G | 598 | A | OLFML2B | E261K | C | 1205 | T |
| MED12L | D1030N | G | 3126 | A | MVP | R49H | G | 284 | A | NFATC3 | D547N | G | 1663 | A | OLFML2B | R203Q | C | 1032 | T |
| MED12L | S919* | C | 2794 | G | MX1 | R522C | C | 2589 | T | NFATC3 | V678E | T | 2057 | A | OLIG1 | R113S | C | 440 | A |
| MED12L | E449* | G | 1383 | T | MX1 | R655C | C | 2988 | T | NFATC3 | E139D | A | 441 | C | OLIG1 | A138V | C | 516 | T |
| MED12L | Q1490H | G | 4508 | T | MX2 | I223V | A | 851 | G | NFATC3 | A1001V | C | 3026 | T | OLIG2 | A53V | C | 402 | T |
| MED12L | L1569I | C | 4743 | A | MX2 | Y21C | A | 246 | G | NFATC4 | E958D | G | 2990 | T | OLIG3 | P116L | G | 571 | A |
| MED12L | A1917V | C | 5788 | T | MX2 | G125R | G | 557 | A | NFATC4 | S44N | G | 247 | A | OLR1 | W148* | C | 557 | T |
| MED13 | R1836S | T | 5585 | G | MX2 | I249V | A | 929 | G | NFATC4 | S280L | C | 955 | T | OMA1 | Y381S | T | 1256 | G |
| MED13 | P1698H | G | 5170 | T | MX2 | V123I | G | 551 | A | NFATC4 | P190L | C | 685 | T | OMA1 | V99A | A | 410 | G |
| MED13 | R471C | G | 1488 | A | MX2 | F495V | T | 1667 | G | NFATC4 | S732A | T | 2310 | G | OMG | K302T | T | 1087 | G |
| MED13 | F1963L | A | 5964 | G | MX2 | F634L | C | 2086 | A | NFE2 | R323H | C | 1241 | T | ONECUT1 | E291D | C | 1001 | G |
| MED13L | S365L | G | 1301 | A | MX2 | F647C | T | 2124 | G | NFE2L2 | R502C | G | 2059 | A | ONECUT1 | R35C | G | 231 | A |
| MED13L | V531M | C | 1798 | T | MX2 | R695I | G | 2268 | T | NFE2L2 | A124V | G | 926 | A | ONECUT2 | R314Q | G | 973 | A |
| MED13L | R440* | G | 1525 | A | MXD1 | R69W | C | 465 | T | NFE2L3 | M395I | G | 1444 | C | ONECUT2 | W378L | G | 1165 | T |
| MED13L | A1278V | G | 4040 | A | MXD3 | R225H | C | 739 | T | NFE2L3 | S297Y | C | 1149 | A | ONECUT3 | S338N | G | 1013 | A |
| MED13L | - | A | 0 | G | MXD4 | A98T | C | 606 | T | NFIA | R83Q | G | 330 | A | ONECUT3 | G310C | G | 928 | T |
| MED13L | K1873T | T | 5825 | G | MXRA5 | R2224Q | C | 6798 | T | NFIA | A432D | C | 1377 | A | OPA1 | R766* | C | 2530 | T |
| MED13L | R1283W | G | 4054 | A | MXRA5 | R2147H | C | 6567 | T | NFIA | L176I | C | 608 | A | OPA1 | I938T | T | 3047 | C |
| MED15 | - | G | 0 | A | MXRA5 | T2672M | G | 8142 | A | NFIA | D221N | G | 743 | A | OPA1 | - | A | 0 | G |
| MED16 | R456S | G | 1517 | T | MXRA5 | V503M | C | 1634 | T | NFIA | P19L | C | 138 | T | OPA3 | R26H | C | 177 | T |
| MED16 | - | C | 0 | A | MXRA5 | G674A | C | 2148 | G | NFIB | L151P | A | 1460 | G | OPALIN | E113K | C | 743 | T |
| MED17 | K235Q | A | 978 | C | MXRA5 | P1841S | G | 5648 | A | NFIL3 | V9I | C | 602 | T | OPCML | V31A | T | 92 | C |
| MED18 | F117L | C | 560 | A | MXRA5 | R682H | C | 2172 | T | NFIX | I148T | T | 443 | C | OPCML | S11Y | C | 361 | A |
| MED20 | R136Q | C | 488 | T | MXRA5 | A1485V | G | 4581 | A | NFIX | E182* | G | 544 | T | OPCML | I138M | T | 414 | G |
| MED20 | Y117H | A | 430 | G | MXRA5 | T586A | T | 1883 | C | NFKB1 | E931K | G | 3258 | A | OPLAH | L980P | A | 3021 | G |
| MED23 | L577S | A | 1904 | G | MXRA5 | K234Q | T | 827 | G | NFKB1 | K105N | A | 782 | T | OPLAH | T242M | G | 807 | A |
| MED23 | D901N | C | 2875 | T | MXRA5 | L162I | G | 611 | T | NFKB1 | A219T | G | 1122 | A | OPLAH | R615W | G | 1925 | A |

| | | | | | | | | | | | | | | | | | | | |
|---|---|---|---|---|---|---|---|---|---|---|---|---|---|---|---|---|---|---|---|
| MED24 | E473* | C | 1521 | A | MXRA5 | Y2395S | T | 7311 | G | NFKB2 | T732M | C | 2396 | T | OPLAH | R1198C | G | 3674 | A |
| MED24 | A457T | C | 1473 | T | MXRA7 | G193A | C | 606 | G | NFKBIB | D318N | G | 1010 | A | OPLAH | M1114T | A | 3423 | G |
| MED24 | C359R | A | 1179 | G | MYADM | R93H | G | 426 | A | NFKBIB | P236L | C | 765 | T | OPLAH | L916M | G | 2828 | T |
| MED24 | R672C | G | 2118 | A | MYBBP1A | G750R | C | 2310 | T | NFKBIB | D184N | G | 608 | A | OPLAH | V1086M | C | 3338 | T |
| MED25 | R132H | G | 448 | A | MYBBP1A | T1229A | T | 3747 | C | NFKBIB | S108Y | C | 381 | A | OPN1LW | E20D | G | 120 | T |
| MED25 | - | G | 0 | T | MYBL1 | R454* | G | 1360 | A | NFKBIB | R275C | C | 881 | T | OPN1SW | K321E | T | 961 | C |
| MED26 | R279Q | C | 1097 | T | MYBL1 | I230N | A | 689 | T | NFKBIL2 | H91Y | G | 301 | A | OPN3 | V190M | C | 675 | T |
| MED26 | R26W | G | 337 | A | MYBL1 | P485Q | G | 1454 | T | NFKBIL2 | E453K | C | 1387 | T | OPN4 | Q454* | C | 1527 | T |
| MED26 | A108T | C | 583 | T | MYBL1 | F224L | A | 672 | C | NFKBIL2 | R550G | T | 1678 | C | OPN4 | G284R | G | 1017 | A |
| MED27 | A150T | C | 512 | T | MYBL2 | K93Q | A | 404 | C | NFKBIL2 | A1166V | G | 3527 | A | OPN4 | P241L | C | 889 | T |
| MED28 | S38R | C | 114 | A | MYBPC1 | E458D | A | 1476 | C | NFKBIL2 | F340V | A | 1048 | C | OPN4 | R387C | C | 1326 | T |
| MED30 | R140Q | G | 570 | A | MYBPC1 | Q315R | A | 1046 | G | NFKBIL2 | R339C | G | 1045 | A | OPN5 | E232D | A | 781 | C |
| MED4 | M229I | C | 713 | A | MYBPC1 | E40K | G | 220 | A | NFKBIZ | R11H | G | 147 | A | OPN5 | Y159H | T | 560 | C |
| MED8 | R282C | G | 888 | A | MYBPC1 | H160Y | C | 580 | T | NFRKB | S1171Y | G | 3633 | T | OPRK1 | R342W | G | 1259 | A |
| MEF2C | I138N | A | 819 | T | MYBPC1 | E676* | G | 2128 | T | NFRKB | A611T | C | 1952 | T | OPRK1 | P215T | G | 878 | T |
| MEF2C | D445N | C | 1739 | T | MYBPC2 | V575I | G | 1774 | A | NFRKB | R37C | G | 230 | A | OPRL1 | A178T | G | 896 | A |
| MEF2D | P236H | G | 1188 | T | MYBPC2 | V1058M | G | 3223 | A | NFRKB | A1082S | C | 3365 | A | OPTN | A99V | C | 969 | T |
| MEF2D | V148L | C | 923 | A | MYBPC2 | R403H | G | 1259 | A | NFS1 | T92I | G | 346 | A | OPTN | R96H | G | 960 | A |
| MEFV | F748L | G | 2284 | T | MYBPC2 | S161F | C | 533 | T | NFS1 | R434Q | C | 1372 | T | OPTN | T145N | C | 1107 | A |
| MEFV | R239* | G | 755 | A | MYBPC2 | K301N | G | 954 | T | NFS1 | R119C | G | 426 | A | OR10A3 | R306Q | C | 917 | T |
| MEFV | E230* | C | 728 | A | MYBPC2 | A324V | C | 1022 | T | NFU1 | P29L | G | 292 | A | OR10A3 | N137S | T | 410 | C |
| MEGF10 | R177H | G | 776 | A | MYBPC2 | E643* | G | 1978 | T | NFX1 | R235Q | G | 766 | A | OR10A7 | R261Q | G | 782 | A |
| MEGF10 | Y1030C | A | 3335 | G | MYBPC3 | G1134D | C | 3456 | T | NFX1 | R895I | G | 2746 | T | OR10A7 | R261W | C | 781 | T |
| MEGF10 | R799H | G | 2642 | A | MYBPC3 | V790A | A | 2424 | G | NFX1 | S742R | T | 2288 | A | OR10A7 | I288V | A | 862 | G |
| MEGF11 | Q1040R | T | 3292 | C | MYBPC3 | R272H | C | 870 | T | NFX1 | K698Q | A | 2154 | C | OR10AG1 | L176F | G | 526 | A |
| MEGF11 | V547I | C | 1812 | T | MYBPC3 | T885M | G | 2709 | A | NFX1 | F982V | T | 3006 | G | OR10AG1 | L85V | A | 253 | C |
| MEGF11 | W471* | C | 1586 | T | MYBPH | E380* | C | 1196 | A | NFXL1 | R144Q | C | 500 | T | OR10G3 | A11V | G | 32 | A |
| MEGF6 | R683H | C | 2050 | T | MYBPH | D240N | C | 776 | T | NFXL1 | R435* | G | 1372 | A | OR10G3 | F104L | G | 312 | T |
| MEGF6 | F766V | A | 2298 | C | MYBPHL | A335V | G | 1054 | A | NFXL1 | A77V | G | 299 | A | OR10G3 | L64F | G | 190 | A |
| MEGF8 | R797Q | G | 3025 | A | MYCBP | E68K | C | 1002 | T | NFXL1 | G540V | C | 1688 | A | OR10G8 | Y176C | A | 527 | G |
| MEGF8 | G604C | G | 2445 | T | MYCBP | R66H | C | 997 | T | NFYA | A307D | C | 1121 | A | OR10G9 | R290S | A | 870 | C |
| MEGF8 | A1699V | C | 5731 | T | MYCBP2 | I67M | A | 292 | C | NFYA | R274C | C | 1021 | T | OR10H4 | T149N | C | 446 | A |
| MEI1 | L915I | C | 2783 | A | MYCBP2 | P181Q | G | 633 | T | NFYA | T98A | A | 493 | G | OR10H5 | E270D | A | 908 | C |
| MEI1 | V84A | T | 291 | C | MYCBP2 | S3056L | G | 9258 | A | NFYB | T75M | G | 425 | A | OR10H5 | F12C | T | 133 | G |
| MEI1 | E491D | G | 1513 | T | MYCBP2 | S962N | C | 2976 | T | NFYC | G450R | G | 1654 | A | OR10J1 | K318T | A | 953 | C |
| MEIS1 | R224H | G | 1128 | A | MYCBP2 | T4245A | T | 12824 | C | NGDN | F52L | C | 183 | A | OR10J1 | F28L | C | 84 | A |
| MEIS1 | D69Y | G | 662 | T | MYCBP2 | Q2017R | T | 6141 | C | NGEF | E190* | C | 847 | A | OR10J1 | S173Y | C | 518 | A |
| MEIS2 | E133K | C | 816 | T | MYCBP2 | Q1968* | G | 5993 | A | NGEF | V458I | C | 1651 | T | OR10J1 | S227Y | C | 680 | A |
| MEIS2 | D222N | C | 1083 | T | MYCBP2 | A4602V | G | 1389 | A | NGEF | R259W | G | 1054 | A | OR10J5 | P209T | G | 625 | T |

| | | | | | | | | 6 | | | | | | | | | | | |
|---|---|---|---|---|---|---|---|---|---|---|---|---|---|---|---|---|---|---|---|
| MEIS3P2 | P405L | G | 1661 | A | MYCBP2 | H3513N | G | 10628 | T | NGEF | T139M | G | 695 | A | OR10K1 | K263N | G | 869 | T |
| MELK | Q512E | C | 1718 | G | MYCBP2 | S2762Y | G | 8376 | T | NGEF | C400F | C | 1478 | A | OR10P1 | L246M | C | 768 | A |
| MELK | I58T | T | 357 | C | MYCBP2 | Y1277H | A | 3920 | G | NGFR | P202L | C | 730 | T | OR10R2 | E216K | G | 646 | A |
| MELK | E289* | G | 1049 | T | MYCBP2 | L1466M | A | 4487 | T | NGFR | F73L | C | 344 | A | OR10S1 | A170T | C | 508 | T |
| MEMO1P2 | R75Q | C | 281 | T | MYCBPAP | D750H | G | 2410 | C | NGLY1 | S446A | A | 1497 | C | OR10S1 | S37F | G | 110 | A |
| MEMO1P2 | G173R | C | 574 | T | MYCBPAP | Y425C | A | 1436 | G | NGLY1 | G144V | C | 592 | A | OR10T2 | K263E | T | 787 | C |
| MEOX1 | A5T | C | 42 | T | MYCBPAP | G448V | G | 1505 | T | NGLY1 | C484R | A | 1611 | G | OR10T2 | L213M | G | 637 | T |
| MEOX1 | L196P | A | 616 | G | MYCN | S64G | A | 487 | G | NGLY1 | T137P | T | 570 | G | OR11H6 | R311* | C | 931 | T |
| MEP1A | E502* | G | 1513 | T | MYCT1 | K11T | A | 32 | C | NGRN | L50P | T | 157 | C | OR11H6 | A137V | C | 410 | T |
| MEP1A | S185T | T | 562 | A | MYCT1 | F156C | T | 467 | G | NHEDC2 | Q257H | C | 933 | A | OR11H6 | F119L | C | 357 | A |
| MEP1A | S630L | C | 1898 | T | MYEF2 | E63D | C | 313 | A | NHEDC2 | L438M | G | 1474 | T | OR11H6 | R252* | C | 754 | T |
| MEP1B | S363A | T | 1134 | G | MYEOV | L144M | C | 880 | A | NHEDC2 | I395M | A | 1347 | C | OR11L1 | Y290H | A | 868 | G |
| MEP1B | E328G | A | 1030 | G | MYEOV | H300L | A | 1349 | T | NHEDC2 | S182P | A | 706 | G | OR13A1 | V212I | C | 943 | T |
| MEP1B | C124R | T | 417 | C | MYF5 | R91C | C | 406 | T | NHEDC2 | Y123D | A | 529 | C | OR13C2 | S270L | G | 809 | A |
| MEPCE | R518* | C | 1940 | T | MYF5 | R129H | G | 521 | A | NHEJ1 | L115I | G | 343 | T | OR13C3 | K119Q | T | 398 | G |
| MEPCE | Y604C | A | 2199 | G | MYF5 | C163Y | G | 623 | A | NHLH1 | R85C | C | 699 | T | OR13C4 | L66I | G | 196 | T |
| MERTK | S661F | C | 2239 | T | MYF5 | R93W | C | 412 | T | NHLH2 | V35M | C | 619 | T | OR13C5 | T288A | T | 862 | C |
| MERTK | N548K | T | 1901 | G | MYF5 | A41V | C | 257 | T | NHLRC1 | P41L | G | 136 | A | OR13C5 | F256V | A | 766 | C |
| MESDC1 | A165T | G | 1811 | A | MYF5 | E136G | A | 542 | G | NHLRC2 | G535R | G | 1815 | A | OR13C8 | E11D | A | 33 | T |
| MESDC1 | R152H | G | 1773 | A | MYF5 | Q239H | G | 852 | T | NHLRC2 | L168P | T | 715 | C | OR13D1 | S277P | T | 872 | C |
| MESDC1 | A136V | C | 1725 | T | MYF6 | S57N | G | 217 | A | NHLRC2 | D84G | A | 463 | G | OR13D1 | F44L | C | 175 | A |
| MEST | T51A | A | 372 | G | MYH1 | V1429A | A | 4381 | G | NHLRC2 | E544K | G | 1842 | A | OR13F1 | L285F | G | 944 | T |
| MEST | L156I | C | 687 | A | MYH1 | E1380D | C | 4235 | A | NHLRC3 | R226Q | G | 999 | A | OR13F1 | Q281K | C | 930 | A |
| MET | R1184* | C | 3737 | T | MYH1 | - | C | 0 | T | NHS | R373* | C | 1455 | T | OR13F1 | Q143H | G | 518 | T |
| MET | F192L | C | 763 | A | MYH1 | R1140C | G | 3513 | A | NHS | T1151M | C | 3790 | T | OR13F1 | R234* | C | 789 | T |
| MET | Y666C | A | 2184 | G | MYH1 | P31H | G | 187 | T | NHS | R562C | C | 2022 | T | OR13G1 | E193D | C | 579 | A |
| MET | R1188* | C | 3749 | T | MYH1 | P378S | G | 1227 | A | NHS | F661L | C | 2321 | A | OR13H1 | F68C | T | 301 | G |
| METAP1 | Y186* | T | 692 | G | MYH1 | R1564H | C | 4786 | T | NHS | - | G | 0 | T | OR13H1 | M257V | A | 867 | G |
| METAP1 | R289Q | G | 1000 | A | MYH1 | E1068* | C | 3297 | A | NHSL1 | R129H | C | 386 | T | OR13J1 | L154F | G | 660 | A |
| METAP2 | K188E | A | 696 | G | MYH1 | - | C | 0 | A | NHSL1 | G697R | C | 2089 | T | OR13J1 | L55M | G | 363 | T |
| METRNL | A178V | C | 658 | T | MYH10 | I588M | T | 1903 | C | NHSL1 | S720T | C | 2159 | G | OR13J1 | R122H | C | 565 | T |
| METT10D | R397* | G | 1337 | A | MYH10 | A1896V | G | 5826 | A | NHSL1 | A1553T | C | 4657 | T | OR14A16 | K305Q | T | 913 | G |
| METT11D1 | E367* | G | 1122 | T | MYH10 | Y857C | T | 2709 | C | NHSL2 | E792G | A | 2441 | G | OR14C36 | S237Y | C | 710 | A |
| METT5D1 | A220V | C | 1011 | T | MYH10 | R1696C | G | 5225 | A | NID1 | T512A | T | 1617 | C | OR14C36 | A123S | G | 367 | T |
| METTL1 | - | A | 0 | G | MYH11 | R778Q | C | 2440 | T | NID1 | V937M | C | 2892 | T | OR14I1 | V166I | C | 496 | T |
| METTL10 | K74T | T | 226 | G | MYH11 | R1511W | G | 4638 | A | NID1 | K437N | C | 1394 | A | OR14K1 | D269Y | G | 805 | T |
| METTL11B | E214* | G | 701 | T | MYH11 | R1138W | G | 3519 | A | NID1 | Y1079C | T | 3319 | C | OR14K1 | L27W | T | 80 | G |
| METTL12 | R205Q | G | 873 | A | MYH11 | Q949* | G | 2952 | A | NID2 | E489* | C | 1465 | A | OR14K1 | E190* | G | 568 | T |

| | | | | | | | | | | | | | | | | | | | |
|---|---|---|---|---|---|---|---|---|---|---|---|---|---|---|---|---|---|---|---|
| METTL12 | P49L | C | 405 | T | MYH11 | A1568V | G | 4810 | A | NID2 | P1103H | G | 3308 | T | OR1A1 | R300W | C | 898 | T |
| METTL13 | R641Q | G | 2188 | A | MYH11 | L1654I | G | 5067 | T | NID2 | A1089T | C | 3265 | T | OR1A1 | R293W | C | 877 | T |
| METTL13 | S218P | T | 918 | C | MYH11 | - | C | 0 | A | NID2 | K1297T | T | 3890 | G | OR1A1 | R54H | G | 161 | A |
| METTL14 | E325* | G | 1140 | T | MYH11 | K1711T | T | 5239 | G | NID2 | R1357Q | C | 4070 | T | OR1C1 | Y132H | A | 394 | G |
| METTL14 | R408Q | G | 1390 | A | MYH11 | A831T | C | 2598 | T | NID2 | S370Y | G | 1109 | T | OR1C1 | S171P | A | 511 | G |
| METTL14 | S399L | C | 1363 | T | MYH11 | D795N | C | 2490 | T | NIF3L1 | C213Y | G | 729 | A | OR1D2 | C189S | A | 565 | T |
| METTL3 | S193L | G | 670 | A | MYH11 | K640N | C | 2027 | A | NIN | C807G | A | 2610 | C | OR1D2 | R122H | C | 365 | T |
| METTL4 | V283A | A | 1647 | G | MYH13 | R707Q | C | 2210 | T | NIN | T662M | G | 2176 | A | OR1D2 | T281I | G | 842 | A |
| METTL4 | K316T | T | 1746 | G | MYH13 | K1000N | C | 3090 | A | NIN | P1987L | G | 6151 | A | OR1E1 | K270N | C | 810 | A |
| METTL5 | F220L | A | 794 | G | MYH13 | R1680H | C | 5129 | T | NIN | R413W | G | 1428 | A | OR1F1 | C189Y | G | 566 | A |
| METTL6 | L267I | G | 1040 | T | MYH13 | R1648H | C | 5033 | T | NIN | N1434T | T | 4492 | G | OR1G1 | F181V | A | 541 | C |
| METTL6 | - | C | 0 | A | MYH13 | V651M | C | 2041 | T | NIN | P1375S | G | 4314 | A | OR1I1 | F238L | C | 800 | A |
| METTL6 | S3Y | G | 249 | T | MYH13 | T1410M | G | 4319 | A | NINL | L262V | G | 858 | C | OR1I1 | A257V | C | 856 | T |
| METTL8 | R72* | G | 430 | A | MYH13 | R1400M | C | 4289 | A | NINL | R1366C | G | 4170 | A | OR1I1 | A349S | G | 1131 | T |
| MEX3B | T145M | G | 870 | A | MYH13 | E435K | C | 1393 | T | NINL | A796T | C | 2460 | T | OR1I1 | A43V | C | 214 | T |
| MEX3B | G20S | C | 494 | T | MYH13 | A1781T | C | 5431 | T | NINL | F527C | A | 1654 | C | OR1I1 | S156Y | C | 553 | A |
| MEX3B | S48N | C | 579 | T | MYH13 | A375V | G | 1214 | A | NIP7 | S149Y | C | 836 | A | OR1J2 | C127Y | G | 394 | A |
| MEX3B | E219D | C | 1093 | A | MYH13 | R109H | C | 416 | T | NIPA2 | R5H | C | 627 | T | OR1K1 | R166H | G | 529 | A |
| MEX3B | D251N | C | 1187 | T | MYH13 | E1820D | C | 5550 | A | NIPAL3 | A199T | G | 963 | A | OR1K1 | S39R | C | 149 | A |
| MEX3C | P312H | G | 935 | T | MYH13 | E1227K | C | 3769 | T | NIPAL4 | T366M | C | 1213 | T | OR1K1 | F179L | C | 569 | A |
| MEX3C | D111E | G | 333 | C | MYH13 | E1114* | C | 3430 | A | NIPAL4 | P324L | C | 1087 | T | OR1L1 | R100H | G | 299 | A |
| MFAP3 | Q361R | A | 1301 | G | MYH13 | E962* | C | 2974 | A | NIPAL4 | V136I | G | 522 | A | OR1L1 | Y85C | A | 254 | G |
| MFF | R48S | G | 585 | T | MYH14 | R410H | G | 1276 | A | NIPAL4 | K312N | G | 1052 | T | OR1L3 | F130S | T | 483 | C |
| MFF | A261T | G | 1222 | A | MYH14 | R1920Q | G | 5806 | A | NIPBL | L471F | G | 1912 | T | OR1L4 | L131V | T | 391 | G |
| MFF | E216G | A | 1088 | G | MYH14 | E1068K | G | 3249 | A | NIPBL | R1682* | C | 5543 | T | OR1L4 | T3A | A | 7 | G |
| MFGE8 | F171L | A | 646 | G | MYH14 | R1958H | G | 5920 | A | NIPBL | R308* | C | 1421 | T | OR1L8 | V127I | C | 379 | T |
| MFGE8 | W255R | A | 898 | G | MYH14 | R1453C | C | 4404 | T | NIPBL | S604T | T | 2309 | A | OR1M1 | G260D | G | 836 | A |
| MFHAS1 | D939N | C | 3378 | T | MYH14 | R98H | G | 340 | A | NIPBL | S1739N | G | 5715 | A | OR1M1 | H175N | C | 580 | A |
| MFHAS1 | L308H | A | 1486 | T | MYH14 | R1436W | C | 4353 | T | NIPBL | R1894C | C | 6179 | T | OR1M1 | Y38C | A | 170 | G |
| MFHAS1 | G711S | C | 2694 | T | MYH14 | A1756T | G | 5313 | A | NIPSNAP1 | R247* | G | 994 | A | OR1M1 | R223H | G | 725 | A |
| MFHAS1 | A771T | C | 2874 | T | MYH14 | R54C | C | 207 | T | NIPSNAP1 | P3L | G | 263 | A | OR1S1 | N13T | A | 38 | C |
| MFHAS1 | N329D | T | 1548 | C | MYH14 | A319T | G | 1002 | A | NIPSNAP3 A | R224W | C | 775 | T | OR1S1 | I322M | T | 966 | G |
| MFI2 | - | C | 0 | T | MYH14 | R1325W | C | 4020 | T | NIPSNAP3 B | R224W | C | 741 | T | OR1S1 | G216A | G | 647 | C |
| MFI2 | G584S | C | 1864 | T | MYH15 | E1508D | C | 4581 | A | NIPSNAP3 B | S113Y | C | 409 | A | OR1S2 | I286T | A | 857 | G |
| MFI2 | Q411K | G | 1345 | T | MYH15 | Y734C | T | 2258 | C | NISCH | R1260W | C | 3850 | T | OR2A14 | A260T | G | 833 | A |
| MFN1 | E450D | G | 1476 | T | MYH15 | R1688W | G | 5119 | A | NISCH | T1256M | C | 3839 | T | OR2A2 | A200T | G | 667 | A |
| MFN1 | H262Y | C | 910 | T | MYH15 | R1356* | G | 4123 | A | NISCH | T913M | C | 2810 | T | OR2A25 | A71T | G | 249 | A |

| | | | | | | | | | | | | | | | | | | |
|---|---|---|---|---|---|---|---|---|---|---|---|---|---|---|---|---|---|---|
| MFN2 | V78I | G | 540 | A | MYH15 | A1440S | C | 4375 | A | NISCH | R963Q | G | 2960 | A | OR2A25 | L39S | T | 154 | C |
| MFN2 | R468C | C | 1710 | T | MYH15 | F585V | A | 1810 | C | NISCH | K65N | G | 267 | T | OR2A25 | Q234H | G | 740 | T |
| MFNG | R4W | G | 234 | A | MYH15 | K582T | T | 1802 | G | NISCH | F773S | T | 2390 | C | OR2AE1 | F28L | G | 108 | T |
| MFNG | H177N | G | 753 | T | MYH15 | F353C | A | 1115 | C | NKAIN3 | I26M | A | 78 | G | OR2AE1 | K80Q | T | 262 | G |
| MFNG | A162T | C | 708 | T | MYH2 | A546T | C | 1764 | T | NKAIN4 | R110C | G | 416 | A | OR2AK2 | Q115P | A | 443 | C |
| MFRP | S332L | G | 1142 | A | MYH2 | R18Q | C | 181 | T | NKAIN4 | R110C | G | 416 | A | OR2AK2 | A315T | G | 1042 | A |
| MFRP | C542Y | C | 1772 | T | MYH2 | D1714N | C | 5268 | T | NKAPL | K395N | G | 1237 | T | OR2AK2 | V118A | T | 452 | C |
| MFRP | C34* | G | 249 | T | MYH2 | A1764V | G | 5419 | A | NKAPL | R317Q | G | 1002 | A | OR2AT4 | F33L | A | 97 | G |
| MFRP | - | C | 0 | T | MYH2 | K1581R | T | 4870 | C | NKD1 | R84W | C | 474 | T | OR2B11 | R126C | G | 376 | A |
| MFRP | V236I | C | 853 | T | MYH2 | R445H | C | 1462 | T | NKD2 | R232Q | G | 924 | A | OR2B11 | Q104* | G | 310 | A |
| MFRP | G145* | C | 580 | A | MYH2 | - | C | 0 | T | NKD2 | P377T | C | 1358 | A | OR2B11 | G90R | C | 268 | G |
| MFSD1 | A72V | C | 356 | T | MYH2 | L1811P | A | 5560 | G | NKG7 | L165V | A | 664 | C | OR2C1 | R235* | C | 755 | T |
| MFSD2A | R199W | C | 738 | T | MYH2 | R445C | G | 1461 | A | NKIRAS1 | E67G | T | 521 | C | OR2C3 | F274L | G | 1184 | T |
| MFSD2A | L138M | C | 555 | A | MYH2 | D1456N | C | 4494 | T | NKPD1 | P165S | G | 493 | A | OR2D2 | R122H | C | 365 | T |
| MFSD2A | E33* | G | 240 | T | MYH2 | E1320K | C | 4086 | T | NKPD1 | G716D | C | 2147 | T | OR2D2 | V140A | A | 419 | G |
| MFSD2B | G379A | G | 1136 | C | MYH2 | D1236Y | C | 3834 | A | NKPD1 | L659I | G | 1975 | T | OR2D2 | R122C | G | 364 | A |
| MFSD5 | L348I | C | 1233 | A | MYH2 | D1183Y | C | 3675 | A | NKPD1 | R211H | C | 632 | T | OR2D3 | V119A | T | 384 | C |
| MFSD5 | A237T | G | 900 | A | MYH2 | E659D | C | 2105 | A | NKRF | K222Q | T | 1317 | G | OR2F1 | I92N | T | 312 | A |
| MFSD6 | V385I | G | 1477 | A | MYH2 | K640N | C | 2048 | A | NKTR | N670H | A | 2196 | C | OR2G3 | L157P | T | 502 | C |
| MFSD6 | A548T | G | 1966 | A | MYH2 | M442I | C | 1454 | T | NKX2-1 | P78L | G | 315 | A | OR2G6 | T4A | A | 42 | G |
| MFSD6L | A70T | C | 356 | T | MYH2 | F315I | A | 1071 | T | NKX2-1 | A345T | C | 1115 | T | OR2L13 | D120N | G | 695 | A |
| MFSD6L | R543Q | C | 1776 | T | MYH2 | N81Y | T | 369 | A | NKX2-3 | L70S | T | 388 | C | OR2L13 | L156S | T | 804 | C |
| MFSD6L | S540T | A | 1766 | T | MYH2 | E986K | C | 3084 | T | NKX2-3 | R362L | G | 1264 | T | OR2L13 | F176L | C | 865 | A |
| MFSD6L | A120T | C | 506 | T | MYH3 | N1165S | T | 3561 | C | NKX2-4 | T293M | G | 888 | A | OR2L2 | F199L | T | 691 | C |
| MFSD6L | G114S | C | 488 | T | MYH3 | E1688D | C | 5131 | G | NKX2-4 | E203K | C | 617 | T | OR2L3 | R306K | G | 937 | A |
| MFSD6L | S192L | G | 723 | A | MYH3 | S1746N | C | 5304 | T | NKX2-5 | A302V | G | 1081 | A | OR2L3 | V247L | G | 759 | T |
| MFSD7 | A163T | C | 503 | T | MYH3 | L1011I | G | 3098 | T | NKX2-5 | R156C | G | 642 | A | OR2L5 | R138I | G | 413 | T |
| MFSD7 | A535T | C | 1619 | T | MYH3 | K1860N | C | 5647 | A | NKX2-5 | I184S | A | 727 | C | OR2L8 | Q6H | A | 99 | C |
| MFSD7 | V328A | A | 999 | G | MYH3 | E1836* | C | 5573 | A | NKX2-5 | K110N | C | 506 | A | OR2L8 | R21I | G | 143 | T |
| MFSD8 | - | C | 0 | G | MYH3 | K1588R | T | 4830 | C | NKX2-6 | G111S | C | 331 | T | OR2M2 | V78A | T | 233 | C |
| MFSD8 | M454T | A | 1489 | G | MYH3 | D1085Y | C | 3320 | A | NKX2-6 | A199V | G | 596 | A | OR2M2 | I199M | T | 597 | G |
| MFSD8 | F53L | A | 285 | G | MYH4 | E1585* | C | 4864 | A | NKX3-1 | R176C | G | 574 | A | OR2M3 | L73F | C | 255 | T |
| MFSD9 | R235Q | C | 748 | T | MYH4 | W511* | C | 1643 | T | NKX3-1 | R126C | G | 424 | A | OR2M4 | T267M | C | 800 | T |
| MGA | R2445* | C | 7514 | T | MYH4 | Y837H | A | 2620 | G | NKX3-1 | H156Y | G | 514 | A | OR2M4 | L68R | T | 203 | G |
| MGA | G1447D | G | 4521 | A | MYH4 | A572T | C | 1825 | T | NKX3-2 | R287C | G | 935 | A | OR2M4 | A254V | C | 761 | T |
| MGA | A1436T | G | 4487 | A | MYH4 | R1617M | C | 4961 | A | NKX6-1 | G351S | C | 1901 | T | OR2M5 | F257L | C | 771 | A |
| MGA | R1867* | C | 5780 | T | MYH4 | K1000T | T | 3110 | G | NKX6-1 | T340M | G | 1869 | A | OR2M7 | V281I | C | 841 | T |
| MGA | N2122K | T | 6547 | G | MYH4 | V828A | A | 2594 | G | NKX6-1 | E309G | T | 1776 | C | OR2M7 | C202* | G | 606 | T |
| MGA | I2251L | A | 6932 | C | MYH4 | R1753H | C | 5369 | T | NLE1 | R229W | G | 713 | A | OR2S2 | V38M | C | 168 | T |

| | | | | | | | | | | | | | | | | | | | |
|---|---|---|---|---|---|---|---|---|---|---|---|---|---|---|---|---|---|---|---|
| MGA | G2339V | G | 7197 | T | MYH4 | K1077T | T | 3341 | G | NLGN1 | R93C | C | 706 | T | OR2T10 | A163T | C | 487 | T |
| MGA | R866* | C | 2777 | T | MYH4 | E525K | C | 1684 | T | NLGN1 | E743D | A | 2658 | C | OR2T11 | Y215H | A | 643 | G |
| MGAM | L1794V | T | 5434 | G | MYH4 | R1348W | G | 4153 | A | NLGN2 | G95S | G | 356 | A | OR2T12 | R227H | C | 680 | T |
| MGAM | L1666V | C | 5050 | G | MYH6 | R714C | G | 2194 | A | NLGN3 | L721I | C | 2464 | A | OR2T12 | E194K | C | 580 | T |
| MGAM | R369W | C | 1159 | T | MYH6 | A1819V | G | 5510 | A | NLGN4X | R730C | G | 2652 | A | OR2T4 | M304I | G | 912 | A |
| MGAM | N1345T | A | 4088 | C | MYH6 | A1704V | G | 5165 | A | NLGN4X | K722T | T | 2629 | G | OR2T4 | V108F | G | 322 | T |
| MGAM | R1570C | C | 4762 | T | MYH6 | A212V | G | 689 | A | NLGN4X | D181Y | C | 1005 | A | OR2T6 | R139Q | G | 416 | A |
| MGAM | F436C | T | 1361 | G | MYH6 | Q1706E | G | 5170 | C | NLGN4X | E748D | C | 2708 | A | OR2T6 | M184T | T | 551 | C |
| MGAM | L905I | C | 2767 | A | MYH6 | K1900N | C | 5754 | A | NLK | G19S | G | 773 | A | OR2W3 | E196K | G | 617 | A |
| MGAM | K2239M | A | 6770 | T | MYH6 | M824V | T | 2524 | C | NLK | P384S | C | 1868 | T | OR2W3 | R223M | G | 699 | T |
| MGAT1 | R427H | C | 1953 | T | MYH6 | R34C | G | 154 | A | NLN | K159N | A | 655 | C | OR2W3 | R122Q | G | 396 | A |
| MGAT2 | W235* | G | 1202 | A | MYH7 | R869C | G | 2683 | A | NLN | K343R | A | 1206 | G | OR2W3 | L37P | T | 141 | C |
| MGAT2 | W362R | T | 1582 | C | MYH7 | R1114H | C | 3419 | T | NLN | A3S | G | 185 | T | OR2W3 | R165H | G | 525 | A |
| MGAT2 | W348* | G | 1541 | A | MYH7 | A100T | C | 376 | T | NLRC3 | R562C | G | 1684 | A | OR2Z1 | T77I | C | 305 | T |
| MGAT3 | Q385R | A | 1369 | G | MYH7 | A13T | C | 115 | T | NLRC3 | A210T | C | 628 | T | OR3A1 | G217C | C | 649 | A |
| MGAT3 | P55L | C | 379 | T | MYH7 | Q1704R | T | 5189 | C | NLRC3 | I341M | G | 1023 | C | OR3A2 | I265V | T | 832 | C |
| MGAT3 | V109M | G | 540 | A | MYH7 | R1689C | G | 5143 | A | NLRC3 | A170T | C | 508 | T | OR3A2 | Y268D | A | 841 | C |
| MGAT4A | Y24D | A | 384 | C | MYH7 | V125I | C | 451 | T | NLRC3 | L913M | G | 2737 | T | OR3A3 | R318* | C | 952 | T |
| MGAT4A | H111P | T | 646 | G | MYH7 | D382N | C | 1222 | T | NLRC4 | I227T | A | 944 | G | OR4A15 | L244I | C | 730 | A |
| MGAT4B | E556K | C | 2430 | T | MYH7 | R1053Q | C | 3236 | T | NLRC4 | Y585* | A | 2019 | C | OR4A15 | R167* | C | 499 | T |
| MGAT4C | R178H | C | 533 | T | MYH7B | R1534W | C | 4692 | T | NLRC4 | R181Q | C | 806 | T | OR4A15 | S252Y | C | 755 | A |
| MGAT4C | E463G | T | 1388 | C | MYH7B | R1840C | C | 5610 | T | NLRC5 | T1578A | A | 4957 | G | OR4A16 | S50I | G | 199 | T |
| MGAT4C | I85L | T | 253 | A | MYH7B | V1262A | T | 3877 | C | NLRC5 | L526P | T | 1802 | C | OR4A16 | A74V | C | 271 | T |
| MGAT5 | A74T | G | 472 | A | MYH7B | D45N | G | 225 | A | NLRC5 | P664H | C | 2216 | A | OR4A16 | F249V | T | 795 | G |
| MGAT5B | W692C | G | 2179 | C | MYH7B | A202T | G | 696 | A | NLRC5 | A186T | G | 781 | A | OR4A47 | V244L | G | 730 | T |
| MGAT5B | R79C | C | 338 | T | MYH8 | Q805H | C | 2488 | A | NLRC5 | R771Q | G | 2537 | A | OR4A47 | K224N | G | 672 | T |
| MGEA5 | R914W | G | 3136 | A | MYH8 | K637N | C | 1984 | A | NLRC5 | G1703D | G | 5333 | A | OR4A5 | A185T | C | 553 | T |
| MGEA5 | E734* | C | 2596 | A | MYH8 | E348D | C | 1117 | A | NLRC5 | G1030W | G | 3313 | T | OR4A5 | R120H | C | 359 | T |
| MGLL | A233T | C | 1237 | T | MYH8 | K408M | T | 1296 | A | NLRC5 | R386W | C | 1381 | T | OR4A5 | L141I | G | 421 | T |
| MGLL | V107I | C | 859 | T | MYH8 | R1563H | C | 4761 | T | NLRC5 | S1507R | A | 4744 | C | OR4A5 | S162I | C | 485 | A |
| MGMT | R159* | C | 477 | T | MYH8 | R1478H | C | 4506 | T | NLRP10 | C227* | G | 843 | T | OR4B1 | T36M | C | 107 | T |
| MGRN1 | R330Q | G | 1125 | A | MYH8 | G1666D | C | 5070 | T | NLRP10 | P141S | G | 583 | A | OR4B1 | L210I | C | 628 | A |
| MGST3 | R13H | G | 101 | A | MYH8 | V1410A | A | 4302 | G | NLRP10 | C357F | C | 1232 | A | OR4B1 | D266Y | G | 796 | T |
| MGST3 | R13H | G | 101 | A | MYH8 | D1624Y | C | 4943 | A | NLRP10 | D524Y | C | 1732 | A | OR4C11 | S50T | C | 149 | G |
| MIA | L14M | C | 188 | A | MYH8 | L1300S | A | 3972 | G | NLRP11 | A188D | G | 1274 | T | OR4C13 | V27F | G | 111 | T |
| MIA | R3L | G | 156 | T | MYH8 | K368N | C | 1177 | A | NLRP11 | T956A | T | 3577 | C | OR4C15 | C260Y | G | 779 | A |
| MIA2 | R252W | C | 953 | T | MYH8 | D299Y | C | 968 | A | NLRP11 | V55M | C | 874 | T | OR4C15 | P337T | C | 1009 | A |
| MIA2 | E65D | G | 394 | T | MYH8 | Q216H | C | 721 | A | NLRP11 | Q490K | G | 2179 | T | OR4C15 | P337H | C | 1010 | A |
| MIA3 | M1320I | G | 3985 | T | MYH9 | D539N | C | 1846 | T | NLRP12 | R206H | C | 837 | T | OR4C15 | F221V | T | 661 | G |

| | | | | | | | | | | | | | | | | | | | |
|---|---|---|---|---|---|---|---|---|---|---|---|---|---|---|---|---|---|---|---|
| MIA3 | A2D | C | 30 | A | MYH9 | E1884G | T | 5882 | C | NLRP12 | R656C | G | 2186 | A | OR4C16 | I305V | A | 913 | G |
| MIA3 | L328M | T | 1007 | A | MYH9 | Y650C | T | 2180 | C | NLRP12 | R656H | C | 2187 | T | OR4C6 | L30R | T | 118 | G |
| MIA3 | G546R | G | 1661 | A | MYH9 | T1504A | T | 4741 | C | NLRP12 | R352H | C | 1275 | T | OR4D1 | N42D | A | 124 | G |
| MIA3 | K269Q | A | 830 | C | MYH9 | T638M | G | 2144 | A | NLRP12 | A235V | G | 924 | A | OR4D11 | R64H | G | 191 | A |
| MIB1 | T129A | A | 649 | G | MYH9 | A363V | G | 1319 | A | NLRP12 | L630M | G | 2108 | T | OR4D11 | N202I | A | 605 | T |
| MIB1 | T923A | A | 3031 | G | MYL1 | F47L | G | 289 | T | NLRP12 | A218T | C | 872 | T | OR4D5 | S67Y | C | 274 | A |
| MIB2 | V67M | G | 326 | A | MYL12B | R104I | G | 655 | T | NLRP13 | F588L | G | 1789 | T | OR4D5 | L214R | T | 715 | G |
| MIB2 | A811T | G | 2558 | A | MYL2 | G162R | C | 555 | T | NLRP13 | E622* | C | 1889 | A | OR4D6 | A43V | C | 151 | T |
| MIB2 | M45I | G | 262 | A | MYL4 | V158I | G | 600 | A | NLRP13 | F460V | A | 1403 | C | OR4D9 | L33V | T | 97 | G |
| MICAL1 | R910H | C | 3041 | T | MYL5 | K171N | G | 618 | T | NLRP14 | I726T | T | 2500 | C | OR4D9 | L85I | C | 253 | A |
| MICAL1 | P626T | G | 2188 | T | MYL7 | R70C | G | 208 | A | NLRP14 | C563R | T | 2010 | C | OR4D9 | V173A | T | 518 | C |
| MICAL2 | D285Y | G | 1141 | T | MYLIP | S53R | A | 355 | C | NLRP14 | E1084* | G | 3573 | T | OR4F15 | S207Y | C | 644 | A |
| MICALCL | R66W | C | 487 | T | MYLK | A350T | C | 1330 | T | NLRP2 | R752* | C | 2317 | T | OR4F15 | Q24H | G | 96 | C |
| MICALCL | S97N | G | 581 | A | MYLK | A1300T | C | 4180 | T | NLRP2 | R467Q | G | 1463 | A | OR4F15 | F47L | C | 165 | A |
| MICALCL | K523N | G | 1860 | T | MYLK | K1376T | T | 4409 | G | NLRP2 | R467* | C | 1462 | T | OR4F15 | S229Y | C | 710 | A |
| MICALL1 | H187R | A | 686 | G | MYLK | K936N | C | 3090 | A | NLRP2 | K45E | A | 196 | G | OR4K1 | H105Q | C | 374 | A |
| MICALL1 | S116L | C | 473 | T | MYLK | E917D | C | 3033 | A | NLRP2 | T220P | A | 721 | C | OR4K1 | V153I | G | 516 | A |
| MICALL1 | P468H | C | 1529 | A | MYLK | K608T | T | 2105 | G | NLRP2 | C914* | C | 2805 | A | OR4K1 | R89C | C | 324 | T |
| MICALL1 | - | T | 0 | C | MYLK | D522Y | C | 1846 | A | NLRP3 | A809T | G | 2563 | A | OR4K1 | N202S | A | 664 | G |
| MICALL2 | G379R | C | 1311 | T | MYLK | A292T | C | 1156 | T | NLRP3 | R178W | C | 670 | T | OR4K14 | Q24H | T | 72 | G |
| MID1 | H178Q | A | 810 | T | MYLK2 | G75D | G | 497 | A | NLRP3 | G895D | G | 2822 | A | OR4K14 | V258F | C | 772 | A |
| MID1 | L8P | A | 299 | G | MYLK2 | G241E | G | 995 | A | NLRP3 | P352L | C | 1193 | T | OR4K14 | K81N | C | 243 | A |
| MID1 | T481A | T | 1717 | C | MYLK3 | A109T | C | 441 | T | NLRP3 | S752Y | C | 2393 | A | OR4K14 | N25T | T | 74 | G |
| MID1 | H178Q | A | 810 | T | MYLK3 | A72V | G | 331 | A | NLRP4 | Q258* | C | 1194 | T | OR4K15 | R261H | G | 857 | A |
| MIDN | R381Q | G | 1657 | A | MYLK3 | V592M | C | 1890 | T | NLRP4 | I219F | A | 1077 | T | OR4K2 | D180E | C | 576 | G |
| MIER1 | F280L | T | 910 | G | MYLK3 | I563T | A | 1804 | G | NLRP4 | G442R | G | 1746 | A | OR4K2 | L195M | C | 619 | A |
| MIER1 | V368I | G | 1172 | A | MYLK3 | F132L | A | 510 | G | NLRP4 | E234K | G | 1122 | A | OR4K2 | F250L | C | 786 | A |
| MIER1 | R202C | C | 674 | T | MYLK4 | E346D | C | 1335 | A | NLRP4 | G823* | G | 2889 | T | OR4K2 | F103C | T | 344 | G |
| MIER2 | A503D | G | 1517 | T | MYLK4 | D144G | T | 728 | C | NLRP5 | E184K | G | 550 | A | OR4K5 | R319G | A | 980 | G |
| MIER2 | G415R | C | 1252 | T | MYO10 | T1214A | T | 4108 | C | NLRP5 | R392H | G | 1175 | A | OR4K5 | L214F | G | 667 | T |
| MIER2 | P234S | G | 709 | A | MYO10 | A756V | G | 2735 | A | NLRP5 | G286R | G | 856 | A | OR4K5 | L62I | C | 209 | A |
| MIER2 | A523T | C | 1576 | T | MYO10 | A986T | C | 3424 | T | NLRP5 | Q827R | A | 2480 | G | OR4K5 | L115R | T | 369 | G |
| MIER3 | R348G | T | 1058 | C | MYO10 | D1101N | C | 3769 | T | NLRP5 | C774* | C | 2322 | A | OR4L1 | T192K | C | 575 | A |
| MIER3 | Q435H | T | 1321 | G | MYO10 | A1152V | G | 3923 | A | NLRP5 | E187* | G | 559 | T | OR4L1 | A145V | C | 434 | T |
| MIF4GD | S194A | A | 660 | C | MYO10 | A210V | G | 1097 | A | NLRP5 | K1092N | G | 3276 | T | OR4L1 | N291I | A | 872 | T |
| MINA | R287W | G | 1442 | A | MYO10 | R1545* | G | 5101 | A | NLRP5 | A1052T | G | 3154 | A | OR4M2 | C141* | C | 521 | A |
| MINK1 | R521* | C | 1757 | T | MYO10 | F632L | G | 2364 | T | NLRP6 | R399H | G | 1196 | A | OR4M2 | S50G | A | 246 | G |
| MINK1 | V871I | G | 2807 | A | MYO10 | R1166K | C | 3965 | T | NLRP6 | A771V | C | 2312 | T | OR4N2 | R260C | C | 778 | T |
| MINPP1 | A310V | C | 970 | T | MYO15A | A1685T | G | 5391 | A | NLRP6 | A344T | G | 1030 | A | OR4N2 | R80W | C | 238 | T |

| | | | | | | | | | | | | | | | | | | | |
|---|---|---|---|---|---|---|---|---|---|---|---|---|---|---|---|---|---|---|---|
| MIOS | L486* | T | 1878 | G | MYO15A | R2588W | C | 8100 | T | NLRP6 | A25V | C | 74 | T | OR4N2 | F279C | T | 836 | G |
| MIP | F189I | A | 597 | T | MYO15A | R3247H | G | 1007 8 | A | NLRP6 | E605D | G | 1815 | T | OR4N4 | R290H | G | 960 | A |
| MIPEP | P519H | G | 1655 | T | MYO15A | S2841N | G | 8860 | A | NLRP6 | E167D | A | 501 | C | OR4N4 | R290H | G | 960 | A |
| MIPEP | Y370H | A | 1207 | G | MYO15A | E1698G | A | 5431 | G | NLRP7 | E556D | C | 2071 | A | OR4N4 | R290H | G | 960 | A |
| MIPEP | W356* | C | 1167 | T | MYO15A | F174L | C | 860 | A | NLRP7 | V616L | C | 2249 | G | OR4P4 | L195F | C | 583 | T |
| MIPEP | R340* | G | 1117 | A | MYO15A | A1300T | G | 4236 | A | NLRP7 | L876M | G | 3029 | T | OR4Q3 | P168S | C | 502 | T |
| MIPOL1 | R279W | C | 1301 | T | MYO15A | A2884V | C | 8989 | T | NLRP7 | D725G | T | 2577 | C | OR4Q3 | K270T | A | 809 | C |
| MIR1279 | S467L | C | 1447 | T | MYO15A | R2071M | G | 6550 | T | NLRP7 | F166S | A | 900 | G | OR4S1 | G239D | G | 716 | A |
| MIR1279 | S539* | C | 1663 | A | MYO15A | P817S | C | 2787 | T | NLRP7 | A98V | G | 696 | A | OR4S1 | R120C | C | 358 | T |
| MIR198 | L121F | G | 718 | A | MYO15A | A441V | C | 1660 | T | NLRP7 | M841I | C | 2926 | A | OR4X1 | G14R | G | 40 | A |
| MIR198 | I202N | A | 962 | T | MYO15A | R3374H | G | 1045 9 | A | NLRP8 | R540C | C | 1689 | T | OR51A4 | R166* | T | 496 | A |
| MIR2277 | T320A | T | 1101 | C | MYO15A | L137F | C | 747 | T | NLRP8 | R824I | G | 2542 | T | OR51A7 | S152N | G | 492 | A |
| MIS12 | R61H | G | 735 | A | MYO15A | R1120C | C | 3696 | T | NLRP8 | E1012* | G | 3105 | T | OR51B2 | G225D | C | 729 | T |
| MITD1 | T155A | T | 540 | C | MYO15A | F3414L | T | 1057 8 | C | NLRP9 | A943V | G | 2856 | A | OR51B6 | A188D | C | 563 | A |
| MITF | R60C | C | 305 | T | MYO15A | G1104D | G | 3649 | A | NLRX1 | T221M | C | 877 | T | OR51B6 | A115S | G | 343 | T |
| MITF | P256S | C | 893 | T | MYO15A | A545V | C | 1972 | T | NMBR | R361H | C | 1223 | T | OR51B6 | R168* | C | 502 | T |
| MITF | E373* | G | 1244 | T | MYO16 | E821* | G | 2587 | T | NMBR | R103H | C | 449 | T | OR51B6 | F214L | C | 642 | A |
| MKI67 | T1775M | G | 5700 | A | MYO16 | A1658T | G | 5098 | A | NMBR | G273C | C | 958 | A | OR51D1 | R67* | C | 275 | T |
| MKI67 | A1778V | G | 5709 | A | MYO16 | R747C | C | 2365 | T | NMD3 | R276* | C | 948 | T | OR51D1 | Q183H | A | 625 | C |
| MKI67 | E247* | C | 1115 | A | MYO16 | A1317T | G | 4075 | A | NMD3 | W68R | T | 324 | C | OR51E2 | S88F | G | 503 | A |
| MKI67 | R2727C | G | 8555 | A | MYO16 | P1487L | C | 4586 | T | NMD3 | E210K | G | 750 | A | OR51E2 | R272C | G | 1054 | A |
| MKI67 | F1068L | A | 3580 | C | MYO16 | D1241N | G | 3847 | A | NME1-NME2 | M141T | T | 598 | C | OR51E2 | T7P | T | 259 | G |
| MKI67 | V729A | A | 2562 | G | MYO16 | T268A | A | 928 | G | NME1-NME2 | A286S | G | 1032 | T | OR51E2 | F154L | A | 702 | C |
| MKI67 | F489V | A | 1841 | C | MYO16 | M303R | T | 1034 | G | NME6 | - | C | 0 | A | OR51F1 | M68V | T | 202 | C |
| MKI67IP | R288* | G | 935 | A | MYO16 | - | G | 0 | A | NME7 | Q81H | T | 500 | A | OR51F2 | M186T | T | 622 | C |
| MKI67IP | H282R | T | 918 | C | MYO16 | F946L | C | 2964 | A | NMNAT1 | E249* | G | 889 | T | OR51F2 | A156V | C | 532 | T |
| MKKS | Q391H | C | 1173 | G | MYO18A | R960Q | C | 2879 | T | NMNAT2 | D294N | C | 1215 | T | OR51G1 | T79A | T | 235 | C |
| MKKS | S504N | C | 1511 | T | MYO18A | R237H | C | 710 | T | NMNAT2 | S244F | G | 1066 | A | OR51G1 | F64L | G | 192 | T |
| MKL2 | G611C | G | 1831 | T | MYO18A | R1875Q | C | 5624 | T | NMNAT3 | E248D | C | 1126 | A | OR51G2 | L225M | G | 673 | T |
| MKL2 | P1084L | C | 3251 | T | MYO18A | R1747W | G | 5239 | A | NMNAT3 | H152R | T | 837 | C | OR51G2 | T82A | T | 244 | C |
| MKL2 | R873C | C | 2617 | T | MYO18A | R1059H | C | 3176 | T | NMRAL1 | R233C | G | 1073 | A | OR51G2 | F256L | A | 766 | G |
| MKLN1 | E451D | G | 1393 | T | MYO18A | K1018N | C | 3054 | A | NMRAL1 | V236M | C | 1082 | T | OR51I1 | L138I | G | 412 | T |
| MKLN1 | A586V | C | 1797 | T | MYO18B | K1480T | A | 4439 | C | NMS | R112* | C | 341 | T | OR51I1 | S121I | C | 362 | A |
| MKLN1 | E593G | A | 1818 | G | MYO18B | M1522V | A | 4564 | G | NMT1 | G392R | G | 1192 | A | OR51I2 | L103I | C | 388 | A |
| MKLN1 | R337W | C | 1049 | T | MYO18B | R2154H | G | 6461 | A | NMT2 | R291Q | C | 956 | T | OR51I2 | R122H | G | 446 | A |
| MKLN1 | K107N | G | 361 | T | MYO18B | A2396V | C | 7187 | T | NMT2 | S289L | G | 950 | A | OR51M1 | I171M | T | 535 | G |

| | | | | | | | | | | | | | | | | | | | |
|---|---|---|---|---|---|---|---|---|---|---|---|---|---|---|---|---|---|---|---|
| MKLN1 | F238L | T | 752 | C | MYO18B | R115H | G | 344 | A | NMUR1 | R175Q | C | 658 | T | OR51M1 | S145L | C | 456 | T |
| MKNK1 | D337G | T | 1174 | C | MYO18B | R1801Q | G | 5402 | A | NMUR1 | M319I | C | 1091 | T | OR51Q1 | G43S | G | 217 | A |
| MKNK2 | E144* | C | 675 | A | MYO18B | G844S | G | 2530 | A | NMUR2 | R260I | C | 945 | A | OR51Q1 | C153Y | G | 548 | A |
| MKNK2 | F149L | A | 690 | G | MYO18B | D2036N | G | 6106 | A | NMUR2 | R108H | C | 489 | T | OR51Q1 | R52C | C | 244 | T |
| MKRN3 | R477Q | G | 1906 | A | MYO18B | P1140H | C | 3419 | A | NMUR2 | R108H | C | 489 | T | OR51T1 | R168M | G | 503 | T |
| MKRN3 | P76Q | C | 703 | A | MYO18B | E209D | G | 627 | T | NNAT | G15D | G | 161 | A | OR51T1 | T253A | A | 757 | G |
| MKRN3 | D483Y | G | 1923 | T | MYO18B | R1024H | G | 3071 | A | NNAT | V21M | G | 178 | A | OR51T1 | S268N | G | 803 | A |
| MKRN3 | D267N | G | 1275 | A | MYO18B | R1720H | G | 5159 | A | NNMT | E80D | G | 984 | C | OR51V1 | F112L | G | 336 | T |
| MKRN9P | R415* | G | 1468 | A | MYO18B | R2385Q | G | 7154 | A | NOB1 | R269H | C | 822 | T | OR52B4 | Q102R | T | 305 | C |
| MKRN9P | E345K | C | 1258 | T | MYO19 | R364Q | C | 2062 | T | NOBOX | R449Q | C | 1346 | T | OR52B4 | R195Q | C | 584 | T |
| MKS1 | G119D | C | 431 | T | MYO19 | E477Q | C | 2400 | G | NOBOX | R303* | G | 907 | A | OR52B4 | L47F | G | 139 | A |
| MKS1 | V299I | C | 970 | T | MYO1A | M575T | A | 1975 | G | NOC2L | A517V | G | 1600 | A | OR52B4 | S30Y | G | 89 | T |
| MKX | R278H | C | 1025 | T | MYO1A | R152H | C | 706 | T | NOC2L | R297I | C | 940 | A | OR52D1 | F65S | T | 216 | C |
| MKX | G69W | C | 397 | A | MYO1A | R639* | G | 2166 | A | NOC3L | A722T | C | 2265 | T | OR52E2 | S55N | C | 164 | T |
| MLEC | K126T | A | 528 | C | MYO1A | - | C | 0 | A | NOD1 | V698I | C | 2618 | T | OR52E2 | N108S | T | 323 | C |
| MLF1 | R131C | C | 529 | T | MYO1A | S501G | T | 1752 | C | NOD2 | K953N | G | 2964 | T | OR52E2 | Y272H | A | 814 | G |
| MLF1IP | S50A | A | 219 | C | MYO1A | R654L | C | 2212 | A | NOD2 | G879R | G | 2740 | A | OR52E2 | A192V | G | 575 | A |
| MLH1 | W714* | G | 2357 | A | MYO1A | R621* | G | 2112 | A | NODAL | T74M | G | 221 | A | OR52E4 | Y219C | A | 678 | G |
| MLH1 | I630S | T | 2105 | G | MYO1B | F77L | T | 478 | G | NOL10 | R208* | G | 728 | A | OR52E4 | R167H | G | 522 | A |
| MLH3 | R637H | C | 2126 | T | MYO1B | R96* | C | 533 | T | NOL10 | L258R | A | 879 | C | OR52E4 | P32L | C | 117 | T |
| MLH3 | D918G | T | 2969 | C | MYO1C | A380T | C | 1352 | T | NOL10 | S548L | G | 1749 | A | OR52E6 | H317N | G | 949 | T |
| MLH3 | K628T | T | 2099 | G | MYO1C | G1040S | C | 3332 | T | NOL11 | E240* | G | 723 | T | OR52E8 | Y67C | T | 200 | C |
| MLKL | R306H | C | 1358 | T | MYO1C | P164H | G | 705 | T | NOL11 | R385* | C | 1158 | T | OR52E8 | H62Y | G | 184 | A |
| MLL | P1314L | C | 3964 | T | MYO1C | A663V | G | 2202 | A | NOL11 | K47N | G | 146 | T | OR52H1 | R178G | T | 532 | C |
| MLL | R2618C | C | 7875 | T | MYO1D | K375E | T | 1135 | C | NOL11 | I143L | A | 432 | C | OR52H1 | R180H | C | 539 | T |
| MLL | R933W | C | 2820 | T | MYO1D | K366N | C | 1110 | A | NOL4 | L568M | G | 2000 | T | OR52H1 | T261I | G | 782 | A |
| MLL | T953I | C | 2881 | T | MYO1D | T921M | G | 2774 | A | NOL4 | R100* | G | 596 | A | OR52J3 | R167H | G | 500 | A |
| MLL | N2275K | T | 6848 | A | MYO1D | R622C | G | 1876 | A | NOL4 | G76A | C | 525 | G | OR52K1 | R268H | G | 825 | A |
| MLL | R933Q | G | 2821 | A | MYO1D | R782G | T | 2356 | C | NOL4 | R344G | G | 1328 | C | OR52K1 | S76Y | C | 249 | A |
| MLL | D3876N | G | 11649 | A | MYO1D | M546V | T | 1648 | C | NOL6 | R694C | G | 2168 | A | OR52K2 | L63I | C | 232 | A |
| MLL | E1361* | G | 4104 | T | MYO1D | V378A | A | 1145 | G | NOL6 | A880V | G | 2727 | A | OR52L1 | L116M | G | 346 | T |
| MLL | R2619C | C | 7878 | T | MYO1D | E246K | C | 748 | T | NOL6 | S547Y | G | 1728 | T | OR52L2P | P36L | G | 107 | A |
| MLL | N3084S | A | 9274 | G | MYO1D | E187* | C | 571 | A | NOL7 | - | G | 0 | A | OR52L2P | P147S | G | 439 | A |
| MLL2 | Y5472C | T | 16415 | C | MYO1E | M95I | C | 656 | T | NOL7 | K254T | A | 793 | C | OR52N1 | S190C | G | 569 | C |
| MLL2 | L4107M | G | 12319 | T | MYO1E | R207Q | C | 991 | T | NOL8 | T517I | G | 1887 | A | OR52N4 | L9I | C | 73 | A |
| MLL2 | Q5170R | T | 15509 | C | MYO1E | H10Q | G | 401 | T | NOL8 | E1145D | C | 3772 | A | OR52N4 | V251I | G | 799 | A |

| | | | | | | | | | | | | | | | | | | |
|---|---|---|---|---|---|---|---|---|---|---|---|---|---|---|---|---|---|---|
| MLL2 | V149I | C | 445 | T | MYO1E | V716I | C | 2517 | T | NOL8 | K762R | T | 2622 | C | OR52N5 | R310C | G | 928 | A |
| MLL2 | R5323C | G | 1596 7 | A | MYO1E | L530R | A | 1960 | C | NOL8 | R1156* | G | 3803 | A | OR52R1 | Q387K | G | 1159 | T |
| MLL2 | Q2514H | C | 7542 | A | MYO1F | R751C | G | 2384 | A | NOL9 | L496P | A | 1520 | G | OR52W1 | I131V | A | 469 | G |
| MLL2 | G1636D | C | 4907 | T | MYO1F | R839Q | C | 2649 | T | NOL9 | A686V | G | 2090 | A | OR56A3 | L103F | C | 307 | T |
| MLL2 | G3189R | C | 9565 | T | MYO1F | A399S | C | 1328 | A | NOLC1 | K470E | A | 1643 | G | OR56A3 | E302* | G | 904 | T |
| MLL2 | C1424W | A | 4272 | C | MYO3A | N727K | T | 2347 | A | NOM1 | A829T | G | 2500 | A | OR56A3 | A184V | C | 551 | T |
| MLL2 | E1738D | C | 5214 | A | MYO3A | T1606A | A | 4982 | G | NOMO2 | M494I | C | 1554 | T | OR56B4 | K272R | A | 910 | G |
| MLL2 | R1258W | G | 3772 | A | MYO3A | E259* | G | 941 | T | NONO | A122V | C | 570 | T | OR56B4 | I141M | A | 518 | G |
| MLL2 | S1398L | G | 4193 | A | MYO3A | N847H | A | 2705 | C | NOP14 | R329H | C | 1052 | T | OR5AC2 | L144F | G | 432 | T |
| MLL5 | R219I | G | 1201 | T | MYO3B | R996C | C | 3129 | T | NOP14 | R537W | G | 1675 | A | OR5AN1 | A41V | C | 169 | T |
| MLL5 | R409W | C | 1770 | T | MYO3B | P902L | C | 2848 | T | NOP14 | R808H | C | 2489 | T | OR5AP2 | A207T | C | 619 | T |
| MLL5 | S638N | G | 2458 | A | MYO3B | F292C | T | 1018 | G | NOP2 | E186K | C | 668 | T | OR5AP2 | R267H | C | 800 | T |
| MLL5 | R227I | G | 1225 | T | MYO3B | A640S | G | 2061 | T | NOP58 | K487N | G | 1687 | T | OR5AR1 | R122H | G | 389 | A |
| MLL5 | D902N | G | 3249 | A | MYO3B | E1022D | G | 3209 | T | NOP58 | K99N | G | 523 | T | OR5B12 | S62G | T | 184 | C |
| MLLT1 | D449N | C | 1509 | T | MYO5A | R984W | G | 3194 | A | NOS1 | K474E | T | 1425 | C | OR5B17 | V295A | A | 884 | G |
| MLLT1 | R204W | G | 774 | A | MYO5A | S693G | T | 2321 | C | NOS1 | A1199V | G | 3601 | A | OR5B2 | L209I | G | 625 | T |
| MLLT10 | R770Q | G | 2487 | A | MYO5A | G1500W | C | 4742 | A | NOS1 | T320S | T | 963 | A | OR5B21 | L302F | G | 904 | A |
| MLLT11 | L13I | C | 2839 | A | MYO5A | H138Y | G | 656 | A | NOS1 | L1167I | G | 3504 | T | OR5B21 | F175L | G | 525 | T |
| MLLT11 | L22M | C | 2866 | A | MYO5A | L1329F | C | 4231 | A | NOS1 | T1184A | T | 3555 | C | OR5B3 | F10L | A | 28 | G |
| MLLT3 | - | C | 0 | T | MYO5A | T654I | G | 2205 | A | NOS1 | E508D | C | 1529 | A | OR5C1 | R265H | G | 856 | A |
| MLLT3 | W32* | C | 382 | T | MYO5A | H424N | G | 1514 | T | NOS1 | K118N | C | 359 | A | OR5C1 | F181L | C | 605 | A |
| MLLT4 | M741I | G | 2223 | A | MYO5B | - | C | 0 | A | NOS1AP | R159W | C | 877 | T | OR5D13 | S192L | C | 575 | T |
| MLLT4 | R1661Q | G | 4982 | A | MYO5B | R999C | G | 3216 | A | NOS1AP | V335M | G | 1405 | A | OR5D14 | L34V | C | 100 | G |
| MLLT4 | R991C | C | 2971 | T | MYO5B | R776Q | C | 2548 | T | NOS2 | D556E | G | 1902 | T | OR5D14 | F170V | T | 508 | G |
| MLLT4 | N778D | A | 2332 | G | MYO5B | A1345S | C | 4254 | A | NOS3 | P232L | C | 1052 | T | OR5D14 | E85D | G | 255 | T |
| MLLT4 | L1542I | C | 4624 | A | MYO5B | R219H | C | 877 | T | NOS3 | R474C | C | 1777 | T | OR5D16 | F222V | T | 664 | G |
| MLLT4 | R914C | C | 2740 | T | MYO5B | R1461L | C | 4603 | A | NOS3 | R875Q | G | 2981 | A | OR5D18 | R237C | C | 723 | T |
| MLLT6 | A327T | G | 1070 | A | MYO5B | P60L | G | 400 | A | NOS3 | R782H | G | 2702 | A | OR5D18 | R237C | C | 723 | T |
| MLLT6 | V635I | G | 1994 | A | MYO5C | R752* | G | 2391 | A | NOSIP | R112W | G | 377 | A | OR5F1 | D191Y | C | 571 | A |
| MLNR | T72A | A | 214 | G | MYO5C | R890S | C | 2807 | A | NOSIP | V209M | C | 668 | T | OR5F1 | E111K | C | 331 | T |
| MLNR | R376M | G | 1127 | T | MYO5C | R879* | G | 2772 | A | NOSTRIN | P460L | C | 2134 | T | OR5F1 | L85S | A | 254 | G |
| MLPH | A582V | C | 2039 | T | MYO5C | Q1605* | G | 4950 | A | NOTCH1 | E1525G | T | 4650 | C | OR5H14 | L62F | C | 184 | T |
| MLST8 | T108I | C | 397 | T | MYO5C | L1380I | G | 4275 | T | NOTCH1 | R1761W | G | 5357 | A | OR5H2 | L155S | T | 464 | C |
| MLST8 | A223T | G | 741 | A | MYO5C | S184L | G | 688 | A | NOTCH1 | S836N | C | 2583 | T | OR5H2 | V213I | G | 637 | A |
| MLX | R202H | G | 670 | A | MYO6 | N349S | A | 1325 | G | NOTCH1 | C1045Y | C | 3210 | T | OR5H6 | V224D | T | 712 | A |
| MLXIP | P879L | C | 2744 | T | MYO6 | R980C | C | 3217 | T | NOTCH1 | A465T | C | 1469 | T | OR5H6 | K40T | A | 160 | C |
| MLXIP | R214C | C | 748 | T | MYO7A | V843M | G | 2799 | A | NOTCH1 | T349I | G | 1122 | A | OR5H6 | E155* | G | 504 | T |
| MLXIPL | R820C | G | 2506 | A | MYO7A | A1437T | G | 4581 | A | NOTCH1 | C987* | G | 3037 | T | OR5H6 | R251Q | G | 793 | A |
| MLXIPL | G334W | C | 1048 | A | MYO7A | D1387N | G | 4431 | A | NOTCH2 | R1895C | G | 5903 | A | OR5H6 | S323I | G | 1009 | T |

| | | | | | | | | | | | | | | | | | | | |
|---|---|---|---|---|---|---|---|---|---|---|---|---|---|---|---|---|---|---|---|
| MLYCD | Q210R | A | 649 | G | MYO7A | H460Y | C | 1650 | T | NOTCH2 | A422S | C | 1484 | A | OR5I1 | L101M | G | 301 | T |
| MLYCD | F288L | T | 884 | G | MYO7A | R1696Q | G | 5359 | A | NOTCH2 | - | T | 0 | C | OR5I1 | K305N | T | 915 | G |
| MMD | N12S | T | 320 | C | MYO7A | L322P | T | 1237 | C | NOTCH2 | R2019L | C | 6276 | A | OR5K2 | F257V | T | 846 | G |
| MMD | R14Q | C | 326 | T | MYO7A | K1270N | G | 4082 | T | NOTCH2 | G1531V | C | 4812 | A | OR5K3 | Y218D | T | 652 | G |
| MMD2 | R165H | C | 689 | T | MYO7B | L1799P | T | 5449 | C | NOTCH3 | C1484Y | C | 4527 | T | OR5K4 | R143W | C | 427 | T |
| MME | D591N | G | 1982 | A | MYO7B | P1353S | C | 4110 | T | NOTCH3 | A1775T | C | 5399 | T | OR5K4 | L182I | C | 544 | A |
| MME | K667* | A | 2210 | T | MYO7B | V950I | G | 2901 | A | NOTCH3 | C1004Y | C | 3087 | T | OR5L1 | T153M | C | 547 | T |
| MMEL1 | R402H | C | 1367 | T | MYO7B | Y1284D | T | 3903 | G | NOTCH3 | V1641I | C | 4997 | T | OR5L1 | F101L | C | 392 | A |
| MMP1 | F70L | G | 278 | T | MYO7B | G1317D | G | 4003 | A | NOTCH3 | D217N | C | 725 | T | OR5L1 | S146Y | C | 526 | A |
| MMP10 | S207L | G | 657 | A | MYO7B | G1789S | G | 5418 | A | NOTCH3 | R1231H | C | 3768 | T | OR5L2 | L222I | C | 664 | A |
| MMP11 | R318H | G | 1005 | A | MYO7B | R733W | C | 2250 | T | NOTCH3 | A906T | C | 2792 | T | OR5M3 | G202S | C | 604 | T |
| MMP11 | V212A | T | 687 | C | MYO7B | L968M | C | 2955 | A | NOTCH3 | G1511S | C | 4607 | T | OR5M8 | F208L | A | 622 | G |
| MMP11 | A375T | G | 1175 | A | MYO7B | Q632H | G | 1949 | C | NOTCH3 | R1785H | C | 5430 | T | OR5M8 | K88N | C | 264 | A |
| MMP11 | T65M | C | 246 | T | MYO9A | R2370W | G | 7581 | A | NOTCH3 | R1526C | G | 4652 | A | OR5M9 | V23A | A | 68 | G |
| MMP11 | R116W | C | 398 | T | MYO9A | R1992W | G | 6447 | A | NOTCH3 | S1594L | G | 4857 | A | OR5P2 | D265G | T | 794 | C |
| MMP13 | T87A | T | 287 | C | MYO9A | S1657P | A | 5442 | G | NOTUM | R453* | G | 1741 | A | OR5P2 | V170I | C | 508 | T |
| MMP14 | Y164C | A | 746 | G | MYO9A | R1441I | C | 4795 | A | NOVA1 | E148D | T | 444 | G | OR5P2 | F174L | G | 522 | T |
| MMP15 | R381C | C | 1926 | T | MYO9A | S1369* | G | 4579 | T | NOVA1 | A357T | C | 1069 | T | OR5R1 | V248A | A | 743 | G |
| MMP15 | R126W | C | 1161 | T | MYO9A | I1142S | A | 3898 | C | NOX3 | R288Q | C | 966 | T | OR5R1 | D121N | C | 361 | T |
| MMP15 | E181D | G | 1328 | T | MYO9A | E68G | T | 676 | C | NOX3 | G371* | C | 1214 | A | OR5T1 | F322L | T | 1050 | C |
| MMP16 | A244S | C | 1012 | A | MYO9B | S762G | A | 2346 | G | NOX3 | F24L | A | 175 | C | OR5T1 | S276F | C | 913 | T |
| MMP16 | N371T | T | 1394 | G | MYO9B | A258T | G | 834 | A | NOX4 | G225S | C | 912 | T | OR5T3 | L215I | C | 643 | A |
| MMP16 | N370I | T | 1391 | A | MYO9B | R874C | C | 2682 | T | NOX4 | R525Q | C | 1813 | T | OR5T3 | K120E | A | 358 | G |
| MMP17 | R125C | C | 475 | T | MYO9B | V1006M | G | 3078 | A | NOX5 | R486C | C | 1497 | T | OR5T3 | I188R | T | 563 | G |
| MMP17 | A482T | G | 1546 | A | MYO9B | P1128S | C | 3444 | T | NOXA1 | P399S | C | 1375 | T | OR5W2 | F101L | G | 303 | T |
| MMP17 | A304V | C | 1013 | T | MYO9B | D82N | G | 306 | A | NOXA1 | R241I | G | 902 | T | OR5W2 | N19S | T | 56 | C |
| MMP17 | P587L | C | 1862 | T | MYO9B | I253L | A | 819 | C | NOXO1 | F24L | A | 74 | G | OR6B1 | C109R | T | 393 | C |
| MMP17 | R473M | G | 1520 | T | MYO9B | V882M | G | 2706 | A | NPAS1 | R351C | C | 1247 | T | OR6C1 | E68D | A | 242 | C |
| MMP17 | A593T | G | 1879 | A | MYO9B | R1352H | G | 4117 | A | NPAS1 | A102T | G | 500 | A | OR6C1 | L104I | C | 348 | A |
| MMP17 | V388M | G | 1264 | A | MYO9B | A2031T | G | 6153 | A | NPAS1 | L42Q | T | 321 | A | OR6C4 | S278L | C | 861 | T |
| MMP17 | V413I | G | 1339 | A | MYOC | A427T | C | 1339 | T | NPAS2 | R824* | C | 2755 | T | OR6C6 | L12I | G | 34 | T |
| MMP17 | R442M | G | 1427 | T | MYOC | A108V | G | 383 | A | NPAS2 | S122L | C | 650 | T | OR6C65 | F10L | C | 129 | A |
| MMP19 | R408Q | C | 1344 | T | MYOC | I432V | T | 1354 | C | NPAS2 | F168L | T | 789 | G | OR6C65 | L104I | C | 409 | A |
| MMP19 | R345Q | C | 1155 | T | MYOCD | F890L | C | 2870 | A | NPAS2 | F237V | T | 994 | G | OR6C68 | S164I | G | 491 | T |
| MMP19 | R130C | G | 509 | A | MYOCD | R103Q | G | 508 | A | NPAS3 | L863F | C | 2587 | T | OR6C68 | F35C | T | 104 | G |
| MMP2 | D324N | G | 1479 | A | MYOCD | A667V | C | 2200 | T | NPAS4 | L591I | C | 1947 | A | OR6C74 | L80I | C | 328 | A |
| MMP2 | R482C | C | 1953 | T | MYOCD | P829S | C | 2685 | T | NPAS4 | A319V | C | 1132 | T | OR6C76 | T288N | C | 863 | A |
| MMP2 | G299D | G | 1405 | A | MYOCD | R384* | C | 1350 | T | NPAS4 | G338V | G | 1189 | T | OR6C76 | Q229H | G | 687 | T |
| MMP20 | S162T | A | 497 | T | MYOD1 | D32N | G | 314 | A | NPAS4 | E396D | A | 1364 | T | OR6F1 | T291M | G | 872 | A |

| | | | | | | | | | | | | | | | | | | | |
|---|---|---|---|---|---|---|---|---|---|---|---|---|---|---|---|---|---|---|---|
| MMP20 | R35M | C | 117 | A | MYOF | - | A | 0 | G | NPAS4 | S554T | G | 1837 | C | OR6F1 | R234W | G | 700 | A |
| MMP20 | Q267R | T | 813 | C | MYOF | - | T | 0 | A | NPAS4 | A654V | C | 2137 | T | OR6F1 | I213V | T | 637 | C |
| MMP24 | I429V | A | 1370 | G | MYOF | - | A | 0 | G | NPAT | S857R | T | 2671 | G | OR6F1 | P18T | G | 52 | T |
| MMP25 | D173N | G | 754 | A | MYOF | R790* | G | 2368 | A | NPAT | G209S | C | 727 | T | OR6K3 | A315V | G | 944 | A |
| OR6K3 | E247* | C | 739 | A | PDE8B | H615R | A | 1889 | G | PLTP | R385H | C | 1234 | T | PSMF1 | P182S | C | 712 | T |
| OR6K6 | T144I | C | 527 | T | PDE8B | R714* | C | 2185 | T | PLTP | R372H | C | 1195 | T | PSMG2 | N130D | A | 1070 | G |
| OR6K6 | L94I | C | 376 | A | PDE9A | R468H | G | 1463 | A | PLUNC | P41S | C | 192 | T | PSMG4 | D73N | G | 288 | A |
| OR6K6 | K325T | A | 1070 | C | PDE9A | Q322R | A | 1025 | G | PLXDC2 | E109G | A | 1167 | G | PSMG4 | L14M | C | 111 | A |
| OR6M1 | A248V | G | 743 | A | PDGFB | R46H | C | 925 | T | PLXNA1 | R618H | G | 1853 | A | PSMG4 | A144T | G | 501 | A |
| OR6M1 | T313I | G | 938 | A | PDGFD | W232L | C | 1067 | A | PLXNA1 | I365T | T | 1094 | C | PSMG4 | - | A | 0 | C |
| OR6M1 | L105M | G | 313 | T | PDGFD | A28T | C | 454 | T | PLXNA1 | D545G | A | 1634 | G | PSPC1 | R290H | C | 1029 | T |
| OR6N1 | R307I | C | 920 | A | PDGFD | I91M | T | 645 | C | PLXNA1 | R544W | C | 1630 | T | PSPC1 | V111F | C | 491 | A |
| OR6N2 | R261W | G | 781 | A | PDGFD | R359* | G | 1447 | A | PLXNA1 | R825C | C | 2473 | T | PSTK | T264I | C | 790 | T |
| OR6P1 | P79H | G | 236 | T | PDGFRA | V140M | G | 749 | A | PLXNA1 | R1082W | C | 3244 | T | PSTK | E216D | G | 647 | T |
| OR6P1 | L201R | A | 602 | C | PDGFRA | E109D | G | 658 | T | PLXNA1 | R1384C | C | 4150 | T | PSTPIP1 | E159D | G | 927 | T |
| OR6P1 | L201R | A | 602 | C | PDGFRL | A107V | C | 533 | T | PLXNA1 | V491I | G | 1471 | A | PTAFR | R133C | G | 1032 | A |
| OR6P1 | D199A | T | 596 | G | PDGFRL | S115A | T | 556 | G | PLXNA1 | D214N | G | 640 | A | PTAR1 | A129S | C | 407 | A |
| OR6P1 | S254F | G | 761 | A | PDGFRL | F167V | T | 712 | G | PLXNA2 | Y1037C | T | 3868 | C | PTAR1 | L111Q | A | 354 | T |
| OR6P1 | G307V | C | 920 | A | PDHA1 | R342Q | G | 1101 | A | PLXNA2 | A1683V | G | 5806 | A | PTAR1 | A173V | G | 540 | A |
| OR6Q1 | A185V | C | 577 | T | PDHA2 | V340M | G | 1150 | A | PLXNA2 | G1208R | C | 4380 | T | PTAR1 | D85Y | C | 275 | A |
| OR6T1 | E133D | C | 399 | A | PDHA2 | R300H | G | 1031 | A | PLXNA2 | E1480K | C | 5196 | T | PTBP1 | A295V | C | 990 | T |
| OR6X1 | L210I | G | 628 | T | PDHA2 | A32T | G | 226 | A | PLXNA2 | D1025N | C | 3831 | T | PTBP1 | D455G | A | 1470 | G |
| OR6Y1 | F109V | A | 325 | C | PDHA2 | Q168* | C | 634 | T | PLXNA2 | A61V | G | 940 | A | PTBP2 | T177A | A | 529 | G |
| OR6Y1 | P317S | G | 949 | A | PDHA2 | M255R | T | 896 | G | PLXNA2 | T865M | G | 3352 | A | PTBP2 | E29K | G | 85 | A |
| OR6Y1 | I214S | A | 641 | C | PDHA2 | L318I | C | 1084 | A | PLXNA2 | A553V | G | 2416 | A | PTBP2 | I420S | T | 1259 | G |
| OR7A10 | Y120C | T | 359 | C | PDIA2 | P118H | C | 353 | A | PLXNA3 | R464* | C | 1565 | T | PTCD1 | E85D | C | 255 | A |
| OR7A17 | Y235H | A | 703 | G | PDIA2 | T129M | C | 386 | T | PLXNA3 | N1499D | A | 4670 | G | PTCD1 | R411Q | C | 1232 | T |
| OR7A5 | R165W | G | 581 | A | PDIA2 | R495H | G | 1484 | A | PLXNA4 | P1170L | G | 3738 | A | PTCD1 | K200N | C | 600 | A |
| OR7C1 | K186E | T | 556 | C | PDIA3 | K271N | G | 961 | T | PLXNA4 | V101I | C | 530 | T | PTCH1 | V1361M | C | 4269 | T |
| OR7C1 | K80R | T | 239 | C | PDIA3 | K288N | G | 1012 | T | PLXNA4 | R370W | G | 1337 | A | PTCH1 | A563T | C | 1875 | T |
| OR7C2 | C150R | T | 448 | C | PDIA4 | M358V | T | 1305 | C | PLXNA4 | R420C | G | 1487 | A | PTCH1 | - | C | 0 | T |
| OR7D4 | C97R | A | 289 | G | PDIA4 | L624V | G | 2103 | C | PLXNA4 | A48T | C | 371 | T | PTCH1 | V1065G | A | 3382 | C |
| OR7D4 | S229P | A | 685 | G | PDIA5 | C465Y | G | 1489 | A | PLXNA4 | R870W | G | 2837 | A | PTCH2 | R561H | C | 1813 | T |
| OR7D4 | A274T | C | 820 | T | PDIA5 | R492H | G | 1570 | A | PLXNA4 | Y1860C | T | 5808 | C | PTCH2 | W193C | C | 710 | A |
| OR7E24 | R157H | G | 584 | A | PDIA5 | A384V | C | 1246 | T | PLXNA4 | C1504R | A | 4739 | G | PTCHD1 | Q290H | G | 1730 | T |
| OR7E24 | K313N | G | 1053 | T | PDIA5 | - | A | 0 | G | PLXNA4 | R859H | C | 2805 | T | PTCHD1 | L717M | C | 3009 | A |
| OR7E24 | G114D | G | 455 | A | PDIA5 | D445Y | G | 1428 | T | PLXNA4 | V575M | C | 1952 | T | PTCHD2 | C1167Y | G | 3638 | A |
| OR7E87P | F116V | T | 346 | G | PDIA5 | K92T | A | 370 | C | PLXNA4 | L33M | G | 326 | T | PTCHD2 | D1040N | G | 3256 | A |
| OR7E8P | A94T | C | 280 | T | PDIA6 | R190C | G | 568 | A | PLXNA4 | I1864V | T | 5819 | C | PTCHD2 | R456C | C | 1504 | T |

| | | | | | | | | | | | | | | | | | | | |
|---|---|---|---|---|---|---|---|---|---|---|---|---|---|---|---|---|---|---|---|
| OR7E8P | V142A | A | 425 | G | PDIK1L | A43T | G | 414 | A | PLXNA4 | R888C | G | 2891 | A | PTCHD2 | V558I | G | 1810 | A |
| OR7G1 | F64C | A | 191 | C | PDILT | R233H | C | 947 | T | PLXNB1 | T2059I | G | 6279 | A | PTCHD2 | R1287W | C | 3997 | T |
| OR7G2 | A146V | G | 437 | A | PDILT | L66I | G | 445 | T | PLXNB1 | P1424T | G | 4373 | T | PTCHD2 | V972M | G | 3052 | A |
| OR7G3 | V151I | C | 451 | T | PDILT | V11L | C | 280 | G | PLXNB1 | V1947M | C | 5942 | T | PTCHD2 | R294H | G | 1019 | A |
| OR7G3 | L185P | A | 554 | G | PDILT | K251R | T | 1001 | C | PLXNB1 | R1305H | C | 4017 | T | PTCHD2 | A896V | C | 2825 | T |
| OR8A1 | T133A | A | 469 | G | PDK1 | V343I | G | 1171 | A | PLXNB1 | P2032L | G | 6198 | A | PTCHD3 | T101I | G | 420 | A |
| OR8A1 | L238I | C | 784 | A | PDK3 | H243P | A | 957 | C | PLXNB1 | R1338H | C | 4116 | T | PTCHD3 | A256V | G | 885 | A |
| OR8A1 | T246I | C | 809 | T | PDK4 | V242M | C | 922 | T | PLXNB1 | A705D | G | 2217 | T | PTCHD3 | L450I | G | 1466 | T |
| OR8B12 | A116V | G | 347 | A | PDK4 | R166H | C | 695 | T | PLXNB1 | E611G | T | 1935 | C | PTDSS1 | I369M | A | 1212 | G |
| OR8B12 | G43D | C | 128 | T | PDK4 | S189N | C | 764 | T | PLXNB1 | R1832H | C | 5598 | T | PTDSS1 | A248V | C | 848 | T |
| OR8B4 | Y60H | A | 178 | G | PDLIM1 | R69I | C | 315 | A | PLXNB2 | L1142P | A | 3566 | G | PTDSS1 | P98S | C | 397 | T |
| OR8D1 | S171P | A | 511 | G | PDLIM1 | P210L | G | 738 | A | PLXNB2 | R820H | C | 2600 | T | PTDSS2 | E343D | G | 1205 | T |
| OR8D4 | L245V | C | 763 | G | PDLIM3 | R241W | G | 795 | A | PLXNB2 | R531Q | C | 1733 | T | PTDSS2 | V103A | T | 484 | C |
| OR8H2 | L211I | C | 631 | A | PDLIM3 | K328T | T | 1057 | G | PLXNB2 | S996I | C | 3128 | A | PTEN | R233* | C | 2054 | T |
| OR8H3 | L222I | C | 664 | A | PDLIM3 | - | C | 0 | T | PLXNB2 | G689D | C | 2207 | T | PTEN | A3D | C | 1365 | A |
| OR8H3 | C112Y | G | 335 | A | PDLIM4 | V5M | G | 77 | A | PLXNB2 | T1096A | T | 3427 | C | PTEN | I28M | T | 1441 | G |
| OR8H3 | L198M | C | 592 | A | PDLIM5 | T74I | C | 265 | T | PLXNB3 | M326V | A | 1090 | G | PTEN | K125T | A | 1731 | C |
| OR8I2 | S164N | G | 522 | A | PDLIM7 | V362M | C | 1151 | T | PLXNB3 | R1505H | G | 4628 | A | PTEN | R130Q | G | 1746 | A |
| OR8I2 | L29W | T | 117 | G | PDLIM7 | A143V | G | 495 | A | PLXNB3 | A1464T | G | 4504 | A | PTEN | H93Y | C | 1634 | T |
| OR8I2 | F28L | C | 115 | A | PDP1 | G354S | G | 1060 | A | PLXNB3 | V1435M | G | 4417 | A | PTER | E76D | G | 406 | T |
| OR8I2 | L66V | T | 227 | G | PDP1 | R550H | G | 1649 | A | PLXNB3 | R1121W | C | 3475 | T | PTF1A | G272D | G | 1019 | A |
| OR8I2 | V11I | G | 62 | A | PDP1 | E588A | A | 1763 | C | PLXNC1 | D202G | A | 605 | G | PTGDR | R310Q | G | 1031 | A |
| OR8J1 | S171F | C | 544 | T | PDP2 | V341I | G | 1183 | A | PLXNC1 | L1086R | T | 3257 | G | PTGER3 | R175C | G | 754 | A |
| OR8J3 | Y60H | A | 178 | G | PDS5A | M1005I | C | 3555 | T | PLXNC1 | N895Y | A | 2683 | T | PTGER4 | Q313* | C | 1961 | T |
| OR8K3 | K297E | A | 889 | G | PDS5A | P998S | G | 3532 | A | PLXNC1 | K1077N | G | 3231 | T | PTGES | A138T | C | 447 | T |
| OR8K5 | E196* | C | 586 | A | PDS5A | E504K | C | 2050 | T | PLXNC1 | E1134K | G | 3400 | A | PTGES2 | R136S | G | 1151 | T |
| OR8K5 | V173A | A | 518 | G | PDS5A | R377C | G | 1669 | A | PLXND1 | A1793T | C | 5556 | T | PTGFR | I83T | T | 443 | C |
| OR8S1 | A237T | G | 709 | A | PDS5B | R289H | G | 1024 | A | PLXND1 | P259L | G | 955 | A | PTGFR | G224* | G | 865 | T |
| OR8S1 | F61L | C | 183 | A | PDS5B | V1222I | G | 3822 | A | PLXND1 | C702G | A | 2283 | C | PTGFR | K339N | A | 1212 | C |
| OR8S1 | R352C | C | 1054 | T | PDS5B | - | T | 0 | C | PLXND1 | R1370C | G | 4287 | A | PTGFRN | A733V | C | 2345 | T |
| OR9A2 | V97A | A | 353 | G | PDSS1 | S216C | C | 700 | G | PMEPA1 | R224H | C | 991 | T | PTGFRN | R214H | G | 788 | A |
| OR9A2 | K223N | C | 732 | A | PDXDC1 | R203C | C | 831 | T | PMFBP1 | T965M | G | 3026 | A | PTGFRN | R654H | G | 2108 | A |
| OR9A4 | F31L | C | 152 | A | PDXDC1 | N273D | A | 1041 | G | PMFBP1 | E921K | C | 2893 | T | PTGFRN | L9P | T | 173 | C |
| OR9G4 | R106H | C | 317 | T | PDXDC2 | - | T | 0 | C | PMFBP1 | D923G | T | 2900 | C | PTGIR | A90T | C | 381 | T |
| OR9I1 | C189R | A | 565 | G | PDYN | R232H | C | 921 | T | PMFBP1 | N952S | T | 2987 | C | PTGIR | A98T | C | 405 | T |
| OR9K2 | A142T | G | 512 | A | PDZD11 | R46Q | C | 270 | T | PML | R872H | G | 2711 | A | PTGIR | S345N | C | 1147 | T |
| OR9K2 | M70T | T | 297 | C | PDZD2 | E1596D | G | 5176 | T | PML | Q483P | A | 1544 | C | PTGIS | V460A | A | 1409 | G |
| OR9K2 | F281L | T | 931 | G | PDZD2 | A2659V | C | 8364 | T | PMM2 | R141C | C | 487 | T | PTGR2 | S270T | T | 1008 | A |
| OR9Q1 | V223M | G | 983 | A | PDZD2 | V806I | G | 2804 | A | PMM2 | S246C | C | 803 | G | PTGS1 | L534I | C | 1605 | A |

| | | | | | | | | | | | | | | | | | | | |
|---|---|---|---|---|---|---|---|---|---|---|---|---|---|---|---|---|---|---|---|
| OR9Q2 | A249T | G | 802 | A | PDZD2 | A372T | G | 1502 | A | PMP22 | V119A | A | 551 | G | PTGS1 | R179H | G | 541 | A |
| ORAI1 | S92G | A | 467 | G | PDZD2 | T1093I | C | 3666 | T | PMPCA | R516H | G | 1556 | A | PTGS2 | R600H | C | 1932 | T |
| ORAI1 | A279V | C | 1029 | T | PDZD2 | P2038L | C | 6501 | T | PMS1 | V791A | T | 2605 | C | PTGS2 | R600H | C | 1932 | T |
| ORAI2 | R180H | G | 774 | A | PDZD2 | G1606C | G | 5204 | T | PMS1 | A145D | C | 667 | A | PTH1R | S333F | C | 998 | T |
| ORAI2 | S49L | C | 381 | T | PDZD2 | L73I | C | 605 | A | PMS1 | G125D | G | 607 | A | PTH1R | D266G | A | 797 | G |
| ORAI3 | R145H | G | 658 | A | PDZD2 | R2083H | G | 6636 | A | PMS2 | I611M | A | 1939 | C | PTH2 | L22V | G | 166 | C |
| ORC1L | V90A | A | 488 | G | PDZD2 | R1117M | G | 3738 | T | PMS2 | Q342H | T | 1132 | G | PTH2R | - | G | 0 | T |
| ORC1L | V518A | A | 1772 | G | PDZD2 | A400V | C | 1587 | T | PNCK | G10R | C | 214 | T | PTH2R | S499N | G | 1913 | A |
| ORC1L | R608* | G | 2041 | A | PDZD2 | P1476S | C | 4814 | T | PNCK | A196T | C | 772 | T | PTH2R | D38N | G | 529 | A |
| ORC1L | A205T | C | 832 | T | PDZD2 | R309H | G | 1314 | A | PNKD | R377W | C | 1180 | T | PTHLH | R94Q | C | 603 | T |
| ORC1L | R134W | G | 619 | A | PDZD3 | D410N | G | 1228 | A | PNKD | R222Q | G | 716 | A | PTHLH | R56* | G | 488 | A |
| ORC2L | R39* | G | 365 | A | PDZD4 | R693W | G | 2346 | A | PNKD | R222Q | G | 716 | A | PTHLH | R94L | C | 603 | A |
| ORC4L | S310I | C | 1066 | A | PDZD4 | P571S | G | 1980 | A | PNKP | R293C | G | 983 | A | PTHLH | R142* | G | 746 | A |
| ORC4L | W435G | A | 1440 | C | PDZD4 | E243D | C | 998 | A | PNKP | W402* | C | 1312 | T | PTK2 | G904S | C | 2710 | T |
| ORC5L | P288L | G | 1006 | A | PDZD4 | A201V | G | 871 | A | PNLDC1 | L492M | C | 1508 | A | PTK2 | - | A | 0 | G |
| ORM1 | A47T | G | 217 | A | PDZD7 | R293Q | C | 1104 | T | PNLDC1 | W53* | G | 192 | A | PTK2 | D811G | T | 2432 | C |
| OS9 | S65F | C | 253 | T | PDZD7 | N137S | T | 636 | C | PNLIP | P464L | C | 1419 | T | PTK2 | A25T | C | 73 | T |
| OSBP | R514Q | C | 2021 | T | PDZD8 | R415Q | C | 1484 | T | PNLIP | G325C | G | 1001 | T | PTK2B | P666S | C | 2804 | T |
| OSBP | L294P | A | 1361 | G | PDZD8 | E1102* | C | 3544 | A | PNLIP | D96G | A | 315 | G | PTK6 | R223Q | C | 696 | T |
| OSBPL10 | G211R | C | 754 | T | PDZD8 | M1091V | T | 3511 | C | PNLIP | L110M | C | 356 | A | PTK6 | R85C | G | 281 | A |
| OSBPL10 | D356N | C | 1189 | T | PDZD8 | R382C | G | 1384 | A | PNLIPRP1 | G169C | G | 539 | T | PTK7 | G1001S | G | 3071 | A |
| OSBPL11 | I293F | T | 1167 | A | PDZRN3 | A947T | C | 2954 | T | PNLIPRP1 | Q84H | A | 286 | C | PTMAP5 | E73* | G | 374 | T |
| OSBPL11 | R143* | G | 717 | A | PDZRN3 | R971C | G | 3026 | A | PNLIPRP1 | S129F | C | 420 | T | PTMS | R30Q | G | 420 | A |
| OSBPL11 | Q704R | T | 2401 | C | PDZRN3 | E686D | C | 2173 | A | PNLIPRP3 | F241V | T | 867 | G | PTN | K146T | T | 865 | G |
| OSBPL11 | F539C | A | 1906 | C | PDZRN3 | R971H | C | 3027 | T | PNMA1 | K334T | T | 1787 | G | PTOV1 | R392C | C | 1344 | T |
| OSBPL11 | M224V | T | 960 | C | PDZRN4 | S581Y | C | 1742 | A | PNMA2 | R332W | G | 994 | A | PTP4A2 | Y149H | A | 1442 | G |
| OSBPL11 | K101Q | T | 591 | G | PDZRN4 | P577S | C | 1729 | T | PNMA5 | S252P | A | 1176 | G | PTP4A2 | A108V | G | 1320 | A |
| OSBPL1A | T353M | G | 1265 | A | PDZRN4 | R958C | C | 2872 | T | PNMAL1 | R126H | C | 683 | T | PTPDC1 | V13M | G | 37 | A |
| OSBPL1A | R835* | G | 2710 | A | PDZRN4 | A469T | G | 1405 | A | PNMAL1 | S328N | C | 1289 | T | PTPDC1 | R39Q | G | 116 | A |
| OSBPL2 | E99G | A | 498 | G | PDZRN4 | T748A | A | 2242 | G | PNMAL1 | P429H | G | 1592 | T | PTPDC1 | R721* | C | 2161 | T |
| OSBPL5 | R813W | G | 2553 | A | PDZRN4 | N500D | A | 1498 | G | PNMAL1 | G155E | C | 770 | T | PTPLA | P195S | G | 621 | A |
| OSBPL5 | R684Q | C | 2167 | T | PDZRN4 | R907Q | G | 2720 | A | PNMAL2 | R167I | C | 500 | A | PTPLA | P28S | G | 120 | A |
| OSBPL5 | P396L | G | 1303 | A | PEAR1 | S983N | G | 3173 | A | PNMT | R73H | G | 440 | A | PTPLAD1 | E2D | G | 151 | T |
| OSBPL5 | L864F | G | 2706 | A | PEAR1 | R190S | C | 793 | A | PNN | E465* | G | 1452 | T | PTPLAD1 | K147T | A | 585 | C |
| OSBPL5 | A817V | G | 2566 | A | PECI | A2V | G | 41 | A | PNN | R247K | G | 799 | A | PTPLAD1 | K358N | G | 1219 | T |
| OSBPL6 | E71K | G | 755 | A | PECI | Q20* | G | 94 | A | PNN | R123H | G | 427 | A | PTPLAD2 | S140F | G | 465 | A |
| OSBPL6 | L762P | T | 2829 | C | PEG10 | A263S | G | 1266 | T | PNO1 | R84I | G | 342 | T | PTPLAD2 | V100A | A | 345 | G |
| OSBPL6 | R180W | C | 1082 | T | PEG3AS | E122D | C | 366 | A | PNPLA1 | R228S | G | 684 | T | PTPLB | R62Q | C | 343 | T |
| OSBPL6 | A348T | G | 1586 | A | PEG3AS | R324H | C | 971 | T | PNPLA1 | N288T | A | 863 | C | PTPLB | V125I | C | 531 | T |

| | | | | | | | | | | | | | | | | | | | |
|---|---|---|---|---|---|---|---|---|---|---|---|---|---|---|---|---|---|---|---|
| OSBPL6 | I604V | A | 2354 | G | PEG3AS | R179C | G | 535 | A | PNPLA2 | V184M | G | 752 | A | PTPN11 | V181I | G | 694 | A |
| OSBPL6 | K711Q | A | 2675 | C | PEG3AS | G1544D | C | 4631 | T | PNPLA2 | V143A | T | 630 | C | PTPN11 | E76G | A | 380 | G |
| OSBPL7 | P497S | G | 1692 | A | PEG3AS | G1065D | C | 3194 | T | PNPLA3 | A325V | C | 1147 | T | PTPN11 | E225D | A | 828 | C |
| OSBPL7 | R382H | C | 1348 | T | PEG3AS | R604H | C | 1811 | T | PNPLA5 | V75F | C | 353 | A | PTPN11 | K325T | A | 1127 | C |
| OSBPL7 | R808W | G | 2625 | A | PEG3AS | G385D | C | 1517 | T | PNPLA6 | N20S | A | 337 | G | PTPN12 | W118R | T | 624 | C |
| OSBPL7 | P180L | G | 742 | A | PEG3AS | K677T | T | 2030 | G | PNPLA6 | S635L | C | 2182 | T | PTPN13 | L1590I | C | 5248 | A |
| OSBPL7 | R150S | G | 651 | T | PEG3AS | K569N | C | 1707 | A | PNPLA6 | A655T | G | 2241 | A | PTPN13 | K224R | A | 1151 | G |
| OSBPL7 | R525H | C | 1777 | T | PEG3AS | F562L | G | 1686 | T | PNPLA6 | G698S | G | 2370 | A | PTPN13 | R1422H | G | 4745 | A |
| OSBPL8 | W639* | C | 2396 | T | PEG3AS | F467C | A | 1400 | C | PNPLA6 | A627V | C | 2158 | T | PTPN13 | S1462F | C | 4865 | T |
| OSBPL8 | A696D | G | 2567 | T | PEG3AS | E112D | C | 336 | A | PNPLA7 | T638M | G | 2250 | A | PTPN13 | P1235L | C | 4184 | T |
| OSBPL8 | F294V | A | 1360 | C | PELI1 | Q101H | C | 746 | A | PNPLA7 | R911C | G | 3068 | A | PTPN13 | R2371C | C | 7591 | T |
| OSBPL9 | F132C | T | 393 | G | PELI2 | V115I | G | 629 | A | PNPLA7 | R348W | G | 1379 | A | PTPN13 | S1245N | G | 4214 | A |
| OSCP1 | G223C | C | 749 | A | PELI2 | S110N | G | 615 | A | PNPLA7 | R1324Q | C | 4308 | T | PTPN13 | P2329S | C | 7465 | T |
| OSCP1 | S245Y | G | 816 | T | PELI3 | V334M | G | 1164 | A | PNPLA7 | R1005W | G | 3350 | A | PTPN13 | F662C | T | 2465 | G |
| OSGEP | G177R | C | 913 | G | PELI3 | R269Q | G | 970 | A | PNPLA7 | R910C | G | 3065 | A | PTPN13 | I2141M | T | 6903 | G |
| OSGIN1 | E161K | G | 864 | A | PELI3 | R224Q | G | 835 | A | PNPLA7 | - | T | 0 | C | PTPN14 | Y119C | T | 551 | C |
| OSGIN1 | A94T | G | 663 | A | PELP1 | F804S | A | 2637 | G | PNPLA7 | S159L | G | 813 | A | PTPN14 | A192T | C | 769 | T |
| OSGIN2 | V150I | G | 708 | A | PENK | A20V | G | 140 | A | PNPLA8 | A255T | C | 1058 | T | PTPN14 | R672W | G | 2209 | A |
| OSGIN2 | - | T | 0 | C | PEPD | R314H | C | 1076 | T | PNPT1 | G604* | C | 1863 | A | PTPN14 | R24Q | C | 266 | T |
| OSM | Q97* | G | 330 | A | PER1 | S523N | C | 1806 | T | PNPT1 | P90H | G | 322 | T | PTPN14 | E1066* | C | 3391 | A |
| OSM | S182L | G | 586 | A | PER1 | - | C | 0 | T | POC1A | A63D | G | 228 | T | PTPN14 | E832A | T | 2690 | G |
| OSMR | V25A | T | 476 | C | PER1 | G24D | C | 309 | T | POC1A | T191A | T | 611 | C | PTPN18 | R443C | C | 1428 | T |
| OSMR | I657M | A | 2373 | G | PER2 | V19M | C | 335 | T | POC1B | N181S | T | 647 | C | PTPN18 | T236A | A | 807 | G |
| OSMR | M947L | A | 3241 | C | PER2 | A1049T | C | 3425 | T | POC1B | L325R | A | 1079 | C | PTPN18 | R321W | C | 1062 | T |
| OSR1 | A88T | C | 607 | T | PER2 | G1055S | C | 3443 | T | POC1B | H227N | G | 784 | T | PTPN2 | K122R | T | 423 | C |
| OSR1 | H94R | T | 626 | C | PER2 | A1049T | C | 3425 | T | POC1B | F191C | A | 677 | C | PTPN21 | L502P | A | 1710 | G |
| OSR2 | A202V | C | 605 | T | PER2 | P27Q | G | 360 | T | POC1B | F95L | G | 390 | T | PTPN21 | R11H | C | 237 | T |
| OSR2 | H327R | A | 980 | G | PES1 | D303N | C | 1014 | T | PODN | A374T | G | 1127 | A | PTPN21 | F1077C | A | 3435 | C |
| OSTF1 | I155V | A | 613 | G | PES1 | R336* | G | 1113 | A | PODN | A113T | G | 344 | A | PTPN22 | K548N | C | 1780 | A |
| OSTF1 | P62S | C | 334 | T | PET112L | V275I | C | 864 | T | PODN | R22C | C | 71 | T | PTPN22 | S588Y | G | 1899 | T |
| OSTM1 | N216K | A | 734 | T | PET112L | A232V | G | 736 | A | POF1B | P538T | G | 1732 | T | PTPN22 | V236A | A | 843 | G |
| OSTM1 | A92V | G | 361 | A | PET112L | A64S | C | 231 | A | POF1B | A451T | C | 1471 | T | PTPN23 | D317N | G | 1026 | A |
| OTOA | S403Y | C | 1209 | A | PET112L | A251V | G | 793 | A | POFUT2 | - | C | 0 | T | PTPN23 | R1158W | C | 3549 | T |
| OTOA | A650T | G | 1949 | A | PEX1 | R1193I | C | 3674 | A | POFUT2 | S390Y | G | 1196 | T | PTPN23 | R1459H | G | 4453 | A |
| OTOF | T1600I | G | 4926 | A | PEX1 | C382Y | C | 1241 | T | POGK | A53V | C | 308 | T | PTPN23 | L338F | G | 1091 | T |
| OTOF | R1589H | C | 4893 | T | PEX1 | D694Y | C | 2176 | A | POGZ | V71I | C | 526 | T | PTPN3 | S372F | G | 1227 | A |
| OTOF | D1798N | C | 5519 | T | PEX1 | E451D | C | 1449 | A | POGZ | R1177K | C | 3845 | T | PTPN3 | R786* | G | 2468 | A |
| OTOG | L2332M | C | 6994 | A | PEX10 | R83W | G | 275 | A | POGZ | R374W | G | 1435 | A | PTPN3 | T744M | G | 2343 | A |
| OTOG | R2365H | G | 7094 | A | PEX11A | G226C | C | 826 | A | POGZ | R1039H | C | 3431 | T | PTPN3 | T854A | T | 2672 | C |

| | | | | | | | | | | | | | | | | | | | |
|---|---|---|---|---|---|---|---|---|---|---|---|---|---|---|---|---|---|---|---|
| OTOP1 | D390G | T | 1194 | C | PEX11B | D100N | G | 447 | A | POGZ | R979* | G | 3250 | A | PTPN3 | R106* | G | 428 | A |
| OTOP1 | H117N | G | 374 | T | PEX11G | L151P | A | 461 | G | POGZ | F822C | A | 2780 | C | PTPN3 | F70V | A | 320 | C |
| OTOP1 | D510A | T | 1554 | G | PEX12 | I62L | T | 718 | G | POLA1 | - | T | 4448 | C | PTPN4 | K665E | A | 2764 | G |
| OTOP1 | D487Y | C | 1484 | A | PEX12 | A235T | C | 1237 | T | POLA2 | P424L | C | 1605 | T | PTPN5 | V162M | C | 915 | T |
| OTOP1 | E376D | C | 1153 | A | PEX13 | T365M | C | 1132 | T | POLA2 | R167Q | G | 834 | A | PTPN5 | E292* | C | 1305 | A |
| OTOP1 | V181I | C | 566 | T | PEX13 | K232Q | A | 732 | C | POLA2 | H369Y | C | 1439 | T | PTPN6 | R277C | C | 964 | T |
| OTOP1 | P36L | G | 132 | A | PEX16 | T318M | G | 1265 | A | POLB | E86G | A | 391 | G | PTPN6 | A515T | G | 1678 | A |
| OTOP2 | A73T | G | 309 | A | PEX19 | E68D | C | 226 | A | POLD1 | R423H | G | 1312 | A | PTPN7 | G161V | C | 1256 | A |
| OTOP2 | D224N | G | 762 | A | PEX2 | G284D | C | 1277 | T | POLD1 | L22F | C | 108 | T | PTPN7 | P460S | G | 2152 | A |
| OTOP2 | V301M | G | 993 | A | PEX26 | A202T | G | 604 | A | POLD1 | S905P | T | 2757 | C | PTPN7 | E205* | C | 1387 | A |
| OTOP2 | S497Y | C | 1582 | A | PEX3 | L292I | C | 937 | A | POLD2 | V165M | C | 1143 | T | PTPN9 | T446M | G | 1850 | A |
| OTOP3 | R222W | C | 664 | T | PEX3 | E372D | G | 1179 | T | POLD3 | E51* | G | 226 | T | PTPN9 | F151C | A | 965 | C |
| OTOP3 | L118M | C | 352 | A | PEX5 | G59D | G | 176 | A | POLD3 | E297D | A | 966 | C | PTPRA | T179A | A | 928 | G |
| OTOS | A32V | G | 326 | A | PEX5L | E529D | C | 1918 | A | POLD3 | E378K | G | 1207 | A | PTPRA | R583W | C | 2140 | T |
| OTP | P59H | G | 324 | T | PEX5L | H485Y | G | 1784 | A | POLDIP3 | E288D | C | 945 | A | PTPRB | - | A | 0 | G |
| OTUB2 | G66V | G | 357 | T | PEX5L | S54P | A | 491 | G | POLDIP3 | A344V | G | 1112 | A | PTPRB | R86H | C | 292 | T |
| OTUD1 | K289T | A | 1055 | C | PEX5L | E489* | C | 1796 | A | POLE | P286R | G | 901 | C | PTPRB | R1046C | G | 3171 | A |
| OTUD4 | T624I | G | 1871 | A | PEX5L | K418N | C | 1585 | A | POLE | F104L | G | 356 | T | PTPRB | Y2043H | A | 6162 | G |
| OTUD4 | D431G | T | 1292 | C | PEX5L | S205* | G | 945 | T | POLE | R266* | G | 840 | A | PTPRB | K1455* | T | 4398 | A |
| OTUD4 | E889* | C | 2665 | A | PEX5L | R186* | G | 887 | A | POLE | A969V | G | 2950 | A | PTPRB | - | C | 0 | A |
| OTUD5 | A437V | G | 1371 | A | PEX5L | R543Q | C | 1959 | T | POLE | L1350P | A | 4093 | G | PTPRB | D2149G | T | 6481 | C |
| OTUD6A | H59R | A | 210 | G | PEX6 | R969Q | C | 2976 | T | POLE | Q2282* | G | 6888 | A | PTPRB | I1565M | A | 4730 | C |
| OTUD7A | R89* | G | 358 | A | PEX7 | A259T | G | 856 | A | POLE | P286R | G | 901 | C | PTPRB | R1541C | G | 4656 | A |
| OTUD7A | S563L | G | 1781 | A | PEX7 | A218V | C | 734 | T | POLE2 | R360C | G | 1092 | A | PTPRB | F5L | A | 50 | C |
| OTUD7A | V77E | A | 323 | T | PEX7 | K186N | G | 639 | T | POLE2 | K105T | T | 328 | G | PTPRB | G569S | C | 1740 | T |
| OTUD7A | L212V | A | 727 | C | PFAS | N995S | A | 3108 | G | POLE3 | R61W | G | 270 | A | PTPRC | I321M | T | 1104 | G |
| OTUD7B | R832W | G | 2789 | A | PFAS | R1201C | C | 3725 | T | POLG | R807C | G | 2753 | A | PTPRC | K600T | A | 1940 | C |
| OTUD7B | - | T | 0 | C | PFAS | A1251V | C | 3876 | T | POLG | - | T | 0 | G | PTPRC | A1267V | C | 3941 | T |
| OTX1 | D315N | G | 1219 | A | PFAS | R610I | G | 1953 | T | POLG | E105K | C | 647 | T | PTPRC | R859C | C | 2716 | T |
| OTX2 | S245P | A | 1010 | G | PFDN1 | K10N | C | 78 | A | POLG2 | M314I | C | 1025 | T | PTPRC | I905L | A | 2854 | C |
| OVCA2 | A90T | G | 333 | A | PFDN6 | V43A | T | 534 | C | POLG2 | K477R | T | 1513 | C | PTPRC | F582Y | T | 1886 | A |
| OVCH1 | P854S | G | 2560 | A | PFKFB1 | R29Q | C | 157 | T | POLH | R377C | C | 1424 | T | PTPRC | A182V | C | 686 | T |
| OVCH1 | C373R | A | 1117 | G | PFKFB2 | F149L | T | 569 | C | POLH | Q464H | G | 1687 | T | PTPRC | L583M | C | 1888 | A |
| OVCH1 | F1075C | A | 3224 | C | PFKFB3 | A390T | G | 1563 | A | POLI | V44A | T | 155 | C | PTPRC | R890* | C | 2809 | T |
| OVCH1 | - | A | 0 | C | PFKFB4 | V114A | A | 454 | G | POLI | V433A | T | 1322 | C | PTPRC | A1189V | C | 3707 | T |
| OVCH1 | I124S | A | 371 | C | PFKFB4 | T464M | G | 1504 | A | POLK | S735Y | C | 2376 | A | PTPRD | A1656S | C | 5722 | A |
| OVCH1 | I119S | A | 356 | C | PFKFB4 | V247M | C | 852 | T | POLK | A113V | C | 510 | T | PTPRD | M1164I | C | 4248 | T |
| OVCH1 | R28C | G | 82 | A | PFKFB4 | A440T | C | 1431 | T | POLM | R230H | C | 736 | T | PTPRD | R139H | C | 1172 | T |
| OVCH2 | R51C | G | 151 | A | PFKL | T659M | C | 1976 | T | POLN | A879S | C | 2635 | A | PTPRD | P187A | G | 1315 | C |

| | | | | | | | | | | | | | | | | | | | |
|---|---|---|---|---|---|---|---|---|---|---|---|---|---|---|---|---|---|---|---|
| OVCH2 | Q41* | G | 121 | A | PFKL | C569Y | G | 1706 | A | POLQ | E701* | C | 2101 | A | PTPRD | S504P | A | 2266 | G |
| OVCH2 | S22Y | G | 65 | T | PFKL | R470Q | G | 1409 | A | POLQ | T418A | T | 1252 | C | PTPRD | R733H | C | 2954 | T |
| OVGP1 | R670S | C | 2010 | A | PFKL | R612S | C | 1834 | A | POLQ | R2305H | C | 6914 | T | PTPRD | Y1361H | A | 4837 | G |
| OXA1L | A34V | C | 101 | T | PFKP | R575Q | G | 1800 | A | POLQ | R2583H | C | 7748 | T | PTPRD | G1719W | C | 5911 | A |
| OXA1L | R394W | C | 1180 | T | PFKP | A445T | G | 1409 | A | POLQ | G2581R | C | 7741 | T | PTPRE | R374H | G | 1400 | A |
| OXCT1 | T471A | T | 1572 | C | PGA5 | R369H | G | 1136 | A | POLQ | R421C | G | 1261 | A | PTPRF | R407C | C | 1559 | T |
| OXCT1 | - | C | 0 | G | PGAM1 | - | T | 0 | C | POLQ | K2541T | T | 7622 | G | PTPRF | R1451W | C | 4691 | T |
| OXCT1 | R110H | C | 490 | T | PGAM5 | R118H | G | 380 | A | POLQ | R2377Q | C | 7130 | T | PTPRF | H1122R | A | 3705 | G |
| OXCT1 | I264V | T | 951 | C | PGAM5 | A65T | G | 594 | A | POLQ | V1444A | A | 4331 | G | PTPRF | R216C | C | 986 | T |
| OXCT2 | V261I | C | 874 | T | PGAM5 | R214C | C | 667 | T | POLR1A | F298C | A | 1272 | C | PTPRF | P440H | C | 1659 | A |
| OXGR1 | G323R | C | 1211 | T | PGAM5 | A120T | G | 423 | A | POLR1A | R565C | G | 2072 | A | PTPRG | G784V | G | 2728 | T |
| OXGR1 | A312T | C | 1178 | T | PGAP1 | N94H | T | 395 | G | POLR1A | R1598C | G | 5171 | A | PTPRH | R390I | C | 1210 | A |
| OXGR1 | V252I | C | 998 | T | PGAP2 | R149H | G | 559 | A | POLR1A | R1501W | G | 4880 | A | PTPRH | M1055T | A | 3205 | G |
| OXNAD1 | R34H | G | 566 | A | PGAP2 | R171H | G | 625 | A | POLR1A | A1609T | C | 5204 | T | PTPRH | R977C | G | 2970 | A |
| OXNAD1 | E221K | G | 1126 | A | PGAP2 | V80I | G | 238 | A | POLR1A | P927L | G | 3159 | A | PTPRH | S662F | G | 2026 | A |
| OXR1 | R88Q | G | 524 | A | PGAP2 | F90C | T | 382 | G | POLR1A | T662K | G | 2364 | T | PTPRH | A477T | C | 1470 | T |
| OXR1 | V159I | G | 736 | A | PGAP3 | H284R | T | 894 | C | POLR1A | P1474L | G | 4800 | A | PTPRH | E1098D | T | 3335 | G |
| OXR1 | E66K | G | 457 | A | PGAP3 | V296I | C | 929 | T | POLR1A | R1385G | T | 4532 | C | PTPRJ | L738P | T | 2568 | C |
| OXR1 | E275* | G | 1084 | T | PGAP3 | C64Y | C | 234 | T | POLR1B | R351Q | G | 1632 | A | PTPRJ | P653S | C | 2312 | T |
| OXSR1 | A163T | G | 859 | A | PGBD1 | K634R | A | 2306 | G | POLR1B | D809N | G | 3005 | A | PTPRJ | A670T | G | 2363 | A |
| OXSR1 | - | T | 0 | C | PGBD1 | K569T | A | 2111 | C | POLR1C | G20V | G | 147 | T | PTPRJ | P598L | C | 2148 | T |
| OXTR | T273A | T | 1442 | C | PGBD3 | R402Q | C | 1407 | T | POLR1E | L306R | T | 1205 | G | PTPRK | N927K | G | 3036 | T |
| OXTR | L331P | A | 1617 | G | PGBD3 | Y165H | A | 695 | G | POLR2A | T1184M | C | 3769 | T | PTPRK | R1107* | G | 3574 | A |
| OXTR | P108L | G | 948 | A | PGBD4 | D423E | T | 1728 | G | POLR2B | E346* | G | 1449 | T | PTPRK | I1097T | A | 3545 | G |
| P2RX1 | G391C | C | 1612 | A | PGBD5 | V471M | C | 1411 | T | POLR2B | R834H | G | 2914 | A | PTPRK | E845D | C | 2790 | A |
| P2RX2 | P101S | C | 301 | T | PGBD5 | T256S | T | 766 | A | POLR2B | A1154T | G | 3873 | A | PTPRK | Q1299H | C | 4152 | A |
| P2RX2 | R286Q | G | 857 | A | PGD | F42L | C | 164 | A | POLR2B | V237F | G | 1122 | T | PTPRK | V129A | A | 641 | G |
| P2RX2 | K207N | A | 621 | T | PGD | F84L | C | 290 | A | POLR2D | E106Q | C | 373 | G | PTPRK | P1441L | G | 4577 | A |
| P2RX3 | S272I | G | 891 | T | PGD | R324W | C | 1008 | T | POLR2E | V105M | C | 390 | T | PTPRK | F1028L | G | 3339 | T |
| P2RX3 | W253R | T | 833 | C | PGF | R127H | C | 380 | T | POLR2H | R111C | C | 390 | T | PTPRK | I961M | A | 3138 | C |
| P2RX3 | R52Q | G | 231 | A | PGGT1B | N296D | T | 907 | C | POLR3A | P777S | G | 2467 | A | PTPRK | C864Y | C | 2846 | T |
| P2RX3 | L32I | C | 170 | A | PGGT1B | D318Y | C | 973 | A | POLR3A | H33R | T | 236 | C | PTPRM | P586H | C | 1757 | A |
| P2RX3 | E161D | A | 559 | C | PGK1 | - | G | 0 | T | POLR3A | F558L | G | 1812 | T | PTPRM | A12V | C | 35 | T |
| P2RX6 | V339I | G | 1163 | A | PGK2 | A347V | G | 1040 | A | POLR3A | T280A | T | 976 | C | PTPRM | S669G | A | 2005 | G |
| P2RX6 | - | T | 0 | C | PGK2 | F244S | A | 731 | G | POLR3B | I20T | T | 228 | C | PTPRM | S67Y | C | 200 | A |
| P2RX6 | S207N | G | 768 | A | PGLS | E121A | A | 412 | C | POLR3B | V191M | G | 740 | A | PTPRM | K766T | A | 2297 | C |
| P2RX7 | G290D | G | 965 | A | PGLYRP1 | G180V | C | 564 | A | POLR3B | I138T | T | 582 | C | PTPRM | - | G | 0 | A |
| P2RX7 | E305D | G | 1011 | T | PGLYRP2 | R233Q | C | 1179 | T | POLR3B | S1119Y | C | 3525 | A | PTPRN | R776Q | C | 2568 | T |
| P2RY1 | N11S | A | 54 | G | PGLYRP2 | Q437* | G | 1790 | A | POLR3B | D375Y | G | 1292 | T | PTPRN | P427H | G | 1521 | T |

| | | | | | | | | | | | | | | | | | | | |
|---|---|---|---|---|---|---|---|---|---|---|---|---|---|---|---|---|---|---|---|
| P2RY10 | - | G | 0 | A | PGLYRP2 | L90P | A | 750 | G | POLR3B | R550Q | G | 1818 | A | PTPRN | D923N | C | 3008 | T |
| P2RY12 | E215* | C | 943 | A | PGLYRP3 | E163D | C | 542 | A | POLR3B | A775T | G | 2492 | A | PTPRN | A340T | C | 1259 | T |
| P2RY13 | E326D | T | 998 | G | PGLYRP4 | R201Q | C | 667 | T | POLR3C | L271F | G | 972 | A | PTPRN | A913V | G | 2979 | A |
| P2RY13 | T93A | T | 297 | C | PGLYRP4 | P318H | G | 1018 | T | POLR3E | G541D | G | 1789 | A | PTPRN2 | - | C | 0 | T |
| P2RY14 | S226L | G | 990 | A | PGLYRP4 | S69I | C | 271 | A | POLR3E | R186C | C | 723 | T | PTPRN2 | R545H | C | 1634 | T |
| P2RY2 | G134S | G | 867 | A | PGM2L1 | K245N | C | 1031 | A | POLR3E | W640* | G | 2087 | A | PTPRN2 | F1020S | A | 3059 | G |
| P2RY2 | A208T | G | 1089 | A | PGM3 | M193T | A | 578 | G | POLR3E | M247I | G | 908 | T | PTPRN2 | R545H | C | 1634 | T |
| P2RY2 | V45M | G | 600 | A | PGM3 | V172A | A | 515 | G | POLR3F | I22V | A | 444 | G | PTPRO | S1137I | G | 3584 | T |
| P2RY4 | S177G | T | 709 | C | PGM5 | V147D | T | 669 | A | POLR3G | E187* | G | 759 | T | PTPRQ | E626* | G | 1876 | T |
| P2RY6 | V271I | G | 1216 | A | PGP | L194M | G | 624 | T | POLR3GL | I129V | T | 492 | C | PTPRQ | C1453Y | G | 4358 | A |
| P2RY6 | A281V | C | 1247 | T | PGPEP1 | R128* | C | 477 | T | POLR3GL | H188R | T | 670 | C | PTPRQ | R1716* | C | 5146 | T |
| P2RY6 | A133T | G | 802 | A | PGPEP1L | A142T | C | 630 | T | POLR3H | - | A | 673 | G | PTPRQ | I918F | A | 2752 | T |
| P2RY6 | P298H | C | 1298 | A | PGR | R740Q | C | 2962 | T | POLR3K | N33I | T | 140 | A | PTPRQ | D389Y | G | 1165 | T |
| P4HA1 | R362Q | C | 1202 | T | PGR | P547L | G | 2383 | A | POLRMT | P294L | G | 937 | A | PTPRQ | E706K | G | 2116 | A |
| P4HA1 | N367T | T | 1217 | G | PGR | P307H | G | 1663 | T | POLRMT | R458C | G | 1428 | A | PTPRQ | K1538N | G | 4614 | T |
| P4HA2 | R498Q | C | 2062 | T | PGR | L727I | G | 2922 | T | POLRMT | M109T | A | 382 | G | PTPRQ | R2263I | G | 6788 | T |
| P4HA2 | F525L | A | 2142 | G | PGR | S99N | C | 1039 | T | POLRMT | A875T | C | 2679 | T | PTPRQ | I2274T | T | 6821 | C |
| P4HB | A178V | G | 731 | A | PGR | R899Q | C | 3439 | T | POLRMT | A961V | G | 2938 | A | PTPRQ | E2018V | A | 6053 | T |
| P4HTM | N428S | A | 1651 | G | PGRMC1 | A8V | C | 134 | T | POM121C | Q522* | G | 1564 | A | PTPRR | Y547C | T | 2056 | C |
| P4HTM | R195W | C | 951 | T | PGS1 | V138A | T | 425 | C | POM121C | S443Y | G | 1328 | T | PTPRR | R308* | G | 1338 | A |
| P4HTM | G217E | G | 1018 | A | PHACTR1 | T236M | C | 707 | T | POM121L1 2 | A166T | G | 526 | A | PTPRR | R531* | G | 2007 | A |
| PA2G4 | L183I | C | 547 | A | PHACTR1 | D139E | T | 417 | A | POM121L1 2 | P273H | C | 848 | A | PTPRS | R979Q | C | 3170 | T |
| PABPC1 | S175F | G | 1028 | A | PHACTR1 | R27I | G | 80 | T | POM121L2 | G982C | C | 2944 | A | PTPRS | G1454S | C | 4594 | T |
| PABPC1 | M573I | C | 2223 | T | PHACTR2 | R257H | G | 969 | A | POM121L2 | A389D | G | 1166 | T | PTPRS | R714H | C | 2375 | T |
| PABPC1L | P412S | C | 1316 | T | PHACTR3 | T480I | C | 1906 | T | POM121L2 | R272I | C | 815 | A | PTPRS | D1144G | T | 3665 | C |
| PABPC1L | R473H | G | 1500 | A | PHACTR3 | R105* | C | 780 | T | POMGNT1 | V525I | C | 2224 | T | PTPRS | V1531I | C | 4825 | T |
| PABPC1L | R89* | C | 347 | T | PHACTR4 | R708C | C | 2336 | T | POMGNT1 | R207K | C | 1271 | T | PTPRS | K1090E | T | 3502 | C |
| PABPC1L | K592N | G | 1858 | T | PHAX | Q104H | G | 1007 | T | POMGNT1 | V101I | C | 952 | T | PTPRS | P292S | G | 1108 | A |
| PABPC1L | Q479P | A | 1518 | C | PHB2 | A275V | G | 1034 | A | POMP | K33R | A | 179 | G | PTPRS | R612S | G | 2068 | T |
| PABPC3 | E149K | G | 506 | A | PHC1 | R962H | G | 2885 | A | POMP | L100I | C | 379 | A | PTPRS | A440V | G | 1553 | A |
| PABPC3 | R41W | C | 182 | T | PHC1 | A902T | G | 2704 | A | POMT1 | H224Y | C | 849 | T | PTPRT | A293S | C | 1113 | A |
| PABPC3 | A217T | G | 710 | A | PHC1 | F148V | T | 442 | G | POMT1 | G77A | G | 409 | C | PTPRT | I521M | G | 1799 | C |
| PABPC3 | K575E | A | 1784 | G | PHC2 | S663F | G | 2042 | A | POMT1 | N435T | A | 1483 | C | PTPRT | R1352C | G | 4290 | A |
| PABPC3 | R94Q | G | 342 | A | PHC2 | V57M | C | 223 | T | POMT1 | S602L | C | 1984 | T | PTPRT | L781M | G | 2577 | T |
| PABPC3 | R519C | C | 1616 | T | PHC2 | P361Q | G | 1136 | T | POMT2 | L113V | G | 538 | C | PTPRT | W1310* | C | 4166 | T |
| PABPC3 | R374C | C | 1181 | T | PHC2 | A694V | G | 2135 | A | POMT2 | G340S | C | 1219 | T | PTPRU | R1177Q | G | 3659 | A |
| PABPC4 | R459H | C | 1826 | T | PHEX | F531I | T | 2156 | A | PON1 | - | C | 0 | A | PTPRU | R1217C | C | 3778 | T |
| PABPC4 | R463H | C | 1838 | T | PHEX | Q714R | A | 2706 | G | PON2 | G84E | C | 498 | T | PTPRU | A1392T | G | 4303 | A |

| | | | | | | | | | | | | | | | | | | | |
|---|---|---|---|---|---|---|---|---|---|---|---|---|---|---|---|---|---|---|---|
| PABPC4 | A551V | G | 2102 | A | PHEX | F81L | T | 806 | C | POP1 | Y636D | T | 1987 | G | PTPRU | I517T | T | 1679 | C |
| PABPC5 | R99C | C | 509 | T | PHEX | M300I | G | 1465 | A | POPDC2 | W188* | C | 1023 | T | PTPRU | S1185L | C | 3683 | T |
| PABPC5 | R280H | G | 1053 | A | PHF10 | R360H | C | 1203 | T | POPDC2 | R164Q | C | 951 | T | PTPRU | V461M | G | 1510 | A |
| PABPC5 | A157T | G | 683 | A | PHF10 | K493N | T | 1603 | G | POPDC2 | F182L | G | 1006 | T | PTPRU | V459I | G | 1504 | A |
| PACRG | H132R | A | 395 | G | PHF10 | N280S | T | 963 | C | PORCN | E145G | A | 477 | G | PTPRU | - | G | 0 | T |
| PACRGL | E141* | G | 812 | T | PHF10 | R112Q | C | 459 | T | POSTN | E298K | C | 1010 | T | PTPRZ1 | L378V | T | 1543 | G |
| PACS1 | - | G | 0 | T | PHF10 | - | C | 0 | T | POSTN | R210* | G | 746 | A | PTPRZ1 | G955C | G | 3274 | T |
| PACS1 | S774G | A | 2454 | G | PHF12 | A307D | G | 1731 | T | POSTN | D146E | A | 556 | T | PTPRZ1 | L990F | C | 3379 | T |
| PACS1 | H181N | C | 675 | A | PHF12 | R357Q | C | 1881 | T | POSTN | F790L | G | 2488 | T | PTPRZ1 | R2108M | G | 6734 | T |
| PACS2 | D226N | G | 793 | A | PHF14 | A172T | G | 949 | A | POSTN | E771* | C | 2429 | A | PTPRZ1 | E356D | G | 1479 | T |
| PACS2 | V311I | G | 1048 | A | PHF14 | Y893C | A | 3113 | G | POSTN | K731N | T | 2311 | G | PTPRZ1 | L375I | C | 1534 | A |
| PACS2 | P298H | C | 1010 | A | PHF14 | D24A | A | 506 | C | POSTN | L521S | A | 1680 | G | PTPRZ1 | R833C | C | 2908 | T |
| PACS2 | D309N | G | 1042 | A | PHF14 | C871Y | G | 3047 | A | POSTN | M86L | T | 374 | G | PTPRZ1 | V1311A | T | 4343 | C |
| PACSIN1 | E100K | G | 493 | A | PHF14 | A483V | C | 1883 | T | POSTN | K61N | C | 301 | A | PTPRZ1 | V1642A | T | 5336 | C |
| PADI2 | V212L | C | 698 | A | PHF14 | R540W | C | 2053 | T | POSTN | I49M | A | 265 | C | PTPRZ1 | K1753N | G | 5670 | T |
| PADI3 | V251M | G | 791 | A | PHF14 | A539V | C | 2051 | T | POSTN | L607S | A | 1938 | G | PTRF | R267W | G | 958 | A |
| PADI3 | L264F | C | 830 | T | PHF14 | K646T | A | 2372 | C | POTEA | V140A | T | 462 | C | PTRF | R286W | G | 1015 | A |
| PADI3 | R383I | G | 1188 | T | PHF15 | R70W | C | 208 | T | POTEC | S393G | T | 1544 | C | PTRH2 | R35Q | C | 1423 | T |
| PADI4 | K377N | G | 1157 | T | PHF15 | G828C | G | 2482 | T | POTEE | A127T | G | 379 | A | PTTG1 | L161M | C | 529 | A |
| PADI4 | M80I | G | 266 | T | PHF15 | P253H | C | 758 | A | POTEF | E130G | T | 441 | C | PTTG2 | S103Y | C | 308 | A |
| PAF1 | S38I | C | 288 | A | PHF15 | G358S | G | 1072 | A | POTEG | E362* | G | 1136 | T | PTTG2 | F117L | C | 351 | A |
| PAFAH1B1 | R241W | C | 1289 | T | PHF15 | Q84P | A | 251 | C | POTEH | P467S | G | 1451 | A | PTX3 | R360W | C | 1223 | T |
| PAFAH1B1 | K351E | A | 1619 | G | PHF16 | T424M | C | 1322 | T | POTEI | L942M | G | 3255 | T | PTX4 | R427W | G | 1279 | A |
| PAFAH1B1 | A210P | G | 1196 | C | PHF16 | R207Q | G | 671 | A | POU1F1 | - | C | 0 | T | PUF60 | R146C | G | 500 | A |
| PAFAH1B1 | R363H | G | 1656 | A | PHF19 | - | C | 0 | T | POU1F1 | W219C | C | 742 | A | PUF60 | A21P | C | 125 | G |
| PAFAH1B2 | L212P | T | 737 | C | PHF2 | P700L | C | 2466 | T | POU1F1 | S8L | G | 108 | A | PUM1 | R854* | G | 2691 | A |
| PAFAH1B2 | S64F | C | 293 | T | PHF2 | D46N | G | 503 | A | POU2F1 | R403H | G | 1417 | A | PUM1 | M861V | T | 2712 | C |
| PAFAH2 | R71C | G | 391 | A | PHF2 | T520M | C | 1926 | T | POU2F1 | R311* | C | 1140 | T | PUM1 | V1134A | A | 3532 | G |
| PAFAH2 | R315Q | C | 1124 | T | PHF2 | V129A | T | 753 | C | POU2F1 | T31A | A | 300 | G | PUM1 | G781D | C | 2473 | T |
| PAG1 | E186K | C | 1267 | T | PHF2 | R285Q | G | 1221 | A | POU2F1 | G32S | G | 303 | A | PUM2 | R174C | G | 543 | A |
| PAG1 | E381K | C | 1852 | T | PHF2 | A399D | C | 1563 | A | POU2F2 | S274R | T | 902 | G | PURA | Q10* | C | 87 | T |
| PAG1 | T147M | G | 1151 | A | PHF2 | T493A | A | 1844 | G | POU2F3 | R36Q | G | 209 | A | PURB | Y94H | A | 293 | G |
| PAG1 | P46L | G | 848 | A | PHF2 | P500T | C | 1865 | A | POU3F2 | V365I | G | 1232 | A | PURB | R117C | G | 362 | A |
| PAG1 | G123W | C | 1078 | A | PHF2 | P700L | C | 2466 | T | POU3F2 | P97H | C | 429 | A | PURG | T201A | T | 1534 | C |
| PAGE2 | G15A | G | 148 | C | PHF2 | L402I | C | 1571 | A | POU3F2 | S106L | C | 456 | T | PUS1 | P254L | C | 1216 | T |
| PAGE5 | Q49R | A | 391 | G | PHF20 | T798A | A | 2521 | G | POU3F2 | I361V | A | 1220 | G | PUS1 | R382W | C | 1599 | T |
| PAGE5 | Q57* | C | 414 | T | PHF20 | R828C | C | 2611 | T | POU4F1 | K411* | T | 1465 | A | PUS10 | E118* | C | 614 | A |
| PAH | E7* | C | 492 | A | PHF20 | H130R | A | 518 | G | POU4F2 | A407T | G | 1467 | A | PUS3 | D455G | T | 1462 | C |
| PAICS | N297H | A | 1064 | C | PHF20 | K547N | G | 1770 | T | POU4F2 | R390C | C | 1416 | T | PUS3 | K99N | C | 395 | A |

| | | | | | | | | | | | | | | | | | | | |
|---|---|---|---|---|---|---|---|---|---|---|---|---|---|---|---|---|---|---|---|
| PAIP1 | L241M | A | 954 | T | PHF20 | K171Q | A | 640 | C | POU4F2 | R349H | G | 1294 | A | PUS7 | D567N | C | 1914 | T |
| PAK1 | R203W | G | 1140 | A | PHF20L1 | Q841* | C | 2820 | T | POU4F2 | L92P | T | 523 | C | PUS7L | A448T | C | 1422 | T |
| PAK2 | F389L | C | 1489 | A | PHF20L1 | P397S | C | 1488 | T | POU4F3 | E273K | G | 906 | A | PUS7L | E227D | T | 761 | G |
| PAK2 | P181L | C | 864 | T | PHF21A | P399L | G | 1820 | A | POU5F1B | K199T | A | 1155 | C | PUSL1 | G300R | G | 975 | A |
| PAK2 | G405R | G | 1535 | A | PHF21A | L255F | G | 1387 | A | POU5F1B | E215* | G | 1202 | T | PVR | G362R | G | 1297 | A |
| PAK2 | A122D | C | 687 | A | PHF21A | P661L | G | 2606 | A | POU6F2 | R494Q | G | 1523 | A | PVR | F365L | C | 1308 | A |
| PAK3 | A154T | G | 487 | A | PHF21B | R303W | G | 1058 | A | POU6F2 | Q441R | A | 1364 | G | PVRIG | T209I | C | 989 | T |
| PAK4 | T83M | C | 410 | T | PHF21B | P86L | G | 408 | A | POU6F2 | A211V | C | 674 | T | PVRL1 | A79V | G | 408 | A |
| PAK4 | R252Q | G | 917 | A | PHF21B | A200T | C | 749 | T | POU6F2 | N629S | A | 1928 | G | PVRL1 | S59N | C | 348 | T |
| PAK4 | A463T | G | 1549 | A | PHF3 | R202Q | G | 945 | A | PPA1 | V50A | A | 249 | G | PVRL1 | D106Y | C | 488 | A |
| PAK4 | D550Y | G | 1810 | T | PHF3 | R1755* | C | 5603 | T | PPA2 | H200Y | G | 607 | A | PVRL2 | G363R | G | 1477 | A |
| PAK6 | R58Q | G | 530 | A | PHF3 | S519N | G | 1896 | A | PPAN | R430H | G | 1461 | A | PVRL2 | A77V | C | 620 | T |
| PAK6 | A75V | C | 581 | T | PHF3 | L939V | C | 3155 | G | PPAN | A515S | G | 1715 | T | PVRL3 | L387F | G | 1436 | T |
| PAK6 | L64H | T | 548 | A | PHF5A | I9V | T | 97 | C | PPAN | R355Q | G | 1236 | A | PVRL3 | Y332C | A | 1270 | G |
| PAK7 | R488* | G | 2009 | A | PHF6 | E117D | G | 553 | T | PPAN | A235V | C | 876 | T | PVRL3 | V409A | T | 1501 | C |
| PAK7 | A398T | C | 1739 | T | PHF8 | L1015F | C | 3404 | G | PPAN-P2RY11 | M687V | A | 2059 | G | PVRL4 | E254D | T | 1065 | G |
| PAK7 | R499Q | C | 2043 | T | PHF8 | Q925H | T | 3134 | G | PPAN-P2RY11 | R727W | C | 2179 | T | PVRL4 | R144Q | C | 734 | T |
| PALB2 | R566H | C | 1897 | T | PHF8 | R809H | C | 2785 | T | PPAP2B | S244A | A | 1282 | C | PVRL4 | E337K | C | 1312 | T |
| PALB2 | P1008T | G | 3222 | T | PHGDH | R135W | C | 539 | T | PPAPDC1B | R179S | G | 556 | T | PVRL4 | T185M | G | 857 | A |
| PALB2 | I887V | T | 2859 | C | PHGDH | R247W | C | 875 | T | PPAPDC1B | D237V | T | 731 | A | PWP1 | G49S | G | 232 | A |
| PALB2 | D777N | C | 2529 | T | PHGDH | A2V | C | 141 | T | PPAPDC3 | A197V | C | 894 | T | PWP1 | V297A | T | 977 | C |
| PALLD | - | G | 0 | T | PHIP | P474L | G | 1589 | A | PPAPDC3 | L241I | C | 1025 | A | PWP2 | A79T | G | 357 | A |
| PALM | G275C | G | 1017 | T | PHIP | S846I | C | 2705 | A | PPARA | T4M | C | 276 | T | PWP2 | Y447H | T | 1461 | C |
| PALM2 | T359M | C | 1146 | T | PHIP | - | C | 0 | T | PPARD | R144C | C | 739 | T | PWP2 | L314R | T | 1063 | G |
| PALM2 | I315L | A | 1013 | C | PHIP | R396* | G | 1354 | A | PPARD | F145V | T | 742 | G | PWWP2A | A217T | C | 706 | T |
| PALM2-AKAP2 | R1077Q | G | 3410 | A | PHKA1 | R1073C | G | 3595 | A | PPARGC1A | A193V | G | 698 | A | PWWP2A | S408P | A | 1279 | G |
| PALM2-AKAP2 | A537T | G | 1789 | A | PHKA1 | S1050N | C | 3527 | T | PPARGC1A | K480T | T | 1559 | G | PWWP2B | F550L | T | 1709 | G |
| PALM3 | N539H | T | 1615 | G | PHKA1 | D66A | T | 575 | G | PPARGC1B | T884M | C | 2683 | T | PWWP2B | E483D | G | 1508 | T |
| PALM3 | G165V | C | 494 | A | PHKA2 | R186H | C | 1223 | T | PPAT | - | A | 0 | G | PWWP2B | R432* | A | 1353 | T |
| PALMD | Q389R | A | 1541 | G | PHKA2 | Y1085C | T | 3920 | C | PPDPF | S53F | C | 303 | T | PXDN | R1198W | G | 3643 | A |
| PAM | V89M | G | 638 | A | PHKA2 | L943V | A | 3493 | C | PPEF2 | E725D | C | 2532 | A | PXDN | A1292V | G | 3926 | A |
| PAM | V597M | G | 2162 | A | PHKB | N423S | A | 1292 | G | PPEF2 | D179Y | C | 892 | A | PXDN | R1198W | G | 3643 | A |
| PAM | G552E | G | 2028 | A | PHKB | I956N | T | 2891 | A | PPEF2 | E350K | C | 1405 | T | PXDN | A973V | G | 2969 | A |
| PAM | R931C | C | 3164 | T | PHKB | F330C | T | 1013 | G | PPEF2 | - | C | 0 | A | PXDN | C736R | A | 2257 | G |

| | | | | | | | | | | | | | | | | | | | |
|---|---|---|---|---|---|---|---|---|---|---|---|---|---|---|---|---|---|---|---|
| PAMR1 | L274F | G | 1266 | A | PHKG1 | E331D | C | 1188 | A | PPFIA1 | K1132N | A | 3594 | C | PXDN | V523F | C | 1618 | A |
| PAMR1 | T249M | G | 1192 | A | PHKG1 | R297W | G | 1084 | A | PPFIA2 | M205I | C | 918 | A | PXDN | Y500C | T | 1550 | C |
| PAMR1 | R514C | G | 1986 | A | PHKG2 | R185* | C | 792 | T | PPFIA2 | E584* | C | 2053 | A | PXDN | G1334D | C | 4052 | T |
| PAMR1 | R197H | C | 1036 | T | PHLDA1 | P48S | G | 177 | A | PPFIA2 | A580V | G | 2042 | A | PXDNL | A570T | C | 1809 | T |
| PAN2 | Q576H | C | 2101 | A | PHLDA3 | R115W | G | 741 | A | PPFIA2 | W1037C | C | 3414 | G | PXDNL | R712H | C | 2236 | T |
| PAN2 | V218A | A | 1026 | G | PHLDB1 | A855D | C | 2917 | A | PPFIA2 | S729N | C | 2489 | T | PXDNL | R905W | G | 2814 | A |
| PAN3 | R870H | G | 2761 | A | PHLDB1 | A241T | G | 1074 | A | PPFIA2 | L950F | G | 3151 | A | PXDNL | R675C | G | 2124 | A |
| PAN3 | A440T | G | 1470 | A | PHLDB1 | S263N | G | 1141 | A | PPFIA2 | L1090I | G | 3571 | T | PXDNL | A750T | C | 2349 | T |
| PAN3 | S291R | A | 1023 | C | PHLDB1 | - | G | 0 | A | PPFIA2 | E1017K | C | 3352 | T | PXDNL | S701N | C | 2203 | T |
| PAN3 | R606K | G | 1969 | A | PHLDB1 | R1316H | G | 4300 | A | PPFIA3 | P708A | C | 2454 | G | PXDNL | N99H | T | 396 | G |
| PAN3 | P794L | C | 2533 | T | PHLDB1 | R1285C | C | 4206 | T | PPFIA3 | T663I | C | 2320 | T | PXK | L248I | C | 851 | A |
| PAN3 | S615Y | C | 1996 | A | PHLDB2 | Q999H | G | 3408 | T | PPFIA3 | L643S | T | 2260 | C | PXK | L395V | T | 1292 | G |
| PAN3 | F662V | T | 2136 | G | PHLDB2 | D516A | A | 1958 | C | PPFIA3 | R234C | C | 1032 | T | PXMP4 | H47R | T | 233 | C |
| PAN3 | E878* | G | 2784 | T | PHLDB2 | R438G | A | 1723 | G | PPFIA4 | G1135D | G | 3931 | A | PYCR1 | H250R | T | 749 | C |
| PANK1 | K360T | T | 1235 | G | PHLDB2 | A199T | G | 1006 | A | PPFIA4 | A1142T | G | 3951 | A | PYCR1 | V126I | C | 376 | T |
| PANK2 | A15P | G | 49 | C | PHLDB2 | R438M | G | 1724 | T | PPFIA4 | T633M | C | 2425 | T | PYCR1 | R231H | C | 692 | T |
| PANK3 | - | A | 1423 | G | PHLDB2 | V473M | G | 1828 | A | PPFIA4 | S864P | T | 3117 | C | PYGB | D413G | A | 1348 | G |
| PANK4 | Q692* | G | 2074 | A | PHLDB2 | Y43* | C | 540 | A | PPFIA4 | R710W | C | 2655 | T | PYGL | R278Q | C | 946 | T |
| PANK4 | R453* | G | 1357 | A | PHLDB2 | R1172Q | G | 3926 | A | PPFIBP2 | - | T | 3017 | C | PYGL | R321H | C | 1075 | T |
| PANK4 | E218K | C | 652 | T | PHLDB3 | R558H | C | 2034 | T | PPFIBP2 | A618V | C | 2241 | T | PYGL | R387H | C | 1273 | T |
| PANK4 | L746M | G | 2236 | T | PHLDB3 | R512Q | C | 1896 | T | PPHLN1 | D362N | G | 1189 | A | PYGL | A329V | G | 1099 | A |
| PANK4 | A319T | C | 955 | T | PHLDB3 | R124H | C | 732 | T | PPHLN1 | R11I | G | 137 | T | PYGL | R243C | G | 840 | A |
| PANX1 | D261N | G | 1166 | A | PHLDB3 | R95* | G | 644 | A | PPHLN1 | T347A | A | 1144 | G | PYGL | F197L | G | 704 | T |
| PANX1 | V290M | G | 1253 | A | PHLDB3 | Q178R | T | 894 | C | PPIA | R144C | C | 475 | T | PYGL | Y114* | G | 455 | C |
| PANX1 | L421H | T | 1647 | A | PHLPP1 | A993T | G | 2977 | A | PPIAL4E | P70L | G | 242 | A | PYGM | R590C | G | 2167 | A |
| PANX2 | S400L | C | 1199 | T | PHLPP1 | G1177C | G | 3529 | T | PPIB | Y88C | T | 482 | C | PYGM | R576* | G | 2125 | A |
| PANX2 | R398H | G | 1193 | A | PHLPP1 | S1416G | A | 4246 | G | PPID | D303E | G | 1017 | C | PYGM | K597T | T | 2189 | G |
| PANX2 | A139T | G | 415 | A | PHLPP1 | R1297* | C | 3889 | T | PPIG | H59N | C | 395 | A | PYGO1 | T394A | T | 1275 | C |
| PANX2 | P440L | C | 1319 | T | PHLPP2 | A809V | G | 2432 | A | PPIG | I145N | T | 654 | A | PYGO1 | A3T | C | 102 | T |
| PANX2 | A260T | G | 778 | A | PHLPP2 | L337P | A | 1016 | G | PPIG | R342Q | G | 1245 | A | PYHIN1 | A217T | G | 894 | A |
| PANX3 | Y280D | T | 838 | G | PHLPP2 | F334L | A | 1006 | G | PPIG | D510E | T | 1750 | G | PYROXD1 | D64N | G | 317 | A |
| PAOX | A280V | C | 922 | T | PHLPP2 | L675P | A | 2030 | G | PPIH | D78E | T | 256 | G | PYROXD1 | R76C | C | 353 | T |
| PAOX | R424W | C | 1353 | T | PHLPP2 | M392V | T | 1180 | C | PPIL5 | S37F | C | 177 | T | PYROXD1 | Q143K | C | 554 | A |
| PAOX | V378F | G | 1215 | T | PHOSPHO2 | I3M | T | 397 | G | PPIL6 | S152F | G | 509 | A | PYROXD2 | A87V | G | 309 | A |
| PAOX | P305L | C | 997 | T | PHOSPHO2 | D15N | G | 431 | A | PPIP5K2 | R680Q | G | 2548 | A | PYROXD2 | K161N | T | 532 | G |
| PAOX | Y211H | T | 714 | C | PHOX2B | A166T | C | 856 | T | PPIP5K2 | R815C | C | 2952 | T | PYY | R122H | C | 689 | T |
| PAPD4 | F207C | T | 1038 | G | PHOX2B | P284L | G | 1211 | A | PPIP5K2 | E917* | G | 3258 | T | PZP | N1174K | G | 3551 | T |
| PAPD5 | V621D | T | 1862 | A | PHOX2B | D136G | T | 767 | C | PPL | R1135H | C | 3494 | T | PZP | R155C | G | 492 | A |

| | | | | | | | | | | | | | | | | | | | |
|---|---|---|---|---|---|---|---|---|---|---|---|---|---|---|---|---|---|---|---|
| PAPD5 | L693F | C | 2077 | T | PHPT1 | C43Y | G | 128 | A | PPL | A1040V | G | 3209 | A | PZP | V1011I | C | 3060 | T |
| PAPD7 | K118N | G | 483 | T | PHRF1 | G1649D | G | 5077 | A | PPL | R1047Q | C | 3230 | T | PZP | G890E | C | 2698 | T |
| PAPD7 | T109M | C | 455 | T | PHRF1 | L422P | T | 1396 | C | PPL | R505W | G | 1603 | A | PZP | V206A | A | 646 | G |
| PAPD7 | D313N | G | 1066 | A | PHRF1 | P1289A | C | 3996 | G | PPL | A89V | G | 356 | A | PZP | R157Q | C | 499 | T |
| PAPD7 | P431L | C | 1421 | T | PHTF1 | S748R | T | 2694 | G | PPL | R354W | G | 1150 | A | QDPR | S163N | C | 668 | T |
| PAPLN | W32* | G | 198 | A | PHTF1 | R326M | C | 1429 | A | PPL | V1643M | C | 5017 | T | QDPR | G151S | C | 631 | T |
| PAPLN | E142K | G | 526 | A | PHTF1 | R414H | C | 1693 | T | PPL | R1597W | G | 4879 | A | QDPR | E206* | C | 796 | A |
| PAPLN | G647R | G | 2041 | A | PHTF1 | V612A | A | 2287 | G | PPL | R905Q | C | 2804 | T | QKI | E77* | G | 762 | T |
| PAPLN | S571A | T | 1813 | G | PHTF1 | E681* | C | 2493 | A | PPL | R1463* | G | 4477 | A | QKI | D61E | C | 716 | G |
| PAPLN | P560L | C | 1781 | T | PHTF1 | K309T | T | 1378 | G | PPL | Q1249H | C | 3837 | A | QKI | K126N | A | 911 | C |
| PAPLN | Y907C | A | 2822 | G | PHTF2 | R202I | G | 820 | T | PPL | E1075D | C | 3315 | A | QRFPR | R32* | G | 506 | A |
| PAPOLA | T611M | C | 2044 | T | PHTF2 | L681I | C | 2256 | A | PPM1A | I152T | T | 551 | C | QRFPR | V291A | A | 1284 | G |
| PAPOLB | V247I | C | 926 | T | PHTF2 | G171E | G | 727 | A | PPM1B | S327Y | C | 1392 | A | QRICH1 | R536* | G | 2079 | A |
| PAPOLB | T617A | T | 2036 | C | PHYHD1 | R78* | C | 652 | T | PPM1B | D114Y | G | 752 | T | QRICH1 | S472Y | G | 1888 | T |
| PAPOLB | D35E | G | 292 | T | PHYHIP | A240V | G | 1291 | A | PPM1D | N512K | T | 1758 | A | QRICH1 | S120P | A | 831 | G |
| PAPOLB | A608T | C | 2009 | T | PHYHIPL | R5H | G | 278 | A | PPM1D | R441H | G | 1544 | A | QRICH1 | T51A | T | 624 | C |
| PAPOLG | T222A | A | 913 | G | PI15 | S181F | C | 721 | T | PPM1D | V190I | G | 790 | A | QRICH2 | R1568H | C | 4883 | T |
| PAPPA2 | R901Q | G | 3866 | A | PI3 | L80S | T | 286 | C | PPM1E | R294H | G | 1008 | A | QRICH2 | R432H | C | 1475 | T |
| PAPPA2 | L577M | C | 2893 | A | PI3 | D95Y | G | 330 | T | PPM1E | R316W | C | 1073 | T | QRICH2 | R673H | C | 2198 | T |
| PAPPA2 | S1018L | C | 4217 | T | PI4K2A | A88V | C | 320 | T | PPM1E | A70E | C | 336 | A | QRICH2 | R1606W | G | 4996 | A |
| PAPPA2 | G854R | G | 3724 | A | PI4KA | V1017I | C | 3310 | T | PPM1G | D135N | C | 668 | T | QRICH2 | G95R | C | 463 | T |
| PAPPA2 | E1647A | A | 6104 | C | PI4KA | S495L | G | 1745 | A | PPM1H | R477C | G | 1843 | A | QRICH2 | F934L | G | 2982 | T |
| PAPPA2 | R526H | G | 2741 | A | PI4KA | A363T | C | 1348 | T | PPM1H | E415D | C | 1659 | A | QRSL1 | Y40C | A | 223 | G |
| PAPPA2 | E1693A | A | 6242 | C | PI4KA | R134* | G | 661 | A | PPM1J | R175C | G | 699 | A | QSER1 | Q106H | G | 585 | T |
| PAPPA2 | G1290R | G | 5032 | A | PI4KA | D739V | T | 2477 | A | PPM1K | A325V | G | 1364 | A | QSER1 | A330V | C | 1256 | T |
| PAPPA2 | R784Q | G | 3515 | A | PI4KA | D2041Y | C | 6382 | A | PPM1K | R33W | G | 487 | A | QSER1 | R1399* | C | 4462 | T |
| PAPPA2 | K1258T | A | 4937 | C | PI4KB | I324L | T | 1330 | G | PPM1M | R118* | C | 717 | T | QSOX1 | W522C | G | 1640 | T |
| PAPPA2 | P1679Q | C | 6200 | A | PIAS1 | E273D | A | 912 | T | PPM1N | E149K | G | 445 | A | QSOX1 | F413V | T | 1311 | G |
| PAPPAS | R408C | C | 1591 | T | PIAS2 | S333G | T | 1155 | C | PPME1 | L79P | T | 335 | C | QSOX2 | T171M | G | 550 | A |
| PAPPAS | P679S | C | 2404 | T | PIAS2 | S543N | C | 1786 | T | PPME1 | E75D | G | 324 | T | QSOX2 | A649T | C | 1983 | T |
| PAPPAS | S549F | C | 2015 | T | PIAS3 | A587T | G | 1849 | A | PPOX | S35N | G | 314 | A | QTRT1 | - | T | 0 | C |
| PAPPAS | E705* | G | 2482 | T | PIAS3 | Q153P | A | 548 | C | PPOX | G358R | G | 1282 | A | QTRT1 | N97I | A | 315 | T |
| PAPPAS | G829S | G | 2854 | A | PIAS4 | D478E | T | 1445 | G | PPP1CA | R240H | C | 719 | T | QTRTD1 | P256L | C | 882 | T |
| PAPPAS | D961N | G | 3250 | A | PIBF1 | R230* | C | 1026 | T | PPP1CC | R187W | G | 804 | A | R3HCC1 | E243A | A | 728 | C |
| PAPPAS | L1058I | C | 3541 | A | PIBF1 | I5M | T | 353 | G | PPP1R12A | T322M | G | 1232 | A | R3HDM2 | A635V | G | 2201 | A |
| PAPPAS | D1610N | G | 5197 | A | PIBF1 | R396* | C | 1524 | T | PPP1R12A | A1020V | G | 3326 | A | R3HDM2 | R1025* | G | 3370 | A |
| PAPSS1 | Q144K | G | 703 | T | PICK1 | A306T | G | 1263 | A | PPP1R12A | T212M | G | 902 | A | R3HDM2 | R1014C | G | 3337 | A |
| PAPSS1 | R333H | C | 1271 | T | PICK1 | R223Q | G | 1015 | A | PPP1R12B | R279Q | G | 980 | A | RAB10 | R130I | G | 888 | T |
| PAPSS1 | T129A | T | 658 | C | PICK1 | V338L | G | 1359 | T | PPP1R12B | R476Q | G | 1571 | A | RAB11A | L42I | C | 124 | A |

| | | | | | | | | | | | | | | | | | | | |
|---|---|---|---|---|---|---|---|---|---|---|---|---|---|---|---|---|---|---|---|
| PAPSS1 | N16H | T | 319 | G | PIF1 | Q206H | C | 713 | A | PPP1R12C | Y146H | A | 452 | G | RAB11A | A24T | G | 70 | A |
| PAPSS2 | R416H | G | 1510 | A | PIGC | V203M | C | 888 | T | PPP1R12C | A217T | C | 665 | T | RAB11B | R30C | C | 184 | T |
| PAQR4 | G122V | G | 795 | T | PIGC | R109W | G | 606 | A | PPP1R13B | V415M | C | 1526 | T | RAB11B | R132Q | G | 491 | A |
| PAQR6 | W227* | C | 681 | T | PIGC | R271H | C | 1093 | T | PPP1R13B | A558V | G | 1956 | A | RAB11FIP1 | Q696H | C | 2132 | A |
| PAQR7 | R15W | G | 710 | A | PIGG | G636V | G | 2013 | T | PPP1R13B | R155H | C | 747 | T | RAB11FIP1 | A882V | G | 2689 | A |
| PAQR7 | A182T | C | 1211 | T | PIGH | L91F | T | 370 | G | PPP1R13B | P367S | G | 1382 | A | RAB11FIP1 | V508G | A | 1567 | C |
| PAQR9 | A126V | G | 377 | A | PIGK | E259* | C | 799 | A | PPP1R13B | R29* | G | 368 | A | RAB11FIP1 | - | C | 0 | A |
| PAQR9 | R334H | C | 1001 | T | PIGK | R287H | C | 884 | T | PPP1R13B | R314C | G | 1223 | A | RAB11FIP2 | E348* | C | 1482 | A |
| PARD3 | R142H | C | 751 | T | PIGK | V243A | A | 752 | G | PPP1R13L | T491M | G | 1551 | A | RAB11FIP2 | R129Q | C | 826 | T |
| PARD3 | R349C | G | 1371 | A | PIGN | I687T | A | 2431 | G | PPP1R13L | A149T | C | 524 | T | RAB11FIP4 | R460C | C | 1607 | T |
| PARD3 | A550T | C | 1974 | T | PIGN | L553M | G | 2028 | T | PPP1R13L | A236T | C | 785 | T | RAB11FIP4 | V344A | T | 1260 | C |
| PARD3 | A928T | C | 3108 | T | PIGN | K471Q | T | 1782 | G | PPP1R13L | R144H | C | 510 | T | RAB11FIP4 | E85D | G | 484 | T |
| PARD3B | D790Y | G | 2575 | T | PIGO | H947N | G | 3234 | T | PPP1R13L | S120P | A | 437 | G | RAB11FIP5 | S214N | C | 882 | T |
| PARD3B | A537T | G | 1816 | A | PIGO | G785D | C | 2749 | T | PPP1R14D | A193V | G | 646 | A | RAB11FIP5 | G577D | C | 1971 | T |
| PARD3B | S675R | A | 2230 | C | PIGO | R999W | G | 3390 | A | PPP1R15A | E411K | G | 1500 | A | RAB11FIP5 | S361L | G | 1323 | A |
| PARD3B | E52* | G | 361 | T | PIGP | V56M | C | 390 | T | PPP1R15A | A593T | G | 2046 | A | RAB12 | K207R | A | 903 | G |
| PARD6A | R338* | C | 1092 | T | PIGR | D136Y | C | 590 | A | PPP1R15A | T379I | C | 1405 | T | RAB12 | R181Q | G | 825 | A |
| PARD6B | R34K | G | 344 | A | PIGR | R272Q | C | 999 | T | PPP1R15A | E172D | G | 785 | T | RAB12 | T161M | C | 765 | T |
| PARD6B | T261A | A | 1024 | G | PIGR | V453M | C | 1541 | T | PPP1R15B | G516D | C | 1927 | T | RAB14 | T157M | G | 708 | A |
| PARD6G | A238T | C | 910 | T | PIGR | K230T | T | 873 | G | PPP1R15B | R586C | G | 2136 | A | RAB17 | A2T | C | 634 | T |
| PARG | D90N | C | 268 | T | PIGS | - | C | 0 | T | PPP1R15B | I496V | T | 1866 | C | RAB18 | R205G | A | 611 | G |
| PARK2 | K129N | C | 490 | A | PIGU | F314C | A | 941 | C | PPP1R16A | Q364H | G | 2001 | T | RAB21 | K111Q | A | 583 | C |
| PARK2 | A398T | C | 1295 | T | PIGU | K104T | T | 311 | G | PPP1R16A | R55W | C | 1072 | T | RAB21 | M180T | T | 791 | C |
| PARK2 | H461R | T | 1485 | C | PIGU | K104N | T | 312 | G | PPP1R16B | A518T | G | 1741 | A | RAB22A | E52* | G | 435 | T |
| PARK2 | F209L | G | 730 | T | PIGV | T22A | A | 395 | G | PPP1R16B | V5M | G | 202 | A | RAB23 | G203S | C | 1227 | T |
| PARK7 | R156Q | G | 621 | A | PIGV | A280V | C | 1170 | T | PPP1R16B | A218V | C | 842 | T | RAB23 | Q27R | T | 700 | C |
| PARK7 | F164C | T | 645 | G | PIGV | L406V | T | 1547 | G | PPP1R16B | R49* | C | 334 | T | RAB24 | R98Q | C | 625 | T |
| PARL | V287I | C | 920 | T | PIGV | E441K | G | 1652 | A | PPP1R16B | A230S | G | 877 | T | RAB26 | R125Q | G | 534 | A |
| PARN | E260* | C | 920 | A | PIGW | Q358R | A | 1128 | G | PPP1R16B | E296V | A | 1076 | T | RAB28 | E35* | C | 318 | A |
| PARP1 | V315I | C | 1087 | T | PIGX | E112D | A | 458 | C | PPP1R1C | - | T | 0 | C | RAB2A | Y3C | A | 216 | G |
| PARP1 | T632M | G | 2039 | A | PIGZ | Y458C | T | 1520 | C | PPP1R1C | E104* | G | 544 | T | RAB2B | D177N | C | 630 | T |
| PARP10 | L577M | G | 1837 | T | PIGZ | W48* | C | 291 | T | PPP1R3A | K532N | T | 1665 | G | RAB31 | R165C | C | 668 | T |
| PARP11 | F82I | A | 389 | T | PIGZ | G14E | C | 188 | T | PPP1R3A | G554V | C | 1730 | A | RAB31 | F160C | T | 654 | G |
| PARP12 | V685I | C | 2927 | T | PIH1D1 | A146T | C | 672 | T | PPP1R3A | T948M | G | 2912 | A | RAB32 | S222P | T | 843 | C |
| PARP14 | D289N | G | 1131 | A | PIH1D2 | L176V | A | 749 | C | PPP1R3A | I1076T | A | 3296 | G | RAB33A | S15L | C | 458 | T |
| PARP14 | W410C | G | 1496 | T | PIH1D2 | V90I | C | 491 | T | PPP1R3A | S1074Y | G | 3290 | T | RAB34 | R37H | C | 111 | T |
| PARP14 | K1663N | G | 5255 | T | PIK3AP1 | C66Y | C | 317 | T | PPP1R3A | T984A | T | 3019 | C | RAB34 | P230T | G | 689 | T |
| PARP15 | K462T | A | 1451 | C | PIK3AP1 | V180A | A | 659 | G | PPP1R3A | S947Y | G | 2909 | T | RAB34 | A13V | G | 39 | A |
| PARP15 | N666T | A | 2063 | C | PIK3C2A | H761R | T | 2348 | C | PPP1R3A | R521I | C | 1631 | A | RAB34 | K238N | T | 715 | G |

| | | | | | | | | | | | | | | | | | | | |
|---|---|---|---|---|---|---|---|---|---|---|---|---|---|---|---|---|---|---|---|
| PARP16 | F138C | A | 859 | C | PIK3C2A | T1261M | G | 3848 | A | PPP1R3C | S192Y | G | 660 | T | RAB36 | L99P | T | 336 | C |
| PARP2 | Q576R | A | 1755 | G | PIK3C2A | L1396I | G | 4252 | T | PPP1R3C | R167Q | C | 585 | T | RAB39 | R119W | C | 373 | T |
| PARP2 | K520E | A | 1586 | G | PIK3C2A | V116A | A | 413 | G | PPP1R3C | D103N | C | 392 | T | RAB39B | F46L | G | 439 | T |
| PARP3 | T314M | C | 1272 | T | PIK3C2A | R1026Q | C | 3143 | T | PPP1R3C | A97T | C | 374 | T | RAB3A | T36M | G | 241 | A |
| PARP4 | H1455R | T | 4470 | C | PIK3C2B | R616C | G | 2403 | A | PPP1R3D | R171C | G | 877 | A | RAB3D | A154T | C | 698 | T |
| PARP4 | F1585L | G | 4861 | C | PIK3C2B | L1352I | G | 4611 | T | PPP1R3F | V385I | G | 1169 | A | RAB3D | A50T | C | 386 | T |
| PARP4 | S1434F | G | 4407 | A | PIK3C2G | R258I | G | 889 | T | PPP1R3G | A6V | C | 17 | T | RAB3GAP1 | V193A | T | 588 | C |
| PARP4 | K594N | C | 1888 | A | PIK3C2G | R738C | C | 2328 | T | PPP1R3G | N342D | A | 1024 | G | RAB3GAP1 | E49K | G | 155 | A |
| PARP4 | R1010H | C | 3135 | T | PIK3C2G | D1133Y | G | 3513 | T | PPP1R7 | V350M | G | 1356 | A | RAB3GAP2 | E127K | C | 496 | T |
| PARP4 | R1130Q | C | 3495 | T | PIK3C2G | I1337V | A | 4125 | G | PPP1R7 | Q10* | C | 336 | T | RAB3GAP2 | V1134M | C | 3517 | T |
| PARP6 | D588N | C | 2220 | T | PIK3C2G | L976V | C | 3042 | G | PPP1R8 | D87E | T | 319 | G | RAB3GAP2 | V1021I | C | 3178 | T |
| PARP8 | M354T | T | 1243 | C | PIK3C2G | F1081C | T | 3358 | G | PPP1R8 | R176Q | G | 585 | A | RAB3GAP2 | W820L | C | 2576 | A |
| PARP8 | R287C | C | 1041 | T | PIK3C2G | R643I | G | 2044 | T | PPP1R9A | A217T | G | 931 | A | RAB3GAP2 | W876C | C | 2745 | A |
| PARP9 | T42A | T | 352 | C | PIK3C3 | R552Q | G | 1713 | A | PPP1R9A | E104* | G | 592 | T | RAB3GAP2 | Y719C | T | 2273 | C |
| PARP9 | D2E | G | 234 | T | PIK3C3 | F250C | T | 807 | G | PPP1R9A | M71I | G | 495 | A | RAB3GAP2 | A1130V | G | 3506 | A |
| PARS2 | R346Q | C | 1120 | T | PIK3C3 | N436H | A | 1364 | C | PPP1R9A | Y439H | T | 1597 | C | RAB3IL1 | M261I | C | 783 | T |
| PARS2 | R195C | G | 666 | A | PIK3CA | K111N | G | 490 | T | PPP1R9A | L252M | C | 1036 | A | RAB3IL1 | E42D | C | 126 | A |
| PARS2 | W134L | C | 484 | A | PIK3CA | H1047R | A | 3297 | G | PPP1R9A | R194Q | G | 863 | A | RAB3IP | R176Q | G | 966 | A |
| PARVA | R206* | C | 665 | T | PIK3CA | K111E | A | 488 | G | PPP2CB | K283N | T | 1028 | G | RAB3IP | D20Y | G | 497 | T |
| PARVB | G318D | G | 1083 | A | PIK3CA | H1047R | A | 3297 | G | PPP2R1A | R134W | C | 458 | T | RAB40A | R99H | C | 2415 | T |
| PARVB | R58I | G | 303 | T | PIK3CA | C604R | T | 1967 | C | PPP2R2A | R262Q | G | 785 | A | RAB40C | G251S | G | 954 | A |
| PARVG | D199Y | G | 1019 | T | PIK3CA | E545K | G | 1790 | A | PPP2R2B | N376S | T | 1416 | C | RAB40C | G251S | G | 954 | A |
| PASD1 | Q399* | C | 1440 | T | PIK3CA | E545K | G | 1790 | A | PPP2R2B | A496V | G | 1776 | A | RAB40C | G251S | G | 954 | A |
| PASD1 | N22Y | A | 309 | T | PIK3CA | R88Q | G | 420 | A | PPP2R2B | R82H | C | 534 | T | RAB41 | A9T | G | 71 | A |
| PASD1 | V650M | G | 2193 | A | PIK3CA | E545A | A | 1791 | C | PPP2R2B | E201K | C | 890 | T | RAB43P1 | A119S | C | 355 | A |
| PASK | R880H | C | 2703 | T | PIK3CA | H1047R | A | 3297 | G | PPP2R2C | V239I | C | 739 | T | RAB43P1 | D23Y | C | 67 | A |
| PASK | S38N | C | 177 | T | PIK3CA | E545K | G | 1790 | A | PPP2R2C | A345V | G | 1058 | A | RAB4A | R207H | G | 828 | A |
| PASK | L619S | A | 1920 | G | PIK3CA | K986N | G | 3115 | T | PPP2R2D | A407V | C | 1220 | T | RAB4A | F16L | C | 256 | A |
| PASK | D653N | C | 2021 | T | PIK3CA | T1025A | A | 3230 | G | PPP2R2D | R232H | G | 695 | A | RAB4B | I38T | T | 113 | C |
| PATE1 | A69T | G | 205 | A | PIK3CA | Q546K | C | 1793 | A | PPP2R2D | R128W | C | 382 | T | RAB5A | N113S | A | 874 | G |
| PATE1 | E44* | G | 130 | T | PIK3CA | E542K | G | 1781 | A | PPP2R2D | - | G | 0 | T | RAB6B | V111I | C | 681 | T |
| PATE3 | R31Q | G | 133 | A | PIK3CA | K111E | A | 488 | G | PPP2R2D | R119* | C | 355 | T | RAB6B | E142K | C | 774 | T |
| PATE4 | G39D | G | 160 | A | PIK3CA | A511T | G | 1688 | A | PPP2R3A | S148P | T | 1059 | C | RAB6B | R168* | G | 852 | A |
| PATL1 | A734V | G | 2344 | A | PIK3CA | C420R | T | 1415 | C | PPP2R4 | R134W | C | 592 | T | RAB7A | A76V | C | 473 | T |
| PATL1 | R304Q | C | 1054 | T | PIK3CA | E545G | A | 1791 | G | PPP2R4 | A192T | G | 766 | A | RAB8A | T49A | A | 367 | G |
| PATL2 | R212H | C | 733 | T | PIK3CA | E545K | G | 1790 | A | PPP2R5A | E332* | G | 1568 | T | RAB8B | F201L | C | 759 | A |
| PATL2 | R157W | G | 567 | A | PIK3CA | H1047R | A | 3297 | G | PPP2R5A | Q43E | C | 701 | G | RAB8B | K100T | A | 455 | C |
| PATL2 | K200R | T | 697 | C | PIK3CA | H419N | C | 1412 | A | PPP2R5A | L285F | A | 1429 | C | RAB8B | W102L | G | 461 | T |
| PAX1 | G166R | G | 550 | A | PIK3CA | T1052K | C | 3312 | A | PPP2R5B | R468W | C | 1987 | T | RAB8B | F147L | C | 597 | A |

| | | | | | | | | | | | | | | | | | | | |
|---|---|---|---|---|---|---|---|---|---|---|---|---|---|---|---|---|---|---|---|
| PAX1 | G163R | G | 541 | C | PIK3CA | K594E | A | 1937 | G | PPP2R5E | A471V | G | 1801 | A | RABEP1 | R75Q | G | 448 | A |
| PAX2 | R399H | G | 1746 | A | PIK3CA | H1047L | A | 3297 | T | PPP2R5E | S69* | G | 595 | C | RABEP1 | V793I | G | 2601 | A |
| PAX2 | R270C | C | 1358 | T | PIK3CA | T1025A | A | 3230 | G | PPP2R5E | K504N | C | 1901 | A | RABEP1 | E258* | G | 996 | T |
| PAX2 | R203C | C | 1157 | T | PIK3CB | A439V | G | 1390 | A | PPP2R5E | E408* | C | 1611 | A | RABEP2 | V529M | C | 2174 | T |
| PAX3 | P462L | G | 1751 | A | PIK3CB | R1065Q | C | 3268 | T | PPP2R5E | E222A | T | 1054 | G | RABEP2 | E407G | T | 1809 | C |
| PAX3 | R156H | C | 833 | T | PIK3CB | K412T | T | 1309 | G | PPP3CA | H151N | G | 1135 | T | RABGGTA | L537I | G | 2031 | T |
| PAX3 | E132D | T | 762 | G | PIK3CB | R321Q | C | 1036 | T | PPP3CB | R411* | G | 1366 | A | RABGGTA | - | C | 0 | T |
| PAX4 | P119L | G | 356 | A | PIK3CD | R38H | G | 228 | A | PPP3CC | R144W | C | 757 | T | RABGGTA | R134H | C | 823 | T |
| PAX5 | R122Q | C | 440 | T | PIK3CD | Q170* | C | 623 | T | PPP3CC | - | T | 0 | C | RABGGTB | R222* | C | 735 | T |
| PAX5 | V37A | A | 185 | G | PIK3CD | R88C | C | 377 | T | PPP3CC | E359* | G | 1402 | T | RABGGTB | M175V | A | 594 | G |
| PAX5 | Y7H | A | 94 | G | PIK3CD | N942K | T | 2941 | G | PPP3R2 | D68N | C | 273 | T | RABGGTB | D284Y | G | 921 | T |
| PAX6 | - | C | 0 | T | PIK3CG | D819N | G | 2540 | A | PPP4R1 | R308C | G | 996 | A | RABIF | G62S | C | 221 | T |
| PAX6 | A153T | C | 926 | T | PIK3CG | P262S | C | 869 | T | PPP4R1 | R230Q | C | 763 | T | RABL3 | R107C | G | 349 | A |
| PAX7 | S155L | C | 1062 | T | PIK3CG | L79M | C | 320 | A | PPP4R1 | V438I | C | 1386 | T | RABL3 | N138S | T | 443 | C |
| PAX7 | P408S | C | 1820 | T | PIK3CG | S257Y | C | 855 | A | PPP4R1 | S411P | A | 1305 | G | RABL3 | L161V | A | 511 | C |
| PAX7 | K139N | G | 1015 | T | PIK3CG | D946N | G | 2921 | A | PPP4R1 | E544D | C | 1706 | A | RAC1 | R68H | G | 400 | A |
| PAX7 | R260C | C | 1376 | T | PIK3R1 | A690V | C | 2111 | T | PPP4R2 | R149Q | G | 699 | A | RAC2 | V36A | A | 229 | G |
| PAX7 | K195N | A | 1183 | C | PIK3R1 | R348* | C | 1084 | T | PPP4R4 | N719S | A | 2310 | G | RACGAP1P | R104* | G | 511 | A |
| PAX8 | H254R | T | 927 | C | PIK3R1 | R574I | G | 1763 | T | PPP4R4 | E43* | G | 281 | T | RAD1 | R109Q | C | 1746 | T |
| PAX9 | D200N | G | 1315 | A | PIK3R1 | R543I | G | 1670 | T | PPP4R4 | L158M | C | 626 | A | RAD17 | S628Y | C | 2561 | A |
| PAXIP1 | T916M | G | 2902 | A | PIK3R1 | R657Q | G | 2012 | A | PPP4R4 | I309S | T | 1080 | G | RAD18 | E227K | C | 796 | T |
| PAXIP1 | C648Y | C | 2098 | T | PIK3R1 | R162* | C | 526 | T | PPP5C | N303D | A | 967 | G | RAD18 | E140* | C | 535 | A |
| PAXIP1 | R483H | C | 1603 | T | PIK3R1 | E674K | G | 2062 | A | PPP5C | D242E | C | 786 | A | RAD18 | N286S | T | 974 | C |
| PAXIP1 | F874L | G | 2777 | T | PIK3R1 | R650* | C | 1990 | T | PPP5C | E255K | G | 823 | A | RAD18 | E280* | C | 955 | A |
| PBK | M283V | T | 1215 | C | PIK3R1 | N285H | A | 895 | C | PPP5C | E428K | G | 1342 | A | RAD18 | Q273P | T | 935 | G |
| PBRM1 | - | C | 0 | T | PIK3R1 | N527K | T | 1623 | G | PPP6C | F228L | A | 903 | G | RAD21 | W18C | C | 342 | G |
| PBRM1 | E1060G | T | 3181 | C | PIK3R1 | N564D | A | 1732 | G | PPPDE1 | T79M | C | 236 | T | RAD21 | I44V | T | 418 | C |
| PBX1 | T88A | A | 450 | G | PIK3R2 | V579M | G | 2335 | A | PPRC1 | K538E | A | 1651 | G | RAD21L1 | T364I | C | 1184 | T |
| PBX3 | R311Q | G | 1012 | A | PIK3R2 | R345Q | G | 1634 | A | PPRC1 | R1400Q | G | 4238 | A | RAD21L1 | R54Q | G | 254 | A |
| PBXIP1 | R583W | G | 1819 | A | PIK3R2 | P20L | C | 659 | T | PPRC1 | L317M | C | 988 | A | RAD21L1 | I355M | T | 1158 | G |
| PC | E932K | C | 2843 | T | PIK3R3 | R368K | C | 1103 | T | PPRC1 | S1360N | G | 4118 | A | RAD23A | K69E | A | 314 | G |
| PC | A1010V | G | 3078 | A | PIK3R3 | K339T | T | 1016 | G | PPRC1 | T739A | A | 2254 | G | RAD23B | N192I | A | 926 | T |
| PC | Y722C | T | 2214 | C | PIK3R4 | V676M | C | 2584 | T | PPRC1 | I1007V | A | 3058 | G | RAD23B | G377R | G | 1480 | A |
| PCBP1 | L100Q | T | 590 | A | PIK3R4 | R107* | G | 877 | A | PPTC7 | P202L | G | 833 | A | RAD23B | E61* | G | 532 | T |
| PCBP1 | L100Q | T | 590 | A | PIK3R5 | A701T | C | 2168 | T | PPYR1 | A99T | G | 714 | A | RAD23B | S252Y | C | 1106 | A |
| PCBP2 | G30R | G | 438 | C | PIKFYVE | A434V | C | 1459 | T | PQBP1 | A6V | C | 217 | T | RAD50 | E977* | G | 3316 | T |
| PCBP3 | P96T | C | 624 | A | PIKFYVE | W1464* | G | 4550 | A | PQLC1 | V263M | C | 970 | T | RAD50 | I1109T | T | 3713 | C |
| PCBP3 | L280M | C | 1176 | A | PIKFYVE | D279Y | G | 993 | T | PQLC2 | A166V | C | 769 | T | RAD50 | R1214C | C | 4027 | T |
| PCBP4 | A159V | G | 476 | A | PIKFYVE | R1038Q | G | 3271 | A | PQLC2 | - | A | 0 | G | RAD50 | A1246V | C | 4124 | T |

| | | | | | | | | | | | | | | | | | | | |
|---|---|---|---|---|---|---|---|---|---|---|---|---|---|---|---|---|---|---|---|
| PCCA | K181T | A | 580 | C | PIKFYVE | Q1183H | A | 3707 | C | PQLC2 | R124C | C | 642 | T | RAD50 | R1200Q | G | 3986 | A |
| PCCB | P338H | C | 1034 | A | PIKFYVE | R1284H | G | 4009 | A | PRAC | S19I | C | 186 | A | RAD51AP1 | S272Y | C | 971 | A |
| PCDH1 | E288K | C | 1010 | T | PIKFYVE | I1310V | A | 4086 | G | PRAME | H464Y | G | 1690 | A | RAD51AP2 | I99L | T | 319 | G |
| PCDH1 | R789H | C | 2514 | T | PIKFYVE | N1635T | A | 5062 | C | PRAME | Q283* | G | 1147 | A | RAD51AP2 | S982G | T | 2968 | C |
| PCDH1 | K581N | C | 1891 | A | PILRA | R228W | C | 894 | T | PRAME | L267F | G | 1099 | A | RAD51C | R319Q | G | 1027 | A |
| PCDH1 | R797C | G | 2537 | A | PIM2 | - | A | 1124 | G | PRAMEF1 | H48Q | C | 247 | G | RAD51L1 | P326T | C | 1053 | A |
| PCDH1 | R695H | C | 2232 | T | PIN4 | G58C | G | 204 | T | PRAMEF1 2 | R319C | C | 982 | T | RAD51L1 | G203R | G | 684 | A |
| PCDH1 | R1233H | C | 3846 | T | PION | A429V | G | 1365 | A | PRAMEF2 2 | C221Y | G | 662 | A | RAD51L1 | E198* | G | 669 | T |
| PCDH1 | L248I | G | 890 | T | PION | E207D | C | 700 | A | PRAP1 | L100M | C | 376 | A | RAD54B | E33D | C | 179 | A |
| PCDH10 | E82K | G | 1070 | A | PIP4K2A | - | C | 0 | T | PRB2 | S173C | G | 554 | C | RAD54B | E469D | T | 1487 | G |
| PCDH10 | G611C | G | 2657 | T | PIP4K2C | R357Q | G | 1070 | A | PRC1 | R568H | C | 1860 | T | RAD54L | - | G | 0 | A |
| PCDH10 | P305L | C | 1740 | T | PIP4K2C | L47R | T | 140 | G | PRC1 | F428V | A | 1439 | C | RAD54L | - | T | 0 | C |
| PCDH10 | E625K | G | 2699 | A | PIP4K2C | - | T | 0 | C | PRCC | R452Q | G | 1627 | A | RAD54L | L490M | C | 1582 | A |
| PCDH11X | L424F | C | 2115 | T | PIP5K1A | A303T | G | 1329 | A | PRCD | A16T | G | 477 | A | RAD54L | R716Q | G | 2261 | A |
| PCDH11X | V17M | G | 894 | A | PIP5K1C | T512M | G | 1592 | A | PRCP | D508Y | C | 1635 | A | RAD54L2 | R467H | G | 1525 | A |
| PCDH11X | H961N | C | 3726 | A | PIP5KL1 | S377G | T | 1174 | C | PRDM1 | A315T | G | 1177 | A | RAD54L2 | V1299I | G | 4020 | A |
| PCDH12 | R929C | G | 3996 | A | PIP5KL1 | R288C | G | 907 | A | PRDM10 | Q1035* | G | 3335 | A | RADIL | T1072M | G | 3403 | A |
| PCDH12 | R470W | G | 2619 | A | PIPOX | R378Q | G | 1459 | A | PRDM10 | R854* | G | 2792 | A | RADIL | R272W | G | 1002 | A |
| PCDH12 | C1183S | A | 4758 | T | PIPOX | R66Q | G | 523 | A | PRDM11 | R450C | C | 1597 | T | RADIL | A929V | G | 2974 | A |
| PCDH12 | V232A | A | 1906 | G | PIPOX | S365I | G | 1420 | T | PRDM11 | A143V | C | 677 | T | RADIL | E166D | C | 686 | A |
| PCDH12 | R59W | G | 1386 | A | PISD | R178Q | C | 757 | T | PRDM11 | K3N | G | 258 | T | RADIL | G108S | C | 510 | T |
| PCDH12 | N221Y | T | 1872 | A | PISD | T324A | T | 1194 | C | PRDM12 | A86V | C | 317 | T | RADIL | E759K | C | 2463 | T |
| PCDH12 | L477I | G | 2640 | T | PITPNA | F224L | G | 928 | T | PRDM13 | E531D | G | 1854 | T | RADIL | G313E | C | 1126 | T |
| PCDH12 | L20I | G | 1269 | T | PITPNA | V198I | C | 848 | T | PRDM13 | C616Y | G | 2108 | A | RAE1 | Q271R | A | 1232 | G |
| PCDH15 | R1468H | C | 4798 | T | PITPNB | R170G | T | 572 | C | PRDM13 | A191T | G | 832 | A | RAE1 | V130I | G | 808 | A |
| PCDH15 | I1501F | T | 4896 | A | PITPNC1 | R157W | C | 843 | T | PRDM14 | R505H | C | 1716 | T | RAET1G | T179I | G | 604 | A |
| PCDH15 | R1526H | C | 4972 | T | PITPNC1 | E277K | G | 1203 | A | PRDM14 | H220R | T | 861 | C | RAF1 | R302Q | C | 1267 | T |
| PCDH15 | L844R | A | 2926 | C | PITPNM1 | - | G | 0 | A | PRDM14 | R332H | C | 1197 | T | RAF1 | R638Q | C | 2275 | T |
| PCDH15 | R1553S | G | 5052 | T | PITPNM1 | A1134V | G | 3627 | A | PRDM14 | K518T | T | 1755 | G | RAG1 | P1021H | C | 3186 | A |
| PCDH15 | Y759* | G | 2672 | T | PITPNM1 | V532M | C | 1820 | T | PRDM14 | H265Q | A | 997 | C | RAG1 | R897Q | G | 2814 | A |
| PCDH15 | D852N | C | 2949 | T | PITPNM1 | I1062V | T | 3410 | C | PRDM15 | A813T | C | 2548 | T | RAG2 | R148Q | C | 631 | T |
| PCDH15 | D748N | C | 2637 | T | PITPNM2 | R828H | C | 2622 | T | PRDM15 | G802S | C | 2515 | T | RAGE | M21I | C | 289 | T |
| PCDH15 | N394S | T | 1576 | C | PITPNM2 | G1180S | C | 3677 | T | PRDM15 | F444I | A | 1441 | T | RAI1 | S805L | C | 2883 | T |
| PCDH17 | A727T | G | 2205 | A | PITPNM2 | R749W | G | 2384 | A | PRDM16 | E64G | A | 240 | G | RAI1 | E764G | A | 2760 | G |
| PCDH17 | T984I | C | 2977 | T | PITPNM2 | P577L | G | 1869 | A | PRDM16 | K448N | G | 1393 | T | RAI1 | A1764T | G | 5759 | A |
| PCDH17 | R1109Q | G | 3352 | A | PITPNM2 | G612S | C | 1973 | T | PRDM16 | S537I | G | 1659 | T | RAI1 | D1800N | G | 5867 | A |
| PCDH17 | A129V | C | 412 | T | PITPNM2 | M220V | T | 797 | C | PRDM16 | A1163T | G | 3536 | A | RAI1 | A535T | G | 2072 | A |
| PCDH17 | A559V | C | 1702 | T | PITPNM2 | P750S | G | 2387 | A | PRDM16 | A845V | C | 2583 | T | RAI14 | S808L | C | 2915 | T |

| | | | | | | | | | | | | | | | | | | | |
|---|---|---|---|---|---|---|---|---|---|---|---|---|---|---|---|---|---|---|---|
| PCDH17 | D1153N | G | 3483 | A | PITPNM2 | V1230M | C | 3827 | T | PRDM2 | G43S | G | 983 | A | RAI2 | P239S | G | 1072 | A |
| PCDH17 | D500E | C | 1526 | A | PITPNM2 | A417V | G | 1389 | A | PRDM2 | D277E | T | 1687 | A | RAI2 | S269P | A | 1162 | G |
| PCDH17 | R622H | G | 1891 | A | PITPNM3 | A837V | G | 2661 | A | PRDM2 | E282D | A | 1702 | T | RALA | F168C | T | 883 | G |
| PCDH17 | R53H | G | 184 | A | PITPNM3 | R493W | G | 1628 | A | PRDM2 | T1365M | C | 4950 | T | RALB | N209S | A | 626 | G |
| PCDH17 | D112N | G | 360 | A | PITRM1 | G981V | C | 2981 | A | PRDM2 | E282D | A | 1702 | T | RALBP1 | Q269* | C | 1028 | T |
| PCDH17 | D264N | G | 816 | A | PITX1 | A186V | G | 974 | A | PRDM2 | R407Q | G | 2076 | A | RALBP1 | S383A | T | 1370 | G |
| PCDH18 | R793Q | C | 2765 | T | PITX1 | R147C | G | 856 | A | PRDM2 | K1523Q | A | 5423 | C | RALBP1 | L443R | T | 1551 | G |
| PCDH18 | V110A | A | 716 | G | PITX1 | N150S | T | 866 | C | PRDM5 | R558* | G | 1913 | A | RALGAPA1 | A617V | G | 2241 | A |
| PCDH18 | S777L | G | 2717 | A | PITX2 | R152C | G | 1097 | A | PRDM5 | R232Q | C | 936 | T | RALGAPA1 | G1106C | C | 3707 | A |
| PCDH18 | D439N | C | 1702 | T | PITX2 | Q102* | G | 947 | A | PRDM6 | V223M | G | 1081 | A | RALGAPA1 | S849G | T | 2936 | C |
| PCDH18 | S655L | G | 2351 | A | PITX3 | G22D | C | 219 | T | PRDM6 | L472I | C | 1828 | A | RALGAPA1 | V585M | C | 2144 | T |
| PCDH18 | T919A | T | 3142 | C | PITX3 | S251F | G | 906 | A | PRDM7 | L66I | G | 216 | T | RALGAPA1 | F1214C | A | 4032 | C |
| PCDH18 | K406Q | T | 1603 | G | PIWIL1 | R275Q | G | 914 | A | PRDM7 | S312* | G | 955 | T | RALGAPA2 | D1206N | C | 3759 | T |
| PCDH18 | T585M | G | 2141 | A | PIWIL1 | R684M | G | 2141 | T | PRDM7 | F73V | A | 237 | C | RALGAPA2 | S1671N | C | 5155 | T |
| PCDH19 | N1100H | T | 4974 | G | PIWIL1 | E154K | G | 550 | A | PRDM8 | R181I | G | 1381 | T | RALGAPA2 | R393Q | C | 1321 | T |
| PCDH19 | S401R | G | 2879 | T | PIWIL1 | R397C | C | 1279 | T | PRDM8 | R118C | C | 1191 | T | RALGAPA2 | R1757S | G | 5412 | T |
| PCDH19 | S924N | C | 4447 | T | PIWIL2 | A21V | C | 193 | T | PRDM8 | V128A | T | 1222 | C | RALGAPA2 | R385Q | C | 1297 | T |
| PCDH19 | E530K | C | 3264 | T | PIWIL2 | V138I | G | 543 | A | PRDM8 | A385T | G | 1992 | A | RALGAPA2 | N233T | T | 841 | G |
| PCDH19 | R395Q | C | 2860 | T | PIWIL3 | A9T | C | 442 | T | PRDM8 | A372V | C | 1954 | T | RALGAPB | K58T | A | 457 | C |
| PCDH20 | I785M | T | 2719 | C | PIWIL3 | D109Y | C | 742 | A | PRDM9 | K232N | G | 878 | T | RALGAPB | K600T | A | 2083 | C |
| PCDH20 | K522Q | T | 1928 | G | PIWIL4 | R283I | G | 1059 | T | PRDM9 | R839Q | G | 2698 | A | RALGAPB | Q1078K | C | 3516 | A |
| PCDH7 | E329D | G | 987 | T | PIWIL4 | R687W | C | 2270 | T | PRDM9 | P222S | C | 846 | T | RALGDS | I254T | A | 846 | G |
| PCDH7 | P22L | C | 65 | T | PIWIL4 | S470P | T | 1619 | C | PRDM9 | Y51* | T | 335 | A | RALGDS | R827S | G | 2564 | T |
| PCDH7 | A167V | C | 500 | T | PJA1 | W351R | A | 1358 | G | PRDM9 | R113H | G | 520 | A | RALGDS | R866H | C | 2682 | T |
| PCDH7 | C1002* | T | 3006 | A | PJA2 | R676H | C | 2267 | T | PRDM9 | P11T | C | 213 | A | RALGPS1 | A307V | C | 1187 | T |
| PCDH7 | E922D | G | 2766 | T | PJA2 | I642M | A | 2166 | C | PRDM9 | T15I | C | 226 | T | RALGPS1 | A359V | C | 1343 | T |
| PCDH8 | P311S | G | 1135 | A | PKD1 | W2094L | C | 6490 | A | PRDM9 | E836K | G | 2688 | A | RALY | F123L | C | 871 | A |
| PCDH8 | R999H | C | 3200 | T | PKD1 | R1587H | C | 4969 | T | PRDM9 | R279Q | G | 1018 | A | RALY | H67N | C | 701 | A |
| PCDH8 | R77H | C | 434 | T | PKD1 | A3653V | G | 11167 | A | PRDX1 | E193* | C | 917 | A | RALYL | S101Y | C | 444 | A |
| PCDH8 | E866* | C | 2800 | A | PKD1 | - | C | 0 | T | PRDX3 | L186H | A | 600 | T | RAMP1 | D71E | C | 345 | G |
| PCDH8 | R173H | C | 722 | T | PKD1 | T1012I | G | 3244 | A | PRDX3 | V209M | C | 668 | T | RAMP2 | Q130* | C | 463 | T |
| PCDH8 | P100L | G | 503 | A | PKD1 | F3380L | A | 10347 | G | PRDX5 | L115P | T | 463 | C | RAN | E90D | G | 566 | T |
| PCDH8 | R907K | C | 2924 | T | PKD1 | L1904M | G | 5919 | T | PRDX5 | G199D | G | 715 | A | RAN | V197A | T | 886 | C |
| PCDH8 | A974T | C | 3124 | T | PKD1 | S3502N | C | 10714 | T | PRDX6 | R108W | C | 454 | T | RANBP1 | R140H | G | 605 | A |
| PCDH8 | R963H | C | 3092 | T | PKD1 | A3442V | G | 10534 | A | PRELID2 | T115A | T | 396 | C | RANBP1 | S85Y | C | 440 | A |
| PCDH8 | S985P | A | 3157 | G | PKD1 | R2951W | G | 9060 | A | PRELP | F262L | T | 974 | C | RANBP10 | P583S | G | 1863 | A |

| | | | | | | | | | | | | | | | | | | | |
|---|---|---|---|---|---|---|---|---|---|---|---|---|---|---|---|---|---|---|---|
| PCDH9 | R249M | C | 881 | A | PKD1 | S3347P | A | 10248 | G | PRELP | - | G | 0 | T | RANBP10 | T179A | T | 651 | C |
| PCDH9 | H178Q | A | 669 | C | PKD1L1 | N970S | T | 2960 | C | PREP | R662H | C | 2178 | T | RANBP10 | A591T | C | 1887 | T |
| PCDH9 | M857I | C | 2706 | A | PKD1L1 | S1701Y | G | 5153 | T | PREP | V660M | C | 2171 | T | RANBP10 | Y462H | A | 1500 | G |
| PCDH9 | E25D | T | 210 | G | PKD1L1 | G1524V | C | 4622 | A | PREP | K75N | C | 418 | A | RANBP10 | S188R | G | 680 | T |
| PCDH9 | F1161V | A | 3616 | C | PKD1L1 | W497* | C | 1542 | T | PREPL | R226C | G | 1114 | A | RANBP10 | V256M | C | 882 | T |
| PCDH9 | - | T | 0 | C | PKD1L1 | A853T | C | 2608 | T | PREPL | R413H | C | 1676 | T | RANBP17 | I811N | T | 2432 | A |
| PCDH9 | - | A | 3847 | G | PKD1L1 | S659I | C | 2027 | A | PREX1 | E1246K | C | 3759 | T | RANBP17 | V526F | G | 1576 | T |
| PCDH9 | W1143C | C | 3564 | A | PKD1L1 | R990W | G | 3019 | A | PREX1 | A1495T | C | 4506 | T | RANBP17 | S620Y | C | 1859 | A |
| PCDH9 | P1026H | G | 3212 | T | PKD1L1 | A1799V | G | 5447 | A | PREX1 | V896I | C | 2709 | T | RANBP2 | K1032N | A | 3222 | C |
| PCDH9 | K1003N | C | 3144 | A | PKD1L1 | E1836D | C | 5559 | A | PREX1 | L1292P | A | 3898 | G | RANBP2 | P1713Q | C | 5264 | A |
| PCDH9 | V473G | A | 1553 | C | PKD1L1 | Y1784H | A | 5401 | G | PREX1 | N264S | T | 814 | C | RANBP2 | R1203C | C | 3733 | T |
| PCDH9 | E95* | C | 418 | A | PKD1L1 | I419M | A | 1308 | C | PREX1 | R1515Q | C | 4567 | T | RANBP2 | G1716R | G | 5272 | A |
| PCDH9 | L26R | A | 212 | C | PKD1L1 | R1674T | C | 5072 | G | PREX1 | A1486T | C | 4479 | T | RANBP2 | A2104V | C | 6437 | T |
| PCDH9 | S905R | G | 2850 | T | PKD1L2 | V878M | C | 2632 | T | PREX1 | P1252H | G | 3778 | T | RANBP2 | S3170Y | C | 9635 | A |
| PCDHA1 | R428W | C | 1437 | T | PKD1L2 | G353S | C | 1057 | T | PREX1 | R394C | G | 1203 | A | RANBP3 | A295T | C | 1110 | T |
| PCDHA1 | P764T | C | 2445 | A | PKD1L2 | S357P | A | 1069 | G | PREX1 | E1499D | C | 4520 | A | RANBP3L | - | T | 0 | C |
| PCDHA1 | R151H | G | 607 | A | PKD1L2 | A984V | G | 2951 | A | PREX1 | E61D | C | 206 | A | RANBP6 | D437N | C | 1320 | T |
| PCDHA1 | D91Y | G | 426 | T | PKD1L2 | P370H | G | 1109 | T | PREX2 | A855T | G | 2840 | A | RANBP6 | L774P | A | 2332 | G |
| PCDHA1 | R197I | G | 745 | T | PKD1L2 | E950K | C | 2848 | T | PREX2 | - | T | 0 | C | RANBP6 | F542V | A | 1635 | C |
| PCDHA1 | E291* | G | 1026 | T | PKD1L3 | M176K | A | 527 | T | PREX2 | V1540A | T | 4896 | C | RANGAP1 | V455M | C | 2076 | T |
| PCDHA1 | E804K | G | 2565 | A | PKD1L3 | K454N | C | 1362 | A | PREX2 | L50V | T | 425 | G | RANGAP1 | P443S | G | 2040 | A |
| PCDHA10 | G382R | G | 1310 | A | PKD1L3 | F1306L | A | 3918 | C | PREX2 | A1467T | G | 4676 | A | RANGAP1 | R239W | G | 1428 | A |
| PCDHA10 | V447M | G | 1505 | A | PKD1L3 | A931D | G | 2792 | T | PREX2 | R427G | A | 1556 | G | RANGAP1 | R448H | C | 2056 | T |
| PCDHA10 | R753Q | G | 2424 | A | PKD2 | R638H | G | 1979 | A | PREX2 | V167A | T | 777 | C | RANGRF | P146L | C | 555 | T |
| PCDHA11 | V507M | G | 2377 | A | PKD2L1 | E706* | C | 2499 | A | PREX2 | R155Q | G | 741 | A | RAP1GAP | S521L | G | 1562 | A |
| PCDHA11 | D559N | G | 2533 | A | PKD2L2 | R448Q | G | 1399 | A | PREX2 | E92K | G | 551 | A | RAP1GAP | V609M | C | 1825 | T |
| PCDHA12 | Y699H | T | 2222 | C | PKD2L2 | M151T | T | 508 | C | PREX2 | A334S | G | 1277 | T | RAP1GAP | P563H | G | 1688 | T |
| PCDHA12 | T205M | C | 741 | T | PKD2L2 | Y120C | A | 415 | G | PREX2 | K367Q | A | 1376 | C | RAP1GAP | G557R | C | 1669 | T |
| PCDHA12 | A567V | C | 1827 | T | PKD2L2 | R82H | G | 301 | A | PREX2 | R577Q | G | 2007 | A | RAP1GAP2 | A728V | C | 2273 | T |
| PCDHA12 | V464M | G | 1517 | A | PKD2L2 | F235L | T | 761 | G | PREX2 | K635N | G | 2182 | T | RAP1GDS1 | R450C | C | 1396 | T |
| PCDHA12 | E93K | G | 404 | A | PKD2L2 | R448Q | G | 1399 | A | PREX2 | K905N | G | 2992 | T | RAP1GDS1 | S172L | C | 563 | T |
| PCDHA12 | E416K | G | 1373 | A | PKDCC | D379G | A | 1316 | G | PREX2 | Y1452D | T | 4631 | G | RAP2A | A59V | C | 425 | T |
| PCDHA12 | A412T | G | 1361 | A | PKDCC | S355R | T | 1245 | G | PREX2 | A1499T | G | 4772 | A | RAP2B | E37D | A | 565 | C |
| PCDHA13 | E301D | A | 903 | C | PKDREJ | S925L | G | 2774 | A | PREX2 | K1578N | G | 5011 | T | RAP2C | M164I | C | 1239 | A |
| PCDHA13 | A488T | G | 1462 | A | PKDREJ | R172Q | C | 515 | T | PRF1 | R119Q | C | 471 | T | RAPGEF1 | A181T | C | 700 | T |
| PCDHA13 | T634M | C | 1901 | T | PKDREJ | R2067G | T | 6199 | C | PRF1 | R54H | C | 276 | T | RAPGEF1 | A418T | C | 1411 | T |
| PCDHA13 | E301* | G | 901 | T | PKDREJ | H459R | T | 1376 | C | PRG2 | R108H | C | 379 | T | RAPGEF1 | E594K | C | 1939 | T |
| PCDHA2 | V555M | G | 1769 | A | PKDREJ | C2090R | A | 6268 | G | PRG2 | T28I | G | 139 | A | RAPGEF2 | R823Q | G | 2887 | A |
| PCDHA2 | V745M | G | 2339 | A | PKDREJ | V1462I | C | 4384 | T | PRG4 | K1008T | A | 3074 | C | RAPGEF2 | S1060F | C | 3598 | T |

| | | | | | | | | | | | | | | | | | | | |
|---|---|---|---|---|---|---|---|---|---|---|---|---|---|---|---|---|---|---|---|
| PCDHA2 | V77A | T | 336 | C | PKDREJ | N958S | T | 2873 | C | PRG4 | T708I | C | 2174 | T | RAPGEF2 | R233H | G | 1117 | A |
| PCDHA2 | A706V | C | 2223 | T | PKDREJ | V2128A | A | 6383 | G | PRICKLE1 | R675H | C | 2311 | T | RAPGEF2 | K240N | G | 1139 | T |
| PCDHA2 | E416K | G | 1352 | A | PKDREJ | V2110L | C | 6328 | A | PRICKLE1 | R657Q | C | 2257 | T | RAPGEF3 | A204T | C | 699 | T |
| PCDHA2 | L800F | A | 2506 | C | PKDREJ | C1619W | A | 4857 | C | PRICKLE1 | R674H | C | 2308 | T | RAPGEF3 | V458I | C | 1461 | T |
| PCDHA3 | R47H | G | 310 | A | PKDREJ | L1447V | A | 4339 | C | PRICKLE1 | R236H | C | 994 | T | RAPGEF3 | L480I | G | 1527 | T |
| PCDHA3 | V35I | G | 273 | A | PKDREJ | A1083T | C | 3247 | T | PRICKLE1 | L502V | G | 1791 | C | RAPGEF3 | D420N | C | 1347 | T |
| PCDHA3 | A488T | G | 1632 | A | PKHD1 | V629A | A | 2162 | G | PRICKLE1 | R702W | G | 2391 | A | RAPGEF4 | I195M | T | 728 | G |
| PCDHA3 | G574D | G | 1891 | A | PKHD1 | F3280C | A | 10115 | C | PRICKLE1 | R693I | C | 2365 | A | RAPGEF4 | F889L | C | 2810 | A |
| PCDHA3 | R722W | C | 2334 | T | PKHD1 | V3263L | C | 10063 | A | PRICKLE2 | R397W | G | 1775 | A | RAPGEF4 | R26Q | G | 220 | A |
| PCDHA3 | V549M | G | 1815 | A | PKHD1 | R793C | G | 2653 | A | PRICKLE2 | V161I | C | 1067 | T | RAPGEF4 | E375* | G | 1266 | T |
| PCDHA3 | V45M | G | 303 | A | PKHD1 | G470D | C | 1685 | T | PRICKLE3 | R188C | G | 560 | A | RAPGEF4 | E349D | G | 1190 | T |
| PCDHA3 | K138N | G | 584 | T | PKHD1 | Q2743K | G | 8503 | T | PRICKLE4 | C55Y | G | 412 | A | RAPGEF4 | N991H | A | 3114 | C |
| PCDHA3 | T43N | C | 298 | A | PKHD1 | R1081H | C | 3518 | T | PRIM1 | F211C | A | 696 | C | RAPGEF5 | E173* | C | 830 | A |
| PCDHA4 | A266T | G | 882 | A | PKHD1 | L2106R | A | 6593 | C | PRIM2 | - | G | 0 | T | RAPGEF5 | H660N | G | 2291 | T |
| PCDHA4 | A266T | G | 882 | A | PKHD1 | M947T | A | 3116 | G | PRIMA1 | L3F | G | 110 | A | RAPGEF5 | K161Q | T | 794 | G |
| PCDHA4 | A484T | G | 1536 | A | PKHD1 | G1427S | C | 4555 | T | PRKAA1 | I211M | A | 818 | C | RAPGEF5 | E100K | C | 611 | T |
| PCDHA4 | K198T | A | 679 | C | PKHD1 | E4072D | C | 12492 | A | PRKAA1 | H291Q | A | 1058 | C | RAPGEFL1 | A355T | G | 1063 | A |
| PCDHA5 | S433L | C | 1470 | T | PKHD1 | E3582K | C | 11020 | T | PRKAA2 | H73R | A | 284 | G | RAPGEFL1 | R613C | C | 1837 | T |
| PCDHA5 | P795H | C | 2556 | A | PKHD1 | R3350K | C | 10325 | T | PRKAA2 | R227* | C | 745 | T | RAPGEFL1 | T345M | C | 1034 | T |
| PCDHA5 | P129L | C | 558 | T | PKHD1 | S3112P | A | 9610 | G | PRKAA2 | N144I | A | 497 | T | RAPH1 | P884H | G | 2651 | T |
| PCDHA6 | V542M | G | 1651 | A | PKHD1 | N2842D | T | 8800 | C | PRKAB1 | I36N | T | 396 | A | RAPH1 | R1227Q | C | 3680 | T |
| PCDHA6 | L659M | C | 2002 | A | PKHD1 | V2552I | C | 7930 | T | PRKAB2 | R179G | T | 674 | C | RAPH1 | K302T | T | 905 | G |
| PCDHA6 | P563L | C | 1715 | T | PKHD1 | I1310V | T | 4204 | C | PRKACA | A148T | C | 642 | T | RAPSN | R282C | G | 1058 | A |
| PCDHA6 | A439T | G | 1342 | A | PKHD1L1 | T145A | A | 537 | G | PRKACA | K77E | T | 429 | C | RAPSN | E368K | C | 1316 | T |
| PCDHA6 | S608L | C | 1850 | T | PKHD1L1 | S4195R | C | 12689 | G | PRKACA | Y331C | T | 1192 | C | RARB | R294* | C | 880 | T |
| PCDHA6 | E243K | G | 754 | A | PKHD1L1 | H1477Y | C | 4533 | T | PRKACA | F351L | A | 1253 | C | RARB | L187H | T | 560 | A |
| PCDHA7 | A615V | C | 1997 | T | PKHD1L1 | A19V | C | 160 | T | PRKACB | T347M | C | 1131 | T | RARG | R367H | C | 1538 | T |
| PCDHA7 | H645Y | C | 2086 | T | PKHD1L1 | S1480N | G | 4543 | A | PRKACB | R181T | G | 633 | C | RARRES1 | K166E | T | 777 | C |
| PCDHA7 | A452T | G | 1507 | A | PKHD1L1 | D219Y | G | 759 | T | PRKACG | G68S | C | 233 | T | RARRES1 | N293H | T | 1158 | G |
| PCDHA7 | A66V | C | 350 | T | PKHD1L1 | L1432I | C | 4398 | A | PRKACG | A4T | C | 41 | T | RARS | G575C | G | 1777 | T |
| PCDHA7 | R681W | C | 2194 | T | PKHD1L1 | W2621C | G | 7967 | T | PRKAG1 | P22T | G | 133 | T | RARS | L565I | C | 1747 | A |
| PCDHA7 | R681Q | G | 2195 | A | PKHD1L1 | E3043* | G | 9231 | T | PRKAG1 | R70Q | C | 278 | T | RASA1 | R427* | C | 1843 | T |
| PCDHA7 | I318V | A | 1105 | G | PKHD1L1 | V3064A | T | 9295 | C | PRKAG1 | A60D | G | 248 | T | RASA1 | S222N | G | 1229 | A |
| PCDHA7 | R646H | G | 2090 | A | PKHD1L1 | L3224I | C | 9774 | A | PRKAG3 | R449Q | C | 1392 | T | RASA1 | E193* | G | 1141 | T |
| PCDHA7 | V624M | G | 2023 | A | PKIA | E37K | G | 504 | A | PRKAG3 | D358Y | C | 1118 | A | RASA1 | M967I | G | 3465 | T |

| | | | | | | | | | | | | | | | | | | | |
|---|---|---|---|---|---|---|---|---|---|---|---|---|---|---|---|---|---|---|---|
| PCDHA8 | N449D | A | 1440 | G | PKMYT1 | R246C | G | 1130 | A | PRKAR1B | A139T | C | 590 | T | RASA1 | E560* | G | 2242 | T |
| PCDHA8 | S491P | T | 1566 | C | PKMYT1 | K149Q | T | 839 | G | PRKAR2B | L384P | T | 1410 | C | RASA2 | R761I | G | 2317 | T |
| PCDHA8 | E243K | G | 822 | A | PKN1 | R133H | G | 429 | A | PRKCB | K304T | A | 1063 | C | RASA2 | N218T | A | 688 | C |
| PCDHA9 | G588S | G | 2486 | A | PKN1 | E828D | G | 2515 | T | PRKCB | R161C | C | 633 | T | RASA3 | T518M | G | 1684 | A |
| PCDHA9 | A484T | G | 2174 | A | PKN1 | T799M | C | 2427 | T | PRKCB | P317L | C | 1102 | T | RASA3 | T297S | T | 1020 | A |
| PCDHAC1 | R281* | C | 841 | T | PKN1 | A326V | C | 1008 | T | PRKCB | K439N | G | 1469 | T | RASA3 | A394V | G | 1312 | A |
| PCDHAC1 | D128N | G | 382 | A | PKN1 | R892C | C | 2705 | T | PRKCB | R161H | G | 634 | A | RASAL1 | A436T | C | 1599 | T |
| PCDHAC1 | A197V | C | 590 | T | PKN3 | A26V | C | 470 | T | PRKCD | T58M | C | 501 | T | RASAL1 | S154T | A | 753 | T |
| PCDHAC2 | A109V | C | 858 | T | PKN3 | P11L | C | 425 | T | PRKCE | M597V | A | 2116 | G | RASAL2 | - | G | 0 | A |
| PCDHAC2 | G425D | G | 1806 | A | PKN3 | R431C | C | 1684 | T | PRKCE | R688H | G | 2390 | A | RASAL2 | R1012* | C | 3386 | T |
| PCDHAC2 | D631A | A | 2424 | C | PKN3 | L590P | T | 2162 | C | PRKCG | A40T | G | 400 | A | RASAL2 | A1243V | C | 4080 | T |
| PCDHAC2 | H777P | A | 2862 | C | PKN3 | A317V | C | 1343 | T | PRKCG | K495M | A | 1766 | T | RASAL2 | L627F | C | 2231 | T |
| PCDHB1 | E435K | G | 1398 | A | PKN3 | R42C | C | 517 | T | PRKCG | A458V | C | 1655 | T | RASAL3 | E567K | C | 1785 | T |
| PCDHB1 | I487V | A | 1554 | G | PKN3 | - | G | 0 | A | PRKCG | - | G | 0 | T | RASD2 | R38C | C | 754 | T |
| PCDHB1 | G218R | G | 747 | A | PKN3 | R560H | G | 2072 | A | PRKCG | S361R | A | 1363 | C | RASEF | A113V | G | 599 | A |
| PCDHB1 | K495N | A | 1580 | T | PKNOX1 | R316Q | G | 1158 | A | PRKCH | S465L | C | 1779 | T | RASGEF1A | A462T | C | 1469 | T |
| PCDHB1 | R806K | G | 2512 | A | PKNOX1 | R340Q | G | 1230 | A | PRKCH | L124P | T | 756 | C | RASGEF1B | L111I | G | 483 | T |
| PCDHB11 | I277L | A | 879 | C | PKNOX2 | D197N | G | 903 | A | PRKCH | L371R | T | 1497 | G | RASGEF1B | R18Q | C | 205 | T |
| PCDHB11 | C278S | G | 883 | C | PKNOX2 | P57T | C | 483 | A | PRKCH | S465L | C | 1779 | T | RASGEF1C | A147V | G | 737 | A |
| PCDHB11 | N80H | A | 288 | C | PKP1 | C477R | T | 1680 | C | PRKCH | F613L | T | 2224 | G | RASGEF1C | V138M | C | 709 | T |
| PCDHB11 | L262V | T | 834 | G | PKP2 | V411I | C | 1346 | T | PRKCI | R192W | C | 879 | T | RASGEF1C | G293S | C | 1174 | T |
| PCDHB11 | D425N | G | 1323 | A | PKP2 | P367T | G | 1214 | T | PRKCI | G398S | G | 1497 | A | RASGEF1C | F392V | A | 1471 | C |
| PCDHB12 | A579V | C | 1925 | T | PKP2 | R79Q | C | 351 | T | PRKCQ | E35D | C | 205 | G | RASGRF1 | T41A | T | 415 | C |
| PCDHB12 | P721L | C | 2351 | T | PKP2 | R871Q | C | 2727 | T | PRKCQ | M267V | T | 899 | C | RASGRF1 | A82V | G | 539 | A |
| PCDHB13 | A677T | G | 2029 | A | PKP3 | R439H | G | 1392 | A | PRKCQ | R600W | G | 1898 | A | RASGRF1 | V404I | C | 1504 | T |
| PCDHB13 | S534T | T | 1600 | A | PKP4 | R621Q | G | 1974 | A | PRKCQ | R635W | G | 2003 | A | RASGRF1 | S873Y | G | 2912 | T |
| PCDHB14 | D342N | G | 1156 | A | PKP4 | R286W | C | 968 | T | PRKCSH | R506C | C | 1651 | T | RASGRF2 | A842V | C | 2592 | T |
| PCDHB14 | V66I | G | 328 | A | PLA1A | L423I | C | 1339 | A | PRKCSH | V110I | G | 463 | A | RASGRF2 | R244I | G | 798 | T |
| PCDHB14 | G299R | G | 1027 | A | PLA1A | S156L | C | 539 | T | PRKCSH | P352L | C | 1190 | T | RASGRF2 | E202K | G | 671 | A |
| PCDHB14 | D69A | A | 338 | C | PLA2G15 | A196V | C | 670 | T | PRKCSH | Q195P | A | 719 | C | RASGRF2 | D588Y | G | 1829 | T |
| PCDHB14 | R790Q | G | 2501 | A | PLA2G16 | Y74H | A | 643 | G | PRKCSH | R489C | C | 1600 | T | RASGRF2 | S877L | C | 2697 | T |
| PCDHB15 | R158W | C | 638 | T | PLA2G1B | N134T | T | 437 | G | PRKCZ | A142V | C | 586 | T | RASGRF2 | R244I | G | 798 | T |
| PCDHB16 | A693V | C | 3233 | T | PLA2G2C | S13L | G | 38 | A | PRKCZ | R127H | G | 541 | A | RASGRF2 | E934K | G | 2867 | A |
| PCDHB16 | T39M | C | 1271 | T | PLA2G2F | A80T | G | 340 | A | PRKCZ | V303M | G | 1068 | A | RASGRP1 | S708R | A | 2302 | T |
| PCDHB16 | P769L | C | 3461 | T | PLA2G3 | D226N | C | 929 | T | PRKCZ | - | A | 0 | G | RASGRP1 | H286R | T | 1035 | C |
| PCDHB16 | E310K | G | 2083 | A | PLA2G3 | V16A | A | 300 | G | PRKD1 | T746I | G | 2467 | A | RASGRP2 | P465T | G | 1437 | T |
| PCDHB18 | A666V | C | 2345 | T | PLA2G3 | S293R | T | 1130 | G | PRKD1 | R732W | G | 2424 | A | RASGRP3 | R60Q | G | 831 | A |
| PCDHB18 | F347V | T | 1387 | G | PLA2G4A | V392A | T | 1327 | C | PRKD1 | R172H | C | 745 | T | RASGRP3 | D119N | G | 1007 | A |
| PCDHB2 | T409M | C | 1364 | T | PLA2G4A | T28A | A | 234 | G | PRKD1 | Y828H | A | 2712 | G | RASGRP3 | - | A | 0 | C |

| | | | | | | | | | | | | | | | | | | | |
|---|---|---|---|---|---|---|---|---|---|---|---|---|---|---|---|---|---|---|---|
| PCDHB2 | E90D | G | 408 | T | PLA2G4C | Q121H | C | 705 | A | PRKD1 | D378N | C | 1362 | T | RASGRP3 | E351* | G | 1703 | T |
| PCDHB2 | L505M | C | 1651 | A | PLA2G4C | V20M | C | 400 | T | PRKD1 | A731D | G | 2422 | T | RASIP1 | R338Q | C | 1210 | T |
| PCDHB3 | K434E | A | 1300 | G | PLA2G4C | H278Y | G | 1174 | A | PRKD2 | A361T | C | 1423 | T | RASIP1 | R806Q | C | 2614 | T |
| PCDHB3 | Y435H | T | 1303 | C | PLA2G4C | K32N | C | 438 | A | PRKD2 | D877N | C | 2971 | T | RASL10A | E121K | C | 871 | T |
| PCDHB3 | R536C | C | 1606 | T | PLA2G4D | P633S | G | 1992 | A | PRKD2 | T748A | T | 2584 | C | RASL10B | A201T | G | 967 | A |
| PCDHB3 | E29K | G | 85 | A | PLA2G4D | P147Q | G | 535 | T | PRKD2 | A202V | G | 947 | A | RASL11A | L223M | C | 1285 | A |
| PCDHB3 | E88D | G | 264 | T | PLA2G4D | A388T | C | 1257 | T | PRKD3 | R355Q | C | 1827 | T | RASSF1 | W129C | C | 423 | A |
| PCDHB3 | E100* | G | 298 | T | PLA2G4D | - | A | 0 | G | PRKD3 | R647* | G | 2702 | A | RASSF1 | R244H | C | 767 | T |
| PCDHB3 | E358D | G | 1074 | T | PLA2G4E | R243Q | C | 1215 | T | PRKD3 | N431T | T | 2055 | G | RASSF1 | P15S | G | 79 | A |
| PCDHB3 | G329R | G | 985 | A | PLA2G4E | L627M | G | 2366 | T | PRKD3 | D107G | T | 1083 | C | RASSF1 | F237L | A | 745 | G |
| PCDHB4 | A414T | G | 1240 | A | PLA2G4E | G553S | C | 2144 | T | PRKDC | D2871N | C | 8668 | T | RASSF1 | R176C | G | 562 | A |
| PCDHB4 | R624C | C | 1870 | T | PLA2G6 | A147T | C | 623 | T | PRKDC | I125L | T | 430 | G | RASSF2 | G36D | C | 303 | T |
| PCDHB4 | L153F | G | 459 | T | PLA2G6 | R6H | C | 201 | T | PRKDC | D3688N | C | 11119 | T | RASSF8 | P82L | C | 444 | T |
| PCDHB4 | D555N | G | 1663 | A | PLA2G6 | P625S | G | 2057 | A | PRKDC | N3696S | T | 11144 | C | RASSF8 | D392Y | G | 1496 | T |
| PCDHB5 | G328* | G | 1189 | T | PLA2G6 | R635Q | C | 2088 | T | PRKDC | W1828* | C | 5541 | T | RASSF8 | V27D | T | 279 | A |
| PCDHB5 | - | A | 2594 | C | PLA2G7 | G164D | C | 688 | T | PRKDC | H2681R | T | 8099 | C | RASSF9 | A302T | C | 1243 | T |
| PCDHB6 | N237S | A | 710 | G | PLA2R1 | R1342Q | C | 4232 | T | PRKDC | A615T | C | 1900 | T | RASSF9 | F218L | G | 993 | T |
| PCDHB7 | P221T | C | 743 | A | PLA2R1 | N1075H | T | 3430 | G | PRKG1 | D26A | A | 494 | C | RASSF9 | P91H | G | 611 | T |
| PCDHB7 | R785H | G | 2436 | A | PLA2R1 | C929W | A | 2994 | C | PRKG1 | K405E | A | 1630 | G | RAVER2 | A282V | C | 923 | T |
| PCDHB7 | A508V | C | 1605 | T | PLAC1L | N19H | A | 240 | C | PRKG2 | P672L | G | 2132 | A | RAVER2 | T477N | C | 1508 | A |
| PCDHB7 | Y563D | T | 1769 | G | PLAC8L1 | L76I | G | 284 | T | PRKG2 | I484T | A | 1568 | G | RAVER2 | F389C | T | 1244 | G |
| PCDHB8 | L405P | T | 1459 | C | PLAG1 | F34C | A | 497 | C | PRKG2 | K122M | T | 482 | A | RAVER2 | M423I | G | 1347 | T |
| PCDHB8 | T268M | C | 1048 | T | PLAGL1 | F325V | A | 2887 | C | PRKRIP1 | A141V | C | 1732 | T | RAVER2 | R652Q | G | 2033 | A |
| PCDHB8 | P540L | C | 1864 | T | PLAGL1 | E17D | C | 1965 | A | PRL | A51V | G | 671 | A | RAX | A60V | G | 366 | A |
| PCDHB8 | V728G | T | 2428 | G | PLAGL2 | Q41P | T | 387 | G | PRLHR | R125H | C | 513 | T | RB1 | G865V | G | 2732 | T |
| PCDHB9 | T657M | C | 2144 | T | PLAT | R524H | C | 1780 | T | PRLHR | V296I | C | 1025 | T | RB1 | S648T | T | 2080 | A |
| PCDHB9 | R530H | G | 1763 | A | PLAT | S414L | G | 1450 | A | PRLR | V237M | C | 1136 | T | RB1CC1 | E1447G | T | 4898 | C |
| PCDHB9 | S173N | G | 692 | A | PLAU | V381I | G | 1234 | A | PRLR | T618A | T | 2279 | C | RB1CC1 | G518A | C | 2111 | G |
| PCDHB9 | L519M | C | 1729 | A | PLAUR | M280T | A | 1069 | G | PRLR | E169* | C | 932 | A | RB1CC1 | F542C | A | 2183 | C |
| PCDHB9 | G762E | G | 2459 | A | PLAUR | T49M | G | 376 | A | PRM3 | R26W | G | 76 | A | RB1CC1 | R57* | G | 727 | A |
| PCDHB9 | H129Y | C | 559 | T | PLB1 | A190V | C | 613 | T | PRMT10 | R616H | C | 2090 | T | RBAK | G156R | G | 732 | A |
| PCDHB9 | V549M | G | 1819 | A | PLB1 | P694L | C | 2125 | T | PRMT2 | V88M | G | 1024 | A | RBAK | R412* | C | 1558 | T |
| PCDHGA1 | V67I | G | 247 | A | PLB1 | L885F | G | 2699 | T | PRMT2 | M405T | T | 1976 | C | RBAK | S524A | T | 1894 | G |
| PCDHGA1 | A696V | C | 2135 | T | PLB1 | P1003S | C | 3051 | T | PRMT5 | R384W | G | 1241 | A | RBBP4 | Q48R | A | 302 | G |
| PCDHGA1 | S296F | C | 935 | T | PLB1 | Q1233H | A | 3743 | T | PRMT5 | T80M | G | 330 | A | RBBP5 | A200T | C | 740 | T |
| PCDHGA1 | L368I | C | 1150 | A | PLB1 | N1275I | A | 3868 | T | PRMT7 | P353H | C | 1346 | A | RBBP6 | T1256A | A | 4198 | G |
| PCDHGA1 | E463K | G | 1435 | A | PLBD1 | R455* | G | 1524 | A | PRMT8 | T285M | C | 1244 | T | RBBP6 | S110Y | C | 761 | A |
| PCDHGA1 2 | L212F | C | 819 | T | PLBD1 | F195L | G | 746 | T | PRMT8 | V339I | G | 1405 | A | RBBP6 | R987G | A | 3391 | G |
| PCDHGA1 | G512S | G | 2432 | A | PLBD2 | Y116H | T | 374 | C | PRMT8 | T96A | A | 676 | G | RBBP6 | G1686D | G | 5489 | A |

| | | | | | | | | | | | | | | | | | | |
|---|---|---|---|---|---|---|---|---|---|---|---|---|---|---|---|---|---|---|
| 2 | | | | | | | | | | | | | | | | | | |
| PCDHGA12 | L750P | T | 3147 | C | PLCB1 | N818K | C | 2481 | A | PRMT8 | T248I | C | 1133 | T | RBBP6 | R1712Q | G | 5567 | A |
| PCDHGA12 | A492T | G | 1659 | A | PLCB1 | R168C | C | 529 | T | PRND | L17F | C | 120 | T | RBBP6 | Y898* | T | 3126 | A |
| PCDHGA12 | E311K | G | 1829 | A | PLCB1 | L353F | C | 1084 | T | PRNP | Q98P | A | 656 | C | RBBP6 | S473Y | C | 1850 | A |
| PCDHGA12 | L231F | C | 691 | T | PLCB1 | G96R | G | 313 | A | PRNT | V6A | A | 495 | G | RBBP7 | R347H | C | 1285 | T |
| PCDHGA12 | G571S | G | 2609 | A | PLCB1 | G305R | G | 940 | A | PROC | P301T | C | 907 | A | RBBP7 | E318K | C | 1197 | T |
| PCDHGA12 | T22M | C | 65 | T | PLCB1 | K1074T | A | 3248 | C | PROC | R59Q | G | 182 | A | RBBP8 | R496Q | G | 1818 | A |
| PCDHGA12 | A696V | C | 2272 | T | PLCB2 | F648L | G | 2245 | T | PROCR | E132K | G | 666 | A | RBBP8 | E742D | A | 2557 | T |
| PCDHGA12 | R627H | G | 2778 | A | PLCB2 | K1045T | T | 3435 | G | PRODH2 | A367V | G | 1100 | A | RBBP8 | E834* | G | 2831 | T |
| PCDHGA12 | G221R | G | 661 | A | PLCB2 | F822L | G | 2767 | T | PRODH2 | T420M | G | 1259 | A | RBCK1 | G485D | G | 2159 | A |
| PCDHGA12 | T805M | C | 2599 | T | PLCB2 | E539* | C | 1916 | A | PRODH2 | R177W | G | 529 | A | RBCK1 | S316C | A | 1651 | T |
| PCDHGA12 | E93G | A | 1176 | G | PLCB2 | E389* | C | 1466 | A | PROK1 | R6Q | G | 34 | A | RBCK1 | R134C | C | 1105 | T |
| PCDHGA12 | V795I | G | 3281 | A | PLCB3 | S404L | C | 1291 | T | PROKR1 | R307C | C | 2626 | T | RBL1 | H498N | G | 1559 | T |
| PCDHGA12 | A633V | C | 1898 | T | PLCB3 | R359H | G | 1156 | A | PROKR1 | R279C | C | 2542 | T | RBL1 | - | C | 0 | T |
| PCDHGA12 | V505I | G | 1513 | A | PLCB3 | T105M | C | 394 | T | PROKR2 | K53N | C | 159 | A | RBL1 | L355P | A | 1131 | G |
| PCDHGA12 | A18V | C | 53 | T | PLCB3 | A725T | G | 2253 | A | PROKR2 | E377* | C | 1129 | A | RBL1 | D837G | T | 2577 | C |
| PCDHGA12 | R751W | C | 2251 | T | PLCB4 | E892A | A | 2811 | C | PROL1 | - | T | 0 | C | RBL1 | R626Q | C | 1944 | T |
| PCDHGA12 | R198C | C | 777 | T | PLCD1 | A251V | G | 975 | A | PROM1 | V338M | C | 1292 | T | RBL1 | K277Q | T | 896 | G |
| PCDHGA12 | P79L | C | 236 | T | PLCD3 | R784H | C | 2465 | T | PROM1 | R472Q | C | 1695 | T | RBL2 | V843A | T | 2636 | C |
| PCDHGC3 | T100A | A | 412 | G | PLCD3 | R539H | C | 1730 | T | PROM1 | R472Q | C | 1695 | T | RBL2 | S982N | G | 3053 | A |
| PCDHGC4 | R337* | C | 1009 | T | PLCD4 | W389G | T | 1407 | G | PROM2 | R48Q | G | 276 | A | RBL2 | A66T | G | 304 | A |
| PCDHGC4 | E111G | A | 332 | G | PLCD4 | E226D | A | 920 | T | PROM2 | Q281H | G | 976 | T | RBL2 | E127* | G | 487 | T |
| PCDHGC4 | S166L | C | 497 | T | PLCD4 | A2V | C | 247 | T | PROM2 | W49* | G | 280 | A | RBL2 | S413R | A | 1345 | C |
| PCDHGC5 | T273I | C | 818 | T | PLCD4 | S60G | A | 420 | G | PROM2 | K833E | A | 2630 | G | RBM10 | V224D | T | 1413 | A |
| PCDHGC5 | D345G | A | 1034 | G | PLCD4 | E173* | G | 759 | T | PROM2 | G453D | G | 1491 | A | RBM10 | A773V | C | 3060 | T |
| PCF11 | R779Q | G | 2681 | A | PLCD4 | E194* | G | 822 | T | PROP1 | R120C | G | 667 | A | RBM11 | K143N | G | 471 | T |
| PCF11 | S330N | G | 1334 | A | PLCE1 | D871N | G | 2846 | A | PROS1 | A428T | C | 1599 | T | RBM11 | R74C | C | 262 | T |

| | | | | | | | | | | | | | | | | | | | |
|---|---|---|---|---|---|---|---|---|---|---|---|---|---|---|---|---|---|---|---|
| PCF11 | R1377Q | G | 4475 | A | PLCE1 | D1613G | A | 5073 | G | PROS1 | F414L | A | 1557 | G | RBM12B | R87I | C | 453 | A |
| PCF11 | Y1453H | T | 4702 | C | PLCE1 | T1412M | C | 4470 | T | PROSC | L175P | T | 601 | C | RBM14 | E145V | A | 573 | T |
| PCF11 | K345Q | A | 1378 | C | PLCE1 | N731I | A | 2427 | T | PROSC | D118N | G | 429 | A | RBM14 | R585Q | G | 1893 | A |
| PCF11 | - | G | 0 | C | PLCE1 | R1053Q | G | 3393 | A | PROX1 | R156H | G | 1075 | A | RBM14 | G505R | G | 1652 | A |
| PCF11 | E1401G | A | 4547 | G | PLCE1 | K1753R | A | 5493 | G | PROX1 | R596C | C | 2394 | T | RBM15 | A601T | G | 2701 | A |
| PCF11 | R565* | C | 2038 | T | PLCE1 | E2133D | G | 6634 | T | PROX2 | Q261R | T | 782 | C | RBM15 | E360* | G | 1978 | T |
| PCGF2 | H284N | G | 1093 | T | PLCE1 | S1292L | C | 4110 | T | PROZ | A53S | G | 164 | T | RBM15 | R663Q | G | 2888 | A |
| PCGF3 | L231P | T | 1087 | C | PLCE1 | R1434Q | G | 4536 | A | PROZ | T267M | C | 807 | T | RBM15B | R559C | C | 1775 | T |
| PCIF1 | R473* | C | 1716 | T | PLCE1 | H1756N | C | 5501 | A | PRPF18 | E295* | G | 1043 | T | RBM15B | T631A | A | 1991 | G |
| PCK1 | R115C | C | 507 | T | PLCG1 | D1075N | G | 3420 | A | PRPF18 | G293V | G | 1038 | T | RBM15B | R392K | G | 1275 | A |
| PCK2 | R561W | C | 1928 | T | PLCG1 | V585M | G | 1950 | A | PRPF18 | T60A | A | 338 | G | RBM15B | R337C | C | 1109 | T |
| PCK2 | A301T | G | 1148 | A | PLCG2 | P950H | C | 3019 | A | PRPF3 | E535* | G | 1768 | T | RBM17 | R168I | G | 728 | T |
| PCLO | P734T | G | 2489 | T | PLCG2 | R742H | G | 2395 | A | PRPF31 | E492K | G | 1823 | A | RBM18 | G91C | C | 412 | A |
| PCLO | E2038* | C | 6401 | A | PLCG2 | K80T | A | 409 | C | PRPF31 | V295M | G | 1232 | A | RBM19 | P931L | G | 2876 | A |
| PCLO | E1238* | C | 4001 | A | PLCG2 | E304D | G | 1082 | T | PRPF38A | V88L | G | 448 | T | RBM19 | Q579P | T | 1820 | G |
| PCLO | E188K | C | 851 | T | PLCG2 | K942N | A | 2996 | C | PRPF38A | E146A | A | 623 | C | RBM19 | V457A | A | 1454 | G |
| PCLO | S1684L | G | 5340 | A | PLCH1 | R54Q | C | 161 | T | PRPF38B | R270Q | G | 1078 | A | RBM19 | R360C | G | 1162 | A |
| PCLO | G4741C | C | 14510 | A | PLCH1 | R215Q | C | 644 | T | PRPF39 | G404* | G | 1352 | T | RBM19 | V627M | C | 1963 | T |
| PCLO | R3050* | G | 9437 | A | PLCH1 | Q638* | G | 1912 | A | PRPF39 | - | G | 0 | A | RBM20 | R634Q | G | 1959 | A |
| PCLO | E5010D | T | 15319 | G | PLCH1 | A924T | C | 2770 | T | PRPF4 | R82* | C | 645 | T | RBM20 | R589L | G | 1824 | T |
| PCLO | L1380R | A | 4428 | C | PLCH1 | R505* | G | 1513 | A | PRPF4 | D313N | G | 1338 | A | RBM20 | P44Q | C | 189 | A |
| PCLO | P5090S | G | 15557 | A | PLCH2 | R952Q | G | 3129 | A | PRPF4 | F415L | C | 1646 | A | RBM20 | V487M | G | 1517 | A |
| PCLO | K3645N | T | 11224 | G | PLCH2 | R110H | G | 603 | A | PRPF40A | R929Q | C | 3136 | T | RBM20 | - | G | 0 | T |
| PCLO | A1914V | G | 6030 | A | PLCH2 | A1353P | G | 4331 | C | PRPF40B | I108N | T | 387 | A | RBM22 | R216W | G | 723 | A |
| PCLO | R3843* | T | 11816 | A | PLCH2 | R583C | C | 2021 | T | PRPF40B | R762W | C | 2348 | T | RBM22 | R216W | G | 723 | A |
| PCLO | R4461Q | C | 13671 | T | PLCH2 | R144H | G | 705 | A | PRPF40B | V573M | G | 1781 | A | RBM23 | R193H | C | 777 | T |
| PCLO | E3557* | C | 10958 | A | PLCH2 | R781H | G | 2616 | A | PRPF40B | S798N | G | 2457 | A | RBM23 | E29* | C | 284 | A |
| PCLO | A4060V | G | 12468 | A | PLCL1 | D159Y | G | 873 | T | PRPF4B | A86V | C | 357 | T | RBM25 | R421W | C | 1469 | T |
| PCLO | R3420* | G | 10547 | A | PLCL1 | A759V | C | 2674 | T | PRPF4B | E119K | G | 455 | A | RBM25 | R549H | G | 1854 | A |
| PCLO | E3193D | T | 9868 | G | PLCL1 | R469Q | G | 1804 | A | PRPF4B | R415Q | G | 1344 | A | RBM25 | R391C | C | 1379 | T |
| PCLO | A3124D | G | 9660 | T | PLCL2 | K789R | A | 2780 | G | PRPF6 | R785W | C | 2464 | T | RBM26 | R184Q | C | 992 | T |
| PCLO | R1646I | C | 5226 | A | PLCL2 | E994K | G | 3394 | A | PRPF6 | D42Y | G | 235 | T | RBM26 | R186Q | C | 998 | T |
| PCLO | R5085* | G | 1554 | A | PLCL2 | R44H | G | 545 | A | PRPF6 | E320* | G | 1069 | T | RBM26 | R152C | G | 895 | A |

| | | | | | | | | | | | | | | | | | | |
|---|---|---|---|---|---|---|---|---|---|---|---|---|---|---|---|---|---|---|
| | | | 2 | | | | | | | | | | | | | | | |
| PCLO | I2583M | A | 8038 | C | PLCL2 | L1005M | T | 3427 | A | PRPF6 | R38H | G | 224 | A | RBM26 | - | A | 0 | G |
| PCLO | I2132M | T | 6685 | C | PLCXD2 | R241C | C | 1307 | T | PRPF8 | R409Q | C | 1318 | T | RBM26 | E996D | T | 3429 | A |
| PCLO | R1994I | C | 6270 | A | PLCXD3 | S254N | C | 836 | T | PRPF8 | A543G | G | 1720 | C | RBM26 | P381S | G | 1582 | A |
| PCLO | P1324S | G | 4259 | A | PLCXD3 | P222H | G | 740 | T | PRPF8 | A179G | G | 628 | C | RBM27 | R510K | G | 1695 | A |
| PCLO | K1063N | T | 3478 | A | PLCXD3 | V307A | A | 995 | G | PRPF8 | A1731T | C | 5283 | T | RBM27 | S566G | A | 1862 | G |
| PCM1 | S1287N | G | 4282 | A | PLCXD3 | L229I | G | 760 | T | PRPF8 | E868K | C | 2694 | T | RBM27 | Q586P | A | 1923 | C |
| PCM1 | S643I | G | 2350 | T | PLCXD3 | F61V | A | 256 | C | PRPF8 | H792Y | G | 2466 | A | RBM28 | V75M | C | 338 | T |
| PCM1 | S938N | G | 3235 | A | PLCZ1 | E511* | C | 1795 | A | PRPF8 | - | T | 0 | G | RBM28 | I701T | A | 2217 | G |
| PCM1 | R348H | G | 1465 | A | PLCZ1 | R391* | G | 1435 | A | PRPF8 | K1344T | T | 4123 | G | RBM28 | R582Q | C | 1860 | T |
| PCM1 | R576* | C | 2148 | T | PLCZ1 | R197H | C | 854 | T | PRPF8 | Y394H | A | 1272 | G | RBM28 | T452M | G | 1470 | A |
| PCM1 | T1558A | A | 5094 | G | PLCZ1 | R385Q | C | 1418 | T | PRPH | A394T | G | 2679 | A | RBM28 | E333* | C | 1112 | A |
| PCM1 | P452L | C | 1777 | T | PLD1 | D573N | C | 1844 | T | PRPH2 | V312M | C | 1174 | T | RBM28 | D244G | T | 846 | C |
| PCM1 | P878H | C | 3055 | A | PLD1 | E125* | C | 500 | A | PRPH2 | R248C | G | 982 | A | RBM3 | R75H | G | 287 | A |
| PCM1 | R354C | C | 1482 | T | PLD1 | P507L | G | 1647 | A | PRPH2 | E321A | T | 1202 | G | RBM33 | R1089Q | G | 3464 | A |
| PCM1 | T518P | A | 1974 | C | PLD1 | A462D | G | 1512 | T | PRPS1L1 | D210A | T | 710 | G | RBM33 | R529* | C | 1783 | T |
| PCM1 | N1448H | A | 4764 | C | PLD1 | E281* | C | 968 | A | PRPS1L1 | S58G | T | 253 | C | RBM34 | R272C | G | 1045 | A |
| PCM1 | R1529H | G | 5008 | A | PLD2 | R202C | C | 735 | T | PRPSAP2 | K335T | A | 1416 | C | RBM39 | K467T | T | 1424 | G |
| PCMT1 | M1T | T | 287 | C | PLD2 | R336Q | G | 1138 | A | PRR11 | S139R | T | 488 | G | RBM39 | R289Q | C | 890 | T |
| PCMT1 | K183N | G | 834 | T | PLD2 | R608Q | G | 1954 | A | PRR12 | C2035Y | G | 6104 | A | RBM4 | R95Q | G | 432 | A |
| PCMTD1 | R200Q | C | 940 | T | PLD2 | A854T | G | 2691 | A | PRR12 | R207H | G | 620 | A | RBM41 | T404A | T | 1241 | C |
| PCMTD1 | Y41H | A | 462 | G | PLD2 | S243F | C | 859 | T | PRR12 | L1289F | C | 3865 | T | RBM42 | R181H | G | 618 | A |
| PCMTD1 | R200Q | C | 940 | T | PLD4 | L142R | T | 571 | G | PRR12 | R381C | C | 1141 | T | RBM42 | R102Q | G | 381 | A |
| PCNT | G2553R | G | 7764 | A | PLD4 | T238M | C | 859 | T | PRR12 | T535A | A | 1603 | G | RBM42 | V18I | G | 128 | A |
| PCNT | K3081E | A | 9348 | G | PLD5 | R173S | C | 1029 | G | PRR12 | A106T | G | 316 | A | RBM42 | R455C | C | 1439 | T |
| PCNT | R1285H | G | 3961 | A | PLD6 | R204C | G | 642 | A | PRR12 | A79V | C | 236 | T | RBM43 | E294D | C | 1034 | A |
| PCNT | T275M | C | 931 | T | PLDN | T75I | C | 449 | T | PRR12 | R228H | G | 683 | A | RBM43 | V34I | C | 252 | T |
| PCNT | Q2785* | C | 8460 | T | PLEC | L2864I | G | 8760 | T | PRR14 | R503W | C | 1765 | T | RBM44 | S472R | C | 1548 | A |
| PCNT | R2691W | C | 8178 | T | PLEC | S3479L | G | 10606 | A | PRR14 | P221T | C | 919 | A | RBM44 | N402H | A | 1336 | C |
| PCNT | T2007M | C | 6127 | T | PLEC | R3991H | C | 12142 | T | PRR14 | R532Q | G | 1853 | A | RBM44 | R919I | G | 2888 | T |
| PCNT | L2251P | T | 6859 | C | PLEC | S2257T | A | 6939 | T | PRR16 | R206W | C | 825 | T | RBM45 | S108* | C | 415 | A |
| PCNT | A493P | G | 1584 | C | PLEC | S984N | C | 3121 | T | PRR19 | V63I | G | 565 | A | RBM46 | S385Y | C | 1389 | A |
| PCNT | R1923* | C | 5874 | T | PLEC | A4398T | C | 13362 | T | PRR19 | V62M | G | 562 | A | RBM46 | G18C | G | 287 | T |
| PCNT | E1503G | A | 4615 | G | PLEC | T1859M | G | 5746 | A | PRR23A | R262* | G | 784 | A | RBM46 | Q217P | A | 885 | C |
| PCNT | L2001V | C | 6108 | G | PLEC | A1182V | G | 3715 | A | PRR23A | A114V | G | 341 | A | RBM47 | I262F | T | 1181 | A |
| PCNT | S1039N | G | 3223 | A | PLEC | A411T | C | 1401 | T | PRR23A | P101S | G | 301 | A | RBM47 | K279N | C | 1234 | A |
| PCNT | R2663C | C | 8094 | T | PLEC | R3935W | G | 1197 | A | PRR23A | A65T | C | 193 | T | RBM4B | R347W | G | 1092 | A |

| | | | | | | | | | | | | | | | | | | |
|---|---|---|---|---|---|---|---|---|---|---|---|---|---|---|---|---|---|---|
| | | | | | | | | 3 | | | | | | | | | | |
| PCNX | R2159* | C | 6921 | T | PLEC | T2504M | G | 7681 | A | PRR23A | S86L | G | 257 | A | RBM4B | A238V | G | 766 | A |
| PCNX | G1968D | G | 6349 | A | PLEC | R2383C | G | 7317 | A | PRR23B | A114V | G | 606 | A | RBM5 | S92N | G | 450 | A |
| PCNX | H2104R | A | 6757 | G | PLEC | R728H | C | 2353 | T | PRR23C | V137I | C | 681 | T | RBM5 | E73K | G | 392 | A |
| PCNX | I1378N | T | 4579 | A | PLEC | V949M | C | 3015 | T | PRR24 | Y51C | A | 152 | G | RBM5 | K162N | A | 661 | C |
| PCNX | A688D | C | 2509 | A | PLEC | R3909H | C | 11896 | T | PRR25 | S127Y | C | 380 | A | RBM5 | R722H | G | 2340 | A |
| PCNXL2 | T1893M | G | 5913 | A | PLEC | R3446H | C | 10507 | T | PRR5 | G297D | G | 1499 | A | RBM6 | V877I | G | 2888 | A |
| PCNXL2 | L1985F | G | 6188 | A | PLEC | E3132K | C | 9564 | T | PRR5-ARHGAP8 | S592N | G | 1775 | A | RBM7 | V21M | G | 117 | A |
| PCNXL2 | C1634R | A | 5135 | G | PLEC | R1159H | C | 3646 | T | PRR5-ARHGAP8 | E417K | G | 1249 | A | RBM8A | L7H | T | 89 | A |
| PCNXL2 | K818M | T | 2688 | A | PLEC | R3897H | C | 11860 | T | PRRG4 | L7P | T | 273 | C | RBM9 | T154M | G | 461 | A |
| PCNXL2 | C467R | A | 1634 | G | PLEC | R1317L | C | 4120 | A | PRRT2 | T151I | C | 735 | T | RBM9 | R442* | G | 1324 | A |
| PCNXL2 | A1461T | C | 4616 | T | PLEC | R4589H | C | 13936 | T | PRRT3 | A438V | G | 1443 | A | RBMS2P1 | R195Q | C | 584 | T |
| PCNXL2 | V2001I | C | 6236 | T | PLEC | T4080A | T | 12408 | C | PRRT4 | A445D | G | 1531 | T | RBMS3 | T350M | C | 1385 | T |
| PCNXL2 | S1035L | G | 3339 | A | PLEC | A2357T | C | 7239 | T | PRRT4 | R763C | G | 2484 | A | RBMS3 | P269L | C | 1142 | T |
| PCNXL3 | R410L | G | 1229 | T | PLEC | G478C | C | 1602 | A | PRRT4 | R413Q | C | 1435 | T | RBMS3 | M321I | G | 1299 | T |
| PCNXL3 | S996Y | C | 2987 | A | PLEC | R2575* | G | 7893 | A | PRRT4 | G395S | C | 1380 | T | RBMX | H128Y | G | 537 | A |
| PCNXL3 | R858H | G | 2573 | A | PLEK | R174C | C | 699 | T | PRRX1 | V172M | G | 827 | A | RBMX2 | E250K | G | 812 | A |
| PCNXL3 | P726L | C | 2177 | T | PLEK | R119Q | G | 535 | A | PRRX1 | F34L | C | 415 | A | RBMXL1 | P198Q | G | 1021 | T |
| PCNXL3 | V1245M | G | 3733 | A | PLEK | F326L | C | 1157 | A | PRSS12 | M806L | T | 2699 | G | RBMXL2 | R268H | G | 990 | A |
| PCNXL3 | R1880W | C | 5638 | T | PLEK | T73K | C | 397 | A | PRSS16 | V71M | G | 226 | A | RBMXL2 | G287R | G | 1046 | A |
| PCNXL3 | R1177Q | G | 3530 | A | PLEK2 | V5E | A | 66 | T | PRSS21 | A307D | C | 962 | A | RBMXL2 | V54I | G | 347 | A |
| PCOLCE | - | T | 0 | C | PLEK2 | R131H | C | 444 | T | PRSS22 | I260V | T | 844 | C | RBMXL2 | R195H | G | 771 | A |
| PCOLCE | G100V | G | 579 | T | PLEKHA1 | R28K | G | 214 | A | PRSS22 | W134* | C | 467 | T | RBMXL2 | G287R | G | 1046 | A |
| PCOLCE2 | V294M | C | 1187 | T | PLEKHA1 | D388Y | G | 1293 | T | PRSS23 | E65K | G | 370 | A | RBMXL3 | G384R | G | 1165 | A |
| PCP4 | R41C | C | 306 | T | PLEKHA2 | V206M | G | 850 | A | PRSS23 | N3D | A | 184 | G | RBMXL3 | S159R | C | 492 | G |
| PCSK1 | R669Q | C | 2244 | T | PLEKHA2 | R421W | C | 1495 | T | PRSS23 | K182T | A | 722 | C | RBMXL3 | A207D | C | 635 | A |
| PCSK1 | R438Q | C | 1551 | T | PLEKHA3 | R240Q | G | 1121 | A | PRSS27 | D223N | C | 722 | T | RBMXL3 | G100S | G | 313 | A |
| PCSK1 | N316H | T | 1184 | G | PLEKHA4 | R204H | C | 1136 | T | PRSS27 | E200K | C | 653 | T | RBMXL3 | R746H | G | 2252 | A |
| PCSK1N | P215A | G | 744 | C | PLEKHA4 | R238C | G | 1237 | A | PRSS3 | E89D | G | 267 | T | RBP1 | N76D | T | 337 | C |
| PCSK2 | T31M | C | 407 | T | PLEKHA4 | R204C | G | 1135 | A | PRSS42 | A29V | G | 86 | A | RBP2 | K102R | T | 362 | C |
| PCSK2 | E625K | G | 2188 | A | PLEKHA4 | P656L | G | 2492 | A | PRSS42 | W93* | C | 279 | T | RBP3 | G967S | C | 3013 | T |
| PCSK2 | P142H | C | 740 | A | PLEKHA4 | F779S | A | 2861 | G | PRSS45 | A130V | G | 389 | A | RBP3 | G967S | C | 3013 | T |
| PCSK4 | A405T | C | 1275 | T | PLEKHA4 | R225Q | C | 1199 | T | PRSS46 | W245* | C | 811 | T | RBP3 | A450T | C | 1462 | T |
| PCSK4 | E414D | C | 1304 | A | PLEKHA5 | S379R | A | 1221 | C | PRSS46 | R3C | G | 83 | A | RBP3 | T471M | G | 1526 | A |

| | | | | | | | | | | | | | | | | | | | |
|---|---|---|---|---|---|---|---|---|---|---|---|---|---|---|---|---|---|---|---|
| PCSK4 | W122* | C | 427 | T | PLEKHA5 | - | G | 0 | A | PRSS48 | R50C | C | 148 | T | RBP3 | P786T | G | 2470 | T |
| PCSK4 | E196D | C | 650 | A | PLEKHA5 | E1216* | G | 3732 | T | PRSS53 | R99C | G | 449 | A | RBP3 | T943M | G | 2942 | A |
| PCSK5 | - | G | 0 | A | PLEKHA6 | R876Q | C | 2944 | T | PRSS53 | Y381C | T | 1296 | C | RBP3 | A910V | G | 2843 | A |
| PCSK6 | R510M | C | 1529 | A | PLEKHA6 | Q654R | T | 2278 | C | PRSS54 | A106T | C | 711 | T | RBP3 | E394* | C | 1294 | A |
| PCSK6 | K490N | C | 1470 | A | PLEKHA6 | R465C | G | 1710 | A | PRSS54 | L132H | A | 790 | T | RBP7 | R22* | C | 71 | T |
| PCSK6 | - | C | 0 | T | PLEKHA6 | P289S | G | 1182 | A | PRSS54 | V32I | C | 489 | T | RBPJ | C92Y | G | 511 | A |
| PCSK6 | D174N | C | 520 | T | PLEKHA7 | R713* | G | 2148 | A | PRSS54 | E379K | C | 1530 | T | RBPJL | T249M | C | 818 | T |
| PCSK7 | W678C | C | 2235 | A | PLEKHA7 | S117P | A | 360 | G | PRSS55 | P258T | C | 812 | A | RBPJL | K79T | A | 308 | C |
| PCSK7 | R429C | G | 1486 | A | PLEKHA7 | R703M | C | 2119 | A | PRSS55 | L144F | C | 470 | T | RBPMS | R38Q | G | 778 | A |
| PCSK7 | D221Y | C | 862 | A | PLEKHA7 | E133Q | C | 408 | G | PRSS55 | G95C | G | 323 | T | RBPMS | A7S | G | 684 | T |
| PCSK7 | - | C | 0 | T | PLEKHB1 | E63D | G | 620 | T | PRSS55 | E188K | G | 602 | A | RBPMS2 | A184T | C | 818 | T |
| PCSK9 | R251C | C | 1041 | T | PLEKHF1 | N155K | C | 465 | G | PRSS8 | R265C | G | 1125 | A | RBPMS2 | R137W | G | 677 | A |
| PCSK9 | R499H | G | 1786 | A | PLEKHF2 | G65D | G | 435 | A | PRTG | R689K | C | 2114 | T | RC3H1 | R852* | G | 2633 | A |
| PCSK9 | A478T | G | 1722 | A | PLEKHG1 | R765W | C | 2605 | T | PRTG | D1135N | C | 3451 | T | RC3H1 | - | C | 0 | A |
| PCYOX1 | D493Y | G | 1505 | T | PLEKHG1 | A968V | C | 3215 | T | PRTG | F667L | G | 2049 | T | RC3H1 | P562A | G | 1763 | C |
| PCYT1A | A217V | G | 660 | A | PLEKHG1 | G711D | G | 2444 | A | PRTG | S267F | G | 848 | A | RC3H1 | R26* | G | 155 | A |
| PCYT1B | L69S | A | 337 | G | PLEKHG1 | S1214A | T | 3952 | G | PRTG | E148K | C | 490 | T | RC3H2 | R699H | C | 2697 | T |
| PCYT1B | T194M | G | 712 | A | PLEKHG2 | R285H | G | 1704 | A | PRUNE | H19Y | C | 211 | T | RC3H2 | A69T | C | 806 | T |
| PCYT1B | R211C | G | 762 | A | PLEKHG2 | S906P | T | 3566 | C | PRUNE | P369T | C | 1261 | A | RCAN3 | A163V | C | 801 | T |
| PCYT1B | D82N | C | 375 | T | PLEKHG2 | W694* | G | 2932 | A | PRUNE2 | W1868C | C | 5728 | A | RCBTB2 | V178G | A | 824 | C |
| PCYT2 | R171L | C | 575 | A | PLEKHG3 | L994P | T | 3128 | C | PRUNE2 | R2704* | G | 8234 | A | RCBTB2 | - | A | 0 | G |
| PDCD1 | R204C | G | 680 | A | PLEKHG3 | R106C | C | 463 | T | PRUNE2 | P853L | G | 2682 | A | RCC1 | G211* | G | 989 | T |
| PDCD1 | P21S | G | 131 | A | PLEKHG3 | D335N | G | 1150 | A | PRUNE2 | G2197D | C | 6714 | T | RCC1 | R170C | C | 866 | T |
| PDCD10 | E140* | C | 741 | A | PLEKHG3 | L232I | C | 841 | A | PRUNE2 | E780K | C | 2462 | T | RCC2 | R415H | C | 1432 | T |
| PDCD11 | A1365V | C | 4188 | T | PLEKHG3 | A606T | G | 1963 | A | PRUNE2 | I2572L | T | 7838 | G | RCN1 | S69P | T | 471 | C |
| PDCD11 | A1233V | C | 3792 | T | PLEKHG4 | S988N | G | 5498 | A | PRUNE2 | S1859Y | G | 5700 | T | RCN1 | K294T | A | 1147 | C |
| PDCD11 | P845T | C | 2627 | A | PLEKHG4 | L897R | T | 5225 | G | PRUNE2 | E1571D | T | 4837 | G | RCOR1 | R371* | C | 1111 | T |
| PDCD11 | R1590H | G | 4863 | A | PLEKHG4 | T312M | C | 3470 | T | PRUNE2 | D633Y | C | 2021 | A | RCOR1 | K221T | A | 662 | C |
| PDCD11 | E114D | A | 436 | C | PLEKHG4 | - | G | 0 | A | PRUNE2 | K118N | T | 478 | G | RCOR2 | - | A | 0 | G |
| PDCD2 | K144R | T | 460 | C | PLEKHG4 | V816M | G | 4981 | A | PRX | V832A | A | 2764 | G | RCOR2 | S68N | C | 591 | T |
| PDCD2L | S290T | G | 902 | C | PLEKHG4B | V412I | G | 1284 | A | PRX | P1146H | G | 3706 | T | RCSD1 | R175* | C | 854 | T |
| PDCD4 | R407I | G | 1494 | T | PLEKHG4B | G1251D | G | 3802 | A | PRX | R1371W | G | 4380 | A | RCVRN | G115R | C | 530 | T |
| PDCD6IP | I323M | T | 1633 | G | PLEKHG4B | E966K | G | 2946 | A | PRX | G877D | C | 2899 | T | RCVRN | E27D | C | 268 | A |
| PDCD6IP | G63D | G | 852 | A | PLEKHG5 | G970V | C | 3407 | A | PRX | A700V | G | 2368 | A | RD3 | R190W | G | 1732 | A |
| PDCD6IP | L68F | C | 866 | T | PLEKHG6 | P123S | C | 515 | T | PRX | A1035T | C | 3372 | T | RDH10 | L40P | T | 379 | C |
| PDCD6IP | V841M | G | 3185 | A | PLEKHH1 | L156P | T | 609 | C | PSAPL1 | A72V | G | 309 | A | RDH10 | F215V | T | 903 | G |
| PDCL | G191D | C | 738 | T | PLEKHH1 | S333N | G | 1140 | A | PSAPL1 | D186Y | C | 650 | A | RDH10 | F220C | T | 919 | G |
| PDCL | R141Q | C | 588 | T | PLEKHH2 | V888I | G | 2772 | A | PSAPL1 | P9S | G | 119 | A | RDH11 | K165N | C | 585 | A |
| PDDC1 | V157I | C | 494 | T | PLEKHH2 | A73V | C | 328 | T | PSD | E706* | C | 2643 | A | RDH11 | R108* | G | 412 | A |

| | | | | | | | | | | | | | | | | | | | |
|---|---|---|---|---|---|---|---|---|---|---|---|---|---|---|---|---|---|---|---|
| PDDC1 | V153M | C | 482 | T | PLEKHH2 | S183L | C | 658 | T | PSD2 | T671I | C | 2217 | T | RDH13 | A46T | C | 136 | T |
| PDE10A | T25P | T | 156 | G | PLEKHH2 | G427V | G | 1390 | T | PSD3 | - | A | 3251 | G | RDH13 | L153W | A | 458 | C |
| PDE10A | G390C | C | 1251 | A | PLEKHH3 | R384C | G | 1617 | A | PSD3 | R328I | C | 1086 | A | RDH13 | K74T | T | 221 | G |
| PDE10A | D278Y | C | 915 | A | PLEKHH3 | A276V | G | 1294 | A | PSD4 | S38N | G | 308 | A | RDH16 | P293T | G | 1049 | T |
| PDE11A | R413Q | C | 1556 | T | PLEKHH3 | G155S | C | 930 | T | PSD4 | L250I | C | 943 | A | RDH16 | L171P | A | 684 | G |
| PDE11A | A130V | G | 707 | A | PLEKHH3 | G155S | C | 930 | T | PSD4 | T53I | C | 353 | T | RDH5 | R75C | C | 406 | T |
| PDE11A | A373T | C | 1435 | T | PLEKHH3 | T626M | G | 2344 | A | PSD4 | R868C | C | 2797 | T | RDM1 | F86V | A | 283 | C |
| PDE11A | - | C | 0 | T | PLEKHH3 | R104W | G | 777 | A | PSEN1 | R42W | C | 396 | T | RDX | R569* | G | 2015 | A |
| PDE11A | R844W | G | 2848 | A | PLEKHJ1 | R49Q | C | 218 | T | PSENEN | W74* | G | 426 | A | RDX | F102L | G | 616 | T |
| PDE11A | P538H | G | 1931 | T | PLEKHM1 | A380S | C | 1272 | A | PSG11 | G229E | C | 761 | T | REC8 | E460Q | G | 1937 | C |
| PDE11A | I212V | T | 952 | C | PLEKHM1P | V501I | C | 1501 | T | PSG3 | R397H | C | 1375 | T | REC8 | Q524H | G | 2131 | C |
| PDE12 | A17V | C | 153 | T | PLEKHM2 | Q982* | C | 2944 | T | PSG3 | S408A | A | 1407 | C | RECK | D891N | G | 3237 | A |
| PDE1A | N216T | T | 647 | G | PLEKHM2 | G221S | G | 661 | A | PSG4 | R127* | G | 617 | A | RECK | I888V | A | 3228 | G |
| PDE1B | N52H | A | 320 | C | PLEKHM2 | R508C | C | 1522 | T | PSG5 | A117T | C | 480 | T | RECK | - | G | 0 | T |
| PDE1B | K358T | A | 1239 | C | PLEKHM3 | N485S | T | 1583 | C | PSG6 | T300M | G | 944 | A | RECK | V847L | G | 3105 | T |
| PDE1B | R89Q | G | 432 | A | PLEKHM3 | R505* | G | 1642 | A | PSG7 | K90T | T | 366 | G | RECQL | Y446C | T | 1685 | C |
| PDE1C | R344Q | C | 1625 | T | PLEKHM3 | M315I | C | 1074 | A | PSG7 | A34S | C | 197 | A | RECQL | - | A | 0 | G |
| PDE1C | A310V | G | 1523 | A | PLEKHN1 | P270T | C | 843 | A | PSG9 | Y120C | T | 456 | C | RECQL | I426L | T | 1624 | G |
| PDE1C | R390* | G | 1762 | A | PLEKHN1 | H634R | A | 1936 | G | PSKH1 | R137W | C | 579 | T | RECQL | H340N | G | 1366 | T |
| PDE1C | R540Q | C | 2213 | T | PLEKHN1 | Q579* | C | 1770 | T | PSKH1 | R369H | G | 1276 | A | RECQL | E176D | T | 876 | G |
| PDE1C | R478Q | C | 2027 | T | PLEKHO2 | R80Q | G | 367 | A | PSKH1 | R302H | G | 1075 | A | RECQL4 | T690A | T | 2100 | C |
| PDE1C | V495M | C | 2077 | T | PLEKHO2 | T423M | C | 1396 | T | PSKH2 | V161I | C | 556 | T | RECQL4 | P1040L | G | 3151 | A |
| PDE2A | P948H | G | 2843 | T | PLEKHO2 | A293E | C | 1006 | A | PSKH2 | A354T | C | 1135 | T | RECQL4 | R758* | G | 2304 | A |
| PDE2A | Q261* | G | 781 | A | PLEKHO2 | G169S | G | 633 | A | PSKH2 | A32T | C | 169 | T | RECQL4 | S1169I | C | 3538 | A |
| PDE2A | - | C | 0 | T | PLG | A419V | C | 1319 | T | PSKH2 | S350I | C | 1124 | A | RECQL4 | G170C | C | 540 | A |
| PDE2A | M909T | A | 2726 | G | PLG | D177N | G | 592 | A | PSKH2 | A32T | C | 169 | T | RECQL5 | R872H | C | 2771 | T |
| PDE2A | K716N | C | 2148 | A | PLG | K664Q | A | 2053 | C | PSMA1 | R9* | G | 25 | A | RECQL5 | A981V | G | 3098 | A |
| PDE2A | V646A | A | 1937 | G | PLIN1 | V234M | C | 866 | T | PSMA2 | G78D | C | 249 | T | RECQL5 | R872H | C | 2771 | T |
| PDE2A | D285Y | C | 853 | A | PLIN2 | K435N | T | 1485 | G | PSMA3 | - | G | 0 | T | RECQL5 | H500Q | G | 1656 | T |
| PDE3A | G45S | G | 155 | A | PLIN3 | A200V | G | 672 | A | PSMA3 | E226* | G | 766 | T | RECQL5 | R696W | G | 2242 | A |
| PDE3A | D34E | C | 124 | G | PLIN3 | A224T | C | 743 | T | PSMA7 | S30L | G | 216 | A | RECQL5 | P694S | G | 2236 | A |
| PDE3A | S446L | C | 1359 | T | PLIN4 | V719I | C | 2155 | T | PSMA8 | G16R | G | 160 | A | REEP2 | E52K | G | 346 | A |
| PDE3A | S863L | C | 2610 | T | PLIN4 | R1290Q | C | 3869 | T | PSMB11 | R106W | C | 375 | T | REEP3 | R113* | C | 520 | T |
| PDE3A | E171K | G | 533 | A | PLIN5 | D242G | T | 778 | C | PSMB11 | P85H | C | 313 | A | REEP4 | R181M | C | 1011 | A |
| PDE3B | S613A | T | 2190 | G | PLK1 | R563C | C | 1798 | T | PSMB4 | R216C | C | 700 | T | REEP5 | - | C | 0 | A |
| PDE4A | W34* | G | 210 | A | PLK1 | S49N | G | 257 | A | PSMB7 | R124W | G | 384 | A | REEP5 | V155A | A | 813 | G |
| PDE4A | A765V | C | 2402 | T | PLK1 | T440A | A | 1429 | G | PSMC2 | D29N | G | 496 | A | REEP6 | R120W | C | 402 | T |
| PDE4A | R696M | G | 2195 | T | PLK1 | S461F | C | 1493 | T | PSMC2 | S380N | G | 1550 | A | REEP6 | Q194R | A | 625 | G |
| PDE4A | R409H | G | 1334 | A | PLK1 | R313H | G | 1049 | A | PSMC4 | A320T | G | 995 | A | REEP6 | R206C | C | 660 | T |

| | | | | | | | | | | | | | | | | | | | |
|---|---|---|---|---|---|---|---|---|---|---|---|---|---|---|---|---|---|---|---|
| PDE4B | D620N | G | 2045 | A | PLK1 | E186* | G | 667 | T | PSMC4 | D148N | G | 479 | A | REG1A | S69L | C | 309 | T |
| PDE4B | Q216H | A | 835 | C | PLK1 | R579Q | G | 1847 | A | PSMC4 | R229W | C | 722 | T | REG3G | M62T | T | 284 | C |
| PDE4C | R489H | C | 1705 | T | PLK1 | - | G | 0 | T | PSMC4 | A314T | G | 977 | A | REG3G | M62I | G | 285 | A |
| PDE4C | V593A | A | 2017 | G | PLK1 | A292T | G | 985 | A | PSMC6 | E49* | G | 151 | T | REL | V148A | T | 763 | C |
| PDE4C | A338E | G | 1252 | T | PLK1 | M486I | G | 1569 | A | PSMD1 | V134I | G | 562 | A | REL | S422P | T | 1584 | C |
| PDE4C | D453N | C | 1596 | T | PLK2 | R446W | G | 1637 | A | PSMD1 | P125T | C | 535 | A | RELA | R330C | G | 1128 | A |
| PDE4D | - | C | 0 | A | PLK2 | L230Q | A | 990 | T | PSMD10 | T53P | T | 255 | G | RELA | R267C | G | 939 | A |
| PDE4DIP | R1226H | C | 3865 | T | PLK2 | R480W | G | 1739 | A | PSMD11 | H375N | C | 1163 | A | RELL1 | E42* | C | 213 | A |
| PDE4DIP | - | C | 0 | T | PLK2 | T298A | T | 1193 | C | PSMD12 | E216K | C | 754 | T | RELL2 | R243G | A | 1575 | G |
| PDE4DIP | R12H | C | 35 | T | PLK3 | R638H | G | 2152 | A | PSMD14 | L184S | T | 1255 | C | RELN | Q2806H | C | 8578 | A |
| PDE4DIP | R1855H | C | 5752 | T | PLK4 | F12C | T | 309 | G | PSMD14 | A93T | G | 981 | A | RELN | R2292H | C | 7035 | T |
| PDE4DIP | - | C | 0 | A | PLK4 | R374H | G | 1395 | A | PSMD14 | C238R | T | 1416 | C | RELN | A3083T | C | 9407 | T |
| PDE4DIP | A910D | G | 2917 | T | PLLP | A118T | C | 499 | T | PSMD3 | Q377R | A | 1304 | G | RELN | G1420V | C | 4419 | A |
| PDE4DIP | E624G | T | 2059 | C | PLLP | F145L | G | 582 | T | PSMD4 | K74E | A | 294 | G | RELN | T508A | T | 1682 | C |
| PDE4DIP | L1441I | G | 4509 | T | PLN | A11V | C | 224 | T | PSMD4 | D261N | G | 855 | A | RELN | G1540V | C | 4779 | A |
| PDE4DIP | T1292A | T | 4062 | C | PLOD1 | D566N | G | 1723 | A | PSMD4 | T367I | C | 1174 | T | RELN | S2304R | T | 7070 | G |
| PDE4DIP | R174I | C | 709 | A | PLOD1 | R457W | C | 1396 | T | PSMD5 | R214I | C | 644 | A | RELN | K2889T | T | 8826 | G |
| PDE5A | V54I | C | 480 | T | PLOD1 | R477H | G | 1457 | A | PSMD6 | D34N | C | 153 | T | RELN | N3438K | A | 1047 4 | C |
| PDE5A | A572T | C | 2034 | T | PLOD1 | D226N | G | 703 | A | PSMD6 | V37M | C | 162 | T | RELN | R3326* | G | 1013 6 | A |
| PDE5A | R577W | G | 2049 | A | PLOD2 | N82S | T | 423 | C | PSMD8 | N70D | A | 274 | G | RELN | A3079D | G | 9396 | T |
| PDE5A | D248N | C | 1062 | T | PLOD2 | R680Q | C | 2217 | T | PSMD8 | K53N | A | 225 | T | RELN | - | C | 0 | A |
| PDE6A | P389L | G | 1286 | A | PLOD2 | R530I | C | 1767 | A | PSMD8 | R13L | G | 104 | T | RELN | R2428W | G | 7442 | A |
| PDE6A | F162L | G | 606 | T | PLOD3 | R498* | G | 1891 | A | PSMD9 | A151V | C | 569 | T | RELN | L2184S | A | 6711 | G |
| PDE6B | R542W | C | 1645 | T | PLP2 | L28M | C | 157 | A | PSME2 | R203W | G | 635 | A | RELN | W1970C | C | 6070 | A |
| PDE6B | F200L | C | 621 | A | PLS3 | K23N | A | 156 | C | PSME4 | R704I | C | 2167 | A | RELN | F1508L | A | 4684 | C |
| PDE6C | I184V | A | 688 | G | PLSCR2 | V224M | C | 1110 | T | PSME4 | R370C | G | 1164 | A | RELN | I859S | A | 2736 | C |
| PDE7B | R307* | C | 919 | T | PLSCR2 | A57T | C | 609 | T | PSME4 | G1711S | C | 5187 | T | RELN | R274C | G | 980 | A |
| PDE8A | R417C | C | 1501 | T | PLSCR2 | M74I | C | 662 | T | PSME4 | R1131H | C | 3448 | T | RELT | L185I | C | 718 | A |
| PDE8B | T47S | A | 184 | T | PLSCR2 | K31E | T | 531 | C | PSME4 | R1658* | G | 5028 | A | REM1 | R181H | G | 835 | A |
| PDE8B | R436C | C | 1351 | T | PLSCR2 | N209T | T | 1066 | G | PSME4 | W242* | C | 782 | T | REM2 | R235H | G | 769 | A |
| PDE8B | N541K | T | 1668 | G | PLSCR3 | R109H | C | 535 | T | PSME4 | T338A | T | 1068 | C | REN | P25L | G | 103 | A |
| PDE8B | D656N | G | 2011 | A | PLSCR4 | Y60* | G | 421 | T | PSME4 | E600* | C | 1854 | A | REN | G11E | C | 61 | T |
| PDE8B | R436H | G | 1352 | A | PLSCR4 | R247C | G | 980 | A | PSME4 | E559A | T | 1732 | G | REN | E144* | C | 459 | A |
| REPIN1 | W126* | G | 571 | A | SASH3 | V384M | G | 1264 | A | SKA2L | L126V | T | 385 | G | SMURF2 | R172I | C | 903 | A |
| REPS1 | E328K | C | 1259 | T | SATB1 | A457D | G | 3099 | T | SKA3 | R327C | G | 1073 | A | SMURF2 | R187H | C | 948 | T |
| REPS1 | R183H | C | 825 | T | SATB2 | E678A | T | 2850 | G | SKA3 | L341V | A | 1115 | C | SMYD1 | F456L | C | 1453 | A |
| REPS1 | Q772* | G | 2591 | A | SATB2 | R239* | G | 1532 | A | SKAP1 | R163W | G | 599 | A | SMYD1 | A192V | C | 660 | T |
| REPS1 | F233V | A | 974 | C | SATB2 | A314T | C | 1757 | T | SKAP2 | I260M | A | 1094 | C | SMYD2 | K127T | A | 402 | C |

| | | | | | | | | | | | | | | | | | | | |
|---|---|---|---|---|---|---|---|---|---|---|---|---|---|---|---|---|---|---|---|
| REPS1 | S162F | G | 762 | A | SATL1 | A248T | C | 822 | T | SKI | A534T | G | 1672 | A | SMYD3 | V43I | C | 344 | T |
| REPS2 | R628W | C | 2053 | T | SATL1 | F618L | A | 1932 | G | SKI | A540V | C | 1691 | T | SMYD3 | F303S | A | 1125 | G |
| RER1 | P89H | C | 399 | A | SATL1 | P590T | G | 1848 | T | SKI | A644V | C | 2003 | T | SMYD4 | S203N | C | 745 | T |
| RERE | A1073V | G | 3853 | A | SBF1 | R1719C | G | 5178 | A | SKI | G552S | G | 1726 | A | SMYD4 | L370F | G | 1245 | A |
| RERE | Q500H | C | 2135 | A | SBF1 | M695R | A | 2107 | C | SKI | S206N | G | 689 | A | SMYD5 | Q152H | G | 501 | T |
| RERE | P788L | G | 2998 | A | SBF1 | A495V | G | 1507 | A | SKI | S151L | C | 524 | T | SMYD5 | R52W | C | 199 | T |
| RERE | S1128G | T | 4017 | C | SBF1 | D150N | C | 471 | T | SKI | S508R | C | 1596 | A | SMYD5 | L107F | C | 364 | T |
| RERG | V17M | C | 339 | T | SBF1 | T275I | G | 847 | A | SKIL | V534I | G | 2271 | A | SMYD5 | A38T | G | 157 | A |
| REST | A991T | G | 3285 | A | SBF2 | P867S | G | 2737 | A | SKIV2L2 | V857A | T | 2824 | C | SMYD5 | D142N | G | 469 | A |
| REST | K249T | A | 1060 | C | SBF2 | A1829T | C | 5623 | T | SKP2 | R154Q | G | 637 | A | SNAI2 | K239R | T | 880 | C |
| RET | F174L | T | 752 | C | SBF2 | L433F | C | 1437 | A | SLA | R173C | G | 595 | A | SNAI2 | P17Q | G | 214 | T |
| RET | V648I | G | 2174 | A | SBF2 | V1341I | C | 4159 | T | SLA | A30T | C | 166 | T | SNAI2 | R205I | C | 778 | A |
| RET | - | G | 0 | T | SBK1 | V246M | G | 1525 | A | SLAIN2 | R559C | C | 2093 | T | SNAI3 | S286Y | G | 925 | T |
| RET | P841L | C | 2754 | T | SBK1 | A131T | G | 1180 | A | SLAMF1 | E101D | C | 653 | A | SNAI3 | R282W | G | 912 | A |
| RET | R959Q | G | 3108 | A | SBK2 | A230T | C | 726 | T | SLAMF1 | R95H | C | 634 | T | SNAP29 | Y189* | C | 695 | A |
| RET | R250C | C | 980 | T | SBK2 | V76I | C | 264 | T | SLAMF8 | R165* | C | 642 | T | SNAP47 | A172T | G | 928 | A |
| RETSAT | R125H | C | 487 | T | SBK2 | D301A | T | 940 | G | SLAMF8 | H222Y | C | 813 | T | SNAP47 | R39H | G | 530 | A |
| RETSAT | R453Q | C | 1471 | T | SBK2 | E106K | C | 354 | T | SLAMF8 | E159K | G | 624 | A | SNAP47 | M332T | T | 1409 | C |
| RETSAT | R125C | G | 486 | A | SBNO1 | W969* | C | 2907 | T | SLAMF9 | F244V | A | 847 | C | SNAP91 | T277M | G | 1424 | A |
| REV1 | P933L | G | 3027 | A | SBNO1 | T455S | T | 1363 | A | SLBP | R188H | C | 625 | T | SNAP91 | A481D | G | 2036 | T |
| REV1 | S417N | C | 1479 | T | SBNO1 | D156Y | C | 466 | A | SLBP | E91* | C | 333 | A | SNAPC1 | W226* | G | 764 | A |
| REV1 | F1165L | G | 3724 | T | SBNO1 | D7N | C | 19 | T | SLC10A1 | V210G | A | 763 | C | SNAPC1 | R47I | G | 226 | T |
| REV1 | E738D | T | 2443 | G | SBNO2 | A478T | C | 1432 | T | SLC10A1 | A67V | G | 334 | A | SNAPC2 | R262C | C | 807 | T |
| REV3L | Q329P | T | 1309 | G | SBNO2 | S662N | C | 1985 | T | SLC10A1 | R180W | G | 672 | A | SNAPC3 | V14A | T | 217 | C |
| REV3L | M2934V | T | 9123 | C | SBNO2 | A199E | G | 596 | T | SLC10A2 | A97V | G | 887 | A | SNAPC3 | T366M | C | 1273 | T |
| REV3L | T2343I | G | 7351 | A | SBNO2 | A338T | C | 1012 | T | SLC10A2 | P65S | G | 790 | A | SNAPC4 | R1352W | G | 4423 | A |
| REV3L | R882H | C | 2968 | T | SC4MOL | I225V | A | 846 | G | SLC10A3 | E514K | C | 1638 | T | SNAPC4 | R508W | G | 1891 | A |
| REV3L | A2775V | G | 8647 | A | SC4MOL | Y168H | T | 675 | C | SLC10A5 | K129T | T | 386 | G | SNAPC4 | R428W | G | 1651 | A |
| REV3L | S640Y | G | 2242 | T | SC5DL | R74C | C | 368 | T | SLC10A6 | V176A | A | 675 | G | SNAPC4 | R730C | G | 2557 | A |
| REXO1 | E96K | C | 381 | T | SCAF1 | A324T | G | 1094 | A | SLC10A6 | H238N | G | 860 | T | SNAPC4 | K265N | C | 1164 | A |
| RFC1 | D167Y | C | 633 | A | SCAF1 | V995M | G | 3107 | A | SLC10A6 | K322N | C | 1114 | A | SNCAIP | N399T | A | 1402 | C |
| RFC1 | A122T | C | 498 | T | SCAI | R531* | G | 1650 | A | SLC12A1 | G478R | G | 1648 | A | SNCAIP | R350Q | G | 1255 | A |
| RFC1 | K1125T | T | 3508 | G | SCAI | T414P | T | 1299 | G | SLC12A1 | T18N | C | 269 | A | SNCAIP | S186L | C | 763 | T |
| RFC1 | D639Y | C | 2049 | A | SCAI | R132* | G | 453 | A | SLC12A1 | E794D | G | 2598 | T | SNCAIP | S418Y | C | 1459 | A |
| RFC1 | I469T | A | 1540 | G | SCAI | R584* | G | 1809 | A | SLC12A1 | Y89H | T | 481 | C | SNCAIP | L483I | C | 1653 | A |
| RFC1 | K263Q | T | 921 | G | SCAMP5 | A215T | G | 805 | A | SLC12A1 | A510S | G | 1744 | T | SNCG | A85V | C | 296 | T |
| RFC3 | K81N | A | 373 | C | SCAMP5 | S104R | A | 472 | C | SLC12A1 | L512R | T | 1751 | G | SND1 | L627V | C | 2073 | G |
| RFC4 | N317H | T | 1231 | G | SCAND3 | E177K | C | 1147 | T | SLC12A1 | N1051I | A | 3368 | T | SND1 | Y682C | A | 2239 | G |
| RFC5 | T67A | A | 317 | G | SCAND3 | I623V | T | 2485 | C | SLC12A2 | D66N | G | 385 | A | SND1 | K388R | A | 1357 | G |

| | | | | | | | | | | | | | | | | | | | |
|---|---|---|---|---|---|---|---|---|---|---|---|---|---|---|---|---|---|---|---|
| RFPL1 | S39N | G | 325 | A | SCAND3 | N284K | A | 1470 | C | SLC12A2 | G1033R | G | 3286 | A | SND1 | G205D | G | 808 | A |
| RFPL1 | P232L | C | 904 | T | SCAP | R736C | G | 2619 | A | SLC12A2 | N690T | A | 2258 | C | SNED1 | C762W | C | 2286 | G |
| RFPL1 | E199* | G | 804 | T | SCAP | E832G | T | 2908 | C | SLC12A2 | Y903H | T | 2896 | C | SNED1 | R715W | C | 2143 | T |
| RFPL3 | R225H | G | 879 | A | SCAPER | R580W | G | 1775 | A | SLC12A2 | V312L | G | 1123 | T | SNED1 | D534G | A | 1601 | G |
| RFT1 | T541I | G | 1684 | A | SCAPER | E618Q | C | 1889 | G | SLC12A2 | K995R | A | 3173 | G | SNED1 | A1236T | G | 3706 | A |
| RFT1 | K212T | T | 697 | G | SCAPER | K744N | T | 2269 | G | SLC12A3 | R243W | C | 748 | T | SNED1 | V58M | G | 172 | A |
| RFTN1 | P135Q | G | 677 | T | SCAPER | R701Q | C | 2139 | T | SLC12A3 | E121K | G | 382 | A | SNED1 | V1151I | G | 3451 | A |
| RFTN1 | V375A | A | 1397 | G | SCAPER | K550Q | T | 1685 | G | SLC12A3 | A805T | G | 2434 | A | SNED1 | R475C | C | 1423 | T |
| RFTN1 | R111I | C | 605 | A | SCARA3 | F61S | T | 323 | C | SLC12A3 | T9M | C | 47 | T | SNHG4 | K755N | A | 2692 | C |
| RFTN2 | K423N | C | 1806 | A | SCARA3 | R456* | C | 1507 | T | SLC12A3 | I673S | T | 2039 | G | SNORA29 | F167I | A | 740 | T |
| RFTN2 | S415G | T | 1780 | C | SCARA5 | W477* | C | 1871 | T | SLC12A5 | E988D | G | 3040 | T | SNORA29 | K33Q | T | 338 | G |
| RFTN2 | F304L | G | 1449 | T | SCARB1 | E77A | T | 483 | G | SLC12A5 | - | G | 0 | A | SNORA29 | A553T | C | 1898 | T |
| RFWD3 | R380Q | C | 1237 | T | SCARB2 | F453C | A | 1708 | C | SLC12A5 | S399L | C | 1272 | T | SNORA29 | N284T | T | 1092 | G |
| RFX1 | K852R | T | 2790 | C | SCARF1 | T357I | G | 1121 | A | SLC12A5 | A265V | C | 870 | T | SNORA63 | F229I | T | 719 | A |
| RFX1 | S567I | C | 1935 | A | SCARF1 | E792G | T | 2426 | C | SLC12A5 | T316M | C | 1023 | T | SNORA63 | K228Q | A | 716 | C |
| RFX2 | Q30H | C | 359 | A | SCARF2 | L484V | G | 1555 | C | SLC12A5 | R348C | C | 1118 | T | SNORD19B | C403* | C | 1382 | A |
| RFX2 | N386D | T | 1425 | C | SCARF2 | R248C | G | 847 | A | SLC12A5 | R754H | G | 2337 | A | SNORD19B | Y19* | T | 230 | A |
| RFX2 | R17C | G | 318 | A | SCARF2 | A66T | C | 301 | T | SLC12A5 | C566G | T | 1772 | G | SNORD21 | T177A | A | 586 | G |
| RFX3 | H319Q | A | 1269 | T | SCARNA15 | R588Q | C | 1945 | T | SLC12A5 | R531H | G | 1668 | A | SNORD21 | E249D | G | 804 | T |
| RFX3 | T238I | G | 1025 | A | SCCPDH | G282D | G | 1221 | A | SLC12A5 | G321D | G | 1038 | A | SNORD21 | N250H | A | 805 | C |
| RFX3 | D530N | C | 1900 | T | SCD | R174Q | G | 902 | A | SLC12A5 | I377V | A | 1205 | G | SNORD73A | R136C | C | 486 | T |
| RFX3 | C431Y | C | 1604 | T | SCD5 | R165* | G | 813 | A | SLC12A6 | R872* | G | 2614 | A | SNORD86 | K478T | A | 1949 | C |
| RFX4 | R717C | C | 2149 | T | SCEL | R402I | G | 1289 | T | SLC12A6 | R659* | G | 1975 | A | SNPH | A455V | C | 2006 | T |
| RFX4 | T568M | C | 1703 | T | SCEL | L273V | C | 901 | G | SLC12A6 | P10L | G | 29 | A | SNPH | C446Y | G | 1979 | A |
| RFX4 | S49P | T | 145 | C | SCFD1 | I380V | A | 1138 | G | SLC12A6 | R856C | G | 2566 | A | SNRK | K117N | G | 733 | T |
| RFX4 | S322* | C | 965 | A | SCFD2 | R551L | C | 1786 | A | SLC12A6 | F316C | A | 947 | C | SNRK | S236P | T | 1088 | C |
| RFX5 | E261* | C | 960 | A | SCG2 | E367G | T | 1333 | C | SLC12A6 | D132N | C | 394 | T | SNRK | T38M | C | 495 | T |
| RFX5 | E187* | C | 738 | A | SCG3 | N341H | A | 1424 | C | SLC12A6 | - | C | 0 | A | SNRNP200 | R681H | C | 2120 | T |
| RFX5 | A204V | G | 790 | A | SCG5 | N204S | A | 611 | G | SLC12A7 | R710H | C | 2195 | T | SNRNP200 | V1741I | C | 5299 | T |
| RFX5 | E491D | T | 1652 | G | SCGB1A1 | A19T | G | 100 | A | SLC12A7 | D854N | C | 2626 | T | SNRNP200 | R1519H | C | 4634 | T |
| RFX5 | K55N | T | 344 | G | SCGB1D1 | K63N | G | 258 | T | SLC12A7 | A597T | C | 1855 | T | SNRNP200 | A1919T | C | 5833 | T |
| RFX6 | A745V | C | 2250 | T | SCGB2A2 | C65R | T | 252 | C | SLC12A7 | V852M | C | 2620 | T | SNRNP200 | L1289R | A | 3944 | C |
| RFX6 | F779L | C | 2353 | A | SCGB3A2 | T11I | C | 125 | T | SLC12A8 | W596C | C | 1838 | A | SNRNP200 | D1155N | C | 3541 | T |
| RFX7 | S843Y | G | 2528 | T | SCGN | R246H | G | 905 | A | SLC12A9 | A205T | G | 738 | A | SNRNP200 | I292M | A | 954 | C |
| RFX7 | R1280Q | C | 3839 | T | SCIN | K299Q | A | 996 | C | SLC12A9 | P233L | C | 823 | T | SNRNP35 | D8N | G | 324 | A |
| RFX7 | S1439Y | G | 4316 | T | SCLT1 | Q80H | C | 744 | A | SLC12A9 | V565I | G | 1818 | A | SNRNP35 | D17E | T | 353 | A |
| RFX7 | P1103S | G | 3307 | A | SCLT1 | L67I | G | 703 | T | SLC12A9 | G215W | G | 768 | T | SNRNP40 | P16S | G | 68 | A |
| RFXANK | A214T | G | 1145 | A | SCLT1 | S395Y | G | 1688 | T | SLC13A1 | M114I | C | 382 | T | SNRNP48 | R176C | C | 585 | T |
| RFXANK | R111W | C | 836 | T | SCLY | I75M | A | 391 | G | SLC13A2 | T205M | C | 813 | T | SNRNP48 | Y208* | C | 683 | A |

| | | | | | | | | | | | | | | | | | | | |
|---|---|---|---|---|---|---|---|---|---|---|---|---|---|---|---|---|---|---|---|
| RG9MTD2 | Q140H | C | 537 | A | SCMH1 | R575* | G | 2023 | A | SLC13A2 | A379S | G | 1334 | T | SNRNP70 | R120Q | G | 798 | A |
| RG9MTD2 | F268C | A | 920 | C | SCMH1 | N165T | T | 794 | G | SLC13A2 | L88R | T | 462 | G | SNRNP70 | R368W | C | 1541 | T |
| RG9MTD2 | R243Q | C | 845 | T | SCML1 | E147D | G | 769 | T | SLC13A3 | R169Q | C | 525 | T | SNRPB | A153T | C | 538 | T |
| RG9MTD2 | R93* | G | 394 | A | SCML2 | Q56E | G | 326 | C | SLC13A3 | P414S | G | 1259 | A | SNRPB2 | N137H | A | 548 | C |
| RG9MTD3 | K63Q | A | 264 | C | SCML4 | V161L | C | 727 | A | SLC13A3 | - | C | 0 | T | SNRPE | N27H | A | 124 | C |
| RGAG1 | E17D | A | 297 | C | SCN10A | F342C | A | 1025 | C | SLC13A3 | R169* | G | 524 | A | SNRPEL1 | P17T | G | 82 | T |
| RGAG1 | T516M | C | 1793 | T | SCN10A | R844H | C | 2531 | T | SLC13A4 | R111H | C | 1022 | T | SNRPN | M21I | G | 63 | A |
| RGAG1 | D673Y | G | 2263 | T | SCN10A | W1748R | A | 5242 | G | SLC13A4 | A78V | G | 923 | A | SNRPN | R69Q | G | 206 | A |
| RGAG1 | E921D | G | 3009 | T | SCN10A | I251T | A | 752 | G | SLC13A4 | R111C | G | 1021 | A | SNRPN | L44M | T | 130 | A |
| RGAG4 | E491K | C | 1832 | T | SCN10A | R534Q | C | 1601 | T | SLC13A5 | S3L | G | 43 | A | SNRPN | Q149R | A | 446 | G |
| RGAG4 | E400* | C | 1559 | A | SCN10A | R497P | C | 1490 | G | SLC13A5 | P445L | G | 1369 | A | SNTA1 | E107D | C | 593 | A |
| RGAG4 | E77K | C | 590 | T | SCN10A | T1936P | T | 5806 | G | SLC13A5 | V319M | C | 990 | T | SNTB1 | R401Q | C | 1428 | T |
| RGL1 | E8* | G | 483 | T | SCN10A | R1250L | C | 3749 | A | SLC13A5 | A561P | C | 1716 | G | SNTG1 | Q18* | C | 97 | T |
| RGL1 | Q204H | G | 1073 | T | SCN10A | - | C | 0 | T | SLC14A1 | A13T | G | 235 | A | SNTG1 | L106I | C | 361 | A |
| RGL1 | S297P | T | 1350 | C | SCN10A | F870L | G | 2610 | T | SLC14A1 | Y243C | A | 926 | G | SNTG1 | K272T | A | 860 | C |
| RGL1 | F511L | C | 1994 | A | SCN10A | A394V | G | 1181 | A | SLC14A1 | S281Y | C | 1040 | A | SNTG2 | T444M | C | 1460 | T |
| RGL3 | A131V | G | 444 | A | SCN10A | T365A | T | 1093 | C | SLC14A2 | A194T | G | 1396 | A | SNURF | R79G | A | 886 | G |
| RGL3 | R500W | G | 1550 | A | SCN10A | K244N | C | 732 | A | SLC15A1 | E677D | C | 2087 | G | SNURF | E25K | G | 724 | A |
| RGL4 | E454V | A | 2531 | T | SCN10A | R149Q | C | 446 | T | SLC15A1 | E677D | C | 2087 | G | SNW1 | R198L | C | 619 | A |
| RGMA | A209V | G | 898 | A | SCN10A | R1576C | G | 4726 | A | SLC15A1 | Q158P | T | 529 | G | SNW1 | R198Q | C | 619 | T |
| RGPD3 | G946D | C | 2919 | T | SCN10A | R1268W | G | 3802 | A | SLC15A1 | A145V | G | 490 | A | SNX1 | R322C | C | 1000 | T |
| RGS11 | R228Q | C | 701 | T | SCN11A | L1398I | G | 4391 | T | SLC15A1 | S532* | G | 1651 | T | SNX1 | N123H | A | 403 | C |
| RGS11 | L73M | G | 235 | T | SCN11A | E606K | C | 2015 | T | SLC15A1 | M421L | T | 1317 | G | SNX11 | S187L | C | 914 | T |
| RGS12 | V47I | G | 1043 | A | SCN11A | P667S | G | 2198 | A | SLC15A1 | V217A | A | 706 | G | SNX13 | R559C | G | 1675 | A |
| RGS12 | P1357L | C | 4974 | T | SCN11A | G724D | C | 2370 | T | SLC15A2 | E622G | A | 2253 | G | SNX13 | N949S | T | 2846 | C |
| RGS12 | K1176E | A | 4430 | G | SCN11A | L1561M | G | 4880 | T | SLC15A2 | R277* | C | 1217 | T | SNX13 | R907* | G | 2719 | A |
| RGS12 | E162D | G | 1390 | T | SCN11A | D1701Y | C | 5300 | A | SLC15A2 | K458T | A | 1761 | C | SNX15 | Y295H | T | 1468 | C |
| RGS12 | R620* | C | 2762 | T | SCN11A | Q866H | T | 2797 | G | SLC15A3 | V160I | C | 712 | T | SNX15 | Y52H | T | 739 | C |
| RGS12 | S1413L | C | 5142 | T | SCN11A | K538T | T | 1812 | G | SLC15A3 | F202V | A | 838 | C | SNX15 | A224T | G | 1255 | A |
| RGS13 | S81Y | C | 530 | A | SCN11A | R1466H | C | 4596 | T | SLC15A4 | R273* | G | 870 | A | SNX15 | A289T | G | 1450 | A |
| RGS14 | E189K | G | 747 | A | SCN11A | R1153K | C | 3657 | T | SLC15A5 | L362M | G | 1084 | T | SNX15 | R3H | G | 593 | A |
| RGS16 | A154V | G | 610 | A | SCN1A | G2008W | C | 6022 | A | SLC15A5 | F119V | A | 355 | C | SNX15 | T236M | C | 1292 | T |
| RGS18 | - | T | 0 | C | SCN1A | N1897K | A | 5691 | T | SLC16A10 | L138P | T | 588 | C | SNX17 | - | T | 0 | C |
| RGS18 | L193M | C | 758 | A | SCN1A | A773T | C | 2317 | T | SLC16A10 | I515M | T | 1720 | G | SNX18 | P382L | C | 1233 | T |
| RGS19 | A106V | G | 584 | A | SCN1A | I747T | A | 2240 | G | SLC16A11 | - | C | 0 | T | SNX18 | E40Q | G | 206 | C |
| RGS20 | P78L | C | 325 | T | SCN1B | S264Y | C | 982 | A | SLC16A11 | D222N | C | 1002 | T | SNX18 | K351R | A | 1140 | G |
| RGS22 | K1232E | T | 3889 | C | SCN2A | D1526A | A | 4867 | C | SLC16A11 | D127N | C | 717 | T | SNX18 | P328L | C | 1071 | T |
| RGS22 | Y703C | T | 2303 | C | SCN2A | V887I | G | 2949 | A | SLC16A12 | R141Q | C | 813 | T | SNX19 | L704M | G | 2658 | T |
| RGS22 | R1023H | C | 3263 | T | SCN2A | P151H | C | 742 | A | SLC16A12 | L27R | A | 471 | C | SNX19 | A763V | G | 2836 | A |

| | | | | | | | | | | | | | | | | | | | |
|---|---|---|---|---|---|---|---|---|---|---|---|---|---|---|---|---|---|---|---|
| RGS7 | W314C | C | 942 | A | SCN2A | V221F | G | 951 | T | SLC16A13 | G341D | G | 1247 | A | SNX19 | R872H | C | 3163 | T |
| RGS8 | R179M | C | 626 | A | SCN2A | F1029V | T | 3375 | G | SLC16A14 | V198I | C | 1051 | T | SNX19 | P499S | G | 2043 | A |
| RGS9 | S581F | C | 1852 | T | SCN2A | E1796D | G | 5678 | T | SLC16A14 | A133T | C | 856 | T | SNX2 | R462K | G | 1493 | A |
| RGS9BP | R94Q | G | 1138 | A | SCN3A | Q1390H | T | 4662 | G | SLC16A2 | R23W | C | 680 | T | SNX2 | D26G | A | 185 | G |
| RHAG | G370R | C | 1135 | G | SCN3A | T304A | T | 1402 | C | SLC16A3 | D260N | G | 917 | A | SNX2 | R482* | C | 1552 | T |
| RHAG | - | C | 0 | A | SCN3A | K1917N | C | 6243 | A | SLC16A3 | V322M | G | 1103 | A | SNX2 | - | T | 0 | G |
| RHAG | F151V | A | 478 | C | SCN3A | R621C | G | 2353 | A | SLC16A4 | I200T | A | 793 | G | SNX2 | R410Q | G | 1337 | A |
| RHBDD1 | D134A | A | 643 | C | SCN3A | A1777S | C | 5821 | A | SLC16A5 | G216S | G | 646 | A | SNX20 | R203C | G | 739 | A |
| RHBDD2 | P179H | C | 671 | A | SCN3A | M1666V | T | 5488 | C | SLC16A5 | A363V | C | 1088 | T | SNX20 | R302* | G | 1036 | A |
| RHBDD2 | A275T | G | 958 | A | SCN3A | D1521E | A | 5055 | C | SLC16A5 | F139L | C | 417 | A | SNX20 | V100D | A | 431 | T |
| RHBDF1 | V228I | C | 825 | T | SCN3A | R534I | C | 2093 | A | SLC16A6 | A26V | G | 265 | A | SNX20 | - | C | 0 | T |
| RHBDF1 | D256N | C | 909 | T | SCN4A | T1351M | G | 4129 | A | SLC16A6 | E58K | C | 360 | T | SNX21 | R211H | G | 700 | A |
| RHBDF1 | R339W | G | 1158 | A | SCN4A | S725N | C | 2251 | T | SLC16A7 | C331Y | G | 1148 | A | SNX21 | P144L | C | 499 | T |
| RHBDF1 | Q354* | G | 1203 | A | SCN4A | R18H | C | 130 | T | SLC16A7 | S353G | A | 1213 | G | SNX21 | L182M | C | 612 | A |
| RHBDF1 | R107H | C | 463 | T | SCN4B | E49D | C | 147 | A | SLC16A7 | E46* | G | 292 | T | SNX21 | S55C | C | 232 | G |
| RHBDF2 | R255C | G | 1056 | A | SCN5A | R535Q | C | 1798 | T | SLC16A7 | R312Q | G | 1091 | A | SNX25 | - | G | 0 | T |
| RHBDF2 | R401W | G | 1494 | A | SCN5A | L736M | G | 2400 | T | SLC16A8 | G373D | C | 1227 | T | SNX27 | R492* | C | 1594 | T |
| RHBDF2 | R234H | C | 994 | T | SCN5A | T370M | G | 1303 | A | SLC16A9 | K5T | T | 651 | G | SNX29 | E194* | G | 636 | T |
| RHBDL3 | R116C | C | 360 | T | SCN5A | A185T | C | 747 | T | SLC17A1 | S261L | G | 898 | A | SNX30 | V165L | G | 657 | T |
| RHBDL3 | P32A | C | 108 | G | SCN5A | M28I | C | 278 | T | SLC17A2 | R339G | T | 1433 | C | SNX31 | D211N | C | 782 | T |
| RHBDL3 | A273V | C | 832 | T | SCN5A | G77R | C | 423 | T | SLC17A3 | R497C | G | 1599 | A | SNX32 | R233C | C | 868 | T |
| RHBDL3 | R392Q | G | 1189 | A | SCN5A | P621H | G | 2056 | T | SLC17A4 | G473D | G | 1537 | A | SNX32 | A355T | G | 1234 | A |
| RHCE | A237V | G | 769 | A | SCN5A | P468S | G | 1596 | A | SLC17A4 | E95* | G | 402 | T | SNX32 | R227Q | G | 851 | A |
| RHCE | A17V | G | 109 | A | SCN5A | C906G | A | 2910 | C | SLC17A4 | S330R | A | 1107 | C | SNX32 | D38N | G | 283 | A |
| RHD | S75G | A | 281 | G | SCN5A | Y774* | G | 2516 | T | SLC17A4 | V340A | T | 1138 | C | SNX32 | E139K | G | 586 | A |
| RHEB | E66* | C | 609 | A | SCN7A | F578L | G | 1861 | T | SLC17A6 | A57V | C | 583 | T | SNX32 | N321S | A | 1133 | G |
| RHEBL1 | H79R | T | 476 | C | SCN7A | K495* | T | 1610 | A | SLC17A6 | L31M | C | 504 | A | SNX33 | R181H | G | 1739 | A |
| RHEBL1 | S44I | C | 371 | A | SCN7A | G607* | C | 1946 | A | SLC17A7 | E280G | T | 1011 | C | SNX4 | E40* | C | 143 | A |
| RHO | K325N | G | 1069 | T | SCN7A | G340D | C | 1146 | T | SLC17A7 | V444M | C | 1502 | T | SNX5 | F99C | A | 609 | C |
| RHOA | T37I | G | 495 | A | SCN7A | I1616L | T | 4973 | G | SLC17A8 | V198A | T | 906 | C | SOAT1 | V184I | G | 693 | A |
| RHOB | F30L | T | 480 | C | SCN7A | D1576N | C | 4853 | T | SLC17A8 | R218* | C | 965 | T | SOAT2 | A213V | C | 726 | T |
| RHOBTB1 | P241L | G | 926 | A | SCN7A | F1518L | G | 4681 | T | SLC17A9 | A40T | G | 249 | A | SOBP | G391S | G | 1830 | A |
| RHOBTB1 | I534V | T | 1804 | C | SCN7A | D1228E | A | 3811 | C | SLC18A1 | H121P | T | 629 | G | SOBP | A752V | C | 2914 | T |
| RHOBTB1 | A207V | G | 824 | A | SCN7A | E820G | T | 2586 | C | SLC18A1 | L179I | G | 802 | T | SOBP | Y258* | C | 1433 | G |
| RHOBTB2 | R93H | G | 815 | A | SCN7A | R754I | C | 2388 | A | SLC18A2 | F178L | C | 677 | A | SOBP | R183Q | G | 1207 | A |
| RHOBTB2 | R552W | C | 2191 | T | SCN7A | R282I | C | 972 | A | SLC18A2 | P272L | C | 958 | T | SOCS1 | A89T | C | 416 | T |
| RHOBTB2 | R145C | C | 970 | T | SCN7A | K77Q | T | 356 | G | SLC18A2 | L462I | C | 1527 | A | SOCS3 | R116H | C | 763 | T |
| RHOBTB2 | E591* | G | 2308 | T | SCN8A | R1866W | C | 5774 | T | SLC18A2 | R471Q | G | 1555 | A | SOCS3 | R94C | G | 696 | A |
| RHOBTB3 | K411N | G | 1741 | T | SCN8A | P554H | C | 1839 | A | SLC18A3 | R412H | G | 1601 | A | SOCS4 | S213F | C | 1203 | T |

| | | | | | | | | | | | | | | | | | | | |
|---|---|---|---|---|---|---|---|---|---|---|---|---|---|---|---|---|---|---|---|
| RHOBTB3 | E568A | A | 2211 | C | SCN8A | F1873I | T | 5795 | A | SLC18A3 | A297T | G | 1255 | A | SOCS5 | S464L | C | 1563 | T |
| RHOG | R49C | G | 274 | A | SCN8A | N909I | A | 2904 | T | SLC19A1 | A158T | C | 625 | T | SOCS6 | R260C | C | 1063 | T |
| RHOH | D58G | A | 789 | G | SCN8A | R1872W | C | 5792 | T | SLC19A2 | G120* | C | 595 | A | SOCS6 | R493W | C | 1762 | T |
| RHOQ | C24Y | G | 390 | A | SCN9A | R586G | T | 1804 | C | SLC19A2 | A467T | C | 1636 | T | SOCS6 | A167V | C | 785 | T |
| RHOQ | V42G | T | 444 | G | SCN9A | F100L | G | 348 | T | SLC19A3 | V435A | A | 1376 | G | SOCS7 | S337P | T | 1132 | C |
| RHOT1 | E12K | G | 273 | A | SCN9A | S502* | G | 1553 | T | SLC19A3 | K208N | C | 696 | A | SOCS7 | - | G | 0 | A |
| RHOT1 | K469I | A | 1645 | T | SCN9A | V1832I | C | 5542 | T | SLC1A1 | R166C | C | 732 | T | SOCS7 | R467H | G | 1523 | A |
| RHOT2 | A557V | C | 1784 | T | SCN9A | V834G | A | 2549 | C | SLC1A1 | I67S | T | 436 | G | SOHLH1 | M272T | A | 876 | G |
| RHOT2 | - | G | 0 | A | SCN9A | V196I | C | 634 | T | SLC1A2 | T50M | G | 432 | A | SOHLH1 | S137L | G | 471 | A |
| RHOXF1 | D63V | T | 263 | A | SCN9A | R599H | C | 1844 | T | SLC1A2 | R17* | G | 332 | A | SOHLH2 | L318I | G | 952 | T |
| RHOXF1 | R107C | G | 394 | A | SCN9A | G176D | C | 575 | T | SLC1A2 | R65H | C | 477 | T | SOHLH2 | A432V | G | 1295 | A |
| RHOXF1 | D8N | C | 97 | T | SCN9A | F812V | A | 2482 | C | SLC1A2 | M76R | A | 510 | C | SOHLH2 | R478W | G | 1432 | A |
| RHPN1 | V66A | T | 197 | C | SCN9A | T1354A | T | 4108 | C | SLC1A2 | R36H | C | 390 | T | SOHLH2 | L280P | A | 839 | G |
| RIC3 | E309D | T | 981 | G | SCN9A | L1840I | G | 5566 | T | SLC1A3 | R122* | C | 840 | T | SON | E1791D | A | 5848 | C |
| RIC8A | R179C | C | 860 | T | SCN9A | - | T | 0 | C | SLC1A3 | V393G | T | 1654 | G | SON | N2418H | A | 7727 | C |
| RIC8A | R343Q | G | 1353 | A | SCN9A | M691I | C | 2121 | A | SLC1A3 | R479W | C | 1911 | T | SON | R1858C | C | 6047 | T |
| RIC8A | F162L | C | 811 | A | SCN9A | E648D | C | 1992 | A | SLC1A4 | R322Q | G | 1208 | A | SON | N2210T | A | 7104 | C |
| RIC8B | A281T | G | 961 | A | SCN9A | E181* | C | 589 | A | SLC1A6 | V365I | C | 1101 | T | SORBS1 | R1158G | T | 3662 | C |
| RIC8B | R378Q | G | 1253 | A | SCN9A | A102V | G | 353 | A | SLC1A6 | V365F | C | 1101 | A | SORBS1 | E598* | C | 1982 | A |
| RIC8B | N465D | A | 1513 | G | SCNN1A | R567* | G | 1963 | A | SLC1A6 | D297N | C | 897 | T | SORBS1 | R1275K | C | 4014 | T |
| RICTOR | R1633H | C | 4920 | T | SCNN1B | G213R | G | 637 | A | SLC1A7 | R329C | G | 1113 | A | SORBS2 | R544H | C | 2345 | T |
| RICTOR | V45M | C | 155 | T | SCNN1B | L209I | C | 625 | A | SLC1A7 | I47M | A | 269 | C | SORBS2 | L424F | G | 1984 | A |
| RICTOR | R336H | C | 1029 | T | SCNN1D | F636L | C | 2058 | A | SLC20A1 | R434C | C | 1839 | T | SORBS2 | P496L | G | 2201 | A |
| RICTOR | R280Q | C | 861 | T | SCNN1G | Q70R | A | 352 | G | SLC20A1 | R648C | C | 2481 | T | SORBS2 | R531C | G | 2305 | A |
| RICTOR | R222Q | C | 687 | T | SCNN1G | F154L | C | 605 | A | SLC20A2 | V421M | C | 1630 | T | SORBS3 | G20D | G | 442 | A |
| RIF1 | R266Q | G | 958 | A | SCNN1G | G448D | G | 1486 | A | SLC20A2 | R254* | G | 1129 | A | SORBS3 | H176Y | C | 909 | T |
| RIF1 | E99* | G | 456 | T | SCNN1G | D592G | A | 1918 | G | SLC20A2 | N384S | T | 1520 | C | SORBS3 | A537T | G | 1992 | A |
| RIF1 | H194N | C | 741 | A | SCO2 | R262C | G | 932 | A | SLC20A2 | G366D | C | 1466 | T | SORCS1 | S667P | A | 1999 | G |
| RILPL1 | Q218H | C | 890 | A | SCO2 | Q146* | G | 584 | A | SLC22A1 | A306T | G | 1079 | A | SORCS1 | R1078* | G | 3232 | A |
| RIMBP2 | K472R | T | 1579 | C | SCPEP1 | R384Q | G | 1204 | A | SLC22A1 | R399H | G | 1359 | A | SORCS1 | R444H | C | 1331 | T |
| RIMBP2 | G632V | C | 2059 | A | SCPEP1 | G226C | G | 729 | T | SLC22A1 | G131S | G | 554 | A | SORCS1 | D1000G | T | 2999 | C |
| RIMBP2 | A196V | G | 751 | A | SCRIB | E77K | C | 236 | T | SLC22A1 | A493T | G | 1640 | A | SORCS1 | A113V | G | 338 | A |
| RIMBP2 | R45W | G | 297 | A | SCRIB | R1483H | C | 4455 | T | SLC22A1 | P34S | C | 263 | T | SORCS1 | I190M | A | 570 | C |
| RIMBP2 | W411* | C | 1397 | T | SCRIB | Q600* | G | 1805 | A | SLC22A1 | I479T | T | 1599 | C | SORCS1 | T142P | T | 424 | G |
| RIMBP3 | R582P | C | 2230 | G | SCRIB | P589S | G | 1772 | A | SLC22A1 | I421F | A | 1424 | T | SORCS2 | N974K | C | 2922 | G |
| RIMBP3 | R1059Q | C | 3661 | T | SCRIB | P1450S | G | 4355 | A | SLC22A10 | F273C | T | 1026 | G | SORCS2 | S159N | G | 476 | A |
| RIMBP3B | E1095K | G | 3768 | A | SCRIB | E899K | C | 2702 | T | SLC22A10 | R90C | C | 476 | T | SORCS2 | D609G | A | 1826 | G |
| RIMKLB | S317T | T | 2211 | A | SCRN1 | P68L | G | 252 | A | SLC22A10 | K309N | G | 1135 | T | SORCS2 | R506C | C | 1516 | T |
| RIMKLB | T160M | C | 1741 | T | SCRN2 | R250W | G | 874 | A | SLC22A10 | N316S | A | 1155 | G | SORCS2 | T189M | C | 566 | T |

| | | | | | | | | | | | | | | | | | | | |
|---|---|---|---|---|---|---|---|---|---|---|---|---|---|---|---|---|---|---|---|
| RIMKLB | F273L | C | 2081 | A | SCRN2 | R250W | G | 874 | A | SLC22A11 | P330L | C | 1363 | T | SORCS2 | S692L | C | 2075 | T |
| RIMS1 | P584H | C | 1828 | A | SCRN2 | P25S | G | 199 | A | SLC22A12 | V388M | G | 1909 | A | SORCS2 | S923Y | C | 2768 | A |
| RIMS1 | P452H | C | 1432 | A | SCRN3 | E64D | A | 280 | C | SLC22A12 | T104M | C | 1058 | T | SORCS2 | F966V | T | 2896 | G |
| RIMS1 | T855I | C | 2641 | T | SCRN3 | H296N | C | 974 | A | SLC22A12 | R89C | C | 1012 | T | SORCS3 | P913H | C | 2965 | A |
| RIMS1 | R721M | G | 2239 | T | SCRN3 | N362H | A | 1172 | C | SLC22A14 | R322W | C | 1006 | T | SORCS3 | A286T | G | 1083 | A |
| RIMS1 | S712R | A | 2211 | C | SCRN3 | M374T | T | 1209 | C | SLC22A15 | V32M | G | 224 | A | SORCS3 | T361A | A | 1308 | G |
| RIMS1 | S229F | C | 763 | T | SCRT1 | H294R | T | 993 | C | SLC22A16 | G56D | C | 234 | T | SORCS3 | - | G | 0 | A |
| RIMS1 | R215* | C | 720 | T | SCRT1 | G46W | C | 248 | A | SLC22A16 | V257M | C | 836 | T | SORCS3 | R927G | A | 3006 | G |
| RIMS1 | L22Q | T | 142 | A | SCRT2 | R178G | G | 1112 | C | SLC22A16 | Q30H | C | 157 | A | SORCS3 | N579D | A | 1962 | G |
| RIMS1 | R526Q | G | 1654 | A | SCTR | E114G | T | 609 | C | SLC22A18 | L37R | T | 328 | G | SORL1 | G738R | G | 2292 | A |
| RIMS1 | R1316C | C | 4023 | T | SCTR | L324R | A | 1239 | C | SLC22A2 | V467I | C | 1658 | T | SORL1 | W1575R | T | 4803 | C |
| RIMS1 | D111Y | G | 408 | T | SCUBE1 | R755H | C | 2391 | T | SLC22A2 | R62C | G | 443 | A | SORL1 | K143Q | A | 507 | C |
| RIMS1 | R136H | G | 484 | A | SCUBE1 | E604G | T | 1938 | C | SLC22A2 | T74M | G | 480 | A | SORL1 | R1473Q | G | 4498 | A |
| RIMS1 | S1083P | T | 3324 | C | SCUBE1 | P514L | G | 1668 | A | SLC22A20 | V192I | G | 615 | A | SORT1 | S756Y | G | 2326 | T |
| RIMS1 | S1369P | T | 4182 | C | SCUBE1 | R923C | G | 2894 | A | SLC22A20 | T421M | C | 1303 | T | SORT1 | L20I | G | 117 | T |
| RIMS2 | S40L | C | 360 | T | SCUBE1 | V263M | C | 914 | T | SLC22A20 | R333* | C | 1038 | T | SOS1 | R547W | G | 1726 | A |
| RIMS2 | R112Q | G | 576 | A | SCUBE2 | K155T | T | 544 | G | SLC22A20 | R84C | C | 291 | T | SOS1 | R854* | G | 2647 | A |
| RIMS2 | R812T | G | 2676 | C | SCUBE2 | R110H | C | 409 | T | SLC22A20 | S542Y | C | 1666 | A | SOS1 | G1129V | C | 3473 | A |
| RIMS2 | R365H | G | 1335 | A | SCUBE2 | A920T | C | 2838 | T | SLC22A23 | G579R | C | 2198 | T | SOS1 | T614M | G | 1928 | A |
| RIMS2 | P549H | C | 1887 | A | SCUBE2 | R963* | G | 2967 | A | SLC22A23 | A327V | G | 1443 | A | SOS2 | R824C | G | 2560 | A |
| RIMS2 | R46I | G | 378 | T | SCUBE2 | R798C | G | 2472 | A | SLC22A23 | S479F | G | 1899 | A | SOS2 | - | T | 0 | C |
| RIMS2 | D225Y | G | 914 | T | SCUBE2 | R557C | G | 1749 | A | SLC22A23 | Y359C | T | 1539 | C | SOS2 | Q1296H | C | 3978 | A |
| RIMS2 | R599Q | G | 2037 | A | SCUBE3 | N260T | A | 785 | C | SLC22A24 | R405H | C | 1656 | T | SOS2 | R824C | G | 2560 | A |
| RIMS2 | E621* | G | 2102 | T | SCUBE3 | A951D | C | 2858 | A | SLC22A24 | A283D | G | 1290 | T | SOS2 | R225* | G | 763 | A |
| RIMS3 | S298F | G | 1363 | A | SCYL1 | T408M | C | 1300 | T | SLC22A24 | R406* | G | 1658 | A | SOX10 | A323V | G | 1233 | A |
| RIMS3 | K210T | T | 1099 | G | SCYL1 | M429I | G | 1364 | T | SLC22A25 | R277G | T | 829 | C | SOX10 | V70A | A | 474 | G |
| RIMS4 | G145D | C | 501 | T | SCYL1 | E440* | G | 1395 | T | SLC22A25 | D136V | T | 407 | A | SOX10 | R159W | G | 740 | A |
| RIN1 | R514W | G | 1667 | A | SCYL2 | R505C | C | 1563 | T | SLC22A25 | I362T | A | 1085 | G | SOX11 | D197N | G | 644 | A |
| RIN1 | R342C | G | 1151 | A | SCYL2 | T737I | C | 2260 | T | SLC22A25 | L6F | G | 16 | A | SOX11 | A102T | G | 359 | A |
| RIN1 | E538K | C | 1739 | T | SCYL3 | R61H | C | 380 | T | SLC22A3 | F279C | T | 863 | G | SOX11 | A45V | C | 189 | T |
| RIN1 | A534V | G | 1728 | A | SDC1 | F211L | G | 878 | T | SLC22A3 | C148R | T | 469 | C | SOX13 | D419N | G | 1255 | A |
| RIN1 | Q212R | T | 762 | C | SDC2 | F162V | T | 1102 | G | SLC22A3 | E452D | A | 1383 | C | SOX13 | A535V | C | 1604 | T |
| RIN2 | R290H | G | 1018 | A | SDC2 | A190V | C | 1187 | T | SLC22A4 | R336Q | G | 1181 | A | SOX15 | R119W | G | 849 | A |
| RIN2 | G422S | G | 1413 | A | SDC3 | A263T | C | 962 | T | SLC22A4 | I221L | A | 835 | C | SOX17 | R204H | G | 815 | A |
| RIN2 | R409Q | G | 1375 | A | SDC3 | K413T | T | 1413 | G | SLC22A4 | K434T | A | 1475 | C | SOX21 | D59G | T | 262 | C |
| RIN2 | D667E | T | 2150 | A | SDC4 | V146A | A | 477 | G | SLC22A4 | A460V | C | 1553 | T | SOX3 | M147V | T | 439 | C |
| RIN3 | S183L | C | 700 | T | SDCBP | V158A | T | 620 | C | SLC22A5 | A404V | C | 1475 | T | SOX3 | R212H | C | 635 | T |
| RIN3 | L791F | C | 2523 | T | SDCBP | S74F | C | 368 | T | SLC22A6 | P245S | G | 1040 | A | SOX30 | R415Q | C | 1586 | T |
| RIN3 | E888K | G | 2814 | A | SDCCAG1 | E847* | C | 2574 | A | SLC22A6 | - | C | 0 | T | SOX30 | V337E | A | 1352 | T |

| | | | | | | | | | | | | | | | | | | | |
|---|---|---|---|---|---|---|---|---|---|---|---|---|---|---|---|---|---|---|---|
| RINL | R170C | G | 896 | A | SDCCAG1 | E64V | T | 226 | A | SLC22A7 | R202H | G | 700 | A | SOX5 | R593H | C | 1880 | T |
| RINT1 | - | G | 0 | T | SDCCAG1 | E628D | T | 1919 | G | SLC22A7 | D426N | G | 1371 | A | SOX5 | G705E | C | 2216 | T |
| RIOK1 | P520H | C | 2066 | A | SDCCAG1 | R327Q | C | 1015 | T | SLC22A8 | R536W | G | 1742 | A | SOX5 | Q259H | T | 879 | G |
| RIOK1 | R379W | C | 1642 | T | SDCCAG8 | L105F | A | 433 | C | SLC22A8 | S565R | G | 1831 | T | SOX6 | R93* | G | 349 | A |
| RIOK3 | I306S | T | 1534 | G | SDK1 | P299L | C | 1035 | T | SLC22A8 | R75H | C | 360 | T | SOX6 | R144Q | C | 503 | T |
| RIOK3 | I306S | T | 1534 | G | SDK1 | A555V | C | 1803 | T | SLC22A8 | L481I | G | 1577 | T | SOX8 | A279T | G | 950 | A |
| RIOK3 | S107G | A | 936 | G | SDK1 | A164D | C | 630 | A | SLC22A8 | S211L | G | 768 | A | SOX8 | G182S | G | 659 | A |
| RIOK3 | R326H | G | 1594 | A | SDK1 | T1456M | C | 4506 | T | SLC22A8 | I187T | A | 696 | G | SOX9 | A116V | C | 719 | T |
| RIOK3 | S360Y | C | 1696 | A | SDK1 | R1796C | C | 5525 | T | SLC22A9 | T451A | A | 1619 | G | SOX9 | A189V | C | 938 | T |
| RIPK1 | M239L | A | 998 | C | SDK1 | A2180V | C | 6678 | T | SLC22A9 | R407* | C | 1487 | T | SP100 | H529Q | C | 1618 | G |
| RIPK1 | M126I | G | 661 | A | SDK1 | D1174N | G | 3659 | A | SLC23A1 | R460H | C | 1418 | T | SP100 | M768V | A | 2333 | G |
| RIPK2 | A454V | C | 1675 | T | SDK1 | A851V | C | 2691 | T | SLC23A1 | A67V | G | 239 | A | SP100 | R838C | C | 2543 | T |
| RIPK3 | E189* | C | 737 | A | SDK1 | R526W | C | 1715 | T | SLC23A2 | - | C | 0 | T | SP100 | S228L | C | 714 | T |
| RIPK3 | P419S | G | 1427 | A | SDK1 | H341Q | C | 1162 | G | SLC23A3 | V196M | C | 697 | T | SP110 | K512N | C | 1614 | A |
| RIPK4 | T654M | G | 2026 | A | SDK1 | V691D | T | 2211 | A | SLC24A1 | A1060V | C | 3466 | T | SP140 | R401C | C | 1315 | T |
| RIPK4 | R260C | G | 843 | A | SDK1 | L701I | C | 2240 | A | SLC24A1 | Q642H | G | 2213 | T | SP2 | A588V | C | 1900 | T |
| RIPK4 | K51N | C | 218 | A | SDK2 | T1296A | T | 3886 | C | SLC24A2 | G362* | C | 1146 | A | SP2 | E409D | G | 1364 | T |
| RIPK4 | R79C | G | 300 | A | SDK2 | R90C | G | 268 | A | SLC24A2 | G561W | C | 1743 | A | SP3 | G289C | C | 1396 | A |
| RIPPLY1 | - | C | 0 | A | SDK2 | S263G | T | 787 | C | SLC24A2 | N407S | T | 1282 | C | SP3 | Q150H | T | 981 | G |
| RIPPLY1 | R68M | C | 203 | A | SDK2 | A1841T | C | 5521 | T | SLC24A2 | V209L | C | 687 | A | SP4 | V538I | G | 1830 | A |
| RIT2 | R104C | G | 482 | A | SDK2 | H595L | T | 1784 | A | SLC24A2 | K558R | T | 1735 | C | SP4 | E7K | G | 237 | A |
| RLF | Q787P | A | 2387 | C | SDK2 | E313A | T | 938 | G | SLC24A2 | W463C | C | 1451 | A | SP4 | E8K | G | 240 | A |
| RLF | R339C | C | 1042 | T | SDK2 | G1658W | C | 4972 | A | SLC24A3 | T634A | A | 2097 | G | SP4 | E711K | G | 2349 | A |
| RLF | E135K | G | 430 | A | SDK2 | A50V | G | 149 | A | SLC24A3 | R82W | C | 441 | T | SP5 | R396W | C | 1348 | T |
| RLF | K617N | G | 1878 | T | SDK2 | T540M | G | 1619 | A | SLC24A4 | V574M | G | 1744 | A | SP6 | H274R | T | 1099 | C |
| RLF | Q684H | A | 2079 | C | SDK2 | S1733G | T | 5197 | C | SLC24A4 | T444M | C | 1355 | T | SP6 | V322I | C | 1242 | T |
| RLF | V1417A | T | 4277 | C | SDPR | L387P | A | 1490 | G | SLC24A5 | D272Y | G | 887 | T | SP7 | S338L | G | 1120 | A |
| RLN1 | V165M | C | 620 | T | SDR16C5 | F231C | A | 1330 | C | SLC24A6 | A533T | C | 1807 | T | SP8 | P498S | G | 1580 | A |
| RLN1 | Q18P | T | 180 | G | SDR16C6 | F198L | A | 594 | C | SLC24A6 | I496V | T | 1696 | C | SP9 | Y159C | A | 623 | G |
| RLN2 | F90C | A | 658 | C | SDR16C6 | R78Q | C | 233 | T | SLC25A11 | A56V | G | 425 | A | SP9 | A176T | G | 673 | A |
| RLTPR | R220Q | G | 779 | A | SDR39U1 | N234S | T | 701 | C | SLC25A12 | S25R | A | 113 | T | SPACA3 | R150M | G | 449 | T |
| RLTPR | K1263T | A | 3908 | C | SDS | A34V | G | 219 | A | SLC25A12 | K416N | T | 1286 | G | SPACA3 | C66G | T | 196 | G |
| RLTPR | G407V | G | 1340 | T | SDSL | S228I | G | 893 | T | SLC25A13 | A341S | C | 1094 | A | SPACA4 | G16C | G | 282 | T |
| RMI1 | N612T | A | 2070 | C | SEC11B | L172V | A | 882 | C | SLC25A13 | D565Y | C | 1766 | A | SPAG1 | - | A | 0 | G |
| RMND5A | P319H | C | 1451 | A | SEC11B | L112V | A | 702 | C | SLC25A15 | A147V | C | 676 | T | SPAG1 | A528V | C | 1669 | T |
| RMND5A | E181D | A | 1038 | C | SEC14L1 | R168C | C | 722 | T | SLC25A15 | V130M | G | 624 | A | SPAG1 | L43I | C | 213 | A |
| RMND5A | K365T | A | 1589 | C | SEC14L1 | R387W | C | 1379 | T | SLC25A16 | T286I | G | 956 | A | SPAG1 | L525M | C | 1659 | A |
| RMND5B | R184H | G | 902 | A | SEC14L1 | T382I | C | 1365 | T | SLC25A16 | N232S | T | 794 | C | SPAG16 | V451A | T | 1447 | C |
| RNASE11 | R122H | C | 828 | T | SEC14L1 | S88Y | C | 483 | A | SLC25A2 | L240I | G | 898 | T | SPAG16 | S467C | A | 1494 | T |

| | | | | | | | | | | | | | | | | | | | |
|---|---|---|---|---|---|---|---|---|---|---|---|---|---|---|---|---|---|---|---|
| RNASE11 | K115E | T | 806 | C | SEC14L3 | P199L | G | 687 | A | SLC25A2 | A15V | G | 224 | A | SPAG16 | G448C | G | 1437 | T |
| RNASE11 | S150N | C | 912 | T | SEC14L3 | E235G | T | 795 | C | SLC25A2 | M37T | A | 290 | G | SPAG16 | D427N | G | 1374 | A |
| RNASE13 | Y73H | A | 355 | G | SEC14L4 | D107N | C | 403 | T | SLC25A2 | R61Q | C | 362 | T | SPAG16 | R628* | C | 1977 | T |
| RNASE13 | - | A | 607 | C | SEC14L5 | R67W | C | 379 | T | SLC25A21 | G71S | C | 521 | T | SPAG17 | N311K | A | 999 | T |
| RNASE13 | E143G | T | 566 | C | SEC16A | P517L | G | 2158 | A | SLC25A22 | A240V | G | 1061 | A | SPAG17 | R1486G | T | 4522 | C |
| RNASE9 | P65H | G | 342 | T | SEC16A | E1866* | C | 6204 | A | SLC25A25 | R315C | C | 970 | T | SPAG17 | E2197* | C | 6655 | A |
| RNASE9 | - | C | 781 | A | SEC16A | A1636V | G | 5515 | A | SLC25A25 | A340T | G | 1045 | A | SPAG17 | I1320M | A | 4026 | C |
| RNASEH1 | G167C | C | 855 | A | SEC16A | D1087N | C | 3867 | T | SLC25A25 | F307L | C | 948 | A | SPAG17 | E991D | C | 3039 | A |
| RNASEH2B | K306T | A | 1316 | C | SEC16B | R546H | C | 2379 | T | SLC25A26 | V269A | T | 1534 | C | SPAG17 | E905* | C | 2779 | A |
| RNASEK | - | A | 326 | G | SEC16B | E149K | C | 1187 | T | SLC25A27 | G206A | G | 869 | C | SPAG17 | V84G | A | 317 | C |
| RNASEL | V319A | A | 1210 | G | SEC22A | G222C | G | 712 | T | SLC25A28 | R112C | G | 363 | A | SPAG4 | K398N | G | 1306 | T |
| RNASEL | F123L | A | 623 | C | SEC22A | R209Q | G | 674 | A | SLC25A28 | T314M | G | 970 | A | SPAG5 | R797W | G | 2481 | A |
| RNASEN | H609Y | G | 1872 | A | SEC22C | I141N | A | 566 | T | SLC25A29 | S64L | G | 492 | A | SPAG6 | A540E | C | 1761 | A |
| RNASEN | R271* | G | 858 | A | SEC23A | R216C | G | 871 | A | SLC25A29 | R129C | G | 686 | A | SPAG6 | V21M | G | 203 | A |
| RNASEN | R778C | G | 2379 | A | SEC23B | E109K | G | 465 | A | SLC25A30 | R227C | G | 815 | A | SPAG8 | Q279R | T | 961 | C |
| RNASEN | R759H | C | 2323 | T | SEC23IP | G770R | G | 2380 | A | SLC25A31 | P221L | C | 830 | T | SPAG8 | R387Q | C | 1285 | T |
| RNASEN | K382N | C | 1193 | A | SEC23IP | S235Y | C | 776 | A | SLC25A32 | F100L | A | 601 | G | SPAG9 | R1094* | G | 3489 | A |
| RNASET2 | K255T | T | 1171 | G | SEC23IP | R569Q | G | 1778 | A | SLC25A32 | G3A | C | 311 | G | SPAG9 | Q69R | T | 415 | C |
| RND1 | L165P | A | 625 | G | SEC24B | I954N | T | 2916 | A | SLC25A32 | P42S | G | 427 | A | SPAG9 | T1112M | G | 3544 | A |
| RND1 | D75N | C | 354 | T | SEC24B | Y1122H | T | 3419 | C | SLC25A32 | F278L | A | 1137 | C | SPAG9 | P1187S | G | 3768 | A |
| RNF10 | T36A | A | 106 | G | SEC24B | S856L | C | 2622 | T | SLC25A36 | R282H | G | 1013 | A | SPAG9 | V1071A | A | 3421 | G |
| RNF10 | R149W | C | 445 | T | SEC24B | N472S | A | 1470 | G | SLC25A37 | P234Q | C | 854 | A | SPAM1 | R156I | G | 824 | T |
| RNF10 | K435N | G | 1305 | T | SEC24B | F847L | T | 2596 | G | SLC25A37 | - | T | 0 | A | SPANXE | R54C | G | 494 | A |
| RNF10 | T418I | C | 1253 | T | SEC24B | R912Q | G | 2790 | A | SLC25A38 | R281H | G | 1219 | A | SPANXN3 | E75D | C | 309 | A |
| RNF103 | E226* | C | 1645 | A | SEC24C | V997I | G | 3071 | A | SLC25A38 | Y248F | A | 1120 | T | SPARC | A282T | C | 1158 | T |
| RNF103 | Q474P | T | 2390 | G | SEC24C | R1040W | C | 3200 | T | SLC25A4 | R244Q | G | 863 | A | SPARC | R195Q | C | 898 | T |
| RNF103 | V560I | C | 2647 | T | SEC24C | I824V | A | 2552 | G | SLC25A4 | A150T | G | 580 | A | SPARC | F6L | G | 332 | T |
| RNF103 | V683I | C | 3016 | T | SEC24C | R222Q | G | 747 | A | SLC25A4 | K23N | G | 201 | T | SPAST | R499C | C | 1620 | T |
| RNF103 | E305K | C | 1882 | T | SEC24C | R661M | G | 2064 | T | SLC25A4 | T24S | A | 202 | T | SPAST | I580V | A | 1863 | G |
| RNF111 | L972R | T | 3337 | G | SEC24C | R1090W | C | 3350 | T | SLC25A42 | Y121C | A | 513 | G | SPAST | P293L | C | 1003 | T |
| RNF111 | E264* | G | 1212 | T | SEC24D | G145D | C | 646 | T | SLC25A43 | F246L | C | 1082 | A | SPAST | F465V | T | 1518 | G |
| RNF113A | P294L | G | 1096 | A | SEC24D | M1T | A | 214 | G | SLC25A45 | R243C | G | 931 | A | SPATA1 | N105H | A | 512 | C |
| RNF115 | F87C | T | 464 | G | SEC24D | V7M | C | 231 | T | SLC25A46 | T226A | A | 676 | G | SPATA12 | S15A | T | 718 | G |
| RNF121 | R78H | G | 594 | A | SEC31A | T406M | G | 1218 | A | SLC25A5 | T24M | C | 187 | T | SPATA13 | R715G | A | 2141 | G |
| RNF123 | E959D | G | 2983 | T | SEC31A | A1016T | C | 3047 | T | SLC25A5 | R31W | C | 207 | T | SPATA13 | K154T | A | 459 | C |
| RNF123 | D629G | A | 1992 | G | SEC31A | R254C | G | 761 | A | SLC26A1 | K401M | T | 1580 | A | SPATA13 | N673H | A | 2015 | C |
| RNF123 | S1080G | A | 3344 | G | SEC31B | A1152V | G | 3553 | A | SLC26A1 | V447M | C | 1717 | T | SPATA16 | F229L | G | 871 | T |
| RNF123 | R1209H | G | 3732 | A | SEC31B | H342Y | G | 1122 | A | SLC26A10 | D25G | A | 385 | G | SPATA17 | E270* | G | 863 | T |
| RNF130 | R232C | G | 1110 | A | SEC61A1 | L181I | C | 786 | A | SLC26A10 | S59Y | C | 487 | A | SPATA18 | A338T | G | 1386 | A |

| | | | | | | | | | | | | | | | | | | | |
|---|---|---|---|---|---|---|---|---|---|---|---|---|---|---|---|---|---|---|---|
| RNF130 | D228N | C | 1098 | T | SEC61A2 | Q398H | G | 1283 | T | SLC26A11 | - | G | 0 | T | SPATA18 | L321P | T | 1336 | C |
| RNF130 | S209P | A | 1041 | G | SEC61A2 | F423L | T | 1356 | C | SLC26A2 | S347A | T | 1307 | G | SPATA18 | R498Q | G | 1867 | A |
| RNF133 | R225* | G | 911 | A | SEC63 | K633N | T | 2079 | G | SLC26A3 | N161T | T | 667 | G | SPATA19 | R139Q | C | 467 | T |
| RNF135 | L425P | T | 1325 | C | SEC63 | E335* | C | 1183 | A | SLC26A3 | - | C | 0 | A | SPATA19 | D152Y | C | 505 | A |
| RNF138 | Y145C | A | 882 | G | SECISBP2 | A512V | C | 1606 | T | SLC26A3 | L659I | G | 2160 | T | SPATA2 | L298P | A | 1124 | G |
| RNF138 | N166K | T | 946 | A | SECISBP2 | L854V | T | 2631 | G | SLC26A3 | K523T | T | 1753 | G | SPATA20 | R47W | C | 259 | T |
| RNF139 | R121Q | G | 705 | A | SECISBP2 L | V861I | C | 2780 | T | SLC26A3 | E277* | C | 1014 | A | SPATA20 | R80H | G | 359 | A |
| RNF139 | A415T | G | 1586 | A | SECISBP2 L | G1077V | C | 3429 | A | SLC26A4 | P140H | C | 643 | A | SPATA21 | T256M | G | 1250 | A |
| RNF139 | W419R | T | 1598 | C | SECISBP2 L | R673I | C | 2217 | A | SLC26A4 | L251I | C | 975 | A | SPATA21 | F280L | G | 1323 | T |
| RNF139 | L402P | T | 1548 | C | SECISBP2 L | K185Q | T | 752 | G | SLC26A5 | K608T | T | 2059 | G | SPATA22 | E352D | C | 1179 | A |
| RNF139 | I147L | A | 782 | C | SECTM1 | G72R | C | 612 | T | SLC26A5 | L440M | G | 1554 | T | SPATA22 | Q206H | T | 741 | G |
| RNF139 | R217Q | G | 993 | A | SEH1L | P145Q | C | 572 | A | SLC26A5 | R15M | C | 280 | A | SPATA24 | S237P | A | 737 | G |
| RNF14 | P312L | C | 974 | T | SEH1L | R406Q | G | 1355 | A | SLC26A5 | P331L | G | 1228 | A | SPATA24 | A218T | C | 680 | T |
| RNF14 | F77C | T | 269 | G | SEL1L | S421L | G | 1307 | A | SLC26A5 | - | C | 0 | A | SPATA24 | - | C | 0 | A |
| RNF144A | R194* | C | 1022 | T | SEL1L | A752V | G | 2300 | A | SLC26A5 | A343V | G | 1264 | A | SPATA4 | S185L | G | 663 | A |
| RNF144B | V251L | G | 1068 | T | SEL1L | R537P | C | 1655 | G | SLC26A6 | T522M | G | 1613 | A | SPATA4 | N166T | T | 606 | G |
| RNF145 | K23R | T | 95 | C | SEL1L2 | A588T | C | 1837 | T | SLC26A6 | A367T | C | 1147 | T | SPATA5 | R879H | G | 2705 | A |
| RNF148 | R272H | C | 1032 | T | SEL1L2 | S497Y | G | 1565 | T | SLC26A7 | C359* | C | 1316 | A | SPATA5 | D152Y | G | 523 | T |
| RNF149 | R283I | C | 956 | A | SEL1L2 | A486T | C | 1531 | T | SLC26A7 | G314R | G | 1179 | A | SPATA5 | K674T | A | 2090 | C |
| RNF149 | R226C | G | 784 | A | SEL1L3 | - | C | 0 | A | SLC26A7 | D139N | G | 654 | A | SPATA6 | E427K | C | 1444 | T |
| RNF150 | G327W | C | 1013 | A | SEL1L3 | L324I | G | 1093 | T | SLC26A8 | L500M | G | 1852 | T | SPATA7 | T76A | A | 377 | G |
| RNF150 | D289Y | C | 899 | A | SEL1L3 | G251D | C | 875 | T | SLC26A8 | K179N | C | 891 | A | SPATA7 | D575N | G | 1874 | A |
| RNF152 | G115R | C | 1443 | T | SEL1L3 | K200N | C | 723 | A | SLC26A9 | A254T | C | 762 | T | SPATC1 | P477S | C | 1531 | T |
| RNF157 | R652Q | C | 2024 | T | SELE | R375* | G | 1263 | A | SLC26A9 | A439T | C | 1317 | T | SPATC1 | A108T | G | 424 | A |
| RNF157 | W674C | C | 2091 | A | SELE | A182V | G | 685 | A | SLC26A9 | P325T | G | 975 | T | SPATC1 | R367Q | G | 1202 | A |
| RNF157 | T601M | G | 1871 | A | SELE | L7I | G | 159 | T | SLC26A9 | Q527P | T | 1582 | G | SPATS2 | A100T | G | 1287 | A |
| RNF157 | S420P | A | 1327 | G | SELENBP1 | R23H | C | 213 | T | SLC26A9 | R317C | G | 951 | A | SPATS2 | S19P | T | 1044 | C |
| RNF160 | N582K | A | 1752 | C | SELENBP1 | R237Q | C | 855 | T | SLC26A9 | Q614K | G | 1842 | T | SPATS2L | E157* | G | 569 | T |
| RNF160 | P10S | G | 34 | A | SELL | - | C | 0 | A | SLC26A9 | P234Q | G | 703 | T | SPATS2L | S399* | C | 1296 | A |
| RNF160 | M1455I | C | 4371 | T | SELL | D151Y | C | 612 | A | SLC26A9 | S218L | G | 655 | A | SPC24 | A90S | C | 274 | A |
| RNF160 | R1111H | C | 3338 | T | SELP | R429W | G | 1353 | A | SLC26A9 | R38C | G | 114 | A | SPC25 | E77D | T | 373 | G |
| RNF160 | S1632N | C | 4901 | T | SELP | R656M | C | 2035 | A | SLC27A1 | Y267H | T | 1042 | C | SPC25 | D10N | C | 170 | T |
| RNF160 | C1220R | A | 3664 | G | SELP | R458W | G | 1440 | A | SLC27A1 | A477T | G | 1672 | A | SPC25 | E5K | C | 155 | T |
| RNF160 | E1355* | C | 4069 | A | SELP | R556* | G | 1734 | A | SLC27A1 | A389V | C | 1409 | T | SPCS2 | A4S | G | 49 | T |
| RNF160 | L974R | A | 2927 | C | SELP | C448* | G | 1412 | T | SLC27A2 | F361L | T | 1312 | C | SPCS2 | K47Q | A | 178 | C |
| RNF160 | L575F | C | 1731 | A | SELPLG | S409N | C | 1384 | T | SLC27A2 | G278* | G | 1063 | T | SPDEF | S264R | G | 1207 | T |

| | | | | | | | | | | | | | | | | | | | |
|---|---|---|---|---|---|---|---|---|---|---|---|---|---|---|---|---|---|---|---|
| RNF160 | K191Q | T | 577 | G | SELPLG | Q122H | C | 524 | A | SLC27A2 | K428N | A | 1515 | C | SPDEF | A299T | C | 1310 | T |
| RNF165 | Q323R | A | 1019 | G | SEMA3A | V14A | A | 356 | G | SLC27A2 | R523C | C | 1798 | T | SPDYC | R290C | C | 950 | T |
| RNF166 | R179C | G | 631 | A | SEMA3A | R613Q | C | 2153 | T | SLC27A3 | G462S | G | 2144 | A | SPDYE3 | R518C | C | 1736 | T |
| RNF167 | R205Q | G | 1270 | A | SEMA3A | R554K | C | 1976 | T | SLC27A3 | R539H | G | 2376 | A | SPEF2 | E1334* | G | 4154 | T |
| RNF168 | A92T | C | 869 | T | SEMA3A | Q433H | T | 1614 | G | SLC27A3 | A173T | G | 1277 | A | SPEF2 | R1366Q | G | 4251 | A |
| RNF168 | E246* | C | 1331 | A | SEMA3C | R528W | G | 2144 | A | SLC27A3 | R599W | C | 2555 | T | SPEF2 | P1321T | C | 4115 | A |
| RNF168 | I80L | T | 833 | G | SEMA3D | N686S | T | 2101 | C | SLC27A3 | R768W | C | 3062 | T | SPEF2 | I248N | T | 897 | A |
| RNF17 | Y743C | A | 2269 | G | SEMA3D | R642I | C | 1969 | A | SLC27A4 | R40H | G | 236 | A | SPEF2 | D279N | G | 989 | A |
| RNF17 | R1587W | C | 4800 | T | SEMA3D | T320I | G | 1003 | A | SLC27A4 | V626I | G | 1993 | A | SPEF2 | E596* | G | 1940 | T |
| RNF17 | A441T | G | 1362 | A | SEMA3D | L296M | G | 930 | T | SLC27A5 | L458I | G | 1482 | T | SPEF2 | R794C | C | 2534 | T |
| RNF17 | F959C | T | 2917 | G | SEMA3E | P769H | G | 2774 | T | SLC27A5 | P23S | G | 177 | A | SPEF2 | A1772V | C | 5469 | T |
| RNF17 | E1554* | G | 4701 | T | SEMA3E | G574* | C | 2188 | A | SLC27A5 | G595D | C | 1894 | T | SPEG | E2291K | G | 7003 | A |
| RNF170 | Y248C | T | 906 | C | SEMA3F | R477C | C | 1913 | T | SLC27A5 | R248H | C | 853 | T | SPEG | R1875Q | G | 5756 | A |
| RNF175 | S221R | T | 1034 | G | SEMA3F | G305D | G | 1398 | A | SLC27A6 | F473S | T | 2428 | C | SPEG | R771Q | G | 2444 | A |
| RNF181 | D22Y | G | 62 | T | SEMA3F | R477C | C | 1913 | T | SLC27A6 | L558F | A | 2684 | C | SPEG | H1355Y | C | 4195 | T |
| RNF182 | I51T | T | 675 | C | SEMA3G | V729M | C | 2185 | T | SLC28A3 | A392V | G | 1225 | A | SPEG | L1327M | C | 4111 | A |
| RNF183 | E153* | C | 731 | A | SEMA3G | R721W | G | 2161 | A | SLC28A3 | R154* | G | 510 | A | SPEG | P845L | C | 2666 | T |
| RNF185 | R109C | C | 525 | T | SEMA3G | R225Q | C | 674 | T | SLC28A3 | G575R | C | 1773 | T | SPEG | P3117S | C | 9481 | T |
| RNF185 | G31R | G | 291 | A | SEMA3G | R278L | C | 833 | A | SLC28A3 | R551C | G | 1701 | A | SPEG | Y3152D | T | 9586 | G |
| RNF187 | R101Q | G | 1057 | A | SEMA4A | A509S | G | 1629 | T | SLC28A3 | A252V | G | 805 | A | SPEG | R1803Q | G | 5540 | A |
| RNF19A | A641T | C | 2019 | T | SEMA4A | R658W | C | 2076 | T | SLC28A3 | R154* | G | 510 | A | SPEG | A3191T | G | 9703 | A |
| RNF19A | N801T | T | 2500 | G | SEMA4B | A574T | G | 2003 | A | SLC29A1 | R190Q | G | 569 | A | SPEM1 | S19N | G | 81 | A |
| RNF19B | S525G | T | 1636 | C | SEMA4B | E65K | G | 476 | A | SLC29A1 | L371P | T | 1112 | C | SPEM1 | Y303C | A | 933 | G |
| RNF2 | A286T | G | 1144 | A | SEMA4B | V479M | G | 1718 | A | SLC29A2 | W29R | A | 314 | G | SPEN | R176Q | G | 731 | A |
| RNF2 | R43Q | G | 416 | A | SEMA4C | R815C | G | 2576 | A | SLC29A3 | S285L | C | 906 | T | SPEN | R1959G | C | 6079 | G |
| RNF20 | - | G | 0 | A | SEMA4D | E510K | C | 2305 | T | SLC29A4 | R424C | C | 1431 | T | SPEN | D2905N | G | 8917 | A |
| RNF20 | K689N | G | 2157 | T | SEMA4D | L322M | G | 1741 | T | SLC29A4 | D286N | G | 1017 | A | SPEN | T3045A | A | 9337 | G |
| RNF20 | G316S | G | 1036 | A | SEMA4D | A130T | C | 1165 | T | SLC29A4 | G498E | G | 1654 | A | SPEN | T907I | C | 2924 | T |
| RNF20 | R748H | G | 2333 | A | SEMA4D | T634M | G | 2678 | A | SLC29A4 | D154N | G | 621 | A | SPEN | K1131T | A | 3596 | C |
| RNF20 | L543V | T | 1717 | G | SEMA4F | A625T | G | 2022 | A | SLC2A10 | A111T | G | 581 | A | SPEN | E1242* | G | 3928 | T |
| RNF207 | K378N | G | 1261 | T | SEMA4F | R569C | C | 1854 | T | SLC2A10 | A449T | G | 1595 | A | SPEN | K1596Q | A | 4990 | C |
| RNF207 | L193P | T | 705 | C | SEMA4F | V577M | G | 1878 | A | SLC2A13 | E7D | C | 72 | A | SPEN | S3492F | C | 1067 9 | T |
| RNF208 | S133Y | G | 567 | T | SEMA4G | R268H | G | 881 | A | SLC2A13 | L109I | G | 376 | T | SPEN | L182I | C | 748 | A |
| RNF212 | F297V | A | 951 | C | SEMA4G | R521Q | G | 1640 | A | SLC2A13 | R323H | C | 1019 | T | SPERT | V98G | T | 373 | G |
| RNF212 | E117* | C | 411 | A | SEMA4G | Y131C | A | 470 | G | SLC2A14 | F235V | A | 703 | C | SPERT | R33I | G | 178 | T |
| RNF213 | A3732T | G | 1133 9 | A | SEMA5A | V989I | C | 3631 | T | SLC2A2 | L368I | G | 1182 | T | SPERT | Q384P | A | 1231 | C |
| RNF213 | R2902C | C | 8849 | T | SEMA5A | R433W | G | 1963 | A | SLC2A2 | V45G | A | 214 | C | SPESP1 | I233M | T | 853 | G |
| RNF213 | Q4029* | C | 1223 | T | SEMA5A | E526G | T | 2243 | C | SLC2A2 | V87L | C | 339 | G | SPG11 | R2211C | G | 6648 | A |

| | | | | | | | | | | | | | | | | | | |
|---|---|---|---|---|---|---|---|---|---|---|---|---|---|---|---|---|---|---|
| | | | 0 | | | | | | | | | | | | | | | |
| RNF213 | T3263M | C | 9933 | T | SEMA5A | S528G | T | 2248 | C | SLC2A3 | A401V | G | 1443 | A | SPG11 | M935T | A | 2821 | G |
| RNF213 | E2162K | G | 6629 | A | SEMA5A | A315V | G | 1610 | A | SLC2A3 | L213F | G | 878 | A | SPG11 | F626L | A | 1893 | G |
| RNF213 | V9F | G | 170 | T | SEMA5B | R985Q | C | 3341 | T | SLC2A4R G | S163N | G | 540 | A | SPG20 | E406A | T | 1278 | G |
| RNF213 | V1469M | G | 4550 | A | SEMA5B | V15I | C | 430 | T | SLC2A4R G | L120I | C | 410 | A | SPG20 | S206Y | G | 678 | T |
| RNF213 | D1600V | A | 4944 | T | SEMA5B | G373R | C | 1504 | T | SLC2A5 | R238C | G | 892 | A | SPG21 | A283T | C | 1143 | T |
| RNF213 | A3193V | C | 9723 | T | SEMA5B | M1I | C | 390 | T | SLC2A5 | - | C | 0 | A | SPG21 | V269I | C | 1101 | T |
| RNF213 | K5095R | A | 1542 9 | G | SEMA5B | R604Q | C | 2198 | T | SLC2A6 | R224W | G | 748 | A | SPG21 | K117T | T | 646 | G |
| RNF214 | K224N | G | 681 | T | SEMA5B | A674V | G | 2408 | A | SLC2A6 | A129V | G | 464 | A | SPG7 | R391W | C | 1186 | T |
| RNF214 | S519N | G | 1565 | A | SEMA5B | R320L | C | 1346 | A | SLC2A7 | R237* | G | 709 | A | SPG7 | A149T | G | 460 | A |
| RNF216 | R518Q | C | 1821 | T | SEMA5B | G613S | C | 2224 | T | SLC2A7 | A151T | C | 451 | T | SPHK1 | Q125H | G | 872 | T |
| RNF216 | R677W | T | 2297 | A | SEMA5B | R635Q | C | 2291 | T | SLC2A7 | G354S | C | 1060 | T | SPHK2 | A516D | C | 2252 | A |
| RNF219 | R235C | G | 762 | A | SEMA6A | R712C | G | 2383 | A | SLC2A8 | R182C | C | 633 | T | SPHK2 | R322C | C | 1669 | T |
| RNF219 | Q647* | G | 1998 | A | SEMA6A | Q783* | G | 2596 | A | SLC2A8 | L210P | T | 718 | C | SPHK2 | P492H | C | 2180 | A |
| RNF219 | S717G | T | 2208 | C | SEMA6C | P937S | G | 2938 | A | SLC2A8 | A310T | G | 1017 | A | SPHKAP | A1113V | G | 3385 | A |
| RNF220 | - | A | 0 | C | SEMA6D | A1007V | C | 3459 | T | SLC2A9 | P434L | G | 1355 | A | SPHKAP | R1142I | C | 3472 | A |
| RNF24 | - | A | 0 | G | SEMA6D | E106* | G | 755 | T | SLC2A9 | R539S | C | 1671 | A | SPHKAP | L1692I | G | 5121 | T |
| RNF25 | S90T | A | 709 | T | SEMA6D | I306T | T | 1356 | C | SLC2A9 | D528G | T | 1637 | C | SPHKAP | S1392Y | G | 4222 | T |
| RNF25 | R233C | G | 1138 | A | SEMA6D | D484E | C | 1891 | G | SLC2A9 | - | C | 0 | A | SPHKAP | S1386Y | G | 4204 | T |
| RNF25 | R323M | C | 1409 | A | SEMA7A | K365N | C | 1136 | A | SLC30A1 | R361* | G | 1192 | A | SPHKAP | F178C | A | 580 | C |
| RNF26 | L426M | C | 1872 | A | SEMA7A | C500* | G | 1541 | T | SLC30A10 | G467R | C | 1561 | T | SPHKAP | R1169W | G | 3552 | A |
| RNF31 | R616C | C | 2015 | T | SEMA7A | R261H | C | 823 | T | SLC30A10 | R177W | G | 691 | A | SPIC | L86M | C | 282 | A |
| RNF31 | R621C | C | 2030 | T | SEMA7A | R261H | C | 823 | T | SLC30A3 | V232I | C | 1047 | T | SPIN1 | F244Y | T | 1009 | A |
| RNF32 | R32* | C | 212 | T | SEMG1 | T139K | C | 473 | A | SLC30A3 | R132H | C | 748 | T | SPIN1 | R122Q | G | 643 | A |
| RNF32 | E109* | G | 443 | T | SEMG1 | H399Y | C | 1252 | T | SLC30A4 | L21P | A | 377 | G | SPINK1 | R67C | G | 408 | A |
| RNF32 | N49K | C | 265 | A | SEMG2 | S386C | C | 1247 | G | SLC30A5 | L467M | C | 2009 | A | SPINK14 | G34V | G | 101 | T |
| RNF34 | R366Q | G | 1274 | A | SEMG2 | V71A | T | 302 | C | SLC30A5 | L163V | T | 1097 | G | SPINK2 | R130Q | C | 469 | T |
| RNF34 | D234A | A | 878 | C | SEMG2 | E163D | A | 579 | C | SLC30A6 | R344* | C | 1067 | T | SPINK5 | E649A | A | 2019 | C |
| RNF34 | A278G | C | 1010 | G | SEMG2 | L273F | C | 907 | T | SLC30A6 | T362I | C | 1122 | T | SPINK5 | Q27R | A | 153 | G |
| RNF34 | E303D | G | 1086 | T | SENP1 | R165H | C | 973 | T | SLC30A6 | L353I | C | 1094 | A | SPINK5 | G144C | G | 503 | T |
| RNF34 | D323N | G | 1144 | A | SENP1 | R352Q | C | 1534 | T | SLC30A7 | K7Q | A | 206 | C | SPINK5 | E292* | G | 947 | T |
| RNF40 | A501T | G | 1655 | A | SENP1 | P399S | G | 1674 | A | SLC30A8 | Q172H | G | 745 | T | SPINK5 | K305N | G | 988 | T |
| RNF40 | S402C | C | 1359 | G | SENP2 | P69L | C | 206 | T | SLC30A8 | L315I | C | 1172 | A | SPINK5 | R853* | C | 2630 | T |
| RNF40 | - | A | 0 | G | SENP3 | R31C | C | 364 | T | SLC30A8 | F363L | C | 1318 | A | SPINT1 | R406H | G | 1426 | A |
| RNF41 | R168* | G | 871 | A | SENP5 | T69M | C | 455 | T | SLC30A9 | E525D | A | 1755 | C | SPINT3 | R20* | G | 58 | A |
| RNF43 | R132* | G | 882 | A | SENP5 | K317R | A | 1199 | G | SLC32A1 | A495T | G | 1746 | A | SPINT4 | F55C | T | 181 | G |
| RNF43 | R132* | G | 882 | A | SENP6 | Y657* | T | 2590 | A | SLC32A1 | F306C | T | 1180 | G | SPIRE1 | A493V | G | 1746 | A |

| | | | | | | | | | | | | | | | | | | | |
|---|---|---|---|---|---|---|---|---|---|---|---|---|---|---|---|---|---|---|---|
| RNF43 | R561Q | C | 2170 | T | SENP6 | R737W | C | 2828 | T | SLC32A1 | M330V | A | 1251 | G | SPIRE1 | E684D | T | 2320 | G |
| RNF43 | T142A | T | 912 | C | SENP6 | E526G | A | 2196 | G | SLC33A1 | Q356H | C | 1449 | A | SPIRE1 | R567H | C | 1968 | T |
| RNF43 | R454C | G | 1848 | A | SENP7 | Y862* | A | 2640 | C | SLC34A1 | E334G | A | 1092 | G | SPIRE1 | F225L | A | 943 | C |
| RNF43 | R609Q | C | 2314 | T | SEPHS1 | E13K | C | 413 | T | SLC34A1 | R439K | G | 1407 | A | SPIRE1 | D312G | T | 1203 | C |
| RNF43 | G133E | C | 886 | T | SEPHS1 | G270R | C | 1184 | T | SLC34A1 | P442S | C | 1415 | T | SPIRE2 | K384T | A | 1203 | C |
| RNF44 | A389V | G | 1691 | A | SEPHS1 | A245V | G | 1110 | A | SLC34A1 | Y511C | A | 1623 | G | SPIRE2 | R294C | C | 932 | T |
| RNF44 | T115M | G | 869 | A | SEPHS1 | M187L | T | 935 | G | SLC34A1 | L551M | C | 1742 | A | SPIRE2 | V595I | G | 1835 | A |
| RNF44 | R390Q | C | 1694 | T | SEPT1 | R246H | C | 924 | T | SLC34A1 | G149R | G | 536 | A | SPIRE2 | P429R | C | 1338 | G |
| RNF44 | R52H | C | 680 | T | SEPT10 | G183D | C | 548 | T | SLC34A2 | T543M | C | 1678 | T | SPIRE2 | S122G | A | 416 | G |
| RNF44 | D221G | T | 1187 | C | SEPT10 | R278H | C | 833 | T | SLC34A2 | R617C | C | 1899 | T | SPIRE2 | R357H | G | 1122 | A |
| RNF44 | S36G | T | 631 | C | SEPT10 | G439D | C | 1316 | T | SLC34A2 | R179W | C | 585 | T | SPN | R278C | C | 968 | T |
| RNF6 | R508I | C | 1685 | A | SEPT12 | A248V | G | 836 | A | SLC34A2 | K585N | G | 1805 | T | SPN | V289M | G | 1001 | A |
| RNF7 | Q70R | A | 347 | G | SEPT14 | R157H | C | 587 | T | SLC34A2 | L547F | C | 1689 | T | SPN | A307V | C | 1056 | T |
| RNF8 | R472Q | G | 1608 | A | SEPT14 | G219D | C | 773 | T | SLC34A3 | G495R | G | 1669 | A | SPNS1 | R239Q | G | 1093 | A |
| RNFT1 | R297Q | C | 946 | T | SEPT14 | S171P | A | 628 | G | SLC35A4 | G274D | G | 1928 | A | SPNS2 | V527I | G | 1793 | A |
| RNFT1 | W423* | C | 1324 | T | SEPT14 | R157H | C | 587 | T | SLC35A5 | N295H | A | 1166 | C | SPNS3 | R289C | C | 909 | T |
| RNFT2 | - | T | 0 | C | SEPT2 | H395R | A | 1184 | G | SLC35B1 | K76E | T | 226 | C | SPNS3 | F63L | C | 233 | A |
| RNFT2 | E252D | G | 989 | T | SEPT2 | Y331C | A | 992 | G | SLC35B2 | R333* | G | 1141 | A | SPOCD1 | K833T | T | 2556 | G |
| RNFT2 | R416C | C | 1479 | T | SEPT3 | E250D | G | 1005 | T | SLC35B2 | R333* | G | 1141 | A | SPOCD1 | W1111C | C | 3391 | G |
| RNGTT | R470H | C | 1596 | T | SEPT4 | R375W | G | 1123 | A | SLC35B3 | S341L | G | 1472 | A | SPOCK3 | G35S | C | 241 | T |
| RNGTT | D205G | T | 801 | C | SEPT4 | K330N | C | 990 | A | SLC35B4 | G178W | C | 832 | A | SPOCK3 | R71H | C | 350 | T |
| RNGTT | R116I | C | 534 | A | SEPT4 | R372W | G | 1114 | A | SLC35B4 | V272I | C | 1114 | T | SPOCK3 | - | C | 0 | A |
| RNMT | G349* | G | 1285 | T | SEPT4 | C392* | G | 1176 | T | SLC35C1 | S92N | G | 987 | A | SPON1 | R634Q | G | 1901 | A |
| RNPC3 | L187I | C | 674 | A | SEPT4 | E368D | C | 1104 | A | SLC35C1 | R108C | C | 1034 | T | SPON1 | I169V | A | 505 | G |
| RNPEPL1 | P286L | C | 1450 | T | SEPT4 | R373* | G | 1117 | A | SLC35C2 | G149W | C | 928 | A | SPON1 | F388L | C | 1164 | A |
| RNPS1 | R66C | G | 637 | A | SEPT6 | S220L | G | 924 | A | SLC35C2 | S111L | G | 815 | A | SPON2 | R175C | G | 856 | A |
| ROBO1 | S915G | T | 2857 | C | SEPT8 | Y258C | T | 1011 | C | SLC35D3 | G157R | G | 634 | A | SPON2 | R245Q | C | 1067 | T |
| ROBO1 | A977V | G | 3044 | A | SEPT8 | P183H | G | 786 | T | SLC35D3 | A189V | C | 731 | T | SPOP | K180E | T | 904 | C |
| ROBO1 | E1329* | C | 4099 | A | SEPT9 | G139D | G | 542 | A | SLC35E1 | D390G | T | 1187 | C | SPOP | A61T | C | 547 | T |
| ROBO1 | S1606P | A | 4930 | G | SEPT9 | T346M | C | 1163 | T | SLC35E4 | R300W | C | 1697 | T | SPP1 | A213V | C | 765 | T |
| ROBO1 | G859V | C | 2690 | A | SEPT9 | R84H | G | 377 | A | SLC35E4 | R42L | G | 924 | T | SPP1 | Q58* | C | 299 | T |
| ROBO1 | T996M | G | 3101 | A | SEPX1 | M1V | T | 32 | C | SLC35F1 | V366M | G | 1297 | A | SPP2 | M10T | T | 137 | C |
| ROBO1 | Q883H | T | 2763 | G | SERAC1 | E97* | C | 421 | A | SLC35F2 | Y209D | A | 786 | C | SPP2 | D75Y | G | 331 | T |
| ROBO1 | R1472C | G | 4528 | A | SERBP1 | P98L | G | 379 | A | SLC35F4 | S177F | G | 530 | A | SPRED1 | V229A | T | 1021 | C |
| ROBO1 | P1505H | G | 4628 | T | SERGEF | R186* | G | 708 | A | SLC35F4 | D444N | C | 1330 | T | SPRED1 | K288T | A | 1198 | C |
| ROBO1 | S1442G | T | 4438 | C | SERHL2 | R183I | G | 650 | T | SLC35F4 | R236W | G | 706 | A | SPRED2 | R323Q | C | 1035 | T |
| ROBO1 | M189I | C | 681 | A | SERINC1 | S301N | C | 1046 | T | SLC35F5 | A213G | G | 1052 | C | SPRED2 | E273K | C | 884 | T |
| ROBO1 | Y1009H | A | 3139 | G | SERINC1 | G77D | C | 374 | T | SLC35F5 | Q506H | C | 1932 | A | SPRED3 | L194F | C | 683 | T |
| ROBO1 | V850A | A | 2663 | G | SERINC1 | P444S | G | 1474 | A | SLC35F5 | C248* | G | 1158 | T | SPRED3 | G199E | G | 699 | A |

| | | | | | | | | | | | | | | | | | | | |
|---|---|---|---|---|---|---|---|---|---|---|---|---|---|---|---|---|---|---|---|
| ROBO1 | E255K | C | 877 | T | SERINC2 | G422S | G | 1537 | A | SLC35F5 | L116* | A | 761 | T | SPRR2A | K66T | T | 283 | G |
| ROBO2 | D385N | G | 1209 | A | SERINC2 | R42H | G | 398 | A | SLC35F5 | R133C | G | 811 | A | SPRR2D | S71I | C | 272 | A |
| ROBO2 | K1053N | G | 3215 | T | SERINC4 | S329G | T | 1220 | C | SLC36A1 | P128S | C | 605 | T | SPRR2G | P70H | G | 248 | T |
| ROBO2 | D1125Y | G | 3429 | T | SERINC4 | R309H | C | 1161 | T | SLC36A2 | S275Y | G | 926 | T | SPRR4 | P56L | C | 216 | T |
| ROBO2 | P1196H | C | 3643 | A | SERINC5 | K111T | T | 489 | G | SLC36A4 | K442N | C | 1423 | A | SPRY1 | R66Q | G | 384 | A |
| ROBO2 | S1334R | A | 4056 | C | SERP1 | S65Y | G | 558 | T | SLC36A4 | R152W | G | 551 | A | SPRY2 | C244W | G | 1113 | C |
| ROBO2 | - | G | 0 | A | SERPINA10 | Y292N | A | 1340 | T | SLC36A4 | Q48R | T | 240 | C | SPRYD3 | R32Q | C | 148 | T |
| ROBO2 | R122Q | G | 421 | A | SERPINA10 | E30K | C | 554 | T | SLC37A1 | A317T | G | 1361 | A | SPRYD5 | W131C | G | 393 | T |
| ROBO2 | R173Q | G | 574 | A | SERPINA10 | G430V | C | 1755 | A | SLC37A1 | A446T | G | 1748 | A | SPRYD5 | A47T | G | 139 | A |
| ROBO2 | R1366Q | G | 4153 | A | SERPINA10 | - | A | 0 | C | SLC37A2 | R22* | C | 315 | T | SPSB3 | W151* | C | 544 | T |
| ROBO3 | R115H | G | 536 | A | SERPINA10 | L232I | G | 1160 | T | SLC37A2 | R479W | C | 1686 | T | SPSB3 | S329P | A | 1076 | G |
| ROBO3 | V1308M | G | 4114 | A | SERPINA12 | L391M | G | 1967 | T | SLC37A3 | R137H | C | 614 | T | SPSB4 | R109H | G | 1070 | A |
| ROBO3 | G78V | G | 425 | T | SERPINA12 | K330Q | T | 1784 | G | SLC38A11 | - | C | 0 | A | SPTA1 | A2097V | G | 6471 | A |
| ROBO4 | E882D | C | 2684 | A | SERPINA3 | S226L | C | 761 | T | SLC38A11 | K151T | T | 682 | G | SPTA1 | Q434E | G | 1481 | C |
| ROBO4 | R96W | G | 324 | A | SERPINA3 | R66* | C | 280 | T | SLC38A11 | F206C | A | 847 | C | SPTA1 | A2226T | C | 6857 | T |
| ROBO4 | A764T | C | 2328 | T | SERPINA5 | R376H | G | 1337 | A | SLC38A4 | F491C | A | 1679 | C | SPTA1 | K1374R | T | 4302 | C |
| ROCK1 | E270K | C | 1749 | T | SERPINA5 | P69H | C | 416 | A | SLC38A4 | R292H | C | 1082 | T | SPTA1 | R268* | G | 983 | A |
| ROCK2 | M1367I | C | 4550 | A | SERPINA9 | A191V | G | 733 | A | SLC38A5 | L143M | G | 1271 | T | SPTA1 | R268* | G | 983 | A |
| ROCK2 | N547I | T | 2089 | A | SERPINB10 | A267V | C | 859 | T | SLC38A6 | F293V | T | 993 | G | SPTA1 | F1085L | A | 3436 | C |
| ROCK2 | W190S | C | 1018 | G | SERPINB13 | M214T | T | 743 | C | SLC38A8 | T358M | G | 1073 | A | SPTA1 | E882* | C | 2825 | A |
| ROCK2 | R1380Q | C | 4588 | T | SERPINB2 | D274N | G | 900 | A | SLC38A8 | A171T | C | 511 | T | SPTA1 | K858T | T | 2754 | G |
| ROCK2 | K718T | T | 2602 | G | SERPINB3 | N161S | T | 626 | C | SLC38A9 | S182T | C | 1137 | G | SPTA1 | K199Q | T | 776 | G |
| ROCK2 | R131I | C | 841 | A | SERPINB3 | K296N | C | 1032 | A | SLC39A1 | S176L | G | 637 | A | SPTAN1 | R1534* | C | 4710 | T |
| ROD1 | R65C | G | 193 | A | SERPINB3 | F387L | G | 1305 | T | SLC39A1 | L38V | G | 222 | C | SPTAN1 | A1027T | G | 3189 | A |
| ROD1 | V163I | C | 487 | T | SERPINB3 | H330R | T | 1133 | C | SLC39A10 | E212* | G | 909 | T | SPTAN1 | Q881K | C | 2751 | A |
| ROD1 | S167R | G | 501 | T | SERPINB3 | L114I | G | 484 | T | SLC39A10 | I732T | T | 2470 | C | SPTAN1 | A2064T | G | 6300 | A |
| ROM1 | E204D | A | 1153 | C | SERPINB4 | F28L | G | 200 | T | SLC39A11 | E306K | C | 1010 | T | SPTAN1 | R2069K | G | 6316 | A |
| ROR1 | R117L | G | 725 | T | SERPINB5 | R110I | G | 621 | T | SLC39A12 | H284N | C | 1123 | A | SPTAN1 | E1033D | G | 3209 | T |
| ROR1 | D922N | G | 3139 | A | SERPINB5 | A10S | G | 320 | T | SLC39A13 | L37I | C | 280 | A | SPTB | V2259I | C | 6817 | T |
| ROR1 | H359R | A | 1451 | G | SERPINB5 | T108M | C | 615 | T | SLC39A13 | P181H | C | 713 | A | SPTB | T856M | G | 2609 | A |
| ROR1 | R332C | C | 1369 | T | SERPINB8 | N133H | A | 653 | C | SLC39A14 | F183L | C | 668 | G | SPTB | I1256T | A | 3809 | G |
| ROR1 | G807V | G | 2795 | T | SERPINB8 | E200K | G | 854 | A | SLC39A3 | V206I | C | 870 | T | SPTB | A2090T | C | 6310 | T |
| ROR2 | A100T | C | 497 | T | SERPINB9 | V315M | C | 1033 | T | SLC39A5 | P287L | C | 1090 | T | SPTB | G801V | C | 2444 | A |

| | | | | | | | | | | | | | | | | | | | |
|---|---|---|---|---|---|---|---|---|---|---|---|---|---|---|---|---|---|---|---|
| ROR2 | T327M | G | 1179 | A | SERPINB9 | V267F | C | 889 | A | SLC39A5 | L140I | C | 648 | A | SPTB | V384L | C | 1192 | G |
| ROR2 | N458D | T | 1571 | C | SERPINC1 | G360S | C | 1197 | T | SLC39A8 | K160N | C | 863 | A | SPTB | R447C | G | 1381 | A |
| ROR2 | G200E | C | 798 | T | SERPINC1 | R356C | G | 1185 | A | SLC3A1 | E483* | G | 1523 | T | SPTB | R959Q | C | 2918 | T |
| ROR2 | C135* | G | 604 | T | SERPINC1 | D393A | T | 1297 | G | SLC3A1 | R301W | C | 977 | T | SPTB | Y83D | A | 289 | C |
| ROR2 | R108Q | C | 522 | T | SERPIND1 | V65I | G | 476 | A | SLC3A2 | N342K | C | 1250 | G | SPTBN1 | D161Y | G | 762 | T |
| RORA | F286L | G | 947 | T | SERPINE1 | R210H | G | 786 | A | SLC3A2 | L416I | C | 1470 | A | SPTBN1 | L2084S | T | 6532 | C |
| RORA | I482M | A | 1535 | C | SERPINE1 | K230T | A | 846 | C | SLC3A2 | R634H | G | 2125 | A | SPTBN1 | H1634R | A | 5182 | G |
| ROS1 | N2234K | G | 6901 | C | SERPINE2 | R393Q | C | 1315 | T | SLC40A1 | G206S | C | 1043 | T | SPTBN1 | R384W | C | 1431 | T |
| ROS1 | R360I | C | 1278 | A | SERPINE2 | A81V | G | 379 | A | SLC40A1 | H125R | T | 801 | C | SPTBN1 | A2240T | G | 6999 | A |
| RP1 | T1173K | C | 3666 | A | SERPINE2 | A71V | G | 349 | A | SLC41A1 | A194V | G | 1596 | A | SPTBN1 | R913H | G | 3019 | A |
| RP1 | D1728V | A | 5331 | T | SERPINF2 | Q312R | A | 1012 | G | SLC41A2 | E154K | C | 627 | T | SPTBN1 | - | G | 0 | T |
| RP1 | V1148I | G | 3590 | A | SERPINF2 | I235T | T | 781 | C | SLC41A3 | P351S | G | 1290 | A | SPTBN1 | R1375Q | G | 4405 | A |
| RP1 | L1186V | T | 3704 | G | SERPING1 | A202T | G | 970 | A | SLC43A1 | - | A | 0 | G | SPTBN1 | W970R | T | 3189 | C |
| RP1 | K2022N | G | 6214 | T | SERPING1 | E76K | G | 592 | A | SLC43A1 | R534C | G | 1856 | A | SPTBN1 | Q1440H | A | 4601 | C |
| aaRP1L1 | V1163I | C | 3716 | T | SERPINH1 | S266N | G | 1026 | A | SLC43A2 | V440M | C | 1407 | T | SPTBN2 | R387H | C | 1232 | T |
| RP1L1 | P888L | G | 2892 | A | SERPINI1 | S232Y | C | 1107 | A | SLC43A2 | P513H | G | 1627 | T | SPTBN2 | E1702K | C | 5176 | T |
| RP1L1 | A1709T | C | 5354 | T | SERPINI2 | E245D | T | 1034 | G | SLC44A1 | - | T | 0 | C | SPTBN2 | R1066Q | C | 3269 | T |
| RP1L1 | R1609C | G | 5054 | A | SERPINI2 | Q141E | G | 720 | C | SLC44A2 | G508D | G | 1604 | A | SPTBN2 | R1959Q | C | 5948 | T |
| RP1L1 | R720K | C | 2388 | T | SERTAD1 | G193D | C | 737 | T | SLC44A2 | L41P | T | 203 | C | SPTBN2 | A1171V | G | 3584 | A |
| RP1L1 | R130Q | C | 618 | T | SERTAD1 | P196A | G | 745 | C | SLC44A3 | S425L | C | 1376 | T | SPTBN2 | L1566M | G | 4768 | T |
| RP1L1 | S484N | C | 1680 | T | SERTAD3 | A74V | G | 388 | A | SLC44A3 | G257C | G | 871 | T | SPTBN2 | A2266T | C | 6868 | T |
| RP1L1 | R423Q | C | 1497 | T | SERTAD4 | R51Q | G | 382 | A | SLC44A3 | E602D | A | 1908 | C | SPTBN2 | R1572C | G | 4786 | A |
| RP1L1 | D2313Y | C | 7166 | A | SERTAD4 | V82M | G | 474 | A | SLC44A5 | L126V | A | 490 | C | SPTBN2 | R1686W | G | 5128 | A |
| RP1L1 | E1423* | C | 4496 | A | SERTAD4 | P238L | C | 943 | T | SLC44A5 | D38Y | C | 226 | A | SPTBN2 | G479V | C | 1508 | A |
| RP1L1 | K1419N | T | 4486 | G | SERTAD4 | N8T | A | 253 | C | SLC44A5 | S587P | A | 1873 | G | SPTBN4 | F280L | C | 942 | A |
| RP1L1 | K311T | T | 1161 | G | SESN1 | A124T | C | 371 | T | SLC44A5 | S197R | T | 703 | G | SPTBN4 | L317R | T | 1052 | G |
| RP1L1 | S89N | C | 495 | T | SESN1 | Y446D | A | 1337 | C | SLC44A5 | Q81R | T | 356 | C | SPTBN4 | V937I | G | 2911 | A |
| RP9 | R110H | C | 347 | T | SESN1 | N322D | T | 965 | C | SLC45A1 | G672R | G | 2014 | A | SPTBN4 | R1191C | C | 3673 | T |
| RPA1 | R412H | G | 1357 | A | SESN3 | D337Y | C | 1231 | A | SLC45A1 | R545C | C | 1633 | T | SPTBN4 | R2285H | G | 6956 | A |
| RPA1 | W197G | T | 711 | G | SESN3 | M476I | C | 1650 | T | SLC45A2 | P351S | G | 1198 | A | SPTBN4 | A586V | C | 1859 | T |
| RPA1 | R210H | G | 751 | A | SESN3 | S258Y | G | 995 | T | SLC45A2 | V115M | C | 490 | T | SPTBN4 | R1844* | C | 5632 | T |
| RPA2 | K125N | C | 445 | A | SESTD1 | R570C | G | 2025 | A | SLC45A2 | G343D | C | 1175 | T | SPTBN5 | A1832V | G | 5723 | A |
| RPA4 | R105Q | G | 612 | A | SESTD1 | E656K | C | 2283 | T | SLC45A2 | L107I | G | 466 | T | SPTBN5 | R646H | C | 2165 | T |
| RPAP1 | R137W | G | 533 | A | SESTD1 | Y554C | T | 1978 | C | SLC45A2 | V504I | C | 1657 | T | SPTBN5 | R430H | C | 1517 | T |
| RPAP1 | E509K | C | 1649 | T | SETBP1 | S830F | C | 2795 | T | SLC45A2 | V217A | A | 797 | G | SPTBN5 | A2200S | C | 6826 | A |
| RPAP2 | P515S | C | 1652 | T | SETBP1 | A549T | G | 1951 | A | SLC45A3 | S169R | T | 801 | G | SPTBN5 | R1931H | C | 6020 | T |
| RPAP3 | N239H | T | 715 | G | SETBP1 | A1385T | G | 4459 | A | SLC45A3 | R272C | G | 1110 | A | SPTBN5 | R1709Q | C | 5354 | T |
| RPAP3 | K430T | T | 1289 | G | SETBP1 | E862A | A | 2891 | C | SLC45A3 | R81H | C | 538 | T | SPTBN5 | A3389V | G | 1039<br>4 | A |
| RPAP3 | K82N | T | 246 | G | SETBP1 | R942W | C | 3130 | T | SLC45A3 | H240N | G | 1014 | T | SPTBN5 | A1818T | C | 5680 | T |

| | | | | | | | | | | | | | | | | | | | |
|---|---|---|---|---|---|---|---|---|---|---|---|---|---|---|---|---|---|---|---|
| RPAP3 | E9K | C | 25 | T | SETBP1 | T508M | C | 1829 | T | SLC45A3 | R81C | G | 537 | A | SPTBN5 | R2371Q | C | 7340 | T |
| RPGR | G1019 W | C | 3232 | A | SETBP1 | S716G | A | 2452 | G | SLC45A4 | A394T | C | 1180 | T | SPTBN5 | G3134R | C | 9628 | T |
| RPGR | L237R | A | 887 | C | SETBP1 | R530Q | G | 1895 | A | SLC45A4 | K543T | T | 1628 | G | SPTBN5 | E2510K | C | 7756 | T |
| RPGR | D378N | C | 1309 | T | SETD1A | R312H | G | 1621 | A | SLC45A4 | R766L | C | 2297 | A | SPTBN5 | E510K | C | 1756 | T |
| RPGRIP1 | R984I | G | 2951 | T | SETD1A | R745W | C | 2919 | T | SLC46A3 | D287N | C | 1358 | T | SPTBN5 | A241D | G | 950 | T |
| RPGRIP1 | - | A | 0 | C | SETD1A | R889W | C | 3351 | T | SLC47A1 | V338I | G | 1098 | A | SPTBN5 | R2885C | G | 8881 | A |
| RPGRIP1 | R1018I | G | 3053 | T | SETD1A | T1446M | C | 5023 | T | SLC47A1 | M521I | G | 1649 | A | SPTLC1 | D205G | T | 652 | C |
| RPGRIP1L | R94W | G | 344 | A | SETD1B | L681P | T | 2048 | C | SLC47A1 | A120V | C | 445 | T | SPTLC1 | G489V | C | 1504 | A |
| RPGRIP1L | A284T | C | 914 | T | SETD1B | S350L | C | 1055 | T | SLC47A1 | E273G | A | 904 | G | SPTLC2 | R88* | G | 450 | A |
| RPGRIP1L | Q684R | T | 2115 | C | SETD1B | R996Q | G | 2993 | A | SLC47A2 | N391H | T | 1222 | G | SPTLC3 | R121* | C | 635 | T |
| RPGRIP1L | T677A | T | 2093 | C | SETD1B | R852C | C | 2560 | T | SLC4A1 | P97Q | G | 446 | T | SPTY2D1 | L7I | G | 235 | T |
| RPGRIP1L | R1150W | G | 3512 | A | SETD1B | R966K | G | 2903 | A | SLC4A1 | D6Y | C | 172 | A | SPZ1 | K74Q | A | 412 | C |
| RPGRIP1L | S989Y | G | 3030 | T | SETD1B | L809F | C | 2431 | T | SLC4A1 | D807G | T | 2576 | C | SPZ1 | E239* | G | 907 | T |
| RPGRIP1L | R744* | G | 2294 | A | SETD1B | S1232R | A | 3700 | C | SLC4A1 | R344* | G | 1186 | A | SPZ1 | N364K | T | 1284 | G |
| RPH3A | P190L | C | 569 | T | SETD1B | D1134V | A | 3407 | T | SLC4A10 | R757* | C | 2362 | T | SQLE | R99K | G | 1222 | A |
| RPH3A | C140R | T | 418 | C | SETD1B | P552S | C | 1660 | T | SLC4A10 | H719R | A | 2249 | G | SQSTM1 | M1V | A | 179 | G |
| RPH3A | Q607K | C | 1819 | A | SETD2 | - | C | 0 | A | SLC4A10 | D762A | A | 2378 | C | SRBD1 | E99* | C | 358 | A |
| RPIA | R136C | C | 461 | T | SETD2 | R1322Q | C | 4008 | T | SLC4A10 | E533V | A | 1691 | T | SRBD1 | A180T | C | 601 | T |
| RPIA | V294I | G | 935 | A | SETD2 | L1855P | A | 5607 | G | SLC4A10 | A348V | C | 1136 | T | SRBD1 | V274I | C | 883 | T |
| RPIA | L84M | C | 305 | A | SETD2 | R433P | C | 1341 | G | SLC4A10 | V424G | T | 1364 | G | SRBD1 | K45N | C | 198 | A |
| RPL10L | K156M | T | 556 | A | SETD2 | R1322Q | C | 4008 | T | SLC4A10 | I487F | A | 1552 | T | SRC | P493L | C | 1653 | T |
| RPL10L | Q133R | T | 487 | C | SETD2 | E1115D | T | 3388 | G | SLC4A10 | W498R | T | 1585 | C | SRC | L367M | C | 1274 | A |
| RPL11 | R35* | C | 148 | T | SETD2 | S511Y | G | 1575 | T | SLC4A11 | V167I | C | 601 | T | SRCAP | R2767Q | G | 8685 | A |
| RPL11 | R16C | C | 91 | T | SETD2 | K115N | T | 388 | G | SLC4A11 | R86* | G | 358 | A | SRCAP | R2151H | G | 6837 | A |
| RPL11 | E66G | A | 242 | G | SETD3 | E495G | T | 1644 | C | SLC4A11 | A578T | C | 1834 | T | SRCAP | S2596L | C | 8172 | T |
| RPL13 | S140Y | C | 504 | A | SETD3 | T364I | G | 1251 | A | SLC4A11 | D480G | T | 1541 | C | SRCAP | T2889P | A | 9050 | C |
| RPL15 | R96Q | G | 926 | A | SETD3 | E94D | C | 442 | A | SLC4A11 | T517M | G | 1652 | A | SRCAP | E95K | G | 668 | A |
| RPL23P8 | D30N | C | 189 | T | SETD3 | M152T | A | 615 | G | SLC4A11 | R374W | G | 1222 | A | SRCAP | R840Q | G | 2904 | A |
| RPL27 | N76S | A | 272 | G | SETD3 | D275G | T | 984 | C | SLC4A11 | I102V | T | 406 | C | SRCIN1 | T772M | G | 2540 | A |
| RPL31 | R44W | C | 153 | T | SETD3 | K8Q | T | 182 | G | SLC4A1AP | G161C | G | 763 | T | SRCIN1 | R120H | C | 584 | T |
| RPL34 | K71N | G | 257 | T | SETD4 | R90Q | C | 509 | T | SLC4A2 | G766S | G | 3336 | A | SRCIN1 | A236V | G | 932 | A |
| RPL35A | A48T | G | 409 | A | SETD5 | T606A | A | 1830 | G | SLC4A2 | T877M | C | 3670 | T | SRCIN1 | R1023H | C | 3293 | T |
| RPL35A | K54N | G | 429 | T | SETD5 | R465W | C | 1407 | T | SLC4A2 | M244V | A | 1770 | G | SRCRB4D | R109L | C | 674 | A |
| RPL36 | R85K | G | 323 | A | SETD5 | A366D | C | 1111 | A | SLC4A2 | S813I | G | 3478 | T | SRCRB4D | R467L | C | 1748 | A |
| RPL36AL | E92K | C | 392 | T | SETD5 | F1149C | T | 3460 | G | SLC4A2 | W992C | G | 4016 | T | SRCRB4D | G508S | C | 1870 | T |
| RPL37A | T78M | C | 919 | T | SETD5 | Q433H | A | 1313 | C | SLC4A3 | R1043W | C | 3341 | T | SRD5A2 | E174* | C | 520 | A |
| RPL3L | T346M | G | 1084 | A | SETD5 | E513* | G | 1551 | T | SLC4A3 | R1130C | C | 3602 | T | SRD5A3 | F76L | T | 456 | G |
| RPL4 | R337Q | C | 1103 | T | SETD5 | F796L | T | 2400 | C | SLC4A3 | E113D | G | 553 | T | SREBF1 | R39G | T | 284 | C |
| RPLP0 | Y24N | A | 307 | T | SETD6 | R31W | C | 141 | T | SLC4A3 | - | G | 0 | T | SREBF1 | G481S | C | 1610 | T |

| | | | | | | | | | | | | | | | | | | | |
|---|---|---|---|---|---|---|---|---|---|---|---|---|---|---|---|---|---|---|---|
| RPN1 | R263C | G | 836 | A | SETD7 | - | T | 0 | C | SLC4A3 | Y1143H | T | 3641 | C | SREBF1 | A249V | G | 915 | A |
| RPN2 | A160V | C | 790 | T | SETD7 | G70D | C | 846 | T | SLC4A4 | N220S | A | 776 | G | SREBF2 | R90Q | G | 435 | A |
| RPN2 | T24M | C | 382 | T | SETD7 | S3R | T | 644 | G | SLC4A4 | K404N | G | 1329 | T | SRFBP1 | R292Q | G | 947 | A |
| RPN2 | P511H | C | 1843 | A | SETD8 | V315I | G | 1369 | A | SLC4A4 | F487I | T | 1576 | A | SRGAP1 | N66Y | A | 252 | T |
| RPP38 | V117I | G | 708 | A | SETD8 | E73D | G | 645 | T | SLC4A4 | F713I | T | 2254 | A | SRGAP1 | T386A | A | 1212 | G |
| RPP40 | S53L | G | 203 | A | SETDB1 | V863M | G | 2704 | A | SLC4A4 | E56D | A | 285 | C | SRGAP1 | L285M | C | 909 | A |
| RPP40 | K326N | T | 1023 | G | SETDB1 | R923W | C | 2884 | T | SLC4A5 | D1099N | C | 3693 | T | SRGAP1 | T945M | C | 2890 | T |
| RPRD1A | E278* | C | 1004 | A | SETDB1 | G391C | G | 1288 | T | SLC4A5 | R1059* | G | 3573 | A | SRGAP1 | L845S | T | 2590 | C |
| RPRD1B | D65Y | G | 595 | T | SETDB1 | T492A | A | 1591 | G | SLC4A5 | R410Q | C | 1627 | T | SRGAP2 | R797H | G | 2390 | A |
| RPRD2 | E284* | G | 854 | T | SETX | A314T | C | 1123 | T | SLC4A7 | R1168W | G | 3723 | A | SRGAP2 | P575S | C | 1723 | T |
| RPRD2 | V1106I | G | 3320 | A | SETX | P2510T | G | 7711 | T | SLC4A7 | R804I | C | 2632 | A | SRGAP3 | K985N | C | 3057 | G |
| RPRD2 | A289S | G | 869 | T | SETX | K2031N | C | 6276 | A | SLC4A8 | A528T | G | 1760 | A | SRGAP3 | R866H | C | 2699 | T |
| RPRD2 | G1303V | G | 3912 | T | SETX | K596Q | T | 1969 | G | SLC4A8 | M1070I | G | 3388 | A | SRGAP3 | R211H | C | 734 | T |
| RPRD2 | F708V | T | 2126 | G | SEZ6 | R523C | G | 1761 | A | SLC4A8 | K411T | A | 1410 | C | SRGAP3 | Q191* | G | 673 | A |
| RPRM | V27M | C | 322 | T | SEZ6L | P645S | C | 2028 | T | SLC4A8 | I1064S | T | 3369 | G | SRGAP3 | T916M | G | 2849 | A |
| RPRM | V61A | A | 425 | G | SEZ6L | T786A | A | 2451 | G | SLC4A9 | A505V | C | 1549 | T | SRI | Y3C | T | 61 | C |
| RPRM | A42T | C | 367 | T | SEZ6L | Y370H | T | 1203 | C | SLC4A9 | S95F | C | 319 | T | SRI | A64V | G | 244 | A |
| RPRML | A46V | G | 378 | A | SEZ6L | E36D | G | 203 | T | SLC5A1 | A575V | C | 1974 | T | SRL | R836H | C | 2507 | T |
| RPS10 | - | C | 0 | T | SEZ6L | A98V | C | 388 | T | SLC5A1 | T519A | A | 1805 | G | SRMS | A353T | C | 1098 | T |
| RPS24 | R110H | G | 471 | A | SEZ6L2 | T477M | G | 1648 | A | SLC5A10 | G515R | G | 1584 | A | SRMS | S94L | G | 322 | A |
| RPS24 | K263T | A | 930 | C | SEZ6L2 | P372H | G | 1333 | T | SLC5A10 | T25I | C | 115 | T | SRMS | D378N | C | 1173 | T |
| RPS27L | L96P | A | 381 | G | SEZ6L2 | R640M | C | 2137 | A | SLC5A10 | T98M | C | 334 | T | SRP14 | A110T | C | 400 | T |
| RPS6 | R198H | C | 652 | T | SEZ6L2 | E513* | C | 1755 | A | SLC5A11 | R331H | G | 1614 | A | SRP68 | R335H | C | 1039 | T |
| RPS6KA1 | R734W | C | 2249 | T | SF1 | R260C | G | 855 | A | SLC5A11 | V658I | G | 2594 | A | SRP68 | R95Q | C | 319 | T |
| RPS6KA1 | R597H | G | 1839 | A | SF1 | R227H | C | 757 | T | SLC5A11 | R244Q | G | 1353 | A | SRP72 | L339S | T | 1737 | C |
| RPS6KA1 | R313W | C | 986 | T | SF1 | R380Q | C | 1216 | T | SLC5A11 | L106V | T | 938 | G | SRPK1 | K284T | T | 851 | G |
| RPS6KA2 | T247M | G | 1081 | A | SF3A2 | R26C | C | 198 | T | SLC5A11 | E234D | G | 1324 | T | SRPK1 | N234I | T | 701 | A |
| RPS6KA3 | A4V | G | 219 | A | SF3A2 | H429R | A | 1408 | G | SLC5A12 | I252T | A | 1065 | G | SRPK2 | K569T | T | 1706 | G |
| RPS6KA3 | A178E | G | 741 | T | SF3A3 | R356Q | C | 2023 | T | SLC5A12 | G195S | C | 893 | T | SRPK2 | G198D | C | 593 | T |
| RPS6KA4 | R511H | G | 1597 | A | SF3A3 | M19V | T | 1011 | C | SLC5A2 | V532M | G | 1613 | A | SRPX | P268S | G | 909 | A |
| RPS6KA4 | V328A | T | 1048 | C | SF3A3 | F130L | G | 1346 | T | SLC5A3 | D223N | G | 1179 | A | SRR | A123V | C | 436 | T |
| RPS6KA4 | Y44H | T | 195 | C | SF3B1 | R1041H | C | 3214 | T | SLC5A3 | R364W | C | 1602 | T | SRRD | R207W | C | 633 | T |
| RPS6KA5 | S263Y | G | 1003 | T | SF3B1 | R939H | C | 2908 | T | SLC5A4 | R414W | G | 1252 | A | SRRM1 | R568Q | G | 1731 | A |
| RPS6KA5 | Y683H | A | 2262 | G | SF3B1 | R568C | G | 1794 | A | SLC5A4 | V33G | A | 110 | C | SRRM1 | R661H | G | 2010 | A |
| RPS6KA5 | E754K | C | 2475 | T | SF3B2 | I43T | T | 168 | C | SLC5A5 | I432V | A | 1641 | G | SRRM1 | P261L | C | 810 | T |
| RPS6KA6 | - | C | 0 | A | SF3B2 | A774V | C | 2361 | T | SLC5A5 | R376Q | G | 1474 | A | SRRM1 | E258* | G | 800 | T |
| RPS6KA6 | T447A | T | 1347 | C | SF3B2 | A164T | G | 530 | A | SLC5A6 | L402I | G | 1678 | T | SRRM1 | R419I | G | 1284 | T |
| RPS6KA6 | L303I | G | 915 | T | SF3B2 | P713Q | C | 2178 | A | SLC5A6 | P564Q | G | 2165 | T | SRRM1 | K799T | A | 2424 | C |
| RPS6KB2 | G245C | G | 815 | T | SF3B3 | R690C | C | 2279 | T | SLC5A6 | R253W | G | 1231 | A | SRRM2 | R796C | C | 2935 | T |

| | | | | | | | | | | | | | | | | | | | |
|---|---|---|---|---|---|---|---|---|---|---|---|---|---|---|---|---|---|---|---|
| RPS6KB2 | R364W | C | 1172 | T | SF3B3 | R692W | C | 2285 | T | SLC5A6 | V545D | A | 2108 | T | SRRM2 | R814C | C | 2989 | T |
| RPS6KC1 | L115V | C | 493 | G | SF3B4 | R374C | G | 1705 | A | SLC5A7 | R38H | G | 402 | A | SRRM2 | R1932H | G | 6344 | A |
| RPS6KC1 | Q21P | A | 212 | C | SF3B4 | R186H | C | 1142 | T | SLC5A7 | L421M | C | 1550 | A | SRRM2 | R280Q | G | 1388 | A |
| RPS6KC1 | - | G | 0 | A | SF4 | A355T | C | 1065 | T | SLC5A9 | S332L | C | 1047 | T | SRRM2 | S1103Y | C | 3857 | A |
| RPS6KL1 | R58Q | C | 424 | T | SF4 | A275V | G | 826 | A | SLC5A9 | S90G | A | 320 | G | SRRM2 | R247W | C | 1288 | T |
| RPS6KL1 | Q335H | C | 1256 | A | SF4 | R310Q | C | 931 | T | SLC6A1 | R579H | G | 2157 | A | SRRM2 | R403H | G | 1757 | A |
| RPTN | K233N | T | 764 | G | SF4 | F83C | A | 250 | C | SLC6A1 | R195H | G | 1005 | A | SRRM2 | R1689C | C | 5614 | T |
| RPTN | A256S | C | 831 | A | SFI1 | R924H | G | 2876 | A | SLC6A1 | V342M | G | 1445 | A | SRRM2 | A2062D | C | 6734 | A |
| RPTOR | S285G | A | 1658 | G | SFI1 | A399T | G | 1300 | A | SLC6A1 | V342M | G | 1445 | A | SRRM2 | P2400L | C | 7748 | T |
| RPTOR | D1042N | G | 3929 | A | SFI1 | R670L | G | 2114 | T | SLC6A11 | T588M | C | 1829 | T | SRRM2 | R682C | C | 2593 | T |
| RPTOR | R1148W | C | 4247 | T | SFMBT1 | R646I | C | 2320 | A | SLC6A11 | R40C | C | 184 | T | SRRM2 | P1084S | C | 3799 | T |
| RPTOR | T1071I | C | 4017 | T | SFMBT1 | L281V | A | 1224 | C | SLC6A11 | E165* | G | 559 | T | SRRM2 | L1505P | T | 5063 | C |
| RPUSD1 | R296W | G | 1021 | A | SFMBT2 | A126T | C | 467 | T | SLC6A11 | D409G | A | 1292 | G | SRRM2 | R2007H | G | 6569 | A |
| RPUSD2 | R373* | C | 1155 | T | SFMBT2 | R617Q | C | 1941 | T | SLC6A12 | A95V | G | 565 | A | SRRM2 | S573Y | C | 2267 | A |
| RPUSD3 | V249M | C | 747 | T | SFMBT2 | R617W | G | 1940 | A | SLC6A13 | G7S | C | 72 | T | SRRM2 | R605W | C | 2362 | T |
| RPUSD4 | F31C | A | 146 | C | SFMBT2 | R772Q | C | 2406 | T | SLC6A13 | A316T | C | 999 | T | SRRM2 | R1945H | G | 6383 | A |
| RQCD1 | P50H | C | 524 | A | SFMBT2 | R219H | C | 747 | T | SLC6A14 | N552S | A | 1743 | G | SRRM2 | R2089Q | G | 6815 | A |
| RQCD1 | D269G | A | 1181 | G | SFMBT2 | R241* | G | 812 | A | SLC6A14 | S204G | A | 698 | G | SRRM4 | R611H | G | 2124 | A |
| RRAD | R249C | G | 897 | A | SFN | Y84C | A | 302 | G | SLC6A15 | A707T | C | 2612 | T | SRRM4 | R209Q | G | 918 | A |
| RRAD | E137D | C | 563 | A | SFN | Q244H | G | 783 | T | SLC6A15 | F512C | A | 2028 | C | SRRM4 | E425G | A | 1566 | G |
| RRAD | R189H | C | 718 | T | SFPQ | E489K | C | 1564 | T | SLC6A15 | I428T | A | 1776 | G | SRRM4 | Q10R | A | 321 | G |
| RRAGA | I89M | T | 553 | G | SFPQ | R571* | G | 1810 | A | SLC6A15 | D59N | C | 668 | T | SRRM4 | D165Y | G | 785 | T |
| RRAGB | R71H | G | 655 | A | SFRP1 | L243R | A | 1042 | C | SLC6A16 | Y210C | T | 863 | C | SRRM4 | R482* | C | 1736 | T |
| RRAGB | T229A | A | 1128 | G | SFRP1 | L25F | G | 387 | A | SLC6A16 | K88T | T | 497 | G | SRRM5 | V242M | G | 745 | A |
| RRAGC | K333N | T | 1176 | A | SFRP2 | Q277* | G | 1114 | A | SLC6A17 | W458* | G | 1852 | A | SRRM5 | P395L | C | 1205 | T |
| RRAGD | R378* | G | 1409 | A | SFRP2 | A255T | C | 1048 | T | SLC6A17 | V637I | G | 2387 | A | SRRM5 | R309I | G | 947 | T |
| RRAS2 | - | C | 0 | T | SFRP4 | G8W | C | 342 | A | SLC6A17 | A62T | G | 662 | A | SRRM5 | R389C | C | 1186 | T |
| RRBP1 | R1021W | G | 3365 | A | SFRP4 | R254Q | C | 1081 | T | SLC6A19 | S431Y | C | 1348 | A | SRRT | R41I | G | 280 | T |
| RRBP1 | K746T | T | 2541 | G | SFRS1 | F280C | A | 1048 | C | SLC6A19 | V536M | G | 1662 | A | SRRT | R690C | C | 2226 | T |
| RRBP1 | R1365C | G | 4397 | A | SFRS11 | R335C | C | 1085 | T | SLC6A19 | P265L | C | 850 | T | SRRT | Q842H | G | 2684 | T |
| RRBP1 | R700H | C | 2403 | T | SFRS11 | R275W | C | 905 | T | SLC6A19 | L443P | T | 1384 | C | SS18L2 | L25I | C | 281 | A |
| RRBP1 | E1284K | C | 4154 | T | SFRS11 | R254C | C | 842 | T | SLC6A19 | P579L | C | 1792 | T | SS18L2 | - | A | 442 | C |
| RRBP1 | R852W | G | 2858 | A | SFRS12 | A56T | G | 325 | A | SLC6A2 | L458M | C | 1423 | A | SSB | M142T | T | 599 | C |
| RRBP1 | R806W | G | 2720 | A | SFRS12 | V94A | T | 440 | C | SLC6A2 | A105V | C | 365 | T | SSB | Q243H | G | 903 | T |
| RRBP1 | E1178K | C | 3836 | T | SFRS12 | G4S | G | 169 | A | SLC6A2 | D298E | C | 945 | A | SSBP1 | S58A | T | 305 | G |
| RREB1 | S970Y | C | 3446 | A | SFRS12IP1 | A64T | C | 299 | T | SLC6A20 | V547M | C | 1755 | T | SSBP2 | R79C | G | 283 | A |
| RREB1 | R1472H | G | 4952 | A | SFRS12IP1 | D53N | C | 266 | T | SLC6A20 | D136N | C | 522 | T | SSBP3 | V31I | C | 502 | T |
| RREB1 | T1240I | C | 4256 | T | SFRS13B | R257H | C | 898 | T | SLC6A3 | G293S | C | 1003 | T | SSBP3 | A285T | C | 1264 | T |
| RREB1 | A1403T | G | 4744 | A | SFRS13B | L158* | A | 601 | C | SLC6A3 | D436N | C | 1432 | T | SSBP4 | P150L | C | 772 | T |

| | | | | | | | | | | | | | | | | | | | |
|---|---|---|---|---|---|---|---|---|---|---|---|---|---|---|---|---|---|---|---|
| RRH | R302W | C | 938 | T | SFRS13B | R122Q | C | 493 | T | SLC6A3 | V34A | A | 227 | G | SSFA2 | G578R | G | 1911 | A |
| RRH | T225I | C | 708 | T | SFRS14 | R982* | G | 3090 | A | SLC6A4 | R276C | G | 1005 | A | SSFA2 | R819Q | G | 2635 | A |
| RRM1 | Y717H | T | 2353 | C | SFRS14 | R680Q | C | 2185 | T | SLC6A4 | A547T | C | 1818 | T | SSFA2 | L1007S | T | 3199 | C |
| RRM2B | K80N | C | 484 | A | SFRS14 | S153Y | G | 604 | T | SLC6A4 | G67* | C | 378 | A | SSH1 | S147N | C | 519 | T |
| RRM2B | F323L | G | 1213 | T | SFRS15 | A247D | G | 740 | T | SLC6A5 | K342N | G | 1299 | T | SSH1 | S777L | G | 2409 | A |
| RRN3 | M318V | T | 1033 | C | SFRS15 | E1136K | C | 3406 | T | SLC6A5 | V421A | T | 1535 | C | SSH1 | D31N | C | 170 | T |
| RRP12 | K1124N | C | 3484 | A | SFRS15 | P1135T | G | 3403 | T | SLC6A5 | D322Y | G | 1237 | T | SSH1 | V397M | C | 1268 | T |
| RRP12 | P762L | G | 2397 | A | SFRS15 | - | C | 0 | T | SLC6A5 | E536D | G | 1881 | T | SSH2 | L1124V | A | 3522 | C |
| RRP12 | V178I | C | 644 | T | SFRS18 | G49* | C | 350 | A | SLC6A5 | D577A | A | 2003 | C | SSH2 | S1322Y | G | 4117 | T |
| RRP8 | R401Q | C | 1226 | T | SFRS18 | R418Q | C | 1458 | T | SLC6A7 | M605I | G | 2186 | T | SSH3 | R303C | C | 1003 | T |
| RRP9 | R81C | G | 315 | A | SFRS18 | - | C | 0 | A | SLC6A7 | Y497C | A | 1861 | G | SSPN | A62T | G | 284 | A |
| RS1 | E215* | C | 684 | A | SFRS2IP | P1277L | G | 4064 | A | SLC6A8 | G518D | G | 1553 | A | SSPN | E113G | A | 438 | G |
| RS1 | R191Q | C | 613 | T | SFRS2IP | R1244C | G | 3964 | A | SLC6A9 | N237D | T | 901 | C | SSPO | A1035V | C | 3104 | T |
| RSAD1 | A15T | G | 128 | A | SFRS2IP | N188I | T | 797 | A | SLC6A9 | A86V | G | 449 | A | SSPO | R3517H | G | 10550 | A |
| RSAD2 | E267K | G | 935 | A | SFRS2IP | P609H | G | 2060 | T | SLC6A9 | A6T | C | 182 | T | SSPO | P2368T | C | 7102 | A |
| RSAD2 | L252V | T | 890 | G | SFRS2IP | L94F | C | 516 | A | SLC7A1 | R16W | G | 329 | A | SSPO | T4404M | C | 13211 | T |
| RSBN1 | R660W | G | 2014 | A | SFRS2IP | K166E | T | 730 | C | SLC7A10 | V150A | A | 596 | G | SSPO | R4078H | G | 12233 | A |
| RSBN1L | A111T | G | 358 | A | SFRS3 | R75C | C | 339 | T | SLC7A10 | A301V | G | 1049 | A | SSPO | Q1002* | C | 3004 | T |
| RSC1A1 | N295S | A | 884 | G | SFRS3 | R141W | C | 537 | T | SLC7A11 | N293H | T | 1157 | G | SSPO | C4449Y | G | 13346 | A |
| RSF1 | D1042N | C | 3244 | T | SFRS4 | S316G | T | 1181 | C | SLC7A14 | A759V | G | 2592 | A | SSPO | L1643M | C | 4927 | A |
| RSF1 | P1340L | G | 4139 | A | SFRS5 | R95Q | G | 572 | A | SLC7A14 | G475V | C | 1740 | A | SSPO | H1981Y | C | 5941 | T |
| RSF1 | R1272* | G | 3934 | A | SFRS5 | R242H | G | 1013 | A | SLC7A14 | S302Y | G | 1221 | T | SSPO | R3381Q | G | 10142 | A |
| RSF1 | L596I | G | 1906 | T | SFRS6 | R111M | G | 438 | T | SLC7A2 | A382T | G | 1261 | A | SSPO | R142Q | G | 425 | A |
| RSL1D1 | - | A | 0 | C | SFRS7 | P193L | G | 816 | A | SLC7A2 | M246I | G | 855 | A | SSPO | T1024I | C | 3071 | T |
| RSPH3 | R403C | G | 1397 | A | SFRS7 | G5W | C | 251 | A | SLC7A2 | T177I | C | 647 | T | SSPO | H3584R | A | 10751 | G |
| RSPH3 | Y260C | T | 969 | C | SFRS7 | R142* | G | 662 | A | SLC7A2 | L404M | C | 1327 | A | SSPO | T4442I | C | 13325 | T |
| RSPH4A | Y657H | T | 2106 | C | SFRS8 | A546V | C | 1777 | T | SLC7A2 | S619G | A | 1972 | G | SSPO | R247W | C | 739 | T |
| RSPH4A | M364I | G | 1229 | A | SFRS8 | T410I | C | 1369 | T | SLC7A2 | T694A | A | 2197 | G | SSPO | V3719I | G | 11155 | A |
| RSPH4A | D528A | A | 1720 | C | SFT2D1 | G71S | C | 321 | T | SLC7A2 | P355L | C | 1181 | T | SSPO | R1708Q | G | 5123 | A |
| RSPH4A | L552F | C | 1791 | T | SFT2D3 | F108L | T | 828 | C | SLC7A3 | G612S | C | 1979 | T | SSPO | V2404M | G | 7210 | A |
| RSPH6A | Q336* | G | 1149 | A | SFTPA2 | P36H | G | 192 | T | SLC7A3 | E56D | C | 313 | A | SSPO | C4292Y | G | 12875 | A |
| RSPO1 | G209V | C | 1414 | A | SFTPB | A226V | G | 683 | A | SLC7A4 | G599V | C | 1864 | A | SSPO | R5118C | C | 15352 | T |

| | | | | | | | | | | | | | | | | | | | |
|---|---|---|---|---|---|---|---|---|---|---|---|---|---|---|---|---|---|---|---|
| RSPO1 | R70C | G | 996 | A | SFXN1 | M108V | A | 576 | G | SLC7A4 | A328V | G | 1051 | A | SSPO | V997M | G | 2989 | A |
| RSPO2 | D108Y | C | 980 | A | SFXN1 | K253N | G | 1013 | T | SLC7A6OS | D162Y | C | 503 | A | SSPO | R1784H | G | 5351 | A |
| RSPO3 | E66K | G | 351 | A | SFXN2 | E251V | A | 919 | T | SLC7A8 | R346* | G | 1762 | A | SSPO | F1149V | T | 3445 | G |
| RSRC1 | R130H | G | 547 | A | SFXN4 | F271L | A | 833 | C | SLC7A9 | T59M | G | 293 | A | SSPO | R4992C | C | 1497 4 | T |
| RSRC2 | S192R | G | 722 | C | SFXN5 | R152H | C | 586 | T | SLC7A9 | V346I | C | 1153 | T | SSPO | R1876C | C | 5626 | T |
| RSU1 | L128I | G | 407 | T | SFXN5 | A22T | C | 195 | T | SLC7A9 | A70V | G | 326 | A | SSPO | P1619S | C | 4855 | T |
| RTDR1 | M298V | T | 1089 | C | SFXN5 | M105T | A | 445 | G | SLC7A9 | I355V | T | 1180 | C | SSR3 | A191T | C | 631 | T |
| RTEL1 | D12Y | G | 861 | T | SGCA | G162R | G | 520 | A | SLC7A9 | I444V | T | 1447 | C | SSRP1 | R22* | G | 331 | A |
| RTEL1 | A1100T | G | 4125 | A | SGCA | R34H | G | 137 | A | SLC7A9 | S373L | G | 1235 | A | SSRP1 | S481P | A | 1708 | G |
| RTEL1 | W89R | T | 1092 | C | SGCB | S279T | C | 1059 | G | SLC8A1 | R255K | C | 925 | T | SSSCA1 | V80M | G | 258 | A |
| RTEL1 | H795Y | C | 3210 | T | SGCD | E5K | G | 532 | A | SLC8A1 | D760Y | C | 2439 | A | SST | E71D | T | 321 | G |
| RTF1 | R707* | C | 2131 | T | SGCD | L70P | T | 728 | C | SLC8A2 | E706D | C | 2396 | G | SSTR1 | A165V | C | 1111 | T |
| RTF1 | L196V | C | 598 | G | SGCE | T386M | G | 1268 | A | SLC8A3 | A759T | C | 3029 | T | SSTR2 | V330I | G | 1348 | A |
| RTKN | V12M | C | 117 | T | SGCG | R79H | G | 360 | A | SLC9A1 | T685M | G | 2630 | A | SSTR3 | V56M | C | 700 | T |
| RTKN | R542Q | C | 1708 | T | SGCG | I211V | A | 755 | G | SLC9A1 | P715S | G | 2719 | A | SSTR5 | A85T | G | 324 | A |
| RTN1 | R552L | C | 1991 | A | SGCG | F189L | T | 691 | G | SLC9A10 | E323D | T | 1222 | G | SSTR5 | A275V | C | 895 | T |
| RTN1 | G391R | C | 1507 | T | SGCZ | - | T | 0 | C | SLC9A10 | G114S | C | 593 | T | SSU72 | D50N | C | 460 | T |
| RTN2 | R87H | C | 488 | T | SGCZ | S267* | G | 1516 | T | SLC9A10 | E643K | C | 2180 | T | SSX1 | P109L | C | 462 | T |
| RTN2 | R135H | C | 632 | T | SGIP1 | S638P | T | 2050 | C | SLC9A11 | F317L | G | 1374 | T | SSX2IP | S290Y | G | 1133 | T |
| RTN3 | M964T | T | 3010 | C | SGIP1 | S304P | T | 1048 | C | SLC9A11 | L99S | A | 719 | G | SSX2IP | I397S | A | 1454 | C |
| RTN3 | L207S | T | 739 | C | SGIP1 | W776R | T | 2464 | A | SLC9A11 | E454D | C | 1785 | A | SSX7 | I18T | A | 186 | G |
| RTN4 | P145H | G | 678 | T | SGIP1 | P729S | C | 2323 | T | SLC9A2 | A31T | G | 233 | A | ST14 | G31V | G | 285 | T |
| RTN4 | T837A | T | 2753 | C | SGIP1 | S107Y | C | 458 | A | SLC9A3 | T374M | G | 1131 | A | ST14 | - | G | 0 | A |
| RTN4IP1 | A213T | C | 1103 | T | SGIP1 | S541L | C | 1760 | T | SLC9A3 | R24W | G | 80 | A | ST14 | H78Y | C | 425 | T |
| RTN4R | R510H | C | 1528 | T | SGIP1 | V140I | G | 556 | A | SLC9A3 | A86V | G | 267 | A | ST18 | A1041T | C | 3941 | T |
| RTN4R | A444V | G | 1330 | A | SGK1 | P58S | G | 770 | A | SLC9A3R2 | A298T | G | 898 | A | ST18 | E932* | C | 3614 | A |
| RTN4R | S162G | T | 483 | C | SGK2 | A119V | C | 575 | T | SLC9A3R2 | R254Q | G | 767 | A | ST18 | D811Y | C | 3251 | A |
| RTP1 | R10C | C | 58 | T | SGMS1 | Q381K | G | 2077 | T | SLC9A3R2 | R275W | C | 829 | T | ST20 | I64F | T | 870 | A |
| RTP2 | G193D | C | 1007 | T | SGOL1 | R476C | G | 1566 | A | SLC9A4 | A327V | C | 1437 | T | ST3GAL1 | T186A | T | 933 | C |
| RTP2 | R151C | G | 880 | A | SGOL2 | R718W | C | 2250 | T | SLC9A4 | A108V | C | 780 | T | ST3GAL1 | R135M | C | 781 | A |
| RTP3 | L70P | T | 781 | C | SGOL2 | R276C | C | 924 | T | SLC9A4 | I592T | T | 2232 | C | ST3GAL2 | E138K | C | 1555 | T |
| RTP3 | R121* | C | 933 | T | SGOL2 | I16S | T | 145 | G | SLC9A4 | S436A | T | 1763 | G | ST3GAL3 | P194S | C | 757 | T |
| RTTN | D1541Y | C | 4689 | A | SGPL1 | L453P | T | 1558 | C | SLC9A5 | R613H | G | 1903 | A | ST3GAL3 | R339Q | G | 1193 | A |
| RTTN | R1501W | G | 4569 | A | SGPL1 | K27N | G | 281 | T | SLC9A5 | V546I | G | 1701 | A | ST3GAL5 | L73F | G | 326 | A |
| RTTN | V1419M | C | 4323 | T | SGPL1 | E187* | G | 759 | T | SLC9A7 | T623M | G | 1894 | A | ST3GAL6 | E222* | G | 807 | T |
| RTTN | T1252M | G | 3823 | A | SGSH | V220A | A | 746 | G | SLC9A7 | A308V | G | 949 | A | ST3GAL6 | R280Q | G | 982 | A |
| RTTN | R772H | C | 2383 | T | SGSH | D484E | G | 1539 | C | SLC9A7 | E152K | C | 480 | T | ST3GAL6 | R148* | C | 585 | T |
| RTTN | R542Q | C | 1693 | T | SGSH | R160W | G | 565 | A | SLC9A8 | L300* | T | 1109 | G | ST3GAL6 | S191Y | C | 715 | A |
| RUFY1 | R368* | C | 1114 | T | SGSH | E447K | C | 1426 | T | SLC9A9 | R524W | G | 1779 | A | ST5 | A159T | C | 661 | T |

| | | | | | | | | | | | | | | | | | | | |
|---|---|---|---|---|---|---|---|---|---|---|---|---|---|---|---|---|---|---|---|
| RUFY1 | A209V | C | 638 | T | SGSM1 | M486V | A | 1463 | G | SLC9A9 | E132* | C | 603 | A | ST5 | R1095Q | C | 3470 | T |
| RUFY1 | E376D | G | 1140 | T | SGSM1 | R319H | G | 963 | A | SLC9A9 | F48Y | A | 352 | T | ST5 | V1038I | C | 3298 | T |
| RUFY2 | Q439K | G | 1642 | T | SGSM2 | M632V | A | 2071 | G | SLCO1A2 | C500Y | C | 2220 | T | ST5 | T986M | G | 3143 | A |
| RUFY2 | E540D | C | 1947 | A | SGSM2 | I207T | T | 797 | C | SLCO1A2 | P452H | G | 2076 | T | ST5 | A267T | C | 985 | T |
| RUFY4 | R64W | C | 600 | T | SGSM2 | A996T | G | 3163 | A | SLCO1B3 | R23C | C | 133 | T | ST6GAL1 | W181* | G | 1217 | A |
| RUFY4 | R515W | C | 1953 | T | SGSM3 | G388S | G | 1351 | A | SLCO1B3 | F410C | T | 1295 | G | ST6GAL2 | R342H | C | 1214 | T |
| RUNDC1 | V489A | T | 1478 | C | SGTB | R16W | G | 282 | A | SLCO1C1 | R197C | C | 957 | T | ST6GALNA C1 | L293P | A | 1052 | G |
| RUNDC2A | D138N | G | 474 | A | SH2B1 | E260D | G | 1052 | T | SLCO1C1 | E201* | G | 969 | T | ST6GALNA C1 | G394R | C | 1354 | T |
| RUNDC2A | V357A | T | 1132 | C | SH2B1 | R555C | C | 1935 | T | SLCO2A1 | F338L | G | 1288 | C | ST6GALNA C1 | F469L | G | 1581 | T |
| RUNX1 | P395L | G | 1629 | A | SH2B2 | T86M | C | 257 | T | SLCO2B1 | S166L | C | 884 | T | ST6GALNA C1 | K229N | C | 861 | A |
| RUNX1 | R232W | G | 1139 | A | SH2B2 | R226C | C | 676 | T | SLCO2B1 | K299E | A | 1282 | G | ST6GALNA C2 | R104H | C | 566 | T |
| RUNX1T1 | S590P | A | 1852 | G | SH2B3 | A84V | C | 608 | T | SLCO2B1 | R434W | C | 1687 | T | ST6GALNA C3 | R59Q | G | 324 | A |
| RUNX1T1 | A501T | C | 1585 | T | SH2D2A | E73K | C | 319 | T | SLCO3A1 | V149I | G | 659 | A | ST6GALNA C4 | L38P | A | 389 | G |
| RUNX1T1 | P593L | G | 1862 | A | SH2D2A | F21V | A | 163 | C | SLCO3A1 | F668L | C | 2218 | A | ST6GALNA C4 | R144* | G | 706 | A |
| RUNX1T1 | R604C | G | 1894 | A | SH2D3A | R541W | G | 1815 | A | SLCO4C1 | E704D | C | 2399 | A | ST6GALNA C6 | P319S | G | 1128 | A |
| RUNX1T1 | D246G | T | 821 | C | SH2D3A | E233K | C | 891 | T | SLCO4C1 | K691E | T | 2358 | C | ST7OT3 | R488H | G | 1483 | A |
| RUNX1T1 | - | C | 0 | A | SH2D3C | A274V | G | 935 | A | SLCO4C1 | I647V | T | 2226 | C | ST7OT3 | A239V | C | 930 | T |
| RUSC2 | R682H | G | 2183 | A | SH2D4A | E120* | G | 769 | T | SLCO4C1 | K337N | T | 1298 | A | ST7OT3 | K43N | A | 343 | C |
| RUSC2 | A950V | C | 2987 | T | SH2D4A | E210* | G | 1039 | T | SLCO5A1 | R694Q | C | 2697 | T | ST8SIA1 | P346S | G | 1518 | A |
| RUSC2 | P1292L | C | 4013 | T | SH2D4A | R285I | G | 1265 | T | SLCO5A1 | E296* | C | 1502 | A | ST8SIA2 | R251H | G | 989 | A |
| RUSC2 | D1283N | G | 3985 | A | SH2D4B | D298Y | G | 892 | T | SLCO5A1 | L360H | A | 1695 | T | ST8SIA2 | I96S | T | 524 | G |
| RUVBL2 | I247M | C | 754 | G | SH2D5 | P5L | G | 12 | A | SLCO6A1 | D647N | C | 2111 | T | ST8SIA2 | R274C | C | 1057 | T |
| RUVBL2 | A189V | C | 579 | T | SH2D5 | R76C | G | 224 | A | SLCO6A1 | R693C | G | 2249 | A | ST8SIA3 | F97Y | T | 647 | A |
| RUVBL2 | E454K | G | 1373 | A | SH2D5 | R256C | G | 764 | A | SLCO6A1 | S587Y | G | 1932 | T | ST8SIA4 | E24* | C | 381 | A |
| RWDD1 | Q185H | A | 634 | C | SH2D5 | V79I | C | 233 | T | SLCO6A1 | D362Y | C | 1256 | A | ST8SIA4 | K305N | C | 1226 | A |
| RWDD2A | R237H | G | 915 | A | SH2D5 | F83V | A | 245 | C | SLCO6A1 | G243* | C | 899 | A | ST8SIA4 | E205V | T | 925 | A |
| RWDD2B | T176A | T | 627 | C | SH3BGRL2 | L97P | T | 469 | C | SLCO6A1 | L611V | G | 2003 | C | ST8SIA4 | E155G | T | 775 | C |
| RXFP1 | K465T | A | 1594 | C | SH3BGRL3 | A117V | C | 350 | T | SLFN11 | G803D | C | 2681 | T | ST8SIA5 | R363* | G | 1748 | A |
| RXFP2 | M503I | G | 1580 | A | SH3BP1 | S318L | C | 1266 | T | SLFN11 | S80Y | G | 512 | T | ST8SIA5 | A185T | C | 1214 | T |
| RXFP2 | N306H | A | 987 | C | SH3BP1 | A524S | G | 1883 | T | SLFN11 | E744* | C | 2503 | A | ST8SIA5 | E269K | C | 1466 | T |
| RXFP2 | D475N | G | 1494 | A | SH3BP1 | Q11* | C | 344 | T | SLFN12 | E263K | C | 1311 | T | ST8SIA5 | F116V | A | 1007 | C |
| RXFP3 | A441T | G | 1678 | A | SH3BP2 | T239M | C | 716 | T | SLFN12 | R545K | C | 2158 | T | ST8SIA6 | E375* | C | 1198 | A |
| RXFP3 | A356V | C | 1424 | T | SH3BP2 | R326Q | G | 977 | A | SLFN12 | R540I | C | 2143 | A | STAB1 | G940R | G | 2894 | A |

| | | | | | | | | | | | | | | | | | | | |
|---|---|---|---|---|---|---|---|---|---|---|---|---|---|---|---|---|---|---|---|
| RXFP3 | V387I | G | 1516 | A | SH3BP4 | V341M | G | 1425 | A | SLFN12 | Y522D | A | 2088 | C | STAB1 | R1601H | G | 4878 | A |
| RXFP3 | V375M | G | 1480 | A | SH3BP5 | R402W | G | 1425 | A | SLFN12 | Q408R | T | 1747 | C | STAB1 | Q26K | C | 152 | A |
| RXFP3 | A300T | G | 1255 | A | SH3BP5L | G307R | C | 2149 | T | SLFN12 | K407N | C | 1745 | A | STAB1 | V901I | G | 2777 | A |
| RXFP3 | S177N | G | 887 | A | SH3BP5L | K112N | T | 1566 | G | SLFN13 | F866V | A | 2872 | C | STAB1 | A394V | C | 1257 | T |
| RXFP3 | A97V | C | 647 | T | SH3GL1 | R65Q | C | 325 | T | SLFN14 | R800I | C | 2435 | A | STAB1 | Q1116H | G | 3424 | T |
| RXFP3 | R81W | C | 598 | T | SH3GL1 | L143I | G | 558 | T | SLFN14 | G77E | C | 266 | T | STAB2 | A1352V | C | 4259 | T |
| RXFP3 | S183L | C | 905 | T | SH3GL2 | D291G | A | 992 | G | SLFN14 | K38T | T | 149 | G | STAB2 | A2343S | G | 7231 | T |
| RXFP4 | V293G | T | 898 | G | SH3GL3 | T97M | C | 782 | T | SLFN14 | T635I | G | 1940 | A | STAB2 | R2540W | C | 7822 | T |
| RXRA | L383F | C | 1199 | T | SH3GLB1 | T290A | A | 873 | G | SLFN5 | S509L | C | 1643 | T | STAB2 | I660T | T | 2183 | C |
| RXRA | A311T | G | 983 | A | SH3GLB2 | D151G | T | 577 | C | SLFN5 | A832V | C | 2612 | T | STAB2 | G86R | G | 460 | A |
| RXRA | A121T | G | 413 | A | SH3GLB2 | R273H | C | 943 | T | SLFN5 | R71C | C | 328 | T | STAB2 | T1333M | C | 4202 | T |
| RXRA | A416T | G | 1298 | A | SH3GLB2 | A119V | G | 481 | A | SLFN5 | I643T | T | 2045 | C | STAB2 | E2528D | A | 7788 | C |
| RXRG | R188C | G | 865 | A | SH3KBP1 | P550L | G | 1940 | A | SLFN5 | D843G | A | 2645 | G | STAB2 | K482N | G | 1650 | T |
| RXRG | R217L | C | 953 | A | SH3KBP1 | - | A | 2287 | G | SLFNL1 | Y246C | T | 955 | C | STAC | A141V | C | 722 | T |
| RXRG | R186H | C | 860 | T | SH3PXD2A | R780H | C | 2616 | T | SLFNL1 | V260M | C | 996 | T | STAC | T151A | A | 751 | G |
| RYBP | D209V | T | 624 | A | SH3PXD2A | S572N | C | 1992 | T | SLIT1 | R429L | C | 1532 | A | STAG1 | V485L | C | 1710 | A |
| RYR1 | E798* | G | 2522 | T | SH3PXD2A | R431C | G | 1568 | A | SLIT1 | D1210N | C | 3874 | T | STAG1 | Q170P | T | 766 | G |
| RYR1 | L822I | C | 2594 | A | SH3PXD2A | K298N | C | 1171 | A | SLIT1 | K1328N | C | 4230 | A | STAG1 | P730S | G | 2445 | A |
| RYR1 | F2973C | T | 9048 | G | SH3PXD2B | I41M | A | 294 | C | SLIT1 | R546Q | C | 1883 | T | STAG1 | D112V | T | 592 | A |
| RYR1 | S3374F | C | 10251 | T | SH3RF2 | S171N | G | 734 | A | SLIT1 | R1222C | G | 3910 | A | STAG1 | Q619R | T | 2113 | C |
| RYR1 | D3529N | G | 10715 | A | SH3RF3 | A298V | C | 893 | T | SLIT1 | Q829R | T | 2732 | C | STAG2 | R1045Q | G | 3644 | A |
| RYR1 | N609D | A | 1955 | G | SH3RF3 | D327G | A | 980 | G | SLIT1 | R178H | C | 779 | T | STAG2 | R954H | G | 3371 | A |
| RYR1 | R1024H | G | 3201 | A | SH3RF3 | S585W | C | 1754 | G | SLIT1 | R1091H | C | 3518 | T | STAG2 | A998V | C | 3503 | T |
| RYR1 | V1551I | G | 4781 | A | SH3TC1 | W177* | G | 598 | A | SLIT1 | R753W | G | 2503 | A | STAG2 | E276D | A | 1338 | C |
| RYR1 | T5005M | C | 15144 | T | SH3TC1 | R1192H | G | 3642 | A | SLIT1 | A451T | C | 1597 | T | STAG3 | E456G | A | 1774 | G |
| RYR1 | R644H | G | 2061 | A | SH3TC1 | C729G | T | 2252 | G | SLIT2 | R832* | C | 2720 | T | STAG3 | R460* | A | 1785 | T |
| RYR1 | A1988T | G | 6092 | A | SH3TC2 | K248N | C | 846 | A | SLIT2 | C869F | G | 2832 | T | STAG3 | P73L | C | 625 | T |
| RYR1 | P4517S | C | 13679 | T | SH3TC2 | R536W | G | 1708 | A | SLIT2 | R2C | C | 230 | T | STAG3 | T148I | C | 850 | T |
| RYR1 | A1962V | C | 6015 | T | SH3TC2 | G177D | C | 632 | T | SLIT2 | A459V | C | 1602 | T | STAG3 | A473T | G | 1824 | A |
| RYR1 | P728Q | C | 2313 | A | SH3TC2 | D285G | T | 956 | C | SLIT2 | R205* | C | 839 | T | STAG3 | E536D | A | 2015 | C |
| RYR1 | P1779S | C | 5465 | T | SH3TC2 | R1099H | C | 3398 | T | SLIT2 | K478N | G | 1660 | T | STAG3 | E956K | G | 3273 | A |
| RYR1 | A4504T | G | 13640 | A | SHANK1 | F1585L | A | 4772 | G | SLIT3 | R1177* | G | 3949 | A | STAM | S358A | T | 1287 | G |
| RYR1 | V2416A | T | 7377 | C | SHANK1 | P810S | G | 2447 | A | SLIT3 | V596M | C | 2206 | T | STAM2 | K163T | T | 838 | G |
| RYR1 | T2538M | C | 7743 | T | SHANK2 | G1286C | C | 3856 | A | SLIT3 | - | C | 0 | A | STAP1 | R216W | C | 728 | T |
| RYR1 | R109Q | G | 456 | A | SHANK2 | E459K | C | 1375 | T | SLIT3 | R1292C | G | 4294 | A | STAR | V125M | C | 637 | T |
| RYR1 | K1903R | A | 5838 | G | SHANK2 | A1095T | C | 3283 | T | SLIT3 | I73V | T | 637 | C | STARD10 | T64A | T | 1134 | C |

| | | | | | | | | | | | | | | | | | | | |
|---|---|---|---|---|---|---|---|---|---|---|---|---|---|---|---|---|---|---|---|
| RYR1 | D1056A | A | 3297 | C | SHANK2 | Y524C | T | 1571 | C | SLITRK1 | R52C | G | 1040 | A | STARD10 | P13H | G | 982 | T |
| RYR1 | E9K | G | 155 | A | SHANK2 | T1312A | T | 3934 | C | SLITRK1 | N481S | T | 2328 | C | STARD13 | P1058L | G | 3290 | A |
| RYR1 | R207H | G | 750 | A | SHANK2 | A802V | G | 2405 | A | SLITRK1 | G407D | C | 2106 | T | STARD13 | T52A | T | 271 | C |
| RYR1 | E2296D | A | 7018 | C | SHANK3 | R91* | C | 271 | T | SLITRK2 | R646H | G | 6192 | A | STARD13 | D1011V | T | 3149 | A |
| RYR2 | D1373E | T | 4443 | G | SHANK3 | V263M | G | 787 | A | SLITRK2 | E555* | G | 5918 | T | STARD3NL | E179* | G | 792 | T |
| RYR2 | A2268S | G | 7126 | T | SHANK3 | A1189V | C | 3566 | T | SLITRK2 | V662I | G | 6239 | A | STARD3NL | E228D | G | 941 | T |
| RYR2 | A3480V | C | 10763 | T | SHANK3 | I765F | A | 2293 | T | SLITRK3 | R249H | C | 1190 | T | STARD4 | E115* | C | 478 | A |
| RYR2 | K4013N | A | 12363 | T | SHANK3 | L626P | T | 1877 | C | SLITRK3 | S938L | G | 3257 | A | STARD7 | R300H | C | 1283 | T |
| RYR2 | M4941L | A | 15145 | T | SHANK3 | R1319Q | G | 3956 | A | SLITRK3 | L658I | G | 2416 | T | STARD7 | R232Q | C | 1079 | T |
| RYR2 | A3527P | G | 10903 | C | SHANK3 | R1379H | G | 4136 | A | SLITRK4 | V516I | C | 1639 | T | STARD7 | L105M | A | 697 | T |
| RYR2 | R1098W | C | 3616 | T | SHANK3 | T163M | C | 488 | T | SLITRK4 | K94T | T | 374 | G | STARD8 | A995T | G | 3197 | A |
| RYR2 | V4008M | G | 12346 | A | SHARPIN | R281C | G | 1325 | A | SLITRK5 | A654T | G | 2179 | A | STARD8 | P1026L | C | 3291 | T |
| RYR2 | T4884I | C | 14975 | T | SHB | P212L | G | 1201 | A | SLITRK5 | R335C | C | 1222 | T | STARD8 | R677C | C | 2243 | T |
| RYR2 | A4245V | C | 13058 | T | SHC2 | V454M | C | 1360 | T | SLITRK5 | A658V | C | 2192 | T | STARD8 | C571* | C | 1927 | A |
| RYR2 | R4571C | C | 14035 | T | SHC2 | V513I | C | 1537 | T | SLITRK5 | P613T | C | 2056 | A | STARD9 | A1381T | G | 4141 | A |
| RYR2 | T856M | C | 2891 | T | SHC2 | R580W | G | 1738 | A | SLITRK5 | L283V | C | 1066 | G | STARD9 | E69D | G | 207 | T |
| RYR2 | E3221K | G | 9985 | A | SHC2 | T238M | G | 713 | A | SLITRK5 | A38T | G | 331 | A | STARD9 | R268H | G | 803 | A |
| RYR2 | R2088Q | G | 6587 | A | SHC3 | M536I | C | 1915 | T | SLITRK5 | E475G | A | 1643 | G | STARD9 | R541K | G | 1622 | A |
| RYR2 | T2830A | A | 8812 | G | SHC3 | R177I | C | 837 | A | SLITRK5 | P631H | C | 2111 | A | STARD9 | S1102L | C | 3305 | T |
| RYR2 | T1347I | C | 4364 | T | SHC4 | I406M | T | 1647 | C | SLITRK5 | E781K | G | 2560 | A | STARD9 | A2821T | G | 8461 | A |
| RYR2 | S558I | G | 1997 | T | SHC4 | R239Q | C | 1145 | T | SLITRK5 | E947K | G | 3058 | A | STARD9 | V2650A | T | 7949 | C |
| RYR2 | T644I | C | 2255 | T | SHC4 | R214I | C | 1070 | A | SLITRK6 | F431L | G | 1892 | T | STARD9 | A2724S | G | 8170 | T |
| RYR2 | G3766D | G | 11621 | A | SHC4 | S162L | G | 914 | A | SLITRK6 | R599* | G | 2394 | A | STARD9 | R2313C | C | 6937 | T |
| RYR2 | R502H | G | 1829 | A | SHD | R166Q | G | 1960 | A | SLITRK6 | T411M | G | 1831 | A | STARD9 | Q3358* | C | 10072 | T |
| RYR2 | P4229L | C | 13010 | T | SHD | L136F | C | 1869 | T | SLITRK6 | T602M | G | 2404 | A | STARD9 | A1506V | C | 4517 | T |
| RYR2 | G348R | G | 1366 | A | SHH | R258W | G | 923 | A | SLITRK6 | L721I | G | 2760 | T | STARD9 | H218N | C | 652 | A |
| RYR2 | A1922T | G | 6088 | A | SHH | L76P | A | 378 | G | SLITRK6 | L565I | G | 2292 | T | STARD9 | N392H | A | 1174 | C |
| RYR2 | E1944K | G | 6154 | A | SHH | R232Q | C | 846 | T | SLITRK6 | F494C | A | 2080 | C | STARD9 | N529D | A | 1585 | G |
| RYR2 | F3886L | T | 11982 | G | SHH | A365T | C | 1244 | T | SLK | F798C | T | 2938 | G | STARD9 | E1105D | G | 3315 | T |
| RYR2 | L4505V | T | 13837 | G | SHISA2 | G51D | C | 237 | T | SLPI | K124E | T | 391 | C | STARD9 | E1165* | G | 3493 | T |

| | | | | | | | | | | | | | | | | | | | |
|---|---|---|---|---|---|---|---|---|---|---|---|---|---|---|---|---|---|---|---|
| RYR2 | V1561L | G | 5005 | T | SHISA3 | S213* | C | 856 | G | SLTM | F1034I | A | 3188 | T | STARD9 | K1709N | G | 5127 | T |
| RYR3 | R2025H | G | 6144 | A | SHISA3 | Q22R | A | 283 | G | SLTM | R635T | C | 1992 | G | STARD9 | E1745* | G | 5233 | T |
| RYR3 | D708N | G | 2192 | A | SHISA6 | R275* | C | 983 | T | SLU7 | E492V | T | 1863 | A | STARD9 | R2326Q | G | 6977 | A |
| RYR3 | E3901* | G | 11771 | T | SHISA6 | R451H | G | 1512 | A | SLU7 | R263W | G | 1175 | A | STARD9 | S2612Y | C | 7835 | A |
| RYR3 | E4026K | G | 12146 | A | SHISA6 | N245S | A | 894 | G | SMAD1 | R282C | C | 1267 | T | STARD9 | E2952D | G | 8856 | T |
| RYR3 | V3368M | G | 10172 | A | SHISA7 | T139M | G | 416 | A | SMAD2 | R120Q | C | 611 | T | STARD9 | S3103F | C | 9308 | T |
| RYR3 | V4072L | G | 12284 | C | SHISA7 | S434N | C | 1301 | T | SMAD2 | W422* | C | 1518 | T | STARD9 | P3785S | C | 11353 | T |
| RYR3 | R3982Q | G | 12015 | A | SHISA7 | R529C | G | 1585 | A | SMAD2 | P177S | G | 781 | A | STAT1 | K550N | T | 2038 | G |
| RYR3 | R402C | C | 1274 | T | SHISA7 | R74W | G | 220 | A | SMAD2 | T303I | G | 1160 | A | STAT1 | R274Q | C | 1209 | T |
| RYR3 | R1575C | C | 4793 | T | SHISA7 | A311V | G | 932 | A | SMAD2 | E142* | C | 676 | A | STAT1 | K298Q | T | 1280 | G |
| RYR3 | R3411W | C | 10301 | T | SHISA9 | G299D | G | 1240 | A | SMAD2 | L179* | A | 788 | C | STAT1 | F94V | A | 668 | C |
| RYR3 | R1518C | C | 4622 | T | SHISA9 | R155Q | G | 808 | A | SMAD2 | R321* | G | 1213 | A | STAT2 | V682F | C | 2122 | A |
| RYR3 | A2214T | G | 6710 | A | SHISA9 | D120N | G | 702 | A | SMAD3 | R90H | G | 579 | A | STAT2 | K55N | C | 243 | A |
| RYR3 | R2321C | C | 7031 | T | SHISA9 | D263G | A | 1132 | G | SMAD3 | R368Q | G | 1413 | A | STAT2 | Q42H | C | 204 | A |
| RYR3 | P1021S | C | 3131 | T | SHKBP1 | P131S | C | 440 | T | SMAD3 | N276I | A | 1137 | T | STAT3 | G342S | C | 1156 | T |
| RYR3 | A1579T | G | 4805 | A | SHKBP1 | D505N | G | 1562 | A | SMAD3 | R373H | G | 1428 | A | STAT3 | L413M | G | 1369 | T |
| RYR3 | R3624C | C | 10940 | T | SHMT1 | R137C | G | 544 | A | SMAD3 | R287W | C | 1169 | T | STAT3 | E74* | C | 352 | A |
| RYR3 | R101M | G | 372 | T | SHMT1 | R397Q | C | 1325 | T | SMAD4 | P246R | C | 1275 | G | STAT4 | F94S | A | 539 | G |
| RYR3 | I528T | T | 1653 | C | SHMT2 | R228Q | G | 683 | A | SMAD4 | R361H | G | 1620 | A | STAT4 | A670V | G | 2267 | A |
| RYR3 | R3350W | C | 10118 | T | SHMT2 | A280V | C | 839 | T | SMAD4 | R361C | C | 1619 | T | STAT4 | D206H | C | 874 | G |
| RYR3 | A3457T | G | 10439 | A | SHOC2 | K416N | G | 1593 | T | SMAD4 | D493A | A | 2016 | C | STAT4 | T369A | T | 1363 | C |
| RYR3 | E4289* | G | 12935 | T | SHOC2 | E100D | G | 645 | T | SMAD4 | S178* | C | 1071 | A | STAT5A | K384R | A | 1793 | G |
| RYR3 | N4447S | A | 13410 | G | SHOC2 | K459T | A | 1721 | C | SMAD4 | P356S | C | 1604 | T | STAT5A | R673H | G | 2660 | A |
| RYR3 | - | A | 14683 | C | SHOC2 | E512* | G | 1879 | T | SMAD4 | R361H | G | 1620 | A | STAT5A | P636S | C | 2548 | T |
| S100A12 | D50Y | C | 266 | A | SHOX2 | D190N | C | 704 | T | SMAD4 | G299R | G | 1433 | A | STAT5A | L455F | C | 2005 | T |
| S100A12 | E6D | C | 136 | A | SHOX2 | V125F | C | 509 | A | SMAD4 | F505Y | T | 2052 | A | STAT5B | V227M | C | 848 | T |
| S100A2 | L122F | G | 414 | A | SHPRH | L1084F | C | 3651 | A | SMAD4 | R361C | C | 1619 | T | STAT5B | I28T | A | 252 | G |
| S100A6 | L29P | A | 400 | G | SHPRH | A1618V | G | 5252 | A | SMAD4 | W509* | G | 2065 | A | STAT5B | R423Q | C | 1437 | T |
| S100A7L2 | G103* | C | 385 | A | SHPRH | V731A | A | 2591 | G | SMAD4 | R380K | G | 1677 | A | STAT6 | E339* | C | 1286 | A |
| S100A7L2 | D34Y | C | 178 | A | SHPRH | S1328* | G | 4382 | T | SMAD4 | D351G | A | 1590 | G | STAT6 | P657H | G | 2241 | T |
| S100A8 | A82T | C | 414 | T | SHPRH | K512N | C | 1935 | A | SMAD4 | R361C | C | 1619 | T | STAT6 | L364R | A | 1362 | C |

| | | | | | | | | | | | | | | | | | | | |
|---|---|---|---|---|---|---|---|---|---|---|---|---|---|---|---|---|---|---|---|
| S100B | F44L | G | 329 | T | SHROOM1 | R732Q | C | 3000 | T | SMAD4 | E108* | G | 860 | T | STAU1 | R115M | C | 344 | A |
| S100PBP | Y373C | A | 1395 | G | SHROOM2 | E1484K | G | 4540 | A | SMAD4 | R361C | C | 1619 | T | STAU1 | C523* | G | 1569 | T |
| S1PR1 | L163V | C | 739 | G | SHROOM2 | A191T | G | 661 | A | SMAD4 | G386V | G | 1695 | T | STAU1 | T396S | T | 1186 | A |
| S1PR1 | A253S | G | 1009 | T | SHROOM2 | A70T | G | 298 | A | SMAD4 | W524S | G | 2109 | C | STAU2 | K273* | T | 817 | A |
| S1PR2 | R296W | G | 997 | A | SHROOM2 | R751W | C | 2341 | T | SMAD5 | E26D | G | 363 | T | STAU2 | A264V | G | 791 | A |
| S1PR2 | P226L | G | 788 | A | SHROOM3 | F834L | C | 3455 | A | SMAD7 | A374V | G | 1408 | A | STAU2 | A178T | C | 532 | T |
| S1PR2 | V84M | C | 361 | T | SHROOM3 | E297K | G | 1842 | A | SMAD7 | N239K | A | 1004 | T | STAU2 | P143T | G | 427 | T |
| S1PR3 | A121V | C | 758 | T | SHROOM3 | R1857H | G | 6523 | A | SMAD9 | F115L | A | 483 | G | STC1 | R112Q | C | 619 | T |
| S1PR5 | L83M | G | 304 | T | SHROOM3 | A1478P | G | 5385 | C | SMAD9 | G77R | C | 369 | T | STC2 | G24R | C | 1380 | T |
| SAA1 | F21L | C | 139 | A | SHROOM4 | S1089L | G | 3550 | A | SMAD9 | R294Q | C | 1021 | T | STEAP1 | A416T | G | 1246 | A |
| SAA2 | E81D | C | 280 | A | SI | I1585L | T | 4816 | G | SMAP2 | R37* | C | 533 | T | STEAP2 | I394S | T | 1574 | G |
| SAC3D1 | R305H | G | 914 | A | SI | T1753P | T | 5320 | G | SMAP2 | - | G | 0 | A | STEAP4 | E454K | C | 1462 | T |
| SACM1L | A379V | C | 1340 | T | SI | R1230H | C | 3752 | T | SMAP2 | G353* | G | 1481 | T | STEAP4 | R171C | G | 613 | A |
| SACM1L | R145W | C | 637 | T | SI | - | C | 0 | A | SMARCA2 | R1349* | C | 4144 | T | STIL | P107H | G | 475 | T |
| SACS | Y2630H | A | 8162 | G | SI | K786T | T | 2420 | G | SMARCA2 | K434N | G | 1401 | T | STIL | A1191T | C | 3726 | T |
| SACS | E2280* | C | 7112 | A | SI | R692H | C | 2138 | T | SMARCA2 | A1188T | G | 3661 | A | STIL | E1243D | C | 3884 | A |
| SACS | E2067* | C | 6473 | A | SI | R248I | C | 806 | A | SMARCA2 | T1321M | C | 4061 | T | STIL | K819N | T | 2612 | G |
| SACS | E1095K | C | 3557 | T | SIAE | K400N | C | 1359 | A | SMARCA4 | D1127G | A | 3664 | G | STIL | D573N | C | 1872 | T |
| SACS | R1682M | C | 5319 | A | SIAH3 | R219W | G | 762 | A | SMARCA4 | R381* | C | 1425 | T | STIL | L247V | A | 894 | C |
| SACS | S4429P | A | 13559 | G | SIAH3 | H78R | T | 340 | C | SMARCA4 | R966W | C | 3180 | T | STIM1 | K384Q | A | 1719 | C |
| SACS | C3329Y | C | 10260 | T | SIDT1 | - | G | 0 | T | SMARCA4 | T910M | C | 3013 | T | STIM1 | K408T | A | 1792 | C |
| SACS | A4101T | C | 12575 | T | SIDT1 | E779K | G | 2986 | A | SMARCA4 | G1146S | G | 3720 | A | STIM2 | R667Q | G | 2269 | A |
| SACS | V631M | C | 2165 | T | SIDT1 | Y673N | T | 2668 | A | SMARCA4 | K1098R | A | 3577 | G | STIP1 | R134C | C | 400 | T |
| SACS | C2484Y | C | 7725 | T | SIDT1 | V637A | T | 2561 | C | SMARCA4 | R1323C | C | 4251 | T | STIP1 | G414C | G | 1240 | T |
| SACS | S3911I | C | 12006 | A | SIDT1 | W446R | T | 1987 | C | SMARCA4 | L456P | T | 1651 | C | STK10 | V118M | C | 652 | T |
| SACS | E4418* | C | 13526 | A | SIDT1 | E786K | G | 3007 | A | SMARCA4 | E371K | G | 1395 | A | STK10 | R817C | G | 2749 | A |
| SACS | R4412I | C | 13509 | A | SIDT2 | R105H | G | 835 | A | SMARCA4 | S1167L | C | 3784 | T | STK10 | D764Y | C | 2590 | A |
| SACS | D3926N | C | 12050 | T | SIDT2 | R461H | G | 1903 | A | SMARCA5 | I427V | A | 1741 | G | STK10 | E37D | C | 411 | A |
| SACS | D3561Y | C | 10955 | A | SIDT2 | R235H | G | 1225 | A | SMARCA5 | R974Q | G | 3383 | A | STK11 | P314H | C | 2056 | A |
| SACS | A2980T | C | 9212 | T | SIGIRR | A23V | G | 253 | A | SMARCA5 | R144C | C | 892 | T | STK11IP | V34A | T | 144 | C |
| SACS | R2906Q | C | 8991 | T | SIGLEC1 | R1555* | G | 4663 | A | SMARCA5 | R501Q | G | 1964 | A | STK11IP | P790S | C | 2411 | T |
| SACS | Q2605H | C | 8089 | A | SIGLEC1 | R578W | G | 1732 | A | SMARCAL1 | E775* | G | 2653 | T | STK11IP | R393C | C | 1220 | T |
| SACS | E2521* | C | 7835 | A | SIGLEC1 | A237T | C | 709 | T | SMARCAL | R334* | C | 1330 | T | STK11IP | S19Y | C | 99 | A |

| | | | | | | | | | | | | | | | | | | | |
|---|---|---|---|---|---|---|---|---|---|---|---|---|---|---|---|---|---|---|---|
| | | | | | | | | | | 1 | | | | | | | | | |
| SACS | E2384K | C | 7424 | T | SIGLEC1 | H410Y | G | 1228 | A | SMARCAL 1 | T541P | A | 1951 | C | STK17A | R299G | A | 1074 | G |
| SACS | R2093H | C | 6552 | T | SIGLEC1 | P1471T | G | 4411 | T | SMARCAL 1 | T541N | C | 1952 | A | STK17B | F259L | A | 1064 | C |
| SACS | F1495L | G | 4759 | T | SIGLEC1 | P539H | G | 1616 | T | SMARCAL 1 | T541I | C | 1952 | T | STK3 | S326C | C | 1193 | G |
| SACS | E1447D | T | 4615 | G | SIGLEC1 | V731M | C | 2191 | T | SMARCB1 | A321T | G | 1168 | A | STK3 | R165P | G | 710 | C |
| SACS | K1109* | T | 3599 | A | SIGLEC1 | E1035K | C | 3103 | T | SMARCB1 | R383W | C | 1354 | T | STK31 | K312N | A | 1055 | C |
| SACS | R961Q | C | 3156 | T | SIGLEC1 | V69M | C | 205 | T | SMARCB1 | Q252* | C | 961 | T | STK31 | Q753H | G | 2378 | T |
| SACS | K853N | T | 2833 | A | SIGLEC10 | R518G | T | 2168 | C | SMARCC1 | R652C | G | 2074 | A | STK31 | Q587R | A | 1879 | G |
| SACS | K514Q | T | 1814 | G | SIGLEC10 | R119W | G | 971 | A | SMARCC1 | H570Y | G | 1828 | A | STK31 | R728Q | G | 2302 | A |
| SAE1 | - | G | 0 | T | SIGLEC10 | - | C | 0 | A | SMARCC1 | K359E | T | 1195 | C | STK31 | N931H | A | 2910 | C |
| SAE1 | K152T | A | 499 | C | SIGLEC10 | E326D | C | 1594 | G | SMARCC1 | E840* | C | 2638 | A | STK31 | F972L | C | 3035 | A |
| SAFB | R629C | C | 1952 | T | SIGLEC10 | L383V | A | 1161 | C | SMARCC1 | Q808H | T | 2544 | G | STK32A | R145M | G | 605 | T |
| SAFB | S455F | C | 1431 | T | SIGLEC11 | A570V | G | 1800 | A | SMARCC1 | S697N | C | 2210 | T | STK32B | N400K | C | 1622 | G |
| SAFB2 | P945L | G | 3046 | A | SIGLEC14 | T542M | G | 1764 | A | SMARCC2 | P1050S | G | 3254 | A | STK32B | R27W | C | 501 | T |
| SAFB2 | R667C | G | 2211 | A | SIGLEC14 | D501V | T | 1641 | A | SMARCC2 | D110N | C | 434 | T | STK32B | K46N | G | 560 | T |
| SAFB2 | G99D | C | 508 | T | SIGLEC14 | L329I | G | 1124 | T | SMARCD1 | R274W | C | 990 | T | STK32B | D207N | G | 1041 | A |
| SAFB2 | T561A | T | 1893 | C | SIGLEC15 | M325T | T | 1123 | C | SMARCD1 | D218E | T | 824 | G | STK32B | L315R | T | 1366 | G |
| SAFB2 | A780T | C | 2550 | T | SIGLEC6 | R29Q | C | 294 | T | SMARCD1 | R183Q | G | 718 | A | STK32C | A120T | C | 465 | T |
| SAGE1 | S382G | A | 1218 | G | SIGLEC6 | T40M | G | 327 | A | SMARCD2 | M375T | A | 1441 | G | STK32C | P255L | G | 871 | A |
| SAGE1 | N95D | A | 357 | G | SIGLEC6 | S60L | G | 387 | A | SMARCD2 | R276Q | C | 1144 | T | STK33 | V312I | C | 1457 | T |
| SAGE1 | L316V | T | 1020 | G | SIGLEC7 | Q164H | G | 561 | T | SMARCD2 | R290W | G | 1185 | A | STK33 | G94D | C | 804 | T |
| SAGE1 | T375A | A | 1197 | G | SIGLEC8 | E18* | C | 119 | A | SMARCD2 | P154L | G | 778 | A | STK35 | I451L | A | 1622 | C |
| SALL1 | F436S | A | 1325 | G | SIGLEC9 | R120H | G | 426 | A | SMARCD2 | R233Q | C | 1015 | T | STK36 | C447F | G | 1619 | T |
| SALL1 | T935M | G | 2822 | A | SIGLEC9 | T223I | C | 735 | T | SMARCD2 | A378T | C | 1449 | T | STK36 | P278H | C | 1112 | A |
| SALL1 | C832* | A | 2514 | T | SIK1 | G582E | C | 1878 | T | SMARCD3 | Q262* | G | 1195 | A | STK36 | R968C | C | 3181 | T |
| SALL1 | L185P | A | 572 | G | SIK1 | R470Q | C | 1542 | T | SMARCD3 | E365D | C | 1506 | A | STK38 | N33D | T | 383 | C |
| SALL1 | G811S | C | 2449 | T | SIK1 | L68V | A | 335 | C | SMARCD3 | S203Y | G | 1019 | T | STK38 | F257V | A | 1055 | C |
| SALL1 | E1251K | C | 3769 | T | SIK1 | S66I | C | 330 | A | SMC1A | A1150V | G | 3577 | A | STK38L | F246L | T | 939 | G |
| SALL1 | R498C | G | 1510 | A | SIK2 | L510I | C | 1624 | A | SMC1A | R509H | C | 1654 | T | STK4 | G350* | G | 1143 | T |
| SALL2 | I66T | A | 479 | G | SIK2 | V777I | G | 2425 | A | SMC1B | L6V | G | 16 | C | STK4 | I473V | A | 1512 | G |
| SALL2 | R413C | G | 1519 | A | SIK2 | R672Q | G | 2111 | A | SMC1B | D332Y | C | 994 | A | STK4 | R304H | G | 1006 | A |
| SALL2 | M825I | C | 2757 | A | SIK3 | T1160A | T | 3476 | C | SMC1B | D555Y | C | 1663 | A | STK40 | A363V | G | 1262 | A |
| SALL3 | R1133H | G | 3398 | A | SIK3 | Q991H | C | 2971 | A | SMC1B | E63* | C | 187 | A | STMN2 | R30C | C | 158 | T |
| SALL3 | S600N | G | 1799 | A | SIK3 | L732I | G | 2192 | T | SMC2 | - | G | 0 | T | STMN4 | R205W | G | 757 | A |
| SALL3 | P376L | C | 1127 | T | SIKE1 | E99D | C | 375 | A | SMC2 | K930E | A | 3076 | G | STMN4 | K123N | C | 513 | A |
| SALL3 | S1124L | C | 3371 | T | SIKE1 | I75T | A | 302 | G | SMC2 | R1132C | C | 3682 | T | STOML1 | R148H | C | 568 | T |
| SALL4 | R890Q | C | 2736 | T | SIL1 | C301R | A | 1114 | G | SMC2 | A375D | C | 1412 | A | STOML1 | S250I | C | 874 | A |

| | | | | | | | | | | | | | | | | | | | |
|---|---|---|---|---|---|---|---|---|---|---|---|---|---|---|---|---|---|---|---|
| SALL4 | E1047* | C | 3206 | A | SIL1 | K144T | T | 644 | G | SMC2 | E841* | G | 2809 | T | STOML1 | R122Q | C | 490 | T |
| SALL4 | A297T | C | 956 | T | SIL1 | R118Q | C | 566 | T | SMC2 | E19K | G | 343 | A | STOML2 | G220R | C | 721 | T |
| SAMD3 | A428V | G | 1283 | A | SILV | L593I | G | 1865 | T | SMC2 | D956Y | G | 3154 | T | STOML2 | L276M | G | 889 | T |
| SAMD3 | R347Q | C | 1040 | T | SILV | M400V | T | 1286 | C | SMC2 | K1079N | A | 3525 | C | STOML3 | R149I | C | 791 | A |
| SAMD4A | P413L | C | 1239 | T | SIM1 | G700D | C | 2882 | T | SMC3 | E975K | G | 3049 | A | STON1-GTF2A1L | Y1171N | T | 3622 | A |
| SAMD4A | G210W | G | 629 | T | SIM1 | H691Y | G | 2854 | A | SMC3 | H55Y | C | 289 | T | STON1-GTF2A1L | I116L | A | 457 | C |
| SAMD4A | G232E | G | 696 | A | SIM2 | A221V | C | 1275 | T | SMC3 | I176L | A | 652 | C | STON1-GTF2A1L | E737D | A | 2322 | C |
| SAMD4A | R152H | G | 456 | A | SIM2 | R163* | C | 1100 | T | SMC4 | S1056L | C | 3618 | T | STON1-GTF2A1L | K205N | G | 726 | T |
| SAMD4B | R259Q | G | 1811 | A | SIM2 | L150M | C | 1061 | A | SMC4 | S1056L | C | 3618 | T | STON2 | T300M | G | 1100 | A |
| SAMD4B | R477W | C | 2464 | T | SIM2 | F93V | T | 890 | G | SMC5 | T841A | A | 2579 | G | STON2 | A669V | G | 2207 | A |
| SAMD4B | R536C | C | 2641 | T | SIN3A | K686N | C | 2373 | A | SMC5 | M1066R | T | 3255 | G | STON2 | Q389H | T | 1368 | G |
| SAMD7 | R67W | C | 588 | T | SIN3A | P1127L | G | 3695 | A | SMC5 | A747E | C | 2298 | A | STOX1 | R503Q | G | 1591 | A |
| SAMD7 | R441* | C | 1710 | T | SIN3A | R537* | G | 1924 | A | SMC5 | T904A | A | 2768 | G | STOX1 | E776A | A | 2410 | C |
| SAMD7 | L318I | C | 1341 | A | SIN3A | D822G | T | 2780 | C | SMC5 | R972* | C | 2972 | T | STOX2 | R759H | G | 3711 | A |
| SAMD8 | G330D | G | 1067 | A | SIN3A | - | T | 0 | C | SMC6 | G266R | C | 989 | T | STOX2 | T237A | A | 2144 | G |
| SAMD9 | S926* | G | 3047 | T | SIN3A | R1205H | C | 3929 | T | SMC6 | R413L | C | 1431 | A | STOX2 | S493L | C | 2913 | T |
| SAMD9 | V162L | C | 754 | G | SIN3A | E752* | C | 2569 | A | SMC6 | R914Q | C | 2934 | T | STRA6 | R272H | C | 815 | T |
| SAMD9 | L1526R | A | 4847 | C | SIN3A | K440N | C | 1635 | A | SMCHD1 | V101A | T | 332 | C | STRA8 | T241M | C | 722 | T |
| SAMD9 | I576S | A | 1997 | C | SIN3B | R856W | C | 2580 | T | SMCR7 | R149H | G | 472 | A | STRA8 | L246H | T | 737 | A |
| SAMD9 | L544I | G | 1900 | T | SIN3B | E916K | G | 2760 | A | SMCR7 | R19W | C | 81 | T | STRADB | R100H | G | 664 | A |
| SAMD9 | R285* | G | 1123 | A | SIN3B | R1083W | C | 3261 | T | SMCR7 | R263H | G | 814 | A | STRAP | E249* | G | 1046 | T |
| SAMD9L | R986C | G | 4173 | A | SIN3B | R960H | G | 2893 | A | SMCR8 | R715C | C | 2531 | T | STRBP | V658I | C | 2438 | T |
| SAMD9L | E961D | T | 4100 | G | SIN3B | R491C | C | 1485 | T | SMCR8 | K703N | A | 2497 | C | STRBP | G577V | C | 2196 | A |
| SAMHD1 | D207Y | C | 819 | A | SIN3B | S122P | T | 378 | C | SMEK1 | F607L | A | 2316 | G | STRBP | R12H | C | 501 | T |
| SAMHD1 | T232M | G | 895 | A | SIN3B | R1035C | C | 3117 | T | SMEK1 | R739W | G | 2712 | A | STRBP | R203Q | C | 1074 | T |
| SAMHD1 | K288T | T | 1063 | G | SIN3B | G384R | G | 1164 | A | SMEK1 | L363V | A | 1584 | C | STRBP | R375Q | C | 1590 | T |
| SAMHD1 | S247Y | G | 940 | T | SIN3B | D1128V | A | 3397 | T | SMEK2 | I566V | T | 1998 | C | STRBP | R5Q | C | 480 | T |
| SAMHD1 | F59C | A | 376 | C | SIN3B | Y848C | A | 2557 | G | SMEK2 | F604L | A | 2114 | C | STRN | N527S | T | 1589 | C |
| SAMM50 | G410S | G | 1228 | A | SIN3B | F72L | C | 230 | A | SMEK2 | S388Y | G | 1465 | T | STRN | A82V | G | 254 | A |
| SAMSN1 | R235C | G | 878 | A | SIN3B | F310L | C | 944 | A | SMG1 | E3129K | C | 9748 | T | STRN | A746T | C | 2245 | T |
| SAMSN1 | R235H | C | 879 | T | SIN3B | P764L | C | 2305 | T | SMG1 | V3359A | A | 10439 | G | STRN | - | A | 0 | C |
| SAMSN1 | K151E | T | 626 | C | SIPA1 | R565G | C | 1989 | G | SMG1 | S1866L | G | 5960 | A | STRN3 | S655T | C | 2180 | G |
| SAP130 | R489* | G | 1652 | A | SIPA1 | A521V | C | 1858 | T | SMG1 | K1877T | T | 5993 | G | STRN4 | A124V | G | 404 | A |
| SAP130 | A209V | G | 813 | A | SIPA1 | A206T | G | 912 | A | SMG5 | V674M | C | 2165 | T | STT3A | R538Q | G | 1723 | A |
| SAP130 | A209P | C | 812 | G | SIPA1 | A997V | C | 3286 | T | SMG5 | Q643* | G | 2072 | A | STT3B | E642D | A | 2135 | C |
| SAP130 | R530* | G | 1775 | A | SIPA1 | S346L | C | 1333 | T | SMG5 | R280Q | C | 984 | T | STUB1 | F98L | C | 544 | A |

| | | | | | | | | | | | | | | | | | | | |
|---|---|---|---|---|---|---|---|---|---|---|---|---|---|---|---|---|---|---|---|
| SAP130 | G554R | C | 1847 | G | SIPA1 | R1015Q | G | 3340 | A | SMG5 | A995V | G | 3129 | A | STUB1 | R154H | G | 711 | A |
| SAP30BP | K256T | A | 821 | C | SIPA1L1 | P766L | C | 2645 | T | SMG6 | R130H | C | 444 | T | STX11 | R126H | G | 560 | A |
| SAPS1 | A381T | C | 1141 | T | SIPA1L1 | S1639L | C | 5264 | T | SMG6 | R739Q | C | 2271 | T | STX19 | V220D | A | 916 | T |
| SAPS1 | S149P | A | 445 | G | SIPA1L1 | H1241Y | C | 4069 | T | SMG6 | P527H | G | 1635 | T | STX4 | L93P | T | 593 | C |
| SAPS1 | A661T | C | 1981 | T | SIPA1L1 | R519Q | G | 1904 | A | SMG7 | D353E | C | 1202 | A | STX5 | R91C | G | 425 | A |
| SAPS1 | E466D | C | 1398 | A | SIPA1L2 | L1278I | G | 4191 | T | SMG7 | R501H | G | 1645 | A | STX6 | R142C | G | 662 | A |
| SAPS2 | A838V | C | 2880 | T | SIPA1L2 | S1029L | G | 3445 | A | SMG7 | A1099V | C | 3439 | T | STXBP1 | R467C | C | 1538 | T |
| SAPS2 | P694S | C | 2447 | T | SIPA1L2 | S1149P | A | 3804 | G | SMG7 | E282D | G | 989 | T | STXBP2 | K204N | G | 638 | T |
| SAPS3 | R582Q | G | 1978 | A | SIPA1L2 | W857R | A | 2928 | G | SMG7 | S364N | G | 1234 | A | STXBP3 | M355V | A | 1113 | G |
| SAPS3 | R42C | C | 357 | T | SIPA1L2 | V710I | C | 2487 | T | SMNDC1 | R171I | C | 716 | A | STXBP3 | Y497H | T | 1539 | C |
| SAPS3 | A629T | G | 2118 | A | SIPA1L2 | R321H | C | 1321 | T | SMO | R763* | C | 2567 | T | STXBP4 | E435D | G | 1512 | T |
| SAPS3 | N363H | A | 1320 | C | SIPA1L2 | R296* | G | 1245 | A | SMO | A235V | C | 984 | T | STXBP5 | R683* | C | 2047 | T |
| SAPS3 | A813V | C | 2671 | T | SIPA1L3 | L890W | T | 3185 | G | SMO | A324T | G | 1250 | A | STXBP5 | A133V | C | 398 | T |
| SAR1A | L165P | A | 621 | G | SIPA1L3 | A380V | C | 1655 | T | SMO | D486E | C | 1738 | A | STXBP5 | G382V | G | 1145 | T |
| SAR1A | G37C | C | 236 | A | SIPA1L3 | S1559I | G | 5192 | T | SMOC1 | G80C | G | 491 | T | STXBP5L | C106Y | G | 588 | A |
| SARDH | S60R | G | 438 | C | SIPA1L3 | A1088T | G | 3778 | A | SMOC1 | A234V | C | 954 | T | STXBP5L | S215I | G | 915 | T |
| SARDH | A364T | C | 1348 | T | SIPA1L3 | E1708G | A | 5639 | G | SMOX | G566S | G | 1897 | A | STXBP5L | A77T | G | 500 | A |
| SARDH | A568V | G | 1961 | A | SIPA1L3 | D1570V | A | 5225 | T | SMOX | S333L | C | 1199 | T | STXBP5L | F165L | C | 766 | A |
| SARDH | A819T | C | 2713 | T | SIRPB1 | V197M | C | 654 | T | SMOX | L414I | C | 1441 | A | STXBP5L | F656C | T | 2238 | G |
| SARDH | S905L | G | 2972 | A | SIRPB2 | Q79* | G | 272 | A | SMOX | V539I | G | 1816 | A | STXBP5L | K932E | A | 3065 | G |
| SARM1 | A142V | C | 425 | T | SIRPB2 | A203V | G | 645 | A | SMPD1 | A346V | C | 1211 | T | STXBP6 | W80C | C | 575 | A |
| SARM1 | I693N | T | 2078 | A | SIRPD | R133W | G | 449 | A | SMPD1 | V114M | G | 514 | A | STYK1 | E240* | C | 1239 | A |
| SARNP | M103I | C | 364 | T | SIRPG | T194S | G | 646 | C | SMPD1 | V512M | G | 1708 | A | STYK1 | R377C | G | 1650 | A |
| SARS | R192Q | G | 586 | A | SIRT3 | W15C | C | 148 | A | SMPD1 | A48V | C | 317 | T | STYX | H165P | A | 556 | C |
| SART1 | E301D | G | 995 | T | SIRT3 | D385A | T | 1257 | G | SMPD2 | A327T | G | 1338 | A | STYX | K103T | A | 370 | C |
| SART1 | R299W | C | 987 | T | SIRT4 | R104Q | G | 331 | A | SMPD2 | I19T | T | 415 | C | STYXL1 | N14T | T | 214 | G |
| SART1 | I273L | A | 909 | C | SIRT4 | V232I | G | 714 | A | SMPD3 | C397Y | C | 1794 | T | SUB1 | S12Y | C | 320 | A |
| SART3 | D362Y | C | 1319 | A | SIRT6 | F82L | G | 306 | T | SMPD3 | S434F | G | 1905 | A | SUCLG1 | E241K | C | 932 | T |
| SART3 | V535I | C | 1838 | T | SIRT7 | W336* | C | 1041 | T | SMPD3 | A470T | C | 2012 | T | SUCLG1 | E109* | C | 536 | A |
| SART3 | A371T | C | 1346 | T | SIX1 | R29H | C | 359 | T | SMPD3 | G594V | C | 2385 | A | SUCLG2 | R392W | G | 1198 | A |
| SART3 | R252W | G | 989 | A | SIX2 | Q167* | G | 793 | A | SMPD4 | R796W | G | 2906 | A | SUCLG2 | R206W | G | 640 | A |
| SART3 | A508T | C | 1757 | T | SIX4 | V418M | C | 1312 | T | SMPD4 | H279R | T | 1356 | C | SUCLG2 | E79* | C | 259 | A |
| SART3 | R623H | C | 2103 | T | SIX4 | A237T | C | 769 | T | SMPDL3A | D399E | C | 1374 | G | SUDS3 | R126Q | G | 516 | A |
| SASH1 | S757Y | C | 2745 | A | SIX5 | A499V | G | 1706 | A | SMPDL3B | A5T | G | 204 | A | SUDS3 | K132N | G | 535 | T |
| SASH1 | R1139W | C | 3890 | T | SIX6 | G259C | G | 846 | T | SMR3B | P26H | C | 226 | A | SULF1 | R522W | C | 2119 | T |
| SASH1 | - | A | 0 | G | SIX6 | R162H | G | 556 | A | SMS | A80V | C | 491 | T | SULF1 | R38* | C | 667 | T |
| SASH1 | R558C | C | 2147 | T | SIX6 | A144T | G | 501 | A | SMTN | R98C | C | 292 | T | SULF1 | G820D | G | 3014 | A |
| SASH1 | V77M | G | 704 | A | SIX6 | R265W | C | 864 | T | SMU1 | D185N | C | 604 | T | SULF1 | F234L | T | 1257 | G |
| SASH1 | R723Q | G | 2643 | A | SKA2 | R63H | C | 464 | T | SMURF1 | - | A | 0 | G | SULF2 | W868* | C | 3456 | T |

| | | | | | | | | | | | | | | | | | | | |
|---|---|---|---|---|---|---|---|---|---|---|---|---|---|---|---|---|---|---|---|
| SULF2 | A557T | C | 2521 | T | THBS4 | R588C | C | 2085 | T | TRBV5-3 | R73G | T | 217 | C | UGT1A7 | L215S | T | 644 | C |
| SULF2 | V83M | C | 1099 | T | THEG | D26N | C | 115 | T | TRBV6-6 | V79I | C | 279 | T | UGT1A8 | A423T | G | 1330 | A |
| SULF2 | A139T | C | 1267 | T | THEM4 | F208L | A | 974 | T | TRBV6-8 | K28* | T | 82 | A | UGT1A8 | V190I | G | 631 | A |
| SULT1A2 | V270M | C | 925 | T | THEM5 | A167T | C | 498 | T | TRBV7-3 | W53* | C | 209 | T | UGT2A2 | E81* | C | 297 | A |
| SULT1B1 | I57M | A | 469 | C | THEM5 | S26Y | G | 76 | T | TRBV7-3 | F84L | G | 302 | T | UGT2A2 | V91I | C | 327 | T |
| SULT1C2 | P251S | C | 928 | T | THEMIS | S381P | A | 1460 | G | TRBV7-4 | P89S | G | 265 | A | UGT2A2 | K682T | T | 2101 | G |
| SULT1C2 | R118W | C | 529 | T | THEMIS | R238* | G | 1031 | A | TRBV7-8 | V31F | C | 130 | A | UGT2A2 | R611I | C | 1888 | A |
| SULT1C2 | P165S | C | 670 | T | THEMIS | E7* | C | 338 | A | TRBV9 | L65P | A | 241 | G | UGT2A2 | D41Y | C | 177 | A |
| SULT1C4 | K289N | G | 1193 | T | THNSL1 | K555E | A | 1913 | G | TRBV9 | E72* | C | 261 | A | UGT2A3 | N211H | T | 662 | G |
| SULT2A1 | N226D | T | 816 | C | THNSL1 | T180A | A | 788 | G | TRDMT1 | E185K | C | 601 | T | UGT2B11 | L445* | A | 1336 | C |
| SULT2A1 | S20F | G | 199 | A | THNSL1 | E312* | G | 1184 | T | TRDMT1 | E63K | C | 235 | T | UGT2B15 | E441D | C | 1333 | A |
| SULT2B1 | R341H | G | 1103 | A | THNSL1 | E683* | G | 2297 | T | TRDN | R713H | C | 2138 | T | UGT2B28 | L9I | C | 34 | A |
| SULT2B1 | Q56H | A | 249 | C | THNSL2 | R473Q | G | 1571 | A | TRDN | E483D | C | 1449 | A | UGT2B28 | R259Q | G | 785 | A |
| SULT4A1 | R32C | G | 215 | A | THOC1 | E386K | C | 1196 | T | TREH | R383C | G | 1192 | A | UGT2B4 | G24* | C | 117 | A |
| SULT4A1 | R32C | G | 215 | A | THOC1 | S38R | G | 154 | T | TREH | - | T | 0 | C | UGT2B7 | E225K | G | 719 | A |
| SUMF2 | V200I | G | 629 | A | THOC2 | R1409H | C | 4258 | T | TREM1 | P202L | G | 669 | A | UGT2B7 | K524N | G | 1618 | T |
| SUMF2 | A192T | G | 605 | A | THOC2 | E1157K | C | 3501 | T | TREML1 | C297R | A | 899 | G | UGT2B7 | T351A | A | 1097 | G |
| SUMF2 | K101N | G | 334 | T | THOC2 | P290L | G | 901 | A | TRERF1 | V1166D | A | 4060 | T | UGT3A1 | E71D | T | 571 | G |
| SUMO3 | P38L | G | 113 | A | THOC4 | H96D | G | 292 | C | TRERF1 | T746M | G | 2800 | A | UGT3A2 | E88* | C | 364 | A |
| SUN2 | S468L | G | 1585 | A | THOC5 | E4* | C | 133 | A | TREX2 | R199H | C | 1738 | T | UGT8 | E102K | G | 826 | A |
| SUN2 | P291S | G | 1053 | A | THOC6 | A259V | C | 1072 | T | TREX2 | P7H | G | 1162 | T | UGT8 | E32G | A | 617 | G |
| SUN3 | Q96* | G | 446 | A | THOC6 | R22W | C | 360 | T | TREX2 | S12R | A | 1178 | C | UGT8 | K22N | A | 588 | C |
| SUN5 | S203Y | G | 701 | T | THPO | T281I | G | 1057 | A | TREX2 | R111H | C | 1474 | T | UGT8 | R443Q | G | 1850 | A |
| SUN5 | R54L | C | 254 | A | THPO | T315I | G | 1159 | A | TRGC1 | K84N | T | 251 | G | UHMK1 | G368D | G | 1261 | A |
| SUPT16H | E1007K | C | 3358 | T | THPO | V166A | A | 712 | G | TRGV4 | L66Q | A | 531 | T | UHMK1 | Q248R | A | 901 | G |
| SUPT16H | A29V | G | 425 | A | THRA | V407A | T | 1800 | C | TRGV5 | R64H | C | 389 | T | UHRF1 | M21T | T | 355 | C |
| SUPT16H | A482V | G | 1784 | A | THRA | R104H | G | 891 | A | TRGV8 | K97Q | T | 403 | G | UHRF1 | D341E | C | 1316 | G |
| SUPT3H | - | C | 0 | T | THRAP3 | R156H | G | 691 | A | TRH | R61W | C | 708 | T | UHRF1BP1 | R621H | G | 2033 | A |
| SUPT3H | E132K | C | 712 | T | THRAP3 | A550V | C | 1873 | T | TRHDE | - | G | 0 | T | UHRF1BP1 | D332N | G | 1165 | A |
| SUPT4H1 | M1V | T | 68 | C | THRAP3 | N113K | T | 563 | G | TRHDE | P147L | C | 536 | T | UHRF2 | E241* | G | 889 | T |
| SUPT5H | R989W | C | 3144 | T | THRB | T138M | G | 654 | A | TRHDE | R250H | G | 845 | A | UHRF2 | Y372C | A | 1283 | G |
| SUPT5H | R813H | G | 2617 | A | THRB | R258W | G | 1013 | A | TRHDE | S126L | C | 473 | T | UHRF2 | R759H | G | 2444 | A |
| SUPT6H | D856N | G | 2656 | A | THRSP | N11I | A | 53 | T | TRHDE | S823C | A | 2563 | T | ULBP1 | F181L | C | 586 | A |
| SUPT6H | R715W | C | 2233 | T | THSD1 | I302V | T | 1083 | C | TRHDE | G1019V | G | 3152 | T | ULK1 | G208S | G | 890 | A |
| SUPT6H | R607G | A | 1909 | G | THSD1 | A52T | C | 333 | T | TRHR | W91C | G | 291 | T | ULK1 | - | T | 0 | C |
| SUPT6H | D1077N | G | 3319 | A | THSD1 | G493D | C | 1657 | T | TRHR | R17* | C | 67 | T | ULK1 | A428T | G | 1550 | A |
| SUPT6H | R818Q | G | 2543 | A | THSD1 | T430I | G | 1468 | A | TRIB2 | D211N | G | 2050 | A | ULK2 | R120Q | C | 878 | T |
| SUPV3L1 | R365W | C | 1153 | T | THSD1 | R753I | C | 2437 | A | TRIB2 | R286* | C | 2275 | T | ULK2 | R465Q | C | 1913 | T |
| SUPV3L1 | R118C | C | 412 | T | THSD1 | L496M | G | 1665 | T | TRIB2 | R290H | G | 2288 | A | ULK2 | S347Y | G | 1559 | T |

| | | | | | | | | | | | | | | | | | | | |
|---|---|---|---|---|---|---|---|---|---|---|---|---|---|---|---|---|---|---|---|
| SURF1 | - | C | 0 | A | THSD1 | E249* | C | 924 | A | TRIB3 | R56H | G | 167 | A | ULK3 | E290V | T | 869 | A |
| SURF1 | R84Q | C | 283 | T | THSD1 | R338K | C | 1192 | T | TRIB3 | R29* | C | 85 | T | ULK4 | V690A | A | 2532 | G |
| SURF4 | A143V | G | 558 | A | THSD1 | E689K | C | 2244 | T | TRIB3 | V188M | G | 562 | A | ULK4 | A984T | C | 3413 | T |
| SURF4 | L111V | A | 461 | C | THSD4 | T872M | C | 2749 | T | TRIM11 | L200P | A | 878 | G | UMOD | G487S | C | 1583 | T |
| SUSD1 | S279Y | G | 877 | T | THSD4 | Q30R | A | 223 | G | TRIM14 | L223S | A | 690 | G | UMOD | A471T | C | 1535 | T |
| SUSD2 | H114R | A | 602 | G | THSD4 | F326L | C | 1112 | A | TRIM14 | G392D | C | 1197 | T | UMOD | A103T | C | 431 | T |
| SUSD2 | R282L | G | 1106 | T | THSD7A | R277H | C | 1082 | T | TRIM16L | F80V | T | 1717 | G | UMOD | C65R | A | 317 | G |
| SUSD2 | R326H | G | 1238 | A | THSD7A | R544C | G | 1882 | A | TRIM2 | A139V | C | 481 | T | UMODL1 | R1294W | C | 3880 | T |
| SUSD5 | E230D | C | 1108 | A | THSD7A | S1526L | G | 4829 | A | TRIM2 | C157Y | G | 535 | A | UMODL1 | P107S | C | 319 | T |
| SUV39H1 | D391N | G | 1171 | A | THSD7A | R1311I | C | 4184 | A | TRIM2 | R647M | G | 2005 | T | UMODL1 | H1199R | A | 3596 | G |
| SUV39H1 | I230M | C | 690 | G | THSD7A | R448H | C | 1595 | T | TRIM2 | R490Q | G | 1534 | A | UMODL1 | A1007T | G | 3019 | A |
| SUV39H1 | I230M | C | 690 | G | THSD7A | L1464I | A | 4642 | T | TRIM22 | W262C | G | 1063 | T | UMODL1 | E1268K | G | 3802 | A |
| SUV39H2 | Q91R | A | 272 | G | THSD7B | S668L | C | 2003 | T | TRIM22 | S312L | C | 1212 | T | UNC119B | R150C | C | 895 | T |
| SUV39H2 | R353Q | G | 1058 | A | THSD7B | P1288S | C | 3862 | T | TRIM22 | V445G | T | 1611 | G | UNC13A | - | C | 0 | T |
| SUV39H2 | L209I | C | 625 | A | THSD7B | R1454H | G | 4361 | A | TRIM22 | W262L | G | 1062 | T | UNC13A | V1707M | C | 5119 | T |
| SUV420H1 | G14C | C | 394 | A | THSD7B | T693M | C | 2078 | T | TRIM23 | E525* | C | 1945 | A | UNC13A | W647L | C | 1940 | A |
| SUV420H1 | - | C | 0 | A | THSD7B | - | A | 0 | G | TRIM23 | K344N | C | 1404 | A | UNC13A | R285H | C | 854 | T |
| SUV420H1 | K776E | T | 2680 | C | THSD7B | R574H | G | 1721 | A | TRIM23 | E525* | C | 1945 | A | UNC13A | G571S | C | 1711 | T |
| SUV420H1 | K612N | C | 2190 | A | THSD7B | R1426H | G | 4277 | A | TRIM24 | V208A | T | 838 | C | UNC13A | R285C | G | 853 | A |
| SUV420H1 | R187Q | C | 914 | T | THSD7B | R1287W | C | 3859 | T | TRIM25 | N225I | T | 734 | A | UNC13A | F337L | G | 1011 | T |
| SUV420H1 | F119V | A | 709 | C | THSD7B | T427M | C | 1280 | T | TRIM25 | A98T | C | 352 | T | UNC13B | R647H | G | 2222 | A |
| SUV420H2 | T69M | C | 454 | T | THSD7B | R151Q | G | 452 | A | TRIM3 | L212P | A | 866 | G | UNC13B | S276P | T | 1108 | C |
| SUV420H2 | R95H | G | 532 | A | THSD7B | R264I | G | 791 | T | TRIM3 | A160V | G | 710 | A | UNC13B | H1584R | A | 5033 | G |
| SUV420H2 | L398M | C | 1440 | A | THSD7B | L732I | C | 2194 | A | TRIM32 | E191A | A | 730 | C | UNC13C | R171H | G | 512 | A |
| SUV420H2 | P411T | C | 1479 | A | THSD7B | I1559M | T | 4677 | G | TRIM32 | C509Y | G | 1684 | A | UNC13C | L1071I | C | 3211 | A |
| SUZ12P | R256H | G | 977 | A | THUMPD3 | L30I | C | 427 | A | TRIM32 | R596H | G | 1945 | A | UNC13C | K1335R | A | 4004 | G |
| SUZ12P | R360C | C | 1288 | T | THUMPD3 | V481F | G | 1780 | T | TRIM32 | T266I | C | 955 | T | UNC13C | R2082C | C | 6244 | T |
| SUZ12P | N642K | T | 2136 | G | THY1 | R39H | C | 172 | T | TRIM32 | A579P | G | 1893 | C | UNC13C | D685A | A | 2054 | C |
| SV2A | - | C | 0 | A | THYN1 | D148A | T | 927 | G | TRIM32 | R540W | C | 1776 | T | UNC13C | E1387* | G | 4159 | T |
| SV2A | L577M | G | 2220 | T | THYN1 | R94C | G | 764 | A | TRIM33 | R440C | G | 1402 | A | UNC13C | D1290Y | G | 3868 | T |
| SV2A | R483H | C | 1939 | T | TIA1 | N315T | T | 1155 | G | TRIM33 | R865* | G | 2677 | A | UNC13C | P1571Q | C | 4712 | A |
| SV2C | A205V | C | 1056 | T | TIAF1 | R25W | G | 73 | A | TRIM35 | V200L | C | 680 | G | UNC13C | G20V | G | 59 | T |
| SV2C | D398G | A | 1635 | G | TIAM1 | R1527W | G | 5051 | A | TRIM35 | R427H | C | 1362 | T | UNC13C | E249K | G | 745 | A |
| SV2C | F230C | T | 1131 | G | TIAM1 | R1540Q | C | 5091 | T | TRIM35 | A469T | C | 1487 | T | UNC13C | Q386H | A | 1158 | C |
| SVEP1 | G3397S | C | 1052 6 | T | TIAM1 | R380H | C | 1611 | T | TRIM35 | L284M | G | 932 | T | UNC13C | R572I | G | 1715 | T |
| SVEP1 | A2556T | C | 8003 | T | TIAM1 | R871* | G | 3083 | A | TRIM36 | C171R | A | 633 | G | UNC13C | E936D | A | 2808 | C |
| SVEP1 | R1213C | G | 3974 | A | TIAM1 | P359S | G | 1547 | A | TRIM37 | A895V | G | 3128 | A | UNC13C | K1134R | A | 3401 | G |
| SVEP1 | S1598A | A | 5129 | C | TIAM1 | L1115I | G | 3815 | T | TRIM37 | R446Q | C | 1781 | T | UNC13C | T1601N | C | 4802 | A |
| SVEP1 | T3108A | T | 9659 | C | TIAM1 | R788W | G | 2834 | A | TRIM37 | E568K | C | 2146 | T | UNC13C | I1931M | T | 5793 | G |

| | | | | | | | | | | | | | | | | | | | |
|---|---|---|---|---|---|---|---|---|---|---|---|---|---|---|---|---|---|---|---|
| SVEP1 | C1035Y | C | 3441 | T | TIAM1 | K1222N | C | 4138 | A | TRIM37 | - | C | 0 | A | UNC13D | K661E | T | 2361 | C |
| SVEP1 | - | C | 0 | A | TIAM1 | A1004T | C | 3482 | T | TRIM38 | K158E | A | 896 | G | UNC13D | L435P | A | 1684 | G |
| SVEP1 | I3470M | A | 1074 7 | C | TIAM1 | K724N | C | 2644 | A | TRIM38 | E82K | G | 668 | A | UNC13D | E39D | C | 497 | A |
| SVEP1 | F2512L | G | 7873 | T | TIAM1 | K443N | C | 1801 | A | TRIM38 | L198I | C | 1016 | A | UNC13D | Q878* | G | 3012 | A |
| SVEP1 | R3032Q | C | 9432 | T | TIAM2 | G49R | G | 353 | A | TRIM38 | L147P | T | 864 | C | UNC45A | A428T | G | 1322 | A |
| SVIL | R1178* | G | 3982 | A | TIAM2 | G1075D | G | 3432 | A | TRIM38 | K389N | A | 1591 | C | UNC45B | L117P | T | 797 | C |
| SVIL | A1494V | G | 4931 | A | TIAM2 | P99T | C | 503 | A | TRIM4 | Y381D | A | 1271 | C | UNC45B | K174M | A | 968 | T |
| SVIL | L1428P | A | 4733 | G | TIAM2 | S92C | C | 483 | G | TRIM41 | R547Q | G | 2350 | A | UNC45B | R225Q | G | 1121 | A |
| SVIL | T2192M | G | 7025 | A | TIAM2 | R62Q | G | 393 | A | TRIM41 | P177H | C | 1240 | A | UNC5B | R143Q | G | 844 | A |
| SVIL | S1314G | T | 4390 | C | TIAM2 | N457S | A | 1578 | G | TRIM41 | A429V | C | 1996 | T | UNC5B | S544R | C | 2048 | A |
| SVIL | S425* | G | 1724 | C | TIAM2 | R464Q | G | 1599 | A | TRIM42 | Y90C | A | 460 | G | UNC5B | G420D | G | 1675 | A |
| SVIL | S1958R | T | 6322 | G | TIAM2 | F588L | T | 1970 | C | TRIM45 | - | C | 0 | T | UNC5B | Y581H | T | 2157 | C |
| SVIL | K1123T | T | 3818 | G | TIAM2 | R1080W | C | 3446 | T | TRIM45 | R373C | G | 1644 | A | UNC5B | G419A | G | 1672 | C |
| SVOPL | R15Q | C | 44 | T | TICAM1 | R463H | C | 1639 | T | TRIM46 | I446V | A | 1336 | G | UNC5C | A29T | C | 434 | T |
| SVOPL | A429T | C | 1285 | T | TICAM1 | Q647* | G | 2190 | A | TRIM46 | V609I | G | 1825 | A | UNC5C | L717I | G | 2498 | T |
| SYAP1 | P155L | C | 557 | T | TICAM1 | P434L | G | 1552 | A | TRIM46 | R711C | C | 2131 | T | UNC5C | R278* | G | 1181 | A |
| SYBU | A570T | C | 1871 | T | TIE1 | V381M | G | 1220 | A | TRIM46 | E617* | G | 1849 | T | UNC5C | K346N | C | 1387 | A |
| SYBU | E342K | C | 1187 | T | TIE1 | R4W | C | 89 | T | TRIM47 | R581C | G | 1768 | A | UNC5CL | R292H | C | 964 | T |
| SYCE1 | L36F | C | 213 | G | TIE1 | R583C | C | 1826 | T | TRIM47 | R573W | G | 1744 | A | UNC5CL | R192C | G | 663 | A |
| SYCE1 | G218E | C | 758 | T | TIE1 | R518H | G | 1632 | A | TRIM48 | S3Y | C | 142 | A | UNC5D | S244L | C | 741 | T |
| SYCE1L | K64N | G | 192 | T | TIE1 | R68H | G | 282 | A | TRIM48 | Y176H | T | 660 | C | UNC5D | A14V | C | 51 | T |
| SYCE1L | S171L | C | 512 | T | TIE1 | R388C | C | 1241 | T | TRIM48 | S33N | G | 232 | A | UNC5D | L927I | C | 2789 | A |
| SYCN | F103L | G | 321 | T | TIE1 | R369W | C | 1184 | T | TRIM5 | K463T | T | 1645 | G | UNC5D | R799H | G | 2406 | A |
| SYCP1 | L530R | T | 1829 | G | TIE1 | Q87R | A | 339 | G | TRIM50 | R348H | C | 1244 | T | UNC5D | L157V | T | 479 | G |
| SYCP2 | I680T | A | 2178 | G | TIE1 | R583H | G | 1827 | A | TRIM50 | T314M | G | 1142 | A | UNC80 | L662I | C | 2064 | A |
| SYCP2 | S1204Y | G | 3750 | T | TIFAB | R55H | C | 365 | T | TRIM52 | N222D | T | 969 | C | UNC80 | N172S | A | 595 | G |
| SYCP2 | K931N | C | 2932 | A | TIFAB | Y13C | T | 239 | C | TRIM54 | E131* | G | 661 | T | UNC80 | L2271I | C | 6891 | A |
| SYCP2 | K741N | C | 2362 | A | TIGD5 | R619C | C | 1924 | T | TRIM55 | T34M | C | 327 | T | UNC80 | S2445L | C | 7414 | T |
| SYCP2 | K459T | T | 1515 | G | TIGD5 | Q63* | C | 256 | T | TRIM55 | E334D | A | 1228 | T | UNC80 | C2027W | T | 6161 | G |
| SYCP2 | Y318C | T | 1092 | C | TIGD5 | R67C | C | 268 | T | TRIM55 | S182N | G | 771 | A | UNC80 | L2320V | C | 7038 | G |
| SYCP2 | K175N | T | 664 | G | TIGD5 | R546K | G | 1706 | A | TRIM55 | K226N | G | 904 | T | UNC80 | P1700L | C | 5179 | T |
| SYCP2L | K782R | A | 2641 | G | TIGD6 | K513R | T | 2313 | C | TRIM56 | R533C | C | 1894 | T | UNC80 | Q1701* | C | 5181 | T |
| SYCP2L | R106W | C | 612 | T | TIGD7 | R313* | G | 2766 | A | TRIM56 | P480L | C | 1736 | T | UNC80 | R2679W | C | 8115 | T |
| SYCP2L | S527Y | C | 1876 | A | TIGIT | T97M | C | 2905 | T | TRIM56 | L19M | C | 352 | A | UNC80 | V1691I | G | 5151 | A |
| SYDE1 | Q294R | A | 912 | G | TIGIT | R238K | G | 3328 | A | TRIM56 | A327V | C | 1277 | T | UNC80 | R911H | G | 2812 | A |
| SYDE1 | R574H | G | 1752 | A | TIMD4 | R113H | C | 395 | T | TRIM56 | A329T | G | 1282 | A | UNC80 | Q1667H | G | 5081 | T |
| SYDE1 | P693S | C | 2108 | T | TIMD4 | T29M | G | 143 | A | TRIM58 | M177I | G | 579 | A | UNC80 | R2604* | C | 7890 | T |
| SYDE2 | I383M | T | 1198 | C | TIMELESS | R801* | G | 2569 | A | TRIM58 | R472M | G | 1463 | T | UNC80 | S974Y | C | 3001 | A |
| SYDE2 | C147Y | C | 489 | T | TIMELESS | F777I | A | 2497 | T | TRIM6 | E130* | G | 632 | T | UNC80 | R2059* | C | 6255 | T |

| | | | | | | | | | | | | | | | | | | | |
|---|---|---|---|---|---|---|---|---|---|---|---|---|---|---|---|---|---|---|---|
| SYDE2 | D357V | T | 1119 | A | TIMELESS | E139G | T | 584 | C | TRIM6 | L350I | C | 1292 | A | UNC80 | E2185K | G | 6633 | A |
| SYDE2 | N756H | T | 2315 | G | TIMELESS | R1033W | G | 3265 | A | TRIM6-TRIM34 | T484A | A | 1623 | G | UNC80 | R2706* | C | 8196 | T |
| SYK | P85L | C | 402 | T | TIMM22 | A76V | C | 253 | T | TRIM60 | E459K | G | 1591 | A | UNC80 | K698R | A | 2173 | G |
| SYK | V633M | G | 2045 | A | TIMM44 | R143G | G | 429 | C | TRIM60 | R390* | C | 1384 | T | UNC80 | R1807C | C | 5499 | T |
| SYK | A52T | G | 302 | A | TIMM50 | V400M | G | 1331 | A | TRIM60 | D433N | G | 1513 | A | UNC93A | K236N | G | 883 | T |
| SYMPK | G1272 W | C | 4059 | A | TIMM8A | R58Q | C | 705 | T | TRIM62 | A358V | G | 1307 | A | UNC93A | A214D | C | 816 | A |
| SYMPK | S598L | G | 2038 | A | TIMM8B | G20C | C | 69 | A | TRIM63 | R230Q | C | 828 | T | UNC93A | F353L | C | 1234 | A |
| SYMPK | G178D | C | 778 | T | TIMP3 | A183T | G | 848 | A | TRIM63 | F307L | G | 1060 | T | UNC93B1 | T184A | T | 630 | C |
| SYMPK | S353L | G | 1303 | A | TIMP4 | S122I | C | 876 | A | TRIM64 | Q415H | A | 1245 | C | UNC93B1 | A121T | C | 441 | T |
| SYN3 | Y177C | T | 773 | C | TINAGL1 | I337T | T | 1086 | C | TRIM64B | R411M | C | 1232 | A | UNG | D72G | A | 285 | G |
| SYN3 | A281T | C | 1084 | T | TINF2 | R338S | C | 1356 | A | TRIM64C | I247V | T | 739 | C | UNK | M1T | T | 2 | C |
| SYN3 | R435C | G | 1546 | A | TINF2 | M286V | T | 1198 | C | TRIM64C | R47I | C | 140 | A | UNK | F678L | T | 2034 | G |
| SYNC | P27H | G | 539 | T | TINF2 | A373V | G | 1460 | A | TRIM64C | G413V | C | 1238 | A | UNKL | T313M | G | 946 | A |
| SYNC | I85T | A | 713 | G | TIPARP | E470D | G | 1578 | T | TRIM66 | S493N | C | 1919 | T | UPB1 | R334W | C | 1344 | T |
| SYNE1 | A1608V | G | 5425 | A | TIPARP | N298T | A | 1061 | C | TRIM66 | A1149V | G | 3887 | A | UPF1 | I404V | A | 1482 | G |
| SYNE1 | S4796Y | G | 1498 9 | T | TJP1 | R170Q | C | 960 | T | TRIM66 | R895L | C | 3125 | A | UPF1 | E415D | G | 1517 | T |
| SYNE1 | A3140V | G | 1002 1 | A | TJP1 | S994L | G | 3432 | A | TRIM66 | G59V | C | 617 | A | UPF1 | A437T | G | 1581 | A |
| SYNE1 | E3099K | C | 9897 | T | TJP1 | S454I | C | 1812 | A | TRIM66 | - | A | 0 | G | UPF1 | R854W | C | 2832 | T |
| SYNE1 | S2326L | G | 7579 | A | TJP1 | - | T | 5698 | G | TRIM66 | R895Q | C | 3125 | T | UPF1 | P504L | C | 1783 | T |
| SYNE1 | A242D | G | 1327 | T | TJP1 | R1342W | G | 4475 | A | TRIM67 | V719M | G | 2197 | A | UPF2 | E1033D | T | 3573 | A |
| SYNE1 | R4764* | G | 1489 2 | A | TJP2 | A909T | G | 2933 | A | TRIM67 | R188Q | G | 605 | A | UPF2 | E78* | C | 706 | A |
| SYNE1 | S8236L | G | 2530 9 | A | TJP2 | T1124M | C | 3579 | T | TRIM67 | R619W | C | 1897 | T | UPF2 | D40N | C | 592 | T |
| SYNE1 | E4538G | T | 1421 5 | C | TJP2 | R731K | G | 2400 | A | TRIM67 | G669R | G | 2047 | A | UPF3A | R377Q | G | 1186 | A |
| SYNE1 | T8698M | G | 2669 5 | A | TJP2 | T335M | C | 1212 | T | TRIM68 | S183G | T | 799 | C | UPF3B | R451* | G | 1421 | A |
| SYNE1 | - | A | 0 | T | TJP2 | F608V | T | 2030 | G | TRIM68 | V347I | C | 1291 | T | UPK1A | R263C | C | 787 | T |
| SYNE1 | R5272W | G | 1641 6 | A | TJP2 | R837Q | G | 2718 | A | TRIM68 | Q264K | G | 1042 | T | UPK3A | G86D | G | 289 | A |
| SYNE1 | R1340* | T | 4620 | A | TJP3 | Y786C | A | 2357 | G | TRIM68 | L97P | A | 542 | G | UPP1 | R175Q | G | 1147 | A |
| SYNE1 | R4982H | C | 1554 7 | T | TJP3 | R201W | C | 601 | T | TRIM69 | E120D | G | 703 | T | UPP1 | T207P | A | 1242 | C |
| SYNE1 | Q1395R | T | 4786 | C | TJP3 | R366Q | G | 1097 | A | TRIM69 | P42S | C | 467 | T | UPP1 | Y85S | A | 877 | C |
| SYNE1 | - | C | 0 | A | TK1 | F108L | G | 563 | T | TRIM69 | V345I | G | 1376 | A | UPP2 | M271I | G | 833 | A |
| SYNE1 | E7676G | T | 2362 9 | C | TK2 | D84G | T | 476 | C | TRIM69 | E218* | G | 995 | T | UPP2 | F290L | T | 888 | C |
| SYNE1 | E5467G | T | 1700 | C | TKTL1 | S564L | C | 1880 | T | TRIM7 | R419C | G | 1317 | A | UPRT | K206N | G | 785 | T |

| | | | | | | | | | | | | | | | | | | |
|---|---|---|---|---|---|---|---|---|---|---|---|---|---|---|---|---|---|---|
| | | | 2 | | | | | | | | | | | | | | | |
| SYNE1 | V3671M | C | 11613 | T | TKTL1 | R375Q | G | 1313 | A | TRIM7 | Q283* | G | 909 | A | UQCRC1 | T343I | G | 1445 | A |
| SYNE1 | R5964C | G | 18492 | A | TKTL2 | R475Q | C | 1585 | T | TRIM71 | S563Y | C | 1751 | A | UQCRC1 | N175K | A | 942 | C |
| SYNE1 | L5095I | G | 15885 | T | TKTL2 | I282R | A | 1006 | C | TRIM71 | V359F | G | 1138 | T | UQCRC2 | A143T | G | 1191 | A |
| SYNE1 | Q541* | G | 2223 | A | TLCD2 | N250S | T | 864 | C | TRIM71 | R496H | G | 1550 | A | UQCRC2 | R259* | C | 1539 | T |
| SYNE1 | T7210S | G | 22231 | C | TLE1 | R565C | G | 2758 | A | TRIM71 | N767K | C | 2364 | A | UQCRFSL1 | R92H | C | 386 | T |
| SYNE1 | G8529E | C | 26188 | T | TLE1 | L119F | C | 1422 | G | TRIM71 | E344K | G | 1093 | A | URB1 | - | A | 0 | G |
| SYNE1 | E7921* | C | 24363 | A | TLE2 | P662L | G | 2248 | A | TRIM72 | R386C | C | 1367 | T | URB1 | L1486P | A | 4573 | G |
| SYNE1 | E7181D | T | 22145 | G | TLE2 | K82R | T | 508 | C | TRIM72 | R169W | C | 716 | T | URB1 | A1234T | C | 3816 | T |
| SYNE1 | L7129F | G | 21987 | A | TLE3 | A198V | G | 1712 | A | TRIM72 | E473K | G | 1628 | A | URB1 | E296K | C | 1002 | T |
| SYNE1 | L5590V | A | 17370 | C | TLE3 | P16T | G | 1165 | T | TRIM75 | V106I | G | 316 | A | URB1 | F1755V | A | 5379 | C |
| SYNE1 | A5450V | G | 16951 | A | TLE3 | M678I | C | 3153 | T | TRIM77 | C29F | G | 86 | T | URB1 | A1229V | G | 3802 | A |
| SYNE1 | D4241Y | C | 13323 | A | TLE4 | M81L | A | 417 | T | TRIM77 | W274R | T | 820 | C | URB1 | A1686T | C | 5172 | T |
| SYNE1 | E3281* | C | 10443 | A | TLE4 | R463H | G | 1564 | A | TRIM77 | D24A | A | 71 | C | URB1 | L277M | G | 945 | T |
| SYNE1 | S1186F | G | 4159 | A | TLE4 | V352I | G | 1230 | A | TRIM77 | K143T | A | 428 | C | URB1 | K468T | T | 1519 | G |
| SYNE1 | F1136L | G | 4010 | T | TLE4 | K25T | A | 250 | C | TRIM9 | A314V | G | 1331 | A | URB1 | K348T | T | 1159 | G |
| SYNE1 | K1056N | C | 3770 | A | TLE6 | R165Q | G | 695 | A | TRIM9 | A280V | G | 1229 | A | URB1 | E2009* | C | 6141 | A |
| SYNE1 | I945V | T | 3435 | C | TLE6 | R134Q | G | 602 | A | TRIML1 | L145R | T | 574 | G | URB2 | I245V | A | 869 | G |
| SYNE1 | L731R | A | 2794 | C | TLK1 | R739* | G | 2620 | A | TRIML1 | T289M | C | 1006 | T | URB2 | D703N | G | 2243 | A |
| SYNE1 | R8735W | G | 26805 | A | TLK2 | A165V | C | 765 | T | TRIML1 | L200P | T | 739 | C | URB2 | P248H | C | 879 | A |
| SYNE1 | R5325* | G | 16575 | A | TLK2 | A284V | C | 1122 | T | TRIML2 | A302T | C | 934 | T | URB2 | K82Q | A | 380 | C |
| SYNE1 | S6857* | G | 21172 | T | TLL1 | L122V | T | 921 | G | TRIML2 | T306M | G | 947 | A | URGCP | A650T | C | 2442 | T |
| SYNE2 | E637K | G | 2139 | A | TLL1 | I246V | A | 1293 | G | TRIO | R1502* | C | 4528 | T | URGCP | W294* | C | 1376 | T |
| SYNE2 | V1094A | T | 3511 | C | TLL1 | F507V | T | 2076 | G | TRIO | R2496Q | G | 7511 | A | URGCP | F250V | A | 1242 | C |
| SYNE2 | L3382I | C | 10374 | A | TLL2 | D520N | C | 1784 | T | TRIO | G1787S | G | 5383 | A | URGCP | W316C | C | 1442 | A |
| SYNE2 | W16G | T | 276 | G | TLL2 | E666A | T | 2223 | G | TRIO | D1817G | A | 5474 | G | UROC1 | T514A | T | 1574 | C |
| SYNE2 | G6670C | G | 20238 | T | TLN1 | L2509P | A | 7880 | G | TRIO | A1290V | C | 3893 | T | UROC1 | F502L | G | 1540 | T |

| | | | | | | | | | | | | | | | | | | | |
|---|---|---|---|---|---|---|---|---|---|---|---|---|---|---|---|---|---|---|---|
| SYNE2 | C6103F | G | 18538 | T | TLN1 | V1851L | C | 5905 | A | TRIO | H679R | A | 2060 | G | UROC1 | G656S | C | 2000 | T |
| SYNE2 | A4314V | C | 13171 | T | TLN1 | Q783* | G | 2701 | A | TRIO | A1147V | C | 3464 | T | UROC1 | G656D | C | 2001 | T |
| SYNE2 | Q5979H | G | 18167 | T | TLN1 | R1368Q | C | 4457 | T | TRIO | R1926H | G | 5801 | A | UROD | E125K | G | 492 | A |
| SYNE2 | G777C | G | 2559 | T | TLN1 | D525G | T | 1928 | C | TRIO | L1440P | T | 4343 | C | UROS | S51Y | G | 377 | T |
| SYNE2 | D20N | G | 288 | A | TLN1 | K2063N | C | 6543 | A | TRIO | W1558C | G | 4698 | T | USF1 | T10M | G | 234 | A |
| SYNE2 | V113A | T | 568 | C | TLN1 | S1427A | A | 4633 | C | TRIO | N163T | A | 512 | C | USF2 | K250N | G | 859 | T |
| SYNE2 | K958R | A | 3103 | G | TLN1 | R1955W | G | 6217 | A | TRIO | N171H | A | 535 | C | USF2 | A48V | C | 252 | T |
| SYNE2 | K2699N | G | 8327 | T | TLN2 | A1565T | G | 4726 | A | TRIOBP | Q336* | C | 1261 | T | USH1C | R754W | G | 2369 | A |
| SYNE2 | K3573T | A | 10948 | C | TLN2 | G1786R | G | 5389 | A | TRIOBP | S2053G | A | 6412 | G | USH1C | V43M | C | 236 | T |
| SYNE2 | E4038* | G | 12342 | T | TLN2 | G2496R | G | 7519 | A | TRIOBP | R546* | C | 1891 | T | USH1C | F700L | G | 2209 | T |
| SYNE2 | L4533M | T | 13827 | A | TLN2 | E850K | G | 2581 | A | TRIOBP | R1309H | G | 4181 | A | USH1C | N207S | T | 729 | C |
| SYNE2 | - | G | 0 | T | TLN2 | A1416V | C | 4280 | T | TRIP10 | S512I | G | 1570 | T | USH1G | E228K | C | 865 | T |
| SYNGAP1 | P38L | C | 214 | T | TLN2 | C2244Y | G | 6764 | A | TRIP10 | R196H | G | 622 | A | USH1G | I147N | A | 623 | T |
| SYNGR3 | L57P | T | 328 | C | TLN2 | Q769R | A | 2339 | G | TRIP10 | A441T | G | 1356 | A | USH1G | M450V | T | 1531 | C |
| SYNGR4 | - | G | 0 | T | TLN2 | A1018S | G | 3085 | T | TRIP11 | R252* | G | 1542 | A | USH1G | E142K | C | 607 | T |
| SYNJ1 | E1023D | C | 3077 | A | TLN2 | G2066V | G | 6230 | T | TRIP11 | A1481V | G | 5230 | A | USH2A | R4192C | G | 12961 | A |
| SYNJ1 | P1167L | G | 3508 | A | TLN2 | T1856M | C | 5600 | T | TRIP11 | F1284C | A | 4639 | C | USH2A | D1904G | T | 6098 | C |
| SYNJ1 | P1111L | G | 3340 | A | TLN2 | N125H | A | 406 | C | TRIP11 | N1003T | T | 3796 | G | USH2A | T4222I | G | 13052 | A |
| SYNJ1 | G883C | C | 2655 | A | TLN2 | E1298* | G | 3925 | T | TRIP11 | D160N | C | 1266 | T | USH2A | L3486F | G | 10843 | A |
| SYNJ1 | M1I | C | 11 | A | TLN2 | S2396F | C | 7220 | T | TRIP12 | A1726V | G | 5346 | A | USH2A | Q4541* | G | 14008 | A |
| SYNJ2 | D226N | G | 751 | A | TLR1 | G417R | C | 1523 | T | TRIP12 | I826V | T | 2645 | C | USH2A | K3892N | T | 12063 | G |
| SYNJ2 | D243N | G | 802 | A | TLR1 | S77Y | G | 504 | T | TRIP12 | R379W | G | 1304 | A | USH2A | T2604N | G | 8198 | T |
| SYNJ2 | P1252L | C | 3830 | T | TLR10 | L727I | G | 2785 | T | TRIP12 | R1193H | C | 3747 | T | USH2A | A1069V | G | 3593 | A |
| SYNJ2 | D726N | G | 2251 | A | TLR10 | L206I | G | 1222 | T | TRIP13 | N365S | A | 1450 | G | USH2A | T648N | G | 2330 | T |
| SYNJ2 | F727L | C | 2256 | A | TLR2 | V451I | G | 1396 | A | TRIP13 | N50S | A | 505 | G | USH2A | C999F | C | 3383 | A |
| SYNJ2 | R730H | G | 2264 | A | TLR2 | R230Q | G | 734 | A | TRIP4 | K474* | A | 1480 | T | USH2A | T4652N | G | 14342 | T |
| SYNJ2BP | R46H | C | 264 | T | TLR2 | R486I | G | 1502 | T | TRIP4 | A4D | C | 71 | A | USH2A | P3777H | G | 11717 | T |
| SYNM | G1178S | G | 3652 | A | TLR3 | E460K | G | 1482 | A | TRIP4 | Q96E | C | 346 | G | USH2A | S3616A | A | 11233 | C |
| SYNM | E1051* | G | 3271 | T | TLR3 | Y302C | A | 1009 | G | TRMT1 | S416I | C | 1497 | A | USH2A | D3076Y | C | 9613 | A |
| SYNM | R802* | C | 2524 | T | TLR3 | R890H | G | 2773 | A | TRMT1 | A531T | C | 1841 | T | USH2A | K2597T | T | 8177 | G |

| | | | | | | | | | | | | | | | | | | | |
|---|---|---|---|---|---|---|---|---|---|---|---|---|---|---|---|---|---|---|---|
| SYNPO | V469A | T | 1780 | C | TLR3 | G869V | G | 2710 | T | TRMT11 | R319I | G | 1077 | T | USH2A | R2509W | G | 7912 | A |
| SYNPO2 | S549Y | C | 1842 | A | TLR3 | K89T | A | 370 | C | TRMT11 | T43S | C | 249 | G | USH2A | - | C | 0 | A |
| SYNPO2 | R401H | G | 1398 | A | TLR3 | L149* | T | 550 | G | TRMT2A | P593L | G | 2094 | A | USH2A | F1583S | A | 5135 | G |
| SYNPO2 | K1188N | G | 3760 | T | TLR3 | E306K | G | 1020 | A | TRMT2A | A432V | G | 1611 | A | USH2A | N856H | T | 2953 | G |
| SYNPO2 | R121* | C | 557 | T | TLR4 | T537M | C | 1711 | T | TRMT5 | R248* | G | 833 | A | USH2A | T495A | T | 1870 | C |
| SYNPO2 | L438I | C | 1508 | A | TLR5 | S615L | G | 2306 | A | TRMT5 | E155* | C | 554 | A | USH2A | Y2214H | A | 7027 | G |
| SYNPO2 | S663L | C | 2184 | T | TLR5 | F622L | G | 2328 | T | TRMT61A | T269M | C | 886 | T | USHBP1 | A199V | G | 738 | A |
| SYNPO2L | T961I | G | 3032 | A | TLR5 | K323T | T | 1430 | G | TRMT61A | A120T | G | 438 | A | USHBP1 | A295S | C | 1025 | A |
| SYNPO2L | A846V | G | 2687 | A | TLR6 | E539* | C | 1681 | A | TRMT61A | I10S | T | 109 | G | USHBP1 | R196C | G | 728 | A |
| SYNPO2L | F849C | A | 2696 | C | TLR6 | D92Y | C | 340 | A | TRMU | I245M | T | 1075 | G | USHBP1 | Q463R | T | 1530 | C |
| SYNPR | K184T | A | 962 | C | TLR7 | L254I | C | 899 | A | TRNAU1AP | S228I | G | 709 | T | USHBP1 | P416S | G | 1388 | A |
| SYNRG | V492I | C | 1474 | T | TLR7 | I735S | T | 2343 | G | TRNT1 | E50G | A | 251 | G | USO1 | A614V | C | 1841 | T |
| SYNRG | R138C | G | 412 | A | TLR8 | I547V | A | 1790 | G | TRO | A1247T | G | 3851 | A | USO1 | A120V | C | 359 | T |
| SYP | Y295H | A | 956 | G | TLR8 | E206K | G | 767 | A | TRO | T373I | C | 1230 | T | USO1 | S645A | T | 1933 | G |
| SYP | S48I | C | 216 | A | TLR8 | V118A | T | 504 | C | TRO | - | G | 0 | T | USP1 | P239L | C | 1044 | T |
| SYPL1 | D153Y | C | 504 | A | TLR8 | R298C | C | 1043 | T | TRO | L863F | C | 2699 | T | USP1 | I530V | A | 1916 | G |
| SYPL2 | Q252H | G | 972 | T | TLR9 | G783S | C | 2981 | T | TRO | F1406C | T | 4329 | G | USP1 | R517* | C | 1877 | T |
| SYS1 | R140W | C | 661 | T | TLR9 | A435V | G | 1938 | A | TROAP | R25H | G | 241 | A | USP1 | K219T | A | 984 | C |
| SYT1 | - | T | 0 | C | TLR9 | R901C | G | 3335 | A | TROAP | E507D | G | 1688 | T | USP10 | I712M | T | 2278 | G |
| SYT10 | R379C | G | 1432 | A | TLR9 | A736T | C | 2840 | T | TROAP | F725L | C | 2342 | A | USP11 | R638Q | G | 1913 | A |
| SYT10 | T204I | G | 908 | A | TLR9 | R247C | G | 1373 | A | TROVE2 | D134Y | G | 776 | T | USP11 | - | G | 0 | T |
| SYT10 | R40W | G | 415 | A | TLR9 | C790R | A | 3002 | G | TRPA1 | V302I | C | 1079 | T | USP11 | E360K | G | 1078 | A |
| SYT10 | R286H | C | 1154 | T | TLR9 | M371I | C | 1747 | T | TRPA1 | K610T | T | 2004 | G | USP12 | R285H | C | 1111 | T |
| SYT10 | N236S | T | 1004 | C | TM2D1 | P94H | G | 398 | T | TRPA1 | S887N | C | 2835 | T | USP12 | D334N | C | 1257 | T |
| SYT11 | V381I | G | 1394 | A | TM2D1 | A66P | C | 313 | G | TRPA1 | S293L | G | 1053 | A | USP12 | Q367R | T | 1357 | C |
| SYT11 | G328S | G | 1235 | A | TM2D2 | T182M | G | 628 | A | TRPA1 | K54N | C | 337 | A | USP12 | L68I | G | 459 | T |
| SYT12 | R419Q | G | 1320 | A | TM2D2 | H67R | T | 283 | C | TRPC1 | R148Q | G | 929 | A | USP13 | V44I | G | 601 | A |
| SYT12 | R236W | C | 770 | T | TM4SF1 | N218H | T | 886 | G | TRPC1 | R486W | C | 1942 | T | USP13 | Q386H | G | 1629 | T |
| SYT12 | R247H | G | 804 | A | TM4SF18 | E132* | C | 660 | A | TRPC1 | R610I | G | 2315 | T | USP13 | Q646R | A | 2408 | G |
| SYT12 | S45L | C | 198 | T | TM4SF20 | A167T | C | 537 | T | TRPC3 | R174C | G | 594 | A | USP13 | A683T | G | 2518 | A |
| SYT12 | K308N | G | 988 | T | TM4SF4 | A88V | C | 1167 | T | TRPC3 | R189H | C | 640 | T | USP14 | S340Y | C | 1019 | A |
| SYT13 | A112T | C | 460 | T | TM4SF5 | L172P | T | 546 | C | TRPC3 | Q498H | C | 1568 | G | USP14 | R162H | G | 485 | A |
| SYT14 | A598V | C | 1902 | T | TM4SF5 | G187D | G | 591 | A | TRPC3 | E879* | C | 2709 | A | USP15 | P672S | C | 2023 | T |
| SYT14 | F296L | T | 995 | C | TM4SF5 | V30A | T | 120 | C | TRPC4 | G425R | C | 2131 | T | USP16 | R305H | G | 1104 | A |
| SYT14 | V328I | G | 1091 | A | TM6SF1 | K268T | A | 912 | C | TRPC4 | D428E | G | 2142 | T | USP17L1P | V369A | T | 1106 | C |
| SYT14 | R380C | C | 1247 | T | TM6SF1 | S278A | T | 941 | G | TRPC4 | N621I | T | 2720 | A | USP17L1P | Q345H | G | 1035 | T |
| SYT15 | R203W | G | 1195 | A | TM7SF2 | M210V | A | 775 | G | TRPC4 | R706K | C | 2975 | T | USP18 | L54P | T | 591 | C |
| SYT15 | D198N | C | 1180 | T | TM7SF2 | G254R | G | 907 | A | TRPC4 | K159T | T | 1334 | G | USP19 | W447* | C | 1553 | T |
| SYT16 | Q622H | A | 2063 | C | TM7SF3 | L377P | A | 1368 | G | TRPC4AP | A657T | C | 2059 | T | USP19 | R881H | C | 2854 | T |

| | | | | | | | | | | | | | | | | | | | |
|---|---|---|---|---|---|---|---|---|---|---|---|---|---|---|---|---|---|---|---|
| SYT16 | R441Q | G | 1519 | A | TM7SF4 | F81C | T | 293 | G | TRPC4AP | N331H | T | 1081 | G | USP19 | R1380H | C | 4351 | T |
| SYT16 | R599C | C | 1992 | T | TM9SF1 | R98Q | C | 652 | T | TRPC5 | A704V | G | 3030 | A | USP19 | G1151S | C | 3663 | T |
| SYT16 | E87D | A | 458 | C | TM9SF2 | E88K | G | 452 | A | TRPC5 | G846S | C | 3455 | T | USP19 | W481R | A | 1653 | G |
| SYT17 | Y3H | T | 405 | C | TM9SF3 | A119V | G | 573 | A | TRPC5 | E595K | C | 2702 | T | USP19 | L1114P | A | 3553 | G |
| SYT17 | R65W | C | 591 | T | TM9SF3 | V311G | A | 1149 | C | TRPC6 | S36L | G | 532 | A | USP19 | - | C | 0 | T |
| SYT17 | S73Y | C | 616 | A | TM9SF4 | S371L | C | 1347 | T | TRPC6 | G20R | C | 483 | T | USP19 | S542G | T | 1836 | C |
| SYT17 | S66N | G | 595 | A | TMBIM4 | K217N | C | 686 | A | TRPC6 | E120* | C | 783 | A | USP19 | S317T | A | 1161 | T |
| SYT17 | M415L | A | 1641 | T | TMBIM4 | I131F | T | 426 | A | TRPC7 | V483M | C | 1730 | T | USP2 | S280* | G | 1134 | T |
| SYT3 | G108C | C | 956 | A | TMBIM4 | F199S | A | 631 | G | TRPC7 | R136H | C | 690 | T | USP2 | - | C | 0 | T |
| SYT3 | R45Q | C | 768 | T | TMC1 | - | T | 0 | A | TRPC7 | R120C | G | 641 | A | USP2 | L185F | G | 848 | A |
| SYT3 | A118V | G | 987 | A | TMC1 | R604* | C | 2350 | T | TRPC7 | S394Y | G | 1464 | T | USP20 | R703Q | G | 2290 | A |
| SYT3 | R97H | C | 924 | T | TMC1 | A166V | C | 1037 | T | TRPM1 | R928W | G | 2896 | A | USP20 | Q809R | A | 2608 | G |
| SYT4 | E80* | C | 607 | A | TMC2 | S840A | T | 2533 | G | TRPM1 | R546H | C | 1751 | T | USP20 | T911M | C | 2914 | T |
| SYT4 | E235* | C | 1072 | A | TMC2 | F611Y | T | 1847 | A | TRPM1 | E223K | C | 781 | T | USP20 | R613C | C | 2019 | T |
| SYT5 | V212A | A | 916 | G | TMC2 | G350S | G | 1063 | A | TRPM1 | R1444W | G | 4444 | A | USP20 | A253T | G | 939 | A |
| SYT7 | L247M | G | 745 | T | TMC2 | K300N | G | 915 | T | TRPM1 | P1028S | G | 3196 | A | USP21 | R445Q | G | 1711 | A |
| SYT8 | A55T | G | 291 | A | TMC2 | F304L | T | 927 | G | TRPM1 | I1485S | A | 4568 | C | USP21 | - | A | 0 | G |
| SYT8 | R261Q | G | 910 | A | TMC2 | R663H | G | 2003 | A | TRPM1 | K1476N | C | 4542 | A | USP21 | R354* | C | 1437 | T |
| SYT8 | W292* | G | 1003 | A | TMC2 | N730S | A | 2204 | G | TRPM1 | K1433T | T | 4412 | G | USP22 | K489N | C | 1484 | A |
| SYT9 | E344K | G | 1267 | A | TMC3 | I718V | T | 2193 | C | TRPM1 | L59I | G | 289 | T | USP22 | G325A | C | 991 | G |
| SYT9 | D209Y | G | 862 | T | TMC3 | S1059N | C | 3217 | T | TRPM2 | S947P | T | 2852 | C | USP24 | R1451C | G | 4604 | A |
| SYT9 | L339R | T | 1253 | G | TMC3 | R535W | G | 1644 | A | TRPM2 | R546C | C | 1649 | T | USP24 | I2297M | A | 7144 | C |
| SYTL1 | G5D | G | 181 | A | TMC3 | H434N | G | 1341 | T | TRPM2 | R1380H | G | 4152 | A | USP24 | R540W | G | 1871 | A |
| SYTL2 | V973A | A | 2918 | G | TMC4 | P550H | G | 1781 | T | TRPM2 | A701T | G | 2114 | A | USP25 | R211Q | G | 1001 | A |
| SYTL2 | I1554T | A | 4661 | G | TMC4 | S35L | G | 236 | A | TRPM2 | A1013T | G | 3050 | A | USP26 | T122I | G | 417 | A |
| SYTL2 | E1617A | T | 4850 | G | TMC5 | Q485H | G | 2204 | T | TRPM2 | V1495I | G | 4496 | A | USP26 | L460P | A | 1431 | G |
| SYTL2 | K1181T | T | 3542 | G | TMC6 | R395Q | C | 1325 | T | TRPM2 | E450K | G | 1361 | A | USP28 | R1060* | G | 3211 | A |
| SYTL2 | F1161L | G | 3483 | T | TMC6 | T76M | G | 368 | A | TRPM2 | A1547T | G | 4652 | A | USP28 | - | C | 0 | A |
| SYTL2 | E1053K | C | 3157 | T | TMC7 | A375P | G | 1235 | C | TRPM3 | R159* | G | 475 | A | USP28 | G905D | C | 2747 | T |
| SYTL2 | E956D | T | 2868 | G | TMC7 | A375P | G | 1235 | C | TRPM3 | L506I | A | 1516 | T | USP28 | F232C | A | 728 | C |
| SYTL2 | K650N | C | 1950 | A | TMC7 | P626T | C | 1988 | A | TRPM3 | P1094S | G | 3280 | A | USP29 | H498Y | C | 1759 | T |
| SYVN1 | F29L | A | 179 | G | TMC8 | A715V | C | 2526 | T | TRPM3 | A723V | G | 2168 | A | USP29 | D91Y | G | 538 | T |
| SYVN1 | P360T | G | 1172 | T | TMC8 | F490L | C | 1852 | A | TRPM3 | K1184R | T | 3551 | C | USP29 | E217* | G | 916 | T |
| SYVN1 | R140H | C | 513 | T | TMCC1 | R618H | C | 2194 | T | TRPM3 | L1107F | C | 3321 | A | USP29 | L537* | T | 1877 | G |
| T | S431L | G | 1576 | A | TMCC2 | Q366* | C | 1485 | T | TRPM3 | S1125L | G | 3374 | A | USP29 | S898Y | C | 2960 | A |
| T | P360S | G | 1362 | A | TMCC3 | A127T | C | 497 | T | TRPM3 | L784I | G | 2350 | T | USP3 | E189D | A | 696 | T |
| T | R422H | C | 1549 | T | TMCO1 | L149I | G | 874 | T | TRPM3 | R652W | G | 1954 | A | USP3 | G305* | G | 1042 | T |
| TAAR1 | I62V | T | 184 | C | TMCO1 | R87Q | C | 689 | T | TRPM3 | R231C | G | 691 | A | USP30 | L45I | C | 226 | A |
| TAAR2 | A173S | C | 517 | A | TMCO2 | K132T | A | 488 | C | TRPM3 | K154T | T | 461 | G | USP31 | R516C | G | 1546 | A |

| | | | | | | | | | | | | | | | | | | | |
|---|---|---|---|---|---|---|---|---|---|---|---|---|---|---|---|---|---|---|---|
| TAAR5 | A3V | G | 8 | A | TMCO3 | R47W | C | 498 | T | TRPM3 | R119Q | C | 356 | T | USP31 | S891R | T | 2671 | G |
| TAAR6 | A10V | C | 29 | T | TMCO3 | A19V | C | 415 | T | TRPM4 | R474C | C | 1492 | T | USP31 | L146V | G | 436 | C |
| TAAR6 | F206L | T | 616 | C | TMCO4 | A282T | C | 1086 | T | TRPM4 | R597Q | G | 1862 | A | USP31 | K1097R | T | 3290 | C |
| TAAR8 | D128N | G | 382 | A | TMCO5A | L186I | C | 658 | A | TRPM4 | R640C | C | 1990 | T | USP32 | R229H | C | 972 | T |
| TAAR9 | L268I | C | 802 | A | TMCO5A | T85A | A | 355 | G | TRPM4 | R645H | G | 2006 | A | USP32 | L529S | A | 1872 | G |
| TAAR9 | C190* | C | 570 | A | TMCO5A | F230L | C | 792 | A | TRPM4 | R39H | G | 188 | A | USP32 | S1017L | G | 3336 | A |
| TAB1 | D123N | G | 429 | A | TMCO6 | R111W | C | 355 | T | TRPM4 | W579C | G | 1809 | T | USP33 | F619V | A | 1855 | C |
| TAB1 | A343T | G | 1089 | A | TMCO7 | E778A | A | 2345 | C | TRPM5 | R903C | G | 2716 | A | USP33 | V709A | A | 2126 | G |
| TAB1 | R83W | C | 309 | T | TMCO7 | K42T | A | 137 | C | TRPM5 | E568K | C | 1711 | T | USP33 | R464I | C | 1391 | A |
| TAB2 | R342C | C | 1202 | T | TMCO7 | E502D | A | 1518 | C | TRPM5 | Q118* | G | 361 | A | USP33 | L287F | C | 861 | A |
| TAB2 | S440G | A | 1496 | G | TMED10 | R209* | G | 692 | A | TRPM5 | G405D | C | 1223 | T | USP33 | Q211P | T | 632 | G |
| TAB3 | R96Q | C | 465 | T | TMED10 | K169E | T | 572 | C | TRPM5 | T690M | G | 2078 | A | USP34 | F957I | A | 2869 | T |
| TACC1 | G83R | G | 626 | A | TMED5 | R183H | C | 1034 | T | TRPM6 | E1649* | C | 5183 | A | USP34 | R403* | G | 1207 | A |
| TACC1 | N330T | A | 1368 | C | TMED5 | P8S | G | 508 | A | TRPM6 | R549I | C | 1884 | A | USP34 | P2602T | G | 7804 | T |
| TACC2 | A1263T | G | 4127 | A | TMED5 | T144P | T | 916 | G | TRPM6 | G1995R | C | 6221 | T | USP34 | I412V | T | 1234 | C |
| TACC2 | R2301G | A | 7241 | G | TMED7-TICAM2 | I378V | T | 1501 | C | TRPM6 | A1239T | C | 3953 | T | USP34 | R211H | C | 632 | T |
| TACC3 | E822K | G | 2570 | A | TMED7-TICAM2 | R169H | C | 875 | T | TRPM6 | R952Q | C | 3093 | T | USP34 | R688Q | C | 2063 | T |
| TACC3 | A443V | C | 1434 | T | TMEFF2 | P346H | G | 2222 | T | TRPM6 | E1736* | C | 5444 | A | USP34 | L3002I | G | 9004 | T |
| TACR2 | R65C | G | 193 | A | TMEM102 | A242S | G | 997 | T | TRPM6 | D223Y | C | 905 | A | USP34 | R2966* | G | 8896 | A |
| TACR2 | R103H | C | 308 | T | TMEM106B | P200H | C | 786 | A | TRPM7 | F1001L | A | 3266 | G | USP34 | K1061T | T | 3182 | G |
| TACR3 | A146V | G | 578 | A | TMEM108 | R200* | C | 808 | T | TRPM7 | R274I | C | 1086 | A | USP34 | - | C | 0 | T |
| TACR3 | D417N | C | 1390 | T | TMEM108 | A7V | C | 230 | T | TRPM7 | R588Q | C | 2028 | T | USP35 | P771H | C | 2558 | A |
| TADA1 | D135N | C | 497 | T | TMEM108 | A156V | C | 677 | T | TRPM8 | D198Y | G | 632 | T | USP35 | R616H | G | 2093 | A |
| TADA2A | R256* | C | 939 | T | TMEM108 | T357M | C | 1280 | T | TRPM8 | N530K | C | 1630 | A | USP35 | R779C | C | 2581 | T |
| TADA2B | D171A | A | 701 | C | TMEM108 | T360A | A | 1288 | G | TRPM8 | T522M | C | 1605 | T | USP35 | P602T | C | 2050 | A |
| TADA3 | R347C | G | 1598 | A | TMEM108 | D71G | A | 422 | G | TRPM8 | E282D | G | 886 | T | USP35 | S423N | G | 1514 | A |
| TADA3 | A335T | C | 1562 | T | TMEM108 | T292A | A | 1084 | G | TRPM8 | D718N | G | 2192 | A | USP35 | A426V | C | 1523 | T |
| TADA3 | R333C | G | 1556 | A | TMEM11 | A178T | C | 975 | T | TRPS1 | E731K | C | 2796 | T | USP36 | T905M | G | 3039 | A |
| TAF1 | D1426G | A | 4328 | G | TMEM11 | A177T | C | 972 | T | TRPS1 | P1292H | G | 4480 | T | USP36 | K813N | T | 2764 | G |
| TAF1 | D1502A | A | 4556 | C | TMEM115 | T184A | T | 1097 | C | TRPS1 | G873R | C | 3222 | T | USP38 | R1010W | C | 3534 | T |
| TAF1 | V1877I | G | 5680 | A | TMEM117 | R97* | C | 416 | T | TRPS1 | Q671K | G | 2616 | T | USP38 | S897G | A | 3195 | G |
| TAF1 | L671P | T | 2063 | C | TMEM117 | P512S | C | 1661 | T | TRPS1 | A852V | G | 3160 | A | USP38 | T922A | A | 3270 | G |
| TAF1 | R122Q | G | 416 | A | TMEM117 | - | A | 0 | G | TRPS1 | A619V | G | 2461 | A | USP38 | L350V | T | 1554 | G |
| TAF1 | T127M | C | 431 | T | TMEM117 | R423H | G | 1395 | A | TRPS1 | R257Q | C | 1375 | T | USP38 | E696K | G | 2592 | A |
| TAF10 | - | C | 0 | A | TMEM117 | L71Q | T | 339 | A | TRPS1 | K236T | T | 1312 | G | USP40 | R103* | G | 307 | A |
| TAF10 | F162C | A | 963 | C | TMEM119 | V88M | C | 425 | T | TRPS1 | R758M | C | 2878 | A | USP40 | G509W | C | 1525 | A |
| TAF11 | R126H | C | 509 | T | TMEM121 | V250I | G | 912 | A | TRPV1 | P429L | G | 1813 | A | USP40 | R1231L | C | 3692 | A |
| TAF12 | E135* | C | 562 | A | TMEM121 | V41M | G | 285 | A | TRPV1 | A209T | C | 1152 | T | USP40 | - | C | 0 | A |

| | | | | | | | | | | | | | | | | | | | |
|---|---|---|---|---|---|---|---|---|---|---|---|---|---|---|---|---|---|---|---|
| TAF12 | F91C | A | 431 | C | TMEM128 | F151C | A | 462 | C | TRPV1 | R628W | G | 2409 | A | USP40 | R574S | T | 1722 | G |
| TAF15 | R483* | C | 1533 | T | TMEM130 | R94C | G | 469 | A | TRPV1 | D423N | C | 1794 | T | USP42 | R584H | G | 1874 | A |
| TAF1B | R261C | C | 969 | T | TMEM131 | R75H | C | 453 | T | TRPV2 | R537H | G | 1977 | A | USP42 | E1153K | G | 3580 | A |
| TAF1B | M1T | T | 190 | C | TMEM131 | S418Y | G | 1482 | T | TRPV2 | F403L | T | 1574 | C | USP42 | V250I | G | 871 | A |
| TAF1B | F519C | T | 1744 | G | TMEM132A | R1014C | C | 3233 | T | TRPV2 | E230D | G | 1057 | T | USP42 | R1131H | G | 3515 | A |
| TAF1C | R425H | C | 1502 | T | TMEM132A | V410A | T | 1422 | C | TRPV3 | R690C | G | 2390 | A | USP42 | A569T | G | 1828 | A |
| TAF1C | P300H | G | 1127 | T | TMEM132B | D719N | G | 2163 | A | TRPV3 | R363C | G | 1409 | A | USP43 | Y672C | A | 2015 | G |
| TAF1C | V100M | C | 526 | T | TMEM132B | T213N | C | 646 | A | TRPV3 | K434N | C | 1624 | A | USP43 | R1071H | G | 3212 | A |
| TAF1C | G407R | C | 1447 | T | TMEM132B | T361M | C | 1090 | T | TRPV4 | V829M | C | 2574 | T | USP45 | V204G | A | 655 | C |
| TAF1C | D113Y | C | 565 | A | TMEM132B | V447A | T | 1348 | C | TRPV4 | R661C | G | 2070 | A | USP46 | L256R | A | 767 | C |
| TAF1L | L1813I | G | 5527 | T | TMEM132B | S863T | G | 2596 | C | TRPV4 | S48L | G | 232 | A | USP47 | F925C | T | 2774 | G |
| TAF1L | D1105N | C | 3403 | T | TMEM132B | F922I | T | 2772 | A | TRPV5 | I341S | A | 1371 | C | USP48 | C512Y | C | 2184 | T |
| TAF1L | V946F | C | 2926 | A | TMEM132C | R148* | C | 442 | T | TRPV5 | S696N | C | 2436 | T | USP49 | R156W | G | 796 | A |
| TAF1L | R1450C | G | 4438 | A | TMEM132C | A549T | G | 1645 | A | TRPV5 | - | T | 0 | C | USP49 | P137Q | G | 740 | T |
| TAF1L | E1819* | C | 5545 | A | TMEM132C | E672V | A | 2015 | T | TRPV6 | R686* | G | 2302 | A | USP5 | R754W | C | 2319 | T |
| TAF1L | R1049C | G | 3235 | A | TMEM132C | G563S | G | 1687 | A | TRPV6 | R628H | C | 2129 | T | USP5 | V465M | G | 1452 | A |
| TAF1L | E680* | C | 2128 | A | TMEM132C | V664I | G | 1990 | A | TRPV6 | R359H | C | 1322 | T | USP5 | V794F | G | 2439 | T |
| TAF1L | D144Y | C | 520 | A | TMEM132C | G472* | G | 1414 | T | TRPV6 | E535K | C | 1849 | T | USP50 | K135N | C | 585 | A |
| TAF1L | R122I | C | 455 | A | TMEM132C | D1038N | G | 3112 | A | TRRAP | R1015W | C | 3252 | T | USP50 | D107G | T | 500 | C |
| TAF2 | E205K | C | 883 | T | TMEM132D | H254N | G | 760 | T | TRRAP | R1447C | C | 4548 | T | USP53 | E898* | G | 3871 | T |
| TAF2 | N149D | T | 715 | C | TMEM132D | S836I | C | 2507 | A | TRRAP | A1918V | C | 5962 | T | USP54 | C1293R | A | 3978 | G |
| TAF2 | R541* | G | 1891 | A | TMEM132D | S716N | C | 2147 | T | TRRAP | R1947Q | G | 6049 | A | USP54 | A885V | G | 2755 | A |
| TAF3 | E122* | G | 570 | T | TMEM132D | P525L | G | 1574 | A | TRRAP | R3618Q | G | 11062 | A | USP54 | T397M | G | 1291 | A |
| TAF3 | R425I | G | 1480 | T | TMEM132D | S737Y | G | 2210 | T | TRRAP | P3647L | C | 11149 | T | USP54 | D1484Y | C | 4551 | A |
| TAF3 | E594D | A | 1988 | C | TMEM132D | E495* | C | 1483 | A | TRRAP | A1055T | G | 3372 | A | USP54 | S1154R | T | 3561 | G |
| TAF4 | T725M | G | 2174 | A | TMEM132E | H134Y | C | 554 | T | TRRAP | R616H | G | 2056 | A | USP54 | K1103T | T | 3409 | G |
| TAF4B | R760C | C | 2767 | T | TMEM132E | R317Q | G | 1104 | A | TRRAP | V2212I | G | 6843 | A | USP54 | R758Q | C | 2374 | T |
| TAF4B | R616L | G | 2336 | T | TMEM132E | A391T | G | 1325 | A | TRRAP | V3428I | G | 10491 | A | USP54 | F508C | A | 1624 | C |
| TAF5 | R610Q | G | 1852 | A | TMEM133 | T31N | C | 321 | A | TRRAP | R816W | C | 2655 | T | USP6 | V338M | G | 3242 | A |
| TAF5 | R505C | C | 1536 | T | TMEM135 | R55W | C | 186 | T | TRRAP | R2469C | C | 7614 | T | USP6 | N842T | A | 4755 | C |
| TAF5L | R491W | G | 1471 | A | TMEM135 | I268N | T | 826 | A | TRRAP | A2V | C | 214 | T | USP6 | R1249W | C | 5975 | T |
| TAF5L | V566I | C | 1696 | T | TMEM136 | L26P | T | 318 | C | TRRAP | V399I | G | 1404 | A | USP6 | R1113Q | G | 5568 | A |
| TAF6 | D443N | C | 1505 | T | TMEM139 | R104H | G | 419 | A | TRRAP | W2374L | G | 7330 | T | USP6 | - | G | 0 | T |
| TAF6 | R103H | C | 486 | T | TMEM140 | R9C | C | 248 | T | TRRAP | H1661R | A | 5191 | G | USP6 | E893D | G | 4909 | T |
| TAF6 | C187Y | C | 738 | T | TMEM143 | R278H | C | 853 | T | TRRAP | A2777V | C | 8539 | T | USP6 | E1273A | A | 6048 | C |
| TAF6 | R459L | C | 1554 | A | TMEM144 | F101C | T | 822 | G | TRRAP | G1024C | G | 3279 | T | USP6NL | A492T | C | 1517 | T |
| TAF6L | R236H | G | 818 | A | TMEM146 | R787H | G | 2421 | A | TRRAP | T1219M | C | 3865 | T | USP6NL | R543W | G | 1670 | A |
| TAF6L | V322A | T | 1076 | C | TMEM146 | S476R | T | 1489 | A | TRRAP | D3636N | G | 11115 | A | USP6NL | T520I | G | 1602 | A |
| TAF7 | K154N | C | 1202 | A | TMEM146 | F72C | T | 276 | G | TRRAP | F2814S | T | 8650 | C | USP6NL | A492T | C | 1517 | T |

| | | | | | | | | | | | | | | | | | | | |
|---|---|---|---|---|---|---|---|---|---|---|---|---|---|---|---|---|---|---|---|
| TAF7 | K129T | T | 1126 | G | TMEM146 | F437L | C | 1372 | A | TRUB1 | - | A | 0 | C | USP6NL | L186V | A | 599 | C |
| TAF7L | - | C | 0 | T | TMEM149 | G226W | C | 774 | A | TRUB2 | L147P | A | 774 | G | USP6NL | R113Q | C | 381 | T |
| TAF7L | S271L | G | 824 | A | TMEM149 | S301L | G | 1000 | A | TRUB2 | W274C | C | 1156 | A | USP6NL | A527P | C | 1622 | G |
| TAF7L | T65M | G | 206 | A | TMEM14C | F86C | T | 568 | G | TRUB2 | R199Q | C | 930 | T | USP7 | S371* | G | 1156 | T |
| TAF8 | V169M | G | 525 | A | TMEM150A | G99V | C | 484 | A | TSC1 | R1097C | G | 3511 | A | USP7 | A579T | C | 1779 | T |
| TAF8 | M194I | G | 602 | T | TMEM150A | A136V | G | 595 | A | TSC1 | A57T | C | 391 | T | USP7 | E555K | C | 1707 | T |
| TAF9B | P144S | G | 525 | A | TMEM150A | G70S | C | 396 | T | TSC2 | V679M | G | 2665 | A | USP7 | H3Q | G | 208 | C |
| TAGAP | N272D | T | 1146 | C | TMEM150B | A205T | C | 796 | T | TSC2 | R1639H | G | 5546 | A | USP8 | N744S | A | 2569 | G |
| TAGLN | A2V | C | 523 | T | TMEM150C | R219C | G | 974 | A | TSC2 | N1214I | A | 4271 | T | USP9X | G985V | G | 3587 | T |
| TAGLN2 | R70C | G | 455 | A | TMEM151A | R256H | G | 911 | A | TSC2 | R901C | C | 3331 | T | USP9X | S722G | A | 2797 | G |
| TAGLN2 | N186H | T | 803 | G | TMEM151A | W289C | G | 1011 | T | TSC2 | V461M | G | 2011 | A | USP9X | D62N | G | 817 | A |
| TAGLN3 | A187V | C | 1013 | T | TMEM151A | T216M | C | 791 | T | TSC22D1 | T962A | T | 3375 | C | USP9X | R371Q | G | 1745 | A |
| TALDO1 | T167A | A | 652 | G | TMEM151B | R174C | C | 520 | T | TSC22D2 | D695E | T | 3101 | G | USP9X | K1156E | A | 4099 | G |
| TANC1 | I1286V | A | 4093 | G | TMEM151B | A533T | G | 1597 | A | TSC22D2 | F580S | T | 2755 | C | USP9Y | - | G | 0 | T |
| TANC1 | A113V | C | 575 | T | TMEM160 | A81T | C | 268 | T | TSEN15 | L135V | T | 482 | G | USPL1 | G831V | G | 2834 | T |
| TANC1 | A184T | G | 787 | A | TMEM161A | V92M | C | 307 | T | TSEN2 | W354C | G | 1217 | T | USPL1 | S23Y | C | 410 | A |
| TANC1 | V941I | G | 3058 | A | TMEM163 | A103T | C | 372 | T | TSEN2 | - | A | 1552 | C | USPL1 | Q743H | G | 2571 | T |
| TANC1 | E1225G | A | 3911 | G | TMEM163 | V228M | C | 747 | T | TSGA10 | R347K | C | 1885 | T | USPL1 | K901N | G | 3045 | T |
| TANC1 | R856H | G | 2804 | A | TMEM164 | G249C | G | 1081 | T | TSGA10 | R17W | G | 894 | A | UST | G130R | G | 491 | A |
| TANC1 | E1238K | G | 3949 | A | TMEM167B | I10T | T | 29 | C | TSGA10 | R330C | G | 1833 | A | UTP11L | R25Q | G | 135 | A |
| TANC2 | E811K | G | 2454 | A | TMEM168 | I381M | A | 1704 | C | TSGA10 | E523* | C | 2412 | A | UTP14A | G177D | G | 558 | A |
| TANC2 | M297V | A | 912 | G | TMEM168 | L44V | A | 691 | C | TSGA10 | R418* | G | 2097 | A | UTP14C | V93I | G | 1010 | A |
| TANC2 | H1089R | A | 3289 | G | TMEM17 | N23I | T | 280 | A | TSHB | M29V | A | 153 | G | UTP14C | E632K | G | 2627 | A |
| TANC2 | G1728R | G | 5205 | A | TMEM17 | E28D | C | 296 | A | TSHR | R701C | C | 2257 | T | UTP14C | E331* | G | 1724 | T |
| TANC2 | T1088A | A | 3285 | G | TMEM170A | V132I | C | 480 | T | TSHZ1 | K600N | G | 2342 | T | UTP15 | Y95H | T | 282 | C |
| TANC2 | L783F | C | 2370 | T | TMEM171 | R107C | C | 792 | T | TSHZ1 | R492H | G | 2017 | A | UTP15 | V230I | G | 687 | A |
| TANC2 | R879W | C | 2658 | T | TMEM173 | R310H | C | 1263 | T | TSHZ1 | P774L | C | 2863 | T | UTP18 | T15A | A | 100 | G |
| TANC2 | R1210Q | G | 3652 | A | TMEM175 | R335C | C | 1188 | T | TSHZ1 | S970L | C | 3451 | T | UTP18 | E374* | G | 1177 | T |
| TANK | D307N | G | 1124 | A | TMEM175 | S497P | T | 1674 | C | TSHZ2 | A222V | C | 1552 | T | UTP20 | R1998W | C | 6148 | T |
| TAOK1 | D418Y | G | 1446 | T | TMEM175 | A305V | C | 1099 | T | TSHZ2 | A222V | C | 1552 | T | UTP20 | A1852V | C | 5711 | T |
| TAOK2 | R1124C | C | 4115 | T | TMEM175 | - | T | 0 | G | TSHZ2 | E59A | A | 1063 | C | UTP20 | I1927M | A | 5937 | G |
| TAOK2 | A41V | C | 867 | T | TMEM175 | R335S | C | 1188 | A | TSHZ2 | S431L | C | 2179 | T | UTP20 | H1066Y | C | 3352 | T |
| TAOK3 | R581Q | C | 2233 | T | TMEM178 | R153Q | G | 514 | A | TSHZ2 | S519Y | C | 2443 | A | UTP20 | L254I | C | 916 | A |
| TAOK3 | A296V | G | 1378 | A | TMEM178 | A141T | G | 477 | A | TSHZ3 | R4K | C | 317 | T | UTP20 | L571R | T | 1868 | G |
| TARBP1 | V1493I | C | 4477 | T | TMEM180 | R159H | G | 695 | A | TSHZ3 | T361M | G | 1388 | A | UTP20 | T1354M | C | 4217 | T |
| TARBP1 | T937A | T | 2809 | C | TMEM180 | A123V | C | 587 | T | TSHZ3 | E715D | T | 2451 | A | UTP20 | F1479L | C | 4593 | A |
| TARBP1 | K1063N | T | 3189 | G | TMEM180 | A93V | C | 497 | T | TSHZ3 | E880Q | C | 2944 | G | UTP20 | E1571* | G | 4867 | T |
| TARBP2 | E191* | G | 1059 | T | TMEM182 | C136S | G | 612 | C | TSHZ3 | N785K | G | 2661 | T | UTP20 | R2378C | C | 7288 | T |
| TARDBP | L248S | T | 857 | C | TMEM184A | A234T | C | 1017 | T | TSHZ3 | G161D | C | 788 | T | UTP3 | R220Q | G | 858 | A |

| | | | | | | | | | | | | | | | | | | | |
|---|---|---|---|---|---|---|---|---|---|---|---|---|---|---|---|---|---|---|---|
| TARS | T316A | A | 1068 | G | TMEM186 | H209Q | A | 661 | C | TSKS | R503H | C | 1547 | T | UTP3 | D179G | A | 735 | G |
| TARS | E739K | G | 2337 | A | TMEM187 | R252I | G | 1502 | T | TSKS | H560N | G | 1717 | T | UTP3 | K387Q | A | 1358 | C |
| TARS | R671* | C | 2133 | T | TMEM192 | H118R | T | 499 | C | TSKU | A347D | C | 1212 | A | UTP6 | E258* | C | 857 | A |
| TARS2 | S349Y | C | 1080 | A | TMEM194A | G170V | C | 555 | A | TSNARE1 | V194G | A | 695 | C | UTP6 | R74H | C | 306 | T |
| TARS2 | T449I | C | 1380 | T | TMEM194A | F186V | A | 602 | C | TSNARE1 | A320T | C | 1072 | T | UTP6 | F425L | A | 1358 | G |
| TARS2 | R196Q | G | 621 | A | TMEM194B | A26E | G | 144 | T | TSNARE1 | A229T | C | 799 | T | UTRN | R1084Q | G | 3251 | A |
| TARS2 | R304C | C | 944 | T | TMEM195 | R68H | C | 373 | T | TSNARE1 | R196W | G | 700 | A | UTRN | R2532* | C | 7594 | T |
| TARSL2 | G689R | C | 2282 | T | TMEM199 | D305Y | G | 1548 | T | TSNARE1 | R166C | G | 610 | A | UTRN | E2847K | G | 8539 | A |
| TARSL2 | I774V | T | 2537 | C | TMEM199 | Y239C | A | 1351 | G | TSNAXIP1 | F53L | C | 204 | A | UTRN | Q832* | C | 2494 | T |
| TARSL2 | L137I | G | 626 | T | TMEM199 | A200V | C | 1234 | T | TSNAXIP1 | I199V | A | 640 | G | UTRN | D3407N | G | 1021 9 | A |
| TARSL2 | E133* | C | 614 | A | TMEM2 | A1342V | G | 4173 | A | TSNAXIP1 | R262W | C | 829 | T | UTRN | V235I | G | 703 | A |
| TAS1R1 | R187C | C | 752 | T | TMEM2 | F391L | G | 1321 | T | TSNAXIP1 | - | G | 0 | A | UTRN | D2933G | A | 8798 | G |
| TAS1R1 | V53A | T | 351 | C | TMEM2 | G991V | C | 3120 | A | TSNAXIP1 | V86A | T | 302 | C | UTRN | E1277* | G | 3829 | T |
| TAS1R2 | R473* | G | 1439 | A | TMEM20 | T153A | A | 518 | G | TSNAXIP1 | D214N | G | 685 | A | UTRN | T3218A | A | 9652 | G |
| TAS1R2 | P440L | G | 1341 | A | TMEM20 | K247Q | A | 800 | C | TSPAN10 | S107Y | C | 410 | A | UTRN | E1260D | G | 3780 | C |
| TAS1R2 | W341R | A | 1043 | G | TMEM200A | A202T | G | 1475 | A | TSPAN11 | R86Q | G | 370 | A | UTS2D | D60Y | C | 965 | A |
| TAS1R2 | R254C | G | 782 | A | TMEM200A | G109S | G | 1196 | A | TSPAN12 | E4* | C | 618 | A | UXS1 | I71T | A | 310 | G |
| TAS1R2 | P241H | G | 744 | T | TMEM200A | V112L | G | 1205 | C | TSPAN15 | N166T | A | 619 | C | VAC14 | R233C | G | 965 | A |
| TAS1R2 | R457Q | C | 1392 | T | TMEM200A | R43W | C | 998 | T | TSPAN17 | A266T | G | 796 | A | VAMP4 | D12N | C | 173 | T |
| TAS1R2 | R707H | C | 2142 | T | TMEM201 | C617Y | G | 1859 | A | TSPAN17 | A266T | G | 796 | A | VAMP5 | R30H | G | 172 | A |
| TAS1R2 | S86N | C | 279 | T | TMEM201 | R474H | G | 1430 | A | TSPAN18 | F116L | C | 504 | A | VANGL1 | R360S | G | 1351 | T |
| TAS2R1 | E74G | T | 540 | C | TMEM201 | V57L | G | 178 | C | TSPAN18 | E216K | G | 802 | A | VANGL2 | G142R | G | 898 | A |
| TAS2R10 | K285N | C | 855 | A | TMEM201 | C67R | T | 208 | C | TSPAN19 | E81K | C | 241 | T | VANGL2 | H430Y | C | 1762 | T |
| TAS2R10 | L129I | G | 385 | T | TMEM201 | R474C | C | 1429 | T | TSPAN2 | A213V | G | 670 | A | VANGL2 | R440Q | G | 1793 | A |
| TAS2R16 | T111A | T | 397 | C | TMEM202 | D200Y | G | 598 | T | TSPAN2 | R118* | G | 384 | A | VAPA | - | T | 0 | C |
| TAS2R16 | F68V | A | 268 | C | TMEM203 | E76D | C | 452 | A | TSPAN2 | - | C | 0 | T | VASH1 | F197L | C | 1521 | A |
| TAS2R20 | M181V | T | 541 | C | TMEM204 | A9V | C | 645 | T | TSPAN32 | R316W | C | 1083 | T | VASH1 | V270M | G | 1738 | A |
| TAS2R3 | E70* | G | 270 | T | TMEM205 | H44P | T | 438 | G | TSPAN5 | D190Y | C | 971 | A | VASH1 | T309A | A | 1855 | G |
| TAS2R38 | D324N | C | 1054 | T | TMEM206 | P144L | G | 569 | A | TSPAN7 | - | T | 0 | C | VASH1 | F60L | C | 1110 | A |
| TAS2R39 | H172Q | C | 516 | G | TMEM206 | R110H | C | 467 | T | TSPAN8 | N37H | T | 662 | G | VASH2 | T106M | C | 627 | T |
| TAS2R40 | N125T | A | 416 | C | TMEM206 | E49K | C | 283 | T | TSPAN9 | A220V | C | 806 | T | VASH2 | V208I | G | 932 | A |
| TAS2R50 | L182I | G | 544 | T | TMEM209 | R427* | G | 1402 | A | TSPAN9 | R146* | C | 583 | T | VASH2 | R265W | C | 1103 | T |
| TAS2R60 | R310H | G | 929 | A | TMEM209 | A315T | C | 1066 | T | TSPYL2 | - | G | 0 | A | VASH2 | E286D | G | 1168 | T |
| TAS2R60 | Y167H | T | 499 | C | TMEM212 | Y117H | T | 384 | C | TSPYL2 | R289C | C | 997 | T | VASN | G467C | G | 1554 | T |
| TAT | C241Y | C | 856 | T | TMEM212 | E142K | G | 459 | A | TSPYL6 | R332P | C | 1070 | G | VASP | R154C | C | 802 | T |
| TAT | - | A | 0 | C | TMEM214 | R611H | G | 1894 | A | TSPYL6 | G194R | C | 655 | T | VASP | G115E | G | 686 | A |
| TAT | S28N | C | 217 | T | TMEM214 | K93T | A | 340 | C | TSR1 | V111A | A | 1289 | G | VAT1 | Q153* | G | 577 | A |
| TATDN1 | R167W | G | 525 | A | TMEM214 | L118R | T | 415 | G | TSR1 | E487D | T | 2418 | G | VAT1L | F291C | T | 969 | G |
| TATDN2 | Y759H | T | 3326 | C | TMEM215 | F197L | C | 956 | A | TSR2 | R13W | C | 58 | T | VAT1L | P268S | C | 899 | T |

| | | | | | | | | | | | | | | | | | | | |
|---|---|---|---|---|---|---|---|---|---|---|---|---|---|---|---|---|---|---|---|
| TATDN3 | A185T | G | 588 | A | TMEM216 | R54H | G | 597 | A | TSSC1 | R218Q | C | 794 | T | VAT1L | R414W | C | 1337 | T |
| TAX1BP1 | R704Q | G | 2246 | A | TMEM217 | R168Q | C | 543 | T | TSSC4 | E172K | G | 1005 | A | VAV1 | W671* | G | 2110 | A |
| TAX1BP3 | V12M | C | 190 | T | TMEM219 | M174L | A | 657 | C | TSSK1B | D97N | C | 439 | T | VAV1 | L258P | T | 870 | C |
| TAZ | K153E | A | 457 | G | TMEM22 | R102Q | G | 933 | A | TSSK1B | R9Q | C | 176 | T | VAV1 | V370A | T | 1206 | C |
| TBC1D1 | Y625C | A | 2229 | G | TMEM22 | W252* | G | 1383 | A | TSSK1B | R135W | G | 553 | A | VAV2 | G605W | C | 1845 | A |
| TBC1D1 | T632A | A | 2249 | G | TMEM22 | V148L | G | 1070 | T | TSSK1B | R168Q | C | 653 | T | VAV2 | F792C | A | 2407 | C |
| TBC1D1 | L104P | T | 666 | C | TMEM220 | D27Y | C | 557 | A | TSSK2 | V124I | G | 962 | A | VAV3 | L11F | G | 125 | A |
| TBC1D1 | K314N | A | 1297 | C | TMEM222 | R84W | C | 288 | T | TSSK2 | A24T | G | 662 | A | VAV3 | T80M | G | 333 | A |
| TBC1D10A | A465V | G | 1414 | A | TMEM229A | N116D | T | 812 | C | TSSK3 | R63C | C | 692 | T | VAV3 | G563D | C | 1782 | T |
| TBC1D10A | Q451* | G | 1371 | A | TMEM229B | R143H | C | 660 | T | TST | A56T | C | 901 | T | VAV3 | V625I | C | 1967 | T |
| TBC1D10A | Q129* | G | 405 | A | TMEM231 | Y84S | T | 290 | G | TSTA3 | Y161C | T | 1274 | C | VAV3 | A52V | G | 249 | A |
| TBC1D10B | L497I | G | 1570 | T | TMEM231 | K80E | T | 277 | C | TSTD1 | E93D | T | 314 | G | VAV3 | K5E | T | 107 | C |
| TBC1D10B | R728Q | C | 2264 | T | TMEM232 | A480T | C | 1489 | T | TTBK1 | A1010T | G | 3111 | A | VAX1 | G94D | C | 281 | T |
| TBC1D10B | T521M | G | 1643 | A | TMEM232 | R476* | G | 1477 | A | TTBK1 | D116N | G | 429 | A | VAX1 | P47L | G | 140 | A |
| TBC1D12 | R416H | G | 1357 | A | TMEM232 | K393Q | T | 1228 | G | TTBK2 | E1163* | C | 3985 | A | VAX2 | E263* | G | 819 | T |
| TBC1D12 | R625H | G | 1984 | A | TMEM232 | K73R | T | 269 | C | TTBK2 | R1578W | G | 5230 | A | VCAM1 | S568Y | C | 1804 | A |
| TBC1D12 | S592P | T | 1884 | C | TMEM232 | E66* | C | 247 | A | TTC12 | Y143C | A | 479 | G | VCAM1 | I351K | T | 1153 | A |
| TBC1D13 | R128Q | G | 533 | A | TMEM25 | A219V | C | 709 | T | TTC13 | E172G | T | 523 | C | VCAM1 | M571V | A | 1812 | G |
| TBC1D14 | P76L | C | 306 | T | TMEM25 | P234S | C | 753 | T | TTC13 | L738I | G | 2220 | T | VCAM1 | K604Q | A | 1911 | C |
| TBC1D14 | Y46* | C | 217 | A | TMEM26 | A351T | C | 1420 | T | TTC15 | A264P | G | 959 | C | VCAN | - | G | 0 | A |
| TBC1D14 | P324T | C | 1049 | A | TMEM26 | K292N | C | 1245 | A | TTC16 | S656L | C | 2047 | T | VCAN | E2002* | G | 6569 | T |
| TBC1D14 | F607L | T | 1898 | C | TMEM27 | N209H | T | 881 | G | TTC17 | V754I | G | 2274 | A | VCAN | S2864Y | C | 9156 | A |
| TBC1D15 | S519F | C | 1620 | T | TMEM30A | N163S | T | 635 | C | TTC17 | A1064V | C | 3205 | T | VCAN | E3211K | G | 1019<br>6 | A |
| TBC1D15 | - | T | 0 | C | TMEM30A | E184A | T | 698 | G | TTC17 | L499P | T | 1510 | C | VCAN | S1158R | T | 4039 | A |
| TBC1D15 | C514Y | G | 1605 | A | TMEM33 | S197L | C | 955 | T | TTC18 | R1029I | C | 3398 | A | VCAN | A360T | G | 1643 | A |
| TBC1D15 | K353E | A | 1121 | G | TMEM38A | A53T | G | 248 | A | TTC18 | S440N | C | 1631 | T | VCAN | A1519S | G | 5120 | T |
| TBC1D16 | R684W | G | 2166 | A | TMEM38B | E71G | A | 329 | G | TTC18 | S781G | T | 2653 | C | VCAN | P348S | C | 1607 | T |
| TBC1D16 | V220M | C | 774 | T | TMEM38B | K256N | G | 885 | T | TTC18 | S438Y | G | 1625 | T | VCAN | A2982V | C | 9510 | T |
| TBC1D17 | L136P | T | 559 | C | TMEM40 | Y210C | T | 750 | C | TTC18 | D430Y | C | 1600 | A | VCAN | Y3111C | A | 9897 | G |
| TBC1D17 | P178L | C | 685 | T | TMEM41A | D47N | C | 235 | T | TTC19 | V104F | G | 328 | T | VCAN | - | A | 1075<br>6 | G |
| TBC1D19 | H287R | A | 1138 | G | TMEM41A | L173H | A | 614 | T | TTC21A | T266A | A | 973 | G | VCAN | R327C | C | 1544 | T |
| TBC1D2 | A345V | G | 1126 | A | TMEM41A | L8I | G | 118 | T | TTC21A | R1187H | G | 3737 | A | VCAN | E1167* | G | 4064 | T |
| TBC1D2 | V788I | C | 2454 | T | TMEM42 | W130G | T | 444 | G | TTC21B | E233D | C | 837 | A | VCAN | S1444L | C | 4896 | T |
| TBC1D2 | A666V | G | 2089 | A | TMEM43 | R96W | C | 540 | T | TTC21B | R1050W | G | 3286 | A | VCAN | K1723N | G | 5734 | T |
| TBC1D2 | R697L | C | 2182 | A | TMEM43 | P373L | C | 1372 | T | TTC23 | A64T | C | 1024 | T | VCAN | E2261* | G | 7346 | T |
| TBC1D20 | L194* | A | 581 | C | TMEM43 | T357I | C | 1324 | T | TTC24 | R31W | C | 91 | T | VCAN | N2281S | A | 7407 | G |
| TBC1D22A | R286H | G | 1023 | A | TMEM44 | P208L | G | 829 | A | TTC24 | D388E | T | 1164 | G | VCAN | S2630Y | C | 8454 | A |
| TBC1D22A | N15I | A | 210 | T | TMEM45A | E186* | G | 1176 | T | TTC24 | - | A | 1749 | G | VCL | A1037D | C | 3204 | A |

| | | | | | | | | | | | | | | | | | | | |
|---|---|---|---|---|---|---|---|---|---|---|---|---|---|---|---|---|---|---|---|
| TBC1D22B | R108I | G | 469 | T | TMEM45B | R44C | C | 191 | T | TTC25 | E648* | G | 2021 | T | VCL | A314T | G | 1034 | A |
| TBC1D25 | R466* | C | 1737 | T | TMEM45B | Y29H | T | 146 | C | TTC25 | - | T | 0 | C | VCL | D810N | G | 2522 | A |
| TBC1D25 | R649H | G | 2287 | A | TMEM45B | I181V | A | 602 | G | TTC25 | K636N | G | 1987 | T | VCP | F575L | G | 2621 | T |
| TBC1D25 | W463C | G | 1730 | T | TMEM45B | F95C | T | 345 | G | TTC27 | Q411R | A | 1463 | G | VCP | E397D | T | 2087 | G |
| TBC1D25 | A491V | C | 1813 | T | TMEM47 | A30V | G | 348 | A | TTC27 | R659H | G | 2207 | A | VCPIP1 | R785* | G | 2642 | A |
| TBC1D26 | K127E | A | 791 | G | TMEM48 | E409* | C | 1824 | A | TTC28 | A1608V | G | 4965 | A | VCPIP1 | F924L | G | 3061 | T |
| TBC1D28 | R109Q | C | 1026 | T | TMEM48 | L382I | G | 1743 | T | TTC28 | A2213V | G | 6780 | A | VCPIP1 | L840* | A | 2808 | C |
| TBC1D2B | L543P | A | 1628 | G | TMEM49 | I324T | T | 1244 | C | TTC28 | S1462N | C | 4527 | T | VDAC1 | E36D | C | 353 | A |
| TBC1D4 | L968I | G | 3249 | T | TMEM49 | K250Q | A | 1021 | C | TTC28 | G1377R | C | 4271 | T | VDAC2 | G298C | G | 1141 | T |
| TBC1D4 | T448M | G | 1690 | A | TMEM51 | H109R | A | 772 | G | TTC28 | S1596L | G | 4929 | A | VDAC2 | - | G | 0 | A |
| TBC1D4 | M1023T | A | 3415 | G | TMEM52 | R95W | G | 291 | A | TTC28 | R1207* | G | 3761 | A | VDAC3 | K111N | G | 476 | T |
| TBC1D4 | - | A | 0 | G | TMEM53 | R165Q | C | 658 | T | TTC28 | Q746H | C | 2380 | A | VDR | R358C | G | 1232 | A |
| TBC1D4 | R293W | G | 1224 | A | TMEM55A | R59H | C | 402 | T | TTC28 | N955D | T | 3005 | C | VDR | R154W | G | 620 | A |
| TBC1D4 | S1000Y | G | 3346 | T | TMEM55A | P11H | G | 258 | T | TTC28 | S2153N | C | 6600 | T | VDR | T365M | G | 1254 | A |
| TBC1D4 | Q850H | T | 2897 | G | TMEM55A | R219Q | C | 882 | T | TTC28 | E356* | C | 1208 | A | VEGFB | G30D | G | 129 | A |
| TBC1D4 | R637* | G | 2256 | A | TMEM55B | R131* | G | 499 | A | TTC28 | M103I | C | 451 | A | VEGFC | E333* | C | 1413 | A |
| TBC1D4 | I171F | T | 858 | A | TMEM57 | A338T | G | 1191 | A | TTC29 | K365T | T | 1294 | G | VEGFC | N251S | T | 1168 | C |
| TBC1D4 | A710T | C | 2475 | T | TMEM57 | A382T | G | 1323 | A | TTC29 | E275* | C | 1023 | A | VENTX | C184Y | G | 562 | A |
| TBC1D5 | R706* | G | 2380 | A | TMEM57 | V110A | T | 508 | C | TTC29 | A124V | G | 571 | A | VENTX | S160L | C | 490 | T |
| TBC1D5 | I667S | A | 2264 | C | TMEM57 | L197F | C | 768 | T | TTC29 | R249C | G | 945 | A | VEPH1 | T103A | T | 615 | C |
| TBC1D7 | R20C | G | 142 | A | TMEM59 | L19P | A | 69 | G | TTC29 | E163K | C | 687 | T | VEPH1 | E789K | C | 2673 | T |
| TBC1D8 | K1136M | T | 3538 | A | TMEM59 | R102* | G | 317 | A | TTC29 | - | C | 0 | T | VEPH1 | L221F | G | 969 | A |
| TBC1D8 | R319Q | C | 1087 | T | TMEM59L | A129V | C | 473 | T | TTC30A | S41I | C | 387 | A | VEZF1 | R91C | G | 414 | A |
| TBC1D8 | S1002I | C | 3136 | A | TMEM59L | R82C | C | 331 | T | TTC30B | L194M | G | 831 | T | VGF | S573L | G | 1958 | A |
| TBC1D8 | R619W | G | 1986 | A | TMEM62 | G27D | G | 359 | A | TTC31 | L109I | C | 332 | A | VGLL1 | Q174H | A | 692 | C |
| TBC1D8B | Y188C | A | 737 | G | TMEM62 | V617A | T | 2129 | C | TTC32 | E92K | C | 406 | T | VGLL3 | R143W | G | 791 | A |
| TBC1D8B | N729T | A | 2360 | C | TMEM63A | G415D | C | 1515 | T | TTC33 | V225A | A | 827 | G | VGLL3 | A79D | G | 600 | T |
| TBC1D9 | T113S | T | 412 | A | TMEM63A | S799T | C | 2667 | G | TTC35 | K127T | A | 415 | C | VGLL4 | S238P | A | 1118 | G |
| TBC1D9 | V1094M | C | 3355 | T | TMEM63A | Y752H | A | 2525 | G | TTC37 | A617T | C | 2148 | T | VGLL4 | R104W | G | 716 | A |
| TBC1D9 | - | C | 0 | A | TMEM63A | R92K | C | 546 | T | TTC37 | G797D | C | 2689 | T | VHL | G29D | G | 926 | A |
| TBC1D9B | S601L | G | 1837 | A | TMEM63B | R542C | C | 1762 | T | TTC37 | A427V | G | 1579 | A | VHL | R205H | G | 1454 | A |
| TBC1D9B | G1189S | C | 3600 | T | TMEM63B | A622T | G | 2002 | A | TTC38 | S216L | C | 723 | T | VHL | L201P | T | 1442 | C |
| TBC1D9B | V706A | A | 2152 | G | TMEM63B | V220I | G | 796 | A | TTC39A | A376T | C | 1175 | T | VHL | G127V | G | 1220 | T |
| TBC1D9B | P852S | G | 2589 | A | TMEM63C | R636H | G | 2119 | A | TTC39B | R553I | C | 1695 | A | VILL | I43T | T | 394 | C |
| TBC1D9B | T646M | G | 1972 | A | TMEM63C | A543T | G | 1839 | A | TTC39C | C446S | G | 1789 | C | VILL | R741W | C | 2487 | T |
| TBC1D9B | A536T | C | 1641 | T | TMEM63C | R60W | C | 390 | T | TTC3L | R1970C | C | 6013 | T | VIPAR | R407* | G | 1219 | A |
| TBCB | G171* | G | 1086 | T | TMEM63C | E94D | G | 494 | T | TTC3L | N121H | A | 466 | C | VIPR1 | E453K | G | 1470 | A |
| TBCC | F104S | A | 334 | G | TMEM63C | I443V | A | 1539 | G | TTC3L | R246I | G | 842 | T | VIPR1 | S431N | G | 1405 | A |
| TBCCD1 | R539H | C | 2096 | T | TMEM65 | A174T | C | 1062 | T | TTC3L | R277C | C | 934 | T | VIPR1 | G28D | G | 196 | A |

| | | | | | | | | | | | | | | | | | | | |
|---|---|---|---|---|---|---|---|---|---|---|---|---|---|---|---|---|---|---|---|
| TBCCD1 | E88K | C | 742 | T | TMEM67 | R549C | C | 1658 | T | TTC3L | V1461M | G | 4486 | A | VIPR1 | H171R | A | 625 | G |
| TBCCD1 | A303T | C | 1387 | T | TMEM67 | K594R | A | 1794 | G | TTC3L | L1118S | T | 3458 | C | VIPR2 | F358L | G | 1162 | T |
| TBCCD1 | C384* | G | 1632 | T | TMEM67 | S116Y | C | 360 | A | TTC3L | V344I | G | 1135 | A | VIT | S272* | C | 1117 | A |
| TBCCD1 | T401M | G | 1682 | A | TMEM67 | F563L | C | 1702 | A | TTC3L | S1749P | T | 5350 | C | VIT | T685A | A | 2355 | G |
| TBCD | - | G | 0 | A | TMEM67 | F637L | C | 1924 | A | TTC3L | - | A | 0 | G | VLDLR | N364H | A | 1446 | C |
| TBCD | A751T | G | 2381 | A | TMEM67 | R757I | G | 2283 | T | TTC3L | E159D | A | 582 | C | VLDLR | R448Q | G | 1699 | A |
| TBCD | V794I | G | 2510 | A | TMEM67 | H790N | C | 2381 | A | TTC7A | D87Y | G | 515 | T | VLDLR | D187E | C | 917 | G |
| TBCE | R386Q | G | 1333 | A | TMEM70 | I95M | T | 372 | G | TTC7A | A288V | C | 1119 | T | VLDLR | A476V | C | 1783 | T |
| TBCEL | F318L | C | 1032 | G | TMEM71 | D83N | C | 476 | T | TTC7A | R163W | C | 743 | T | VLDLR | G604R | G | 2166 | A |
| TBCK | K742T | T | 2673 | G | TMEM71 | D155G | T | 693 | C | TTC7A | T520M | C | 1815 | T | VLDLR | G54E | G | 517 | A |
| TBCK | R41C | G | 569 | A | TMEM71 | D83N | C | 476 | T | TTC7A | A868V | C | 2859 | T | VLDLR | I769M | T | 2663 | G |
| TBK1 | Q289R | A | 965 | G | TMEM72 | Y149C | A | 759 | G | TTC7B | R750W | G | 2370 | A | VN1R1 | R81K | C | 242 | T |
| TBK1 | R440* | C | 1417 | T | TMEM79 | A207V | C | 781 | T | TTC7B | E143Q | C | 549 | G | VN1R2 | C299Y | G | 980 | A |
| TBK1 | R724C | C | 2269 | T | TMEM80 | R118H | G | 353 | A | TTC7B | Q670H | C | 2132 | A | VN1R2 | L128I | C | 466 | A |
| TBKBP1 | E255D | G | 1614 | T | TMEM81 | T76A | T | 366 | C | TTC7B | Q641* | G | 2043 | A | VN1R2 | S344F | C | 1115 | T |
| TBL1X | H359Y | C | 1443 | T | TMEM82 | L159M | C | 613 | A | TTC8 | Y53N | T | 231 | A | VN1R4 | W299* | C | 897 | T |
| TBL1X | V511I | G | 1899 | A | TMEM82 | A160T | G | 616 | A | TTC8 | A346T | G | 1110 | A | VNN1 | A351T | C | 1065 | T |
| TBL1XR1 | E351* | C | 1311 | A | TMEM86A | V147M | G | 522 | A | TTC8 | K460N | G | 1454 | T | VNN2 | D81Y | C | 252 | A |
| TBL1XR1 | T396A | T | 1446 | C | TMEM87B | T187M | C | 929 | T | TTC8 | V463I | G | 1461 | A | VNN2 | - | C | 0 | A |
| TBL1XR1 | S447G | T | 1599 | C | TMEM87B | Y208H | T | 991 | C | TTF1 | F527C | A | 1620 | C | VPRBP | R177Q | C | 530 | T |
| TBL1XR1 | K335Q | T | 1263 | G | TMEM87B | D119N | G | 724 | A | TTF1 | N615S | T | 1884 | C | VPRBP | L1505P | A | 4514 | G |
| TBL2 | K446N | C | 1580 | A | TMEM87B | Y241S | A | 1091 | C | TTF2 | S883G | A | 2691 | G | VPRBP | V1242I | C | 3724 | T |
| TBL2 | L82M | G | 486 | T | TMEM8A | M474I | C | 1583 | T | TTF2 | R733Q | G | 2242 | A | VPRBP | E1439D | C | 4317 | G |
| TBL2 | R33C | G | 339 | A | TMEM8A | T357M | G | 1231 | A | TTF2 | K871Q | A | 2655 | C | VPRBP | A906T | C | 2716 | T |
| TBL3 | R322C | C | 1092 | T | TMEM8B | R377H | G | 2145 | A | TTK | T458A | A | 1483 | G | VPRBP | K1020N | C | 3060 | A |
| TBL3 | R708H | G | 2251 | A | TMEM8B | R428C | C | 2297 | T | TTK | V208A | T | 734 | C | VPREB1 | R38H | G | 252 | A |
| TBL3 | R148* | C | 570 | T | TMEM8B | R364C | C | 2105 | T | TTLL1 | D196G | T | 828 | C | VPREB1 | S73L | C | 357 | T |
| TBL3 | S54L | C | 289 | T | TMEM8C | R62C | G | 286 | A | TTLL10 | T351M | C | 1225 | T | VPREB3 | D87N | C | 358 | T |
| TBR1 | S22N | G | 382 | A | TMEM90A | G100V | C | 547 | A | TTLL10 | L213R | T | 811 | G | VPS11 | Y497H | T | 1489 | C |
| TBRG4 | T156I | G | 516 | A | TMEM90A | L162P | A | 733 | G | TTLL11 | V80A | A | 427 | G | VPS11 | G231D | G | 692 | A |
| TBX1 | R171Q | G | 641 | A | TMEM90B | V246M | G | 1369 | A | TTLL11 | W242C | C | 914 | A | VPS13A | D2745Y | G | 8493 | T |
| TBX1 | Y195C | A | 713 | G | TMEM91 | A156V | C | 1127 | T | TTLL11 | A611T | C | 2019 | T | VPS13A | S978N | G | 3193 | A |
| TBX15 | R404Q | C | 1221 | T | TMEM92 | V60I | G | 288 | A | TTLL12 | R252Q | C | 819 | T | VPS13A | N1338D | A | 4272 | G |
| TBX15 | G343D | C | 1038 | T | TMEM97 | P134L | C | 506 | T | TTLL13 | R439W | C | 1605 | T | VPS13A | L555M | C | 1923 | A |
| TBX15 | R172I | C | 525 | A | TMF1 | P70H | G | 426 | T | TTLL13 | K46N | G | 428 | T | VPS13A | V1716I | G | 5406 | A |
| TBX18 | A421G | G | 1600 | C | TMF1 | N969H | T | 3122 | G | TTLL2 | S459L | C | 1464 | T | VPS13A | A2748V | C | 8503 | T |
| TBX18 | P9L | G | 364 | A | TMF1 | E914* | C | 2957 | A | TTLL4 | - | G | 0 | T | VPS13A | F1565S | T | 4954 | C |
| TBX19 | A430T | G | 1339 | A | TMF1 | E695* | C | 2300 | A | TTLL4 | G624E | G | 2211 | A | VPS13A | I591L | A | 2031 | C |
| TBX19 | N59D | A | 226 | G | TMF1 | R398Q | C | 1410 | T | TTLL4 | R1025Q | G | 3414 | A | VPS13B | K254E | A | 871 | G |

| | | | | | | | | | | | | | | | | | | | |
|---|---|---|---|---|---|---|---|---|---|---|---|---|---|---|---|---|---|---|---|
| TBX2 | R436C | C | 1587 | T | TMF1 | R325I | C | 1191 | A | TTLL5 | R71* | C | 416 | T | VPS13B | R3741W | C | 11332 | T |
| TBX20 | R315C | G | 1470 | A | TMIGD2 | R35M | C | 150 | A | TTLL5 | Q1136* | C | 3611 | T | VPS13B | L1166I | C | 3607 | A |
| TBX21 | E396K | G | 1397 | A | TMIGD2 | K58E | T | 218 | C | TTLL5 | D47N | G | 344 | A | VPS13B | N204T | A | 722 | C |
| TBX21 | G202S | G | 815 | A | TMLHE | R70H | C | 355 | T | TTLL6 | Q58H | C | 209 | A | VPS13B | E288* | G | 973 | T |
| TBX21 | A481S | G | 1652 | T | TMOD1 | N279S | A | 1049 | G | TTLL6 | - | C | 0 | A | VPS13B | D361N | G | 1192 | A |
| TBX21 | S511I | G | 1743 | T | TMOD4 | R340H | C | 1154 | T | TTLL7 | R619H | C | 2234 | T | VPS13B | E792* | G | 2485 | T |
| TBX3 | A664V | G | 2955 | A | TMOD4 | V305M | C | 1048 | T | TTLL7 | T382A | T | 1522 | C | VPS13B | L2257I | C | 6880 | A |
| TBX3 | P579S | G | 2699 | A | TMPO | D14Y | G | 244 | T | TTLL7 | R669Q | C | 2384 | T | VPS13B | F2936L | C | 8919 | A |
| TBX3 | A472V | G | 2379 | A | TMPO | E406D | A | 1422 | C | TTLL7 | D503Y | C | 1885 | A | VPS13B | I3016S | T | 9158 | G |
| TBX4 | D375N | G | 1168 | A | TMPO | P280S | C | 1079 | T | TTLL7 | E231D | C | 1071 | A | VPS13C | T2261M | G | 6856 | A |
| TBX4 | Y427C | A | 1325 | G | TMPRSS11A | T80M | G | 986 | A | TTLL7 | N73T | T | 596 | G | VPS13C | T2256M | G | 6841 | A |
| TBX4 | R496C | C | 1531 | T | TMPRSS11A | Y364H | A | 1837 | G | TTLL7 | R152Q | C | 833 | T | VPS13C | M2014T | A | 6115 | G |
| TBX5 | P307T | G | 1586 | T | TMPRSS11A | A32T | C | 841 | T | TTLL8 | R431Q | C | 1292 | T | VPS13C | R3388Q | C | 10237 | T |
| TBX5 | K325M | T | 1641 | A | TMPRSS11A | Q293* | G | 1624 | A | TTLL9 | A342V | C | 1278 | T | VPS13C | K2310Q | T | 7002 | G |
| TBX6 | A423T | C | 1327 | T | TMPRSS11A | T34A | T | 847 | C | TTN | I28650F | T | 86173 | A | VPS13D | R2621H | G | 7992 | A |
| TCAP | F21L | C | 1236 | A | TMPRSS11B | G206D | C | 779 | T | TTN | A25142T | C | 75649 | T | VPS13D | E187V | A | 690 | T |
| TCEA1 | R132L | C | 718 | A | TMPRSS11B | G23E | C | 230 | T | TTN | T7172A | T | 21739 | C | VPS13D | L2118P | T | 6483 | C |
| TCEA1 | E88K | C | 585 | T | TMPRSS11B | A176S | C | 688 | A | TTN | T4942M | G | 15050 | A | VPS13D | R3253* | C | 9887 | T |
| TCEA2 | A12T | G | 203 | A | TMPRSS11D | V276G | A | 926 | C | TTN | D3897G | T | 11915 | C | VPS13D | R4231H | G | 12822 | A |
| TCEAL2 | R218Q | G | 872 | A | TMPRSS11E | S254L | C | 825 | T | TTN | Y1764C | T | 5516 | C | VPS13D | R694C | C | 2210 | T |
| TCEAL3 | K123N | G | 727 | T | TMPRSS11E | R330* | C | 1052 | T | TTN | F30298L | G | 91119 | T | VPS13D | P1695H | C | 5214 | A |
| TCEAL4 | L202V | T | 843 | G | TMPRSS11F | A149D | G | 506 | T | TTN | P26987S | G | 81184 | A | VPS13D | E2437K | G | 7439 | A |
| TCEAL7 | E47* | G | 393 | T | TMPRSS11F | S202Y | G | 665 | T | TTN | L20402I | G | 61429 | T | VPS13D | E578* | G | 1862 | T |
| TCEANC | E62A | A | 430 | C | TMPRSS12 | A59V | C | 223 | T | TTN | E15863K | C | 47812 | T | VPS13D | S1663Y | C | 5118 | A |
| TCEB3 | E132A | A | 666 | C | TMPRSS13 | R272Q | C | 815 | T | TTN | S15320G | T | 46183 | C | VPS13D | F2325C | T | 7104 | G |
| TCEB3B | N314D | T | 1297 | C | TMPRSS13 | G535D | C | 1604 | T | TTN | L14685M | G | 44278 | T | VPS16 | C3Y | G | 80 | A |
| TCEB3B | R530H | C | 1946 | T | TMPRSS13 | L342P | A | 1025 | G | TTN | T11982N | G | 36170 | T | VPS16 | R544H | G | 1703 | A |

| | | | | | | | | | | | | | | | | | | | |
|---|---|---|---|---|---|---|---|---|---|---|---|---|---|---|---|---|---|---|---|
| TCERG1 | D144N | G | 468 | A | TMPRSS13 | R132Q | C | 395 | T | TTN | E5276* | C | 16051 | A | VPS18 | R590H | G | 2109 | A |
| TCERG1 | R153W | C | 495 | T | TMPRSS15 | A60V | G | 213 | A | TTN | A4832T | C | 14719 | T | VPS18 | R48H | G | 483 | A |
| TCERG1 | L671P | T | 2050 | C | TMPRSS15 | F984C | A | 2985 | C | TTN | R3505* | G | 10738 | A | VPS18 | R301C | C | 1241 | T |
| TCERG1 | R693W | C | 2115 | T | TMPRSS15 | F123C | A | 402 | C | TTN | R31176 C | G | 93751 | A | VPS24 | E16K | C | 46 | T |
| TCERG1 | V256I | G | 804 | A | TMPRSS2 | A386G | G | 1292 | C | TTN | S30811 T | A | 92656 | T | VPS24 | S24G | T | 70 | C |
| TCERG1 | V675M | G | 2061 | A | TMPRSS2 | V275I | C | 958 | T | TTN | G5585S | C | 16978 | T | VPS26A | I195M | T | 1238 | G |
| TCERG1L | P221L | G | 748 | A | TMPRSS2 | K449N | C | 1482 | A | TTN | H2732Y | G | 8419 | A | VPS26B | R283H | G | 1304 | A |
| TCERG1L | A246T | C | 822 | T | TMPRSS3 | D418N | C | 1956 | T | TTN | Y2009* | A | 6252 | C | VPS26B | D146G | A | 893 | G |
| TCERG1L | R458* | G | 1458 | A | TMPRSS4 | R20C | C | 349 | T | TTN | A848V | G | 2768 | A | VPS29 | K76R | T | 227 | C |
| TCERG1L | Q220H | C | 746 | A | TMPRSS5 | H136R | T | 556 | C | TTN | A427V | G | 1505 | A | VPS33A | L433I | G | 1410 | T |
| TCERG1L | T350M | G | 1135 | A | TMPRSS5 | R130C | G | 537 | A | TTN | S26537 R | T | 79834 | G | VPS33A | R505W | G | 1626 | A |
| TCERG1L | L527I | G | 1665 | T | TMPRSS5 | D363G | T | 1237 | C | TTN | R24459 G | T | 73600 | C | VPS33B | Y121D | A | 715 | C |
| TCF12 | G444* | G | 1614 | T | TMPRSS6 | R802Q | C | 2546 | T | TTN | R22482 C | G | 67669 | A | VPS35 | E171* | C | 570 | A |
| TCF12 | R603Q | G | 2092 | A | TMPRSS6 | Q743H | C | 2370 | A | TTN | R16839 H | C | 50741 | T | VPS36 | V255M | C | 791 | T |
| TCF12 | R603W | C | 2091 | T | TMPRSS6 | R103C | G | 448 | A | TTN | R28672 H | C | 86240 | T | VPS36 | L108P | A | 351 | G |
| TCF12 | Q179* | C | 819 | T | TMPRSS6 | G690D | C | 2210 | T | TTN | P19409 L | G | 58451 | A | VPS36 | K34Q | T | 128 | G |
| TCF12 | R603Q | G | 2092 | A | TMPRSS6 | D512N | C | 1675 | T | TTN | G13785 R | C | 41578 | T | VPS37A | P203S | C | 960 | T |
| TCF12 | R90* | C | 552 | T | TMPRSS7 | R102C | C | 304 | T | TTN | P12822 L | G | 38690 | A | VPS37A | F123I | T | 720 | A |
| TCF12 | K181N | A | 827 | C | TMPRSS7 | R713H | G | 2138 | A | TTN | S14405 G | T | 43438 | C | VPS37D | A97T | G | 423 | A |
| TCF12 | R611C | C | 2115 | T | TMPRSS7 | T385A | A | 1153 | G | TTN | A1021V | G | 3287 | A | VPS39 | H2Y | G | 155 | A |
| TCF15 | E25G | T | 235 | C | TMPRSS7 | Q543R | A | 1628 | G | TTN | R139W | G | 640 | A | VPS39 | M634V | T | 2051 | C |
| TCF20 | A1681V | G | 5179 | A | TMPRSS7 | R785Q | G | 2354 | A | TTN | T24383 M | G | 73373 | A | VPS39 | A171V | G | 663 | A |
| TCF20 | A1292T | C | 4011 | T | TMPRSS9 | G630* | G | 1888 | T | TTN | R21486 H | C | 64682 | T | VPS41 | N836D | T | 2561 | C |
| TCF20 | D1728Y | C | 5319 | A | TMPRSS9 | R900C | C | 2698 | T | TTN | E11410 G | T | 34454 | C | VPS41 | R611C | G | 1886 | A |
| TCF21 | G143R | G | 687 | A | TMPRSS9 | A359D | C | 1076 | A | TTN | V24067 F | C | 72424 | A | VPS41 | E788G | T | 2418 | C |
| TCF25 | A514T | G | 1646 | A | TMSB10 | K19N | G | 166 | T | TTN | K12741 T | T | 38447 | G | VPS41 | R757C | G | 2324 | A |
| TCF25 | D33E | C | 205 | G | TMTC1 | R441W | G | 1795 | A | TTN | R12428 C | G | 37507 | A | VPS41 | M663V | T | 2042 | C |

| | | | | | | | | | | | | | | | | | | | |
|---|---|---|---|---|---|---|---|---|---|---|---|---|---|---|---|---|---|---|---|
| TCF25 | L354P | T | 1167 | C | TMTC2 | R29C | C | 517 | T | TTN | D32581 G | T | 97967 | C | VPS41 | R208W | G | 677 | A |
| TCF3 | R563W | G | 1754 | A | TMTC2 | K378T | A | 1565 | C | TTN | G28117 D | C | 84575 | T | VPS45 | - | T | 0 | C |
| TCF4 | A766T | C | 2353 | T | TMTC3 | A756S | G | 2486 | T | TTN | F18429 V | A | 55510 | C | VPS45 | R433* | C | 1843 | T |
| TCF7 | P415S | C | 1438 | T | TMTC3 | S703R | A | 2327 | C | TTN | R14769 Q | C | 44531 | T | VPS45 | R205H | G | 1160 | A |
| TCF7L1 | V440I | G | 1593 | A | TMTC3 | F230L | T | 910 | G | TTN | A11661 V | G | 35207 | A | VPS4A | V338M | G | 1148 | A |
| TCF7L1 | A34E | C | 376 | A | TMTC3 | F326L | C | 1198 | A | TTN | A471V | G | 1637 | A | VPS4A | A42T | G | 260 | A |
| TCF7L1 | L211I | C | 906 | A | TMTC3 | I197M | T | 811 | G | TTN | R32112 C | G | 96559 | A | VPS53 | R361C | G | 1228 | A |
| TCF7L2 | R465C | C | 1698 | T | TMTC4 | R611C | G | 1831 | A | TTN | I27746F | T | 83461 | A | VPS53 | S363F | G | 1235 | A |
| TCF7L2 | L48P | T | 448 | C | TMTC4 | L638M | G | 1912 | T | TTN | V14125I | C | 42598 | T | VPS54 | E908* | C | 2877 | A |
| TCF7L2 | Q147* | C | 744 | T | TMTC4 | T312A | T | 934 | C | TTN | A15126 V | G | 45602 | A | VPS54 | E899K | C | 2850 | T |
| TCF7L2 | T182M | C | 850 | T | TMTC4 | A171V | G | 512 | A | TTN | G13988 V | C | 42188 | A | VPS54 | K420T | T | 1414 | G |
| TCF7L2 | E17* | G | 354 | T | TMTC4 | R717C | G | 2149 | A | TTN | R31516 G | T | 94771 | C | VPS8 | V936G | T | 2984 | G |
| TCF7L2 | R465H | G | 1699 | A | TMUB2 | R301* | C | 1050 | T | TTN | D30806 N | C | 92641 | T | VPS8 | A201T | G | 778 | A |
| TCF7L2 | A374V | C | 1426 | T | TMX2 | - | A | 0 | G | TTN | Y28221 H | A | 84886 | G | VPS8 | E1293* | G | 4054 | T |
| TCFL5 | G295R | C | 976 | T | TMX2 | A241D | C | 722 | A | TTN | M22710I | C | 68355 | A | VPS8 | F1298C | T | 4070 | G |
| TCHH | R739S | C | 2217 | A | TMX3 | D120V | T | 503 | A | TTN | A8079T | C | 24460 | T | VRK1 | K211T | A | 738 | C |
| TCHH | N1233S | T | 3698 | C | TNC | T1844A | T | 5845 | C | TTN | R17850 G | T | 53773 | C | VRK2 | Y488D | T | 1607 | G |
| TCHH | D1290N | C | 3868 | T | TNC | A1010T | C | 3343 | T | TTN | R12194* | G | 36805 | A | VRK3 | A222T | C | 1010 | T |
| TCHH | E908A | T | 2723 | G | TNC | T1760A | T | 5593 | C | TTN | A6934T | C | 21025 | T | VSIG1 | A95T | G | 344 | A |
| TCHH | W1865* | C | 5595 | T | TNC | I1313T | A | 4253 | G | TTN | D32583 N | C | 97972 | T | VSIG1 | A310T | G | 989 | A |
| TCHH | L1086P | A | 3257 | G | TNC | R293Q | C | 1193 | T | TTN | R30584 H | C | 91976 | T | VSIG10 | A235T | C | 1037 | T |
| TCHH | R1496H | C | 4487 | T | TNC | Q515H | C | 1860 | G | TTN | A26933 T | C | 81022 | T | VSIG10L | R361C | G | 1081 | A |
| TCHHL1 | G587C | C | 1824 | A | TNC | F739V | A | 2530 | C | TTN | P26769 S | G | 80530 | A | VSIG10L | T326M | G | 977 | A |
| TCHHL1 | S90A | A | 333 | C | TNFAIP1 | D304N | G | 1177 | A | TTN | K24941 N | C | 75048 | A | VSIG10L | R190W | G | 568 | A |
| TCHP | Y191C | A | 725 | G | TNFAIP1 | R272C | C | 1081 | T | TTN | T24772 A | T | 74539 | C | VSIG2 | V143F | C | 528 | A |
| TCHP | G382E | G | 1298 | A | TNFAIP3 | G367V | G | 1166 | T | TTN | A20725 T | C | 62398 | T | VSIG4 | R134H | C | 510 | T |

| | | | | | | | | | | | | | | | | | | | |
|---|---|---|---|---|---|---|---|---|---|---|---|---|---|---|---|---|---|---|---|
| TCHP | A371T | G | 1264 | A | TNFAIP8 | F179C | T | 926 | G | TTN | R19559S | G | 58900 | T | VSIG4 | Q313H | T | 1048 | G |
| TCIRG1 | V728I | G | 2290 | A | TNFAIP8L1 | R89S | C | 380 | A | TTN | P14745Q | G | 44459 | T | VSTM1 | Q70* | G | 384 | A |
| TCIRG1 | G451S | G | 1459 | A | TNFAIP8L3 | C16F | C | 147 | A | TTN | L14391M | G | 43396 | T | VSTM2A | F29L | T | 402 | G |
| TCL1A | R83Q | C | 378 | T | TNFRSF10A | R282C | G | 909 | A | TTN | G14327C | C | 43204 | A | VSX1 | G270W | C | 1072 | A |
| TCL1A | A33T | C | 227 | T | TNFRSF10C | A234T | G | 783 | A | TTN | A10976T | C | 33151 | T | VTA1 | L10F | C | 86 | T |
| TCN1 | Q390H | C | 1268 | A | TNFRSF11A | M416V | A | 1284 | G | TTN | Y9751C | T | 29477 | C | VTA1 | G108E | G | 381 | A |
| TCN1 | A108T | C | 420 | T | TNFRSF11A | G72C | G | 252 | T | TTN | K925N | C | 3000 | A | VTCN1 | S246L | G | 776 | A |
| TCOF1 | G1319V | G | 4064 | T | TNFRSF11A | - | G | 0 | A | TTN | T29987A | T | 90184 | C | VTI1A | R192C | C | 690 | T |
| TCP10L | G23* | C | 181 | A | TNFRSF11B | K268N | C | 1127 | A | TTN | K25210R | T | 75854 | C | VWA1 | G114V | G | 419 | T |
| TCP11 | P15S | G | 461 | A | TNFRSF11B | L227V | A | 1002 | C | TTN | A18540V | G | 55844 | A | VWA2 | R520W | C | 1808 | T |
| TCP11L1 | A111V | C | 732 | T | TNFRSF13B | R20H | C | 72 | T | TTN | R16654C | G | 50185 | A | VWA3A | R576W | C | 1822 | T |
| TCP11L2 | A418T | G | 1426 | A | TNFRSF13B | R14C | G | 53 | A | TTN | T16275M | G | 49049 | A | VWA3A | - | T | 0 | C |
| TCP11L2 | K440T | A | 1493 | C | TNFRSF19 | S336F | C | 1306 | T | TTN | G15660S | C | 47203 | T | VWA3A | N569D | A | 1801 | G |
| TCP11L2 | R239C | C | 889 | T | TNFRSF1A | E342D | C | 1307 | A | TTN | R8839C | G | 26740 | A | VWA3A | G1144R | G | 3526 | A |
| TCTE1 | G398S | C | 1315 | T | TNFRSF1A | A321T | C | 1242 | T | TTN | R18623H | C | 56093 | T | VWA3A | E225K | G | 769 | A |
| TCTE1 | R158W | G | 595 | A | TNFRSF1A | L329I | G | 1266 | T | TTN | R25853* | G | 77782 | A | VWA5A | K72T | A | 424 | C |
| TCTE1 | V141M | C | 544 | T | TNFRSF1B | T84I | C | 340 | T | TTN | T33385A | T | 100378 | C | VWA5A | F588L | C | 1973 | A |
| TCTE1 | R62H | C | 308 | T | TNFRSF1B | D257N | G | 858 | A | TTN | I32909S | A | 98951 | C | VWA5A | S766L | C | 2506 | T |
| TCTE1 | N498H | T | 1615 | G | TNFRSF21 | D455N | C | 1757 | T | TTN | R32269* | G | 97030 | A | VWA5B1 | A17T | G | 245 | A |
| TCTEX1D2 | N76D | T | 362 | C | TNFRSF21 | K545N | C | 2029 | A | TTN | R31265C | G | 94018 | A | VWA5B1 | A1146T | G | 3632 | A |
| TCTEX1D2 | A2T | C | 140 | T | TNFRSF25 | S201P | A | 669 | G | TTN | F31096L | G | 93513 | T | VWA5B1 | G981S | G | 3137 | A |
| TCTN1 | E551* | G | 1670 | T | TNFRSF4 | G92V | C | 280 | A | TTN | F28376V | A | 85351 | C | VWA5B1 | V462M | G | 1580 | A |
| TCTN1 | V312I | G | 953 | A | TNFRSF8 | R417W | C | 1471 | T | TTN | R28188I | C | 84788 | A | VWA5B1 | S258N | G | 969 | A |
| TCTN3 | F564V | A | 1880 | C | TNFRSF9 | S145Y | G | 601 | T | TTN | D27637E | A | 83136 | C | VWA5B1 | D702V | A | 2301 | T |
| TCTN3 | Y542C | T | 1815 | C | TNFRSF9 | E111* | C | 498 | A | TTN | T27302A | T | 82129 | C | VWA5B1 | R356I | G | 1263 | T |
| TCTN3 | E463D | C | 1579 | A | TNFRSF9 | T207M | G | 787 | A | TTN | R26935 | G | 81028 | A | VWA5B1 | D677G | A | 2226 | G |

| | | | | | | | | | | | | | | | | | | | |
|---|---|---|---|---|---|---|---|---|---|---|---|---|---|---|---|---|---|---|---|
| | | | | | | | | | | | C | | | | | | | | |
| TDG | S366N | G | 1320 | A | TNFSF11 | R89K | G | 417 | A | TTN | S23775 Y | G | 71549 | T | VWA5B1 | R993H | G | 3174 | A |
| TDO2 | R283W | C | 911 | T | TNFSF11 | R118I | G | 504 | T | TTN | R19873 C | G | 59842 | A | VWC2 | R58W | C | 728 | T |
| TDO2 | L226F | C | 740 | T | TNFSF11 | S115* | C | 495 | A | TTN | P19187 S | G | 57784 | A | VWC2L | E33* | G | 899 | T |
| TDO2 | R94* | C | 344 | T | TNFSF12 | K97* | A | 552 | T | TTN | F16123L | G | 48594 | T | VWC2L | G52W | G | 956 | T |
| TDO2 | F97Y | T | 354 | A | TNFSF13B | R33W | C | 275 | T | TTN | E16122* | C | 48589 | A | VWC2L | C109W | C | 1129 | G |
| TDO2 | F388C | T | 1227 | G | TNFSF14 | A132T | C | 776 | T | TTN | D14757 G | T | 44495 | C | VWC2L | G104R | G | 1112 | A |
| TDP1 | N369D | A | 1404 | G | TNFSF8 | L221H | A | 776 | T | TTN | E13451 K | C | 40576 | T | VWCE | C493* | G | 1758 | T |
| TDP1 | R137M | G | 709 | T | TNFSF8 | L229V | A | 799 | C | TTN | K12684 E | T | 38275 | C | VWCE | G242D | C | 1004 | T |
| TDP1 | D6N | G | 315 | A | TNFSF8 | V27M | C | 193 | T | TTN | R12540 Q | C | 37844 | T | VWDE | C1585R | A | 4753 | G |
| TDP1 | K177N | G | 830 | T | TNIK | R1340Q | C | 4364 | T | TTN | R11257 Q | C | 33995 | T | VWDE | R1540H | C | 4619 | T |
| TDP2 | G288D | C | 1034 | T | TNIK | A201V | G | 947 | A | TTN | E11160 D | C | 33705 | A | VWDE | W813R | A | 2437 | G |
| TDP2 | N239D | T | 886 | C | TNIK | - | A | 0 | G | TTN | P11021 L | G | 33287 | A | VWDE | P823H | G | 2468 | T |
| TDRD1 | L888R | T | 2816 | G | TNIK | A777V | G | 2675 | A | TTN | K10844 N | C | 32757 | A | VWDE | F1171C | A | 3512 | C |
| TDRD10 | P208L | C | 708 | T | TNIK | R299C | G | 1240 | A | TTN | P10702 T | G | 32329 | T | VWDE | L264F | C | 792 | A |
| TDRD10 | C69Y | G | 291 | A | TNIK | R404W | G | 1555 | A | TTN | K9860N | T | 29805 | G | VWF | G2560D | C | 7929 | T |
| TDRD12 | Q394* | C | 1480 | T | TNIK | Q614H | C | 2187 | A | TTN | V9344A | A | 28256 | G | VWF | R1956W | G | 6116 | A |
| TDRD3 | A654T | G | 2515 | A | TNIK | A279S | C | 1180 | A | TTN | E9190D | T | 27795 | G | VWF | H95N | G | 533 | T |
| TDRD5 | E538D | G | 1864 | T | TNIP1 | P549L | G | 2235 | A | TTN | P9076S | G | 27451 | A | VWF | E333* | C | 1247 | A |
| TDRD5 | A181V | C | 792 | T | TNIP1 | S107N | C | 909 | T | TTN | D8899N | C | 26920 | T | VWF | R1837W | G | 5759 | A |
| TDRD6 | K559* | A | 1675 | T | TNIP2 | G84A | C | 338 | G | TTN | K8800T | T | 26624 | G | VWF | P2506L | G | 7767 | A |
| TDRD6 | S861Y | C | 2582 | A | TNIP3 | T95M | G | 363 | A | TTN | K8214T | T | 24866 | G | VWF | V657M | C | 2219 | T |
| TDRD6 | R103H | G | 308 | A | TNK1 | R367Q | G | 1260 | A | TTN | L8055R | A | 24389 | C | VWF | N2636H | T | 8156 | G |
| TDRD6 | H980R | A | 2939 | G | TNK1 | A574T | G | 1880 | A | TTN | W7619R | A | 23080 | G | VWF | D1832N | C | 5744 | T |
| TDRD6 | S68L | C | 203 | T | TNK2 | A735V | G | 2349 | A | TTN | R6737W | G | 20434 | A | VWF | E990D | C | 3220 | A |
| TDRD6 | R270H | G | 809 | A | TNK2 | A210T | C | 773 | T | TTN | F6717L | A | 20376 | C | VWF | D400Y | C | 1448 | A |
| TDRD6 | A53V | C | 158 | T | TNK2 | S561T | C | 1827 | G | TTN | E6044D | T | 18357 | G | WAC | T552M | C | 1816 | T |
| TDRD6 | F335L | T | 1003 | C | TNK2 | R116Q | C | 492 | T | TTN | V5621G | A | 17087 | C | WAC | E172* | G | 675 | T |
| TDRD6 | L634I | C | 1900 | A | TNKS | R383* | C | 1173 | T | TTN | S5541L | G | 16847 | A | WAPAL | I331V | T | 1076 | C |
| TDRD7 | L907* | T | 3015 | G | TNKS | Q629* | C | 1911 | T | TTN | F4163V | A | 12712 | C | WAPAL | S95N | C | 369 | T |
| TDRD7 | R1041Q | G | 3417 | A | TNKS | T686M | C | 2083 | T | TTN | F3926C | A | 12002 | C | WAPAL | V705F | C | 2198 | A |

| | | | | | | | | | | | | | | | | | | | |
|---|---|---|---|---|---|---|---|---|---|---|---|---|---|---|---|---|---|---|---|
| TDRD9 | R203H | G | 656 | A | TNKS | R1161W | C | 3507 | T | TTN | S3376L | G | 10352 | A | WAPAL | E237K | C | 794 | T |
| TDRD9 | T878M | C | 2681 | T | TNKS | R537C | C | 1635 | T | TTN | F2202C | A | 6830 | C | WAPAL | D161Y | C | 566 | A |
| TDRD9 | A196T | G | 634 | A | TNKS1BP1 | D924N | C | 2935 | T | TTN | F1757C | A | 5495 | C | WAPAL | E142D | T | 511 | G |
| TDRD9 | E258K | G | 820 | A | TNKS1BP1 | R155C | G | 628 | A | TTN | S1433P | A | 4522 | G | WAPAL | K85N | T | 340 | G |
| TDRD9 | R564H | G | 1739 | A | TNKS1BP1 | R1018Q | C | 3218 | T | TTN | E1153* | C | 3682 | A | WARS2 | R269H | C | 833 | T |
| TDRKH | S282P | A | 1014 | G | TNKS1BP1 | R1543Q | C | 4793 | T | TTN | D592Y | C | 1999 | A | WAS | G11R | G | 106 | A |
| TDRKH | R203I | C | 778 | A | TNKS1BP1 | R427Q | C | 1445 | T | TTN | E197* | C | 814 | A | WAS | R41Q | G | 197 | A |
| TEAD3 | R85Q | C | 442 | T | TNKS2 | R153Q | G | 837 | A | TTN | R29463 C | G | 88612 | A | WASF1 | D244N | C | 1162 | T |
| TEAD3 | R398H | C | 1381 | T | TNKS2 | P829Q | C | 2865 | A | TTN | A14034 V | G | 42326 | A | WASF1 | T301A | T | 1333 | C |
| TEAD3 | E454K | C | 1548 | T | TNN | P200L | C | 712 | T | TTN | P18494 T | G | 55705 | T | WASF3 | P10S | C | 254 | T |
| TEAD4 | S134L | C | 675 | T | TNN | E752D | G | 2369 | T | TTN | L1188H | A | 3788 | T | WASF3 | K5R | A | 240 | G |
| TEAD4 | A56T | G | 440 | A | TNN | P10H | C | 142 | A | TTN | - | C | 0 | G | WASF4 | K148T | T | 659 | G |
| TEC | T560M | G | 1837 | A | TNN | R214C | C | 753 | T | TTPA | F250L | G | 768 | T | WASL | K269T | T | 1139 | G |
| TEC | V234I | C | 858 | T | TNN | E961D | G | 2996 | T | TTYH1 | Q36H | A | 135 | C | WASL | K233N | C | 1032 | A |
| TEC | R617H | C | 2008 | T | TNN | K1235N | G | 3818 | T | TTYH1 | A51V | C | 179 | T | WASL | N208H | T | 955 | G |
| TEC | - | C | 0 | A | TNN | R1297Q | G | 4003 | A | TTYH3 | R302H | G | 1110 | A | WBP1 | R110H | G | 532 | A |
| TEC | D620Y | C | 2016 | A | TNNC1 | R83Q | C | 303 | T | TTYH3 | A49T | G | 350 | A | WBP11 | R3W | G | 168 | A |
| TEC | A396T | C | 1344 | T | TNNC1 | S37N | C | 165 | T | TUB | R323C | C | 1208 | T | WBP11 | D292N | C | 1035 | T |
| TECPR1 | C920R | A | 2963 | G | TNNI1 | A24V | G | 164 | A | TUB | R164I | G | 732 | T | WBP11 | P580S | G | 1899 | A |
| TECPR1 | G801S | C | 2606 | T | TNNI3K | R164H | G | 507 | A | TUB | H48Q | C | 385 | A | WBP11 | D356N | C | 1227 | T |
| TECPR1 | A935T | C | 3008 | T | TNNI3K | - | G | 0 | T | TUB | I324S | T | 1212 | G | WBP2 | P207L | G | 743 | A |
| TECPR1 | - | T | 0 | C | TNNT1 | I66N | A | 286 | T | TUBA1C | W388* | G | 1264 | A | WBP4 | S225L | C | 1074 | T |
| TECPR2 | S523T | T | 1715 | A | TNNT2 | R151Q | C | 452 | T | TUBA1C | E447G | A | 1440 | G | WBP5 | I88T | T | 574 | C |
| TECPR2 | V962M | G | 3032 | A | TNNT2 | E33D | C | 99 | A | TUBA3C | E279K | C | 940 | T | WBSCR16 | E44D | C | 188 | A |
| TECPR2 | A1363V | C | 4236 | T | TNNT2 | Q159H | C | 477 | A | TUBA3E | A333T | C | 1098 | T | WBSCR17 | G239W | G | 1349 | T |
| TECPR2 | T903M | C | 2856 | T | TNPO1 | F729L | C | 2337 | A | TUBA3E | R339H | C | 1117 | T | WBSCR17 | V477I | G | 2063 | A |
| TECPR2 | E710* | G | 2276 | T | TNPO2 | G866D | C | 2782 | T | TUBA4A | R2C | G | 178 | A | WBSCR17 | R262C | C | 1418 | T |
| TECR | A214T | G | 640 | A | TNPO2 | L178P | A | 718 | G | TUBA4A | R402M | C | 1379 | A | WBSCR17 | C364F | G | 1725 | T |
| TECR | R66H | G | 197 | A | TNPO3 | R852Q | C | 2929 | T | TUBA8 | S16Y | C | 344 | A | WDFY2 | R212W | C | 814 | T |
| TECR | A111V | C | 332 | T | TNPO3 | R297H | C | 1264 | T | TUBA8 | E308K | G | 1219 | A | WDFY3 | P66L | G | 605 | A |
| TECR | R66H | G | 197 | A | TNR | R1007W | G | 3728 | A | TUBA8 | R238C | C | 1009 | T | WDFY3 | E2468Q | C | 7810 | G |
| TECRL | S272Y | G | 926 | T | TNR | G543R | C | 2336 | T | TUBB1 | A275P | G | 1092 | C | WDFY3 | I1369V | T | 4513 | C |
| TECTA | S45N | G | 405 | A | TNR | R1227H | C | 4389 | T | TUBB2B | S75L | G | 416 | A | WDFY3 | P2031S | G | 6499 | A |
| TECTA | F706S | T | 2388 | C | TNR | A397T | C | 1898 | T | TUBB2B | K58R | T | 365 | C | WDFY3 | R1003W | G | 3415 | A |
| TECTA | R767Q | G | 2571 | A | TNR | Q43P | T | 837 | G | TUBB2C | N48D | A | 290 | G | WDFY3 | T3049A | T | 9553 | C |
| TECTA | E1750A | A | 5520 | C | TNR | - | T | 0 | G | TUBB2C | K336E | A | 1154 | G | WDFY3 | R1183* | G | 3955 | A |
| TECTA | G1732C | G | 5465 | T | TNR | P463S | G | 2096 | A | TUBB4 | A352V | G | 1162 | A | WDFY3 | S796Y | G | 2795 | T |

| | | | | | | | | | | | | | | | | | | | |
|---|---|---|---|---|---|---|---|---|---|---|---|---|---|---|---|---|---|---|---|
| TECTA | D1444N | G | 4601 | A | TNR | S1060P | A | 3887 | G | TUBB4 | A63V | G | 295 | A | WDFY4 | P919L | C | 2783 | T |
| TECTA | A1510T | G | 4799 | A | TNR | D532N | C | 2303 | T | TUBB6 | I345V | A | 1255 | G | WDFY4 | V2722M | G | 8191 | A |
| TECTA | R586* | C | 2027 | T | TNR | Q509H | C | 2236 | A | TUBB6 | T95M | C | 506 | T | WDFY4 | Q3092E | C | 9301 | G |
| TECTA | A1736T | G | 5477 | A | TNR | S497L | G | 2199 | A | TUBD1 | I3T | A | 286 | G | WDFY4 | V1286I | G | 3883 | A |
| TECTA | V1645M | G | 5204 | A | TNRC18 | A540T | C | 1967 | T | TUBG1 | L243M | C | 789 | A | WDFY4 | E2344K | G | 7057 | A |
| TECTA | R2021H | G | 6333 | A | TNRC18 | S2713L | G | 8487 | A | TUBG2 | S239N | G | 972 | A | WDFY4 | Y2762S | A | 8312 | C |
| TECTA | Q1160* | C | 3749 | T | TNRC18 | A811T | C | 2780 | T | TUBGCP2 | A124V | G | 411 | A | WDFY4 | L724M | C | 2197 | A |
| TECTA | R2101Q | G | 6573 | A | TNRC18 | R2832H | C | 8844 | T | TUBGCP2 | V217M | C | 689 | T | WDFY4 | A165T | G | 520 | A |
| TECTA | R63C | C | 458 | T | TNRC18 | G987C | C | 3308 | A | TUBGCP3 | Y544C | T | 1818 | C | WDFY4 | E374* | G | 1147 | T |
| TECTA | R108* | C | 593 | T | TNRC18 | T1026A | T | 3425 | C | TUBGCP3 | H387N | G | 1346 | T | WDFY4 | D502N | G | 1531 | A |
| TECTA | F664L | C | 2263 | A | TNRC18 | A811T | C | 2780 | T | TUBGCP3 | E838* | C | 2699 | A | WDFY4 | A1017V | C | 3077 | T |
| TECTA | E185K | G | 824 | A | TNRC18 | P1169H | G | 3855 | T | TUBGCP3 | R64Q | C | 378 | T | WDFY4 | D1117N | G | 3376 | A |
| TECTB | V27D | T | 80 | A | TNRC18 | K1577N | C | 5080 | A | TUBGCP3 | Q31P | T | 279 | G | WDFY4 | E1242* | G | 3751 | T |
| TECTB | F165L | C | 495 | A | TNRC18 | E469D | C | 1756 | A | TUBGCP3 | V277G | A | 1017 | C | WDFY4 | R1304I | G | 3938 | T |
| TEF | S150P | T | 534 | C | TNRC18 | D1106N | C | 3665 | T | TUBGCP4 | L34I | C | 360 | A | WDFY4 | D1388Y | G | 4189 | T |
| TEK | K808T | A | 2548 | C | TNRC18 | F869V | A | 2954 | C | TUBGCP5 | V399M | G | 1325 | A | WDFY4 | R1495S | G | 4512 | T |
| TEK | V948M | G | 2967 | A | TNRC6A | P402S | C | 1333 | T | TUBGCP5 | I820M | T | 2590 | G | WDFY4 | S2001Y | C | 6029 | A |
| TEK | R849W | C | 2670 | T | TNRC6A | G754R | G | 2389 | A | TUBGCP6 | S1342R | T | 4129 | G | WDFY4 | R2249Q | G | 6773 | A |
| TEK | C233Y | G | 823 | A | TNRC6A | A1097V | C | 3419 | T | TUBGCP6 | W1266* | C | 3903 | T | WDFY4 | N2774H | A | 8347 | C |
| TEK | S654L | C | 2086 | T | TNRC6A | A664P | G | 2119 | C | TUBGCP6 | T408M | G | 1328 | A | WDFY4 | K2816T | A | 8474 | C |
| TEK | K690N | G | 2195 | T | TNRC6A | - | A | 0 | G | TUFT1 | N166H | A | 558 | C | WDFY4 | R1091C | C | 3298 | T |
| TEK | C1074* | C | 3347 | A | TNRC6A | V1637D | T | 5039 | A | TUFT1 | E366D | G | 1160 | T | WDFY4 | R1091C | C | 3298 | T |
| TEKT1 | V205G | A | 744 | C | TNRC6A | A1672V | C | 5144 | T | TULP1 | R519W | G | 1635 | A | WDR1 | F342L | G | 1100 | T |
| TEKT1 | K95T | T | 414 | G | TNRC6A | S1599L | C | 4925 | T | TULP1 | V435A | A | 1384 | G | WDR1 | A339S | C | 1089 | A |
| TEKT2 | R268W | C | 929 | T | TNRC6A | L718Q | T | 2282 | A | TULP2 | T406I | G | 1362 | A | WDR1 | A172T | C | 588 | T |
| TEKT2 | E341K | G | 1148 | A | TNRC6B | T1726A | A | 5544 | G | TULP2 | K404E | T | 1355 | C | WDR11 | G30D | G | 327 | A |
| TEKT2 | D340N | G | 1145 | A | TNRC6B | D1735G | A | 5572 | G | TULP3 | D282G | A | 926 | G | WDR12 | R63* | G | 937 | A |
| TEKT2 | R268Q | G | 930 | A | TNRC6B | P1359L | C | 4444 | T | TULP3 | P318L | C | 1034 | T | WDR13 | R84H | G | 427 | A |
| TEKT3 | K383N | C | 1336 | A | TNRC6B | G856C | G | 2934 | T | TULP3 | Y418C | A | 1334 | G | WDR16 | E549* | G | 1645 | T |
| TEKT3 | E222D | T | 853 | G | TNRC6B | A1755V | C | 5632 | T | TULP4 | R403C | C | 2564 | T | WDR16 | R502H | G | 1505 | A |
| TEKT3 | A385V | G | 1341 | A | TNRC6B | Q1291L | A | 4240 | T | TULP4 | R1504Q | G | 5868 | A | WDR16 | E524* | G | 1570 | T |
| TEKT4 | R82H | G | 382 | A | TNRC6B | A1333T | G | 4365 | A | TULP4 | T232M | C | 2052 | T | WDR16 | A489T | G | 1465 | A |
| TEKT4 | R157C | C | 606 | T | TNRC6B | R1129* | C | 3753 | T | TULP4 | R369H | G | 2463 | A | WDR16 | E549* | G | 1645 | T |
| TEKT4 | A406T | G | 1353 | A | TNRC6B | I205S | T | 982 | G | TULP4 | P683S | C | 3404 | T | WDR16 | Q9* | C | 25 | T |
| TEKT5 | D329E | A | 1059 | T | TNRC6B | W1035* | G | 3473 | A | TULP4 | A386T | G | 2513 | A | WDR17 | R249C | C | 901 | T |
| TEKT5 | R197* | G | 661 | A | TNS1 | G1732D | C | 5648 | T | TULP4 | R567W | C | 3056 | T | WDR17 | L559F | G | 1833 | T |
| TELO2 | Q228* | C | 903 | T | TNS1 | R618H | C | 2306 | T | TUSC1 | A127V | G | 917 | A | WDR17 | V108A | T | 479 | C |
| TELO2 | W74* | G | 443 | A | TNS1 | P1447L | G | 4793 | A | TUSC3 | G5S | G | 223 | A | WDR17 | N606K | T | 1974 | A |
| TENC1 | R1012W | C | 3229 | T | TNS1 | A1375T | C | 4576 | T | TUSC3 | R312W | C | 1144 | T | WDR17 | K84N | A | 408 | C |

| | | | | | | | | | | | | | | | | | | | |
|---|---|---|---|---|---|---|---|---|---|---|---|---|---|---|---|---|---|---|---|
| TENC1 | V461M | G | 1576 | A | TNS1 | R1163Q | C | 3941 | T | TUSC5 | A86V | C | 596 | T | WDR17 | E1107* | G | 3475 | T |
| TEP1 | D1738N | C | 5253 | T | TNS1 | L1655I | G | 5416 | T | TUT1 | R464Q | C | 1391 | T | WDR17 | D405E | T | 1371 | A |
| TEP1 | R528W | G | 1623 | A | TNS1 | T392I | G | 1628 | A | TUT1 | R818L | C | 2451 | A | WDR19 | L649V | C | 2099 | G |
| TEP1 | G1885D | C | 5695 | T | TNS1 | V216M | C | 1099 | T | TWISTNB | D119N | C | 426 | T | WDR19 | S110R | T | 484 | G |
| TEP1 | R942H | C | 2866 | T | TNS1 | E1578* | C | 5185 | A | TWISTNB | E176A | T | 598 | G | WDR19 | G294A | G | 1035 | C |
| TEP1 | S2461N | C | 7423 | T | TNS1 | R1062M | C | 3638 | A | TWSG1 | A124T | G | 561 | A | WDR19 | N374S | A | 1275 | G |
| TEP1 | R2364Q | C | 7132 | T | TNS1 | D1086Y | C | 3709 | A | TXK | E382G | T | 1231 | C | WDR19 | R828C | C | 2636 | T |
| TEP1 | E92D | C | 317 | A | TNS3 | R558Q | C | 2031 | T | TXLNB | S284G | T | 1083 | C | WDR19 | L721I | C | 2315 | A |
| TEPP | A216E | C | 684 | A | TNS3 | R250H | C | 1107 | T | TXLNG | P516L | C | 1608 | T | WDR20 | T542M | C | 1674 | T |
| TERF1P3 | A260V | C | 804 | T | TNS3 | G1394D | C | 4539 | T | TXNDC11 | R895H | C | 2746 | T | WDR24 | T163M | G | 488 | A |
| TERF1P3 | N281D | A | 866 | G | TNS3 | R15C | G | 401 | A | TXNDC11 | R902H | C | 2767 | T | WDR25 | R466C | C | 1451 | T |
| TERF1P3 | A303V | C | 933 | T | TNS3 | V218A | A | 1011 | G | TXNDC11 | S662Y | G | 2047 | T | WDR25 | R465W | C | 1448 | T |
| TERF2 | R192I | C | 686 | A | TNS3 | G1204V | C | 3969 | A | TXNDC16 | D520Y | C | 1930 | A | WDR26 | R315W | G | 1137 | A |
| TERT | P1124H | G | 3429 | T | TNS3 | G494V | C | 1839 | A | TXNDC16 | R627G | T | 2251 | C | WDR27 | A519T | C | 2075 | T |
| TERT | R653H | C | 2016 | T | TNS4 | G469E | C | 1470 | T | TXNDC2 | S419R | C | 1456 | A | WDR3 | V282A | T | 892 | C |
| TERT | R446C | G | 1394 | A | TNS4 | S446Y | G | 1401 | T | TXNDC2 | A540T | G | 1817 | A | WDR33 | R834* | G | 2659 | A |
| TERT | A716V | G | 2205 | A | TOB2 | G290V | C | 1576 | A | TXNDC3 | C208Y | G | 995 | A | WDR33 | R1196C | G | 3745 | A |
| TERT | A1040V | G | 3177 | A | TOB2 | P255S | G | 1470 | A | TXNDC3 | Q482* | C | 1816 | T | WDR33 | Q571H | T | 1872 | G |
| TERT | A855V | G | 2622 | A | TOLLIP | G125C | C | 404 | A | TXNDC3 | A101S | G | 673 | T | WDR33 | K216T | T | 806 | G |
| TES | R374Q | G | 1336 | A | TOLLIP | P310L | G | 960 | A | TXNDC5 | R296H | C | 925 | T | WDR34 | R202Q | C | 666 | T |
| TES | I40V | A | 333 | G | TOM1 | F452L | T | 1481 | G | TXNDC6 | G270D | C | 837 | T | WDR35 | R148C | G | 558 | A |
| TES | E15K | G | 258 | A | TOM1L1 | V151I | G | 626 | A | TXNDC6 | R70H | C | 237 | T | WDR35 | R700C | G | 2214 | A |
| TESK1 | V194M | G | 830 | A | TOM1L1 | E138K | G | 587 | A | TXNDC6 | E75K | C | 251 | T | WDR35 | E474* | C | 1536 | A |
| TESK2 | E462A | T | 1786 | G | TOM1L2 | R278H | C | 917 | T | TXNDC8 | G33C | C | 146 | A | WDR36 | G369C | G | 1678 | T |
| TESK2 | Y491C | T | 1873 | C | TOMM5 | K19E | T | 162 | C | TXNIP | P106H | C | 651 | A | WDR36 | F362C | T | 1658 | G |
| TESK2 | R299C | G | 1296 | A | TOMM70A | E320* | C | 1407 | A | TXNL1 | R234C | G | 949 | A | WDR36 | S692F | C | 2648 | T |
| TESK2 | A79T | C | 636 | T | TOP1 | R138K | G | 663 | A | TXNL1 | K181T | T | 791 | G | WDR38 | D74N | G | 276 | A |
| TET1 | S879L | C | 2845 | T | TOP1 | D83N | G | 497 | A | TXNL1 | R151Q | C | 701 | T | WDR4 | R84H | C | 287 | T |
| TET1 | S1012* | C | 3244 | A | TOP1MT | T405I | G | 1233 | A | TXNL4A | R124H | C | 511 | T | WDR4 | P393L | G | 1214 | A |
| TET1 | R81H | G | 451 | A | TOP2B | E1042* | C | 3124 | A | TXNL4B | D148N | C | 764 | T | WDR4 | R341C | G | 1057 | A |
| TET1 | A2129V | C | 6595 | T | TOP2B | K571Q | T | 1711 | G | TXNRD1 | N267I | A | 822 | T | WDR43 | E661* | G | 2037 | T |
| TET1 | C1482Y | G | 4654 | A | TOP3A | G739* | C | 2411 | A | TXNRD1 | R238Q | G | 735 | A | WDR44 | R738C | C | 2607 | T |
| TET1 | A1896V | C | 5896 | T | TOP3A | F436C | A | 1503 | C | TXNRD1 | N298T | A | 915 | C | WDR44 | L384F | C | 1545 | T |
| TET1 | K795I | A | 2593 | T | TOP3A | R103* | G | 503 | A | TXNRD2 | G46D | C | 144 | T | WDR44 | F898S | T | 3088 | C |
| TET1 | E417A | A | 1459 | C | TOP3B | T156M | G | 652 | A | TXNRD2 | G235D | C | 711 | T | WDR44 | G605* | G | 2208 | T |
| TET1 | K540T | A | 1828 | C | TOPBP1 | R769C | G | 2437 | A | TXNRD3IT 1 | G32W | C | 573 | A | WDR44 | K518T | A | 1948 | C |
| TET1 | K792T | A | 2584 | C | TOPBP1 | A932V | G | 2927 | A | TYK2 | R633C | G | 1999 | A | WDR46 | R319W | G | 1312 | A |
| TET1 | K1056N | A | 3377 | C | TOPORS | R490M | C | 1586 | A | TYK2 | R220C | G | 760 | A | WDR46 | E107* | C | 676 | A |
| TET1 | Q1322* | C | 4173 | T | TOPORS | R188Q | C | 680 | T | TYK2 | R224W | G | 772 | A | WDR47 | S549G | T | 2021 | C |

| | | | | | | | | | | | | | | | | | | | |
|---|---|---|---|---|---|---|---|---|---|---|---|---|---|---|---|---|---|---|---|
| TET2 | S289L | C | 1663 | T | TOPORS | R181C | G | 658 | A | TYK2 | R565W | G | 1795 | A | WDR47 | T548P | T | 2018 | G |
| TET2 | Y1169* | T | 4304 | A | TOPORS | R360Q | C | 1196 | T | TYK2 | R992W | G | 3076 | A | WDR47 | E13* | C | 413 | A |
| TET2 | T118I | C | 1150 | T | TOPORS | R188* | G | 679 | A | TYK2 | R868L | C | 2705 | A | WDR48 | R249* | C | 773 | T |
| TET2 | A1497V | C | 5287 | T | TOR1A | R292W | G | 874 | A | TYMP | E51K | C | 273 | T | WDR48 | K168N | A | 532 | C |
| TET2 | K108T | A | 1120 | C | TOR1A | A193T | C | 577 | T | TYRO3 | G817D | G | 2674 | A | WDR48 | R235C | C | 731 | T |
| TET2 | F373L | C | 1916 | A | TOR1A | R188* | G | 562 | A | TYRO3 | M600I | G | 2024 | A | WDR49 | Y452H | A | 1660 | G |
| TET2 | V1857M | G | 6366 | A | TOR1AIP1 | E531K | G | 2052 | A | TYRO3 | - | A | 0 | C | WDR49 | E642* | C | 2230 | A |
| TET3 | M977V | A | 2994 | G | TOR2A | R214C | G | 687 | A | TYRO3 | - | A | 0 | C | WDR49 | E558* | C | 1978 | A |
| TET3 | R1576Q | G | 4792 | A | TOR2A | R137H | C | 457 | T | TYRP1 | T98I | C | 422 | T | WDR5 | R196C | C | 757 | T |
| TET3 | G1398R | G | 4257 | A | TOR2A | S315P | A | 990 | G | TYRP1 | L133I | C | 526 | A | WDR52 | K718N | C | 4756 | A |
| TET3 | R1576W | C | 4791 | T | TOR2A | T200M | G | 646 | A | TYRP1 | F220S | T | 788 | C | WDR52 | P992S | G | 5576 | A |
| TET3 | T165M | C | 559 | T | TOX | S354L | G | 1282 | A | TYSND1 | A544T | C | 1630 | T | WDR53 | C214Y | C | 1209 | T |
| TET3 | A874T | G | 2685 | A | TOX | D227N | C | 900 | T | TYW1 | V582M | G | 1908 | A | WDR53 | A2T | C | 572 | T |
| TEX10 | L304P | A | 1088 | G | TOX2 | A424T | G | 1270 | A | U2AF1 | R188H | C | 656 | T | WDR54 | A109V | C | 414 | T |
| TEX10 | V849A | A | 2723 | G | TOX3 | E65K | C | 364 | T | U2AF1 | R229K | C | 779 | T | WDR54 | L193V | C | 665 | G |
| TEX10 | R550H | C | 1826 | T | TOX3 | A318V | G | 1124 | A | U2AF1L4 | A139V | G | 460 | A | WDR55 | R248H | G | 803 | A |
| TEX10 | W903R | A | 2884 | G | TOX3 | M469I | C | 1578 | T | U2AF2 | R89H | G | 1321 | A | WDR55 | A186S | G | 616 | T |
| TEX10 | S682R | T | 2221 | G | TOX3 | S559P | A | 1846 | G | U2AF2 | K408R | A | 2278 | G | WDR59 | R631C | G | 1993 | A |
| TEX10 | R598Q | C | 1970 | T | TOX4 | D8Y | G | 298 | T | UACA | L1277* | A | 3982 | C | WDR59 | A411V | G | 1334 | A |
| TEX10 | K529T | T | 1763 | G | TP53 | R213* | G | 827 | A | UACA | A142T | C | 576 | T | WDR6 | A568T | G | 1982 | A |
| TEX10 | R115Q | C | 521 | T | TP53 | P152L | G | 645 | A | UACA | Q1393K | G | 4329 | T | WDR6 | G629S | G | 2165 | A |
| TEX10 | V506F | C | 1693 | A | TP53 | S241Y | G | 912 | T | UACA | T1220N | G | 3811 | T | WDR6 | S1085N | G | 3534 | A |
| TEX101 | T148A | A | 442 | G | TP53 | P151H | G | 642 | T | UACA | E1157D | C | 3623 | A | WDR60 | R84M | G | 409 | T |
| TEX11 | R639* | G | 2071 | A | TP53 | R273H | C | 1008 | T | UACA | L1061F | T | 3335 | G | WDR60 | E406D | A | 1376 | T |
| TEX11 | M123V | T | 523 | C | TP53 | R158H | C | 663 | T | UACA | K1003N | T | 3161 | G | WDR62 | K838T | A | 2604 | C |
| TEX11 | K738E | T | 2368 | C | TP53 | R196* | G | 776 | A | UAP1 | E32* | G | 396 | T | WDR62 | T1101M | C | 3393 | T |
| TEX11 | R120* | G | 514 | A | TP53 | Y234H | A | 890 | G | UAP1L1 | V305A | T | 946 | C | WDR62 | R1169H | G | 3597 | A |
| TEX11 | L576I | G | 1882 | T | TP53 | - | C | 0 | G | UAP1L1 | V358A | T | 1105 | C | WDR62 | A162T | G | 575 | A |
| TEX12 | G34V | G | 233 | T | TP53 | I232T | A | 885 | G | UAP1L1 | R244L | G | 763 | T | WDR63 | S800F | C | 2579 | T |
| TEX13A | K113E | T | 449 | C | TP53 | R196* | G | 776 | A | UBA1 | M539I | G | 1707 | T | WDR64 | E457* | G | 1576 | T |
| TEX13A | G31S | C | 203 | T | TP53 | - | T | 0 | A | UBA2 | R610H | G | 1899 | A | WDR64 | V925A | T | 2981 | C |
| TEX13A | R91W | G | 383 | A | TP53 | R273C | G | 1007 | A | UBA2 | N423H | A | 1337 | C | WDR64 | F115L | C | 552 | A |
| TEX13B | A26T | C | 169 | T | TP53 | R337L | C | 1200 | A | UBA3 | A318S | C | 1007 | A | WDR64 | I1007F | A | 3226 | T |
| TEX14 | F578L | G | 1852 | C | TP53 | C176Y | C | 717 | T | UBA3 | Y156H | A | 521 | G | WDR64 | L1028P | T | 3290 | C |
| TEX14 | S1296F | G | 4005 | A | TP53 | C238F | C | 903 | A | UBA6 | S411Y | G | 1292 | T | WDR64 | D689Y | G | 2272 | T |
| TEX14 | P1201T | G | 3719 | T | TP53 | I254S | A | 951 | C | UBA6 | K27T | T | 140 | G | WDR64 | R991* | C | 3178 | T |
| TEX14 | F881L | A | 2761 | C | TP53 | L257Q | A | 960 | T | UBA7 | G800D | C | 2558 | T | WDR64 | - | A | 3453 | C |
| TEX14 | F252L | G | 874 | T | TP53 | R175H | C | 714 | T | UBA7 | F574L | G | 1881 | T | WDR65 | G53C | G | 278 | T |
| TEX15 | E1603Q | C | 4807 | G | TP53 | R213* | G | 827 | A | UBA7 | Q92H | T | 435 | G | WDR66 | R213S | A | 781 | C |

| | | | | | | | | | | | | | | | | | | | |
|---|---|---|---|---|---|---|---|---|---|---|---|---|---|---|---|---|---|---|---|
| TEX15 | S583Y | G | 1748 | T | TP53 | T125M | G | 564 | A | UBAC1 | R404H | C | 1429 | T | WDR66 | M350I | G | 1192 | T |
| TEX15 | S88L | G | 263 | A | TP53 | - | C | 0 | T | UBAP1 | K60N | G | 415 | T | WDR66 | G750E | G | 2391 | A |
| TEX15 | P2762T | G | 8284 | T | TP53 | G245S | C | 923 | T | UBAP2 | S685P | A | 2171 | G | WDR66 | K828N | A | 2626 | C |
| TEX15 | S2494Y | G | 7481 | T | TP53 | R175H | C | 714 | T | UBAP2 | L575F | C | 1843 | A | WDR67 | S131L | C | 482 | T |
| TEX15 | L1437R | A | 4310 | C | TP53 | R248L | C | 933 | A | UBAP2L | P17L | C | 217 | T | WDR67 | D638N | G | 2002 | A |
| TEX15 | S401A | A | 1201 | C | TP53 | G245D | C | 924 | T | UBASH3A | T118M | C | 384 | T | WDR67 | L842H | T | 2615 | A |
| TEX15 | S30Y | G | 89 | T | TP53 | R282W | G | 1034 | A | UBASH3A | T325M | C | 1005 | T | WDR67 | A843T | G | 2617 | A |
| TEX15 | A29V | G | 86 | A | TP53 | R273H | C | 1008 | T | UBASH3A | P603L | C | 1839 | T | WDR7 | - | G | 0 | A |
| TEX2 | E543* | C | 1778 | A | TP53 | L257Q | A | 960 | T | UBASH3B | D597G | A | 2150 | G | WDR7 | N1461H | A | 4592 | C |
| TEX2 | S91L | G | 423 | A | TP53 | R273H | C | 1008 | T | UBASH3B | A182V | C | 905 | T | WDR7 | V1107A | T | 3531 | C |
| TEX2 | V785M | C | 2504 | T | TP53 | R175H | C | 714 | T | UBASH3B | F132L | C | 756 | A | WDR7 | R1474C | C | 4631 | T |
| TEX2 | G359R | C | 1226 | T | TP53 | - | C | 0 | A | UBASH3B | R416H | G | 1607 | A | WDR72 | K142N | T | 666 | G |
| TEX2 | K1087N | C | 3412 | A | TP53 | R282W | G | 1034 | A | UBE2A | I33T | T | 272 | C | WDR72 | S928Y | G | 3023 | T |
| TEX2 | E470K | C | 1559 | T | TP53 | R248W | G | 932 | A | UBE2D4 | R90L | G | 358 | T | WDR72 | S894Y | G | 2921 | T |
| TEX9 | E296D | G | 912 | T | TP53 | C176F | C | 717 | A | UBE2E2 | - | G | 0 | T | WDR72 | Y874C | T | 2861 | C |
| TEX9 | S391A | T | 1195 | G | TP53 | R273C | G | 1007 | A | UBE2E3 | I211M | T | 1026 | G | WDR72 | L862V | A | 2824 | C |
| TF | G636D | G | 3635 | A | TP53 | R248Q | C | 933 | T | UBE2F | F153C | T | 652 | G | WDR72 | T719I | G | 2396 | A |
| TF | A398T | G | 2920 | A | TP53 | R248W | G | 932 | A | UBE2G1 | P97H | G | 648 | T | WDR72 | E499* | C | 1735 | A |
| TF | V120M | G | 2086 | A | TP53 | H193Y | G | 767 | A | UBE2G2 | A155V | G | 735 | A | WDR75 | F233L | T | 759 | G |
| TF | G484D | G | 3179 | A | TP53 | R175H | C | 714 | T | UBE2I | G47R | G | 256 | A | WDR76 | P234Q | C | 771 | A |
| TFAM | - | G | 0 | A | TP53 | G244C | C | 920 | A | UBE2N | A126V | G | 743 | A | WDR76 | K45Q | A | 203 | C |
| TFAP2B | A381V | C | 1308 | T | TP53BP1 | R1405* | G | 4341 | A | UBE2N | I39V | T | 481 | C | WDR76 | E264A | A | 861 | C |
| TFAP2B | S125L | C | 540 | T | TP53BP1 | R1405* | G | 4341 | A | UBE2NL | P98T | C | 322 | A | WDR77 | G82E | C | 452 | T |
| TFAP2B | S216L | C | 813 | T | TP53BP1 | Q1943* | G | 5955 | A | UBE2NL | A137D | C | 440 | A | WDR78 | K382N | T | 1202 | G |
| TFAP2B | V280F | G | 1004 | T | TP53BP1 | L1822M | G | 5592 | T | UBE2O | D884N | C | 2715 | T | WDR78 | - | C | 0 | T |
| TFAP2D | R132W | C | 622 | T | TP53BP1 | P1144H | G | 3559 | T | UBE2O | V169I | C | 570 | T | WDR8 | R115W | G | 417 | A |
| TFAP2D | A119V | C | 584 | T | TP53BP1 | R1961I | C | 6010 | A | UBE2O | P892S | G | 2739 | A | WDR8 | E55D | C | 239 | A |
| TFAP2D | D17N | G | 277 | A | TP53BP1 | S899R | T | 2823 | G | UBE2Q1 | Q173* | G | 597 | A | WDR8 | E371K | C | 1185 | T |
| TFAP2D | E267D | G | 1029 | T | TP53BP1 | E687* | C | 2187 | A | UBE2Q1 | V131I | C | 471 | T | WDR8 | V356I | C | 1140 | T |
| TFAP2E | S434L | C | 1509 | T | TP53BP2 | H924R | T | 2771 | C | UBE2R2 | A60V | C | 370 | T | WDR8 | V75A | A | 298 | G |
| TFAP4 | R293Q | C | 1132 | T | TP53BP2 | F1052C | A | 3155 | C | UBE2R2 | A44T | G | 321 | A | WDR81 | V928M | G | 2782 | A |
| TFB2M | C42G | A | 310 | C | TP53BP2 | D837A | T | 2510 | G | UBE2S | G30D | C | 457 | T | WDR81 | V1071I | G | 3211 | A |
| TFB2M | K356E | T | 1252 | C | TP53BP2 | K739N | C | 2217 | A | UBE2T | R35* | G | 253 | A | WDR81 | A1277T | G | 3829 | A |
| TFCP2 | G370A | C | 1568 | G | TP53INP1 | Y240N | A | 1102 | T | UBE2U | L16I | C | 470 | A | WDR81 | P759L | C | 2276 | T |
| TFCP2 | - | C | 0 | A | TP53TG5 | R280K | C | 996 | T | UBE2V2 | N92H | A | 294 | C | WDR81 | P318L | C | 953 | T |
| TFCP2L1 | S310L | G | 1027 | A | TP63 | R266* | C | 885 | T | UBE2Z | D140Y | G | 553 | T | WDR81 | A478T | G | 1432 | A |
| TFCP2L1 | T229A | T | 783 | C | TP63 | R655Q | G | 2053 | A | UBE2Z | R164W | C | 625 | T | WDR81 | P191S | C | 571 | T |
| TFDP1 | G115D | G | 556 | A | TP63 | T39A | A | 204 | G | UBE2Z | K113T | A | 473 | C | WDR81 | T420I | C | 1259 | T |
| TFDP1 | A72V | C | 427 | T | TPCN1 | A413T | G | 1237 | A | UBE2Z | N241S | A | 857 | G | WDR81 | R1673H | G | 5018 | A |

| | | | | | | | | | | | | | | | | | | | |
|---|---|---|---|---|---|---|---|---|---|---|---|---|---|---|---|---|---|---|---|
| TFDP1 | I153T | T | 670 | C | TPCN1 | T498M | C | 1493 | T | UBE3A | R168Q | C | 503 | T | WDR81 | L1397M | C | 4189 | A |
| TFDP2 | N262S | T | 1216 | C | TPCN1 | - | T | 0 | C | UBE3A | R424Q | C | 1271 | T | WDR82 | R219C | G | 937 | A |
| TFDP3 | V34M | C | 189 | T | TPCN1 | A448V | C | 1343 | T | UBE3A | I352T | A | 1055 | G | WDR82 | R193Q | C | 860 | T |
| TFDP3 | K229N | C | 776 | A | TPCN1 | R800W | C | 2398 | T | UBE3A | M545T | A | 1634 | G | WDR83 | R304* | C | 1245 | T |
| TFEB | S381Y | G | 1147 | T | TPCN1 | A873T | G | 2617 | A | UBE3A | A67T | C | 199 | T | WDR87 | A2576V | G | 7888 | A |
| TFEB | A85V | G | 259 | A | TPCN2 | R187C | C | 675 | T | UBE3A | E190* | C | 568 | A | WDR87 | E1952D | C | 6017 | A |
| TFEB | A82V | G | 250 | A | TPD52 | T162A | T | 806 | C | UBE3B | R686H | G | 2660 | A | WDR87 | K1410N | C | 4391 | A |
| TFEB | P419L | G | 1261 | A | TPD52L1 | A95V | C | 503 | T | UBE3B | A415T | G | 1846 | A | WDR87 | H2609Q | A | 7988 | C |
| TFF2 | E99K | C | 466 | T | TPD52L3 | K93N | G | 500 | T | UBE3B | N136S | A | 1010 | G | WDR87 | H885R | T | 2815 | C |
| TFG | A382V | C | 1242 | T | TPH2 | E26K | G | 217 | A | UBE3B | L444I | C | 1933 | A | WDR87 | P2673T | G | 8178 | T |
| TFIP11 | K170N | C | 794 | A | TPH2 | K374N | G | 1263 | T | UBE3C | A897V | C | 3050 | T | WDR87 | L2595I | G | 7944 | T |
| TFIP11 | R386Q | C | 1441 | T | TPK1 | I135M | A | 508 | C | UBE4A | L929R | T | 2915 | G | WDR87 | R986H | C | 3118 | T |
| TFIP11 | A714V | G | 2425 | A | TPM1 | E82K | G | 323 | A | UBE4B | T1274M | C | 3896 | T | WDR87 | E2828K | C | 8643 | T |
| TFIP11 | R386Q | C | 1441 | T | TPM3 | Q217H | T | 691 | G | UBE4B | R1255C | C | 3838 | T | WDR87 | K2331N | C | 7154 | A |
| TFIP11 | E405K | C | 1497 | T | TPM4 | R91C | C | 389 | T | UBE4B | R331C | C | 1066 | T | WDR87 | K1883N | C | 5810 | A |
| TFPI | R243H | C | 756 | T | TPM4 | E234D | G | 820 | T | UBE4B | F953L | T | 2934 | G | WDR87 | K1867N | C | 5762 | A |
| TFPI | C175Y | C | 552 | T | TPMT | F99C | A | 382 | C | UBE4B | L1064F | G | 3267 | T | WDR87 | R1841M | C | 5683 | A |
| TFPI | R69I | C | 234 | A | TPO | G842R | G | 2607 | A | UBL5 | L30P | T | 193 | C | WDR87 | D1284N | C | 4011 | T |
| TFPI2 | Q63R | T | 501 | C | TPO | G534V | G | 1684 | T | UBLCP1 | - | G | 0 | A | WDR87 | D1219Y | C | 3816 | A |
| TFPI2 | A185S | C | 866 | A | TPO | D585N | G | 1836 | A | UBLCP1 | E283* | G | 983 | T | WDR87 | R906Q | C | 2878 | T |
| TFPI2 | G108W | C | 635 | A | TPO | Q550R | A | 1732 | G | UBN1 | P328H | C | 1322 | A | WDR87 | Q726H | C | 2339 | A |
| TFPT | R190W | G | 974 | A | TPO | R438C | C | 1395 | T | UBN1 | A455V | C | 1703 | T | WDR87 | K32N | C | 257 | A |
| TFR2 | T553A | T | 1698 | C | TPP1 | T188S | T | 596 | A | UBN2 | G1101C | G | 3858 | T | WDR88 | D364Y | G | 1168 | T |
| TFR2 | R152C | G | 495 | A | TPP1 | A344T | C | 1064 | T | UBN2 | L1302V | T | 4461 | G | WDR88 | A25T | G | 151 | A |
| TFR2 | D204G | T | 652 | C | TPP1 | S233N | C | 732 | T | UBOX5 | G135D | C | 876 | T | WDR88 | R308* | C | 1000 | T |
| TG | A2692D | C | 8116 | A | TPP2 | V1261A | T | 3798 | C | UBP1 | S291F | G | 1402 | A | WDR89 | C221G | A | 800 | C |
| TG | A53V | C | 199 | T | TPP2 | R282W | C | 860 | T | UBQLN1 | I84F | T | 774 | A | WDR90 | V364I | G | 1144 | A |
| TG | L2634F | C | 7941 | T | TPP2 | A272T | G | 830 | A | UBQLN1 | R274C | G | 1344 | A | WDR90 | D669N | G | 2059 | A |
| TG | R2379H | G | 7177 | A | TPP2 | A507V | C | 1536 | T | UBQLN1 | G483W | C | 1971 | A | WDR90 | S916L | C | 2801 | T |
| TG | Q45H | A | 176 | C | TPPP3 | D97N | C | 451 | T | UBQLN2 | F46L | T | 371 | C | WDR90 | V1659A | T | 5030 | C |
| TG | E560D | G | 1721 | T | TPR | E584D | C | 2049 | G | UBQLN3 | F65V | A | 341 | C | WDR90 | T142I | C | 479 | T |
| TG | L1322S | T | 4006 | C | TPR | R1808Q | C | 5720 | T | UBQLN3 | G429C | C | 1433 | A | WDR90 | A52T | G | 208 | A |
| TG | A1886V | C | 5698 | T | TPR | M1320T | A | 4256 | G | UBQLN3 | R624W | G | 2018 | A | WDR90 | T1709I | C | 5180 | T |
| TG | S1988Y | C | 6004 | A | TPR | G1901E | C | 5999 | T | UBQLN3 | R624W | G | 2018 | A | WDR90 | V575M | G | 1777 | A |
| TG | R2555H | G | 7705 | A | TPR | A1995D | G | 6281 | T | UBQLN3 | A2V | G | 153 | A | WDR91 | E611K | C | 1863 | T |
| TGFA | R140Q | C | 816 | T | TPRA1 | A232T | C | 985 | T | UBQLN4 | G346S | C | 1129 | T | WDR91 | R476C | G | 1458 | A |
| TGFB1 | A262T | C | 1651 | T | TPRX1 | G283C | C | 918 | A | UBQLN4 | R298Q | C | 986 | T | WDR91 | Y620H | A | 1890 | G |
| TGFB1I1 | R95W | C | 399 | T | TPSD1 | N223K | T | 817 | A | UBQLNL | E210D | T | 894 | G | WDR91 | A731T | C | 2223 | T |
| TGFB1I1 | E394D | G | 1298 | T | TPSG1 | A127T | C | 381 | T | UBQLNL | A22E | G | 329 | T | WDR92 | P100S | G | 415 | A |

| | | | | | | | | | | | | | | | | | | | |
|---|---|---|---|---|---|---|---|---|---|---|---|---|---|---|---|---|---|---|---|
| TGFB2 | P255S | C | 1230 | T | TPSG1 | R56H | C | 169 | T | UBQLNL | D43Y | C | 391 | A | WDSUB1 | C160W | A | 721 | C |
| TGFB2 | R181C | C | 1008 | T | TPSG1 | V107M | C | 321 | T | UBR1 | L1718F | G | 5231 | A | WEE1 | P517L | C | 1601 | T |
| TGFB2 | K301E | A | 1368 | G | TPSG1 | D321E | G | 965 | T | UBR1 | R1716W | G | 5225 | A | WEE1 | D271G | A | 863 | G |
| TGFBI | T238I | C | 874 | T | TPST2 | A200V | G | 870 | A | UBR1 | L790V | A | 2447 | C | WEE1 | R533W | C | 1648 | T |
| TGFBI | A650V | C | 2110 | T | TPST2 | A90T | C | 539 | T | UBR1 | E686* | C | 2135 | A | WEE2 | I218N | T | 1059 | A |
| TGFBI | L509P | T | 1687 | C | TPST2 | R78L | C | 504 | A | UBR2 | L1504M | C | 4768 | A | WFDC12 | L56M | G | 184 | T |
| TGFBI | N609S | A | 1987 | G | TPT1 | E32A | T | 112 | G | UBR2 | R522H | G | 1823 | A | WFDC12 | C34Y | C | 119 | T |
| TGFBI | A659T | G | 2136 | A | TPTE | - | C | 0 | T | UBR2 | S377I | G | 1388 | T | WFDC6 | K67N | C | 201 | A |
| TGFBI | V322A | T | 1126 | C | TPTE | L183V | A | 547 | C | UBR2 | R200W | C | 856 | T | WFDC6 | I139V | T | 415 | C |
| TGFBI | N437S | A | 1471 | G | TPTE | G341S | C | 1021 | T | UBR2 | E1303D | A | 4167 | C | WFDC8 | S15I | C | 123 | A |
| TGFBR2 | R403M | G | 1491 | T | TPTE | L463* | A | 1388 | C | UBR3 | D342V | A | 1025 | T | WFDC8 | T121I | G | 441 | A |
| TGFBR3 | T747M | G | 2706 | A | TPTE | Q434H | T | 1302 | G | UBR3 | E790K | G | 2368 | A | WFIKKN1 | R266C | C | 1118 | T |
| TGFBRAP1 | R426C | G | 1703 | A | TPTE | L271I | G | 811 | T | UBR3 | P1556H | C | 4667 | A | WFIKKN1 | R413H | G | 1560 | A |
| TGFBRAP1 | V244M | C | 1157 | T | TPTE | N332T | T | 995 | G | UBR4 | R2703* | G | 8111 | A | WFIKKN1 | C347Y | G | 1362 | A |
| TGFBRAP1 | E747* | C | 2666 | A | TPTE2 | R214S | C | 687 | A | UBR4 | R666Q | C | 2001 | T | WFIKKN1 | R283* | C | 1169 | T |
| TGIF1 | Q195* | C | 886 | T | TPTE2 | E512* | C | 1579 | A | UBR4 | T1162M | G | 3489 | A | WFS1 | V803M | G | 2574 | A |
| TGIF2 | A115T | G | 542 | A | TPTE2 | V88A | A | 308 | G | UBR4 | R2272H | C | 6819 | T | WHAMM | L701M | T | 2607 | A |
| TGIF2 | E140K | G | 617 | A | TPX2 | E651K | G | 2479 | A | UBR4 | E3196K | C | 9590 | T | WHSC1 | G92D | G | 423 | A |
| TGIF2LX | G150C | G | 497 | T | TPX2 | R267* | C | 1327 | T | UBR4 | Q4234* | G | 12704 | A | WHSC1 | A427T | G | 1427 | A |
| TGM2 | R296C | G | 1060 | A | TPX2 | E474D | G | 1950 | T | UBR4 | L1492R | A | 4479 | C | WHSC1 | E104* | G | 458 | T |
| TGM2 | R213C | G | 811 | A | TRA2A | R189I | C | 783 | A | UBR4 | A428V | G | 1287 | A | WHSC1 | E1339D | G | 4165 | T |
| TGM2 | T360M | G | 1253 | A | TRA2B | T201M | G | 745 | A | UBR4 | C4049R | A | 12149 | G | WHSC1 | E104K | G | 458 | A |
| TGM2 | N586T | T | 1931 | G | TRAC | R84C | C | 248 | T | UBR4 | I4213V | T | 12641 | C | WHSC1 | R660Q | G | 2127 | A |
| TGM2 | D400N | C | 1372 | T | TRADD | R278H | C | 890 | T | UBR4 | R3498W | G | 10496 | A | WHSC1 | R401W | C | 1349 | T |
| TGM3 | R53K | G | 221 | A | TRAF2 | A127V | C | 425 | T | UBR4 | C1380* | A | 4144 | T | WHSC1 | G795R | G | 2531 | A |
| TGM3 | G93D | G | 341 | A | TRAF3IP2 | Q377P | T | 1130 | G | UBR4 | S950Y | G | 2853 | T | WHSC1 | A165T | G | 641 | A |
| TGM3 | S67L | C | 263 | T | TRAF3IP3 | L502R | T | 1925 | G | UBR5 | R1978* | G | 5965 | A | WHSC1L1 | E246* | C | 1254 | A |
| TGM3 | F330V | T | 1051 | G | TRAF3IP3 | K214T | A | 1061 | C | UBR5 | A2585V | G | 7787 | A | WHSC1L1 | R211C | G | 1149 | A |
| TGM3 | P647L | C | 2003 | T | TRAF5 | N97K | T | 376 | G | UBR5 | I2449V | T | 7378 | C | WHSC1L1 | E728G | T | 2701 | C |
| TGM4 | N550D | A | 1716 | G | TRAF6 | R332Q | C | 1020 | T | UBR5 | L419S | A | 1289 | G | WHSC1L1 | S854I | C | 3079 | A |
| TGM4 | F15L | C | 113 | A | TRAF7 | S348N | G | 1175 | A | UBR5 | S1857P | A | 5602 | G | WHSC1L1 | A564S | C | 2208 | A |
| TGM4 | R431W | C | 1359 | T | TRAF7 | R371W | C | 1243 | T | UBR5 | R2353Q | C | 7091 | T | WHSC2 | G29V | C | 1204 | A |
| TGM4 | N595S | A | 1852 | G | TRAFD1 | K101T | A | 919 | C | UBR5 | N2795K | A | 8418 | C | WHSC2 | P253L | G | 1876 | A |
| TGM4 | P340S | C | 1086 | T | TRAK1 | R506Q | G | 1917 | A | UBR5 | R2485Q | C | 7487 | T | WIF1 | R107C | G | 464 | A |
| TGM6 | G199S | G | 656 | A | TRAK1 | R351W | C | 1451 | T | UBR5 | W494G | A | 1513 | C | WIF1 | W332* | C | 1141 | T |
| TGM6 | D160N | G | 539 | A | TRAK1 | R401C | C | 1601 | T | UBR7 | E90* | G | 504 | T | WIF1 | P308S | G | 1067 | A |
| TGM6 | Q683* | C | 2108 | T | TRAK2 | R196Q | C | 1016 | T | UBTD1 | R154C | C | 796 | T | WIPF1 | D60H | C | 342 | G |
| TGOLN2 | K288T | T | 925 | G | TRAM1L1 | L218F | C | 841 | A | UBTD1 | R64H | G | 527 | A | WIPF1 | P458S | G | 1536 | A |
| TGOLN2 | G186S | C | 618 | T | TRAM1L1 | V358A | A | 1260 | G | UBTD2 | R28H | C | 489 | T | WIPF1 | P458L | G | 1537 | A |

| | | | | | | | | | | | | | | | | | | | |
|---|---|---|---|---|---|---|---|---|---|---|---|---|---|---|---|---|---|---|---|
| TGS1 | L43* | T | 605 | G | TRANK1 | E1320* | C | 3958 | A | UBTF | L365M | G | 1160 | T | WIPF3 | P309S | C | 1175 | T |
| TGS1 | I790M | T | 2847 | G | TRANK1 | A1729T | C | 5185 | T | UBTF | D689G | T | 2133 | C | WIPF3 | R409* | C | 1475 | T |
| TH1L | Q444P | A | 1373 | C | TRANK1 | P723L | G | 2168 | A | UBTFL2 | N50Y | T | 157 | A | WIPI1 | V385I | C | 1244 | T |
| THADA | Y563H | A | 1827 | G | TRANK1 | Q663R | T | 1988 | C | UBTFL2 | K49T | T | 155 | G | WIPI2 | S114I | G | 573 | T |
| THADA | K1633N | C | 5039 | A | TRANK1 | D83N | C | 247 | T | UBXN10 | R38M | G | 197 | T | WISP1 | P279S | C | 911 | T |
| THADA | P1582S | G | 4884 | A | TRANK1 | E1548D | C | 4644 | G | UBXN4 | R463K | G | 1699 | A | WISP1 | P60L | C | 255 | T |
| THADA | C1398G | A | 4332 | C | TRAP1 | A571T | C | 1767 | T | UBXN4 | L428F | A | 1595 | C | WISP2 | L47M | C | 482 | A |
| THADA | F665L | G | 2135 | T | TRAP1 | K126N | T | 434 | G | UBXN8 | G39D | G | 116 | A | WISP3 | K233T | A | 743 | C |
| THADA | I85M | A | 395 | C | TRAP1 | R402Q | C | 1261 | T | UCHL5 | L190F | T | 570 | G | WIZ | G1492D | C | 4689 | T |
| THADA | K67T | T | 340 | G | TRAP1 | D395G | T | 1240 | C | UCK1 | V179M | C | 561 | T | WIZ | R1332H | C | 4209 | T |
| THAP1 | D15E | G | 282 | T | TRAPPC1 | A103V | G | 427 | A | UCK1 | A4V | G | 37 | A | WIZ | G42D | C | 339 | T |
| THAP11 | K38N | G | 359 | T | TRAPPC1 | R127H | C | 499 | T | UCK1 | R177Q | C | 556 | T | WIZ | R1357W | G | 4283 | A |
| THAP2 | R41H | G | 628 | A | TRAPPC10 | S193Y | C | 753 | A | UCK2 | L138R | T | 716 | G | WLS | P508Q | G | 1769 | T |
| THAP2 | S88Y | C | 769 | A | TRAPPC10 | F223L | C | 844 | A | UCKL1 | M463T | A | 1431 | G | WLS | A539T | C | 1861 | T |
| THAP5 | V338D | A | 1167 | T | TRAPPC10 | R330C | C | 1163 | T | UCMA | A39V | G | 189 | A | WLS | R73* | G | 463 | A |
| THAP5 | R73* | G | 371 | A | TRAPPC10 | S1109L | C | 3501 | T | UCMA | E88* | C | 335 | A | WNK1 | I1371S | T | 4755 | G |
| THAP7 | T134I | G | 576 | A | TRAPPC10 | E1255G | A | 3939 | G | UCN | L54F | G | 459 | A | WNK1 | A1233V | C | 4341 | T |
| THAP9 | D394N | G | 1231 | A | TRAPPC5 | R72H | G | 285 | A | UCP2 | V285M | C | 1233 | T | WNK1 | S1686R | C | 5701 | A |
| THAP9 | F607C | T | 1871 | G | TRAPPC5 | A112V | C | 405 | T | UCP2 | R71H | C | 592 | T | WNK1 | A779T | G | 2978 | A |
| THBD | A555V | G | 1901 | A | TRAPPC5 | G155A | G | 534 | C | UCP2 | V289I | C | 1245 | T | WNK1 | A1024E | C | 3714 | A |
| THBD | Y376C | T | 1364 | C | TRAPPC6A | A144T | C | 439 | T | UCP3 | R119W | G | 710 | A | WNK2 | S2093C | A | 6277 | T |
| THBD | T500A | T | 1735 | C | TRAPPC9 | - | A | 0 | G | UCP3 | R87H | C | 615 | T | WNK2 | P885T | C | 2653 | A |
| THBD | R200C | G | 835 | A | TRAPPC9 | R1091G | T | 3271 | C | UEVLD | G13D | C | 118 | T | WNK2 | A837T | G | 2509 | A |
| THBS1 | G1136* | G | 3738 | T | TRAPPC9 | - | C | 0 | T | UEVLD | R313* | G | 1017 | A | WNK2 | A1036T | G | 3106 | A |
| THBS1 | A165V | C | 826 | T | TRAPPC9 | E388D | T | 1164 | G | UFD1L | R292I | C | 953 | A | WNK2 | Q1249* | C | 3745 | T |
| THBS2 | P386L | G | 1407 | A | TRBC2 | A68T | G | 201 | A | UFD1L | - | T | 1110 | G | WNK2 | L1239M | C | 3715 | A |
| THBS2 | R59H | C | 426 | T | TRBC2 | - | A | 0 | C | UGCG | R246Q | G | 1187 | A | WNK3 | E380* | C | 1577 | A |
| THBS2 | P623L | G | 2118 | A | TRBC2 | A68T | G | 201 | A | UGDH | F71L | A | 556 | C | WNK3 | C120Y | C | 798 | T |
| THBS2 | R496H | C | 1737 | T | TRBJ2-3 | R12Q | G | 35 | A | UGGT1 | R1552C | C | 4701 | T | WNK3 | E1461D | C | 4822 | A |
| THBS2 | C622Y | C | 2115 | T | TRBJ2-3 | P9S | C | 25 | T | UGGT1 | V451E | T | 1399 | A | WNK3 | K443R | T | 1767 | C |
| THBS2 | S162N | C | 735 | T | TRBJ2-6 | G12S | G | 32 | A | UGGT2 | R665C | G | 2064 | A | WNK4 | R151Q | G | 473 | A |
| THBS2 | P357S | G | 1319 | A | TRBJ2-6 | S2Y | C | 3 | A | UGGT2 | L810F | G | 2499 | A | WNK4 | R469W | C | 1426 | T |
| THBS2 | D551Y | C | 1901 | A | TRBV10-2 | A70V | G | 260 | A | UGGT2 | F711L | G | 2204 | T | WNT10A | F294L | T | 1513 | C |
| THBS2 | T16M | G | 297 | A | TRBV10-2 | T101A | T | 352 | C | UGGT2 | K604Q | T | 1881 | G | WNT10A | T387M | C | 1793 | T |
| THBS2 | K474N | C | 1672 | A | TRBV19 | A111V | C | 540 | T | UGT1A1 | L66V | T | 236 | G | WNT10B | F19L | A | 404 | G |
| THBS2 | D687N | C | 2309 | T | TRBV28 | R8H | G | 43 | A | UGT1A1 | F145V | T | 473 | G | WNT16 | L6P | T | 307 | C |
| THBS3 | D540N | C | 1639 | T | TRBV29-1 | G55E | G | 231 | A | UGT1A10 | A25T | G | 142 | A | WNT16 | A4V | C | 301 | T |
| THBS3 | R401H | C | 1223 | T | TRBV5-1 | L109I | C | 634 | A | UGT1A3 | E72D | G | 235 | T | WNT16 | F185V | T | 843 | G |
| THBS3 | L59V | A | 196 | C | TRBV5-3 | R86C | G | 256 | A | UGT1A7 | D131Y | G | 391 | T | WNT16 | S241Y | C | 1012 | A |

| | | | | | | | | | | | | | | | | | | | |
|---|---|---|---|---|---|---|---|---|---|---|---|---|---|---|---|---|---|---|---|
| WNT2 | V334M | C | 1300 | T | ZFAT | N495I | T | 1659 | A | ZNF317 | Q54H | G | 450 | T | ZNF616 | R625I | C | 2157 | A |
| WNT2 | E272D | C | 1116 | A | ZFC3H1 | L1017P | A | 3409 | G | ZNF318 | R525* | G | 1651 | A | ZNF616 | E15* | C | 326 | A |
| WNT2 | D232N | C | 994 | T | ZFC3H1 | P1825L | G | 5833 | A | ZNF318 | R225Q | C | 752 | T | ZNF618 | A805V | C | 2513 | T |
| WNT2B | R112H | G | 820 | A | ZFC3H1 | P549L | G | 2005 | A | ZNF318 | K566M | T | 1775 | A | ZNF618 | E823K | G | 2566 | A |
| WNT2B | G195S | G | 1068 | A | ZFC3H1 | R696Q | C | 2446 | T | ZNF318 | A766T | C | 2374 | T | ZNF620 | R7H | G | 169 | A |
| WNT2B | R115H | G | 829 | A | ZFC3H1 | Q388H | T | 1523 | G | ZNF318 | L1400I | G | 4276 | T | ZNF621 | H285N | C | 1076 | A |
| WNT2B | K330Q | A | 1473 | C | ZFC3H1 | K294N | T | 1241 | G | ZNF318 | G1912* | C | 5812 | A | ZNF622 | A40D | G | 246 | T |
| WNT3 | A223V | G | 686 | A | ZFC3H1 | S40I | C | 478 | A | ZNF318 | D2005G | T | 6092 | C | ZNF622 | - | C | 0 | A |
| WNT4 | T42M | G | 169 | A | ZFC3H1 | N32T | T | 454 | G | ZNF318 | R1196W | G | 3664 | A | ZNF623 | R301C | C | 990 | T |
| WNT4 | V288M | C | 906 | T | ZFHX3 | C1988R | A | 6635 | G | ZNF319 | Q354* | G | 1683 | A | ZNF624 | S207F | G | 703 | A |
| WNT5A | V123G | A | 890 | C | ZFHX3 | P192L | G | 1248 | A | ZNF319 | Q61H | C | 806 | A | ZNF624 | T684A | T | 2133 | C |
| WNT5A | R354H | C | 1583 | T | ZFHX3 | R60C | G | 851 | A | ZNF319 | A569V | G | 2329 | A | ZNF624 | S568Y | G | 1786 | T |
| WNT7A | R90L | C | 574 | A | ZFHX3 | R2149H | C | 7119 | T | ZNF320 | Q100R | T | 447 | C | ZNF624 | N184K | A | 635 | C |
| WNT7A | C200W | A | 905 | C | ZFHX3 | V907A | A | 3393 | G | ZNF320 | R313* | G | 1085 | A | ZNF625 | E218D | C | 654 | A |
| WNT7A | T338M | G | 1318 | A | ZFHX3 | A102V | G | 978 | A | ZNF324 | D87G | A | 354 | G | ZNF625 | D190Y | C | 568 | A |
| WNT7A | G303S | C | 1212 | T | ZFHX3 | R990H | C | 3642 | T | ZNF324 | R161Q | G | 576 | A | ZNF626 | R361S | T | 1233 | A |
| WNT7B | C218Y | C | 1131 | T | ZFHX3 | L1788R | A | 6036 | C | ZNF324 | V482M | G | 1538 | A | ZNF626 | K124E | T | 520 | C |
| WNT7B | R246W | G | 1214 | A | ZFHX3 | Y3066C | T | 9870 | C | ZNF324B | R152C | C | 561 | T | ZNF627 | R351* | C | 1259 | T |
| WNT7B | K224T | T | 1149 | G | ZFHX4 | R2917* | C | 9128 | T | ZNF324B | C198Y | G | 700 | A | ZNF627 | R435Q | G | 1512 | A |
| WNT8A | L90F | C | 504 | T | ZFHX4 | R104H | G | 690 | A | ZNF326 | A288V | C | 863 | T | ZNF627 | R271* | C | 1019 | T |
| WNT8A | E128K | G | 618 | A | ZFHX4 | A1707T | G | 5498 | A | ZNF329 | R528Q | C | 1819 | T | ZNF628 | P91H | C | 837 | A |
| WNT8A | K240R | A | 955 | G | ZFHX4 | A2547T | G | 8018 | A | ZNF329 | R223Q | C | 904 | T | ZNF628 | A783V | C | 2913 | T |
| WNT8A | G134W | G | 636 | T | ZFHX4 | P395L | C | 1563 | T | ZNF329 | N495K | G | 1721 | T | ZNF628 | T221M | C | 1227 | T |
| WNT8A | K333T | A | 1234 | C | ZFHX4 | F1872V | T | 5993 | G | ZNF329 | Y483C | T | 1684 | C | ZNF628 | A734T | G | 2765 | A |
| WNT8B | T101P | A | 429 | C | ZFHX4 | N2181T | A | 6921 | C | ZNF329 | K378N | T | 1370 | G | ZNF629 | E528G | T | 1791 | C |
| WNT8B | R239H | G | 844 | A | ZFHX4 | S122I | G | 744 | T | ZNF331 | K437E | A | 2642 | G | ZNF630 | T598P | T | 2019 | G |
| WNT9A | R70H | C | 220 | T | ZFHX4 | A2394T | G | 7559 | A | ZNF331 | R225W | C | 2006 | T | ZNF641 | R124W | G | 1079 | A |
| WNT9A | G240D | C | 730 | T | ZFHX4 | A366T | G | 1475 | A | ZNF333 | R360H | G | 1213 | A | ZNF642 | E12K | G | 1040 | A |
| WNT9B | R30Q | G | 126 | A | ZFHX4 | L363V | T | 1466 | G | ZNF333 | G418R | G | 1386 | A | ZNF643 | R300I | G | 898 | T |
| WRN | R499I | G | 2284 | T | ZFHX4 | D883Y | G | 3026 | T | ZNF333 | V523M | G | 1701 | A | ZNF644 | E519* | C | 1773 | A |
| WRN | K934Q | A | 3588 | C | ZFHX4 | L1355S | T | 4443 | C | ZNF333 | R554Q | G | 1795 | A | ZNF644 | R1099H | C | 3514 | T |
| WRNIP1 | R624G | A | 2079 | G | ZFHX4 | S1564Y | C | 5070 | A | ZNF334 | E648D | C | 3138 | A | ZNF644 | M1082L | T | 3462 | G |
| WRNIP1 | A621T | G | 2070 | A | ZFHX4 | N2468S | A | 7782 | G | ZNF334 | R615Q | C | 3038 | T | ZNF646 | R1300C | C | 4162 | T |
| WRNIP1 | D9N | G | 234 | A | ZFHX4 | S2789L | C | 8745 | T | ZNF334 | R420I | C | 2453 | A | ZNF646 | P1374H | C | 4385 | A |
| WRNIP1 | A149E | C | 655 | A | ZFP1 | F184C | T | 715 | G | ZNF334 | E136D | C | 1602 | A | ZNF646 | G1432* | G | 4558 | T |
| WSB1 | A306T | G | 1232 | A | ZFP1 | R288I | G | 1027 | T | ZNF335 | R1131M | C | 3493 | A | ZNF646 | P45T | C | 397 | A |
| WSB1 | F70S | T | 525 | C | ZFP106 | S1509Y | G | 4853 | T | ZNF335 | R505C | G | 1614 | A | ZNF646 | A1408V | C | 4487 | T |
| WSB1 | I109V | A | 641 | G | ZFP106 | R935* | G | 3130 | A | ZNF335 | R1092Q | C | 3376 | T | ZNF646 | H832Y | C | 2758 | T |
| WSB2 | F58L | A | 172 | G | ZFP106 | K1354Q | T | 4387 | G | ZNF335 | A1064V | G | 3292 | A | ZNF646 | R1062H | G | 3449 | A |

| | | | | | | | | | | | | | | | | | | | |
|---|---|---|---|---|---|---|---|---|---|---|---|---|---|---|---|---|---|---|---|
| WSCD1 | A2V | C | 332 | T | ZFP112 | H586Y | G | 1836 | A | ZNF335 | A702T | C | 2205 | T | ZNF648 | E96D | T | 496 | G |
| WSCD1 | T275A | A | 1150 | G | ZFP112 | R556G | T | 1746 | C | ZNF337 | H342Y | G | 1564 | A | ZNF648 | R272H | C | 1023 | T |
| WSCD2 | C300Y | G | 1643 | A | ZFP112 | K901T | T | 2782 | G | ZNF33A | E563K | G | 1760 | A | ZNF648 | R384H | C | 1359 | T |
| WSCD2 | Y583* | C | 2493 | A | ZFP112 | E595D | C | 1865 | A | ZNF33A | E647K | G | 2012 | A | ZNF649 | G108R | C | 590 | T |
| WSCD2 | Y583* | C | 2493 | A | ZFP112 | N350T | T | 1129 | G | ZNF33A | K319* | A | 1028 | T | ZNF649 | P129A | G | 653 | C |
| WSCD2 | D69G | A | 950 | G | ZFP112 | S182I | C | 625 | A | ZNF33A | D149A | A | 519 | C | ZNF653 | F509L | G | 1580 | T |
| WSCD2 | F349S | T | 1790 | C | ZFP14 | R75I | C | 317 | A | ZNF33B | T753I | G | 2258 | A | ZNF653 | A580V | G | 1792 | A |
| WT1 | C484G | A | 1735 | C | ZFP14 | W36R | A | 199 | G | ZNF33B | G559V | C | 1676 | A | ZNF653 | Q564P | T | 1744 | G |
| WT1 | T346M | G | 1322 | A | ZFP14 | R385C | G | 1246 | A | ZNF33B | R461K | C | 1382 | T | ZNF654 | R454I | G | 1568 | T |
| WT1 | R485W | G | 1738 | A | ZFP161 | T194A | T | 919 | C | ZNF34 | I351M | A | 1155 | C | ZNF655 | L458R | T | 1470 | G |
| WTAP | T148M | C | 2200 | T | ZFP161 | E32K | C | 433 | T | ZNF345 | H112N | C | 334 | A | ZNF658 | E998G | T | 3096 | C |
| WWC1 | R661* | C | 2047 | T | ZFP161 | S74L | G | 560 | A | ZNF345 | R194I | G | 581 | T | ZNF658 | K254N | T | 865 | G |
| WWC2 | F150L | C | 450 | A | ZFP2 | K417N | A | 1760 | C | ZNF345 | R250K | G | 749 | A | ZNF658 | H205N | G | 716 | T |
| WWC2 | - | G | 0 | T | ZFP2 | E239K | G | 1224 | A | ZNF347 | T643A | T | 1992 | C | ZNF660 | R173C | C | 850 | T |
| WWC3 | P433H | C | 1689 | A | ZFP2 | E271* | G | 1320 | T | ZNF347 | K381E | T | 1206 | C | ZNF662 | P165S | C | 679 | T |
| WWC3 | T323M | C | 1359 | T | ZFP2 | R402I | G | 1714 | T | ZNF347 | R302C | G | 969 | A | ZNF662 | R341Q | G | 1208 | A |
| WWC3 | R1027W | C | 3470 | T | ZFP2 | R455Q | G | 1873 | A | ZNF347 | Y374S | T | 1186 | G | ZNF662 | R172H | G | 701 | A |
| WWC3 | R423* | C | 1658 | T | ZFP28 | R581I | G | 1813 | T | ZNF35 | E130K | G | 623 | A | ZNF662 | R172C | C | 700 | T |
| WWP1 | K33N | G | 109 | T | ZFP28 | R693I | G | 2149 | T | ZNF354A | R523* | G | 1765 | A | ZNF662 | K210I | A | 815 | T |
| WWP1 | R427W | C | 1289 | T | ZFP28 | K702T | A | 2176 | C | ZNF354A | K121T | T | 560 | G | ZNF662 | R294I | G | 1067 | T |
| WWP1 | R394C | C | 1190 | T | ZFP28 | E754D | A | 2333 | C | ZNF354B | V19M | G | 281 | A | ZNF665 | H308N | G | 1022 | T |
| WWP1 | G610D | G | 1839 | A | ZFP28 | K755Q | A | 2334 | C | ZNF354B | I120T | T | 585 | C | ZNF665 | R562T | C | 1785 | G |
| WWP2 | R330Q | G | 1090 | A | ZFP3 | R133K | G | 523 | A | ZNF354B | I144L | A | 656 | C | ZNF667 | R310G | T | 928 | C |
| WWP2 | L794P | T | 2482 | C | ZFP30 | R170Q | C | 1067 | T | ZNF354C | R348S | A | 1350 | C | ZNF668 | R141H | C | 1042 | T |
| WWP2 | D759N | G | 2376 | A | ZFP36 | D244Y | G | 822 | T | ZNF358 | C380Y | G | 1309 | A | ZNF668 | A206V | G | 1237 | A |
| WWP2 | A294V | C | 982 | T | ZFP36L1 | A188T | C | 692 | T | ZNF358 | D107N | G | 489 | A | ZNF668 | A206E | G | 1237 | T |
| WWP2 | G691S | G | 2172 | A | ZFP36L2 | G128S | C | 676 | T | ZNF362 | A108V | C | 342 | T | ZNF668 | R570H | C | 2329 | T |
| WWTR1 | R248* | G | 999 | A | ZFP37 | R313I | C | 966 | A | ZNF365 | Q14* | C | 320 | T | ZNF669 | E331* | C | 1164 | A |
| WWTR1 | S307L | G | 1177 | A | ZFP37 | R453S | T | 1387 | G | ZNF365 | S379G | A | 1415 | G | ZNF670 | R120G | T | 517 | C |
| WWTR1 | K148N | T | 701 | G | ZFP37 | D50N | C | 176 | T | ZNF365 | H51N | C | 431 | A | ZNF670 | E135* | C | 562 | A |
| XAB2 | A600T | C | 1824 | T | ZFP37 | K229* | T | 713 | A | ZNF365 | S151A | T | 731 | G | ZNF671 | R7Q | C | 119 | T |
| XAB2 | G701D | C | 2128 | T | ZFP41 | A118V | C | 711 | T | ZNF365 | - | G | 0 | T | ZNF671 | R286M | C | 956 | A |
| XAB2 | D14H | C | 66 | G | ZFP42 | G16D | G | 455 | A | ZNF366 | D716E | A | 2639 | C | ZNF672 | A55D | C | 910 | A |
| XAB2 | R154H | C | 487 | T | ZFP42 | V136G | T | 815 | G | ZNF366 | E103D | C | 800 | A | ZNF673 | K158T | A | 1104 | C |
| XAB2 | A802T | C | 2430 | T | ZFP62 | Q39H | C | 453 | A | ZNF366 | N89H | T | 756 | G | ZNF676 | E340K | C | 1336 | T |
| XAB2 | G498S | C | 1518 | T | ZFP62 | K79T | T | 572 | G | ZNF367 | E179D | C | 834 | A | ZNF676 | E29D | C | 405 | A |
| XAB2 | E322V | T | 991 | A | ZFP64 | R359C | G | 1425 | A | ZNF367 | S163* | G | 785 | T | ZNF677 | E484D | C | 1618 | A |
| XAF1 | R211C | C | 873 | T | ZFP64 | R441H | C | 1672 | T | ZNF37A | F420L | C | 1605 | A | ZNF677 | W363L | C | 1254 | A |
| XAF1 | R132C | C | 636 | T | ZFP64 | G405R | C | 1620 | T | ZNF37A | G333R | G | 1342 | A | ZNF677 | R479I | C | 1602 | A |

| | | | | | | | | | | | | | | | | | | | |
|---|---|---|---|---|---|---|---|---|---|---|---|---|---|---|---|---|---|---|---|
| XBP1 | T65M | G | 226 | A | ZFP64 | G616C | C | 2196 | A | ZNF37A | E157D | G | 816 | T | ZNF678 | R229I | G | 1031 | T |
| XBP1 | N277H | T | 861 | G | ZFP64 | D274E | G | 965 | T | ZNF37A | R487I | G | 1805 | T | ZNF678 | E353* | G | 1402 | T |
| XCR1 | R139H | C | 773 | T | ZFP82 | E63* | C | 187 | A | ZNF382 | W21* | G | 175 | A | ZNF680 | K257T | T | 922 | G |
| XCR1 | I166V | T | 853 | C | ZFP90 | C499G | T | 1530 | G | ZNF382 | E168D | G | 617 | T | ZNF680 | E319* | C | 1107 | A |
| XCR1 | I166V | T | 853 | C | ZFP90 | R601I | G | 1837 | T | ZNF382 | Y242C | A | 838 | G | ZNF680 | K510I | T | 1681 | A |
| XCR1 | R127H | C | 737 | T | ZFP90 | R536L | G | 1642 | T | ZNF382 | E377K | G | 1242 | A | ZNF681 | E109D | C | 469 | A |
| XCR1 | A33T | C | 454 | T | ZFP91 | R161H | G | 653 | A | ZNF382 | S491A | T | 1584 | G | ZNF681 | A10D | G | 171 | T |
| XDH | T954A | T | 2909 | C | ZFP91 | R326H | G | 1148 | A | ZNF383 | G390D | G | 1752 | A | ZNF681 | K149N | T | 589 | G |
| XDH | D430G | T | 1338 | C | ZFPL1 | R222Q | G | 830 | A | ZNF384 | R410Q | C | 1437 | T | ZNF681 | S94A | A | 422 | C |
| XDH | V931D | A | 2841 | T | ZFPM1 | V291I | G | 1193 | A | ZNF384 | D209G | T | 834 | C | ZNF682 | E109D | C | 450 | A |
| XDH | I403M | T | 1258 | C | ZFPM1 | T151M | C | 774 | T | ZNF385A | Q278R | T | 888 | C | ZNF682 | E226D | C | 801 | A |
| XIRP1 | G491V | C | 1701 | A | ZFPM2 | D39Y | G | 138 | T | ZNF385B | Q335H | C | 1609 | A | ZNF682 | R55C | G | 286 | A |
| XIRP1 | A265T | C | 1022 | T | ZFPM2 | V538I | G | 1635 | A | ZNF385D | A96V | G | 806 | A | ZNF683 | L211I | G | 709 | T |
| XIRP1 | R379C | G | 1364 | A | ZFPM2 | Y540N | T | 1641 | A | ZNF385D | - | A | 0 | G | ZNF684 | V313M | G | 1188 | A |
| XIRP2 | S203G | A | 696 | G | ZFPM2 | T804M | C | 2434 | T | ZNF385D | T280M | G | 1358 | A | ZNF684 | N180K | T | 791 | G |
| XIRP2 | A990V | C | 3058 | T | ZFPM2 | S581P | T | 1764 | C | ZNF385D | G237E | C | 1229 | T | ZNF687 | R1154C | C | 3606 | T |
| XIRP2 | A3258T | G | 9861 | A | ZFPM2 | R787I | G | 2383 | T | ZNF385D | S207L | G | 1139 | A | ZNF687 | L579F | C | 1881 | T |
| XIRP2 | R793* | C | 2466 | T | ZFPM2 | N919Y | A | 2778 | T | ZNF394 | R560H | C | 1883 | T | ZNF687 | R1152* | C | 3600 | T |
| XIRP2 | E215* | G | 732 | T | ZFR | R647* | G | 2042 | A | ZNF394 | R356I | C | 1271 | A | ZNF687 | V445M | G | 1479 | A |
| XIRP2 | C3199G | T | 9684 | G | ZFR | S855* | G | 2667 | T | ZNF394 | E473* | C | 1621 | A | ZNF687 | R1033H | G | 3244 | A |
| XIRP2 | V1370I | G | 4197 | A | ZFR | S9Y | G | 129 | T | ZNF395 | R503W | G | 1639 | A | ZNF687 | D313H | G | 1083 | C |
| XIRP2 | L1605I | C | 4902 | A | ZFR2 | A171V | G | 524 | A | ZNF395 | R487C | G | 1591 | A | ZNF689 | R380C | G | 1476 | A |
| XIRP2 | R1497Q | G | 4579 | A | ZFR2 | V656I | C | 1978 | T | ZNF395 | R158K | C | 605 | T | ZNF689 | R331H | C | 1330 | T |
| XIRP2 | Q2695R | A | 8173 | G | ZFR2 | A507T | C | 1531 | T | ZNF397 | P424S | C | 1426 | T | ZNF689 | V422M | C | 1602 | T |
| XIRP2 | R1018M | G | 3142 | T | ZFR2 | R73Q | C | 230 | T | ZNF397OS | E25D | T | 303 | G | ZNF689 | Q162R | T | 823 | C |
| XIRP2 | K3055N | A | 9254 | T | ZFX | F557L | T | 2096 | C | ZNF398 | R570Q | G | 1709 | A | ZNF69 | Y444D | T | 1470 | G |
| XIRP2 | S73L | C | 307 | T | ZFY | R760Q | G | 2473 | A | ZNF398 | L28I | C | 82 | A | ZNF69 | H500R | A | 1639 | G |
| XIRP2 | M529I | G | 1676 | T | ZFYVE16 | R1260Q | G | 3959 | A | ZNF404 | R504C | G | 1510 | A | ZNF69 | P331L | C | 1132 | T |
| XIRP2 | Q606H | A | 1907 | C | ZFYVE16 | M1353I | G | 4239 | T | ZNF407 | G793R | G | 2434 | A | ZNF691 | H189Y | C | 565 | T |
| XIRP2 | D746Y | G | 2325 | T | ZFYVE19 | Q122* | C | 878 | T | ZNF407 | R1413* | C | 4294 | T | ZNF697 | D293N | C | 991 | T |
| XIRP2 | R3223C | C | 9756 | T | ZFYVE19 | D436N | G | 1820 | A | ZNF407 | G2140E | G | 6476 | A | ZNF697 | T516M | G | 1661 | A |
| XIRP2 | F3516C | T | 10636 | G | ZFYVE20 | R566H | C | 2311 | T | ZNF407 | R1705H | G | 5171 | A | ZNF699 | E448G | T | 1343 | C |
| XKR3 | F88S | A | 366 | G | ZFYVE26 | R1378* | G | 4271 | A | ZNF407 | Q1965* | C | 5950 | T | ZNF699 | V205M | C | 613 | T |
| XKR3 | R59Q | C | 279 | T | ZFYVE26 | P136S | G | 545 | A | ZNF407 | S293A | T | 934 | G | ZNF699 | D468E | A | 1404 | T |
| XKR4 | P588L | C | 1763 | T | ZFYVE26 | C247Y | C | 879 | T | ZNF407 | N1774S | A | 5378 | G | ZNF699 | H353Y | G | 1057 | A |
| XKR4 | S303T | G | 908 | C | ZFYVE26 | G633V | C | 2037 | A | ZNF407 | S1262R | A | 3841 | C | ZNF699 | K472N | C | 1416 | A |
| XKR4 | S584* | C | 1751 | A | ZFYVE26 | F2317L | G | 7090 | T | ZNF408 | E151K | G | 681 | A | ZNF699 | K417T | T | 1250 | G |
| XKR4 | G155S | G | 463 | A | ZFYVE26 | A1353S | C | 4196 | A | ZNF408 | R608H | G | 2053 | A | ZNF7 | V135I | G | 403 | A |
| XKR5 | A214T | C | 791 | T | ZFYVE26 | H1176N | G | 3665 | T | ZNF408 | R352W | C | 1284 | T | ZNF7 | A177T | G | 529 | A |

| | | | | | | | | | | | | | | | | | | | |
|---|---|---|---|---|---|---|---|---|---|---|---|---|---|---|---|---|---|---|---|
| XKR5 | L409P | A | 1377 | G | ZFYVE26 | E361* | C | 1220 | A | ZNF408 | V75L | G | 453 | C | ZNF7 | S122F | C | 365 | T |
| XKR5 | N275D | T | 974 | C | ZFYVE27 | P311L | C | 1132 | T | ZNF414 | P279L | G | 938 | A | ZNF7 | K542Q | A | 1624 | C |
| XKR6 | L606M | G | 1816 | T | ZFYVE27 | R338Q | G | 1213 | A | ZNF414 | A306T | C | 1018 | T | ZNF70 | R327L | C | 1441 | A |
| XKR6 | T572N | G | 1715 | T | ZFYVE27 | A104T | G | 510 | A | ZNF415 | N180S | T | 708 | C | ZNF70 | R436H | C | 1768 | T |
| XKR6 | T555M | G | 1664 | A | ZFYVE28 | V86I | C | 596 | T | ZNF415 | R359Q | C | 1245 | T | ZNF700 | K567E | A | 1825 | G |
| XKR6 | R439* | G | 1315 | A | ZFYVE28 | R389C | G | 1505 | A | ZNF415 | I207S | A | 789 | C | ZNF700 | E387D | G | 1287 | T |
| XKR6 | S374Y | G | 1121 | T | ZFYVE9 | R118I | G | 525 | T | ZNF416 | A66V | G | 368 | A | ZNF703 | G333C | G | 1186 | T |
| XKR7 | P293L | C | 1052 | T | ZG16 | R140C | C | 463 | T | ZNF416 | T312I | G | 1106 | A | ZNF704 | R386W | G | 1388 | A |
| XKR7 | V420I | G | 1432 | A | ZG16 | G77C | G | 274 | T | ZNF417 | R540I | C | 1818 | A | ZNF705A | R154H | G | 584 | A |
| XKR9 | A160V | C | 1013 | T | ZG16B | P190L | C | 648 | T | ZNF417 | A465V | G | 1593 | A | ZNF705E | S173* | G | 590 | T |
| XKR9 | P360Q | C | 1613 | A | ZGPAT | - | T | 0 | A | ZNF417 | V453I | C | 1556 | T | ZNF705G | K63Q | T | 469 | G |
| XKRX | H441R | T | 1888 | C | ZGPAT | L459I | C | 1502 | A | ZNF417 | K331N | T | 1192 | G | ZNF709 | R576Q | C | 1899 | T |
| XKRX | P110L | G | 895 | A | ZHX1 | E106K | C | 746 | T | ZNF418 | Q549E | G | 1937 | C | ZNF709 | R496M | C | 1659 | A |
| XPA | A199V | G | 661 | A | ZHX1 | K190E | T | 998 | C | ZNF418 | R368I | C | 1395 | A | ZNF709 | T385A | T | 1325 | C |
| XPC | T169M | G | 721 | A | ZHX1 | P309S | G | 1355 | A | ZNF419 | A97V | C | 530 | T | ZNF709 | R188I | C | 735 | A |
| XPC | G802S | C | 2619 | T | ZHX1 | E129D | C | 817 | A | ZNF419 | - | T | 0 | C | ZNF709 | R156Q | C | 639 | T |
| XPNPEP1 | F514V | A | 1660 | C | ZHX1 | R741W | T | 2651 | A | ZNF419 | A5T | G | 253 | A | ZNF71 | G192S | G | 812 | A |
| XPNPEP1 | - | C | 0 | T | ZHX2 | E504* | G | 2077 | T | ZNF419 | R503Q | G | 1748 | A | ZNF71 | P25L | C | 312 | T |
| XPNPEP1 | K137T | T | 530 | G | ZHX3 | N249K | G | 747 | T | ZNF420 | H497N | C | 1704 | A | ZNF710 | V464M | G | 1641 | A |
| XPNPEP2 | Q208K | C | 814 | A | ZHX3 | R933C | G | 2797 | A | ZNF420 | S483Y | C | 1663 | A | ZNF710 | P79L | C | 487 | T |
| XPNPEP2 | G671D | G | 2204 | A | ZHX3 | R646I | C | 1937 | A | ZNF423 | S15L | G | 342 | A | ZNF710 | L563P | T | 1939 | C |
| XPNPEP2 | R89H | G | 458 | A | ZHX3 | N249K | G | 747 | T | ZNF423 | R380C | G | 1436 | A | ZNF710 | R172W | C | 765 | T |
| XPNPEP2 | A2T | G | 196 | A | ZIC1 | N50K | C | 879 | A | ZNF423 | L681M | G | 2339 | T | ZNF710 | K576E | A | 1977 | G |
| XPNPEP2 | - | G | 0 | A | ZIC1 | K320R | A | 1688 | G | ZNF423 | A508T | C | 1820 | T | ZNF711 | R625H | G | 2760 | A |
| XPNPEP3 | R242C | C | 808 | T | ZIC1 | R294H | G | 1610 | A | ZNF423 | A434V | G | 1599 | A | ZNF711 | L215I | T | 1529 | A |
| XPNPEP3 | S81Y | C | 326 | A | ZIC1 | M249V | A | 1474 | G | ZNF423 | D144N | C | 728 | T | ZNF713 | R340H | G | 1865 | A |
| XPO1 | T1030S | G | 3817 | C | ZIC1 | G338S | G | 1741 | A | ZNF423 | A842G | G | 2823 | C | ZNF714 | K282N | A | 1206 | C |
| XPO1 | R749Q | C | 2974 | T | ZIC1 | D368N | G | 1831 | A | ZNF425 | G532S | C | 1727 | T | ZNF714 | V19L | G | 415 | C |
| XPO4 | R384* | G | 1186 | A | ZIC2 | P269H | C | 1099 | A | ZNF425 | G298R | C | 1025 | T | ZNF716 | E349D | G | 1159 | T |
| XPO4 | M1T | A | 38 | G | ZIC2 | S430P | T | 1581 | C | ZNF425 | R297C | G | 1022 | A | ZNF716 | G330D | G | 1101 | A |
| XPO4 | N960H | T | 2914 | G | ZIC3 | R30C | C | 638 | T | ZNF425 | C587Y | C | 1893 | T | ZNF716 | R11Q | G | 144 | A |
| XPO5 | L939R | A | 3027 | C | ZIC3 | D366G | A | 1647 | G | ZNF425 | T236M | G | 840 | A | ZNF716 | E121D | G | 475 | T |
| XPO6 | R566C | G | 2197 | A | ZIC4 | R55Q | C | 164 | T | ZNF425 | C218F | C | 786 | A | ZNF716 | K232T | A | 807 | C |
| XPO6 | P745S | G | 2734 | A | ZIC4 | R320C | G | 958 | A | ZNF426 | R548Q | C | 1907 | T | ZNF716 | R316I | G | 1059 | T |
| XPO6 | V564M | C | 2191 | T | ZIC4 | V325A | A | 974 | G | ZNF426 | V165M | C | 757 | T | ZNF724P | N488H | T | 1580 | G |
| XPO6 | K431T | T | 1793 | G | ZIC4 | K224E | T | 670 | C | ZNF429 | R67Q | G | 337 | A | ZNF724P | R358I | C | 1191 | A |
| XPO7 | Y818C | A | 2555 | G | ZIC5 | G55D | C | 398 | T | ZNF43 | K501T | T | 1639 | G | ZNF725 | L3V | A | 7 | C |
| XPO7 | R97W | C | 391 | T | ZIC5 | R556* | G | 1900 | A | ZNF431 | E544* | G | 1777 | T | ZNF726 | G132* | G | 479 | T |
| XPO7 | R672Q | G | 2117 | A | ZIC5 | Y476* | G | 1662 | T | ZNF433 | S518L | G | 1724 | A | ZNF729 | L1007P | T | 3020 | C |

| | | | | | | | | | | | | | | | | | | | |
|---|---|---|---|---|---|---|---|---|---|---|---|---|---|---|---|---|---|---|---|
| XPO7 | L141V | T | 523 | G | ZIK1 | P189T | C | 662 | A | ZNF433 | R522* | G | 1735 | A | ZNF729 | V56A | T | 167 | C |
| XPOT | R705C | C | 2539 | T | ZIK1 | E299K | G | 992 | A | ZNF433 | R274I | C | 992 | A | ZNF733 | M82I | C | 517 | T |
| XPOT | I570M | A | 2136 | G | ZKSCAN1 | R248W | C | 976 | T | ZNF438 | T47M | G | 470 | A | ZNF735 | E12* | G | 303 | T |
| XPOT | A378V | C | 1559 | T | ZKSCAN1 | R296K | G | 1121 | A | ZNF438 | P251L | G | 1082 | A | ZNF735 | S149P | T | 714 | C |
| XPOT | V285G | T | 1280 | G | ZKSCAN1 | S183L | C | 782 | T | ZNF439 | R203I | G | 808 | T | ZNF735 | P87S | C | 528 | T |
| XPR1 | K422R | A | 1463 | G | ZKSCAN1 | R528H | G | 1817 | A | ZNF439 | R398* | C | 1392 | T | ZNF735 | Q157L | A | 739 | T |
| XPR1 | P431A | C | 1489 | G | ZKSCAN2 | G542S | C | 2031 | T | ZNF439 | K380N | A | 1340 | C | ZNF735 | - | G | 0 | T |
| XRCC1 | P270A | G | 928 | C | ZKSCAN2 | R586W | G | 2163 | A | ZNF439 | A422V | C | 1465 | T | ZNF735 | T250A | A | 1017 | G |
| XRCC1 | A214V | G | 761 | A | ZKSCAN2 | T852M | G | 2962 | A | ZNF439 | F52C | T | 355 | G | ZNF736 | R352C | C | 1176 | T |
| XRCC1 | E424* | C | 1390 | A | ZKSCAN3 | A63V | C | 404 | T | ZNF439 | E233A | A | 898 | C | ZNF736 | Q58H | A | 296 | C |
| XRCC2 | V256G | A | 853 | C | ZKSCAN3 | V531I | G | 1807 | A | ZNF44 | Y469H | A | 1524 | G | ZNF736 | F381L | C | 1265 | A |
| XRCC3 | T340S | T | 1341 | A | ZKSCAN5 | C460Y | G | 1502 | A | ZNF44 | S510F | G | 1648 | A | ZNF74 | G251S | G | 1265 | A |
| XRCC4 | F88L | T | 363 | G | ZKSCAN5 | A61T | G | 304 | A | ZNF44 | P607H | G | 1939 | T | ZNF74 | N320S | A | 1473 | G |
| XRCC5 | A372T | G | 1575 | A | ZKSCAN5 | I749T | T | 2369 | C | ZNF440 | R297H | G | 1054 | A | ZNF74 | R239H | G | 1230 | A |
| XRCC5 | G63D | G | 649 | A | ZMAT1 | E275G | T | 872 | C | ZNF440 | G112* | G | 498 | T | ZNF74 | A585D | C | 2268 | A |
| XRCC6 | - | A | 0 | C | ZMIZ1 | N905D | A | 3285 | G | ZNF440 | T479A | A | 1599 | G | ZNF746 | R159C | G | 642 | A |
| XRCC6 | A151V | C | 694 | T | ZMIZ1 | D885N | G | 3225 | A | ZNF441 | E675* | G | 2125 | T | ZNF746 | R347Q | C | 1207 | T |
| XRCC6 | M1T | T | 244 | C | ZMIZ1 | S237N | G | 1282 | A | ZNF442 | R180H | C | 1122 | T | ZNF746 | K271E | T | 978 | C |
| XRCC6BP1 | E89K | G | 390 | A | ZMIZ1 | S939I | G | 3388 | T | ZNF442 | R449* | G | 1928 | A | ZNF746 | T278M | G | 1000 | A |
| XRN1 | R73H | C | 336 | T | ZMIZ2 | R605C | C | 1936 | T | ZNF442 | R309* | G | 1508 | A | ZNF749 | P223H | C | 918 | A |
| XRN1 | V1064D | A | 3309 | T | ZMIZ2 | I787L | A | 2482 | C | ZNF442 | R624I | C | 2454 | A | ZNF749 | T155M | C | 714 | T |
| XRN2 | E28* | G | 177 | T | ZMYM1 | R814C | C | 2590 | T | ZNF442 | R393Q | C | 1761 | T | ZNF750 | H39R | T | 427 | C |
| XRN2 | - | T | 0 | C | ZMYM1 | K313Q | A | 1087 | C | ZNF442 | S335N | C | 1587 | T | ZNF750 | R693S | C | 2390 | A |
| XRRA1 | R433* | G | 1629 | A | ZMYM1 | K98R | A | 443 | G | ZNF442 | R313I | C | 1521 | A | ZNF750 | A86V | G | 568 | A |
| XYLB | V65L | G | 283 | T | ZMYM1 | K801T | A | 2552 | C | ZNF442 | R309Q | C | 1509 | T | ZNF75A | N76H | A | 699 | C |
| XYLB | R476Q | G | 1517 | A | ZMYM1 | L1097V | T | 3439 | G | ZNF443 | H303L | T | 1106 | A | ZNF75A | N115H | A | 816 | C |
| XYLB | G107R | G | 409 | A | ZMYM2 | G461D | G | 1633 | A | ZNF443 | E306D | C | 1116 | A | ZNF75D | C74* | G | 2932 | T |
| XYLB | K435N | G | 1395 | T | ZMYM2 | M558V | A | 1923 | G | ZNF444 | T76M | C | 594 | T | ZNF75D | S490T | A | 4178 | T |
| XYLT1 | S908L | G | 2808 | A | ZMYM2 | C750R | T | 2499 | C | ZNF445 | R931Q | C | 2943 | T | ZNF76 | R253H | G | 1024 | A |
| XYLT1 | W416C | C | 1333 | A | ZMYM2 | F237V | T | 960 | G | ZNF445 | Y433H | A | 1448 | G | ZNF764 | E200K | C | 679 | T |
| XYLT1 | F355L | G | 1150 | T | ZMYM2 | K384E | A | 1401 | G | ZNF446 | S408L | C | 1340 | T | ZNF765 | E324D | G | 1089 | T |
| XYLT2 | R406C | C | 1265 | T | ZMYM3 | N1071D | T | 3298 | C | ZNF446 | R46Q | G | 254 | A | ZNF765 | E463D | G | 1506 | T |
| XYLT2 | - | G | 0 | T | ZMYM3 | T417A | T | 1336 | C | ZNF446 | P309L | C | 1043 | T | ZNF765 | G361V | G | 1199 | T |
| XYLT2 | G862R | G | 2633 | A | ZMYM4 | V300A | T | 979 | C | ZNF45 | F512L | G | 2627 | T | ZNF768 | R259W | G | 958 | A |
| YAP1 | R319W | C | 1325 | T | ZMYM4 | E1532K | G | 4674 | A | ZNF451 | K301N | G | 1147 | T | ZNF768 | C350R | A | 1231 | G |
| YAP1 | D194N | G | 950 | A | ZMYM4 | L759F | C | 2355 | T | ZNF451 | L530I | C | 1832 | A | ZNF77 | R456* | G | 1497 | A |
| YARS2 | L20I | G | 124 | T | ZMYM4 | E1338* | G | 4092 | T | ZNF454 | N303T | A | 1129 | C | ZNF77 | R424Q | C | 1402 | T |
| YBX2 | R232W | G | 751 | A | ZMYM5 | C475Y | C | 1689 | T | ZNF454 | R348I | G | 1264 | T | ZNF770 | L440S | A | 1650 | G |
| YDJC | F88V | A | 312 | C | ZMYM5 | - | A | 2273 | G | ZNF454 | R376I | G | 1348 | T | ZNF770 | K510E | T | 1859 | C |

| | | | | | | | | | | | | | | | | | | |
|---|---|---|---|---|---|---|---|---|---|---|---|---|---|---|---|---|---|---|
| YEATS2 | P386Q | C | 1352 | A | ZMYM5 | L488* | A | 1728 | C | ZNF454 | R401Q | G | 1423 | A | ZNF772 | R305H | C | 1175 | T |
| YEATS2 | L78P | T | 428 | C | ZMYM6 | - | A | 0 | G | ZNF460 | A51V | C | 474 | T | ZNF773 | R351H | G | 1192 | A |
| YEATS2 | T1009A | A | 3220 | G | ZMYM6 | Y106H | A | 544 | G | ZNF460 | R272Q | G | 1137 | A | ZNF773 | F339L | C | 1157 | A |
| YEATS2 | T44A | A | 325 | G | ZMYM6 | - | C | 0 | A | ZNF460 | E127K | G | 701 | A | ZNF775 | E2K | G | 111 | A |
| YEATS2 | P613L | C | 2033 | T | ZMYND10 | E171D | C | 1786 | A | ZNF461 | G528V | C | 1583 | A | ZNF775 | R201C | C | 708 | T |
| YEATS2 | V106A | T | 512 | C | ZMYND11 | R453Q | G | 1567 | A | ZNF461 | K420R | T | 1259 | C | ZNF775 | L96M | C | 393 | A |
| YES1 | - | C | 0 | T | ZMYND11 | Q587* | C | 1968 | T | ZNF461 | R388W | G | 1162 | A | ZNF777 | E692D | C | 2239 | A |
| YIF1A | R297W | G | 1053 | A | ZMYND11 | Q429P | A | 1495 | C | ZNF462 | E361G | A | 1371 | G | ZNF777 | T180M | G | 702 | A |
| YIF1A | P24S | G | 234 | A | ZMYND11 | E67K | G | 408 | A | ZNF462 | E2473K | G | 7706 | A | ZNF777 | R567H | C | 1863 | T |
| YIPF1 | R159Q | C | 813 | T | ZMYND11 | F119V | T | 564 | G | ZNF462 | G2107R | G | 6608 | A | ZNF778 | R581Q | G | 2081 | A |
| YIPF1 | S116R | G | 685 | T | ZMYND12 | E278* | C | 1102 | A | ZNF462 | E725D | G | 2464 | T | ZNF780A | - | C | 0 | A |
| YIPF1 | A216T | C | 983 | T | ZMYND15 | D522Y | G | 1621 | T | ZNF462 | G1425D | G | 4563 | A | ZNF780A | T497A | T | 1637 | C |
| YIPF2 | E253A | T | 858 | G | ZMYND15 | G457C | G | 1426 | T | ZNF462 | A1290T | G | 4157 | A | ZNF780A | R239C | G | 863 | A |
| YIPF6 | G70W | G | 952 | T | ZMYND8 | R375M | C | 1151 | A | ZNF462 | A2078V | C | 6522 | T | ZNF780B | G395R | C | 1249 | T |
| YIPF6 | K72T | A | 959 | C | ZMYND8 | F1058L | A | 3201 | C | ZNF462 | Q556H | G | 1957 | T | ZNF780B | R680C | G | 2104 | A |
| YKT6 | R71W | C | 298 | T | ZMYND8 | A406T | C | 1243 | T | ZNF462 | G325C | G | 1262 | T | ZNF780B | L292I | G | 940 | T |
| YLPM1 | R1126* | C | 3500 | T | ZMYND8 | R1229Q | C | 3713 | T | ZNF462 | S1737N | G | 5499 | A | ZNF780B | R131* | G | 457 | A |
| YLPM1 | R1665C | C | 5117 | T | ZMYND8 | S464C | G | 1418 | C | ZNF462 | S339L | C | 1305 | T | ZNF781 | E165* | C | 1235 | A |
| YLPM1 | P153S | C | 581 | T | ZMYND8 | D108Y | C | 349 | A | ZNF462 | K668N | G | 2293 | T | ZNF781 | R81K | C | 984 | T |
| YLPM1 | R1261* | C | 3905 | T | ZNF100 | Y426C | T | 1277 | C | ZNF467 | S340Y | G | 1433 | T | ZNF781 | G136V | C | 1149 | A |
| YLPM1 | E266G | A | 921 | G | ZNF100 | S245* | G | 734 | T | ZNF467 | P156Q | G | 881 | T | ZNF781 | A46V | G | 879 | A |
| YLPM1 | S2075N | G | 6348 | A | ZNF101 | T33M | C | 266 | T | ZNF467 | A328V | G | 1397 | A | ZNF781 | E249* | C | 1487 | A |
| YLPM1 | R1032Q | G | 3219 | A | ZNF107 | Q234* | C | 1152 | T | ZNF469 | G1358R | G | 4072 | A | ZNF782 | Q685* | G | 2715 | A |
| YME1L1 | R177C | G | 678 | A | ZNF114 | T303I | C | 1132 | T | ZNF469 | S1491L | C | 4472 | T | ZNF782 | R414I | C | 1903 | A |
| YME1L1 | M91T | A | 421 | G | ZNF117 | E386D | C | 2443 | A | ZNF469 | R1601C | C | 4801 | T | ZNF783 | P402H | C | 1368 | A |
| YME1L1 | F525C | A | 1723 | C | ZNF12 | R626I | C | 2260 | A | ZNF469 | R2853C | C | 8557 | T | ZNF783 | V98M | G | 455 | A |
| YME1L1 | R512Q | C | 1684 | T | ZNF12 | F614L | G | 2225 | T | ZNF469 | V3591M | G | 10771 | A | ZNF783 | R456H | G | 1530 | A |
| YOD1 | P111L | G | 379 | A | ZNF121 | L271P | A | 1044 | G | ZNF469 | Q2021P | A | 6062 | C | ZNF784 | L282P | A | 859 | G |
| YOD1 | R143G | T | 474 | C | ZNF124 | A218V | G | 1115 | A | ZNF469 | P485A | C | 1453 | G | ZNF784 | R250H | C | 763 | T |
| YOD1 | R245I | C | 781 | A | ZNF132 | E596* | C | 2187 | A | ZNF469 | R2607K | G | 7820 | A | ZNF786 | F406C | A | 1282 | C |
| YSK4 | K862N | C | 2617 | A | ZNF133 | R635I | G | 2153 | T | ZNF470 | R445I | G | 2011 | T | ZNF787 | G351R | C | 1170 | T |
| YSK4 | N526H | T | 1607 | G | ZNF133 | N482S | A | 1694 | G | ZNF470 | E513* | G | 2214 | T | ZNF789 | H219Q | T | 927 | A |
| YSK4 | I512L | T | 1565 | G | ZNF133 | H542Y | C | 1873 | T | ZNF470 | R669I | G | 2683 | T | ZNF789 | H219Q | T | 927 | A |
| YSK4 | N431D | T | 1322 | C | ZNF134 | S40Y | C | 420 | A | ZNF470 | M88T | T | 940 | C | ZNF789 | R312H | G | 1205 | A |
| YTHDC1 | - | C | 0 | T | ZNF134 | Y176C | A | 828 | G | ZNF470 | R445I | G | 2011 | T | ZNF789 | F105C | T | 584 | G |
| YTHDC1 | R679H | C | 2372 | T | ZNF135 | G238R | G | 711 | A | ZNF470 | E478D | G | 2111 | T | ZNF79 | P84R | C | 657 | G |
| YTHDC1 | R687Q | C | 2396 | T | ZNF135 | D628N | G | 1881 | A | ZNF470 | E534D | G | 2279 | T | ZNF79 | R170H | G | 915 | A |
| YTHDC2 | E183* | G | 760 | T | ZNF135 | Q495* | C | 1482 | T | ZNF470 | R641I | G | 2599 | T | ZNF790 | R358I | C | 1192 | A |
| YTHDC2 | E201* | G | 814 | T | ZNF135 | R364* | C | 1089 | T | ZNF471 | E45D | G | 268 | T | ZNF790 | T218R | G | 772 | C |

| | | | | | | | | | | | | | | | | | | | |
|---|---|---|---|---|---|---|---|---|---|---|---|---|---|---|---|---|---|---|---|
| YTHDC2 | F793C | T | 2591 | G | ZNF135 | K426E | A | 1275 | G | ZNF471 | E322* | G | 1097 | T | ZNF791 | K98N | A | 456 | C |
| YTHDC2 | R169* | C | 718 | T | ZNF135 | R672I | G | 2014 | T | ZNF471 | A383S | G | 1280 | T | ZNF791 | R484W | C | 1612 | T |
| YTHDC2 | R1137Q | G | 3623 | A | ZNF136 | R324* | C | 1110 | T | ZNF471 | D439Y | G | 1448 | T | ZNF792 | D370N | C | 1495 | T |
| YTHDF1 | K266R | T | 1091 | C | ZNF136 | R408* | C | 1362 | T | ZNF471 | R619I | G | 1989 | T | ZNF792 | C483Y | C | 1835 | T |
| YTHDF1 | S156R | T | 760 | G | ZNF136 | R412I | G | 1375 | T | ZNF473 | K9T | A | 385 | C | ZNF793 | E398* | G | 1227 | T |
| YTHDF1 | R327H | C | 1274 | T | ZNF136 | R380Q | G | 1279 | A | ZNF474 | N319K | T | 1340 | G | ZNF793 | R24W | C | 105 | T |
| YTHDF1 | R542H | C | 1919 | T | ZNF136 | R436* | C | 1446 | T | ZNF474 | H349R | A | 1429 | G | ZNF793 | K144N | G | 467 | T |
| YTHDF1 | L56M | G | 460 | T | ZNF138 | I188N | T | 744 | A | ZNF48 | R716C | C | 2144 | T | ZNF799 | S235Y | G | 905 | T |
| YWHAG | E114K | C | 563 | T | ZNF138 | K221N | A | 844 | C | ZNF48 | E184* | G | 548 | T | ZNF80 | R124C | G | 397 | A |
| YWHAG | D202N | C | 827 | T | ZNF138 | I133T | T | 579 | C | ZNF480 | N97D | A | 355 | G | ZNF804A | P799Q | C | 2990 | A |
| YWHAQ | - | C | 0 | A | ZNF138 | G110C | G | 509 | T | ZNF480 | N97H | A | 355 | C | ZNF804A | Y5C | A | 608 | G |
| YWHAQ | G147* | C | 595 | A | ZNF138 | E192D | G | 757 | T | ZNF480 | R360Q | G | 1145 | A | ZNF804A | G514R | G | 2134 | A |
| YWHAQ | M121T | A | 518 | G | ZNF14 | R547* | G | 1793 | A | ZNF483 | K9N | G | 185 | T | ZNF804A | D165Y | G | 1087 | T |
| YWHAQ | - | T | 0 | C | ZNF14 | I524M | A | 1726 | C | ZNF483 | R408I | G | 1381 | T | ZNF804A | S277Y | C | 1424 | A |
| YY1 | R375Q | G | 1384 | A | ZNF14 | R631* | G | 2045 | A | ZNF484 | M836V | T | 2506 | C | ZNF804A | F1163V | T | 4081 | G |
| ZADH2 | R265C | G | 1083 | A | ZNF14 | R547* | G | 1793 | A | ZNF484 | R742I | C | 2225 | A | ZNF804B | E430* | G | 1897 | T |
| ZAN | S2187L | C | 6725 | T | ZNF14 | H114N | G | 494 | T | ZNF484 | R496I | C | 1487 | A | ZNF804B | S941* | C | 3431 | A |
| ZAN | A2468V | C | 7568 | T | ZNF141 | F292C | T | 1024 | G | ZNF485 | Y86C | A | 451 | G | ZNF804B | H1032N | C | 3703 | A |
| ZAN | T693M | C | 2243 | T | ZNF141 | R303Q | G | 1057 | A | ZNF488 | R94H | G | 508 | A | ZNF804B | Q938P | A | 3422 | C |
| ZAN | R1590C | C | 4933 | T | ZNF142 | R1165H | C | 3916 | T | ZNF490 | F334C | A | 1124 | C | ZNF804B | E243D | A | 1338 | C |
| ZAN | A2526T | G | 7741 | A | ZNF142 | K169N | C | 929 | A | ZNF490 | R214I | C | 764 | A | ZNF804B | K21T | A | 671 | C |
| ZAN | E659* | G | 2140 | T | ZNF142 | R1472H | C | 4837 | T | ZNF490 | R210Q | C | 752 | T | ZNF804B | S233Y | C | 1307 | A |
| ZAN | E799* | G | 2560 | T | ZNF142 | R879C | G | 3057 | A | ZNF490 | A232T | C | 817 | T | ZNF805 | P370S | C | 1315 | T |
| ZAP70 | R306Q | G | 1132 | A | ZNF142 | R834Q | C | 2923 | T | ZNF491 | K132T | A | 726 | C | ZNF808 | Q411R | A | 1412 | G |
| ZAP70 | E225K | G | 888 | A | ZNF142 | R834* | G | 2922 | A | ZNF491 | A251S | G | 1082 | T | ZNF808 | H893Y | C | 2857 | T |
| ZAP70 | R465H | G | 1609 | A | ZNF142 | R236C | G | 1128 | A | ZNF491 | K241N | A | 1054 | C | ZNF808 | I419M | A | 1437 | G |
| ZAP70 | G113R | G | 552 | A | ZNF142 | R287C | G | 1281 | A | ZNF491 | R175* | C | 854 | T | ZNF808 | T794A | A | 2560 | G |
| ZAP70 | K75N | A | 440 | C | ZNF143 | Q315H | G | 1063 | T | ZNF491 | K35Q | A | 434 | C | ZNF808 | R363I | G | 1268 | T |
| ZAP70 | D285N | G | 1068 | A | ZNF143 | R238* | C | 830 | T | ZNF491 | R343Q | G | 1359 | A | ZNF812 | D122Y | C | 883 | A |
| ZAP70 | R514H | G | 1756 | A | ZNF146 | E213K | G | 2045 | A | ZNF491 | R403I | G | 1539 | T | ZNF812 | A34T | C | 619 | T |
| ZAP70 | E567A | A | 1915 | C | ZNF148 | N629K | G | 2373 | T | ZNF493 | K536E | A | 1715 | G | ZNF812 | K218N | C | 1173 | A |
| ZAR1L | R267* | G | 828 | A | ZNF148 | E117D | T | 837 | G | ZNF493 | K711N | A | 2242 | C | ZNF813 | Q128K | C | 510 | A |
| ZAR1L | R297* | G | 918 | A | ZNF157 | N221H | A | 747 | C | ZNF493 | S362A | T | 1193 | G | ZNF814 | D636N | C | 2178 | T |
| ZBBX | E544D | C | 1916 | A | ZNF160 | G683V | C | 2464 | A | ZNF493 | F22L | C | 175 | A | ZNF814 | H213R | T | 910 | C |
| ZBBX | S261P | A | 1065 | G | ZNF160 | H51N | G | 567 | T | ZNF496 | R480Q | C | 1964 | T | ZNF814 | K779Q | T | 2607 | G |
| ZBBX | A166T | C | 780 | T | ZNF160 | E261V | T | 1198 | A | ZNF496 | W110C | C | 855 | A | ZNF816A | V429A | A | 1587 | G |
| ZBBX | R240H | C | 1003 | T | ZNF160 | L101V | A | 717 | C | ZNF497 | A415T | C | 1432 | T | ZNF816A | T89S | T | 566 | A |
| ZBBX | E819D | C | 2741 | A | ZNF165 | S13P | T | 1121 | C | ZNF497 | E347G | T | 1229 | C | ZNF816A | R141* | G | 722 | A |
| ZBBX | D767Y | C | 2583 | A | ZNF165 | G274E | G | 1905 | A | ZNF497 | C416R | A | 1435 | G | ZNF816A | E590D | C | 2071 | A |

| | | | | | | | | | | | | | | | | | | | |
|---|---|---|---|---|---|---|---|---|---|---|---|---|---|---|---|---|---|---|---|
| ZBBX | K742T | T | 2509 | G | ZNF167 | A198T | G | 999 | A | ZNF498 | R506I | G | 1844 | T | ZNF821 | E92K | C | 654 | T |
| ZBBX | E474* | C | 1704 | A | ZNF167 | D337N | G | 1416 | A | ZNF498 | A54V | C | 488 | T | ZNF823 | K553N | T | 1813 | G |
| ZBBX | R424L | C | 1555 | A | ZNF167 | R487I | G | 1867 | T | ZNF500 | P289L | G | 1113 | A | ZNF823 | I380V | T | 1292 | C |
| ZBBX | K416T | T | 1531 | G | ZNF167 | R655I | G | 2371 | T | ZNF500 | R429Q | C | 1533 | T | ZNF823 | A396S | C | 1340 | A |
| ZBED2 | E207D | C | 1630 | A | ZNF169 | D171N | G | 601 | A | ZNF502 | R117H | G | 511 | A | ZNF823 | R376Q | C | 1281 | T |
| ZBED4 | P1100L | C | 3776 | T | ZNF17 | L80V | T | 238 | G | ZNF502 | A27V | C | 241 | T | ZNF823 | K193T | T | 732 | G |
| ZBED4 | A943V | C | 3305 | T | ZNF17 | Q239P | A | 716 | C | ZNF502 | R424W | C | 1431 | T | ZNF827 | E734K | C | 2428 | T |
| ZBTB1 | N172T | A | 906 | C | ZNF174 | E92G | A | 860 | G | ZNF503 | A580T | C | 2225 | T | ZNF827 | R692Q | C | 2303 | T |
| ZBTB1 | I325T | T | 1365 | C | ZNF180 | R625I | C | 2156 | A | ZNF503 | A568T | C | 2189 | T | ZNF827 | L290I | G | 1096 | T |
| ZBTB1 | L526* | T | 1968 | G | ZNF180 | K435N | C | 1587 | A | ZNF503 | S321P | A | 1448 | G | ZNF827 | S362P | A | 1312 | G |
| ZBTB10 | E763* | G | 3066 | T | ZNF180 | H655Q | A | 2247 | C | ZNF507 | R482W | C | 1716 | T | ZNF827 | A882V | G | 2873 | A |
| ZBTB10 | K515N | G | 2324 | T | ZNF180 | R541I | C | 1904 | A | ZNF507 | E56* | G | 438 | T | ZNF828 | P333H | C | 1307 | A |
| ZBTB11 | H725R | T | 2755 | C | ZNF180 | Q525P | T | 1856 | G | ZNF507 | A826V | C | 2749 | T | ZNF828 | A643V | C | 2237 | T |
| ZBTB16 | K572N | A | 2096 | C | ZNF180 | R265I | C | 1076 | A | ZNF507 | E56* | G | 438 | T | ZNF828 | R420C | C | 1567 | T |
| ZBTB16 | S471L | C | 1792 | T | ZNF180 | E154* | C | 742 | A | ZNF510 | R452I | C | 2006 | A | ZNF83 | S243N | C | 1129 | T |
| ZBTB17 | R389C | G | 1404 | A | ZNF181 | S373Y | C | 1286 | A | ZNF510 | V83I | C | 898 | T | ZNF83 | C462Y | C | 1786 | T |
| ZBTB17 | D452N | C | 1593 | T | ZNF182 | R226I | C | 1028 | A | ZNF510 | N145T | T | 1085 | G | ZNF83 | E174D | C | 923 | A |
| ZBTB17 | R477* | G | 1668 | A | ZNF184 | R624* | G | 2155 | A | ZNF512B | D646N | C | 1990 | T | ZNF830 | P214H | C | 678 | A |
| ZBTB2 | R482* | G | 1585 | A | ZNF184 | R207S | T | 906 | G | ZNF512B | R585C | G | 1807 | A | ZNF831 | L154P | T | 461 | C |
| ZBTB2 | A232V | G | 836 | A | ZNF185 | D435Y | G | 1340 | T | ZNF512B | - | C | 0 | T | ZNF831 | V501I | G | 1501 | A |
| ZBTB20 | T107M | G | 499 | A | ZNF185 | G468D | G | 1440 | A | ZNF512B | R676Q | C | 2081 | T | ZNF831 | C607Y | G | 1820 | A |
| ZBTB20 | R4W | G | 189 | A | ZNF185 | R638Q | G | 1950 | A | ZNF512B | G193E | C | 632 | T | ZNF831 | T193M | C | 578 | T |
| ZBTB20 | R654C | G | 2139 | A | ZNF185 | A217V | C | 687 | T | ZNF514 | E341* | C | 1484 | A | ZNF831 | S1003N | G | 3008 | A |
| ZBTB20 | V379I | C | 1314 | T | ZNF189 | R420I | G | 1388 | T | ZNF516 | A598T | C | 2022 | T | ZNF831 | D1025G | A | 3074 | G |
| ZBTB26 | E79K | C | 309 | T | ZNF189 | E453D | G | 1488 | T | ZNF516 | D545V | T | 1864 | A | ZNF831 | S1412N | G | 4235 | A |
| ZBTB3 | R168Q | C | 625 | T | ZNF189 | L527I | C | 1708 | A | ZNF516 | A316T | C | 1176 | T | ZNF831 | S1412I | G | 4235 | T |
| ZBTB32 | P170S | C | 718 | T | ZNF189 | R529Q | G | 1715 | A | ZNF516 | A1141T | C | 3651 | T | ZNF831 | G736D | G | 2207 | A |
| ZBTB32 | P170S | C | 718 | T | ZNF189 | K541T | A | 1751 | C | ZNF516 | A373T | C | 1347 | T | ZNF831 | D1391N | G | 4171 | A |
| ZBTB34 | V475M | G | 1508 | A | ZNF19 | E45D | C | 391 | A | ZNF517 | A290V | C | 976 | T | ZNF835 | R289C | G | 865 | A |
| ZBTB37 | R26C | C | 352 | T | ZNF19 | M43L | T | 383 | A | ZNF517 | R486W | C | 1563 | T | ZNF835 | T463M | G | 1388 | A |
| ZBTB37 | F448V | T | 1618 | G | ZNF19 | R349I | C | 1302 | A | ZNF518A | F570I | T | 2539 | A | ZNF835 | G380R | C | 1138 | T |
| ZBTB38 | D690G | A | 2348 | G | ZNF19 | E45D | C | 391 | A | ZNF518A | V1174I | G | 4351 | A | ZNF835 | R301C | G | 901 | A |
| ZBTB39 | D463Y | C | 1473 | A | ZNF192 | R426I | G | 1461 | T | ZNF518A | T532S | A | 2425 | T | ZNF835 | H318R | T | 953 | C |
| ZBTB39 | G37E | C | 196 | T | ZNF192 | R563* | C | 1871 | T | ZNF518B | R991H | C | 3411 | T | ZNF836 | E579D | C | 2259 | A |
| ZBTB39 | A446T | C | 1422 | T | ZNF195 | A24T | C | 115 | T | ZNF519 | E401D | C | 1356 | A | ZNF836 | F145Y | A | 956 | T |
| ZBTB39 | S85N | C | 340 | T | ZNF195 | Q283K | G | 892 | T | ZNF521 | L1071P | A | 3459 | G | ZNF836 | L317I | G | 1471 | T |
| ZBTB4 | E648K | C | 2217 | T | ZNF195 | V43A | A | 173 | G | ZNF521 | R159H | C | 723 | T | ZNF836 | E803D | C | 2931 | A |
| ZBTB4 | E641* | C | 2196 | A | ZNF197 | R76H | G | 412 | A | ZNF521 | L1083H | A | 3495 | T | ZNF837 | G277C | C | 1152 | A |
| ZBTB4 | A771T | C | 2586 | T | ZNF20 | R218Q | C | 799 | T | ZNF521 | R163H | C | 735 | T | ZNF839 | D843G | A | 2535 | G |

| | | | | | | | | | | | | | | | | | | | |
|---|---|---|---|---|---|---|---|---|---|---|---|---|---|---|---|---|---|---|---|
| ZBTB40 | A1174V | C | 4032 | T | ZNF200 | R246W | G | 1006 | A | ZNF521 | E836K | C | 2753 | T | ZNF839 | G327* | G | 986 | T |
| ZBTB40 | A1174V | C | 4032 | T | ZNF202 | R107W | G | 719 | A | ZNF521 | S526Y | G | 1824 | T | ZNF839 | L831P | T | 2499 | C |
| ZBTB40 | T1164M | C | 4002 | T | ZNF205 | L215I | C | 848 | A | ZNF524 | R131C | C | 474 | T | ZNF839 | A721V | C | 2169 | T |
| ZBTB41 | R826C | G | 2545 | A | ZNF205 | E333K | G | 1202 | A | ZNF526 | R583H | G | 1904 | A | ZNF839 | P902L | C | 2712 | T |
| ZBTB41 | E377K | C | 1198 | T | ZNF205 | E473K | G | 1622 | A | ZNF526 | S638P | T | 2068 | C | ZNF841 | N611H | T | 1918 | G |
| ZBTB45 | M450I | C | 1642 | G | ZNF205 | A493V | C | 1683 | T | ZNF526 | F149S | T | 602 | C | ZNF843 | H51Y | G | 151 | A |
| ZBTB46 | A137T | C | 565 | T | ZNF208 | K454T | T | 1510 | G | ZNF527 | H380N | C | 1249 | A | ZNF844 | S304N | G | 1072 | A |
| ZBTB46 | S194N | C | 737 | T | ZNF208 | K507N | T | 1670 | G | ZNF528 | A199T | G | 996 | A | ZNF844 | R447H | G | 1501 | A |
| ZBTB47 | F73C | T | 499 | G | ZNF208 | K843T | T | 2677 | G | ZNF528 | I318N | T | 1354 | A | ZNF844 | L209R | T | 787 | G |
| ZBTB47 | A92T | G | 555 | A | ZNF208 | L85H | A | 403 | T | ZNF528 | R232* | C | 1095 | T | ZNF844 | D436N | G | 1467 | A |
| ZBTB47 | K193N | G | 860 | T | ZNF208 | E58D | T | 323 | G | ZNF528 | R289I | G | 1267 | T | ZNF844 | S570Y | C | 1870 | A |
| ZBTB47 | S44L | C | 412 | T | ZNF211 | R486I | G | 1637 | T | ZNF528 | I600S | T | 2200 | G | ZNF845 | H261R | A | 899 | G |
| ZBTB47 | R399Q | G | 1477 | A | ZNF211 | A218T | G | 832 | A | ZNF529 | C538R | A | 1612 | G | ZNF845 | R682H | G | 2162 | A |
| ZBTB47 | R448C | C | 1623 | T | ZNF211 | R486K | G | 1637 | A | ZNF530 | R256* | C | 986 | T | ZNF845 | A903T | G | 2824 | A |
| ZBTB47 | R584C | C | 2031 | T | ZNF213 | R441H | G | 1769 | A | ZNF530 | R287I | G | 1080 | T | ZNF846 | F123L | G | 900 | T |
| ZBTB48 | P199L | C | 754 | T | ZNF214 | R510H | C | 1832 | T | ZNF532 | E341D | G | 1799 | T | ZNF846 | D92Y | C | 805 | A |
| ZBTB49 | E702K | G | 2225 | A | ZNF217 | C133Y | C | 824 | T | ZNF532 | K1137E | A | 4185 | G | ZNF846 | Y310H | A | 1459 | G |
| ZBTB6 | P238S | G | 801 | A | ZNF217 | K121N | C | 789 | A | ZNF532 | R638Q | G | 2689 | A | ZNF846 | H416Y | G | 1777 | A |
| ZBTB6 | Q252R | T | 844 | C | ZNF217 | K54Q | T | 586 | G | ZNF532 | S388P | T | 1938 | C | ZNF846 | R330* | G | 1519 | A |
| ZBTB7A | L463M | G | 1539 | T | ZNF219 | G642V | C | 2337 | A | ZNF534 | R545C | C | 1694 | T | ZNF846 | R302K | C | 1436 | T |
| ZBTB7A | C412Y | C | 1387 | T | ZNF219 | A423V | G | 1680 | A | ZNF536 | E899D | G | 2835 | T | ZNF846 | K196N | T | 1119 | G |
| ZBTB7A | C387F | C | 1312 | A | ZNF222 | C246Y | G | 832 | A | ZNF536 | R90W | C | 406 | T | ZNF846 | Q91H | T | 804 | G |
| ZBTB7B | V317A | T | 950 | C | ZNF222 | Q124E | C | 465 | G | ZNF536 | S1235I | G | 3842 | T | ZNF852 | R235K | C | 865 | T |
| ZBTB7C | P204H | G | 627 | T | ZNF222 | R477H | G | 1525 | A | ZNF536 | P60S | C | 316 | T | ZNF860 | I97N | T | 839 | A |
| ZBTB7C | E175* | C | 539 | A | ZNF223 | P84H | C | 504 | A | ZNF536 | A272T | G | 952 | A | ZNF860 | R492H | G | 2024 | A |
| ZBTB8A | L105R | T | 543 | G | ZNF224 | D164Y | G | 792 | T | ZNF536 | V248I | G | 880 | A | ZNF860 | F159V | T | 1024 | G |
| ZBTB8B | L384M | C | 1240 | A | ZNF224 | R467W | C | 1701 | T | ZNF536 | P161L | C | 620 | T | ZNF860 | F434L | T | 1851 | G |
| ZBTB8B | A283V | C | 938 | T | ZNF224 | F185L | T | 857 | G | ZNF536 | A841T | G | 2659 | A | ZNF862 | E90K | G | 513 | A |
| ZC3H10 | A296T | G | 1075 | A | ZNF224 | R588I | G | 2065 | T | ZNF536 | R221Q | G | 800 | A | ZNF862 | V588E | T | 2008 | A |
| ZC3H10 | R250W | C | 937 | T | ZNF226 | R285C | C | 997 | T | ZNF536 | G852E | G | 2693 | A | ZNF862 | A938T | G | 3057 | A |
| ZC3H11A | P150H | C | 1446 | A | ZNF226 | H773N | C | 2461 | A | ZNF536 | D1200Y | G | 3736 | T | ZNF862 | A875T | G | 2868 | A |
| ZC3H11A | E528* | G | 2579 | T | ZNF227 | M84T | T | 456 | C | ZNF540 | R451C | C | 1670 | T | ZNF862 | E123K | G | 612 | A |
| ZC3H12A | Q529R | A | 1702 | G | ZNF227 | R400S | G | 1405 | T | ZNF541 | R566Q | C | 1697 | T | ZNF878 | R111* | T | 331 | A |
| ZC3H12C | R29C | C | 85 | T | ZNF229 | L51M | G | 585 | T | ZNF541 | G747D | C | 2240 | T | ZNF878 | R462Q | C | 1385 | T |
| ZC3H12C | V642M | G | 1924 | A | ZNF229 | N268S | T | 1237 | C | ZNF541 | P615T | G | 1843 | T | ZNF878 | Y344C | T | 1031 | C |
| ZC3H12C | P585L | C | 1754 | T | ZNF229 | G580D | C | 2173 | T | ZNF541 | I673T | A | 2018 | G | ZNF878 | E46K | C | 136 | T |
| ZC3H12C | P705L | C | 2114 | T | ZNF23 | P419H | G | 2070 | T | ZNF541 | E1188* | C | 3562 | A | ZNF879 | C430W | T | 1455 | G |
| ZC3H12D | R112W | G | 653 | A | ZNF23 | R412I | C | 2049 | A | ZNF543 | S239N | G | 1061 | A | ZNF880 | R307I | G | 969 | T |
| ZC3H13 | E190* | C | 917 | A | ZNF23 | R384I | C | 1965 | A | ZNF543 | W356* | G | 1413 | A | ZNF880 | P169L | C | 555 | T |

| | | | | | | | | | | | | | | | | | | | |
|---|---|---|---|---|---|---|---|---|---|---|---|---|---|---|---|---|---|---|---|
| ZC3H13 | R1343* | G | 4376 | A | ZNF230 | D116Y | G | 589 | T | ZNF543 | E539A | A | 1961 | C | ZNF880 | R257* | C | 818 | T |
| ZC3H13 | R711C | G | 2480 | A | ZNF230 | I376S | T | 1455 | G | ZNF543 | G140D | G | 764 | A | ZNF880 | K105R | A | 363 | G |
| ZC3H13 | R493H | C | 1827 | T | ZNF230 | K351N | A | 1296 | C | ZNF544 | R81* | C | 715 | T | ZNF90 | L100P | T | 410 | C |
| ZC3H13 | R784Q | C | 2700 | T | ZNF230 | C452R | T | 1682 | C | ZNF544 | K415E | A | 1717 | G | ZNF90 | R326H | G | 1088 | A |
| ZC3H13 | S1217G | T | 3998 | C | ZNF230 | R503C | C | 1750 | T | ZNF544 | T182A | A | 1018 | G | ZNF90 | K156T | A | 578 | C |
| ZC3H13 | R1224Q | C | 4020 | T | ZNF230 | R283I | G | 1145 | T | ZNF544 | V125E | T | 848 | A | ZNF90 | R333I | G | 1109 | T |
| ZC3H13 | E623Q | C | 2216 | G | ZNF230 | R426I | G | 1279 | T | ZNF544 | F200L | C | 1074 | A | ZNF90 | E360* | G | 1189 | T |
| ZC3H13 | R629Q | C | 2235 | T | ZNF232 | R94Q | C | 936 | T | ZNF544 | R540Q | G | 2093 | A | ZNF90 | R436I | G | 1605 | T |
| ZC3H13 | P1103L | G | 3657 | A | ZNF233 | F112C | T | 462 | G | ZNF546 | L344P | T | 1287 | C | ZNF90 | R112I | G | 672 | T |
| ZC3H13 | E722D | C | 2515 | A | ZNF235 | R254C | G | 760 | A | ZNF546 | K246T | A | 993 | C | ZNF91 | K1051T | T | 3265 | G |
| ZC3H13 | V142A | A | 774 | G | ZNF235 | R254H | C | 761 | T | ZNF547 | R182I | G | 738 | T | ZNF92 | H387R | A | 1359 | G |
| ZC3H13 | R681W | G | 2390 | A | ZNF235 | A532V | G | 1595 | A | ZNF548 | F301L | C | 1106 | A | ZNF92 | S94Y | C | 480 | A |
| ZC3H14 | E316G | A | 1103 | G | ZNF235 | E173* | C | 517 | A | ZNF548 | N251K | T | 956 | A | ZNF92 | E289* | G | 1064 | T |
| ZC3H3 | A657V | G | 1999 | A | ZNF236 | T321I | C | 1160 | T | ZNF549 | E157D | G | 652 | T | ZNF99 | K409N | C | 1227 | A |
| ZC3H3 | H473Y | G | 1446 | A | ZNF236 | S198L | C | 791 | T | ZNF549 | C277R | T | 1010 | C | ZNF99 | K130N | C | 390 | A |
| ZC3H4 | G626S | C | 1914 | T | ZNF236 | A154D | C | 659 | A | ZNF549 | C529R | T | 1766 | C | ZNF99 | L349P | A | 1046 | G |
| ZC3H4 | R245W | G | 771 | A | ZNF236 | R1148H | G | 3641 | A | ZNF551 | R383I | G | 1323 | T | ZNF99 | R958H | C | 2873 | T |
| ZC3H4 | P1242T | G | 3762 | T | ZNF236 | A154V | C | 659 | T | ZNF551 | C589Y | G | 1941 | A | ZNF99 | S402P | A | 1204 | G |
| ZC3H4 | E779Q | C | 2373 | G | ZNF236 | A1764V | C | 5489 | T | ZNF551 | Q436E | C | 1481 | G | ZNF99 | L209H | A | 626 | T |
| ZC3H6 | N372H | A | 1515 | C | ZNF236 | R499H | G | 1694 | A | ZNF551 | P200H | C | 774 | A | ZNFX1 | F148L | G | 529 | T |
| ZC3H6 | E831* | G | 2892 | T | ZNF236 | A154T | G | 658 | A | ZNF554 | R359* | C | 1273 | T | ZNFX1 | R1517C | G | 4634 | A |
| ZC3H7A | E657D | T | 2125 | G | ZNF236 | Y971* | T | 3111 | G | ZNF554 | A210V | C | 827 | T | ZNHIT1 | R114H | G | 869 | A |
| ZC3H7A | R760H | C | 2433 | T | ZNF238 | R195* | C | 732 | T | ZNF554 | G246D | G | 935 | A | ZNHIT3 | V49A | T | 211 | C |
| ZC3H7A | R40C | G | 272 | A | ZNF238 | Y432C | A | 1444 | G | ZNF554 | R484I | G | 1649 | T | ZNHIT3 | R47H | G | 205 | A |
| ZC3H7B | G720W | G | 2262 | T | ZNF239 | S249R | A | 1400 | C | ZNF555 | S69Y | C | 304 | A | ZNRF3 | R102* | C | 469 | T |
| ZC3H7B | F568L | C | 1808 | G | ZNF24 | R148W | G | 622 | A | ZNF556 | V306M | G | 1003 | A | ZNRF3 | R102* | C | 469 | T |
| ZC3H7B | A125T | G | 477 | A | ZNF24 | R271I | C | 992 | A | ZNF557 | L109F | C | 798 | T | ZNRF3 | R111* | C | 496 | T |
| ZC3H7B | P301S | C | 1005 | T | ZNF24 | E75K | C | 403 | T | ZNF559 | I206M | T | 1014 | G | ZNRF3 | - | T | 0 | C |
| ZC3H7B | Q772L | A | 2419 | T | ZNF248 | Q440R | T | 1870 | C | ZNF559 | R373I | G | 1514 | T | ZNRF3 | R502Q | G | 1670 | A |
| ZC3H7B | H403N | C | 1311 | A | ZNF248 | E70* | C | 759 | A | ZNF560 | R542I | C | 1835 | A | ZNRF3 | P743S | C | 2392 | T |
| ZC3H7B | A354V | C | 1165 | T | ZNF25 | K424N | C | 1485 | A | ZNF560 | R694Q | C | 2291 | T | ZNRF3 | N476K | C | 1593 | G |
| ZC3H8 | F90V | A | 398 | C | ZNF25 | C347R | A | 1252 | G | ZNF560 | R536Q | C | 1817 | T | ZNRF4 | R68W | C | 279 | T |
| ZC3H8 | E4D | C | 142 | A | ZNF250 | R546C | G | 1726 | A | ZNF561 | K99N | C | 661 | A | ZNRF4 | H249R | A | 823 | G |
| ZC3HAV1 | G334S | C | 1023 | T | ZNF251 | R281H | C | 1098 | T | ZNF561 | N292I | T | 1239 | A | ZNRF4 | R46Q | G | 214 | A |
| ZC3HAV1L | D161Y | C | 493 | A | ZNF252 | R637I | C | 1910 | A | ZNF561 | K254N | C | 1126 | A | ZP1 | A605V | C | 1834 | T |
| ZC4H2 | E34A | T | 208 | G | ZNF254 | N135D | A | 537 | G | ZNF563 | E278D | C | 1040 | A | ZP2 | L414R | A | 1241 | C |
| ZCCHC11 | E378D | T | 1368 | G | ZNF254 | E459G | A | 1510 | G | ZNF563 | Q313* | G | 1143 | A | ZP3 | C46R | T | 194 | C |
| ZCCHC11 | G922D | C | 2999 | T | ZNF256 | R343I | C | 1263 | A | ZNF563 | R437Q | C | 1516 | T | ZP4 | L518R | A | 1712 | C |
| ZCCHC11 | R1554H | C | 4895 | T | ZNF256 | L184I | G | 785 | T | ZNF563 | A3T | C | 213 | T | ZPBP2 | N84T | A | 390 | C |

| | | | | | | | | | | | | | | | | | | | |
|---|---|---|---|---|---|---|---|---|---|---|---|---|---|---|---|---|---|---|---|
| ZCCHC11 | M741I | C | 2457 | T | ZNF256 | A2V | G | 240 | A | ZNF564 | R157Q | C | 651 | T | ZPLD1 | L26R | T | 177 | G |
| ZCCHC11 | K1322T | T | 4199 | G | ZNF256 | Y211H | A | 866 | G | ZNF565 | P46S | G | 181 | A | ZPLD1 | P32L | C | 195 | T |
| ZCCHC11 | D1070N | C | 3442 | T | ZNF256 | P490S | G | 1703 | A | ZNF565 | V76I | C | 271 | T | ZPLD1 | V193M | G | 677 | A |
| ZCCHC11 | E754* | C | 2494 | A | ZNF256 | K324R | T | 1206 | C | ZNF565 | G436S | C | 1351 | T | ZRANB1 | R549Q | G | 2017 | A |
| ZCCHC11 | H288P | T | 1097 | G | ZNF256 | S533R | T | 1832 | G | ZNF565 | G67* | C | 244 | A | ZRANB1 | E708* | G | 2493 | T |
| ZCCHC12 | R85H | G | 761 | A | ZNF257 | - | A | 0 | G | ZNF566 | N416H | T | 1381 | G | ZRANB1 | D369N | G | 1476 | A |
| ZCCHC14 | A647T | C | 1966 | T | ZNF257 | R448Q | G | 1512 | A | ZNF567 | K201T | A | 602 | C | ZRANB1 | R671Q | G | 2383 | A |
| ZCCHC14 | A807T | C | 2446 | T | ZNF257 | E198D | G | 763 | T | ZNF568 | R494I | G | 1987 | T | ZRANB2 | R230H | C | 991 | T |
| ZCCHC14 | P564H | G | 1718 | T | ZNF257 | R526C | C | 1745 | T | ZNF568 | Q347K | C | 1545 | A | ZRANB3 | P266L | G | 914 | A |
| ZCCHC17 | K242N | G | 934 | T | ZNF259 | I219V | T | 715 | C | ZNF568 | E331D | G | 1499 | T | ZRANB3 | I202S | A | 722 | C |
| ZCCHC17 | R89* | C | 473 | T | ZNF263 | R404I | G | 1582 | T | ZNF568 | R491* | C | 1977 | T | ZRSR1 | R434H | G | 1325 | A |
| ZCCHC17 | N54D | A | 368 | G | ZNF263 | G136R | G | 777 | A | ZNF568 | R524* | C | 2053 | T | ZRSR1 | R37W | C | 133 | T |
| ZCCHC2 | A150V | C | 449 | T | ZNF264 | E456K | G | 1779 | A | ZNF569 | E463G | T | 1946 | C | ZRSR2 | R216M | G | 671 | T |
| ZCCHC2 | T409P | A | 1225 | C | ZNF264 | I420T | T | 1672 | C | ZNF569 | F123C | A | 926 | C | ZSCAN1 | R229Q | G | 933 | A |
| ZCCHC3 | G177V | G | 554 | T | ZNF266 | T250A | T | 1380 | C | ZNF57 | M106T | T | 465 | C | ZSCAN1 | A49V | C | 393 | T |
| ZCCHC4 | M484V | A | 1474 | G | ZNF266 | R344Q | C | 1663 | T | ZNF57 | F110L | C | 478 | A | ZSCAN1 | T188A | A | 809 | G |
| ZCCHC6 | A526T | C | 1749 | T | ZNF267 | C450Y | G | 1558 | A | ZNF570 | L203V | T | 1136 | G | ZSCAN1 | R260C | C | 1025 | T |
| ZCCHC6 | I864S | A | 2764 | C | ZNF267 | R596Q | G | 1996 | A | ZNF570 | K181N | G | 1072 | T | ZSCAN1 | M408T | T | 1470 | C |
| ZCCHC6 | N63T | T | 361 | G | ZNF268 | D120G | A | 545 | G | ZNF570 | S231A | T | 1220 | G | ZSCAN1 | V384I | G | 1397 | A |
| ZCWPW1 | K518T | T | 1801 | G | ZNF268 | E45D | G | 321 | T | ZNF571 | G369R | C | 1224 | T | ZSCAN10 | R486W | G | 1544 | A |
| ZCWPW1 | N35T | T | 352 | G | ZNF268 | S357Y | C | 1256 | A | ZNF572 | R289H | G | 1021 | A | ZSCAN10 | P154L | G | 549 | A |
| ZCWPW2 | S308G | A | 1110 | G | ZNF268 | K431Q | A | 1477 | C | ZNF572 | W81* | G | 398 | A | ZSCAN10 | G661V | C | 2070 | A |
| ZCWPW2 | E76* | G | 414 | T | ZNF268 | S9F | C | 356 | T | ZNF573 | R330I | C | 1088 | A | ZSCAN10 | V468M | C | 1490 | T |
| ZDBF2 | R198I | G | 979 | T | ZNF268 | K132T | A | 725 | C | ZNF573 | R218I | C | 752 | A | ZSCAN10 | P131S | G | 479 | A |
| ZDBF2 | E1645D | G | 5321 | T | ZNF268 | I460S | T | 1709 | G | ZNF574 | V139M | G | 415 | A | ZSCAN12 | R46H | C | 283 | T |
| ZDBF2 | V1028I | G | 3468 | A | ZNF268 | R940I | G | 3149 | T | ZNF575 | A178V | C | 533 | T | ZSCAN12 | Q458R | T | 1519 | C |
| ZDBF2 | E1183D | G | 3935 | T | ZNF273 | D41Y | G | 192 | T | ZNF575 | K169N | G | 507 | T | ZSCAN12 | L75P | A | 370 | G |
| ZDBF2 | R2040W | C | 6504 | T | ZNF274 | R464H | G | 1391 | A | ZNF575 | R163C | C | 487 | T | ZSCAN18 | D400N | C | 1228 | T |
| ZDBF2 | T581I | C | 2128 | T | ZNF274 | S417L | C | 1250 | T | ZNF577 | E402* | C | 1570 | A | ZSCAN20 | R1007I | G | 3173 | T |
| ZDBF2 | R641M | G | 2308 | T | ZNF274 | N107S | A | 320 | G | ZNF577 | E402K | C | 1570 | T | ZSCAN20 | R750H | G | 2402 | A |
| ZDBF2 | E888K | G | 3048 | A | ZNF274 | P305L | C | 914 | T | ZNF577 | L398S | A | 1559 | G | ZSCAN20 | P958H | C | 3026 | A |
| ZDBF2 | E256* | G | 1152 | T | ZNF274 | C595Y | G | 1784 | A | ZNF581 | E116K | G | 520 | A | ZSCAN20 | R943C | C | 2980 | T |
| ZDBF2 | K444I | A | 1717 | T | ZNF275 | R272W | C | 814 | T | ZNF581 | R171C | C | 685 | T | ZSCAN20 | L408M | C | 1375 | A |
| ZDBF2 | G1141V | G | 3808 | T | ZNF275 | P352S | C | 1054 | T | ZNF581 | L102I | C | 478 | A | ZSCAN20 | L204P | T | 764 | C |
| ZDBF2 | E1387* | G | 4545 | T | ZNF276 | V364I | G | 1187 | A | ZNF583 | K462N | A | 1579 | C | ZSCAN20 | W326R | T | 1129 | C |
| ZDBF2 | N1433H | A | 4683 | C | ZNF276 | R562Q | G | 1782 | A | ZNF583 | E328* | G | 1175 | T | ZSCAN20 | A706V | C | 2270 | T |
| ZDBF2 | D1711A | A | 5518 | C | ZNF276 | - | T | 0 | C | ZNF583 | G460R | G | 1571 | A | ZSCAN22 | A384V | C | 1298 | T |
| ZDBF2 | E1782* | G | 5730 | T | ZNF28 | R626H | C | 1998 | T | ZNF583 | E517* | G | 1742 | T | ZSCAN22 | G117R | G | 496 | A |
| ZDBF2 | V1999A | T | 6382 | C | ZNF28 | Q646L | T | 2058 | A | ZNF583 | R540I | G | 1812 | T | ZSCAN23 | D163Y | C | 633 | A |

| | | | | | | | | | | | | | | | | | | | |
|---|---|---|---|---|---|---|---|---|---|---|---|---|---|---|---|---|---|---|---|
| ZDHHC1 | E173K | C | 859 | T | ZNF280A | V40A | A | 372 | G | ZNF585A | G210R | C | 859 | T | ZSCAN4 | G265C | G | 1490 | T |
| ZDHHC1 | T224M | G | 1013 | A | ZNF280B | E400K | C | 1941 | T | ZNF585A | S731P | A | 2422 | G | ZSCAN5A | R173H | C | 1214 | T |
| ZDHHC11 | F328V | A | 982 | C | ZNF280B | G456E | C | 2110 | T | ZNF585B | G20D | C | 59 | T | ZSCAN5B | P340L | G | 1019 | A |
| ZDHHC12 | S205L | G | 644 | A | ZNF280B | S90R | A | 1013 | C | ZNF585B | E330K | C | 988 | T | ZSCAN5B | R477H | C | 1430 | T |
| ZDHHC13 | F390L | T | 1275 | G | ZNF280B | K277T | T | 1573 | G | ZNF585B | G210E | C | 629 | T | ZSWIM1 | R88G | A | 357 | G |
| ZDHHC13 | L202F | C | 709 | T | ZNF280C | P78T | G | 386 | T | ZNF589 | A45T | G | 501 | A | ZSWIM2 | - | C | 0 | G |
| ZDHHC14 | P400L | C | 1696 | T | ZNF281 | G34S | C | 225 | T | ZNF589 | V274A | T | 1189 | C | ZSWIM3 | M189V | A | 774 | G |
| ZDHHC14 | E189* | G | 1062 | T | ZNF281 | A233T | C | 822 | T | ZNF589 | M330V | A | 1356 | G | ZSWIM3 | M1T | T | 211 | C |
| ZDHHC15 | N301K | A | 1134 | T | ZNF282 | R613C | C | 1942 | T | ZNF592 | E36D | G | 130 | T | ZSWIM3 | A476T | G | 1635 | A |
| ZDHHC15 | A179S | C | 766 | A | ZNF285 | R560I | C | 1744 | A | ZNF592 | S451N | G | 1374 | A | ZSWIM3 | L645P | T | 2143 | C |
| ZDHHC16 | R5Q | G | 203 | A | ZNF285 | E173D | C | 584 | A | ZNF592 | H542R | A | 1647 | G | ZSWIM4 | S458N | G | 1562 | A |
| ZDHHC16 | W287* | G | 1050 | A | ZNF287 | Q749R | T | 2699 | C | ZNF592 | L395P | T | 1206 | C | ZSWIM4 | Q332* | C | 1183 | T |
| ZDHHC16 | V154I | G | 649 | A | ZNF287 | E185* | C | 1006 | A | ZNF594 | Q380H | C | 1296 | G | ZSWIM4 | A755T | G | 2452 | A |
| ZDHHC17 | T373M | C | 1767 | T | ZNF287 | R64* | G | 643 | A | ZNF594 | A337S | C | 1165 | A | ZSWIM4 | P348L | C | 1232 | T |
| ZDHHC18 | V279M | G | 930 | A | ZNF292 | E63D | G | 230 | T | ZNF594 | R753I | C | 2414 | A | ZSWIM5 | N464D | T | 1596 | C |
| ZDHHC18 | R354W | C | 1155 | T | ZNF292 | C456* | T | 1409 | A | ZNF596 | R355Q | G | 1170 | A | ZSWIM5 | A681T | C | 2247 | T |
| ZDHHC19 | R181C | G | 621 | A | ZNF292 | R633* | C | 1938 | T | ZNF597 | K92T | T | 506 | G | ZSWIM5 | R761C | G | 2487 | A |
| ZDHHC2 | A2V | C | 402 | T | ZNF292 | T1017A | A | 3090 | G | ZNF597 | R222C | G | 895 | A | ZSWIM6 | V1119L | G | 3355 | T |
| ZDHHC2 | A111T | G | 728 | A | ZNF292 | R1349Q | G | 4087 | A | ZNF598 | V301I | C | 916 | T | ZSWIM6 | A273T | G | 817 | A |
| ZDHHC2 | R139C | C | 812 | T | ZNF292 | T1294R | C | 3922 | G | ZNF598 | R274C | G | 835 | A | ZSWIM6 | Q712R | A | 2135 | G |
| ZDHHC22 | F173L | G | 722 | T | ZNF292 | E825G | A | 2515 | G | ZNF599 | R275H | C | 1212 | T | ZSWIM6 | K309T | A | 926 | C |
| ZDHHC23 | P9H | C | 325 | A | ZNF292 | A2209V | C | 6667 | T | ZNF599 | H142Q | G | 814 | C | ZSWIM6 | W1074R | T | 3220 | C |
| ZDHHC3 | R96W | G | 544 | A | ZNF292 | T927A | A | 2820 | G | ZNF600 | R350I | C | 1317 | A | ZW10 | A411S | C | 1268 | A |
| ZDHHC5 | R592I | G | 3137 | T | ZNF292 | A1182T | G | 3585 | A | ZNF600 | K160N | T | 748 | G | ZWILCH | K520N | G | 1806 | T |
| ZDHHC5 | A60T | G | 1540 | A | ZNF292 | E393* | G | 1218 | T | ZNF605 | R556I | C | 1667 | A | ZWINT | E2* | C | 43 | A |
| ZDHHC6 | R317C | G | 1373 | A | ZNF292 | E582D | A | 1787 | C | ZNF606 | R476I | C | 2045 | A | ZWINT | I22T | A | 104 | G |
| ZDHHC6 | R364* | G | 1514 | A | ZNF292 | S1004Y | C | 3052 | A | ZNF606 | Q684H | C | 2670 | A | ZXDA | D87Y | C | 472 | A |
| ZDHHC6 | P80L | G | 663 | A | ZNF292 | L1063V | T | 3228 | G | ZNF606 | K678N | T | 2652 | G | ZXDC | R386Q | C | 1211 | T |
| ZDHHC8 | G378D | G | 1133 | A | ZNF292 | L1454I | C | 4401 | A | ZNF606 | K569T | T | 2324 | G | ZXDC | A739T | C | 2269 | T |
| ZDHHC8 | P668H | C | 2003 | A | ZNF292 | R2270Q | G | 6850 | A | ZNF607 | E453* | C | 1953 | A | ZYG11A | R411C | C | 1379 | T |
| ZDHHC8 | R523H | G | 1568 | A | ZNF295 | E741K | C | 2405 | T | ZNF608 | H979Y | G | 3058 | A | ZYG11A | - | G | 0 | A |
| ZEB1 | K507R | A | 1558 | G | ZNF295 | A896S | C | 2870 | A | ZNF608 | H1339R | T | 4139 | C | ZYG11A | N446H | A | 1484 | C |
| ZEB1 | A604V | C | 1849 | T | ZNF295 | R742W | G | 2408 | A | ZNF609 | R462C | C | 1384 | T | ZYG11A | K469N | G | 1555 | T |
| ZEB1 | S976F | C | 2965 | T | ZNF295 | P998H | G | 3177 | T | ZNF609 | R240H | G | 719 | A | ZYG11B | M355V | A | 1208 | G |
| ZEB1 | A1015V | C | 3082 | T | ZNF295 | F1043C | A | 3312 | C | ZNF609 | D1164G | A | 3491 | G | ZYG11B | Y334* | C | 1147 | G |
| ZEB1 | R968C | C | 2940 | T | ZNF295 | N949H | T | 3029 | G | ZNF609 | S626Y | C | 1877 | A | ZYG11B | A101D | C | 447 | A |
| ZEB1 | P96S | C | 324 | T | ZNF296 | K107T | T | 377 | G | ZNF610 | G350D | G | 1505 | A | ZYG11B | E573* | G | 1862 | T |
| ZEB2 | Q3* | G | 529 | A | ZNF3 | S409L | G | 1533 | A | ZNF610 | V29M | G | 541 | A | ZZEF1 | A2169V | G | 6631 | A |
| ZER1 | R35W | G | 510 | A | ZNF30 | H283N | C | 1291 | A | ZNF610 | W251* | G | 1208 | A | ZZEF1 | L2237R | A | 6835 | C |

| | | | | | | | | | | | | | | | | | | | |
|---|---|---|---|---|---|---|---|---|---|---|---|---|---|---|---|---|---|---|---|
| ZER1 | S79N | C | 643 | T | ZNF30 | H615Y | C | 2287 | T | ZNF611 | E451D | C | 1670 | A | ZZEF1 | V991M | C | 3096 | T |
| ZER1 | - | C | 0 | T | ZNF30 | H335Y | C | 1447 | T | ZNF613 | G542C | G | 1822 | T | ZZEF1 | R2584C | G | 7875 | A |
| ZER1 | F473V | A | 1824 | C | ZNF30 | - | G | 0 | A | ZNF613 | V205G | T | 812 | G | ZZEF1 | E2486K | C | 7581 | T |
| ZFAND1 | R91H | C | 286 | T | ZNF300 | D229V | T | 686 | A | ZNF613 | E369D | G | 1305 | T | ZZEF1 | G1818S | C | 5577 | T |
| ZFAND2A | R156C | G | 701 | A | ZNF300 | K395N | T | 1185 | G | ZNF614 | L10M | G | 430 | T | ZZEF1 | R898H | C | 2818 | T |
| ZFAND3 | S48P | T | 589 | C | ZNF300 | R529I | C | 1586 | A | ZNF614 | R335Q | C | 1406 | T | ZZEF1 | Y1864C | T | 5716 | C |
| ZFAT | R419H | C | 1431 | T | ZNF300 | G375R | C | 1123 | T | ZNF615 | R711I | C | 2429 | A | ZZEF1 | R1228H | C | 3808 | T |
| ZFAT | R787H | C | 2535 | T | ZNF302 | C425Y | G | 1274 | A | ZNF615 | R674H | C | 2318 | T | ZZEF1 | R1539* | G | 4740 | A |
| ZFAT | G690A | C | 2244 | G | ZNF304 | Q181P | A | 930 | C | ZNF615 | T630A | T | 2185 | C | ZZZ3 | G350R | C | 1524 | T |
| ZFAT | R474C | G | 1595 | A | ZNF304 | R317I | G | 1338 | T | ZNF615 | R515Q | C | 1841 | T | ZZZ3 | R584H | C | 2227 | T |
| ZFAT | A1190V | G | 3744 | A | ZNF304 | H544R | A | 2019 | G | ZNF615 | K387T | T | 1457 | G | ZZZ3 | R118I | C | 829 | A |
| ZFAT | A650T | C | 2123 | T | ZNF317 | M521I | G | 1851 | A | ZNF615 | R263I | C | 1085 | A | ZZZ3 | E110* | C | 804 | A |

Exons were captured using Nimblegen SeqCap EZ human exome library v2.0 and sequenced on HiSeq 2000 (Illumina, CA) to generate 75 bp paired-end sequencing reads. The targeted regions had a mean coverage of 179x with 97.4% bases covered at ≥10 times. 95,075 somatic mutations in the 72 colon tumor samples analyzed were identified of which 36,303 were protein-altering. Two MSS samples showed an unusually large number of mutations (24,830 and 5,780 mutations of which 9,479 and 2,332 were protein-altering mutations respectively). These were designated as hypermutated samples and were not considered for calculating the background mutation rate. 52,312 somatic mutations in the 15 MSI samples (18,436 missense, 929 nonsense, 22 stop lost, 436 essential splice site, 363 protein-altering indels, 8,065 synonymous, 16,675 intronic and 7,386 others) and 12,153 somatic mutations in the 55 MSS samples (3,922 missense, 289 nonsense, 6 stop lost, 69 essential splice site, 20 protein-altering indels 1,584 synonymous, 4,375 intronic and 1,888 others) studied (Table 2 and 3) were found. About 98% (35,524/36,303) of the protein altering single nucleotide variants reported in these examples are novel and have not been reported in COSMIC v54 (Forbes, S. A. et al., Nucleic Acids Res. 38:D652-657 (2010)). Thirty seven percent of the somatic mutations reported were validated using RNA-seq data or mass spectrometry genotyping with a validation rate of 93% (Table 2). All the indels reported were confirmed somatic using Sanger sequencing (Table 3-Somatic Indels). A mean non-synonymous mutation rate of 2.8/Mb (31-149 coding region mutations in the 55 samples) in the MSS samples and 40/Mb (764-3113 coding region mutations in the 15 samples) in the MSI samples was observed, consistent with the MMR defect in the later.

***Table 3 - Somatic Indels***

| Gene | Location | Pos. cDNA | Pos. protein | AA chq | Ref | Var |
|---|---|---|---|---|---|---|
| PRMT6 | 1:107599370 | 70 | 11 | - | CG | C |
| KCNA10 | 1:111060763 | 1035 | 216 | - | AC | A |
| CSDE1 | 1:115262367 | 0 | 0 | - | GA | G |
| SIKE1 | 1:115316998 | 0 | 0 | - | GA | G |
| SYCP1 | 1:115537601 | 3132 | 964 | - | GA | G |
| VANGL1 | 1:116206586 | 780 | 170 | - | CT | C |
| PRDM2 | 1:14108749 | 5315 | 1487 | - | CA | C |
| PIAS3 | 1:145585533 | 1888 | 600 | - | TG | T |
| BCL9 | 1:147091501 | 2280 | 514 | - | AC | A |
| BCL9 | 1:147092681-147092680 | 3459 | 907 | - | - | C |
| ZNF687 | 1:151261079 | 2337 | 731 | - | AC | A |
| RFX5 | 1:151318741 | 235 | 19 | - | TG | T |
| RFX5 | 1:151318741 | 235 | 19 | - | TG | T |
| PYGO2 | 1:154932028 | 620 | 150 | - | TG | T |
| UBQLN4 | 1:156020953 | 519 | 142 | - | GC | G |
| NES | 1:156640235 | 3878 | 1249 | - | AC | A |
| KIRREL | 1:158057655 | 0 | 0 | - | AG | A |
| BRP44 | 1:167893779 | 0 | 0 | - | GA | G |
| CACYBP | 1:174976327 | 874 | 142 | - | CA | C |
| RASAL2 | 1:178426849-178426857 | 2774 | 808 | DNT/- | GGACAACACA (SEQ ID NO:84) | G |
| ASPM | 1:197059222 | 0 | 0 | - | GA | G |
| UBE2T | 1:202304824 | 209 | 20 | - | TG | T |
| PLEKHA6 | 1:204228411 | 1359 | 348 | - | | |
| PLEKHA6 | 1:204228411 | 1359 | 348 | - | AC | A |
| PLEKHA6 | 1:204228411 | 1359 | 348 | - | AC | A |
| PLEKHA6 | 1:204228411 | 1359 | 348 | - | AC | A |

(continued)

| Gene | Location | Pos. cDNA | Pos. protein | AA chq | Ref | Var |
|---|---|---|---|---|---|---|
| DYRK3 | 1:206821441 | 1066 | 300 | - | TA | T |
| RPS6KC1 | 1:213414598 | 1929 | 593 | - | CA | C |
| CENPF | 1:214815702 | 4189 | 1341 | - | GA | G |
| TGFB2 | 1:218609371 | 1365 | 300 | - | GA | G |
| ITPKB | 1:226924541 | 619 | 207 | - | TC | T |
| OBSCN | 1:228481047 | 0 | 0 | - | TC | T |
| CHRM3 | 1:240071597 | 1625 | 282 | - | AC | A |
| TCEB3 | 1:24078404 | 1658 | 463 | - | TA | T |
| AHCTF1 | 1:247014550 | 4872 | 1624 | - | CA | C |
| RHD | 1:25599125 | 145 | 29 | - | AT | A |
| FAM54B | 1:26156056 | 741 | 203 | - | TC | T |
| EPHA10 | 1:38185238 | 2690 | 868 | - | TG | T |
| PTCH2 | 1:45293652-45293653 | 2051 | 640 | - | GAC | G |
| FAM151A | 1:55078268-55078270 | 850 | 230 | KM/M | ATCT | A |
| L1TD1 | 1:62675692-62675694 | 1541 | 416 | E/- | GGAA | G |
| RPE65 | 1:68904737 | 940 | 296 | - | CT | C |
| ZNF644 | 1:91406040 | 1089 | 291 | - | CT | C |
| ADD3 | 10:111893350 | 2462 | 699 | - | CA | C |
| DHTKD1 | 10:12139966-12139967 | 1704 | 548 | - | GCA | G |
| TACC2 | 10:123842278 | 603 | 88 | - | AG | A |
| KIAA1217 | 10:24783491 | 1772 | 581 | - | CT | C |
| PTCHD3 | 10:27702951 | 347 | 77 | - | CG | C |
| SVIL | 10:29760116 | 6036 | 1862 | - | TC | T |
| ZEB1 | 10:31815887-31815886 | 3107 | 1023 | - | - | GA |
| ANK3 | 10:61831290-61831289 | 9541 | 3117 | - | - | T |
| SIRT1 | 10:69648852 | 813 | 254 | - | CA | C |
| DDX50 | 10:70666693-70666692 | 420 | 105 | - | - | A |
| USP54 | 10:75290284 | 0 | 0 | - | TA | T |
| BTAF1 | 10:93756247 | 3443 | 1144 | - | AT | A |
| MYOF | 10:95079629 | 5598 | 1866 | - | CT | C |
| HELLS | 10:96352051-96352050 | 1937 | 611 | - | - | A |
| GOLGA7B | 10:99619319-99619318 | 181 | 39 | - | - | C |
| AP2A2 | 11:1000475 | 2184 | 668 | - | GC | G |
| ZBED5 | 11:10875781 | 1211 | 238 | - | AT | A |
| C11orf57 | 11:111953460 | 769 | 216 | - | CA | C |
| SIDT2 | 11:117052572 | 876 | 119 | - | GC | G |
| MFRP | 11:119213688 | 1297 | 384 | - | TG | T |

(continued)

| Gene | Location | Pos. cDNA | Pos. protein | AA chq | Ref | Var |
|---|---|---|---|---|---|---|
| PKNOX2 | 11:125237794 | 454 | 47 | - | GC | G |
| ZBTB44 | 11:130131353 | 710 | 139 | - | CT | C |
| COPB1 | 11:14504704 | 0 | 0 | - | TA | T |
| MYOD1 | 11:17742463-17742462 | 864 | 215 | - | - | C |
| KCNC1 | 11:17794004 | 1418 | 455 | - | GA | G |
| PTPN5 | 11:18751286-18751285 | 1840 | 470 | - | - | G |
| PAX6 | 11:31812317 | 1635 | 389 | - | TG | T |
| CCDC73 | 11:32635625 | 2283 | 747 | - | GT | G |
| UBQLN3 | 11:5529015 | 1922 | 592 | - | GA | G |
| TNKS1BP1 | 11:57080526 | 1801 | 546 | - | TC | T |
| FAM111B | 11:58892377 | 998 | 269 | - | CA | C |
| PATL1 | 11:59434440 | 0 | 0 | - | TA | T |
| PRPF19 | 11:60666410 | 0 | 0 | - | GA | G |
| STX5 | 11:62598585 | 285 | 44 | - | TG | T |
| RIN1 | 11:66102953-66102955 | 597 | 157 | LP/P | GGGA | G |
| SPTBN2 | 11:66457417 | 0 | 0 | - | TG | T |
| PC | 11:66617803 | 2655 | 869 | - | GC | G |
| SWAP70 | 11:9735070 | 397 | 100 | - | CA | C |
| NCOR2 | 12:124846685 | 3240 | 1028 | - | CG | C |
| SFRS8 | 12:132210169 | 966 | 276 | - | GA | G |
| GOLGA3 | 12:133375067 | 0 | 0 | - | TA | T |
| ATF7IP | 12:14578133-14578134 | 1437 | 428 | - | ACT | A |
| KDM5A | 12:416953 | 3960 | 1199 | - | CT | C |
| FAM113B | 12:47628998 | 883 | 51 | - | AG | A |
| MLL2 | 12:49434492 | 7061 | 2354 | - | AG | A |
| ACVR1B | 12:52374795 | 665 | 208 | - | GT | G |
| ESPL1 | 12:53677181 | 3027 | 979 | - | CA | C |
| DGKA | 12:56347514 | 2434 | 724 | - | AC | A |
| BAZ2A | 12:57004252 | 1920 | 576 | - | TC | T |
| GLI1 | 12:57860075 | 893 | 272 | - | TG | T |
| LRIG3 | 12:59279691 | 0 | 0 | - | GA | G |
| ATN1 | 12:7045535 | 1342 | 369 | - | GC | G |
| PTPRB | 12:70981054 | 0 | 0 | - | GA | G |
| ZFC3H1 | 12:72021721 | 0 | 0 | - | TA | T |
| ZFC3H1 | 12:72021721 | 0 | 0 | - | TA | T |
| PTPRQ | 12:80904230-80904229 | 0 | 0 | - | - | T |
| PTPRQ | 12:81063246 | 0 | 0 | - | TA | T |

(continued)

| Gene | Location | Pos. cDNA | Pos. protein | AA chq | Ref | Var |
|---|---|---|---|---|---|---|
| MGAT4C | 12:86373479 | 1112 | 371 | - | AG | A |
| ELK3 | 12:96641029 | 798 | 173 | - | GC | G |
| TMPO | 12:98921672 | 492 | 96 | - | CA | C |
| UPF3A | 13:115057211 | 846 | 264 | - | CA | C |
| KL | 13:33628153-33628152 | 1076 | 356 | - | - | A |
| SPG20 | 13:36909782-36909783 | 246 | 62 | - | CTT | C |
| MRPS31 | 13:41323308-41323307 | 961 | 308 | - | - | C |
| NAA16 | 13:41892982 | 504 | 60 | - | GA | G |
| ZC3H13 | 13:46543661-46543660 | 3367 | 1006 | - | - | T |
| DIAPH3 | 13:60348388 | 0 | 0 | - | TA | T |
| DYNC1H1 | 14:102483256 | 7932 | 2590 | - | GC | G |
| TPPP2 | 14:21498757-21498756 | 140 | 6 | - | - | A |
| CHD8 | 14:21862450 | 5180 | 1727 | - | TG | T |
| ACIN1 | 14:23549379 | 1667 | 447 | - | GC | G |
| CBLN3 | 14:24898079 | 653 | 61 | - | TC | T |
| CTAGE5 | 14:39788502 | 0 | 0 | - | CT | C |
| C14orf106 | 14:45693722 | 2527 | 690 | - | CT | C |
| MAP4K5 | 14:50952368 | 0 | 0 | - | CA | C |
| SPTB | 14:65259995 | 2440 | 800 | - | CG | C |
| ISM2 | 14:77948984-77948983 | 711 | 218 | - | - | A |
| PTPN21 | 14:88940113 | 2750 | 849 | - | AT | A |
| DICER1 | 14:95583036 | 0 | 0 | - | GA | G |
| NIPA2 | 15:23021236 | 714 | 34 | - | GC | G |
| DUOXA2 | 15:45406932 | 414 | 43 | - | CG | C |
| ADAM10 | 15:59009931 | 0 | 0 | - | TA | T |
| TLN2 | 15:63054019 | 4811 | 1593 | - | GA | G |
| HERC1 | 15:64015557 | 0 | 0 | - | TA | T |
| ISL2 | 15:76633583-76633582 | 1063 | 301 | - | - | A |
| KIAA1024 | 15:79750586 | 2172 | 699 | - | TA | T |
| BNC1 | 15:83933100 | 989 | 301 | - | CT | C |
| ANPEP | 15:90334189 | 2978 | 888 | - | TA | T |
| SV2B | 15:91832792-91832791 | 2219 | 583 | - | - | T |
| UBE2I | 16:1370650 | 662 | 182 | - | CG | C |
| ARHGAP17 | 16:24942180 | 2533 | 814 | - | TG | T |
| GTF3C1 | 16:27509009 | 2339 | 767 | - | CT | C |
| ZNF785 | 16:30594709-30594710 | 433 | 130 | - | CTT | C |
| ZNF434 | 16:3433715 | 0 | 0 | - | GA | G |

(continued)

| Gene | Location | Pos. cDNA | Pos. protein | AA chq | Ref | Var |
|---|---|---|---|---|---|---|
| CREBBP | 16:3817721 | 4055 | 1084 | - | CT | C |
| CTCF | 16:67645339-67645338 | 1047 | 201 | - | - | A |
| CDH1 | 16:68863582 | 2512 | 774 | - | AG | A |
| FTSJD1 | 16:71318173-71318172 | 1988 | 551 | - | - | A |
| ZFHX3 | 16:72992483 | 2235 | 521 | - | CT | C |
| USP7 | 16:9017275 | 0 | 0 | - | CA | C |
| NUFIP2 | 17:27614342 | 759 | 224 | - | CT | C |
| EVI2B | 17:29632035 | 741 | 198 | - | GT | G |
| MED1 | 17:37564512 | 4168 | 1321 | - | AC | A |
| WIPF2 | 17:38420993 | 805 | 189 | - | AC | A |
| FKBP10 | 17:39975559 | 929 | 275 | - | TC | T |
| COL1A1 | 17:48271492 | 1786 | 556 | - | AG | A |
| SFRS1 | 17:56083739 | 553 | 115 | - | TG | T |
| RNF43 | 17:56435161 | 2464 | 659 | - | AC | A |
| RNF43 | 17:56438159-56438161 | 1320 | 278 | E/- | ACTC | A |
| USP32 | 17:58300952 | 0 | 0 | - | TA | T |
| SMURF2 | 17:62602763 | 0 | 0 | - | TA | T |
| TP53 | 17:7578222-7578223 | 816 | 209 | - | TTC | T |
| TP53 | 17:7578262-7578263 | 776 | 196 | - | TCG | T |
| TP53 | 17:7578475 | 645 | 152 | - | CG | C |
| TP53 | 17:7579420 | 457 | 89 | - | AG | A |
| DNAH2 | 17:7697598-7697597 | 7609 | 2532 | - | - | C |
| CBX8 | 17:77768662 | 1060 | 314 | - | TG | T |
| TEX19 | 17:80320302-80320301 | 585 | 92 | - | - | G |
| RNF138 | 18:29709075-29709074 | 0 | 0 | - | - | T |
| KLHL14 | 18:30350229-30350231 | 712 | 108 | SS/S | GGAA | G |
| RTTN | 18:67697249 | 5812 | 1915 | - | CT | C |
| SMARCA4 | 19:11141498 | 3759 | 1159 | - | TG | T |
| DAZAP1 | 19:1430254 | 953 | 255 | - | GC | G |
| CLEC17A | 19:14698433-14698435 | 167 | 43 | ME/M | TGGA | T |
| NOTCH3 | 19:15302611 | 823 | 249 | - | TC | T |
| TMEM59L | 19:18727842-18727841 | 680 | 198 | - | - | G |
| C19orf12 | 19:30193879 | 326 | 67 | - | GC | G |
| TLE2 | 19:3028804 | 0 | 0 | - | TG | T |
| CLIP3 | 19:36509879 | 1332 | 368 | - | AG | A |
| ZNF585A | 19:37644213-37644212 | 819 | 196 | - | - | A |
| RYR1 | 19:38979989 | 5850 | 1907 | - | GA | G |

(continued)

| Gene | Location | Pos. cDNA | Pos. protein | AA chq | Ref | Var |
|---|---|---|---|---|---|---|
| SUPT5H | 19:39961164-39961163 | 1856 | 559 | - | - | GT |
| C19orf69 | 19:41949132 | 70 | 20 | - | AC | A |
| ZNF284 | 19:44590645 | 1172 | 338 | - | CA | C |
| ZNF230 | 19:44635227 | 703 | 154 | - | TA | T |
| ZNF541 | 19:48025197 | 3682 | 1228 | - | AT | A |
| GRIN2D | 19:48908418 | 981 | 298 | - | GC | G |
| TEAD2 | 19:49850473 | 974 | 295 | - | TG | T |
| SLC17A7 | 19:49933867 | 1764 | 531 | - | CG | C |
| PPP1R12C | 19:55607456 | 1132 | 372 | - | TG | T |
| IL11 | 19:55877466 | 645 | 170 | - | GC | G |
| MAP2K7 | 19:7968894-7968893 | 64 | 22 | - | | |
| MAP2K7 | 19:7975006 | 325 | 109 | - | CG | C |
| GCC2 | 2:109087914 | 2176 | 710 | - | GT | G |
| LYPD1 | 2:133426062-133426061 | 170 | 57 | - | - | T |
| RIF1 | 2:152319747 | 3874 | 1238 | - | TC | T |
| NEB | 2:152471104 | 0 | 0 | - | TA | T |
| PXDN | 2:1670168 | 1160 | 370 | - | CG | C |
| NOSTRIN | 2:169721406 | 2367 | 538 | - | GA | G |
| GAD1 | 2:171702015 | 0 | 0 | - | AG | A |
| RAD51AP2 | 2:17698737 | 970 | 316 | - | GT | G |
| CERKL | 2:182430854 | 0 | 0 | - | TA | T |
| AOX1 | 2:201469483 | 975 | 245 | - | TC | T |
| BMPR2 | 2:203420130 | 2281 | 581 | - | GA | G |
| BMPR2 | 2:203420130 | 2281 | 581 | - | GA | G |
| AAMP | 2:219132279 | 427 | 112 | - | AC | A |
| ZNF142 | 2:219507691-219507692 | 3969 | 1183 | - | GCT | G |
| RNF25 | 2:219528925 | 1576 | 379 | - | AG | A |
| NGEF | 2:233785196 | 905 | 209 | - | CG | C |
| HJURP | 2:234746304 | 0 | 0 | - | GA | G |
| AGAP1 | 2:236649677 | 1672 | 392 | - | GC | G |
| HDAC4 | 2:240002823 | 3495 | 901 | - | TG | T |
| EMILIN1 | 2:27305819 | 1879 | 460 | - | TG | T |
| FAM82A1 | 2:38178783 | 541 | 142 | - | AT | A |
| SLC8A1 | 2:40656343 | 1239 | 360 | - | CT | C |
| OXER1 | 2:42991089 | 313 | 77 | - | AC | A |
| STON1-GTF2A1L | 2:48808425 | 764 | 218 | - | CA | C |

(continued)

| Gene | Location | Pos. cDNA | Pos. protein | AA chq | Ref | Var |
|---|---|---|---|---|---|---|
| PCYOX1 | 2:70502282 | 714 | 229 | - | AC | A |
| DNAH6 | 2:84752697 | 371 | 78 | - | TA | T |
| TXNDC9 | 2:99936266-99936270 | 0 | 0 | - | TAAAAA | T |
| ESF1 | 20:13740507 | 0 | 0 | - | GA | G |
| POFUT1 | 20:30804473 | 553 | 164 | - | CT | C |
| ASXL1 | 20:31022442 | 2353 | 643 | - | AG | A |
| ROMO1 | 20:34287672 | 298 | 40 | - | CT | C |
| RBL1 | 20:35663914 | 0 | 0 | - | TA | T |
| ZNF831 | 20:57766220 | 146 | 49 | - | GC | G |
| SYCP2 | 20:58467047 | 2501 | 788 | - | AT | A |
| NRIP1 | 21:16338330 | 2788 | 728 | - | CT | C |
| CXADR | 21:18933045 | 1345 | 199 | - | TA | T |
| KRTAP25-1 | 21:31661780 | 53 | 10 | - | GA | G |
| DOPEY2 | 21:37619932 | 0 | 0 | - | AT | A |
| BRWD1 | 21:40558989 | 7254 | 2309 | - | TA | T |
| ZNF295 | 21:43412316-43412315 | 2073 | 630 | - | - | TC |
| TRPM2 | 21:45837907 | 3257 | 1082 | - | GC | G |
| SMARCB1 | 22:24175857-24175859 | 1319 | 371 | EK/E | GAGA | G |
| ZNRF3 | 22:29445999-29445998 | 1694 | 510 | - | - | G |
| TIMP3 | 22:33255324 | 897 | 199 | - | GC | G |
| LARGE | 22:33733727-33733726 | 1764 | 398 | - | - | G |
| TRIOBP | 22:38130773 | 4685 | 1477 | - | TG | T |
| ATF4 | 22:39917951 | 1172 | 134 | - | GC | G |
| CERK | 22:47086002 | 1541 | 476 | - | TC | T |
| CERK | 22:47103788 | 780 | 223 | - | CG | C |
| PLXNB2 | 22:50714395 | 0 | 0 | - | TG | T |
| MORC1 | 3:108813922 | 0 | 0 | - | TA | T |
| KIAA2018 | 3:113375178 | 5762 | 1784 | - | TG | T |
| POLQ | 3:121248570-121248569 | 1429 | 477 | - | - | A |
| NPHP3 | 3:132420382-132420381 | 0 | 0 | - | - | A |
| TMEM108 | 3:133099024-133099023 | 678 | 156 | - | - | C |
| HDAC11 | 3:13538268 | 468 | 95 | - | TC | T |
| ATR | 3:142274740 | 2442 | 774 | - | AT | A |
| SLC9A9 | 3:143567076-143567075 | 298 | 30 | - | - | A |
| C3orf16 | 3:149485161-149485160 | 1745 | 430 | - | - | T |
| NR2C2 | 3:15084406 | 1956 | 580 | - | CT | C |
| DHX36 | 3:154007619 | 0 | 0 | - | TA | T |

(continued)

| Gene | Location | Pos. cDNA | Pos. protein | AA chq | Ref | Var |
|---|---|---|---|---|---|---|
| METTL6 | 3:15466599 | 0 | 0 | - | TG | T |
| SMC4 | 3:160134209-160134210 | 0 | 0 | - | GTT | G |
| SMC4 | 3:160143940 | 3008 | 853 | - | CA | C |
| FAM131A | 3:184062513-184062512 | 1034 | 285 | - | - | C |
| TGFBR2 | 3:30691872 | 732 | 150 | - | GA | G |
| TRAK1 | 3:42242450 | 1731 | 444 | - | AC | A |
| PTH1R | 3:46930537 | 0 | 0 | - | TG | T |
| SETD2 | 3:47165283 | 886 | 281 | - | CT | C |
| PLXNB1 | 3:48465485 | 639 | 179 | - | AC | A |
| COL7A1 | 3:48612871 | 6189 | 2027 | - | CG | C |
| APEH | 3:49713809-49713808 | 0 | 0 | - | - | A |
| HESX1 | 3:57232526 | 0 | 0 | - | GA | G |
| ATXN7 | 3:63981832 | 2887 | 778 | - | GC | G |
| UBA3 | 3:69111085 | 0 | 0 | - | TA | T |
| EMCN | 4:101337124 | 0 | 0 | - | GA | G |
| GSTCD | 4:106640295 | 725 | 169 | - | GC | G |
| TBCK | 4:106967842 | 0 | 0 | - | GA | G |
| ANK2 | 4:114280135 | 10414 | 3454 | - | AG | A |
| KIAA1109 | 4:123192271-123192270 | 7964 | 2531 | - | - | C |
| SLC7A11 | 4:139153539 | 0 | 0 | - | TA | T |
| UCP1 | 4:141484372-141484373 | 0 | 0 | - | GAA | G |
| FGFBP1 | 4:15938178 | 373 | 26 | - | CT | C |
| FGFBP1 | 4:15938178 | 373 | 26 | - | CT | C |
| SNX25 | 4:186272695 | 2200 | 636 | - | GA | G |
| FAT1 | 4:187549521 | 0 | 0 | - | TA | T |
| LGI2 | 4:25005321 | 1576 | 464 | - | GC | G |
| SH3BP2 | 4:2831451-2831450 | 901 | 301 | - | - | C |
| RGS12 | 4:3432431 | 4767 | 1288 | - | AC | A |
| KLF3 | 4:38690460 | 617 | 104 | - | TA | T |
| ZBTB49 | 4:4304019-4304018 | 576 | 152 | - | - | C |
| TEC | 4:48169933-48169935 | 689 | 177 | ED/D | ATCT | A |
| KIAA1211 | 4:57179443 | 826 | 145 | - | TC | T |
| UGT2A2 | 4:70512968-70512967 | 451 | 132 | - | - | T |
| APC | 5:112116587-112116586 | 1011 | 211 | - | | |
| APC | 5:112164566 | 2020 | 547 | - | GT | G |
| APC | 5:112173784-112173783 | 2872 | 831 | - | | |
| APC | 5:112173987 | 3076 | 899 | - | AC | A |

(continued)

| Gene | Location | Pos. cDNA | Pos. protein | AA chq | Ref | Var |
|---|---|---|---|---|---|---|
| APC | 5:112174659-112174658 | 3747 | 1123 | - | | |
| APC | 5:112175162 | 4251 | 1291 | - | TC | T |
| APC | 5:112175212-112175216 | 4301 | 1307 | - | TAAAAG | T |
| APC | 5:112175530-112175529 | 4618 | 1413 | - | | |
| APC | 5:112175548-112175549 | 4637 | 1419 | - | GCC | G |
| APC | 5:112175746 | 4835 | 1485 | - | CT | C |
| APC | 5:112175752 | 4841 | 1487 | - | CT | C |
| APC | 5:112175752-112175755 | 4841 | 1487 | - | CTTTA | C |
| ZNF608 | 5:123983544 | 2656 | 845 | - | GC | G |
| FSTL4 | 5:132534947-132534946 | 2619 | 790 | - | - | C |
| PCDHB1 | 5:140431111 | 151 | 19 | - | AT | A |
| PCDHGC3 | 5:140857742 | 2173 | 687 | - | GA | G |
| PCDH1 | 5:141244531-141244533 | 1511 | 455 | K/- | ACTT | A |
| PDE6A | 5:149301270 | 981 | 287 | - | AT | A |
| C5orf52 | 5:157106903 | 438 | 126 | - | GA | G |
| GABRA6 | 5:161115971-161115970 | 516 | 81 | - | - | T |
| DOCK2 | 5:169081434 | 123 | 24 | - | GC | G |
| LCP2 | 5:169677853 | 1567 | 454 | - | GT | G |
| FAM193B | 5:176958525 | 0 | 0 | - | TG | T |
| CANX | 5:179149920 | 1403 | 468 | - | AT | A |
| TBC1D9B | 5:179306627 | 0 | 0 | - | AC | A |
| CDH10 | 5:24488219-24488218 | 2428 | 640 | - | - | T |
| NIPBL | 5:37064899 | 8819 | 2774 | - | CA | C |
| KIAA0947 | 5:5464626 | 5401 | 1727 | - | TG | T |
| DEPDC1 B | 5:59893744-59893743 | 0 | 0 | - | - | A |
| COL4A3BP | 5:74807153 | 558 | 88 | - | TG | T |
| CHD1 | 5:98236745 | 779 | 210 | - | CT | C |
| GRIK2 | 6:102503432 | 3029 | 847 | - | CA | C |
| C6orf203 | 6:107361137 | 863 | 58 | - | CT | C |
| KIAA1919 | 6:111587361 | 949 | 199 | - | AT | A |
| LAMA4 | 6:112440366-112440365 | 5105 | 1605 | - | - | T |
| PHACTR1 | 6:13206135 | 504 | 168 | - | TG | T |
| IYD | 6:150690252 | 225 | 29 | - | GA | G |
| IGF2R | 6:160485488 | 4090 | 1314 | - | CG | C |
| ATXN1 | 6:16327163 | 2317 | 460 | - | AG | A |
| THBS2 | 6:169641977 | 1021 | 257 | - | TG | T |
| LRRC16A | 6:25600800 | 3746 | 1126 | - | TA | T |

(continued)

| Gene | Location | Pos. cDNA | Pos. protein | AA chq | Ref | Var |
|---|---|---|---|---|---|---|
| TEAD3 | 6:35446237 | 753 | 189 | - | TG | T |
| DLK2 | 6:43418413 | 1267 | 339 | - | AG | A |
| DSP | 6:7581583-7581585 | 5501 | 1720 | LE/L | TAGA | T |
| SENP6 | 6:76331349 | 0 | 0 | - | AT | A |
| CYB5R4 | 6:84634231 | 874 | 245 | - | CA | C |
| MANEA | 6:96053922 | 1164 | 344 | - | AT | A |
| SFRS18 | 6:99849343 | 1696 | 497 | - | CT | C |
| DNAJC2 | 7:102964992 | 841 | 197 | - | AT | A |
| RELN | 7:103301977 | 0 | 0 | - | TA | T |
| DOCK4 | 7:111368605 | 5724 | 1909 | - | AG | A |
| IFRD1 | 7:112112339 | 1577 | 369 | - | TA | T |
| WNT16 | 7:120971879 | 784 | 165 | - | TG | T |
| TRIM24 | 7:138264224-138264223 | 2746 | 844 | - | - | C |
| ETV1 | 7:13978876 | 0 | 0 | - | GA | G |
| DENND2A | 7:140218541 | 0 | 0 | - | TA | T |
| PRKAG2 | 7:151372597-151372596 | 1098 | 198 | - | - | G |
| BAGE3 | 7:151845524 | 13878 | 4553 | - | TA | T |
| NEUROD6 | 7:31378635 | 571 | 83 | - | CT | C |
| AEBP1 | 7:44146447 | 861 | 186 | - | AC | A |
| AUTS2 | 7:70236570 | 2091 | 590 | - | TC | T |
| CLIP2 | 7:73731913 | 364 | 13 | - | TG | T |
| STYXL1 | 7:75651314 | 0 | 0 | - | TA | T |
| PION | 7:76950143 | 0 | 0 | - | TA | T |
| MAGI2 | 7:77762294 | 3369 | 1039 | - | AG | A |
| LMTK2 | 7:97784092 | 766 | 158 | - | AC | A |
| CSMD3 | 8:113516210 | 0 | 0 | - | GA | G |
| EIF2C2 | 8:141561430 | 1415 | 459 | - | TG | T |
| MAPK15 | 8:144803436-144803437 | 1178 | 353 | - | CGA | C |
| BIN3 | 8:22487477 | 435 | 113 | - | CT | C |
| C8orf80 | 8:27888776 | 2035 | 631 | - | AT | A |
| MYBL1 | 8:67488453-67488452 | 1259 | 420 | - | - | T |
| NR4A3 | 9:102607096 | 1497 | 485 | - | CT | C |
| INVS | 9:103054983 | 2629 | 815 | - | CG | C |
| ZNF618 | 9:116770795 | 814 | 239 | - | GA | G |
| NR6A1 | 9:127287159-127287160 | 0 | 0 | - | GAA | G |
| BRD3 | 9:136918529 | 257 | 24 | - | CG | C |
| MTAP | 9:21815490 | 143 | 48 | - | GA | G |

(continued)

| Gene | Location | Pos. cDNA | Pos. protein | AA chq | Ref | Var |
|---|---|---|---|---|---|---|
| LINGO2 | 9:27949751 | 1373 | 307 | - | GC | G |
| IL33 | 9:6254556 | 0 | 0 | - | TA | T |
| ZCCHC6 | 9:88937823 | 3015 | 948 | - | TA | T |
| HNRNPH2 | X:100668112 | 1294 | 379 | - | CT | C |
| CLDN2 | X:106171948-106171952 | 816 | 164 | - | TCTTTA | T |
| APLN | X:128782615 | 529 | 37 | - | TG | T |
| BCORL1 | X:129190011 | 5372 | 1753 | - | TC | T |
| BCORL1 | X:129190011 | 5372 | 1753 | - | TC | T |
| BCORL1 | X:129190011 | 5372 | 1753 | - | TC | T |
| ARHGEF6 | X:135790933 | 0 | 0 | - | GA | G |
| ATP11C | X: 138840030 | 0 | 0 | - | GA | G |
| AFF2 | X:148037457 | 2361 | 628 | - | GA | G |
| PNMA3 | X:152225667 | 591 | 85 | - | AG | A |
| F8 | X: 154159223 | 3043 | 948 | - | AG | A |
| PHKA2 | X: 18942259-18942258 | 0 | 0 | - | - | A |
| DMD | X:32366648 | 0 | 0 | - | TA | T |
| PRRG1 | X:37312611-37312610 | 555 | 131 | - | - | C |
| RP2 | X:46713008 | 361 | 67 | - | TG | T |
| WNK3 | X:54328300-54328299 | 0 | 0 | - | - | A |
| VSIG4 | X:65242709 | 0 | 0 | - | GA | G |
| EFNB1 | X:68060323-68060322 | 1646 | 289 | - | - | G |
| IL2RG | X:70327614 | 1174 | 361 | - | TG | T |
| RGAG4 | X:71350840 | 912 | 184 | - | GC | G |
| ZDHHC15 | X:74649036 | 0 | 0 | - | TA | T |
| FAM9A | X:8759221 | 0 | 0 | - | CA | C |

The analysis of the base level transitions and transversions at mutated sites revealed that in CRCs C to T transitions to be predominant, regardless of the MMR status, both in the whole exome and whole genome analysis. This was consistent with previous mutation reports (Wood, L. D. et al., Science 318:1108-1113 (2007); Sjoblom, T. et al., Science 314:268-274 (2006); Bass, A. J. et al., Nat. Genet. 43:964-968 (2011)). The two hyper mutated tumors samples examined also showed higher proportion of C to A and T to G transversions, consistent with the much higher mutation rate observed for these samples.

Consistent with the exome mutation data, the MSS whole genome analyzed showed 17,651 mutations compared to the 97,968 mutations observed in the MSI whole genome. The average whole genome mutation rate was 6.2/Mb and 34.5/Mb for the MSS and MSI genome respectively. A mutation rate of 4.0-9.8/Mb was previously reported for MSS CRC genomes (Bass, A. J. et al., Nat. Genet. 43:964-968 (2011)).

***Example 2-Analysis of Mutated Genes***

The mutation analysis identified protein altering somatic single nucleotide variants in 12,956 genes including 3,257 in the MSS samples, 9,851 in the MSI samples and 6,891 in the two hyper mutated samples. Among the frequently mutated class of proteins are human kinases including RTKs, G-protein coupled receptors, and nuclear hormone receptors. In an effort to understand the impact of the mutations on gene function SIFT (Ng, P. C. & Henikoff, S., Genome Res 12:436-446 (2002)), Polyphen (Ramensky, V. et al., Nucleic Acids Res 30:3894-3900 (2002)) and mCluster (Yue, P. et al., Hum. Mutat. 31:264-271 (2010)) was applied and 36.7% of the mutations were found likely to have a functional consequence, in contrast to 12% for germline variants from the normal samples, based on at least two of the three methods (Table 2).

To further understand the relevance of the mutated genes, a previously described q-score metric was applied to rank significantly mutated cancer genes (Kan, Z. et al., Nature 466:869-873 (2010)). In MSS samples, 18 significant cancer genes (q-score >=1; ≤10% false discovery rate) were identified (*KRAS*, *TP53*, *APC*, *PIK3CA*, *SMAD4*, *FBXW7*, *CSMD1*, *NRXN1*, *DNAH5*, *MRVI1*, *TRPS1*, *DMD, KIF2B*, *ATM, FAM5C*, *EVC2, OR2W3, TMPRSS11A*, and *SCN10A*). The significantly mutated MSS colon cancer genes included previously reported genes including *KRAS, APC, TP53, SMAD4, FBXW7,* and *PIK3CA* and several new genes including the cell cycle checkpoint gene *ATM.* Genes like *KRAS* and *TP53* were among the top mutated MSI colon cancer genes, however, none of the genes achieved statistical significance due to the limited number of MSI samples analyzed.

In an effort to establish the relevance of the mutated genes, the mutated genes were compared against 399 candidate colon cancer genes identified in screens involving mouse models of cancer (Starr, T. K. et al., Science 323, 1747-1750 (2009); March, H. N. et al., Nat. Genet. 43, 1202-1209 (2011)). Of the 399 genes mutations were found in 327. When the data sets were analyzed via an alternative method, of the 432 genes, mutations were found in 356. The frequently mutated genes in the data set that overlapped with mouse colon cancer model hits included *KRAS*, *APC*, *SMAD4*, *FBXW7* and *EP400.* Additionally, genes involved in chromatin remodeling like *SIN3A*, *SMARCA5* and *NCOR1* and histone modifying enzyme *JARID2* found in the mouse CRC screen (Starr, T. K. et al., Science 323, 1747-1750 (2009); March, H. N. et al., Nat. Genet. 43, 1202-1209 (2011)) were also mutated in our exome screen. Further, *TCF12*, identified in the mouse colon cancer model screen, was mutated in 5 (Q179*, G444*, and R603W/Q) of our samples (7%) and contained a hotspot mutation at R603 (3 of 5 mutations; R603W/Q). This hotspot mutation within the TCF12 helix-loop-helix domain will likely abolish its ability to bind DNA, suggesting a loss-function mutation. Interestingly, all of the TCF12 mutations were identified in MSI samples. The TCF12 transcription factor has been previously implicated in colon cancer metastasis (Lee, C. C. et al., J. of Biol. Chem. 287:2798-2809 (2011)). The presence of hotspots in this gene and its identification in mouse CRC model screen indicates that it likely functions as a CRC driver gene.

Mutational hotspots, where the same position in a gene was mutated across independent samples, are indicative of functionally relevant driver cancer gene. In this study, 270 genes were identified with hotspot mutation (Table 4). Seventy of these genes were not previously reported in COSMIC. Comparison of our mutations with those reported in COSMIC identified an additional 245 hotspot mutations in 166 genes (Table 5). Utilizing an alternative data analysis method, 274 genes were identified with hotspot mutations with forty of these genes not previously in COSMI and an additional 435 hotspot mutations in 361 genes. Genes with novel hotspot mutations include transcriptional regulators (*TCF12*, *TCF7L2* and *PHF2*), Ras/Rho related regulators (*SOS1* (*e.g.*, R547W, T614M R854*, G1129V), *SOS2* (*e.g.*, R225*, R854C,and Q1296H), *RASGRF2, ARHGAP10*, *ARHGEF33* and *Rab40c* (*e.g.*, G251S)), chromatin modifying enzymes (*TET2*, *TET3*, *EP400* and *MLL*), glutamate receptors (*GRIN3A* and *GRM8*), receptor tyrosine kinases (*ERBB3*, *EPHB4, EFNB3, EPHA1*, *TYRO3*, *TIE1* and *FLT4*), other kinases (*RIOK3*, *PRKCB*, *MUSK*, *MAP2K7* and *MAP4K5*), protein phosphatase (*PTPRN2*), GPRCs (*GPR4* and *GPR98*) and E3-ligase (*TOPORS*). Of further interest in this gene set are *TET2* and *TET3*, both of which encode methylcytosine dioxygenase involved in DNA methylation (Mohr, F. et al., Exp. Hematol. 39:272-281 (2011)). While mutations in *TET2* have been reported in myeloid cancers, thus far mutations in *TET3* or *TET1* have not been reported in solid tumors, especially, in CRC (Mohr, F. et al., Exp. Hematol. 39:272-281 (2011)). All the three family members *TET1* (*e.g.*, R81H, E417A, K540T, K792T, S879L, S1012*, Q1322*, C1482Y, A1896V, and A2129V), *TET2* (*e.g.*, K108T, T118I, S289L, F373L, K1056N, Y1169*, A1497V, and V1857M), and *TET3* (*e.g.*, T165M, A874T, M977V, G1398R, and R1576Q/W) are mutated in these examples.

***Table 4 – Hotspot mutations***

| Gene | Pos. Prot. | Mutation | Locations | Gene | Pos. Prot. | Mutation | Locations |
|---|---|---|---|---|---|---|---|
| SEPT14 | 157 | R157H | 7:55910723, 7:55910723 | MAP7D2 | 546 | E546* | X:20031734, X:20031734 |
| ACMSD | 162 | A162V | 2:135621200, 2:135621200 | MDN1 | 3240 | R3240C | 6:90405377, 6:90405377 |
| ACRV1 | 257 | R257Q | 11:125542516, 11:125542516 | MGST3 | 13 | R13H | 1:165619080, 1:165619080 |

| | | | | | | | |
|---|---|---|---|---|---|---|---|
| ADAMTS12 | 604 | R604W | 5:33637760, 5:33637760 | MID1 | 178 | H178Q | X:10535054, X:10535054 |
| ADAMTS14 | 297 | D297N | 10:72489068, 10:72489068 | MLL | 933 | R933W | 11:118344671, 11:118344672 |
| ALDH16A1 | 581 | A581V | 19:49969344, 19:49969344 | MPP3 | 257 | R257H | 17:41898416, 17:41898416 |
| ALK | 551 | R551Q | 2:29519919, 2:29519920 | MRPL18 | 108 | R108H | 6:160218402, 6:160218402 |
| ANGPTL4 | 136 | R136Q | 19:8430926, 19:8430926 | MRVI1 | 517 | P517H | 11:10628314, 11:10628314 |
| ANKRD28 | 401 | R401H | 3:15753727, 3:15753728 | MUSK | 842 | R842H | 9:113563165, 9:113563165 |
| ANKRD28 | 208 | R208C | 3:15776944, 3:15776944 | MYH2 | 445 | R445H | 17:10442604, 17:10442605 |
| APC | 1450 | R1450* | 5:112175639, 5:112175639 | NBEA | 203 | R203* | 13:35619164, 13:35619164 |
| APC | 232 | R232* | 5:112128191, 5:112128191 | NKAIN4 | 110 | R110C | 20:61879073, 20:61879073 |
| APC | 564 | R564* | 5:112164616, 5:112164616 | NLRP12 | 656 | R656C | 19:54312947, 19:54312946 |
| APC | 876 | R876* | 5:112173917, 5:112173917, 5:112173918, 5:112173917 | NLRP2 | 467 | R467Q | 19:55494466, 19:55494465 |
| APC | 1378 | Q1378* | 5:112175423, 5:112175423 | NMUR2 | 108 | R108H | 5:151784352, 5:151784352 |
| APC | 653 | R653M | 5:112170862, 5:112170862 | NUDCD1 | 521 | R521H | 8:110255428, 8:110255428 |
| APOB | 3036 | S3036Y | 2:21230633, 2:21230633 | NUP93 | 77 | R77* | 16:56792499, 16:56792499 |
| APOB | 1513 | R1513Q | 2:21235202, 2:21235202 | OIT3 | 506 | R506H | 10:74692161, 10:74692161 |
| ARHGAP10 | 348 | V348I | 4:148827796, 4:148827796 | OLFML2B | 679 | V679I | 1:161953686, 1:161953686 |
| ARHGEF33 | 48 | Q48K | 2:39156114, 2:39156114 | OR10A7 | 261 | R261Q | 12:55615590, 12:55615589 |
| ASB10 | 242 | A242V | 7:150878540, 7:150878540 | OR2D2 | 122 | R122H | 11:6913367, 11:6913368 |
| ASPG | 270 | R270C | 14:104569983, 14:104569983 | OR4N4 | 290 | R290H | 15:22383341, 15:22383341, 15:22383341 |
| ATF7IP | 159 | P159A | 12:14577324, 12:14577324 | OR5D18 | 237 | R237C | 11:55587808, 11:55587808 |
| BCL6 | 594 | R594Q | 3:187443345, 3:187443345 | OR6P1 | 201 | L201R | 1:158532793, 1:158532793 |
| BDKRB2 | 128 | T128M | 14:96707048, 14:96707048 | PCBP1 | 100 | L100Q | 2:70315174, 2:70315174 |
| BEST3 | 388 | R388Q | 12:70049531, 12:70049532 | PCDHA13 | 301 | E301* | 5:140262754, 5:140262756 |
| BNC2 | 575 | S575R | 9:16436469, 9:16436469 | PCDHA4 | 266 | A266T | 5:140187568, 5:140187568 |
| BRAF | 600 | V600E | 7:140453136, 7:140453136, 7:140453136, 7:140453136 | PCDHA7 | 681 | R681W | 5:140216009, 5:140216010 |
| BRIP1 | 745 | A745T | 17:59821817, 17:59821817 | PCMTD1 | 200 | R200Q | 8:52744111, 8:52744111 |
| BTBD7 | 667 | T667M | 14:93714943, 14:93714943 | PDE8B | 436 | R436H | 5:76703224, 5:76703223 |
| C10orf90 | 84 | A84T | 10:128193519, 10:128193519 | PDZRN3 | 971 | R971H | 3:73432805, 3:73432806 |
| C12orf35 | 235 | N235K | 12:32134594, 12:32134592 | PER2 | 1049 | A1049T | 2:239160369, 2:239160369 |
| C12orf4 | 335 | R335Q | 12:4627253, 12:4627253 | PHF2 | 700 | P700L | 9:96428129, 9:96428129 |
| C13orf1 | 58 | A58T | 13:50505205, 13:50505205 | PHLDB2 | 438 | R438M | 3:111604237, 3:111604236 |
| C20orf132 | 57 | Q57E | 20:35807795, 20:35807795 | PIK3CA | 545 | E545A | 3:178936092, 3:178936091, 3:178936091, 3:178936092, 3:178936091, 3:178936091 |
| C2orf86 | 227 | R227Q | 2:63661024, 2:63661024 | PIK3CA | 1025 | T1025A | 3:178952018, 3:178952018 |
| C5orf49 | 66 | Y66H | 5:7835563, 5:7835563 | PIK3CA | 1047 | H1047R | 3:178952085, 3:178952085, 3:178952085, 3:178952085, 3:178952085 |
| C6orf118 | 212 | A212T | 6:165715177, 6:165715176 | PIK3CA | 111 | K111E | 3:178916944, 3:178916946, 3:178916944 |
| C6orf174 | 368 | G368C | 6:127768362, 6:127768362 | PKD2L2 | 448 | R448Q | 5:137257339, 5:137257339 |
| C7orf63 | 125 | K125N | 7:89894633, 7:89894633 | PLEKHA4 | 204 | R204H | 19:49362807, 19:49362808 |
| C8A | 484 | R484C | 1:57378145, 1:57378146 | PLEKHH3 | 155 | G155S | 17:40825688, 17:40825688 |
| C9orf167 | 145 | A145V | 9:140173575, 9:140173575 | PNKD | 222 | R222Q | 2:219206751, 2:219206751 |
| CACNA1A | 110 | A110V | 19:13565991, 19:13565991 | POLE | 286 | P286R | 12:133253184, 12:133253184 |
| CACNA1D | 1278 | A1278T | 3:53787695, 3:53787695 | PRDM2 | 282 | E282D | 1:14105136, 1:14105136 |
| CACNA1E | 398 | E398* | 1:181684494, 1:181684494 | PRKCB | 161 | R161C | 16:24046820, 16:24046821 |
| CACNA1I | 601 | R601Q | 22:40045722, 22:40045721 | PRKCH | 465 | S465L | 14:61952335, 14:61952335 |
| CBX6 | 199 | R199C | 22:39262858, 22:39262857 | PROM1 | 472 | R472Q | 4:16008200, 4:16008200 |
| CCDC117 | 277 | M277I | 22:29182305, 22:29182305 | PSKH2 | 32 | A32T | 8:87081758, 8:87081758 |
| CCDC157 | 469 | R469Q | 22:30769656, 22:30769655 | PTGS2 | 600 | R600H | 1:186643501, 1:186643501 |

| | | | | | | | |
|---|---|---|---|---|---|---|---|
| CCDC6 | 139 | E139* | 10:61612349, 10:61612349 | PTHLH | 94 | R94Q | 12:28116524, 12:28116524 |
| CCRL1 | 26 | Q26* | 3:132319317, 3:132319317 | PTPRN2 | 545 | R545H | 7:157903599, 7:157903599 |
| CDH8 | 291 | L291H | 16:61854981, 16:61854981 | PXDN | 1198 | R1198W | 2:1651960, 2:1651960 |
| CLEC2L | 145 | E145A | 7:139226768, 7:139226767 | RAB40C | 251 | G251S | 16:677527, 16:677527, 16:677527 |
| CLEC3A | 156 | R156C | 16:78064610, 16:78064610 | RASGRF2 | 244 | R244I | 5:80369115, 5:80369115 |
| COL14A1 | 1048 | F1048S | 8:121282343, 8:121282343 | RBM22 | 216 | R216W | 5:150075168, 5:150075168 |
| CRISP2 | 88 | R88C | 6:49667526, 6:49667525 | RBMXL2 | 287 | G287R | 11:7111210, 11:7111210 |
| CSNK1G2 | 263 | R263W | 19:1979336, 19:1979336 | RBP3 | 967 | G967S | 10:48387979, 10:48387979 |
| CYP11A1 | 86 | G86D | 15:74659670, 15:74659670 | RECQL5 | 872 | R872H | 17:73624488, 17:73624488 |
| CYP2E1 | 328 | E328* | 10:135350581, 10:135350581 | RETSAT | 125 | R125C | 2:85578127, 2:85578126 |
| DAB2IP | 333 | R333H | 9:124522546, 9:124522545 | RIOK3 | 306 | I306S | 18:21053494, 18:21053494 |
| DDX21 | 440 | R440C | 10:70730038, 10:70730039 | RNF43 | 132 | R132* | 17:56440943, 17:56440943 |
| DENND2A | 572 | S572Y | 7:140266950, 7:140266950 | SAMSN1 | 235 | R235C | 21:15882693, 21:15882692 |
| DICER1 | 1813 | E1813Q | 14:95557630, 14:95557630 | SCRN2 | 250 | R250W | 17:45916181, 17:45916181 |
| DLGAP2 | 912 | R912Q | 8:1645425, 8:1645424 | SEMA3F | 477 | R477C | 3:50222220, 3:50222220 |
| DNAH11 | 1281 | A1281V | 7:21646341, 7:21646341 | SEMA7A | 261 | R261H | 15:74708935, 15:74708935 |
| DNAJC10 | 180 | R180Q | 2:183593627, 2:183593626 | SETD2 | 1322 | R1322Q | 3:47162161, 3:47162161 |
| DPYD | 561 | R561Q | 1:97981340, 1:97981340 | SFMBT2 | 617 | R617W | 10:7218087, 10:7218086 |
| DSEL | 56 | K56R | 18:65181709, 18:65181709 | SLC13A3 | 169 | R169Q | 20:45239120, 20:45239121 |
| DSP | 2586 | R2586* | 6:7585251, 6:7585252 | SLC13A4 | 111 | R111H | 7:135392895, 7:135392896 |
| DVL1L1 | 227 | R227C | 1:1275810, 1:1275809 | SLC15A1 | 677 | E677D | 13:99337074, 13:99337074 |
| EFNB3 | 106 | R106H | 17:7611470, 17:7611469 | SLC1A6 | 365 | V365F | 19:15067364, 19:15067364 |
| EGFR | 671 | R671C | 7:55240767, 7:55240767 | SLC28A3 | 154 | R154* | 9:86917179, 9:86917179 |
| EMR1 | 887 | A887T | 19:6937648, 19:6937648 | SLC35B2 | 333 | R333* | 6:44222745, 6:44222745 |
| ENOX1 | 298 | R298H | 13:43918817, 13:43918818 | SLC45A3 | 81 | R81H | 1:205632677, 1:205632678 |
| EP400 | 1786 | R1786C | 12:132512700, 12:132512700 | SLC6A1 | 342 | V342M | 3:11067991, 3:11067991 |
| EP400 | 2523 | A2523T | 12:132537755, 12:132537755 | SMAD4 | 361 | R361H | 18:48591919, 18:48591918, 18:48591918, 18:48591918, 18:48591919, 18:48591918 |
| EPHA1 | 844 | R844W | 7:143090930, 7:143090929 | SMARCAL1 | 541 | T541N | 2:217300197, 2:217300196, 2:217300197 |
| EPHB4 | 866 | R866H | 7:100403204, 7:100403204 | SMC4 | 1056 | S1056L | 3:160149483, 3:160149483 |
| EPHB4 | 535 | R535W | 7:100411629, 7:100411629 | SNW1 | 198 | R198L | 14:78203359, 14:78203359 |
| EPS8 | 571 | R571Q | 12:15793746, 12:15793747 | SOS2 | 824 | R824C | 14:50612229, 14:50612229 |
| ERC2 | 619 | R619Q | 3:56044541, 3:56044541 | SPTA1 | 268 | R268* | 1:158648201, 1:158648201 |
| EXOC6B | 785 | R785Q | 2:72406546, 2:72406547 | SULT4A1 | 32 | R32C | 22:44258169, 22:44258169 |
| F8 | 2166 | R2166* | X:154091436, X:154091436 | SUV39H1 | 230 | I230M | X:48558973, X:48558973 |
| FAM110B | 160 | A160V | 8:59059268, 8:59059267 | TCF12 | 603 | R603W | 15:57565289, 15:57565290, 15:57565290 |
| FAM43B | 273 | D273E | 1:20880285, 1:20880285 | TCF7L2 | 465 | R465C | 10:114925333, 10:114925334 |
| FAM90A1 | 71 | P71L | 12:8376723, 12:8376724 | TECR | 66 | R66H | 19:14674503, 19:14674503 |
| FAT4 | 132 | A132T | 4:126237960, 4:126237960 | TEKT2 | 268 | R268W | 1:36552859, 1:36552860 |
| FBXL17 | 216 | R216* | 5:107216863, 5:107216863 | TET3 | 1576 | R1576Q | 2:74328921, 2:74328920 |
| FBXW7 | 465 | R465C | 4:153249385, 4:153249385, 4:153249384, 4:153249384 | TFIP11 | 386 | R386Q | 22:26895242, 22:26895242 |
| FBXW7 | 582 | S582L | 4:153245446, 4:153245446 | TIE1 | 583 | R583C | 1:43778092, 1:43778093 |
| FBXW7 | 505 | R505C | 4:153247289, 4:153247289 | TMC7 | 375 | A375P | 16:19049313, 16:19049313 |
| FBXW7 | 369 | E369* | 4:153251901, 4:153251901 | TMEM175 | 335 | R335C | 4:951772, 4:951772 |
| FCAR | 110 | R110W | 19:55396904, 19:55396904 | TMEM201 | 474 | R474H | 1:9669925, 1:9669924 |
| FHOD3 | 1353 | R1353C | 18:34340727, 18:34340727 | TMEM71 | 83 | D83N | 8:133764098, 8:133764098 |
| FKBP1C | 19 | R19C | 6:63921516, 6:63921516 | TNRC18 | 811 | A811T | 7:5417032, 7:5417032 |
| FLT4 | 1031 | R1031* | 5:180043905, 5:180043905 | TOPORS | 188 | R188Q | 9:32543960, 9:32543961 |

| | | | | | | | |
|---|---|---|---|---|---|---|---|
| FRMD4A | 851 | R851C | 10:13699038, 10:13699037 | TP53 | 176 | C176Y | 17:7578403, 17:7578403 |
| FRY | 2194 | T2194M | 13:32813912, 13:32813912 | TP53 | 175 | R175H | 17:7578406, 17:7578406, 17:7578406, 17:7578406 |
| FSTL5 | 404 | R404C | 4:162459420, 4:162459420 | TP53 | 213 | R213* | 17:7578212, 17:7578212 |
| FSTL5 | 252 | D252Y | 4:162577620, 4:162577620 | TP53 | 248 | R248L | 17:7577538, 17:7577539, 17:7577538, 17:7577539 |
| FUBP1 | 451 | R451C | 1:78428511, 1:78428511 | TP53 | 273 | R273H | 17:7577120, 17:7577121, 17:7577120, 17:7577120, 17:7577121 |
| GAL3ST2 | 326 | G326S | 2:242743360, 2:242743360 | TP53 | 282 | R282W | 17:7577094, 17:7577094 |
| GALNTL2 | 395 | E395K | 3:16252734, 3:16252734 | TP53 | 196 | R196* | 17:7578263, 17:7578263 |
| GBF1 | 1243 | A1243V | 10:104135186, 10:104135186 | TP53 | 257 | L257Q | 17:7577511, 17:7577511 |
| GCG | 65 | Y65* | 2:163003931, 2:163003931 | TP53 | 245 | G245S | 17:7577548, 17:7577547 |
| GCM2 | 265 | R265I | 6:10874955, 6:10874955 | TP53BP1 | 1405 | R1405* | 15:43713260, 15:43713260 |
| GDF3 | 84 | R84C | 12:7848075, 12:7848075 | TRBC2 | 68 | A68T | 7:142498925, 7:142498925 |
| GNAS | 844 | R844C | 20:57484420, 20:57484421, 20:57484420 | TRIM22 | 262 | W262C | 11:5729415, 11:5729414 |
| GPR4 | 14 | R14H | 19:46095084, 19:46095085 | TRIM23 | 525 | E525* | 5:64887748, 5:64887748 |
| GPR98 | 2200 | S2200Y | 5:89985786, 5:89985786 | TRIM66 | 895 | R895Q | 11:8643322, 11:8643322 |
| GRHL1 | 434 | R434* | 2:10130854, 2:10130855 | TSHZ2 | 222 | A222V | 20:51870662, 20:51870662 |
| GRIN3A | 225 | R225C | 9:104499589, 9:104499589 | TSPAN17 | 266 | A266T | 5:176083806, 5:176083806 |
| GRLF1 | 1187 | R1187Q | 19:47425492, 19:47425492 | TYRO3 | 0 | - | 15:41870082 |
| GRM8 | 30 | R30I | 7:126883170, 7:126883170 | UBQLN3 | 624 | R624W | 11:5528919, 11:5528919 |
| GSR | 233 | R233C | 8:30553995, 8:30553994 | UNC13A | 285 | R285H | 19:17769048, 19:17769049 |
| GYLTL1B | 267 | R267W | 11:45947619, 11:45947619 | UROC1 | 656 | G656S | 3:126207045, 3:126207044 |
| HAO1 | 84 | R84H | 20:7915169, 20:7915169 | USP6NL | 492 | A492T | 10:11505504, 10:11505504 |
| HCFC2 | 191 | E191* | 12:104473320, 12:104473320 | WDFY4 | 1091 | R1091C | 10:49986751, 10:49986751 |
| HERC2 | 4634 | A4634V | 15:28359770, 15:28359770 | WDR16 | 549 | E549* | 17:9545080, 17:9545080 |
| HGF | 234 | R234C | 7:81374362, 7:81374361 | WHSC1 | 104 | E104K | 4:1902691, 4:1902691 |
| HHIPL2 | 303 | K303N | 1:222716944, 1:222716944 | WIPF1 | 458 | P458S | 2:175431882, 2:175431881 |
| HIST1H1T | 167 | G167W | 6:26107823, 6:26107823 | WSCD2 | 583 | Y583* | 12:108642111, 12:108642111 |
| HIVEP2 | 1028 | R1028* | 6:143092794, 6:143092794, 6:143092794 | XCR1 | 166 | I166V | 3:46062944, 3:46062944 |
| HMCN1 | 1647 | T1647M | 1:185985120, 1:185985120 | ZBTB32 | 170 | P170S | 19:36206036, 19:36206036 |
| HRASLS5 | 118 | K118T | 11:63256365, 11:63256365 | ZBTB40 | 1174 | A1174V | 1:22850933, 1:22850933 |
| HSD17B3 | 184 | S184Y | 9:99007682, 9:99007682 | ZHX3 | 249 | N249K | 20:39832810, 20:39832810 |
| HTR1A | 50 | A50T | 5:63257399, 5:63257398 | ZNF14 | 547 | R547* | 19:19822451, 19:19822451 |
| HYI | 118 | R118Q | 1:43917949, 1:43917949 | ZNF142 | 834 | R834* | 2:219508739, 2:219508738 |
| IGDCC3 | 132 | R132C | 15:65667450, 15:65667450 | ZNF19 | 45 | E45D | 16:71512807, 16:71512807 |
| IGLL5 | 176 | A176V | 22:23237753, 22:23237753 | ZNF211 | 486 | R486I | 19:58153272, 19:58153272 |
| IKZF4 | 255 | R255Q | 12:56426393, 12:56426392 | ZNF235 | 254 | R254C | 19:44792828, 19:44792827 |
| ITGAD | 669 | V669I | 16:31424528, 16:31424528 | ZNF236 | 154 | A154D | 18:74580744, 18:74580744, 18:74580743 |
| KBTBD3 | 356 | R356Q | 11:105924349, 11:105924350 | ZNF442 | 309 | R309* | 19:12461474, 19:12461473 |
| KBTBD6 | 670 | R670H | 13:41704639, 13:41704640 | ZNF470 | 445 | R445I | 19:57089131, 19:57089131 |
| KCNA3 | 105 | R105H | 1:111217118, 1:111217118 | ZNF480 | 97 | N97H | 19:52819176, 19:52819176 |
| KCND2 | 247 | R247H | 7:119915426, 7:119915425 | ZNF507 | 56 | E56* | 19:32843902, 19:32843902 |
| KIAA0895 | 282 | A282T | 7:36396534, 7:36396534 | ZNF577 | 402 | E402* | 19:52376039, 19:52376039 |
| KIAA1024 | 73 | V73A | 15:79748707, 15:79748707 | ZNF662 | 172 | R172H | 3:42956002, 3:42956001 |
| KIF21A | 911 | G911C | 12:39726518, 12:39726518 | ZNF668 | 206 | A206V | 16:31075164, 16:31075164 |
| KIF27 | 623 | R623Q | 9:86504110, 9:86504110 | ZNF789 | 219 | H219Q | 7:99084490, 7:99084490 |
| KIF7 | 841 | R841W | 15:90176988, 15:90176988 | ZNF831 | 1412 | S1412I | 20:57828999, 20:57828999 |

| | | | | | | | |
|---|---|---|---|---|---|---|---|
| LAMB3 | 367 | R367H | 1:209803114, 1:209803115 | ZNRF3 | 102 | R102* | 22:29439389, 22:29439389 |
| LASS3 | 95 | E95D | 15:101031058, 15:101031058 | LSM14A | 272 | R272C | 19:34710328, 19:34710328 |
| LCT | 694 | A694S | 2:136570154, 2:136570154 | MAP2 | 905 | R905* | 2:210559607, 2:210559607 |
| LDLRAD2 | 148 | L148M | 1:22141247, 1:22141247 | MAP2K7 | 195 | R195L | 19:7975348, 19:7975348 |
| LRP2 | 3726 | R3726C | 2:170028612, 2:170028611 | MAP4K5 | 172 | R172* | 14:50941823, 14:50941823 |
| LRP2 | 2095 | R2095* | 2:170066149, 2:170066149 | LRRC8D | 588 | R588W | 1:90400389, 1:90400390 |
| KRAS | 12 | G12V | 12:25398284, 12:25398284, 12:25398284, 12:25398284, 12:25398285, 12:25398284, 12:25398284, 12:25398285, 12:25398284, 12:25398284, 12:25398285, 12:25398284, 12:25398284, 12:25398284, 12:25398284, 12:25398284, 12:25398284, 12:25398284, 12:25398284, 12:25398284, 12:25398284, 12:25398284, 12:25398285, 12:25398284, 12:25398284, 12:25398284, 12:25398284, 12:25398284, 12:25398284 | KRAS | 13 | G13D | 12:25398281, 12:25398281, 12:25398281, 12:25398281, 12:25398281 |

*Table 5 – Hotspot mutations identified through metanalysis using COSMIC mutation data*

| Gene | Prot. | Mut. | Locations | Gene | Prot. | Mut. | Locations |
|---|---|---|---|---|---|---|---|
| SEPT9 | 346 | T346M | 17:75483629 | MYT1 | 503 | T503M | 20:62843482 |
| ABP1 | 660 | N660S | 7:150557654 | NBEA | 2219 | R2219H | 13:36124684 |
| ACSL4 | 133 | R133H | X:108926079 | NCAN | 871 | T871M | 19:19339041 |
| ADAMTS14 | 682 | V682I | 10:72503414 | NEB | 3538 | R3538W | 2:152471050 |
| AGRN | 0 | - | 1:985612 | NEURL4 | 366 | R366H | 17:7229863 |
| ALDH18A1 | 64 | R64H | 10:97402861 | NF1 | 416 | R416* | 17:29528489 |
| ALDH8A1 | 69 | R69C | 6:135265038 | NF1 | 1858 | A1858T | 17:29654820 |
| ALK | 401 | R401* | 2:29606679 | NF2 | 459 | Q459H | 22:30070861 |
| ALOX15 | 500 | R500* | 17:4536198 | NGEF | 259 | R259W | 2:233785047 |
| ANO2 | 704 | R704* | 12:5708776 | NHS | 373 | R373* | X:17742490 |
| ANO2 | 657 | D657N | 12:5722087 | NLRP4 | 442 | G442R | 19:56370083 |
| ANTXR1 | 192 | A192V | 2:69304553 | NOS3 | 474 | R474C | 7:150698505 |
| APC | 1705 | T1705A | 5:112176404 | NPSR1 | 85 | F85L | 7:34724271 |
| APC | 1400 | S1400L | 5:112175490 | NRAS | 61 | Q61L | 1:115256529 |
| APC | 1355 | S1355Y | 5:112175355 | NRAS | 12 | G12A | 1:115258747 |
| APC | 117 | S117* | 5:112103015 | NTN3 | 440 | D440N | 16:2523319 |
| APC | 499 | R499* | 5:112162891 | NUP98 | 493 | Y493H | 11:3756486 |
| APC | 302 | R302* | 5:112151261 | OR5T1 | 322 | F322L | 11:56044078 |
| APC | 283 | R283* | 5:112151204 | OR6Y1 | 214 | I214S | 1:158517255 |
| APC | 1386 | R1386* | 5:112175447 | OXGR1 | 252 | V252I | 13:97639260 |
| APC | 1114 | R1114* | 5:112174631 | PALB2 | 1008 | P1008T | 16:23632774 |
| APC | 1367 | Q1367* | 5:112175390 | PBRM1 | 0 | - | 3:52678719 |
| APC | 1338 | Q1338* | 5:112175303 | PGR | 740 | R740Q | 11:100922293 |
| APC | 1009 | H1009R | 5:112174317 | PIK3CA | 1052 | T1052K | 3:178952100 |
| APC | 1312 | G1312* | 5:112175225 | PIK3CA | 88 | R88Q | 3:178916876 |
| APC | 1408 | E1408* | 5:112175513 | PIK3CA | 546 | Q546K | 3:178936094 |
| APC | 1379 | E1379* | 5:112175426 | PIK3CA | 986 | K986N | 3:178951903 |
| APC | 1306 | E1306* | 5:112175207 | PIK3CA | 594 | K594E | 3:178937392 |
| ARHGAP20 | 987 | D987Y | 11:110450711 | PIK3CA | 542 | E542K | 3:178936082 |

| | | | | | | | |
|---|---|---|---|---|---|---|---|
| ARID1A | 1276 | R1276Q | 1:27099948 | PIK3CA | 420 | C420R | 3:178927980 |
| ASPM | 1610 | V1610D | 1:197073552 | PIK3R1 | 574 | R574I | 5:67591128 |
| ATM | 352 | I352N | 11:108117844 | PIK3R1 | 543 | R543I | 5:67591035 |
| ATP10A | 793 | R793W | 15:25953415 | PIK3R1 | 348 | R348* | 5:67588951 |
| ATP10A | 1211 | A1211T | 15:25926004 | PIK3R1 | 162 | R162* | 5:67569823 |
| ATP6V1E2 | 135 | R135C | 2:46739448 | PIK3R1 | 564 | N564D | 5:67591097 |
| AZGP1 | 46 | A46T | 7:99569570 | PIK3R1 | 527 | N527K | 5:67590988 |
| B3GAT1 | 11 | V11I | 11:134257523 | PIK3R1 | 285 | N285H | 5:67576771 |
| BAP1 | 128 | G128* | 3:52441470 | PKHD1 | 1081 | R1081H | 6:51897950 |
| BCL11B | 358 | S358A | 14:99642101 | PNLIPRP1 | 129 | S129F | 10:118354297 |
| BTBD3 | 218 | L218H | 20:11900472 | PPP1R3A | 948 | T948M | 7:113518304 |
| CARD11 | 353 | T353A | 7:2977627 | PPP1R3A | 554 | G554V | 7:113519486 |
| CC2D1B | 534 | R534Q | 1:52823367 | PPP5C | 242 | D242E | 19:46887063 |
| CD40LG | 11 | R11Q | X:135730439 | PRPS1L1 | 58 | S58G | 7:18067234 |
| CDC73 | 54 | Y54H | 1:193094270 | PTCH1 | 563 | A563T | 9:98238357 |
| CDK5RAP1 | 169 | R169Q | 20:31979986 | PTEN | 233 | R233* | 10:89717672 |
| CDKN2A | 80 | R80* | 9:21971120 | PTEN | 130 | R130Q | 10:89692905 |
| CDKN2A | 124 | R124H | 9:21970987 | PTEN | 125 | K125T | 10:89692890 |
| CDKN2A | 107 | R107H | 9:21971038 | PTEN | 28 | I28M | 10:89653786 |
| CDKN2A | 76 | A76T | 9:21971132 | PTEN | 93 | H93Y | 10:89692793 |
| CDKN2B | 60 | R60H | 9:22006224 | PTEN | 3 | A3D | 10:89624234 |
| COL11A1 | 1770 | A1770V | 1:103345240 | PTPN11 | 76 | E76G | 12:112888211 |
| COL3A1 | 420 | G420S | 2:189859023 | PTPRC | 582 | F582Y | 1:198697493 |
| CORO2B | 113 | R113Q | 15:69003075 | RAD50 | 1109 | I1109T | 5:131953923 |
| CREB3L1 | 235 | A235V | 11:46332691 | RAP1GAP | 609 | V609M | 1:21926031 |
| CTNNB1 | 41 | T41A | 3:41266124 | RASGEF1C | 293 | G293S | 5:179546376 |
| CTNNB1 | 45 | S45P | 3:41266136 | RBM14 | 505 | G505R | 11:66392860 |
| CYTH1 | 386 | A386T | 17:76672214 | RNF175 | 221 | S221R | 4:154636784 |
| DAPK3 | 454 | R454C | 19:3959104 | RPN1 | 263 | R263C | 3:128350847 |
| DAXX | 306 | R306Q | 6:33288635 | RPS6KA5 | 263 | S263Y | 14:91386568 |
| DGKB | 466 | R466H | 7:14647098 | SAMD7 | 67 | R67W | 3:169639114 |
| DLEC1 | 844 | S844L | 3:38139094 | SEC23IP | 770 | G770R | 10:121685734 |
| DMTF1 | 315 | T315A | 7:86813835 | SETD4 | 90 | R90Q | 21:37420633 |
| DNAH3 | 3772 | Y3772C | 16:20959833 | SF3B1 | 568 | R568C | 2:198268326 |
| DNAH5 | 4200 | K4200R | 5:13717530 | SIK1 | 68 | L68V | 21:44845358 |
| DOCK1 | 1665 | A1665T | 10:129224219 | SLC24A3 | 82 | R82W | 20:19261704 |
| DPF3 | 79 | R79H | 14:73220034 | SLC27A3 | 462 | G462S | 1:153749660 |
| DSCAML1 | 1762 | V1762I | 11:117303143 | SLC2A5 | 238 | R238C | 1:9100032 |
| ECE2 | 438 | R438C | 3:184001714 | SLC45A3 | 272 | R272C | 1:205632105 |
| EPHB6 | 106 | R106* | 7:142561874 | SMAD4 | 509 | W509* | 18:48604705 |
| ERBB2 | 755 | L755M | 17:37880219 | SMAD4 | 356 | P356S | 18:48591903 |
| ERBB3 | 104 | V104M | 12:56478854 | SMAD4 | 386 | G386V | 18:48593406 |
| ERBB3 | 284 | G284R | 12:56481922 | SMAD4 | 493 | D493A | 18:48604656 |
| FAM184A | 723 | T723M | 6:119301436 | SMAD4 | 351 | D351G | 18:48591889 |
| FAM71B | 318 | I318N | 5:156590323 | SMARCA4 | 966 | R966W | 19:11134230 |
| FBLN7 | 407 | T407M | 2:112944983 | SMARCB1 | 383 | R383W | 22:24176329 |
| FBXL7 | 160 | T160M | 5:15928350 | SMO | 324 | A324T | 7:128846040 |
| FBXW7 | 367 | R367* | 4:153251907 | SNTB1 | 401 | R401Q | 8:121561133 |
| FBXW7 | 224 | R224Q | 4:153268137 | SOX6 | 93 | R93* | 11:16340160 |
| FBXW7 | 470 | H470R | 4:153249369 | SPCS2 | 4 | A4S | 11:74660340 |
| FER1L6 | 810 | G810D | 8:125047660 | SPEN | 907 | T907I | 1:16255455 |
| FREM2 | 484 | V484A | 13:39262932 | STK11 | 314 | P314H | 19:1223004 |
| FTSJ2 | 53 | R53W | 7:2279194 | SYNE1 | 3671 | V3671M | 6:152674795 |

| | | | | | | | |
|---|---|---|---|---|---|---|---|
| FZD7 | 390 | A390T | 2:202900538 | TAF1B | 519 | F519C | 2:10059940 |
| GJD4 | 340 | A340T | 10:35897459 | TAS1R2 | 707 | R707H | 1:19166493 |
| GKN1 | 118 | K118N | 2:69206110 | TDRD9 | 564 | R564H | 14:104471720 |
| GPR113 | 771 | A771V | 2:26534284 | TET2 | 1857 | V1857M | 4:106197173 |
| GPR149 | 542 | R542C | 3:154056060 | TET2 | 108 | K108T | 4:106155359 |
| GRIK2 | 723 | E723* | 6:102483297 | TET2 | 373 | F373L | 4:106156155 |
| GRM3 | 271 | V271I | 7:86415919 | TEX11 | 639 | R639* | X:69828950 |
| GRM8 | 219 | S219L | 7:126746621 | TFDP1 | 115 | G115D | 13:114287470 |
| GTF3C1 | 733 | G733W | 16:27509111 | THSD7A | 1526 | S1526L | 7:11419270 |
| HCFC2 | 239 | G239V | 12:104474557 | TLR9 | 901 | R901C | 3:52255631 |
| HCK | 389 | V389F | 20:30681738 | TMEM132C | 563 | G563S | 12:129180490 |
| HCN3 | 293 | S293L | 1:155254337 | TMEM38A | 53 | A53T | 19:16790827 |
| HEPHL1 | 687 | F687L | 11:93819336 | TP53 | 234 | Y234H | 17:7577581 |
| HERC2 | 3384 | L3384I | 15:28414709 | TP53 | 125 | T125M | 17:7579313 |
| HSD17B7 | 245 | P245L | 1:162773312 | TP53 | 241 | S241Y | 17:7577559 |
| IQUB | 735 | R735H | 7:123092969 | TP53 | 337 | R337L | 17:7574017 |
| ITGA8 | 895 | R895* | 10:15600156 | TP53 | 158 | R158H | 17:7578457 |
| ITGB2 | 439 | V439M | 21:46311821 | TP53 | 152 | P152L | 17:7578475 |
| ITPR3 | 1849 | R1849H | 6:33653483 | TP53 | 151 | P151H | 17:7578478 |
| JAG1 | 959 | A959V | 20:10622148 | TP53 | 254 | I254S | 17:7577520 |
| JUNB | 250 | R250L | 19:12903334 | TP53 | 232 | I232T | 17:7577586 |
| KIAA0100 | 804 | N804T | 17:26962194 | TP53 | 193 | H193Y | 17:7578272 |
| KIAA1109 | 0 | - | 4:123201138 | TP53 | 244 | G244C | 17:7577551 |
| KIAA1377 | 68 | R68* | 11:101793445 | TP53 | 238 | C238F | 17:7577568 |
| KIF26B | 2024 | R2024H | 1:245862232 | TP53 | 0 | - | 17:7577018 |
| KIT | 52 | D52G | 4:55561765 | TP53 | 0 | - | 17:7577156 |
| KL | 920 | R920H | 13:33638043 | TP53 | 0 | - | 17:7577157 |
| KRAS | 19 | L19F | 12:25398262 | TP53 | 0 | - | 17:7578555 |
| KRAS | 146 | A146T | 12:25378562 | TPO | 585 | D585N | 2:1491748 |
| KRTAP21-1 | 15 | G15S | 21:32127654 | TREX2 | 7 | P7H | X:152713281 |
| LAMC1 | 327 | P327S | 1:183079747 | TRIM37 | 895 | A895V | 17:57089700 |
| LRFN5 | 445 | R445H | 14:42357162 | UBR5 | 1978 | R1978* | 8:103292691 |
| MAEA | 357 | R357H | 4:1332266 | VHL | 127 | G127V | 3:10188237 |
| MAGI1 | 971 | V971M | 3:65365020 | WT1 | 346 | T346M | 11:32421555 |
| MAK | 272 | R272* | 6:10802142 | YIPF1 | 159 | R159Q | 1:54337050 |
| MARK4 | 418 | R418H | 19:45783969 | YSK4 | 512 | I512L | 2:135744908 |
| MKNK2 | 149 | F149L | 19:2043171 | ZDBF2 | 888 | E888K | 2:207171914 |
| MKRN3 | 76 | P76Q | 15:23811156 | ZFHX4 | 2394 | A2394T | 8:77766385 |
| MSH2 | 580 | E580* | 2:47698180 | ZNF429 | 67 | R67Q | 19:21713460 |
| MUC16 | 2683 | E2683* | 19:9083768 | ZNF564 | 157 | R157Q | 19:12638452 |
| MYST4 | 1373 | E1373G | 10:76788700 | | | | |

***Example 3-Expression and Copy Number Alteration***

The RNA-seq data was used to compute differentially expressed genes between tumor and normal samples (Table 6). The top differentially overexpressed genes include FOXQ1 and CLND1 which have both been implicated in tumorigenesis (Kaneda, H. et al., Cancer Res. 70:2053-2063 (2010)). Importantly, in analyzing the RNA-seq data, IGF2 upregulation was identified in 12% (8/68) of the colon tumors examined. A majority (7/8) of the tumors with *IGF2* overexpression also showed focal amplification of the IGF2 locus as measured by Illumina 2.5M array. Overall the differentially expressed genes affect multiple signaling pathways including Calcium Signaling, cAMP-mediated signaling, Glutamate Receptor Signaling, Amyotrophic Lateral Sclerosis Signaling, Nitrogen Metabolism, Axonal Guidance Signaling, Role of IL-17A in Psoriasis, Serotonin Receptor Signaling, Airway Pathology in Chronic Obstructive Pulmonary Disease, Protein Kinase A Signaling, Bladder Cancer Signaling, HIF1α Signaling, Cardiac β-adrenergic Signaling, Synaptic Long Term Potentiation, Atherosclerosis Signaling, Circadian Rhythm Signaling, CREB Signaling in Neurons, G-Protein Coupled Receptor Signaling, Leukocyte Extravasation Signaling, Complement System, Eicosanoid Signaling, Tyrosine Metabolism, Cysteine Metabolism, Synaptic Long Term Depression, Role of IL-17A in Arthritis, Cellular Effects of Sildenafil (Viagra), Neuropathic Pain Signaling In Dorsal Horn Neurons, D-arginine and D-ornithine Metabolism, Role of IL-17F in Allergic Inflammatory Airway Diseases, Thyroid Cancer Signaling, Hepatic Fibrosis / Hepatic Stellate Cell Activation, Dopamine Receptor Signaling, Role of NANOG in Mammalian Embryonic Stem Cell Pluripotency, Chondroitin Sulfate Biosynthesis, Endothelin-1 Signaling, Keratan Sulfate Biosynthesis, Phototransduction Pathway, Wnt/β-catenin Signaling, Chemokine Signaling, Alanine and Aspartate Metabolism, Glycosphingolipid Biosynthesis - Neolactoseries, Bile Acid Biosynthesis, Role of Macrophages, Fibroblasts and Endothelial Cells in Rheumatoid Arthritis, α-Adrenergic Signaling, Taurine and Hypotaurine Metabolism, LPS/IL-1 Mediated Inhibition of RXR Function, Colorectal Cancer Metastasis Signaling, CCR3 Signaling in Eosinophils, and O-Glycan Biosynthesis.

***Table 6 – Differentially Expressed Genes***

| Gene | Med. Ratio | Gene | Med. Ratio | Gene | Med. Ratio | Gene | Med. Ratio |
|---|---|---|---|---|---|---|---|
| GRIN2D | 5.527911151 | ULBP2 | 3.251373 | TMEM35 | -5.87287 | UBE2QL1 | -3.20056 |
| ESM1 | 5.8492323 | PDZRN4 | -5.95469 | NEGR1 | -4.18072 | GPT | -3.45351 |
| SCARA5 | -5.385767469 | KLK6 | 6.329258 | LDB3 | -6.44118 | CORO6 | -3.60142 |
| CLEC3B | -4.299952709 | TNS1 | -4.19155 | ELANE | -3.01674 | PKIB | -3.53135 |
| CDH3 | 5.215804799 | TLX2 | -3.09629 | ABCA6 | -3.1197 | TRIM9 | -3.56341 |
| FAM107A | -3.972772143 | PGR | -4.27086 | ZNF471 | -3.10221 | MORN5 | -6.87885 |
| ETV4 | 5.202149185 | FXYD6 | -3.75281 | GFRA1 | -4.85831 | TRPM6 | -4.2107 |
| LIFR | -3.797126397 | ENSG00000186198 | -3.577 | DCLK1 | -4.28576 | AP3B2 | -3.96509 |
| CFD | -3.553187855 | CA10 | -3.80922 | PAPPA2 | -4.80217 | DYNC1I1 | -3.84378 |
| ABCA8 | -5.344364012 | P2RX2 | -3.60054 | SFTA2 | 3.697678 | TLX1 | 3.90657 |
| ADH1B | -6.387892211 | SNTG2 | -3.04582 | MYOCD | -5.20677 | SMYD1 | -6.92391 |
| CLDN1 | 5.012197386 | ADD2 | -3.37298 | HMGCLL1 | -3.57011 | TPO | -3.03245 |
| PCSK2 | -6.510043576 | C7orf58 | -3.71657 | SYT9 | -3.72752 | FEZF1 | 4.145292 |
| CADM3 | -5.656232948 | NTNG1 | -4.33834 | MMP11 | 3.476176 | STXBP5L | -4.38119 |
| GCNT2 | -3.893699055 | MT1M | -3.55477 | PKNOX2 | -3.41966 | C15orf59 | -3.11512 |
| NFE2L3 | 3.030392992 | PPP1R1A | -6.04336 | ATP2B2 | -3.50563 | CSPG4 | -3.24734 |
| PLP1 | -6.925097821 | SPEG | -4.57945 | PLIN4 | -6.50771 | HOXB8 | 3.758374 |
| GREM2 | -4.936580737 | RBFOX3 | -6.45602 | RGS9 | -3.41372 | DNASE1L3 | -3.78422 |
| KRT80 | 5.779751934 | MYL9 | -4.27584 | GALNTL1 | -3.71028 | STK32A | -3.58912 |
| GNG7 | -3.111266907 | GRIK1 | -3.25517 | VWA2 | 4.684454 | NIPAL4 | -3.75232 |
| FIGF | -5.893082321 | LRP1B | -3.73288 | EPHA7 | -5.68169 | SYPL2 | -3.51243 |

| | | | | | | | |
|---|---|---|---|---|---|---|---|
| ABI3BP | -3.927046547 | SLC4A11 | 3.038906 | KHDRBS2 | -3.32022 | BTNL8 | -3.56206 |
| BMP3 | -6.026497259 | FRMPD4 | -5.18841 | SLC9A9 | -3.02137 | GDF1 | -3.06235 |
| FAM135B | -5.249518149 | SALL4 | 3.82405 | CEND1 | -3.89797 | KRT16 | 3.228284 |
| TMEM100 | -4.113484387 | SORBS1 | -3.59918 | ADH1A | -3.53935 | LRRTM4 | -3.28156 |
| FOXQ1 | 5.961706421 | LRFN5 | -3.93986 | FAM70A | -3.22263 | CA9 | 4.115683 |
| PRIMA1 | -6.536400714 | GDNF | -3.38792 | ATP2B3 | -4.40254 | BEND4 | -3.23908 |
| RXRG | -5.17454591 | LRRC55 | -3.23821 | SLC5A7 | -5.54508 | PENK | -5.56339 |
| NPY2R | -5.14798919 | PALM | -3.04045 | BCHE | -5.9095 | TRPV3 | -3.25367 |
| STMN2 | -4.313406115 | POU5F1B | 3.400104 | NRG2 | -4.68132 | ST6GAL2 | -3.08256 |
| FGL2 | -3.470259436 | MSRB3 | -3.5926 | EPHA5 | -4.17595 | C9orf71 | -4.08237 |
| XKR4 | -5.330615225 | NACAD | -3.3653 | SEMA6D | -3.01017 | FLNA | -3.69003 |
| PMP2 | -5.699849035 | SLC30A10 | -5.73614 | HAND2 | -5.22194 | SLC26A3 | -5.74678 |
| LGI1 | -5.654013059 | PRICKLE2 | -3.00229 | CNN1 | -5.8107 | TPM2 | -3.48339 |
| OGN | -5.532547559 | CORO2B | -3.16284 | GPC5 | -3.57394 | C8orf85 | -3.63174 |
| STMN4 | -5.165270827 | JPH2 | -4.49583 | TUB | -3.23422 | MMP3 | 4.001157 |
| CNTN2 | -5.725939567 | RNF150 | -4.85505 | PRKG2 | -3.49777 | MS4A12 | -5.72245 |
| MAL | -4.946126006 | SCARA3 | -3.1352 | ACTG2 | -6.10699 | NPY | -4.33465 |
| CMA1 | -4.728693462 | SALL2 | -3.43114 | SLC25A34 | -3.9354 | MPPED2 | -3.44536 |
| TRIB3 | 3.512044792 | SLC17A8 | -4.17524 | ZNF229 | -3.21126 | ALPI | -4.27169 |
| C16orf89 | -4.647446159 | MAOB | -3.46607 | SLC35F1 | -3.74017 | KCNC1 | -3.18694 |
| NKX2-3 | -3.772558945 | ADAMTS8 | -4.17885 | RASGEF1C | -4.3263 | TMEM72 | -4.72328 |
| NRXN1 | -6.423571094 | OTOP3 | -4.14905 | ZNF727 | -3.30848 | FAM163B | -3.57859 |
| SGCG | -4.315399416 | PACSIN1 | -3.12832 | ABCB5 | -3.98259 | DPP10 | -4.59947 |
| ASPA | -4.85466365 | UCHL1 | -3.37593 | LRRK2 | -3.12594 | CLEC5A | 3.260118 |
| PRPH | -5.709414092 | TNNI3 | 3.475204 | FAM176A | 3.177313 | CPNE6 | -3.37143 |
| SCGN | -5.617899565 | MFAP5 | -3.73929 | RBM20 | -4.1105 | ITGB1BP2 | -3.00778 |
| FXYD1 | -4.366726331 | ITGA7 | -3.5897 | MEIS1 | -3.19375 | SLITRK5 | -3.90369 |
| PDK4 | -3.783018003 | DNAJB5 | -3.77773 | DES | -6.69236 | PLA2G5 | -3.71785 |
| SCN9A | -4.210073456 | C14orf180 | -3.28894 | C1QTNF9 | -3.92526 | UCN3 | -3.72869 |
| LYVE1 | -4.003213022 | CA1 | -6.9112 | SLC17A7 | -3.3932 | CALD1 | -3.05258 |
| ADCY5 | -4.897621234 | ATP2B4 | -3.48549 | EFHC2 | -3.27123 | STON1-GTF2A1L | -3.0375 |
| SCN11A | -4.89796532 | MRVI1 | -3.02877 | TMEM130 | -4.36447 | PDE6A | -3.60006 |
| LGI4 | -3.654270687 | SIGLEC6 | -3.16606 | DIRAS1 | -3.16403 | KRT6B | 4.798528 |
| TNXB | -4.618096417 | CCBE1 | -5.06789 | ZMAT4 | -3.40709 | GPIHBP1 | -3.50724 |
| TUBB4 | -5.392668311 | BVES | -4.20565 | PTPRZ1 | -5.77615 | KLK10 | 3.487382 |
| AFF3 | -4.544564729 | TMIGD1 | -6.41231 | CPEB1 | -4.46103 | C4orf39 | -3.02818 |
| PDX1 | 4.962327216 | KCNQ5 | -4.00333 | PHOX2A | -4.23422 | STAC | -3.35799 |
| FHL1 | -5.16962219 | L1CAM | -4.14268 | NLGN4X | -3.04296 | CRLF1 | -3.20379 |
| TMEFF2 | -4.698800032 | PTH1R | -3.19452 | ATP6V1G2 | -3.55979 | SLC4A10 | -3.13074 |
| SLCO4A1 | 3.054897403 | MYEOV | 3.166568 | BEST4 | -5.95684 | AKR1B10 | -3.46237 |
| MGAT4C | -3.527256991 | SLC2A4 | -4.46266 | THRB | -3.20412 | CST2 | 3.483231 |
| MMRN1 | -4.358473391 | ZCCHC12 | -3.49788 | WISP2 | -5.3983 | NKX3-2 | -3.21332 |
| KIAA1199 | 4.989222927 | VIPR2 | -3.68461 | GRIK5 | -4.77377 | REEP1 | -3.46272 |
| PLAC9 | -3.544659302 | PSD | -5.87501 | DARC | -3.24148 | HRASLS5 | -4.03008 |
| PI16 | -6.329320626 | CHRNA3 | -3.10067 | C6orf174 | -3.92882 | TUSC5 | -4.62354 |

| | | | | | | | |
|---|---|---|---|---|---|---|---|
| MAMDC2 | -6.16899378 | NRXN2 | -3.13659 | GUCA2A | -5.3278 | KRT23 | 4.884049 |
| SFRP1 | -5.719553754 | C8orf46 | -4.37921 | SLC6A15 | -4.37144 | TUBB2B | -3.24294 |
| ANK2 | -4.698529299 | GPR17 | -3.52967 | AOC3 | -3.97636 | CPLX2 | -3.94707 |
| SPHKAP | -3.648224781 | CACNA1H | -3.64108 | NGFR | -3.93572 | DSCR6 | 3.028702 |
| SCN7A | -7.144549308 | DKK4 | 3.476871 | LGI3 | -4.24132 | FCER2 | -4.78069 |
| ENSG000 00170091 | -5.71036492 | PDLIM3 | -3.71073 | NFASC | -3.11179 | MYADML2 | 3.209455 |
| CDH19 | -6.322889292 | SCN3B | -3.3718 | GRIA1 | -3.57011 | KCNA2 | -3.13365 |
| SCG2 | -3.422093337 | GYLTL1B | 4.082537 | SYP | -3.15922 | SV2C | -3.78632 |
| CXCL12 | -3.487164375 | AGTR1 | -4.79524 | EPHX4 | 3.512462 | DCHS2 | -4.2511 |
| CDH10 | -3.421342024 | ULBP1 | 3.320975 | DUSP26 | -4.13989 | PCYT1B | -3.17282 |
| RERGL | -5.731261829 | AQP8 | -7.23747 | CTHRC1 | 3.080178 | ZNF385B | -3.25358 |
| MPZ | -3.920611558 | ARL4D | -3.38549 | PCDH9 | -4.11247 | PTGIS | -3.7594 |
| SYT10 | -4.190609336 | FAM46B | -4.53516 | CA7 | -6.19335 | C6orf168 | -3.30589 |
| RELN | -3.986177885 | RND2 | -3.61077 | EGFL6 | 3.166084 | SNCA | -3.01935 |
| CMTM5 | -4.756084449 | ARHGEF25 | -3.24015 | FBXO32 | -3.02151 | LRAT | -3.89481 |
| CTNND2 | -4.740498304 | PRKAA2 | -4.51677 | PYY | -6.36724 | TMEM74 | -3.406 |
| NOVA1 | -5.061410431 | TACR1 | -3.80639 | KIAA1644 | -5.0075 | SCN4A | -3.72869 |
| CADM2 | -5.485961881 | NBEA | -3.79003 | NRSN1 | -4.23319 | CA2 | -5.11198 |
| ZNF536 | -4.571820763 | FABP4 | -5.42586 | SEMA3E | -5.7604 | SLC8A2 | -4.48591 |
| RBM24 | -3.569579564 | ODZ1 | -3.89586 | C1orf173 | -3.89609 | KCNA5 | -3.45695 |
| S100B | -3.827538343 | C5orf4 | -3.0289 | CCL23 | -4.10995 | TPH1 | -3.20483 |
| ADHFE1 | -3.662707626 | PPP1R14A | -4.03457 | ATP1B2 | -3.35903 | WSCD2 | -4.87618 |
| GLP2R | -4.345544907 | HTR1D | 3.884431 | DIRAS2 | -4.285 | KCNMB2 | -3.10173 |
| PHOX2B | -5.937887122 | MMP13 | 3.671083 | CXCL3 | 3.414119 | ENSG000 00241186 | 3.118557 |
| VAT1L | -3.228136479 | RPH3A | -3.35741 | PCP4L1 | -5.84118 | CIDEA | -3.26865 |
| PIRT | -6.031181735 | SGCA | -4.55537 | C2orf70 | 3.623413 | GABRB3 | -4.50283 |
| SDPR | -4.38545828 | MAPK15 | 3.320975 | NPTX1 | -6.3263 | KCNIP1 | -3.16613 |
| GRIK3 | -5.197048843 | FEV | -4.02478 | PCOLCE2 | -3.83253 | C6orf105 | -3.61541 |
| GSTM5 | -3.615514934 | GDF15 | 3.02245 | HEPACAM | -4.285 | NOTUM | 4.401768 |
| SST | -5.824093007 | RIMS4 | -4.24267 | CNTNAP3 | -4.46258 | KLHL34 | -3.1504 |
| PKHD1L1 | -4.242036298 | SULT1A2 | -3.79483 | CAV1 | -3.2595 | C1orf70 | -3.00556 |
| SLC7A14 | -5.520042397 | C6orf186 | -4.60198 | KIAA1045 | -4.0874 | CLDN8 | -4.97278 |
| CHRDL1 | -5.107430525 | TTYH1 | -3.33098 | LRRTM1 | -4.44609 | DPEP1 | 6.134526 |
| DPT | -5.051072538 | HSPB7 | -4.74217 | SEZ6L | -4.32666 | SCNN1G | -4.65465 |
| NAP1L2 | -4.961540922 | SLITRK3 | -6.10753 | CRYAB | -3.85914 | STRA6 | 3.757395 |
| SOX10 | -5.724445462 | CD1C | -3.12922 | ADAMTSL3 | -4.67756 | OMD | -3.85155 |
| CTSG | -4.258813557 | GPR133 | -3.04867 | ELAVL3 | -4.63805 | CARTPT | -5.03476 |
| KIAA1257 | 3.264630691 | EDN3 | -3.70756 | CCL21 | -3.44647 | CCL24 | 3.328538 |
| CNR1 | -5.472912411 | KCNA1 | -4.65058 | SYT5 | -4.12123 | SLCO1B3 | 4.350979 |
| C2orf88 | -3.489231209 | RERG | -3.17221 | GFRA3 | -5.01204 | PLIN1 | -4.0474 |
| VIP | -4.860630378 | CA14 | -3.58713 | FIGN | -3.00533 | TMEM82 | -3.60685 |
| TMEM151B | -5.008283549 | SORCS3 | -4.02347 | PCDH10 | -4.341 | CALB2 | -3.70005 |
| ANO5 | -4.232602678 | ZG16 | -5.39174 | MMP7 | 6.216617 | CES1 | -3.1966 |
| PTN | -3.44306466 | CNTNAP3B | -3.6873 | SPARCL1 | -3.36702 | DAO | -4.48241 |

| | | | | | | | |
|---|---|---|---|---|---|---|---|
| ST8SIA3 | -4.79377543 | DOCK3 | -3.39657 | OTOP2 | -8.12168 | INSL5 | -5.05983 |
| MUSTN1 | -3.245149184 | DACT3 | -3.71844 | CNTD2 | 4.300648 | AK5 | -3.0314 |
| GFRA2 | -3.811511174 | SIM2 | 3.536988 | SFRP5 | -5.11522 | KRTAP13-2 | -4.63517 |
| ATP1A2 | -7.307217248 | CHRM2 | -7.34891 | ABCA9 | -3.81151 | NXPH3 | -3.40456 |
| PRKCB | -3.797860637 | PTPRT | -3.37251 | BEND5 | -3.66782 | GTF2A1L | -3.15117 |
| FAM123A | -3.035990832 | ADH1C | -3.51198 | FAM163A | -3.67521 | CWH43 | -4.40603 |
| ANGPTL7 | -5.947492322 | FAM189A1 | -3.40677 | TMEM132B | -3.32426 | CDO1 | -3.38273 |
| WNT2 | 4.717355945 | ASCL2 | 3.879815 | COL11A1 | 4.703239 | DSG3 | 3.778247 |
| ARPP21 | -3.941970851 | SERTM1 | -3.06772 | IGFBP6 | -3.05252 | TMEM211 | 3.460662 |
| DNER | -4.314790344 | POPDC2 | -4.95848 | PYGM | -5.86766 | PRUNE2 | -3.08848 |
| VSTM2A | -5.109872721 | WBSCR17 | -3.51278 | LYNX1 | -3.79672 | PKP1 | 3.65574 |
| GPM6B | -4.031255119 | SULT4A1 | -5.00147 | ST8SIA1 | -3.0922 | NPPC | -3.53724 |
| MYOM1 | -4.650824187 | HLF | -3.91765 | TLL1 | -3.01592 | RAET1L | 3.027935 |
| ASTN1 | -5.126882925 | DDN | 3.337204 | EML1 | -3.36098 | DHRS9 | -3.13217 |
| RASGRP2 | -3.503626906 | MAP1B | -3.10167 | SLC4A4 | -4.54921 | CCDC136 | -3.33404 |
| C6orf223 | 4.226814021 | CLDN11 | -3.45731 | MAP2 | -3.16049 | CDON | -3.00288 |
| ANGPTL1 | -5.424044031 | PLCXD3 | -4.84211 | CCNO | 3.479898 | PRDM6 | -3.28755 |
| ENPP6 | -3.963010538 | MAP6 | -3.67268 | COL19A1 | -3.66553 | PCSK1N | -4.0894 |
| LRRN2 | -3.5025362 | MADCAM1 | -3.50743 | HTR3A | -4.72177 | CCL19 | -3.40271 |
| BAALC | -3.426625507 | CTNNA2 | -4.70269 | CNTN1 | -4.35232 | DLX1 | -3.38643 |
| C2orf40 | -5.929905648 | RET | -3.70964 | ADRA1A | -3.46392 | NKAIN2 | -3.32274 |
| ATCAY | -5.088408777 | AZGP1 | 3.513263 | DMD | -3.60911 | KLK7 | 3.937762 |
| ADAM33 | -3.969644735 | VWC2 | -3.11767 | TMEM179 | -3.23581 | GPR15 | -3.81204 |
| IGSF10 | -4.187581248 | GCG | -5.94559 | TACR2 | -5.57163 | FAM19A4 | -3.27095 |
| INHBA | 3.61816183 | STK31 | 3.869912 | DPYSL5 | -4.68945 | TMEM236 | -3.94135 |
| ADCYAP1R1 | -5.525027043 | OSR1 | -3.8245 | CSRP1 | -3.16604 | RGS13 | -3.26189 |
| GRIN2A | -4.44436921 | TAGLN | -3.54734 | SCNN1B | -4.78493 | ADAMTS19 | -3.28724 |
| CHL1 | -3.413871889 | RAB9B | -3.67691 | CNTFR | -5.48107 | AFF2 | -3.37251 |
| NTN1 | -3.354856128 | FBXL22 | -3.44664 | GPM6A | -7.05382 | HS6ST2 | 3.561665 |
| MYLK | -4.40930035 | NPAS3 | -3.21742 | CASQ2 | -6.97291 | MMP10 | 3.376316 |
| FOXF2 | -3.273857064 | FGF10 | -3.65639 | CHGB | -4.37302 | ADRA1D | -3.54704 |
| USP2 | -3.134670717 | ADCY2 | -3.40603 | EEF1A2 | -4.32423 | COMP | 3.932262 |
| CNGB1 | -3.796951333 | GRHL3 | 3.473116 | RBPMS2 | -5.2819 | SMPX | -5.10753 |
| PTGS1 | -3.928784334 | DDR2 | -3.12621 | MMP1 | 4.611965 | CYP4B1 | -3.06758 |
| JAM2 | -3.225588456 | EPHA6 | -5.87065 | TAGLN3 | -5.51147 | LGALS9C | -3.00879 |
| SETBP1 | -3.299570168 | WNT7B | 3.107819 | ASXL3 | -3.25378 | FAM150A | 3.651605 |
| C2CD4A | 4.171923278 | TNS4 | 3.872147 | CNKSR2 | -3.76265 | TG | 3.001709 |
| MAB21L1 | -4.648224781 | ENSG000 00172901 | -3.34783 | FGFBP2 | -3.4953 | ANPEP | -3.23022 |
| HBB | -3.10879867 | CACNA2D1 | -3.1969 | GHR | -3.12319 | TNFRSF13B | -3.86004 |
| VSNL1 | 3.375999204 | AQP4 | -3.03599 | CELF4 | -4.19572 | HSPB3 | -3.48254 |
| NGB | -5.687368193 | TWIST2 | -3.06429 | CUX2 | -3.78755 | CD22 | -3.53242 |
| MYOC | -6.743818793 | SCRG1 | -5.53503 | DLG2 | -3.41983 | HSD17B2 | -3.25123 |
| KIF1A | -5.583478047 | FNDC9 | -3.67385 | GRIA2 | -3.13335 | CLEC17A | -3.32539 |
| LEMD1 | 5.429399854 | C11orf86 | -4.68391 | SPIB | -4.95933 | FAM5C | -3.97373 |
| KRT24 | -5.939566634 | SULT2B1 | 3.1843 | AR | -3.46973 | RPRM | -4.18572 |

| | | | | | | | |
|---|---|---|---|---|---|---|---|
| CHODL | -4.306804825 | PNCK | -5.38004 | LMX1A | -3.07579 | PCP4 | -4.67099 |
| MYH11 | -6.614033693 | ZDHHC15 | -3.06835 | NAP1L3 | -3.15647 | PIWIL1 | 3.12939 |
| SCN2B | -5.019950619 | CLDN2 | 5.310113 | HEPN1 | -3.48966 | BLK | -3.69271 |
| BAI3 | -5.029545504 | FILIP1 | -3.78534 | SLITRK2 | -3.62411 | SLC17A4 | -3.31472 |
| SORCS1 | -5.345853041 | ABCC8 | -3.0022 | FAM181B | -4.05256 | PEG10 | -3.43391 |
| SYNPO2 | -5.938491333 | CAP2 | -3.2824 | KRT222 | -3.88727 | ZIC2 | 3.206285 |
| C9orf4 | -3.946781299 | LIX1 | -4.29903 | RASD2 | -3.08403 | UGT2A3 | -3.67931 |
| C7 | -4.817175938 | PRRT4 | -3.06141 | ENSG000 00156475 | -3.70456 | TF | -4.10524 |
| HSPB6 | -5.759563929 | B3GALT1 | -3.69549 | ABCG2 | -4.10507 | THBS4 | -4.81204 |
| OLFM3 | -5.152622362 | CPNE4 | -3.60054 | AKAP6 | -3.99525 | ENSG000 00181495 | -3.35886 |
| SNAP91 | -5.039150058 | STAC2 | -3.70576 | KCNMB1 | -5.21732 | FCRLA | -3.79316 |
| ASB2 | -4.463866848 | PPP1R3C | -3.27984 | FOXD3 | -4.61265 | TLR10 | -3.13859 |
| HPSE2 | -3.786836392 | NECAB2 | -3.2714 | MRGPRF | -3.788 | CXCL5 | 4.082364 |
| C12orf53 | -3.50784602 | ASB5 | -6.21444 | ANKRD35 | -3.15042 | PRSS33 | 3.145979 |
| CHGA | -5.718288794 | PTPRN | -3.45244 | HSPB8 | -5.19288 | PHYHIP | -3.00667 |
| KIF5A | -4.179157002 | NNAT | -4.58578 | IBSP | 3.429821 | ASPG | -3.38654 |
| CCDC69 | -3.785092508 | MGP | -3.10442 | CFL2 | -3.60155 | C6 | -3.27127 |
| PPP1R12B | -3.964688977 | WDR72 | 4.380471 | CNGA3 | -4.70795 | MYPN | -3.1019 |
| GPER | -3.374629722 | CLMP | -3.01603 | KCNB1 | -5.91463 | B4GALNT2 | -3.65998 |
| RIC3 | -5.121450191 | KRT6A | 3.797132 | PRELP | -4.32292 | B3GALT5 | -3.27156 |
| CAMK2A | -3.315318636 | MPP2 | -3.37321 | KIRREL3 | -3.7696 | MT1H | -3.33951 |
| UNC5D | -3.456610995 | PCK1 | -3.24127 | CST1 | 6.01139 | SLC6A19 | -5.20458 |
| NLGN1 | -5.36205776 | KCNK2 | -3.80447 | CNTN3 | -3.89004 | WFIKKN2 | -3.02818 |
| CBLN2 | -4.410205906 | IL11 | 3.803898 | LIMS2 | -3.73614 | HRASLS2 | -3.11679 |
| CLU | -3.575663389 | LGR5 | 3.195895 | BEX1 | -5.05729 | FCRL1 | -3.96835 |
| C1orf95 | -5.541950034 | CRABP1 | -4.05718 | FOXP2 | -4.26963 | PNPLA3 | 3.007076 |
| ENTPD3 | -3.440071356 | UNC80 | -3.71831 | BHMT2 | -4.36555 | TEX11 | -3.50005 |
| ZBTB16 | -5.143639363 | CASQ1 | -4.56195 | TCEAL2 | -5.6985 | CNR2 | -3.60619 |
| MAPK4 | -6.268370446 | UST | -3.03978 | FLNC | -5.09657 | UNC93A | 3.098461 |
| ENSG000 00234602 | 3.542010519 | NOS1 | -6.01896 | SYNGR1 | -3.54338 | MS4A1 | -4.05133 |
| PDE2A | -3.622736206 | JPH3 | -3.656 | CXCL1 | 3.08057 | FAM129C | -3.4555 |
| CPNE7 | 4.696574774 | CPB1 | -3.22272 | SEMA3D | -3.33337 | PTGDR | -3.38298 |
| RALYL | -3.54986467 | ATRNL1 | -4.89143 | CAND2 | -3.47155 | SOX2 | -3.87896 |
| CHST9 | -3.858149202 | LRRC4C | -3.78069 | GRIA4 | -3.67598 | TCL1A | -4.87298 |
| SLIT3 | -3.701786983 | KCNK3 | -4.66311 | KIAA0408 | -4.1775 | NEUROD1 | -3.91126 |
| SRPX | -3.676380924 | KY | -4.27669 | KLK8 | 4.906754 | FCRL4 | -3.59163 |
| ALK | -4.400128747 | SNAP25 | -4.69627 | REEP2 | -3.92231 | ABCB11 | -3.61699 |
| FMN2 | -5.931523283 | AKAP12 | -3.03021 | CILP | -4.88337 | OR51E2 | -3.21721 |
| MED12L | -3.505446576 | ADRB3 | -3.86996 | COL10A1 | 6.229643 | MSLN | 3.156575 |
| GNAO1 | -5.424519258 | NPTXR | -3.0905 | PTCHD1 | -5.72018 | NTSR1 | -4.19058 |
| GABRG2 | -4.48694237 | C10orf140 | -3.44724 | FGF13 | -3.1075 | SFRP2 | -3.06381 |
| PLEKHN1 | 3.36299512 | EXTL1 | -3.23226 | TCEAL6 | -3.90028 | CR2 | -4.33926 |
| PGM5 | -5.403079028 | TCN1 | 5.883899 | PRSS22 | 3.796724 | CNTNAP5 | -3.28156 |
| IGSF11 | -5.005562617 | SOHLH2 | -3.7527 | CD300LG | -4.20088 | HS3ST5 | -3.32274 |

| | | | | | | | |
|---|---|---|---|---|---|---|---|
| RYR3 | -4.359671118 | SLC26A2 | -3.4259 | ZDHHC22 | -4.05715 | GDF5 | -3.6779 |
| FAM189A2 | -3.291843764 | ANO3 | -3.40677 | GPRASP1 | -3.07048 | IGJ | -3.37943 |
| SCN3A | -3.249263581 | SERPINB5 | 3.010596 | SV2B | -3.47286 | SLC6A17 | -3.03858 |
| ZIM2 | -3.923857044 | TACSTD2 | 3.803266 | NDE1 | -4.07805 | CEACAM7 | -3.71794 |
| MUSK | -4.806618761 | COL21A1 | -3.21866 | CTNNA3 | -4.63484 | NPR3 | -3.0056 |
| PDZD4 | -4.652064044 | CLCA4 | -5.73343 | DMRTA1 | -3.4379 | HSD3B2 | -3.65443 |
| LCN6 | -3.528251776 | WNT9A | -3.10701 | HTR4 | -4.20483 | SLC6A20 | 3.640564 |
| IL8 | 3.733680463 | SCG3 | -4.84991 | CA4 | -5.90306 | PITX2 | 3.733959 |
| OTX1 | 5.606699636 | DSCAML1 | -4.05228 | NPAS4 | -3.90303 | VPREB3 | -3.55929 |
| NTRK3 | -4.190549367 | WDR17 | -4.00891 | NECAB1 | -4.4301 | CLCA1 | -4.54287 |
| SPOCK3 | -5.313979085 | ADIPOQ | -6.95511 | MAPT | -4.07028 | SI | -3.14912 |
| FAM129A | -4.00370568 | TESC | 3.379012 | TNNT3 | -3.6104 | PLA2G2D | -3.10473 |
| NEFM | -4.972634341 | HAND1 | -7.23383 | INA | -4.86742 | FSTL5 | -3.95247 |
| TMEM59L | -4.351475682 | ART4 | -3.18603 | LMO3 | -6.04405 | FCRL3 | -3.28603 |
| TCEAL5 | -4.044195288 | GLDN | -3.09313 | CLIP4 | -3.26924 | C4orf7 | -4.10287 |
| SNCG | -3.194688135 | KCNIP3 | -3.54139 | MASP1 | -5.93003 | SERPINA9 | -3.05435 |
| SLC27A6 | -3.944375846 | SLIT2 | -3.26504 | SEZ6 | -3.81918 | LEP | -3.10313 |
| GAD1 | 4.607492087 | RNF183 | 3.39193 | SYT4 | -5.08841 | PAX5 | -3.45097 |
| CAMK2B | -3.748134652 | LRCH2 | -3.28776 | CLVS2 | -3.44001 | CNNM1 | -3.01846 |
| ARHGAP20 | -3.301303729 | SH3GL2 | -3.57011 | TCEAL7 | -3.00191 | MEP1B | -3.1861 |
| GUCA2B | -7.224954766 | KCTD8 | -3.83424 | PLN | -4.77387 | OTC | -3.16879 |
| MYOT | -4.653308928 | CHRNB4 | -3.62563 | KCTD4 | -3.30001 | ITLN1 | -3.06475 |
| VIT | -3.54751268 | CERS1 | -3.17135 | SLC10A4 | -3.7343 | GALNT13 | -3.23173 |
| LONRF2 | -6.377805944 | CHD5 | -3.20136 | C1QTNF7 | -4.12134 | FCGBP | -3.06625 |
| LMOD1 | -5.04599233 | DTNA | -3.82362 | RSPO2 | -5.33522 | REG1A | 3.21229 |
| CALY | -5.271272834 | CCDC80 | -3.0985 | P2RY12 | -3.56585 | GP2 | -3.17456 |
| GAP43 | -4.71341546 | ENSG000 00166869 | -3.90266 | CHST8 | -3.13524 | APOB | -4.0069 |
| MYT1L | -3.629480911 | CPXM2 | -4.17959 | STOX2 | -3.05401 | FABP6 | 4.971592 |
| ELAVL4 | -4.406765367 | DAND5 | -3.98467 | MAB21L2 | -5.0333 | REG3A | 4.052759 |
| JPH4 | -3.596788653 | DGKB | -4.15446 | SLC18A3 | -3.99774 | GDF10 | -3.18603 |
| RGMA | -3.985267039 | HIF3A | -3.6805 | IL17B | -3.26935 | TTR | -3.00706 |
| KCNMA1 | -4.992859998 | HPCAL4 | -3.24851 | SHISA3 | -3.12044 | MTTP | -3.07406 |
| KIAA2022 | -5.25714319 | CCDC169 | -3.48135 | RAB3C | -3.7531 | | |

Copy number alterations in 74 tumor/normal pairs were assessed by applying GISTIC to the PICNIC segmented copy number data. In addition to the *IGF2* amplifications, known amplifications were found involving *KRAS* (13%; 10/74) and *MYC* (31%; 23/74) located in a broad amplicon on chromosome 8q (Table 7). Focal deletion involving *FHIT*, a tumor suppressor was observed in 21% (16/74) of the samples (Table 8). *FHIT*, which encodes a diadenosine 5',5'''-P1,P3-triphosphate hydrolase involved in purine metabolism, has previously been reported to be lost in other cancers (Pichiorri, F. et al., Future Oncol. 4:815-824 (2008)). Deletion of *APC* (18%; 14/74) and *SMAD4* (29%; 22/74) was also observed. Finally, chromosome 20q was found to be frequently gained and in contrast, 18q to be lost.

When copy number alterations were analyzed using PICNIC probe-level copy number calling, CBS segmentation of the copy number tumor/normal ratios and GISTIC on these tumor/normal ratios, the top set of genes with copy number alterations were similar though the percentages varied slightly. Known amplifications involving KRAS (13%; 10/74) and MYC (23%; 17/74) located in a broad amplicon on chromosome 8q. Deletion involving FHIT, a tumor suppressor was observed in 30% (22/74) of the samples. Deletion of APC (8%; 6/74), PTEN (4%, 3/74) and SMAD3 (9%, 10/74). SMAD4 and SMAD2 are both altered in 27% (20/74) of the samples and are located within 3 Mb from each other on 18q which is frequently lost.

*Table 7 - Genes with significant copy number gain*

| GeneName | Freq. | GeneName | Freq. | GeneName | Freq. | GeneName | Freq. |
|---|---|---|---|---|---|---|---|
| LYZL1 | 0.040541 | SMOX | 0.216216 | OSR2 | 0.27027 | ANGPT1 | 0.256757 |
| TH | 0.108108 | MRPS33P4 | 0.364865 | SYBU | 0.243243 | FAM91A1 | 0.297297 |
| IGF2 | 0.108108 | SUMO1P1 | 0.364865 | GPR20 | 0.243243 | PLEKHF2 | 0.202703 |
| INS-IGF2 | 0.108108 | C20orf112 | 0.351351 | SQLE | 0.324324 | C8orf37 | 0.202703 |
| INS | 0.108108 | COMMD7 | 0.351351 | VPS13B | 0.324324 | RALYL | 0.243243 |
| ERC1 | 0.121622 | DNMT3B | 0.337838 | KIAA0196 | 0.324324 | ATAD2 | 0.256757 |
| RAD52 | 0.121622 | CDK5RAP1 | 0.337838 | MMP16 | 0.243243 | C8orf34 | 0.216216 |
| CASC1 | 0.135135 | RALY | 0.351351 | STAU2 | 0.256757 | ZFPM2 | 0.27027 |
| LRMP | 0.121622 | EIF2S2 | 0.351351 | NSMCE2 | 0.324324 | KCNK9 | 0.27027 |
| C12orf77 | 0.108108 | ASIP | 0.364865 | CSMD3 | 0.283784 | TRAPPC9 | 0.27027 |
| IFLTD1 | 0.162162 | AHCY | 0.364865 | TRIB1 | 0.256757 | OXR1 | 0.310811 |
| C12orf5 | 0.094595 | ITCH | 0.405405 | FAM84B | 0.283784 | CHMP4C | 0.243243 |
| SLCO1A2 | 0.121622 | KIF16B | 0.256757 | POU5F1B | 0.351351 | SCRIB | 0.243243 |
| IAPP | 0.121622 | CHRNA4 | 0.378378 | MYC | 0.310811 | TMED10P1 | 0.243243 |
| PYROXD1 | 0.121622 | KCNQ2 | 0.378378 | TOX | 0.27027 | RHPN1 | 0.283784 |
| RECQL | 0.121622 | EEF1A2 | 0.378378 | TMEM75 | 0.283784 | MAFA | 0.27027 |
| GOLT1B | 0.108108 | C20orf203 | 0.351351 | GSDMC | 0.256757 | ZC3H3 | 0.27027 |
| C12orf39 | 0.108108 | BAK1P1 | 0.351351 | FAM49B | 0.27027 | GSDMD | 0.256757 |
| GYS2 | 0.108108 | BPIFB5P | 0.337838 | COX6C | 0.27027 | C8orf73 | 0.256757 |
| LDHB | 0.108108 | BPIFB9P | 0.337838 | RGS22 | 0.283784 | PUF60 | 0.243243 |
| NECAP1 | 0.135135 | TPM3P2 | 0.351351 | ASAP1 | 0.256757 | NAPRT1 | 0.256757 |
| SLC2A14 | 0.135135 | RPS2P1 | 0.351351 | TRPS1 | 0.22973 | NRBP2 | 0.243243 |
| NANOGP1 | 0.135135 | XPOTP1 | 0.364865 | FBXO43 | 0.27027 | EEF1D | 0.243243 |
| SLC2A3 | 0.135135 | CDC42P1 | 0.391892 | POLR2K | 0.27027 | EPPK1 | 0.243243 |
| LYRM5 | 0.135135 | ITCH-AS1 | 0.391892 | ADCY8 | 0.27027 | PLEC | 0.22973 |
| KRAS | 0.135135 | ITCH-IT1 | 0.391892 | GDAP1 | 0.256757 | SLC39A4 | 0.22973 |
| POTEM | 0.067568 | FDX1P1 | 0.391892 | EIF3H | 0.22973 | VPS28 | 0.22973 |
| OR4N2 | 0.067568 | HMGB3P1 | 0.378378 | SPAG1 | 0.297297 | TONSL | 0.22973 |
| OR4Q3 | 0.067568 | MT1P3 | 0.378378 | RNF19A | 0.310811 | CYHR1 | 0.22973 |
| OR4M1 | 0.067568 | NCRNA00154 | 0.378378 | EFR3A | 0.256757 | WISP1 | 0.22973 |
| OR4K2 | 0.067568 | SYS1-DBNDD2 | 0.351351 | CRISPLD1 | 0.256757 | NDRG1 | 0.22973 |
| OR4K5 | 0.067568 | SRMP1 | 0.351351 | UTP23 | 0.22973 | ODF1 | 0.310811 |
| OR4K1 | 0.067568 | TOP3B | 0.081081 | ANKRD46 | 0.297297 | KLF10 | 0.310811 |
| C14orf17 | 0.067568 | IGLVI-70 | 0.081081 | HNF4G | 0.27027 | COL14A1 | 0.27027 |
| OR11K2P | 0.067568 | IGLV4-69 | 0.081081 | OC90 | 0.256757 | AZIN1 | 0.310811 |
| OR4H12P | 0.067568 | IGLVI-68 | 0.081081 | NKAIN3 | 0.256757 | ESRP1 | 0.283784 |
| OR4K6P | 0.067568 | IGLV10-67 | 0.081081 | HHLA1 | 0.256757 | ST3GAL1 | 0.256757 |
| MIR193B | 0.108108 | IGLVIV-66-1 | 0.081081 | ZFHX4 | 0.243243 | ZBTB10 | 0.283784 |

| | | | | | | | |
|---|---|---|---|---|---|---|---|
| MIR365-1 | 0.108108 | IGLVV-66 | 0.081081 | SNX31 | 0.297297 | ZFAT | 0.256757 |
| SHISA9 | 0.081081 | IGLVIV-65 | 0.081081 | KCNQ3 | 0.256757 | ATP6V1C1 | 0.310811 |
| ERCC4 | 0.108108 | IGLVIV-64 | 0.081081 | PABPC1 | 0.310811 | ZNF704 | 0.243243 |
| MKL2 | 0.094595 | IGLVI-63 | 0.081081 | MED30 | 0.22973 | ZNF7 | 0.202703 |
| MIR144 | 0.081081 | IGLV1-62 | 0.081081 | PEX2 | 0.243243 | MRPL13 | 0.243243 |
| MIR451 | 0.081081 | IGLV8-61 | 0.081081 | EXT1 | 0.27027 | C8orf56 | 0.310811 |
| C17orf63 | 0.081081 | IGLV4-60 | 0.081081 | PKIA | 0.283784 | MTBP | 0.243243 |
| ERAL1 | 0.081081 | IGLVIV-59 | 0.081081 | LRRC6 | 0.216216 | BAALC | 0.310811 |
| NUFIP2 | 0.081081 | IGLVV-58 | 0.081081 | FAM164A | 0.283784 | PMP2 | 0.283784 |
| TAOK1 | 0.081081 | IGLV6-57 | 0.081081 | IL7 | 0.283784 | SNTB1 | 0.310811 |
| ABHD15 | 0.081081 | IGLVI-56 | 0.081081 | SAMD12 | 0.256757 | FABP9 | 0.283784 |
| TP53I13 | 0.081081 | IGLV11-55 | 0.081081 | TNFRSF11B | 0.27027 | HAS2 | 0.324324 |
| GIT1 | 0.081081 | IGLV10-54 | 0.081081 | STMN2 | 0.256757 | FABP4 | 0.283784 |
| ANKRD13B | 0.081081 | IGLVIV-53 | 0.081081 | YWHAZ | 0.297297 | FZD6 | 0.310811 |
| CORO6 | 0.081081 | PRAMEL | 0.081081 | TMEM71 | 0.216216 | FABP12 | 0.283784 |
| SSH2 | 0.081081 | FAM108A6P | 0.081081 | COLEC10 | 0.27027 | COMMD5 | 0.202703 |
| TRAF4 | 0.081081 | SOCS2P2 | 0.081081 | NOV | 0.243243 | IMPA1 | 0.283784 |
| ZNF761 | 0.135135 | BMP6P1 | 0.081081 | ENPP2 | 0.283784 | ZNF250 | 0.202703 |
| TPM3P6 | 0.135135 | SPINK5 | 0.027027 | PHF20L1 | 0.216216 | ZHX2 | 0.27027 |
| ZNF813 | 0.148649 | SPINK14 | 0.027027 | ZNF706 | 0.27027 | CTHRC1 | 0.283784 |
| ZNF331 | 0.135135 | SNORA9 | 0.202703 | GRHL2 | 0.297297 | DERL1 | 0.22973 |
| GHRH | 0.337838 | SNORA5A | 0.202703 | TG | 0.22973 | SLC25A32 | 0.283784 |
| CTNNBL1 | 0.351351 | SNORA5C | 0.202703 | TAF2 | 0.283784 | DCAF13 | 0.283784 |
| KIAA1755 | 0.337838 | SNORA5B | 0.202703 | TPD52 | 0.22973 | WDR67 | 0.22973 |
| BPI | 0.337838 | RNU7-35P | 0.216216 | NCALD | 0.297297 | ZNF16 | 0.243243 |
| LBP | 0.337838 | DNAH11 | 0.216216 | DSCC1 | 0.27027 | SLC10A5 | 0.283784 |
| PTPRT | 0.297297 | RAMP3 | 0.202703 | DEPTOR | 0.27027 | RIMS2 | 0.243243 |
| TOX2 | 0.378378 | NACAD | 0.202703 | RRM2B | 0.283784 | ZNF252 | 0.243243 |
| JPH2 | 0.364865 | TBRG4 | 0.202703 | SLA | 0.22973 | KHDRBS3 | 0.202703 |
| MATN4 | 0.351351 | C7orf40 | 0.202703 | UBR5 | 0.310811 | C8orf77 | 0.243243 |
| RBPJL | 0.351351 | CCM2 | 0.202703 | ENY2 | 0.27027 | C8orf33 | 0.243243 |
| SDC4 | 0.351351 | GLCCI1 | 0.22973 | EYA1 | 0.27027 | CPA6 | 0.22973 |
| SYS1 | 0.351351 | ICA1 | 0.216216 | NDUFB9 | 0.297297 | C8orf38 | 0.202703 |
| TP53TG5 | 0.351351 | MYO1G | 0.202703 | DENND3 | 0.256757 | ZFAND1 | 0.283784 |
| DBNDD2 | 0.351351 | CDCA7L | 0.216216 | POP1 | 0.243243 | FAM135B | 0.243243 |
| PIGT | 0.351351 | AQP1 | 0.202703 | MTSS1 | 0.283784 | PREX2 | 0.256757 |
| WFDC2 | 0.351351 | STEAP1B | 0.216216 | PKHD1L1 | 0.27027 | FAM83A | 0.243243 |
| C20orf123 | 0.351351 | POU6F2 | 0.22973 | NIPAL2 | 0.256757 | TM7SF4 | 0.22973 |
| SLC13A3 | 0.351351 | HECW1 | 0.216216 | STK3 | 0.310811 | C8orf76 | 0.256757 |
| ZFP64 | 0.405405 | KIAA0087 | 0.216216 | NUDCD1 | 0.27027 | DPYS | 0.22973 |
| TSHZ2 | 0.364865 | CREB5 | 0.216216 | RSPO2 | 0.310811 | COL22A1 | 0.256757 |
| BCAS1 | 0.364865 | CHN2 | 0.216216 | TSPYL5 | 0.22973 | LRP12 | 0.22973 |
| MIR499 | 0.378378 | HECW1-IT1 | 0.216216 | MTDH | 0.216216 | ZHX1 | 0.256757 |
| MIR644 | 0.391892 | RNU7-67P | 0.256757 | LAPTM4B | 0.256757 | FAM83H | 0.243243 |
| EDEM2 | 0.378378 | RNU7-84P | 0.256757 | EIF3E | 0.310811 | TRAPPC2P2 | 0.27027 |
| PROCR | 0.378378 | RNY4P5 | 0.22973 | FER1L6 | 0.310811 | PRKRIRP7 | 0.283784 |
| MMP24 | 0.378378 | MIR1208 | 0.283784 | TMEM65 | 0.324324 | RPL3P9 | 0.256757 |
| EIF6 | 0.378378 | MIR548D1 | 0.256757 | TRMT12 | 0.310811 | RPSAP47 | 0.283784 |
| FAM83C | 0.378378 | MIR1204 | 0.310811 | RNF139 | 0.310811 | MCART5P | 0.243243 |
| DYNLRB1 | 0.391892 | MIR1205 | 0.283784 | TATDN1 | 0.310811 | CKS1BP7 | 0.243243 |
| MAP1LC3A | 0.391892 | MIR1207 | 0.283784 | TTC35 | 0.256757 | HMGB1P41 | 0.243243 |
| PIGU | 0.391892 | MIR30B | 0.243243 | TMEM74 | 0.27027 | BOP1 | 0.22973 |
| TP53INP2 | 0.378378 | MIR30D | 0.243243 | TRHR | 0.310811 | HSF1 | 0.22973 |

| | | | | | | | |
|---|---|---|---|---|---|---|---|
| NCOA6 | 0.378378 | MIR937 | 0.243243 | WDYHV1 | 0.256757 | DGAT1 | 0.22973 |
| GGT7 | 0.378378 | MIR939 | 0.22973 | C8orf17 | 0.202703 | PTP4A3 | 0.283784 |
| ACSS2 | 0.378378 | MIR1234 | 0.22973 | CHRAC1 | 0.189189 | SCRT1 | 0.22973 |
| GSS | 0.378378 | MIR2053 | 0.27027 | EIF2C2 | 0.22973 | GPR172A | 0.22973 |
| MYH7B | 0.378378 | MIR548A3 | 0.22973 | FBXO32 | 0.297297 | TSNARE1 | 0.216216 |
| TRPC4AP | 0.378378 | MIR1273 | 0.256757 | KLHL38 | 0.310811 | FBXL6 | 0.22973 |
| EBAG9 | 0.22973 | MIR875 | 0.283784 | ANXA13 | 0.310811 | BAI1 | 0.243243 |
| KCNS2 | 0.243243 | MIR599 | 0.283784 | ABRA | 0.256757 | ARC | 0.243243 |
| ZNF572 | 0.310811 | SLC45A4 | 0.243243 | PTK2 | 0.22973 | ADCK5 | 0.22973 |
| CPSF1 | 0.22973 | LY6H | 0.256757 | MAL2 | 0.27027 | TSTA3 | 0.22973 |
| PSCA | 0.256757 | ZNF707 | 0.243243 | RPL35AP19 | 0.256757 | LY6E | 0.256757 |
| LY6K | 0.256757 | GPIHBP1 | 0.22973 | MRPS36P3 | 0.256757 | ZNF623 | 0.243243 |
| C8orf55 | 0.256757 | ZFP41 | 0.256757 | HMGB1P19 | 0.22973 | AK3P2 | 0.256757 |
| SLURP1 | 0.256757 | GLI4 | 0.256757 | UBA52P5 | 0.256757 | C8orf31 | 0.256757 |
| LYPD2 | 0.256757 | ZNF696 | 0.256757 | DUTP2 | 0.256757 | C8orf51 | 0.283784 |
| LYNX1 | 0.27027 | TOP1MT | 0.283784 | IMPDH1P6 | 0.256757 | MTND2P7 | 0.256757 |
| LY6D | 0.27027 | CCDC166 | 0.243243 | FER1L6-AS1 | 0.310811 | MAPRE1P1 | 0.22973 |
| GML | 0.27027 | MAPK15 | 0.243243 | ARF1P3 | 0.310811 | TMCC1P1 | 0.27027 |
| CYP11B1 | 0.256757 | FTH1P11 | 0.283784 | RPL19P14 | 0.283784 | NCRNA00051 | 0.22973 |
| TIGD5 | 0.243243 | IMPA1P | 0.283784 | MRP63P7 | 0.27027 | JRK | 0.243243 |
| PYCRL | 0.243243 | NIPA2P4 | 0.283784 | GAPDHP62 | 0.297297 | HPYR1 | 0.216216 |
| CYP11B2 | 0.256757 | RPS26P34 | 0.283784 | RPS26P6 | 0.297297 | ST13P6 | 0.256757 |
| HNRNPA1P4 | 0.27027 | PVT1 | 0.310811 | RPS10P16 | 0.22973 | RPL5P24 | 0.310811 |
| TAGLN2P1 | 0.256757 | NACAP1 | 0.256757 | RPS26P35 | 0.243243 | MTND1P5 | 0.310811 |
| HMGB1P46 | 0.256757 | RPS12P15 | 0.310811 | RPS17P14 | 0.27027 | | |
| PGAM1P13 | 0.27027 | POU5F1P2 | 0.310811 | TPM3P3 | 0.243243 | | |

*Table 8 - Genes with significant copy number loss*

| GeneName | Freq. | GeneName | Freq. | GeneName | Freq. | GeneName | Freq. |
|---|---|---|---|---|---|---|---|
| ZNF29P | 0.216216 | APC | 0.189189 | CDH20 | 0.297297 | PBK | 0.175676 |
| CDRT15L1 | 0.216216 | MRO | 0.297297 | NEFL | 0.162162 | INTS10 | 0.243243 |
| IL6STP1 | 0.216216 | ME2 | 0.310811 | RNF152 | 0.297297 | FBXO16 | 0.189189 |
| MEIS3P1 | 0.216216 | ELAC1 | 0.297297 | PIGN | 0.297297 | FZD3 | 0.202703 |
| NCRNA00188 | 0.243243 | TRAPPC8 | 0.297297 | KIAA1468 | 0.310811 | EXTL3 | 0.189189 |
| HS3ST3A1 | 0.243243 | SMAD4 | 0.297297 | PHLPP1 | 0.297297 | RBFOX1 | 0.121622 |
| COX10 | 0.22973 | MEX3C | 0.283784 | ZNF521 | 0.297297 | IRF2 | 0.202703 |
| CDRT15 | 0.22973 | DCC | 0.364865 | VPS4B | 0.283784 | PPP2CB | 0.216216 |
| PMP22 | 0.216216 | MBD2 | 0.351351 | SERPINB7 | 0.27027 | CASP3 | 0.202703 |
| TEKT3 | 0.22973 | POLI | 0.351351 | SERPINB2 | 0.310811 | TEX15 | 0.22973 |
| MACROD2-AS1 | 0.189189 | STARD6 | 0.364865 | SERPINB10 | 0.310811 | PURG | 0.22973 |
| GAS7 | 0.243243 | C18orf54 | 0.364865 | HMSD | 0.310811 | WRN | 0.22973 |
| MYH13 | 0.216216 | C18orf26 | 0.324324 | SERPINB8 | 0.297297 | NRG1 | 0.202703 |
| TRIM16 | 0.216216 | RAB27B | 0.310811 | CHST9 | 0.297297 | CCDC111 | 0.202703 |
| ZNF286A | 0.216216 | KIAA1456 | 0.216216 | CDH7 | 0.405405 | MLF1IP | 0.202703 |
| TBC1D26 | 0.216216 | MTND4P7 | 0.22973 | CDH2 | 0.297297 | SORBS2 | 0.22973 |
| TTC19 | 0.22973 | RNF138 | 0.297297 | CDH19 | 0.391892 | MIR1539 | 0.243243 |
| DSEL | 0.418919 | ADAM3A | 0.283784 | ARHGEF10 | 0.175676 | MIR744 | 0.243243 |
| TMX3 | 0.364865 | SYT4 | 0.337838 | ADAMDEC1 | 0.216216 | MIR1288 | 0.22973 |
| CCDC102B | 0.405405 | SLC14A2 | 0.256757 | FHIT | 0.216216 | MIR1305 | 0.22973 |
| DOK6 | 0.391892 | SLC14A1 | 0.27027 | ADAM7 | 0.216216 | MIR596 | 0.175676 |
| CD226 | 0.364865 | PSTPIP2 | 0.283784 | CSMD1 | 0.256757 | MIR383 | 0.256757 |
| RTTN | 0.337838 | ATP5A1 | 0.283784 | NEFM | 0.162162 | MIR1261 | 0.22973 |
| SOCS6 | 0.324324 | HAUS1 | 0.283784 | RPL23AP53 | 0.202703 | SNORD58C | 0.243243 |
| CBLN2 | 0.364865 | DYM | 0.310811 | FAM87A | 0.202703 | SNORA37 | 0.324324 |
| NETO1 | 0.391892 | C18orf32 | 0.243243 | MCPH1 | 0.189189 | SNORD49B | 0.243243 |
| ZNF407 | 0.351351 | RPL17 | 0.243243 | ARHGAP28 | 0.216216 | SNORD49A | 0.243243 |
| GALR1 | 0.351351 | BHLHA9 | 0.216216 | ANGPT2 | 0.189189 | SNORD65 | 0.243243 |
| ATP9B | 0.27027 | TUSC5 | 0.216216 | HLA-H | 0.094595 | LONRF1 | 0.202703 |
| LSM12P1 | 0.189189 | SLC25A37 | 0.202703 | HLA-T | 0.148649 | DLC1 | 0.256757 |
| KIAA1328 | 0.310811 | OR4F21 | 0.202703 | DDX39BP1 | 0.148649 | C8orf48 | 0.256757 |
| ADAM5P | 0.283784 | ZNF596 | 0.202703 | MCCD1P1 | 0.148649 | SGCZ | 0.283784 |
| ADNP2 | 0.27027 | FBXO25 | 0.202703 | HLA-K | 0.135135 | PSD3 | 0.216216 |
| PARD6G | 0.27027 | C8orf42 | 0.202703 | DEFA6 | 0.202703 | CSGALNACT1 | 0.202703 |
| PIK3C3 | 0.337838 | ADAM28 | 0.216216 | PAICSP4 | 0.256757 | ESCO2 | 0.175676 |
| CHST9-AS1 | 0.310811 | ERICH1 | 0.202703 | MSRA | 0.22973 | ODZ3 | 0.22973 |
| RIT2 | 0.310811 | DLGAP2 | 0.202703 | RAP1GAP2 | 0.216216 | FUT10 | 0.189189 |
| CTSB | 0.189189 | NAT2 | 0.22973 | ROBO1 | 0.162162 | CADM2 | 0.162162 |
| CCDC110 | 0.22973 | UNC5D | 0.189189 | | | | |

Besides assessing expression, the RNA-seq data can be exploited to examine splicing patterns. Among the mutated genes there are several that carry somatic mutations in canonical splice sites that will likely affect their splicing. 112 genes were found with canonical splice site mutations that show evidence for splicing defects based on RNA-seq data. The affected genes include *TP53*, *NOTCH2* and *EIF5B* (Table 9). RNA-seq data was also used to analyze tumor specific expression of certain exons in gene coding regions. Two novel tumor specific exons upstream of the first 5'annotated exon of a mitochondrial large subunit *MRPL33* gene were identified (Figure 1). Analysis of this genomic region identified transcription factor binding sites 5' of these novel exons, further supporting our observation.

*Table 9 – Splice Site Mutation Effects*

| GeneName | Position | Ref. | Var. | GeneName | Position | Ref. | Var. | GeneName | Position | Ref. | Var. |
|---|---|---|---|---|---|---|---|---|---|---|---|
| TP53 | 7577157 | T | A | IVNS1ABP | 185274666 | A | G | PDXDC2 | 70072890 | T | C |
| EYA3 | 28369163 | T | C | EPRS | 220191851 | C | A | PRPF8 | 1554252 | T | G |
| RAD54L | 46739138 | G | A | KIF13B | 29024889 | C | A | TP53 | 7578555 | C | T |
| RAD54L | 46743654 | T | C | PKD1 | 2156679 | C | T | PER1 | 8050991 | C | T |
| TBCD | 80895237 | G | A | ASPHD1 | 29916287 | G | T | HDAC5 | 42155785 | T | C |
| MYO5B | 47380018 | C | A | INPP5K | 1417274 | C | A | MED16 | 871254 | C | A |
| ZNF780A | 40590706 | C | A | DUS3L | 5788189 | T | C | SAE1 | 47712415 | G | T |
| NAV1 | 201757595 | G | A | SFRS15 | 33078671 | C | T | TTC3L | 38572531 | A | G |
| EIF5B | 100010862 | G | A | PRKCZ | 2106661 | A | G | USP11 | 47099703 | G | T |
| KNTC1 | 123042146 | G | T | SLC2A5 | 9098566 | C | A | FANCC | 97887468 | C | A |
| ANKS1A | 35054827 | G | A | LEPRE1 | 43213085 | T | C | OTUD7B | 149949513 | T | C |
| IP6K2 | 48728917 | T | G | ARNT | 150790507 | C | A | C1orf9 | 172554157 | G | T |
| ATP13A1 | 19757157 | C | T | ARHGEF11 | 156915955 | C | A | SLC4A3 | 220500394 | G | T |
| YWHAQ | 9728458 | C | A | YWHAQ | 9731646 | T | C | CLASP2 | 33614847 | C | A |
| SETD2 | 47127805 | C | A | USP40 | 234451010 | C | A | LRRFIP2 | 37100402 | C | A |
| REEP5 | 112238216 | C | A | METTL6 | 15455670 | C | A | SLC2A9 | 9909970 | C | A |
| PHF19 | 123631609 | C | T | GLB1 | 33055803 | C | A | ACSL1 | 185678862 | T | C |
| TAF10 | 6632535 | C | A | USP19 | 49149716 | C | T | FAT1 | 187527368 | C | A |
| YES1 | 756836 | C | T | LPCAT1 | 1474801 | C | A | C5orf42 | 37125512 | C | A |
| LAMP2 | 119575751 | T | G | LHFPL2 | 77784977 | C | A | SFRS18 | 99858841 | C | A |
| SETD7 | 140439198 | T | C | SNX2 | 122153070 | T | G | FAM184A | 119332597 | C | A |
| FAM102A | 130707645 | C | T | AARS2 | 44278899 | C | A | PPP3CC | 22380264 | T | C |
| BRAP | 112093368 | A | G | PHIP | 79727301 | C | T | RAB11FIP1 | 37720632 | C | A |
| SEL1L3 | 25785913 | C | A | TECPR1 | 97863225 | T | C | CDH17 | 95143103 | C | T |
| TEC | 48140840 | C | A | TRAPPC9 | 141321346 | C | T | EXT1 | 119122323 | C | A |
| PTPRB | 70932795 | C | A | NAPRT1 | 144659348 | C | A | ALDH1A1 | 75527039 | C | A |
| TP53 | 7577156 | C | A | ANXA1 | 75778390 | A | G | DNLZ | 139256633 | C | A |
| NOTCH2 | 120529707 | T | C | PTCH1 | 98239040 | C | T | MTPAP | 30604966 | C | A |
| MRPS2 | 138393821 | T | C | PKN3 | 131475777 | G | A | TFAM | 60147949 | G | A |
| CORO1B | 67206140 | A | G | ZER1 | 131493674 | C | T | RSL1D1 | 11933550 | A | C |
| C2CD3 | 73768590 | C | T | DNMBP | 101667853 | T | C | GPCPD1 | 5545725 | C | A |
| ALG8 | 77820487 | C | A | SUV420H1 | 67953396 | C | A | CXADR | 18933019 | G | A |
| POLG | 89865248 | T | G | USP28 | 113683227 | C | A | KIF13A | 17799672 | T | C |
| LIMD2 | 61776073 | T | G | KIRREL3 | 126299185 | T | C | CELSR2 | 109815787 | G | A |
| VAPA | 9931961 | T | C | CHD4 | 6688084 | C | A | MTO1 | 74189850 | G | C |
| TFCP2 | 51497987 | C | A | CAPRIN2 | 30869611 | C | A | SOS2 | 50655420 | T | C |
| ABI3BP | 100469455 | C | A | CSAD | 53566434 | T | C | RPS10 | 34389506 | C | T |
| ABCD4 | 74753521 | T | G | PDS5B | 33347464 | T | C | XPNPEP1 | 111640599 | C | T |
| CNOT1 | 58573864 | C | T | SIN3A | 75682164 | T | C | | | | |

***Example 4- Recurrent R-spondin fusions activate Wnt pathway signaling***

RNA-seq data was next used to identify intra- and inter-chromosomal rearrangements such as gene fusions that occur in cancer genomes (Ozsolak, F. & Milos, P. M. Nature Rev Genet. 12:87-98 (2011)). In mapping the paired-end RNA-seq data, 36 somatic gene fusions, including two recurrent ones, were indentified in the analyzed CRC transcriptomes. The somatic nature of the fusions was established by confirming it presence in the tumors and absence in corresponding matched normal using RT-PCR. Further, all fusions reported in these examples were Sanger sequenced and validated (Table 10). The majority of predicted somatic fusions identified were intra-chromosomal (89%; 32/36).

***Table 10 - Gene Fusions***

| 5' GeneName | 3' GeneName | Type | Genomic position | 5' PCR primer | 3' PCR primer | bp |
|---|---|---|---|---|---|---|
| PVT1 | ENST00000502082 | intrachrom. | 8:128806980-8:128433074 | CTTGCGGAAAGGATGTTGG (SEQ ID NO:11) | TGGTGATCCAGAGAAGAAGC (SEQ ID NO:40) | 150 |
| EIF3E(e1) | RSPO2(e2) | deletion | 8:109260842-8:109095035 | ACTACTCGCATCGCGCACT (SEQ ID NO:12) | GGGAGGACTCAGAGGGAGAC (SEQ ID NO:41) | 155 |
| EIF3E(e1) | RSPO2(e2) | deletion | 8:109260842-8:109095035 | ACTACTCGCATCGCGCACT (SEQ ID NO:12) | GGGAGGACTCAGAGGGAGAC (SEQ ID NO:41) | 155 |
| EIF3E(e1) | RSPO2(e3) | deletion | 8:109260842-8:109001472 | ACTACTCGCATCGCGCACT (SEQ ID NO:12) | TGCAGGCACTCTCCATACTG (SEQ ID NO:42) | 205 |
| EIF3E(e1) | RSPO2(e3) | deletion | 8:109260842-8:109001472 | ACTACTCGCATCGCGCACT (SEQ ID NO:12) | TGCAGGCACTCTCCATACTG (SEQ ID NO:42) | 205 |
| PTPRK(e1) | RSPO3(e2) | inversion | 6:128841404-6:127469793 | AAACTCGGCATGGATACGAC (SEQ ID NO:13) | GCTTCATGCCAATTCTTTCC (SEQ ID NO:43) | 226 |
| PTPRK(e1) | RSPO3(e2) | inversion | 6:128841404-6:127469793 | AAACTCGGCATGGATACGAC (SEQ ID NO:13) | GCTTCATGCCAATTCTTTCC (SEQ ID NO:43) | 226 |
| PTPRK(e1) | RSPO3(e2) | inversion | 6:128841404-6:127469793 | AAACTCGGCATGGATACGAC (SEQ ID NO:13) | GCTTCATGCCAATTCTTTCC (SEQ ID NO:43) | 226 |
| PTPRK(e1) | RSPO3(e2) | inversion | 6:128841404-6:127469793 | AAACTCGGCATGGATACGAC (SEQ ID NO:13) | GCTTCATGCCAATTCTTTCC (SEQ ID NO:43) | 226 |
| PTPRK(e7) | RSPO3(e2) | inversion | 6:128505577-6:127469793 | TGCAGTCAATGCTCCAACTT (SEQ ID NO:14) | GCCAATTCTTTCCAGAGCAA (SEQ ID NO:44) | 250 |
| ETV6 | NTRK3 | translocation | 12: 12022903-15: 88483984 | AAGCCCATCAACCTCTCTCA (SEQ ID NO:15) | GGGCTGAGGTTGTAGCACTC (SEQ ID NO:45) | 206 |
| ANXA2 | RORA | intrachrom. | 15: 60674541-15: 60824050 | CTCTACACCCCCAAGTGCAT (SEQ ID NO:16) | TGACACCATAATGGATTCCTG (SEQ ID NO:46) | 164 |
| TUBGCP3 | PDS5B | inversion | 13: 113200013-13: 33327470 | AACAGGAGACCCGTACATGC (SEQ ID NO:17) | AAAGGGCACAGATTGCCATA (SEQ ID NO:47) | 221 |

(continued)

| 5' GeneName | 3' GeneName | Type | Genomic position | 5' PCR primer | 3' PCR primer | bp |
|---|---|---|---|---|---|---|
| ARHGEF18 | NCRNA00157 | translocation | 19:7460133-21: 19212970 | CCAGCTGCTAGCTACTGTGGA (SEQ ID NO:18) | ACTAGGTGGTCCAGGGTGTG (SEQ ID NO:48) | 186 |
| NT5C2 | ASAH2 | deletion | 10: 104899163-10: 51978390 | TGAACCGAAGTTTAGCAATGG (SEQ ID NO:19) | TGCTCAAGCAGGTAAGATGC (SEQ ID NO:49) | 156 |
| NRBP2 | VPS28 | intrachrom. | 8:144919211-8: 145649651 | TGATGAACTTTGCAGCCACT (SEQ ID NO:20) | ATGGTCTCCATCAGCTCTCG (SEQ ID NO:50) | 208 |
| CDC42SE2 | KIAA0146 | translocation | 5:130651837-8: 48612965 | AGGGCCAGATTTGAGTGTGT (SEQ ID NO:21) | AAACTGAAAATCCCCGCTGT (SEQ ID NO:51) | 188 |
| MED13L | LAG3 | inversion | 12: 116675273-12: 6886957 | GTGTATGGCGTCGTGATGTC (SEQ ID NO:22) | GCTCCAGTCACCAAAAGGAG (SEQ ID NO:52) | 205 |
| PEX5 | LOC389634 | inversion | 12:7362838-12: 8509737 | CATGTCGGAGAACATCTGGA (SEQ ID NO:23) | TGTGGAGTCTCTTGCGTGTC (SEQ ID NO:53) | 230 |
| PLCE1 | CYP2C19 | deletion | 10: 95792009-10: 96602594 | CCTTACTGCCTTGTGGGAGA (SEQ ID NO:24) | TGGGGATGAGGTCGATGTAT (SEQ ID NO:54) | 224 |
| TPM3 | NTRK1 | inversion | 1:154142876-1: 156844363 | CAGAGACCCGTGCTGAGTTT (SEQ ID NO:25) | CCAAAAGGTGTTTCGTCCTT (SEQ ID NO:55) | 124 |
| PAN3 | RFC3 | deletion | 13: 28752072-13: 34395269 | GACTTTGGTGCCCTCAACAT (SEQ ID NO:26) | CAATTTTTCCACTCCAACACC (SEQ ID NO:56) | 150 |
| CWC27 | RNF180 | intrachrom. | 5:64181373-5: 63665442 | AACGGGAACTCTTAGCAGCA (SEQ ID NO:27) | CATGTCAAACCACCATCCAC (SEQ ID NO:57) | 182 |
| CAPN1 | SPDYC | intrachrom. | 11: 64956217-11: 64939414 | GAGACTTCATGCGGGAGTTC (SEQ ID NO:28) | ATCTGGAAGCAGGGGTCTTT (SEQ ID NO:58) | 199 |
| COG8 | TERF2 | intrachrom. | 16: 69373079-16: 69391464 | TGGCCTTCGCTAACTACAAGA (SEQ ID NO:29) | TCCCCATATTTCTGCACTCC (SEQ ID NO:59) | 233 |

(continued)

| 5' GeneName | 3' GeneName | Type | Genomic position | 5' PCR primer | 3' PCR primer | bp |
|---|---|---|---|---|---|---|
| TADA2A | MEF2B | translocation | 17: 35767040-19: 19293492 | GCTCTTTGGCGCGGATTA (SEQ ID NO:30) | GGAGCTACCTGTGGCCCT (SEQ ID NO:60) | 152 |
| STRBP | DENND1A | intrachrom. | 9:125935956-9: 126220176 | GTTGCAAAAGGCTTGCTGAT (SEQ ID NO:31) | ACGAAGGCTTCCTCACAGAA (SEQ ID NO:61) | 155 |
| CXorf56 | UBE2A | inversion | X:118694231-X: 118717090 | TGATTGATGCTGCCAAACAT (SEQ ID NO:32) | CACGCTTTTCATATTCCCGT (SEQ ID NO:62) | 161 |
| MED13L | CD4 | inversion | 12: 116675273-12: 6923308 | GTGTATGGCGTCGTGATGTC (SEQ ID NO:22) | TCCCAAAGGCTTCTTCTTGA (SEQ ID NO:63) | 151 |
| PRR12 | PRRG2 | intrachrom. | 19: 50097872-19: 50093157 | ATGAACCTTATCTCGGCCCT (SEQ ID NO:33) | GTCGTGTACCCCAGAGGCT (SEQ ID NO:64) | 227 |
| ATP9A | ARFGEF2 | inversion | 20: 50307278-20: 47601266 | ATGTGTACGCAGAAGAGCCA (SEQ ID NO:34) | GTGCAGGAATTGGGCTATGT (SEQ ID NO:65) | 150 |
| ANKRD17 | HS3ST1 | deletion | 4:73956384-4: 11401737 | GGAAAATCCTCATATTTGCCA (SEQ ID NO:35) | AGCAGGGAAGCCTCCTAGTC (SEQ ID NO:66) | 158 |
| RBM47 | ATP8A1 | intrachrom. | 4:40517884-4: 42629126 | AGACCCAGGAGGAGTGAGGT (SEQ ID NO:36) | GGTCAGCCAGTGAGGTCTTC (SEQ ID NO:67) | 151 |
| FRS2 | RAP1B | intrachrom. | 12: 69924740-12: 69042479 | AGATGCCCAGATGCAAAAGT (SEQ ID NO:37) | CAAAGCAGACTTTCCAACGC (SEQ ID NO:68) | 161 |
| CHEK2 | PARVB | inversion | 22: 29137757-22: 44553862 | GGCTGAGGGTGGAGTTTGTA (SEQ ID NO:38) | CTTCTGATCGAAGCTTTCCG (SEQ ID NO:69) | 191 |
| SFI1 | TPST2 | inversion | 22: 31904362-22: 26940641 | CCCCAGTTAGAAGGGGAAGA (SEQ ID NO:39) | CACTCTCATCTCTGGGCTCC (SEQ ID NO:70) | 190 |

The recurrent fusions identified in these examples involve the R-spondin family members, *RSPO2* (3%; 2/68) and *RSPO3* (8%; 5/68; Figure 2A) found in MSS CRC samples. R-spondins are secreted proteins known to potentiate canonical Wnt signaling (Yoon, J. K. & Lee, J. S. Cell Signal. 24(2):369-77 (2012)), potentially by binding to the LGR family of GPCRs (Carmon, K. S. et al., Proceedings of the National Academy of Sciences of the United States of America 108:11452-11457 (2011); de Lau, W. et al., Nature 476:293-297 (2011); Glinka, A. et al., EMBO Reports 12:1055-1061 (2011)). The recurrent *RSPO2* fusion identified in two tumor samples involves *EIF3E* (eukaryotic translation initiation factor 3) exon 1 and RSPO2 exon 2 (Figure 2B). This fusion transcript was expected to produce a functional RSPO2 protein driven by *EIF3E* promoter (Figure 2D). A second *RSPO2* fusion detected in the same samples involves *EIF3E* exon 1 and *RSPO2* exon 3 (Table 10). However, this *EIF3E(e1)-RSPO2(e3)* was not expected to produce a functional protein. To confirm the nature of the alteration at the genome level, whole genome sequencing (WGS) of the tumors was performed containing RSPO2 fusions. Analysis of junction spanning reads, mate-pair reads and copy number data derived from the WGS data, identified a 158kb deletion in one sample and a 113kb deletion in the second sample, both of which places exon 1 of EIF3E in close proximity to the 5' end of RSPO2.

*RSPO3* translocations were observed in 5 of 68 tumors and they involve *PTPRK* (protein tyrosine kinase receptor kappa) as its 5' partner. WGS reads from the 5 tumors expressing the RSPO3 fusions showed rearrangements involving a simple (3 samples) or a complex (2 samples) inversion that places RSPO3 in proximity to PTPRK on the same strand as PTPRK on chromosome 6q. Two different *RSPO3* fusion variants were identified consisting either of exon 1 (e1) or exon 7 (e7) of *PTPRK* and exon 2 (e2) of *RSPO3* (Figure 3 and Figure 4). The *RSPO3* fusions likely arise from a deletion-inversion event at the chromosomal level as normally *PTPRK* and *RSPO3* are 850 Kb apart on opposing strands on chromosome 6q. The *PTPRK*(e1)-*RSPO3*(e2), found in four samples, was an in-frame fusion that preserves the entire coding sequence of *RSPO3* and replaces its secretion signal sequence with that of *PTPRK* (Figure 3C). The *PTPRK*(e7)-*RSPO3*(e2), detected in one sample, was also an in-frame fusion that encodes a ~70 KDa protein consisting of the first 387 amino acids of PTPRK, including its secretion signal sequence, and the RSPO3 amino acids 34-272 lacking its native signal peptide (Figure 4C). Interestingly, PTPRK contains a much stronger secretion signal sequence compared to RSPO3 and potentially leads to more efficient secretion of the fusion variants identified. Additionally, RNA-seq data showed that the mRNA expression of *RSPO2* and *RSPO3* in colon tumor samples containing the fusions was elevated compared to their matched normal samples and tumor samples lacking R-spondin fusions (Figure 2E). Further, all the RSPO positive fusion tumors expressed the potential R-spondin receptors LGR4/5/623-25, though LGR6 expression was lower compared to LGR4/5.

To determine if the predicted R-spondin fusion proteins were functional, expression constructs containing a C-terminal flag tag were generated and tested their expression following transfecting into mammalian 293T cells. Western blot analysis of the conditioned media showed that the fusion proteins were expressed and secreted (Figure 5A). The R-spondin fusion products were biologically active as determined by their ability to potentiate Wnt signaling using a Wnt luciferase reporter. As observed with the wildtype *RSPO2/3*, stimulation with conditioned media of cells transfected with RSPO fusion expression constructs led to activation of the Wnt luciferase reporter (Figure 5B) compared to that of control transfected cells. The observed activation, while apparent in the absence of exogenous WNT, was further potentiated in the presence of recombinant WNT, consistent with the known role of R-spondins in Wnt signaling (Carmon, K. S. et al., Proceedings of the National Academy of Sciences of the United States of America 108:11452-11457 (2011); de Lau, W. et al., Nature 476:293-297 (2011); Glinka, A. et al., EMBO Reports 12:1055-1061 (2011)).

To further characterize the *RSPO* gene fusions, *RSPO* gene fusions were analyzed in the context of mutations and other alterations that occur in components of cellular signaling pathways including the Wnt signaling cascade (Figure 6B). The *RSPO2* and *RSPO3* fusions were mutually exclusive between themselves, besides being mutually exclusive with *APC* mutations (Figure 5E), except for one sample that had a single copy deletion in the *APC* coding region (Figure 5E). Also, the *RSPO* gene fusions were mutually exclusive with *CTNNB1*, another Wnt pathway gene that was mutated in CRC. Further, all of the samples with *RSPO* gene fusions also carried mutation in *KRAS* or *BRAF* (Figure 6A). The majority of *APC* mutant samples had *RAS* pathway gene mutations, indicating that the *RSPO* gene fusions are likely to play the same role as *APC* mutations by promoting Wnt signaling during colon tumor development. In data not shown, tumors with RSPO gene fusions were shown to exhibit a WNT expression signature similar to that of APC mutant tumors indicating that R-Spondins can activate the WNT pathway in colon tumors in the absence of downstream WNT mutations. These findings indicate that the R-spondins likely function as drivers in human CRCs.

In these examples, an in-depth extensive genomic analysis of human primary colon tumors was reported. In sequencing and analyzing human CRC exomes and transcriptomes, multiple new recurrent somatic mutations were found. Many of the significantly mutated genes in these examples (APC, KRAS, PIK3CA, SMAD4, FBXW7, TP53, TCF7L2) agree with the previous findings. In addition, multiple mutations in 111 out of the 140 genes they highlighted in their study were reported. Further, 11 additional significant colon cancer genes including ATM and TMPRSS11A have been identified that have not been previously reported. The examples identified multiple hotspot containing genes including *TCF12* and *ERBB3.* The *ERBB3* oncogenic mutants identified here potentially provide new opportunities for therapeutic intervention in CRC. Combined analysis of expression and copy number data identified *IGF2* overexpression in a subset of our human CRC samples.

Finally, using RNA-seq data, new recurrent fusions involving R-spondins have been identified that occur at a frequency of approximately 10%. The fusions results in functional R-spondin proteins that potentiate Wnt signaling. R-spondins provide attractive targets for antibody based therapy in colon cancer patients that harbor them. Besides directly targeting R-spondins, other therapeutic strategies that block Wnt signaling will likely be effective against tumors positive for R-spondin fusions.

RSPO1 Nuclic Acid Sequence (SEQ ID NO:1)

ATGCGGCTTGGGCTGTGTGTGGTGGCCCTGGTTCTGAGCTGGACGCACCTCACCATCAGCAGCCGGGGGA
TCAAGGGGAAAAGGCAGAGGCGGATCAGTGCCGAGGGGAGCCAGGCCTGTGCCAAAGGCTGTGAGCTCTG
CTCTGAAGTCAACGGCTGCCTCAAGTGCTCACCCAAGCTGTTCATCCTGCTGGAGAGGAACGACATCCGC
CAGGTGGGCGTCTGCTTGCCGTCCTGCCCACCTGGATACTTCGACGCCCGCAACCCCGACATGAACAAGT
GCATCAAATGCAAGATCGAGCACTGTGAGGCCTGCTTCAGCCATAACTTCTGCACCAAGTGTAAGGAGGG
CTTGTACCTGCACAAGGGCCGCTGCTATCCAGCTTGTCCCGAGGGCTCCTCAGCTGCCAATGGCACCATG
GAGTGCAGTAGTCCTGCGCAATGTGAAATGAGCGAGTGGTCTCCGTGGGGGCCCTGCTCCAAGAAGCAGC
AGCTCTGTGGTTTCCGGAGGGGCTCCGAGGAGCGGACACGCAGGGTGCTACATGCCCCTGTGGGGGACCA
TGCTGCCTGCTCTGACACCAAGGAGACCCGGAGGTGCACAGTGAGGAGAGTGCCGTGTCCTGAGGGGCAG
AAGAGGAGGAAGGGAGGCCAGGGCCGGCGGGAGAATGCCAACAGGAACCTGGCCAGGAAGGAGAGCAAGG
AGGCGGGTGCTGGCTCTCGAAGACGCAAGGGGCAGCAACAGCAGCAGCAGCAAGGGACAGTGGGGCCACT
CACATCTGCAGGGCCTGCCTAG

RSPO1 Amino Acid Sequence (SEQ ID NO:2)

MRLGLCVVALVLSWTHLTISSRGIKGKRQRRISAEGSQACAKGCELCSEVNGCLKCSPKLFILLERNDIR
QVGVCLPSCPPGYFDARNPDMNKCIKCKIEHCEACFSHNFCTKCKEGLYLHKGRCYPACPEGSSAANGTM
ECSSPAQCEMSEWSPWGPCSKKQQLCGFRRGSEERTRRVLHAPVGDHAACSDTKETRRCTVRRVPCPEGQ
KRRKGGQGRRENANRNLARKESKEAGAGSRRRKGQQQQQQGTVGPLTSAGPA

RSPO2 Nucleic Acid Sequence (SEQ ID NO:3)

ATGCAGTTTCGCCTTTTCTCCTTTGCCCTCATCATTCTGAACTGCATGGATTACAGCCACTGCCAAGGCA
ACCGATGGAGACGCAGTAAGCGAGCTAGTTATGTATCAAATCCCATTTGCAAGGGTTGTTTGTCTTGTTC
AAAGGACAATGGGTGTAGCCGATGTCAACAGAAGTTGTTCTTCTTCCTTCGAAGAGAAGGGATGCGCCAG
TATGGAGAGTGCCTGCATTCCTGCCCATCCGGGTACTATGGACACCGAGCCCCAGATATGAACAGATGTG
CAAGATGCAGAATAGAAAACTGTGATTCTTGCTTTAGCAAAGACTTTTGTACCAAGTGCAAAGTAGGCTT
TTATTTGCATAGAGGCCGTTGCTTTGATGAATGTCCAGATGGTTTTGCACCATTAGAAGAAACCATGGAA
TGTGTGGAAGGATGTGAAGTTGGTCATTGGAGCGAATGGGGAACTTGTAGCAGAAATAATCGCACATGTG
GATTTAAATGGGGTCTGGAAACCAGAACACGGCAAATTGTTAAAAAGCCAGTGAAAGACACAATACTGTG
TCCAACCATTGCTGAATCCAGGAGATGCAAGATGACAATGAGGCATTGTCCAGGAGGGAAGAGAACACCA
AAGGCGAAGGAGAAGAGGAACAAGAAAAAGAAAAGGAAGCTGATAGAAAGGGCCCAGGAGCAACACAGCG
TCTTCCTAGCTACAGACAGAGCTAACCAATAA

RSPO2 Amino Acid Sequence (SEQ ID NO:4)

MQFRLFSFALIIILNCMDYSHCQGNRWRRSKRASYVSNPICKGCLSCSKDNGCSRCQQKLFFFLRREGMRQ
YGECLHSCPSGYYGHRAPDMNRCARCRIENCDSCFSKDFCTKCKVGFYLHRGRCFDECPDGFAPLEETME
CVEGCEVGHWSEWGTCSRNNRTCGFKWGLETRTRQIVKKPVKDTILCPTIAESRRCKMTMRHCPGGKRTP
KAKEKRNKKKKRKLIERAQEQHSVFLATDRANQ

RSPO3 Nucleic Acid Sequence (SEQ ID NO:5)

ATGCACTTGCGACTGATTTCTTGGCTTTTTATCATTTTGAACTTTATGGAATACATCGGCAGCCAAAACG
CCTCCCGGGGAAGGCGCCAGCGAAGAATGCATCCTAACGTTAGTCAAGGCTGCCAAGGAGGCTGTGCAAC
ATGCTCAGATTACAATGGATGTTTGTCATGTAAGCCCAGACTATTTTTTGCTCTGGAAAGAATTGGCATG
AAGCAGATTGGAGTATGTCTCTCTTCATGTCCAAGTGGATATTATGGAACTCGATATCCAGATATAAATA
AGTGTACAAAATGCAAAGCTGACTGTGATACCTGTTTCAACAAAAATTTCTGCACAAAATGTAAAAGTGG
ATTTTACTTACACCTTGGAAAGTGCCTTGACAATTGCCCAGAAGGGTTGGAAGCCAACAACCATACTATG
GAGTGTGTCAGTATTGTGCACTGTGAGGTCAGTGAATGGAATCCTTGGAGTCCATGCACGAAGAAGGGAA
AAACATGTGGCTTCAAAAGAGGGACTGAAACACGGGTCCGAGAAATAATACAGCATCCTTCAGCAAAGGG
TAACCTGTGTCCCCCAACAAATGAGACAAGAAAGTGTACAGTGCAAAGGAAGAAGTGTCAGAAGGGAGAA
CGAGGAAAAAAAGGAAGGGAGAGGAAAAGAAAAAAACCTAATAAAGGAGAAAGTAAAGAAGCAATACCTG

ACAGCAAAAGTCTGGAATCCAGCAAAGAAATCCCAGAGCAACGAGAAAACAAACAGCAGCAGAAGAAGCG
AAAAGTCCAAGATAAACAGAAATCGGTATCAGTCAGCACTGTACACTAG

RSPO3 Amino Acid Sequence (SEQ ID NO:6)

MHLRLISWLFIILNFMEYIGSQNASRGRRQRRMHPNVSQGCQGGCATCSDYNGCLSCKPRLFFALERIGM
KQIGVCLSSCPSGYYGTRYPDINKCTKCKADCDTCFNKNFCTKCKSGFYLHLGKCLDNCPEGLEANNHTM
ECVSIVHCEVSEWNPWSPCTKKGKTCGFKRGTETRVREIIQHPSAKGNLCPPTNETRKCTVQRKKCQKGE
RGKKGRERK

RSPO4 Nucleic Acid Sequence (SEQ ID NO:7)

ATGCGGGCGCCACTCTGCCTGCTCCTGCTCGTCGCCCACGCCGTGGACATGCTCGCCCTGAACCGAAGGA
AGAAGCAAGTGGGCACTGGCCTGGGGGGCAACTGCACAGGCTGTATCATCTGCTCAGAGGAGAACGGCTG
TTCCACCTGCCAGCAGAGGCTCTTCCTGTTCATCCGCCGGGAAGGCATCCGCCAGTACGGCAAGTGCCTG
CACGACTGTCCCCCTGGGTACTTCGGCATCCGCGGCCAGGAGGTCAACAGGTGCAAAAAATGTGGGGCCA
CTTGTGAGAGCTGCTTCAGCCAGGACTTCTGCATCCGGTGCAAGAGGCAGTTTTACTTGTACAAGGGGAA
GTGTCTGCCCACCTGCCCGCCGGGCACTTTGGCCCACCAGAACACACGGGAGTGCCAGGGGGAGTGTGAA
CTGGGTCCCTGGGGCGGCTGGAGCCCCTGCACACACAATGGAAAGACCTGCGGCTCGGCTTGGGGCCTGG
AGAGCCGGGTACGAGAGGCTGGCCGGGCTGGGCATGAGGAGGCAGCCACCTGCCAGGTGCTTTCTGAGTC
AAGGAAATGTCCCATCCAGAGGCCCTGCCCAGGAGAGAGGAGCCCCGGCCAGAAGAAGGGCAGGAAGGAC
CGGCGCCCACGCAAGGACAGGAAGCTGGACCGCAGGCTGGACGTGAGGCCGCGCCAGCCCGGCCTGCAGC
CCTGA

RSPO4 Amino Acid Sequence (SEQ ID NO:8)

MRAPLCLLLLVAHAVDMLALNRRKKQVGTGLGGNCTGCIICSEENGCSTCQQRLFLFIRREGIRQYGKCL
HDCPPGYFGIRGQEVNRCKKCGATCESCFSQDFCIRCKRQFYLYKGKCLPTCPPGTLAHQNTRECQGECE
LGPWGGWSPCTHNGKTCGSAWGLESRVREAGRAGHEEAATCQVLSESRKCPIQRPCPGERSPGQKKGRKD
RRPRKDRKLDRRLDVRPRQPGLQP

EIF3E(e1)-RSPO2(e2) translocation fusion polynucleotide (SEQ ID NO:74)

GAGCACAGACTCCCTTTTCTTTGGCAAGATGGCGGAGTACGACTTGACTACTCGCATCGCGCACTTTTTG
GATCGGCATCTAGTCTTTCCGCTTCTTGAATTTCTCTCTGTAAAGGAGGTTCGTGGCGGAGAGATGCTGA
TCGCGCTGAACTGACCGGTGCGGCCCGGGGGTGAGTGGCGAGTCTCCCTCTGAGTCCTCCCCAGCAGCGC
GGCCGGCGCCGGCTCTTTGGGCGAACCCTCCAGTTCCTAGACTTTGAGAGGCGTCTCTCCCCCGCCCGAC
CGCCCAGATGCAGTTTCGCCTTTTCTCCTTTGCCCTCATCATTCTGAACTGCATGGATTACAGCCACTGC
CAAGGCAACCGATGGAGACGCAGTAAGCGAGCTAGTTATGTATCAAATCCCATTTGCAAGGGTTGTTTGT
CTTGTTCAAAGGACAATGGGTGTAGCCGATGTCAACAGAAGTTGTTCTTCTTCCTTCGAAGAGAAGGGAT
GCGCCAGTATGGAGAGTGCCTGCATTCCTGCCCATCCGGGTACTATGGACACCGAGCCCCAGATATGAAC
AGATGTGCAAGATGCAGAATAGAAAACTGTGATTCTTGCTTTAGCAAAGACTTTTGTACCAAGTGCAAAG
TAGGCTTTTATTTGCATAGAGGCCGTTGCTTTGATGAATGTCCAGATGGTTTTGCACCATTAGAAGAAAC
CATGGAATGTGTGGAAGGATGTGAAGTTGGTCATTGGAGCGAATGGGGAACTTGTAGCAGAAATAATCGC
ACATGTGGATTTAAATGGGGTCTGGAAACCAGAACACGGCAAATTGTTAAAAAGCCAGTGAAAGACACAA
TACTGTGTCCAACCATTGCTGAATCCAGGAGATGCAAGATGACAATGAGGCATTGTCCAGGAGGGAAGAG
AACACCAAAGGCGAAGGAGAAGAGGAACAAGAAAAAGAAAAGGAAGCTGATAGAAAGGGCCCAGGAGCAA
CACAGCGTCTTCCTAGCTACAGACAGAGCTAACCAATAA

EIF3E(e1)-RSPO2(e2) translocation fusion polypeptide sequence (SEQ ID NO:75)

MAEYDLTTRIAHFLDRHLVFPLLEFLSVKEVRGGEMLIALNMQFRLFSFALIIILNCMDYSHCQGNRWRRS
KRASYVSNPICKGCLSCSKDNGCSRCQQKLFFFLRREGMRQYGECLHSCPSGYYGHRAPDMNRCARCRIE
NCDSCFSKDFCTKCKVGFYLHRGRCFDECPDGFAPLEETMECVEGCEVGHWSEWGTCSRNNRTCGFKWGL
ETRTRQIVKKPVKDTILCPTIAESRRCKMTMRHCPGGKRTPKAKEKRNKKKKRKLIERAQEQHSVFLATD
RANQ

PTPRK(e1)-RSPO3(e2) translocation fusion polynucleotide sequence (SEQ ID NO:76)

ATGGATACGACTGCGGCGGCGGCGCTGCCTGCTTTTGTGGCGCTCTTGCTCCTCTCTCCTTGGCCTCTCC
TGGGATCGGCCCAAGGCCAGTTCTCCGCAGTGCATCCTAACGTTAGTCAAGGCTGCCAAGGAGGCTGTGC
AACATGCTCAGATTACAATGGATGTTTGTCATGTAAGCCCAGACTATTTTTTGCTCTGGAAAGAATTGGC
ATGAAGCAGATTGGAGTATGTCTCTCTTCATGTCCAAGTGGATATTATGGAACTCGATATCCAGATATAA

ATAAGTGTACAAAATGCAAAGCTGACTGTGATACCTGTTTCAACAAAAATTTCTGCACAAAATGTAAAAG
TGGATTTTACTTACACCTTGGAAAGTGCCTTGACAATTGCCCAGAAGGGTTGGAAGCCAACAACCATACT
ATGGAGTGTGTCAGTATTGTGCACTGTGAGGTCAGTGAATGGAATCCTTGGAGTCCATGCACGAAGAAGG
GAAAAACATGTGGCTTCAAAAGAGGGACTGAAACACGGGTCCGAGAAATAATACAGCATCCTTCAGCAAA
GGGTAACCTGTGTCCCCCAACAAATGAGACAAGAAAGTGTACAGTGCAAAGGAAGAAGTGTCAGAAGGGA
GAACGAGGAAAAAAAGGAAGGGAGAGGAAAAGAAAAAAACCTAATAAAGGAGAAAGTAAAGAAGCAATAC
CTGACAGCAAAAGTCTGGAATCCAGCAAAGAAATCCCAGAGCAACGAGAAAACAAACAGCAGCAGAAGAA
GCGAAAAGTCCAAGATAAACAGAAATCGGTATCAGTCAGCACTGTACACTAG

PTPRK(e1)-RSPO3(e2) translocation fusion polypeptide sequence (SEQ ID NO:77)

MDTTAAAALPAFVALLLLSPWPLLGSAQGQFSAVHPNVSQGCQGGCATCSDYNGCLSCKPRLFFALERIG
MKQIGVCLSSCPSGYYGTRYPDINKCTKCKADCDTCFNKNFCTKCKSGFYLHLGKCLDNCPEGLEANNHT
MECVSIVHCEVSEWNPWSPCTKKGKTCGFKRGTETRVREIIQHPSAKGNLCPPTNETRKCTVQRKKCQKG
ERGKKGR

PTPRK(e7)-RSPO3(e2) translocation fusion polynucleotide sequence (SEQ ID NO:78)

ATGGATACGACTGCGGCGGCGGCGCTGCCTGCTTTTGTGGCGCTCTTGCTCCTCTCTCCTTGGCCTCTCC
TGGGATCGGCCCAAGGCCAGTTCTCCGCAGGTGGCTGTACTTTTGATGATGGTCCAGGGGCCTGTGATTA
CCACCAGGATCTGTATGATGACTTTGAATGGGTGCATGTTAGTGCTCAAGAGCCTCATTATCTACCACCC
GAGATGCCCCAAGGTTCCTATATGATAGTGGACTCTTCAGATCACGACCCTGGAGAAAAAGCCAGACTTC
AGCTGCCTACAATGAAGGAGAACGACACTCACTGCATTGATTTCAGTTACCTATTATATAGCCAGAAAGG
ACTGAATCCTGGCACTTTGAACATATTAGTTAGGGTGAATAAAGGACCTCTTGCCAATCCAATTTGGAAT
GTGACTGGATTCACGGGTAGAGATTGGCTTCGGGCTGAGCTAGCAGTGAGCACCTTTTGGCCCAATGAAT
ATCAGGTAATATTTGAAGCTGAAGTCTCAGGAGGGAGAAGTGGTTATATTGCCATTGATGACATCCAAGT
ACTGAGTTATCCTTGTGATAAATCTCCTCATTTCCTCCGTCTAGGGGATGTAGAGGTGAATGCAGGGCAA
AACGCTACATTTCAGTGCATTGCCACAGGGAGAGATGCTGTGCATAACAAGTTATGGCTCCAGAGACGAA
ATGGAGAAGATATACCAGTAGCCCAGACTAAGAACATCAATCATAGAAGGTTTGCCGCTTCCTTCAGATT
GCAAGAAGTGACAAAAACTGACCAGGATTTGTATCGCTGTGTAACTCAGTCAGAACGAGGTTCCGGTGTG
TCCAATTTTGCTCAACTTATTGTGAGAGAACCGCCAAGACCCATTGCTCCTCCTCAGCTTCTTGGTGTTG
GGCCTACATATTTGCTGATCCAACTAAATGCCAACTCGATCATTGGCGATGGTCCTATCATCCTGAAAGA
AGTAGAGTACCGAATGACATCAGGATCCTGGACAGAAACCCATGCAGTCAATGCTCCAACTTACAAATTA
TGGCATTTAGATCCAGATACCGAATATGAGATCCGAGTTCTACTTACAAGACCTGGTGAAGGTGGAACGG
GGCTCCCAGGACCTCCACTAATCACCAGAACAAAATGTGCAGTGCATCCTAACGTTAGTCAAGGCTGCCA
AGGAGGCTGTGCAACATGCTCAGATTACAATGGATGTTTGTCATGTAAGCCCAGACTATTTTTTGCTCTG
GAAAGAATTGGCATGAAGCAGATTGGAGTATGTCTCTCTTCATGTCCAAGTGGATATTATGGAACTCGAT
ATCCAGATATAAATAAGTGTACAAAATGCAAAGCTGACTGTGATACCTGTTTCAACAAAAATTTCTGCAC
AAAATGTAAAAGTGGATTTTACTTACACCTTGGAAAGTGCCTTGACAATTGCCCAGAAGGGTTGGAAGCC
AACAACCATACTATGGAGTGTGTCAGTATTGTGCACTGTGAGGTCAGTGAATGGAATCCTTGGAGTCCAT
GCACGAAGAAGGGAAAAACATGTGGCTTCAAAAGAGGGACTGAAACACGGGTCCGAGAAATAATACAGCA
TCCTTCAGCAAAGGGTAACCTGTGTCCCCCAACAAATGAGACAAGAAAGTGTACAGTGCAAAGGAAGAAG
TGTCAGAAGGGAGAACGAGGAAAAAAAGGAAGGGAGAGGAAAAGAAAAAAACCTAATAAAGGAGAAAGTA
AAGAAGCAATACCTGACAGCAAAAGTCTGGAATCCAGCAAAGAAATCCCAGAGCAACGAGAAAACAAACA
GCAGCAGAAGAAGCGAAAAGTCCAAGATAAACAGAAATCGGTATCAGTCAGCACTGTACACTAG

PTPRK(e7)-RSPO3(e2) translocation fusion polypeptide sequence (SEQ ID NO:79)

MDTTAAAALPAFVALLLLSPWPLLGSAQGQFSAGGCTFDDGPGACDYHQDLYDDFEWVHVSAQEPHYLPP
EMPQGSYMIVDSSDHDPGEKARLQLPTMKENDTHCIDFSYLLYSQKGLNPGTLNILVRVNKGPLANPIWN
VTGFTGRDWLRAELAVSTFWPNEYQVIFEAEVSGGRSGYIAIDDIQVLSYPCDKSPHFLRLGDVEVNAGQ
NATFQCIATGRDAVHNKLWLQRRNGEDIPVAQTKNINHRRFAASFRLQEVTKTDQDLYRCVTQSERGSGV
SNFAQLIVREPPRPIAPPQLLGVGPTYLLIQLNANSIIGDGPIILKEVEYRMTSGSWTETHAVNAPTYKL
WHLDPDTEYEIRVLLTRPGEGGTGLPGPPLITRTKCAVHPNVSQGCQGGCATCSDYNGCLSCKPRLFFAL
ERIGMKQIGVCLSSCPSGYYGTRYPDINKCTKCKADCDTCFNKNFCTKCKSGFYLHLGKCLDNCPEGLEA
NNHTMECVSIVHCEVSEWNPWSPCTKKGKTCGFKRGTETRVREIIQHPSAKGNLCPPTNETRKCTVQRKK
CQKGERGKKGRERKRKKPNKGESKEAIPDSKSLESSKEIPEQRENKQQQKKRKVQDKQKSVSVSTVH

**Claims**

1. An antibody which binds to R-spondin 3 (RSPO3) for use in treating a cancer cell, wherein the cancer cell comprises an RSPO3 translocation.

2. An antibody which binds to R-spondin 3 (RSPO3) for use in treating cancer, wherein the cancer comprises an RSPO3 translocation.

3. The antibody for the use as defined in claim 1 or 2, wherein the RSPO3 translocation comprises *PTPRK* and *RSPO3.*

4. The antibody for the use as defined in claim 1 or 2, wherein the RSPO3 translocation comprises *PTPRK* exon 1 and *RSPO3* exon 2.

5. The antibody for the use as defined in claim 1 or 2, wherein the RSPO3 translocation comprises *PTPRK* exon 7 and *RSPO3* exon 2.

6. The antibody for the use as defined in claim 1 or 2, wherein the RSPO3 translocation comprises SEQ ID NO:72 and/or SEQ ID NO:73.

7. The antibody for the use as defined in any one of claims 1-6, wherein the R-spondin translocation is detected at the chromosomal level, DNA level, RNA level, and/or protein level.

8. The antibody for the use as defined in any one of claims 1-7, wherein the cancer or cancer cell is colorectal cancer, colon cancer or rectal cancer.

9. The antibody for the use as defined in any one of claims 1-8, wherein the antibody is an isolated monoclonal antibody which binds RSPO3.

10. The antibody for the use as defined in any one of claims 1-9, wherein the antibody is an R-spondin-translocation antagonist.

11. The antibody for the use as defined in any one of claims 1-10, wherein an additional therapeutic agent is to be administered.

12. A method of identifying an individual with cancer who is more or less likely to exhibit benefit from treatment with an anti-cancer therapy comprising an antibody which binds to RSPO3, the method comprising: determining presence or absence of an RSPO3 translocation in a sample obtained from the individual, wherein presence of the RSPO3 translocation in the sample indicates that the individual is more likely to exhibit benefit from treatment with the anti-cancer therapy comprising the antibody which binds to RSPO3 or absence of the RSPO3 translocation in the sample indicates that the individual is less likely to exhibit benefit from treatment with the anti-cancer therapy comprising the antibody which binds to RSPO3.

**Patentansprüche**

1. Antikörper, der an R-Spondin 3 (RSPO3) bindet, zur Verwendung beim Behandeln einer Krebszelle, wobei die Krebszelle eine RSPO3-Translokation umfasst.

2. Antikörper, der an R-Spondin 3 (RSPO3) bindet, zur Verwendung beim Behandeln von Krebs, wobei der Krebs eine RSPO3-Translokation umfasst.

3. Antikörper zur Verwendung nach der Definition in Anspruch 1 oder 2, wobei die RSPO3-Translokation *PTPRK* und *RSPO3* umfasst.

4. Antikörper zur Verwendung nach der Definition in Anspruch 1 oder 2, wobei die RSPO3-Translokation das *PTPRK*-Exon 1 und das *RSPO3*-Exon 2 umfasst.

5. Antikörper zur Verwendung nach der Definition in Anspruch 1 oder 2, wobei die RSPO3-Translokation das *PTPRK*-Exon 7 und das *RSPO3*-Exon 2 umfasst.

6. Antikörper zur Verwendung nach der Definition in Anspruch 1 oder 2, wobei die RSPO3-Translokation SEQ ID NO:72 und/oder SEQ ID NO:73 umfasst.

7. Antikörper zur Verwendung nach der Definition in einem der Ansprüche 1-6, wobei die R-Spondin-Translokation auf chromosomaler Ebene, DNS-Ebene, RNS-Ebene und/oder Proteinebene nachgewiesen wird.

8. Antikörper zur Verwendung nach der Definition in einem der Ansprüche 1-7, wobei der Krebs oder die Krebszelle Dickdarmkrebs, Kolonkrebs oder Rektumkrebs ist.

9. Antikörper zur Verwendung nach der Definition in einem der Ansprüche 1-8, wobei der Antikörper ein isolierter monoklonaler Antikörper ist, der RSPO3 bindet.

10. Antikörper zur Verwendung nach der Definition in einem der Ansprüche 1-9, wobei der Antikörper ein R-Spondin-Translokationsantagonist ist.

11. Antikörper zur Verwendung nach der Definition in einem der Ansprüche 1-10, wobei ein zusätzliches therapeutisches Mittel zu verabreichen ist.

12. Verfahren zum Identifizieren eines Individuums mit Krebs, das mehr oder weniger wahrscheinlich von einer Behandlung mit einer Antikrebstherapie umfassend einen Antikörper, der an RSPO3 bindet, profitieren wird, wobei das Verfahren Folgendes umfasst: das Bestimmen des Vorhandenseins oder des Nichtvorhandenseins einer RSPO3-Translokation in einer Probe, die von dem Individuum erhalten worden ist, wobei das Vorhandensein der RSPO3-Translokation in der Probe darauf hinweist, dass das Individuum mit größerer Wahrscheinlichkeit von einer Behandlung mit der Antikrebstherapie umfassend den Antikörper, der an RSPO3 bindet, profitieren wird, oder das Nichtvorhandensein der RSPO3-Translokation in der Probe darauf hinweist, dass das Individuum mit geringerer Wahrscheinlichkeit von einer Behandlung mit der Antikrebstherapie umfassend den Antikörper, der an RSPO3 bindet, profitieren wird.

**Revendications**

1. Anticorps qui se lie à la R-spondine 3 (RSPO3) pour une utilisation dans le traitement d'une cellule cancéreuse, dans lequel la cellule cancéreuse comprend une translocation de la RSPO3.

2. Anticorps qui se lie à la R-spondine 3 (RSPO3) pour une utilisation dans le traitement d'un cancer, dans lequel le cancer comprend une translocation de la RSPO3.

3. Anticorps pour une utilisation selon la revendication 1 ou 2, dans lequel la translocation de la RSPO3 comprend la *PTPRK* et la *RSPO3.*

4. Anticorps pour une utilisation selon la revendication 1 ou 2, dans lequel la translocation de la RSPO3 comprend l'exon 1 de la *PTPRK* et l'exon 2 de la *RSPO3.*

**5.** Anticorps pour une utilisation selon la revendication 1 ou 2, dans lequel la translocation de la RSPO3 comprend l'exon 7 de la *PTPRK* et l'exon 2 de la *RSPO3.*

**6.** Anticorps pour une utilisation selon la revendication 1 ou 2, dans lequel la translocation de la RSPO3 comprend la SEQ ID NO: 72 et/ou la SEQ ID NO: 73.

**7.** Anticorps pour une utilisation selon l'une quelconque des revendications 1 à 6, dans lequel la translocation de la R-spondine est détectée au niveau chromosomique, au niveau de l'ADN, au niveau de l'ARN et/ou au niveau protéique.

**8.** Anticorps pour une utilisation selon l'une quelconque des revendications 1 à 7, dans lequel le cancer ou la cellule cancéreuse est un cancer colorectal, un cancer du colon ou un cancer du rectum.

**9.** Anticorps pour une utilisation selon l'une quelconque des revendications 1 à 8, l'anticorps étant un anticorps monoclonal isolé qui se lie à la RSPO3.

**10.** Anticorps pour une utilisation selon l'une quelconque des revendications 1 à 9, l'anticorps étant un antagoniste de translocation de la R-spondine.

**11.** Anticorps pour une utilisation selon l'une quelconque des revendications 1 à 10, dans lequel un agent thérapeutique supplémentaire doit être administré.

**12.** Procédé d'identification d'un individu atteint d'un cancer qui est plus ou moins susceptible de tirer un bienfait du traitement par une thérapie anticancéreuse comprenant un anticorps qui se lie à la RSPO3, le procédé comprenant : la détermination de la présence ou de l'absence d'une translocation de la RSPO3 dans un échantillon obtenu à partir de l'individu, dans lequel la présence de la translocation de la RSPO3 dans l'échantillon indique que l'individu est plus susceptible de tirer un bienfait du traitement par la thérapie anticancéreuse comprenant l'anticorps qui se lie à la RSPO3 ou l'absence de la translocation de la RSPO3 dans l'échantillon indique que l'individu est moins susceptible de tirer un bienfait du traitement par la thérapie anticancéreuse comprenant l'anticorps qui se lie à la RSPO3.

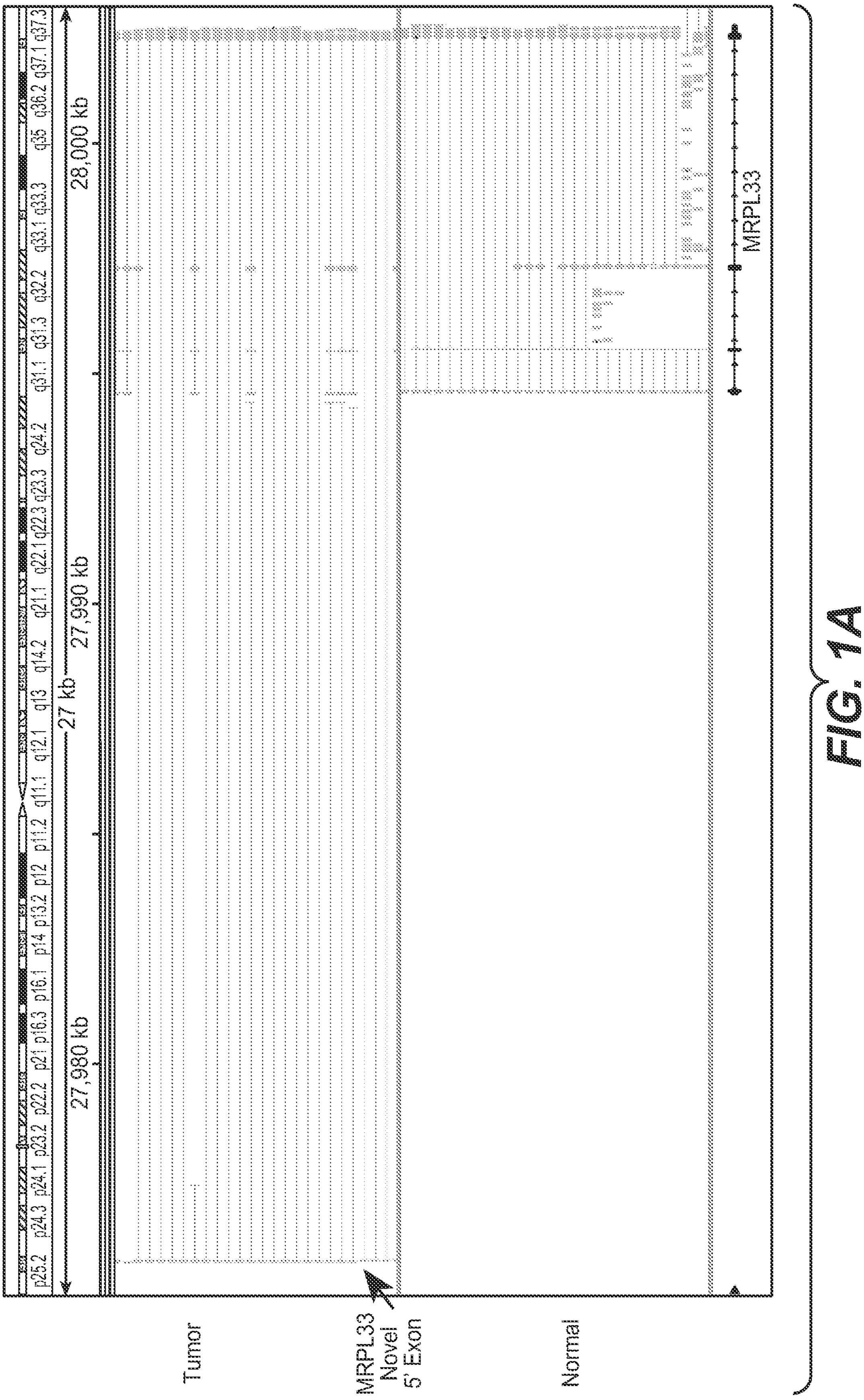
27,980 kb
27 kb
27,990 kb
28,000 kb
MRPL33
Tumor
MRPL33
Novel
5' Exon
Normal

FIG. 1A

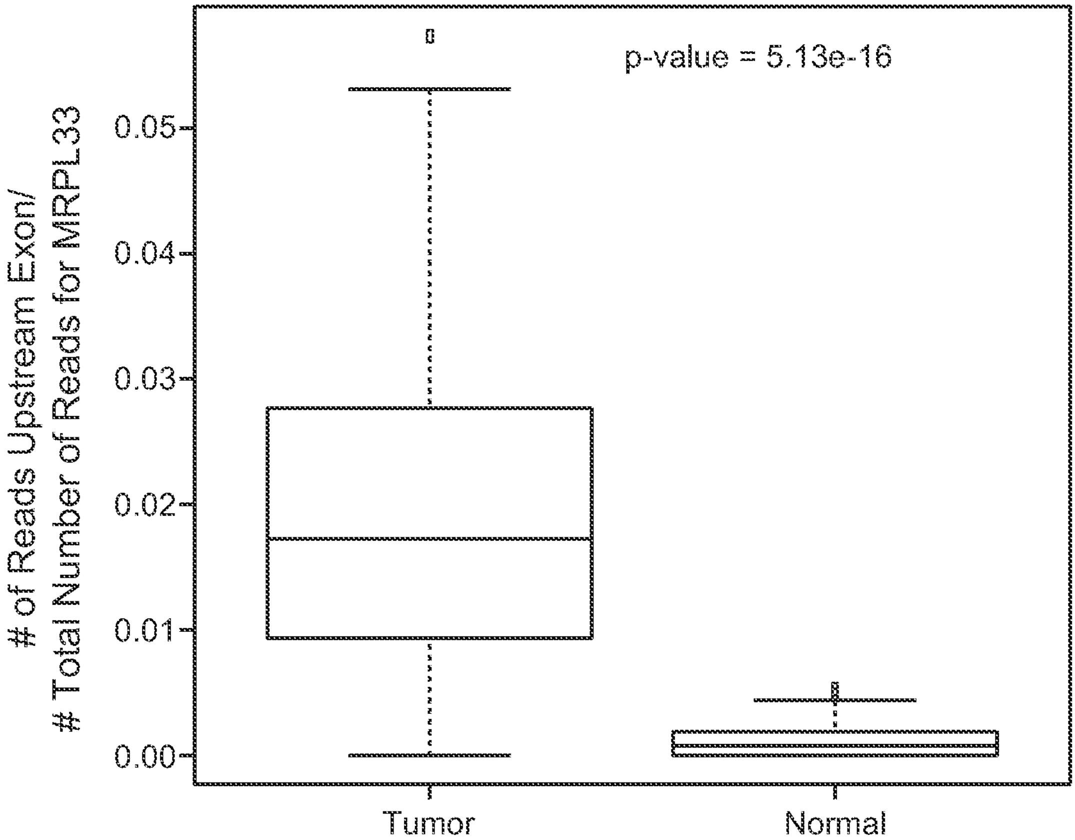


FIG. 1B

| Fusion | Frequency |
|---|---|
| EIF3E(e1)-RSPO2(e2) | 2.9% (2/68) |
| PTPRK(e1)-RSPO3(e2) | 5.9% (4/68) |
| PTPRK(e7)-RSPO3(e2) | 1.5% (1/68) |

FIG. 2A

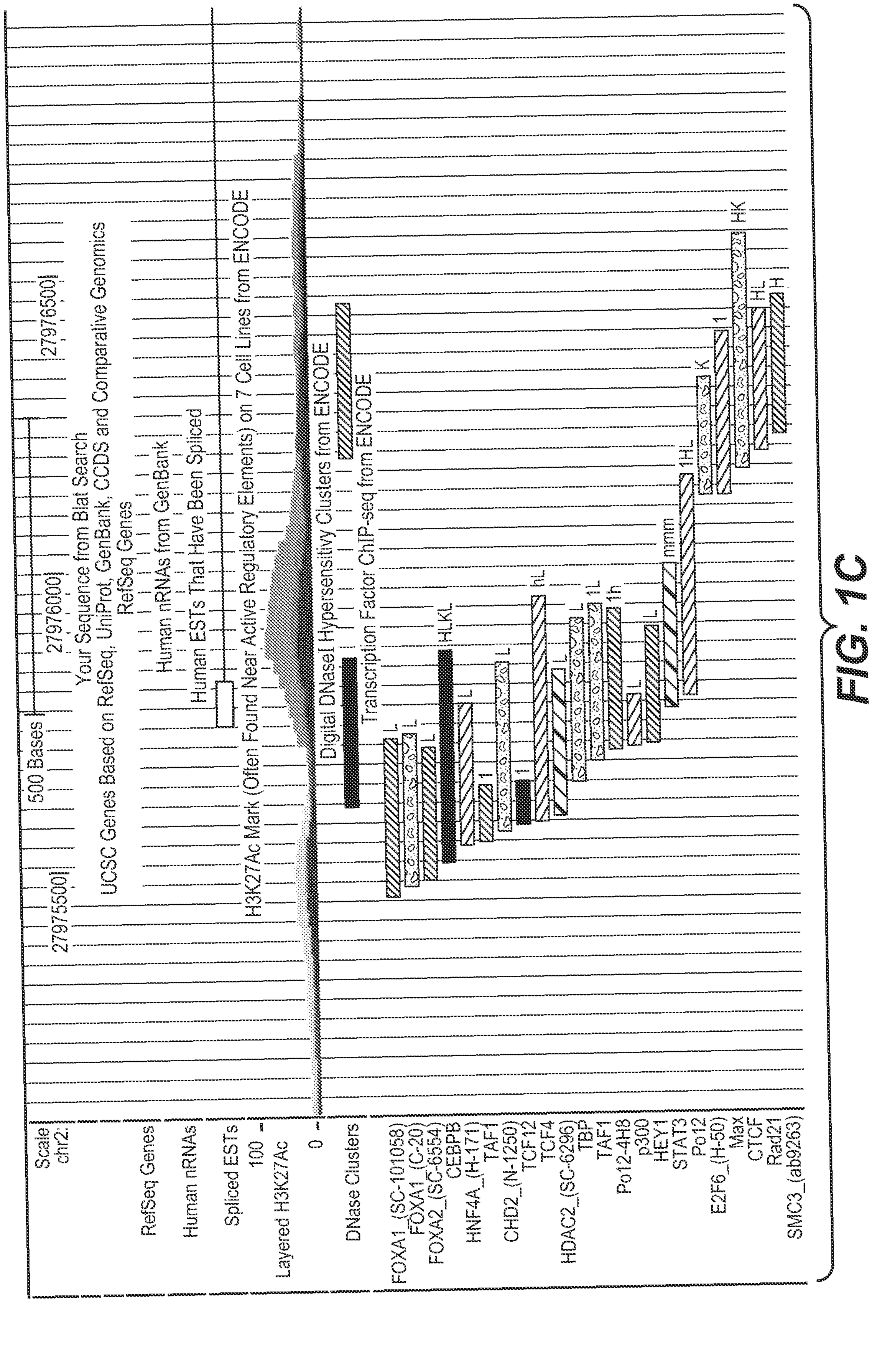
Scale
chr2:
500 Bases
27975500|
27976000|
27976500|
Your Sequence from Blat Search
UCSC Genes Based on RefSeq, UniProt, GenBank, CCDS and Comparative Genomics
RefSeq Genes
Human nRNAs from GenBank
Human ESTs That Have Been Spliced
H3K27Ac Mark (Often Found Near Active Regulatory Elements) on 7 Cell Lines from ENCODE
Digital DNaseI Hypersensitivity Clusters from ENCODE
Transcription Factor ChIP-seq from ENCODE
RefSeq Genes
Human nRNAs
Spliced ESTs
100 _
Layered H3K27Ac
0 _
DNase Clusters
FOXA1_(SC-101058)
FOXA1_(C-20)
FOXA2_(SC-6554)
CEBPB
HNF4A_(H-171)
TAF1
CHD2_(N-1250)
TCF12
TCF4
HDAC2_(SC-6296)
TBP
TAF1
Po12-4H8
p300
HEY1
STAT3
Po12
E2F6_(H-50)
Max
CTCF
Rad21
SMC3_(ab9263)
HLKL
hL
1L
1h
mmm
1HL
K
HL
H
HK

FIG. 1C

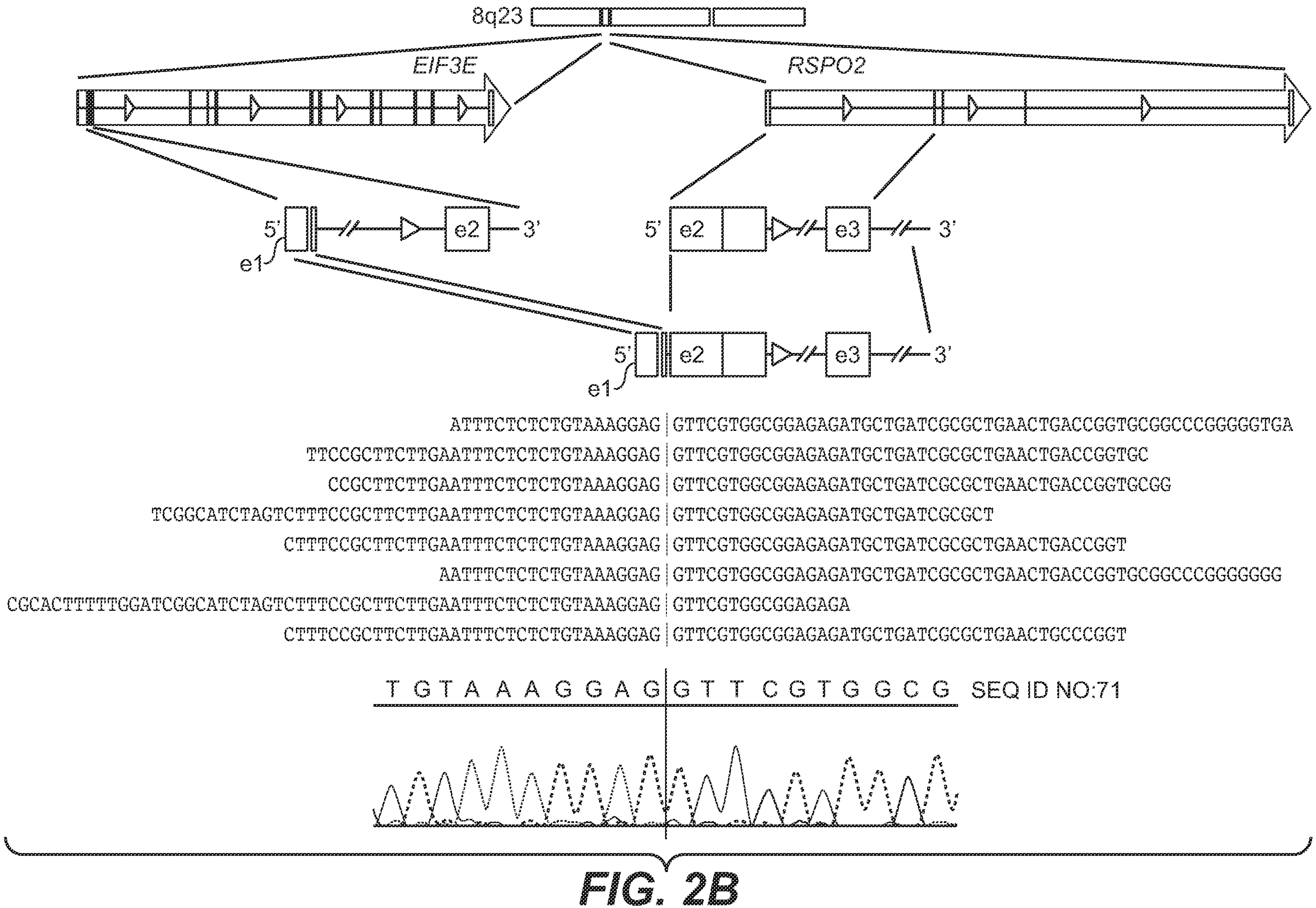
8q23
EIF3E
RSPO2
5'
e1
e2
3'
5'
e2
e3
3'
5'
e1
e2
e3
3'
ATTTCTCTCTGTAAAGGAG GTTCGTGGCGGAGAGATGCTGATCGCGCTGAACTGACCGGTGCGGCCCGGGGGTGA
TTCCGCTTCTTGAATTTCTCTCTGTAAAGGAG GTTCGTGGCGGAGAGATGCTGATCGCGCTGAACTGACCGGTGC
CCGCTTCTTGAATTTCTCTCTGTAAAGGAG GTTCGTGGCGGAGAGATGCTGATCGCGCTGAACTGACCGGTGCGG
TCGGCATCTAGTCTTTCCGCTTCTTGAATTTCTCTCTGTAAAGGAG GTTCGTGGCGGAGAGATGCTGATCGCGCT
CTTTCCGCTTCTTGAATTTCTCTCTGTAAAGGAG GTTCGTGGCGGAGAGATGCTGATCGCGCTGAACTGACCGGT
AATTTCTCTCTGTAAAGGAG GTTCGTGGCGGAGAGATGCTGATCGCGCTGAACTGACCGGTGCGGCCCGGGGGGG
CGCACTTTTTGGATCGGCATCTAGTCTTTCCGCTTCTTGAATTTCTCTCTGTAAAGGAG GTTCGTGGCGGAGAGA
CTTTCCGCTTCTTGAATTTCTCTCTGTAAAGGAG GTTCGTGGCGGAGAGATGCTGATCGCGCTGAACTGCCCGGT
T G T A A A G G A G G T T C G T G G C G
SEQ ID NO:71

FIG. 2B

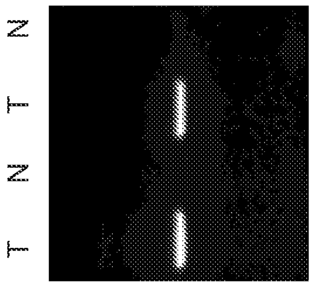


FIG. 2C

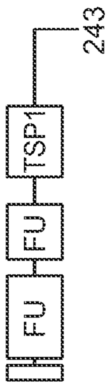


FIG. 2D

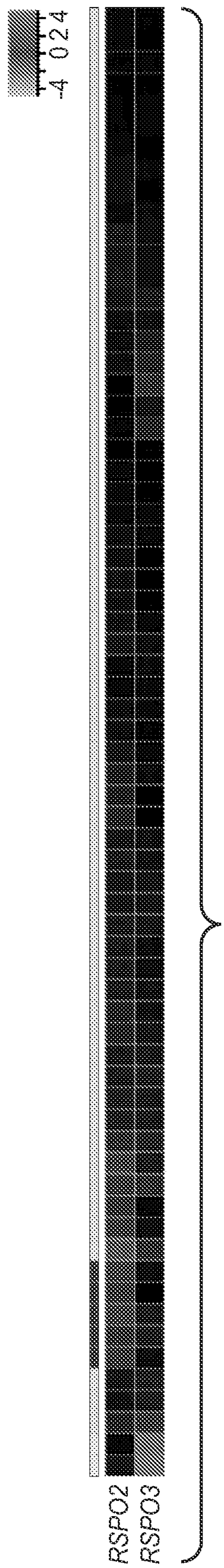


FIG. 2E

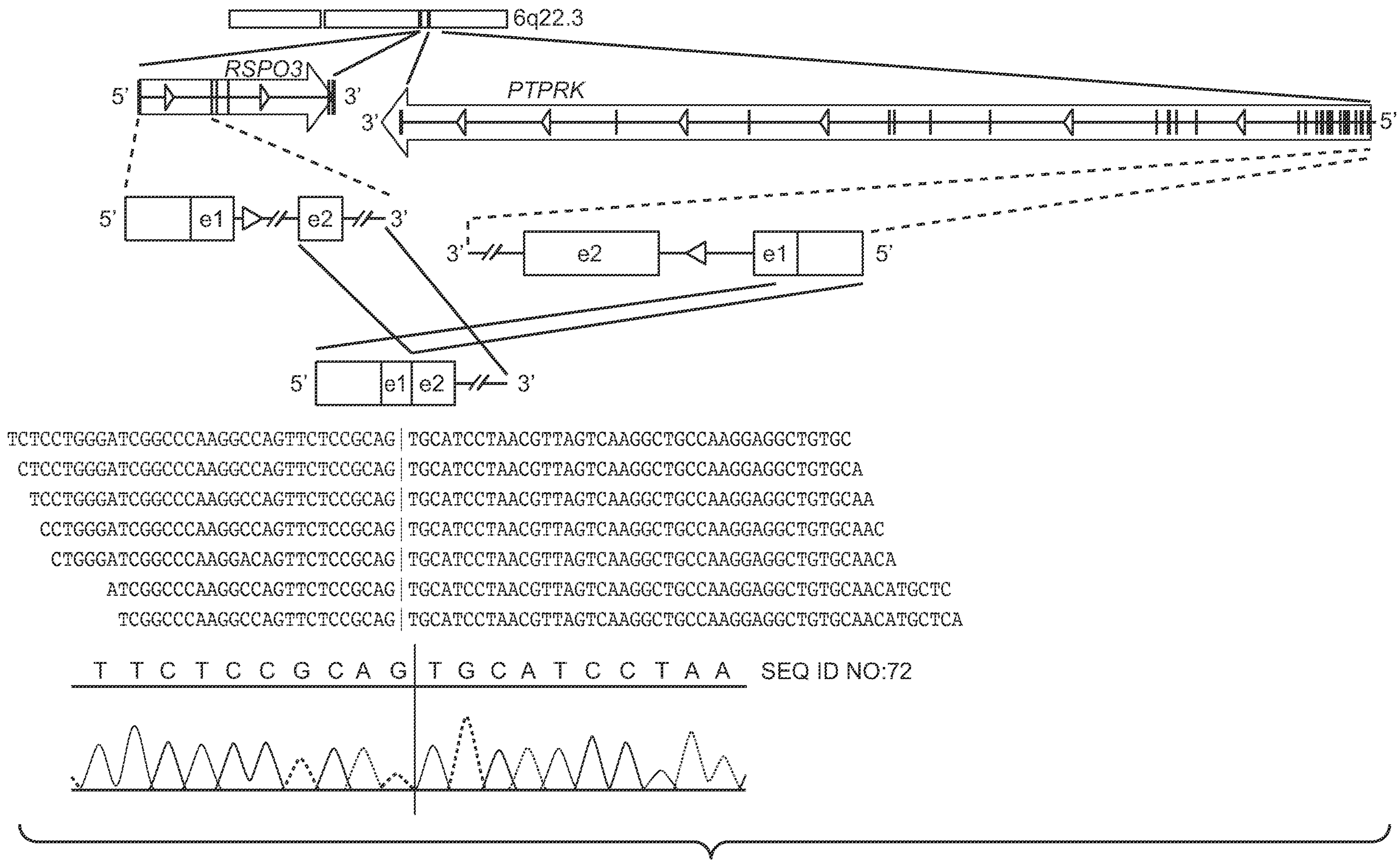
6q22.3
RSPO3
PTPRK
5'
3'
3'
5'
5'
e1
e2
3'
3'
e2
e1
5'
5'
e1
e2
3'
TCTCCTGGGATCGGCCCAAGGCCAGTTCTCCGCAG TGCATCCTAACGTTAGTCAAGGCTGCCAAGGAGGCTGTGC
CTCCTGGGATCGGCCCAAGGCCAGTTCTCCGCAG TGCATCCTAACGTTAGTCAAGGCTGCCAAGGAGGCTGTGCA
TCCTGGGATCGGCCCAAGGCCAGTTCTCCGCAG TGCATCCTAACGTTAGTCAAGGCTGCCAAGGAGGCTGTGCAA
CCTGGGATCGGCCCAAGGCCAGTTCTCCGCAG TGCATCCTAACGTTAGTCAAGGCTGCCAAGGAGGCTGTGCAAC
CTGGGATCGGCCCAAGGACAGTTCTCCGCAG TGCATCCTAACGTTAGTCAAGGCTGCCAAGGAGGCTGTGCAACA
ATCGGCCCAAGGCCAGTTCTCCGCAG TGCATCCTAACGTTAGTCAAGGCTGCCAAGGAGGCTGTGCAACATGCTC
TCGGCCCAAGGCCAGTTCTCCGCAG TGCATCCTAACGTTAGTCAAGGCTGCCAAGGAGGCTGTGCAACATGCTCA
T T C T C C G C A G T G C A T C C T A A
SEQ ID NO:72

FIG. 3A

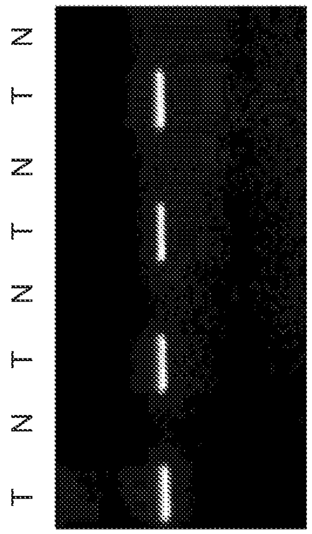
T N T N T N T N

FIG. 3B

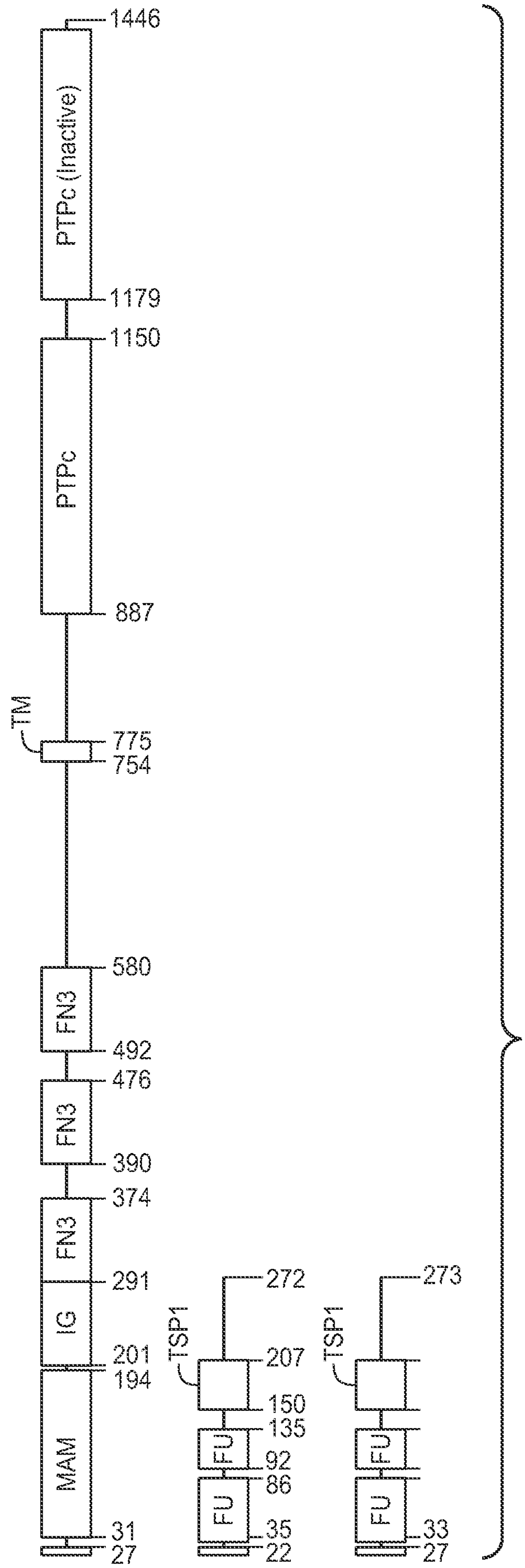
1446
PTPc (Inactive)
1179
1150
PTPc
887
TM
775
754
580
FN3
492
476
FN3
390
374
FN3
291
IG
201
194
MAM
31
27
272
TSP1
207
150
135
FU
92
86
FU
35
22
273
TSP1
FU
FU
33
27

FIG. 3C

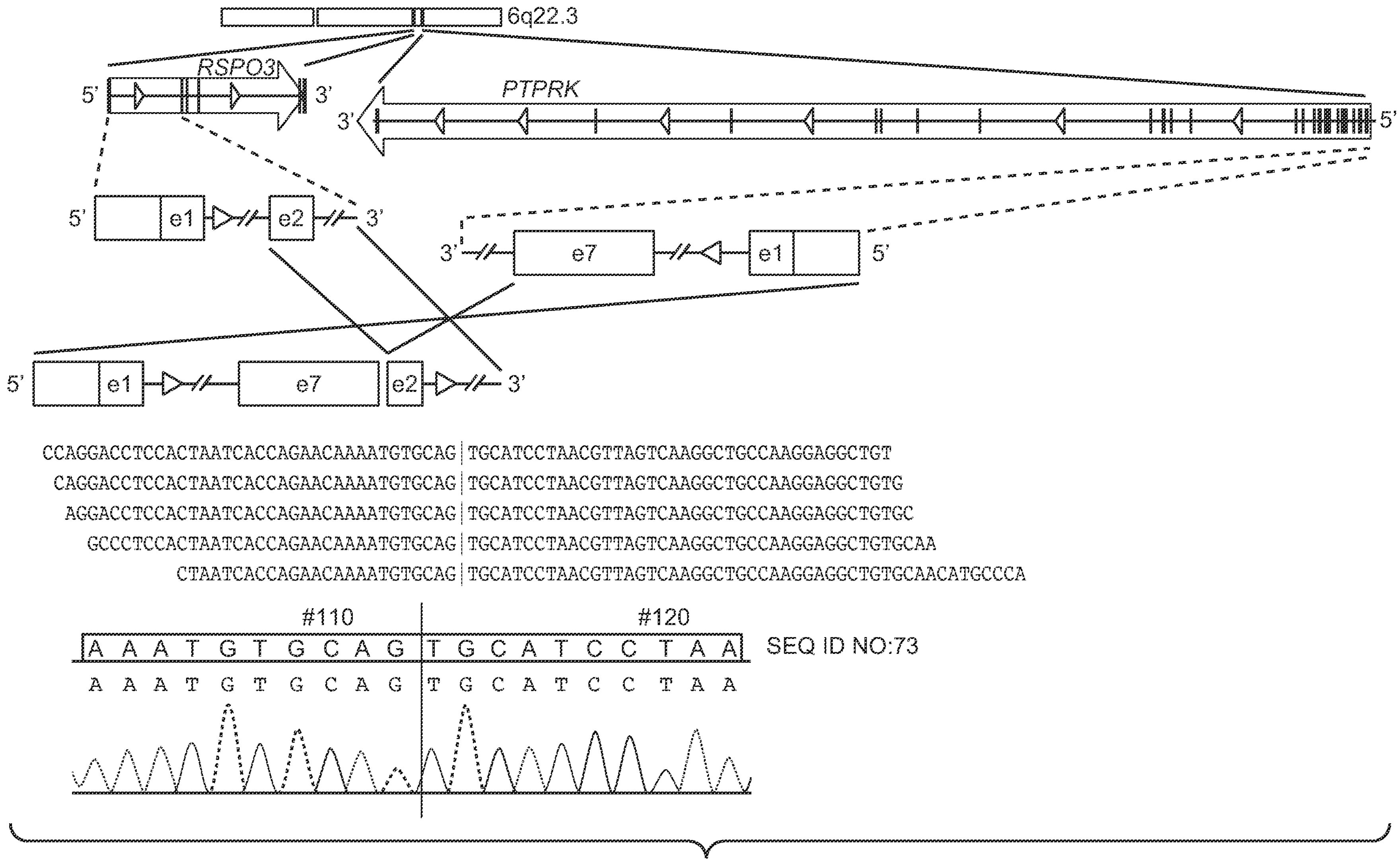
6q22.3
RSPO3
PTPRK
5'
3'
e1
e2
e7
CCAGGACCTCCACTAATCACCAGAACAAAATGTGCAG TGCATCCTAACGTTAGTCAAGGCTGCCAAGGAGGCTGT
CAGGACCTCCACTAATCACCAGAACAAAATGTGCAG TGCATCCTAACGTTAGTCAAGGCTGCCAAGGAGGCTGTG
AGGACCTCCACTAATCACCAGAACAAAATGTGCAG TGCATCCTAACGTTAGTCAAGGCTGCCAAGGAGGCTGTGC
GCCCTCCACTAATCACCAGAACAAAATGTGCAG TGCATCCTAACGTTAGTCAAGGCTGCCAAGGAGGCTGTGCAA
CTAATCACCAGAACAAAATGTGCAG TGCATCCTAACGTTAGTCAAGGCTGCCAAGGAGGCTGTGCAACATGCCCA
#110
#120
A A A T G T G C A G T G C A T C C T A A
SEQ ID NO:73
A A A T G T G C A G T G C A T C C T A A

FIG. 4A

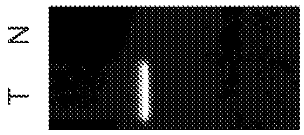
T
N

FIG. 4B

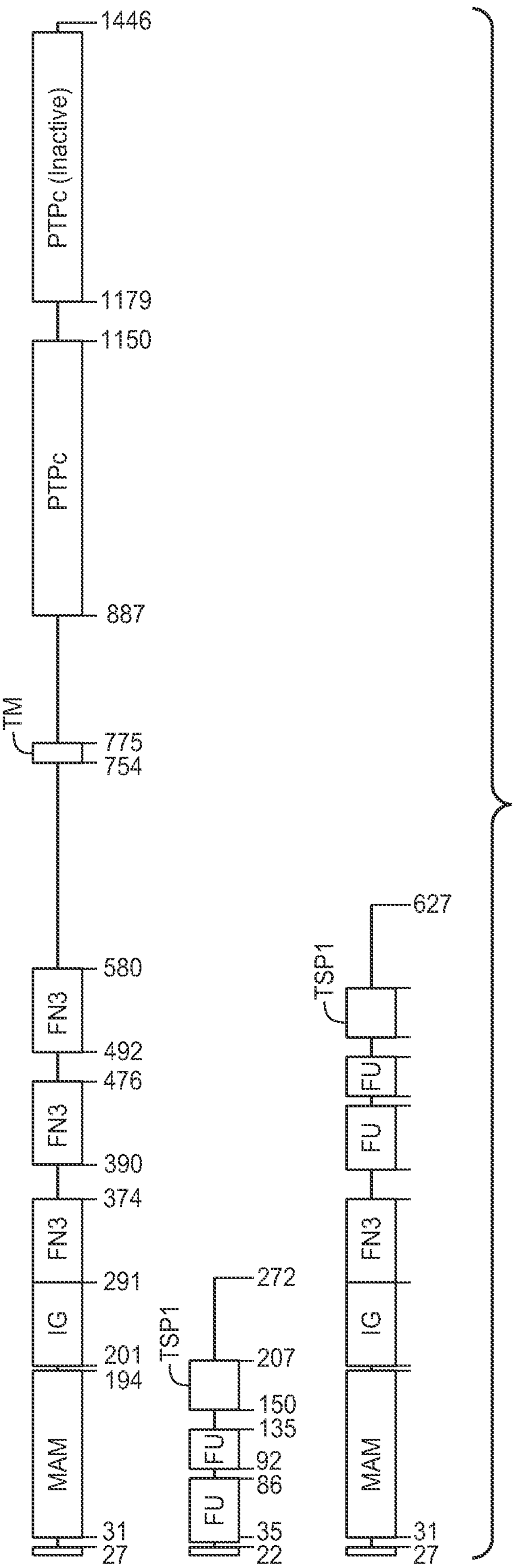
PTPc (Inactive)
PTPc
TM
FN3
FN3
FN3
IG
MAM
1446
1179
1150
887
775
754
580
492
476
390
374
291
201
194
31
27
TSP1
FU
FU
272
207
150
135
92
86
35
22
TSP1
FU
FU
FN3
IG
MAM
627
31
27

FIG. 4C

FIG. 5A

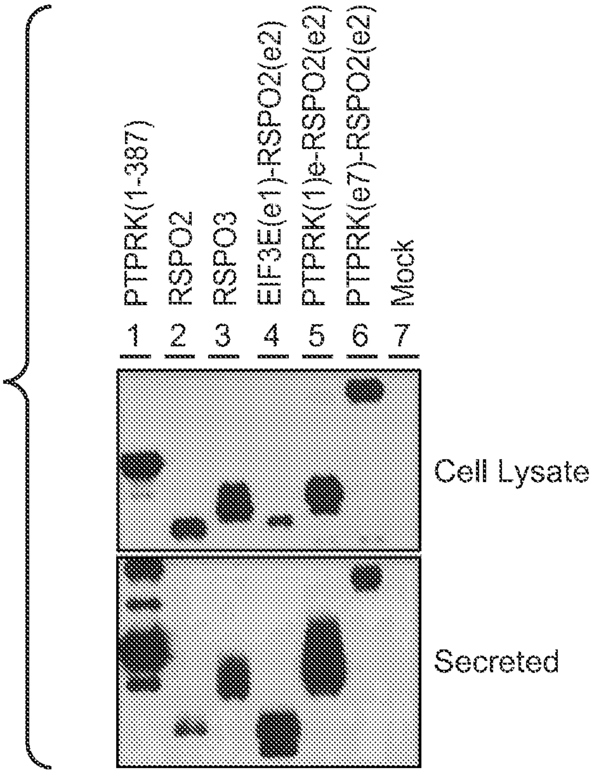
1 PTPRK(1-387)
2 RSPO2
3 RSPO3
4 EIF3E(e1)-RSPO2(e2)
5 PTPRK(1)e-RSPO2(e2)
6 PTPRK(e7)-RSPO2(e2)
7 Mock
Cell Lysate
Secreted

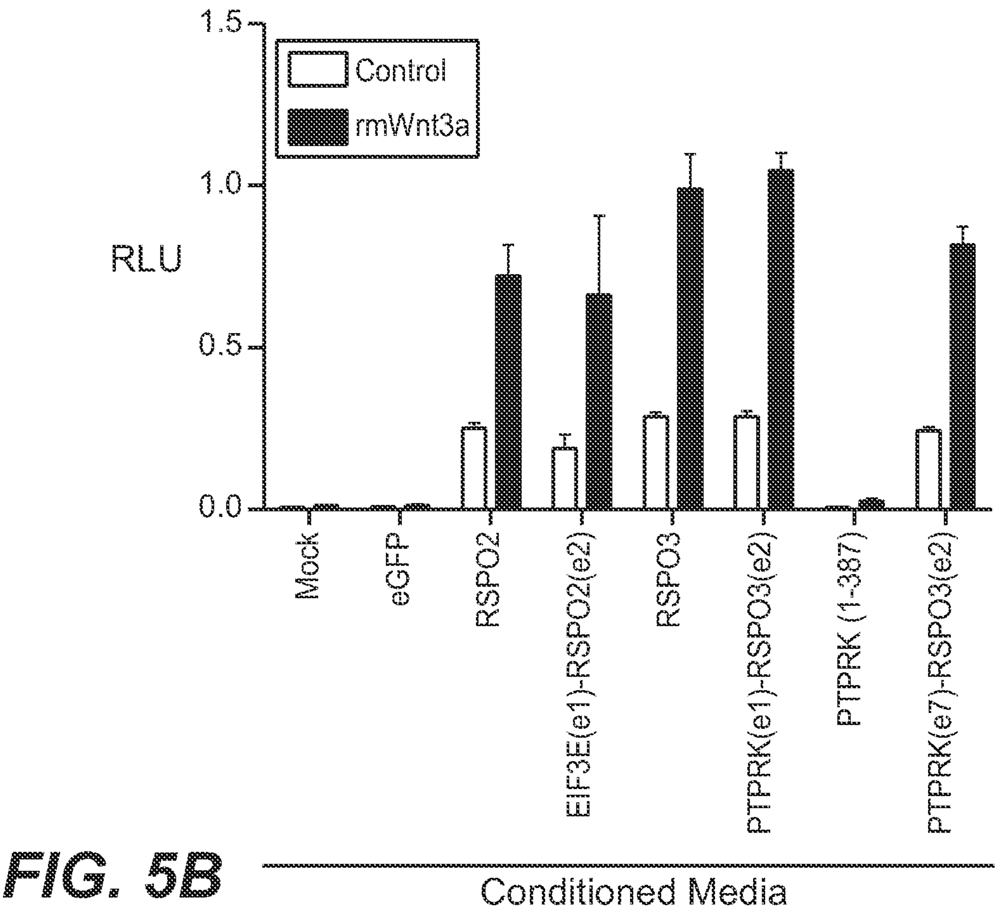
1.5
1.0
0.5
0.0
RLU
Control
rmWnt3a
Mock
eGFP
RSPO2
EIF3E(e1)-RSPO2(e2)
RSPO3
PTPRK(e1)-RSPO3(e2)
PTPRK (1-387)
PTPRK(e7)-RSPO3(e2)
Conditioned Media

FIG. 5B

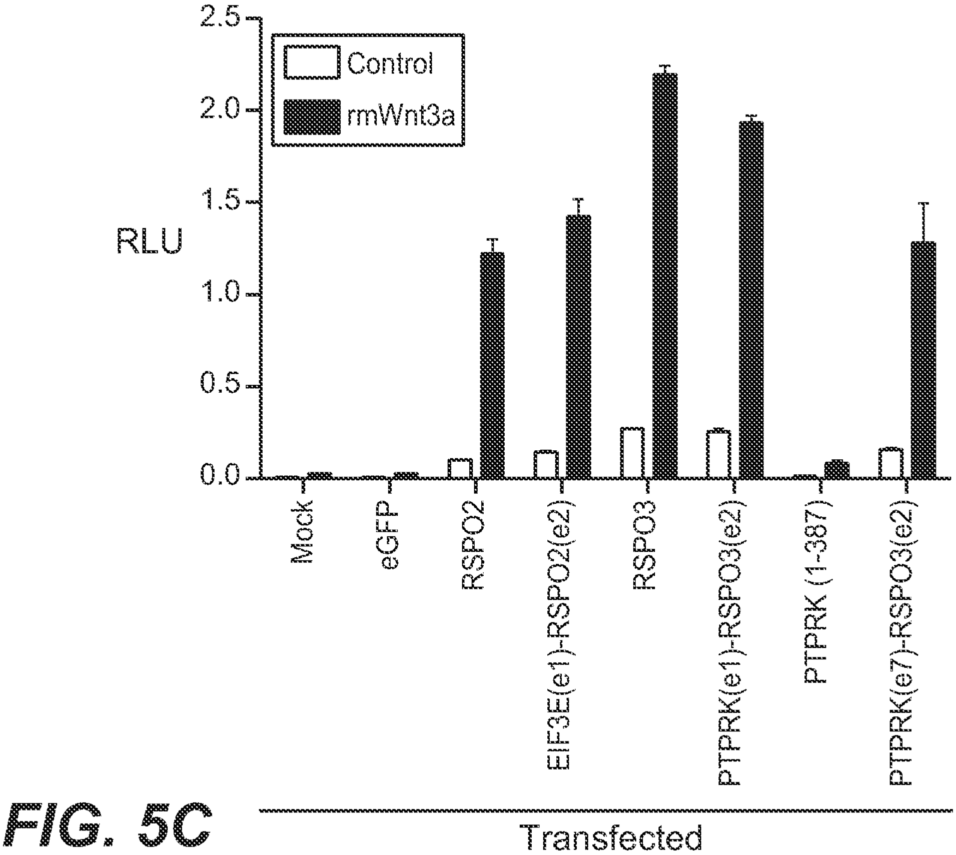
2.5
2.0
1.5
1.0
0.5
0.0
RLU
Control
rmWnt3a
Mock
eGFP
RSPO2
EIF3E(e1)-RSPO2(e2)
RSPO3
PTPRK(e1)-RSPO3(e2)
PTPRK (1-387)
PTPRK(e7)-RSPO3(e2)
Transfected

FIG. 5C

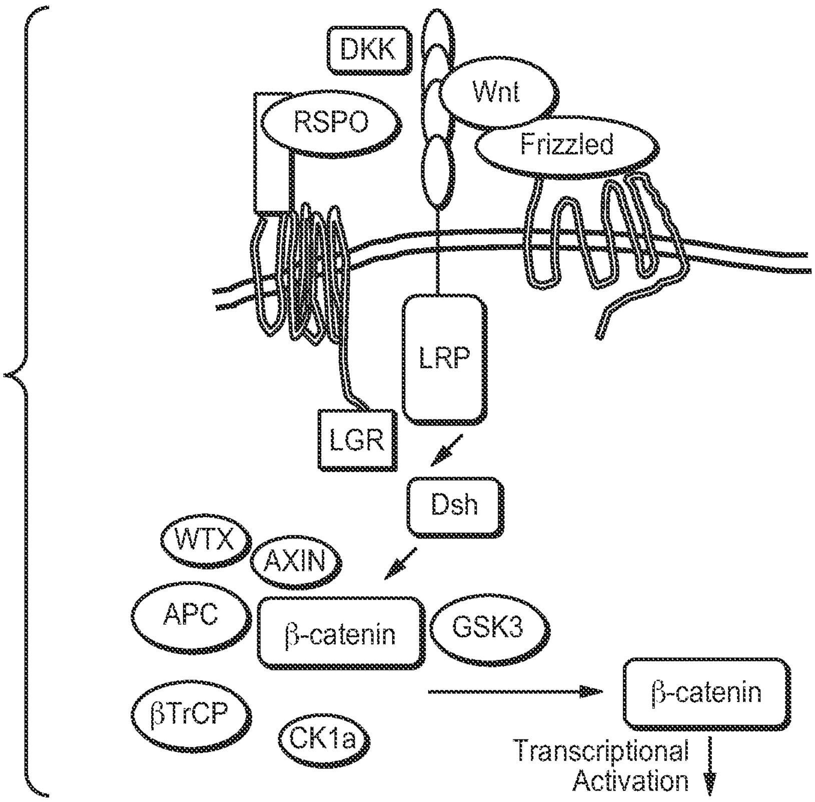
DKK
Wnt
RSPO
Frizzled
LRP
LGR
Dsh
WTX
AXIN
APC
β-catenin
GSK3
β-catenin
βTrCP
CK1a
Transcriptional
Activation

FIG. 5D

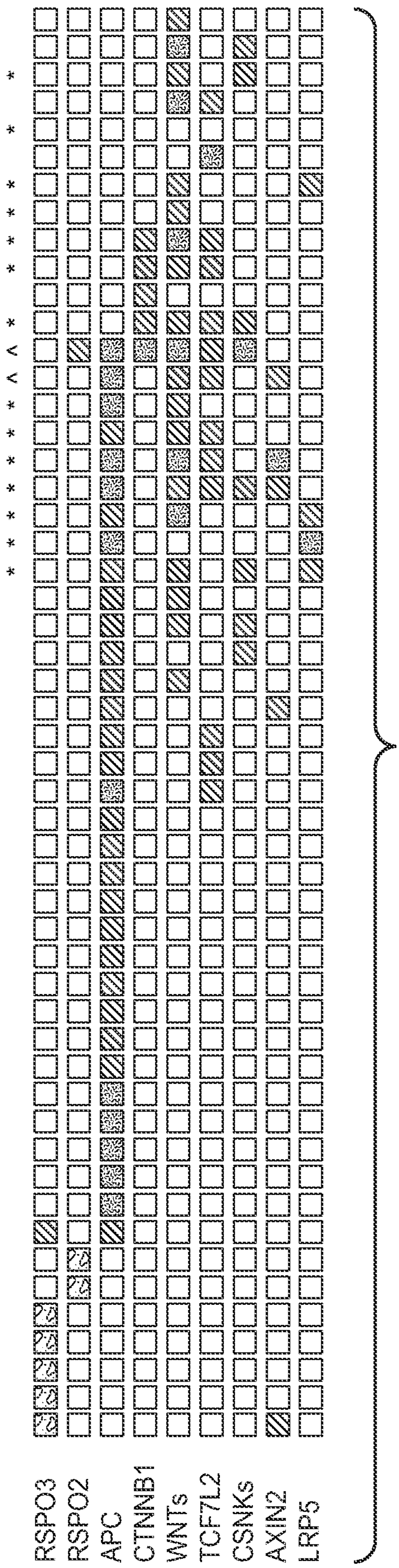


FIG. 5E

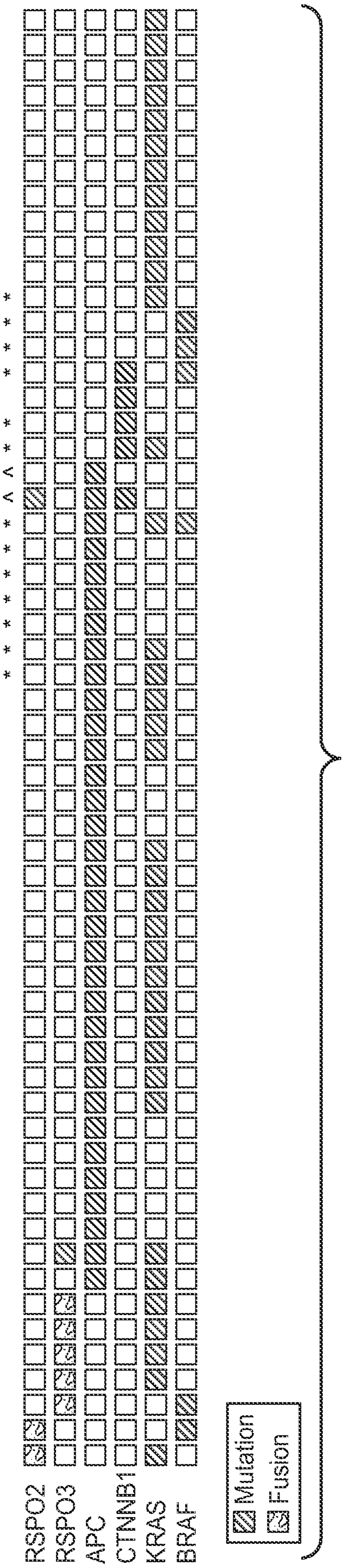
RSPO2
RSPO3
APC
CTNNB1
KRAS
BRAF
Mutation
Fusion

FIG. 6A

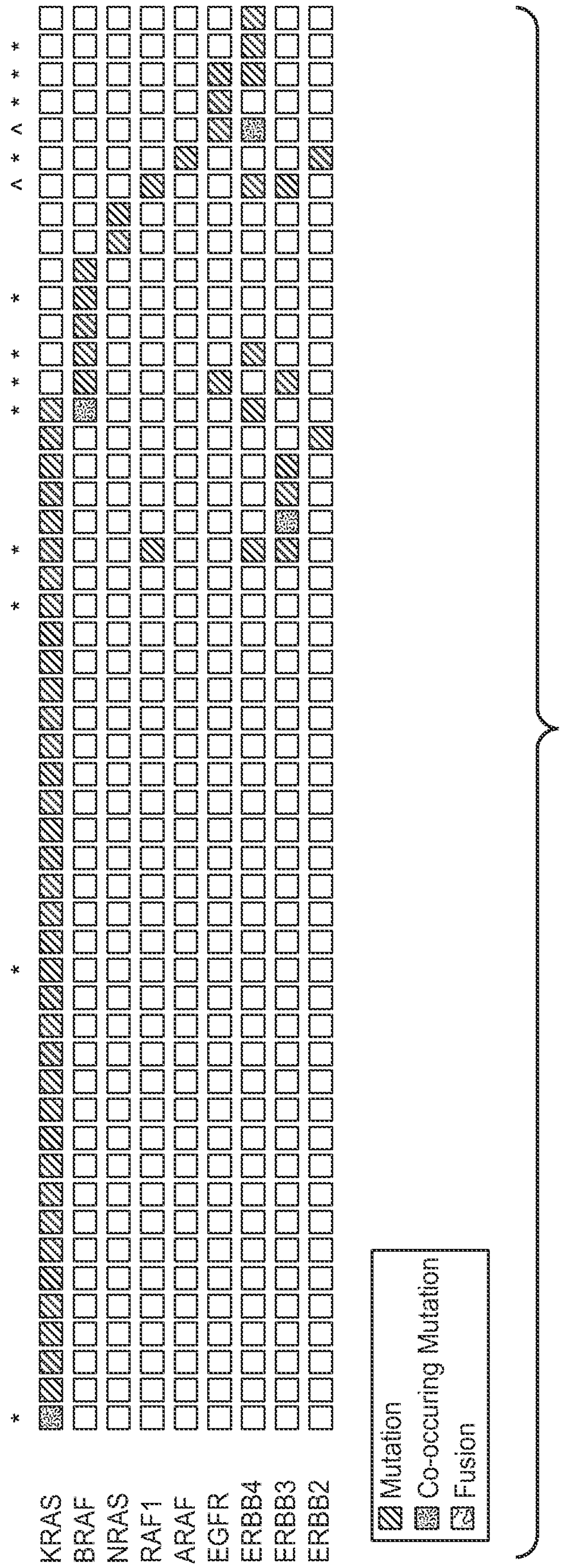
KRAS
BRAF
NRAS
RAF1
ARAF
EGFR
ERBB4
ERBB3
ERBB2
Mutation
Co-occuring Mutation
Fusion

FIG. 6B

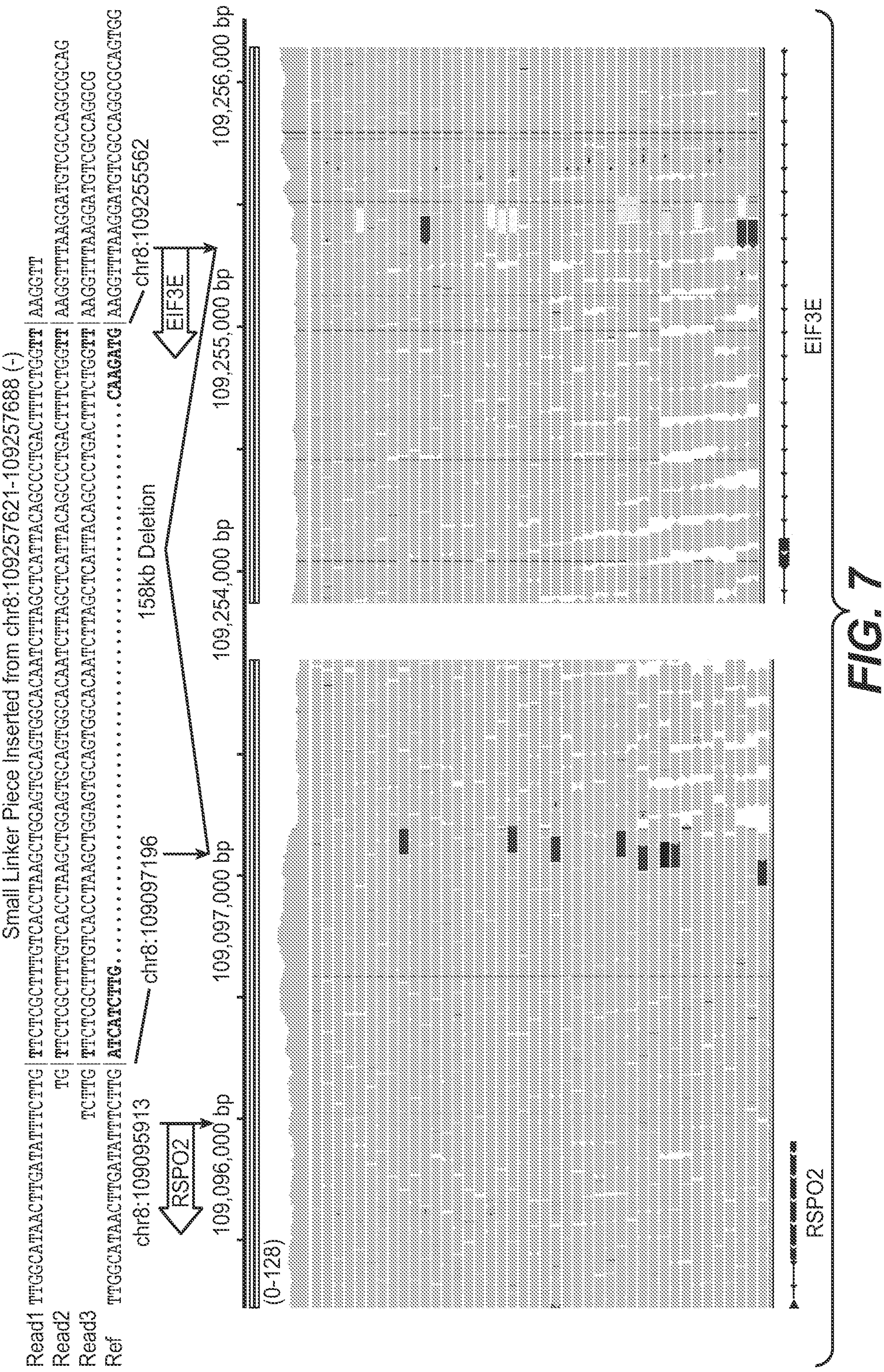
EIF3E-RSPO2
Small Linker Piece Inserted from chr8:109257621-109257688 (-)
Read1 TTGGCATAACTTGATATTTCTTG TTCTCGCTTTGTCACCTAAGCTGGAGTGCAGTGCAGTGGCACAATCTTAGCTCATTACAGCCCTGACTTTCTGGTT AAGGTT
Read2 TG TTCTCGCTTTGTCACCTAAGCTGGAGTGCAGTGCAGTGGCACAATCTTAGCTCATTACAGCCCTGACTTTCTGGTT AAGGTTTAAGGATGTCGCCAGGGCAG
Read3 TCTTG TTCTCGCTTTGTCACCTAAGCTGGAGTGCAGTGCAGTGGCACAATCTTAGCTCATTACAGCCCTGACTTTCTGGTT AAGGTTTAAGGATGTCGCCAGGCG
Ref TTGGCATAACTTGATATTTCTTG ATCATCTTG..........................CAAGATG AAGGTTTAAGGATGTCGCCAGGGCAGTGG
chr8:109095913
chr8:109097196
158kb Deletion
chr8:109255562
RSPO2
EIF3E
109,096,000 bp
109,097,000 bp
109,254,000 bp
109,255,000 bp
109,256,000 bp
(0-128)
RSPO2
EIF3E

FIG. 7

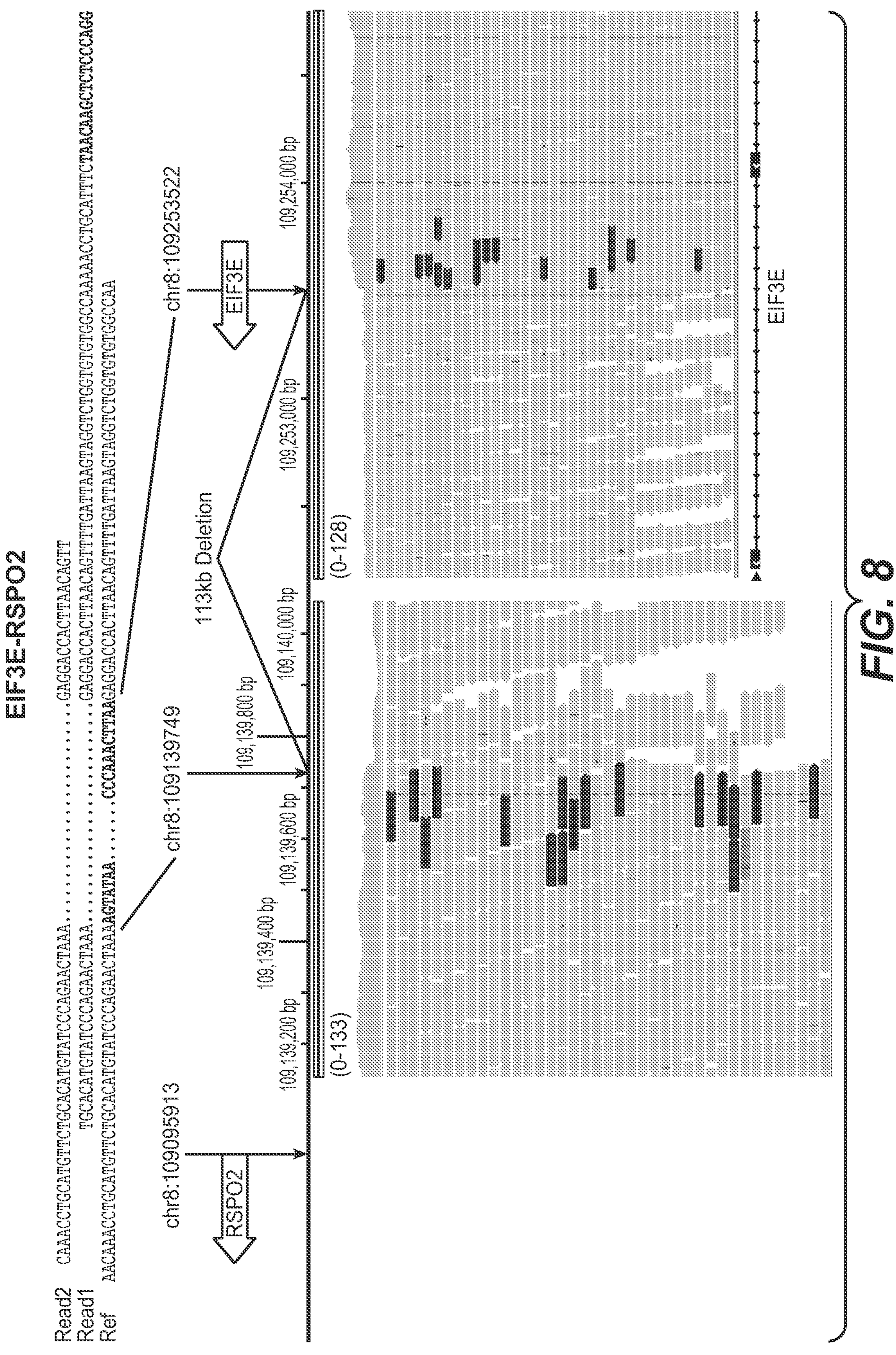
EIF3E-RSPO2
Read2
Read1
Ref
chr8:109095913
chr8:109139749
chr8:109253522
RSPO2
EIF3E
113kb Deletion
109,139,200 bp
109,139,400 bp
109,139,600 bp
109,139,800 bp
109,140,000 bp
109,253,000 bp
109,254,000 bp
(0-133)
(0-128)
EIF3E

FIG. 8

PTPRK-RSPO3

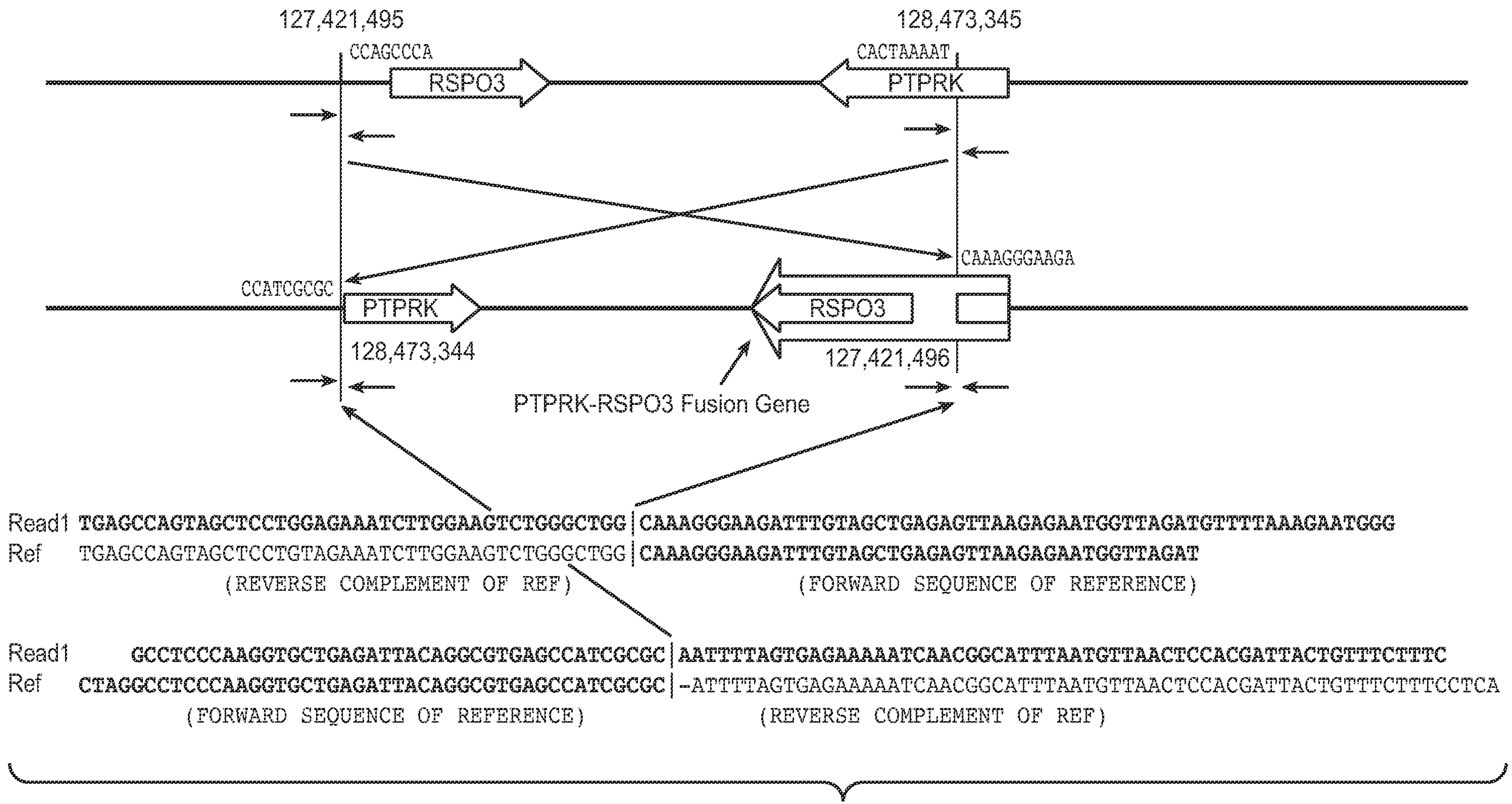


*FIG. 9*

**PTPRK-RSPO3**

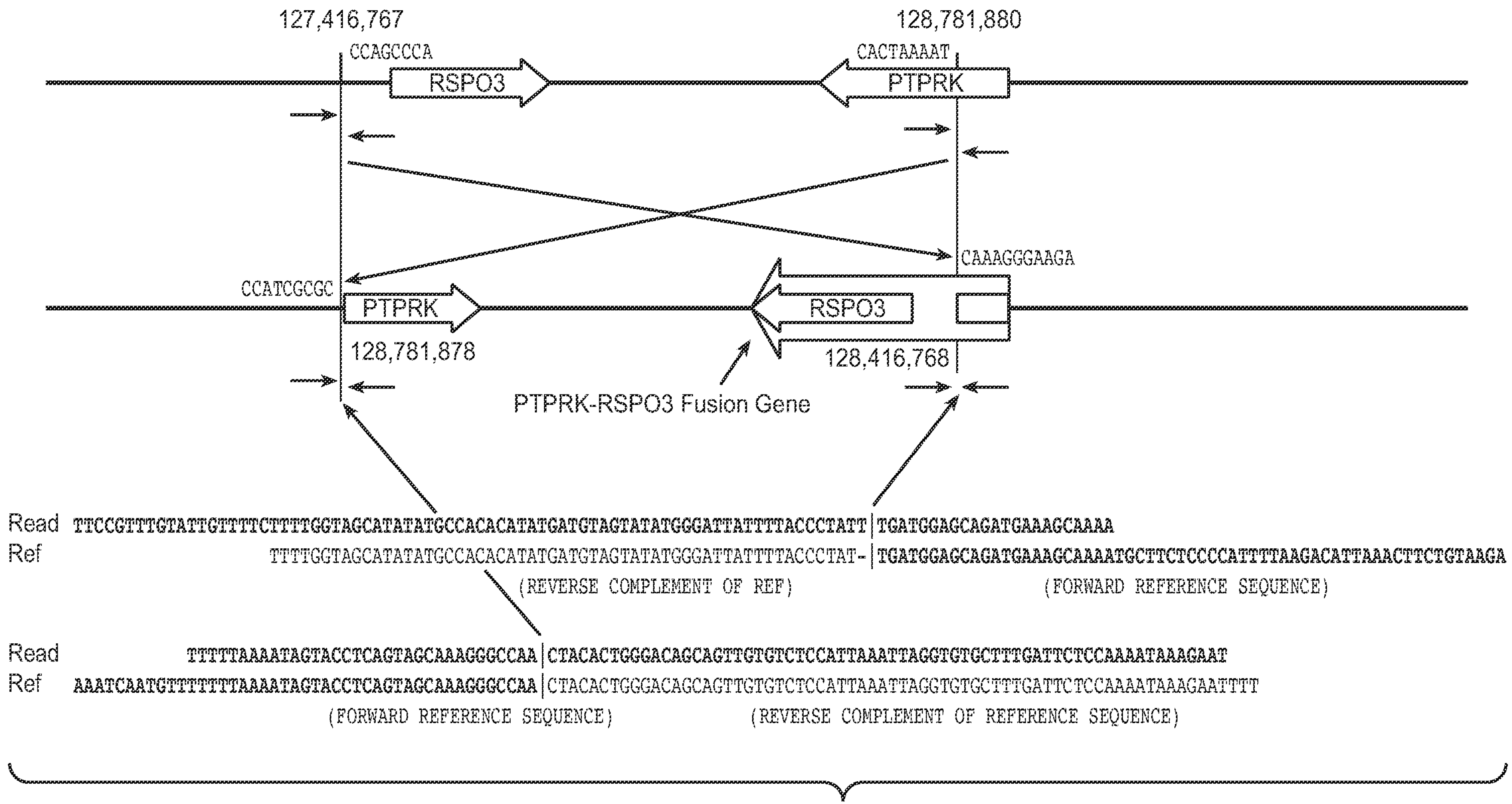


*FIG. 10*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4275149 A
- US 4737456 A
- US 4318980 A
- US 4016043 A
- US 4424279 A
- US 4018653 A
- WO 2008046649 A
- WO 2008020942 A
- WO 2007013666 A
- WO 2005040418 A
- WO 2009005809 A
- US 8088374 B
- US 7541431 B
- WO 2011076932 A
- US 20090074782 A
- US 20110256127 A
- US 7947277 B
- US 7173115 B
- US 7741439 B
- US 7855278 B
- US 7566536 B
- US 7005499 B
- US 8158757 B
- US 8158758 B
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- WO 2004056312 A, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S
- US 5500362 A
- WO 2006029879 A
- WO 2005100402 A
- US 6737056 B
- US 7332581 B
- US 6194551 B
- WO 9951642 A
- US 20050014934 A1, Hinton
- US 7371826 B
- US 5648260 A
- US 5624821 A
- WO 9429351 A
- US 7521541 B
- US 5208020 A
- US 5416064 A
- EP 0425235 B1

- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A
- US 7977064 B
- US 7695928 B
- US 6387657 B
- US 7455834 B
- US 7732567 B
- US 7687460 B
- US 7101850 B
- US 20060292150 A
- US 5556762 A
- US 4708871 A
- US 4833092 A
- US 5223409 A
- US 5403484 A
- US 5571689 A
- US 5663143 A
- WO 8403506 A
- WO 8403564 PCT
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 0000823 A
- WO 0039585 PCT
- US 5648237 A
- US 5789199 A
- US 5840523 A
- US 5959177 A
- US 6040498 A
- US 6420548 B
- US 7125978 B
- US 6417429 B
- US 20050260186 A
- US 20060104968 A
- US 6267958 B
- US 6171586 B
- WO 2006044908 A
- WO 61674763 A
- WO 61597746 A

**Non-patent literature cited in the description**

- **SIEGEL, R. et al.** *CA: A Cancer Journal for Clinicians,* 2011, vol. 61, 212-236
- **FEARON, E. R.** *Annu. Rev. Pathol.,* 2011, vol. 6, 479-507
- **WOOD, L. D. et al.** *Science,* 2007, vol. 318, 1108-1113
- **TIMMERMANN, B. et al.** *PloS One,* 2010, vol. 5, e15661
- **STARR, T. K. et al.** *Science,* 2009, vol. 323, 1747-1750
- **MARCH, H. N. et al.** *Nat. Genet.,* 2011, vol. 43, 1202-1209
- **FLATMAN et al.** *J. Chromatogr. B,* 2007, vol. 848, 79-87
- **KABAT et al.** Sequences of Proteins of Immunological Interest. Public Health Service, National Institutes of Health, 1991
- **KABAT et al.** Sequences of Proteins of Immunological Interest. 1991, vol. 1-3
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **ALMAGRO ; FRANSSON.** *Front. Biosci.,* 2008, vol. 13, 1619-1633
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **NICOLAOU et al.** *Angew. Chem Intl. Ed. Engl.,* 1994, vol. 33, 183-186
- Prodrugs in Cancer Chemotherapy. **WILMAN.** Biochemical Society Transactions. 1986, vol. 14, 375-382

- Prodrugs: A Chemical Approach to Targeted Drug Delivery. **STELLA et al.** Directed Drug Delivery. Humana Press, 1985, 247-267
- Cell cycle regulation, oncogenes, and antineoplastic drugs. **MURAKAMI et al.** The Molecular Basis of Cancer. WB Saunders, 1995
- Current Protocols In Molecular Biology. 1995
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natl. Acad. Sci. USA,* 1993, vol. 90, 6444-6448
- **MORRISON et al.** *Proc. Natl. Acad. Sci. USA,* 1984, vol. 81, 6851-6855
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Experimental and Clin. Pharma.,* 2005, vol. 27 (3), 185-91
- **HOOGENBOOM et al.** METHODS IN MOL. BIOL. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** METHODS IN MOL. BIOL. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natl. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Natl. Acad. Sci. USA,* 1998, vol. 95, 652-656
- **GAZZANO-SANTORO et al.** *J. Immunol. Methods,* 1996, vol. 202, 163

- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117 (587
- **KIM et al.** *J. Immunol.,* 1994, vol. 24, 249
- **DUNCAN ; WINTER.** *Nature,* 1988, vol. 322, 738-40
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natl. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI.** *Cancer Res.,* 1992, vol. 52, 127-131
- **GEYSEN et al.** *Proc. Natl. Acad. Sci. U.S.A.,* 1984, vol. 81, 3998-4002
- **GEYSEN et al.** *Proc. Natl. Acad. Sci. U.S.A.,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol,* 1991, vol. 222, 581
- **KANG, A.S. et al.** *Proc. Natl. Acad. Sci. USA,* 1991, vol. 88, 8363
- **SMITH, G. P.** *Current Opin. Biotechnol.,* 1991, vol. 2, 668
- **SCOTT, J.K. ; SMITH, G. P.** *Science,* 1990, vol. 249, 386
- **MARKS, J. D. et al.** *J. Mol. Biol.,* 1991, vol. 222, 581
- **REN et al.** *Gene,* 1998, vol. 215, 439
- **ZHU et al.** *Cancer Research,* 1998, vol. 58 (15), 3209-3214
- **JIANG et al.** *Infection & Immunity,* 1997, vol. 65 (11), 4770-4777
- **REN et al.** *Gene,* 1997, vol. 195 (2), 303-311
- **REN.** *Protein Sci.,* 1996, vol. 5, 1833
- **EFIMOV et al.** *Virus Genes,* 1995, vol. 10, 173
- **SMITH ; SCOTT.** *Methods in Enzymology,* 1993, vol. 217, 228-257
- **LI et al.** *Mol Biotech.,* 1998, vol. 9, 187
- **WAGNER, R.** *Nature,* 1994, vol. 372, 333-335
- **BERNOIST ; CHAMBON.** *Nature,* 1981, vol. 29, 304-310
- **YAMAMOTO et al.** *Cell,* 1980, vol. 22, 787-797
- **WAGNER et al.** *Proc. Natl. Acad. Sci. U.S.A.,* 1981, vol. 78, 1441-1445
- **BRINSTER et al.** *Nature,* 1982, vol. 296, 39-42
- **CHOWDHURY.** *Methods Mol. Biol.,* 2008, vol. 207, 179-196
- **CUNNINGHAM ; WELLS.** *Science,* 1989, vol. 244, 1081-1085
- **KAM et al.** *Proc. Natl. Acad. Sci. USA,* 2005, vol. 102, 11600-11605
- **CHARLTON.** METHODS IN MOL. BIOL. Humana Press, 2003, vol. 248, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36 (59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** METHODS IN MOL. BIOL. Humana Press, 2003, vol. 248, 255-268
- **STEWART et al.** Solid-Phase Peptide Synthesis. W.H. Freeman Co, 1969
- **MERRIFIELD.** *J. Am. Chem. Soc.,* 1963, vol. 85, 2149-2154
- Antibodies, A Laboratory Manual. Cold Spring Harbor Laboratory, 1988
- REMINGTON'S PHARMA. SCI. 1980
- **MORGAN, M. et al.** *Bioinformatics,* 2009, vol. 25, 2607-2608
- **LI, H. ; DURBIN, R.** *Bioinformatics,* 2009, vol. 25, 1754-1760
- **DEPRISTO, M. A. et al.** *Nat. Genet.,* 2011, vol. 43, 491-498
- **SHERRY, S. T. et al.** *Nucleic Acids Res,* 2001, vol. 29, 308-311
- **FORBES, S. A. et al.** *Nucleic Acids Res,* 2010, vol. 38, D652-657

- **KAN, Z. et al.** *Nature,* 2010, vol. 466, 869-873
- **WU, T. D. ; NACU, S.** *Bioinformatics,* 2010, vol. 26, 873-881
- **NG, P. C. ; HENIKOFF, S.** *Genome Res,* 2002, vol. 12, 436-446
- **RAMENSKY, V. ; BORK, P. ; SUNYAEV, S.** *Nucleic Acids Res,* 2002, vol. 30, 3894-3900
- **SUBRAMANIAN A. et al.** *Proc. of the Natl Acad. Of Sci. USA,* 2005, vol. 102, 15545-15550
- **CHEN, K. et al.** *Nat. Methods,* 2009, vol. 6, 677-681
- **KRZYWINSKI, M. et al.** *Genome Res.,* 2009, vol. 19, 1639-1459
- **WU, T.D. ; NACU, S.** *Bioinformatics,* 2010, vol. 26, 873-881
- **ANDERS, S. ; HUBER, W.** *Genome Biology,* 2010, vol. 11, R106
- **GREENMAN, C. D. et al.** *Biostatistics,* 2010, vol. 11, 164-175
- **VENKATRAMAN, E. S. ; OLSHEN, A. B.** *Bioinformatics,* 2007, vol. 23, 657-663
- **MERMEL, C. H. et al.** *Genome Biology,* 2011, vol. 12, R41
- **NACU, S. et al.** *BMC Med Genomics,* 2011, vol. 4, 11
- **WU, T. D. ; WATANABE, C. K.** *Bioinformatics,* 2005, vol. 21, 1859-1875
- *Nature Chem. Biol.,* 2009, vol. 5, 217-219
- **FORBES, S. A. et al.** *Nucleic Acids Res.,* 2010, vol. 38, D652-657
- **SJOBLOM, T. et al.** *Science,* 2006, vol. 314, 268-274
- **BASS, A. J. et al.** *Nat. Genet.,* 2011, vol. 43, 964-968
- **RAMENSKY, V. et al.** *Nucleic Acids Res,* 2002, vol. 30, 3894-3900
- **YUE, P. et al.** *Hum. Mutat.,* 2010, vol. 31, 264-271
- **LEE, C. C. et al.** *J. of Biol. Chem.,* 2011, vol. 287, 2798-2809
- **MOHR, F. et al.** *Exp. Hematol.,* 2011, vol. 39, 272-281
- **KANEDA, H. et al.** *Cancer Res.,* 2010, vol. 70, 2053-2063
- **PICHIORRI, F. et al.** *Future Oncol.,* 2008, vol. 4, 815-824
- **OZSOLAK, F. ; MILOS, P. M.** *Nature Rev Genet.,* 2011, vol. 12, 87-98
- **YOON, J. K. ; LEE, J. S.** *Cell Signal.,* 2012, vol. 24 (2), 369-77
- **CARMON, K. S. et al.** *Proceedings of the National Academy of Sciences of the United States of America,* 2011, vol. 108, 11452-11457
- **LAU, W. et al.** *Nature,* 2011, vol. 476, 293-297
- **GLINKA, A. et al.** *EMBO Reports,* 2011, vol. 12, 1055-1061
- **DE LAU, W. et al.** *Nature,* 2011, vol. 476, 293-297